# EUROPEAN PATENT SPECIFICATION

(11) **EP 1 509 537 B1**
(45) Date of publication and mention of the grant of the patent: **18.07.2007**
(21) Application number: 03747326.1
(22) Date of filing: 25.04.2003
(51) Int. Cl.: C07F 9/40, C07F 9/02, C07F 9/38

(54) **CELLULAR ACCUMULATION OF PHOSPHONATE ANALOGS OF HIV PROTEASE INHIBITOR COMPOUNDS AND THE COMPOUNDS AS SUCH**
ANREICHERUNG IN DER ZELLE AN PHOSPHONAT ANALOGA VON HIV PROTEASE INHIBITOR VERBINDUNGEN UND DIE VERBINDUNGEN SELBST
ACCUMULATION CELLULAIRE D' ANALOGUES DE PHOSPHONATE DE COMPOSES INHIBITEURS DE LA PROTEASE DU VIH

(30) Priority: 26.04.2002 US 375665 P; 26.04.2002 US 375834 P; 26.04.2002 US 375779 P; 26.04.2002 US 375622 P
(43) Date of publication of application: 02.03.2005
(73) Proprietor: GILEAD SCIENCES, INC., Foster City, California 94404 (US)
(72) Inventor: ARIMILLI, Murty, N., Greensboro, NC 27409 (US); BECKER, Mark, W., Redwood City,CA 94065 (US); BRYANT, Clifford, Millbrae, CA 94030 (US); CHEN, James, M., San Ramon, CA 94583 (US); CHEN, Xiaowu, San Mateo, CA 94403 (US); DASTGAH, Azar, Sunnyvale, CA 94085 (US); FARDIS, Maria, San Carlos, CA 94070 (US); HE, Gong-Xin, Fremont, CA 94555 (US); JIN, Haolun, Foster City, CA 94404 (US); KIM, Choung, U., San Carlos, CA 94070 (US); LEE, William, A., Los Altos, CA 94024 (US); LEE, Christopher, P., San Francisco, CA 94102 (US); LIN, Kuei-Ying, Fremont, CA 94555 (US); LIU, Hongtao, Foster City, CA 94404 (US); MACKMAN, Richard, L., Millbrae, CA 94030 (US); MITCHELL, Michael, L., Foster City, CA 94404 (US); NELSON, Peter, H., Los Altos, CA 94022 (US); PYUN, Hyung-Jung, Fremont, CA 94536 (US); ROWE, Tanisha, D., Modesto, CA 95355 (US); SWAMINATHAN, Sundaramoorthi, Burlingame, CA 94010 (US); TARIO, James, D., San Mateo , CA 94401 (US); ZHANG, Lijun, Palo Alto,CA 94303 (US); SPARACINO, Mark, Morgan Hill, CA 95037 (US); WANG, Jianying, Foster City, CA 94404 (US); WILLIAMS, Matthew, A., San Mateo, CA 94402 (US); XU, Lianhong, San Mateo, CA 94402 (US); YANG, Zheng-Yu, Foster City, CA 94404 (US); YU, Richard, H., San Francisco, CA 94116 (US); ZHANG, Jiancun, Oakland, CA 94618 (US)
(74) Representative: Reitstötter - Kinzebach
(86) International application number: PCT/US2003/012901
(87) International publication number: WO 2003/090690

(56) References cited:
- EP-A- 0 441 192
- WO-A-00/04033
- WO-A-02/06292
- WO-A-02/14344
- WO-A-96/14314
- WO-A-98/11906
- US-A- 5 670 497
- US-A- 5 750 493
- US-A- 5 914 332
- US-A1- 2001 031 773
- MENENDEZ-ARIAS L: "Targeting HIV: antiretroviral therapy and development of drug resistance" TRENDS IN PHARMACOLOGICAL SCIENCES, ELSEVIER, AMSTERDAM, NL, vol. 23, no. 8, 1 August 2002 (2002-08-01), pages 381-388, XP004386181 ISSN: 0165-6147
- ABDEL-MEGUID S S ET AL: "Inhibition of human immunodeficiency virus-1 protease by a C2-symmetric phosphinate. Synthesis and crystallographic analysis" BIOCHEMISTRY 1993 UNITED STATES, vol. 32, no. 31, 1993, pages 7972-7980, XP002257745 ISSN: 0006-2960
- CLERCQ DE E: "NEW DEVELOPMENTS IN ANTI-HIV CHEMOTHERAPY" CURRENT MEDICINAL CHEMISTRY, BENTHAM SCIENCE PUBLISHERS BV, BE, vol. 8, no. 13, November 2001 (2001-11), pages 1543-1572, XP009012547 ISSN: 0929-8673
- DVORAKOVA, HANA ET AL: "Synthesis of 2'-Aminomethyl Derivatives of 9-(2- (Phosphonomethoxy)ethyl) Nucleotide Analogs as Potential Antiviral Agents" JOURNAL OF MEDICINAL CHEMISTRY , 39(17), 3263-3268 CODEN: JMCMAR; ISSN: 0022-2623, 1996, XP002273095

## Description

This non-provisional application claims the benefit of Provisional Applications 60/375,622, filed April 26, 2002; Provisional Application No. 60/375,779 filed April 26, 2002; Provisional Application No. 60/375,834, filed April 26, 2002, and Provisional Application No. 60/375,665 filed April 26, 2002.

### FIELD OF THE INVENTION

The invention relates generally to compounds with antiviral activity and more specifically with anti-HIV protease properties.

### BACKGROUND OF THE INVENTION

AIDS is a major public health problem worldwide. Although drugs targeting HIV viruses are in wide use and have shown effectiveness, toxicity and development of resistant strains have limited their usefulness. Assay methods capable of determining the presence, absence or amounts of HIV viruses are of practical utility in the search for inhibitors as well as for diagnosing the presence of HIV.

Human immunodeficiency virus (HIV) infection and related disease is a major public health problem worldwide. The retrovirus human immunodeficiency virus type 1 (HIV-1), a member of the primate lentivirus family (DeClercq E (1994) Annals of the New York Academy of Sciences, 724:438-456; Barre-Sinoussi F (1996) Lancet, 348:31-35), is generally accepted to be the causative agent of acquired immunodeficiency syndrome (AIDS) Tarrago etal FASEB Journal 1994, 8:497-503). AIDS is the result of repeated replication of HIV-1 and a decrease in immune capacity, most prominently a fall in the number of CD4+ lymphocytes. The mature virus has a single stranded RNA genome that encodes 15 proteins (Frankel etal (1998) Annual Review of Biochemistry, 67:1-25; Katz etal (1994) Annual Review of Biochemistry, 63:133-173), including three key enzymes: (i) protease (Prt) (von der Helm K (1996) Biological Chemistry, 377:765-774); (ii) reverse transcriptase (RT) (Hottiger etal (1996) Biological Chemistry Hoppe-Seyler, 377:97-120), an enzyme unique to retroviruses; and (iii) integrase (Asante etal (1999) Advances in Virus Research 52:351-369; Wlodawer A (1999) Advances in Virus Research 52:335-350; Esposito etal (1999) Advances in Virus Research 52:319-333). Protease is responsible for processing the viral precursor polyproteins, integrase is responsible for the integration of the double stranded DNA form of the viral genome into host DNA and RT is the key enzyme in the replication of the viral genome. In viral replication, RT acts as both an RNA- and a DNA-dependent DNA polymerase, to convert the single stranded RNA genome into double stranded DNA. Since virally encoded Reverse Transcriptase (RT) mediates specific reactions during the natural reproduction of the virus, inhibition of HIV RT is an important therapeutic target for treatment of HIV infection and related disease.

Sequence analysis of the complete genomes from several infective and non-infective HIV-isolates has shed considerable light on the make-up of the virus and the types of molecules that are essential for its replication and maturation to an infective species. The HIV protease is essential for the processing of the viral gag and gag-pol polypeptides into mature virion proteins. L. Ratner, et al., Nature, 313:277-284 (1985); L. H. Pearl and W. R. Taylor, Nature, 329:351 (1987). HIV exhibits the same gag/pol/env organization seen in other retroviruses. L. Ratner, et al., above; S. Wain-Hobson, et al, Cell, 40:9-17 (1985); R. Sanchez-Pescador, et al., Science, 227:484-492 (1985); and M. A. Muesing, et al., Nature, 313:450-458 (1985).

A therapeutic target in AIDS involves inhibition of the viral protease (or proteinase) that is essential for processing HIV-fusion polypeptide precursors. In HIV and several other retroviruses, the proteolytic maturation of the gag and gag/pol fusion polypeptides (a process indispensable for generation of infective viral particles) has been shown to be mediated by a protease that is, itself, encoded by the pol region of the viral genome. Y. Yoshinaka, et al., Proc. Natl. Acad. Sci. USA, 82:1618-1622 (1985); Y. Yoshinaka, et al., J. Virol., 55:870-873 (1985); Y. Yoshinaka, et al., J. Virol., 57:826-832 (1986); and K. von der Helm, Proc. Natl. Acad. Sci., USA, 74:911-915 (1977). Inhibition of the protease has been shown to inhibit the processing of the HIV p55 in mammalian cell and HIV replication in T lymphocytes. T. J. McQuade, et al., Science, 247:454 (1990).

Drugs approved in the United States for AIDS therapy include nucleoside inhibitors of RT (Smith et al (1994) Clinical Investigator, 17:226-243), protease inhibitors and non-nucleoside RT inhibitors (NNRTI), (Johnson et al (2000) Advances in Internal Medicine, 45 (1-40; Porche DJ (1999) Nursing Clinics of North America, 34:95-112).

The protease (or proteinase), consisting of only 99 amino acids, is among the smallest enzymes known, and its demonstrated homology to aspartyl proteases such as pepsin and renin (L. H. Pearl and W. R. Taylor, Nature, 329:351-354 (1987); and I. Katoh, et al., Nature, 329:654-656 (1987)), led to inferences regarding the three-dimensional structure and mechanism of the enzyme (L. H. Pearl and W. R. Taylor, above) that have since been borne out experimentally. Active HIV protease has been expressed in bacteria (see, e.g., P. L. Darke, et al., J. Biol. Chem., 264:2307-2312 (1989)) and chemically synthesized (J. Schneider and S. B. Kent, Cell, 54:363-368 (1988); and R. F. Nutt, et al., Proc. Natl. Acad. Sci., USA, 85:7129-7133 (1988)). Site directed mutagenesis (P. L. Darke, et al., above); and N. E. Kohl, et al., Proc. Natl. Acad. Sci., USA, 85:4686-4690 (1988)) and pepstatin inhibition (P. L. Darke, et al., J. BioL Chem., 264:2307-2312 (1989); S. Seelmeier, et al., Proc. Natl. Acad. Sci., USA, 85:6612-6616 (1988); C.-Z. Giam and I. Borsos, J. Biol. Chem., 263:14617-14720 (1988); and J. Hansen, et al., EMBO J., 7:1785-1791 (1988)) have provided evidence for HIV protease's mechanistic function as an aspartyl protease. A study has demonstrated that the protease cleaves at the sites expected in peptides modeled after the regions actually cleaved by the enzyme in the gag and pol precursor proteins during viral maturation. P. L. Darke, et al., Biochem. Biophys. Res. Communs., 156:297-303 (1988). X-ray crystallographic analysis of the HIV-protease (M. A. Navia, et al., Nature, 337:615-620 (1989)) and a related retroviral enzyme from Rous sarcoma virus (M. Miller, et al., Nature, 337:576-579 (1989)) reveal an active site in the protease dimer that is identical to that seen in other aspartyl proteases, thus supporting the supposition (L. H. Pearl and W. R. Taylor, above) that the HIV enzyme is active as a dimer. See also Joseph A. Martin, "Recent Advances in the Design of HIV Proteinase Inhibitors," Antiviral Research, 17 (1992) 265-278. In J. HIV Ther. 2001, 6(4) 96-99 GW 433 908 is disclosed which is a phosphate derivative of the HIV protease inhibitor amprenavir and has the formula 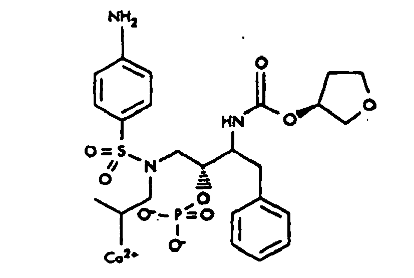 NLM 11968788 Medline Print-out (April 2002) (Japanese Journal of Clinical Medicine) refers to HIV protease inhibitors in general and specifically mentions atazanavir, GW 433908, L-756, 423, mozenavir (DMP-450) and tipranavir.

Inhibitors of HIV protease are useful to limit the establishment and progression of infection by therapeutic administration as well as in diagnostic assays for HIV. Protease inhibitor drugs approved by the FDA include:
- saquinavir (Invirase®, Fortovase®, Hoffman-La Roche, EP-00432695 and EP-00432694)
- ritonavir (Norvir®, Abbott Laboratories)
- indinavir (Crixivan®, Merck & Co.)
- nelfinavir (Viracept®, Pfizer)
- amprenavir (Agenerase®, GlaxoSmithKline, Vertex Pharmaceuticals)
- lopinavir/ritonavir (Kaletra®, Abbott Laboratories)

Experimental protease inhibitor drugs include:
- fosamprenavir (GlaxoSmithKline, Vertex Pharmaceuticals)
- tipranavir (Boehringer Ingelheim)
- atazanavir (Bristol-Myers Squibb).

There is a need for anti-HIV therapeutic agents, i.e. drugs having improved antiviral and pharmacokinetic properties with enhanced activity against development of HIV resistance, improved oral bioavailability, greater potency and extended effective half-life in *vivo.* New HIV protease inhibitors (PI) should be active against mutant HIV strains, have distinct resistance profiles, fewer side effects, less complicated dosing schedules, and orally active. In particular, thère is a need for a less onerous dosage regimen, such as one pill, once per day. Although drugs targeting HIV protease are in wide use and have shown effectiveness, particularly when employed in combination, toxicity and development of resistant strains have limited their usefulness (Palella, et al N. Engl. J. Med. (1998) 338:853-860; Richman, D. D. Nature (2001) 410:995-1001).

Combination therapy of PI and RT inhibitors has proven to be highly effective in suppressing viral replication to unquantifiable levels for a sustained period of time. Also, combination therapy with RT and protease inhibitors have shown synergistic effects in suppressing HIV replication. Unfortunately, many patients currently fail combination therapy due to the development of drug resistance, non-compliance with complicated dosing regimens, pharmacokinetic interactions, toxicity, and lack of potency. Therefore, there is a need for new HIV protease inhibitors that are synergistic in combination with other HIV inhibitors.

Improving the delivery of drugs and other agents to target cells and tissues has been the focus of considerable research for many years. Though many attempts have been made to develop effective methods for importing biologically active molecules into cells, both *in vivo* and *in vitro,* none has proved to be entirely satisfactory. Optimizing the association of the inhibitory drug with its intracellular target, while minimizing intercellular redistribution of the drug, e.g. to neighboring cells, is often difficult or inefficient.

Most agents currently administered to a patient parenterally are not targeted, resulting in systemic delivery of the agent to cells and tissues of the body where it is unnecessary, and often undesirable. This may result in adverse drug side effects, and often limits the dose of a drug (e.g., cytotoxic agents and other anti-cancer or anti-viral drugs) that can be administered. By comparison, although oral administration of drugs is generally recognized as a convenient and economical method of administration, oral administration can result in either (a) uptake of the drug through the cellular and tissue barriers, e.g. blood/brain, epithelial, cell membrane, resulting in undesirable systemic distribution, or (b) temporary residence of the drug within the gastrointestinal tract. Accordingly, a major goal has been to develop methods for specifically targeting agents to cells and tissues. Benefits of such treatment includes avoiding the general physiological effects of inappropriate delivery of such agents to other cells and tissues, such as uninfected cells. Intracellular targeting may be achieved by methods and compositions which allow accumulation or retention of biologically active agents inside cells.

### SUMMARY OF THE INVENTION

The present invention provides novel compounds with HIV protease activity, i.e. novel human retroviral protease inhibitors. Therefore, the compounds of the invention may inhibit retroviral proteases and thus inhibit the replication of the virus. They are useful for treating human patients infected with a human retrovirus, such as human immunodeficiency virus (strains of HIV-1 or HIV-2) or human T-cell leukemia viruses (HTLV-I or HTLV-II) which results in acquired immunodeficiency syndrome (AIDS) and/or related diseases. The present invention includes novel phosphonate HIV protease inhibitor (PI) compounds and phosphonate analogs of known approved and experimental protease inhibitors. The compounds of the invention optionally provide cellular accumulation as set forth below.

The present invention relates generally to the accumulation or retention of therapeutic compounds inside cells. The invention is more particularly related to attaining high concentrations of phosphonate-containing molecules in HIV infected cells. Intracellular targeting may be achieved by methods and compositions which allow accumulation or retention of biologically active agents inside cells. Such effective targeting may be applicable to a variety of therapeutic formulations and procedures.

Compositions of the invention include new PI compounds having at least one phosphonate group. The invention includes all known approved and experimental protease inhibitors with at least one phosphonate group.

In one aspect, the invention includes compounds having Formulas I, II, III, IV, V and VI :

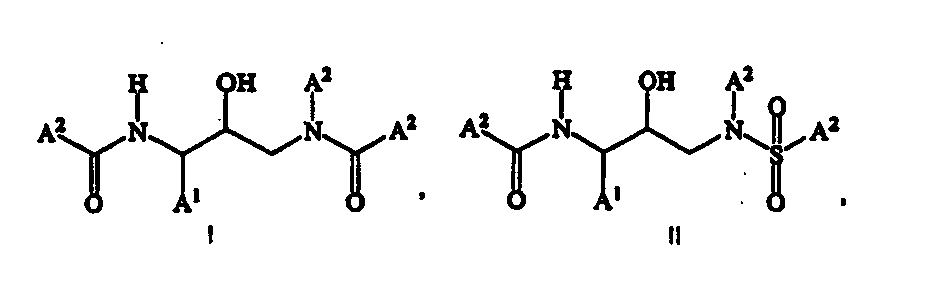

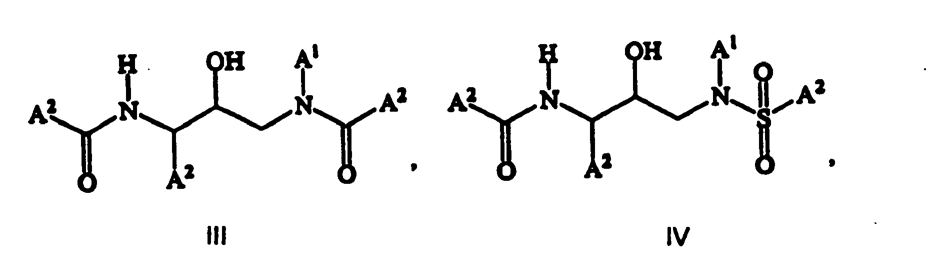

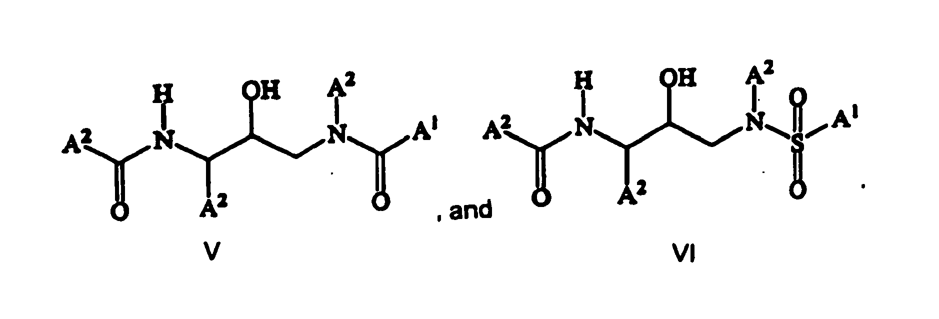

Formulas I-VI are substituted with one or more covalently attached groups, including at least one phosphonate group. Formulas I-Vm are "scaffolds", i.e. substructures which are common to the specific compounds encompassed therein.

Another aspect of the invention provides a pharmaceutical combination comprising an effective amount of a compound selected from Formulas I-VIII and a second compound having anti-HIV properties.

Another aspect of the invention provides the use of the compounds of formulas I-VI for preparing a pharmaceutical composition for the treatment or prevention of the symptoms or effects of an HIV infection.

The invention provides a pharmaceutical composition comprising an effective amount of a compound selected from Formulas I-VI, or a pharmaceutically acceptable salt thereof, in combination with a pharmaceutically acceptable diluent or carrier.

This invention pertains to increasing cellular accumulation and retention of drug compounds, thus improving their therapeutic and diagnostic value.

The invention also provides the use of the compounds of formulas I-VI for preparing a pharmaceutical composition for inhibiting HIV. The compounds of formulas I-VIII are effective to inhibit the growth of HIV infected cells.

The invention also provides a compound selected from Formulas I-VIII for use in medical therapy (preferably for use in treating cancer, e.g. solid tumors), as well as the use of a compound of Formulas I-VIII for the manufacture of a medicament useful for the treatment of cancer, e.g. solid tumors.

The invention also provides processes and novel intermediates disclosed herein which are useful for preparing compounds of the invention. Some of the compounds of Formulas I-VI are useful to prepare other compounds of Formulas I-VI.

In another aspect of the invention, the activity of HIV protease is inhibited by a method comprising the step of treating a sample suspected of containing HIV virus with a compound or composition of the invention.

Another aspect of the invention provides a method for inhibiting the activity of HIV protease comprising the step of contacting a sample suspected of containing HIV virus with a composition of the invention.

In other aspects, novel methods for synthesis analysis, separation, isolation, purification, characterization, and testing of the compounds of this invention are provided.

### DETAILED DESCRIPTION OF EXEMPLARY EMBODIMENTS

Reference will now be made in detail to certain embodiments of the invention, examples of which are illustrated in the accompanying description, structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims.

### DEFINITIONS

Unless stated otherwise, the following terms and phrases as used herein are intended to have the following meanings:

The terms "phosphonate" and "phosphonate group" mean a functional group or moiety within a molecule that comprises at least one phosphorus-carbon bond, and at least one phosphorus-oxygen double bond. The phosphorus atom is further substituted with oxygen, sulfur, and nitrogen substituents. These substituents may be part of a prodrug moiety. As defined herein, "phosphonate" and "phosphonate group" include molecules with phosphonic acid, phosphonic monoester, phosphonic diester, phosphonamidate, phosphondiamidate and phosphonthioate functional groups.

The term "prodrug" as used herein refers to any compound that when administered to a biological system generates the drug substance, i.e. active ingredient, as a result of spontaneous chemical reaction(s), enzyme catalyzed chemical reaction(s), photolysis, and/or metabolic chemical reaction(s). A prodrug is thus a covalently modified analog or latent form of a therapeutically-active compound.

"Pharmaceutically acceptable prodrug" refers to a compound that is metabolized in the host, for example hydrolyzed or oxidized, by either enzymatic action or by general acid or base solvolysis, to form an active ingredient. Typical examples of prodrugs of the compounds of the invention have biologically labile protecting groups on a functional moiety of the compound. Prodrugs include compounds that can be oxidized, reduced, aminated, deaminated, esterified, deesterified, alkylated, dealkylated, acylated, deacylated, phosphorylated, dephosphorylated, photolyzed, hydrolyzed, or other functional group change or conversion involving forming or breaking chemical bonds on the prodrug.

"Prodrug moiety" means a labile functional group which separates from the active inhibitory compound during metabolism, systemically, inside a cell, by hydrolysis, enzymatic cleavage, or by some other process (Bundgaard, Hans, "Design and Application of Prodrugs" in Textbook of Drug Design and Development (1991), P. Krogsgaard-Larsen and H. Bundgaard, Eds. Harwood Academic Publishers, pp. 113-191). Enzymes which are capable of an enzymatic activation mechanism with the phosphonate prodrug compounds of the invention include, but are not limited to, amidases, esterases, microbial enzymes, phospholipases, cholinesterases, and phosphases. Prodrug moieties can serve to enhance solubility, absorption and lipophilicity to optimize drug delivery, bioavailability and efficacy.

Exemplary prodrug moieties include the hydrolytically sensitive or labile acyloxymethyl esters -CH₂OC(=O)R⁹ and acyloxymethyl carbonates -CH₂OC(=O)OR⁹ where R⁹ is C₁-C₆ alkyl, C₁-C₆ substituted alkyl, C₆-C₂₀ aryl or C₆-C₂₀ substituted aryl. The acyloxyalkyl ester was first used as a prodrug strategy for carboxylic acids and then applied to phosphates and phosphonates by Farquhar et al (1983) J. Pharm. Sci. 72: 324; also US Patent Nos. 4816570, 4968788, 5663159 and 5792756. In certain compounds of the invention, a prodrug moiety is part of a phosphonate group. Subsequently, the acyloxyalkyl ester was used to deliver phosphonic acids across cell membranes and to enhance oral bioavailability. A close variant of the acyloxyalk-yl ester, the alkoxycarbonyloxyalkyl ester (carbonate), may also enhance oral bioavailability as a prodrug moiety in the compounds of the combinations of the invention. An exemplary acyloxymethyl ester is pivaloyloxymethoxy, (POM) -CH₂OC(=O)C(CH₃)₃. An exemplary acyloxymethyl carbonate prodrug moiety is pivaloyloxymethylcarbonate (POC) -CH₂OC(=O)OC(CH₃)₃.

The phosphonate group may be a phosphonate prodrug moiety. The prodrug moiety may be sensitive to hydrolysis, such as, but not limited to a pivaloyloxymethyl carbonate (POC) or POM group. Alternatively, the prodrug moiety may be sensitive to enzymatic potentiated cleavage, such as a lactate ester or a phosphonamidate-ester group.

Aryl esters of phosphorus groups, especially phenyl esters, are reported to enhance oral bioavailability (DeLambert et al (1994) J. Med. Chem. 37: 498). Phenyl esters containing a carboxylic ester ortho to the phosphate have also been described (Khamnei and Torrence, (1996) J. Med. Chem. 39:4109-4115). Benzyl esters are reported to generate the parent phosphonic acid. In some cases, substituents at the *ortho-*or *para*-position may accelerate the hydrolysis. Benzyl analogs with an acylated phenol or an alkylated phenol may generate the phenolic compound through the action of enzymes, e.g. esterases, oxidases, etc., which in turn undergoes cleavage at the benzylic C-O bond to generate the phosphoric acid and the quinone methide intermediate. Examples of this class of prodrugs are described by Mitchell et al (1992) J. Chem. Soc. Perkin Trans. I 2345; Brook et al WO 91/19721. Still other benzylic prodrugs have been described containing a carboxylic ester-containing group attached to the benzylic methylene (Glazier et al WO 91/19721). Thio-containing prodrugs are reported to be useful for the intracellular delivery of phosphonate drugs. These proesters contain an ethylthio group in which the thiol group is either esterified with an acyl group or combined with another thiol group to form a disulfide. Deesterification or reduction of the disulfide generates the free thio intermediate which subsequently breaks down to the phosphoric acid and episulfide (Puech et al (1993) Antiviral Res., 22: 155-174; Benzaria et al (1996) J. Med Chem. 39: 4958). Cyclic phosphonate esters have also been described as prodrugs of phosphorus-containing compounds (Erion et al, US Patent No. 6312662).

"Protecting group" refers to a moiety of a compound that masks or alters the properties of a functional group or the properties of the compound as a whole. The chemical substructure of a protecting group varies widely. One function of a protecting group is to serve as intermediates in the synthesis of the parental drug substance. Chemical protecting groups and strategies for protection/deprotection are well known in the art. See: "Protective Groups in Organic Chemistry", Theodora W. Greene (John Wiley & Sons, Inc., New York, 1991. Protecting groups are often utilized to mask the reactivity of certain functional groups, to assist in the efficiency of desired chemical reactions, e.g. making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

Protected compounds may also exhibit altered, and in some cases, optimized properties *in vitro* and *in vivo,* such as passage through cellular membranes and resistance to enzymatic degradation or sequestration. In this role, protected compounds with intended therapeutic effects may be referred to as prodrugs. Another function of a protecting group is to convert the parental drug into a prodrug, whereby the parental drug is released upon conversion of the prodrug *in vivo.* Because active prodrugs may be absorbed more effectively than the parental drug, prodrugs may possess greater potency *in vivo* than the parental drug. Protecting groups are removed either *in vitro,* in the instance of chemical intermediates, or in *vivo,* in the case of prodrugs. With chemical intermediates, it is not particularly important that the resulting products after deprotection, e.g. alcohols, be physiologically acceptable, although in general it is more desirable if the products are pharmacologically innocuous.

Any reference to any of the compounds of the invention also includes a reference to a physiologically acceptable salt thereof. Examples of physiologically acceptable salts of the compounds of the invention include salts derived from an appropriate base, such as an alkali metal (for example, sodium), an alkaline earth (for example, magnesium), ammonium and NX₄⁺ (wherein X is C₁-C₄ alkyl). Physiologically acceptable salts of an hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound of an hydroxy group include the anion of said compound in combination with a suitable cation such as Na⁺ and NXa⁺ (wherein X is independently selected from H or a C₁-C₄ alkyl group).

For therapeutic use, salts of active ingredients of the compounds of the invention will be physiologically acceptable, i.e. they will be salts derived from a physiologically acceptable acid or base. However, salts of acids or bases which are not physiologically acceptable may also find use, for example, in the preparation or purification of a physiologically acceptable compound. All salts, whether or not derived form a physiologically acceptable acid or base, are within the scope of the present invention.

"Alkyl" is C₁-C₁₈ hydrocarbon containing normal, secondary, tertiary or cyclic carbon atoms. Examples are methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH2CH₂CH₃), 2-methyl-l-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃.

"Alkenyl" is C₂-C₁₈ hydrocarbon containing normal, secondary, tertiary or cyclic carbon atoms with at least one site of unsaturation, i.e. a carbon-carbon, *sp²* double bond. Examples include, but are not limited to: ethylene or vinyl (-CH=CH₂), allyl (-CH₂CH=CH₂), cyclopentenyl (-C₅H₇), and 5-hexenyl (-CH₂CH₂CH₂CH₂CH=CH₂)

"Alkynyl" is C₂-C₁₈ hydrocarbon containing normal, secondary, tertiary or cyclic carbon atoms with at least one site of unsaturation, i.e. a carbon-carbon, *sp* triple bond. Examples include, but are not limited to: acetylenic (-C≡CH) and propargyl (-CH₂C≡CH),

"Alkylene" refers to a saturated, branched or straight chain or cyclic hydrocarbon radical of 1-18 carbon atoms, and having two monovalent radical centers derived by the removal of two hydrogen atoms from the same or two different carbon atoms of a parent alkane. Typical alkylene radicals include, but are not limited to: methylene (-CH₂-) 1,2-ethyl (-CH₂CH₂-), 1,3-propyl (-CH₂CH₂CH₂-), 1,4-butyl (-CH₂CH₂CH₂CH₂-), and the like.

"Alkenylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to: 1,2-ethylene (-CH=CH-).

"Alkynylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkyne. Typical alkynylene radicals include, but are not limited to: acetylene (-C≡C-), propargyl (-CH₂C≡C-), and 4-pentynyl (-CH₂CH₂CH₂C≡CH-).

"Aryl" means a monovalent aromatic hydrocarbon radical of 6-20 carbon atoms derived by the removal of one hydrogen atom from a single carbon atom of a parent aromatic ring system. Typical aryl groups include, but are not limited to, radicals derived from benzene, substituted benzene, naphthalene, anthracene, biphenyl, and the like.

"Arylalkyl" refers to an acyclic alkyl radical in which one of the hydrogen atoms bonded to a carbon atom, typically a terminal or sp³ carbon atom, is replaced with an aryl radical. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. The arylalkyl group comprises 6 to 20 carbon atoms, e.g. the alkyl moiety, including alkanyl, alkenyl or alkynyl groups, of the arylalkyl group is 1 to 6 carbon atoms and the aryl moiety is 5 to 14 carbon atoms.

"Substituted alkyl", "substituted aryl", and "substituted arylalkyl" mean alkyl, aryl, and arylalkyl respectively, in which one or more hydrogen atoms are each independently replaced with a substituent. Typical substituents include, but are not limited to, -X, -R, -O-, -OR, -SR, -S⁻, -NR₂, -NR₃, =NR, -CX₃, -CN, -OCN, -SCN, -N=C=O, -NCS, -NO, -NO₂, =N₂, -N₃, NC(=O)R, -C(=O)R, -C(=O)NRR -S(=O)₂O-, -S(=O)₂OH, -S(=O)₂R, -OS(=O)₂OR, -S(=O)₂NR, -S(=O)R, -OP(=O)O₂RR,-P(=O)O₂RR -P(=O)(O⁻)₂, -P(=O)(OH)₂, -C(=O)R, -C(=O)X, -C(S)R, -C(O)OR, -C(O)O-, -C(S)OR, -C(O)SR, -C(S)SR, -C(O)NRR, -C(S)NRR, -C(NR)NRR, where each X is independently a halogen: F, Cl, Br, or I; and each R is independently -H, alkyl, aryl, heterocycle, protecting group or prodrug moiety. Alkylene, alkenylene, and alkynylene groups may also be similarly substituted.

"Heterocycle" as used herein includes by way of example and not limitation these heterocycles described in Paquette, Leo A.; "Principles of Modem Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566.

Examples of heterocycles include by way of example and not limitation pyridyl, dihydroypyridyl, tetrahydropyridyl (piperidyl), thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thienyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, and isatinoyl.

One embodiment of the bis-tetrahydrofuranyl group is:

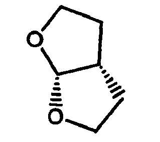

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline. Still more typically, nitrogen bonded heterocycles include 1-aziridyl, 1-azetedyl, 1-pyrrolyl, 1-imidazolyl, 1-pyrazolyl, and 1-piperidinyl.

"Carbocycle" means a saturated, unsaturated or aromatic ring having 3 to 7 carbon atoms as a monocycle or 7 to 12 carbon atoms as a bicycle. Monocyclic carbocycles have 3 to 6 ring atoms, still more typically 5 or 6 ring atoms. Bicyclic carbocycles have 7 to 12 ring atoms, e.g. arranged as a bicyclo [4,5], [5,5], [5,6] or [6,6] system, or 9 or 10 ring atoms arranged as a bicyclo [5,6] or [6,6] system Examples of monocyclic carbocycles include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, phenyl, spiryl and naphthyl.

"Linker" or "link" means a chemical moiety comprising a covalent bond or a chain of atoms that covalently attaches a phosphonate group to a drug. Linkers include portions of substituents A¹ and A³ enumerated in Formula I, or substituents A₁ and A₃ enumerated in Formula II, which include moieties such as: repeating units of alkyloxy (e.g. polyethylenoxy, PEG, polymethyleneoxy) and alkylamino (e.g. polyethyleneamino, Jeffamine™); and diacid ester and amides including succinate, succinamide, diglycolate, malonate, and caproamide.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L or R and S are used to denote the absolute configuration of the molecule about its chiral center(s). The prefixes d and 1, D and L, or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereo selection or stereospecificity in a chemical reaction or process. The terms "racemic mixture" and "racemate" refer to an equimolar mixture of two enantiomeric species, devoid of optical activity.

### HIV Protease Inhibitor Compounds

The compounds of the invention include those with HIV protease inhibitory activity. In particular, the compounds include HIV protease inhibitors. The compounds of the inventions bear a phosphonate group, which may be a prodrug moiety.

Whenever a compound described herein is substituted with more than one of the same designated group, e.g., "R¹" or "R^{6a}", then it will be understood that the groups may be the same or different, i.e., each group is independently selected. Wavy lines indicate the site of covalent bond attachments to the adjoining groups, moieties, or atoms.

Compounds of the invention are set forth in the schemes, examples, descriptions and claims below and have the Formulas I, II, III, IV, V, and VI: 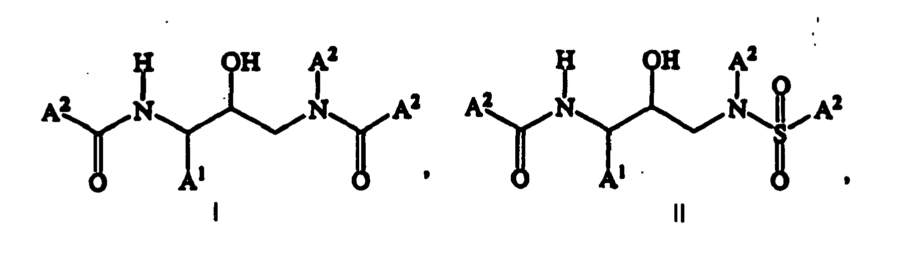 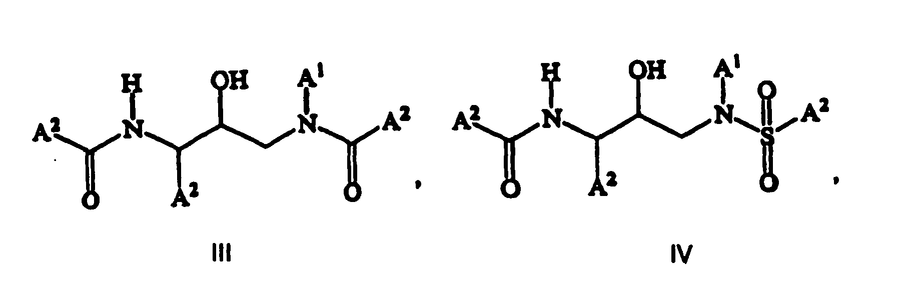 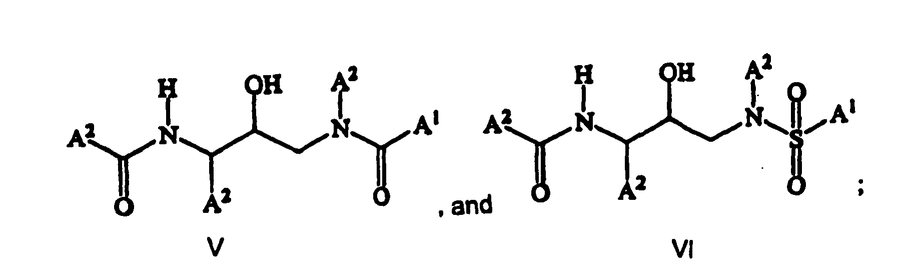 wherein:
A¹ is: 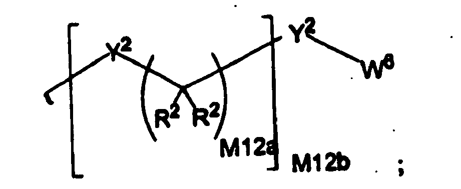
A² is: 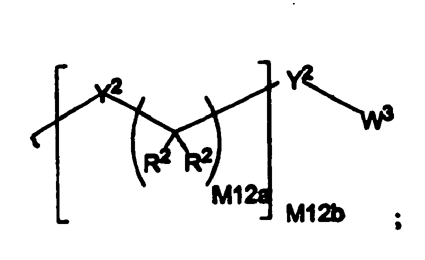
A³ is: 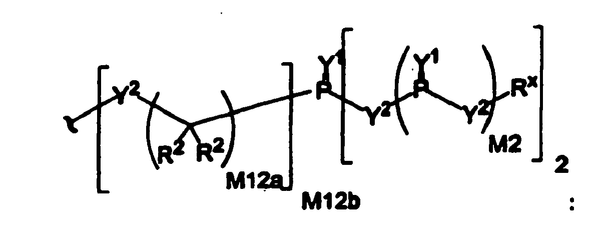
   Y¹ Is Independently O, S, N(R^{x}), N(O)(R^{x}), N(OR^{x}), N(O)(OR^{x}), or N(N(R^{x})(R^{x}));
   Y² is independently a bond, O, N(R^{x}), N(O)(R^{x}), N(OR^{x}), N(O)(OR^{x}), N(N(R^{x})(R^{x})), -S(O)_{M2-}, or -S(O)_{M2}-S(O)_{M2}-;
   R^{x} Is independently H, R¹, W³, a protecting group, or the formula: 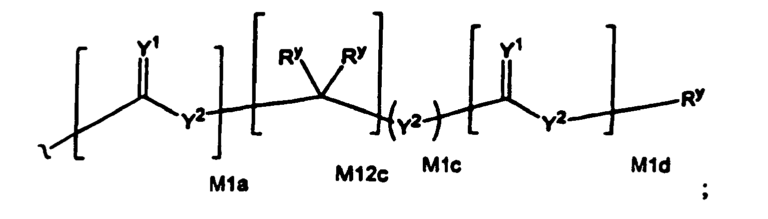
   R^{y} is Independently H, W³, R² or a protecting group;
   R¹ is Independently H or an alkyl of 1 to 18 carbon atoms;
   R² is Independently H, R¹, R³ or R⁴ wherein each R⁴ Is independently substituted with 0 to 3 R³ groups, or taken together at a carbon atom, two R² groups form a ring of 3 to 8 carbons and the ring may be substituted with 0 to 3 R³ groups;
   R³ is R^{3a}, R^{3b}, R^{3c}, or R^{3d}, provided that when R³ is bound to a heteroatom, then R³ is R^{3c} or R^{3d};
   R^{3a} is F, Cl, Br, l, -CN, N₃ or -NO₂;
   R^{3b} is Y¹;
   R^{3c} is -R^{x}, -N(R^{x})(R^{x}), -SR^{x}, -S(O)R^{x}, -S(O)₂R^{x}, -S(O)(OR^{x}), -S(O)₂(OR^{x}), OC(Y¹)R^{x}, -OC(Y¹)OR^{x}, -OC(Y¹)(N(R^{x})(R^{x})), -SC(Y¹)R^{x}, -SC(Y¹)OR^{x}, -SC(Y¹)(N(R^{x})(R^{x})), -N(R^{x})C(Y¹)R^{x}, -N(R^{x})C(Y¹)OR^{x}, or -N(R^{x})C(Y¹)(N(R^{x})(R^{x})) :
   R^{3d} is -C(Y¹)R^{x}, -C(Y¹)OR^{x} or -C(Y¹)(N(R^{x})(R^{x})):
   R⁴ is an alkyl of 1 to 18 carbon atoms, alkenyl of 2 to 18 carbon atoms, or alkynyl of 2 to 18 carbon atoms;
   R⁵ is R⁴ wherein each R⁴ is substituted with 0 to 3 R³ groups;
   W³ is W⁴ or W⁵;
   W⁴ is R⁵, -C(Y¹)R⁵, -C(Y¹)W⁵, -SO₂R⁵, or SO₂W⁵;
   W⁵ is carbocycle or heterocycle wherein W⁵ is independently substituted with 0 to 3 R² groups:
   W⁶ is W³ independently substituted with 1. 2, or 3 A³ groups:
   M2 is 0, 1 or 2;
   M12a is 1, 2, 3, 4, 5, 8, 7, 8, 9, 10, 11 or 12;
   M12b is 0, 1, 2, 3, 4, 5, 6, 7, 8, 9, 10, 11 or 12;
   M1a, M1c, and M1d are independently 0 or 1; and
   M12c is 0, 1, 2, 3, 4, 5, 6, 7, 8, 9, 10, 11 or 12, and
the enantiomers and diastereomers, as well as the physiologically acceptable salts thereof.

W⁵ and W^{5a} carbocycles and W⁵ and W^{5a} heterocycles may be independently substituted with 0 to 3 R² groups. W⁵ may be a saturated, unsaturated or aromatic ring comprising a mono- or bicyclic carbocycle or heterocycle. W⁵ may have 3 to 10 ring atoms, e.g., 3 to 7 ring atoms. The W⁵ rings are saturated when containing 3 ring atoms, saturated or mono-unsaturated when containing 4 ring atoms, saturated, or mono- or di-unsaturated when containing 5 ring atoms, and saturated, mono- or di-unsaturated, or aromatic when containing 6 ring atoms.

A W⁵ heterocycle may be a monocycle having 3 to 7 ring members (2 to 6 carbon atoms and 1 to 3 heteroatoms selected from N, O P, and S) or a bicycle having 7 to 10 ring members (4 to 9 carbon atoms and 1 to 3 heteroatoms selected from N, O, P, and S). W⁵ heterocyclic monocycles may have 3 to 6 ring atoms (2 to 5 carbon atoms and 1 to 2 heteroatoms selected from N, O and S); or 5 or 6 ring atoms (3 to 5 carbon atoms and 1 to 2 heteroatoms selected from N and S). W⁵ heterocyclic bicycles have 7 to 10 ring atoms (6 to 9 carbon atoms and 1 to 2 heteroatoms selected from N, O and S) arranged as a bicyclo [4,5], [5,5], [5,6], or [6,6] system; or 9 to 10 ring atoms (8 to 9 carbon atoms and 1 to 2 hetero atoms selected from N and S) arranged as a bicyclo [5,6] or [6,6] system. The W⁵ heterocycle may be bonded to Y² through a carbon, nitrogen, sulfur or other atom by a stable covalent bond.

W⁵ heterocycles include for example, pyridyl, dihydropyridyl isomers, piperidine, pyridazinyl, pyrimidinyl, pyrazinyl, s-triazinyl, oxazolyl, imidazolyl, thiazolyl, isoxazolyl, pyrazolyl, isothiazolyl, furanyl, thiofuranyl, thienyl, and pyrrolyl. W⁵ also includes, but is not limited to, examples such as:

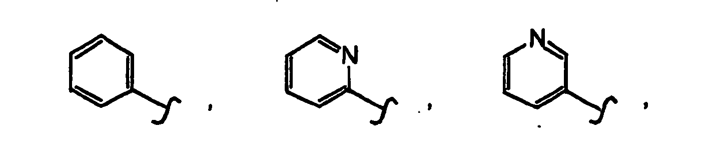

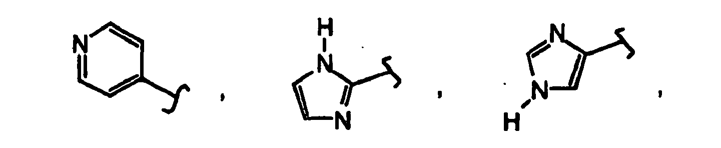

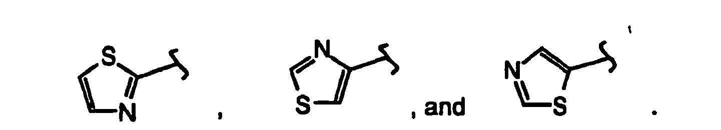

W⁵ carbocycles and heterocycles may be independently substituted with 0 to R² groups, as defined above. For example, substituted W⁵ carbocycles include:

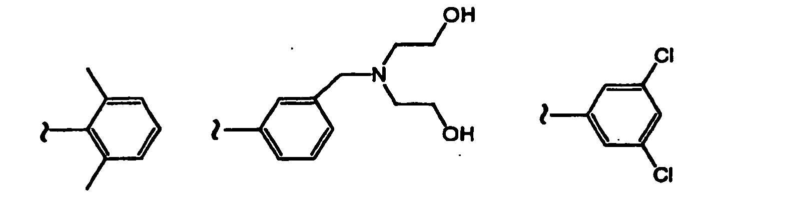

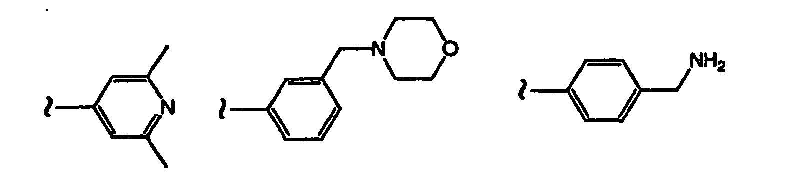

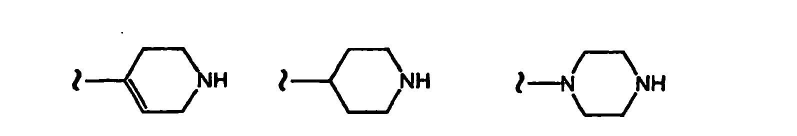

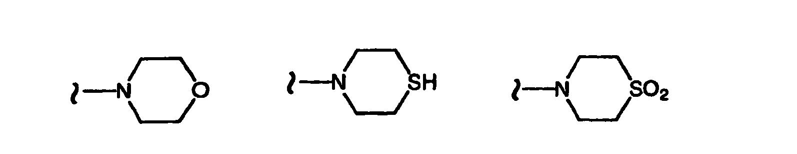

Examples of substituted phenyl carbocycles include:

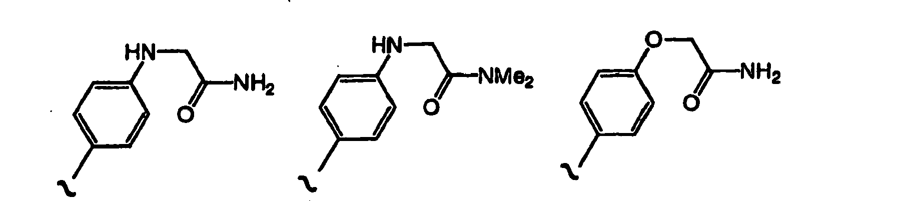

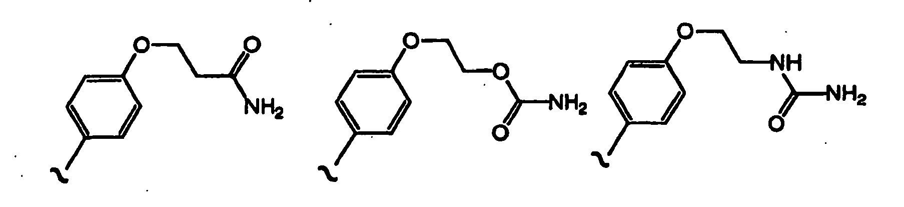

Embodiments of A¹ include: 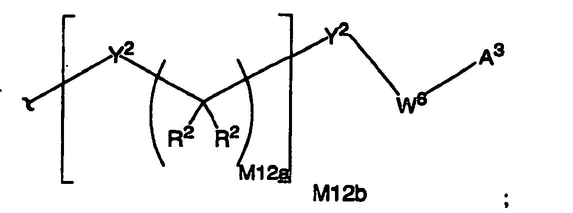 and where one or more Y² are a bond, such as: 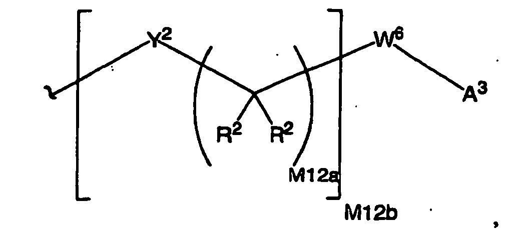 and

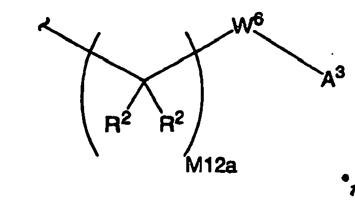

Embodiments of A³ include where M2 is 0, such as: 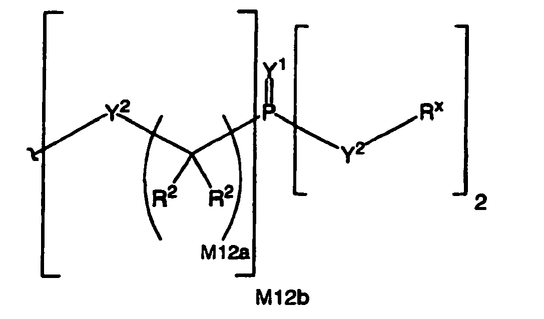 and where M12b is 1, Y¹ is oxygen, and Y^{2b} is oxygen (O) or nitrogen (N(R^{x})) such as:

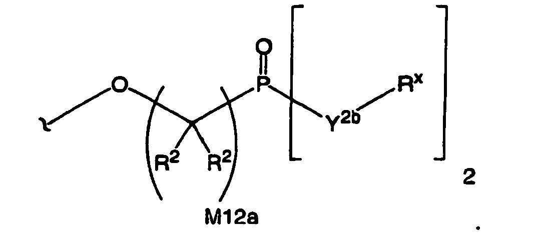

An embodiment of A³ includes: 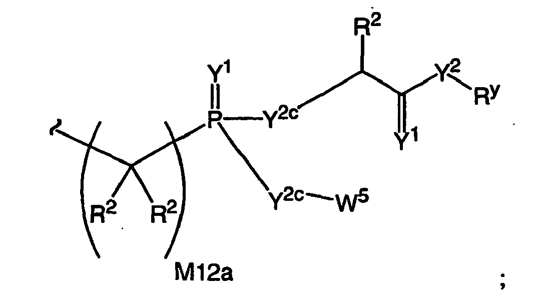 where Y^{2c} is O, N(R^{y}) or S. For example, R¹ may be H and n may be 1.

Another embodiment of A³ includes: 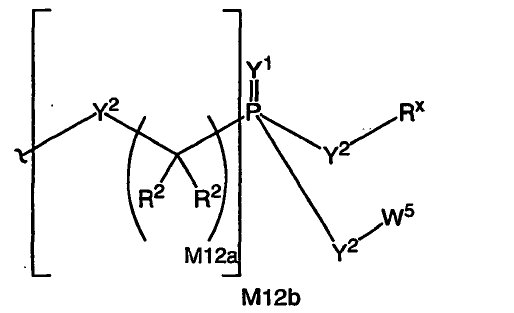 where W⁵ is a carbocycle such as phenyl or substituted phenyl. Such embodiments include: 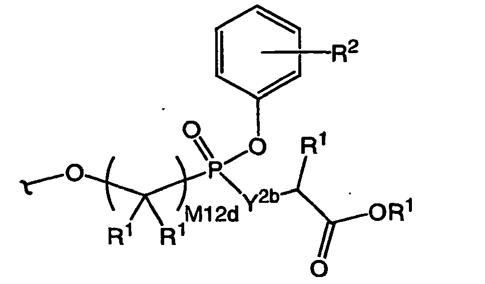 where Y^{2b} is 0 or N(R^{x}); Ml2d is 1, 2, 3, 4, 5, 6, 7 or 8; and the phenyl carbocycle is substituted with 0 to 3 R² groups. Such embodiments of A³ include phenyl phosphonamidate amino acid, e.g. alanate esters and phenyl phosphonate-lactate esters:

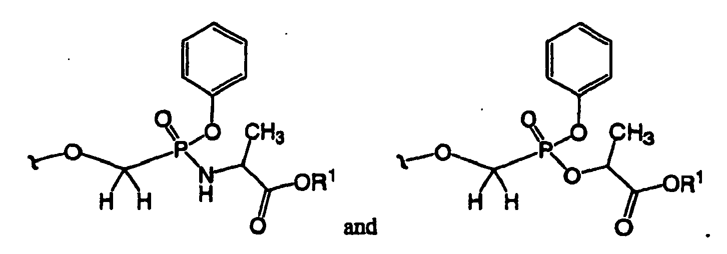

The chiral carbon of the amino acid and lactate moieties may be either the R or S configuration or the racemic mixture.

Embodiments of R^{x} include esters, carbamates, carbonates, thioesters, amides, thioamides, and urea groups:

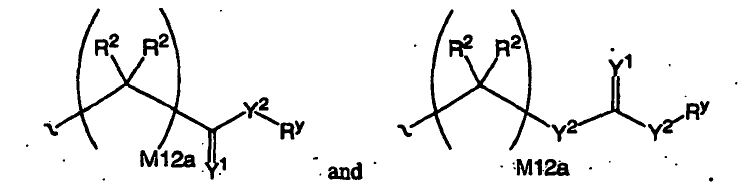

Embodiments of A² include where W³ is W⁵, such as: 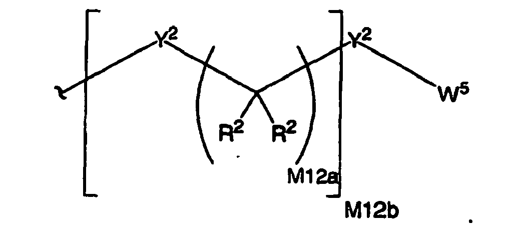 Alternatively, A² is phenyl, substituted phenyl, benzyl, substituted benzyl, pyridyl or substituted pyridyl

Exemplary embodiments of Formula II compounds include, but are not limited to, structures: 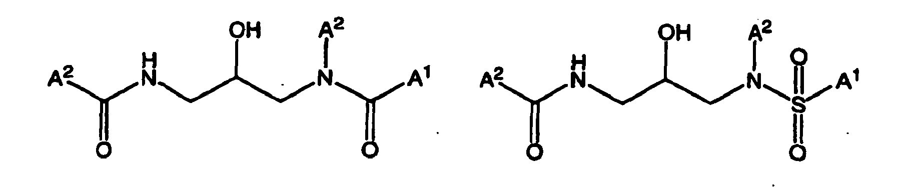 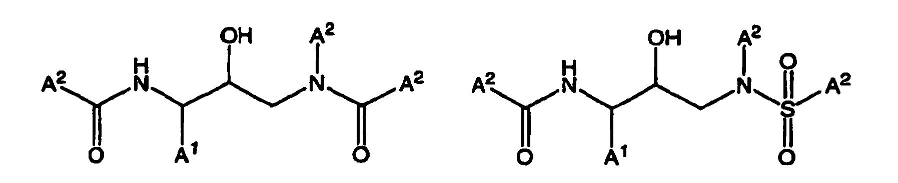 where A¹ denotes a covalent attachment site of a phosphonate group.

### A Cellular Accumulation Embodiment

Another embodiment of the invention is directed toward an HIV protease inhibitor compound capable of accumulating in human PBMCs. Accumulation in human PBMCs is described in the examples herein. Typically, the compounds of this embodiment further comprise a phosponate or phosphonate prodrug. More typically, the phosphonate or phosphonate prodrug has the structure A³ as described herein. Each of the preferred embodiments of A³ described herein is a preferred embodiment of A³ in the present embodiment.

Optionally, the compounds of this embodiment demonstrate improved intracellular half-life of the compounds or intracellular metabolites of the compounds in human PBMCs when compared to analogs of the compounds not having the phosphonate or phosphonate prodrug. Typically, the half-life is improved by at least about 50%, more typically at least in the range 50-100%, still more typically at least about 100%, more typically yet greater than about 100%.

In a preferred embodiment, the intracellular half-life of a metabolite of the compound in human PBMCs is improved when compared to an analog of the compound not having the phosphonate or phosphonate prodrug. In such embodiments, the metabolite is typically generated intracellularly, more typically, it is generated within human PBMCs. Still more typically, the metabolite is a product of the cleavage of a phosphonate prodrug within human PBMCs. More typically yet, the phosphonate prodrug is cleaved to form a metabolite having at least one negative charge at physiological pH. Most typically, the phosphonate prodrug is enzymatically cleaved within human PBMCs to form a phosphonate having at least one active hydrogen atom of the form P-OH.

In another aspect of the invention, A³ is of the formula: 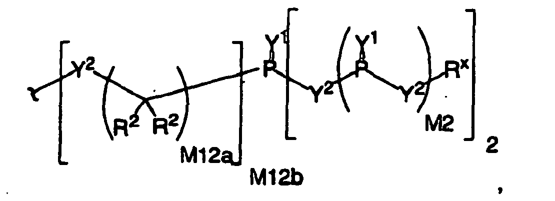 M12a is other than 0 and at least one phosphonate group present in the compound is not bonded directly to W³. More typically, the phosphonate is not bonded directly to W⁵. In such an embodiment, the phosphorous atom of the phosphonate is not bonded directly to a carbon atom of a ring.

In another aspect of the invention an Amprenavir like phosphonate protease inhibitor, as described above in the description and below in the claims, contains an A³ group of the formula: 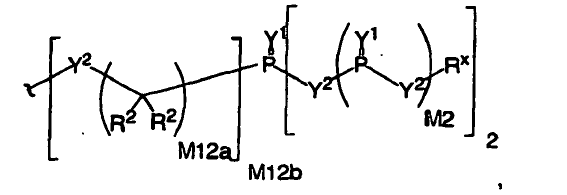 M12a is other than 0 and at least one phosphonate group present in the compound is not bonded directly to W³. More typically, the phosphonate is not bonded directly to W⁵. In such an embodiment, the phosphorous atom of the phosphonate is not bonded directly to a carbon atom of a ring.

One embodiment of Amprenavir like phosphonate protease inhibitors as described above in the description and below in the claims excludes compounds of the formulas: 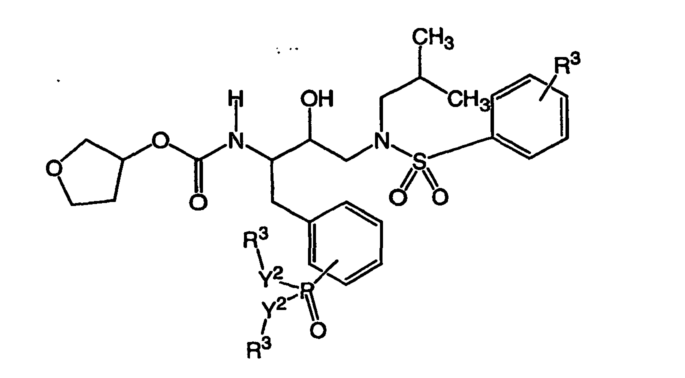 or

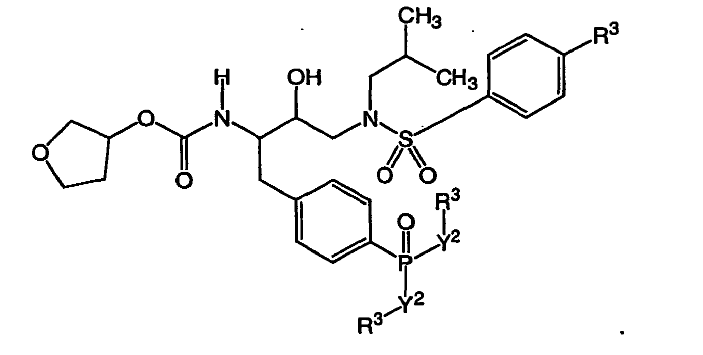

In another aspect of the invention, A³ is of the formula: 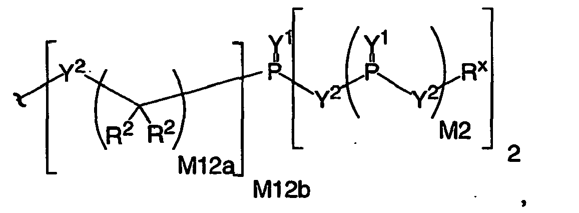 M12a is 0 and at least one phosphonate group present in the compound is bonded directly to W³. More typically, the phosphonate is bonded directly to W⁵. In such an embodiment, the phosphorous atom of the phosphonate is bonded directly to a carbon atom of a ring.

In another aspect of the invention an Amprenavir like phosphonate protease inhibitor, as described above in the description and below in the claims, contains an A³ group of the formula: 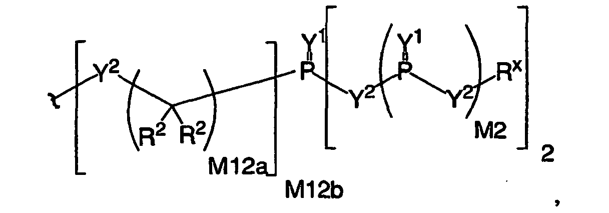 M12a is 0 and at least one phosphonate group present in the compound is bonded directly to W³. More typically, the phosphonate is bonded directly to W⁵. In such an embodiment, the phosphorous atom of the phosphonate is bonded directly to a carbon atom of a ring.

One embodiment of Amprenavir like phosphonate protease inhibitors as described above in the description and below in the claims is directed to compounds of the formulas: 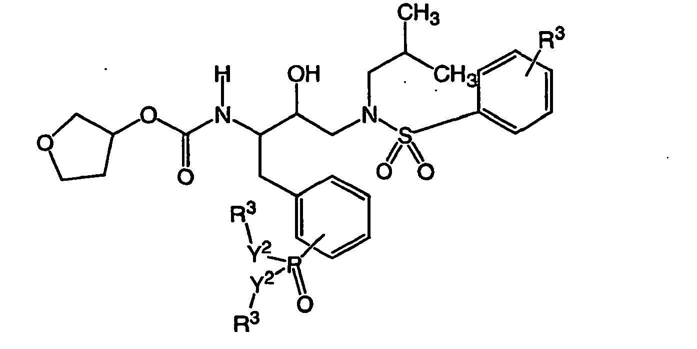 or

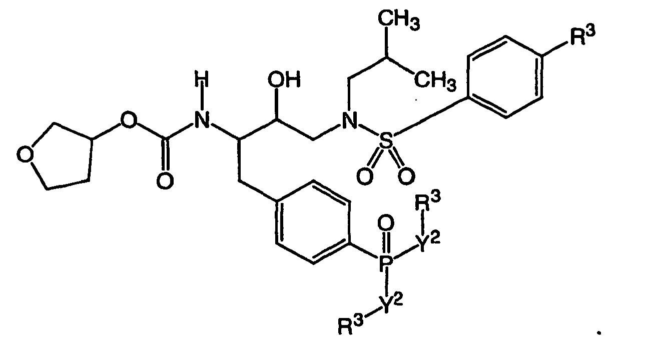

### Recursive Substituents

Selected substituents within the compounds of the invention are present to a recursive degree. In this context, "recursive substituent" means that a substituent may recite another instance of itself. Because of the recursive nature of such substituents, theoretically, a large number of compounds may be present in any given embodiment. For example, R^{x} contains a R^{y} substituent. R^{y} can be R², which in turn can be R³. If R³ is selected to be R^{3c}, then a second instance of R^{x} can be selected. One of ordinary skill in the art of medicinal chemistry understands that the total number of such substituents is reasonably limited by the desired properties of the compound intended. Such properties include, by way of example and not limitation, physical properties such as molecular weight, solubility or log P, application properties such as activity against the intended target, and practical properties such as ease of synthesis.

By way of example and not limitation, W³, R^{y} and R³ are all recursive substituents in certain embodiments. Typically, each of these may independently occur 20, 19, 18, 17, 16, 15, 14, 13, 12, 11, 10, 9, 8, 7, 6, 5, 4, 3, 2, 1, or 0, times in a given embodiment. More typically, each of these may independently occur 12 or fewer times in a given embodiment. More typically yet, W³ will occur 0 to 8 times, R^{y} will occur 0 to 6 times and R³ will occur 0 to 10 times in a given embodiment. Even more typically, W³ will occur 0 to 6 times, R^{y} will occur 0 to 4 times and R³ will occur 0 to 8 times in a given embodiment.

Recursive substituents are an intended aspect of the invention. One of ordinary skill in the art of medicinal chemistry understands the versatility of such substituents. To the degree that recursive substituents are present in an embodiment of the invention, the total number will be determined as set forth above.

### Protecting Groups

In the context of the present invention, embodiments of protecting groups include prodrug moieties and chemical protecting groups.

Protecting groups are available, commonly known and used, and are optionally used to prevent side reactions with the protected group during synthetic procedures, i.e. routes or methods to prepare the compounds of the invention. For the most part the decision as to which groups to protect, when to do so, and the nature of the chemical protecting group "PRT" will be dependent upon the chemistry of the reaction to be protected against (e.g., acidic, basic, oxidative, reductive or other conditions) and the intended direction of the synthesis. The PRT groups do not need to be, and generally are not, the same if the compound is substituted with multiple PRT. In general, PRT will be used to protect functional groups such as carboxyl, hydroxyl or amino groups and to thus prevent side reactions or to otherwise facilitate the synthetic efficiency. The order of deprotection to yield free, deprotected groups is dependent upon the intended direction of the synthesis and the reaction conditions to be encountered, and may occur in any order as determined by the artisan.

Various functional groups of the compounds of the invention may be protection. For example, protecting groups for -OH groups (whether hydroxyl, carboxylic acid, phosphonic acid, or other functions) are embodiments of "ether- or ester-forming groups". Ether- or ester-forming groups are capable of functioning as chemical protecting groups in the synthetic schemes set forth herein. However, some hydroxyl and thio protecting groups are neither ether- nor ester-forming groups, as will be understood by those skilled in the art, and are included with amides, discussed below.

A very large number of hydroxyl protecting groups and amide-forming groups and corresponding chemical cleavage reactions are described in "Protective Groups in Organic Chemistry", Theodora W. Greene (John Wiley & Sons, Inc., New York, 1991, ISBN 0-471-62301-6) ("Greene"). See also Kocienski, Philip J.; "Protecting Groups" (Georg Thieme Verlag Stuttgart, New York, 1994), which is incorporated by reference in its entirety herein. In particular Chapter 1, Protecting Groups: An Overview, pages 1-20, Chapter 2, Hydroxyl Protecting Groups, pages 21-94, Chapter 3, Diol Protecting Groups, pages 95-117, Chapter 4, Carboxyl Protecting Groups, pages 118-154, Chapter 5, Carbonyl Protecting Groups, pages 155-184. For protecting groups for carboxylic acid, phosphonic acid, phosphonate, sulfonic acid and other protecting groups for acids see Greene as set forth below. Such groups include by way of example and not limitation, esters, amides, hydrazides, and the like.

### Ether- and Ester-forming protecting groups

Ester-forming groups include: (1) phosphonate ester-forming groups, such as phosphonamidate esters, phosphorothioate esters, phosphonate esters, and phosphon-bisamidates; (2) carboxyl ester-forming groups, and (3) sulphur ester-forming groups, such as sulphonate, sulfate, and sulfinate.

The phosphonate moieties of the compounds of the invention may or may not be prodrug moieties, i.e. they may or may not be susceptible to hydrolytic or enzymatic cleavage or modification. Certain phosphonate moieties are stable under most or nearly all metabolic conditions. For example, a dialkylphosphonate, where the alkyl groups are two or more carbons, may have appreciable stability *in vivo* due to a slow rate of hydrolysis.

Within the context of phosphonate prodrug moieties, a large number of structurally-diverse prodrugs have been described for phosphonic acids (Freeman and Ross in Progress in Medicinal Chemistry 34: 112-147 (1997) and are included within the scope of the present invention. An exemplary embodiment of a phosphonate ester-forming group is the phenyl carbocycle in substructure A₃ having the formula: 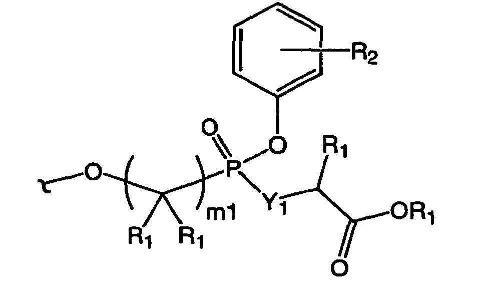 wherein ml is 1, 2, 3, 4, 5, 6, 7 or 8, and the phenyl carbocycle is substituted with 0 to 3 R₂ groups. Also, in this embodiment, where Y₁ is O, a lactate ester is formed. Alternatively, where Y₁ is N(R₂), N(OR₂) or N(N(R₂)₂, then phosphonamidate esters result. R₁ may be H or C₁-C₁₂ alkyl. The corollary exemplary substructure A³ is included in the invention with Y¹, R¹ and R² substituents.

In its ester-forming role, a protecting group typically is bound to any acidic group such as, by way of example and not limitation, a -CO₂H or -C(S)OH group, thereby resulting in -CO₂R^{x} where R^{x} is defined herein. Also, R^{x} for example includes the enumerated ester groups of WO 95/07920.

Examples of protecting groups include:
C₃-C₁₂ heterocycle (described above) or aryl. These aromatic groups optionally are polycyclic or monocyclic. Examples include phenyl, spiryl, 2- and 3-pyrrolyl, 2- and 3-thienyl, 2- and 4-imidazolyl, 2-, 4- and 5-oxazolyl, 3- and 4-isoxazolyl, 2-, 4- and 5-thiazolyl, 3-, 4- and 5-isothiazolyl, 3- and 4-pyrazolyl, 1-, 2-, 3- and 4-pyridinyl, and 1-, 2-, 4- and 5-pyrimidinyl,
C₃-C₁₂ heterocycle or aryl substituted with halo, R¹, R¹-O-C₁-C₁₂ alkylene, C₁-C₁₂ alkoxy, CN, NO₂, OH, carboxy, carboxyester, thiol, thioester, C₁-C₁₂ haloalkyl (1-6 halogen atoms), C₂-C₁₂ alkenyl or C₂-C₁₂ alkynyl. Such groups include 2-, 3- and 4-alkoxyphenyl (C₁-C₁₂ alkyl), 2-, 3- and 4-methoxyphenyl, 2-, 3- and 4-ethoxyphenyl, 2,3-, 2,4-, 2,5-, 2,6-, 3,4- and 3,5-diethoxyphenyl, 2- and 3-carboethoxy-4-hydroxyphenyl, 2- and 3-ethoxy-4-hydroxyphenyl, 2- and 3-ethoxy-5-hydroxyphenyl, 2- and 3-ethoxy-6-hydroxyphenyl, 2-, 3-and 4-O-acetylphenyl, 2-, 3- and 4-dimethylaminophenyl, 2-, 3- and 4-methylmercaptophenyl, 2-, 3- and 4-halophenyl (including 2-, 3- and 4-fluorophenyl and 2-, 3- and 4-chlorophenyl), 2,3-, 2,4-, 2,5-, 2,6-, 3,4- and 3,5-dimethylphenyl, 2,3-, 2,4-, 2,5-, 2,6-, 3,4- and 3,5-biscarboxyethylphenyl, 2,3-, 2,4-, 2,5-, 2,6-, 3,4- and 3,5-dimethoxyphenyl, 2,3-, 2,4-, 2,5-, 2,6-, 3,4- and 3,5-dihalophenyl (including 2,4-difluorophenyl and 3,5-difluorophenyl), 2-, 3- and 4-haloalkylphenyl (1 to 5 halogen atoms, C₁-C₁₂ alkyl including 4-trifluoromethylphenyl), 2-, 3- and 4-cyanophenyl, 2-, 3- and 4-nitrophenyl, 2-, 3- and 4-haloalkylbenzyl (1 to 5 halogen atoms, C₁-C₁₂ alkyl including 4-trifluoromethylbenzyl and 2-, 3- and 4-trichloromethylphenyl and 2-, 3- and 4-trichloromethylphenyl), 4-N-methylpiperidinyl, 3-N-methylpiperidinyl, 1-ethylpiperazinyl, benzyl, alkylsalicylphenyl (C₁-C₄ alkyl, including 2-, 3- and 4-ethylsalicylphenyl), 2-,3- and 4-acetylphenyl, 1,8-dihydroxynaphthyl (-C₁₀H₆-OH) and aryloxy ethyl [C₆-C₉ aryl (including phenoxy ethyl)], 2,2'-dihydroxybiphenyl, 2-, 3- and 4-N,N-dialkylaminophenol, -C₆H₄CH₂-N(CH₃)₂, trimethoxybenzyl, triethoxybenzyl, 2-alkyl pyridinyl (C₁₋₄ alkyl); 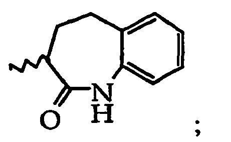 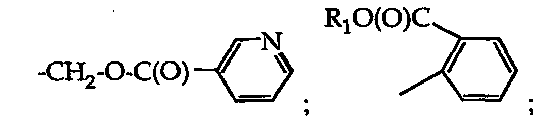 C₄ - C₈ esters of 2-carboxyphenyl; and C₁-C₄ alkylene-C₃-C₆ aryl (including benzyl, -CH₂-pyrrolyl, -CH₂-thienyl, -CH₂-imidazolyl, -CH₂-oxazolyl, -CH₂-isoxazolyl, -CH₂-thiazolyl, -CH₂-isothiazolyl, -CH₂-pyrazolyl, -CH₂-pyridinyl and -CH₂-pyrimidinyl) substituted in the aryl moiety by 3 to 5 halogen atoms or 1 to 2 atoms or groups selected from halogen, C₁-C₁₂ alkoxy (including methoxy and ethoxy), cyano, nitro, OH, C₁-C₁₂ haloalkyl (1 to 6 halogen atoms; including -CH₂CCl₃), C₁-C₁₂ alkyl (including methyl and ethyl), C₂-C₁₂ alkenyl or C₂-C₁₂ alkynyl; alkoxy ethyl [C₁-C₆ alkyl including -CH₂-CH₂-O-CH₃ (methoxy ethyl)]; alkyl substituted by any of the groups set forth above for aryl, in particular OH or by 1 to 3 halo atoms (including -CH₃, -CH(CH₃)₂, -C(CH₃)₃, -CH₂CH₃, -(CH₂)₂CH₃, -(CH₂)₃CH₃, -(CH₂)₄CH₃, -(CH₂)₅CH₃,CH₂CH₂F, -CH₂CH₂Cl, -CH₂CF₃, and -CH₂CCl₃); 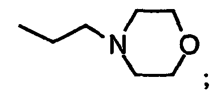 N-2-propylmorpholino, 2,3-dihydro-6-hydroxyindene, sesamol, catechol monoester, -CH₂-C(O)-N(R¹)₂, -CH₂-S(O)(R¹), -CH₂-S(O)₂(R¹), -CH₂-CH(OC(O)CH₂R¹)-CH₂(OC(O)CH₂P¹), cholesteryl, enolpyruvate (HOOC-C(=CH₂)-), glycerol;
a 5 or 6 carbon monosaccharide, disaccharide or oligosaccharide (3 to 9 monosaccharide residues);
triglycerides such as α-D-β-diglycerides (wherein the fatty acids composing glyceride lipids generally are naturally occurring saturated or unsaturated C₆₋₂₆, C₆₋₁₈ or C₆₋₁₀ fatty acids such as linoleic, lauric, myristic, palmitic, stearic, oleic, palmitoleic, linolenic and the like fatty acids) linked to acyl of the parental compounds herein through a glyceryl oxygen of the triglyceride;
phospholipids linked to the carboxyl group through the phosphate of the phospholipid;
phthalidyl (shown in Fig. 1 of Clayton et al., Antimicrob. Agents Chemo. (1974) 5(6):670-671;
cyclic carbonates such as (S-R_{d}-2-oxo-1,3-dioxolen-4-yl) methyl esters (Sakamoto et al., Chem. Pharm. Bull. (1984) 32(6)2241-2248) where R_{d} is R₁, R₄ or aryl; and

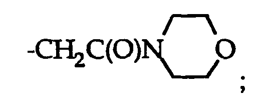

The hydroxyl groups of the compounds of this invention optionally are substituted with one of groups III, IV or V disclosed in WO 94/21604, or with isopropyL

As further embodiments, Table A lists examples of protecting group ester moieties that for example can be bonded via oxygen to -C(O)O- and -P(O)(O-)₂ groups. Several amidates also are shown, which are bound directly to -C(O)- or -P(O)₂. Esters of structures 1-5, 8-10 and 16, 17, 19-22 are synthesized by reacting the compound herein having a free hydroxyl with the corresponding halide (chloride or acyl chloride and the like) and N ,N-dicyclohexyl-N-morpholine carboxamidine (or another base such as DBU, triethylamine, CsCO₃, N,N-dimethylaniline and the like) in DMF (or other solvent such as acetonitrile or N-methylpyrrolidone). When the compound to be protected is a phosphonate, the esters of structures 5-7, 11, 12, 21, and 23-26 are synthesized by reaction of the alcohol or alkoxide salt (or the corresponding amines in the case of compounds such as 13, 14 and 15) with the monochlorophosphonate or dichlorophosphonate (or another activated phosphonate).

**TABLE A**

| | |
|---|---|
| 1. -CH₂-C(O)-N(R₁)₂ * | 10. -CH₂-O-C(O)-C(CH₃)₃ |
| 2. -CH₂-S(O)(R₁) | 11. -CH₂-CCl₃ |
| 3. -CH₂-S(O)₂(R₁) | 12. -C₆H₅ |
| 4. -CH₂-O-C(O)-C7H₂-C₆H₅ | 13. -NH-CH₂-C(O)O-CH₂CH₃ |
| 5. 3-cholesteryl | 14. -N(CH₃)-CH₂-C(O)O-CH₂CH₃ |
| 6. 3-pyridyl | 15. -NHR₁ |
| 7. N-ethylmorpholino | 16. -CH₂-O-C(O)-C₁₀H₁₅ |
| 8. -CH₂-O-C(O)-C₆H₅ | 17. -CH₂-O-C(O)-CH(CH₃)₂ |
| 9. -CH₂-O-C(O)-CH₂CH₃ | 18. -CH₂-C#H(OC(O)CH₂R₁)-CH₂-(OC(O)CH₂R₁)* |
| 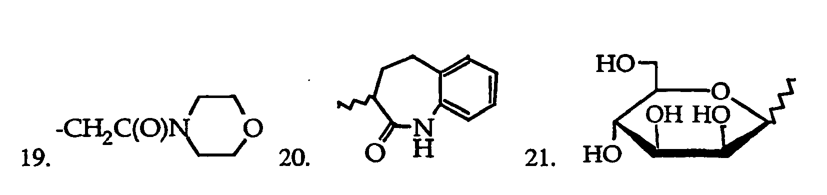 | |
| 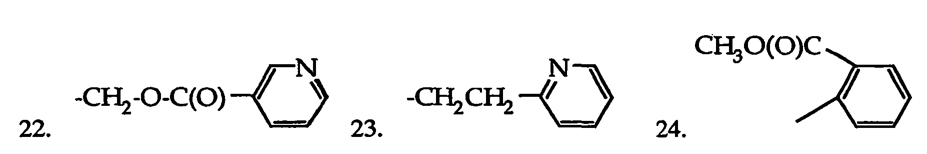 | |
| 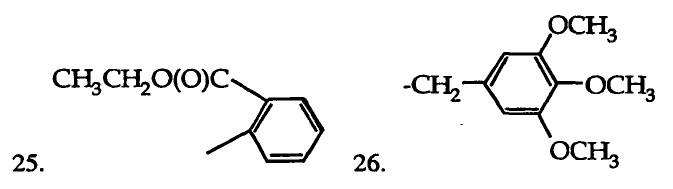 | |

| | |
|---|---|
| # - chiral center is (R), (S) or racemate. | |

Other esters that are suitable for use herein are described in EP 632048.

Protecting groups also includes "double ester" forming profunctionalities such as -CH₂OC(O)OCH₃, 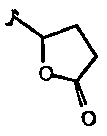 -CH₂SCOCH₃, -CH₂OCON(CH₃)₂, or alkyl- or arylacyloxyalkyl groups of the structure -CH(R¹ or W⁵)O((CO)R³⁷) or -CH(R¹ or W⁵)((CO)OR³⁸) (linked to oxygen of the acidic group) wherein R³⁷ and R³⁸ are alkyl, aryl, or alkylaryl groups (see U.S. Patent No. 4,968,788). Frequently R³⁷ and R³⁸ are bulky groups such as branched alkyl, ortho-substituted aryl, meta-substituted aryl, or combinations thereof, including normal, secondary, iso- and tertiary alkyls of 1-6 carbon atoms. An example is the pivaloyloxymethyl group. These are of particular use with prodrugs for oral administration. Examples of such useful protecting groups are alkylacyloxymethyl esters and their derivatives, including - CH(CH₂CH₂OCH₃)OC(O)C(CH₃)₃, 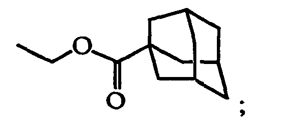 -CH₂OC(O)C₁₀H₁₅, -CH₂OC(O)C(CH₃)₃, -CH(CH₂OCH₃)OC(O)C(CH₃)₃, -CH(CH(CH₃)₂)OC(O)C(CH₃)₃, -CH₂OC(O)CH₂CH(CH₃)₂, -CH₂OC(O)C₆H₁₁, -CH₂OC(O)C₆H₅, -CH₂OC(O)C₁₀H₁₅, -CH₂OC(O)CH₂CH₃, -CH₂OC(O)CH(CH₃)₂, -CH₂OC(O)C(CH₃)₃ and -CH₂OC(O)CH₂C₆H₅.

For prodrug purposes, the ester typically chosen is one heretofore used for antibiotic drugs, in particular the cyclic carbonates, double esters, or the phthalidyl, aryl or alkyl esters.

In some embodiments the protected acidic group is an ester of the acidic group and is the residue of a hydroxyl-containing functionality. In other embodiments, an amino compound is used to protect the acid functionality. The residues of suitable hydroxyl or amino-containing functionalities are set forth above or are found in WO 95/07920. Of particular interest are the residues of amino acids, amino acid esters, polypeptides, or aryl alcohols. Typical amino acid, polypeptide and carboxyl-esterified amino acid residues are described on pages 11-18 and related text of WO 95/07920 as groups L1 or L2. WO 95/07920 expressly teaches the amidates of phosphonic acids, but it will be understood that such amidates are formed with any of the acid groups set forth herein and the amino acid residues set forth in WO 95/07920.

Typical esters for protecting acidic functionalities are also described in WO 95/07920, again understanding that the same esters can be formed with the acidic groups herein as with the phosphonate of the '920 publication. Typical ester groups are defined at least on WO 95/07920 pages 89-93 (under R³¹ or R³⁵), the table on page 105, and pages 21-23 (as R). Of particular interest are esters of unsubstituted aryl such as phenyl or arylalkyl such benzyl, or hydroxy-, halo-, alkoxy-, carboxy- and/or alkylestercarboxy-substituted aryl or alkylaryl, especially phenyl, ortho-ethoxyphenyl, or C₁-C₄ alkylestercarboxyphenyl (salicylate C₁-C₁₂ alkylesters).

The protected acidic groups, particularly when using the esters or amides of WO 95/07920, are useful as prodrugs for oral administration. However, it is not essential that the acidic group be protected in order for the compounds of this invention to be effectively administered by the oral route. When the compounds of the invention having protected groups, in particular amino acid amidates or substituted and unsubstituted aryl esters are administered systemically or orally they are capable of hydrolytic cleavage *in vivo* to yield the free acid.

One or more of the acidic hydroxyls are protected. If more than one acidic hydroxyl is protected then the same or a different protecting group is employed, e.g., the esters may be different or the same, or a mixed amidate and ester may be used.

Typical hydroxy protecting groups described in Greene (pages 14-118) include substituted methyl and alkyl ethers, substituted benzyl ethers, silyl ethers, esters including sulfonic acid esters, and carbonates. For example:
- Ethers (methyl, *t*-butyl, allyl);
- Substituted Methyl Ethers (Methoxymethyl, Methylthiomethyl, *t*-Butylthiomethyl, (Phenyldimethylsilyl)methoxymethyl, Benzyloxymethyl, *p*-Methoxybenzyloxymethyl, (4-Methoxyphenoxy)methyl, Guaiacolmethyl, *t*-Butoxymethyl, 4-Pentenyloxymethyl, Siloxymethyl, 2-Methoxyethoxymethyl, 2,2,2-Trichloroethoxymethyl, Bis(2-chloroethoxy)methyl, 2-(Trimethylsilyl)ethoxymethyl, Tetrahydropyranyl, 3-Bromotetrahydropyranyl, Tetrahydropthiopyranyl, 1-Methoxycyclohexyl, 4-Methoxytetrahydropyranyl, 4-Methoxytetrahydrothiopyranyl, 4-Methoxytetrahydropthiopyranyl *S,S*-Dioxido, 1-[(2-Chloro-4-rnethyl)phenyl]-4-methoxypiperidin-4-yl, 1,4-Dioxan-2-yl, Tetrahydrofuranyl, Tetrahydrothiofuranyl, 2,3,3a,4,5,6,7,7a Octahydro-7,8,8-trimethyl-4,7-methanobenzofuran-2-yl));
- Substituted Ethyl Ethers (1-Ethoxyethyl, 1-(2-Chloroethoxy)ethyl, 1-Methyl-1-methoxyethyl, 1-Methyl-1-benzyloxyethyl, 1-Methyl-1-benzyloxy-2-fluoroethyl, 2,2,2-Trichloroethyl, 2-Trimethylsilylethyl, 2-(Phenylselenyl)ethyl,
- *p*-Chlorophenyl, *p*-Methoxyphenyl, 2,4-Dinitrophenyl, Benzyl);
- Substituted Benzyl Ethers (*p*-Methoxybenzyl, 3,4-Dimethoxybenzyl, *o*-Nitrobenzyl, *p-*Nitrobenzyl, *p*-Halobenzyl, 2,6-Dichlorobenzyl, *p*-Cyanobenzyl, *p*-Phenylbenzyl, 2- and 4-Picolyl, 3-Methyl-2-picolyl *N*-Oxido, Diphenylmethyl, *p,p*'-Dinitrobenzhydryl, 5-Dibenzosuberyl, Triphenylmethyl, α-Naphthyldiphenylmethyl, *p-*methoxyphenyldiphenylmethyl, Di(*p*-methoxyphenyl)phenylmethyl, Tri(*p-*methoxyphenyl)methyl, 4-(4'-Bromophenacyloxy)phenyldiphenylmethyl, 4,4',4"-Tris(4,5-dichlorophthalimidophenyl)methyl, 4,4',4"-Tris(levulinoyloxyphenyl)methyl, 4,4',4"-Tris(benzoyloxyphenyl)methyl, 3-(Imidazol-1-ylmethyl)bis(4',4"-dimethoxyphenyl)methyl, 1,1-Bis(4-methoxyphenyl)-1'-pyrenylmethyl, 9-Anthryl, 9-(9-Phenyl)xanthenyl, 9-(9-Phenyl-10-oxo)anthryl, 1,3-Benzodithiolan-2-yl, Benzisothiazolyl *S,S*-Dioxido);
- Silyl Ethers (Trimethylsilyl, Triethylsilyl, Triisopropylsilyl, Dimethylisopropylsilyl, Diethylisopropylsilyl, Dimethylthexylsilyl, *t*-Butyldimethylsilyl, *t*-Butyldiphenylsilyl, Tribenzylsilyl, Tri-*p*-xylylsilyl, Triphenylsilyl, Diphenylmethylsilyl, *t-*Butylmethoxyphenylsilyl);
- Esters (Formate, Benzoylformate, Acetate, Choroacetate, Dichloroacetate, Trichloroacetate, Trifluoroacetate, Methoxyacetate, Triphenylmethoxyacetate, Phenoxyacetate, *p*-Chlorophenoxyacetate, *p*-poly-Phenylacetate, 3-Phenylpropionate, 4-Oxopentanoate (Levulinate), 4,4-(Ethylenedithio)pentanoate, Pivaloate, Adamantoate, Crotonate, 4-Methoxycrotonate, Benzoate, p-Phenylbenzoate, 2,4,6-Trimethylbenzoate (Mesitoate));
- Carbonates (Methyl, 9-Fluorenylmethyl, Ethyl, 2,2,2-Trichloroethyl, 2-(Trimethylsilyl)ethyl, 2-(Phenylsulfonyl)ethyl, 2-(Triphenylphosphonio)ethyl, Isobutyl, Vinyl, Allyl, *p*-Nitrophenyl, Benzyl, *p*-Methoxybenzyl, 3,4-Dimethoxybenzyl, *o-*Nitrobenzyl, *p*-Nitrobenzyl, *S*-Benzyl Thiocarbonate, 4-Ethoxy-1-naphthyl, Methyl Dithiocarbonate);
- Groups With Assisted Cleavage (2-Iodobenzoate, 4-Azidobutyrate, 4-Nitro-4-methylpentanoate, *o*-(Dibromomethyl)benzoate, 2-Formylbenzenesulfonate, 2-(Methylthiomethoxy)ethyl Carbonate, 4-(Methylthiomethoxy)butyrate, 2-(Methylthiomethoxymethyl)benzoate); Miscellaneous Esters (2,6-Dichloro-4-methylphenoxyacetate, 2,6-Dichloro-4-(1,1,3,3 tetramethylbutyl)phenoxyacetate, 2,4-Bis(1,1-dimethylpropyl)phenoxyacetate, Chlorodiphenylacetate, Isobutyrate, Monosuccinate, (*E*)-2-Methyl-2-butenoate (Tigloate), *o*-(Methoxycarbonyl)benzoate, *p-*poly-Benzoate, α-Naphthoate, Nitrate, Alkyl *N,N,N',N*'-Tetramethylphosphorodiamidate, *N*-Phenylcarbamate, Borate, Dimethylphosphinothioyl, 2,4-Dinitrophenylsulfenate); and
- Sulfonates (Sulfate, Methanesulfonate (Mesylate), Benzylsulfonate, Tosylate).

Typical 1,2-diol protecting groups (thus, generally where two OH groups are taken together with the protecting functionality) are described in Greene at pages 118-142 and include Cyclic Acetals and Ketals (Methylene, Ethylidene, 1-*t*-Butylethylidene, 1-Phenylethylidene, (4-Methoxyphenyl)ethylidene, 2,2,2-Trichloroethylidene, Acetonide (Isopropylidene), Cyclopentylidene, Cyclohexylidene, Cycloheptylidene, Benzylidene, *p-*Methoxybenzylidene, 2,4-Dimethoxybenzylidene, 3,4-Dimethoxybenzylidene, 2-Nitrobenzylidene); Cyclic Ortho Esters (Methoxymethylene, Ethoxymethylene, Dimethoxymethylene, 1-Methoxyethylidene, 1-Ethoxyethylidine, 1,2-Dimethoxyethylidene, α-Methoxybenzylidene, 1*-*(*N,N*-Dimethylamino)ethylidene Derivative, α*-(NN-*Dimethylamino)benzylidene Derivative, 2-Oxacyclopentylidene); Silyl Derivatives (Di-*t-*butylsilylene Group, 1,3-(1,1,3,3-Tetraisopropyldisiloxanylidene), and Tetra-*t-*butoxydisiloxane-1,3-diylidene), Cyclic Carbonates, Cyclic Boronates, Ethyl Boronate and Phenyl Boronate.

More typically, 1,2-diol protecting groups include those shown in Table B, still more typically, epoxides, acetonides, cyclic ketals and aryl acetals.

**Table B**

| |
|---|
| 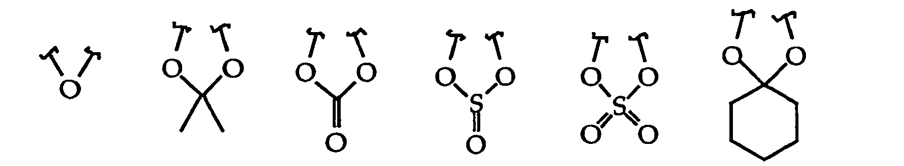 |
| 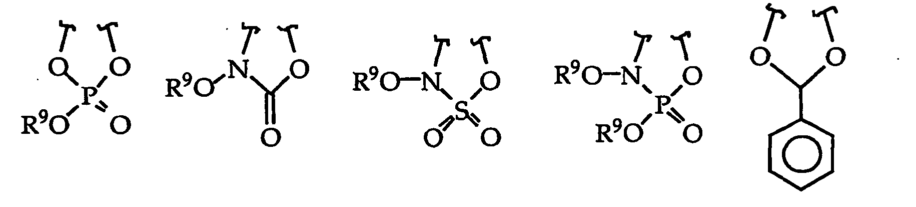 |

wherein R⁹ is C₁-C₆ alkyl.

### Amino protecting groups

Another set of protecting groups include any of the typical amino protecting groups described by Greene at pages 315-385. They include:
- Carbamates: (methyl and ethyl, 9-fluorenylmethyl, 9(2-sulfo)fluorenylmethyl, 9-(2,7-dibromo)fluorenylmethyl, 2,7-di-*t*-butyl-[9-(10,10-dioxo-10,10,10,10-tetrahydrothioxanthyl)]methyl, 4-methoxyphenacyl);
- Substituted Ethyl: (2,2,2-trichoroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, 2-*(N,N* dicyclohexylcarboxamido)ethyl, *t*-butyl, 1-adamantyL vinyl, allyl, 1-isopropylallyl, cinnamyl, 4-nitrocinnamyl, 8-quinolyl, *N-*hydroxypiperidinyl, alkyldithio, benzyl, *p*-methoxybenzyl, p-nitrobenzyl, *p*-bromobenzyl, *p*-chlorobenzyl, 2,4-dichlorobenzyl, 4-methylsulfinylbenzyl, 9-anthrylmethyl, diphenylmethyl);
- Groups With Assisted Cleavage: (2-methylthioethyl, 2-methylsulfonylethyl, 2-(*p-*toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, *m*-choro*-p*-acyloxybenzyl, *p*-(dihydroxyboryl)benzyl, 5-benzisoxazolylmethyl, 2-(trifluororriethyl)-6-chromonylmethyl);
- Groups Capable of Photolytic Cleavage: (*m*-nitrophenyl, 3,5-dimethoxybenzyl, o-nitrobenzyl, 3,4-dimethoxy-6-nitrobenzyl, phenyl(*o*-nitrophenyl)methyl); Urea-Type Derivatives (phenothiazinyl-(10)-carbonyl, *N*'-*p*-toluenesulfonylaminocarbonyl, *N*'-phenylaminothiocarbonyl);
- Miscellaneous Carbamates: (*t*-amyl, *S*-benzyl thiocarbamate, *p*-cyanobenzyl, cyclobutyl, cyclohexyl, cyclopentyl, cyclopropylmethyl, *p*-decyloxybenzyl, diisopropylmethyl, 2,2-dimethoxycarbonylvinyl, *o-*(*N,N*-dimethylcarboxamido)benzyl, 1,1-dimethyl-3*-*(*N,N-*dimethylcarboxamido)propyl, 1,1-dimethylpropynyl, di(2-pyridyl)methyl, 2-furanylmethyl, 2-Iodoethyl, Isobornyl, Isobutyl, Isonicotinyl, *p-(p'-*Methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p-*phenylazophenyl)ethyl, 1-methyl-1-phenylethyl, 1-methyl-1-(4-pyridyl)ethyl, phenyl, *p-*(phenylazo)benzyl, 2,4,6-tri-*t*-butylphenyl, 4-(trimethylammonium)benzyl, 2,4,6-trimethylbenzyl);
- Amides: (*N*-formyl, *N*-acetyl, *N*-choroacetyl, *N*-trichoroacetyl, *N*-trifluoroacetyl, *N-*phenylacetyl, *N*-3-phenylpropionyl, *N*-picolinoyl, *N*-3-pyridylcarboxamide, *N-*benzoylphenylalanyl, *N*-benzoyl, *N*-*p*-phenylbenzoyl);
- Amides With Assisted Cleavage: (*N*-*o*-nitrophenylacetyl, *N*-*o*-nitrophenoxyacetyl, *N-*acetoacetyl, (*N*'-dithiobenzyloxycarbonylamino)acetyl, *N*-3-(*p*-hydroxyphenyl)propionyl, *N*-3-(*o*-nitrophenyl)propionyl, *N*-2-methyl- 2-(*o*-nitrophenoxy)propionyl, *N*-2-methyl-2-(*o*-phenylazophenoxy)propionyl, *N*-4-chlorobutyryl, *N*-3-methyl-3-nitrobutyryl, *N-o-*nitrocinnamoyl, *N*-acetylmethionine, *N*-*o*-nitrobenzoyl, *N*-*o*-(benzoyloxymethyl)benzoyl, 4,5-diphenyl-3-oxazolin-2-one);
- Cyclic Imide Derivatives: (*N*-phthalimide, *N*-dithiasuccinoyl, *N*-2,3-diphenylmaleoyl, *N-*2,5-dimethylpyrrolyl, *N*-1,1,4,4-tetramethyldisilylazacyclopentane adduct, 5-substituted 1,3-dimethyl-1,3,5-triazacyclohexan-2-one, 5-substituted 1,3-dibenzyl-1,3-5-triazacyclohexan-2-one, 1-substituted 3,5-dinitro-4-pyridonyl);
- *N*-Alkyl and *N*-Aryl Amines: (*N*-methyl, *N*-allyl, *N-*[2-(trimethylsilyl)ethoxy]methyl, *N-*3-acetoxypropyl, *N*-(1-isopropyl-4-nitro-2-oxo-3-pyrrolin-3-yl), Quaternary Ammonium Salts, *N*-benzyl, *N*-di(4-methoxyphenyl)methyl, *N*-5-dibenzosuberyl, *N*-triphenylmethyl, *N*-(4-methoxyphenyl)diphenylmethyl, *N*-9-phenylfluorenyl, *N-*2,7-dichloro-9-fluorenylmethylene, *N*-ferrocenylmethyl, *N*-2-picolylamine N-oxide);
- Imine Derivatives: (*N*-1,1-dimethylthiomethylene, *N*-benzylidene, *N-p-*methoxybenylidene, *N*-diphenylmethylene, *N*-[(2-pyridyl)mesityl]methylene, *N,*(*N',N'-*dimethylaminomethylene, *N,N'*-isopropylidene, *N-p*-nitrobenzylidene, *N*-salicylidene, *N-*5-chlorosalicylidene, *N*-(5-chloro-2-hydroxyphenyl)phenylmethylene, *N-*cyclohexylidene);
- Enamine Derivatives: (*N*-(5,5-dimethyl-3-oxo-1-cyclohexenyl));
- *N*-Metal Derivatives (*N*-borane derivatives, *N*-diphenylborinic acid derivatives, *N-*[phenyl(pentacarbonylchromium- or -tungsten)]carbenyl, *N*-copper or *N*-zinc chelate);
- N-N Derivatives: (*N*-nitro, *N*-nitroso, *N*-oxide);
- N-P Derivatives: (*N*-diphenylphosphinyl, *N*-dimethylthiophosphinyl, *N-*diphenylthiophosphinyl, *N*-dialkyl phosphoryl, *N*-dibenzyl phosphoryl, *N*-diphenyl phosphoryl);
- N-Si Derivatives, N-S Derivatives, and N-Sulfenyl Derivatives: (*N*-benzenesulfenyl, *N-o-*nitrobenzenesulfenyl, *N*-2,4-dinitrobenzenesulfenyl, *N*-pentachlorobenzenesulfenyl, *N*-2-nitro-4-methoxybenzenesulfenyl, *N*-triphenylmethylsulfenyl, *N*-3-nitropyridinesulfenyl); and *N*-sulfonyl Derivatives (*N*-*p*-toluenesulfonyl, *N*-benzenesulfonyl, *N*-2,3,6-trimethyl-4-methoxybenzenesulfonyl, *N-*2,4,6-trimethoxybenzenesulfonyl, *N-*2,6-dimethyl-4-methoxybenzenesulfonyl, *N-*pentamethylbenzenesulfonyl, *N*-2,3,5,6,-tetramethyl-4-methoxybenzenesulfonyl, *N*-4-methoxybenzenesulfonyl, *N*-2,4,6-trimethylbenzenesulfonyl, *N*-2,6-dimethoxy-4-methylbenzenesulfonyl, *N-*2,2,5,7,8-pentamethylchroman-6-sulfonyl, *N*-methanesulfonyl, *N*-β-trimethylsilyethanesulfonyl, *N-*9-anthracenesulfonyl, *N*-4-(4',8'-dimethoxynaphthylmethyl)benzenesulfonyl, *N-*benzylsulfonyl, *N*-trifluoromethylsulfonyl, *N*-phenacylsulfonyl).

Protected amino groups include carbamates, amides and amidines, e.g. -NHC(O)OR¹, -NHC(O)R¹ or -N=CRN(R¹)₂. Another protecting group, also useful as a prodrug for amino or -NH(R⁵), is: 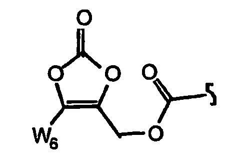 See for example Alexander, J. et al (1996) J. Med. Chem. 39:480-486.

### Amino acid and polypeptide protecting group and conjugates

An amino acid or polypeptide protecting group of a compound of the invention has the structure R¹⁵NHCH(R¹⁶)C(O)-, where R¹⁵ is H, an amino acid or polypeptide residue, or R⁵, and R¹⁶ is defined below.

R¹⁶ is lower alkyl or lower alkyl (C₁-C₆) substituted with amino, carboxyl, amide, carboxyl ester, hydroxyl, C₆-C₇ aryl, guanidinyl, imidazolyl, indolyl, sulfhydryl, sulfoxide, and/or alkylphosphate. R¹⁶ also is taken together with the amino acid α,-N to form a proline residue (R¹⁶ = -CH₂)₃-). However, R¹⁶ is generally the side group of a naturally-occurring amino acid such as H, -CH₃, -CH(CH₃)₂, -CH₂-CH(CH₃)₂, -CHCH₃-CH₂-CH₃, -CH₂-C₆H₅, -CH₂CH₂-S-CH₃, -CH₂OH, -CH(OH)-CH₃, -CH₂-SH, -CH₂-C₆H₄OH, -CH₂-CO-NH₂, -CH₂-CH₂-CO-NH₂, -CH₂-COOH, -CH₂-CH₂-COOH, -(CH₂)₄-NH₂ and -(CH₂)₃-NH-C(NH₂)-NH₂. R¹⁶ also includes 1-guanidinoprop-3-yl, benzyl, 4-hydroxybenzyl, imidazol-4-yl, indol-3-yl, methoxyphenyl and ethoxyphenyl.

Another set of protecting groups include the residue of an amino-containing compound, in particular an amino acid, a polypeptide, a protecting group, -NHSO₂R, NHC(O)R, -N(R)₂, NH₂ or -NH(R)(H), whereby for example a carboxylic acid is reacted, i.e. coupled, with the amine to form an amide, as in C(O)NR₂. A phosphonic acid may be reacted with the amine to form a phosphonamidate, as in -P(0)(OR)(NR₂).

Amino acids have the structure R¹⁷C(O)CH(R¹⁶)NH-, where R¹⁷ is -OH, -OR, an amino acid or a polypeptide residue. Amino acids are low molecular weight compounds, on the order of less than about 1000 MW and which contain at least one amino or imino group and at least one carboxyl group. Generally the amino acids will be found in nature, i.e., can be detected in biological material such as bacteria or other microbes, plants, animals or man. Suitable amino acids typically are alpha amino acids, i.e. compounds characterized by one amino or imino nitrogen atom separated from the carbon atom of one carboxyl group by a single substituted or unsubstituted alpha carbon atom. Of particular interest are hydrophobic residues such as mono-or di-alkyl or aryl amino acids, cycloalkylamino acids and the like. These residues contribute to cell permeability by increasing the partition coefficient of the parental drug. Typically, the residue does not contain a sulfhydryl or guanidino substituent.

Naturally-occurring amino acid residues are those residues found naturally in plants, animals or microbes, especially proteins thereof. Polypeptides most typically will be substantially composed of such naturally-occurring amino acid residues. These amino acids are glycine, alanine, valine, leucine, isoleucine, serine, threonine, cysteine, methionine, glutamic acid, aspartic acid, lysine, hydroxylysine, arginine, histidine, phenylalanine, tyrosine, tryptophan, proline, asparagine, glutamine and hydroxyproline. Additionally, unnatural amino acids, for example, valanine, phenylglycine and homoarginine are also included. Commonly encountered amino acids that are not gene-encoded may also be used in the present invention. All of the amino acids used in the present invention may be either the D- or L- optical isomer. In addition, other peptidomimetics are also useful in the present invention. For a general review, see Spatola, A. F., in Chemistry and Biochemistry of Amino Acids, Peptides and Proteins, B. Weinstein, eds., Marcel Dekker, New York, p. 267 (1983).

When protecting groups are single amino acid residues or polypeptides they optionally are substituted at R³ of substituents A¹, A² or A³ in Formula I, or substituted at R₃ of substituents A₁, A₂ or A₃ in Formula II. These conjugates are generally produced by forming an amide bond between a carboxyl group of the amino acid (or C-terminal amino acid of a polypeptide for example). Alternatively, conjugates are formed between R³ (Formula I) or R₃ (Formula II) and an amino group of an amino acid or polypeptide. Generally, only one of any site in the scaffold drug-like compound is amidated with an amino acid as described herein, although it is within the scope of this invention to introduce amino acids at more than one permitted site. Usually, a carboxyl group of R³ is amidated with an amino acid. In general, the α-amino or α-carboxyl group of the amino acid or the terminal amino or carboxyl group of a polypeptide are bonded to the scaffold, parental functionalities. Carboxyl or amino groups in the amino acid side chains generally may be used to form the amide bonds with the parental compound or these groups may need to be protected during synthesis of the conjugates as described further below.

With respect to the carboxyl-containing side chains of amino acids or polypeptides it will be understood that the carboxyl group optionally will be blocked, e.g. by R¹, esterified with R⁵ or amidated. Similarly, the amino side chains R¹⁶ optionally will be blocked with R¹ or substituted with R⁵.

Such ester or amide bonds with side chain amino or carboxyl groups, like the esters or amides with the parental molecule, optionally are hydrolyzable in *vivo* or *in vitro* under acidic (pH <3) or basic (pH >10) conditions. Alternatively, they are substantially stable in the gastrointestinal tract of humans but are hydrolyzed enzymatically in blood or in intracellular environments. The esters or amino acid or polypeptide amidates also are useful as intermediates for the preparation of the parental molecule containing free amino or carboxyl groups. The free acid or base of the parental compound, for example, is readily formed from the esters or amino acid or polypeptide conjugates of this invention by conventional hydrolysis procedures.

When an amino acid residue contains one or more chiral centers, any of the D, L, meso, threo or erythro (as appropriate) racemates, scalemates or mixtures thereof may be used. In general, if the intermediates are to be hydrolyzed non-enzymatically (as would be the case where the amides are used as chemical intermediates for the free acids or free amines), D isomers are useful. On the other hand, L isomers are more versatile since they can be susceptible to both non-enzymatic and enzymatic hydrolysis, and are more efficiently transported by amino acid or dipeptidyl transport systems in the gastrointestinal tract.

Examples of suitable amino acids whose residues are represented by R^{x} or R^{y} include the following:
Glycine;
Aminopolycarboxylic acids, e.g., aspartic acid, β-hydroxyaspartic acid, glutamic acid, β -hydroxyglutamic acid, β-methylaspartic acid, β-methylglutamic acid, β, β-dimethylaspartic acid, γ-hydroxyglutamic acid, β, γ-dihydroxyglutamic acid, β -phenylglutamic acid, γ-methyleneglutamic acid, 3-aminoadipic acid, 2-aminopimelic acid, 2-aminosuberic acid and 2-aminosebacic acid;
Amino acid amides such as glutamine and asparagine;
Polyamino- or polybasic-monocarboxylic acids such as arginine, lysine, β-aminoalanine, γ -aminobutyrine, ornithine, citruline, homoarginine, homocitrulline, hydroxylysine, allohydroxylsine and diaminobutyric acid;
Other basic amino acid residues such as histidine;
Diaminodicarboxylic acids such as α, α'-diaminosuccinic acid, α, α'-diaminoglutaric acid, α, α'-diaminoadipic acid, α, α'-diaminopimelic acid, α, α'-diamino- β-hydroxypimelic acid, α, α'-diaminosuberic acid, α, α'-diaminoazelaic acid, and α, α'-diaminosebacic acid;
Imino acids such as proline, hydroxyproline, allohydroxyproline, γ-methylproline, pipecolic acid, 5-hydroxypipecolic acid, and azetidine-2-carboxylic acid;
A mono- or di-alkyl (typically C₁-C₈ branched or normal) amino acid such as alanine, valine, leucine, allylglycine, butyrine, norvaline, norleucine, heptyline, α-methylserine, α-amino-α-methyl-γ-hydroxyvalcric acid, α-amino- α-methyl-δ-hydroxyvaleric acid, α-amino-α-methyl-ε-hydroxycaproic acid, isovaline, α-methylglutamic acid, α-aminoisobutyric acid, α-aminodiethylacetic acid, α-aminodiisopropylacetic acid, α-aminodi-n-propylacetic acid, α-aminodiisobutylacetic acid, α-aminodi-n-butylacetic acid, α-aminoethylisopropylacetic acid, α-amino-n-propylacetic acid, α-aminodiisoamyacetic acid, α-methylaspartic acid, α-methylglutamic acid, 1-aminocyclopropane-1-carboxylic acid, isoleucine, alloisoleucine, *tert-*leucine, β-methyltryptophan and α-amino- β-ethyl-β-phenylpropionic acid;
β-phenylserinyl;
Aliphatic α-amino-β-hydroxy acids such as serine, β-hydroxyleucine, β-hydroxynorleucine,β -hydroxynorvaline, and α-amino-β-hydroxystearic acid;
α-Amino, α-, γ-, δ- or ε-hydroxy acids such as homoserine, δ -hydroxynorvaline, γ-hydroxynorvaline and ε-hydroxynorleucine residues; canavine and canaline; γ-hydroxyornithine;
2-hexosaminic acids such as D-glucosaminic acid or D-galactosaminic acid;
α-Amino-β-thiols such as penicillamine, β-thiolnorvali.ne or β-thiolbutyrine;

Other sulfur containing amino acid residues including cysteine; homocystine, β-phenylmethionine, methionine, S-allyl-L-cysteine sulfoxide, 2-thiolhistidine, cystathionine, and thiol ethers of cysteine or homocysteine;

Phenylalanine, tryptophan and ring-substituted α-amino acids such as the phenyl- or cyclohexylamino acids α-aminophenylacetic acid, α-aminocyclohexylacetic acid and α-amino-β-cyclohexylpropionic acid; phenylalanine analogues and derivatives comprising aryl, lower alkyl, hydroxy, guanidino, oxyalkylether, nitro, sulfur or halo-substituted phenyl (e.g., tyrosine, methyltyrosine and o-chloro-, p-chloro-, 3,4-dichloro, *o-, m-* or p-methyl-, 2,4,6-trimethyl-, 2-ethoxy-5-nitro-, 2-hydroxy-5-nitro- and p-nitro-phenylalanine); furyl-, thienyl-, pyridyl-, pyrimidinyl-, purinyl- or naphthyl-alanines; and tryptophan analogues and derivatives including kynurenine, 3-hydroxykynurenine, 2-hydroxytryptophan and 4-carboxytryptophan;

α-Amino substituted amino acids including sarcosine (N-methylglycine), N-benzylglycine, N-methylalanine, N-benzylalanine, N-methylphenylalanine, N-benzylphenylalanine, N-methylvaline and N-benzylvaline; and

α-Hydroxy and substituted α-hydroxy amino acids including serine, threonine, allothreonine, phosphoserine and phosphothreonine.

Polypeptides are polymers of amino acids in which a carboxyl group of one amino acid monomer is bonded to an amino or imino group of the next amino acid monomer by an amide bond. Polypeptides include dipeptides, low molecular weight polypeptides (about 1500-5000 MW) and proteins. Proteins optionally contain 3, 5, 10, 50, 75, 100 or more residues, and suitably are substantially sequence-homologous with human, animal, plant or microbial proteins. They include enzymes (e.g., hydrogen peroxidase) as well as immunogens such as KLH, or antibodies or proteins of any type against which one wishes to raise an immune response. The nature and identity of the polypeptide may vary widely.

The polypeptide amidates are useful as immunogens in raising antibodies against either the polypeptide (if it is not immunogenic in the animal to which it is administered) or against the epitopes on the remainder of the compound of this invention.

Antibodies capable of binding to the parental non-peptidyl compound are used to separate the parental compound from mixtures, for example in diagnosis or manufacturing of the parental compound. The conjugates of parental compound and polypeptide generally are more immunogenic than the polypeptides in closely homologous animals, and therefore make the polypeptide more immunogenic for facilitating raising antibodies against it. Accordingly, the polypeptide or protein may be immunogenic in an animal typically used to raise antibodies, e.g., rabbit, mouse, horse, or rat. The polypeptide optionally contains a peptidolytic enzyme cleavage site at the peptide bond between the first and second residues adjacent to the acidic heteroatom. Such cleavage sites are flanked by enzymatic recognition structures, e.g. a particular sequence of residues recognized by a peptidolytic enzyme.

Peptidolytic enzymes for cleaving the polypeptide conjugates of this invention are well known, and in particular include carboxypeptidases, which digest polypeptides by removing C-terminal residues, and are specific in many instances for particular C-terminal sequences. Such enzymes and their substrate requirements in general are well known. For example, a dipeptide (having a given pair of residues and a free carboxyl terminus) is covalently bonded through its α-amino group to the phosphorus or carbon atoms of the compounds herein. In certain embodiments, a phosphonate group substituted with an amino acid or peptide will be cleaved by the appropriate peptidolytic enzyme, leaving the carboxyl of the proximal amino acid residue to autocatalytically cleave the phosphonoamidate bond.

Suitable dipeptidyl groups (designated by their single letter code) are AA, AR, AN, AD, AC, AE, AQ, AG, AH, AI, AL, AK, AM, AF, AP, AS, AT, AW, AY, AV, RA, RR, RN, RD, RC, RE, RQ, RG, RH, RI, RL, RK, RM, RF, RP, RS, RT, RW, RY, RV, NA, NR, NN, ND, NC, NE, NQ, NG, NH, NI, NL, NK, NM, NF, NP, NS, NT, NW, NY, NV, DA, DR, DN, DD, DC, DE, DQ, DG, DH, DI, DL, DK, DM, DF, DP, DS, DT, DW, DY, DV, CA, CR, CN, CD, CC, CE, CQ, CG, CH, CI, CL, CK, CM, CF, CP, CS, CT, CW, CY, CV, EA, ER, EN, ED, EC, EE, EQ, EG, EH, EI, EL, EK, EM, EF, EP, ES, ET, EW, EY, EV, QA, QR, QN, QD, QC, QE, QQ, QG, QH, QI, QL, QK, QM, QF, QP, QS, QT, QW, QY, QV, GA, GR, GN, GD, GC, GE, GQ, GG, GH, GI, GL, GK, GM, GF, GP, GS, GT, GW, GY, GV, HA, HR, HN, HD, HC, HE, HQ, HG, HH, HI, HL, HK, HM, HF, HP, HS, HT, HW, HY, HV, IA, IR, IN, ID, IC, IE, IQ, IG, IH, II, IL., IK, IM, IF, IP, IS, IT, IW, IY, IV, LA, I-.R, LN, LD, LC, LE, LQ, LG, LH, LI, LL, LK, LM, LF, LP, LS, LT, LW, LY, LV, KA, KR, KN, KD, KC, KE, KQ, KG, KH, KI, KL, KK, KM, KF, KP, KS, KT, KW, KY, KV, MA, MR, MN, MD, MC, ME, MQ, MG, MH, MI, ML, MK, MM, MF, MP, MS, MT, MW, MY, MV, FA, FR, FN, FD, FC, FE, FQ, FG, FH, FI, FL, FK, FM, FF, FP, FS, FT, FW, FY, FV, PA, PR, PN, PD, PC, PE, PQ, PG, PH, PI, PL, PK, PM, PF, PP, PS, PT, PW, PY, PV, SA, SR, SN, SD, SC, SE, SQ, SG, SH, SI, SL, SK, SM, SF, SP, SS, ST, SW, SY, SV, TA, TR, TN, TD, TC, TE, TQ, TG, TH, TI, TL, TK, TM, TF, TP, TS, TT, TW, TY, TV, WA, WR, WN, WD, WC, WE, WQ, WG, WH, WI, WL, WK, WM, WF, WP, WS, WT, WW, WY, WV, YA, YR, YN, YD, YC, YE, YQ, YG, YH, YI, YL, YK, YM, YF, YP, YS, YT, YW, YY, YV, VA, VR, VN, VD, VC, VE, VQ, VG, VH, VI, VL, VK, VM, VF, VP, VS, VT, VW, VY and VV.

Tripeptide residues are also useful as protecting groups. When a phosphonate is to be protected, the sequence -X⁴-pro-X⁵- (where X⁴ is any amino acid residue and X⁵ is an amino acid residue, a carboxyl ester of proline, or hydrogen) will be cleaved by luminal carboxypeptidase to yield X⁴ with a free carboxyl, which in turn is expected to autocatalytically cleave the phosphonoamidate bond. The carboxy group of X⁵ optionally is esterified with benzyl.

Dipeptide or tripeptide species can be selected on the basis of known transport properties and/or susceptibility to peptidases that can affect transport to intestinal mucosal or other cell types. Dipeptides and tripeptides lacking an α-amino group are transport substrates for the peptide transporter found in brush border membrane of intestinal mucosal cells (Bai, J.P.F., (1992) Pharm Res. 9:969-978. Transport competent peptides can thus be used to enhance bioavailability of the amidate compounds. Di- or tripeptides having one or more amino acids in the D configuration may be compatible with peptide transport. Amino acids in the D configuration can be used to reduce the susceptibility of a di- or tripeptide to hydrolysis by proteases common to the brush border such as aminopeptidase N. In addition, di- or tripeptides alternatively are selected on the basis of their relative resistance to hydrolysis by proteases found in the lumen of the intestine. For example, tripeptides or polypeptides lacking asp and/or glu are poor substrates for aminopeptidase A, di- or tripeptides lacking amino acid residues on the N-terminal side of hydrophobic amino acids (leu, tyr, phe, val, trp) are poor substrates for endopeptidase, and peptides lacking a pro residue at the penultimate position at a free carboxyl terminus are poor substrates for carboxypeptidase P. Similar considerations can also be applied to the selection of peptides that are either relatively resistant or relatively susceptible to hydrolysis by cytosolic, renal, hepatic, serum or other peptidases. Such poorly cleaved polypeptide amidates are immunogens or are useful for bonding to proteins in order to prepare immunogens.

### Phosphonate analogs of known experimental or approved Protease Inhibitor Drugs

The known experimental or approved protease inhibitor drugs which can be derivatized in accord with the present invention must contain at least one functional group capable of linking, i.e. bonding to the phosphorus atom in the phosphonate moiety. The phosphonate derivatives of Formulas I-VI may cleave *in vivo* in stages after they have reached the desired site of action, i.e. inside a cell. One mechanism of action inside a cell may entail a first cleavage, e.g. by esterase, to provide a negatively-charged "locked-in" intermediate. Cleavage of a terminal ester grouping in Formulas I-VI thus affords an unstable intermediate which releases a negatively charged "locked in" intermediate.

After passage inside a cell, intracellular enzymatic cleavage or modification of the phosphonate prodrug compound may result in an intracellular accumulation of the cleaved or modified compound by a "trapping" mechanism. The cleaved or modified compound may then be "locked-in" the cell, i.e. accumulate in the cell by a significant change in charge, polarity, or other physical property change which decreases the rate at which the cleaved or modified compound can exit the cell, relative to the rate at which it entered as the phosphonate prodrug. Other mechanisms by which a therapeutic effect is achieved may be operative as well. Enzymes which are capable of an enzymatic activation mechanism with the phosphonate prodrug compounds of the invention include, but are not limited to, amidases, esterases, microbial enzymes, phospholipases, cholinesterases, and phosphatases.

In selected instances in which the drug is of the nucleoside type, such as is the case of zidovudine and numerous other antiretroviral agents, it is known that the drug is activated *in vivo* by phosphorylation. Such activation may occur in the present system by enzymatic conversion of the "locked-in" intermediate with phosphokinase to the active phosphonate diphosphate and/or by phosphorylation of the drug itself after its release from the "locked-in" intermediate as described above. In either case, the original nucleoside-type drug will be converted, via the derivatives of this invention, to the active phosphorylated species.

From the foregoing, it will be apparent that many structurally different known approved and experimental HIV protease inhibitor drugs can be derivatized in accord with the present invention. Numerous such drugs are specifically mentioned herein. However, it should be understood that the discussion of drug families and their specific members for derivatization according to this invention is not intended to be exhaustive, but merely illustrative.

As another example, when the selected drug contains multiple reactive hydroxyl functions, a mixture of intermediates and final products may again be obtained. In the unusual case in which all hydroxy groups are approximately equally reactive, there is not expected to be a single, predominant product, as each mono-substituted product will be obtained in approximate by equal amounts, while a lesser amount of multiply-substituted product will also result. Generally speaking, however, one of the hydroxyl groups will be more susceptible to substitution than the other(s), e.g. a primary hydroxyl will be more reactive than a secondary hydroxyl, an unhindered hydroxyl will be more reactive than a hindered one. Consequently, the major product will be a mono-substituted one in which the most reactive hydroxyl has been derivatized while other mono-substituted and multiply-substituted products may be obtained as minor products.

Formula I to VI compounds having a 2-hydroxy-1, 3-amino-propylamide or 2-hydroxy-1,3-amino-propylaminosulfone core include Amprenavir-like phosphonate protease inhibitors (AMLPPI). Compounds of the invention include phosphonate analogs of other known PI compounds with a 2-hydroxy-3-amido-propylamide or 2-hydroxy-3-amido-propylaminosulfone core which have been identified as Droxinavir, Telinavir, Iddb51 (Searle); Ph4556 (WO 95/29922; Ph5145 (WO 96/31527; DPC-681, DPC-684 (DuPont); VB- 11328 (Vertex); TMC-114 (Tibotech/Johnson & Johnson). The compounds also include phosphonate analogs of fosamprenavir where the 2-hydroxy is phosphorylated, i.e. having a or 2-phosphate-1,3-amino-propylaminosulfone core (US Patent No. 6,436,989).

The embodiments of the invention also include the following phosphonate analogs represented as Formulas IIa-IIg: 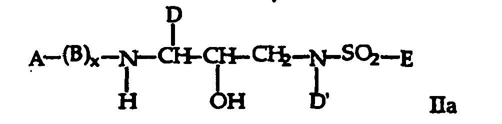 described as "(I)" in: WO 94/05639 (published 17 March 1994) at page 4, line 15, to page 6, line 27, page 15, line 21, to page 17, line 33, and Claim 1; US Patent No. 5,585,397 (issued 17 December 1996) at coL 2, line 45, to col. 3, line 53, and col. 8, line 1, to col. 9, line 12; US Patent No. 5,783, 701 (issued 21 July 1998) at col. 2, line 43, to col. 3, line 64, col. 8, line 13, to coL 9, line 33, and Claim 1; US Patent No. 5,856,353 (issued 5 January 1999) at coL 2, line 45, to col. 3, line 65, col. 8, line 14, to coL 9, line 37, and Claim 1; US Patent No. 5,977,137 (issued 2 November 1999) at col. 2, line 43, to col. 3, line 65, col. 8, line 15, to col. 9, line 38, and Claim 1; and US Patent No. 6,004,957 (issued 21 December 1999) at coL 2, line 47, to col. 4, line 3, col. 8, line 18, to col. 9, line 41, and Claim 1 therein. 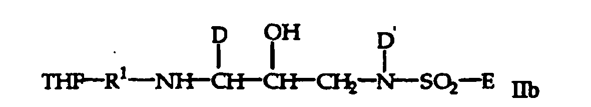 described as "(I)" in: WO 96/33184 (published 24 October 1996) at page 4, line 19, to page 6, line 5, page 17, line 11, to page 19, line 31, and Claim 1; and US Patent No. 5,723,490 (issued 3 March 1998) at col. 2, line 49, to coL 3, line 39, col. 8, line 66, to coL 10, line 36, and Claim 1. 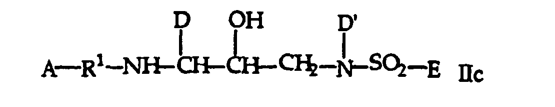 described as "(I)" in: WO 96/33187 (published 24 October 1996) at page 4, line 23, to page 6, line 18, page 18, line 8, to page 21, line 18, and Claims 1 and 6; US Patent No. 5,691,372 (issued 25 November 1997) at col. 2, line 43, to col. 3, line 47, col. 9, line 21, to col. 11, line 5, and Claims 1 and 5; and US Patent No. 5,990,155 (issued 23 November 1999) at col. 2, line 46, to col. 3, line 55, coL 9, line 25, to col. 11, line 13, and Claims 1 and 3. 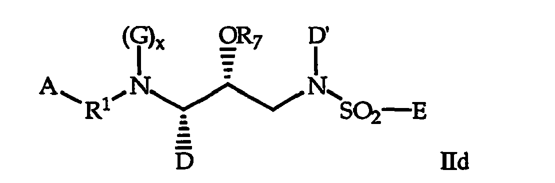 described as "(I)" in: WO 99/33793 (published 8 July 1999) at page 4, line 1, to page 7, line 29, page 17, line 1, to page 20, line 33, and Claim 1. 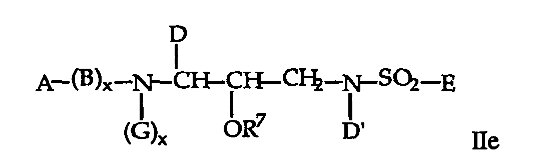 described as "(I)" in: WO 99/33815 (published 8 July 1999) at page 4, line 1, to page 7, line 19, page 12, line 18, to page 16, line 7, and Claim 1; and WO 99/65870 (published 23 December 1999) at page 4, line 7, to page 8, line 4, page 12, line 7, to page 16, line 4, and Claim 1. 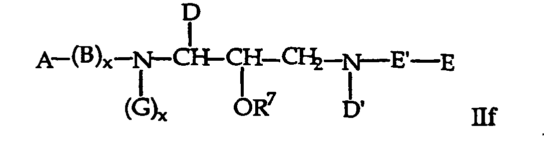 described as "(I)" in: WO 00/47551 (published 17 August 2000) at page 4, line 10, to page 8, line 29, page 13, line 14, to page 17, line 32, and Claim 1. 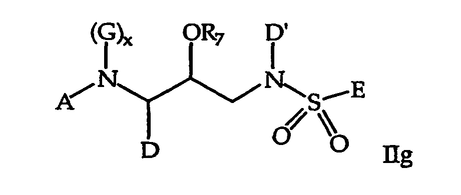 described as "(I)" in: WO 00/76961 (published 21 December 2000) at page 5, line 1, to page 10, line 24, page 14, line 28, to page 20, line 21, and Claim 1. 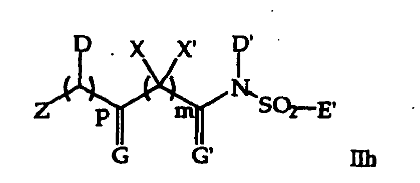 described as "(I)" in: WO 99/33792 (published 8 July 1999) at page 4, line 5, to page 7, line 35, page 17, line 10, to page 21, line 6, and Claim 1; WO 95/24385 (published 14 September 1995) at page 4, line 24, to page 7, line 14, page 16, line 20, to page 19, line 8, and Claims 1 and 29; and US Patent No. 6,127,372 (issued 3 October 2000) at col. 2, line 58, to coL 4, line 28, coL 8, line 66, to coL 10, line 37, and Claim 1.

### Stereoisomers

The compounds of the invention may have chiral centers, e.g. chiral carbon or phosphorus atoms. The compounds of the invention thus include racemic mixtures of all stereoisomers, including enantiomers, diastereomers, and atropisomers. In addition, the compounds of the invention include enriched or resolved optical isomers at any or all asymmetric, chiral atoms. In other words, the chiral centers apparent from the depictions are provided as the chiral isomers or racemic mixtures. Both racemic and diastereomeric mixtures, as well as the individual optical isomers isolated or synthesized, substantially free of their enantiomeric or diastereomeric partners, are all within the scope of the invention. The racemic mixtures are separated into their individual, substantially optically pure isomers through well-known techniques such as, for example, the separation of diastereomeric salts formed with optically active adjuncts, e.g., acids or bases followed by conversion back to the optically active substances. In most instances, the desired optical isomer is synthesized by means of stereospecific reactions, beginning with the appropriate stereoisomer of the desired starting material.

The compounds of the invention can also exist as tautomeric isomers in certain cases. All though only one delocalized resonance structure may be depicted, all such forms are contemplated within the scope of the iinvention. For example, ene-amine tautomers can exist for purine, pyrimidine, imidazole, guanidine, amidine, and tetrazole systems and all their possible tautomeric forms are within the scope of the invention.

### Salts and Hydrates

The compositions of this invention optionally comprise salts of the compounds herein, especially pharmaceutically acceptable non-toxic salts containing, for example, Na⁺, Li⁺, K⁺, Ca⁺² and Mg⁺². Such salts may include those derived by combination of appropriate cations such as alkali and alkaline earth metal ions or ammonium and quaternary amino ions with an acid anion moiety, typically a carboxylic acid. Monovalent salts are preferred if a water soluble salt is desired.

Metal salts typically are prepared by reacting the metal hydroxide with a compound of this invention. Examples of metal salts which are prepared in this way are salts containing Li⁺, Na⁺, and K⁺. A less soluble metal salt may be precipitated from the solution of a more soluble salt by addition of the suitable metal compound.

In addition, salts may be formed from acid addition of certain organic and inorganic acids, e.g., HCl, HBr, H₂SO₄, H₃PO₄ or organic sulfonic acids, to basic centers, typically amines, or to acidic groups. Finally, it is to be understood that the compositions herein comprise compounds of the invention in their un-ionized, as well as zwitterionic form, and combinations with stoichiometric amounts of water as in hydrates.

Also included within the scope of this invention are the salts of the parental compounds with one or more amino acids. Any of the amino acids described above are suitable, especially the naturally-occurring amino acids found as protein components, although the amino acid typically is one bearing a side chain with a basic or acidic group, e.g., lysine, arginine or glutamic acid, or a neutral group such as glycine, serine, threonine, alanine, isoleucine, or leucine.

### Methods of Inhibition of HIV Protease

Another aspect of the invention relates to methods of inhibiting the activity of HIV protease comprising the step of treating a sample suspected of containing HIV with a composition of the invention.

Compositions of the invention may act as inhibitors of HIV protease, as intermediates for such inhibitors or have other utilities as described below. The inhibitors will bind to locations on the surface or in a cavity of HIV protease having a geometry unique to HIV protease. Compositions binding HIV protease may bind with varying degrees of reversibility. Those compounds binding substantially irreversibly are ideal candidates for use in this method of the invention. Once labeled, the substantially irreversibly binding compositions are useful as probes for the detection of HIV protease. Accordingly, the invention relates to methods of detecting HIV protease in a sample suspected of containing HIV protease comprising the steps of: treating a sample suspected of containing HIV protease with a composition comprising a compound of the invention bound to a label; and observing the effect of the sample on the activity of the label Suitable labels are well known in the diagnostics field and include stable free radicals, fluorophores, radioisotopes, enzymes, chemiluminescent groups and chromogens. The compounds herein are labeled in conventional fashion using functional groups such as hydroxyl, carboxyl, sulfhydryl or amino.

Within the context of the invention, samples suspected of containing HIV protease include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing an organism which produces HIV protease, frequently a pathogenic organism such as HIV. Samples can be contained in any medium including water and organic solvent\water mixtures. Samples include living organisms such as humans, and man made materials such as cell cultures.

The treating step of the invention comprises adding the composition of the invention to the sample or it comprises adding a precursor of the composition to the sample. The addition step comprises any method of administration as described above.

If desired, the activity of HIV protease after application of the composition can be observed by any method including direct and indirect methods of detecting HIV protease activity. Quantitative, qualitative, and semiquantitative methods of determining HIV protease activity are all contemplated. Typically one of the screening methods described above are applied, however, any other method such as observation of the physiological properties of a living organism are also applicable.

Organisms that contain HIV protease include the HIV virus. The compounds of this invention are useful in the treatment or prophylaxis of HIV infections in animals or in man.

However, in screening compounds capable of inhibiting human immunodeficiency viruses, it should be kept in mind that the results of enzyme assays may not correlate with cell culture assays. Thus, a cell based assay should be the primary screening tooL

### Screens for HIV protease Inhibitors.

Compositions of the invention are screened for inhibitory activity against HIV protease by any of the conventional techniques for evaluating enzyme activity. Within the context of the invention, typically compositions are first screened for inhibition of HIV protease *in vitro* and compositions showing inhibitory activity are then screened for activity *in vivo.* Compositions having *in vitro* Ki (inhibitory constants) of less then about 5 X 10⁻⁶ M, typically less than about 1 X 10⁻⁷ M and preferably less than about 5 X 10⁻⁸ M are preferred for in *vivo* use.

Useful *in vitro* screens have been described in detail and will not be elaborated here. However, the examples describe suitable *in vitro* assays.

### Pharmaceutical Formulations

The compounds of this invention are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as those set forth in the "Handbook of Pharmaceutical Excipients" (1986). Excipients include ascorbic acid and other antioxidants, chelating agents such as EDTA, carbohydrates such as dextran, hydroxyalkylcellulose, hydroxyalkylmethylcellulose, stearic acid and the like. The pH of the formulations ranges from about 3 to about 11, but is ordinarily about 7 to 10.

While it is possible for the active ingredients to be administered alone it may be preferable to present them as pharmaceutical formulations. The formulations, both for veterinary and for human use, of the invention comprise at least one active ingredient, as above defined, together with one or more acceptable carriers therefor and optionally other therapeutic ingredients. The carrier(s) must be "acceptable" in the sense of being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet is made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom

For infections of the eye or other external tissues e.g. mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc.), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the invention include Tween^{®} 60, Span^{®} 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils are used.

Pharmaceutical formulations according to the present invention comprise a combination according to the invention together with one or more pharmaceutically acceptable carriers or excipients and optionally other therapeutic agents. Pharmaceutical formulations containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10%, and particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for intrapulmonary or nasal administration have a particle size for example in the range of 0.1 to 500 microns, such as 0.5, 1, 30, 35 etc., which is administered by rapid inhalation through the nasal passage or by inhalation through the mouth so as to reach the alveolar sacs. Suitable formulations include aqueous or oily solutions of the active ingredient. Formulations suitable for aerosol or dry powder administration may be prepared according to conventional methods and may be delivered with other therapeutic agents such as compounds heretofore used in the treatment or prophylaxis of HIV infections as described below.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulations of this invention may include other agents conventional in the art having regard to the type of formulation in question, for example those suitable for oral administration may include flavoring agents.

The invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds of the invention are used to provide controlled release pharmaceutical formulations containing as active ingredient one or more compounds of the invention ("controlled release formulations") in which the release of the active ingredient are controlled and regulated to allow less frequency dosing or to improve the pharmacokinetic or toxicity profile of a given active ingredient.

Effective dose of active ingredient depends at least on the nature of the condition being treated, toxicity, whether the compound is being used prophylactically (lower doses) or against an active viral infection, the method of delivery, and the pharmaceutical formulation, and will be determined by the clinician using conventional dose escalation studies. It can be expected to be from about 0.0001 to about 100 mg/kg body weight per day. Typically, from about 0.01 to about 10 mg/kg body weight per day. More typically, from about .01 to about 5 mg/kg body weight per day. More typically, from about .05 to about 0.5 mg/kg body weight per day. For example, the daily candidate dose for an adult human of approximately 70 kg body weight will range from 1 mg to 1000 mg, preferably between 5 mg and 500 mg, and may take the form of single or multiple doses.

### Routes of Administration

One or more compounds of the invention (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the recipient. An advantage of the compounds of this invention is that they are orally bioavailable and can be dosed orally.

### Combination Therapy

Compositions of the invention are also used in combination with other active ingredients. Such combinations are selected based on the condition to be treated, cross-reactivities of ingredients and pharmaco-properties of the combination. For example, when treating viral infections the compositions of the invention may be combined with other antivirals such as other protease inhibitors, nucleoside reverse transcriptase inhibitors, non-nucleoside reverse transcriptase inhibitors or HIV integrase inhibitors.

It is possible to combine any compound of the invention with one or more other active ingredients in a unitary dosage form for simultaneous or sequential administration to an HIV infected patient. The combination therapy may be administered as a simultaneous or sequential regimen. When administered sequentially, the combination may be administered in two or more administrations. Second and third active ingredients in the combination may have anti-HIV activity. Exemplary active ingredients to be administered in combination with compounds of the invention are protease inhibitors, nucleoside reverse transcriptase inhibitors, non-nucleoside reverse transcriptase inhibitors, and HIV integrase inhibitors.

The combination therapy may provide "synergy" and "synergistic", i.e. the effect achieved when the active ingredients used together is greater than the sum of the effects that results from using the compounds separately. A synergistic effect may be attained when the active ingredients are: (1) co-formulated and administered or delivered simultaneously in a combined formulation; (2) delivered by alternation or in parallel as separate formulations; or (3) by some other regimen. When delivered in alternation therapy, a synergistic effect may be attained when the compounds are administered or delivered sequentially, e.g. in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together. A synergistic anti-viral effect denotes an antiviral effect which is greater than the predicted purely additive effects of the individual compounds of the combination.

### Metabolites of the Compounds of the Invention

Also falling within the scope of this invention are the *in vivo* metabolic products of the compounds described herein, to the extent such products are novel and unobvious over the prior art. Such products may result for example from the oxidation, reduction, hydrolysis, amidation, esterification and the like of the administered compound, primarily due to enzymatic processes. Accordingly, the invention includes novel and unobvious compounds produced by a process comprising contacting a compound of this invention with a mammal for a period of time sufficient to yield a metabolic product thereof. Such products typically are identified by preparing a radiolabelled (e.g. ¹⁴C or ³H) compound of the invention, administering it parenterally in a detectable dose (e.g. greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g. by MS or NMR analysis. In general, analysis of metabolites is done in the same way as conventional drug metabolism studies well-known to those skilled in the art. The conversion products, so long as they are not otherwise found *in vivo,* are useful in diagnostic assays for therapeutic dosing of the compounds of the invention even if they possess no HIV protease inhibitory activity of their own.

Recipes and methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37°C. Simply because the compounds are stable to the gastrointestinal tract does not mean that they cannot be hydrolyzed *in vivo*. The phosphonate prodrugs of the invention typically will be stable in the digestive system but may be substantially hydrolyzed to the parental drug in the digestive lumen, liver or other metabolic organ, or within cells in general.

### Exemplary Methods of Making the Compounds of the Invention.

The invention provides many methods of making the compositions of the invention. The compositions are prepared by any of the applicable techniques of organic synthesis. Many such techniques are well known in the art, such as those elaborated in "Compendium of Organic Synthetic Methods" (John Wiley, & Sons New York), Vol. 1, Ian T. Harrison and Shuyen Harrison, 1971; Vol. 2, Ian T. Harrison and Shuyen Harrison, 1974; Vol. 3, Louis S. Hegedus and Leroy Wade, 1977; Vol. 4, Leroy G. Wade, jr., 1980; Vol. 5, Leroy G. Wade, Jr., 1984; and VoL 6, Michael B. Smith; as well as March, J., "Advanced Organic Chemistry, Third Edition", (John Wiley & Sons, New York, 1985), "Comprehensive Organic Synthesis. Selectivity, Strategy & Efficiency in Modem Organic Chemistry. In 9 Volumes", Barry M. Trost, Editor-in-Chief (Pergamon Press, New York, 1993 printing).

Dialkyl phosphonates may be prepared according to the methods of: Quast et al (1974) Synthesis 490; Stowell et al (1990) Tetrahedron Lett. 3261; US Patent No. 5,663,159.

In general, synthesis of phosphonate esters is achieved by coupling a nucleophile amine or alcohol with the corresponding activated phosphonate electrophilic precursor. For example, chlorophosphonate addition on to 5'-hydroxy of nucleoside is a well known method for preparation of nucleoside phosphate monoesters. The activated precursor can be prepared by several well known methods. Chlorophosphonates useful for synthesis of the prodrugs are prepared from the substituted-1,3-propanediol (Wissner, et al, (1992) J. Med Chem. 35:1650)*.* Chlorophosphonates are made by oxidation of the corresponding chlorophospholanes (Anderson, et al, (1984) J. Org. Chem. 49:1304) which are obtained by reaction of the substituted diol with phosphorus trichloride. Alternatively, the chlorophosphonate agent is made by treating substituted-1,3-diols with phosphorusoxychloride (Patois, et al, (1990) J. Chem. Soc. Perkin Trans. I, 1577). Chlorophosphonate species may also be generated in situ from corresponding cyclic phosphites (Silverburg, et al., (1996) Tetrahedron Lett., 37:771-774), which in turn can be either made from chlorophospholane or phosphoramidate intermediate. Phosphoroflouridate intermediate prepared either from pyrophosphate or phosphoric acid may also act as precursor in preparation of cyclic prodrugs (Watanabe et al., (1988) Tetrahedron Lett., 29:5763-66). Caution: fluorophosphonate compounds may be highly toxic!

Phosphonate prodrugs of the present invention may also be prepared from the precursor free acid by Mitsunobu reactions (Mitsunobu, (1981) Synthesis, 1; Campbell, (1992) J. Org. Chem., 52:6331), and other acid coupling reagents including, but not limited to, carbodiimides (Alexander, et al, (1994) Collect. Czech. Chem. Commun. 59:1853; Casara, et al, (1992) Bioorg. Med. Chem. Lett., 2:145; Ohashi, et al, (1988) Tetrahedron Lett., 29:1189), and benzotriazolyloxytris-(dimethylamino)phosphonium salts (Campagne, et al, (1993) Tetrahedron Lett., 34:6743).

Aryl halides undergo Ni⁺² catalyzed reaction with phosphite derivatives to give aryl phosphonate containing compounds (Balthazar, et al (1980) J. Org. Chem. 45:5425). Phosphonates may also be prepared from the chlorophosphonate in the presence of a palladium catalyst using aromatic triflates (Petrakis, et al, (1987) J. Am. Chem. Soc. 109:2831; Lu, et al, (1987) Synthesis, 726). In another method, aryl phosphonate esters are prepared from aryl phosphates under anionic rearrangement conditions (Melvin (1981) Tetrahedron Lett. 22:3375; Casteel, et al, (1991) Synthesis, 691). N-Alkoxy aryl salts with alkali metal derivatives of cyclic alkyl phosphonate provide general synthesis for heteroaryl-2-phosphonate linkers (Redmore (1970) J. Org. Chem. 35:4114). These above mentioned methods can also be extended to compounds where the W⁵ group is a heterocycle. Cyclic-1,3-propanyl prodrugs of phosphonates are also synthesized from phosphonic diacids and substituted propane-1,3-diols using a coupling reagent such as 1,3-dicyclohexylcarbodiimide (DCC) in presence of a base (e.g., pyridine). Other carbodiimide based coupling agents like 1,3-disopropylcarbodiimide or water soluble reagent, 1-(3-dimethylaminopropyl)-3-ethylcarbodiimide hydrochloride (EDCI) can also be utilized for the synthesis of cyclic phosphonate prodrugs.

The carbamoyl group may be formed by reaction of a hydroxy group according to the methods known in the art, including the teachings of Ellis, US 2002/0103378 A1 and Hajima, US Patent No. 6,018,049.

### Schemes and Examples

A number of exemplary methods for the preparation of the compositions of the invention are provided below. These methods are intended to illustrate the nature of such preparations are not intended to limit the scope of applicable methods.

General aspects of these exemplary methods are described below and in the Examples. Each of the products of the following processes is optionally separated, isolated, and/or purified prior to its use in subsequent processes.

Generally, the reaction conditions such as temperature, reaction time, solvents, work up procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20°C), although for metal hydride reductions frequently the temperature is reduced to 0°C to -100°C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0°C to -100°C) are also common. Solvents can be either protic (common in equilibrating reactions) or aprotic (common in kinetically controlled reactions).

Standard synthetic techniques such as azeotropic removal of reaction by-products and use of anhydrous reaction conditions (e.g. inert gas environments) are common in the art and will be applied when applicable.

The terms "treated", "treating", "treatment", and the like, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two.

"Treating" indicates the reasonable and usual manner in which organic chemicals are allowed to react. Normal concentrations (0.01M to 10M, typically 0.1M to 1M), temperatures (-100°C to 250°C, typically -78°C to 150°C, more typically -78°C to 100°C, still more typically 0°C to 100°C), reaction vessels (typically glass, plastic, metal), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic synthesis selects conditions and apparatus reasonably expected to successfully carry out the chemical reactions of the described processes based on the knowledge in the art.

Modifications of each of the exemplary schemes above and in the examples (hereafter "exemplary schemes") leads to various analogs of the specific exemplary materials produce. The above cited citations describing suitable methods of organic synthesis are applicable to such modifications.

In each of the exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

A single stereoisomer, e.g. an enantiomer, substantially free of its stereoisomer may be obtained by resolution of the racemic mixture using a method such as formation of diastereomers using optically active resolving agents ("Stereochemistry of Carbon Compounds," (1962) by E. L. Eliel, McGraw Hill; Lochmuller, C. H., (1975) J. Chromatogr., 113:(3) 283-302). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions.

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (Eliel, E. and Wilen, S. (1994) Stereochemistry of Organic Compounds, John Wiley & Sons, Inc., p. 322). Diastereomeric compounds can be formed by reacting asymmetric compounds with enantiomerically pure chiral derivatizing reagents, such as menthyl derivatives, followed by separation of the diastereomers and hydrolysis to yield the free, enantiomerically enriched xanthene.. A method of determining optical purity involves making chiral esters, such as a menthyl ester, e.g. (-) menthyl chloroformate in the presence of base, or Mosher ester, α-methoxy-α-(trifluoromethyl)phenyl acetate (Jacob III. (1982) J. Org. Chem. 47:4165), of the racemic mixture, and analyzing the NMR spectrum for the presence of the two atropisomeric diastereomers. Stable diastereomers of atropisomeric compounds can be separated and isolated by normal- and reverse-phase chromatography following methods for separation of atropisomeric naphthyl-isoquinolines (Hoye, T., WO 96/15111). By method (3), a racemic mixture of two enantiomers can be separated by chromatography using a chiral stationary phase (Chiral Liquid Chromatography (1989) W. J. Lough, Ed. Chapman and Hall, New York; Okamoto, (1990) J. of Chromatogr. 513:375-378). Enriched or purified enantiomers can be distinguished by methods used to distinguish other chiral molecules with asymmetric carbon atoms, such as optical rotation and circular dichroism.

All literature and patent citations above are hereby expressly incorporated by reference at the locations of their citation. Specifically cited sections or pages of the above cited works are incorporated by reference with specificity. The invention has been described in detail sufficient to allow one of ordinary skill in the art to make and use the subject matter of the following Embodiments. It is apparent that certain modifications of the methods and compositions of the following Embodiments can be made within the scope and spirit of the invention.

### Examples General Section

The following Examples refer to the Schemes.

Some Examples have been performed multiple times. In repeated Examples, reaction conditions such as time, temperature, concentration and the like, and yields were within normal experimental ranges. In repeated Examples where significant modifications were made, these have been noted where the results varied significantly from those described. In Examples where different starting materials were used, these are noted. When the repeated Examples refer to a "corresponding" analog of a compound, such as a "corresponding ethyl ester", this intends that an otherwise present group, in this case typically a methyl ester, is taken to be the same group modified as indicated.

In a number of the following schemes, the term "etc" appears as a substituent on chemical structures and as a term within the schemes. When used in the charts, the term is defined for each chart. When the term "etc" appears in a scheme and is not a substituent on a chemical structure, it means "and the like".

### Amprenavir-like phosphonate protease inhibitors (AMLPPI)

### Preparation of the intermediate phosphonate esters 1-13.

The structures of the intermediate phosphonate esters **1** to **13** and the structures of the component groups R¹, R⁵, X of this invention are shown in Charts **1-2.** The structures of the R²NH₂ components are shown in Chart **3;** the structures of the R³-Cl components are shown in Chart **4**; the structures of the R₄COOH groups are shown in Chart **5a-c;** and the structures of the R⁹CH₂NH₂ amine components are illustrated in Chart **6.**
Specific stereoisomers of some of the structures are shown in Charts **1- 6;** however, all stereoisomers are utilized in the syntheses of the compounds 1 to 13. Subsequent chemical modifications to the compounds **1** to **10,** as described herein, permit the synthesis of the final compounds of this invention.
The intermediate compounds **1** to **10** incorporate a phosphonate moiety (R¹O)₂P(O) connected to the nucleus by means of a variable linking group, designated as "link" in the attached structures. Charts **7,** and **8** illustrate examples of the linking groups present in the structures **1- 10.**
Schemes **1- 99** illustrate the syntheses of the intermediate phosphonate compounds of this invention, **1 - 10,** and of the intermediate compounds necessary for their synthesis. The preparation of the phosphonate esters **11, 12** and **13,** in which a phosphonate moiety is incorporated into one of the groups R⁴, R³, R², respectively, is also described below.

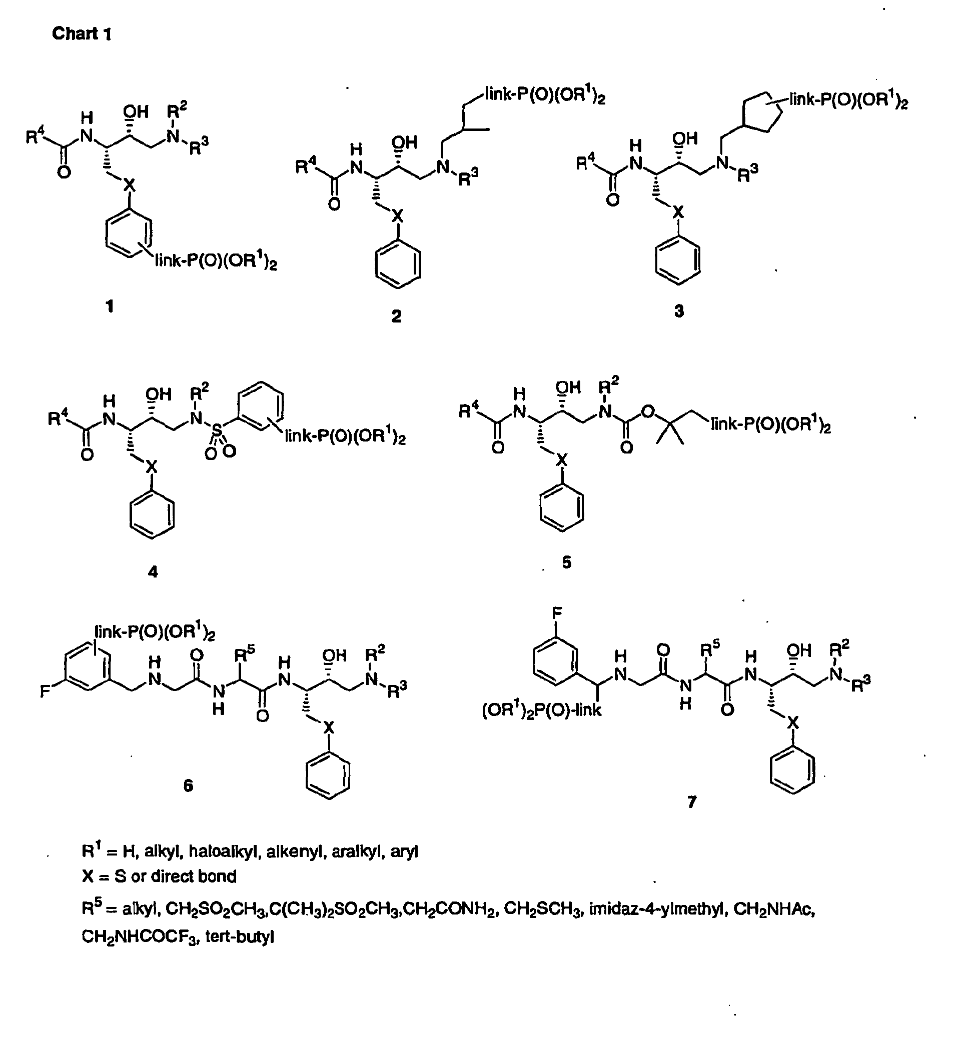

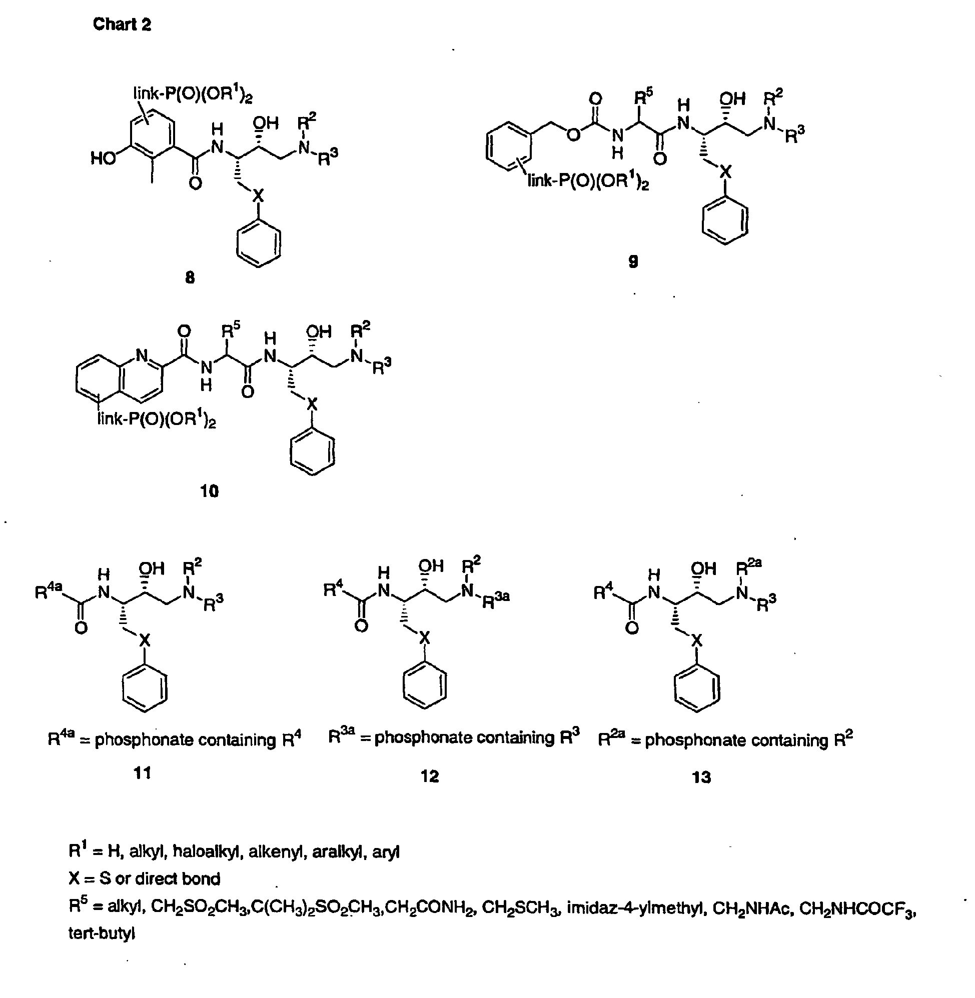

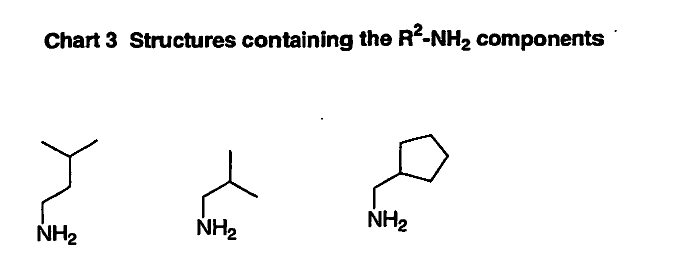

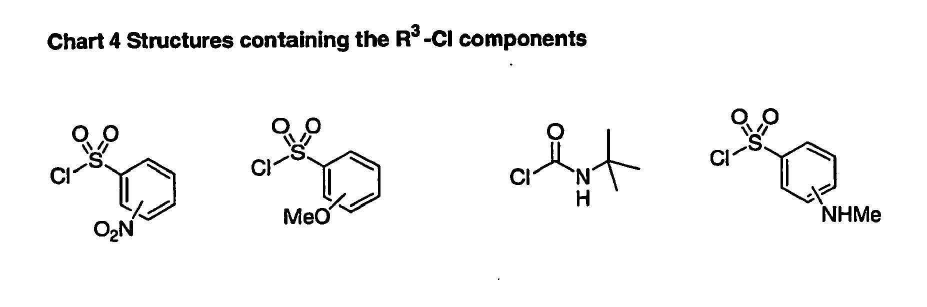

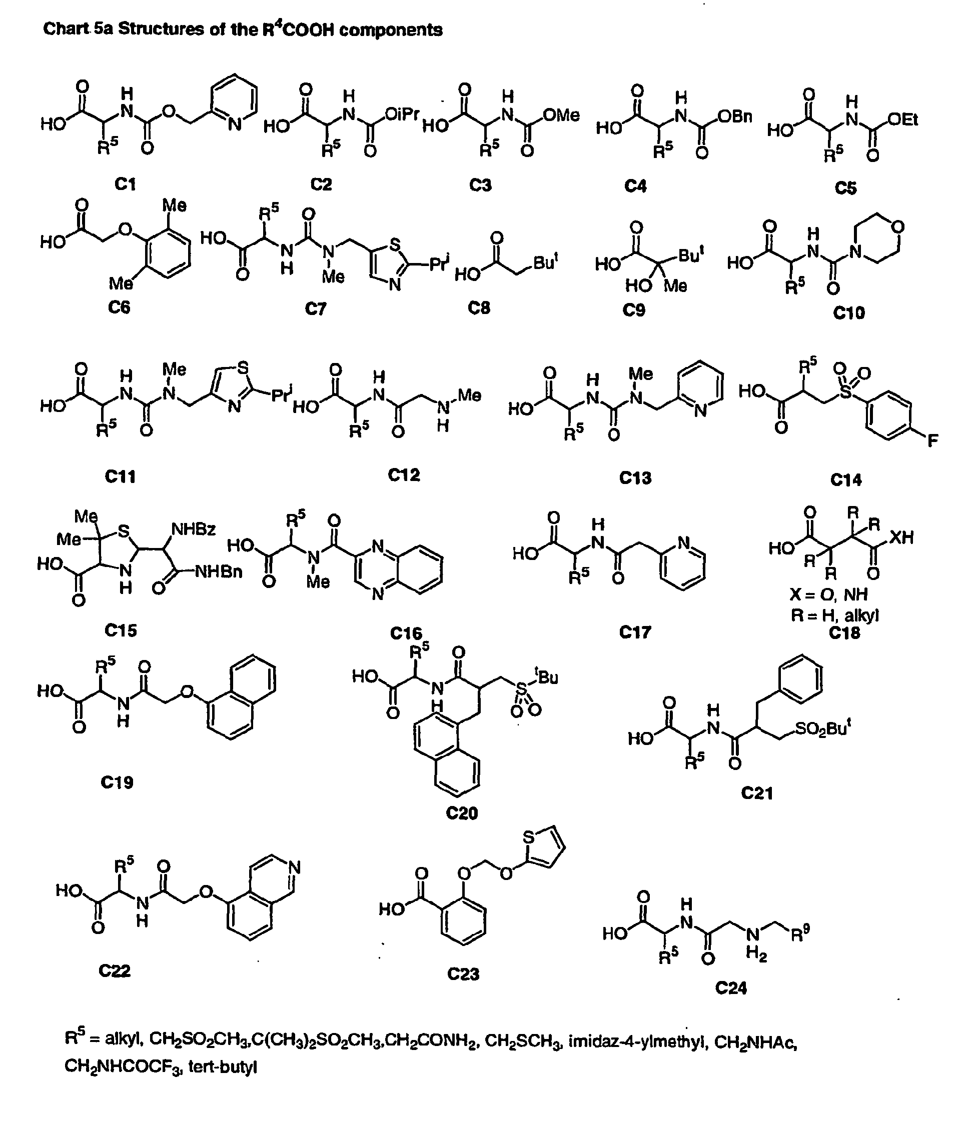

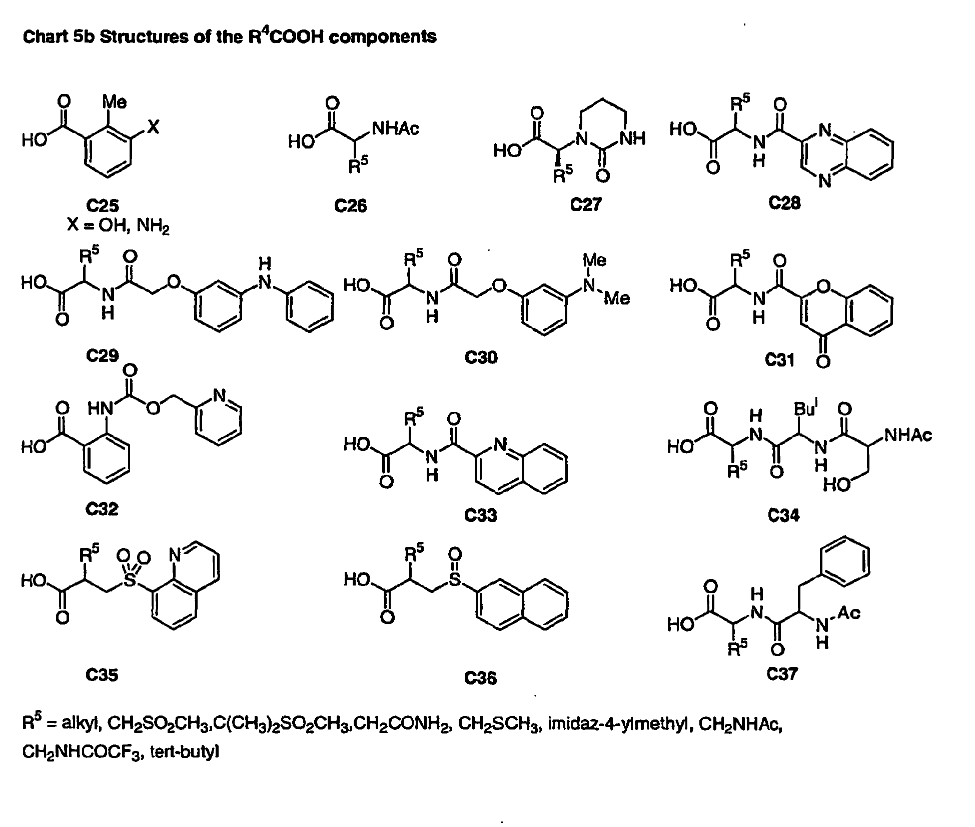

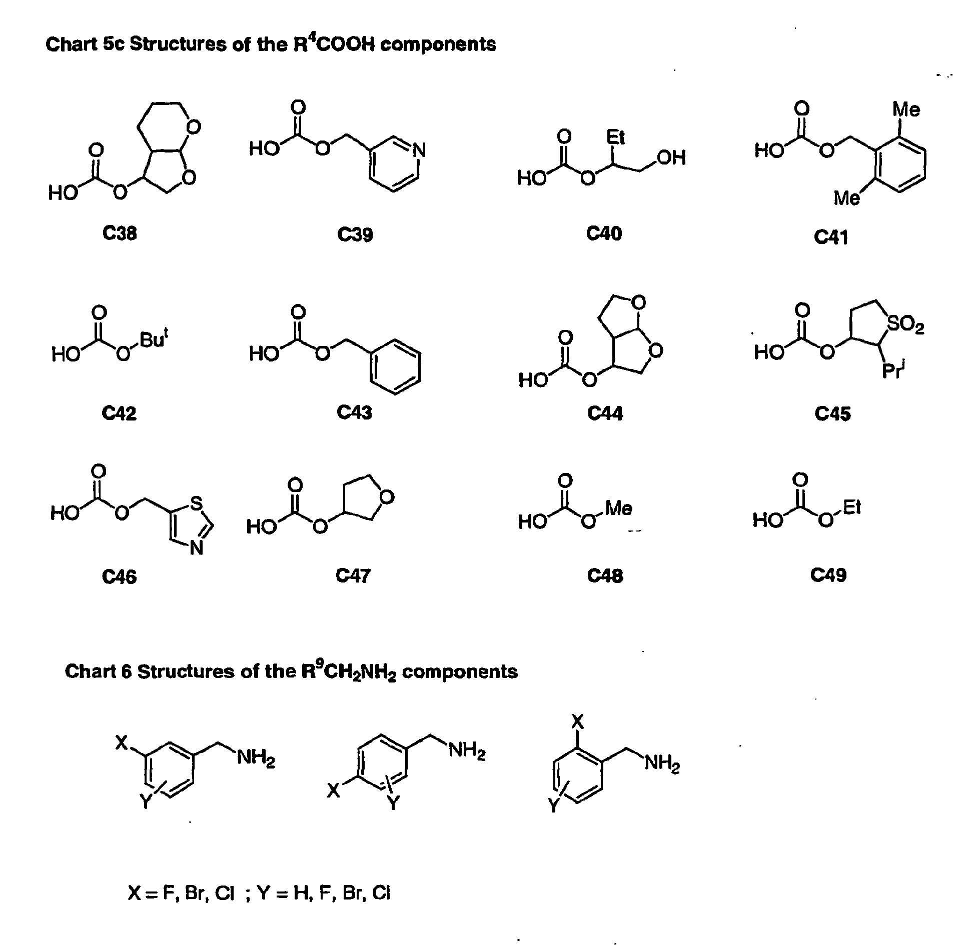

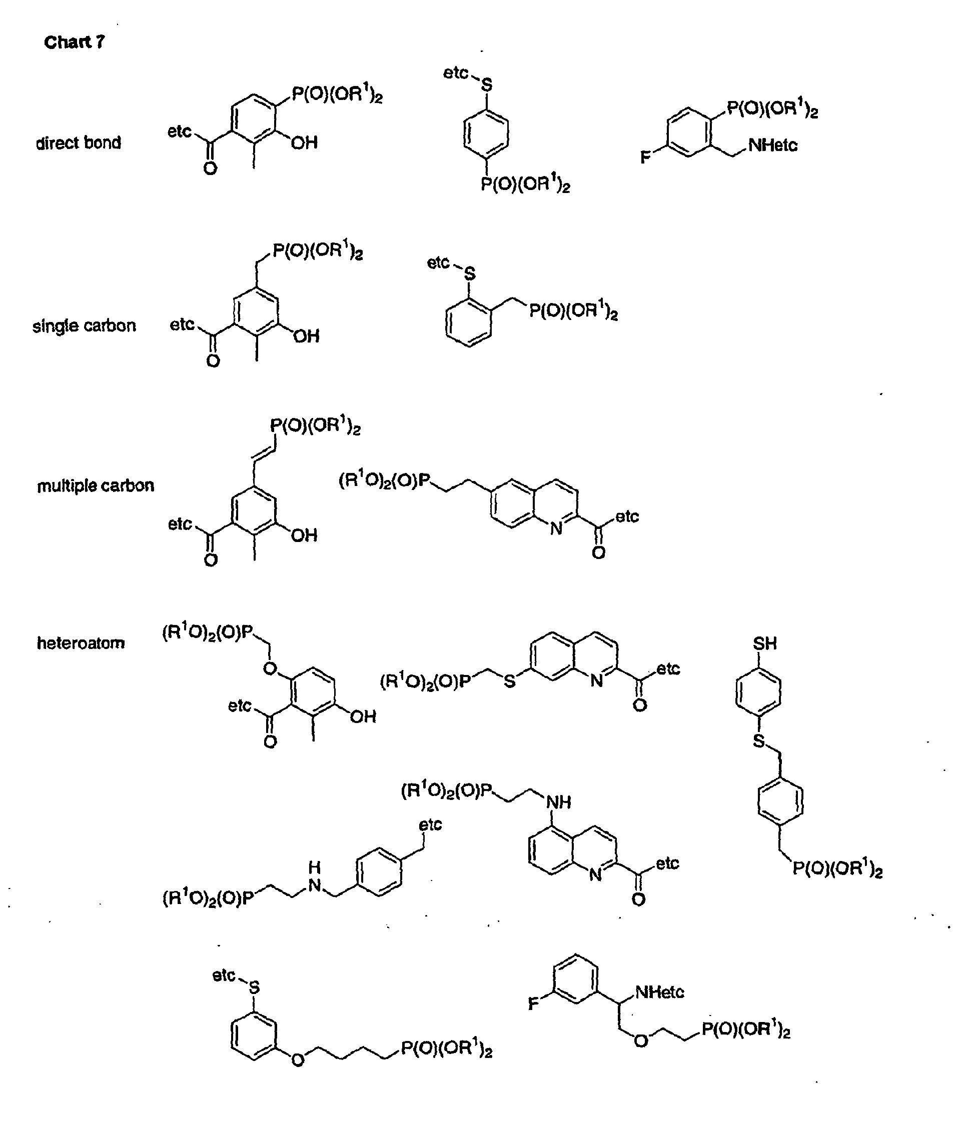

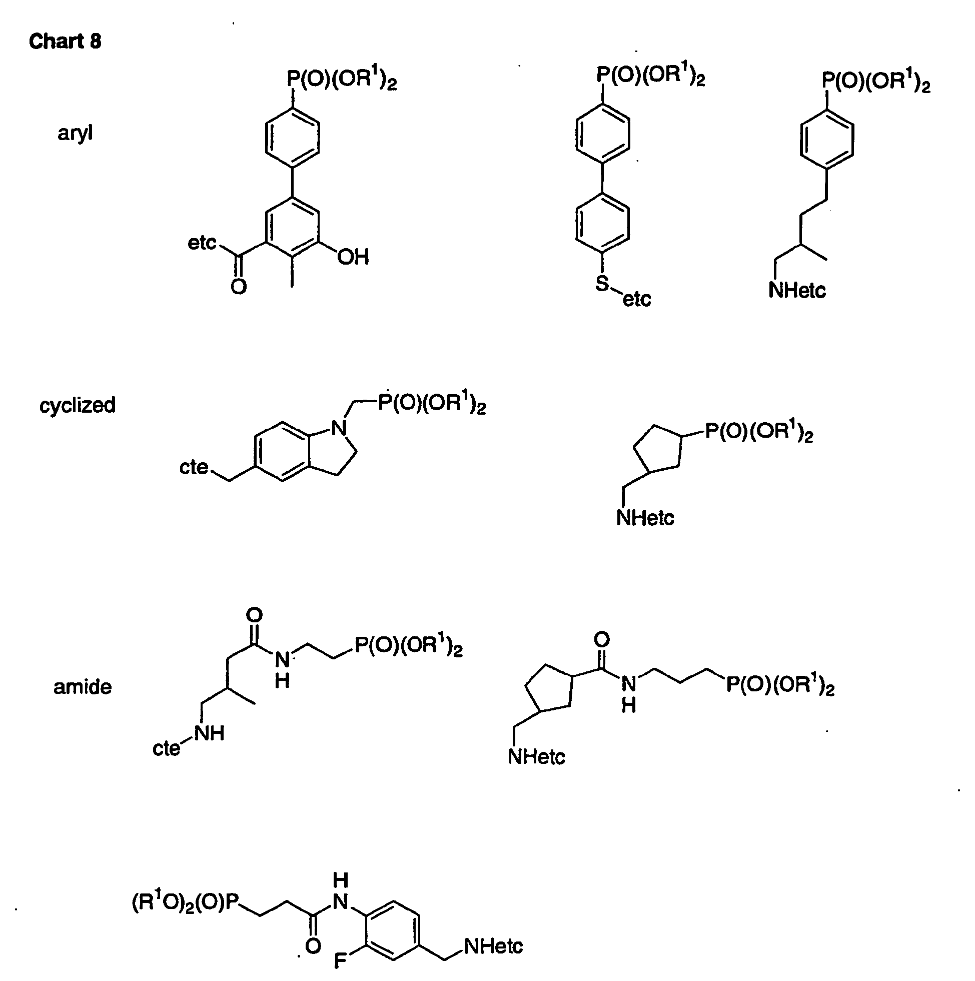

### Protection of reactive substituents.

Depending on the reaction conditions employed, it may be necessary to protect certain reactive substituents from unwanted reactions by protection before the sequence described, and to deprotect the substituents afterwards, according to the knowledge of one skilled in the art. Protection and deprotection of functional groups are described, for example, in Protective Groups in Organic Synthesis, by T.W. Greene and P.G.M Wuts, Wiley, Second Edition 1990 or Third Edition 1999. Reactive substituents which may be protected are shown in the accompanying schemes as, for example, [OH], [SH], etc.

### Preparation of the phosphonate ester intermediates 1 in which X is a direct bond.

The intermediate phosphonate esters **1,** in which the group A is attached to the aryl moiety, the R₄COOH group does not contain an secondary amine, and in which the substituent A is either the group link-P(O)(OR¹)₂ or a precursor such as [OH], [SH], [NH], Br etc are prepared as shown in Schemes **1-2.** The epoxide **1.1** in which the substituent A is either the group link-P(O)(OR¹)₂ or a precursor such as [OH], [SH], [NH], Br is prepared as described in Schemes **56-59** below. Treatment of the epoxide **1.1** with the amine **1.2** affords the aminoalcohol **1.3.** The preparation of aminoalcohols by reaction between an amine and an epoxide is described, for example, in Advanced Organic Chemistry, by J. March, McGraw Hill, 1968, p 334. In atypical procedure, equimolar amounts of the reactants are combined in a polar solvent such as an alcohol or dimethylformamide and the like, at from ambient to about 100°, for from 1 to 24 hours, to afford the product **1.3.** The amino alcohol **1.3** is then treated with an acylating agent **1.4** to afford the product **1.5.** The acylating agent is typically a chloroformate or a sulfonyl chloride as shown in chart **4.** Coupling conditions for amines with sulfonyl chlorides is described in Protective Groups in Organic Synthesis, by T.W. Greene and P.G.M Wuts, Wiley, Third Edition 1999 p. 603-615 or for chloroformates, p494ff. Preferably, the amine **1.3** is treated with the sulfonyl chloride **1.4** in the presence of a base such as pyridine, potassium carbonate etc and THF / water to give the product **1.5.** Product **1.5** is deprotected using conditions described in Protective Groups in Organic Synthesis, by T.W. Greene and P.G.M Wuts, Wiley, Third Edition 1999 p. 503ff. Preferably, the BOC amine is treated with TFA in an aprotic solvent such as THF. Conversion to the amide **1.8** is performed using standard coupling conditions between an acid **1.7** and the amine. The preparation of amides from carboxylic acids and derivatives is described, for example, in Organic Functional Group Preparations, by S.R.Sandler and W. Karo, Academic Press, 1968, p. 274. The carboxylic acid is reacted with the amine in the presence of an activating agent, such as, for example, dicyclohexylcarbodiimide or diisopropylcarbodiimide, optionally in the presence of, for example, hydroxybenztriazole, in a non-protic solvent such as, for example, pyridine, DMF or dichloromethane, to afford the amide.
Alternatively, the carboxylic acid may first be converted into an activated derivative such as the acid chloride or anhydride, and then reacted with the amine, in the presence of an organic base such as, for example, pyridine, to afford the amide.

The conversion of a carboxylic acid into the corresponding acid chloride is effected by treatment of the carboxylic acid with a reagent such as, for example, thionyl chloride or oxalyl chloride in an inert organic solvent such as dichloromethane.
Preferably, the carboxylic acid **1.7** is reacted with an equimolar amount of the amine **1.6** in the presence of dicyclohexylcarbodiimide and hydroxybenztriazole, in an aprotic solvent such as, for example, tetrahydrofuran, at about ambient temperature, so as to afford the amide product **1.8.** The compound **1.8,** and analogous acylation products described below, in which the carboxylic acid R⁴COOH is one of the carbonic acid derivatives **C38-C49**, as defined in Chart **5c,** are carbamates. Methods for the preparation of carbamates are described below, Scheme **98.**

Scheme **2** illustrates an alternative method for the preparation of intermediate phosphonate esters **1,** in which the group A is attached to the aryl moiety, the R₄COOH group does not contain an secondary amine, and in which the substituent A is either the group link-P(O)(OR¹)₂ or a precursor such as [OH], [SH], [NH], Br etc. The oxazolidinone **2.1,** prepared as described in Schemes **60-62,** is first activated as shown in **2.2** and then treated with amine **1.2** to afford the secondary amine **2.3.** The hydroxyl group can be activated by converting into a bromo derivative, for example by reaction with triphenylphosphine and carbon tetrabromide, as described in J. Am. Chem Soc., 92, 2139, 1970, or a methanesulfonyloxy derivative, by reaction with methanesulfonyl chloride and a base, or, preferably, into the 4-nitrobenzenesulfonyloxy derivative **2.2,** by reaction in a solvent such as ethyl acetate or tetrahydrofuran, with 4-nitrobenzenesulfonyl chloride and a base such as triethylamine or N-methylmorpholine, as described in WO 9607642. The nosylate product **2.2** is then reacted with the amine component **1.2** to afford the displacement product **2.3.** Equimolar amounts of the reactants are combined in an inert solvent such as dimethylformamide, acetonitrile or acetone, optionally in the presence of an organic or inorganic base such as triethylamine or sodium carbonate, at from about 0°C to 100°C to afford the amine product **2.3**. Preferably, the reaction is performed in methyl isobutyl ketone at 80°C, in the presence of sodium carbonate, as described in WO 9607642. Treatment of the amine product **2.3** with the R3 chloride **1.4** as described in Scheme **1** then affords the product **2.4.** The oxazolidinone group present in the product **2.4** is then hydrolyzed to afford the hydroxyamine **2.5.** The hydrolysis reaction is effected in the presence of aqueous solution of a base such as an alkali metal hydroxide, optionally in the presence of an organic co-solvent. Preferably, the oxazolidinone compound **2.4** is reacted with aqueous ethanolic sodium hydroxide at reflux temperature, as described in WO 9607642, to afford the amine **2.5.** This product is then reacted with the R⁴COOH carboxylic acid or activated derivative thereof, **1.7,** to afford the product **1.8.** The amide-forming reaction is conducted under the same conditions as described above, (Scheme **1**).

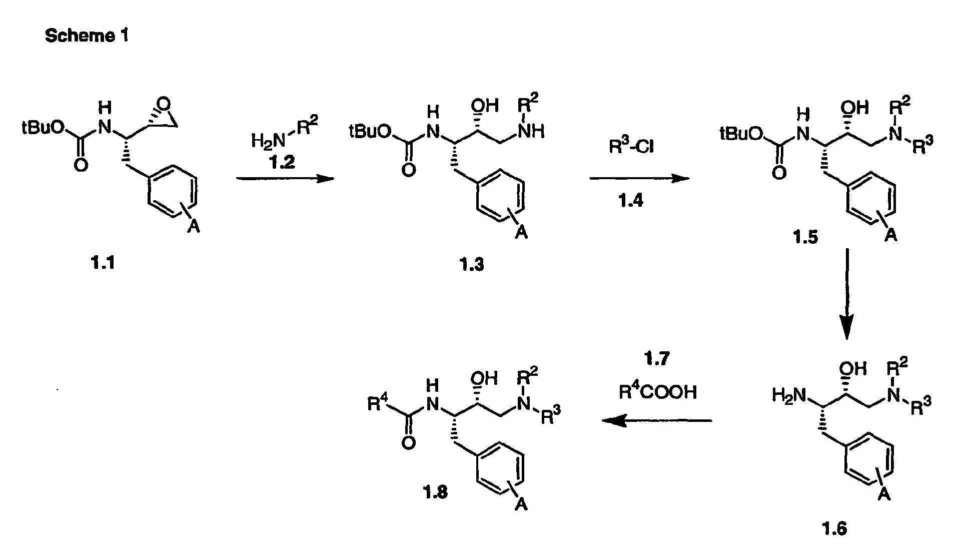

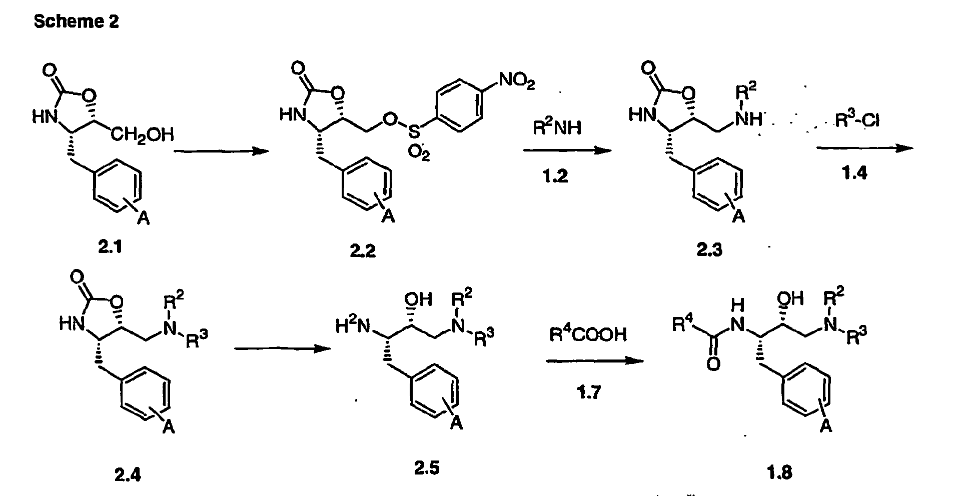

Scheme **3** illustrates the preparation of intermediate phosphonate esters **1,** in which the group A is attached to the aryl moiety, the R₄COOH group contains an secondary amine, and in which the substituent A is either the group link-P(O)(OR¹)₂ or a precursor such as [OH], [SH], [NH], Br etc. The dibenzyl amine **3.2** is prepared from epoxide **3.1** and amine **1.2,** following the same procedures described in Scheme **1** for the preparation of **1.3.** Epoxide **3.1** is prepared as described below in Schemes **56a.** The amine **3.2** is then converted to the amine **3.4** as described inUS6391919. Preferably, the amine is first protected as the BOC carbamate and then treated with palladium hydroxide on carbon (20%) in methanol under hydrogen at high pressure to give the amine **3.4.** Treatment of **3.4** with the R₄COOH acid **1.7** which contains a secondary or primary amine, under standard amide bond forming conditions as described above, Scheme **1,** then affords the amide **3.5.** Preferably, the acid **1.7,** EDC and n-hydroxybenzotriazole in DMF is treated with the amine **3.4** to give the amide **3.5.** Removal of the BOC group as described in Protective Groups in Organic Synthesis, by T.W. Greene and P.G.M Wuts, Wiley, Third Edition 1999 p. 520-525 then affords the amine **3.6.** Preferably the BOC amine **3.5** is treated with HCl in dioxane and water to give the free amine **3.6.** The amine **3.6** is then treated with an acylating agent such as an acid, chloroformate or sulfonyl chloride to give the final product **1.8.** Standard coupling conditions for amines with acids or sulfonyl chlorides is indicated above Scheme **1.** Preferably, the amine **3.6** is treated with nitro-sulfonyl chloride in THF and water in the presence of a base such as potassium carbonate to give the sulfonamide **1.8.**

The reactions shown in Scheme **1-3** illustrate the preparation of the compound **1.8** in which the substituent A is either the group link-P(O)(OR¹)₂ or a precursor such as [OH], [SH], [NH], Br etc. Scheme **4** depicts the conversion of **1.8** in which A is [OH], [SH], [NH], Br etc, into the phosphonate ester **1** in which X is a direct bond. In this procedure **1.8** is converted, using the procedures described below, Schemes **47-99,** into the compound **1.** Also, in the preceding and following Schemes, the amino substituted sulfonamide reagents are typically introduced as a nitro-sulfonamide reagents. Therefore, where appropriate, an additonal step of nitro group reduction as described in Comprehensive Organic Transformations, by R. C. Larock, 2nd Edition, 1999, p.821ff, is performed to give the final amino products.

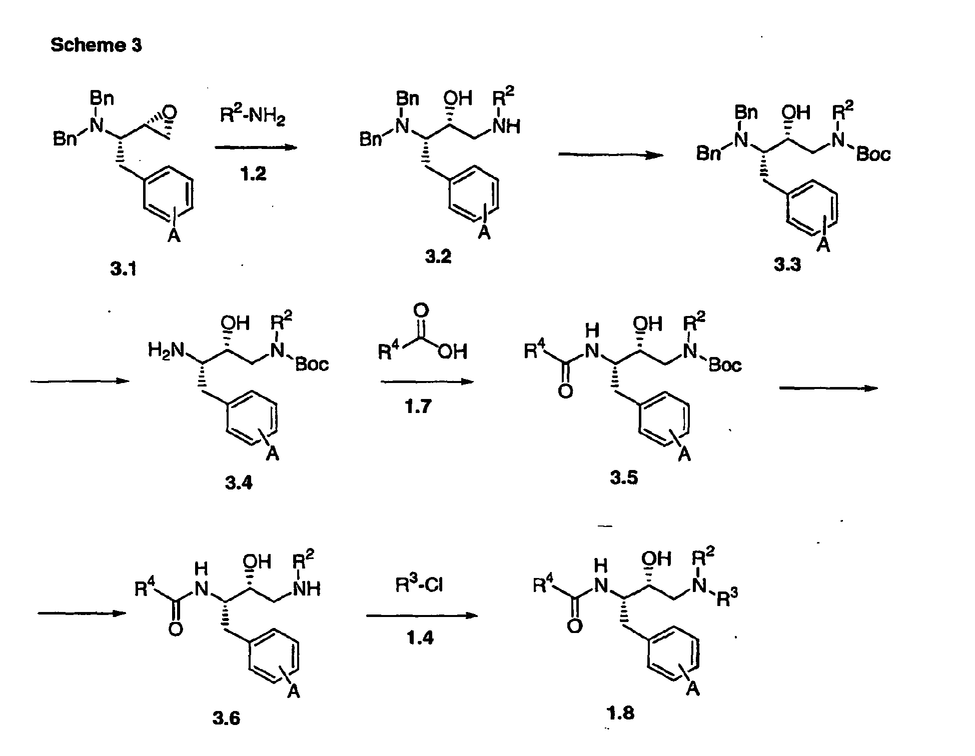

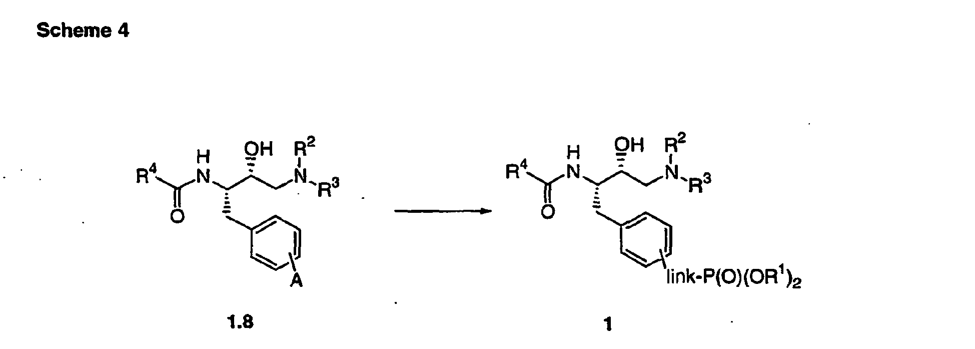

Scheme **5** illustrates an alternative method for the preparation of the compound **1** in which the group A is attached to the aryl moiety, the R₄COOH group contains a primary or secondary amine and in which the substituent A is either the group link-P(O)(OR¹)₂ or a precursor such as [OH], [SH], [NH], Br etc. The amine **3.4,** (Scheme 3) is treated with an amino acid **5.1** under typical amide bond forming conditions to give the amide **5.2** as described above, Scheme **1.** Preferably the acid **5.1** is first treated with EDC and n-hydroxybenzotriazole in DMF and then the amine **3.4** is added in DMF followed by N-methyl morpholine to give the amide **5.2.** Reduction of the amide under the same catalytic hydrogenation conditions as described above in Scheme **3** gives the free amine **5.3.** The amine is further treated with chloroacetyl chloride to provide the chloro compound **5.4.** Preferably treatment with the chloroacetyl chloride is performed in ethyl acetate and water mixture in the presence of a base such as potassium hydrogen carbonate. The chloro compound **5.4** is treated with hydrochloric acid in dioxane and ethyl acetate to give the salt of the free amine **5.5**. The salt **5.5** is then treated with a nitro-sulfonyl chloride **1.4** in THF and water in the presence of a base such as potassium carbonate to give the sulfonamide **5.6.** Alternatively the free amine **5.5** is treated with a chloroformate **1.4** in the presence of a base such as triethylamine to afford the carbamate. Methods for the preparation of carbamates are also described below, Scheme **98.** Compound **5.6** is then treated with the amine **5.7** to give the secondary amine 5.8. Preferably the chloride is refluxed in the presence of the amine **5.7** in THF.

The reactions shown in Scheme **5** illustrate the preparation of the compound **5.8** in which the substituent A is either the group link-P(O)(OR¹)₂ or a precursor such as [OH], [SH], [NH], Br etc. Scheme **6** depicts the conversion of **5.8** in which A is [OH], [SH], [NH], Br etc, into the phosphonate ester **1** in which X is a direct bond. In this procedure **5.8** is converted, using the procedures described below, Schemes **47-99,** into the compound **1.**

In the preceding and following schemes, the conversion of various substituents into the group link-P(O)(OR¹)₂ can be effected at any convenient stage of the synthetic sequence, or in the final step. The selection of an appropriate step for the introduction of the phosphonate substituent is made after consideration of the chemical procedures required, and the stability of the substrates to those procedures. It may be necessary to protect reactive groups, for example hydroxyl, during the introduction of the group link-P(O)(OR¹)₂.

In the preceding and succeeding examples, the nature of the phosphonate ester group can be varied, either before or after incorporation into the scaffold, by means of chemical transformations. The transformations, and the methods by which they are accomplished, are described below (Scheme **99**).

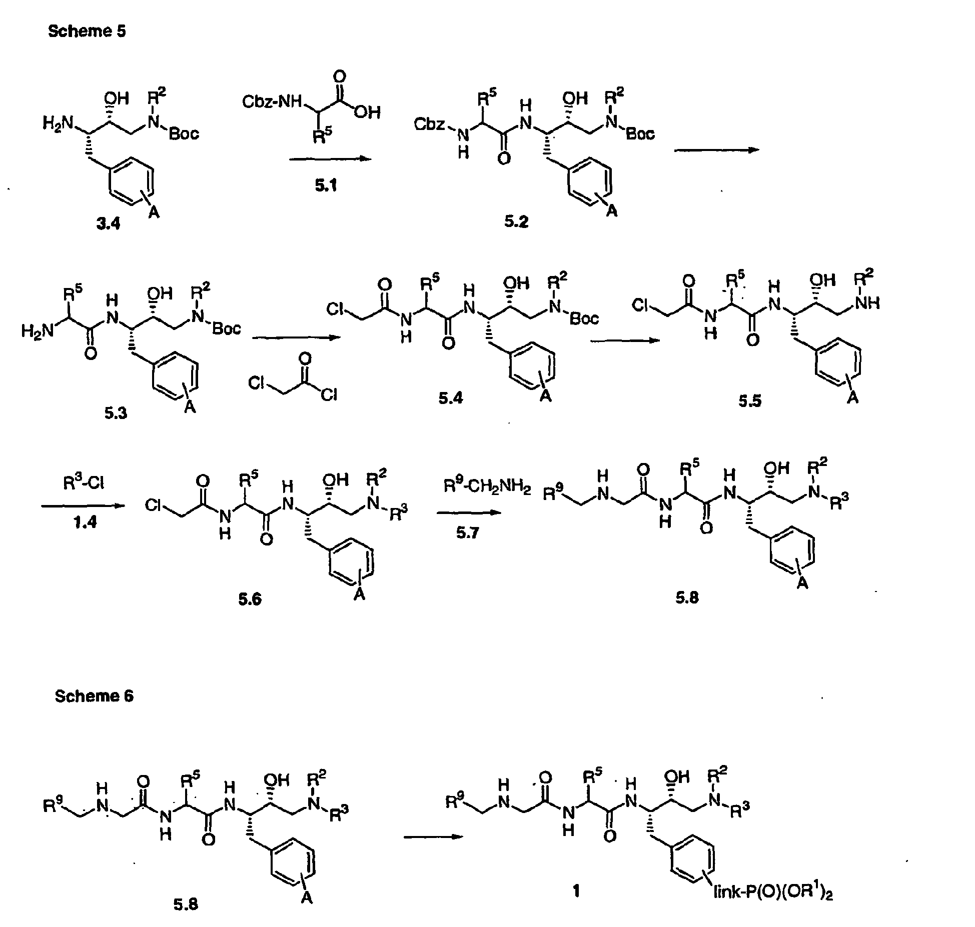

### Preparation of the phosphonate ester intermediates 1 in which X is a sulfur.

The intermediate phosphonate esters **1**, in which X is sulfur, the R₄COOH group does not contain a amine group, and in which substituent A is either the group link-P(O)(OR¹)₂ or a precursor such as [OH], [SH], [NH], Br etc, are prepared as shown in Schemes **7-9.**

Scheme **7** illustrates one method for the preparation of the compounds **1** in which the substituent X is S, and in which the group A is either the group link-P(O)(OR¹)₂ or a precursor thereto, such as [OH], [SH] Br etc. In this sequence, methanesulfonic acid 2-benzoyloxycarbonylamino-2-(2,2-dimethyl-[1,3]dioxolan-4-yl)-ethyl ester, **7.1,** prepared as described in J. Org. Chem, 2000, 65, 1623, is reacted with a thiol **7.2** to afford the thioether **7.3.** The preparation of thiol **7.2** is described in Schemes **63-72.** The reaction is conducted in a suitable solvent such as, for example, pyridine, DMF and the like, in the presence of an inorganic or organic base, at from 0°C to 80°C, for from 1-12 hours, to afford the thioether **7.3.** Preferably the mesylate **7.1** is reacted with an equimolar amount of the thiol, in a mixture of a water-immiscible organic solvent such as toluene, and water, in the presence of a phase-transfer catalyst such as, for example, tetrabutyl ammonium bromide, and an inorganic base such as sodium hydroxide, at about 50°C, to give the product **7.3.** The 1,3-dioxolane protecting group present in the compound **7.3** is then removed by acid catalyzed hydrolysis or by exchange with a reactive carbonyl compound to afford the diol **7.4.** Methods for conversion of 1,3-dioxolanes to the corresponding diols are described in Protective Groups in Organic Synthesis, by T.W. Greene and P.G.M Wuts, Second Edition 1990, p191. For example, the 1,3-dioxolane compound **7.3** is hydrolyzed by reaction with a catalytic amount of an acid in an aqueous organic solvent mixture. Preferably, the 1,3-dioxolane **7.3** is dissolved in aqueous methanol containing hydrochloric acid, and heated at ca. 50°C, to yield the product **7.4.**
The primary hydroxyl group of the diol **7.4** is then selectively acylated by reaction with an electron-withdrawing acyl halide such as, for example, pentafluorobenzoyl chloride or mono-or di-nitrobenzoyl chlorides. The reaction is conducted in an inert solvent such as dichloromethane and the like, in the presence of an inorganic or organic base.
Preferably, equimolar amounts of the diol **7.4** and 4-nitrobenzoyl chloride are reacted in a solvent such as ethyl acetate, in the presence of a tertiary organic base such as 2-picoline, at ambient temperature, to afford the hydroxy ester **7.5.** The hydroxy ester is next reacted with a sulfonyl chloride such as methanesulfonyl chloride, 4-toluenesulfonyl chloride and the like, in the presence of a base, in an aprotic polar solvent at low temperature, to afford the corresponding sulfonyl ester **7.6.** Preferably, equimolar amounts of the carbinol **7.5** and methanesulfonyl chloride are reacted together in ethyl acetate containing triethylamine, at about 10°C, to yield the mesylate **7.6.** The compound **7.6** is then subjected to a hydrolysis-cyclization reaction to afford the oxirane **7.7.** The mesylate or analogous leaving group present in **7.6** is displaced by hydroxide ion, and the carbinol thus produced, without isolation, spontaneously transforms into the oxirane **7.7** with elimination of 4-nitrobenzoate. To effect this transformation, the sulfonyl ester **7.6** is reacted with an alkali metal hydroxide or tetraalkylammonium hydroxide in an aqueous organic solvent. Preferably, the mesylate **7.6** is reacted with potassium hydroxide in aqueous dioxan at ambient temperature for about 1 hour, to afford the oxirane **7.7.**
The oxirane compound **7.7** is then subjected to regiospecific ring-opening reaction by treatment with a secondary amine **1.2,** to give the aminoalcohol **7.8.** The amine and the oxirane are reacted in a protic organic solvent, optionally in the additional presence of water, at 0°C to 100°C, and in the presence of an inorganic base, for 1 to 12 hours, to give the product **7.8.** Preferably, equimolar amounts of the reactants **7.7** and **1.2** are reacted in aqueous methanol at about 60°C in the presence of potassium carbonate, for about 6 hours, to afford the aminoalcohol **7.8.** The free amine is then substituted by treatment with an acid, chloroformate or sulfonyl chloride as described above in Scheme **1** to give the amine **7.9.** The carbobenzyloxy (cbz) protecting group in the product **7.9** is removed to afford the free amine **7.10.** Methods for removal of cbz groups are described, for example, in Protective Groups in Organic Synthesis, by T.W. Greene and P.G.M Wuts, Second Edition, p. 335. The methods include catalytic hydrogenation and acidic or basic hydrolysis. For example, the cbz-protected amine **7.9** is reacted with an alkali metal or alkaline earth hydroxide in an aqueous organic or alcoholic solvent, to yield the free amine **7.10.** Preferably, the cbz group is removed by the reaction of **7.9** with potassium hydroxide in an alcohol such as isopropanol at ca. 60°C to afford the amine **7.10.** The amine **7.10** so obtained is next acylated with a carboxylic acid or activated derivative **1.7,** using the conditions described above in Scheme **1** to afford the product **7.11**

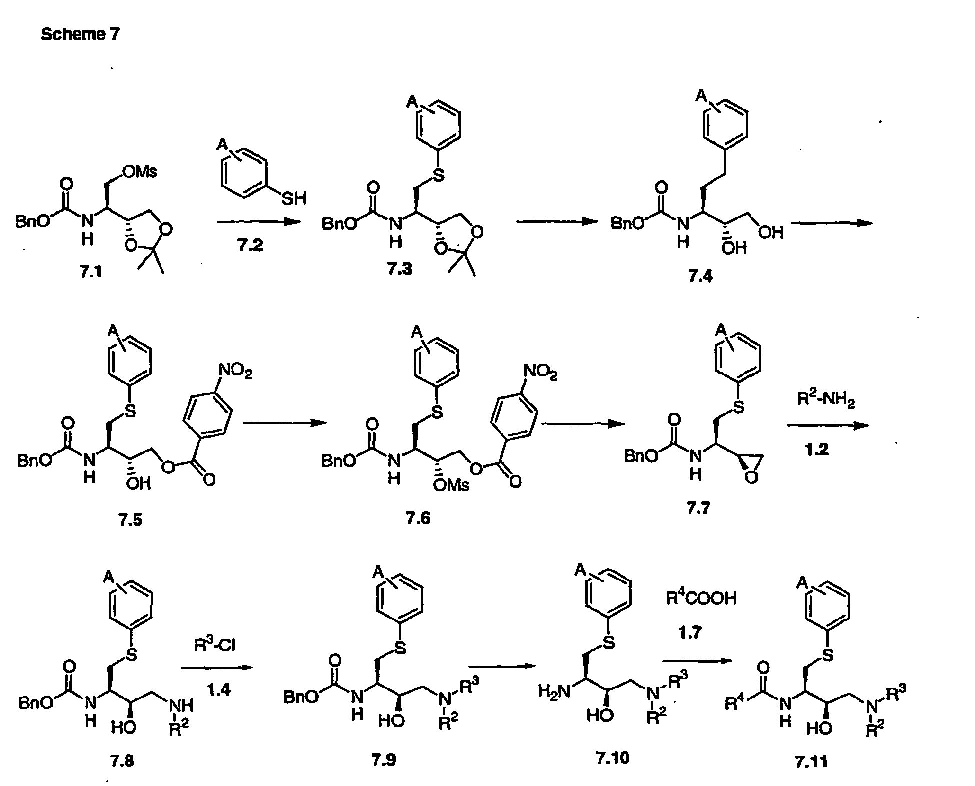

Scheme **8** illustrates an alternative preparation of the compounds **1** in which the substituent X is S, and in which the group A is either the group link-P(O)(OR¹)₂ or a precursor thereto, such as [OH], [SH] Br etc. In this sequence, 4-amino-tetrahydro-furan-3-ol, **8.1,** the preparation of which is described in Tet. Lett., 2000, 41, 7017, is reacted with a carboxylic acid or activated derivative thereof, R⁴COOH, **1.7,** using the conditions described above for in Scheme **1** for the preparation of amides, to afford the amide **8.2.** The amide product **8.2** is then transformed, using the sequence of reactions shown in Scheme **8,** into the isoxazoline compound **8.5.** The hydroxyl group on the tetrahydrofuran moiety in **8.2** is converted into a leaving group such as p-toluenesulfonyl or the like, by reaction with a sulfonyl chloride in an aprotic solvent such as pyridine or dichloromethane. Preferably, the hydroxy amide **8.2** is reacted with an equimolar amount of methanesulfonyl chloride in pyridine, at ambient temperature, to afford the methanesulfonyl ester **8.3.** The product **8.3,** bearing a suitable sulfonyl ester leaving group, is then subjected to acid-catalyzed rearrangement to afford the isoxazoline **8.4.** The rearrangement reaction is conducted in the presence of an acylating agent such as a carboxylic anhydride, in the presence of a strong acid catalyst. Preferably, the mesylate **8.3** is dissolved in an acylating agent such as acetic anhydride at about 0°C, in the presence of about 5 mole % of a strong acid such as sulfuric acid, to afford the isoxazoline mesylate **8.4.** The leaving group, for example a mesylate group, is next subjected to a displacement reaction with an amine. The compound **8.4** is reacted with an amine **1.2**, as defined in Chart **3**, in a protic solvent such as an alcohol, in the presence of an organic or inorganic base, to yield the displacement product **8.5.** Preferably, the mesylate compound **8.4** is reacted with an equimolar amount of the amine **1.2,** in the presence of an excess of an inorganic base such as potassium carbonate, at ambient temperature, to afford the product **8.5.** The product **8.5** is then treated with R³Cl, chart **6** as described above in Scheme **1** to afford the amine **8.6.** The compound **8.6** is then reacted with a thiol **7.2** to afford the thioether **7.11.** The reaction is conducted in a polar solvent such as DMF, pyridine or an alcohol, in the presence of a weak organic or inorganic base, to afford the product **7.11.** Preferably, the isoxazoline **8.6** is reacted, in methanol, with an equimolar amount of the thiol **7.2,** in the presence of an excess of a base such as potassium bicarbonate, at ambient temperature, to afford the thioether **7.11.**

The procedures illustrated in Scheme **7-8** depict the preparation of the compounds **7.11** in which X is S, and in which the substituent A is either the group link-P(O)(OR¹)₂ or a precursor thereto, such as [OH], [SH] Br etc, as described below. Scheme **9** illustrates the conversion of compounds **7.11** in which A is a precursor to the group link-P(O)(OR¹)₂ into the compounds **1** in which X=S. Procedures for the conversion of the substituent A into the group link-P(O)(OR¹)₂ are illustrated below, (Schemes **47 - 99**).

Scheme **9a-9b** depicts the preparation of phosphonate esters **1,** in which X is sulfur, the R₄COOH group does contain a amine group, and in which substituent A is either the group link-P(O)(OR¹)₂ or a precursor such as [OH], [SH], [NH], Br etc. The amine **7.10** prepared in Scheme **7** is treated with the CBZ protected amine **5.1** using the same conditions described in Scheme **5** for the preparation of **5.2** to give CBZ amine **9a.1.** Removal of the CBZ group as described in Scheme **5** to give **9a.2** followed by treatment with chloroacetyl chloride as described in Scheme **5** gives chloride **9a.3.** The chloride **9a.3** is then treated with the amine **5.7** to give the amine **9a.4** as described in Scheme **5.**

The reactions shown in Scheme **9a** illustrate the preparation of the compound **9a.4** in which the substituent A is either the group link-P(O)(OR¹)₂ or a precursor such as [OH], [SH], [NH], Br etc. Scheme **9b** depicts the conversion of **9a.4** in which A is [OH], [SH], [NH], Br etc, into the phosphonate ester **1** in which X is sulfur. In this procedure **9a.4** is converted, using the procedures described below, Schemes **47-99,** into the compound **1.**

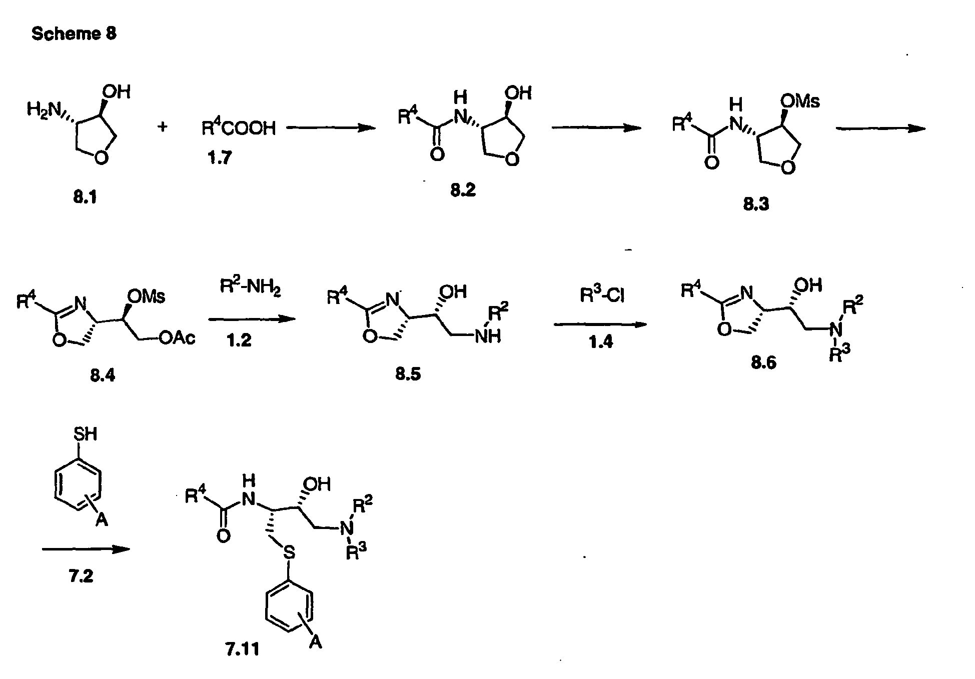

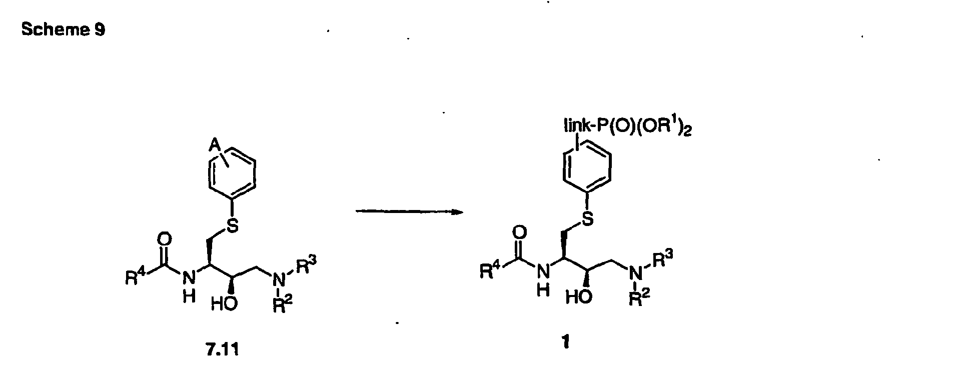

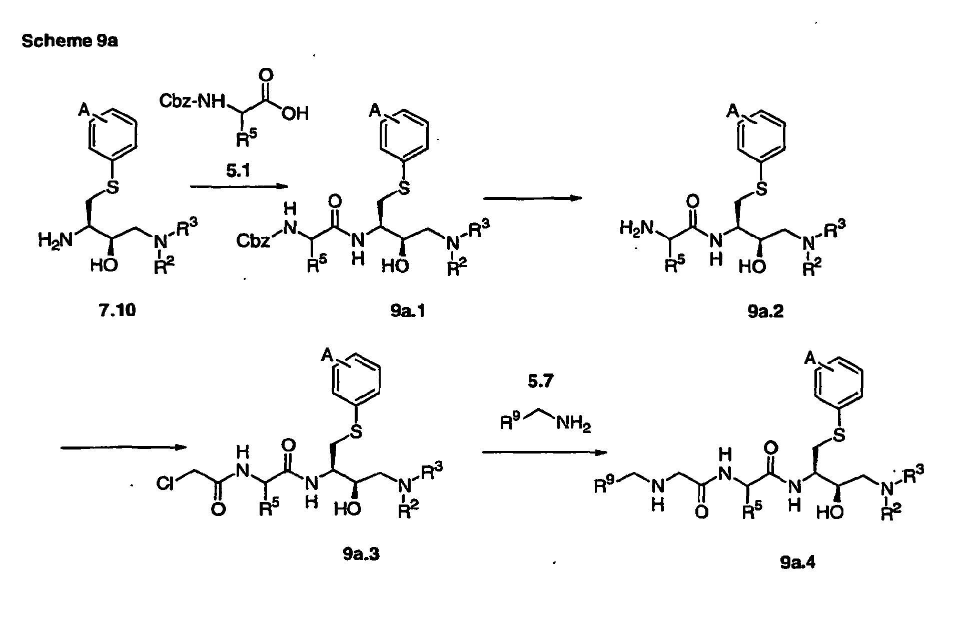

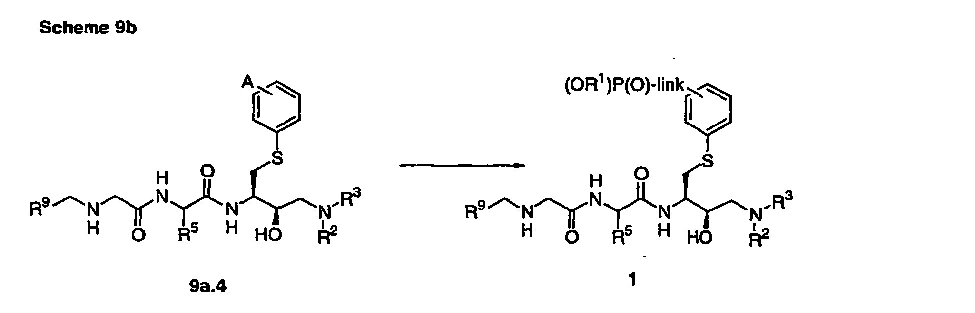

### Preparation of the phosphonate ester intermediates 2 and 3 in which X is a direct bond

Schemes **10-12** illustrate the preparation of the phosphonate esters **2** and **3** in which X is a direct bond and the R₄COOH group does not contain a primary or secondary amine group. As shown in Scheme **10,** the epoxide **10.1,** prepared as described in J. Med. Chem 1994, 37, 1758 is reacted with the amine **10.2** or **10.5,** in which the substituent A is either the group link-P(O)(OR¹)₂ or a precursor such as [OH], [SH], [NH], Br etc, to afford the amine **10.3** and **10.6** respectively. The reaction is performed under the same conditions as described above, Scheme **1** for the preparation of the amine **1.3.** The preparation of the amines **10.2** is described in Schemes **73-75** and amines **10.5** in schemes **76-78.** The products **10.3** and **10.6** are then transformed, using the sequence of reactions described above, Scheme **1,** for the conversion of the amine **1.3** into the amide **1.8,** into the aminoamide **10.4** and **10.7** respectively.

An alternative route to the amines **10.4** and **10.7** is shown in Scheme **11** in which sulfonyl ester **11.1** prepared according to Chimia 1996, 50, 532 is treated under conditions described in Scheme **2** with the amines **10.2** or **10.5** to give the amines **11.2** or **11.3** respectively. These amine products are then converted as described above, Scheme **2,** into the amides **10.4** and **10.7** respectively.

The reactions shown in Scheme **10** and **11** illustrate the preparation of the compounds **10.4** and **10.7** in which the substituent A is either the group link-P(O)(OR¹)₂ or a precursor such as [OH], [SH], [NH], Br etc. Scheme **12** depicts the conversion of these compounds **10.4** and **10.7** in which A is [OH], [SH], [NH], Br etc, into the phosphonate esters 2 and 3 respectively, in which X is a direct bond. In this procedure, the amines **10.4** and **10.7** are converted, using the procedures described below, Schemes **47-99,** into the compounds 2 and 3 respectively. 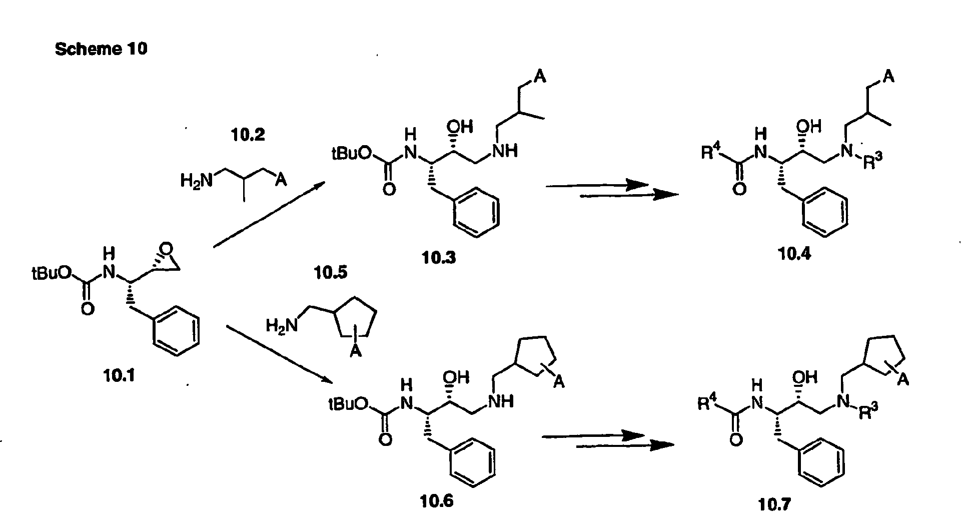 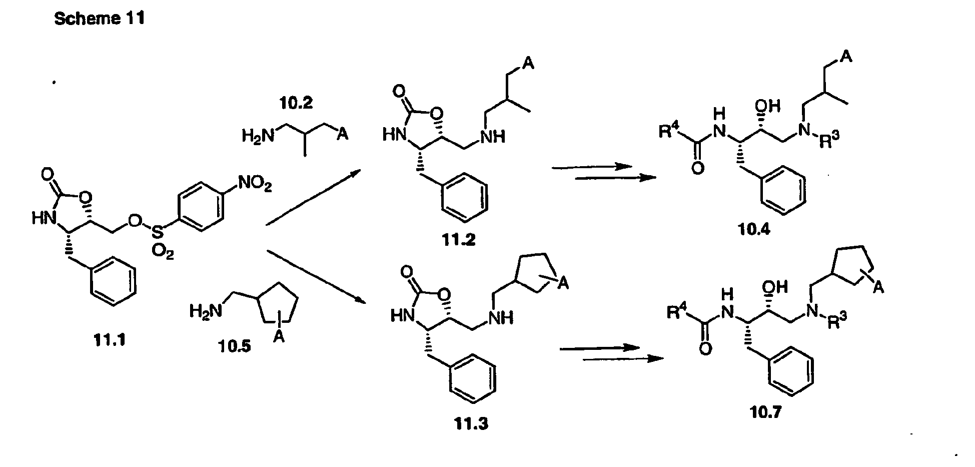 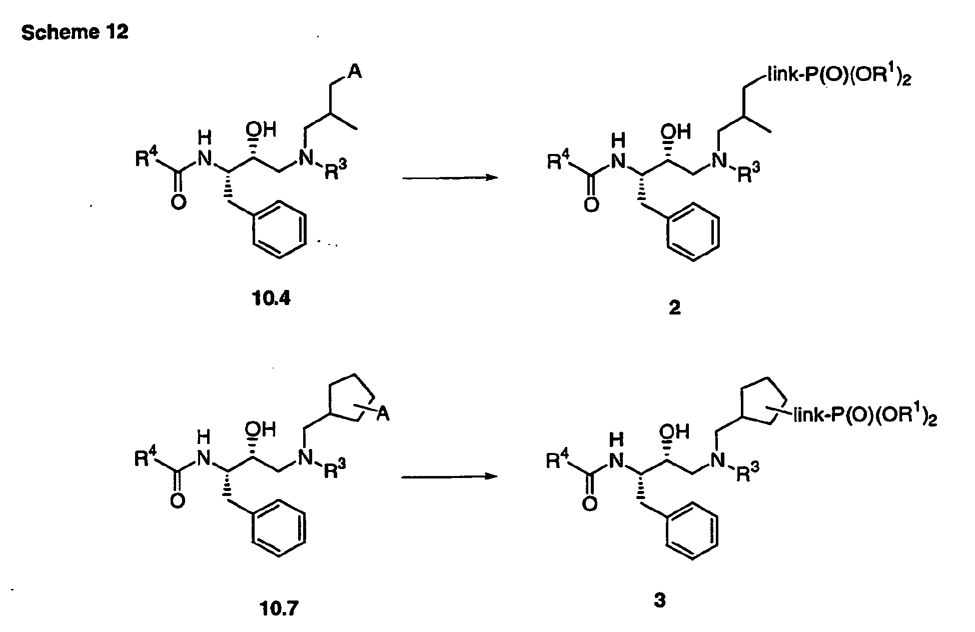 Schemes **13-14** illustrates the preparation of the phosphonate esters **2** and **3** in which X is a direct bond and the R₄COOH group contains an amine. The epoxide **13.1,** prepared as described in US 6391919B1, or J. Org. Chem 1996, 61, 3635 is reacted, as described above, (Scheme **1**) with the amine **10.2** or **10.5,** in which substituent A is either the group link-P(O)(OR¹)₂ or a precursor such as [OH], [SH], [NH], Br etc, to give the amino alcohols **13.2** and **13.4,** respectively. These amines are then converted as described in Scheme **3** for the conversion of **3.2** into **3.4** and Scheme **5** for the conversion of **3.4** into **5.8,** into the amine products 13.3 and 13.5 respectively.

The reactions shown in Scheme **13** illustrate the preparation of the compounds **13.3** and **13.5** in which the substituent A is either the group link-P(O)(OR¹)₂ or a precursor such as [OH], [SH], [NH], Br etc. Scheme **14** depicts the conversion of the compounds 1**3.3** and **13.5** in which A is [OH], [SH], [NH], Br etc, into the phosphonate esters **2** and **3** in which X is a direct bond. In this procedure, the compounds **13.3** and **13.5** are converted, using the procedures described below, Schemes **47-99,** into the compounds **2** and **3** respectively.

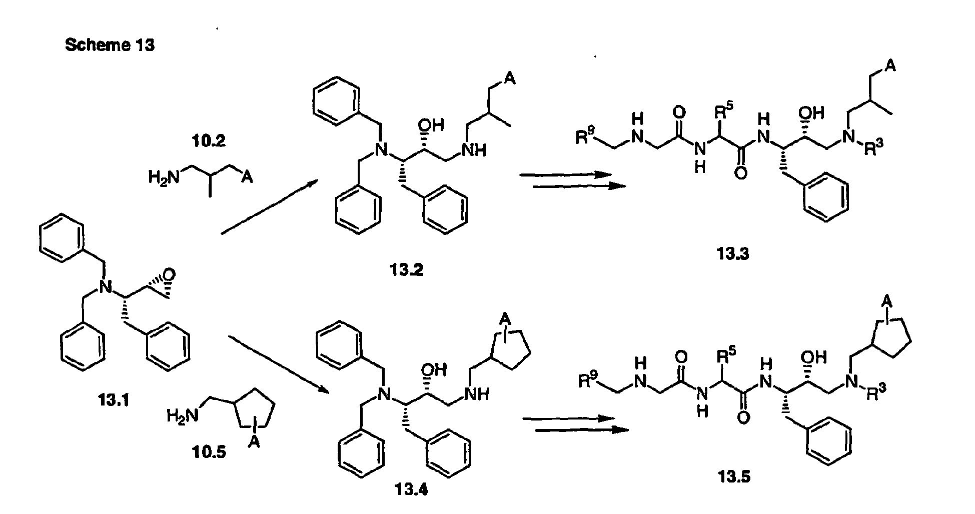

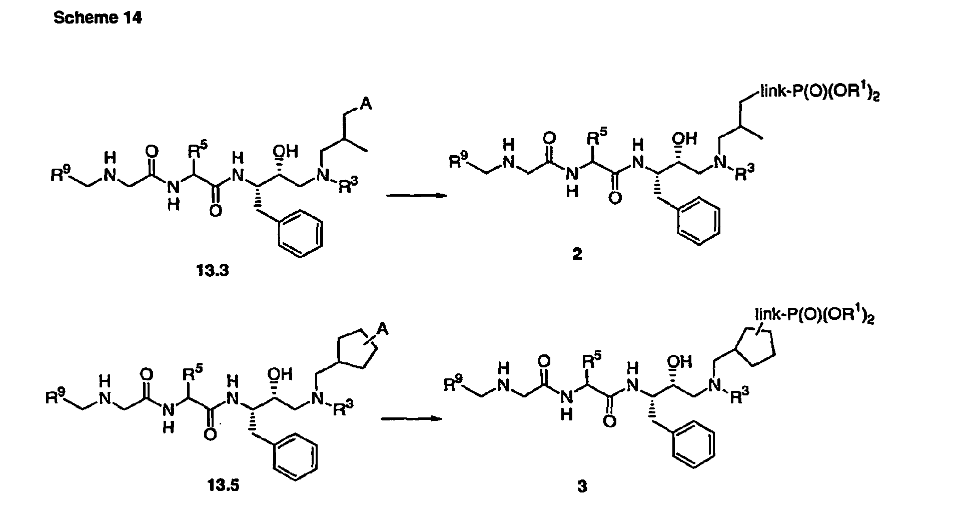

### Preparation of the phosphonate ester intermediates 2 and 3 in which X is a sulfur

The intermediate phosphonate esters **2** and **3,** in which the group A is attached to a sulfur linked aryl moiety, and the R₄COOH group does not contain an amine group, are prepared as shown in Schemes **15-17.** In Scheme **15,** epoxide **15.1** is prepared from mesylate **7.1** using the conditions described in Scheme **7** for the preparation of **7.7** from **7.1,** except incorporating thiophenol for thiol **7.2.** The epoxide **15.1** is then treated with amine **10.2** or amine **10.5,** in which substituent A is either the group link-P(O)(OR¹)₂ or a precursor such as [OH], [SH], [NH], Br etc, as described in Scheme 7, to give the amines **15.2** and **15.4.** Further application of Scheme **7** on the amines **15.2** and **15.4** yields the alcohols **15.3** and **15.5** respectively. Alternatively, Scheme 16 depicts the preparation of **15.3** and **15.5** using the mesylate **8.4.** The amines **10.2** and **10.5** are reacted with mesylate **8.4** under conditions described in Scheme **8** to give amines **16.1** and **16.2** respectively. Further modification of **16.1** and **16.2** according to the conditions described in Scheme **8** then affords alcohols **15.3** and **15.5** respectively.

The reactions shown in Scheme **15-16** illustrate the preparation of the compounds **15.3** and **15.5** in which the substituent A is either the group link-P(O)(OR¹)₂ or a precursor such as [OH], [SH], [NH], Br etc. Scheme **17** depicts the conversion of **15.3** and **15.5** in which A is [OH], [SH], [NH], Br etc, into the phosphonate ester **2** and **3** in which X is sulfur. In this procedure **15.3** or **15.5** is converted, using the procedures described below, Schemes **47-99,** into the compound **2** and **3.** 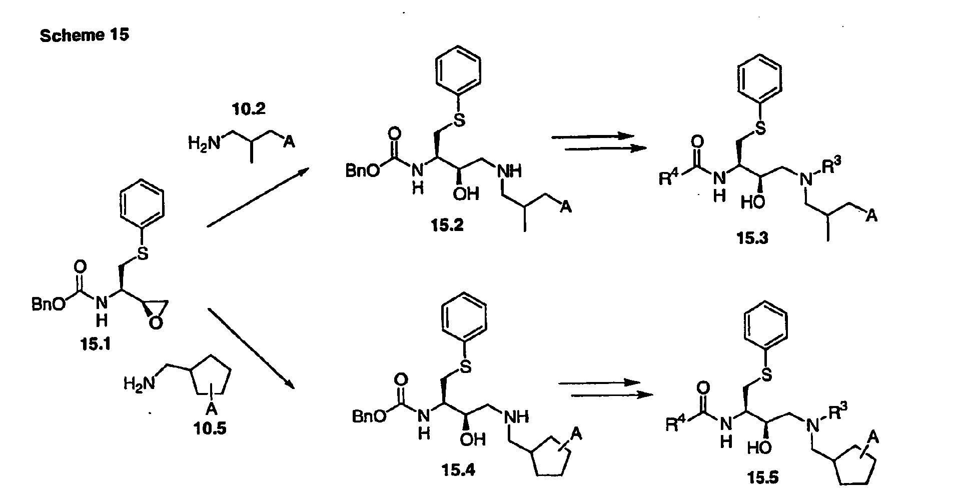 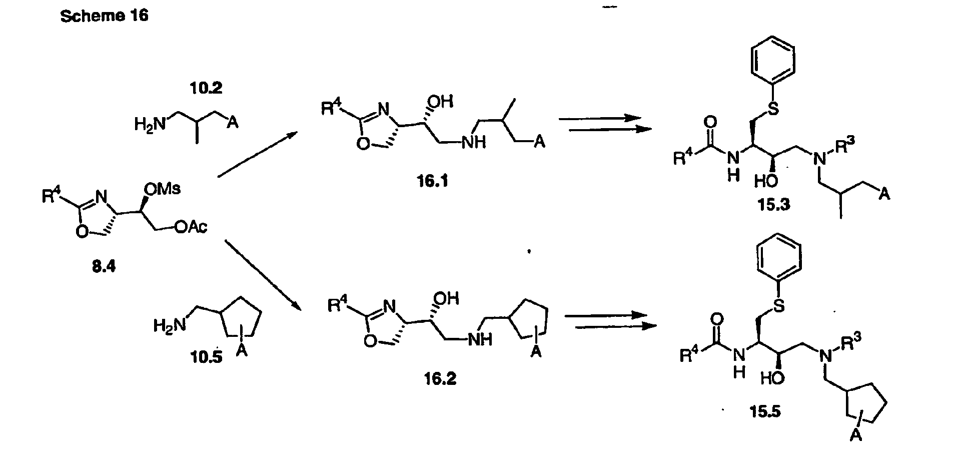 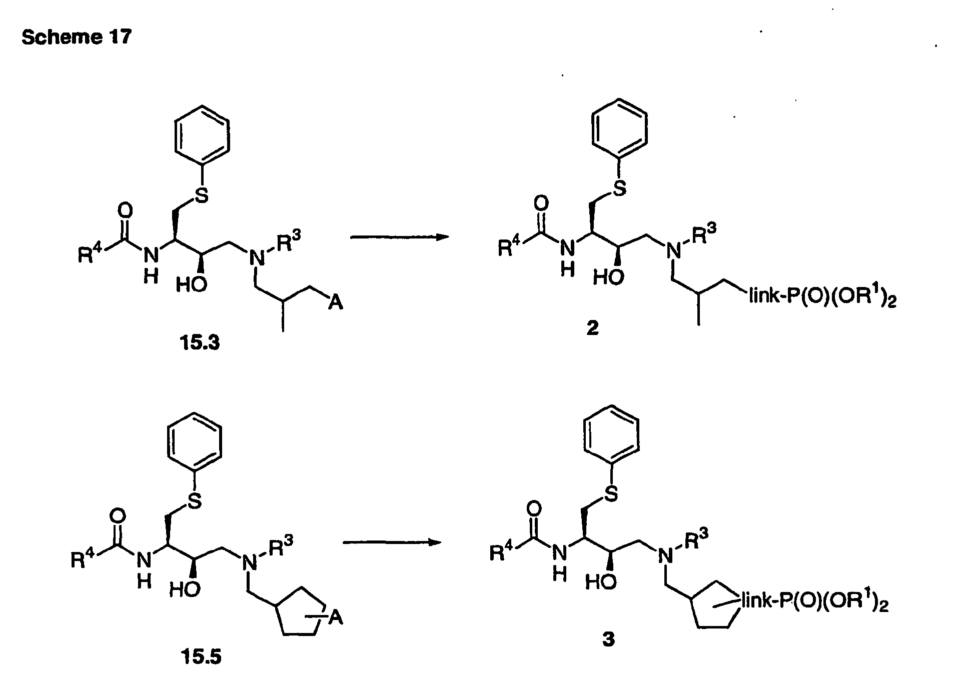 Scheme **18-19** depict the preparation of phosphonate esters **2** and **3, in** which the group A is attached to a sulfur linked aryl moiety, and the R₄COOH group contains a amine group. The amines **15.2** and **15.4,** in which substituent A is either the group link-P(O)(OR¹)₂ or a precursor such as [OH], [SH], [NH], Br etc, prepared in Scheme **15,** are converted using the same conditions described in Scheme **7** for the preparation of the amine **7.10** from **7.8** and Scheme **9a** for the preparation of **9a.4** from **7.10** to give **18.1** and **18.2** respectively.

The reactions shown in Scheme **18** illustrate the preparation of the compound **18.1** and **18.2** in which the substituent A is either the group link-P(O)(OR¹)₂ or a precursor such as [OH], [SH], [NH], Br etc. Scheme **19** depicts the conversion of **18.1** and **18.2** in which A is [OR], [SH], [NH], Br etc, into the phosphonate ester **2** and **3** respectively in which X is sulfur. In this procedure **18.1** and **18.2** are converted, using the procedures described below, Schemes **47-99,** into the compounds **2** and **3**

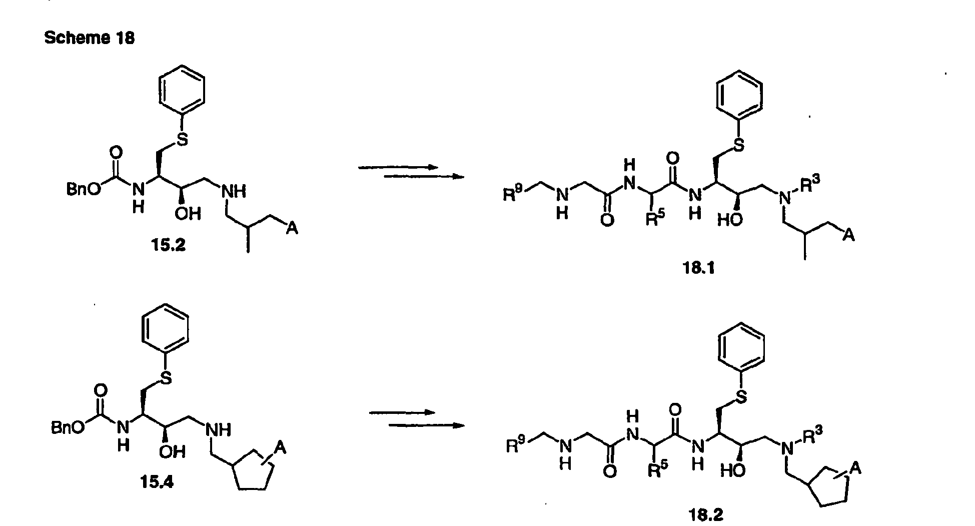

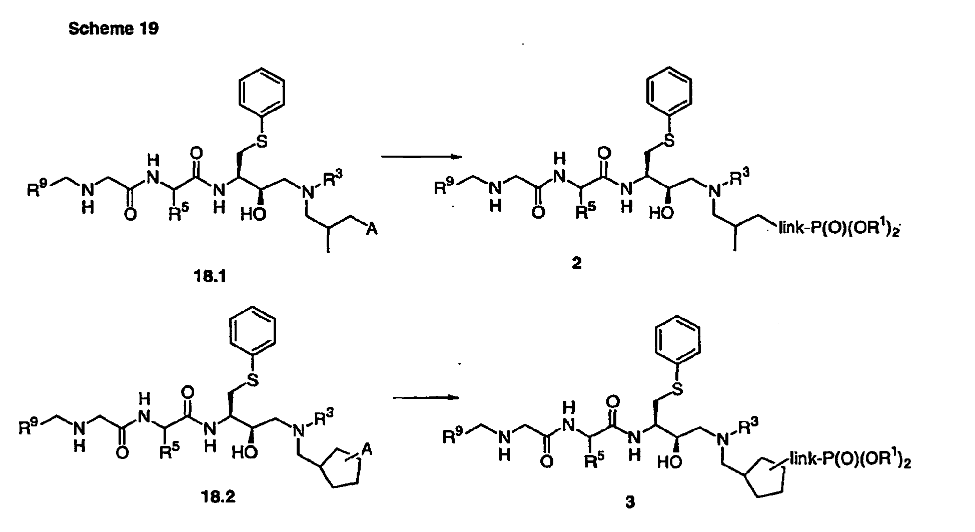

### Preparation of the phosphonate ester intermediates 4 in which X is a direct bond

Schemes **20-22** illustrate the preparation of the phosphonate esters **4** in which X is a direct bond and the R group does not contain a primary or secondary amine group. As shown in Scheme **20,** the amine **20.1** is reacted with the sulfonyl chloride **20.2** in which the substituent A is either the group link-P(O)(OR¹)₂ or a precursor such as [OH], [SH], [NH], Br etc, to afford the product **20.3.** The reaction is performed under the same conditions as described above, Scheme **1** for the preparation of the sulfonamide **1.5.** Amine **20.1** is prepared by treatment of epoxide **10.1** with the amine **1.2** as described in Scheme 1 for the preparation of **1.3.** The preparation of sulfonyl chloride **20.2** is described in Schemes **92-97.** The product **20.3** is then transformed, using the sequence of reactions described above, Scheme **1,** for the conversion of the amide **1.5** into the amide **1.8,** into the product **20.4.**

An alternative route to the product **20.4** is shown in Scheme **21** in which amine **11.1** is treated under conditions described in Scheme **2** with the amine **1.2** to give the amine **21.1.** The amine **21.1** is then sulfonylated with **20.2** in which the substituent A is either the group link-P(O)(OR¹)₂ or a precursor such as [OH], [SH], [NH], Br etc, as described in Scheme **2,** to afford the product **21.2.** The product **21.2** is then converted as described above, Scheme **2,** into the sulfonamide **20.4.**

The reactions shown in Scheme **20** and **21** illustrate the preparation of the compound **20.4** in which the substituent A is either the group link-P(O)(OR¹)₂ or a precursor such as [OH], [SH], [NH], Br etc. Scheme **22** depicts the conversion of this compounds **20.4** in which A is [OH], [SH], [NH], Br etc, into the phosphonate esters **4** respectively, in which X is a direct bond. In this procedure, the amines **20.4** is converted, using the procedures described below, Schemes **47-99,** into the compounds **4.** 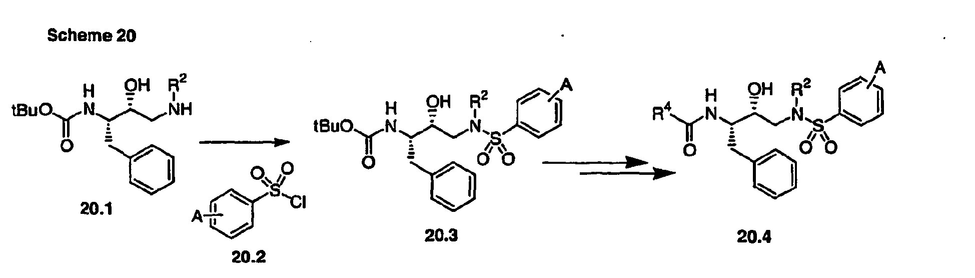 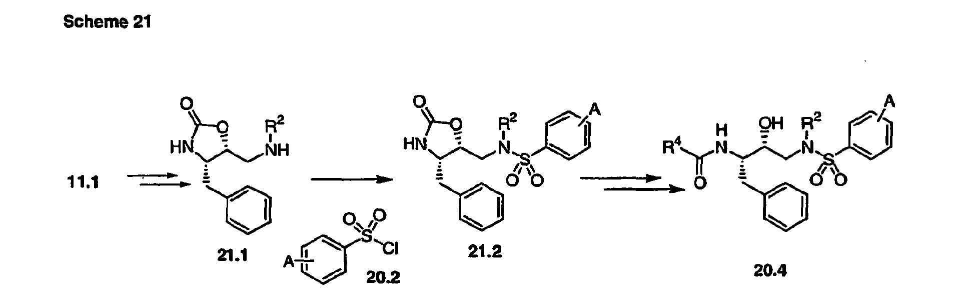 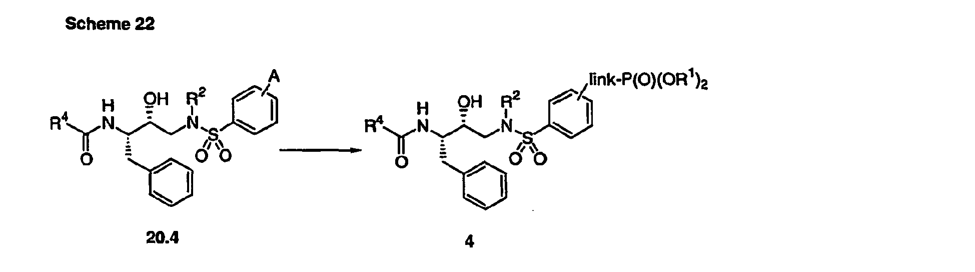 Schemes **23** illustrates the preparation of the phosphonate esters **4** in which X is a direct bond and the R₄COOH group contains an amine group. The amine **23.1,** prepared from the epoxide **13.1** and an amine **1.2** as described in Scheme **13** for the synthesis of **13.2** from **13.1,** is reacted with the sulfonyl chloride **20.2** in which the substituent A is either the group link-P(O)(OR¹)₂ or a precursor such as [OH], [SH], [NH], Br etc, as described in Schemes **1** for the synthesis of **1.5,** to give the product **23.2.** The product **23.2** is then reduced to amine **23.3** according to the conditions described in Scheme **3** for the preparation of **3.4** from **3.3.** The amine product is then converted as described in Scheme **5** into the chloride **23.4.** The chloride is treated with the amine **5.7** to afford the amine **23.5,** as described in Scheme 5 for the preparation of **5.8** from **5.7.**

The reactions shown in Scheme **23** illustrate the preparation of the compound **23.5** in which the substituent A is either the group link-P(O)(OR¹)₂ or a precursor such as [OH], [SH], [NH], Br etc. Scheme **24** depicts the conversion of the compound **23.5** in which A is [OH], [SH], [NH], Br etc, into the phosphonate esters **4** in which X is a direct bond. In this procedure, the compound **23.5** is converted, using the procedures described below, Schemes **47-99,** into the compound **4.**

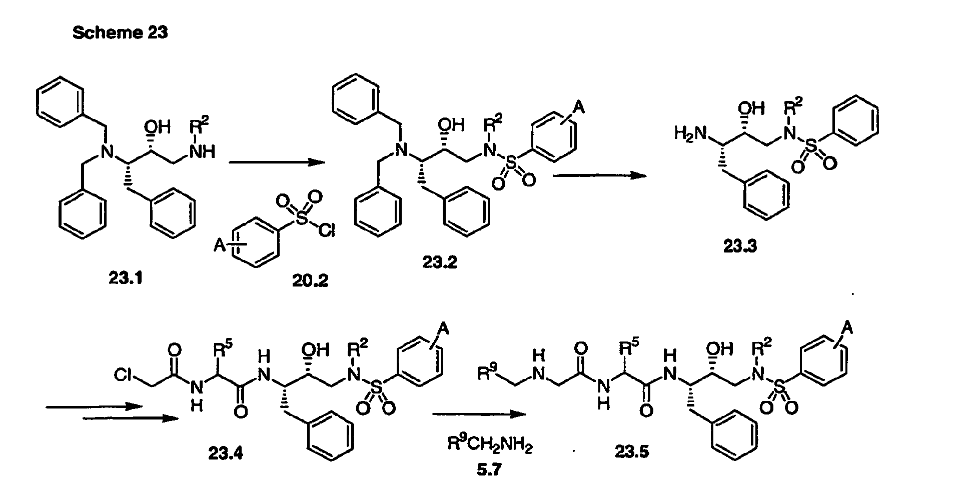

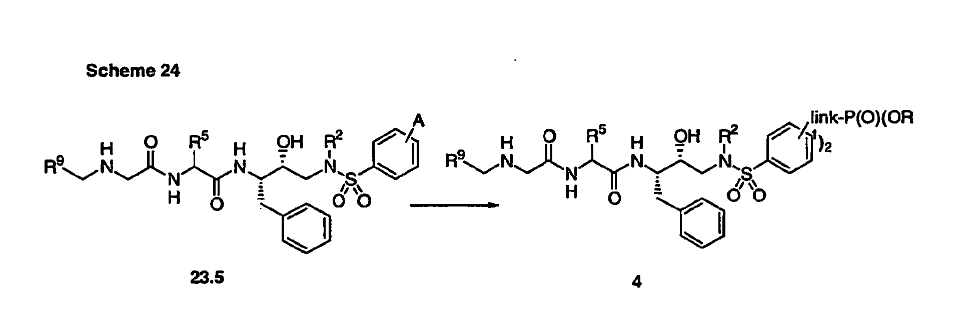

### Preparation of the phosphonate ester intermediates 4 in which X is a sulfur

The intermediate phosphonate ester **4,** in which the group A is attached to a sulfur linked aryl moiety, and the R₄COOH group does not contain an amine is prepared as shown in Schemes **25-27.** Amine **25.1** prepared from epoxide **15.1** and amine **1.2** as described in Scheme **15** is treated with sulfonamide **20.2** in which the substituent A is either the group link-P(O)(OR¹)₂ or a precursor such as [OH], [SH], [NH], Br etc, using the conditions described in Scheme **7,** to give the sulfonamide **25.2.** The sulfonamide **25.2** is then converted as described in Scheme 7 for the conversion **of 7.9** to **7.10,** and Scheme **9a** for the conversion of **7.10** into **9a.4,** to the product **25.3.** Alternatively, Scheme **26,** illustrates how the amine **8.5** prepared according to Scheme **8** is reacted with **20.2** under conditions described in Scheme **8** for the preparation of **8.6** from **8.5,** to give the sulfonamide **26.1.** Further modification according to the conditions described in Scheme **8** for the preparation of **7.11,** affords sulfonamide **25.3.**

The reactions shown in Scheme **25-26** illustrate the preparation of the compounds sulfonamide **25.3** in which the substituent A is either the group link-P(O)(OR¹)₂ or a precursor such as [OH], [SH], [NH], Br etc. Scheme **27** depicts the conversion of **25.3** in which A is [OH], [SH], [NH], Br etc, into the phosphonate **4** in which X is sulfur. In this procedure **25.3** is converted, using the procedures described below, Schemes **47-99,** into the compound **4.**

Preparation of the intermediate phosphonate ester **4,** in which the group A is attached to a sulfur linked aryl moiety, and the R₄COOH group contains an amine are prepared as shown in Schemes **28-29.** Amine **25.2** (Scheme **25**) in which the substituent A is either the group link-P(O)(OR¹)₂ or a precursor such as [OH], [SH], [NH], Br etc, is converted to **28.1** as described in Scheme **7** for the preparation of the amine **7.10** from **7.9** and Scheme **9a** for the preparation of **9a.4** from **7.10.**

The reactions shown in Scheme **28** illustrate the preparation of the compounds sulfonamide **28.1** in which the substituent A is either the group link-P(O)(OR¹)₂ or a precursor such as [OH], [SH], [NH], Br etc. Scheme **29** depicts the conversion of **28.1** in which A is [OH], [SH], [NH], Br etc, into the phosphonate **4** in which X is sulfur. In this procedure **28.1** is converted, using the procedures described below, Schemes **47-99,** into the compound 4.

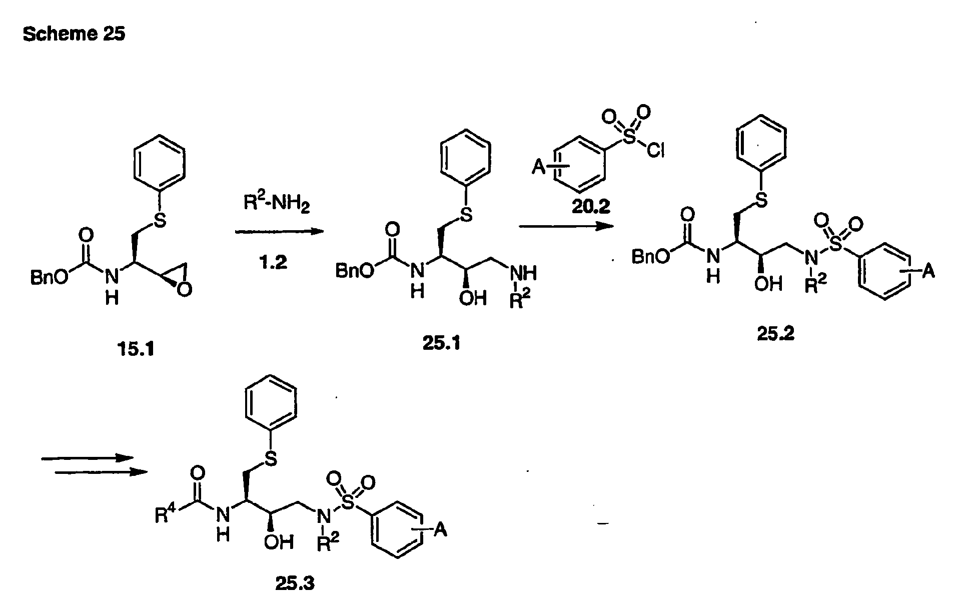

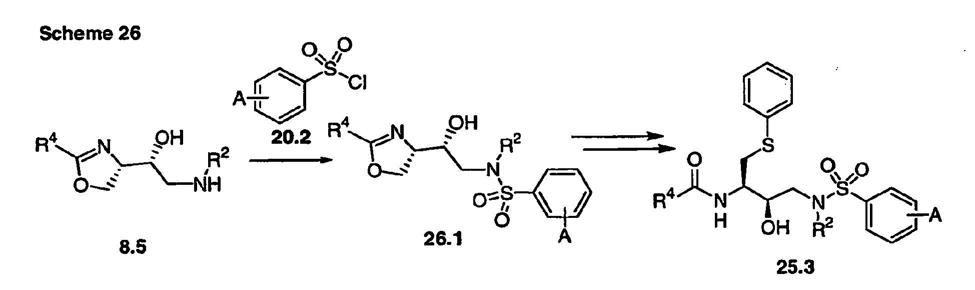

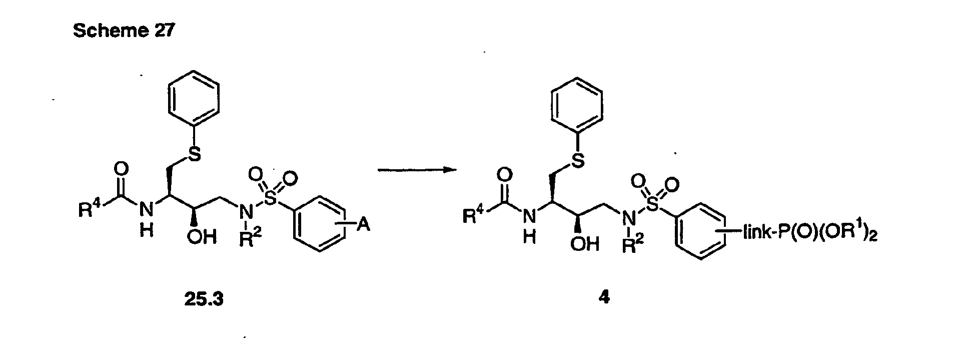

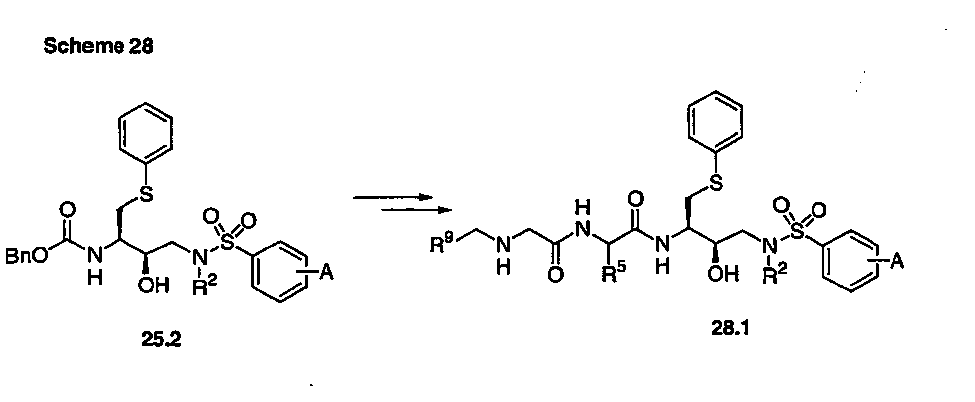

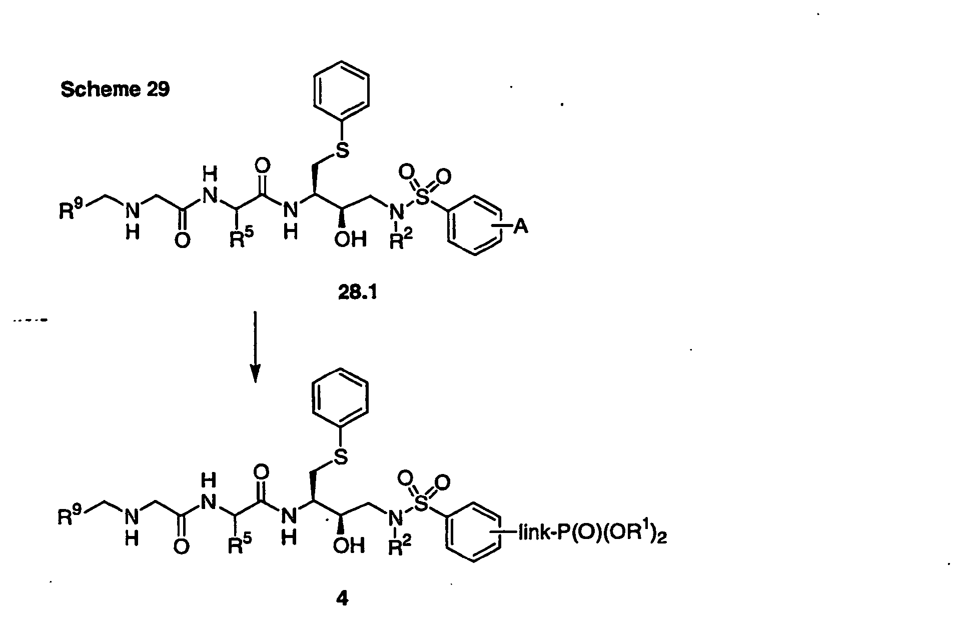

### Preparation of the phosphonate ester intermediates 5 in which X is a direct bond

Schemes **30** illustrates the preparation of the phosphonate esters **5** in which X is a direct bond and the R group does not contain a primary or secondary amine group. As shown in Scheme **30,** the amine **23.1** (Scheme **23**) is reacted with the alcohol **30.1** in which the substituent A is either the group link-P(O)(OR¹)₂ or a precursor such as [OH], [SH], [NH], Br etc, to afford the carbamate **30.2.** The reaction is performed under conditions described below, Scheme 98, for making carbamates from amines and alcohols. The preparation of the **30.1** is described in Schemes **83-86.** The carbamate **30.2** is then deprotected using conditions described in Scheme **3** for removal of the benzyl groups to give **30.3.** Treatment of **30.3** with the R⁴COOH acid **1.7** using the conditions described in Scheme 1 then afford the amide **30.4**

The reactions shown in Scheme **30** illustrate the preparation of the compound **30.4** in which the substituent A is either the group link-P(O)(OR¹)₂ or a precursor such as [OH], [SH], [NH], Br etc. Scheme **31** depicts the conversion of this compounds **30.4** in which A is [OH], [SH], [NH], Br etc, into the phosphonate esters **5** respectively, in which X is a direct bond. In this procedure, the amines **30.4** is converted, using the procedures described below, Schemes **47-99,** into the compounds **5.**

Schemes **32** illustrates the preparation of the phosphonate esters 5 in which X is a direct bond and the R₄COOH group contains an amine. The carbamate **30.2** in which the substituent A is either the group link-P(O)(OR¹)₂ or a precursor such as [OR], [SH], [NH], Br etc, is converted into the chloride **32.1** using conditions as described in Scheme **9a.** Chloride **32.1** is then treated with amine **5.7** to give the amine **32.2,** as described in Scheme **9a** for the conversion of **7.10** into **9a.3.**

The reactions shown in Scheme **32** illustrate the preparation of the compound **32.2** in which the substituent A is either the group link-P(O)(OR¹)₂ or a precursor such as [OH], [SH], [NH], Br etc. Scheme **33** depicts the conversion of the compound **32.2** in which A is [OH], [SH], [NH], Br etc, into the phosphonate esters **5** in which X is a direct bond. In this procedure, the compound **32.2** is converted, using the procedures described below, Schemes **47-99,** into the compound **5.**

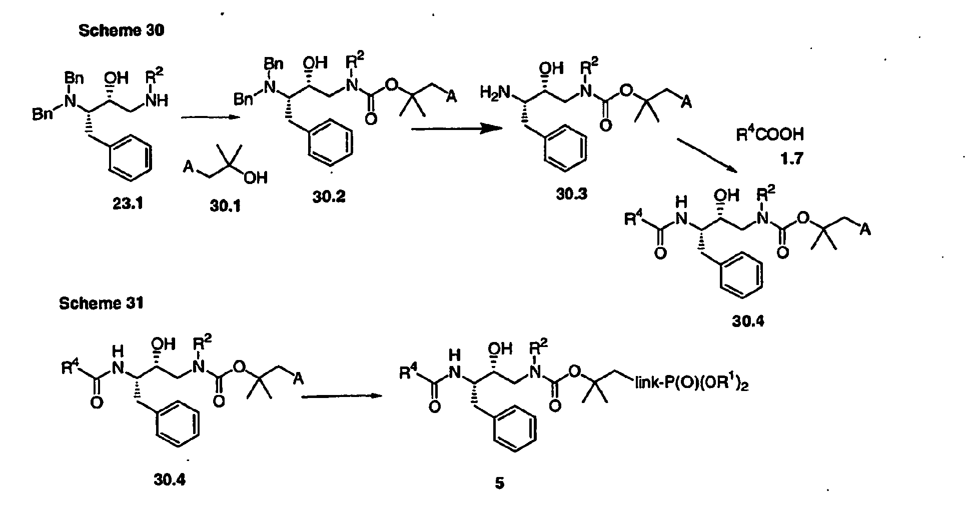

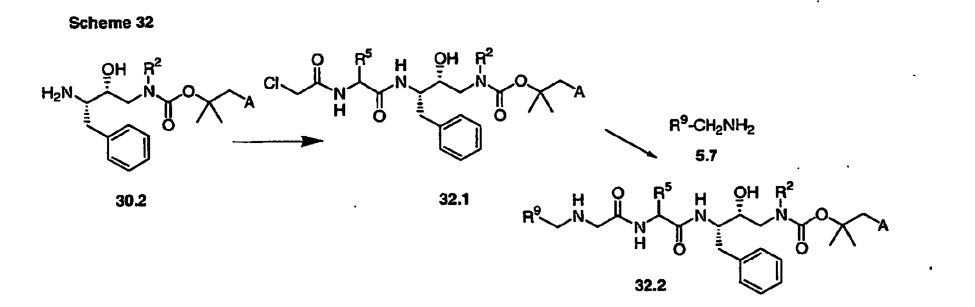

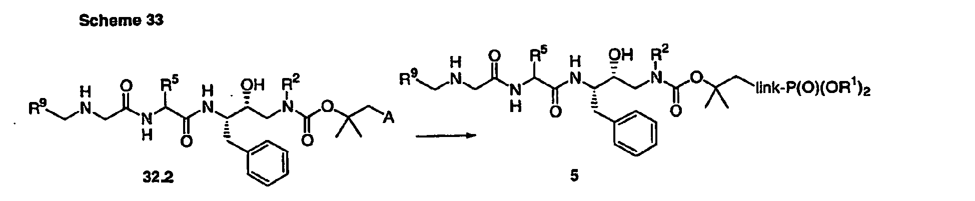

### Preparation of the phosphonate ester intermediates 5 in which X is a sulfur

The intermediate phosphonate ester **5,** in which the group A is attached to a sulfur linked aryl moiety, is prepared as shown in Schemes **34-36.** Amine **25.1** prepared according to Scheme **25,** is treated with alcohol **30.1** in which the substituent A is either the group link-P(O)(OR¹)₂ or a precursor such as [OH], [SH], [NH], Br etc, using the conditions described below , Scheme 98, to give the carbamate **34.1.** The carbamate **34.1** is then converted as described in Scheme **7,** for the conversion of **7.9** to **7.11,** to the product **34.2.** Alternatively the amine **8.5** prepared according to Scheme **8** can be reacted with alcohol **30.1** under conditions described in Scheme **98** to give the carbamate **35.1.** Further modification according to the conditions described in Scheme **8,** except incorporating thiophenol, then affords sulfonamide **34.2.**

The reactions shown in Scheme **34-35** illustrate the preparation of the compounds sulfonamide **34.2** in which the substituent A is either the group link-P(O)(OR¹)₂ or a precursor such as [OH], [SH], [NH], Br etc. Scheme **36** depicts the conversion of **34.2** in which A is [OH], [SH], [NH], Br etc, into the phosphonate **5** in which X is sulfur. In this procedure **34.2** is converted, using the procedures described below, Schemes **47-99,** into the compound **5.**

Preparation of the intermediate phosphonate ester **5,** in which the group A is attached to a sulfur linked aryl moiety, and the R₄COOH group contains an amine are prepared as shown in Schemes **37-38.** Carbamate **34.1** (Scheme **35)** in which the substituent A is either the group link-P(O)(OR¹)₂ or a precursor such as [OH], [SH], [NH], Br etc, is converted to **37.1,** as described in Scheme **7** for the preparation of the amine **7.10** from **7.9** and Scheme **9a** for the preparation of **9a.4** from **7.10.**

The reactions shown in Scheme **37** illustrate the preparation of the compounds sulfonamide **37.1** in which the substituent A is either the group link-P(O)(OR¹)₂ or a precursor such as [OH], [SH], [NH], Br etc. Scheme **38** depicts the conversion of **37.1** in which A is [OH], [SH], [NH], Br etc, into the phosphonate **5** in which X is sulfur. In this procedure **37.1** is converted, using the procedures described below, Schemes **47-99,** into the compound **5.**

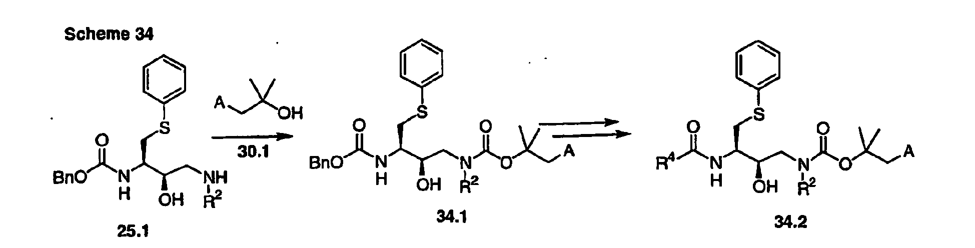

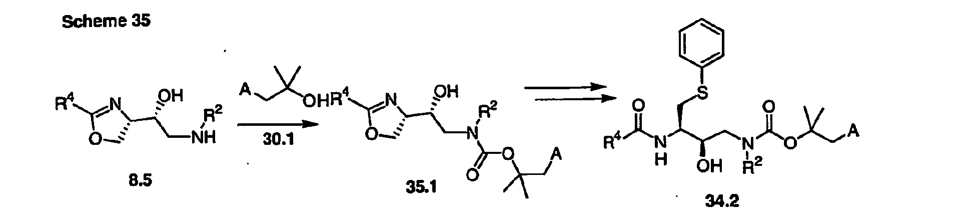

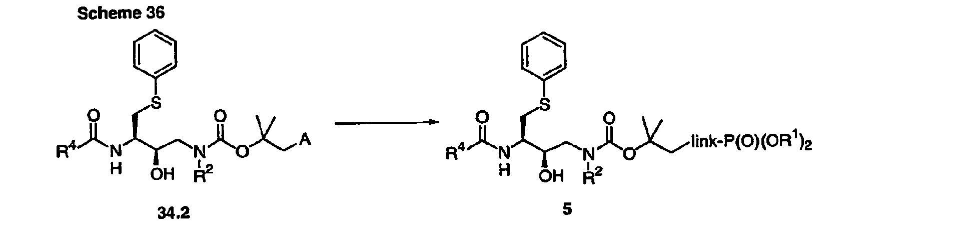

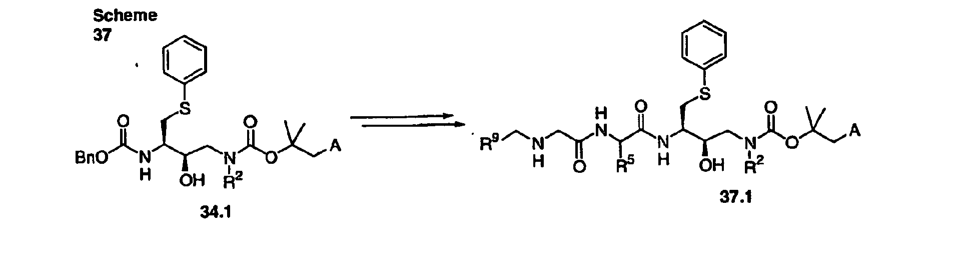

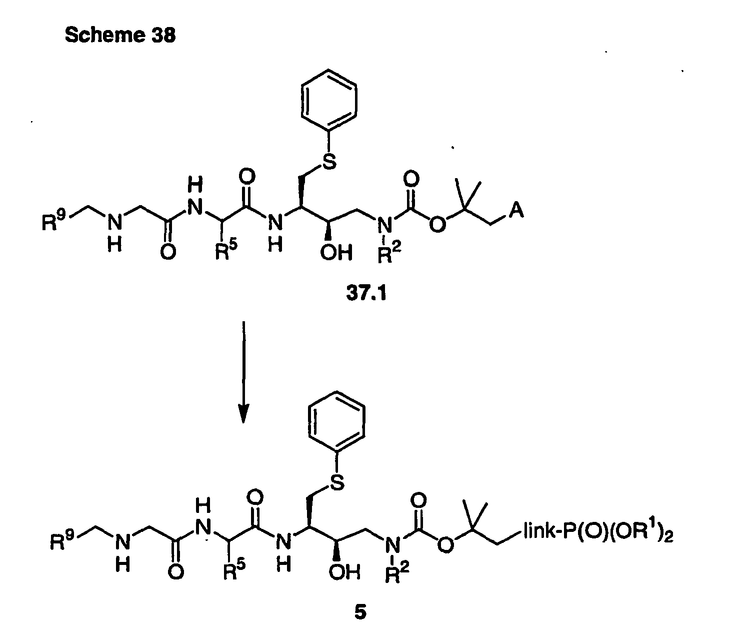

### Preparation of the phosphonate ester intermediates 6 and 7 in which X is a direct bond

Schemes **39-40** illustrate the preparation of the phosphonate esters **6** and **7** in which X is a direct bond. As shown in Scheme **39,** the epoxide **13.1,** prepared as described in Scheme **13** is converted to the chloride **39.1,** as described in Scheme **3,** for the preparation of **3.4,** and Scheme **5,** for the conversion of **3.4** into **5.6.** The chloride **39.1** is then reacted with the amine **39.2** or **39.4,** in which the substituent A is either the group link-P(O)(OR¹)₂ or a precursor such as [OH], [SH], [NH], Br etc, to afford the amine **39.3** and **39.5** respectively. The reaction is performed under the same conditions as described above, Scheme **5** for the preparation of the amine **5.8** from **5.6.** The prepartion of **39.2** and **39.4,** amines in which A is link-P(O)(OR¹)₂, are shown in Schemes **79-80** and Schemes **81-82** respectively.

The reactions shown in Scheme **39** illustrate the preparation of the compounds **39.3** and **39.5** in which the substituent A is either the group link-P(O)(OR¹)₂ or a precursor such as [OH], [SH], [NH], Br etc. Scheme **40** depicts the conversion of these compounds **39.3** and **39.5** in which A is [OH], [SH], [NH], Br etc, into the phosphonate esters **6** and **7** respectively, in which X is a direct bond. In this procedure, the amines **39.3** and **39.5** are converted, using the procedures described below, Schemes **47-99,** into the compounds **6** and **7** respectively.

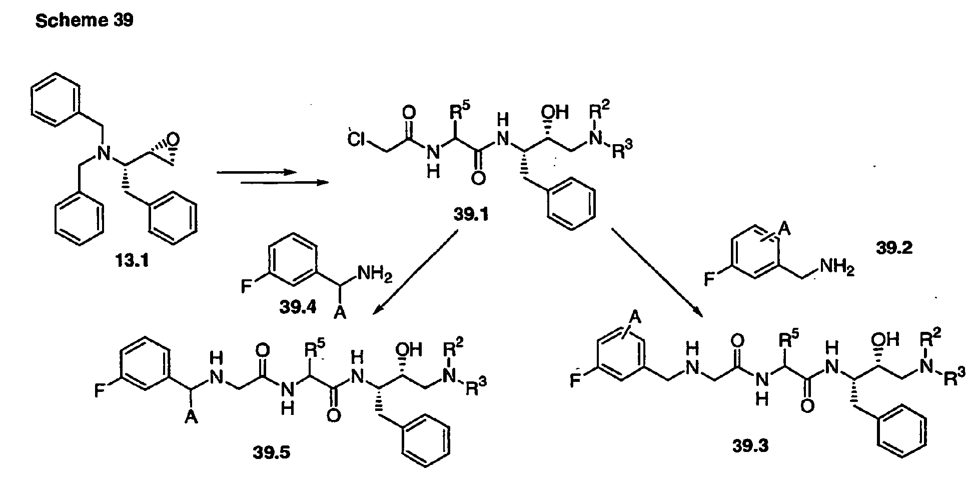

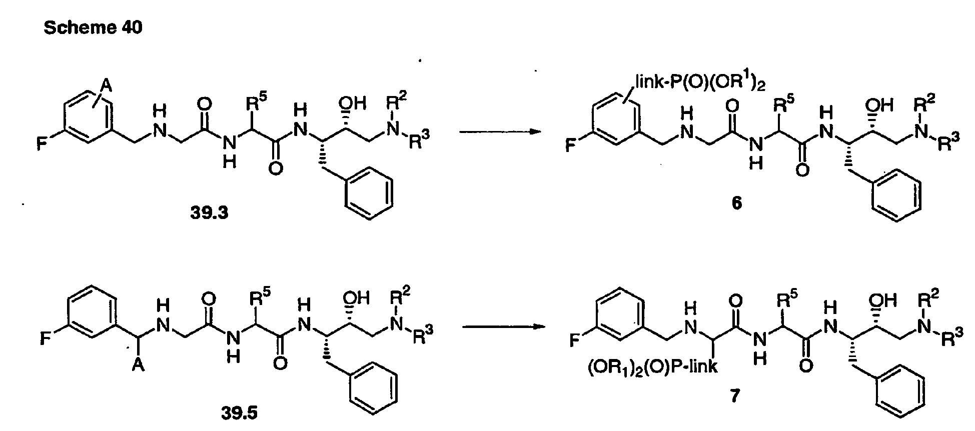

### Preparation of the phosphonate ester intermediates 6 and 7 in which X is a sulfur

The intermediate phosphonate esters **6** and **7,** in which the group A is attached to a sulfur linked aryl moiety, are prepared as shown in Scheme **41-42.** The amine **25.1** (Scheme **25**) is converted to the chloride **41.1** as described in Scheme **7** for the preparation of **7.10** from **7.8,** and Scheme **9a** for conversion of **7.10** to **9a3.** The chloride **41.1** is then treated with amine **39.2** or amine **39.4,** in which substituent A is either the group link-P(O)(OR¹)₂ or a precursor such as [OH], [SH], [NH], Br etc, as described in Scheme **5,** to give the amines **41.2** and **41.3** respectively.

The reactions shown in Scheme **41** illustrate the preparation of the compounds **41.2** and **41.3** in which the substituent A is either the group link-P(O)(OR¹)₂ or a precursor such as [OH], [SH], [NH], Br etc. Scheme **42** depicts the conversion of **41.2** and **41.3** in which A is [OH], [SH], [NH], Br etc, into the phosphonate ester **6** and **7** in which X is sulfur. In this procedure **41.2** or **41.3** is converted, using the procedures described below, Schemes **47-99,** into the compound **6** and **7.**

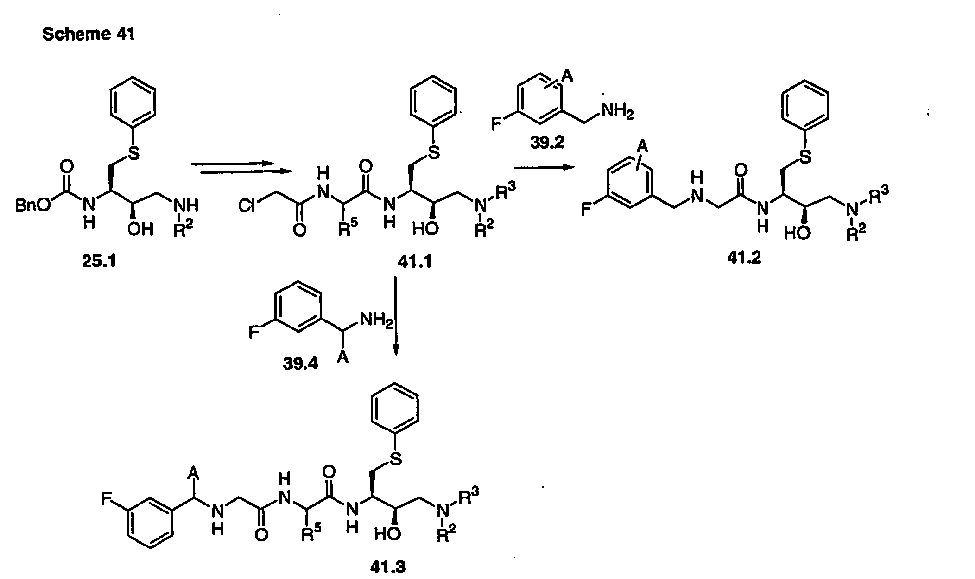

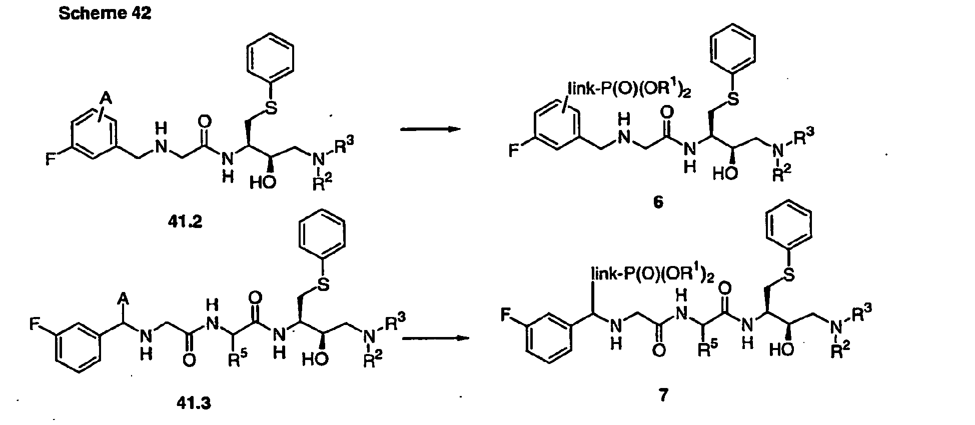

### Preparation of the phosphonate ester intermediates 8-10 in which X is a direct bond

Schemes **43-44** illustrate the preparation of the phosphonate esters **8-10** in which X is a direct bond. As shown in Scheme **43,** the amine **43.1** prepared from **10.1** or **21.2** is reacted with the acid **43.2, 43.4** or **43.6,** in which the substituent A is either the group link-P(O)(OR¹)₂ or a precursor such as [OH], [SH], [NH], Br etc, to afford the amide **43.3, 43.5** and **43.7** respectively. The reaction is performed under the same conditions as described above, Scheme **1** for the preparation of the amide **1.8.** Amine **43.1** is prepared from epoxide **10.1** using the conditions described in Scheme **1** except utilising **10.1** in place of **1.1.** Amine **43.1** is prepared from **21.2** according to the conditions described in Scheme **2** except utilizing **21.2** in place of **2.1.** The preparation of the acid **43.2** is described in Schemes **47-51,** acid **43.4** is described in Schemes **87-91,** and acid **43.6** is described in Schemes **52.55.**

The reactions shown in Scheme **43** illustrate the preparation of the compounds **43.3, 43.5** and **43.7** in which the substituent A is either the group link-P(O)(OR¹)₂ or a precursor such as [OH], [SH], [NH], Br etc. Scheme **44** depicts the conversion of these compounds **43.3, 43.5,** and **43.7** in which A is [OH], [SH], [NH], Br etc, into the phosphonate esters **8, 9** and **10** respectively, in which X is a direct bond. In this procedure, the amines **43.3, 43.5** and **43.7** are converted, using the procedures described below, Schemes **47-99,** into the compounds **8, 9,** and **10** respectively.

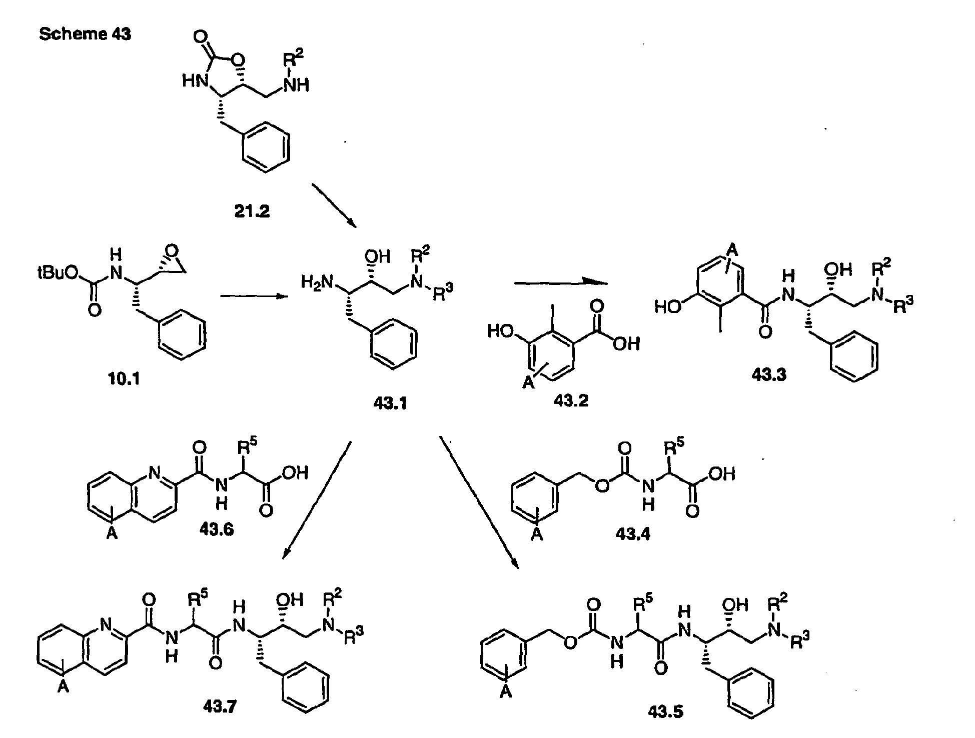

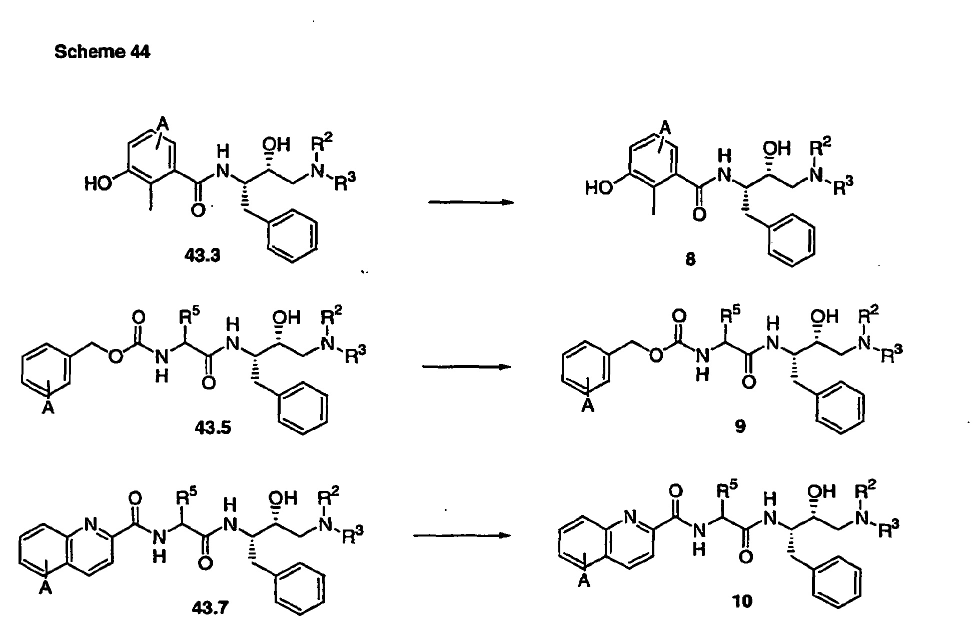

### Preparation of the phosphonate ester intermediates 8-10 in which X is a sulfur

The intermediate phosphonate esters **8-10,** in which the group A is attached to a sulfur linked aryl moiety, are prepared as shown in Schemes **45-46.** In Scheme **45,** epoxide **15.1** is prepared from mesylate **7.1** using the conditions described in Scheme **7** except incorporating thiophenol for thiol **7.2.** The epoxide **15.1** is then converted to amine **45.1** according to the conditions described in Scheme **7** for the preparation of **7.10** from **7.7.** Amine **45.1** is then treated with acids **43.2, 43.4** or **43.6,** in which substituent A is either the group link-P(O)(OR¹)₂ or a precursor such as [OH], [SH], [NH], Br etc, as described in Scheme **7,** to give the amides **45.2, 45.3,** and **45.4** respectively.

The reactions shown in Scheme **45** illustrate the preparation of the compounds **45.2, 45.3,** and **45.4** in which the substituent A is either the group link-P(O)(OR¹)₂ or a precursor such as [OH], [SH], [NH], Br etc. Scheme **46** depicts the conversion **45.2, 45.3,** and **45.4** in which A is [OH], [SH], [NH], Br etc, into the phosphonate ester **8, 9** and **10** respectively in which X is sulfur. In this procedure **45.2, 45.3,** and **45.4** is converted, using the procedures described below, Schemes **47-99,** into the compounds **8, 9** and **10** respectively.

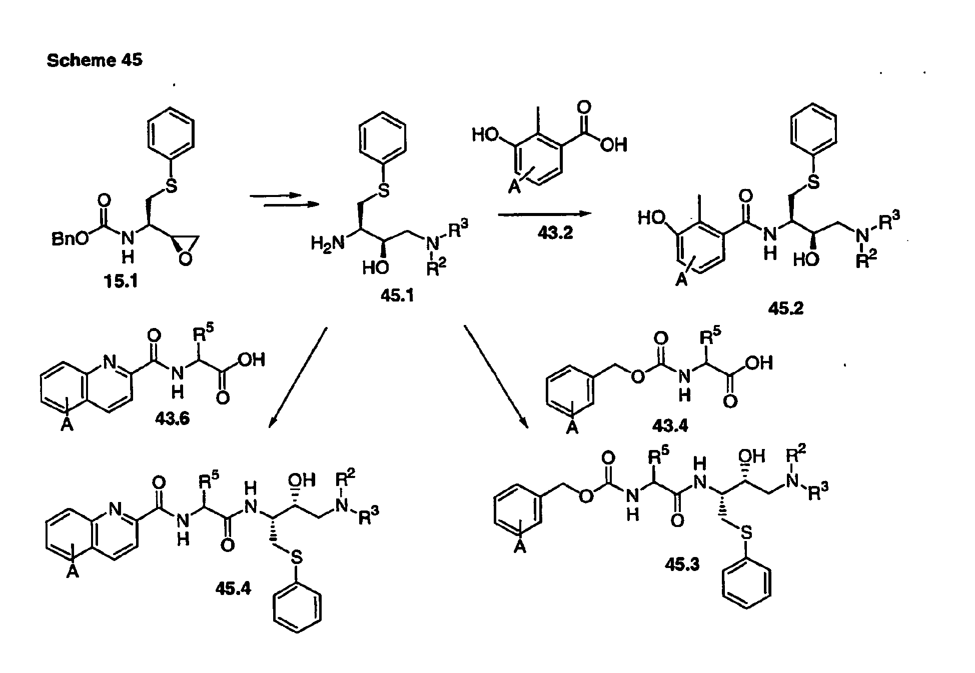

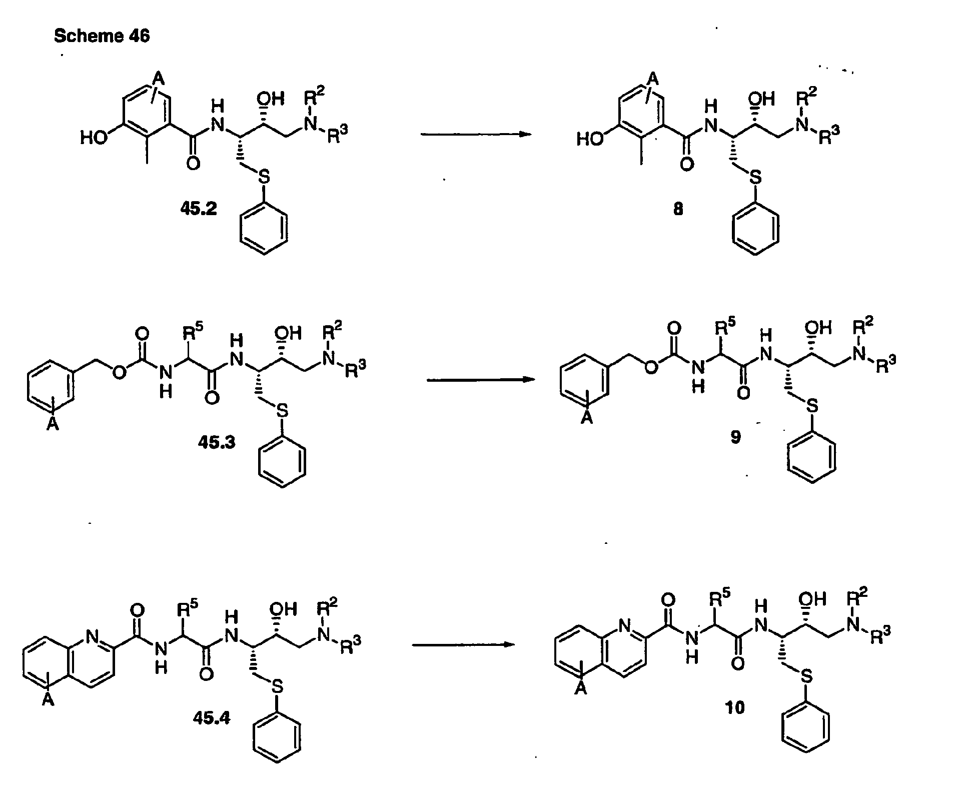

### Preparation of phosphonate-containing hydroxymethyl benzoic acids 43.2.

Schemes **47 - 51** illustrate methods for the preparation of phosphonate-containing hydroxymethyl benzoic acids **43.2** which are employed in the preparation of the phosphonate esters **8**.

Scheme **47** illustrates a method for the preparation of hydroxymethylbenzoic acid reactants in which the phosphonate moiety is attached directly to the phenyl ring. In this method, a suitably protected bromo hydroxy methyl benzoic acid **47.1** is subjected to halogen-methyl exchange to afford the organometallic intermediate **47.2.** This compound is reacted with a chlorodialkyl phosphite **47.3** to yield the phenylphosphonate ester **47.4,** which upon deprotection affords the carboxylic acid **47.5.**

For example, 4-bromo-3-hydroxy-2-methylbenzoic acid, **47.6,** prepared by bromination of 3-hydroxy-2-methylbenzoic acid, as described, for example, J. Am. Chem Soc., 55, 1676, 1933, is converted into the acid chloride, for example by reaction with thionyl chloride. The acid chloride is then reacted with 3-methyl-3-hydroxymethyloxetane **47.7,** as described in Protective Groups in Organic Synthesis, by T. W. Greene and P.G.M. Wuts, Wiley, 1991, pp. 268, to afford the ester **47.8.** This compound is treated with boron trifluoride at 0⁰ to effect rearrangement to the orthoester **47.9,** known as the OBO ester. This material is treated with a silylating reagent, for example tert-butyl chlorodimethylsilane, in the presence of a base such as imidazole, to yield the silyl ether **47.10.** Halogen-metal exchange is performed by the reaction of the substrate **47.10** with butyllithium, and the lithiated intermediate is then coupled with a chlorodialkyl phosphite **47.3,** to produce the phosphonate **47.11.** Deprotection, for example by treatment with 4-toluenesulfonic acid in aqueous pyridine, as described in Can. J. Chem, 61, 712, 1983, removes both the OBO ester and the silyl group, to produce the carboxylic acid **47.12.**
Using the above procedures, but employing, in place of the bromo compound **47.6,** different bromo compounds **47.1,** there are obtained the corresponding products **47.5.**

Scheme **48** illustrates the preparation of hydroxymethylbenzoic acid derivatives in which the phosphonate moiety is attached by means of a one-carbon link.
In this method, a suitably protected dimethyl hydroxybenzoic acid, **48.1,** is reacted with a brominating agent, so as to effect benzylic bromination. The product **48.2** is reacted with a sodium dialkyl phosphite, **48.3,** as described in J. Med. Chem., 1992, 35, 1371, to effect displacement of the benzylic bromide to afford the phosphonate **48.4.** Deprotection of the carboxyl function then yields the carboxylic acid **48.5.**
For example, 2,5-dimethyl-3-hydroxybenzoic acid, **48.6,** the preparation of which is described in Can. J. Chem., 1970, 48, 1346, is reacted with excess methoxymethyl chloride, as described in Protective Groups in Organic Synthesis, by T.W. Greene and P.G.M Wuts, Second Edition 1990, p.17, to afford the ether ester **48.7.** The reaction is performed in an inert solvent such as dichloromethane, in the presence of an organic base such as N-methylinoipholine or diisopropylethylamine. The product **48.7** is then reacted with a brominating agent, for example N-bromosuccinimide, in an inert solvent such as, for example, ethyl acetate, at reflux, to afford the bromomethyl product **48.8.** This compound is then reacted with a sodium dialkyl phosphite **48.3** in tetrahydrofuran, as described above, to afford the phosphonate **48.9.** Deprotection, for example by brief treatment with a trace of mineral acid in methanol, as described in J. Chem. Soc. Chem. Comm., 1974, 298, then yields the carboxylic acid **48.10.** Using the above procedures, but employing, in place of the methyl compound **48.6,** different methyl compounds **48.1,** there are obtained the corresponding products **48.5.**

Scheme **49** illustrates the preparation of phosphonate-containing hydroxymethylbenzoic acids in which the phosphonate group is attached by means of an oxygen or sulfur atom.
In this method, a suitably protected hydroxy- or mercapto-substituted hydroxy methyl benzoic acid **49.1** is reacted, under the conditions of the Mitsonobu reaction, with a dialkyl hydroxymethyl phosphonate **49.2,** to afford the coupled product **49.3,** which upon deprotection affords the carboxylic acid **49.4.**
For example, 3,6-dihydroxy-2-methylbenzoic acid, **49.5,** the preparation of which is described in Yakugaku Zasshi 1971, 91, 257, is converted into the diphenylmethyl ester **49.6,** by treatment with diphenyldiazomethane, as described in Protective Groups in Organic Synthesis, by T. W. Greene and P.G.M. Wuts, Wiley, 1991, pp. 253. The product is then reacted with one equivalent of a silylating reagent, such as, for example, tert butylchlorodimethylsilane, as described in Protective Groups in Organic Synthesis, by T.W. Greene and P.G.M Wuts, Wiley, Second Edition 1990, p 77, to afford the mono-silyl ether **49.7.** This compound is then reacted with a dialkyl hydroxymethylphosphonate **49.2,** under the conditions of the Mitsonobu reaction. The preparation of aromatic ethers by means of the Mitsonobu reaction is described, for example, in Comprehensive Organic Transformations, by R. C. Larock, VCH, 1989, p. 448, and in Advanced Organic Chemistry, Part B, by F.A. Carey and R. J. Sundberg, Plenum, 2001, p. 153-4. The phenol or thiophenol and the alcohol component are reacted together in an aprotic solvent such as, for example, tetrahydrofuran, in the presence of a dialkyl azodicarboxylate and a triarylphosphine, to afford the ether or thioether products. The procedure is also described in Org. React., 1992, 42, 335-656. The reaction affords the coupled product **49.8.** Deprotection, for example by treatment with trifluoroacetic acid at ambient temperature, as described in J. Chem. Soc., C, 1191, 1966, then affords the phenolic carboxylic acid **49.9.**
Using the above procedures, but employing, in place of the phenol **49.5,** different phenols or thiophenols **49.1,** there are obtained the corresponding products **49.4.**

Scheme **50** depicts the preparation of phosphonate esters attached to the hydroxymethylbenzoic acid moiety by means of unsaturated or saturated carbon chains.
In this method, a dialkyl alkenylphosphonate **50.2** is coupled, by means of a palladium catalyzed Heck reaction, with a suitably protected bromo substituted hydroxymethylbenzoic acid **50.1.** The coupling of aryl halides with olefins by means of the Heck reaction is described, for example, in Advanced Organic Chemistry, by F. A. Carey and R. J. Sundberg, Plenum, 2001, p. 503ff and in Acc. Chem. Res., 12, 146, 1979. The aryl bromide and the olefin are coupled in a polar solvent such as dimethylformamide or dioxan, in the presence of a palladium(0) catalyst such as tetrakis(triphenylphosphine)palladium(0) or a palladium(II) catalyst such as palladium(II) acetate, and optionally in the presence of a base such as triethylamine or potassium carbonate. The product **50.3** is deprotected to afford the phosphonate **50.4;** the latter compound is subjected to catalytic hydrogenation to afford the saturated carboxylic acid **50.5.**
For example, 5-bromo-3-hydroxy-2-methylbenzoic acid **50.6,** prepared as described in WO 9218490, is converted as described above, into the silyl ether OBO ester **50.7** as described above. This compound is coupled with, for example, a dialkyl 4-buten-1-ylphosphonate **50.8,** the preparation of which is described in J. Med. Chem., 1996, 39, 949, using the conditions described above to afford the product **50.9.** Deprotection, or hydrogenation/deprotection, of this compound, as described above, then affords respectively the unsaturated and saturated products **50.10** and **50.11.**
Using the above procedures, but employing, in place of the bromo compound **50.6,** different bromo compounds **50.1,** and/or different phosphonates **50.2,** there are obtained the corresponding products **50.4** and **50.5.**

Scheme **51** illustrates the preparation of phosphonate esters linked to the hydroxymethylbenzoic acid moiety by means of an aromatic ring.
In this method, a suitably protected bromo-substituted hydroxymethylbenzoic acid **51.1** is converted to the corresponding boronic acid **51.2,** by metallation with butyllithium and boronation, as described in J. Organomet. Chem, 1999, 581, 82. The product is subjected to a Suzuki coupling reaction with a dialkyl bromophenyl phosphonate **51.3.** The product **51.4** is then deprotected to afford the diaryl phosphonate product **51.5.**

For example, the silylated OBO ester **51.6,** prepared as described above, (Scheme **47**), from 5-bromo-3-hydroxybenzoic acid, the preparation of which is described in J. Labelled. Comp. Radiopharm., 1992, 31, 175, is converted into the boronic acid **51.7,** as described above. This material is coupled with a dialkyl 4-bromophenyl phosphonate **51.8,** prepared as described in J. Chem. Soc. Perkin Trans., 1977, 2, 789, using tetrakis(triphenylphosphine)palladium(0) as catalyst, in the presence of sodium bicarbonate, as described, for example, in Palladium reagents and catalysts J. Tsuji, Wiley 1995, p 218, to afford the diaryl phosphonate **51.9.** Deprotection, as described above, then affords the benzoic acid **51.10.**
Using the above procedures, but employing, in place of the bromo compound **51.6,** different bromo compounds **51.1,** and/or different phosphonates **51.3,** there are obtained the corresponding carboxylic acid products **51.5.**

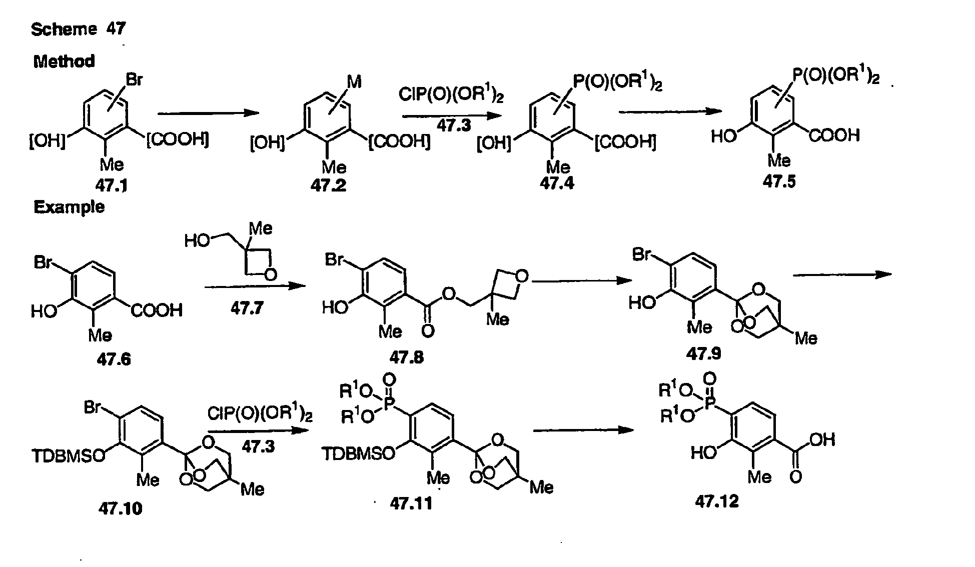

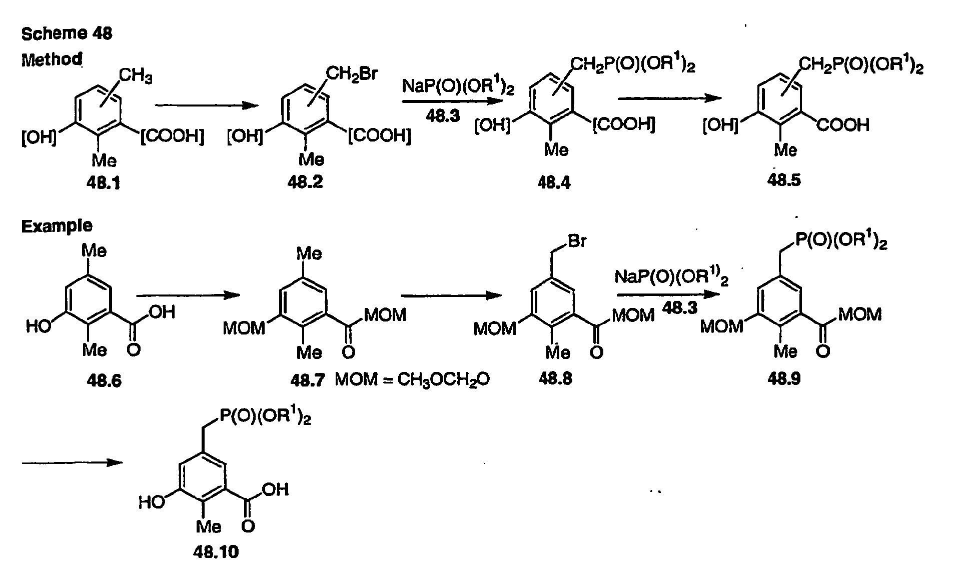

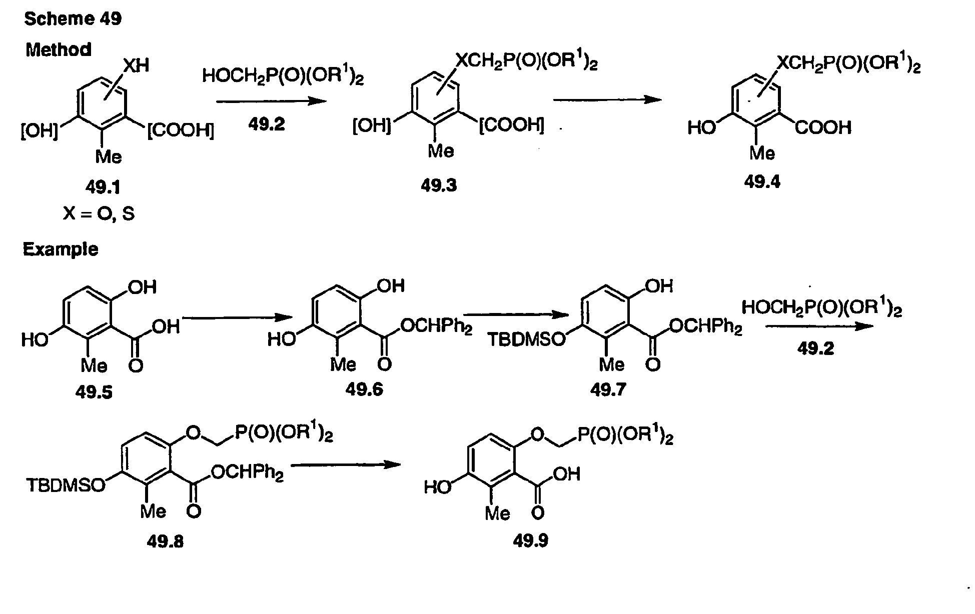

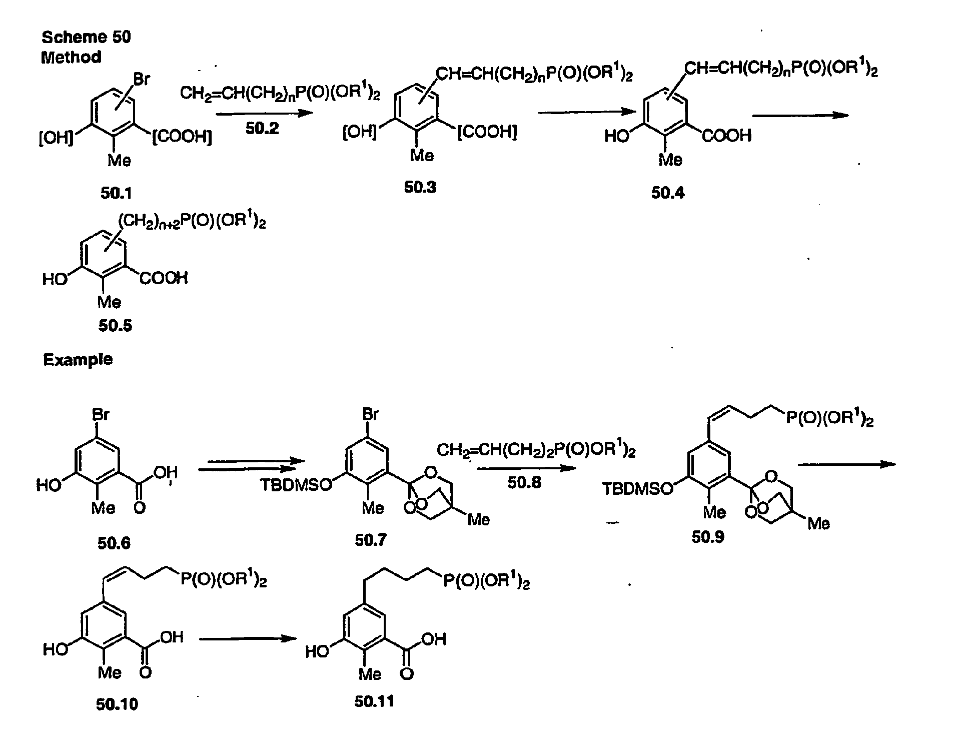

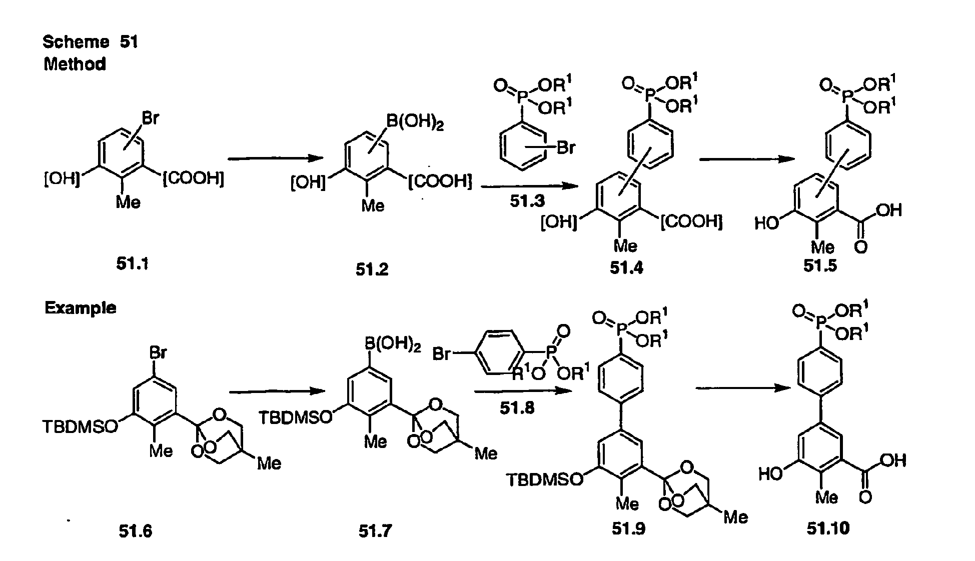

### Preparation of quinoline 2-carboxylic acids 43.6 incorporating phosphonate moieties.

The reaction sequences depicted in Schemes **43 - 46** for the preparation of the phosphonate esters 10 employ a quinoline-2-carboxylic acid reactant **43.6** in which the substituent A is either the group link-P(O)(OR¹)₂ or a precursor thereto, such as [OH], [SH] Br etc.
A number of suitably substituted quinoline-2-carboxylic acids are available commercially or are described in the chemical literature. For example, the preparations of 6-hydroxy, 6-amino and 6-bromoquinoline-2-carboxylic acids are described respectively in DE 3004370, J. Het. Chem., 1989, 26, 929 and J. Labelled Comp. Radiopharm., 1998, 41, 1103, and the preparation of 7-aminoquinoline-2-carboxylic acid is described in J. Am Chem. Soc., 1987, 109, 620. Suitably substituted quinoline-2-carboxylic acids can also be prepared by procedures known to those skilled in the art. The synthesis of variously substituted quinolines is described, for example, in Chemistry of Heterocyclic Compounds, VoL 32, G. Jones, ed., Wiley, 1977, p 93ff. Quinoline-2-carboxylic acids can be prepared by means of the Friedlander reaction, which is described in Chemistry of Heterocyclic Compounds, Vol. 4, R C. Elderfield, ed., Wiley, 1952, p. 204.

Scheme 52 illustrates the preparation of quinoline-2-carboxylic acids by means of the Friedlander reaction, and further transformations of the products obtained. In this reaction sequence, a substituted 2-aminobenzaldehyde **52.1** is reacted with an alkyl pyruvate ester **52.2,** in the presence of an organic or inorganic base, to afford the substituted quinoline-2-carboxylic ester **52.3.** Hydrolysis of the ester, for example by the use of aqueous base, then afford the corresponding carboxylic acid **52.4.** The carboxylic acid product **52.4** in which X is NH₂ can be further transformed into the corresponding compounds **52.6** in which Z is OH, SH or Br. The latter transformations are effected by means of a diazotization reaction. The conversion of aromatic amines into the corresponding phenols and bromides by means of a diazotization reaction is described respectively in Synthetic Organic Chemistry, R. B. Wagner, H. D. Zook, Wiley, 1953, pages 167 and 94; the conversion of amines into the corresponding thiols is described in Sulfur Lett., 2000, 24, 123. The amine is first converted into the diazonium salt by reaction with nitrous acid. The diazonium salt, preferably the diazonium tetrafluoborate, is then heated in aqueous solution, for example as described in Organic Functional Group Preparations, by S.R.Sandler and W. Karo, Academic Press, 1968, p. 83, to afford the corresponding phenol **52.6,** Y = OH. Alternatively, the diazonium salt is reacted in aqueous solution with cuprous bromide and lithium bromide, as described in Organic Functional Group Preparations, by S.R.Sandler and W. Karo, Academic Press, 1968, p. 138, to yield the corresponding bromo compound, **52.6,** Y = Br. Alternatively, the diazonium tetrafluoborate is reacted in acetonitrile solution with a sulfhydryl ion exchange resin, as described in Sulfur Lett., 2000, 24, 123, to afford the thiol **52.6,** Y = SH. Optionally, the diazotization reactions described above can be performed on the carboxylic esters 52.3 instead of the carboxylic acids **52.5.**
For example, 2,4-diaminobenzaldehyde **52.7** (Apin Chemicals) is reacted with one molar equivalent of methyl pyruvate **52.2** in methanol, in the presence of a base such as piperidine, to afford methyl-7-aminoquinoline-2-carboxylate **52.8.** Basic hydrolysis of the product, employing one molar equivalent of lithium hydroxide in aqueous methanol, then yields the carboxylic acid **52.9.** The amino-substituted carboxylic acid is then converted into the diazonium tetrafluoborate **52.10** by reaction with sodium nitrite and tetrafluoboric acid. The diazonium salt is heated in aqueous solution to afford the 7-hydroxyquinoline-2-carboxylic acid, **52.11,** Z = OH. Alternatively, the diazonium tetrafluoborate is heated in aqueous organic solution with one molar equivalent of cuprous bromide and lithium bromide, to afford 7-bromoquinoline-2-carboxylic acid **52.11,** Z = Br. Alternatively, the diazonium tetrafluoborate **52.10** is reacted in acetonitrile solution with the sulfhydryl form of an ion exchange resin, as described in Sulfur Lett., 2000, 24, 123, to prepare 7-mercaptoquinoline-2-carboxylic acid **52.11,** Z = SH.
Using the above procedures, but employing, in place of 2,4-diaminobenzaldehyde **52.7,** different aminobenzaldehydes **52.1,** the corresponding amino, hydroxy, bromo or mercapto-substituted quinoline-2-carboxylic acids **52.6** are obtained. The variously substituted quinoline carboxylic acids and esters can then be transformed, as described herein, (Schemes **53 - 55)** into phosphonate-containing derivatives.

Scheme **53** depicts the preparation of quinoline-2-carboxylic acids incorporating a phosphonate moiety attached to the quinoline ring by means of an oxygen or a sulfur atom. In this procedure, an amino-substituted quinoline-2-carboxylate ester **53.1** is transformed, via a diazotization procedure as described above (Scheme **52**) into the corresponding phenol or thiol **53.2.** The latter compound is then reacted with a dialkyl hydroxymethylphosphonate **53.3,** under the conditions of the Mitsonobu reaction, to afford the phosphonate ester **53.4.** The preparation of aromatic ethers by means of the Mitsonobu reaction is described, for example, in Comprehensive Organic Transformations, by R. C. Larock, VCH, 1989, p. 448, and in Advanced Organic Chemistry, Part B, by F.A. Carey and R. J. Sundberg, Plenum, 2001, p. 153-4. The phenol or thiophenol and the alcohol component are reacted together in an aprotic solvent such as, for example, tetrahydrofuran, in the presence of a dialkyl azodicarboxylate and a triarylphosphine, to afford the ether or thioether products 53.4. Basic hydrolysis of the ester group, for example employing one molar equivalent of lithium hydroxide in aqueous methanol, then yields the carboxylic acid **53.5.** The product is then coupled with a suitably protected aminoacid derivative **53.6** to afford the amide **53.7.** The reaction is performed under similar conditions to those described above, Scheme **1.** The ester protecting group is then removed to yield the carboxylic acid **53.8.**
For example, methyl 6-amino-2-quinoline carboxylate **53.9,** prepared as described in J. Het. Chem, 1989, 26, 929, is converted, by means of the diazotization procedure described above, into methyl 6-mercaptoquinoline-2-carboxylate **53.10.** This material is reacted with a dialkyl hydroxymethylphosphonate **53.11** (Aldrich) in the presence of diethyl azodicarboxylate and triphenylphosphine in tetrahydrofuran solution, to afford the thioether **53.12.** Basic hydrolysis then afford the carboxylic acid **53.13.** The latter compound is then converted, as described above, into the aminoacid derivative **53.16.**
Using the above procedures, but employing, in place of methyl 6-amino-2-quinoline carboxylate **53.9,** different aminoquinoline carboxylic esters **53.1,** and/or different dialkyl hydroxymethylphosphonates **53.3** the corresponding phosphonate ester products **53.8** are obtained.

Scheme **54** illustrates the preparation of quinoline-2-carboxylic acids incorporating phosphonate esters attached to the quinoline ring by means of a saturated or unsaturated carbon chain. In this reaction sequence, a bromo-substituted quinoline carboxylic ester **54.1** is coupled, by means of a palladium-catalyzed Heck reaction, with a dialkyl alkenylphosphonate **54.2.** The coupling of aryl halides with olefins by means of the Heck reaction is descnbed, for example, in Advanced Organic Chemistry, by F. A. Carey and R J. Sundberg, Plenum, 2001, p. 503ff. The aryl bromide and the olefin are coupled in a polar solvent such as dimethylformamide or dioxan, in the presence of a palladium(0) catalyst such as tetrakis(triphenylphosphine)palladium(0) or palladium(II) catalyst such as palladium(II) acetate, and optionally in the presence of a base such as triethylamine or potassium carbonate. Thus, Heck coupling of the bromo compound **54.1** and the olefin **54.2** affords the olefinic ester **54.3.** Hydrolysis, for example by reaction with lithium hydroxide in aqueous methanol, or by treatment with porcine liver esterase, then yields the carboxylic acid **54.4.** The latter compound is then transformed, as described above, into the homolog **54.5.** Optionally, the unsaturated carboxylic acid **54.4** can be reduced to afford the saturated analog **54.6.** The reduction reaction can be effected chemically, for example by the use of diimide or diborane, as described in Comprehensive Organic Transformations, by R. C. Larock, VCH, 1989, p. 5, or catalytically. The product **54.6** is then converted, as described above (Scheme **53)** into the aminoacid derivative **54.7.**
For example, methyl 7-bromoquinoline-2-carboxylate, **54.8,** prepared as described in J. Labelled Comp. Radiopharm., 1998, 41, 1103, is reacted in dimethylformamide at 60° with a dialkyl vinylphosphonate **54.9** (Aldrich) in the presence of 2 mol% of tetrakis(triphenylphosphine)palladium and triethylamine, to afford the coupled product **54.10** The product is then reacted with lithium hydroxide in aqueous tetrahydrofuran to produce the carboxylic acid **54.11.** The latter compound is reacted with diimide, prepared by basic hydrolysis of diethyl azodicarboxylate, as described in Angew. Chem. Int. Ed., 4, 271, 1965, to yield the saturated product **54.12.** The latter compound is then converted, as described above, into the aminoacid derivative **54.13.** The unsaturated product **54.11** is similarly converted into the analog **54.14.**
Using the above procedures, but employing, in place of methyl 6-bromo-2-quinolinecarboxylate **54.8,** different bromoquinoline carboxylic esters **54.1,** and/or different dialkyl alkenylphosphonates **54.2,** the corresponding phosphonate ester products **54.5** and **54.7** are obtained.

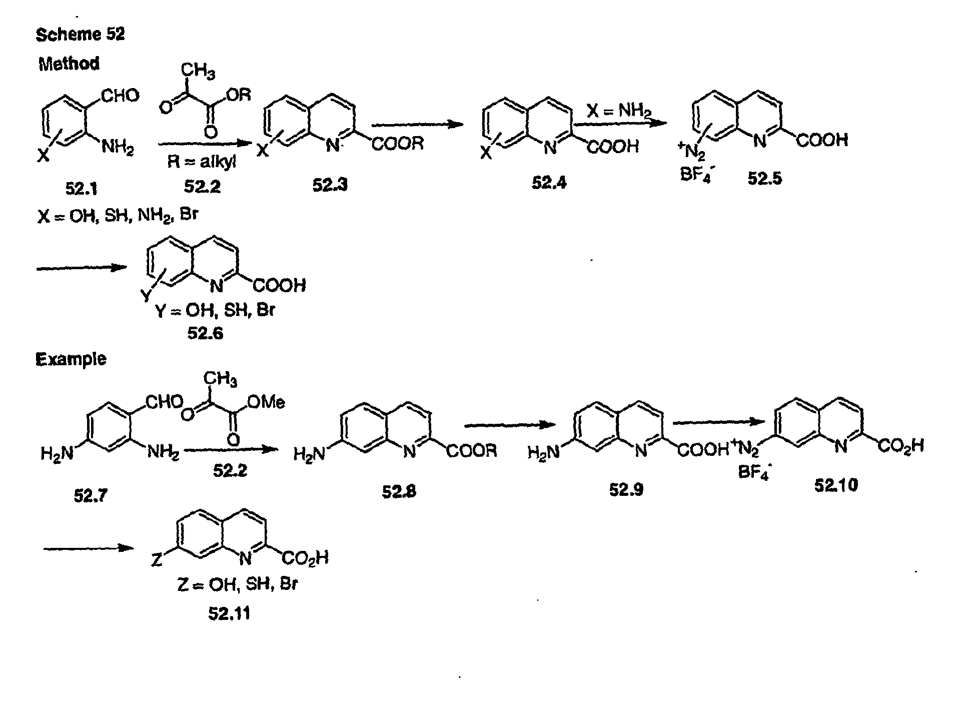

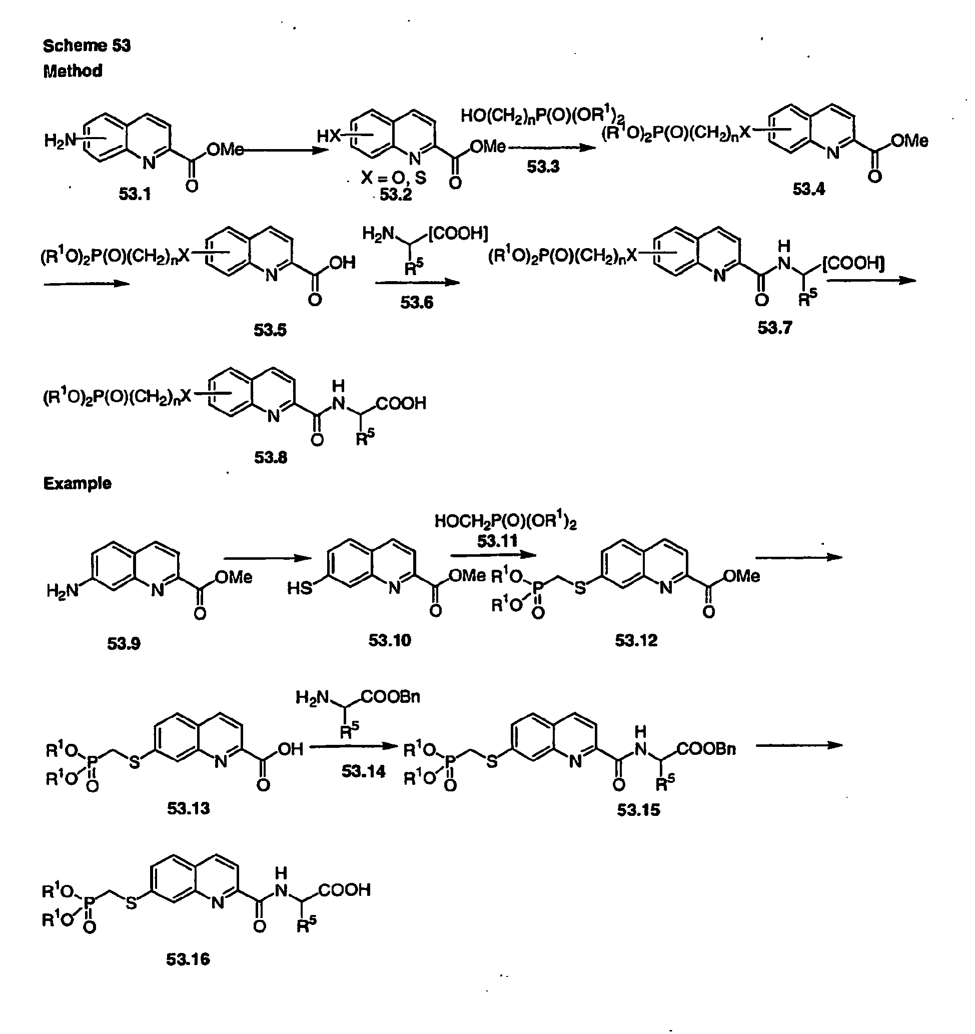

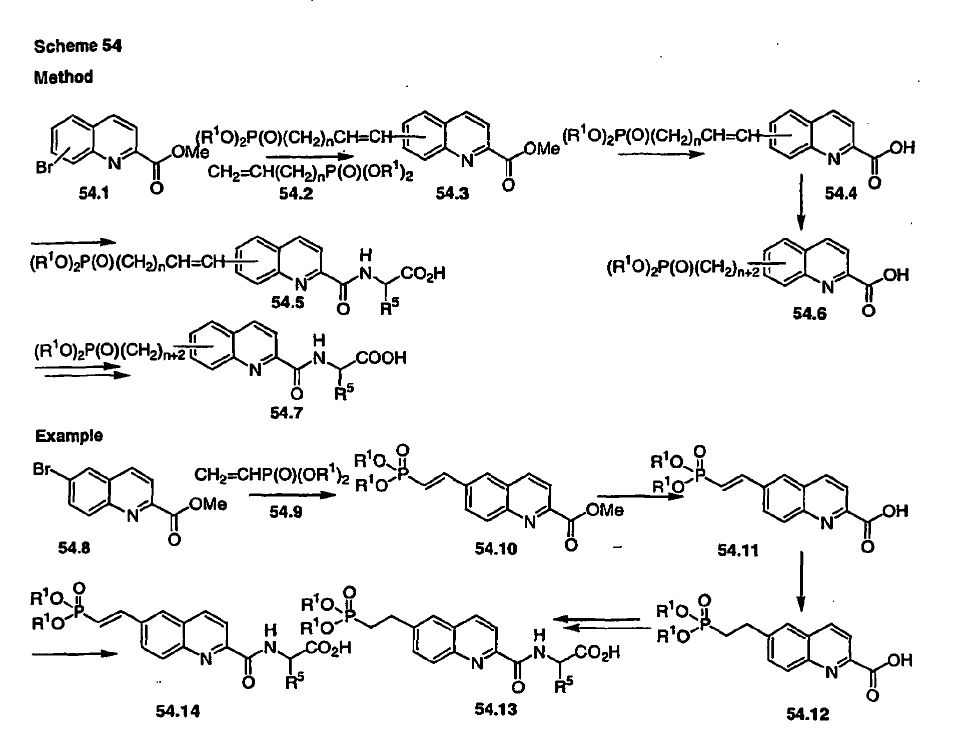

Scheme **55** depicts the preparation of quinoline-2-carboxylic acid derivatives **55.5** in which the phosphonate group is attached by means of a nitrogen atom and an alkylene chain. In this reaction sequence, a methyl aminoquinoline-2-carboxylate **55.1** is reacted with a phosphonate aldehyde **55.2** under reductive amination conditions, to afford the aminoalkyl product **55.3.**
The preparation of amines by means of reductive amination procedures is described, for example, in Comprehensive Organic Transformations, by R. C. Larock, VCH, p 421, and in Advanced Organic Chemistry, Part B, by F.A. Carey and R. J. Sundberg, Plenum, 2001, p 269. In this procedure, the amine component and the aldehyde or ketone component are reacted together in the presence of a reducing agent such as, for example, borane, sodium cyanoborohydride, sodium triacetoxyborohydride or diisobutylaluminum hydride, optionally in the presence of a Lewis acid, such as titanium tetraisopropoxide, as described in J. Org. Chem, 55, 2552, 1990. The ester product **55.3** is then hydrolyzed to yield the free carboxylic acid **55.4.** The latter compound is then converted, as described above, into the aminoacid derivative **55.5.**

For example, methyl 7-aminoquinoline-2-carboxylate **55.6,** prepared as described in J. Am. Chem. Soc., 1987, 109, 620, is reacted with a dialkyl formylmethylphosphonate **55.7** (Aurora) in methanol solution in the presence of sodium borohydride, to afford the alkylated product **55.8.** The ester is then hydrolyzed, as described above, to yield the carboxylic acid **55.9.** The latter compound is then converted, as described above, into the aminoacid derivative **55.10.** Using the above procedures, but employing, in place of the formylmethyl phosphonate **55.7,** different formylalkyl phosphonates **55.2,** and/or different aminoquinolines **55.1,** the corresponding products **55.5** are obtained.

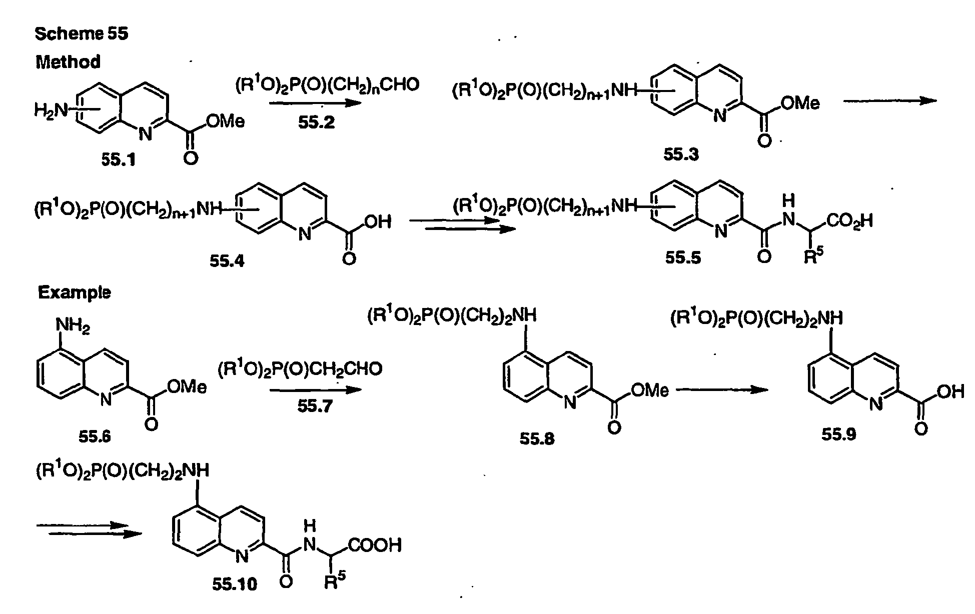

### Preparation of phenylalanine derivatives 1.1 incorporating phosphonate moieties.

Scheme **56** illustrates the conversion of variously substituted phenylalanine derivatives **56.1** into epoxides **1.1,** the incorporation of which into the compounds **1** is depicted in Schemes **1** and **3.**
A number of compounds **56.1** or **56.2,** for example those in which X is 2, 3, or 4-OH, or X is 4-NH₂ are commercially available. The preparations of different compounds **56.1** or **56.2** are described in the literature. For example, the preparation of compounds **56.1** or **56.2** in which X is 3-SH, 4-SH, 3-NH₂, 3-CH₂OH or 4-CH₂OH, are described respectively in WO0036136, J. Am Chem Soc., 1997, 119, 7173, Helv. Chim. Acta, 1978, 58, 1465, Acta Chem Scand., 1977, B31, 109 and Syn. Com., 1998, 28, 4279. Resolution of compounds **56.1,** if required, can be accomplished by conventional methods, for example as described in Recent Dev. Synth. Org. Chem., 1992, 2, 35.
The variously substituted aminoacids **56.2** are protected, for example by conversion to the BOC derivative **56.3,** by treatment with BOC anhydride, as described in J. Med. Chem, 1998, 41, 1034. The product **56.3** is then converted into the methyl ester **56.4,** for example by treatment with ethereal diazomethane. The substituent X in **56.4** is then transformed, using the methods described below, Schemes **57-59,** into the group A. The products **56.5** are then converted, via the intermediates **56.6 - 56.9,** into the epoxides **1.1.** The methyl ester **56.5** is first hydrolyzed, for example by treatment with one molar equivalent of aqueous methanolic lithium hydroxide, or by enzymatic hydrolysis, using, for example, porcine liver esterase, to afford the carboxylic acid **56.6.** The conversion of the carboxylic acid **56.6** into the epoxide **1.1,** for example using the sequence of reactions which is described in J. Med. Chem, 1994, 37, 1758, is then effected. The carboxylic acid is first converted into the acid chloride, for example by treatment with oxalyl chloride, or into a mixed anhydride, for example by treatment with isobutyl chloroformate, and the activated derivative thus obtained is reacted with ethereal diazomethane, to afford the diazoketone **56.7.** The diazoketone is converted into the chloroketone **56.8** by reaction with anhydrous hydrogen chloride, in a suitable solvent such as diethyl ether. The latter compound is then reduced, for example by the use of sodium borohydride, to produce a mixture of chlorohydrins from which the desired 2S, 3S diastereomer **56.9** is separated by chromatography. This material is reacted with ethanolic potassium hydroxide at ambient temperature to afford the epoxide **1.1.** Optionally, the above described series of reactions can be performed on the methyl ester **56.4,** so as to yield the epoxide **1.1** in which A is OH, SH, NH, Nalkyl or CH₂OH.
Methods for the transformation of the compounds **56.4**, in which X is a precursor group to the substituent link-P(O)(OR¹)₂, are illustrated in Schemes **57-59.**

Scheme **56a** illustrates the conversion of variously substituted phenylalanine derivatives **56a.1** into epoxides **3.1,** the incorporation of which into the compounds **1** is depicted in Schemes 3. Starting from the same reagents described above, Scheme **56,** the compound **56.2** is converted into the epoxide **56a.6** as described in J. Org. Chem 1996,61, 3635. The amino acid **56.2** is converted to the tribenzyl ester **56a.3** by treatment with benzyl bromide in ethanol in the presence of potassium carbonate. The substituent X in **56a.3** is then transformed, using the methods described below, Schemes **57-59,** into the group A, compound **56a.4.** These methods describe procedures in which the amine is BOC protected. However the same procedures are applicable to other amine protecting groups such as dibenzyl. The products **56a.4** are then converted, via the intermediates **56a.5** into the epoxides **3.1.** The ester **56a.4** is reduced with lithium aluminum hydride to the alcohol which is then oxidized to the aldehyde **56a.4** by treatment with pyridine sulfur trioxide in DMSO and triethylamine. The aldehyde **56a.4** is then converted to the epoxide **3.1** by treatment with chloromethylbromide and excess lithium in THF at -65 °C. A mixture of isomers are produced which are separated by chromatography.

Scheme **57** depicts the preparation of epoxides **57.4** incorporating a phosphonate group linked to the phenyl ring by means of a heteroatom O, S or N. In this procedure, the phenol, thiol, amine or carbinol **57.1** is reacted with a derivative of a dialkyl hydroxymethyl phosphonate **57.2.** The reaction is accomplished in the presence of a base, the nature of which depends on the nature of the substituent X. For example, if X is OH, SH, NH₂ or NHalkyl, an inorganic base such as cesium carbonate, or an organic base such as diazabicyclononene, can be employed. If X is CH₂OH, a base such as lithium hexamethyldisilylazide or the like can be employed. The condensation reaction affords the phosphonate-substituted ester **57.3,** which, employing the sequence of reactions shown in Scheme **56** or **56a,** is transformed into the epoxide **57.4.**
For example, 2-tert.-butoxycarbonylamino-3-(4-hydroxy-phenyl)-propionic acid methyl ester, **57.5** (Fluka) is reacted with a dialkyl trifluoromethanesulfonyloxy phosphonate **57.6,** prepared as described in Tet. Lett., 1986, 27, 1477, in the presence of cesium carbonate, in dimethylformamide at ca 60°, to afford the ether product **57.5.** The latter compound is then converted, using the sequence of reactions shown in Scheme **56,** into the epoxide **57.8.**
Using the above procedures, but employing different phenols, thiols, amines and carbinols **57.1** in place of **57.5,** and/or different phosphonates **57.2,** the corresponding products **57.4** are obtained.

Scheme **58** illustrates the preparation of a phosphonate moiety is attached to the phenylalanine scaffold by means of a heteroatom and a multi-carbon chain.
In this procedure, a substituted phenylalanine derivative **58.1** is reacted with a dialkyl bromoalkyl phosphonate **58.2** to afford the product **58.3.** The reaction is conducted in a polar organic solvent such as dimethylformamide or acetonitrile, in the presence of a suitable base such as sodium hydride or cesium carbonate. The product is then transformed, using the sequence of reactions shown in Scheme **56,** into the epoxide **58.4.**
For example, the protected aminoacid **58.5,** prepared as described above (Scheme **56)** from 3-mercaptophenylalanine, the preparation of which is described in WO 0036136, is reacted with a dialkyl 2-bromoethyl phosphonate **58.6,** prepared as described in Synthesis, 1994, 9, 909, in the presence of cesium carbonate, in dimethylformamide at ca 60°, to afford the thioether product **58.7.** The latter compound is then converted, using the sequence of reactions shown in Scheme **56,** into the epoxide **58.8.**
Using the above procedures, but employing different phenols, thiols, and amines **58.1** in place of **58.5,** and/or different phosphonates **58.2,** the corresponding products **58.4** are obtained.

Scheme **59** depicts the preparation of phosphonate-substituted phenylalanine derivatives in which the phosphonate moiety is attached by means of an alkylene chain incorporating a heteroatom
In this procedure, a protected hydroxymethyl-substituted phenylalanine **59.1** is converted into the halomethyl-substituted compound **59.2.** For example, the carbinol **59.1** is treated with triphenylphosphine and carbon tetrabromide, as described in J. Am. Chem. Soc., 108, 1035, 1986 to afford the product **59.2** in which Z is Br. The bromo compound is then reacted with a dialkyl terminally hetero-substituted alkylphosphonate **59.3.** The reaction is accomplished in the presence of a base, the nature of which depends on the nature of the substituent X. For example, if X is SH, NH₂ or NHalkyl, an inorganic base such as cesium carbonate, or an organic base such as diazabicyclononene, can be employed. If X is OH, a strong base such as lithium hexamethyldisilylazide or the like can be employed. The condensation reaction affords the phosphonate-substituted ester **59.4,** which, employing the sequence of reactions shown in Scheme **56,** is transformed into the epoxide **59.5.**
For example, the protected 4-hydroxymethyl-substituted phenylalanine derivative **59.6,** obtained from the 4-hydroxymethyl phenylalanine, the preparation of which is described in Syn. Comm, 1998, 28, 4279, is converted into the bromo derivative **59.7,** as described above.
The product is then reacted with a dialkyl 2-aminoethyl phosphonate **59.8,** the preparation of which is described in J. Org. Chem., 2000, 65, 676, in the presence of cesium carbonate in dimethylformamide at ambient temperature, to afford the amine product **59.9.** The latter compound is then converted, using the sequence of reactions shown in Scheme **56,** into the epoxide **59.10.**
Using the above procedures, but employing different carbinols **59.1** in place of **59.6,** and/or different phosphonates **59.3,** the corresponding products **59.5** are obtained.

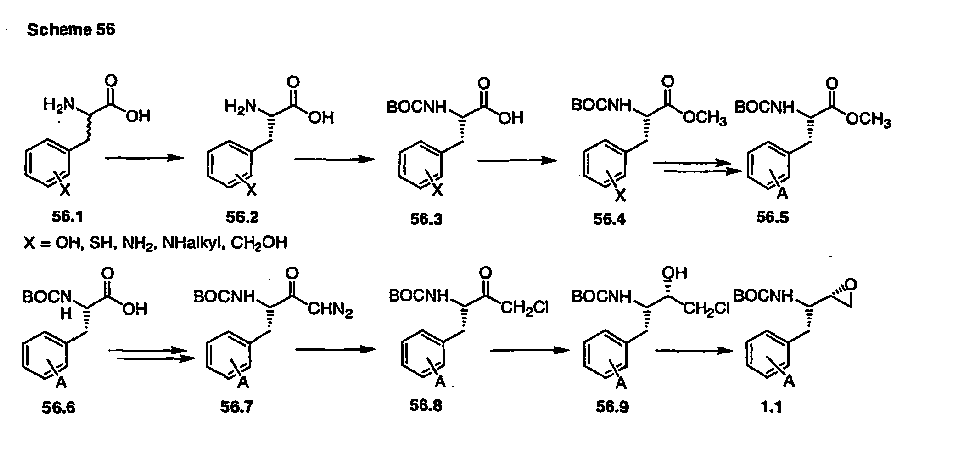

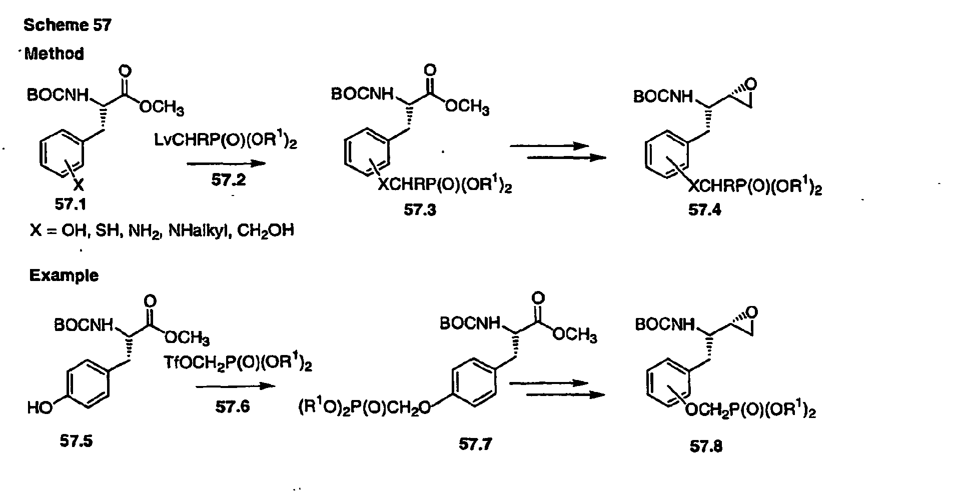

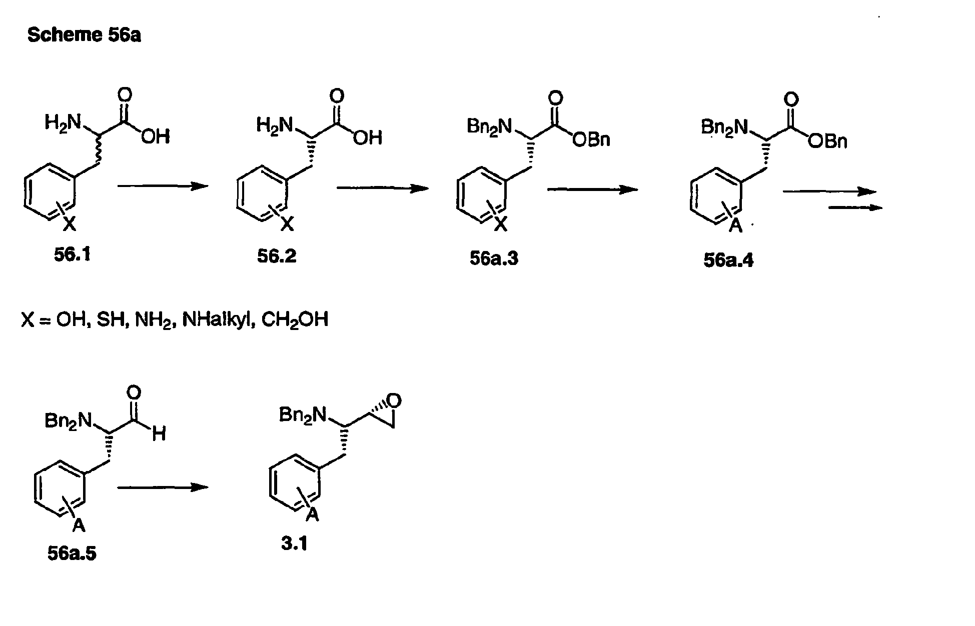

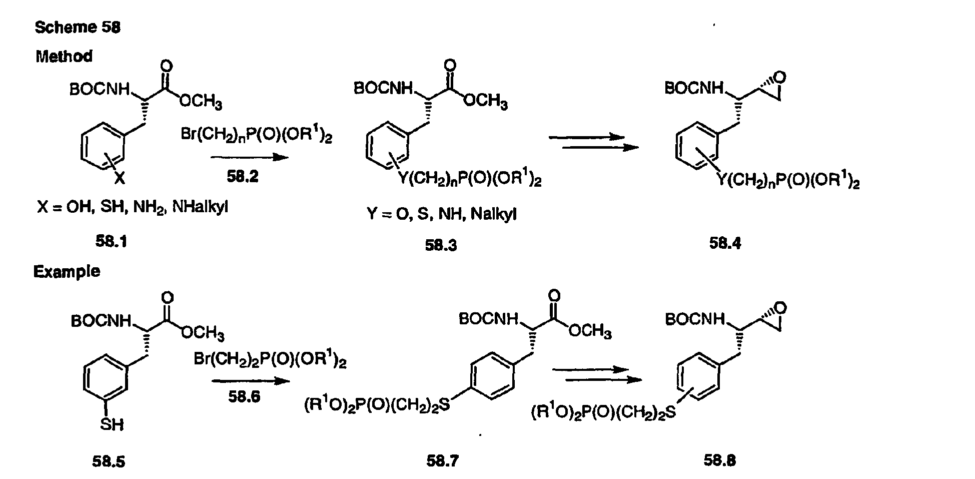

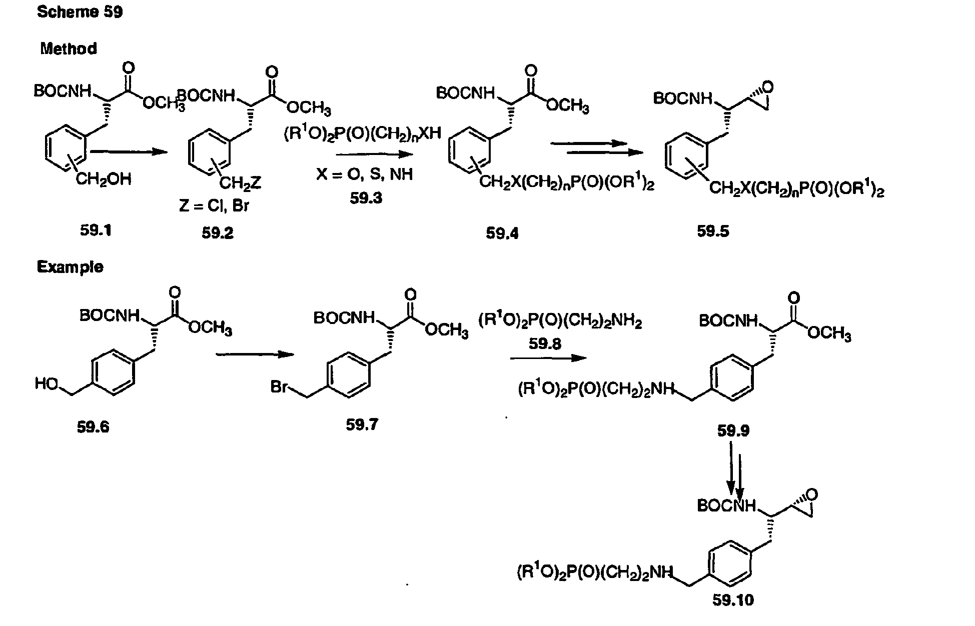

### Preparation of phenylalanine derivatives 2.1 incorporating phosphonate moieties or precursors thereto.

Scheme **60** illustrates the preparation of the hydroxymethyl oxazolidine derivative **2.1,** in which the substituent A is either the group link-P(O)(OR¹)₂ or a precursor thereto, such as [OH], [SH] Br etc. In this reaction sequence, the substituted phenylalanine **60.1,** in which A is as defined above, is transformed, via the intermediates **60.2 - 60.9,** into the hydroxymethyl product **2.1.** In this procedure, phenylalanine, or a substituted derivative thereof, **60.1,** is converted into the phthalimido derivative **60.2.** The conversion of amines into phthalimido derivatives is described, for example, in Protective Groups in Organic Synthesis, by T.W. Greene and P.G.M Wuts, Wiley, Second Edition 1990, p. 358. The amine is reacted with phthalic anhydride, 2-carboethoxybenzoyl chloride or N-carboethoxyphthalimide, optionally in the presence of a base such as triethylamine or sodium carbonate, to afford the protected amine **60.2.** Preferably, the aminoacid is reacted with phthalic anhydride in toluene at reflux, to yield the phthalimido product. The carboxylic acid is then transformed into an activated derivative such as the acid chloride **60.3,** in which X is Cl. The conversion of a carboxylic acid into the corresponding acid chloride can be effected by treatment of the carboxylic acid with a reagent such as, for example, thionyl chloride or oxalyl chloride in an inert organic solvent such as dichloromethane, optionally in the presence of a catalytic amount of a tertiary amide such as dimethylformamide. Preferably, the carboxylic acid is transformed into the acid chloride by reaction with oxalyl chloride and a catalytic amount of dimethylformamide, in toluene solution at ambient temperature, as described in WO 9607642. The acid chloride **60.3,** X = Cl, is then converted into the aldehyde **60.4** by means of a reduction reaction. This procedure is described, for example, in Comprehensive Organic Transformations, by R. C. Larock, VCH, 1989, p. 620. The transformation can be effected by means of catalytic hydrogenation, a procedure which is referred to as the Rosenmund reaction, or by chemical reduction employing, for example, sodium borohydride, lithium aluminum tri-tertiarybutoxy hydride or triethylsilane. Preferably, the acid chloride **60.3** X = Cl, is hydrogenated in toluene solution over a 5% palladium on carbon catalyst, in the presence of butylene oxide, as described in WO 9607642,- to afford the aldehyde **60.4.** The aldehyde **60.4** is then transformed into the cyanohydrin derivative **60.5.** The conversion of aldehydes into cyanohydrins is described in Protective Groups in Organic Synthesis, by T.W. Greene and P.G.M Wuts, Wiley, Second Edition 1990, p. 211. For example, the aldehyde **60.4** is converted into the cyanohydrin **60.5** by reaction with trimethylsilyl cyanide in an inert solvent such as dichloromethane, followed by treatment with an organic acid such as citric acid, as described in WO 9607642, or by alternative methods described therein. The cyanohydrin is then subjected to acidic hydrolysis, to effect conversion of the cyano group into the corresponding carboxy group, with concomitant hydrolysis of the phthalimido substituent to afford the aminoacid **60.6** The hydrolysis reactions are effected by the use of aqueous mineral acid. For example, the substrate **60.5** is reacted with aqueous hydrochloric acid at reflux, as described in WO 9607642, to afford the carboxylic acid product **60.6.** The aminoacid is then converted into a carbamate, for example the ethyl carbamate **60.7.** The conversion of amines into carbamates is described in Protective Groups in Organic Synthesis, by T.W. Greene and P.G.M Wuts, Wiley, Second Edition 1990, p. 317. The amine is reacted with a chloroformate, for example ethyl chloroformate, in the presence of a base such as potassium carbonate, to afford the carbamate **60.7.** For example, the aminoacid **60.6** is reacted, in aqueous solution, with ethyl chloroformate and sufficient aqueous sodium hydroxide to maintain a neutral pH, as described in WO 9607642, to afford the carbamate **60.7.** The latter compound is then transformed into the oxazolidinone **60.8,** for example by treatment with aqueous sodium hydroxide at ambient temperature, as described in WO 9607642. The resultant carboxylic acid is transformed into the methyl ester **60.9** by means of a conventional esterification reaction. The conversion of carboxylic acids into esters is described for example, in Comprehensive Organic Transformations, by R. C. Larock, VCH, 1989, p. 966. The conversion can be effected by means of an acid-catalyzed reaction between the carboxylic acid and an alcohol, or by means of a base-catalyzed reaction between the carboxylic acid and an alkyl halide, for example an alkyl bromide. For example, the carboxylic acid **60.8** is converted into the methyl ester **60.9** by treatment with methanol at reflux temperature, in the presence of a catalytic amount of sulfuric acid, as described in WO 9607642. The carbomethoxyl group present in the compound **60.9** is then reduced to yield the corresponding carbinol **2.1.** The reduction of carboxylic esters to the carbinols is described in Comprehensive Organic Transformations, by R. C. Larock, VCH, 1989, p. 550. The transformation can be effected by the use of reducing agents such as borane-dimethylsulfide, lithium borohydride, diisobutyl aluminum hydride, lithium aluminum hydride and the like. For example, the ester **60.9** is reduced to the carbinol **2.1** by reaction with sodium borohydride in ethanol at ambient temperature, as described in WO 9607642.

The conversion of the substituent A into the group link-P(O)(OR¹)₂ may be effected at any convenient step in the reaction sequence, or after the reactant **2.1** has been incorporated into the intermediates **1.** Specific examples of the preparation of the hydroxymethyl oxazolidinone reactant **2.1** are shown below, (Schemes **61-62)**

Scheme **61** depicts the preparation of hydroxymethyloxazolidinones **61.9** in which the phosphonate ester moiety is attached directly to the phenyl ring. In this procedure, a bromo-substituted phenylalanine **61.1** is converted, using the series of reactions illustrated in Scheme **60,** into the bromophenyloxazolidinone **61.2.** The bromophenyl compound is then coupled, in the presence of a palladium (0) catalyst, with a dialkyl phosphite **61.3,** to afford the phosphonate product **61.4.** The reaction between aryl bromide and dialkyl phosphites to yield aryl phosphonates is described in Synthesis, 56, 1981, and in J. Med. Chem., 1992, 35, 1371. The reaction is conducted in an inert solvent such as toluene or xylene, at about 100°, in the presence of a palladium(0) catalyst such as tetrakis(triphenylphosphine)palladium and a tertiary organic base such as triethylamine. The carbomethoxy substituent in the resultant phosphonate ester **61.4** is then reduced with sodium borohydride to the corresponding hydroxymethyl derivative **61.5,** using the procedure described above (Scheme **60)**
For example, 3-bromophenylalanine **61.6,** prepared as described in Pept. Res., 1990, 3, 176, is converted, using the sequence of reactions shown in Scheme **60,** into 4-(3-bromo-benzyl)-2-oxo-oxazolidine-5-carboxylic acid methyl ester **61.7.** This compound is then coupled with a dialkyl phosphite **61.3,** in toluene solution at reflux, in the presence of a catalytic amount of tetrakis(triphenylphosphine)palladium(0) and triethylamine, to afford the phosphonate ester **61.8.** The carbomethoxy substituent is then reduced with sodium borohydride, as described above, to afford the hydroxymethyl product **61.9.**
Using the above procedures, but employing, in place of 3-bromophenylalanine **61.6** different bromophenylalanines **61.1.** and/or different dialkyl phosphites **61.3,** the corresponding products **61.5** are obtained.

Scheme **62** illustrates the preparation of phosphonate-containing hydroxymethyl oxazolidinones **62.9** and **62.12** in which the phosphonate group is attached by means of a heteroatom and a carbon chain. In this sequence of reactions, a hydroxy or thio-substituted phenylalanine **62.1** is converted into the benzyl ester **62.2** by means of a conventional acid catalyzed esterification reaction. The hydroxyl or mercapto group is then protected. The protection of phenyl hydroxyl and thiol groups are described, respectively, in Protective Groups in Organic Synthesis, by T.W. Greene and P.G.M Wuts, Wiley, Second Edition 1990, p. 10, and p. 277. For example, hydroxyl and thiol substituents can be protected as trialkylsilyloxy groups. Trialkylsilyl groups are introduced by the reaction of the phenol or thiophenol with a chlorotrialkylsilane and a base such as imidazole, for example as described in Protective Groups in Organic Synthesis, by T.W. Greene and P.G.M Wuts, Wiley, Second Edition 1990, p. 10, p. 68-86. Alternatively, thiol substituents can be protected by conversion to tert-butyl or adamantyl thioethers, or 4-methoxybenzyl thioethers, prepared by the reaction between the thiol and 4-methoxybenzyl chloride in the presence of ammonium hydroxide, as described in Bull. Chem. Soc. Jpn., 37, 433, 1974. The protected ester **62.3** is then reacted with phthalic anhydride, as described above (Scheme **60)** to afford the phthalimide **62.4.** The benzyl ester is then removed, for example by catalytic hydrogenation or by treatment with aqueous base, to afford the carboxylic acid **62.5.** This compound is transformed, by means of the series of reactions shown in Scheme **60,** into the carbomethoxy oxazolidinone **62.6,** using in each step the same conditions as are described above (Scheme **60).** The protected OH or SH group is then deprotected. Deprotection of phenols and thiophenols is described in Protective Groups in Organic Synthesis, by T.W. Greene and P.G.M Wuts, Wiley, Second Edition 1990, p. For example, trialkylsilyl ethers or thioethers can be deprotected by treatment with a tetraalkylammonium fluoride in an inert solvent such as tetrahydrofuran, as described in J. Am Chem. Soc., 94, 6190, 1972. Tert-butyl or adamantyl thioethers can be converted into the corresponding thiols by treatment with mercuric trifluoroacetate in aqueous acetic acid at ambient temperatures, as described in Chem. Pharm. Bull., 26, 1576, 1978. The resultant phenol or thiol **62.7** is then reacted with a hydroxyalkyl phosphonate **62.20** under the conditions of the Mitsonobu reaction, as described above (Scheme **49**), to afford the ether or thioether **62.8.** The latter compound is then reduced with sodium borohydride, as described above (Scheme **60)** to afford the hydroxymethyl analog **62.9.**
Alternatively, the phenol or thiophenol **62.7** is reacted with a dialkyl bromoalkyl phosphonate **62.10** to afford the alkylation product **62.11.** The alkylation reaction is performed in a polar organic solvent such as dimethylformamide, acetonitrile and the like, optionally in the presence of potassium iodide, and in the presence of an inorganic base such as potassium or cesium carbonate, or an organic base such as diazabicyclononene or dimethylaminopyridine. The ether or thioether product is then reduced with sodium borohydride to afford the hydroxymethyl compound **62.12.**

For example, 3-hydroxyphenylalanine **62.13** (Fluka) is converted in to the benzyl ester **62.14** by means of a conventional acid-catalyzed esterification reaction. The ester is then reacted with tert-butylchlorodimethylsilane and imidazole in dimethylformamide, to afford the silyl ether **62.15.** The protected ether is then reacted with phthalic anhydride, as described above (Scheme **60)** to yield the phthalimido-protected compound **62.16.** Basic hydrolysis, for example by reaction with lithium hydroxide in aqueous methanol, then affords the carboxylic acid **62.17.** This compound is then transformed, by means of the series of reactions shown in Scheme **60,** into the carbomethoxy-substituted oxazolidinone **62.18.** The silyl protecting group is then removed by treatment with tetrabutylammonium fluoride in tetrahydrofuran at ambient temperature, to produce the phenol **62.19.** The latter compound is reacted with a dialkyl hydroxymethyl phosphonate **62.20** diethylazodicarboxylate and triphenylphosphine, by means of the Mitsonobu reaction. The preparation of aromatic ethers by means of the Mitsonobu reaction is described, for example, in Comprehensive Organic Transformations, by R C. Larock, VCH, 1989, p. 448, and in Advanced Organic Chemistry, Part B, by F.A. Carey and R. J. Sundberg, Plenum, 2001, p. 153-4 and in Org. React., 1992, 42, 335. The phenol or thiophenol and the alcohol component are reacted together in an aprotic solvent such as, for example, tetrahydrofuran, in the presence of a dialkyl azodicarboxylate and a triarylphosphine, to afford the ether or thioether products. The procedure is also described in Org. React., 1992, 42, 335-656. The reaction yields the phenolic ether **62.21.** The carbomethoxy group is then reduced by reaction with sodium borohydride, as described above, to afford the carbinol **62.22.**
Using the above procedures, but employing, in place of 3-hydroxyphenylalanine **62.13,** different hydroxy or mercapto-substituted phenylalanines **62.1,** and/or different dialkyl hydroxyalkyl phosphonates **62.20,** the corresponding products **62.9** are obtained.
As a further example of the methods illustrated in Scheme **62,** 4-mercaptophenylalanine **62.23,** prepared as described in J. Am. Chem. Soc., 1997, 119, 7173, is converted into the benzyl ester **62.24** by means of a conventional acid-catalyzed esterification reaction. The mercapto group is then protected by conversion to the S-adamantyl group, by reaction with 1-adamantanol and trifluoroacetic acid at ambient temperature as described in Chem. Pharm. Bull., 26, 1576, 1978. The amino group is then converted into the phthalimido group as described above, and the ester moiety is hydrolyzed with aqueous base to afford the carboxylic acid **62.27.** The latter compound is then transformed, by means of the series of reactions shown in Scheme **60,** into the carbomethoxy oxazolidinone **62.28.** The adamantyl protecting group is then removed by treatment of the thioether **62.28** with mercuric acetate in trifluoroacetic acid at 0°, as described in Chem. Pharm Bull., 26, 1576, 1978, to produce the thiol **62.29.** The thiol is then reacted with one molar equivalent of a dialkyl bromoethylphosphonate **62.30,** (Aldrich) and cesium carbonate in dimethylformamide at 70°, to afford the thioether product **62.31.** The carbomethoxy group is then reduced with sodium borohydride, as described above, to prepare the carbinol **62.32.**
Using the above procedures, but employing, in place of 4-mercaptophenylalanine **62.23,** different hydroxy or mercapto-substituted phenylalanines **62.1,** and/or different dialkyl bromoalkyl phosphonates **62.10,** the corresponding products **62.12** are obtained.

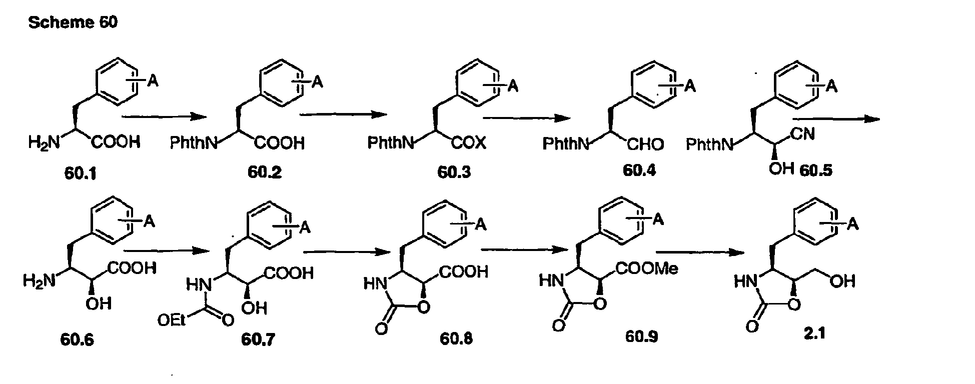

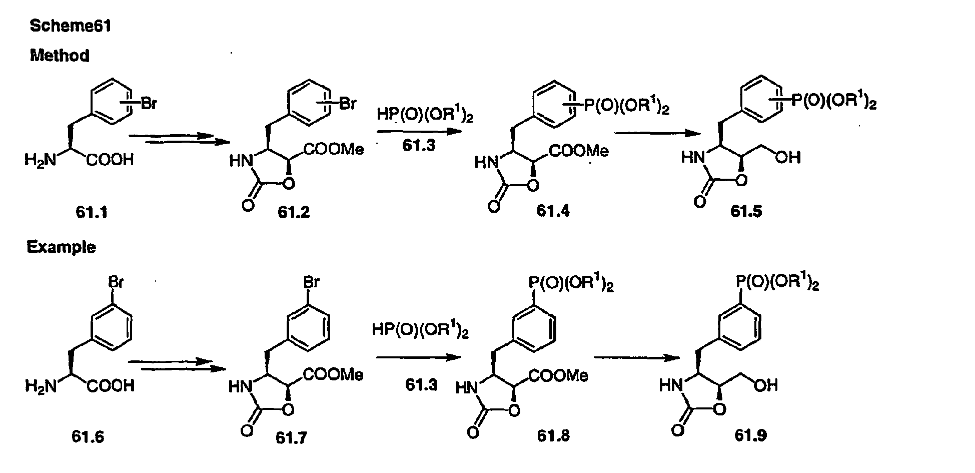

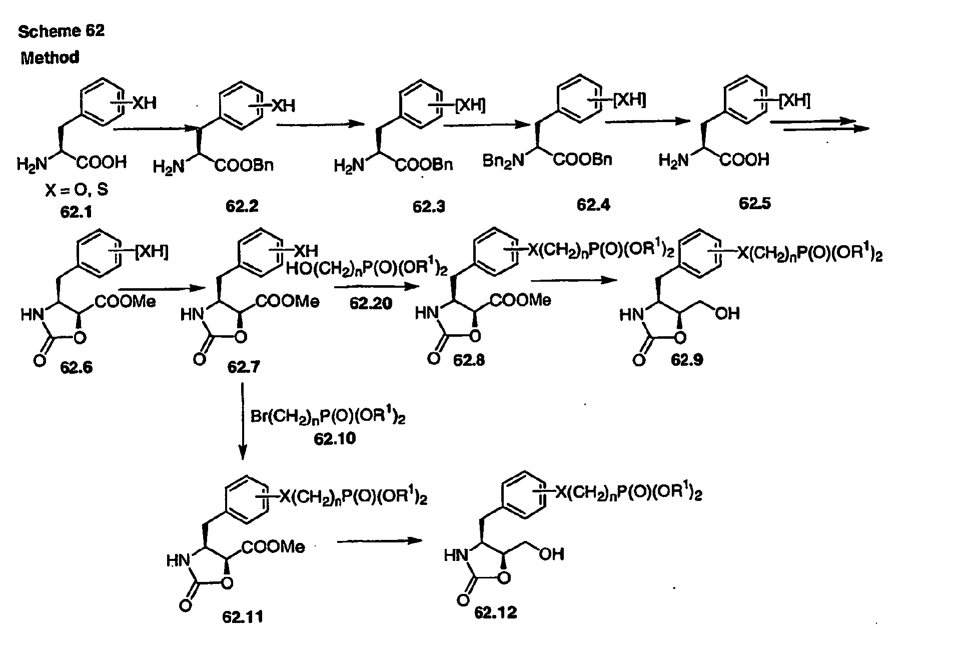

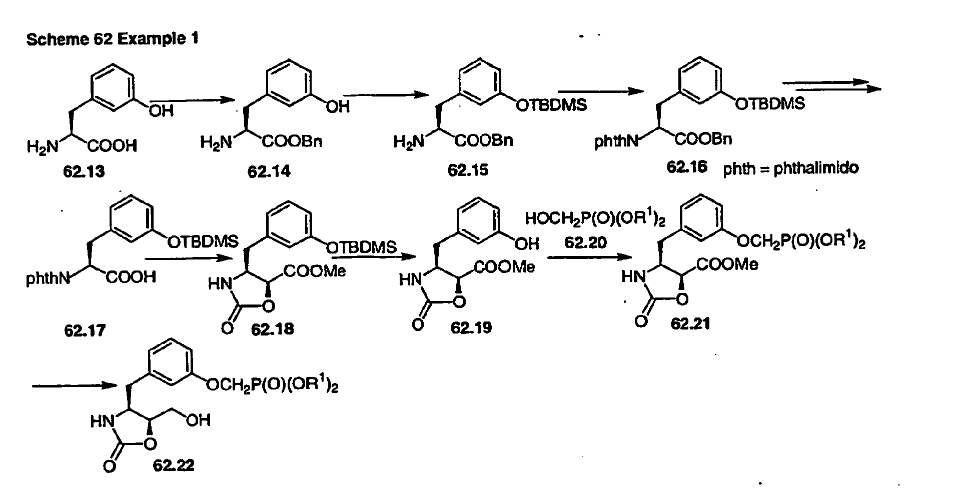

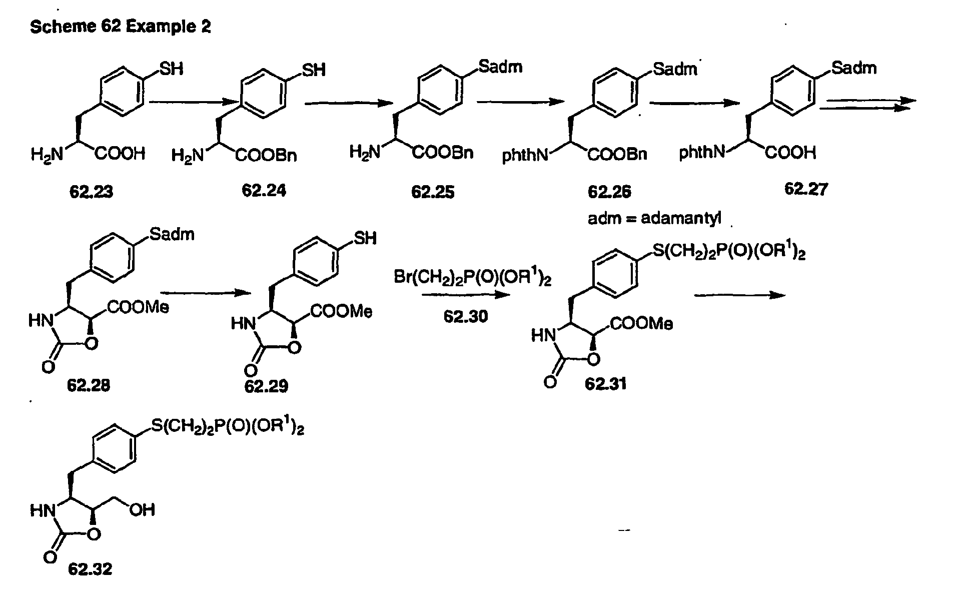

### Preparation of the phosphonate-containing thiophenol derivatives 7.2.

Schemes **63 - 83** describe the preparation of phosphonate-containing thiophenol derivatives **7.2** which are employed as described above (Schemes **7 - 9**) in the preparation of the phosphonate ester intermediates **1** in which X is sulfur.

Scheme **63** depicts the preparation of thiophenol derivatives in which the phosphonate moiety is attached directly to the phenyl ring. In this procedure, a halo-substituted thiophenol **63.1** is protected to afford the product **63.2.** The protection of phenyl thiol groups is described in Protective Groups in Organic Synthesis, by T.W. Greene and P.G.M Wuts, Wiley, Second Edition 1990, p. 277. For example, thiol substituents can be protected as trialkylsilyloxy groups. Trialkylsilyl groups are introduced by the reaction of the thiophenol with a chlorotrialkylsilane and a base such as imidazole, for example as described in Protective Groups in Organic Synthesis, by T.W. Greene and P.G.M Wuts, Wiley, Second Edition 1990, p. 10, p. 68-86. Alternatively, thiol substituents can be protected by conversion to tert-butyl or adamantyl thioethers, or 4-methoxybenzyl thioethers, prepared by the reaction between the thiol and 4-methoxybenzyl chloride in the presence of ammonium hydroxide, as described in Bull. Chem. Soc. Jpn., 37, 433, 1974. The product is then coupled, in the presence of triethylamine and tetrakis(triphenylphosphine)palladium(0), as described in J. Med. Chem., 35, 1371, 1992, with a dialkyl phosphite **63.3,** to afford the phosphonate ester **63.4.** The thiol protecting group is then removed, as described above, to afford the thiol **63.5.**
For example, 3-bromothiophenol **63.6** is converted into the 9-fluorenylmethyl (Fm) derivative **63.7** by reaction with 9-fluorenylmethyl chloride and diisopropylethylamine in dimethylformamide, as described in Int. J. Pept. Protein Res., 20, 434, 1982. The product is then reacted with a dialkyl phosphite **63.3,** as described above, to afford the phosphonate ester **63.8.** The Fm protecting group is then removed by treatment of the product with piperidine in dimethylformamide at ambient temperature, as described in J. Chem. Soc., Chem. Comm., 1501, 1986, to give the thiol **63.9.**
Using the above procedures, but employing, in place of 3-bromothiophenol **63.6,** different thiophenols 63.1, and/or different dialkyl phosphites **63.3,** the corresponding products **63.5** are obtained.

Scheme **64** illustrates an alternative method for obtaining thiophenols with a directly attached phosphonate group. In this procedure, a suitably protected halo-substituted thiophenol **64.2** is metallated, for example by reaction with magnesium or by transmetallation with an alkyllithium reagent, to afford the metallated derivative **64.3.** The latter compound is reacted with a halodialkyl phosphite **64.4** to afford the product **64.5;** deprotection then affords the thiophenol **64.6**
For example, 4-bromothiophenol **64.7** is converted into the S-triphenylmethyl (trityl) derivative **64.8,** as described in Protective Groups in Organic Synthesis, by T. W. Greene and P.G.M. Wuts, Wiley, 1991, pp. 287. The product is converted into the lithium derivative **64.9** by reaction with butyllithium in an ethereal solvent at low temperature, and the resulting lithio compound is reacted with a dialkyl chlorophosphite **64.10** to afford the phosphonate **64.11.** Removal of the trityl group, for example by treatment with dilute hydrochloric acid in acetic acid, as described in J. Org. Chem., 31, 1118, 1966, then affords the thiol **64.12.**
Using the above procedures, but employing, in place of the bromo compound **64.7,** different halo compounds **64.1,** and/or different halo dialkyl phosphites **64.4,** there are obtained the corresponding thiols **64.6.**

Scheme **65** illustrates the preparation of phosphonate-substituted thiophenols in which the phosphonate group is attached by means of a one-carbon link. In this procedure, a suitably protected methyl-substituted thiophenol **65.1** is subjected to free-radical bromination to afford a bromomethyl product **65.2.** This compound is reacted with a sodium dialkyl phosphite **65.3** or a trialkyl phosphite, to give the displacement or rearrangement product **65.4,** which upon deprotection affords the thiophenol **65.5.**
For example, 2-methylthiophenol **65.6** is protected by conversion to the benzoyl derivative **65.7,** as described in Protective Groups in Organic Synthesis, by T. W. Greene and P.G.M. Wuts, Wiley, 1991, pp. 298. The product is reacted with N-bromosuccinimide in ethyl acetate to yield the bromomethyl product **65.8.** This material is reacted with a sodium dialkyl phosphite **65.3,** as described in J. Med. Chem., 35, 1371, 1992, to afford the product **65.9.** Alternatively, the bromomethyl compound **65.8** is converted into the phosphonate **65.9** by means of the Arbuzov reaction, for example as described in Handb. Organophosphorus Chem, 1992, 115. In this procedure, the bromomethyl compound **65.8** is heated with a trialkyl phosphate P(OR¹)₃ at ca. 100° to produce the phosphonate **65.9.** Deprotection of the phosphonate **65.9,** for example by treatment with aqueous ammonia, as described in J. Am. Chem. Soc., 85, 1337, 1963, then affords the thiol **65.10.**
Using the above procedures, but employing, in place of the bromomethyl compound **65.8,** different bromomethyl compounds **65.2,** there are obtained the corresponding thiols **65.5.**

Scheme **66** illustrates the preparation of thiophenols bearing a phosphonate group linked to the phenyl nucleus by oxygen or sulfur. In this procedure, a suitably protected hydroxy or thio-substituted thiophenol **66.1** is reacted with a dialkyl hydroxyalkylphosphonate **66.2** under the conditions of the Mitsonobu reaction, for example as described in Org. React., 1992, 42, 335, to afford the coupled product **66.3.** Deprotection then yields the O- or S-linked products **66.4.**
For example, the substrate 3-hydroxythiophenol, **66.5,** is converted into the monotrityl ether **66.6,** by reaction with one equivalent of trityl chloride, as described above. This compound is reacted with diethyl azodicarboxylate, triphenyl phosphine and a dialkyl 1-hydroxymethyl phosphonate **66.7** in benzene, as described in Synthesis, 4, 327, 1998, to afford the ether compound **66.8.** Removal of the trityl protecting group, as described above, then affords the thiophenol **66.9.**

Using the above procedures, but employing, in place of the phenol **66.5,** different phenols or thiophenols **66.1,** there are obtained the corresponding thiols **66.4.**

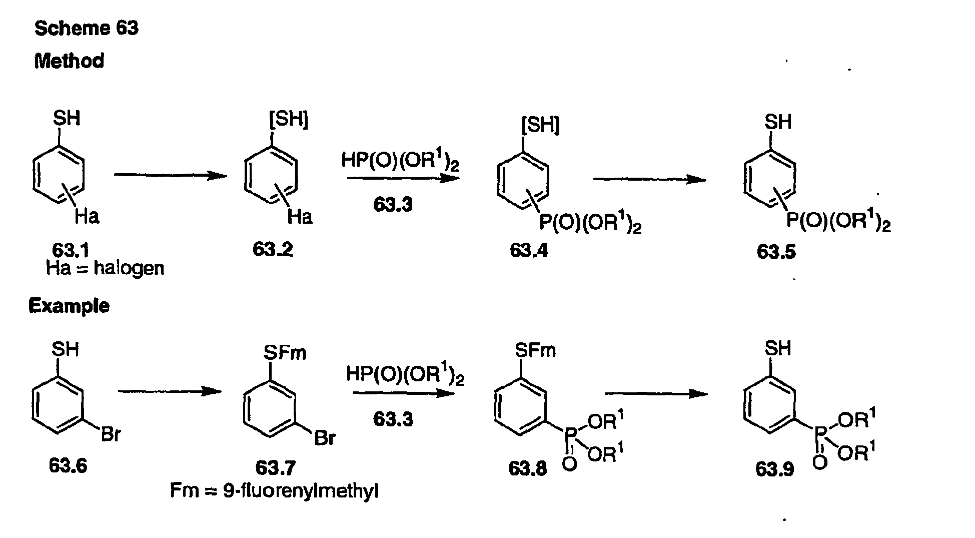

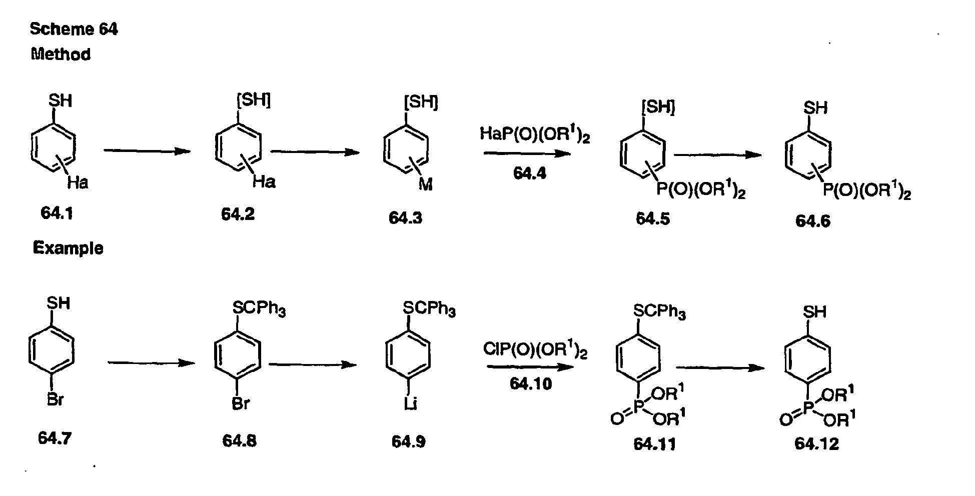

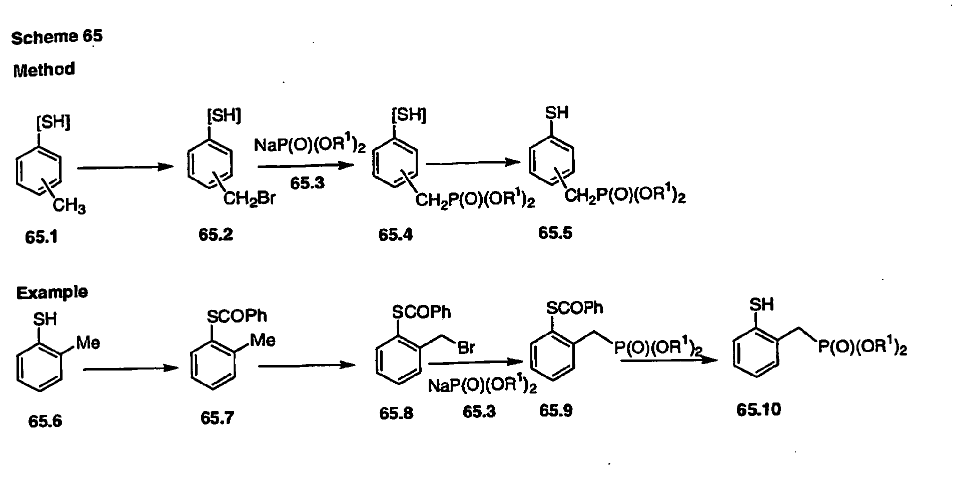

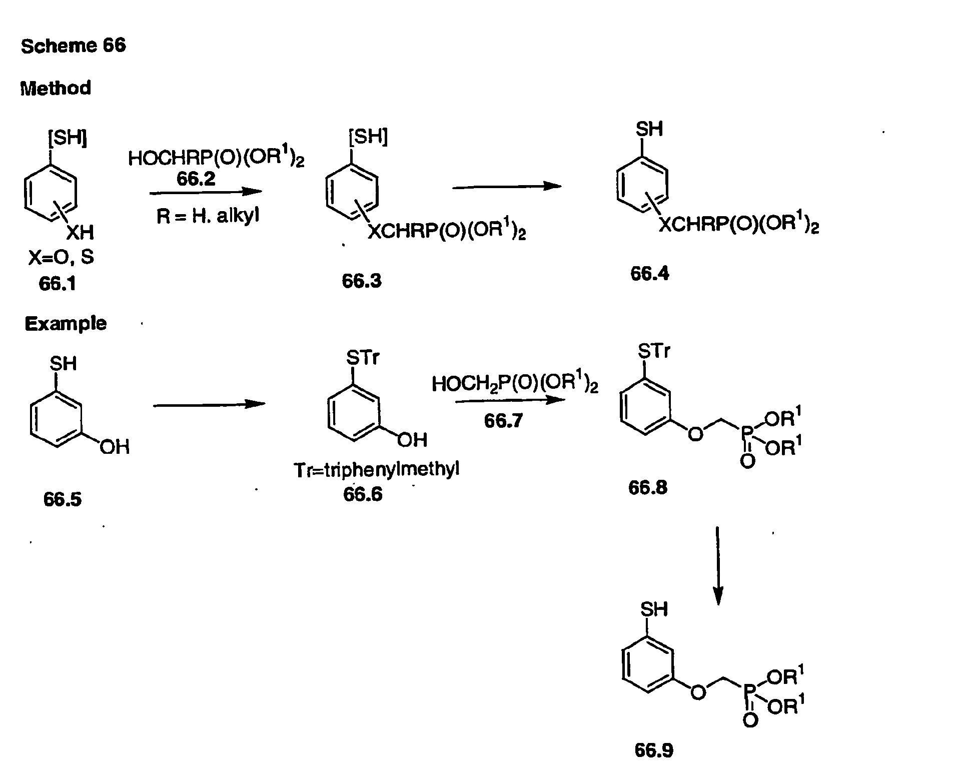

Scheme **67** illustrates the preparation of thiophenols **67.4** bearing a phosphonate group linked to the phenyl nucleus by oxygen, sulfur or nitrogen. In this procedure, a suitably protected O, S or N-substituted thiophenol **67.1** is reacted with an activated ester, for example the trifluoromethanesulfonate **67.2,** of a dialkyl hydroxyalkyl phosphonate, to afford the coupled product **67.3.** Deprotection then affords the thiol **67.4.**
For example, 4-methylaminothiophenol **67.5** is reacted in dichloromethane solution with one equivalent of acetyl chloride and a base such as pyridine, as described in Protective Groups in Organic Synthesis, by T. W. Greene and P.G.M. Wuts, Wiley, 1991, pp. 298, to afford the S-acetyl product **67.6.** This material is then reacted with a dialkyl trifluoromethanesulfonylmethyl phosphonate **67.7,** the preparation of which is described in Tet. Lett., 1986, 27, 1477, to afford the displacement product **67.8.** Preferably, equimolar amounts of the phosphonate **67.7** and the amine **67.6** are reacted together in an aprotic solvent such as dichloromethane, in the presence of a base such as 2,6-lutidine, at ambient temperatures, to afford the phosphonate product **67.8.** Deprotection, for example by treatment with dilute aqueous sodium hydroxide for two minutes, as described in J. Am. Chem. Soc., 85, 1337, 1963, then affords the thiophenol **67.9.**
Using the above procedures, but employing, in place of the thioamine **67.5,** different phenols, thiophenols or amines **67.1,** and/or different phosphonates **67.2,** there are obtained the corresponding products **67.4.**

Scheme 68 illustrates the preparation of phosphonate esters linked to a thiophenol nucleus by means of a heteroatom and a multiple-carbon chain, employing a nucleophilic displacement reaction on a dialkyl bromoalkyl phosphonate **68.2.** In this procedure, a suitably protected hydroxy, thio or amino substituted thiophenol **68.1** is reacted with a dialkyl bromoalkyl phosphonate **68.2** to afford the product **68.3.** Deprotection then affords the free thiophenol **68.4.**
For example, 3-hydroxythiophenol **68.5** is converted into the S-trityl compound **68.6,** as described above. This compound is then reacted with, for example, a dialkyl 4-bromobutyl phosphonate **68.7,** the synthesis of which is described in Synthesis, 1994, 9, 909. The reaction is conducted in a dipolar aprotic solvent, for example dimethylformamide, in the presence of a base such as potassium carbonate, and optionally in the presence of a catalytic amount of potassium iodide, at about 50°, to yield the ether product **68.8.** Deprotection, as described above, then affords the thiol **68.9.**
Using the above procedures, but employing, in place of the phenol **68.5,** different phenols, thiophenols or amines **68.1,** and/or different phosphonates **68.2,** there are obtained the corresponding products **68.4.**

Scheme **69** depicts the preparation of phosphonate esters linked to a thiophenol nucleus by means of unsaturated and saturated carbon chains. The carbon chain linkage is formed by means of a palladium catalyzed Heck reaction, in which an olefinic phosphonate **69.2** is coupled with an aromatic bromo compound **69.1.** The coupling of aryl halides with olefins by means of the Heck reaction is described, for example, in Advanced Organic Chemistry, by F. A. Carey and R. J. Sundberg, Plenum, 2001, p. 503ff and in Acc. Chem Res., 12, 146, 1979. The aryl bromide and the olefin are coupled in a polar solvent such as dimethylformamide or dioxan, in the presence of a palladium(0) catalyst such as tetrakis(triphenylphosphine)palladium(0) or palladium(II) catalyst such as palladium(II) acetate, and optionally in the presence of a base such as triethylamine or potassium carbonate, to afford the coupled product **69.3.** Deprotection, or hydrogenation of the double bond followed by deprotection, affords respectively the unsaturated phosphonate 69.4, or the saturated analog **69.6.**
For example, 3-bromothiophenol is converted into the S-Fm derivative **69.7,** as described above, and this compound is reacted with a dialkyl 1-butenyl phosphonate **69.8,** the preparation of which is described in J. Med. Chem., 1996, 39, 949, in the presence of a palladium (II) catalyst, for example, bis(triphenylphosphine) palladium (II) chloride, as described in J. Med. Chem, 1992, 35, 1371. The reaction is conducted in an aprotic dipolar solvent such as, for example, dimethylformamide, in the presence of triethylamine, at about 100° to afford the coupled product **69.9.** Deprotection, as described above, then affords the thiol **69.10.** Optionally, the initially formed unsaturated phosphonate **69.9** is subjected to reduction, for example using diimide, as described above, to yield the saturated product **69.11,** which upon deprotection affords the thiol **69.12.**
Using the above procedures, but employing, in place of the bromo compound **69.7,** different bromo compounds **69.1,** and/or different phosphonates **69.2,** there are obtained the corresponding products **69.4** and **69.6**

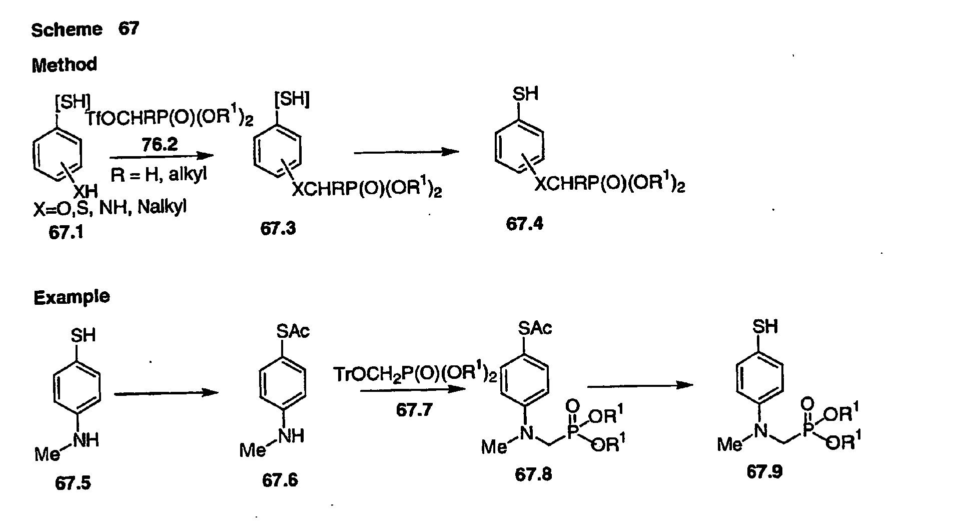

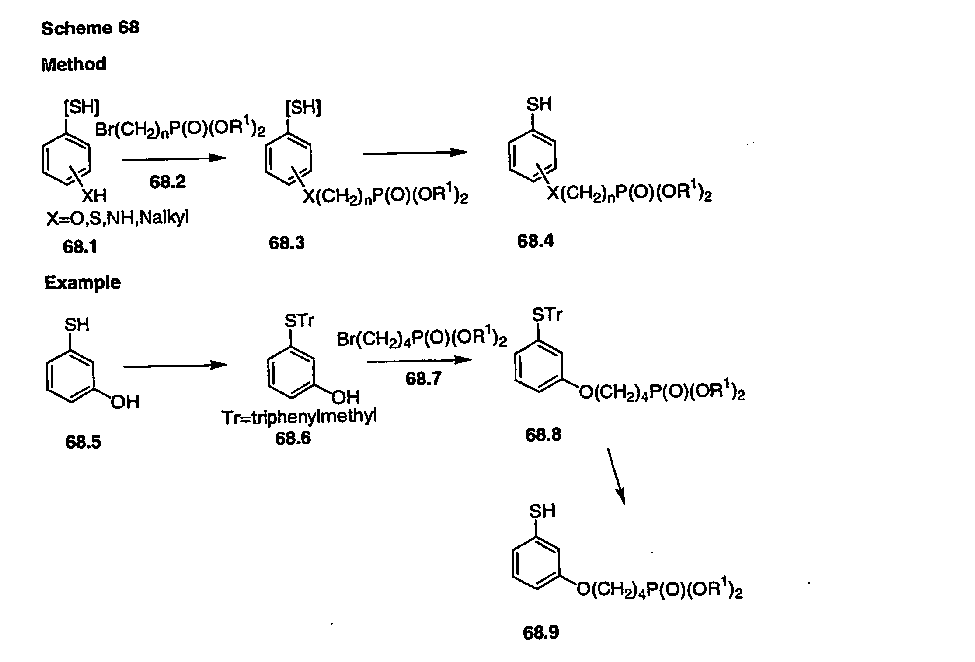

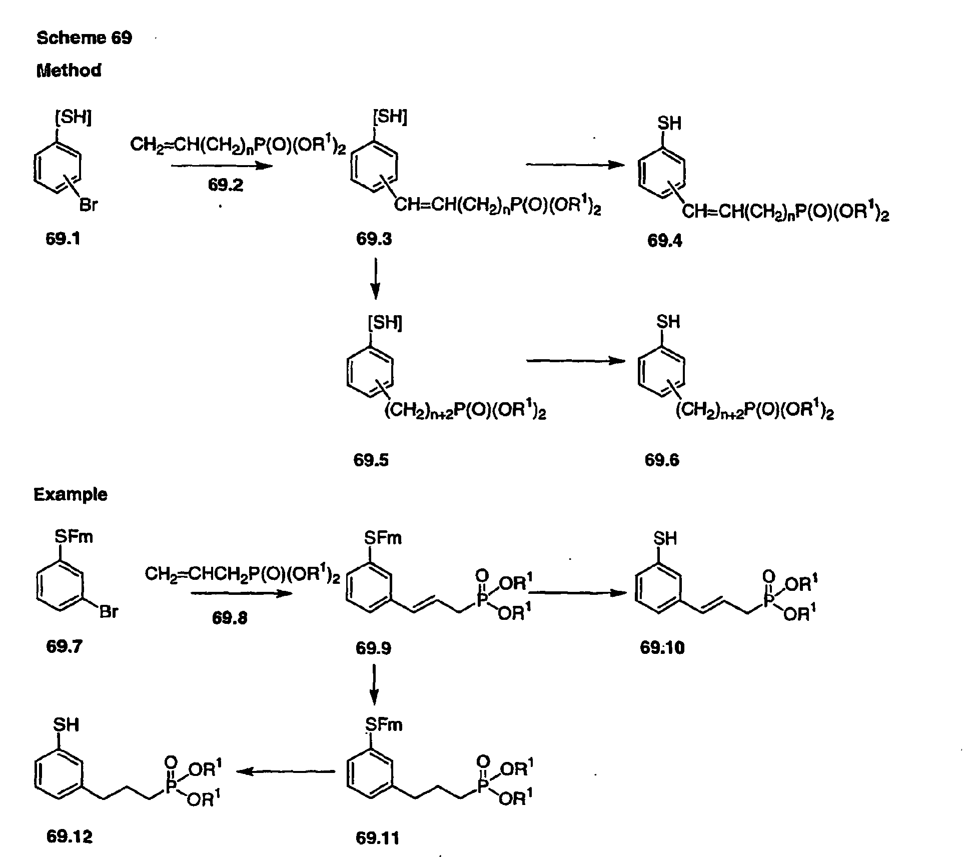

Scheme **70** illustrates the preparation of an aryl-linked phosphonate ester **70.4** by means of a palladium(0) or palladium(II) catalyzed coupling reaction between a bromobenzene and a phenylboronic acid, as described in Comprehensive Organic Transformations, by R. C. Larock, VCH, 1989, p. 57. The sulfur-substituted phenylboronic acid **70.1** is obtained by means of a metallation-boronation sequence applied to a protected bromo-substituted thiophenol, for example as described in J. Org. Chem, 49, 5237, 1984. A coupling reaction then affords the diaryl product **70.3** which is deprotected to yield the thiol **70.4.**
For example, protection of 4-bromothiophenol by reaction with tert-butylchlorodimethylsilane, in the presence of a base such as imidazole, as described in Protective Groups in Organic Synthesis, by T. W. Greene and P.G.M. Wuts, Wiley, 1991, p. 297, followed by metallation with butyllithium and boronation, as described in J. Organomet. Chem., 1999, 581, 82, affords the boronate **70.5.** This material is reacted with a dialkyl 4-bromophenylphosphonate **70.6,** the preparation of which is described in J. Chem. Soc., Perkin Trans., 1977, 2, 789, in the presence of tetrakis(triphenylphosphine) palladium (0) and an inorganic base such as sodium carbonate, to afford the coupled product **70.7.** Deprotection, for example by the use of tetrabutylammonium fluoride in anhydrous tetrahydrofuran, then yields the thiol **70.8.**
Using the above procedures, but employing, in place of the boronate **70.5,** different boronates **70.1,** and/or different phosphonates **70.2,** there are obtained the corresponding products **70.4.**

Scheme **71** depicts the preparation of dialkyl phosphonates in which the phosphonate moiety is linked to the thiophenyl group by means of a chain which incorporates an aromatic or heteroaromatic ring. In this procedure, a suitably protected O, S or N-substituted thiophenol **71.1** is reacted with a dialkyl bromomethyl-substituted aryl or heteroarylphosphonate **71.2,** prepared, for example, by means of an Arbuzov reaction between equimolar amounts of a bis(bromo-methyl) substituted aromatic compound and a trialkyl phosphite. The reaction product **71.3** is then deprotected to afford the thiol **71.4.** For example, 1,4-dimercaptobenzene is converted into the monobenzoyl ester **71.5** by reaction with one molar equivalent of benzoyl chloride, in the presence of a base such as pyridine. The monoprotected thiol **71.5** is then reacted with a dialkyl 4-(bromomethyl)phenylphosphonate, **71.6,** the preparation of which is described in Tetrahedron, 1998, 54, 9341. The reaction is conducted in a solvent such as dimethylformamide, in the presence of a base such as potassium carbonate, at about 50°. The thioether product **71.7** thus obtained is deprotected, as described above, to afford the thiol **71.8.**
Using the above procedures, but employing, in place of the thiophenol **71.5,** different phenols, thiophenols or amines **71.1,** and/or different phosphonates **71.2,** there are obtained the corresponding products **71.4.**

Scheme **72** illustrates the preparation of phosphonate-containing thiophenols in which the attached phosphonate chain forms a ring with the thiophenol moiety.
In this procedure, a suitably protected thiophenol **72.1,** for example an indoline (in which X-Y is (CH₂)₂), an indole (X-Y is CH=CH) or a tetrahydroquinoline (X-Y is (CH₂)₃) is reacted with a dialkyl trifluoromethanesulfonyloxymethyl phosphonate **72.2**, in the presence of an organic or inorganic base, in a polar aprotic solvent such as, for example, dimethylformamide, to afford the phosphonate ester **72.3.** Deprotection, as described above, then affords the thiol **72.4.** The preparation of thio-substituted indolines is described in EP 209751. Thio-substituted indoles, indolines and tetrahydroquinolines can also be obtained from the corresponding hydroxy-substituted compounds, for example by thermal rearrangement of the dimethylthiocarbamoyl esters, as described in J. Org. Chem, 31, 3980, 1966. The preparation of hydroxy-substituted indoles is described in Syn., 1994, 10, 1018; preparation of hydroxy-substituted indolines is described in Tet. Lett., 1986, 27, 4565, and the preparation of hydroxy-substituted tetrahydroquinolines is described in J. Het. Chem, 1991, 28, 1517, and in J. Med. Chem, 1979, 22, 599. Thio-substituted indoles, indolines and tetrahydroquinolines can also be obtained from the corresponding amino and bromo compounds, respectively by diazotization, as described in Sulfur Letters, 2000, 24, 123, or by reaction of the derived organolithium or magnesium derivative with sulfur, as described in Comprehensive Organic Functional Group Preparations, A. R. Katritzky et al, eds, Pergamon, 1995, Vol. 2, p 707.
For example, 2,3-dihydro-1H-indole-5-thiol, **72.5,** the preparation of which is described in EP 209751, is converted into the benzoyl ester **72.6,** as described above, and the ester is then reacted with the trifluoromethanesulfonate **72.7,** in a polar organic solvent such as dimethylformamide, in the presence of a base such as potassium carbonate, to yield the phosphonate **72.8.** Deprotection, for example by reaction with dilute aqueous ammonia, as described above, then affords the thiol **72.9.**
Using the above procedures, but employing, in place of the thiol **72.5,** different thiols **72.1,** and/or different triflates **72.2,** there are obtained the corresponding products **72.4.**

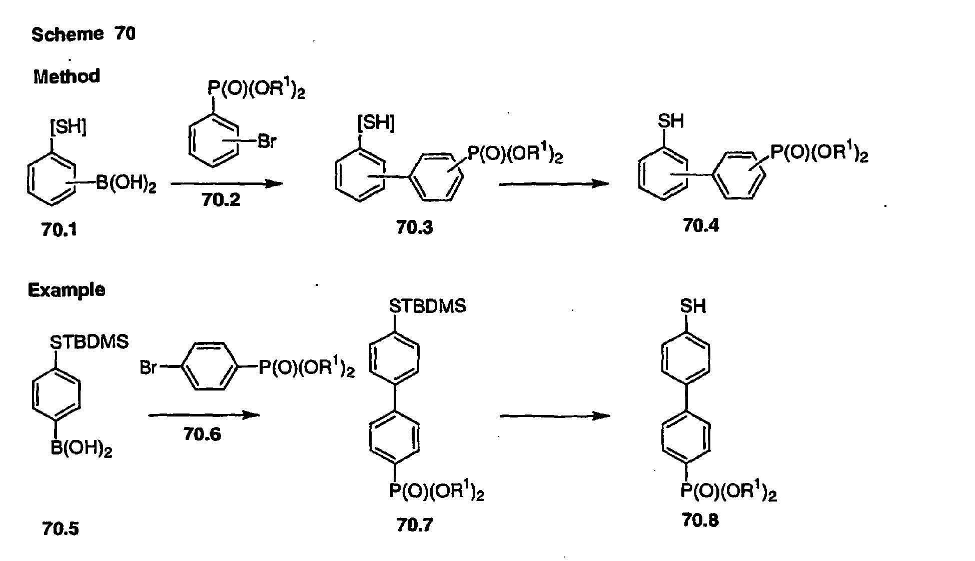

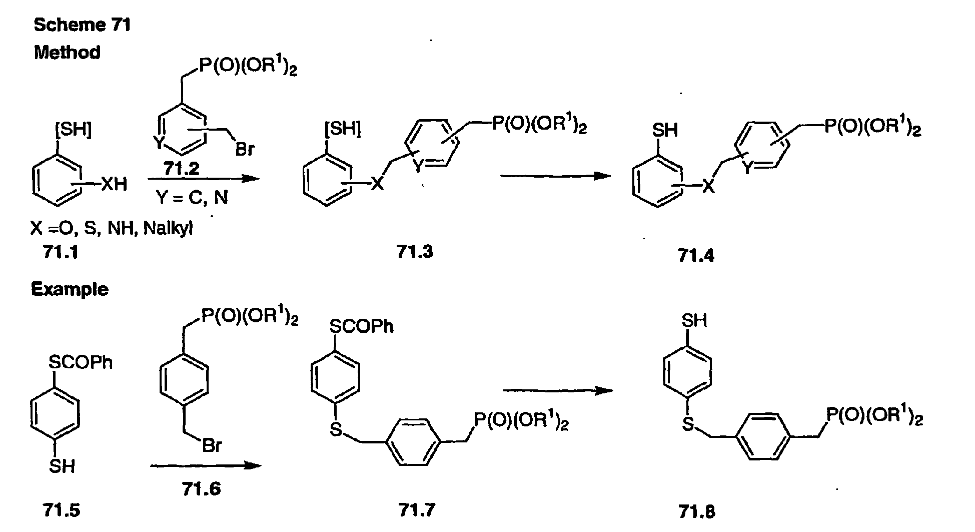

### Preparation of phosphonate-containing analogs of isobutylamine 10.2.

Schemes **73 - 75** illustrate the preparation of the phosphonate-containing analogs of isobutylamine which are employed in the preparation of the phosphonate esters **2.**

Scheme **73** depicts the preparation of phosphonates which are attached to the isobutylamine by means of an amide linkage. In this procedure, an aminoacid **73.1** is protected to afford the product **73.2.** The protection of amino groups is described in Protective Groups in Organic Synthesis, by T.W. Greene and P.G.M Wuts, Wiley, Second Edition 1990, 309. Amino groups are protected, for example, by conversion into carbamates such as the tert. butoxycarbamate (BOC) derivative, or by reaction with phthalic anhydride to afford the phthalimido (phth) derivative. The amine-protected aminoacid **73.2** is then coupled with a dialkyl aminoalkyl phosphonate **73.3,** to yield the amide **73.4.** The preparation of amides from carboxylic acids and derivatives is described, for example, in Organic Functional Group Preparations, by S.R.Sandler and W. Karo, Academic Press, 1968, p. 274, and Comprehensive Organic Transformations, by R. C. Larock, VCH, 1989, p. 972ff. The carboxylic acid is reacted with the amine in the presence of an activating agent, such as, for example, dicyclohexylcarbodiimide or diisopropylcarbodiimide, optionally in the presence of, for example, hydroxybenztriazole, N-hydroxysuccinimide or N-hydroxypyridone, in a non-protic solvent such as, for example, pyridine, DMF or dichloromethane, to afford the amide. Alternatively, the carboxylic acid may first be converted into an activated derivative such as the acid chloride, anhydride, mixed anhydride, imidazolide and the like, and then reacted with the amine, in the presence of an organic base such as, for example, pyridine, to afford the amide. The protecting group is then removed to afford the amine 73.5. Deprotection of amines is described in Protective Groups in Organic Synthesis, by T.W. Greene and P.G.M Wuts, Wiley, Second Edition 1990, p 309ff. For example, BOC groups are removed by treatment with acids such as trifluoroacetic acid, and phthalimido groups are removed by reaction with hydrazine hydrate.
For example, 2-methyl-4-aminobutyric acid **73.6** (Acros) is reacted with phthalic anhydride in refluxing toluene, as described in Protective Groups in Organic Synthesis, by T.W. Greene and P.G.M Wuts, Wiley, Second Edition 1990, p 358, to give the phthalimido derivative **73.7.** The product is coupled with a dialkyl aminoethyl phosphonate **73.8,** the preparation of which is described in J. Org. Chem., 2000, 65, 676, in the presence of dicyclohexyl carbodiimide, to give the amide **73.9.** The protecting group is removed by reaction of the product with ethanolic hydrazine at ambient temperature, as described in Protective Groups in Organic Synthesis, by T.W. Greene and P.G.M Wuts, Wiley, Second Edition 1990, p 358, to afford the amine **73.10.**
Using the above procedures, but employing, in place of the acid **73.6,** different acids **73.1,** and/or different amines **73.3,** the corresponding amides **73.5** are obtained.

Scheme **74** depicts the preparation of isobutylamine phosphonates in which the phosphonate is attached by means of an aromatic ring. In this procedure, 2-methyl-but-3-enylamine **74.1,** prepared as described in Org. Prep. Proc. Int. 1976, 8, 75, is coupled, in the presence of a palladium catalyst, as described above (Scheme **50**) with a dialkyl bromophenyl phosphonate **74.2** to afford the olefinic product **74.3.** Optionally, the product is reduced to afford the saturated analog **74.4.** The reduction is effected catalytically, for example by the use of a palladium catalyst, or chemically, for example by the use of diimide.
For example, the amine **74.1** is coupled with a dialkyl 4-bromophenyl phosphonate **74.5,** prepared as described in J. Organomet. Chem, 1999, 581, 62, to yield the product **74.6.** Catalytic hydrogenation in ethanol, using a 5% palladium catalyst, then affords the saturated compound **74.7.**
Using the above procedures, but employing, in place of the phosphonate **74.5,** different phosphonates **74.2** the corresponding products **74.3** and **74.4** are obtained.

Scheme 75 illustrates the preparation of isobutylamine phosphonates in which the phosphonate group is attached by means of an alkylene chain. In this procedure, a bromoamine **75.1** is protected, as described in Scheme **73,** to afford the derivative **75.2.** The product is then reacted with a trialkyl phosphite **75.3,** in an Arbuzov reaction, as described in Scheme **65,** to give the phosphonate **75.4.** Deprotection then affords the amine **75.5.**
For example, 4-bromo-2-methyl-butylamine **75.6,** prepared as described in Tet., 1998, 54, 2365, is converted, as described above, into the phthalimido derivative **75.7.** The product is then heated at 110° with a trialkyl phosphite **75.3** to yield the phosphonate **75.8,** which upon reaction with ethanolic hydrazine affords the amine **75.9.**
Using the above procedures, but employing, in place of the bromide **75.6,** different bromides **75.1,** and/or different phosphites **75.3,** the corresponding products **75.5** are obtained.

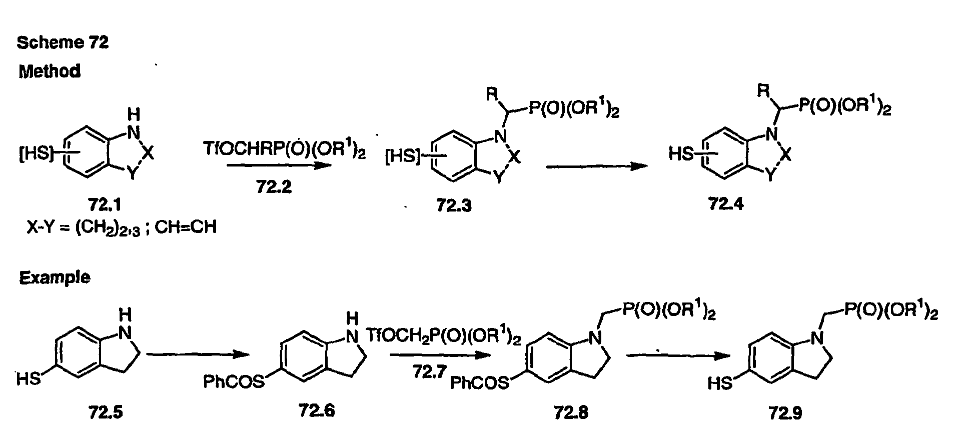

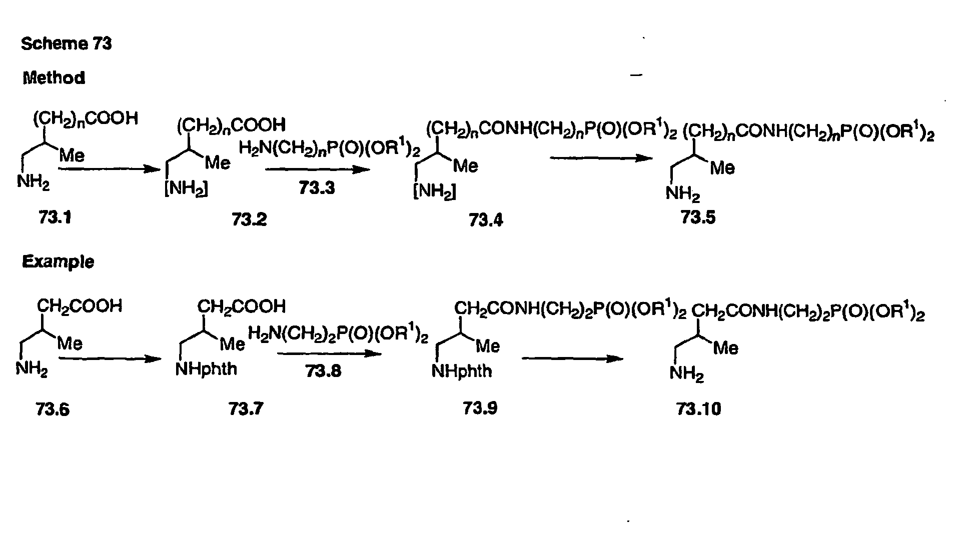

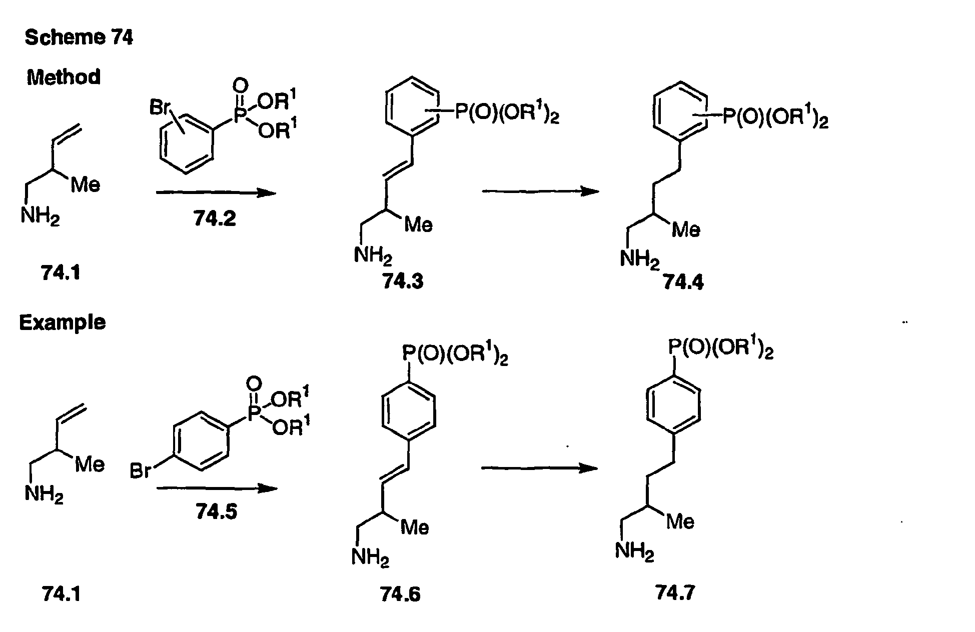

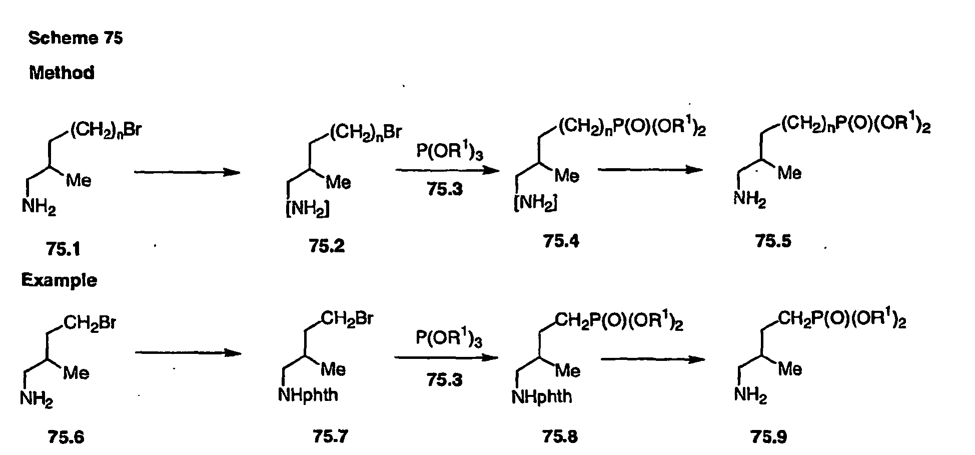

### Preparation of cyclopentylmethylamine phosphonates.

Schemes **76 - 78** illustrate the preparation of cyclopentylmethylamine phosphonates which are employed, as shown in Schemes **10 -12,** in the preparation of the phosphonate esters **3.**

Scheme **76** depicts the preparation of phosphonates attached to the cyclbpentyl ring either directly or by means of an alkoxy link. In this procedure, a hydroxy-substituted cyclopentylmethylamine **76.1** is protected, and the protected derivative **76.2** is converted into the corresponding bromide **76.3,** for example by treatment with carbon tetrabromide and triphenyl phosphine as described in Scheme **59.** The bromo compound is then reacted with a trialkyl phosphite **76.4** in an Arbuzov reaction, as described above, to afford the phosphonate **76.5** which is then deprotected to give the amine **76.6.** Alternatively, the protected amine **76.2** is reacted with a dialkyl bromoalkyl phosphonate **76.7** to give the ether **76.8.** The alkylation reaction is conducted at ca 100° in a polar organic solvent such as dimethylformamide in the presence of a base such as sodium hydride or lithium hexamethyl disilylazide. The product is then deprotected to give the amine **76.9.**
For example, 3-aminomethyl-cyclopentanol **76.10,** prepared as described in Tet., 1999, 55, 10815, is converted, as described above, into the phthalimido derivative **76.11.** The product is then converted, as described above, into the bromo analog **76.12.** The latter compound is reacted at ca 120° with a trialkyl phosphite **76.4** to afford the phosphonate **76.13,** which upon deprotection by reaction with hydrazine yields the amine **76.14.**
Using the above procedures, but employing, in place of the bromide **76.12,** different bromides **76.3,** and/or different phosphites **76.4,** the corresponding products **76.6** are obtained.
Alternatively, 2-aminomethyl-cyclopentanol **76.15,** prepared as described in Tet., 1999, 55, 10815, is converted into the phthalimido derivative **76.16.** The product is then reacted in dimethylformamide solution with an equimolar amount of a dialkyl bromopropyl phosphonate **76.17,** prepared as described in J. Am. Chem. Soc., 2000, 122, 1554, and sodium hydride, to give the ether **76.18.** Deprotection, as described above, then affords the amine **76.19.**
Using the above procedures, but employing, in place of the carbinol **76.15,** different carbinols **76.1,** and/or different phosphonates **76.7,** the corresponding products **76.9** are obtained.

Scheme **77** illustrates the preparation of cyclopentylmethylamines in which the phosphonate group is attached by means of an amide group. In this procedure, a carboxyalkyl-substituted cyclopentylmethylamine **77.1** is protected to afford the derivative **77.2.** The product is then coupled, as described above, (Scheme **1**) with a dialkyl aminoalkyl phosphonate **77.3** to yield the amide **77.4.** Deprotection then affords the amine **77.5.**
For example, 3-aminomethyl-cyclopentanecarboxylic acid **77.6** prepared as described in J. Chem. Soc. Perkin 2, 1995, 1381, is converted into the BOC derivative **77.7,** by reaction with BOC anhydride in aqueous sodium hydroxide, as described in Proc. Nat. Acad. Sci., 69, 730, 1972. The product is then coupled, in the presence of dicyclohexyl carbodiimide, with a dialkyl aminopropyl phosphonate **77.8** to produce the amide **77.9.** Removal of the BOC group, for example by treatment with hydrogen chloride in ethyl acetate, then affords the amine **77.10.** Using the above procedures, but employing, in place of the carboxylic acid **77.6,** different carboxylic acids **77.1,** and/or different phosphonates **77.3,** the corresponding products **77.5** are obtained.

Scheme **78** illustrates the preparation of cyclopentylmethylamines in which the phosphonate group is attached by means of an aminoalkyl group. In this procedure, the more reactive amino group of an amino-substituted cyclopentylmethylamine **78.1** is protected, to give the derivative **78.2.** The product is then coupled, by means of a reductive amination reaction, as described in Scheme **55,** with a dialkyl formylalkyl phosphonate **78.3** to give the amine product **78.4,** which upon deprotection affords the amine **78.5.**
For example, 2-aminomethyl-cyclopentylamine **78.6** prepared as described in WO 9811052, is reacted with one molar equivalent of phthalic anhydride in refluxing tetrahydrofuran, to yield the phthalimido derivative **78.7.** The latter compound is reacted, in the presence of sodium cyanoborohydride, with a dialkyl formylmethyl phosphonate **78.8,** prepared as described in Zh. Obschei. Khim, 1987, 57, 2793, to afford the product **78.9.** Deprotection, as described above, then yields the amine **78.10.**
Using the above procedures, but employing, in place of the diamine **78.6,** different diamines **78.1,** and/or different phosphonates **78.3,** the corresponding products **78.5** are obtained.

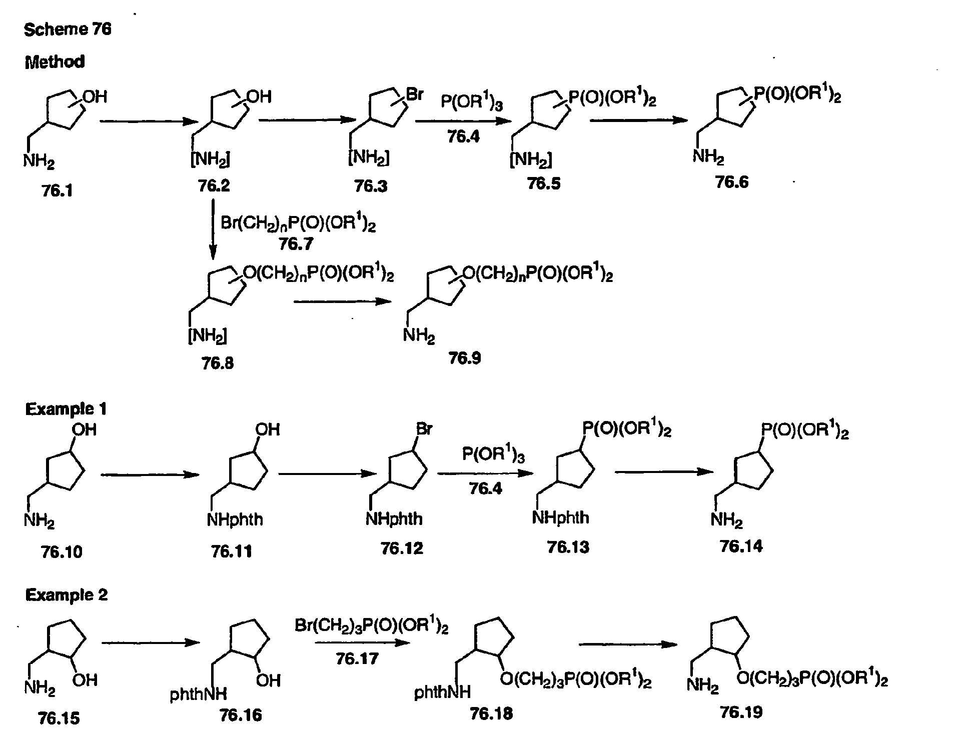

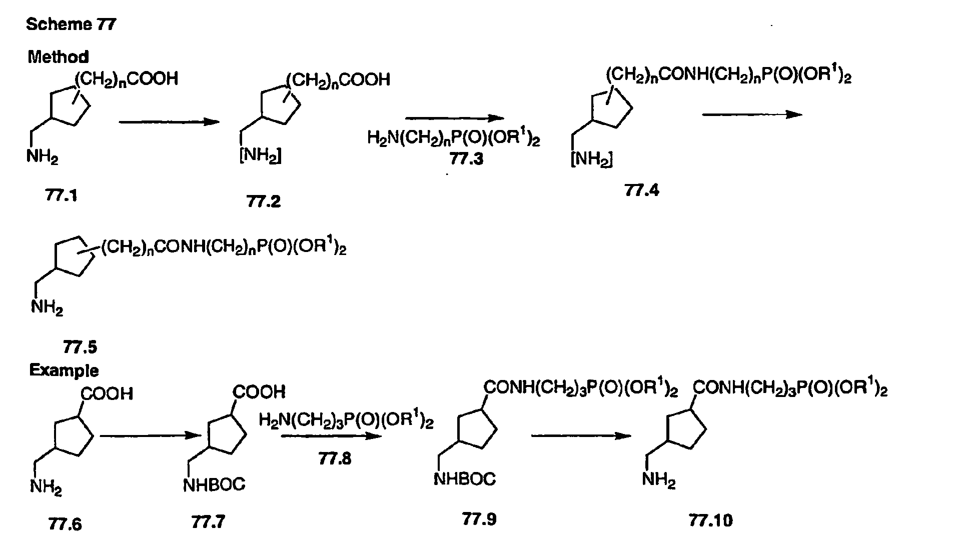

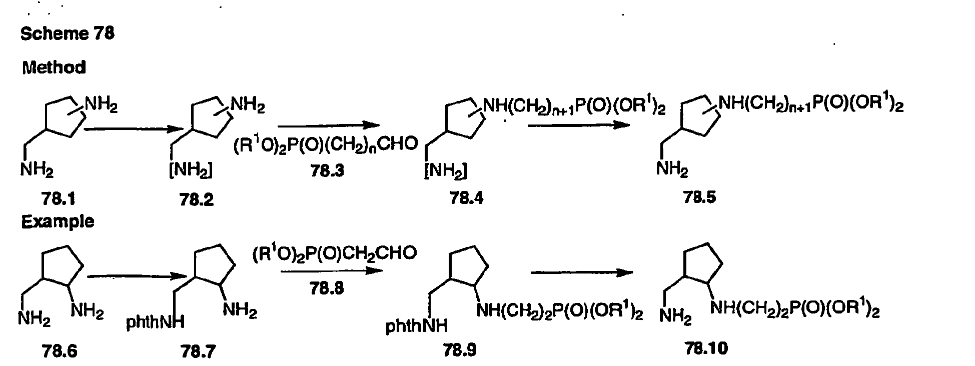

### Preparation of phosphonate-substituted fluorobenzylamines 39.2.

Schemes **79** and **80** illustrate the preparation of phosphonate-substituted 3-fluorobenzylamines **39.2** which are used in the preparation of the phosphonate esters **6.**

Scheme **79** depicts the preparation of fluorobenzylamines in which the phosphonate is attached by means of an amide or aminoalkyl linkage. In this procedure, the more reactive amino group in an amino-substituted 3-fluorobenzylamine **79.1** is protected. The product **79.2** is then coupled with a dialkyl carboxyalkyl phosphonate **79.3** to give the amide **79.4,** which upon deprotection yields the free amine **79.5.** Alternatively, the mono-protected diamine **79.2** is coupled, under reductive amination conditions, with a dialkyl formylalkyl phosphonate **79.6,** to produce the amine **79.7,** which upon deprotection affords the benzylamine **79.8.**
For example, 4-amino-3-fluorobenzylamine **79.9,** prepared as described in WO 9417035, is reacted in pyridine solution with one molar equivalent of acetic anhydride, to give the acetylamino product **79.10.** The product is reacted with a dialkyl carboxyethyl phosphonate **79.11,** (Epsilon) and dicyclohexyl carbodiimide, to afford the amide **79.12.** Deprotection, for example by reaction with 85% hydrazine, as described in J. Org. Chem, 43, 4593, 1978, then gives the amine **79.13.**
Using the above procedures, but employing, in place of the diamine **79.9,** different diamines **79.1,** and/or different phosphonates **79.3,** the corresponding products **79.5** are obtained. As a further example, the mono-protected diamine **79.10** is reacted, as described above, with a dialkyl formyl phosphonate **79.13,** (Aurora) and sodium cyanoborohydride, to give the amination product **79.14.** Deprotection then affords the amine **79.15.**

Using the above procedures, but employing, in place of the diamine **79.10** different diamines **79.2,** and/or different phosphonates **79.6,** the corresponding products **79.8** are obtained.

Scheme **80** depicts the preparation of fluorobenzylamines in which the phosphonate is attached either directly or by means of a saturated or unsaturated alkylene linkage. In this procedure, a bromo-substituted 3-fluorobenzylamine **80.1** is protected. The product **80.2** is coupled, by means of a palladium-catalyzed Heck reaction, as described in Scheme 50, with a dialkyl alkenyl phosphonate **80.3,** to give the olefinic product **80.4** which upon deprotection affords the amine **80.5.** Optionally, the double bond is reduced, for example by catalytic hydrogenation over a palladium catalyst, to yield the saturated analog **80.9.** Alternatively, the protected bromobenzylamine **80.6** is coupled, as described in Scheme **61,** in the presence of a palladium catalyst, with a dialkyl phosphite **80.6** to produce the phosphonate **80.7.** Deprotection then affords the amine **80.8.**
For example, 2-bromo-5-fluorobenzylamine **80.10,** (Esprix Fine Chemicals) is converted, as described above, into the N-acetyl derivative **80.11.** The product is the coupled in dimethylformamide solution with a dialkyl vinyl phosphonate **80.12,** (Fluka) in the presence of palladium (II) acetate and triethylamine, to give the coupled product **80.13.** Deprotection then affords the amine **80.14** and hydrogenation of the latter compound yields the saturated analog **80.15.**
Using the above procedures, but employing, in place of the bromo compound **80.10** different bromo compounds **80.1,** and/or different phosphonates **80.3,** the corresponding products **80.5** and **80.9** are obtained. As a further example, the protected amine **80.11** is coupled, in toluene at 100°, with a dialkyl phosphite **80.6,** in the presence of tetrakis(triphenylphosphine)palladium and a tertiary organic base such as triethylamine, to give the phosphonate **80.16.** Deprotection then affords the amine **80.17.**
Using the above procedures, but employing, in place of the bromo compound **80.11** different bromo compounds **80.2,** and/or different phosphites **80.6,** the corresponding products **80.8** are obtained.

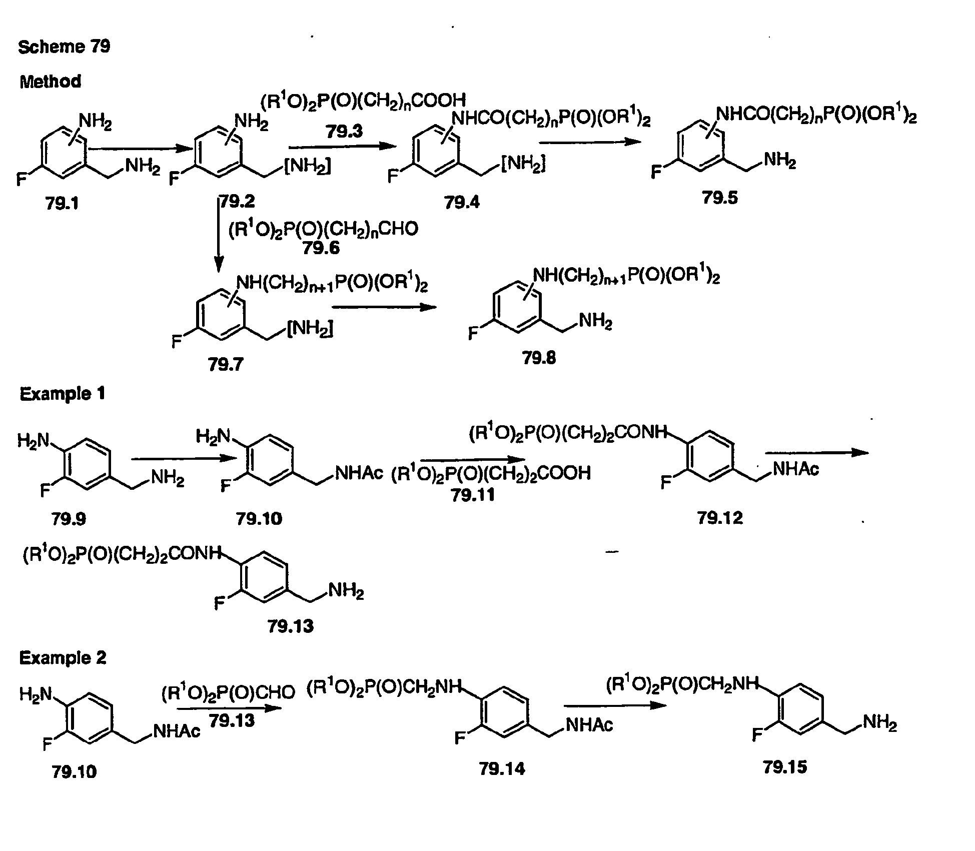

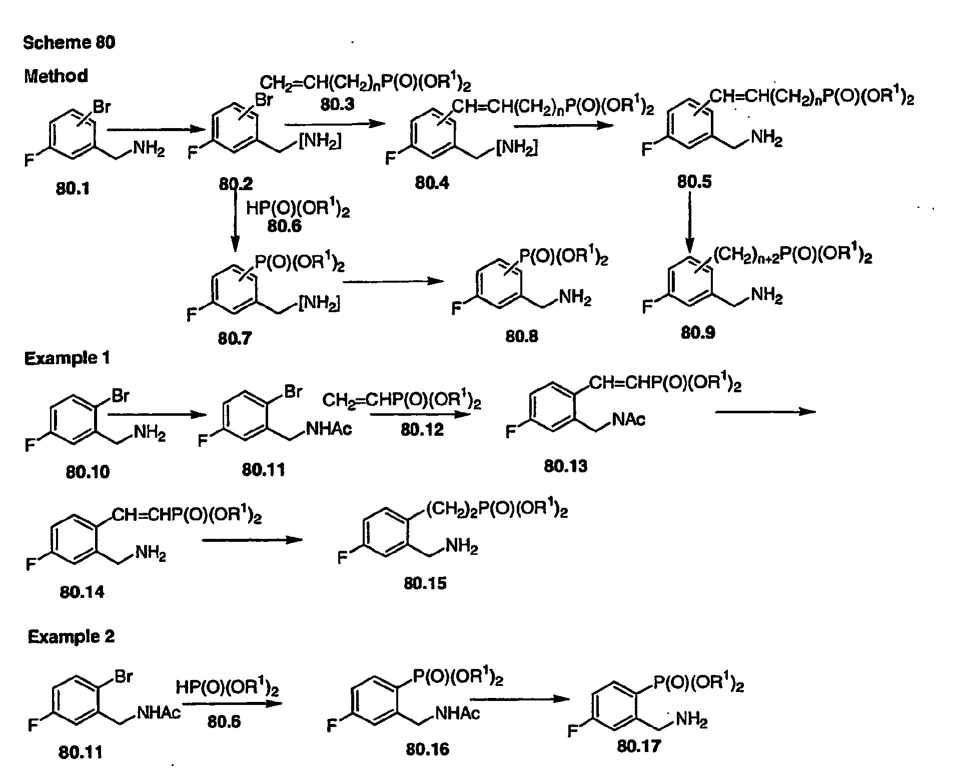

### Preparation of phosphonate-substituted fluorobenzylamines 39.4.

Schemes **81** and **82** illustrate the preparation of phosphonate-substituted 3-fluorobenzylamines **39.4** which are used in the preparation of the phosphonate esters 7.

Scheme **81** depicts the preparation of 3-fluorobenzylamines in which the phosphonate group is attached by means of an amide linkage. In this procedure, 3-fluorophenylalanine **81.1,** (Alfa Aesar) is converted into the BOC derivative **81.2.** The product is then coupled with a dialkyl aminoalkyl phosphonate **81.3** to afford the amide **81.4,** which upon deprotection gives the amine **81.5.**
For example, the BOC-protected aminoacid **81.2** is coupled, in the presence of dicyclohexyl carbodiimide, with a dialkyl aminomethyl phosphonate **81.6** (Interchim), to prepare the amide **81.7.** Deprotection then affords the amine **81.8.**
Using the above procedures, but employing, in place of the amine **81.6** different amines **81.3,** the corresponding products **81.5** are obtained.

Scheme **82** illustrates the preparation of fluorobenzylamine derivatives in which the phosphonate group is attached by means of an alkyl or alkoxy chain. In this procedure, a hydroxyalkyl-substituted 3-fluorobenzylamine **82.1** is converted into the BOC derivative **82.2.** This compound is then reacted with a dialkyl bromoalkyl phosphonate **82.3** to give the ether **82.4.** The alkylation reaction is conducted in a polar organic solvent such as N-methylpyrrolidinone in the presence of a strong base such as sodium bis(trimethylsilyl)amide. Deprotection of the product then affords the amine **82.5.** Alternatively, the N-protected carbinol **82.2** is converted into the corresponding bromide **82.6,** for example by reaction with N-bromoacetamide and triphenyl phosphine. The bromo compound is then reacted with a trialkyl phosphite in an Arbuzov reaction, as described above, to give the phosphonate **82.8,** which upon deprotection affords the amine **82.9.**
For example, 2-amino-2-(3-fluoro-phenyl)-ethanol **82.10,** prepared as described in DE 4443892, is converted into the BOC derivative **82.11.** The latter compound is then reacted in dimethylformamide at 100° with a dialkyl bromoethyl phosphonate **82.12** (Aldrich) and sodium hydride, to give the ether product **82.13.** Removal of the BOC group then yields the amine **82.14.**
Using the above procedures, but employing, in place of the carbinol **82.10** different carbinols **82.1,** and/or different phosphonates **82.3** the corresponding products **82.5** are obtained.
As a further example, the BOC-protected carbinol **82.11** is reacted with carbon tetrabromide and triphenylphosphine to produce the bromo compound **82.15.** This material is heated at 120° with an excess of a trialkyl phosphite **82.7** to give the phosphonate **82.16.** Deprotection then yields the amine **82.17.**
Using the above procedures, but employing, in place of the carbinol 82.11 different carbinols **82.2,** and/or different phosphonates **82.7** the corresponding products **82.9** are obtained.

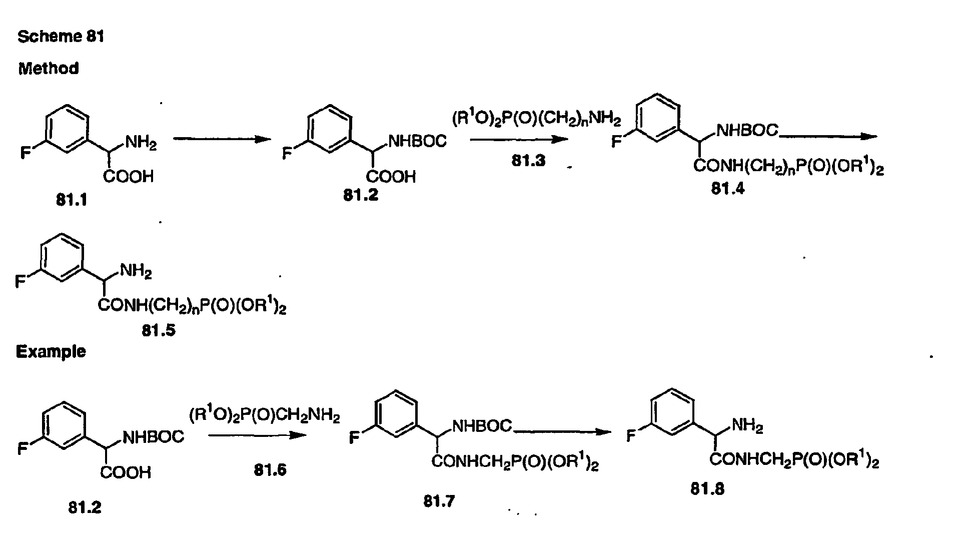

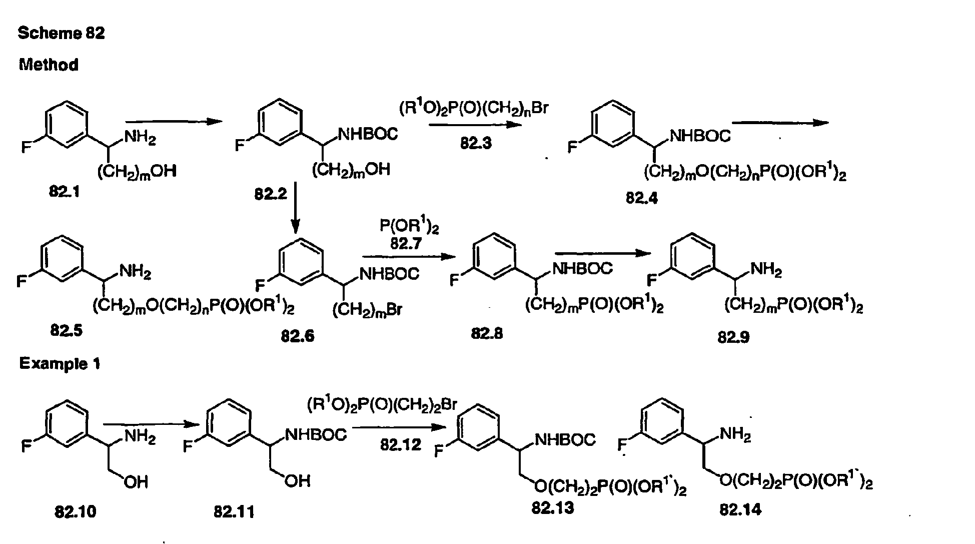

### Preparation of the phosphonate-containing tert. butanol derivatives 30.1.

Schemes **83 - 86** illustrate the preparation of the tert. butanol derivatives **30.1** which are employed in the preparation of the phosphonate esters **5.**

Scheme **83** depicts the preparation of tert. butanol derivatives in which the phosphonate is attached by means of an alkylene chain. In this procedure, a bromoalkyl carbinol **83.1** is reacted with a trialkyl phosphite **83.2** in an Arbuzov reaction, to afford the phosphonate **83.3.** For example, 4-bromo-2-methyl-butan-2-ol **83.4** prepared as described in Bioorg. Med. Chem. Lett., 2001, 9, 525, and a trialkyl phosphite **83.2** are heated at ca. 120° to produce the phosphonate **83.5.**
Using the above procedures, but employing, in place of the bromo compound **83.4** different bromo compounds **83.1,** and/or different phosphites **83.2** the corresponding products **83.3** are obtained.

Scheme **84** depicts the preparation of tert. butanol derivatives in which the phosphonate is attached by means of an amide linkage. In this procedure, a carboxylic acid **84.1** is coupled with a dialkyl aminoalkyl phosphonate **84.2** to afford the amide **84.3.** The reaction is conducted under the conditions previously described (Scheme **1**) for the preparation of amides.
For example, equimolar amounts of 3-hydroxy-3-methyl-butyric acid **84.4,** (Fluka) and a dialkyl aminoethyl phosphonate **84.5,** the preparation of which is described in J. Org. Chem, 2000, 65, 676 are reacted in tetrahydrofuran in the presence of dicyclohexylcarbodiimide to yield the amide **84.6.**
Using the above procedures, but employing, in place of the carboxylic acid **84.4** different acids **84.1,** and/or different amines **84.2** the corresponding products **84.3** are obtained.

Scheme **85** depicts the preparation of tert. butanol derivatives in which the phosphonate is attached by means of a heteroatom and an alkylene chain. In this procedure, a hydroxy, mercapto or amino-substituted carbinol **85.1** is reacted with a dialkyl bromoalkyl phosphonate **85.2** to afford the ether, thioether or amine products **85.3.** The reaction is conducted in a polar organic solvent in the presence of suitable base such as sodium hydride or cesium carbonate. For example, 4-mercapto-2-methyl-butan-2-ol **85.4** prepared as described in Bioorg. Med. Chem Lett., 1999, 9, 1715, is reacted in tetrahydrofuran containing cesium carbonate with a dialkyl bromobutyl phosphonate **85.5,** the preparation of which is described in Synthesis, 1994, 9, 909, to yield the thioether **85.6.**
Using the above procedures, but employing, in place of the thiol **85.4** different alcohols, thiol or amines **85.1,** and/or different bromides **85.2** the corresponding products **85.3** are obtained.

Scheme **86** depicts the preparation of tert. butanol derivatives in which the phosphonate is attached by means of a nitrogen and an alkylene chain. In this procedure, a hydroxyaldehyde **86.1** is reacted with a dialkyl aminoalkyl phosphonate **86.2** under reductive amination conditions, as described above, (Scheme 55) to afford the amine **86.3.**
For example, 3-hydroxy-3-methyl-butyraldehyde **86.4** and a dialkyl aminoethyl phosphonate **86.5** the preparation of which is described in J. Org. Chem, 2000, 65, 676 are reacted together in the presence of sodium triacetoxyborohydride, to yield the amine **86.6.**
Using the above procedures, but employing, in place of the aldehyde **86.4** different aldehydes **86.1,** and/or different amines **86.2** the corresponding products **86.3** are obtained.

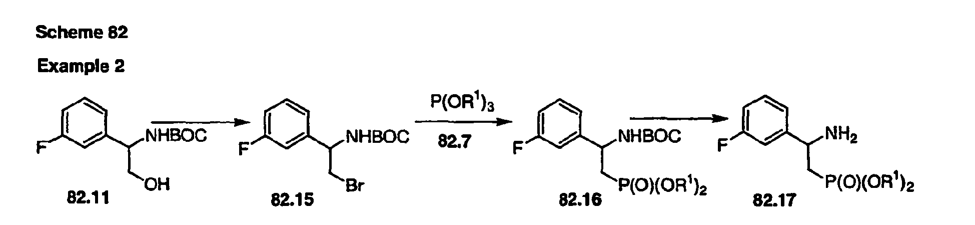

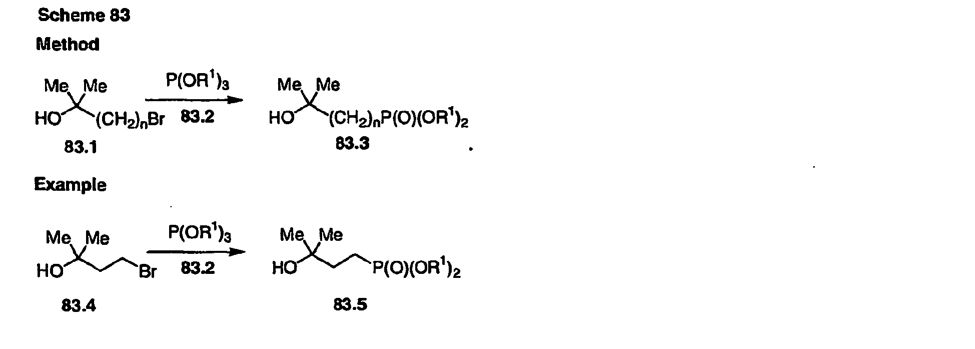

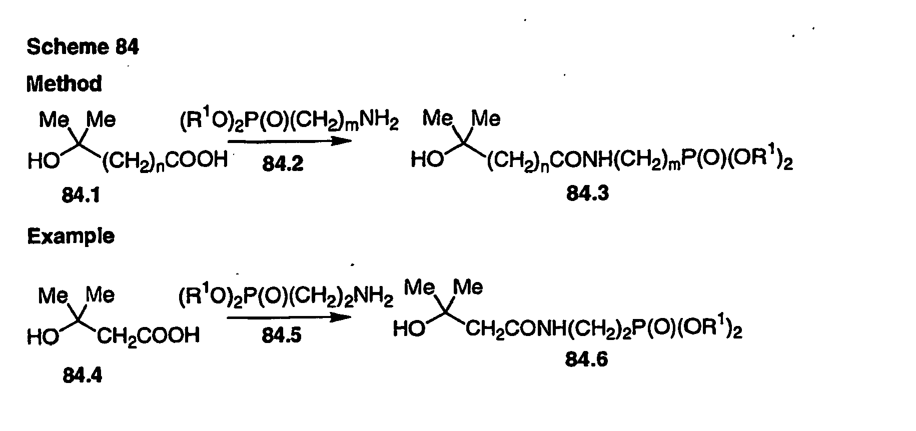

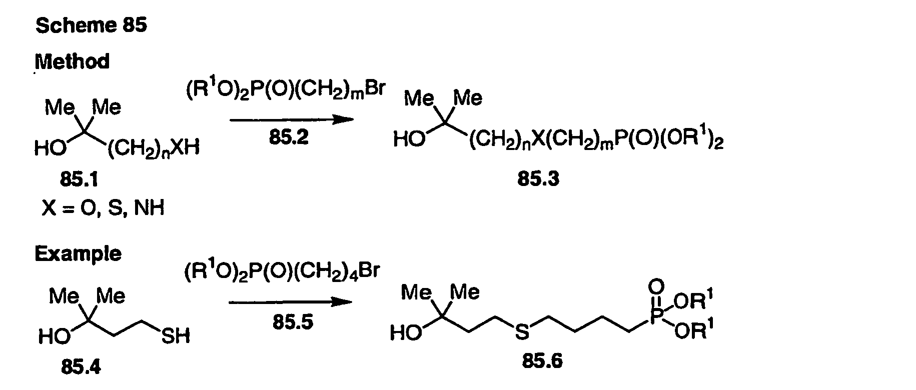

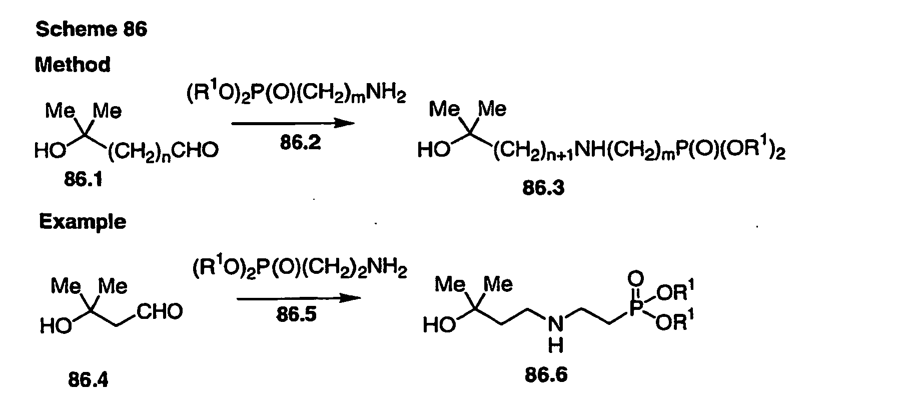

### Preparation of the phosphonate-containing benzyl carbamates 43.4.

Schemes **87 - 91** illustrate methods for the preparation of the benzyl carbamates **43.4** which are employed in the preparation of the phosphonate esters **9.** The benzyl alcohols are obtained by reduction of the corresponding benzaldehydes, the preparation of which is described in Schemes **87 - 90.**

Scheme **87** illustrates the preparation of benzaldehyde phosphonates **87.3** in which the phosphonate group is attached by means of an alkylene chain incorporation a nitrogen atom. In this procedure, a benzene dialdehyde **87.1** is reacted with one molar equivalent of a dialkyl aminoalkyl phosphonate **87.2,** under reductive amination conditions, as described above in Scheme **55,** to yield the phosphonate product **87.3.**
For example, benzene-1,3-dialdehyde **87.4** is reacted with a dialkyl aminopropyl phosphonate **87.5,** (Acros) and sodium triacetoxyborohydride, to afford the product **87.6.**
Using the above procedures, but employing, in place of benzene-1,3-dicarboxaldehyde **87.4,** different benzene dialdehydes **87.1,** and/or different phosphonates **87.2,** the corresponding products **87.3** are obtained.

Scheme **88** illustrates the preparation of benzaldehyde phosphonates either directly attached to the benzene ring or attached by means of a saturated or unsaturated carbon chain. In this procedure, a bromobenzaldehyde **88.1** is coupled, as described above, with a dialkyl alkenylphosphonate **88.2,** to afford the alkenyl phosphonate **88.3.** Optionally, the product is reduced to afford the saturated phosphonate ester **88.4.** Alternatively, the bromobenzaldehyde is coupled, as described above, with a dialkyl phosphite **88.5** to afford the formylphenylphosphonate **88.6.**
For example, as shown in Example 1, 3-bromobenzaldehyde **88.7** is coupled with a dialkyl propenylphosphonate **88.8** (Aldrich) to afford the propenyl product **88.9.** Optionally, the product is reduced, for example by the use of diimide, to yield the propyl phosphonate **88.10.** Using the above procedures, but employing, in place of 3-bromobenzaldehyde **88.7,** different bromobenzaldehydes **88.1,** and/or different alkenyl phosphonates **88.2,** the corresponding products **88.3** and **88.4** are obtained.
Alternatively, as shown in Example 2, 4-bromobenzaldehyde is coupled, in the presence of a palladium catalyst, with a dialkyl phosphite **88.5** to afford the 4-formylphenyl phosphonate product **88.12.**
Using the above procedures, but employing, in place of 4-bromobenzaldehyde **88.11,** different bromobenzaldehydes **88.1,** the corresponding products **88.6** are obtained.

Scheme **89** illustrates the preparation of formylphenyl phosphonates in which the phosphonate moiety is attached by means of alkylene chains incorporating two heteroatoms O, S or N. In this procedure, a formyl phenoxy, phenylthio or phenylamino alkanol, alkanethiol or alkylamine **89.1** is reacted with a an equimolar amount of a dialkyl haloalkyl phosphonate **89.2,** to afford the phenoxy, phenylthio or phenylamino phosphonate product **89.3.** The alkylation reaction is effected in a polar organic solvent such as dimethylformamide or acetonitrile, in the presence of a base. The base employed depends on the nature of the nucleophile **89.1.** In cases in which Y is O, a strong base such as sodium hydride or lithium hexamethyldisilazide is employed. In cases in which Y is S or N, a base such as cesium carbonate or dimethylaminopyridine is employed.
For example, 2-(4-formylphenylthio)ethanol **89.4,** prepared as described in Macromolecules, 1991, 24, 1710, is reacted in acetonitrile at 60° with one molar equivalent of a dialkyl iodomethyl phosphonate **89.5,** (Lancaster) to give the ether product **89.6.**
Using the above procedures, but employing, in place of the carbinol **89.4,** different carbinols, thiols or amines **89.1,** and/or different haloalkyl phosphonates **89.2,** the corresponding products **89.3** are obtained.

Scheme **90** illustrates the preparation of formylphenyl phosphonates in which the phosphonate group is linked to the benzene ring by means of an aromatic or heteroaromatic ring. In this procedure, a formylbenzeneboronic acid **90.1** is coupled, in the presence of a palladium catalyst, with one molar equivalent of a dibromoarene, **90.2**, in which the group Ar is an aromatic or heteroaromatic group. The coupling of aryl boronates with aryl bromides to afford diaryl compounds is described in Palladium Reagents and Catalysts, by J. Tsuji, Wiley 1995, p. 218. The components are reacted in a polar solvent such as dimethylformamide in the presence of a palladium(0) catalyst and sodium bicarbonate. The product **90.3** is then coupled, as described above (Scheme **50)** with a dialkyl phosphite **90.4** to afford the phosphonate **90.5.** For example, 4-formylbenzeneboronic acid **90.6** is coupled with 2,5-dibromothiophene **90.7** to yield the phenylthiophene product **90.8.** This compound is then coupled with the dialkyl phosphite **90.4** to afford the thienyl phosphonate **90.9.**
Using the above procedures, but employing, in place of dibromothiophene **90.7,** different dibromoarenes **90.2,** and/or different formylphenyl boronates **90.1,** the corresponding products **90.5** are obtained.

Scheme **91** illustrates the preparation of the benzyl carbamates **43.4** which are employed in the preparation of the phosphonate esters **9.** In this procedure, the substituted benzaldehydes **91.1,** prepared as shown in Schemes **87 - 90,** are converted into the corresponding benzyl alcohols **91.2.** The reduction of aldehydes to afford alcohols is described in Comprehensive Organic Transformations, by R. C. Larock, VCH, 1989, p. 527ff. The transformation is effected by the use of reducing agents such as sodium borohydride, lithium aluminum tri-tertiarybutoxy hydride, diisobutyl aluminum hydride and the like. The resultant benzyl alcohol is then reacted with the aminoester **91.3** to afford the carbamate **91.4.** The reaction is performed under the conditions described below, Scheme **98.** For example, the benzyl alcohol is reacted with carbonyldiimidazole to produce an intermediate benzyloxycarbonyl imidazole, and the intermediate is reacted with the aminoester **91.3** to afford the carbamate **91.4.** The methyl ester is then hydrolyzed to yield the carboxylic acid **43.4.**

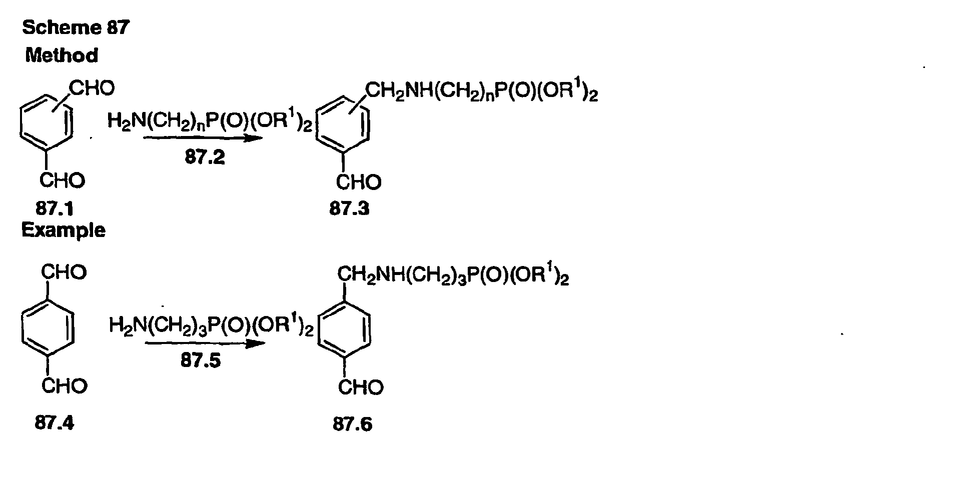

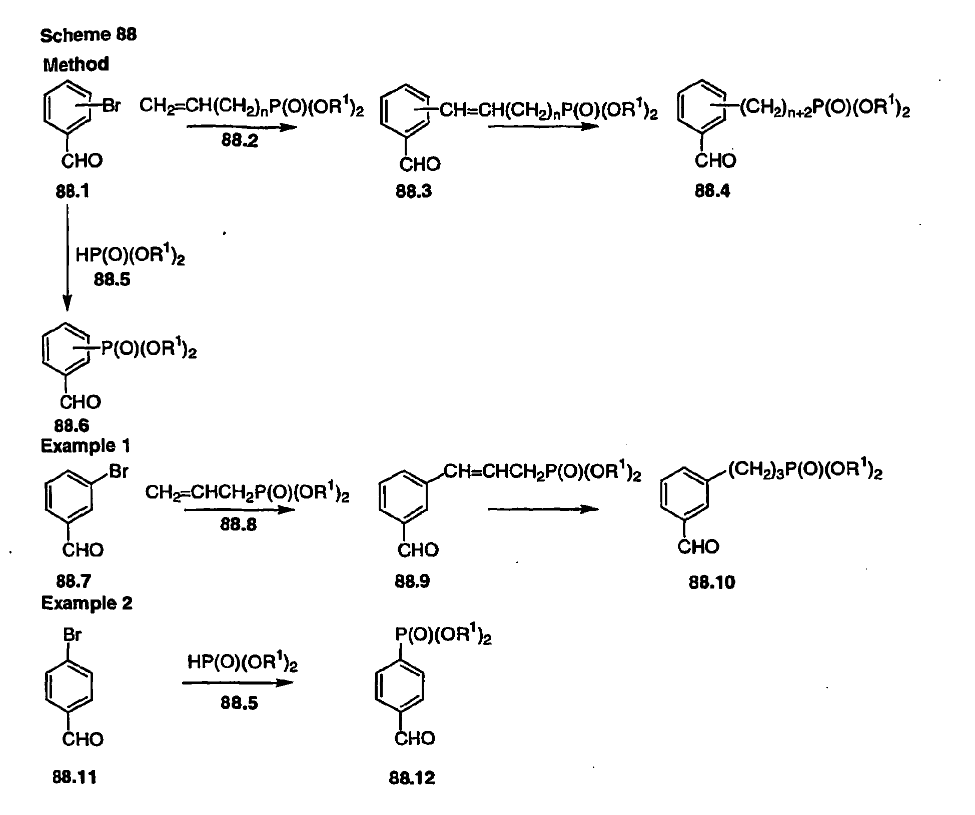

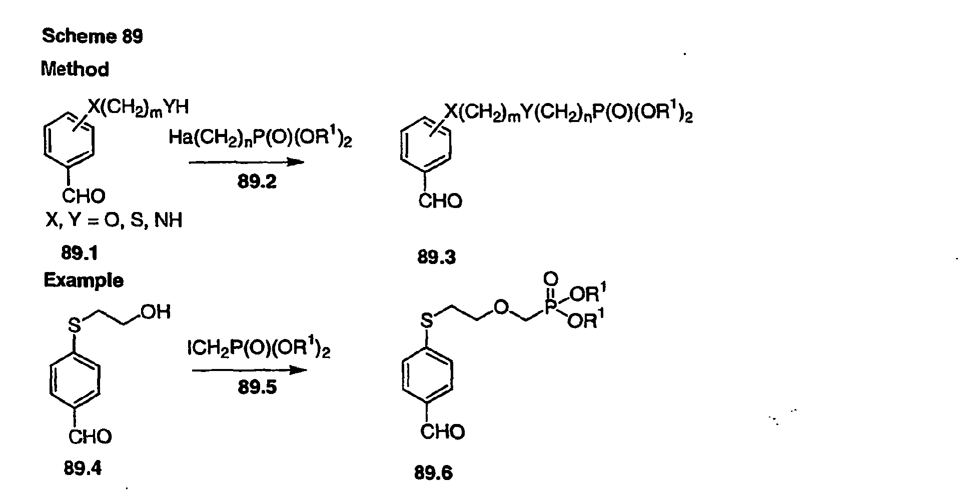

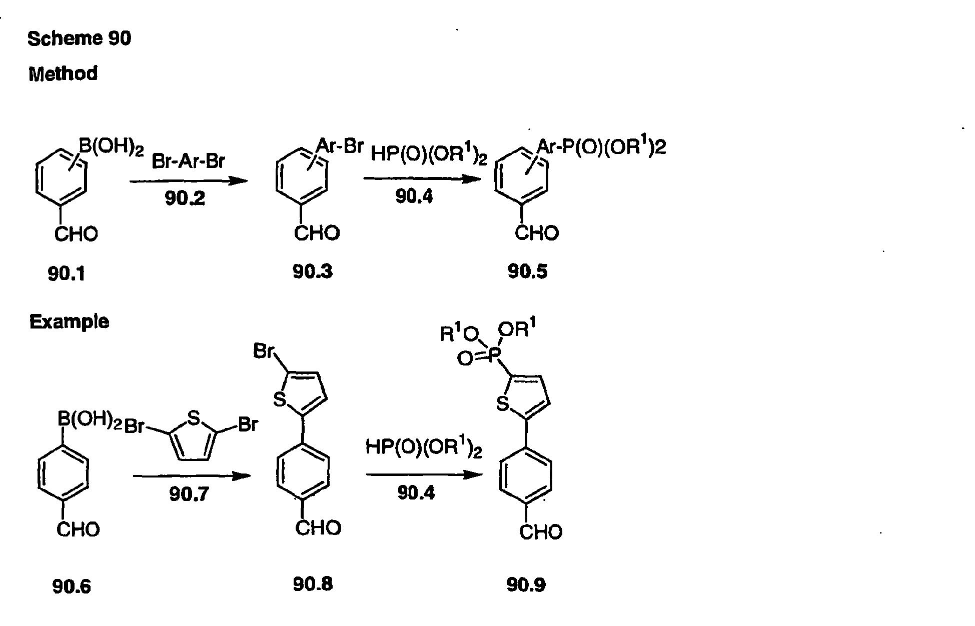

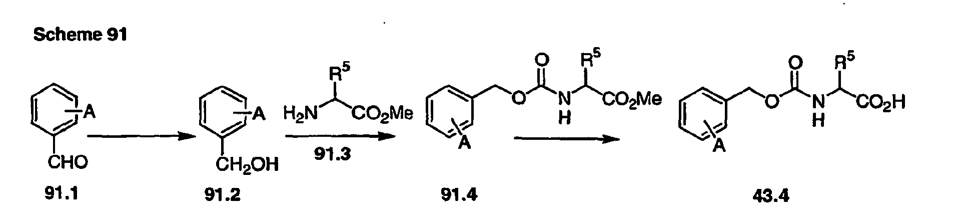

### Preparation of phosphonate-containing benzenesulfonyl chlorides 20.2.

Schemes **92 - 97** illustrate methods for the preparation of the sulfonyl chlorides **20.2** which are employed in the preparation of the phosphonate esters **4.** Sulfonic acids and/or sulfonyl halides are obtained by oxidation of the corresponding thiols, as described in Synthetic Organic Chemistry, R B. Wagner, H. D. Zook, Wiley, 1953, p. 813, and in Tet. 1965, 21, 2271. For example, the phosphonate-containing thiols which are prepared according to Schemes **63 - 72** are transformed into the corresponding sulfonic acids by oxidation with bromine in aqueous organic solution, as described in J. Am. Chem. Soc., 59, 811, 1937, or by oxidation with hydrogen peroxide, as described in Rec. Trav. Chim., 54, 205, 1935, or by reaction with oxygen in alkaline solution, as described in Tet. Let., 1963,1131, or by the use of potassium superoxide, as described in Aust. J. Chem., 1984, 37, 2231. Schemes **92- 96** describe the preparation of phosphonate-substituted benzenesulfonic acids; Scheme **97** describes the conversion of the sulfonic acids into the corresponding sulfonyl chlorides. Alternatively, the intermediate thiols, when propduced, can be directly converted to the sulfonyl chloride as described in Scheme **97a**

Scheme **92** depicts the preparation of variously substituted benzenesulfonic acids in which the phosphonate group is directly attached to the benzene ring. In this procedure, a bromo-substituted benzenethiol **92.1** is protected, as previously described. The protected product **92.2** is then reacted, in the presence of a palladium catalyst, with a dialkyl phosphite **92.3,** to give the corresponding phosphonate **92.4.** The thiol group is then deprotected to afford the thiol **92.5,** and this compound is oxidized to afford the sulfonic acid **92.6.**
For example, 4-bromobenzenethiol **92.7** is converted into the S-adamantyl derivative **92.8,** by reaction with 1-adamantanol in trifluoroacetic acid, as described in Chem Pharm. Bull., 26, 1576, 1978. The product is then reacted with a dialkyl phosphite and a palladium catalyst, as described previously, to yield the phosphonate **92.9.** The adamantyl group is then removed by reaction with mercuric acetate in trifluoroacetic acid, as described in Chem Pharm Bull., 26, 1576, 1978, to give the thiol **92.10.** The product is then reacted with bromine in aqueous solution to prepare the sulfonic acid **92.11.**
Using the above procedures, but employing, in place of the thiol **92.7,** different thiols **92.1,** and/or different dialkyl phosphites **92.3,** the corresponding products **92.6** are obtained.

Scheme **93** illustrates the preparation of amino-substituted benzenesulfonic acids in which the phosphonate group is attached by means of an alkoxy group. In this procedure, a hydroxy amino-substituted benzenesulfonic acid **93.1** is reacted with a dialkyl bromoalkyl phosphonate **93.2** to afford the ether **93.3.** The reaction is performed in a polar solvent such as dimethylformamide in the presence of a base such as potassium carbonate. The yield of the product **93.3** is increased by treatment of the crude reaction product with dilute aqueous base, so as to hydrolyze any sulfonic esters which are produced.
For example, 3-amino-4-hydroxybenzenesulfonic acid **93.4** (Fluka) is reacted with a dialkyl bromopropyl phosphonate **93.5** prepared as described in J. Am. Chem Soc., 2000, 122, 1554, in dimethylformamide containing potassium carbonate, followed by the addition of water, to produce the ether **93.6.**
Using the above procedures, but employing, in place of the phenol **93.4,** different phenols **93.1,** and/or different phosphonates **93.2,** the corresponding products **93.3** are obtained.

Scheme **94** illustrates the preparation of methoxyl-substituted benzenesulfonic acids in which the phosphonate group is attached by means of an amide group. In this procedure, a methoxy amino-substituted benzenesulfonic acid **94.1** is reacted, as described previously for the preparation of amides, with a dialkyl carboxyalkyl phosphonate **94.2** to produce the amide **94.3.**
For example, 3-amino-4-methoxybenzenesulfonic acid **94.4,** (Acros) is reacted in dimethylformamide solution with a dialkyl phosphonoacetic acid **94.2** (Aldrich) and dicyclohexyl carbodiimide, to produce the amide **94.6.**
Using the above procedures, but employing, in place of the amine **94.4,** different amines **94.1,** and/or different phosphonates **94.2,** the corresponding products **94.3** are obtained.

Scheme **95** illustrates the preparation of substituted benzenesulfonic acids in which the phosphonate group is attached by means of a saturated or unsaturated alkylene group. In this procedure, a halo-substituted benzenesulfonic acid **95.1** is coupled, in a palladium catalyzed Heck reaction with a dialkyl alkenyl phosphonate **95.2** to afford the phosphonate **95.3.** Optionally, the product is reduced, for example by catalytic hydrogenation over a palladium catalyst, to give the saturated analog **95.4.**
For example, 4-amino-3-chlorobenzenesulfonic aid **95.5** (Acros) is reacted in N-methylpyrrolidinone solution at 80° with a dialkyl vinylphosphonate **95.6** (Aldrich), palladium (II) chloride bis(acetonitrile), sodium acetate and tetraphenylphosphonium chloride, as described in Ang. Chem. Int. Ed. Engl., 37, 481, 1998, to produce the olefinic product 95.7. Catalytic hydrogenation using a 5% palladium on carbon catalyst then affords the saturated analog **95.8.**
Using the above procedures, but employing, in place of the chloro compound **95.5,** different chlorides **95.1,** and/or different phosphonates **95.2,** the corresponding products **95.3** and **95.4** are obtained.

Scheme **96** depicts the preparation of benzenesulfonic acids in which the phosphonate group is attached by means of an amide linkage. In this procedure, an amino carboxy substituted benzene thiol **96.1** is coupled with a dialkyl aminoalkyl phosphonate **96.2** to produce the amide **96.3.** The product is then oxidized, as described above, to afford the corresponding sulfonic acid **96.4.**
For example, 2-amino-5-mercaptobenzoic acid **96.5,** prepared as described in Pharmazie, 1973, 28, 433, is reacted with a dialkyl aminoethyl phosphonate **96.6** and dicyclohexyl carbodiimide, to prepare the amide **96.7.** The product is then oxidized with aqueous hydrogen peroxide to yield the sulfonic acid **96.8.**
Using the above procedures, but employing, in place of the carboxylic acid **96.5,** different acids **96.1,** and/or different phosphonates **96.2,** the corresponding products **96.4** are obtained.

Scheme **97** illustrates the conversion of benzenesulfonic acids into the corresponding sulfonyl chlorides. The conversion of sulfonic acids into sulfonyl chlorides is described in Synthetic Organic Chemistry, R. B. Wagner, H. D. Zook, Wiley, 1953, p. 821. The transformation is effected by the use of reagents such as thionyl chloride or phosphorus pentachloride.
For example, as shown in Scheme **97,** the variously substituted phosphonate-containing benzenesulfonic acids **97.1,** prepared as described above, are treated with thionyl chloride, oxalyl chloride, phosphorus pentachloride, phosphorus oxychloride and the like to prepare the corresponding sulfonyl chlorides **97.2.**

Scheme **97a** illustrates the conversion of thiols into the corresponding sulfonyl chlorides which can be applied to any of the thiol intermediates in Schemes **92-96.** The thiol is oxidized as described in Synthesis 1987, 4, 409 or J. Med. Chem. 1980, 12, 1376 to afford the sulfonyl chloride directly. For example, treatment of protected thiol **97a.1,** prepared from **96.7** using standard protecting groups for amines as described in Greene and Wuts, third edition, ch 7, with HC1 and chlorine affords the sulfonyl chloride **97a.2.** Alternatively treatment of **92.10** with the same conditions gives the sulfonyl chloride **97a.3.**

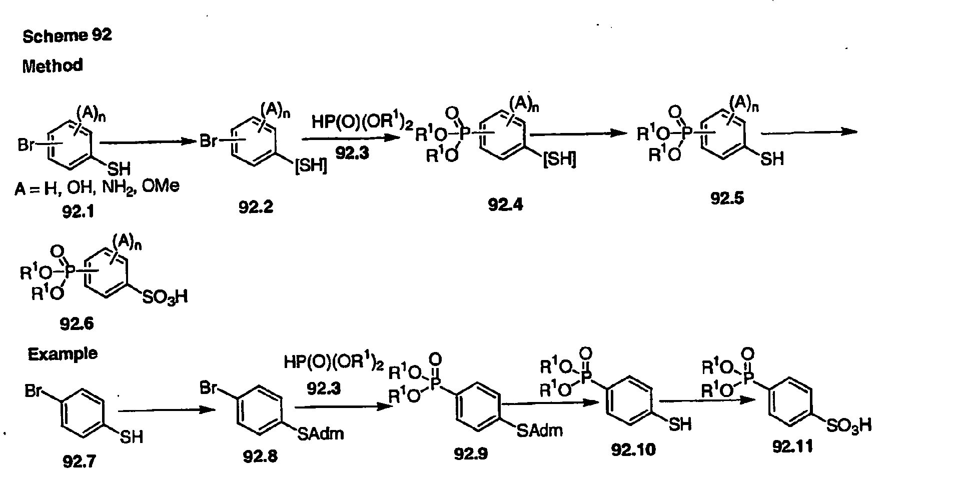

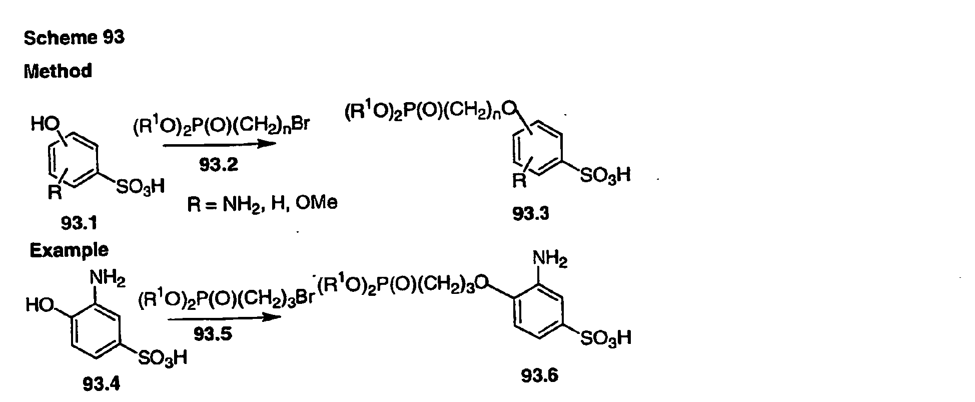

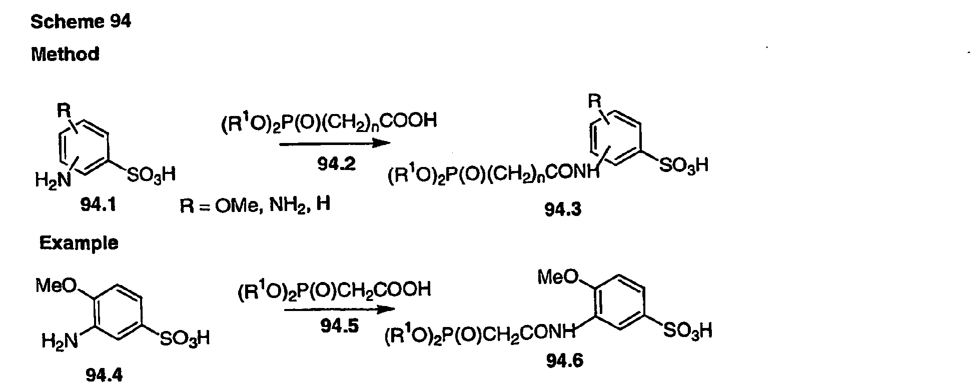

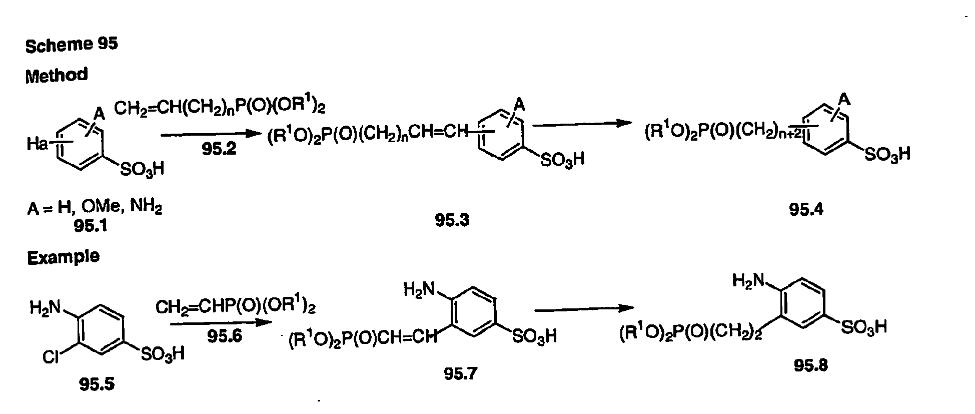

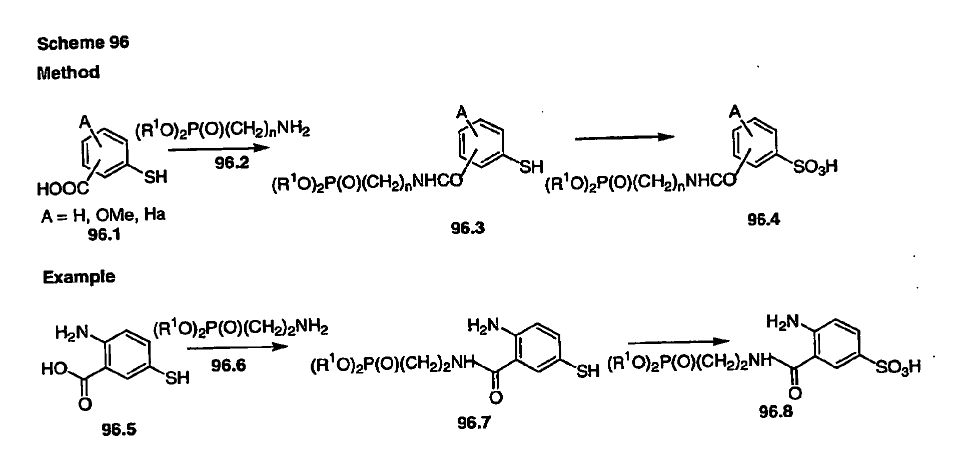

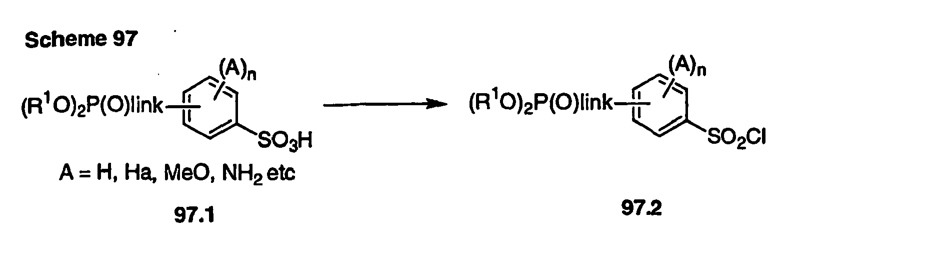

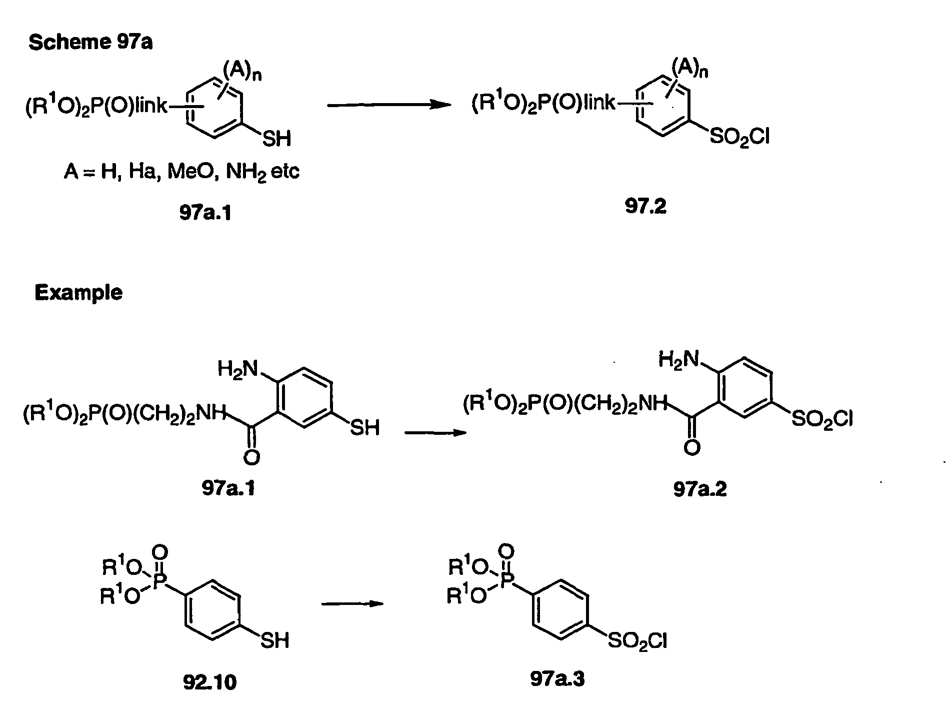

### Preparation of carbamates.

The phosphonate esters **1 - 4** in which R⁴ is formally derived from the carboxylic acids shown in Chart **5c,** and the phosphonate esters **5** and **9** contain a carbamate linkage. The preparation of carbamates is described in Comprehensive Organic Functional Group Transformations, A. R. Katritzky, ed., Pergamon, 1995, Vol. 6, p. 416ff, and in Organic Functional Group Preparations, by S. R. Sandler and W. Karo, Academic Press, 1986, p. 260ff.

Scheme **98** illustrates various methods by which the carbamate linkage is synthesized. As shown in Scheme **98,** in the general reaction generating carbamates, a carbinol **98.1,** is converted into the activated derivative **98.2** in which Lv is a leaving group such as halo, imidazolyl, benztriazolyl and the like, as described below. The activated derivative **98.2** is then reacted with an amine **98.3,** to afford the carbamate product **98.4.** Examples **1- 7** in Scheme **98** depict methods by which the general reaction is effected. Examples **8 -10** illustrate alternative methods for the preparation of carbamates.
Scheme **98,** Example 1 illustrates the preparation of carbamates employing a chloroformyl derivative of the carbinol **98.1.** In this procedure, the carbinol is reacted with phosgene, in an inert solvent such as toluene, at about 0°, as described in Org. Syn. Coll. Vol. 3, 167, 1965, or with an equivalent reagent such as trichloromethoxy chloroformate, as described in Org. Syn. Coll. Vol. 6, 715, 1988, to afford the chloroformate **98.6.** The latter compound is then reacted with the amine component **98.3,** in the presence of an organic or inorganic base, to afford the carbamate **98.7.** For example, the chloroformyl compound **98.6** is reacted with the amine **98.3** in a water-miscible solvent such as tetrahydrofuran, in the presence of aqueous sodium hydroxide, as described in Org. Syn. Coll. Vol. 3, 167, 1965, to yield the carbamate **98.7.** Alternatively, the reaction is performed in dichloromethane in the presence of an organic base such as diisopropylethylamine or dimethylaminopyridine.
Scheme **98,** Example 2 depicts the reaction of the chloroformate compound **98.6** with imidazole to produce the imidazolide **98.8.** The imidazolide product is then reacted with the amine **98.3** to yield the carbamate **98.7.** The preparation of the imidazolide is performed in an aprotic solvent such as dichloromethane at 0°, and the preparation of the carbamate is conducted in a similar solvent at ambient temperature, optionally in the presence of a base such as dimethylaminopyridine, as described in J. Med. Chem., 1989, 32, 357.
Scheme **98** Example 3, depicts the reaction of the chloroformate **98.6** with an activated hydroxyl compound R"OH, to yield the mixed carbonate ester **98.10.** The reaction is conducted in an inert organic solvent such as ether or dichloromethane, in the presence of a base such as dicyclohexylamine or triethylamine. The hydroxyl component R"OH is selected from the group of compounds **98.19 - 98.24** shown in Scheme **98,** and similar compounds. For example, if the component R"OH is hydroxybenztriazole **98.19,** N-hydroxysuccinimide **98.20,** or pentachlorophenol, **98.21,** the mixed carbonate **98.10** is obtained by the reaction of the chloroformate with the hydroxyl compound in an ethereal solvent in the presence of dicyclohexylamine, as described in Can. J. Chem., 1982, 60, 976. A similar reaction in which the component R"OH is pentafluorophenol **98.22** or 2-hydroxypyridine **98.23** is performed in an ethereal solvent in the presence of triethylamine, as descnbed in Syn., 1986, 303, and Chem Ber. 118, 468, 1985.
Scheme **98** Example **4** illustrates the preparation of carbamates in which an alkyloxycarbonylimidazole **98.8** is employed. In this procedure, a carbinol **98.5** is reacted with an equimolar amount of carbonyl diimidazole **98.11** to prepare the intermediate **98.8.** The reaction is conducted in an aprotic organic solvent such as dichloromethane or tetrahydrofuran. The acyloxyimidazole **98.8** is then reacted with an equimolar amount of the amine R'NH₂ to afford the carbamate **98.7.** The reaction is performed in an aprotic organic solvent such as dichloromethane, as described in Tet. Lett., 42, 2001, 5227, to afford the carbamate **98.7.**
Scheme **98**, Example **5** illustrates the preparation of carbamates by means of an intermediate alkoxycarbonylbenztriazole **98.13.** In this procedure, a carbinol ROH is reacted at ambient temperature with an equimolar amount of benztriazole carbonyl chloride **98.12,** to afford the alkoxycarbonyl product **98.13.** The reaction is performed in an organic solvent such as benzene or toluene, in the presence of a tertiary organic amine such as triethylamine, as described in Syn., 1977, 704. The product is then reacted with the amine R'NH₂ to afford the carbamate **98.7.** The reaction is conducted in toluene or ethanol, at from ambient temperature to about 80° as described in Syn., 1977, 704.
Scheme **98**, Example **6** illustrates the preparation of carbamates in which a carbonate (R"O)₂CO, **98.14,** is reacted with a carbinol **98.5** to afford the intermediate alkyloxycarbonyl intermediate **98.15.** The latter reagent is then reacted with the amine R'NH₂ to afford the carbamate **98.7.** The procedure in which the reagent **98.15** is derived from hydroxybenztriazole **98.19** is described in Synthesis, 1993, 908; the procedure in which the reagent **98.15** is derived from N-hydroxysuccinimide **98.20** is described in Tet. Lett., 1992, 2781; the procedure in which the reagent **98.15** is derived from 2-hydroxypyridine **98.23** is described in Tet. Lett., 1991,4251; the procedure in which the reagent **98.15** is derived from 4-nitrophenol **98.24** is described in Syn. 1993, 199. The reaction between equimolar amounts of the carbinol ROH and the carbonate **98.14** is conducted in an inert organic solvent at ambient temperature.

Scheme **98**, Example **7** illustrates the preparation of carbamates from alkoxycarbonyl azides **98.16.** In this procedure, an alkyl chloroformate **98.6** is reacted with an azide, for example sodium azide, to afford the alkoxycarbonyl azide **98.16.** The latter compound is then reacted with an equimolar amount of the amine R'NH₂ to afford the carbamate **98.7.** The reaction is conducted at ambient temperature in a polar aprotic solvent such as dimethylsulfoxide, for example as described in Syn., 1982, 404.
Scheme **98**, Example **8** illustrates the preparation of carbamates by means of the reaction between a carbinol ROH and the chloroformyl derivative of an amine **98.17.** In this procedure, which is described in Synthetic Organic Chemistry, R. B. Wagner, H. D. Zook, Wiley, 1953, p. 647, the reactants are combined at ambient temperature in an aprotic solvent such as acetonitrile, in the presence of a base such as triethylamine, to afford the carbamate **98.7.** Scheme **98,** Example **9** illustrates the preparation of carbamates by means of the reaction between a carbinol ROH and an isocyanate **98.18.** In this procedure, which is described in Synthetic Organic Chemistry, R. B. Wagner, H. D. Zook, Wiley, 1953, p. 645, the reactants are combined at ambient temperature in an aprotic solvent such as ether or dichloromethane and the like, to afford the carbamate **98.7.**
Scheme **98**, Example **10** illustrates the preparation of carbamates by means of the reaction between a carbinol ROH and an amine R'NH₂. In this procedure, which is described in Chem Lett. 1972, 373, the reactants are combined at ambient temperature in an aprotic organic solvent such as tetrahydrofuran, in the presence of a tertiary base such as triethylamine, and selenium. Carbon monoxide is passed through the solution and the reaction proceeds to afford the carbamate **98.7.**

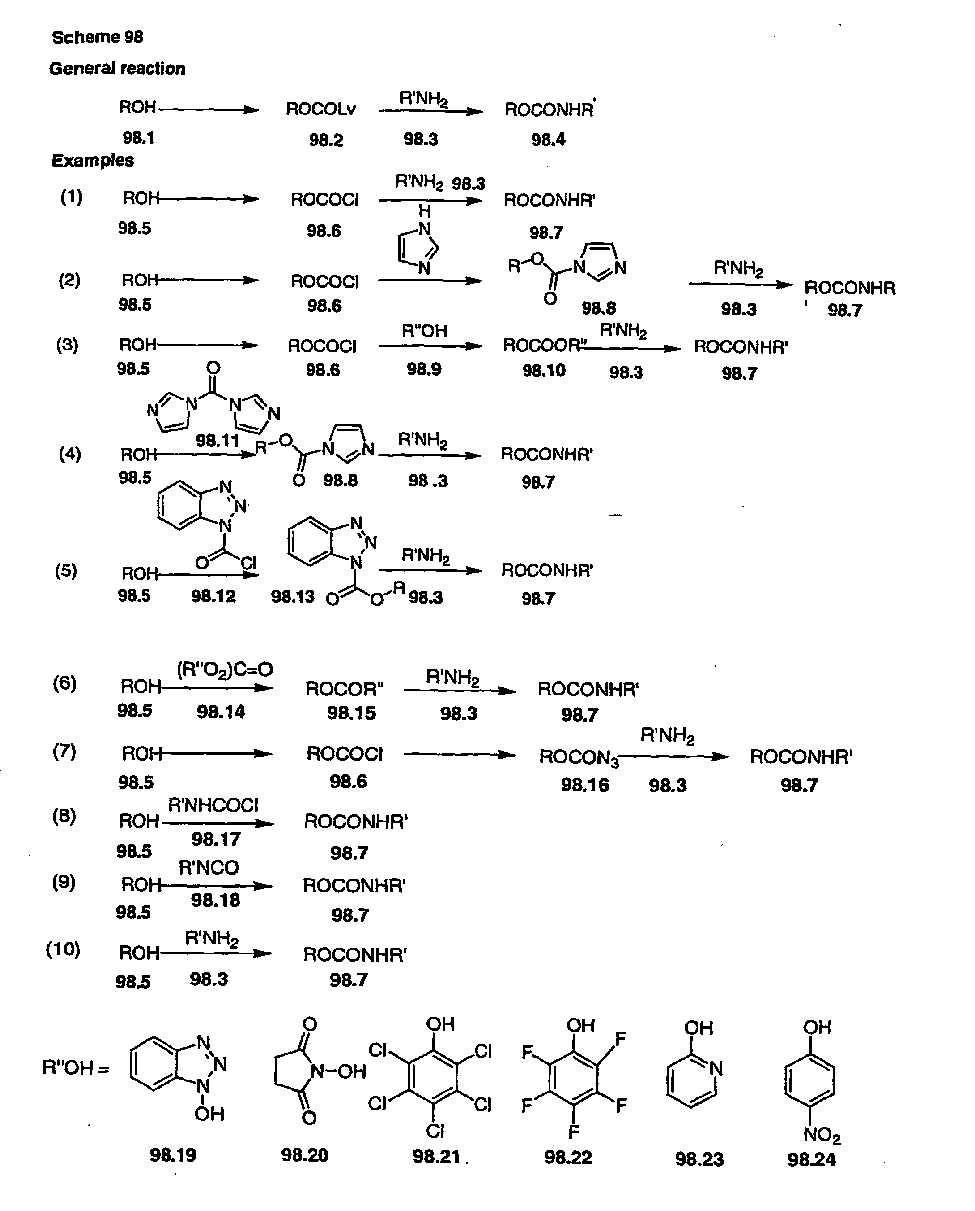

### Interconversions of the phosphonates R-link-P(O)(OR¹)₂, R-link-P(O)(OR¹)(OH) and R-link-P(O)(OH)₂.

Schemes **1- 97** described the preparations of phosphonate esters of the general structure R-link-P(O)(OR¹)₂, in which the groups R¹, the structures of which are defined in Charts **1** and **2,** may be the same or different. The R¹ groups attached to the phosphonate esters **1 - 13,** or to precursors thereto, may be changed using established chemical transformations. The interconversions reactions of phosphonates are illustrated in Scheme **99.** The group R in Scheme **99** represents the substructure to which the substituent link-P(O)(OR¹)₂ is attached, either in the compounds **1 - 13** or in precursors thereto. The R¹ group may be changed, using the procedures described below, either in the precursor compounds, or in the esters **1 - 13.** The methods employed for a given phosphonate transformation depend on the nature of the substituent R¹. The preparation and hydrolysis of phosphonate esters is described in Organic Phosphorus Compounds, G. M. Kosolapoff, L. Maeir, eds, Wiley, 1976, p. 9ff.

The conversion of a phosphonate diester **99.1** into the corresponding phosphonate monoester **99.2** (Scheme **99,** Reaction **1**) is accomplished by a number of methods. For example, the ester **99.1** in which R¹ is an aralkyl group such as benzyl, is converted into the monoester compound **99.2** by reaction with a tertiary organic base such as diazabicyclooctane (DABCO) or quinuclidine, as described in J. Org. Chem, 1995, 60, 2946. The reaction is performed in an inert hydrocarbon solvent such as toluene or xylene, at about 110°. The conversion of the diester **99.1** in which R¹ is an aryl group such as phenyl, or an alkenyl group such as allyl, into the monoester **99.2** is effected by treatment of the ester **99.1** with a base such as aqueous sodium hydroxide in acetonitrile or lithium hydroxide in aqueous tetrahydrofuran. Phosphonate diesters **99.1** in which one of the groups R¹ is aralkyl, such as benzyl, and the other is alkyl, are converted into the monoesters **99.2** in which R¹ is alkyl by hydrogenation, for example using a palladium on carbon catalyst. Phosphonate diesters in which both of the groups R¹ are alkenyl, such as allyl, are converted into the monoester **99.2** in which R¹ is alkenyl, by treatment with chlorotris(triphenylphosphine)rhodium (Wilkinson's catalyst) in aqueous ethanol at reflux, optionally in the presence of diazabicyclooctane, for example by using the procedure described in J. Org. Chem., 38, 3224, 1973 for the cleavage of allyl carboxylates.

The conversion of a phosphonate diester **99.1** or a phosphonate monoester **99.2** into the corresponding phosphonic acid **99.3** (Scheme **99,** Reactions **2** and **3**) is effected by reaction of the diester or the monoester with trimethylsilyl bromide, as described in J. Chem. Soc., Chem. Comm., 739, 1979. The reaction is conducted in an inert solvent such as, for example, dichloromethane, optionally in the presence of a silylating agent such as bis(trimethylsilyl)triffuoroacetamide, at ambient temperature. A phosphonate monoester **99.2** in which R¹ is aralkyl such as benzyl, is converted into the corresponding phosphonic acid **99.3** by hydrogenation over a palladium catalyst, or by treatment with hydrogen chloride in an ethereal solvent such as dioxan. A phosphonate monoester **99.2** in which R¹ is alkenyl such as, for example, allyl, is converted into the phosphonic acid **99.3** by reaction with Wilkinson's catalyst in an aqueous organic solvent, for example in 15% aqueous acetonitrile, or in aqueous ethanol, for example using the procedure described in Helv. Chim. Acta., 68, 618, 1985. Palladium catalyzed hydrogenolysis of phosphonate esters **99.1** in which R¹ is benzyl is described in J. Org. Chem., 24, 434, 1959. Platinum-catalyzed hydrogenolysis of phosphonate esters **99.1** in which R¹ is phenyl is described in J. Am. Chem Soc., 78, 2336, 1956.

The conversion of a phosphonate monoester **99.2** into a phosphonate diester **99.1** (Scheme **99,** Reaction **4**) in which the newly introduced R¹ group is alkyl, aralkyl, haloalkyl such as chloroethyl, or aralkyl is effected by a number of reactions in which the substrate 99.2 is reacted with a hydroxy compound R¹OH, in the presence of a coupling agent. Suitable coupling agents are those employed for the preparation of carboxylate esters, and include a carbodiimide such as dicyclohexylcarbodiimide, in which case the reaction is preferably conducted in a basic organic solvent such as pyridine, or (benzotriazol-1-yloxy)tripyrrolidinophosphonium hexafluorophosphate (PYBOP, Sigma), in which case the reaction is performed in a polar solvent such as dimethylformamide, in the presence of a tertiary organic base such as diisopropylethylamine, or Aldrithiol-2 (Aldrich) in which case the reaction is conducted in a basic solvent such as pyridine, in the presence of a triaryl phosphine such as triphenylphosphine. Alternatively, the conversion of the phosphonate monoester **99.2** to the diester **99.1** is effected by the use of the Mitsonobu reaction, as described above, Scheme **49.** The substrate is reacted with the hydroxy compound R¹OH, in the presence of diethyl azodicarboxylate and a triarylphosphine such as triphenyl phosphine. Alternatively, the phosphonate monoester **99.2** is transformed into the phosphonate diester **99.1,** in which the introduced R¹ group is alkenyl or aralkyl, by reaction of the monoester with the halide R¹Br, in which R¹ is as alkenyl or aralkyl. The alkylation reaction is conducted in a polar organic solvent such as dimethylformamide or acetonitrile, in the presence of a base such as cesium carbonate. Alternatively, the phosphonate monoester is transformed into the phosphonate diester in a two step procedure. In the first step, the phosphonate monoester **99.2** is transformed into the chloro analog RP(O)(OR¹)Cl by reaction with thionyl chloride or oxalyl chloride and the like, as described in Organic Phosphorus Compounds, G. M. Kosolapoff, L. Maeir, eds, Wiley, 1976, p. 17, and the thus-obtained product RP(O)(OR¹)Cl is then reacted with the hydroxy compound R¹OH, in the presence of a base such as triethylamine, to afford the phosphonate diester **99.1.**

A phosphonic acid R-link-P(O)(OH)₂ is transformed into a phosphonate monoester RP(O)(OR¹)(OH) (Scheme **99,** Reaction **5**) by means of the methods described above of for the preparation of the phosphonate diester R-link-P(O)(OR¹)₂ **99.1,** except that only one molar proportion of the component R¹OH or R¹Br is employed.

A phosphonic acid R-link-P(O)(OH)₂ **99.3** is transformed into a phosphonate diester R-link-P(O)(OR¹)₂ **99.1** (Scheme **99,** Reaction **6**) by a coupling reaction with the hydroxy compound R¹OH, in the presence of a coupling agent such as Aldrithiol-2 (Aldrich) and triphenylphosphine. The reaction is conducted in a basic solvent such as pyridine. Alternatively, phosphonic acids **99.3** are transformed into phosphonic esters **99.1** in which R¹ is aryl, by means of a coupling reaction employing, for example, dicyclohexylcarbodiimide in pyridine at ca 70°. Alternatively, phosphonic acids **99.3** are transformed into phosphonic esters **99.1** in which R¹ is alkenyl, by means of an alkylation reaction. The phosphonic acid is reacted with the alkenyl bromide R¹Br in a polar organic solvent such as acetonitrile solution at reflux temperature, the presence of a base such as cesium carbonate, to afford the phosphonic ester **99.1.**

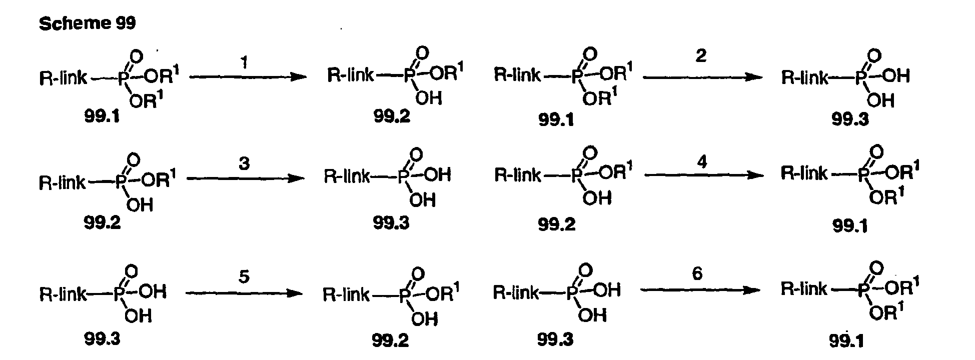

General applicability of methods for introduction of phosphonate substituents. The procedures described for the introduction of phosphonate moieties (Schemes **47 - 97**) are, with appropriate modifications known to one skilled in the art, transferable to different chemical substrates. Thus, the methods described above for the introduction of phosphonate groups into hydroxymethyl benzoic acids, (Schemes **47 - 51**) are applicable to the introduction of phosphonate moieties into quinolines, thiophenols, isobutylamines, cyclopentylamines, tert. butanols, benzyl alcohols, phenylalanines, benzylamines and benzenesulfonic acids, and the methods described for the introduction of phosphonate moieties into the above-named substrates (Schemes **52 - 97**) are applicable to the introduction of phosphonate moieties into hydroxymethyl benzoic acid substrates.

### Preparation of phosphonate intermediates 11 - 13 with phosphonate moieties incorporated into the R², R³ or R⁴ groups.

The chemical transformations described in Schemes **1- 99** illustrate the preparation of compounds **1-10** in which the phosphonate ester moiety is attached to the substructures listed above. The various chemical methods employed for the introduction of phosphonate ester groups into the above-named moieties can, with appropriate modifications known to those skilled in the art, be applied to the introduction of a phosphonate ester group into the compounds R⁴COOH, R³Cl, R²NH₂. The resultant phosphonate-containing analogs, designated as R^{4a}COOH, R^{3a} Cl and NH₂R^{2a} are then, using the procedures described above, employed in the preparation of the compounds **11, 12** and **13.** The procedures required for the utilization of the phosphonate-containing analogs are the same as those described above for the utilization of the compounds R²NH₂, R³Cl and R⁴COOH.

### Scheme General Section

General aspects of these exemplary methods are described below and in the Example. Each of the products of the following processes is optionally separated, isolated, and/or purified prior to its use in subsequent processes.

The terms "treated", "treating", "treatment", and the like, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two.

"Treating" indicates the reasonable and usual manner in which organic chemicals are allowed to react. Normal concentrations (0.01M to 10M, typically 0.1M to 1M), temperatures (-100°C to 250°C, typically -78°C to 150°C, more typically -78°C to 100°C, still more typically 0°C to 100°C), reaction vessels (typically glass, plastic, metal), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis is used in selecting the conditions and apparatus for "treating" in a given process. In particular, one of ordinary skill in the art of organic synthesis selects conditions and apparatus reasonably expected to successfully carry out the chemical reactions of the described processes based on the knowledge in the art.

Modifications of each of the exemplary schemes above and in the examples (hereafter "exemplary schemes") leads to various analogs of the specific exemplary materials produce. The above cited citations describing suitable methods of organic synthesis are applicable to such modifications.

In each of the exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example, size exclusion or ion exchange chromatography, high, medium, or low pressure liquid chromatography, small scale and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

All literature and patent citations above are hereby expressly incorporated by reference at the locations of their citation. Specifically cited sections or pages of the above cited works are incorporated by reference with specificity. The invention has been described in detail sufficient to allow one of ordinary skill in the art to make and use the subject matter of the following Embodiments. It is apparent that certain modifications of the methods and compositions of the following Embodiments can be made within the scope and spirit of the invention.

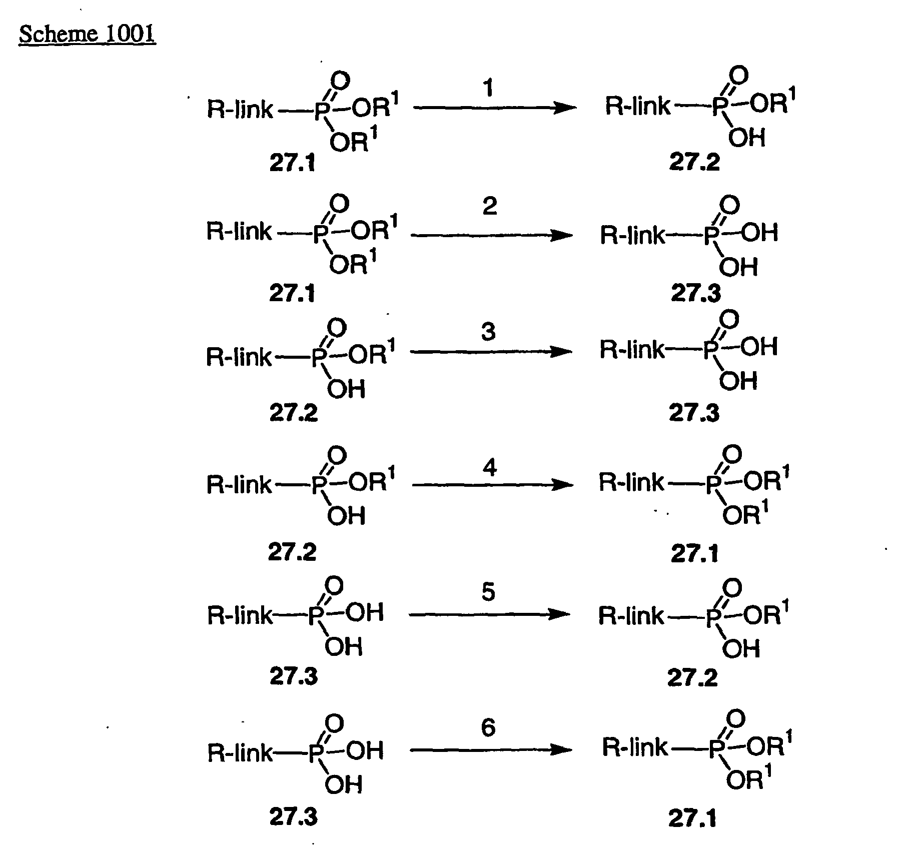

Scheme 1001 shows the interconversions of certain phosphonate compounds: acids - P(O)(OH)₂; mono-esters -P(O)(OR₁)(OH); and diesters -P(O)(OR₁)₂ in which the R¹ groups are independently selected, and defined herein before, and the phosphorus is attached through a carbon moiety (link, i.e. linker), which is attached to the rest of the molecule, e.g. drug or drug intermediate (R). The R¹ groups attached to the phosphonate esters in Scheme 1001 may be changed using established chemical transformations. The interconversions may be carried out in the precursor compounds or the final products using the methods described below. The methods employed for a given phosphonate transformation depend on the nature of the substituent R¹. The preparation and hydrolysis of phosphonate esters is described in Organic Phosphorus Compounds, G. M. Kosolapoff, L. Maeir, eds, Wiley, 1976, p. 9ff.

The conversion of a phosphonate diester **27.1** into the corresponding phosphonate monoester **27.2** (Scheme 1001, Reaction 1) can be accomplished by a number of methods. For example, the ester **27.1** in which R¹ is an arylalkyl group such as benzyl, can be converted into the monoester compound **27.2** by reaction with a tertiary organic base such as diazabicyclooctane (DABCO) or quinuclidine, as described in J. Org. Chem., 1995, 60:2946. The reaction is performed in an inert hydrocarbon solvent such as toluene or xylene, at about 110°C. The conversion of the diester **27.1** in which R¹ is an aryl group such as phenyl, or an alkenyl group such as allyl, into the monoester **27.2** can be effected by treatment of the ester **27.1** with a base such as aqueous sodium hydroxide in acetonitrile or lithium hydroxide in aqueous tetrahydrofuran. Phosphonate diesters **27.2** in which one of the groups R¹ is arylalkyl, such as benzyl, and the other is alkyl, can be converted into the monoesters **27.2** in which R¹ is alkyl, by hydrogenation, for example using a palladium on carbon catalyst. Phosphonate diesters in which both of the groups R¹ are alkenyl, such as allyl, can be converted into the monoester **27.2** in which R¹ is alkenyl, by treatment with chlorotris(triphenylphosphine)rhodium (Wilkinson's catalyst) in aqueous ethanol at reflux, optionally in the presence of diazabicyclooctane, for example by using the procedure described in J. Org. Chem., 38:3224 1973 for the cleavage of allyl carboxylates.

The conversion of a phosphonate diester **27.1** or a phosphonate monoester **27.2** into the corresponding phosphonic acid **27.3** (Scheme 1001, Reactions 2 and 3) can be effected by reaction of the diester or the monoester with trimethylsilyl bromide, as described in J. Chem. Soc., Chem. Comm., 739, 1979. The reaction is conducted in an inert solvent such as, for example, dichloromethane, optionally in the presence of a silylating agent such as bis(trimethylsilyl)trifluoroacetamide, at ambient temperature. A phosphonate monoester **27.2** in which R¹ is arylalkyl such as benzyl, can be converted into the corresponding phosphonic acid 27.3 by hydrogenation over a palladium catalyst, or by treatment with hydrogen chloride in an ethereal solvent such as dioxane. A phosphonate monoester **27.2** in which R¹ is alkenyl such as, for example, allyl, can be converted into the phosphonic acid **27.3** by reaction with Wilkinson's catalyst in an aqueous organic solvent, for example in 15% aqueous acetonitrile, or in aqueous ethanol, for example using the procedure described in Helv. Chim. Acta., 68:618, 1985. Palladium catalyzed hydrogenolysis of phosphonate esters **27.1** in which R¹ is benzyl is described in J. Org. Chem., 24:434, 1959. Platinum-catalyzed hydrogenolysis of phosphonate esters **27.1** in which R¹ is phenyl is described in J. Amer. Chem. Soc., 78:2336, 1956.

The conversion of a phosphonate monoester **27.2** into a phosphonate diester **27.1** (Scheme 1001, Reaction 4) in which the newly introduced R¹ group is alkyl, arylalkyl, or haloalkyl such as chloroethyl, can be effected by a number of reactions in which the substrate **27.2** is reacted with a hydroxy compound R¹OH, in the presence of a coupling agent. Suitable coupling agents are those employed for the preparation of carboxylate esters, and include a carbodiimide such as dicyclohexylcarbodiimide, in which case the reaction is preferably conducted in a basic organic solvent such as pyridine, or (benzotriazol-1-yloxy)tripyrrolidinophosphonium hexafluorophosphate (PYBOP, Sigma), in which case the reaction is performed in a polar solvent such as dimethylformamide, in the presence of a tertiary organic base such as diisopropylethylamine, or Aldrithiol-2 (Aldrich) in which case the reaction is conducted in a basic solvent such as pyridine, in the presence of a triaryl phosphine such as triphenylphosphine. Alternatively, the conversion of the phosphonate monoester **27.1** to the diester **27.1** can be effected by the use of the Mitsunobu reaction. The substrate is reacted with the hydroxy compound R¹OH, in the presence of diethyl azodicarboxylate and a triarylphosphine such as triphenyl phosphine. Alternatively, the phosphonate monoester **27.2** can be transformed into the phosphonate diester **27.1,** in which the introduced R¹ group is alkenyl or arylalkyl, by reaction of the monoester with the halide R¹Br, in which R¹ is as alkenyl or arylalkyl. The alkylation reaction is conducted in a polar organic solvent such as dimethylformamide or acetonitrile, in the presence of a base such as cesium carbonate. Alternatively, the phosphonate monoester can be transformed into the phosphonate diester in a two step procedure. In the first step, the phosphonate monoester **27.2** is transformed into the chloro analog -P(O)(OR¹)Cl by reaction with thionyl chloride or oxalyl chloride and the like, as described in Organic Phosphorus Compounds, G. M. Kosolapoff, L. Maeir, eds, Wiley, 1976, p. 17, and the thus-obtained product -P(O)(OR¹)Cl is then reacted with the hydroxy compound R¹OH, in the presence of a base such as triethylamine, to afford the phosphonate diester **27.1.**

A phosphonic acid -P(O)(OH)₂ can be transformed into a phosphonate monoester -P(O)(OR¹)(OH) (Scheme 1001, Reaction 5) by means of the methods described above of for the preparation of the phosphonate diester -P(O)(OR¹)₂ **27.1,** except that only one molar proportion of the component R¹OH or R¹Br is employed.

A phosphonic acid -P(O)(OH)₂ **27.3** can be transformed into a phosphonate diester -P(O)(OR¹)₂ **27.1** (Scheme 1, Reaction 6) by a coupling reaction with the hydroxy compound R¹OH, in the presence of a coupling agent such as Aldrithiol-2 (Aldrich) and triphenylphosphine. The reaction is conducted in a basic solvent such as pyridine. Alternatively, phosphonic acids **27.3** can be transformed into phosphonic esters **27.1** in which R¹ is aryl, such as phenyl, by means of a coupling reaction employing, for example, phenol and dicyclohexylcarbodiimide in pyridine at about 70°C. Alternatively, phosphonic acids **27.3** can be transformed into phosphonic esters **27.1** in which R¹ is alkenyl, by means of an alkylation reaction. The phosphonic acid is reacted with the alkenyl bromide R¹Br in a polar organic solvent such as acetonitrile solution at reflux temperature, in the presence of a base such as cesium carbonate, to afford the phosphonic ester **27.1.**

Amino alkyl phosphonate compounds 809: 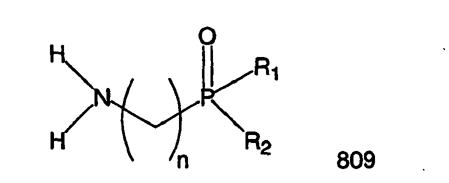 are a generic representative of compounds 811, 813, 814, 816 and 818. Some methods to prepare embodiments of 809 are shown in Scheme 1002. Commercial amino phosphonic acid 810 was protected as carbamate 811. The phosphonic acid 811 was converted to phosphonate 812 upon treatment with ROH in the presence of DCC or other conventional coupling reagents. Coupling of phosphonic acid 811 with esters of amino acid 820 provided bisamidate 817. Conversion of acid 811 to bisphenyl phosphonate followed by hydrolysis gave mono-phosphonic acid 814 (Cbz = C₆H₅CH₂C(O)-), which was then transformed to mono-phosphonic amidate 815. Carbamates 813, 816 and 818 were converted to their corresponding amines upon hydrogenation. Compounds 811, 813, 814, 816 and 818 are useful intermediates to form the phosphonate compounds of the invention.

### Preparation of carboalkoxy-substituted phosphonate bisamidates, monoamidates, diesters and monoesters.

A number of methods are available for the conversion of phosphonic acids into amidates and esters. In one group of methods, the phosphonic acid is either converted into an isolated activated intermediate such as a phosphoryl chloride, or the phosphonic acid is activated in situ for reaction with an amine or a hydroxy compound.

The conversion of phosphonic acids into phosphoryl chlorides is accomplished by reaction with thionyl chloride, for example as described in J. Gen. Chem USSR, 1983, 53, 480, Zh. Obschei Khim., 1958, 28, 1063, or J. Org. Chem, 1994, 59, 6144, or by reaction with oxalyl chloride, as described in J. Am. Chem. Soc., 1994, 116, 3251, or J. Org. Chem, 1994, 59, 6144, or by reaction with phosphorus pentachloride, as described in J. Org. Chem., 2001, 66, 329, or in J. Med. Chem, 1995, 38,1372. The resultant phosphoryl chlorides are then reacted with amines or hydroxy compounds in the presence of a base to afford the amidate or ester products.

Phosphonic acids are converted into activated imidazolyl derivatives by reaction with carbonyl diimidazole, as described in J. Chem. Soc., Chem Comm., 1991, 312, or Nucleosides Nucleotides 2000, 19, 1885. Activated sulfonyloxy derivatives are obtained by the reaction of phosphonic acids with trichloromethylsulfonyl chloride, as described in J. Med. Chem. 1995, 38, 4958, or with triisopropylbenzenesulfonyl chloride, as described in Tet. Lett., 1996, 7857, or Bioorg. Med. Chem. Lett., 1998, 8, 663. The activated sulfonyloxy derivatives are then reacted with amines or hydroxy compounds to afford amidates or esters. Alternatively, the phosphonic acid and the amine or hydroxy reactant are combined in the presence of a diimide coupling agent. The preparation of phosphonic amidates and esters by means of coupling reactions in the presence of dicyclohexyl carbodiimide is described, for example, in J. Chem. Soc., Chem. Comm., 1991, 312, or J. Med. Chem, 1980, 23, 1299 or Coll. Czech. Chem Comm, 1987, 52, 2792. The use of ethyl dimethylaminopropyl carbodiimide for activation and coupling of phosphonic acids is described in Tet. Lett., 2001, 42, 8841, or Nucleosides Nucleotides, 2000, 19, 1885.

A number of additional coupling reagents have been described for the preparation of amidates and esters from phosphonic acids. The agents include Aldrithiol-2, and PYBOP and BOP, as described in J. Org. Chem, 1995, 60, 5214, and J. Med. Chem., 1997, 40, 3842, mesitylene-2-sulfonyl-3-nitro-1,2,4-triazole (MSNT), as described in J. Med. Chem., 1996, 39, 4958, diphenylphosphoryl azide, as described in J. Org. Chem., 1984, 49, 1158, 1-(2,4,6-triisopropylbenzenesulfonyl-3-nitro-1,2,4-triazole (TPSNT) as described in Bioorg. Med. Chem Lett., 1998, 8, 1013, bromotris(dimethylamino)phosphonium hexafluorophosphate (BroP), as described in Tet. Lett., 1996, 37, 3997, 2-chloro-5,5-dimethyl-2-oxo-1,3,2-dioxaphosphinane, as described in Nucleosides Nucleotides 1995, 14, 871, and diphenyl chlorophosphate, as described in J. Med. Chem., 1988, 31, 1305..

Phosphonic acids are converted into amidates and esters by means of the Mitsonobu reaction, in which the phosphonic acid and the amine or hydroxy reactant are combined in the presence of a triaryl phosphine and a dialkyl azodicarboxylate. The procedure is described in Org. Lett., 2001, 3, 643, or J. Med. Chem., 1997, 40, 3842.

Phosphonic esters are also obtained by the reaction between phosphonic acids and halo compounds, in the presence of a suitable base. The method is described, for example, in Anal. Chem, 1987, 59, 1056, or J. Chem Soc. Perkin Trans., 1,1993,19, 2303, or J. Med. Chem., 1995, 38, 1372, or Tet. Lett., 2002, 43, 1161.

Schemes **1- 4** illustrate the conversion of phosphonate esters and phosphonic acids into carboalkoxy-substituted phosphorobisamidates (Scheme **1**), phosphoroamidates (Scheme **2**), phosphonate monoesters (Scheme **3**) and phosphonate diesters, (Scheme **4**).

Scheme **1** illustrates various methods for the conversion of phosphonate diesters **1.1** into phosphorobisamidates **1.5.** The diester **1.1,** prepared as described previously, is hydrolyzed, either to the monoester **1.2** or to the phosphonic acid **1.6.** The methods employed for these transformations are described above. The monoester **1.2** is converted into the monoamidate **1.3** by reaction with an aminoester **1.9,** in which the group R² is H or alkyl, the group R⁴ is an alkylene moiety such as, for example, CHCH₃, CHPr^{I}, CH(CH₂Ph), CH₂CH(CH₃) and the like, or a group present in natural or modified aminoacids, and the group R⁵ is alkyl. The reactants are combined in the presence of a coupling agent such as a carbodiimide, for example dicyclohexyl carbodiimide, as described in J. Am. Chem. Soc., 1957, 79, 3575, optionally in the presence of an activating agent such as hydroxybenztriazole, to yield the amidate product **1.3.** The amidate-forming reaction is also effected in the presence of coupling agents such as BOP, as described in J. Org. Chem, 1995, 60, 5214, Aldrithiol, PYBOP and similar coupling agents used for the preparation of amides and esters. Alternatively, the reactants **1.2** and **1.9** are transformed into the monoamidate **1.3** by means of a Mitsonobu reaction. The preparation of amidates by means of the Mitsonobu reaction is described in J. Med. Chem, 1995, 38, 2742. Equimolar amounts of the reactants are combined in an inert solvent such as tetrahydrofuran in the presence of a triaryl phosphine and a dialkyl azodicarboxylate. The thus-obtained monoamidate ester **1.3** is then transformed into amidate phosphonic acid **1.4.** The conditions used for the hydrolysis reaction depend on the nature of the R¹ group, as described previously. The phosphonic acid amidate **1.4** is then reacted with an aminoester **1.9,** as described above, to yield the bisamidate product **1.5,** in which the amino substituents are the same or different.

An example of this procedure is shown in Scheme **1,** Example **1.** In this procedure, a dibenzyl phosphonate **1.14** is reacted with diazabicyclooctane (DABCO) in toluene at reflux, as described in J. Org. Chem., 1995, 60, 2946, to afford the monobenzyl phosphonate **1.15.** The product is then reacted with equimolar amounts of ethyl alaninate **1.16** and dicyclohexyl carbodiimide in pyridine, to yield the amidate product **1.17.** The benzyl group is then removed, for example by hydrogenolysis over a palladium catalyst, to give the monoacid product **1.18.** This compound is then reacted in a Mitsonobu reaction with ethyl leucinate **1.19,** triphenyl phosphine and diethylazodicarboxylate, as described in J. Med. Chem, 1995, 38, 2742, to produce the bisamidate product **1.20.**

Using the above procedures, but employing, in place of ethyl leucinate **1.19** or ethyl alaninate **1.16,** different aminoesters **1.9,** the corresponding products **1.5** are obtained.

Alternatively, the phosphonic acid **1.6** is converted into the bisamidate **1.5** by use of the coupling reactions described above. The reaction is performed in one step, in which case the nitrogen-related substituents present in the product 1.5 are the same, or in two steps, in which case the nitrogen-related substituents can be different.
An example of the method is shown in Scheme **1**, Example **2**. In this procedure, a phosphonic acid **1.6** is reacted in pyridine solution with excess ethyl phenylalaninate **1.21** and dicyclohexylcarbodiimide, for example as described in J. Chem. Soc., Chem Comm, 1991, 1063, to give the bisamidate product **1.22.**

Using the above procedures, but employing, in place of ethyl phenylalaninate, different aminoesters **1.9,** the corresponding products **1.5** are obtained.

As a further alternative, the phosphonic acid **1.6** is converted into the mono or bis-activated derivative **1.7,** in which Lv is a leaving group such as chloro, imidazolyl, triisopropylbenzenesulfonyloxy etc. The conversion of phosphonic acids into chlorides **1.7** (Lv = Cl) is effected by reaction with thionyl chloride or oxalyl chloride and the like, as described in Organic Phosphorus Compounds, G. M. Kosolapoff, L. Maeir, eds, Wiley, 1976, p. 17. The conversion of phosphonic acids into monoimidazolides **1.7** (Lv = imidazolyl) is described in J. Med. Chem., 2002, 45, 1284 and in J. Chem Soc. Chem. Comm, 1991, 312. Alternatively, the phosphonic acid is activated by reaction with triisopropylbenzenesulfonyl chloride, as described in Nucleosides and Nucleotides, 2000, 10, 1885. The activated product is then reacted with the aminoester **1.9,** in the presence of a base, to give the bisamidate **1.5.** The reaction is performed in one step, in which case the nitrogen substituents present in the product **1.5** are the same, or in two steps, via the intermediate **1.11,** in which case the nitrogen substituents can be different.

Examples of these methods are shown in Scheme 1, Examples **3** and **5**. In the procedure illustrated in Scheme **1,** Example **3,** a phosphonic acid **1.6** is reacted with ten molar equivalents of thionyl chloride, as described in Zh. Obschei Khim., 1958, 28, 1063, to give the dichloro compound **1.23.** The product is then reacted at reflux temperature in a polar aprotic solvent such as acetonitrile, and in the presence of a base such as triethylamine, with butyl serinate **1.24** to afford the bisamidate product **1.25.**

Using the above procedures, but employing, in place of butyl serinate **1.24,** different aminoesters **1.9,** the corresponding products **1.5** are obtained.

In the procedure illustrated in Scheme **1,** Example **5,** the phosphonic acid **1.6** is reacted, as described in J. Chem. Soc. Chem. Comm., 1991, 312, with carbonyl diimidazole to give the imidazolide **1.32.** The product is then reacted in acetonitrile solution at ambient temperature, with one molar equivalent of ethyl alaninate **1.33** to yield the monodisplacement product **1.34.** The latter compound is then reacted with carbonyl diimidazole to produce the activated intermediate **1.35,** and the product is then reacted, under the same conditions, with ethyl N-methylalaninate **1.33a** to give the bisamidate product **1.36.**

Using the above procedures, but employing, in place of ethyl alaninate **1.33** or ethyl N-methylalaninate **1.33a,** different aminoesters **1.9,** the corresponding products **1.5** are obtained.

The intermediate monoamidate **1.3** is also prepared from the monoester **1.2** by first converting the monoester into the activated derivative **1.8** in which Lv is a leaving group such as halo, imidazolyl etc, using the procedures described above. The product **1.8** is then reacted with an aminoester **1.9** in the presence of a base such as pyridine, to give an intermediate monoamidate product **1.3.** The latter compound is then converted, by removal of the R¹ group and coupling of the product with the aminoester **1.9,** as described above, into the bisamidate **1.5.**

An example of this procedure, in which the phosphonic acid is activated by conversion to the chloro derivative **1.26**, is shown in Scheme **1**, Example **4**. In this procedure, the phosphonic monobenzyl ester **1.15** is reacted, in dichloromethane, with thionyl chloride, as described in Tet. Let., 1994, 35, 4097, to afford the phosphoryl chloride **1.26.** The product is then reacted in acetonitrile solution at ambient temperature with one molar equivalent of ethyl 3-amino-2-methylpropionate **1.27** to yield the monoamidate product **1.28.** The latter compound is hydrogenated in ethyl acetate over a 5% palladium on carbon catalyst to produce the monoacid product **1.29.** The product is subjected to a Mitsonobu coupling procedure, with equimolar amounts of butyl alaninate **1.30,** triphenyl phosphine, diethylazodicarboxylate and triethylamine in tetrahydrofuran, to give the bisamidate product **1.31.**

Using the above procedures, but employing, in place of ethyl 3-amino-2-methylpropionate **1.27** or butyl alaninate **1.30,** different aminoesters **1.9,** the corresponding products **1.5** are obtained.

The activated phosphonic acid derivative **1.7** is also converted into the bisamidate **1.5** via the diamino compound **1.10**. The conversion of activated phosphonic acid derivatives such as phosphoryl chlorides into the corresponding amino analogs **1.10,** by reaction with ammonia, is described in Organic Phosphorus Compounds, G. M. Kosolapoff, L. Maeir, eds, Wiley, 1976. The diamino compound **1.10** is then reacted at elevated temperature with a haloester **1.12,** in a polar organic solvent such as dimethylformamide, in the presence of a base such as dimethylaminopyridine or potassium carbonate, to yield the bisamidate **1.5.**
An example of this procedure is shown in Scheme **1,** Example **6**. In this method, a dichlorophosphonate **1.23** is reacted with ammonia to afford the diamide **1.37.** The reaction is performed in aqueous, aqueous alcoholic or alcoholic solution, at reflux temperature. The resulting diamino compound is then reacted with two molar equivalents of ethyl 2-bromo-3-methylbutyrate **1.38,** in a polar organic solvent such as N-methylpyrrolidinone at ca. 150°C, in the presence of a base such as potassium carbonate, and optionally in the presence of a catalytic amount of potassium iodide, to afford the bisamidate product **1.39.**

Using the above procedures, but employing, in place of ethyl 2-bromo-3-methylbutyrate **1.38,** different haloesters **1.12** the corresponding products **1.5** are obtained.

The procedures shown in Scheme **1** are also applicable to the preparation of bisamidates in which the aminoester moiety incorporates different functional groups. Scheme **1**, Example **7** illustrates the preparation of bisamidates derived from tyrosine. In this procedure, the monoimidazolide **1.32** is reacted with propyl tyrosinate **1.40,** as described in Example **5**, to yield the monoamidate **1.41.** The product is reacted with carbonyl diimidazole to give the imidazolide **1.42,** and this material is reacted with a further molar equivalent of propyl tyrosinate to produce the bisamidate product **1.43.**

Using the above procedures, but employing, in place of propyl tyrosinate **1.40,** different aminoesters **1.9,** the corresponding products **1.5** are obtained. The aminoesters employed in the two stages of the above procedure can be the same or different, so that bisamidates with the same or different amino substituents are prepared.

Scheme **2** illustrates methods for the preparation of phosphonate monoamidates.
In one procedure, a phosphonate monoester **1.1** is converted, as described in Scheme **1,** into the activated derivative **1.8.** This compound is then reacted, as described above, with an aminoester **1.9,** in the presence of a base, to afford the monoamidate product **2.1.**
The procedure is illustrated in Scheme **2**, Example **1**. In this method, a monophenyl phosphonate **2.7** is reacted with, for example, thionyl chloride, as described in J. Gen. Chem USSR., 1983, 32, 367, to give the chloro product **2.8.** The product is then reacted, as described in Scheme **1,** with ethyl alaninate **2.9,** to yield the amidate **2.10.**

Using the above procedures, but employing, in place of ethyl alaninate **2.9,** different aminoesters **1.9,** the corresponding products **2.1** are obtained.

Alternatively, the phosphonate monoester **1.1** is coupled, as described in Scheme **1,** with an aminoester **1**.**9** to produce the amidate **2.1.** If necessary, the R¹ substituent is then altered, by initial cleavage to afford the phosphonic acid **2.2.** The procedures for this transformation depend on the nature of the R¹ group, and are described above. The phosphonic acid is then transformed into the ester amidate product **2.3,** by reaction with the hydroxy compound R³OH, in which the group R³ is aryl, heteroaryl, alkyl cycloalkyl, haloalkyl etc, using the same coupling procedures (carbodiimide, Aldrithiol-2, PYBOP, Mitsonobu reaction etc) described in Scheme **1** for the coupling of amines and phosphonic acids.

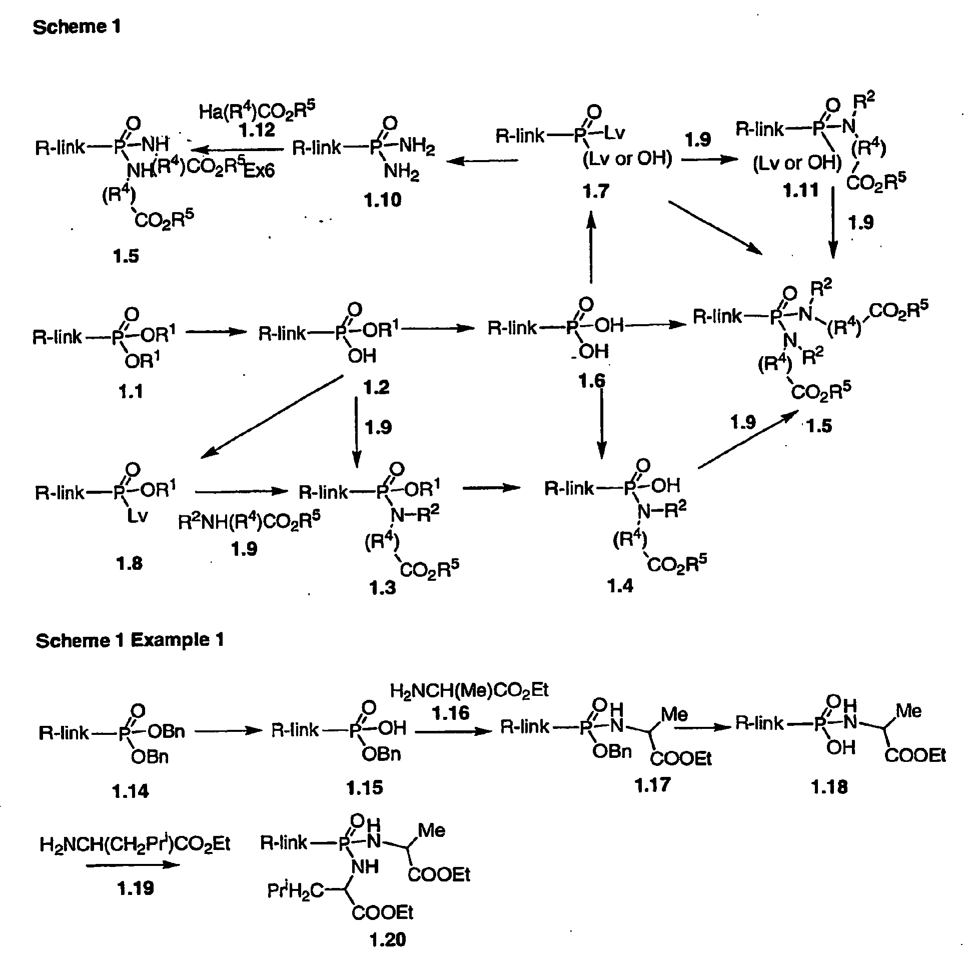

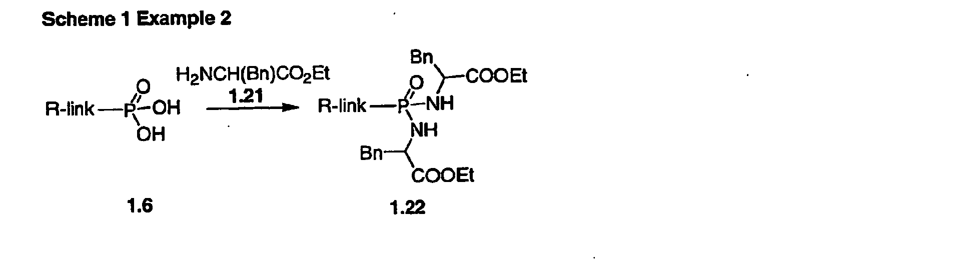

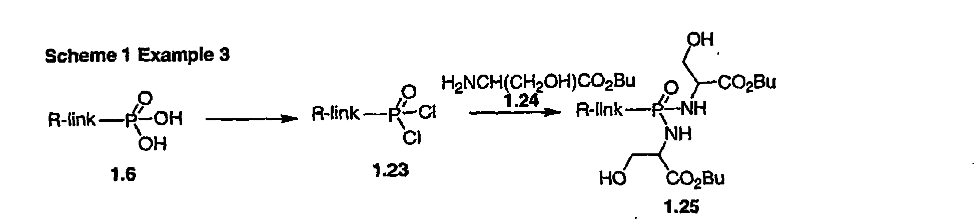

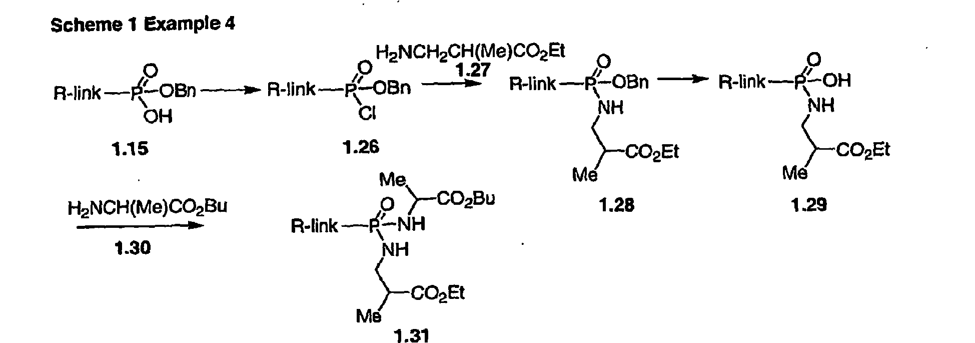

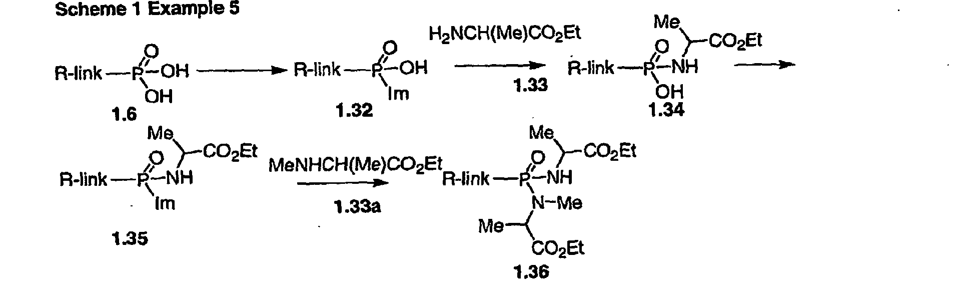

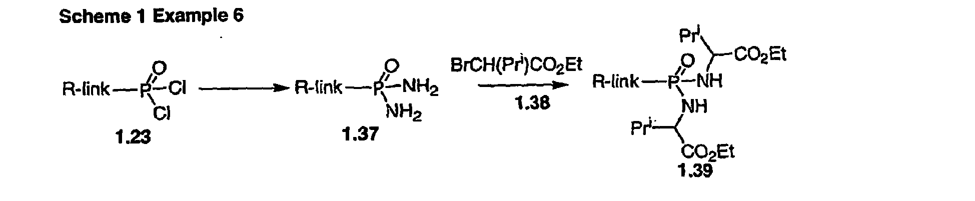

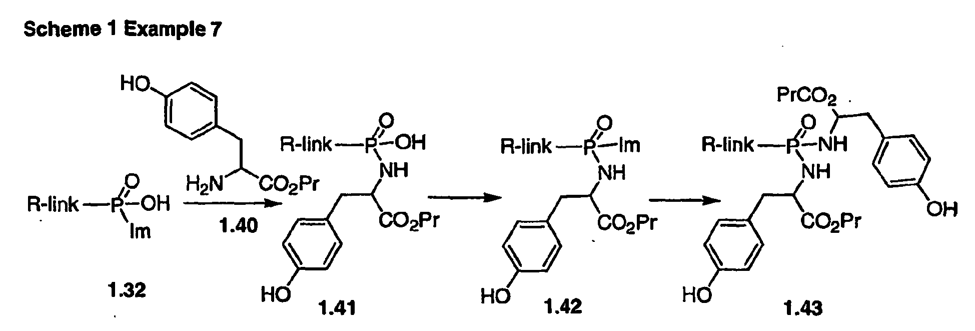

Examples of this method are shown in Scheme **2,** Examples and **2** and **3.** In the sequence shown in Example **2**, a monobenzyl phosphonate **2.11** is transformed by reaction with ethyl alaninate, using one of the methods described above, into the monoamidate **2.12.** The benzyl group is then removed by catalytic hydrogenation in ethyl acetate solution over a 5% palladium on carbon catalyst, to afford the phosphonic acid amidate **2.13.** The product is then reacted in dichloromethane solution at ambient temperature with equimolar amounts of 1-(dimethylaminopropyl)-3-ethylcarbodiimide and trifluoroethanol **2.14,** for example as described in Tet. Lett., 2401, 42, 8841, to yield the amidate ester **2.15.**

In the sequence shown in Scheme **2**, Example **3**, the monoamidate **2.13** is coupled, in tetrahydrofuran solution at ambient temperature, with equimolar amounts of dicyclohexyl carbodiimide and 4-hydroxy-N-methylpiperidine **2.16,** to produce the amidate ester product **2.17.**

Using the above procedures, but employing, in place of the ethyl alaninate product **2.12** different monoacids **2.2,** and in place of trifluoroethanol **2.14** or 4-hydroxy-N-methylpiperidine **2.16,** different hydroxy compounds R³OH, the corresponding products **2.3** are obtained.

Alternatively, the activated phosphonate ester **1.8** is reacted with ammonia to yield the amidate **2.4.** The product is then reacted, as described in Scheme 1, with a haloester **2.5,** in the presence of a base, to produce the amidate product **2.6.** If appropriate, the nature of the R¹ group is changed, using the procedures described above, to give the product **2.3.** The method is illustrated in Scheme **2**, Example **4**. In this sequence, the monophenyl phosphoryl chloride **2.18** is reacted, as described in Scheme **1,** with ammonia, to yield the amino product **2.19.** This material is then reacted in N-methylpyrrolidinone solution at 170°C with butyl 2-bromo-3-phenylpropionate **2.20** and potassium carbonate, to afford the amidate product **2.21.** Using these procedures, but employing, in place of butyl 2-bromo-3-phenylpropionate **2.20,** different haloesters **2.5,** the corresponding products **2.6** are obtained.

The monoamidate products **2.3** are also prepared from the doubly activated phosphonate derivatives **1.7.** In this procedure, examples of which are described in Synlett., 1998, 1, 73, the intermediate **1.7** is reacted with a limited amount of the aminoester **1.9** to give the monodisplacement product **1.11.** The latter compound is then reacted with the hydroxy compound R³OH in a polar organic solvent such as dimethylformamide, in the presence of a base such as diisopropylethylamine, to yield the monoamidate ester **2.3.**

The method is illustrated in Scheme **2**, Example **5**. In this method, the phosphoryl dichloride **2.22** is reacted in dichloromethane solution with one molar equivalent of ethyl N-methyl tyrosinate **2.23** and dimethylaminopyridine, to generate the monoamidate **2.24.** The product is then reacted with phenol **2.25** in dimethylformamide containing potassium carbonate, to yield the ester amidate product **2.26.**

Using these procedures, but employing, in place of ethyl N-methyl tyrosinate **2.23** or phenol **2.25,** the aminoesters **1.9** and/or the hydroxy compounds R³OH, the corresponding products **2.3** are obtained.

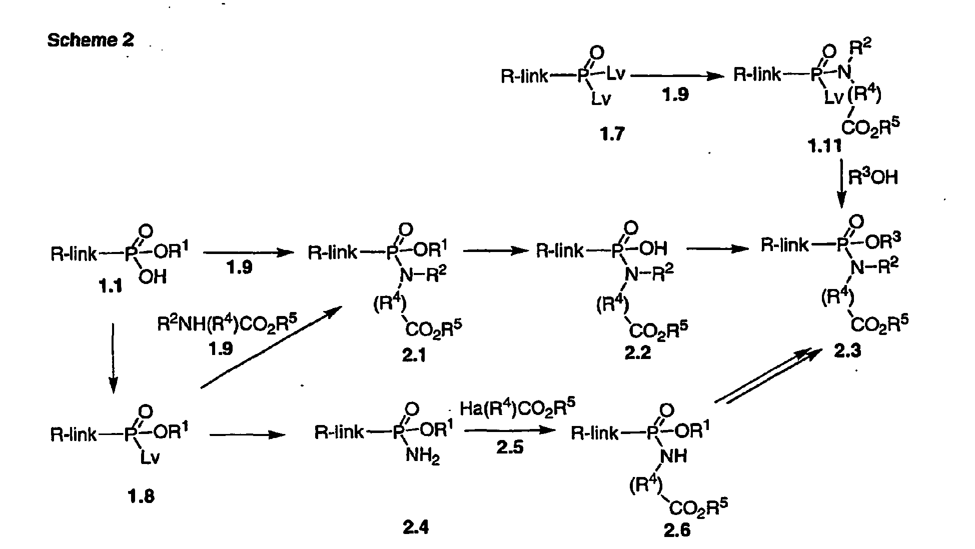

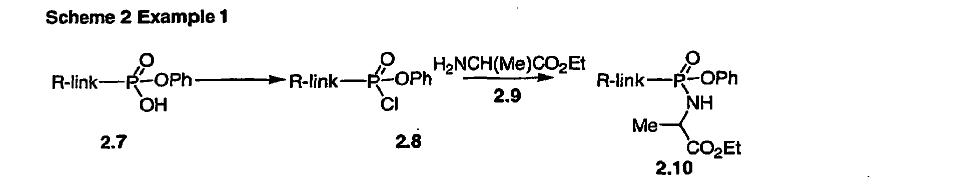

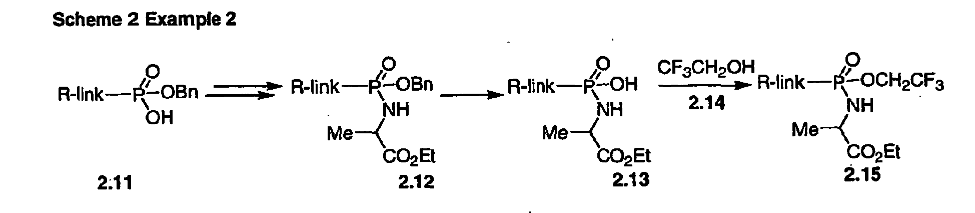

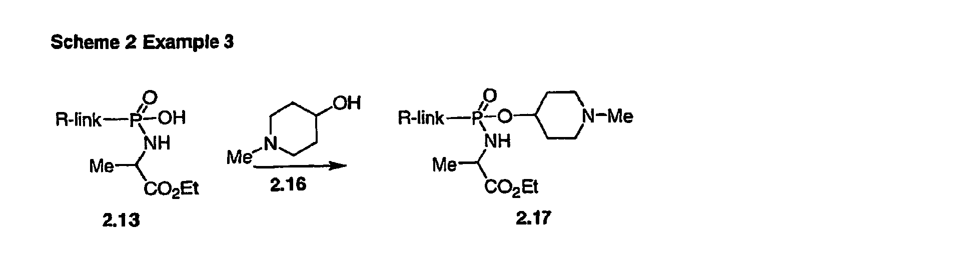

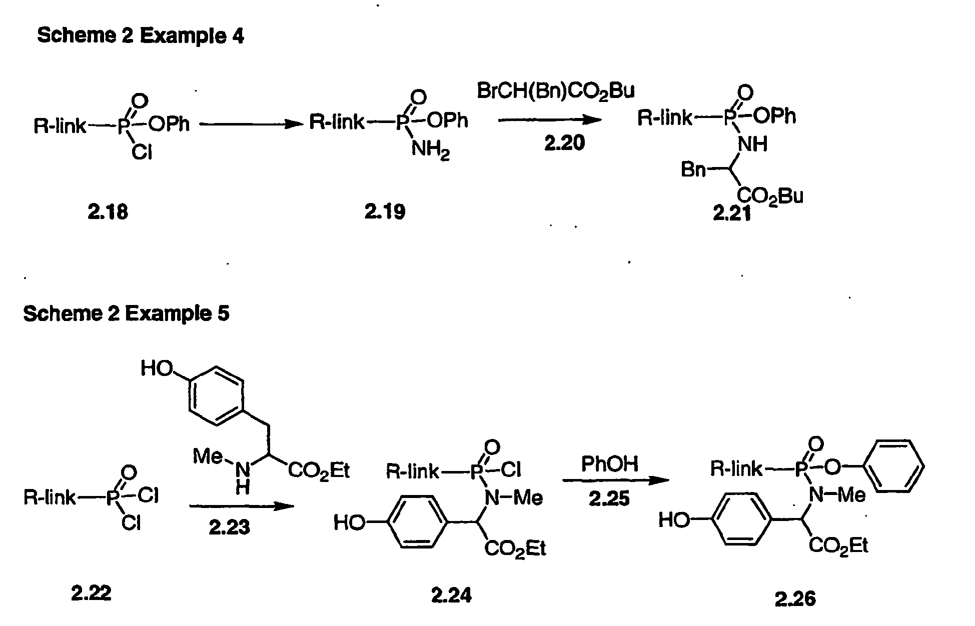

Scheme **3** illustrates methods for the preparation of carboalkoxy-substituted phosphonate diesters in which one of the ester groups incorporates a carboalkoxy substituent.
In one procedure, a phosphonate monoester **1.1,** prepared as described above, is coupled, using one of the methods described above, with a hydroxyester **3.1,** in which the groups R⁴ and R⁵ are as described in Scheme **1.** For example, equimolar amounts of the reactants are coupled in the presence of a carbodiimide such as dicyclohexyl carbodiimide, as described in Aust. J. Chem, 1963, 609, optionally in the presence of dimethylaminopyridine, as described in Tet., 1999, 55, 12997. The reaction is conducted in an inert solvent at ambient temperature.

The procedure is illustrated in Scheme **3**, Example **1**. In this method, a monophenyl phosphonate **3.9** is coupled, in dichloromethane solution in the presence of dicyclohexyl carbodiimide, with ethyl 3-hydroxy-2-methylpropionate **3.10** to yield the phosphonate mixed diester **3.11.**

Using this procedure, but employing, in place of ethyl 3-hydroxy-2-methylpropionate **3.10,** different hydroxyesters **3.1,** the corresponding products **3.2** are obtained.

The conversion of a phosphonate monoester **1.1** into a mixed diester **3.2** is also accomplished by means of a Mitsonobu coupling reaction with the hydroxyester **3.1,** as described in Org. Lett., 2001, 643. In this method, the reactants **1.1** and **3.1** are combined in a polar solvent such as tetrahydrofuran, in the presence of a triarylphosphine and a dialkyl azodicarboxylate, to give the mixed diester **3.2.** The R¹ substituent is varied by cleavage, using the methods described previously, to afford the monoacid product **3.3.** The product is then coupled, for example using methods described above, with the hydroxy compound R³OH, to give the diester product **3.4.**

The procedure is illustrated in Scheme **3,** Example **2.** In this method, a monoallyl phosphonate **3.12** is coupled in tetrahydrofuran solution, in the presence of triphenylphosphine and diethylazodicarboxylate, with ethyl lactate **3.13** to give the mixed diester **3.14.** The product is reacted with tris(triphenylphosphine) rhodium chloride (Wilkinson catalyst) in acetonitrile, as described previously, to remove the allyl group and produce the monoacid product **3.15.** The latter compound is then coupled, in pyridine solution at ambient temperature, in the presence of dicyclohexyl carbodiimide, with one molar equivalent of 3-hydroxypyridine **3.16** to yield the mixed diester **3.17.**

Using the above procedures, but employing, in place of the ethyl lactate **3.13** or 3-hydroxypyridine, a different hydroxyester **3.1** and/or a different hydroxy compound R³OH, the corresponding products **3.4** are obtained.

The mixed diesters **3.2** are also obtained from the monoesters **1.1** via the intermediacy of the activated monoesters **3.5.** In this procedure, the monoester **1.1.** is converted into the activated compound **3.5** by reaction with, for example, phosphorus pentachloride, as described in J. Org. Chem., 2001, 66, 329, or with thionyl chloride or oxalyl chloride (Lv = Cl), or with triisopropylbenzenesulfonyl chloride in pyridine, as described in Nucleosides and Nucleotides, 2000, 19, 1885, or with carbonyl diimidazole, as described in J. Med. Chem., 2002, 45, 1284. The resultant activated monoester is then reacted with the hydroxyester **3.1,** as described above, to yield the mixed diester **3.2.**

The procedure is illustrated in Scheme **3**, Example **3**. In this sequence, a monophenyl phosphonate **3.9** is reacted, in acetonitrile solution at 70°C, with ten equivalents of thionyl chloride, so as to produce the phosphoryl chloride **3.19.** The product is then reacted with ethyl 4-carbamoyl-2-hydroxybutyrate **3.20** in dichloromethane containing triethylamine, to give the mixed diester **3.21.**

Using the above procedures, but employing, in place of ethyl 4-carbamoyl-2-hydroxybutyrate **3.20,** different hydroxyesters **3.1,** the corresponding products **3.2** are obtained.

The mixed phosphonate diesters are also obtained by an alternative route for incorporation of the R³O group into intermediates **3.3** in which the hydroxyester moiety is already incorporated. In this procedure, the monoacid intermediate **3.3** is converted into the activated derivative **3.6** in which Lv is a leaving group such as chloro, imidazole, and the like, as previously described. The activated intermediate is then reacted with the hydroxy compound R³OH, in the presence of a base, to yield the mixed diester product **3.4.**

The method is illustrated in Scheme **3**, Example **4**. In this sequence, the phosphonate monoacid **3.22** is reacted with trichloromethanesulfonyl chloride in tetrahydrofuran containing collidine, as described in J. Med. Chem., 1995, 38, 4648, to produce the trichloromethanesulfonyloxy product **3.23.** This compound is reacted with 3-(morpholinomethyl)phenol **3.24** in dichloromethane containing triethylamine, to yield the mixed diester product **3.25.**

Using the above procedures, but employing, in place of with 3-(morpholinomethyl)phenol **3.24,** different carbinols R³OH, the corresponding products **3.4** are obtained.

The phosphonate esters **3.4** are also obtained by means of alkylation reactions performed on the monoesters **1.1.** The reaction between the monoacid **1.1** and the haloester **3.7** is performed in a polar solvent in the presence of a base such as diisopropylethylamine, as described in AnaL Chem., 1987, 59, 1056; or triethylamine, as described in J. Med. Chem., 1995, 38, 1372, or in a non-polar solvent such as benzene, in the presence of 18-crown-6, as described in Syn. Comm., 1995, 25, 3565.

The method is illustrated in Scheme **3**, Example **5**. In this procedure, the monoacid **3.26** is reacted with ethyl 2-bromo-3-phenylpropionate **3.27** and diisopropylethylamine in dimethylformamide at 80°C to afford the mixed diester product **3.28.**

Using the above procedure, but employing, in place of ethyl 2-bromo-3-phenylpropionate **3.27,** different haloesters **3.7,** the corresponding products **3.4** are obtained.

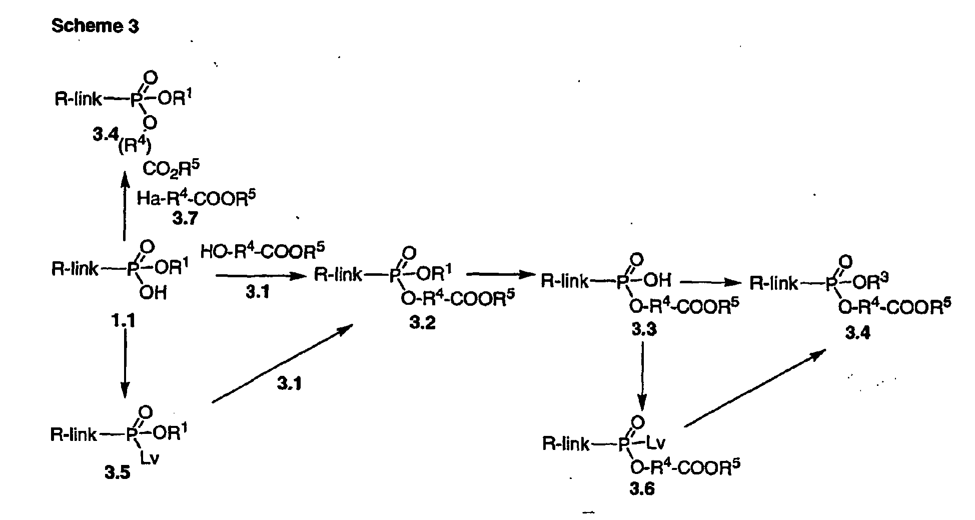

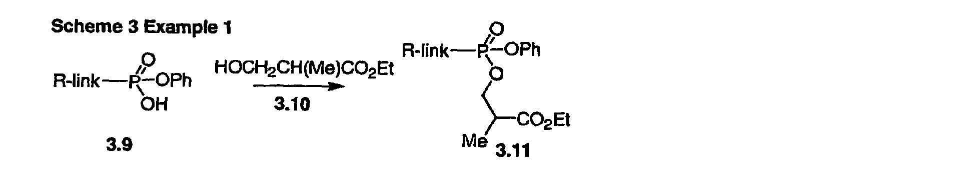

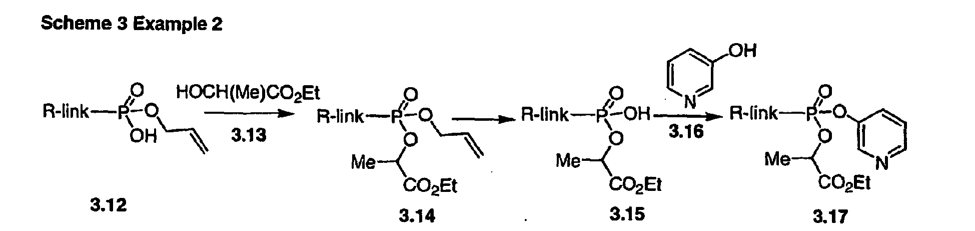

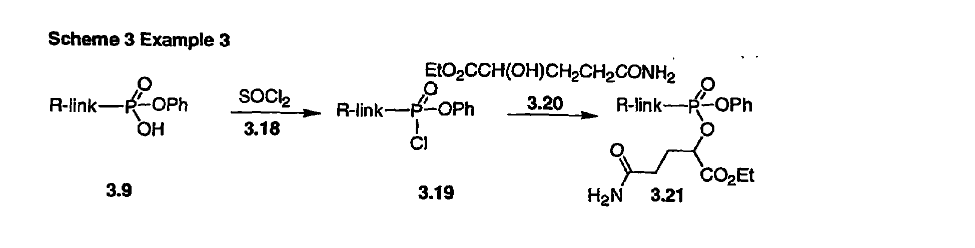

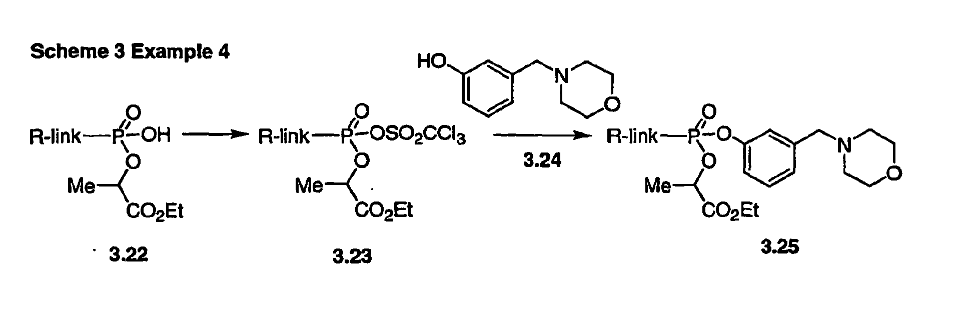

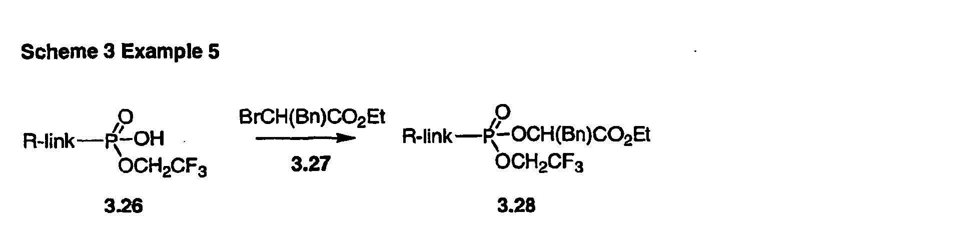

Scheme **4** illustrates methods for the preparation of phosphonate diesters in which both the ester substituents incorporate carboalkoxy groups.

The compounds are prepared directly or indirectly from the phosphonic acids **1.6.** In one alternative, the phosphonic acid is coupled with the hydroxyester **4.2,** using the conditions described previously in Schemes **1 - 3,** such as coupling reactions using dicyclohexyl carbodiimide or similar reagents, or under the conditions of the Mitsonobu reaction, to afford the diester product **4.3** in which the ester substituents are identical.

This method is illustrated in Scheme **4**, Example **1**. In this procedure, the phosphonic acid **1.6** is reacted with three molar equivalents of butyl lactate **4.5** in the presence of Aldrithiol-2 and triphenyl phosphine in pyridine at ca. 70°C, to afford the diester **4.6.**
Using the above procedure, but employing, in place of butyl lactate **4.5,** different hydroxyesters **4.2,** the corresponding products **4.3** are obtained.

Alternatively, the diesters **4.3** are obtained by alkylation of the phosphonic acid **1.6** with a haloester **4.1.** The alkylation reaction is performed as described in Scheme **3** for the preparation of the esters **3.4.**

This method is illustrated in Scheme **4,** Example **2.** In this procedure, the phosphonic acid **1.6** is reacted with excess ethyl 3-bromo-2-methylpropionate **4.7** and diisopropylethylamine in dimethylformamide at ca. 80°C, as described in Anal. Chem., 1987, 59, 1056, to produce the diester **4.8.**
Using the above procedure, but employing, in place of ethyl 3-bromo-2-methylpropionate **4.7,** different haloesters **4.1,** the corresponding products **4.3** are obtained.

The diesters **4.3** are also obtained by displacement reactions of activated derivatives **1.7** of the phosphonic acid with the hydroxyesters **4.2.** The displacement reaction is performed in a polar solvent in the presence of a suitable base, as described in Scheme **3.** The displacement reaction is performed in the presence of an excess of the hydroxyester, to afford the diester product **4.3** in which the ester substituents are identical, or sequentially with limited amounts of different hydroxyesters, to prepare diesters **4.3** in which the ester substituents are different. The methods are illustrated in Scheme **4,** Examples **3** and **4.** As shown in Example **3,** the phosphoryl dichloride **2.22** is reacted with three molar equivalents of ethyl 3-hydroxy-2-(hydroxymethyl)propionate **4.9** in tetrahydrofuran containing potassium carbonate, to obtain the diester product **4.10.**
Using the above procedure, but employing, in place of ethyl 3-hydroxy-2-(hydroxymethyl)propionate **4.9,** different hydroxyesters **4.2,** the corresponding products **4.3** are obtained.
Scheme **4**, Example **4** depicts the displacement reaction between equimolar amounts of the phosphoryl dichloride **2.22** and ethyl 2-methyl-3-hydroxypropionate **4.11,** to yield the monoester product **4.12.** The reaction is conducted in acetonitrile at 70°C in the presence of diisopropylethylamine. The product **4.12** is then reacted, under the same conditions, with one molar equivalent of ethyl lactate **4.13,** to give the diester product **4.14.**
Using the above procedures, but employing, in place of ethyl 2-methyl-3-hydroxypropionate **4.11** and ethyl lactate **4.13,** sequential reactions with different hydroxyesters **4.2,** the corresponding products **4.3** are obtained.

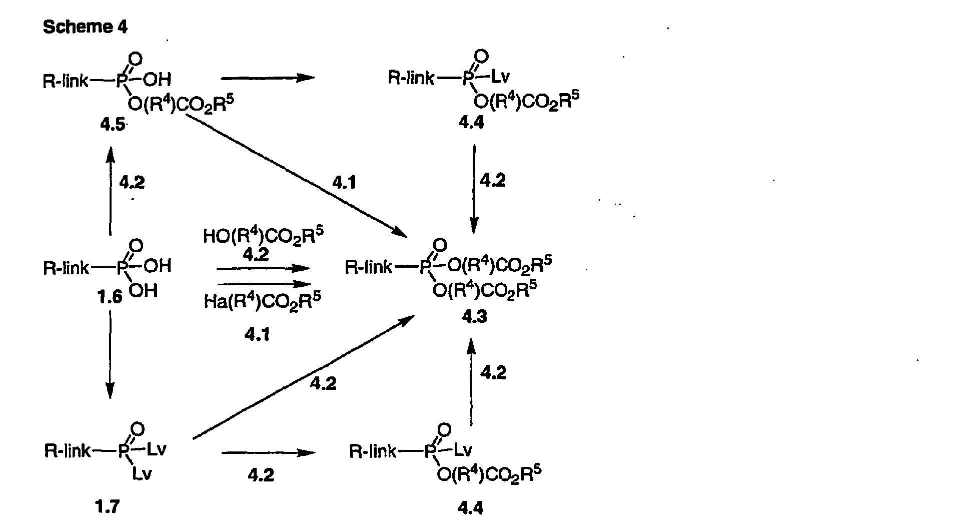

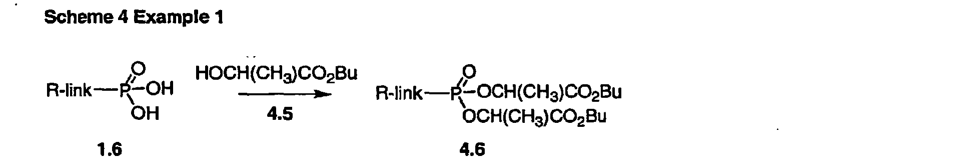

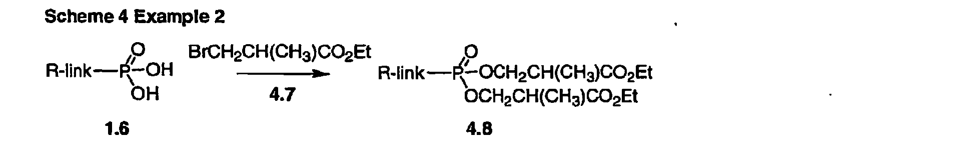

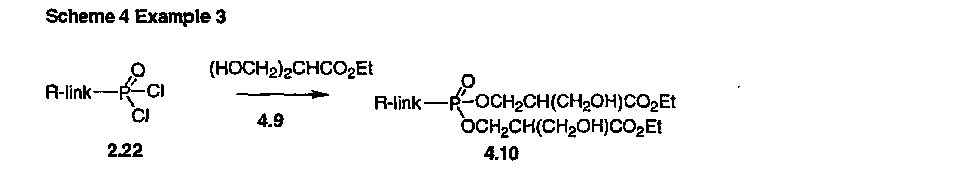

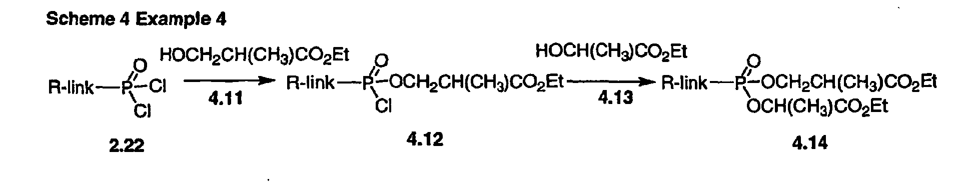

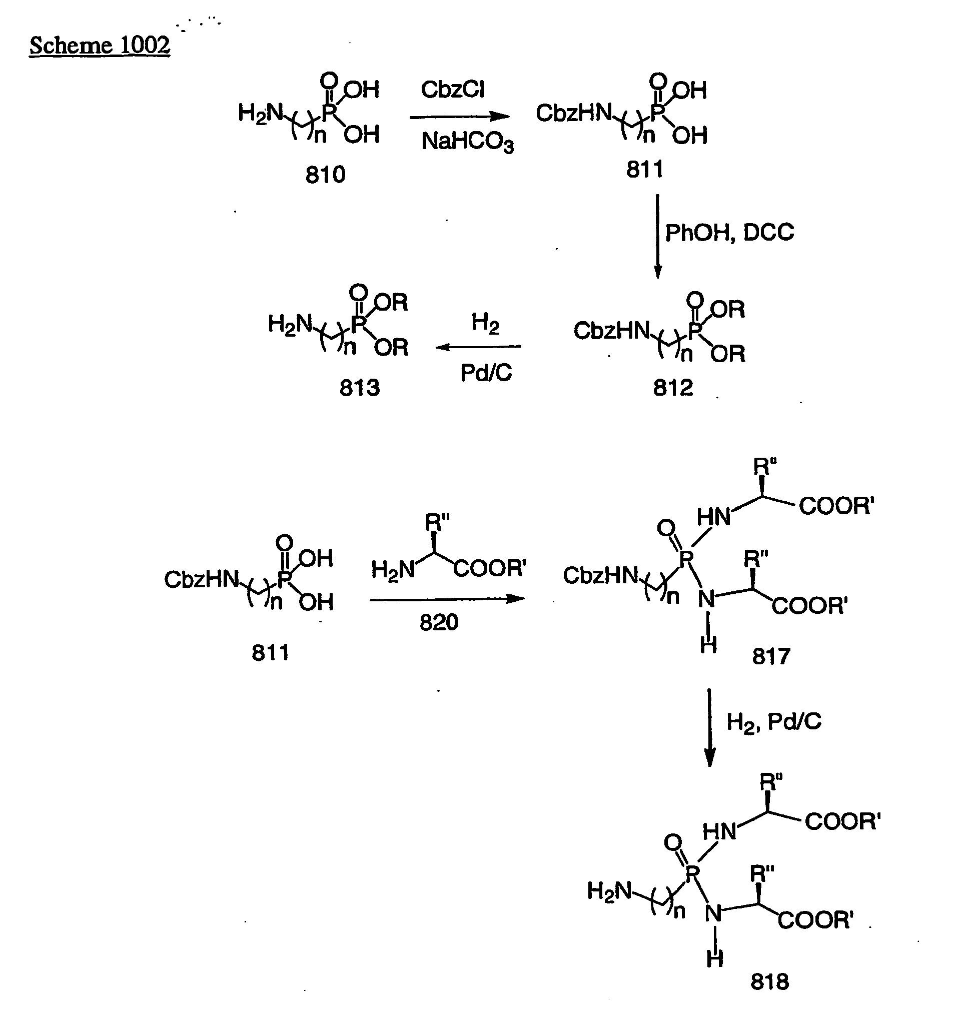

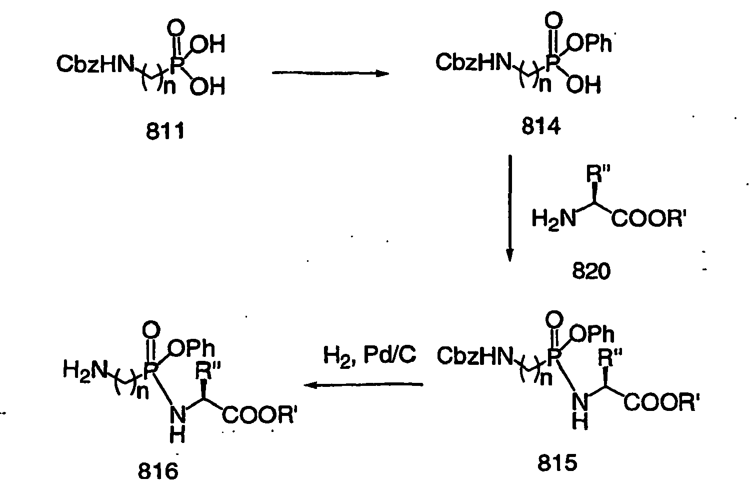

Following the similar procedures, replacement of amino acid esters 820 with lactates 821 (Scheme 1003) provides mono-phosphonic lactates 823. Lactates 823 are useful intermediates to form the phosphonate compounds of the invention.

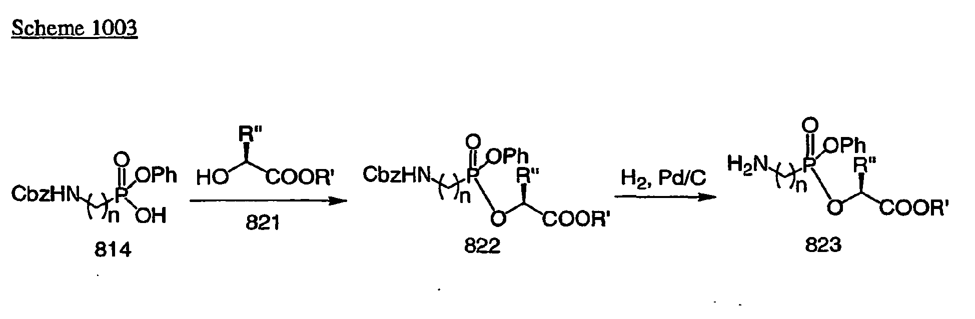

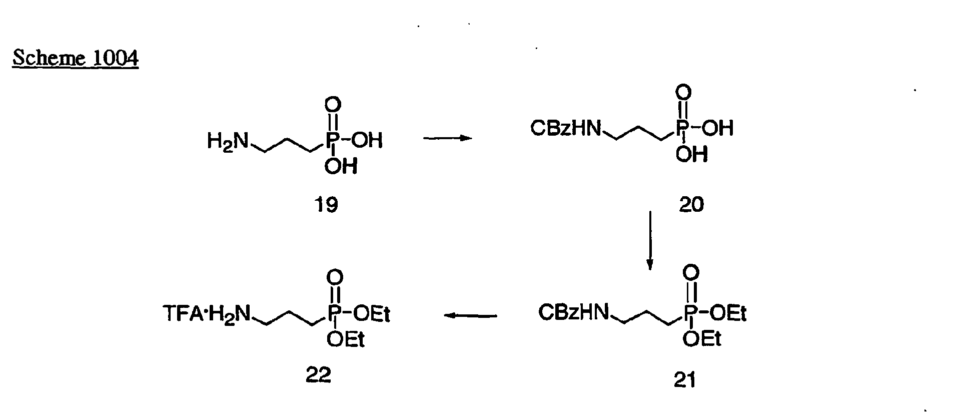

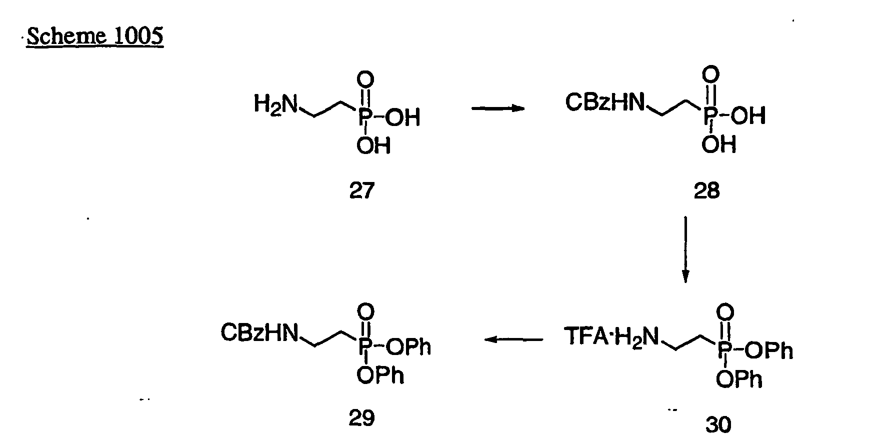

### Example 1

To a solution of 2-aminoethylphosphonic acid (1.26 g, 10.1 mmol) in 2N NaOH (10.1 mL, 20.2 mmol) was added benzyl chloroformate (1.7 mL, 12.1 mmol). After the reaction mixture was stirred for 2 d at room temperature, the mixture was partitioned between Et₂O and water. The aqueous phase was acidified with 6N HCl until pH = 2. The resulting colorless solid was dissolved in MeOH (75 mL) and treated with Dowex 50WX8-200 (7 g). After the mixture was stirred for 30 minutes, it was filtered and evaporated under reduced pressure to give carbamate **28** (2.37 g, 91%) as a colorless solid (Scheme 1005).

To a solution of carbamate **28** (2.35 g, 9.1 mmol) in pyridine (40 mL) was added phenol (8.53 g, 90.6 mmol) and 1,3-dicyclohexylcarbodiimide (7.47 g, 36.2 mmol). After the reaction mixture was warmed to 70°C and stirred for 5 h, the mixture was diluted with CH₃CN and filtered. The filtrate was concentrated under reduced pressure and diluted with EtOAc. The organic phase was washed with sat. NH₄Cl, sat. NaHCO₃, and brine, then dried over Na₂SO₄, filtered, and evaporated under reduced pressure. The crude product was chromatographed on silica gel twice (eluting 40-60% EtOAc/hexane) to give phosphonate **29** (2.13 g, 57%) as a colorless solid.

To a solution of phosphonate **29** (262 mg, 0.637 mmol) in iPrOH (5 mL) was added TFA (0.05 mL, 0.637 mmol) and 10% Pd/C (26 mg). After the reaction mixture was stirred under H₂ atmosphere (balloon) for 1 h, the mixture was filtered through Celite. The filtrate was evaporated under reduced pressure to give amine **30** (249 mg, 100%) as a colorless oil (Scheme 1005).

### Scheme Section A

Exemplary methods of preparing the compounds of the invention are shown in **Schemes 1-7** below. A detailed description of the methods is found in the Experimental section below.

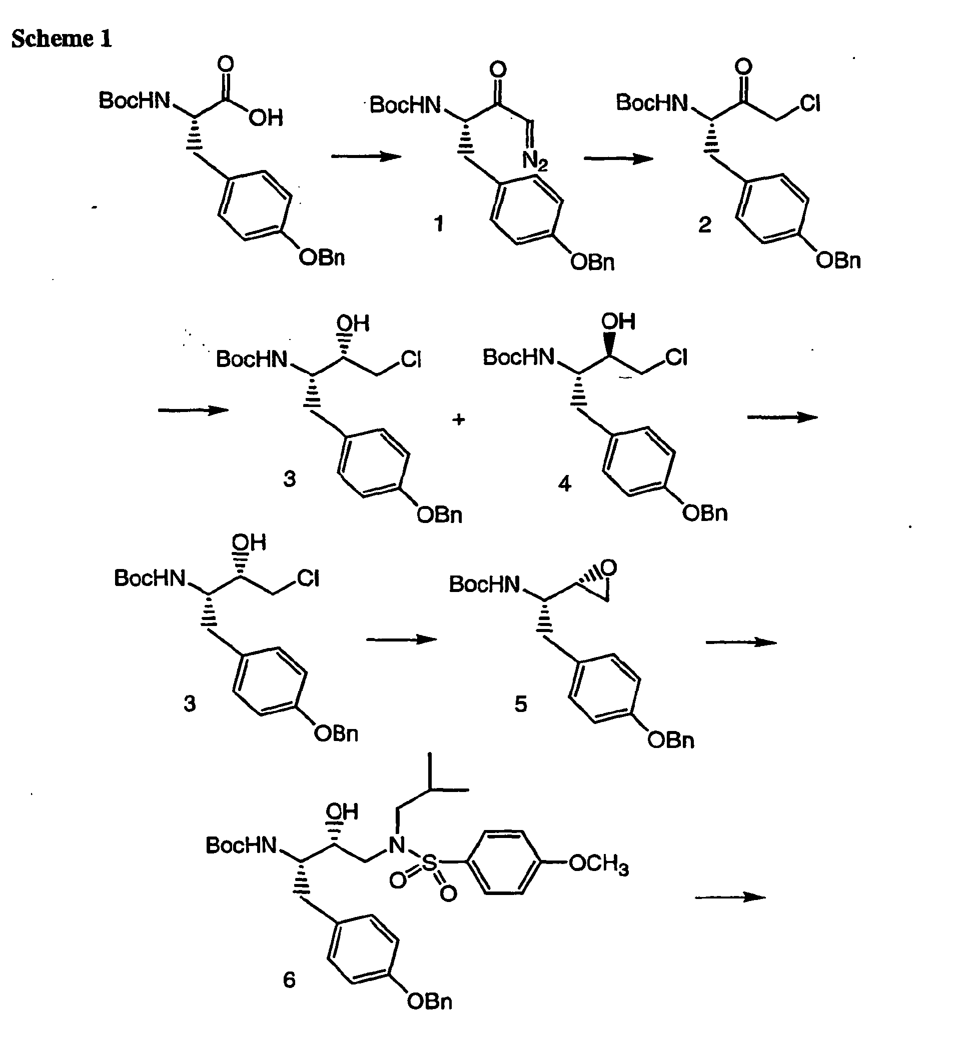

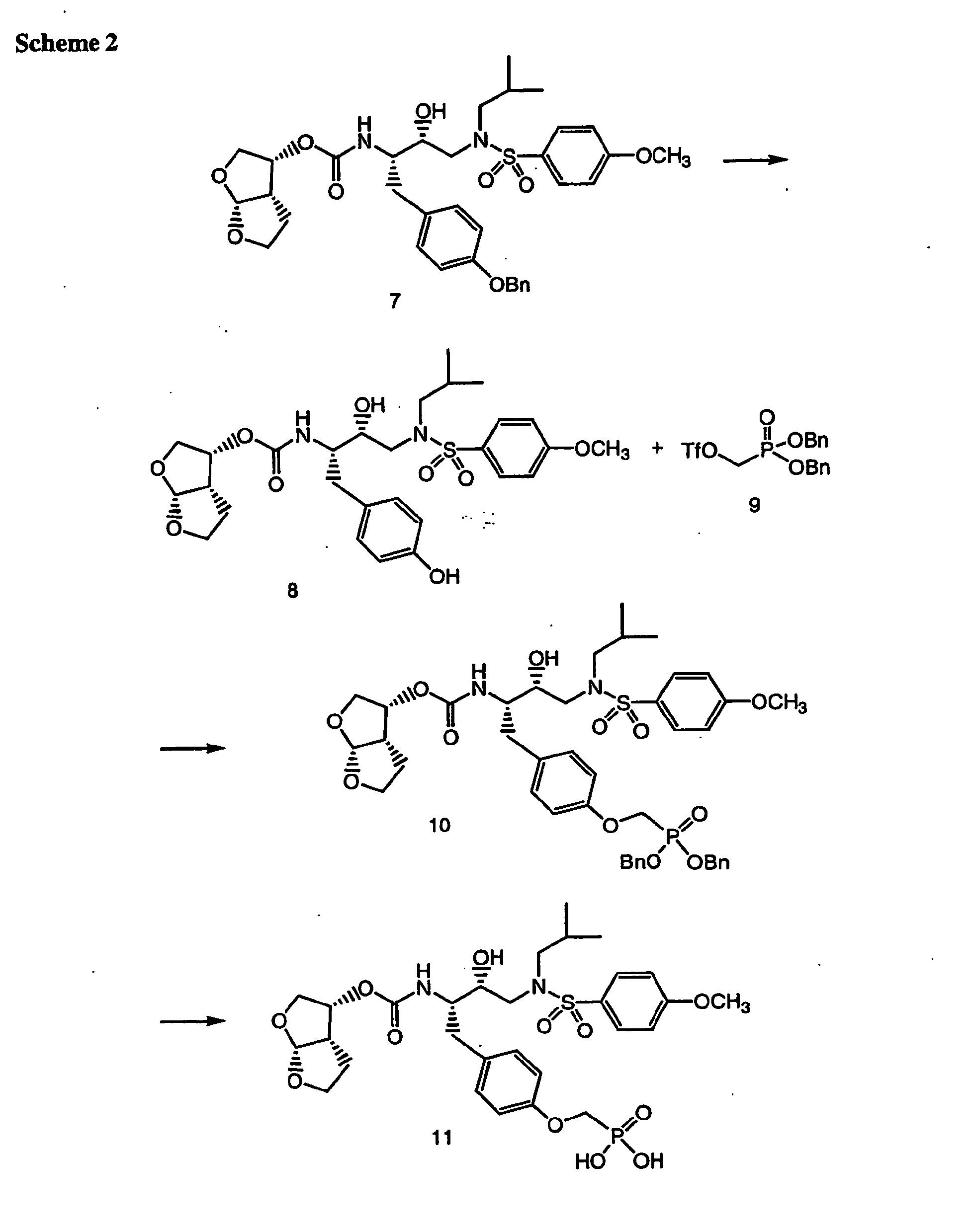

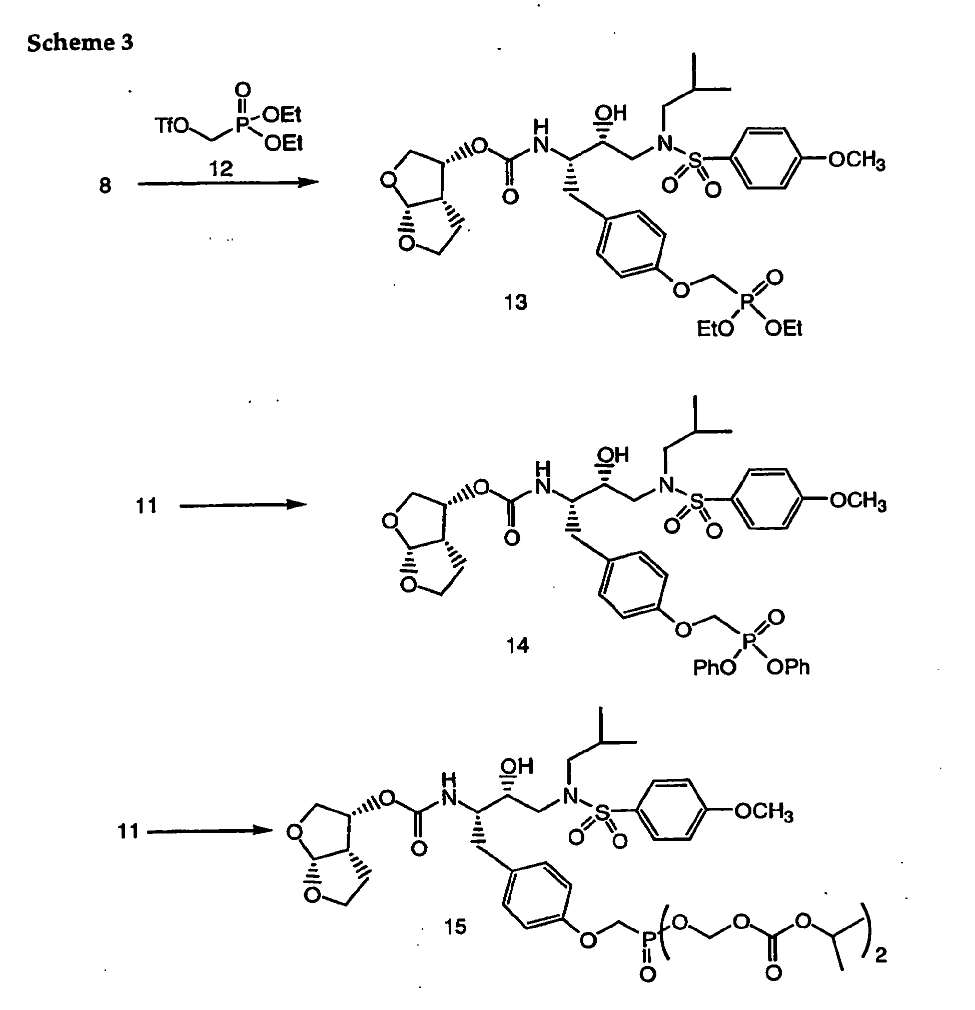

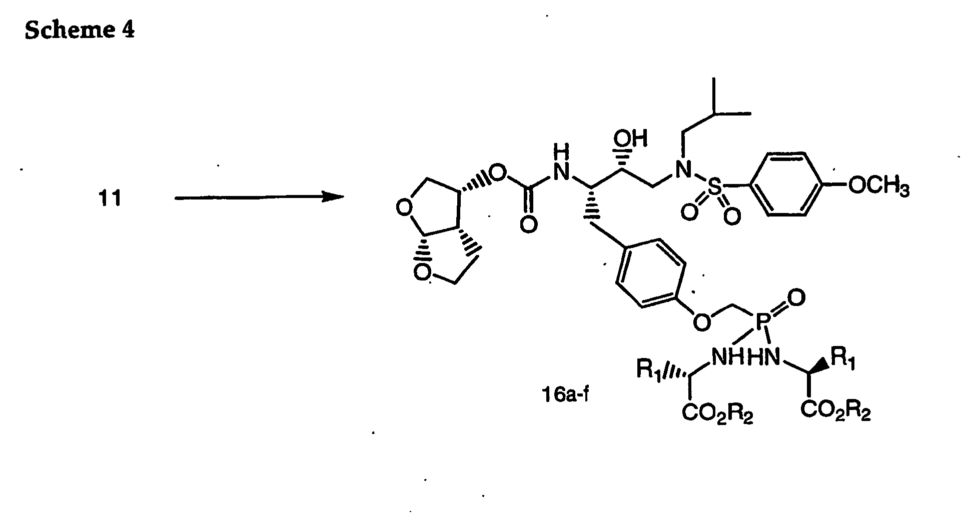

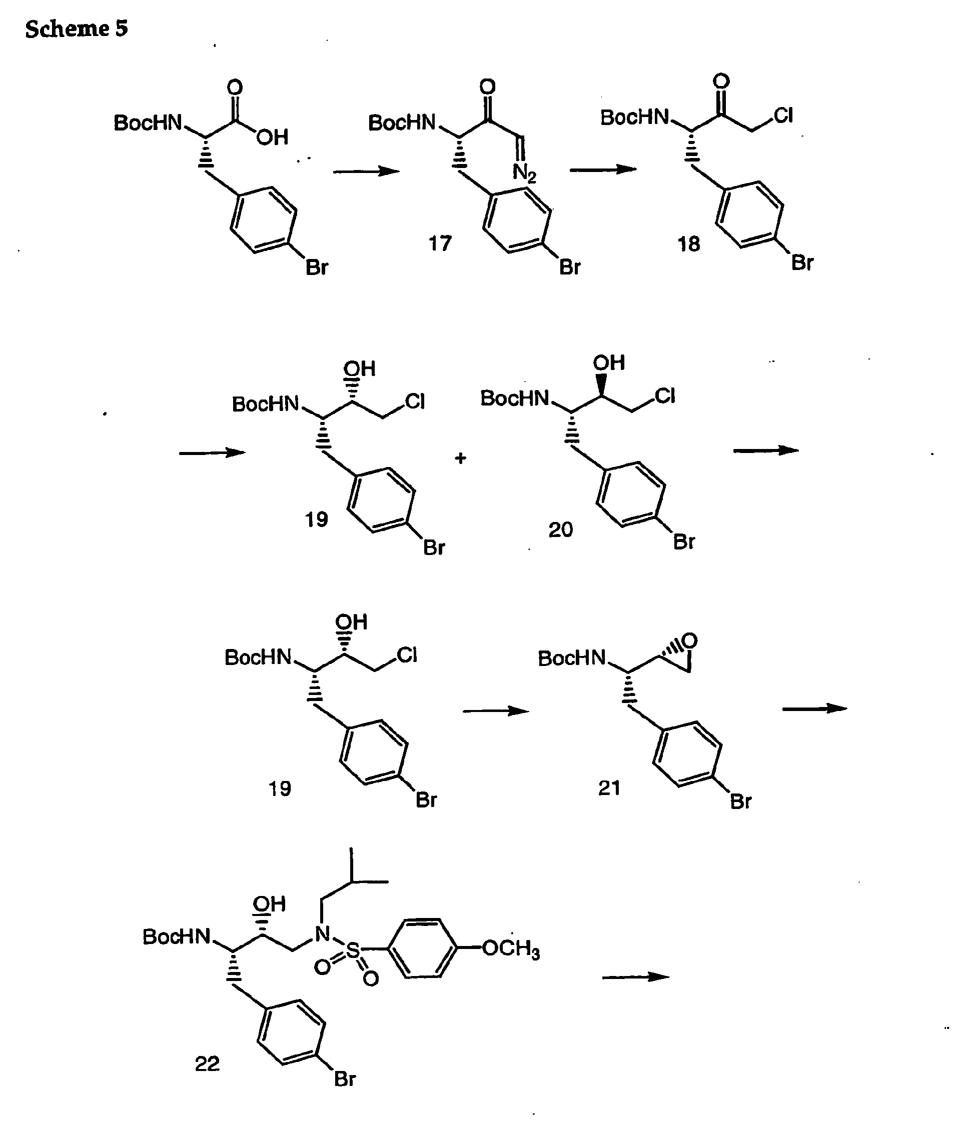

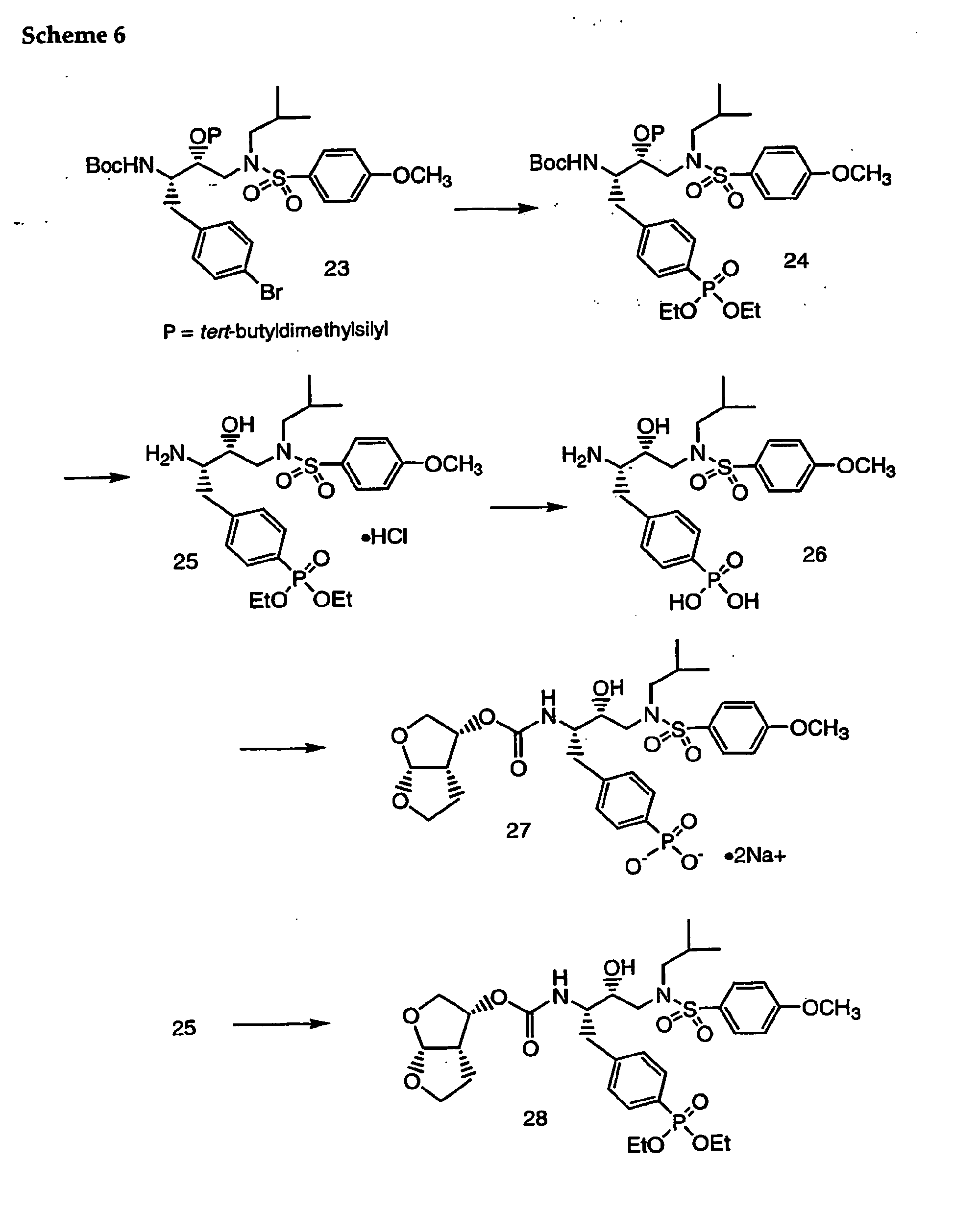

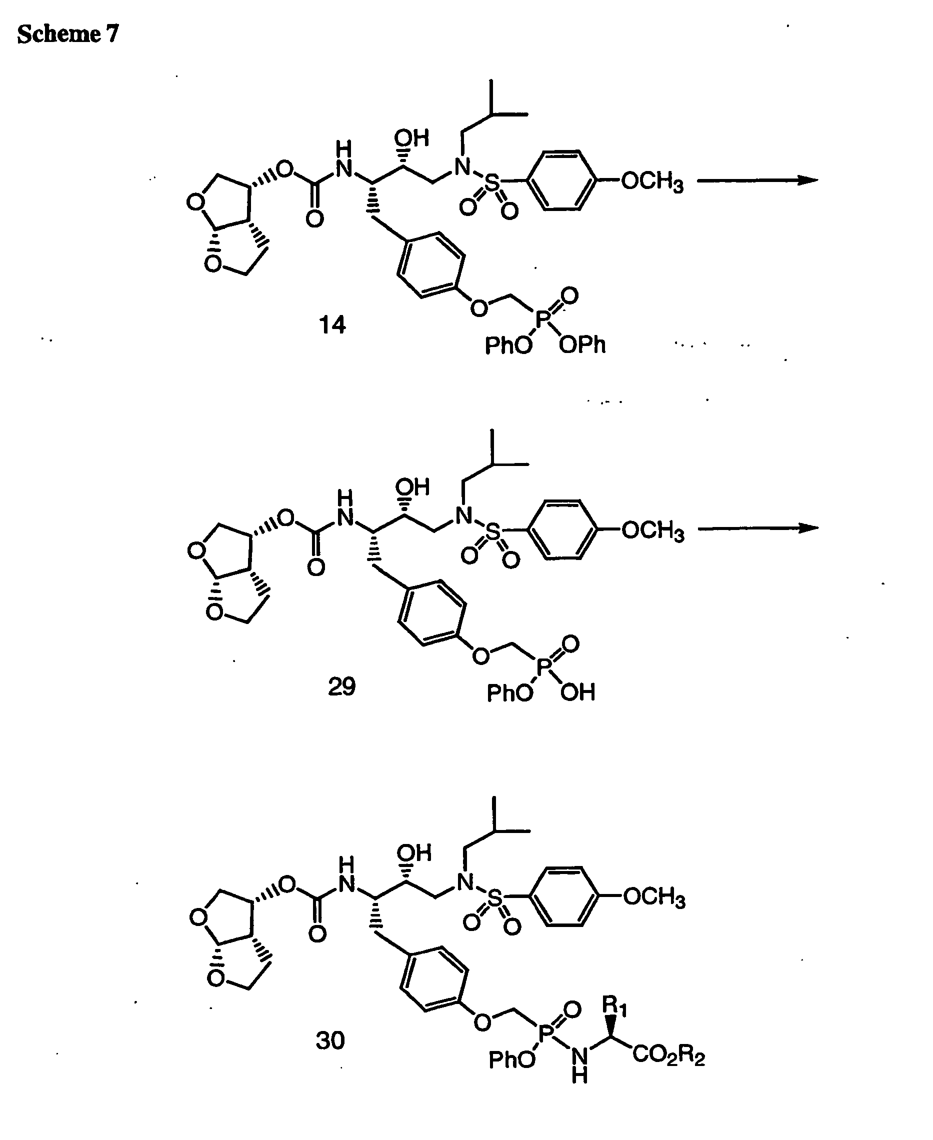

### Scheme Section B

Alternative exemplary methods of preparing the compounds of the invention are shown in **Schemes 101-113** below. 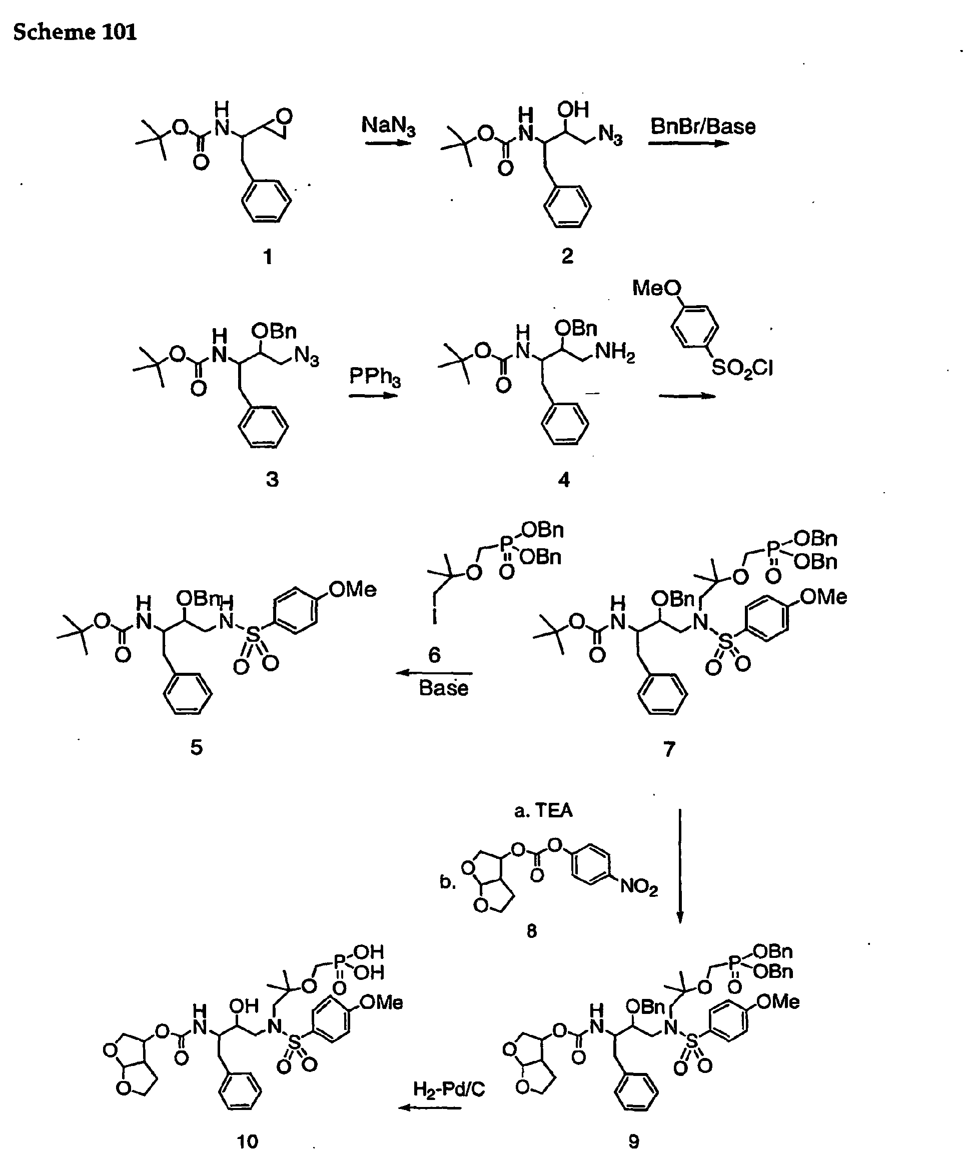 Treatment of commercially available epoxide 1 with sodum azide (Bioorg. & Med. Chem. Lett., 5, 459, 1995) furnishes the azide intermediate 2. The free hydroxyl is converted to benzyl ether 3 by treating it with benzyl bromide in the presence of base such as potassium carbonate. Compound 4 is achieved by the reduction of the azide group with triphenyl phosphine, as described in the publication Bioorg. & Med. Chem. Lett., 7, 1847, 1997. Conversion of the amino group to its sulfonamide derivative 5 is achieved by treating the amine with stoichiometric amounts of sulfonyl chloride. Regioselective alkylation is performed (as shown in the article J. Med. Chem., 40, 2525, 1997) on the sulfonamide nitrogen using the iodide 6 (J. Med. Chem, 35, 2958, 1992) to get the compound 7. Upon TFA catalyzed deprotection of BOC group followed by the reaction with bisfuranyl carbonate 8 (for a similar coupling see, J. Med. Chem., 39, 3278, 1996) furnishes the compound 9. Final deprotection of the protecting groups by catalytic hydrogenolysis result the compound 10. 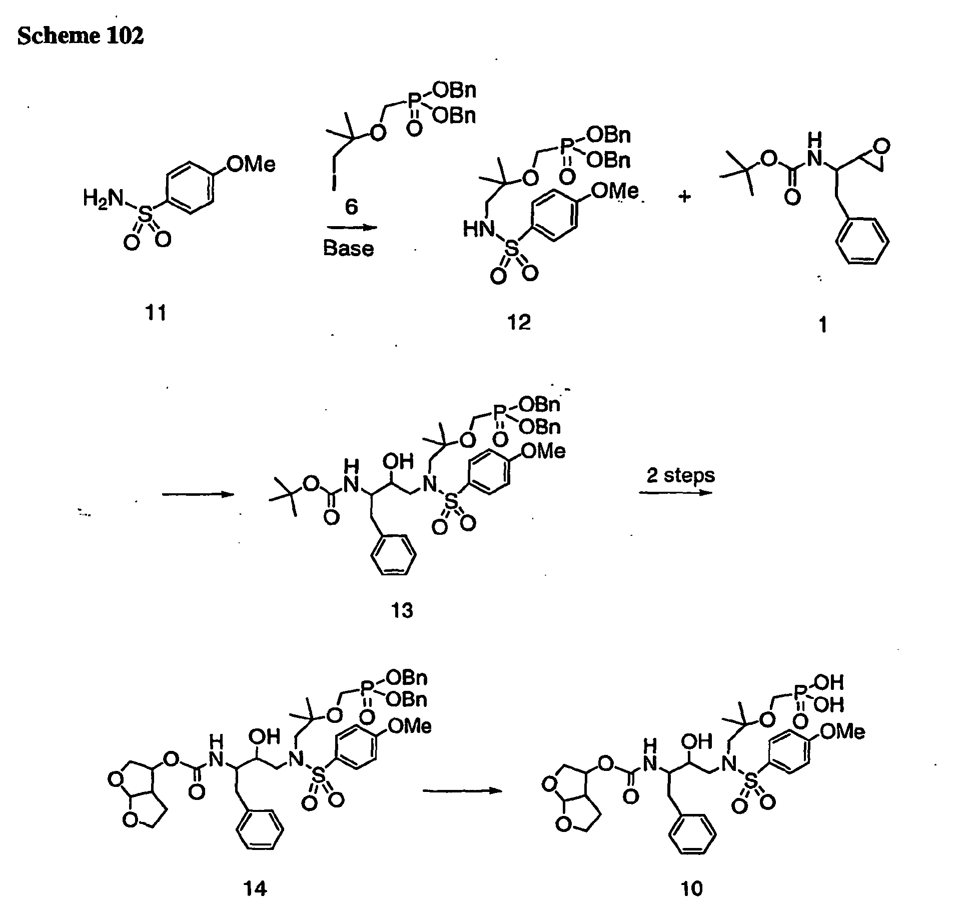 The sulfonamide 11 is readily alkylated with the iodide 6 (J. Med. Chem., 35, 2958, 1992) to get the intermediate 12. Regioselective epoxide opening (JP -9124630) of the epoxide 1 with 12 furnishes the intermediate 13. Deprotection of the BOC group followed by the treatment of bisfuranyl carbonate 8 yields the intermediate 14 which is subjected to hydrogenation to furnish the compound 10. 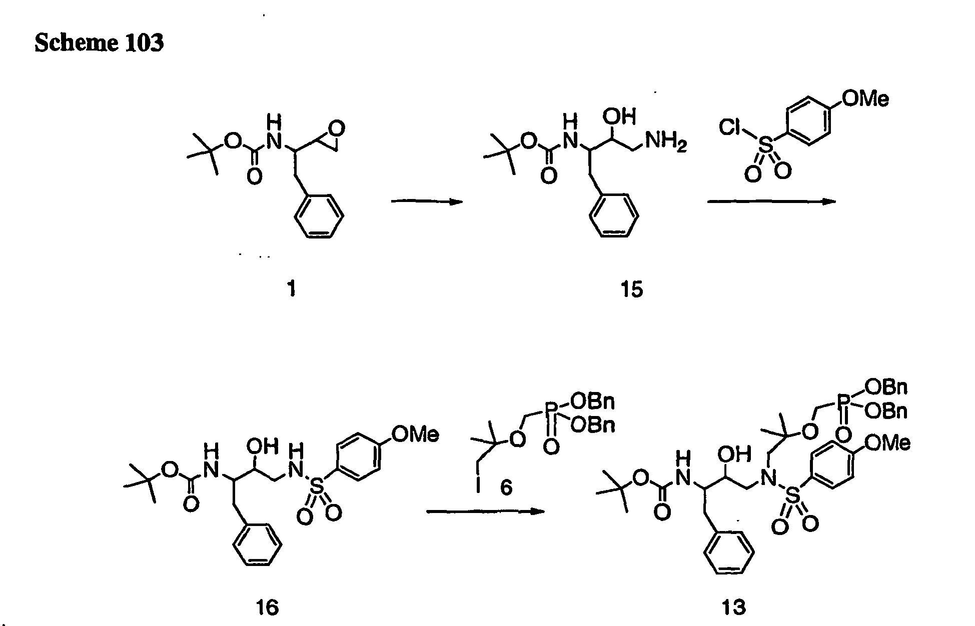 The epoxide 1 is converted to the aminohydroxyl derivative 15 using the known procedure (J. Med. Chem., 37, 1758, 1994). Sulfonylation of 15 using benzene sulfonylchloride affords the compound 16. Installation of the side chain to get the intermediate 13 is achieved by alkylation of sulfonamide nitrogen with iodide 6. The intermediate 13 is converted to the compound 10 using the same sequence as shown in scheme 102. 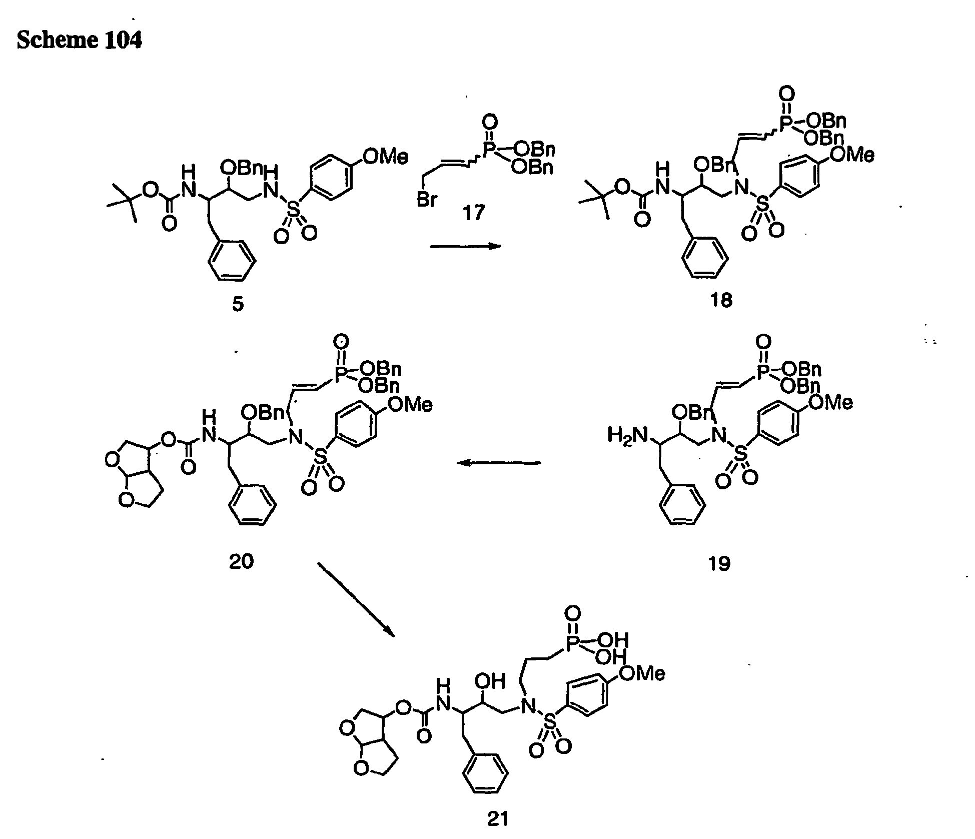 Sulfonamide 5 is alkylated under basic conditions using the allyl bromide 17 (Chem. Pharm. Bull., 30, 111, 1982) to get the intermediate 18. Similar transformation is reported in literature (J. Med. Chem, 40, 2525, 1997). Hydrolysis of BOC group with TFA and acylation of the resulting amine 19 with bisfuranyl carbonate 8 yields the compound 20. Hydrogenation using Pd/C catalysis under H₂ atmosphere affords the phosphonic acid 21. 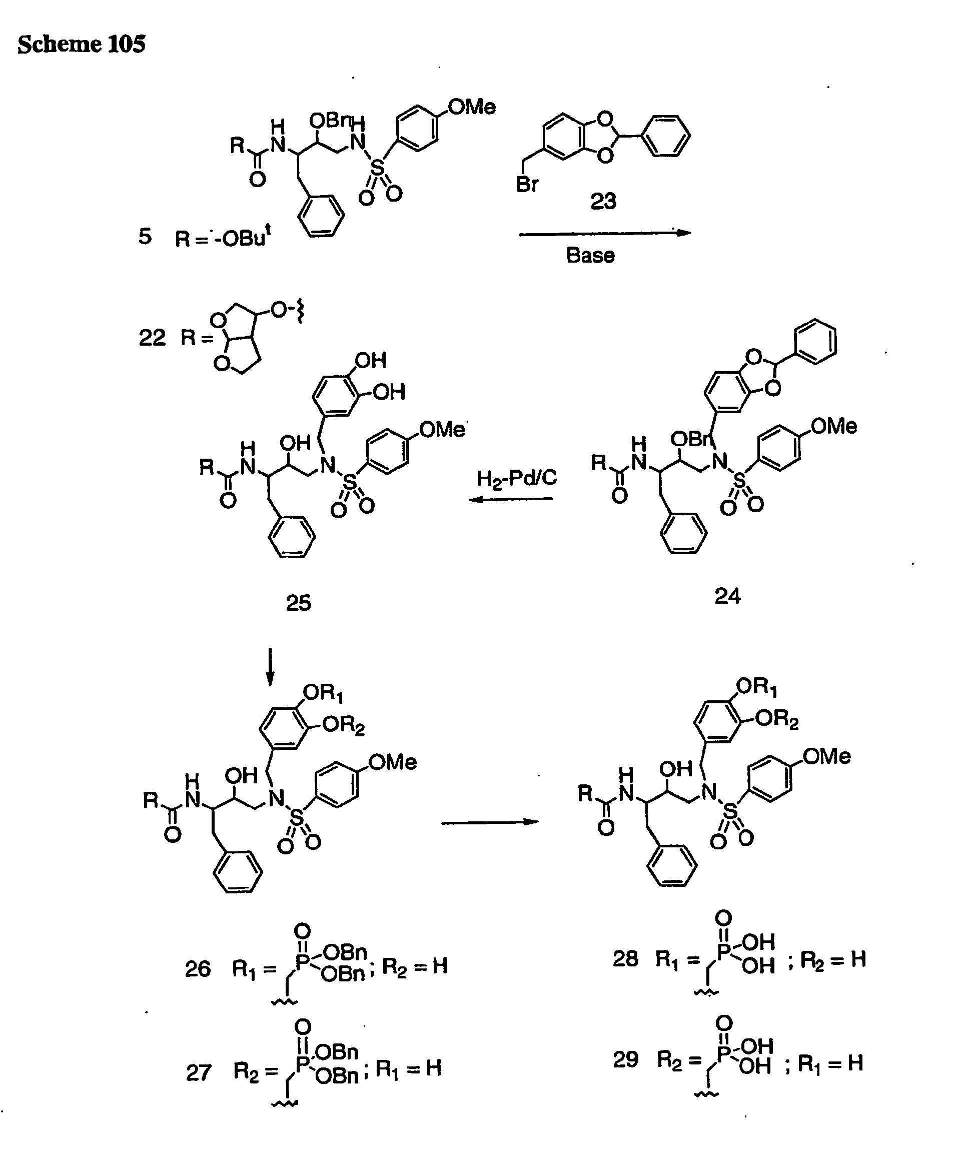 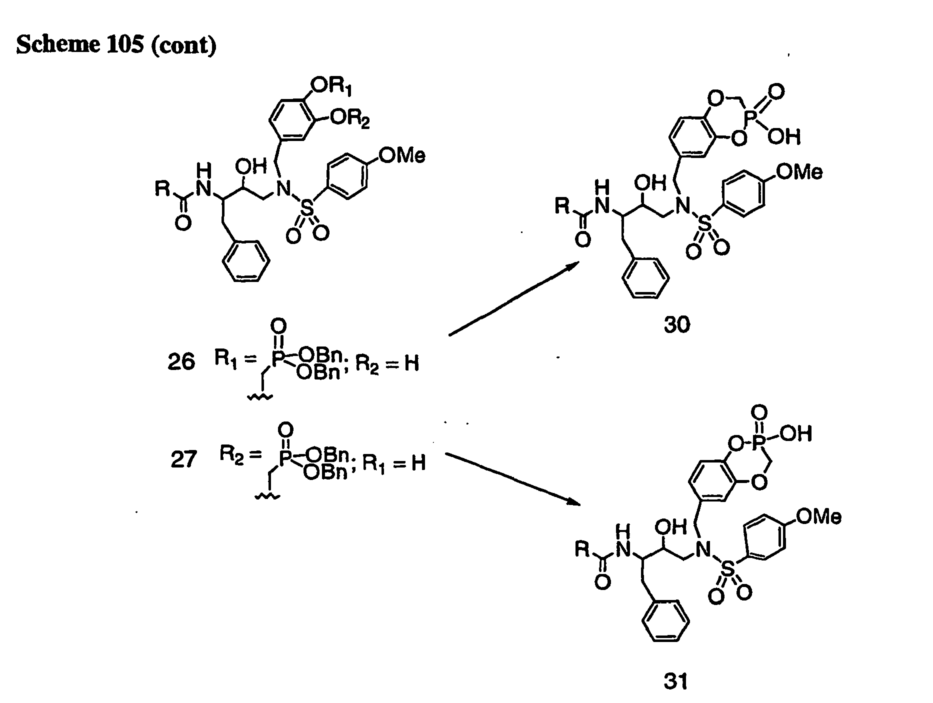 Sulfonamide 5 is converted to 22 via hydrolysis of BOC group with TFA and acylation with bisfuranyl carbonate 8. The sulfonamide 22 is alkylated with the bromide 23 (J. Med. Chem., 40, 2525, 1997) to get the compound 24, which upon hydrogenolysis gives the catechol 25. Alkylation of the phenolic groups using dibenzylhydroxymethyl phosphonate (J. Org. Chem., 53, 3457, 1988) affords regioisomeric compounds 26 and 27. These compounds 26 and 27 are hydrogenated to get the phophonic acids 28 and 29, respectively. Individual cyclic phosphonic acids 30 and 31 are obtained under basic (like NaH) conditions (US 5886179) followed by hydrogenolysis of the dibenzyl ester derivatives 26 and 27.

### Scheme 106

In this route, compound 25 is obtained by conducting a reaction between the epoxide 32 and the sulfonamide 33 using the conditions described in the Japanese Patent No. 9124630.

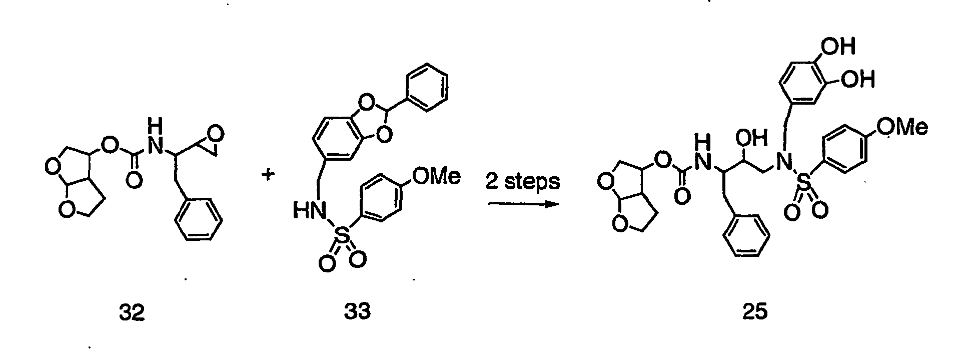

Epoxide 32 and sulfonamide 33 are synthesized utilizing similar methodology delineated in the same patent. 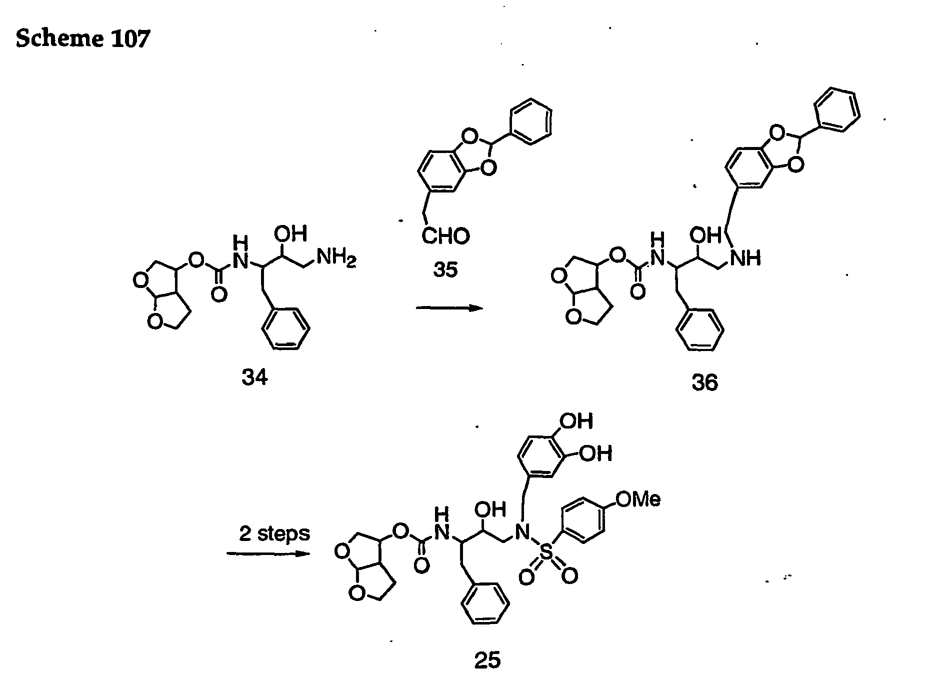 Compound 34 is obtained from 32 using similar sequence depicted in J. Med. Chem, 37, 1758, 1994. Reductive amination (for similar transformation see WO 00/47551) of compound 34 with aldehyde 35 furnishes the intermediate 36 which is converted to the compound 25 by sulfonylation followed by hydrogenation. 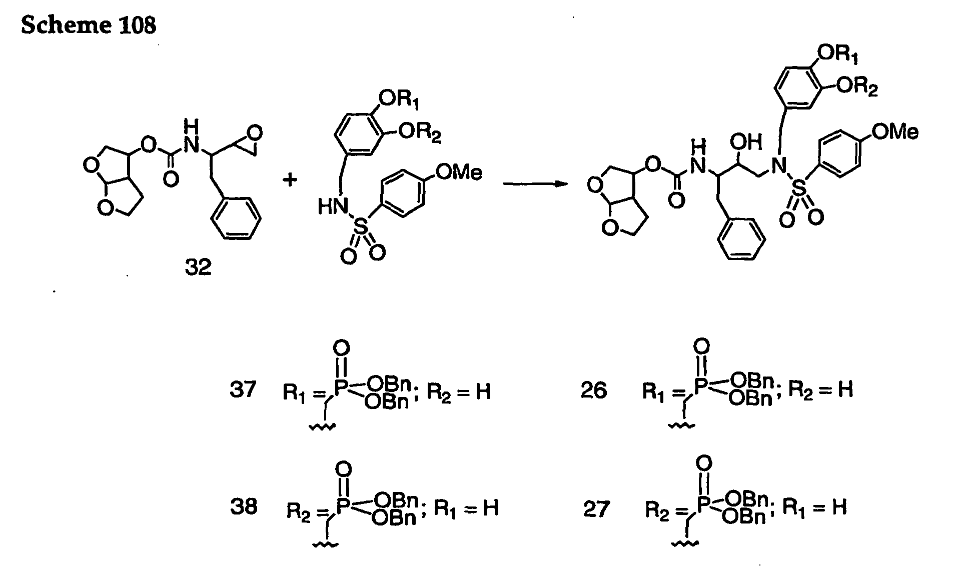 Treatment of epoxide 32 with sulfonamides 37 and/or 38 under conditions described in Japanese Patent No. 9124630 furnishes 26 and 27.

### Scheme 109

Reductive amination of aminohydroxyl intermediate 34 with the aldehydes 39 and 40 as described in patent WO 00/47551, furnish 41 and 42 which undergoes smooth sulfonylation to give 26 and 27.

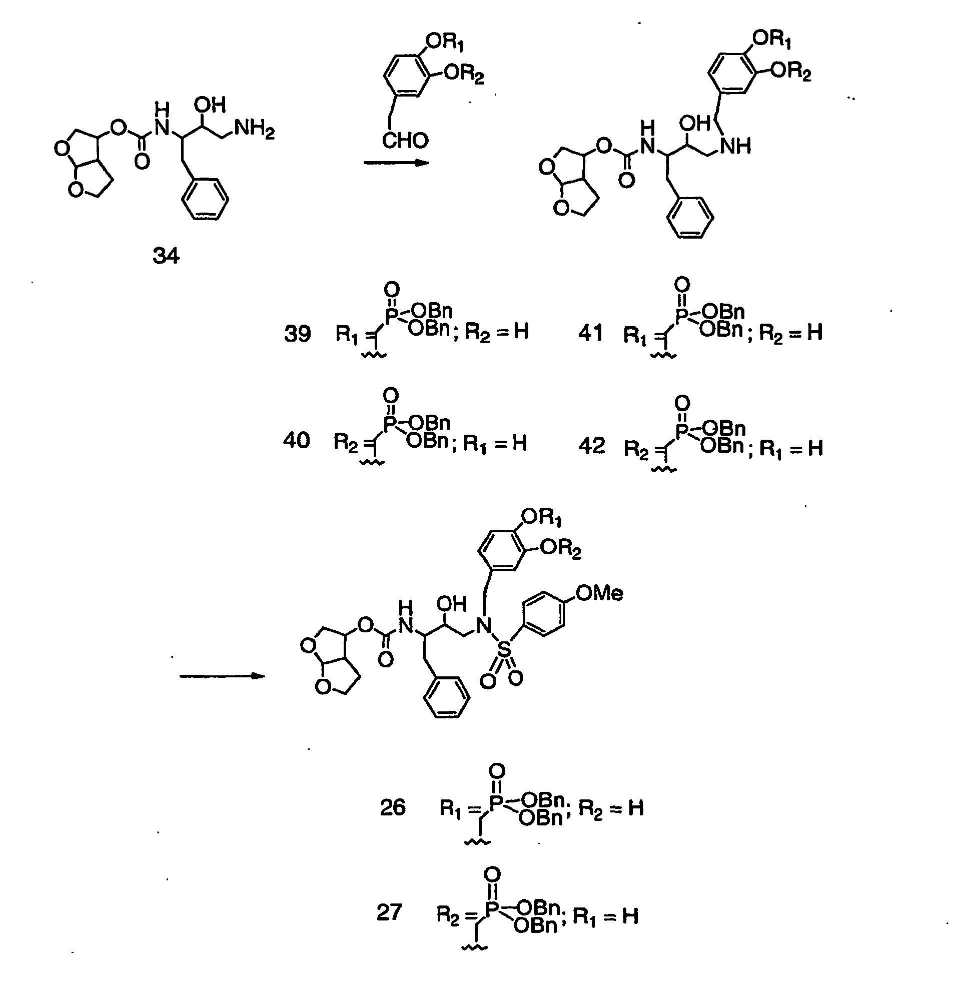

### Scheme 110

In an alternate approach, where epoxide 32 is opened with benzyl amines 43 and 44 under conditions described above furnishes 41 and 42, respectively. Similar transformations were documented in the Japanese Patent No. 9124630.

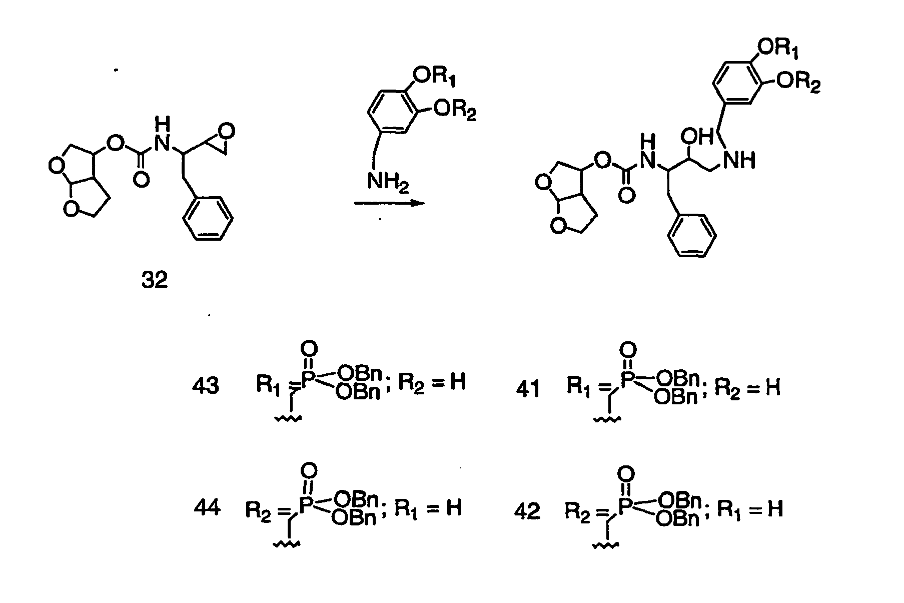

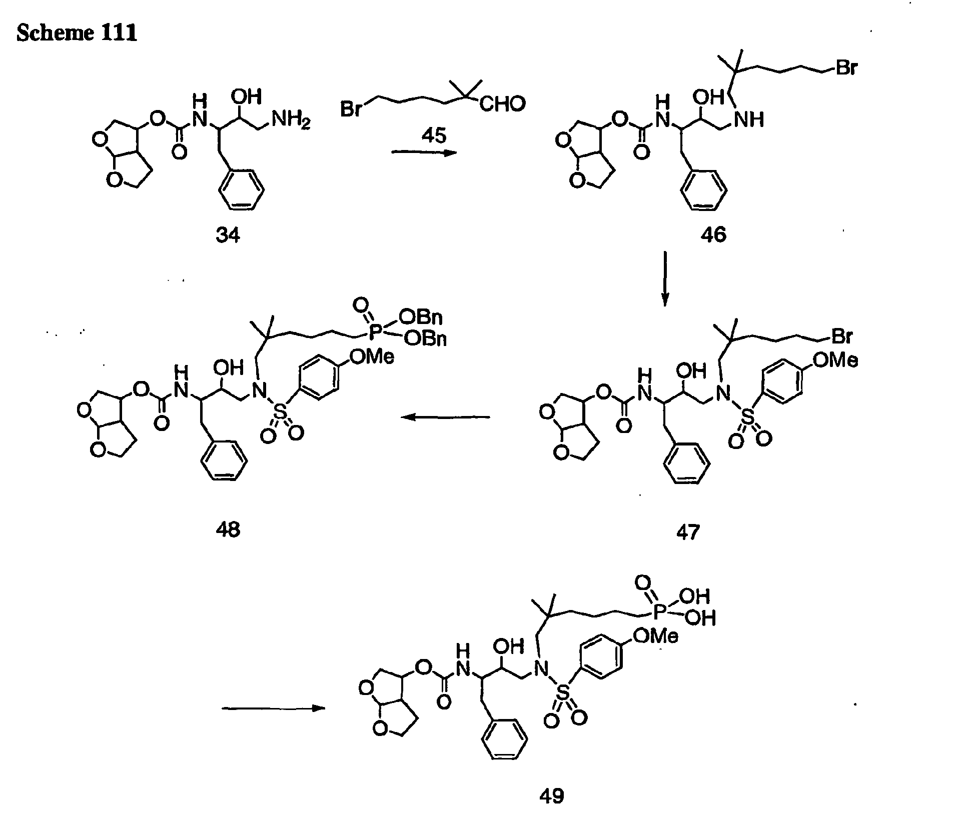

Reductive amination of the bromoaldehyde 45 (J. Organomet. Chem., FR; 122, 123, 1976) with the amine 34 gives 46 which then undergoes sulfonylation to furnish 47. The bromoderivative 47 is converted to the phosphonate 48 under Michaelis-Arbuzov reaction conditions (Bioorg. Med. Chem. Lett., 9, 3069, 1999). Final hydrogenation of 48 delivers the phosphonic acid 49.

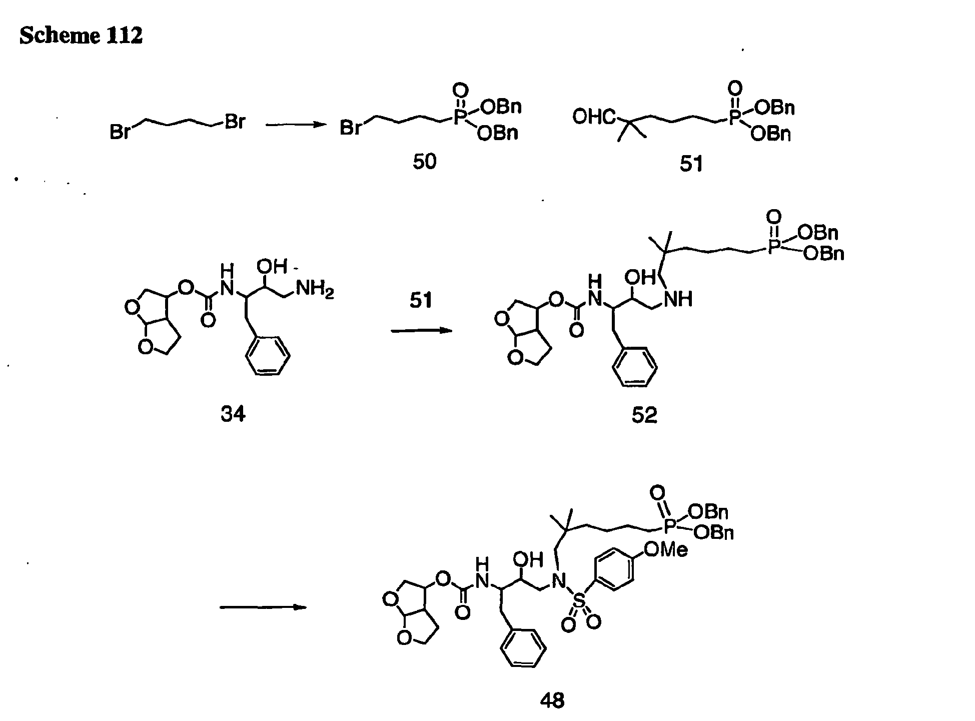

The intermediate 48 is also obtained as shown in scheme 112. Reductive amination of the aldehyde 52 with the amine 34 offers the phosphonate 52 and sulfonylation of this intermediate furnishes 48.

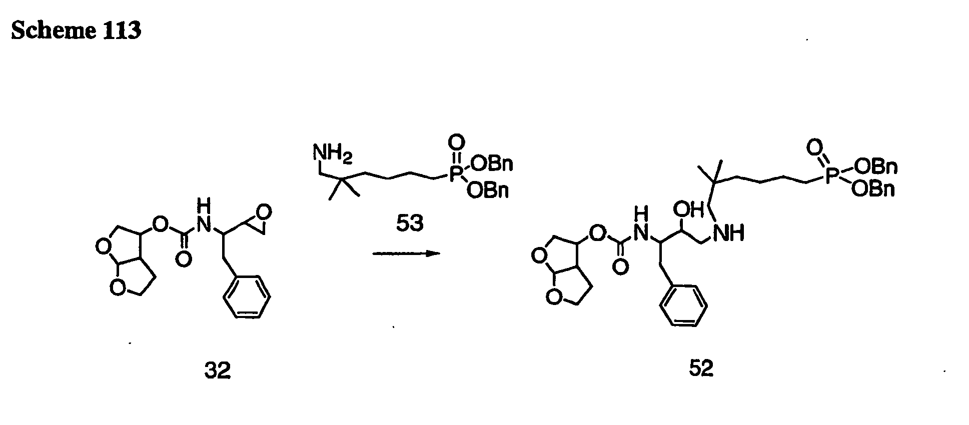

Alternatively, compound 52 is obtained from the epoxide 32 by a ring opening reaction with the aminophosphonate 53 (Scheme 113).

### Scheme Section C

Scheme 9 is described in the Examples.

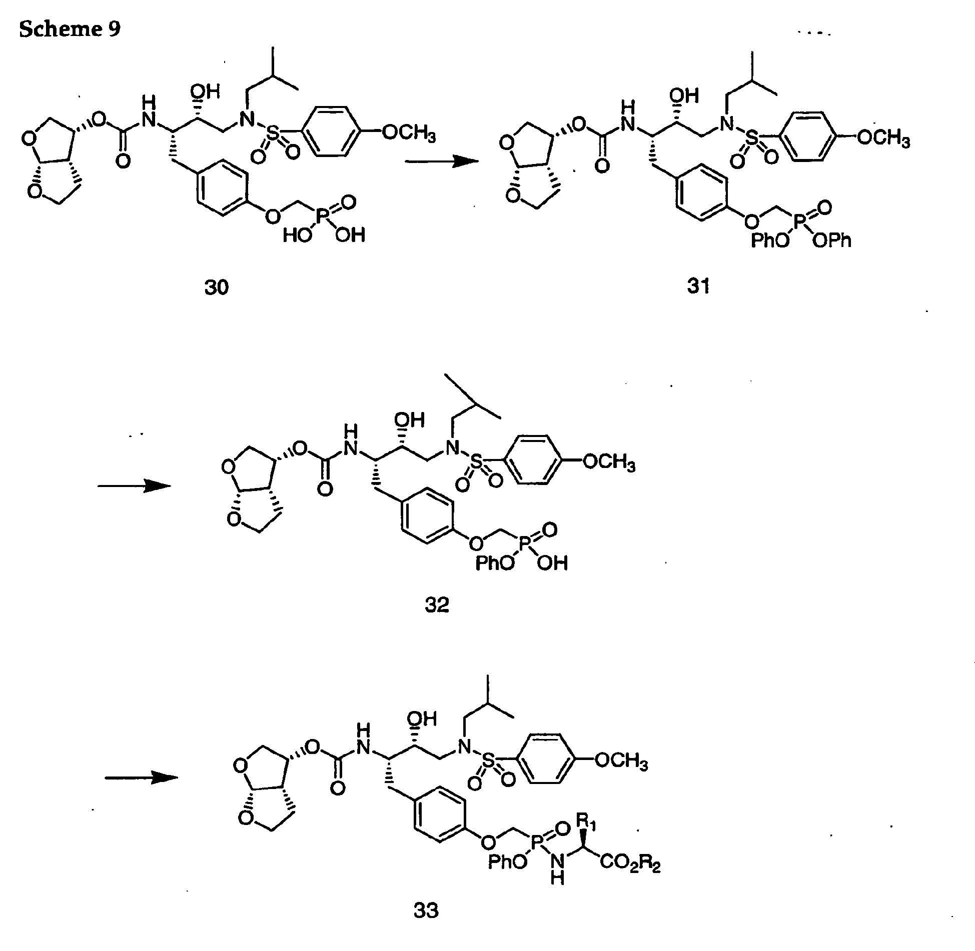

### Scheme Section D

The following schemes are described in the Examples.

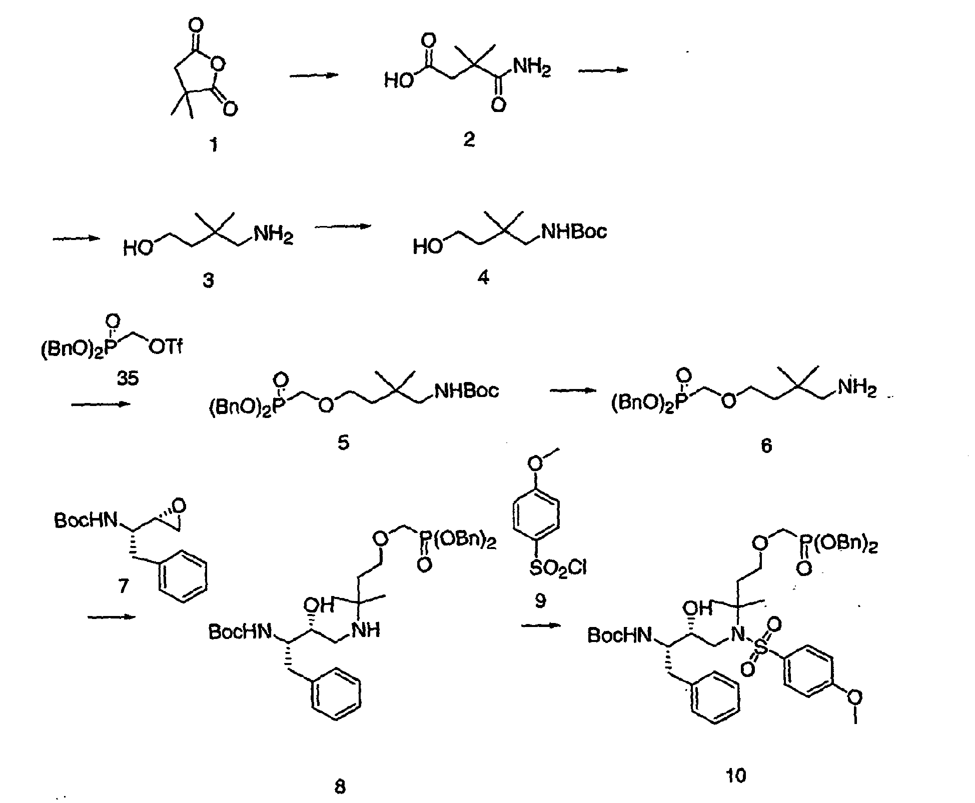

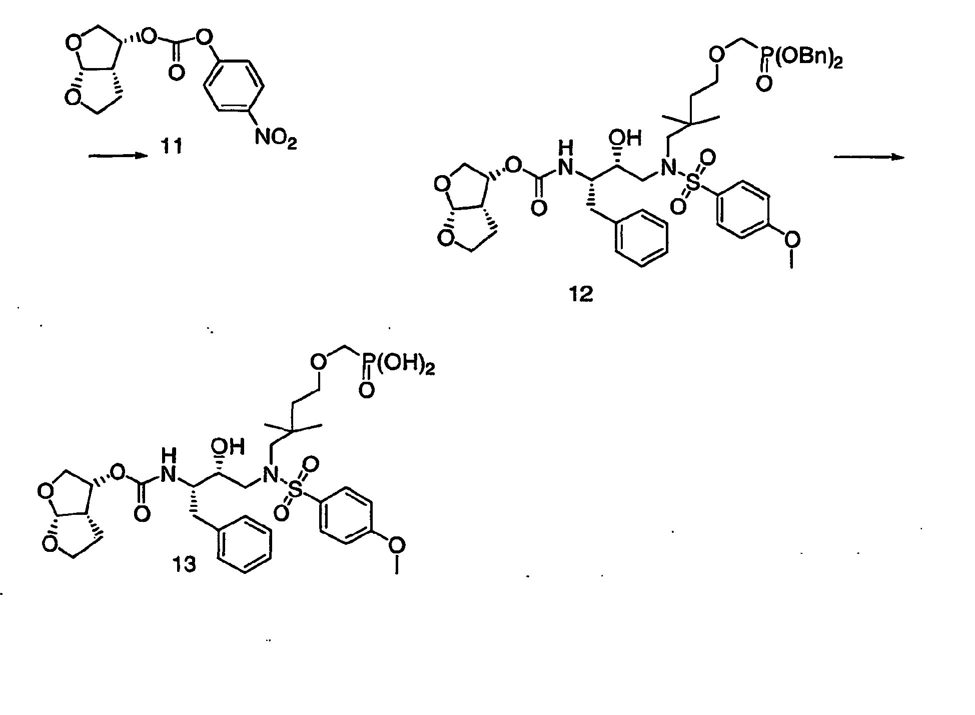

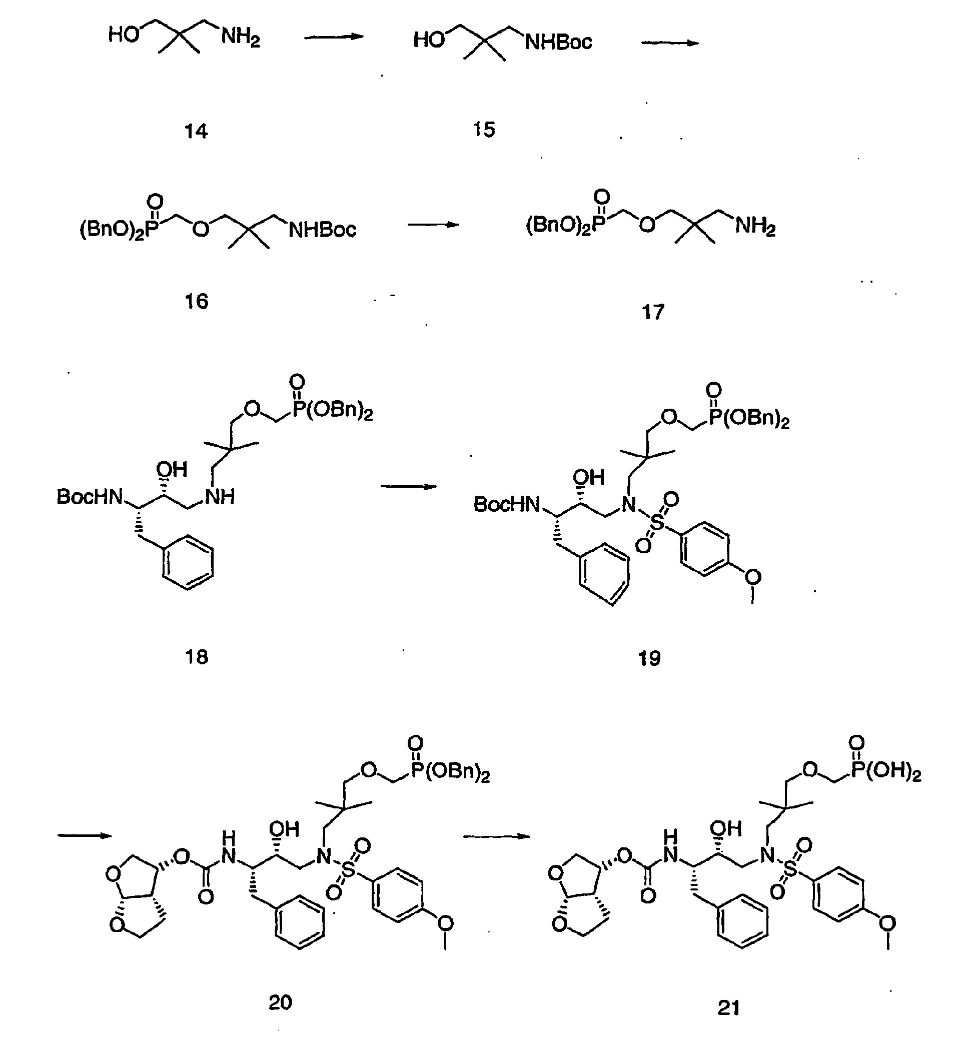

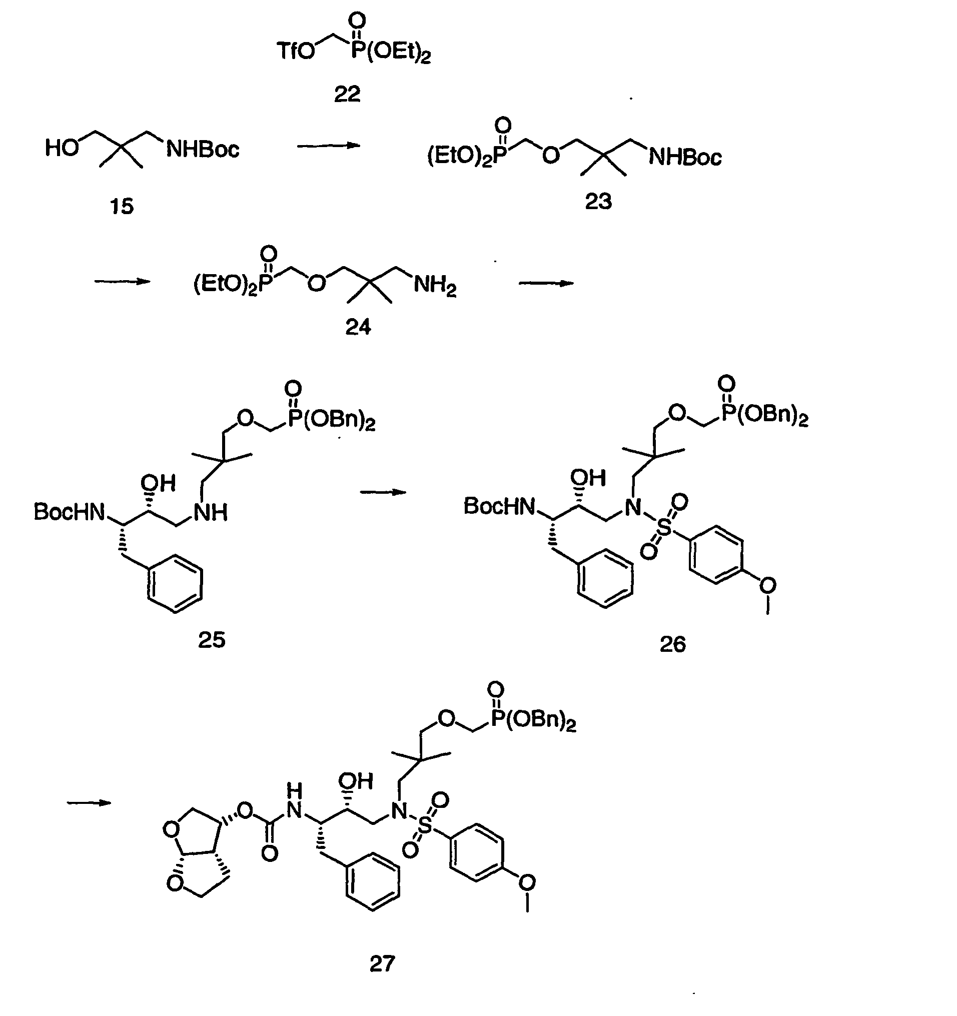

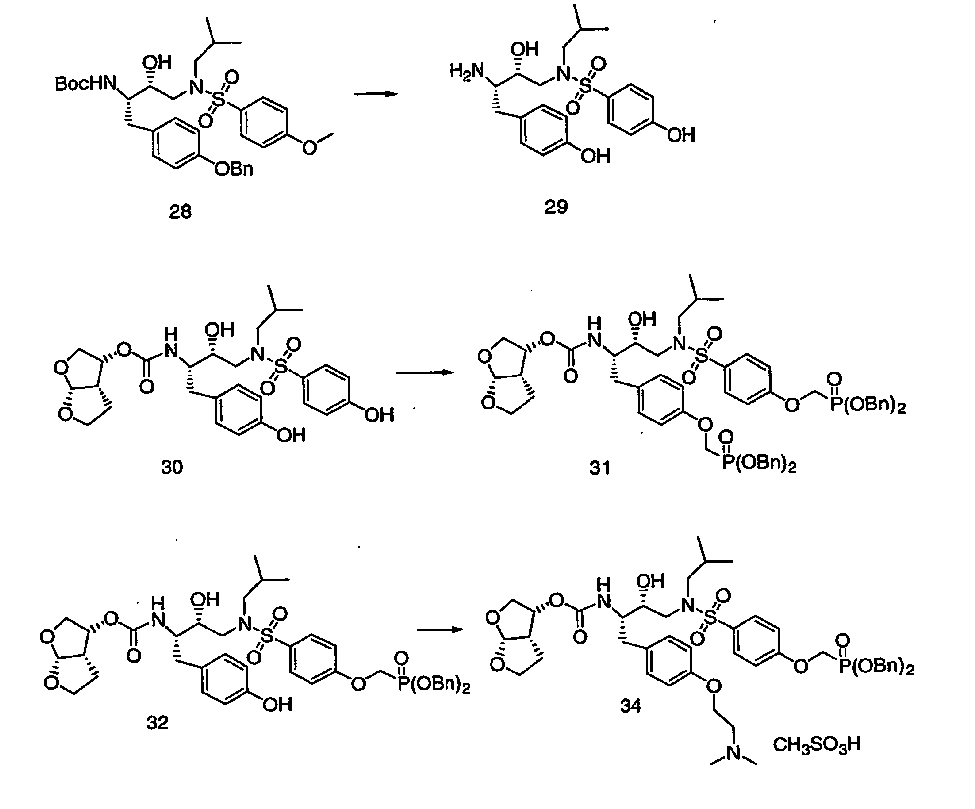

### Scheme Section E

Schemes 1-3 are described in the examples.

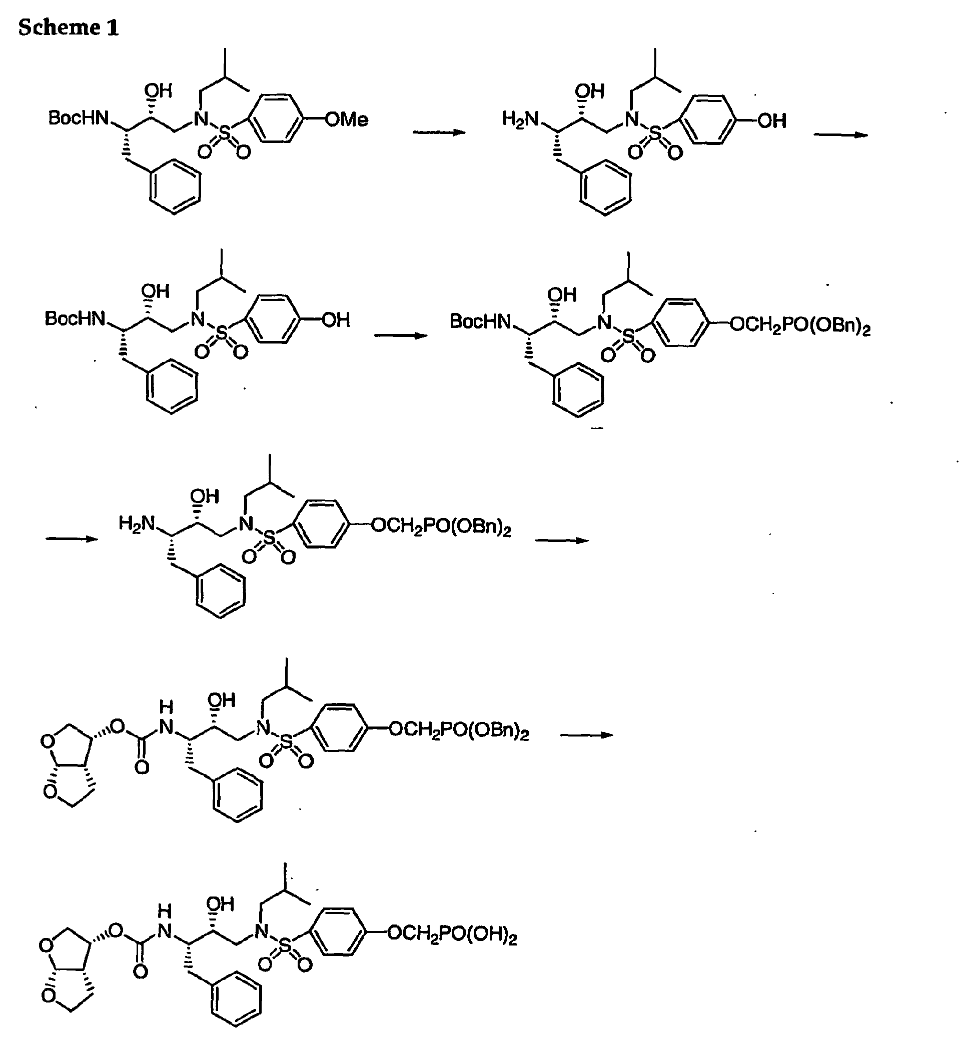

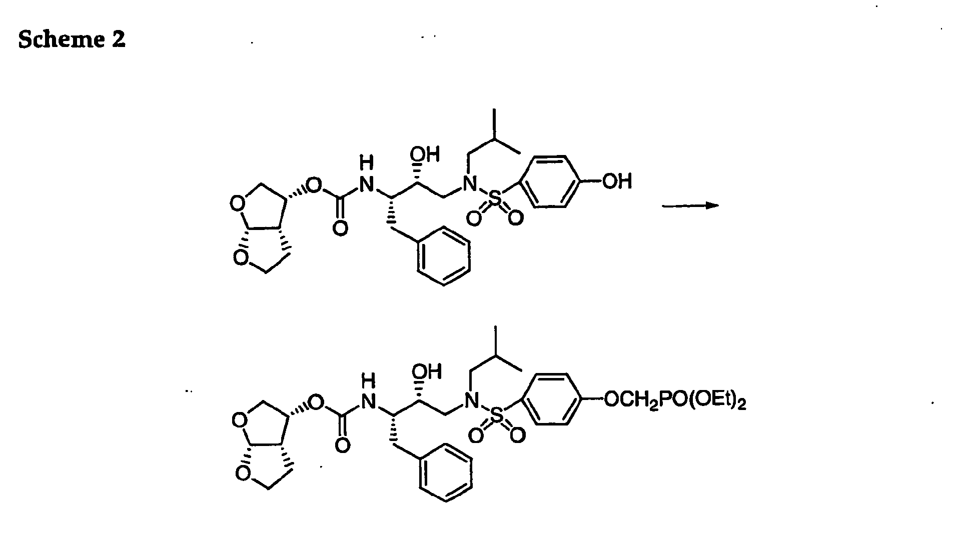

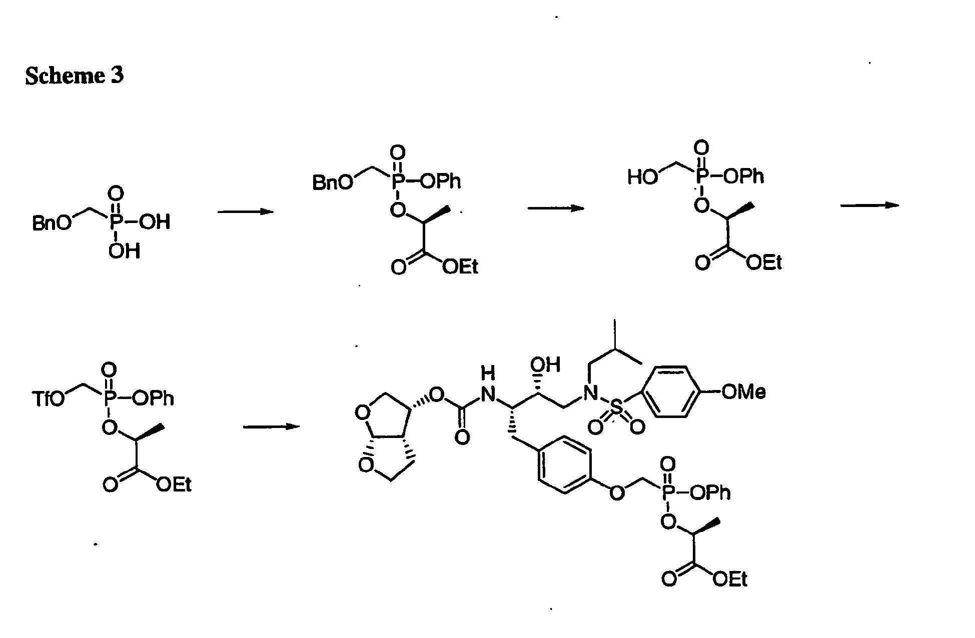

### Scheme Section F

Schemes 1-5 are described in the examples.

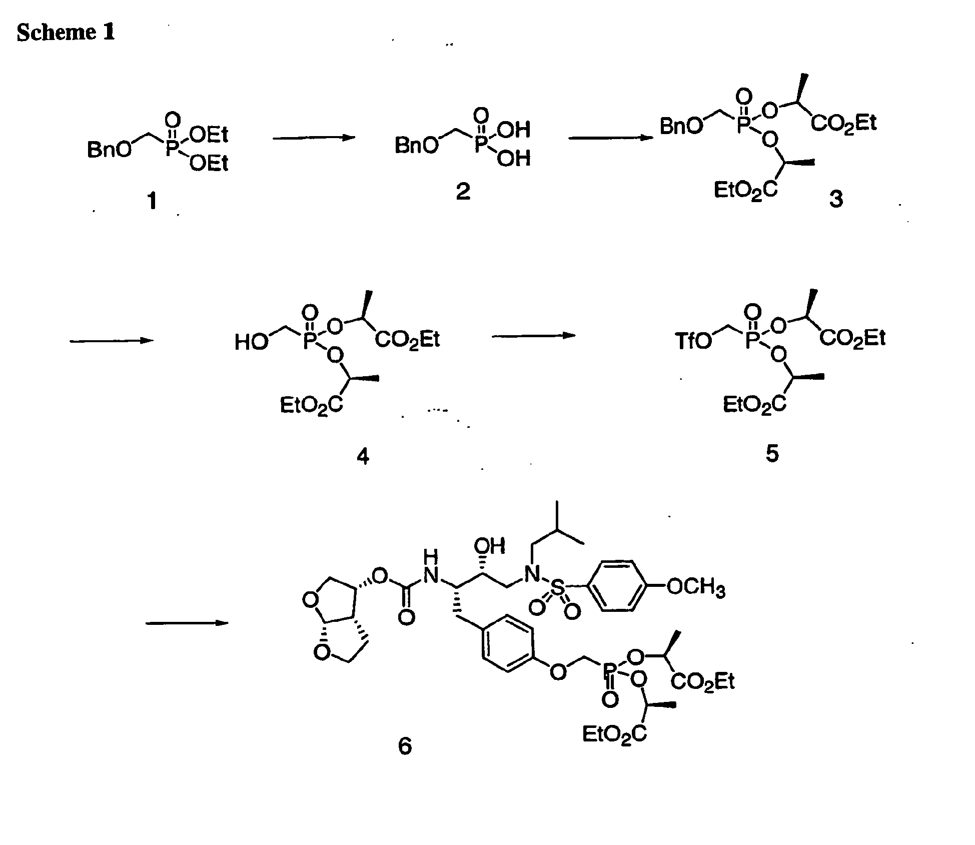

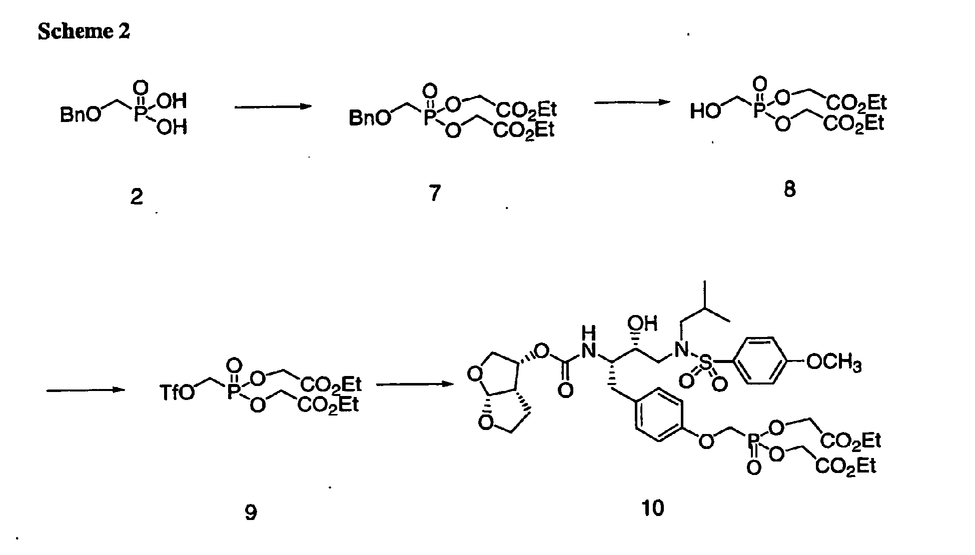

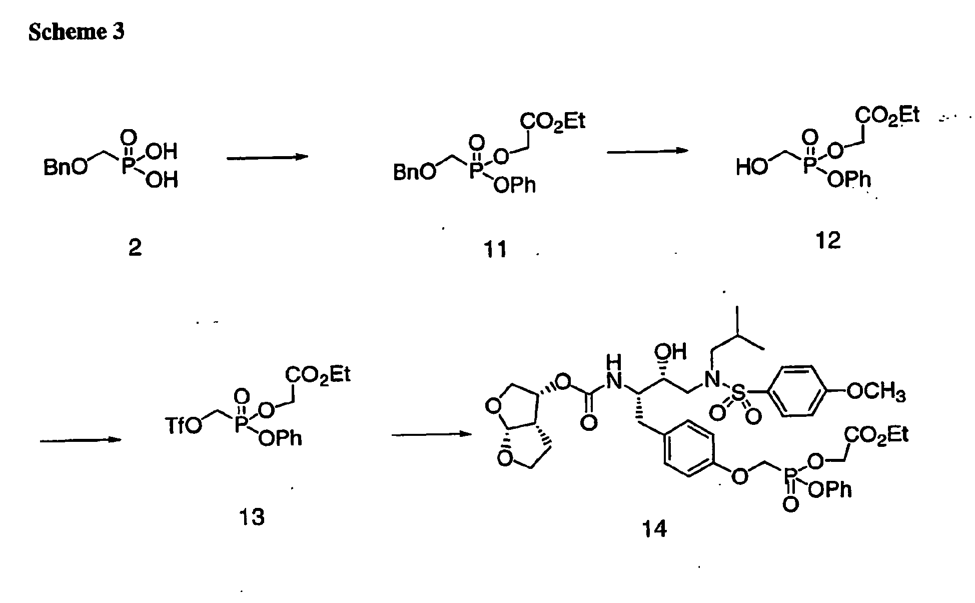

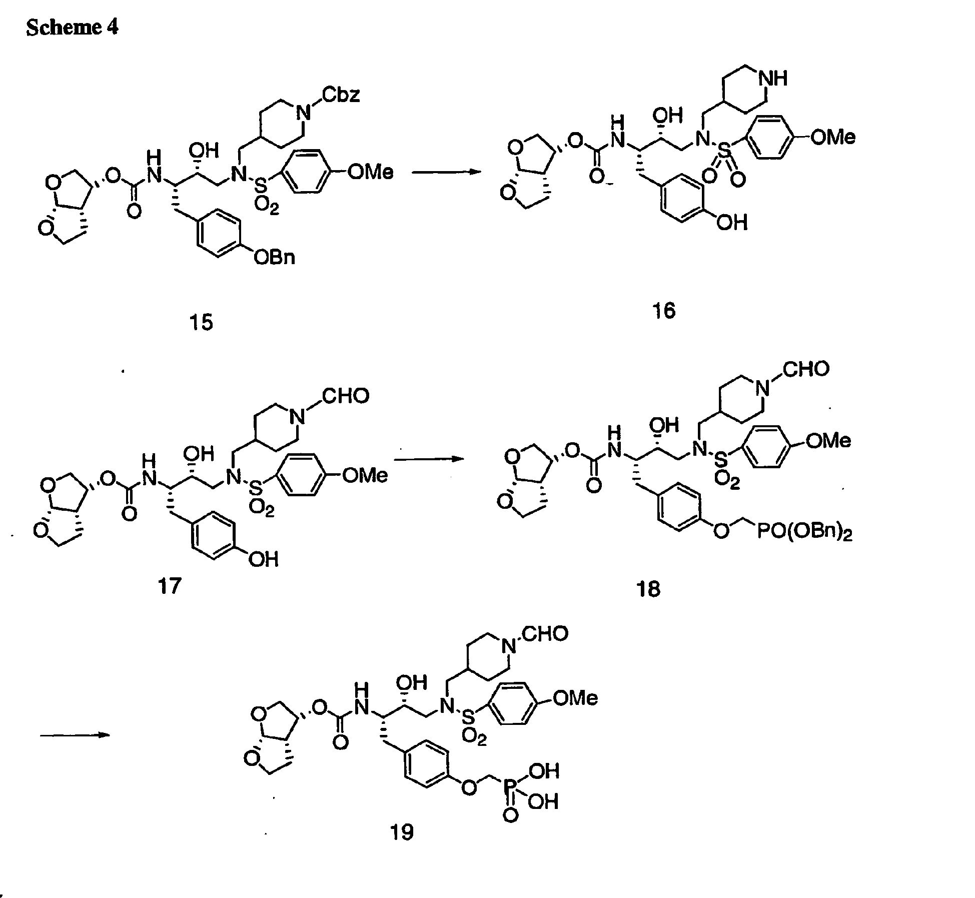

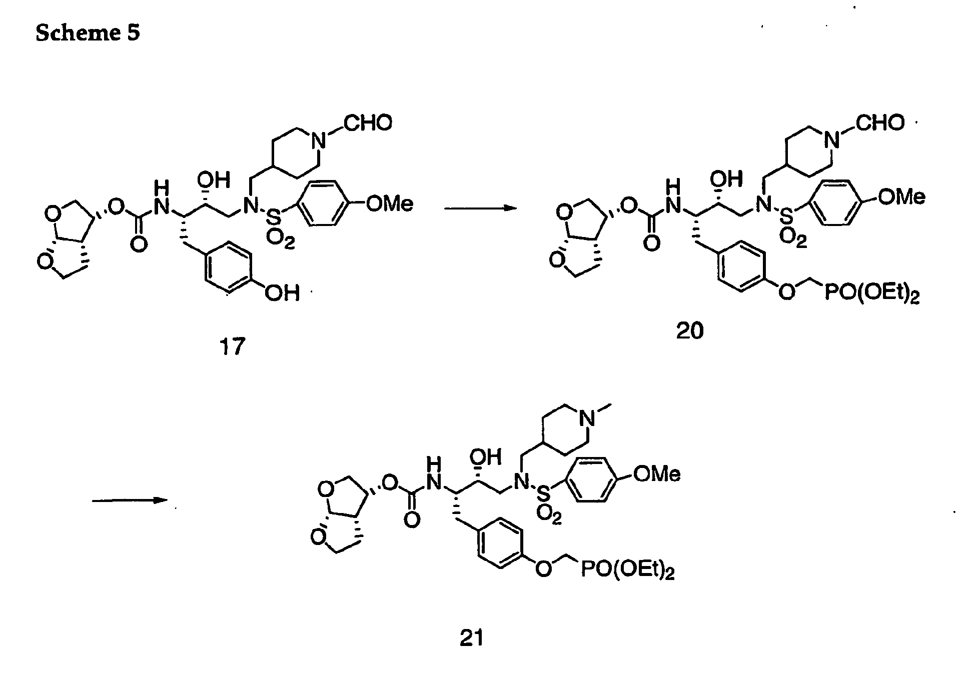

### Scheme Section G

Schemes 1 to 9 are described in the examples.

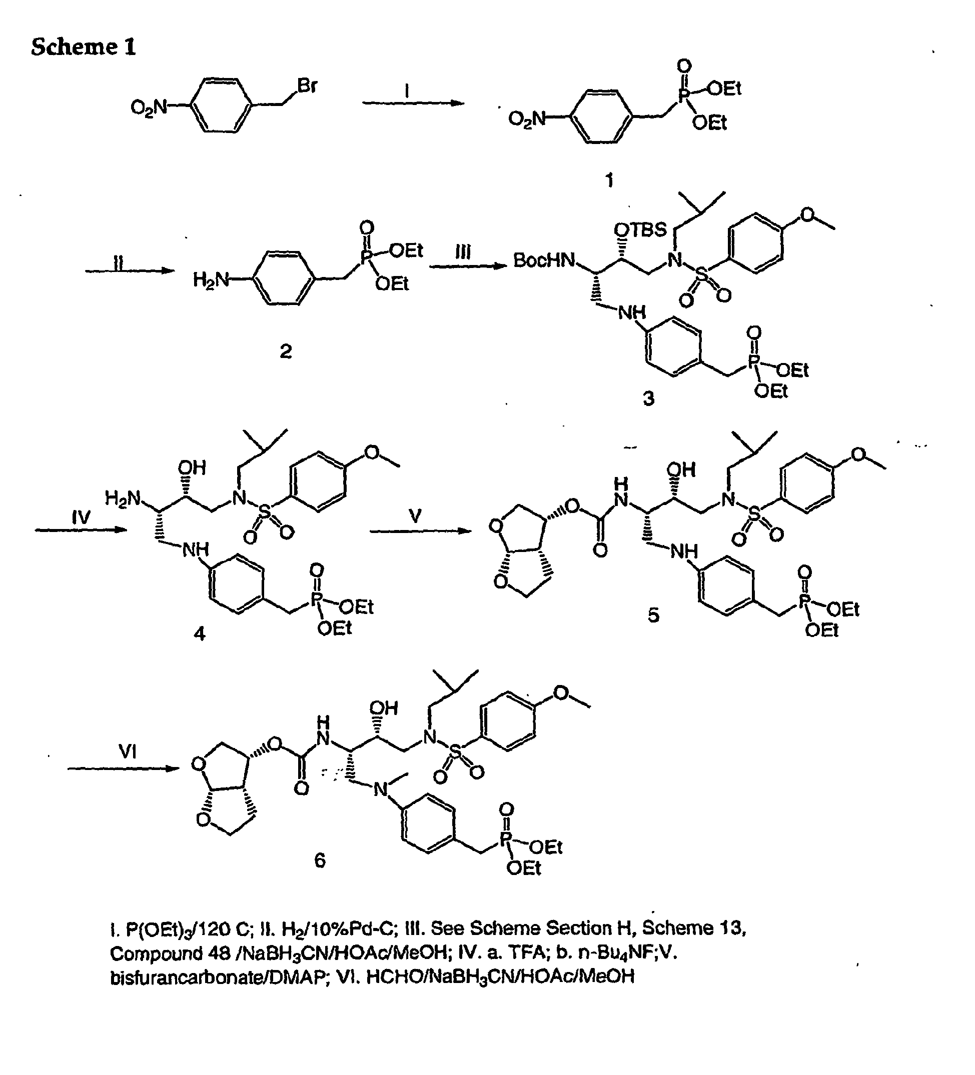

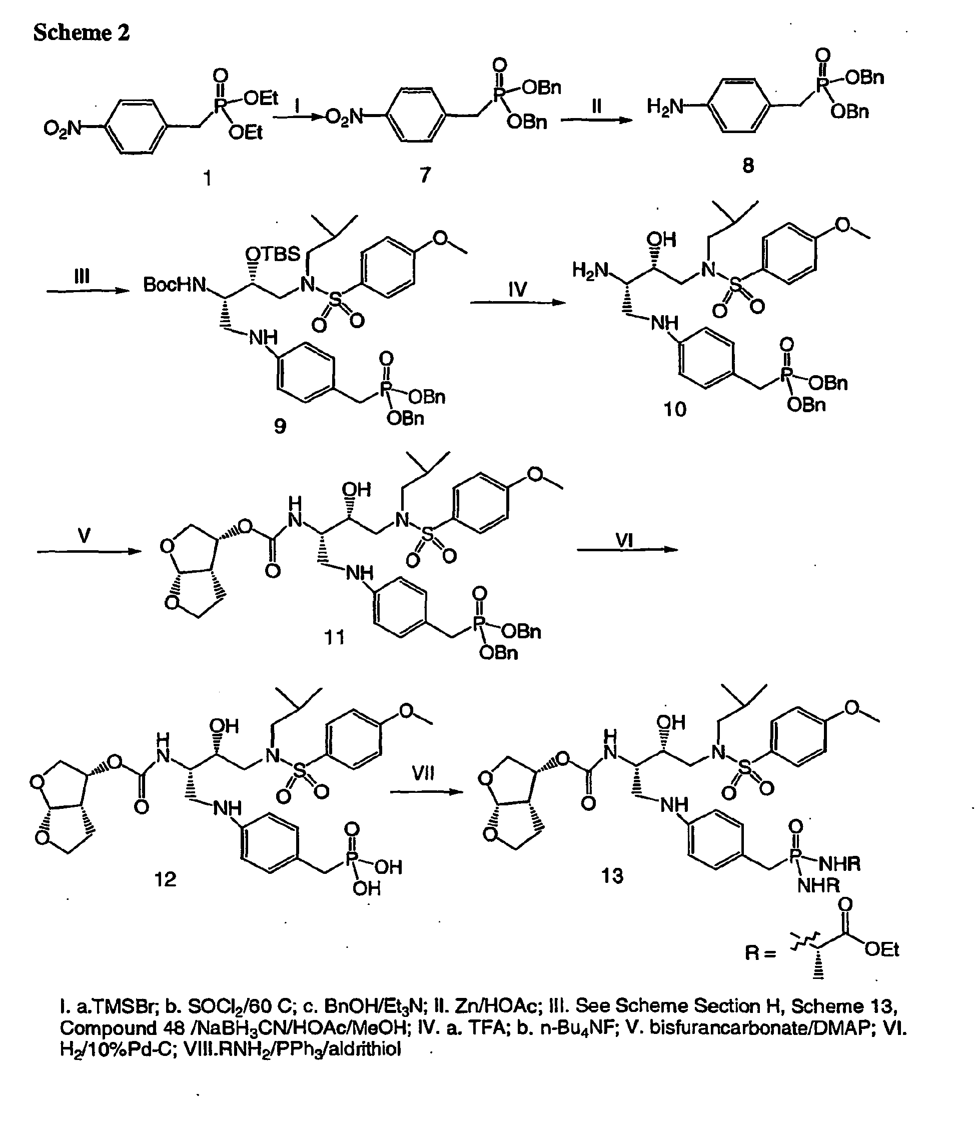

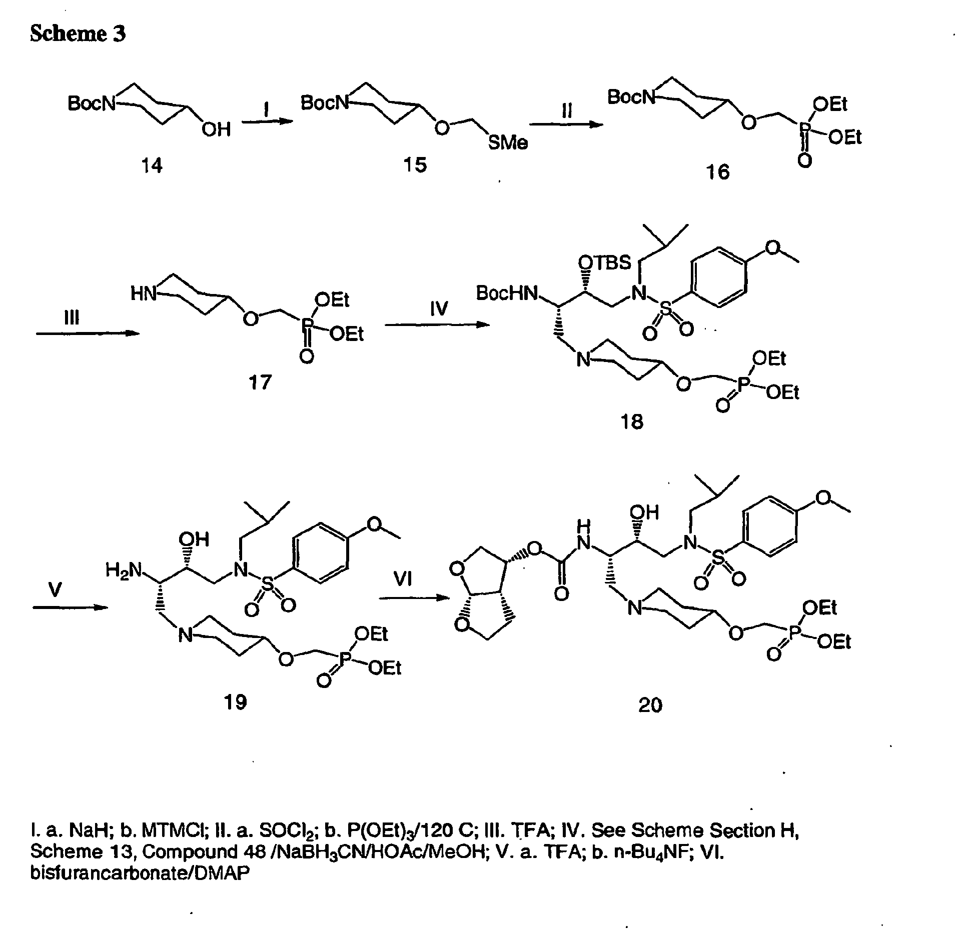

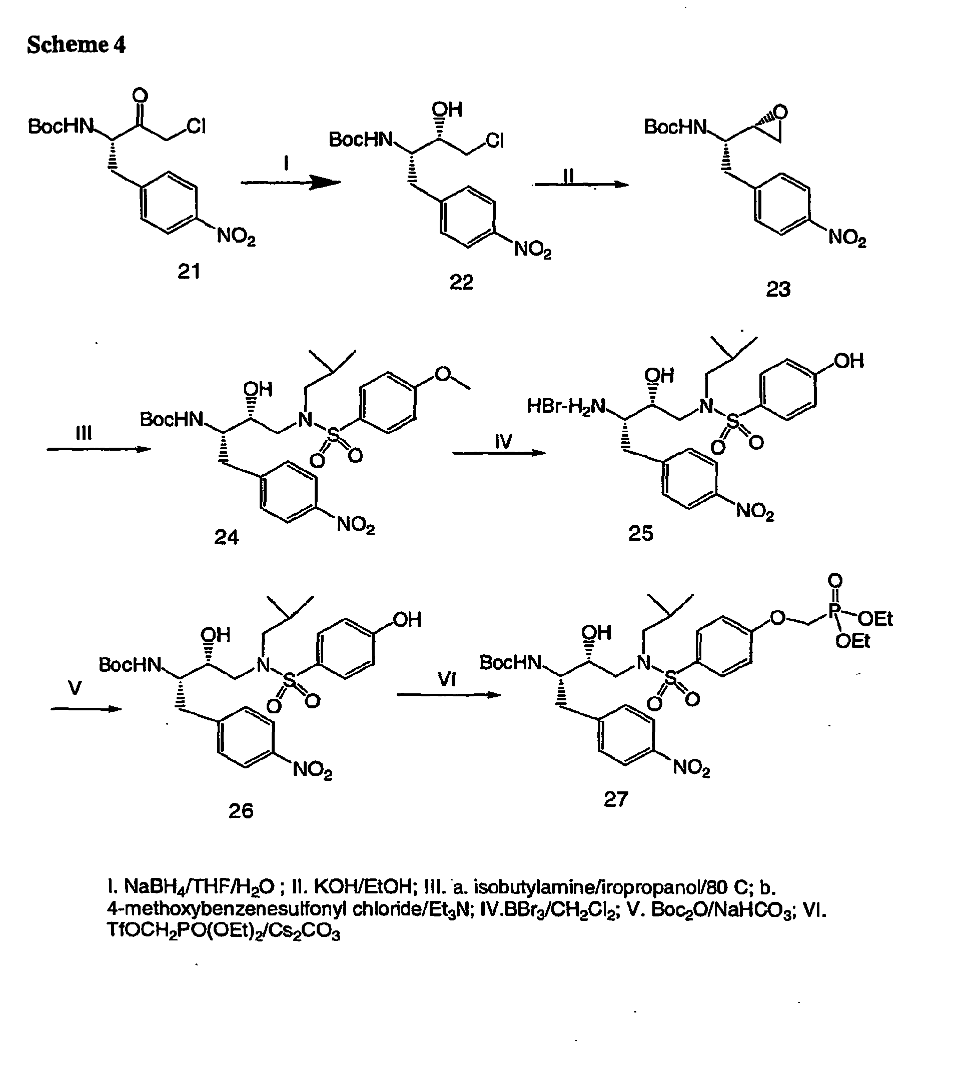

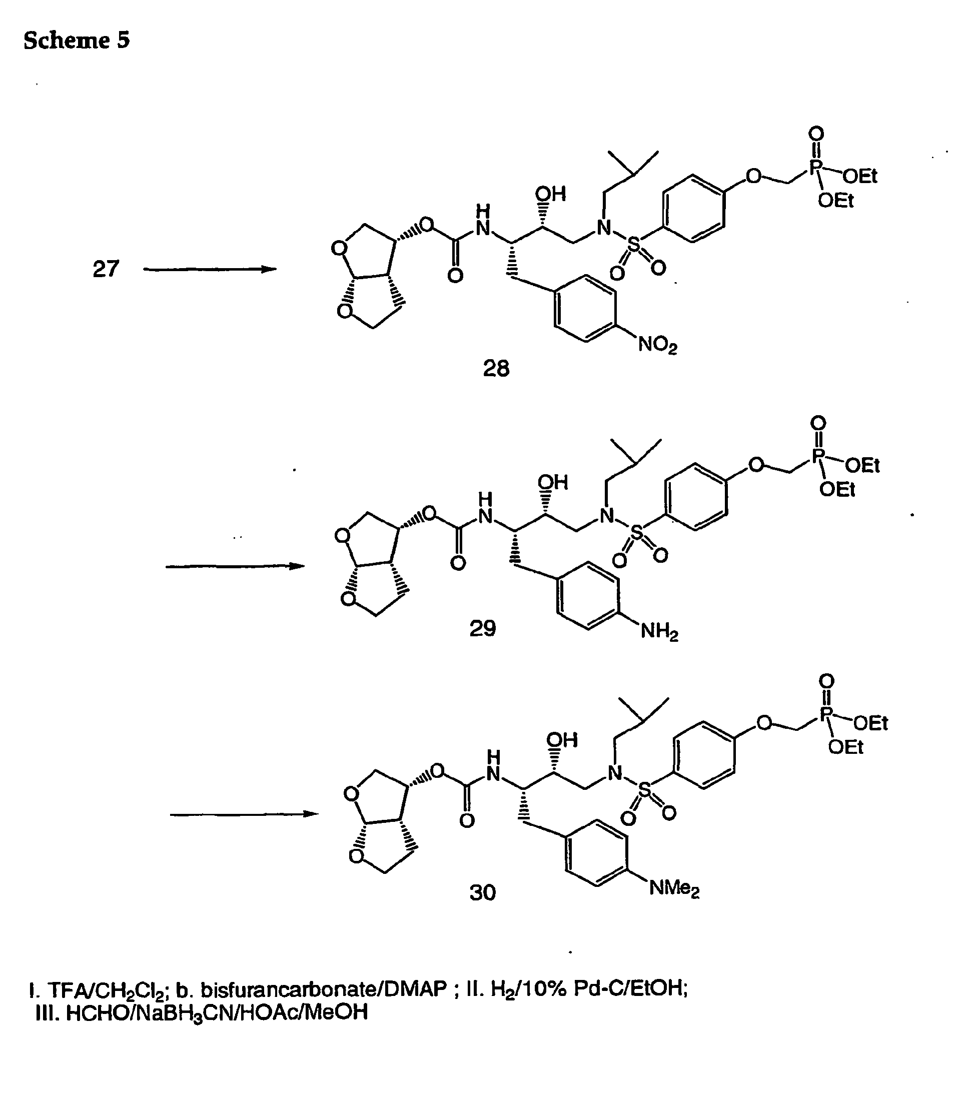

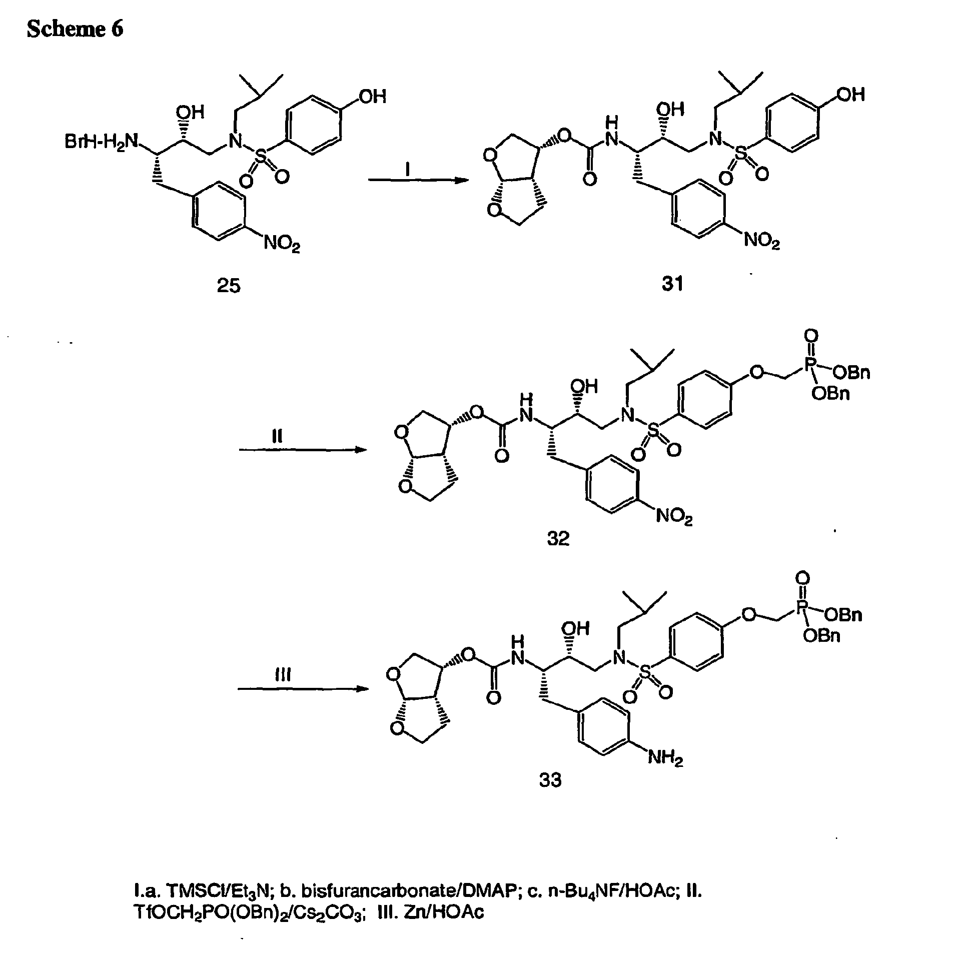

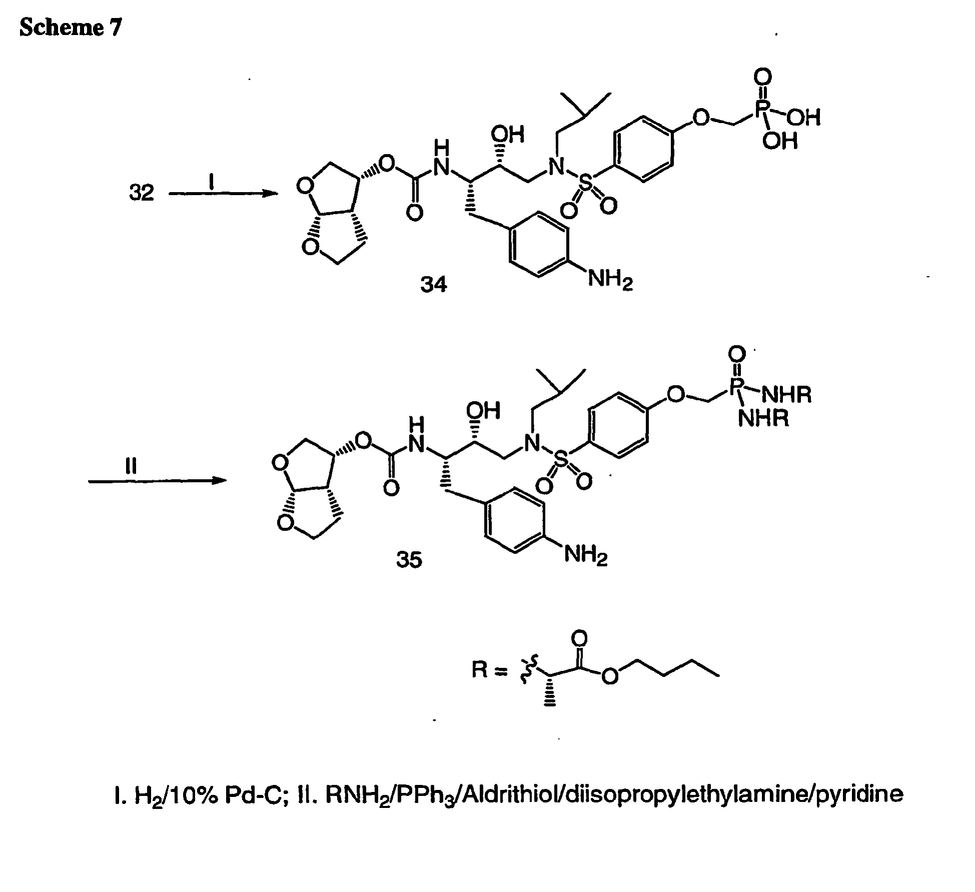

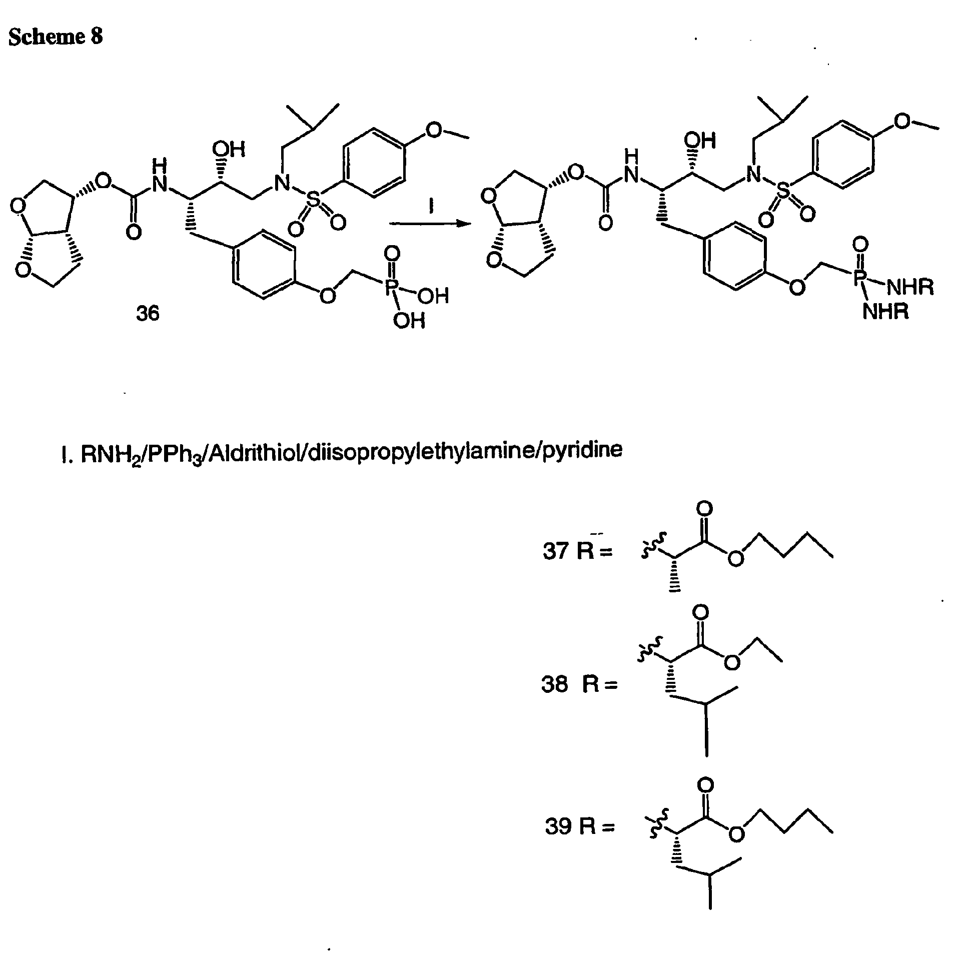

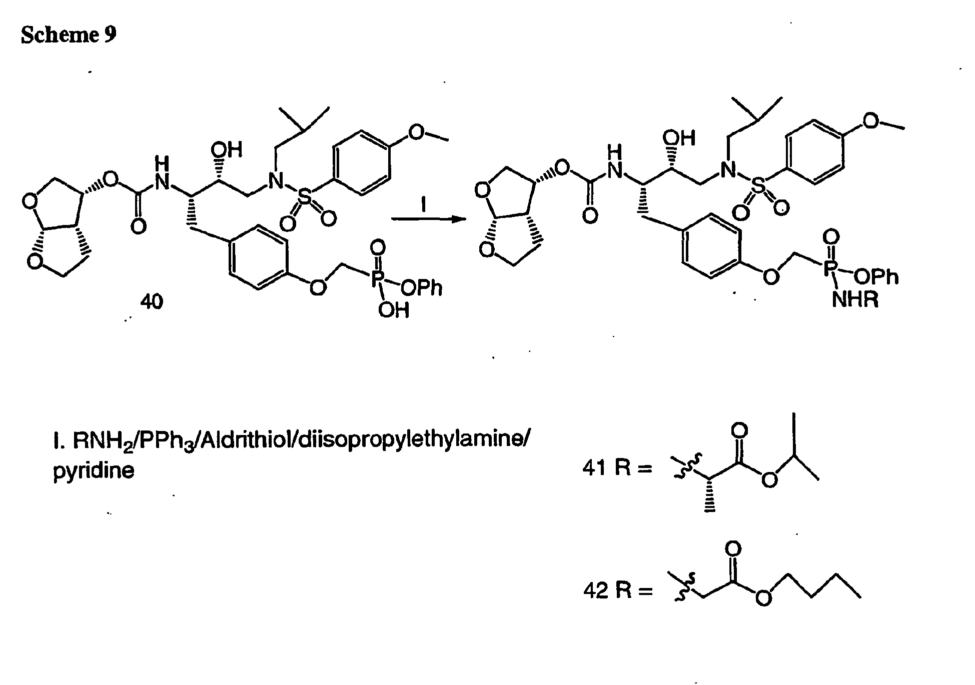

### Scheme Section H

Schemes 1-14 are described in the examples.

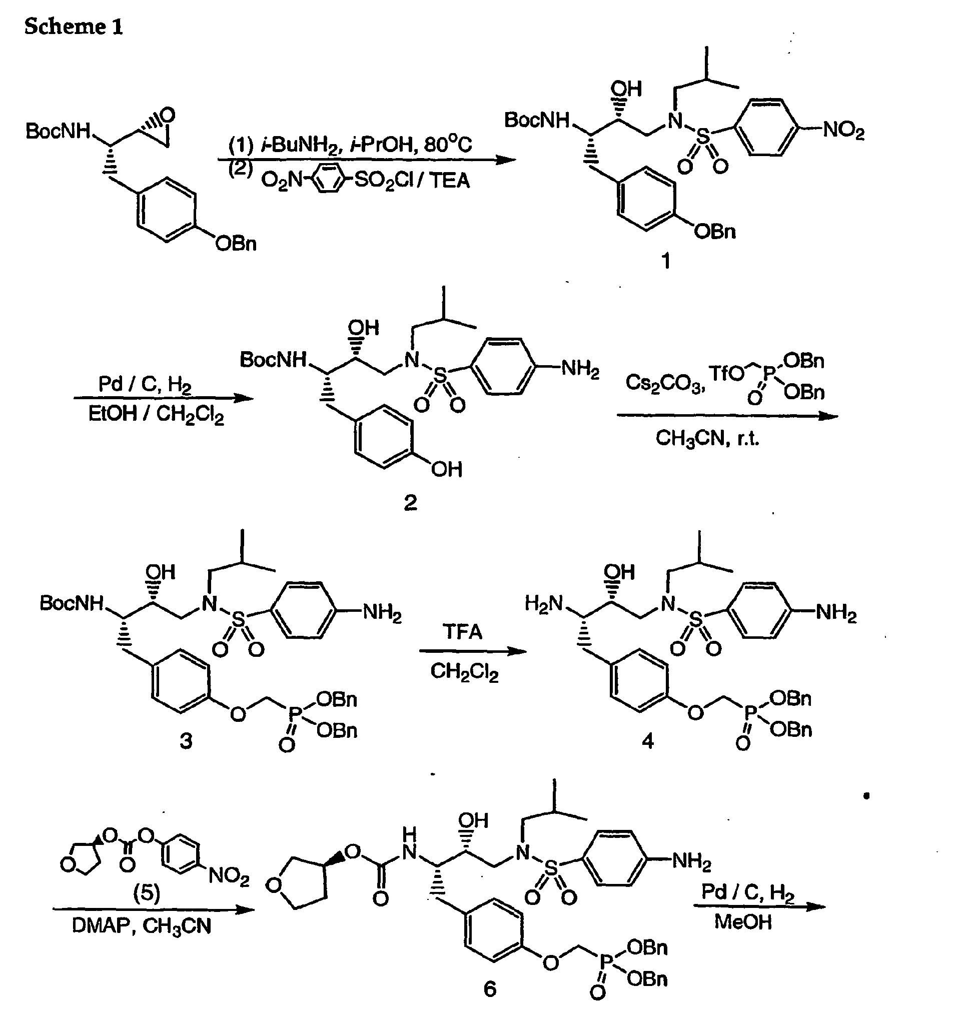

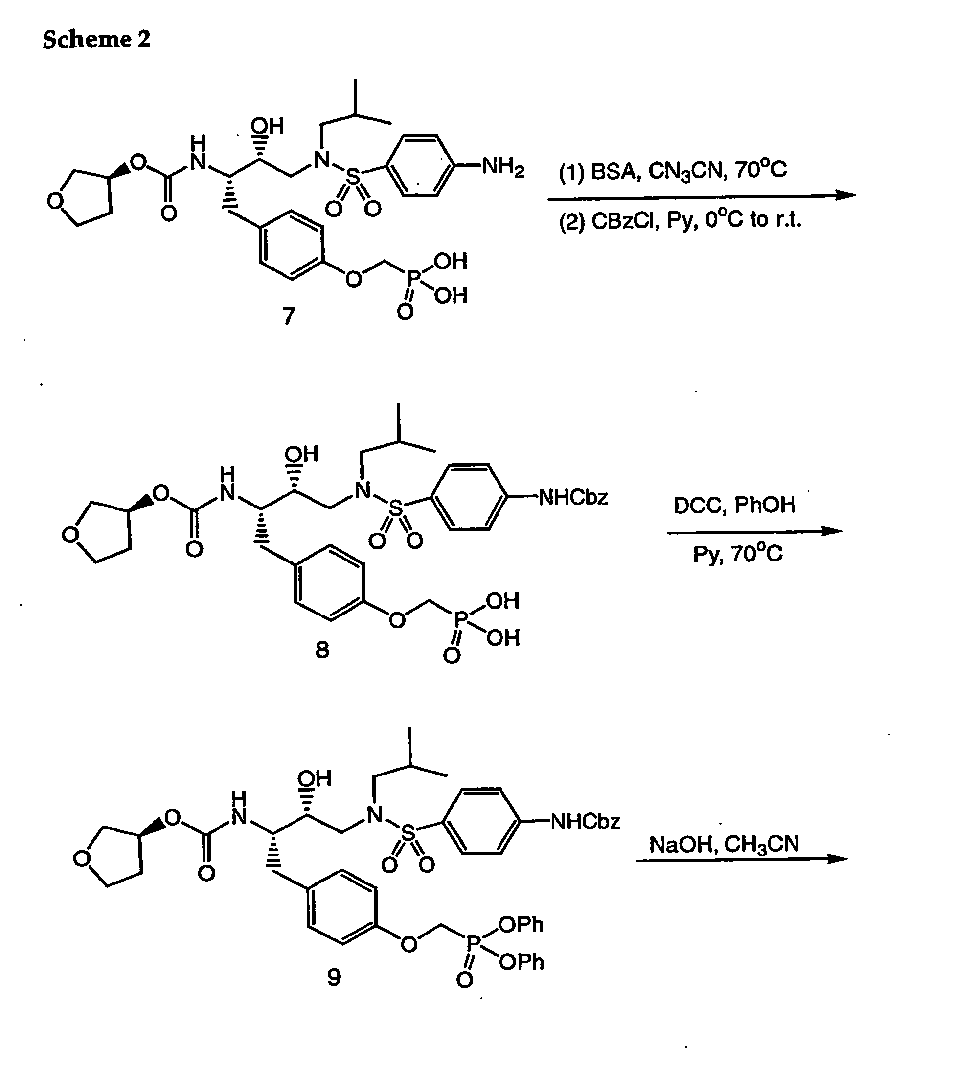

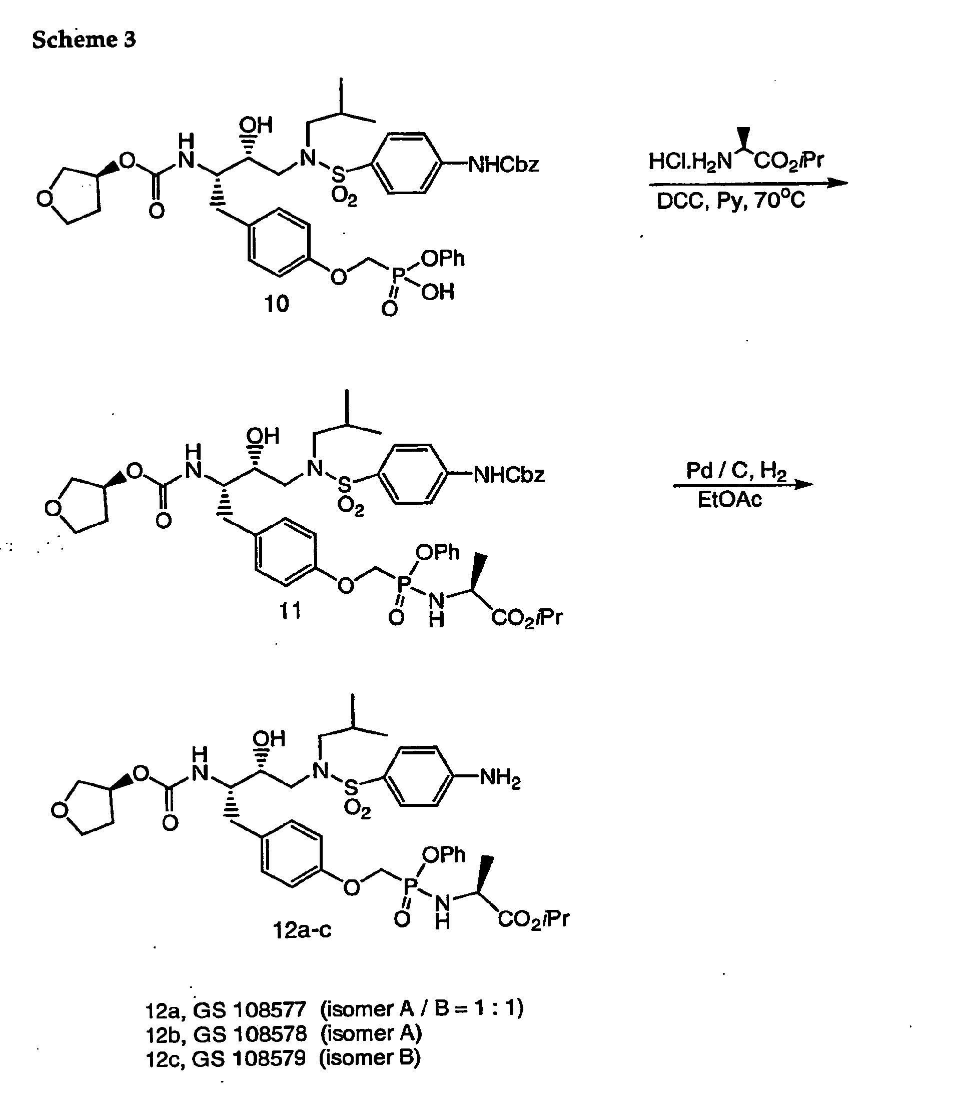

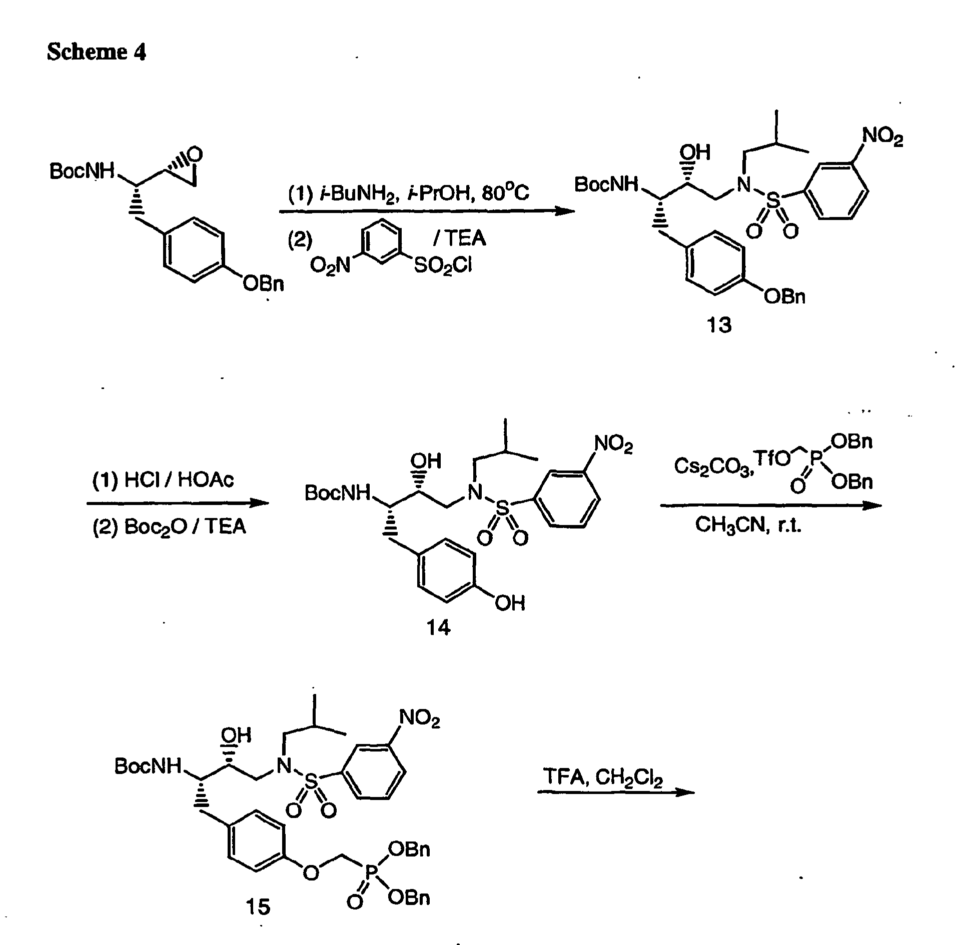

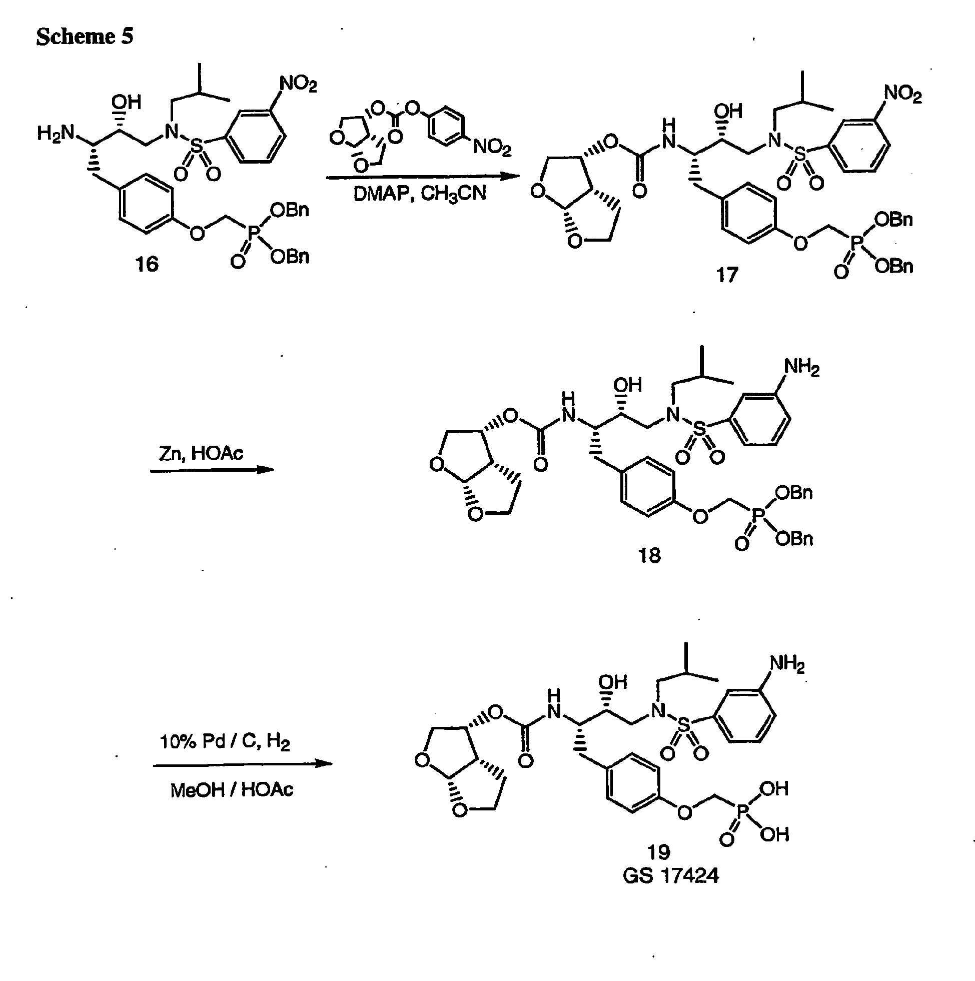

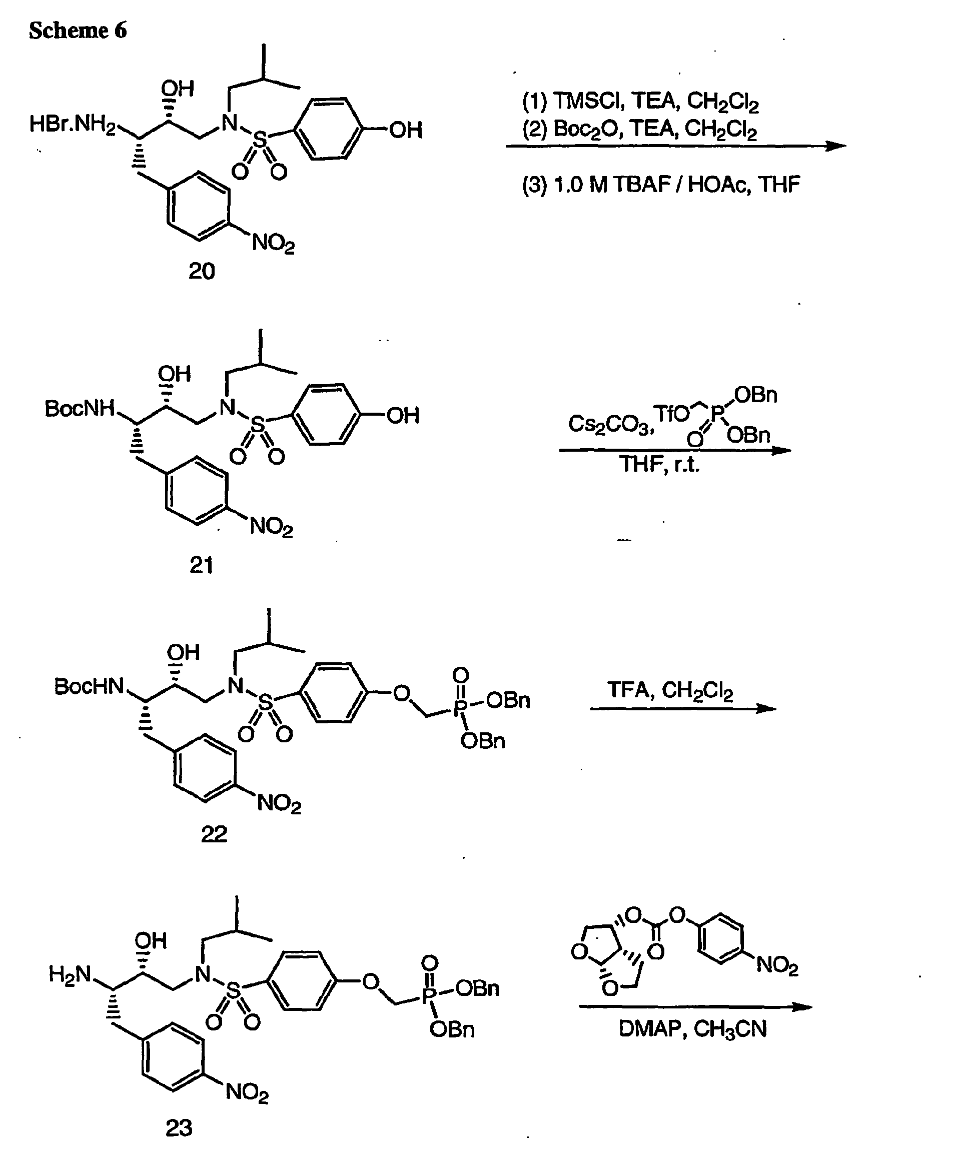

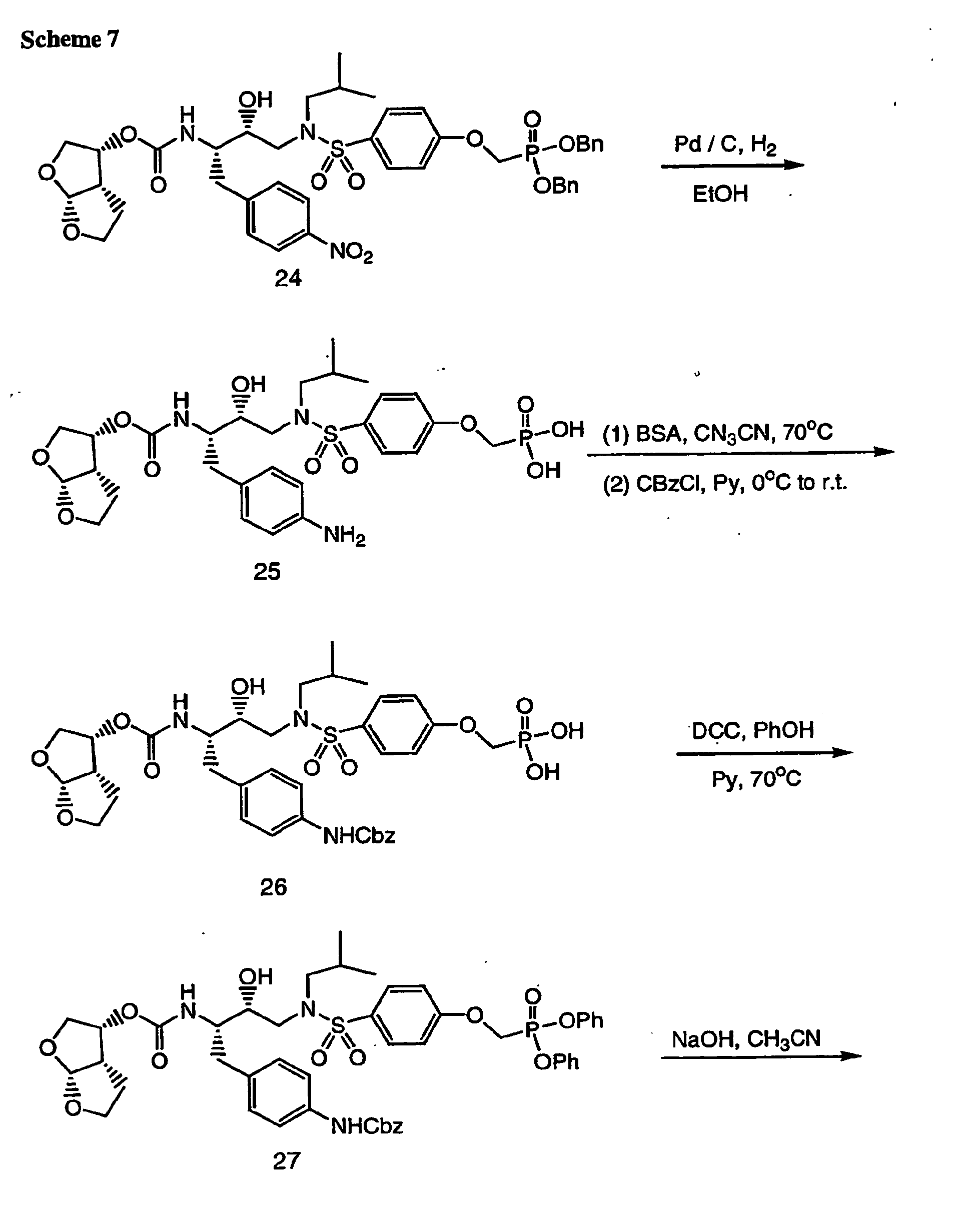

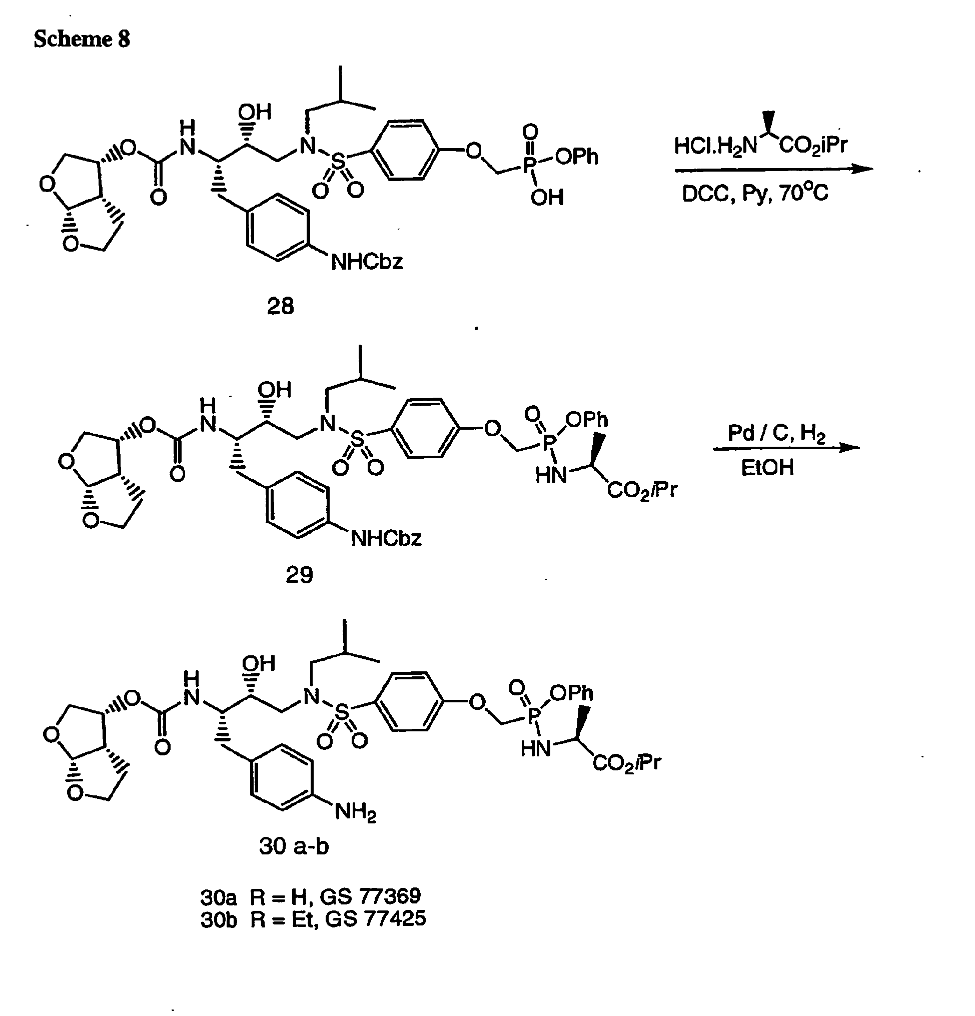

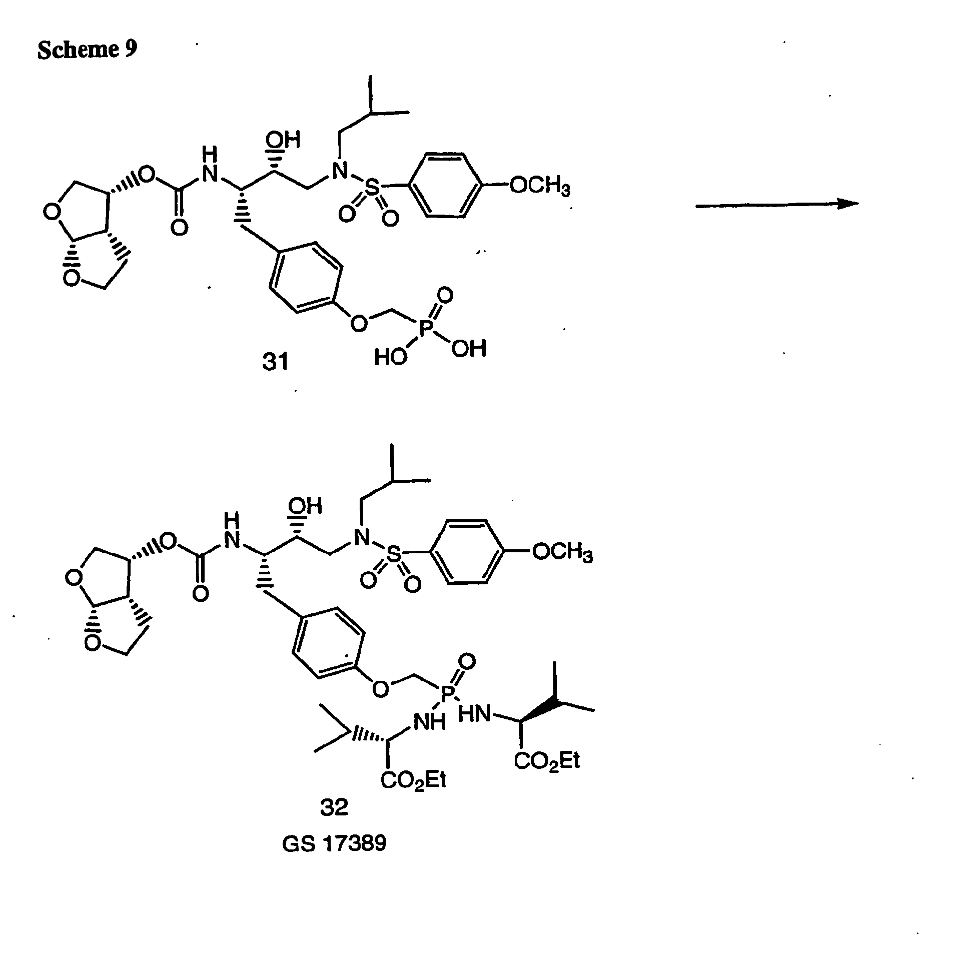

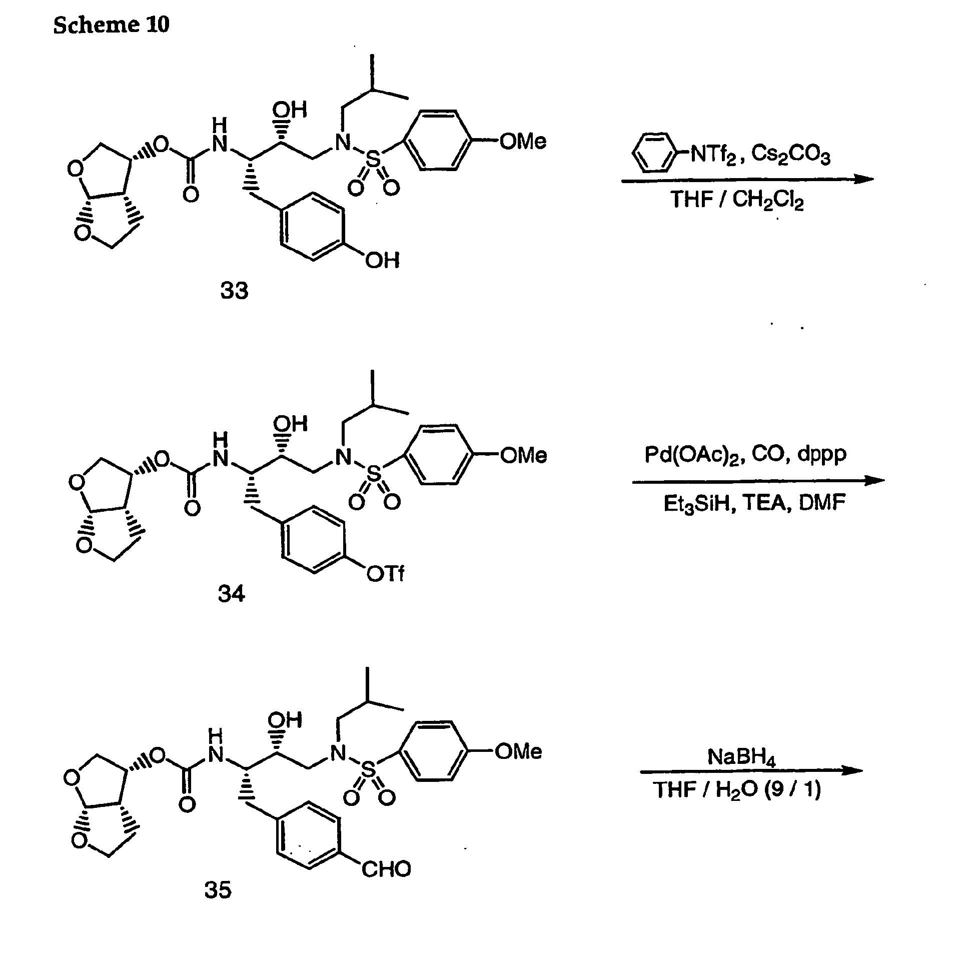

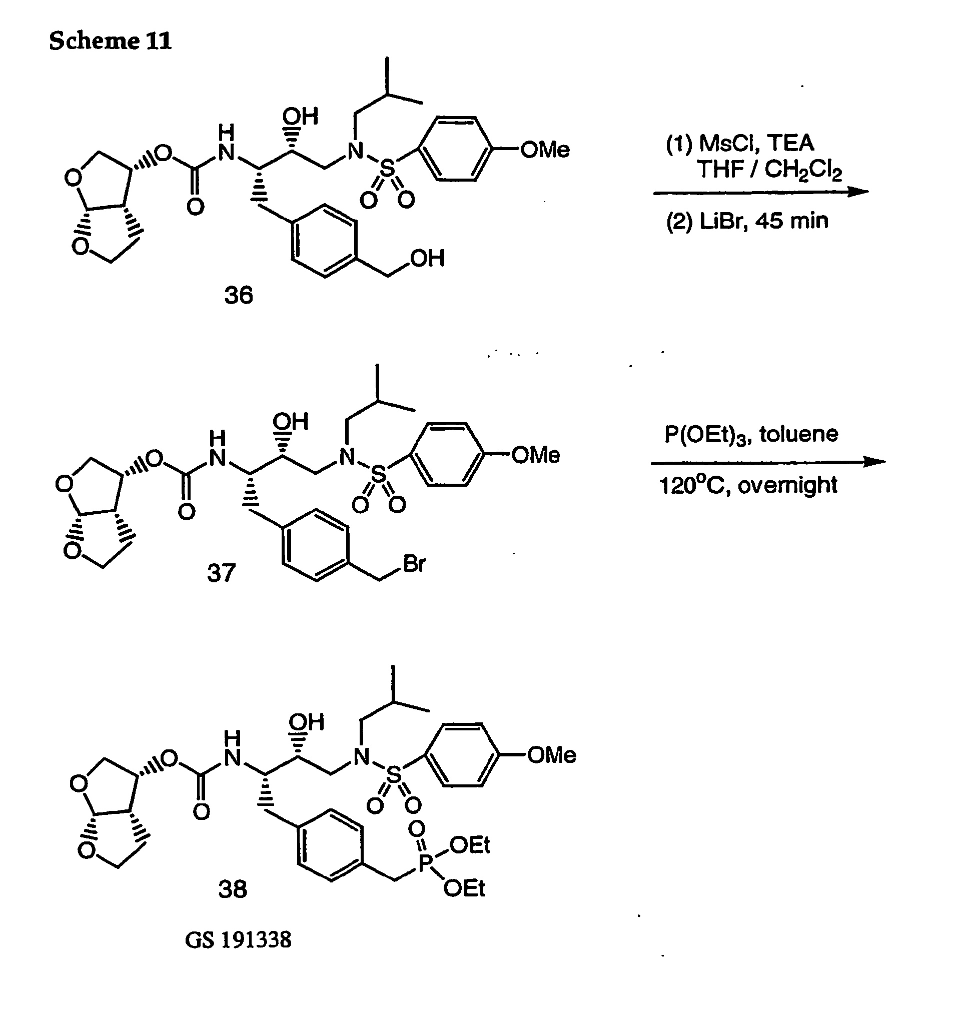

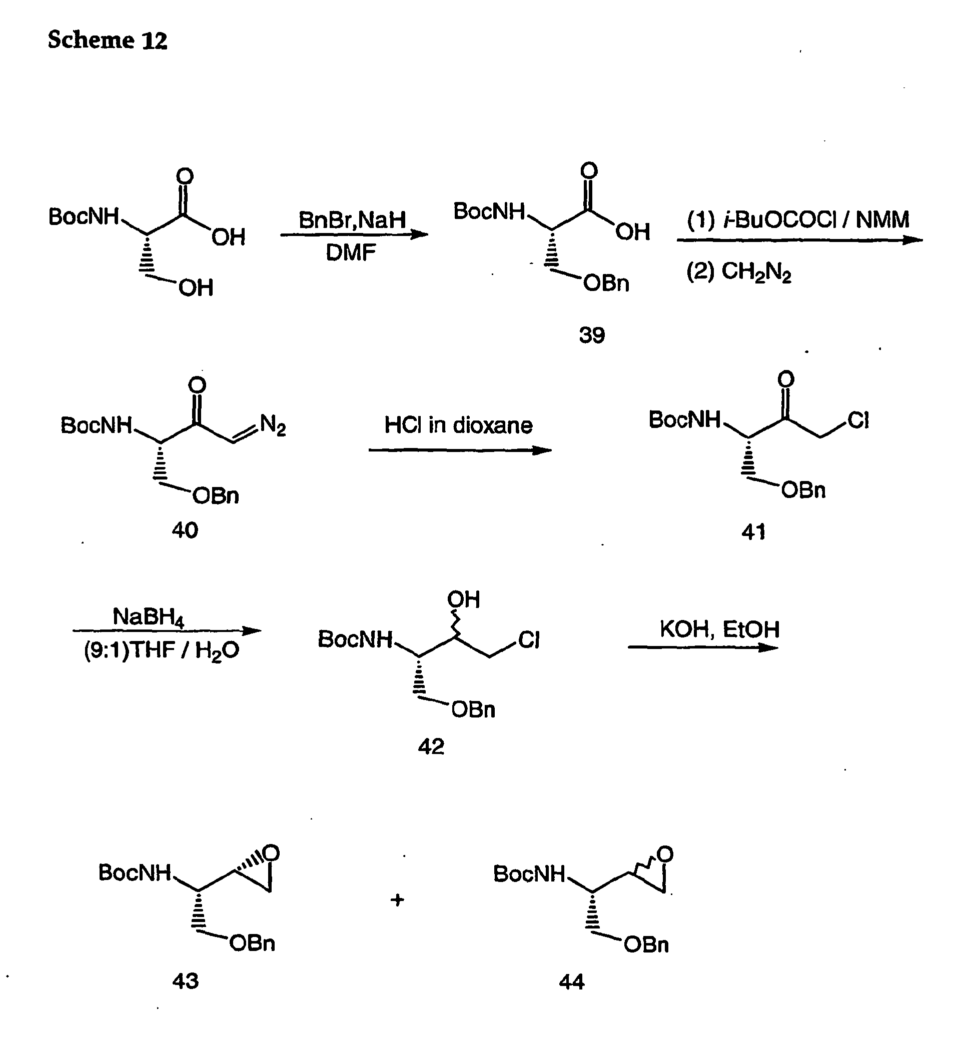

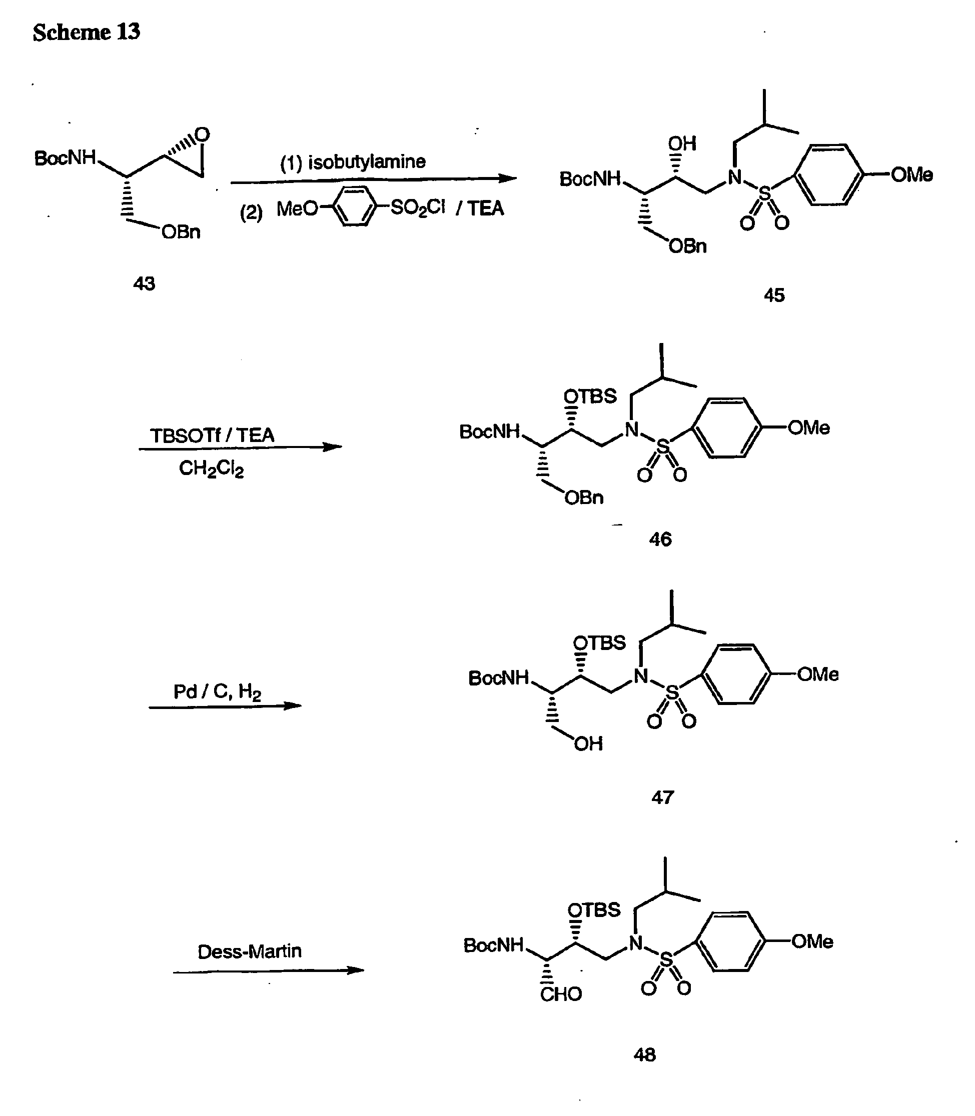

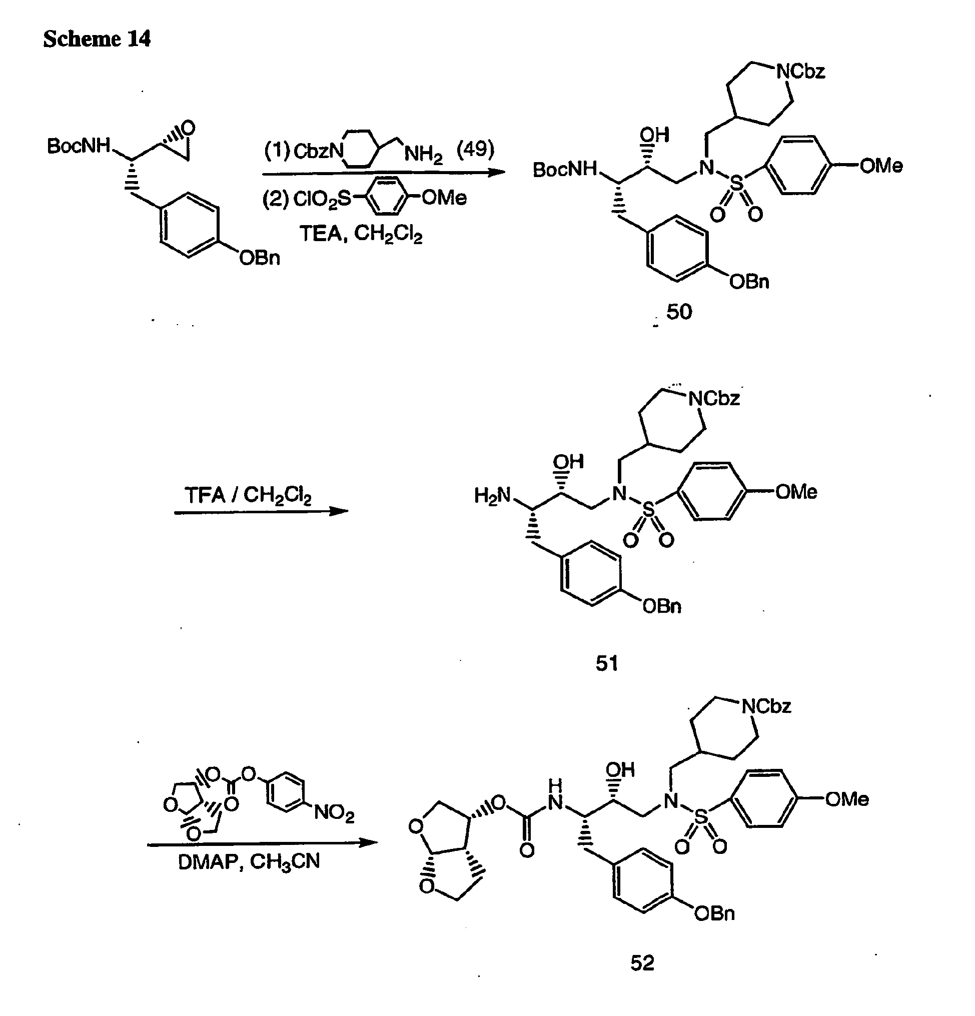

### Scheme Section I

Schemes 1 to 3 are described in the examples.

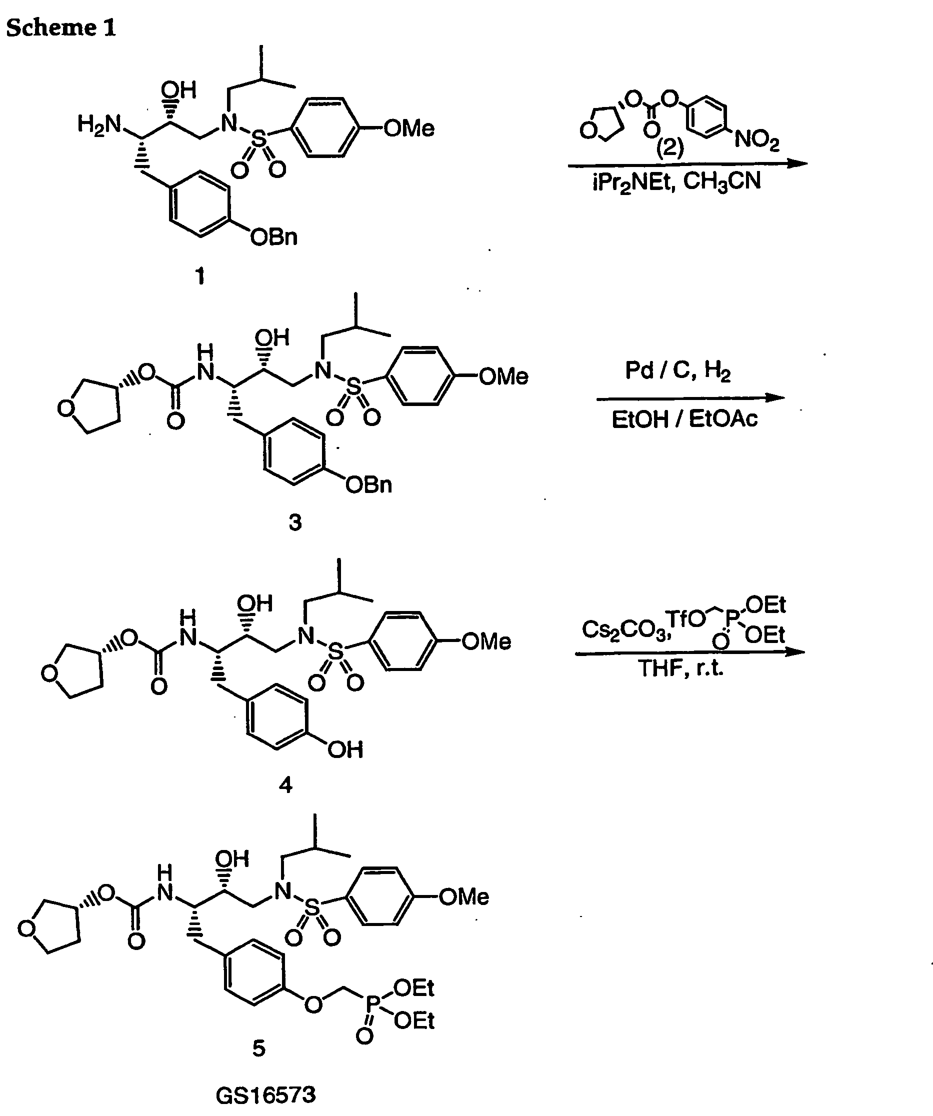

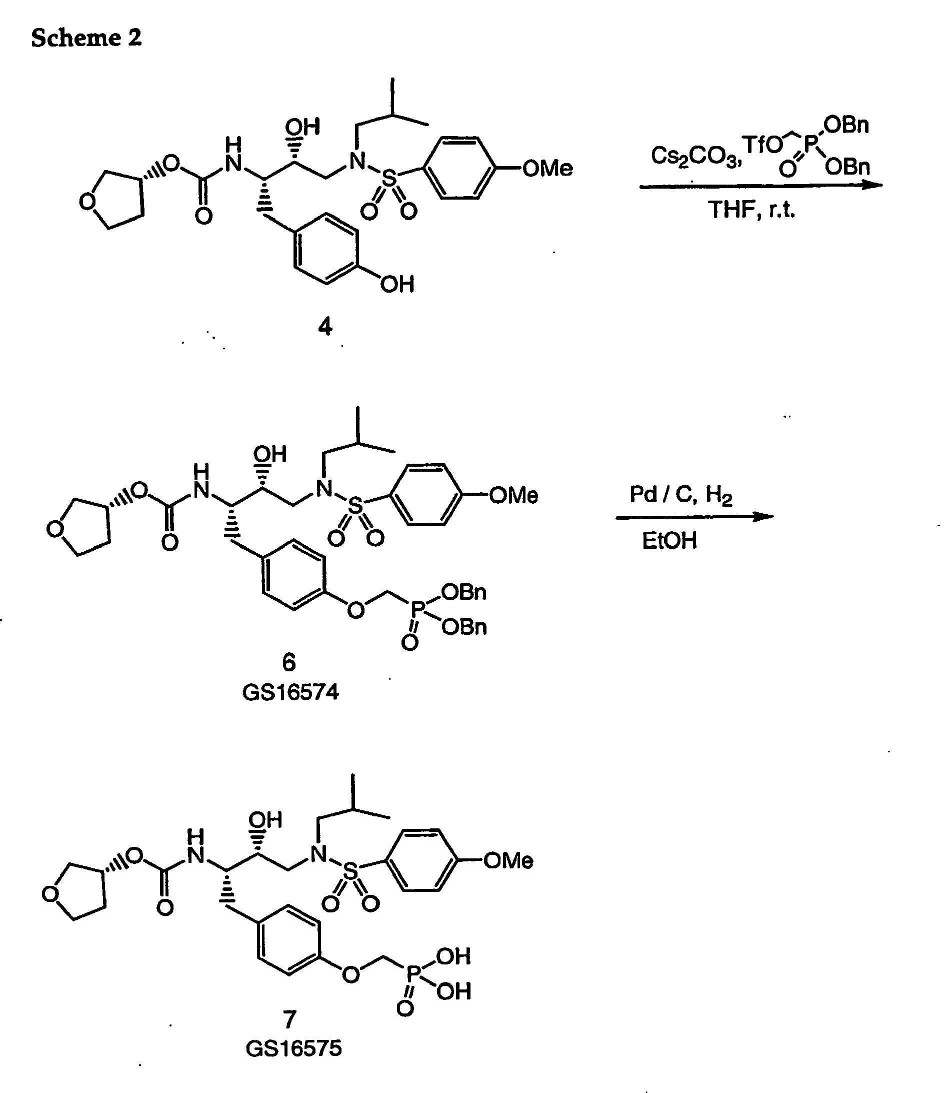

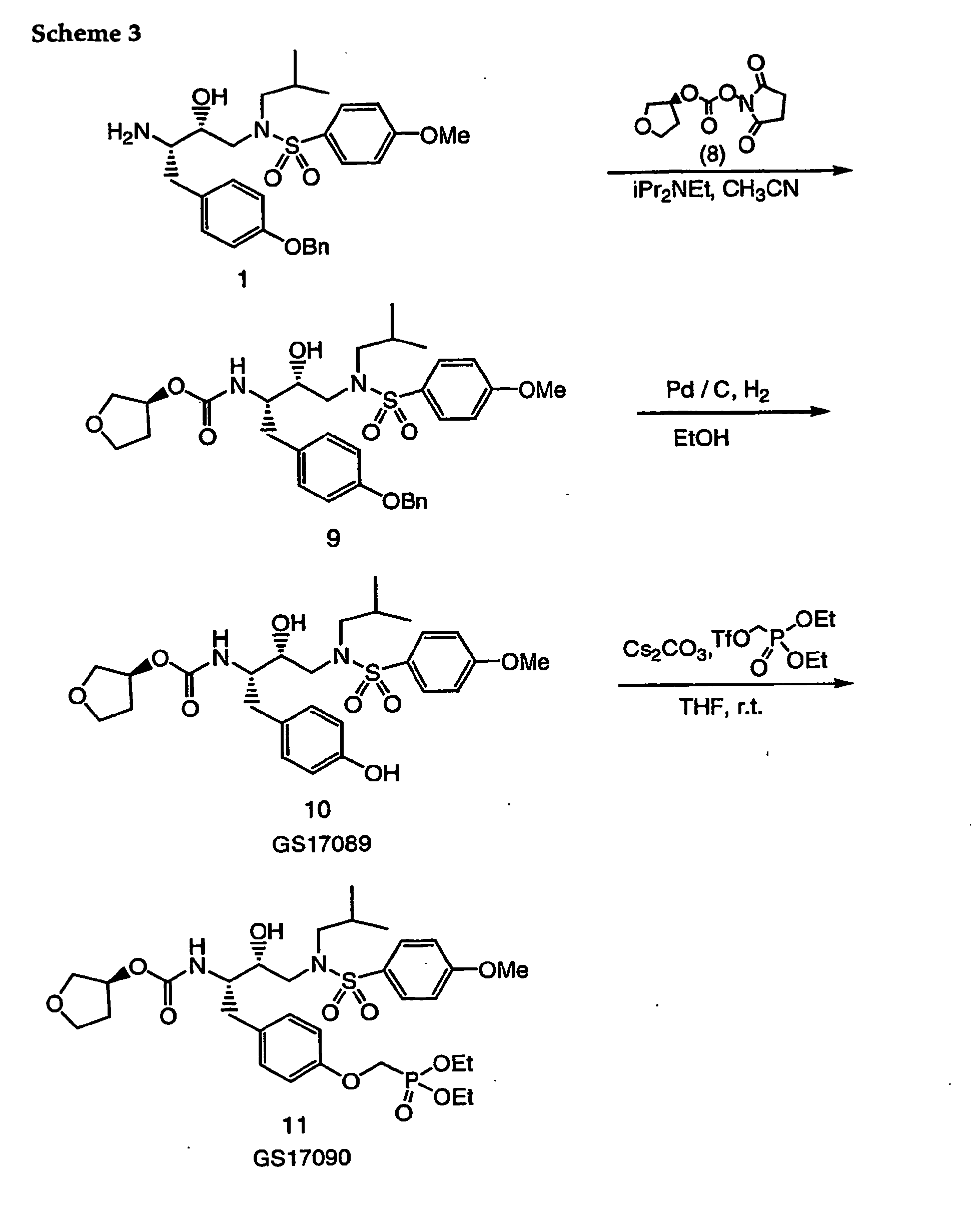

### Scheme Section I

Schemes 1-4 are described in the examples.

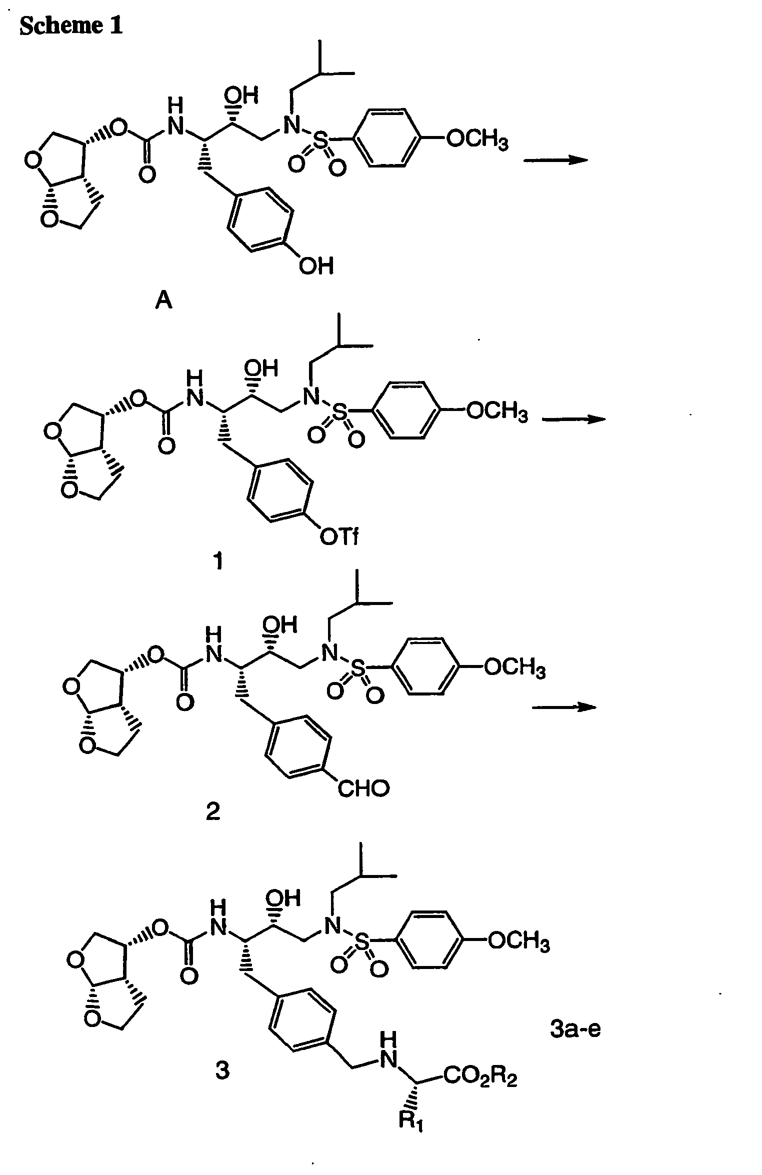

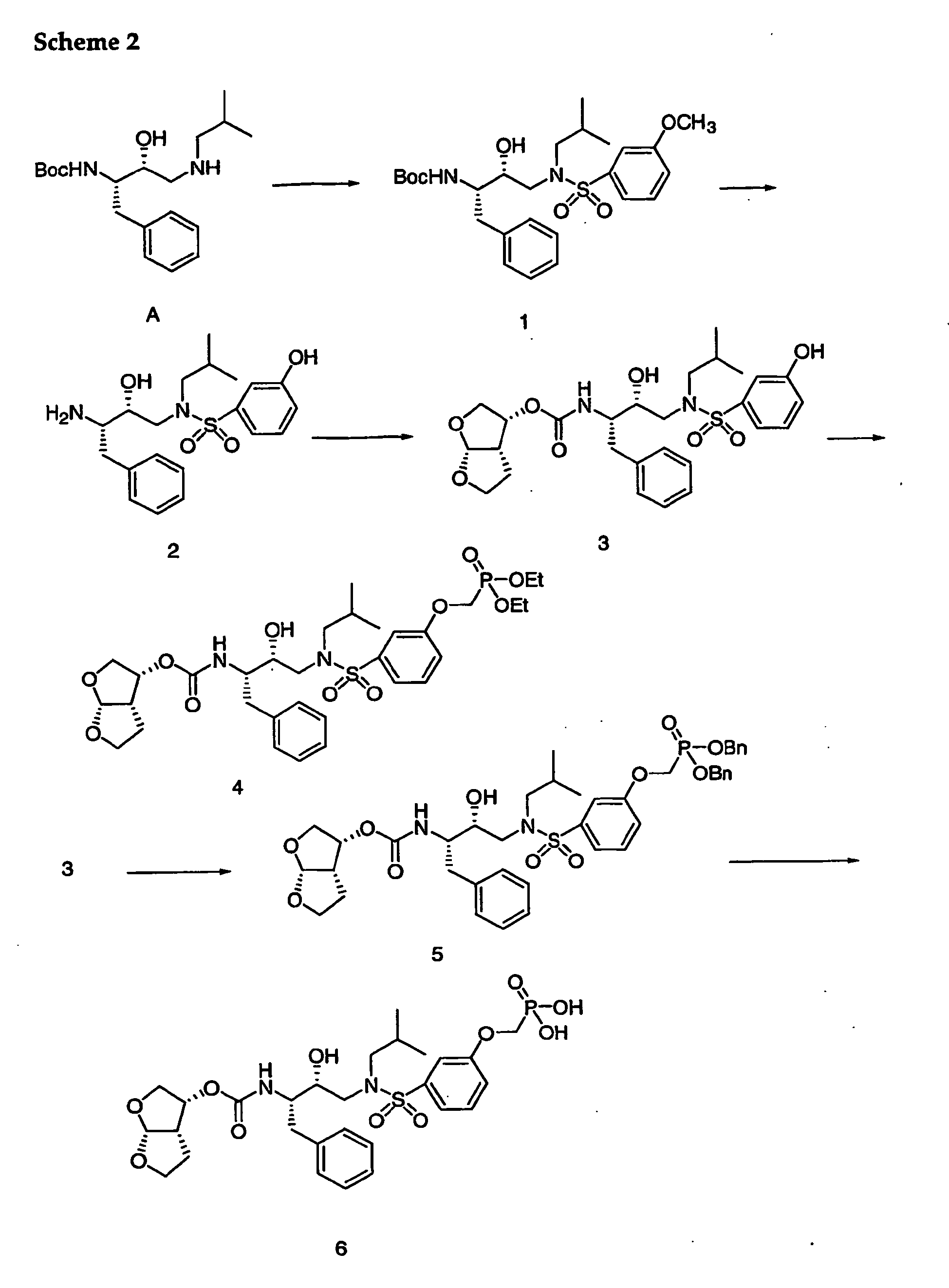

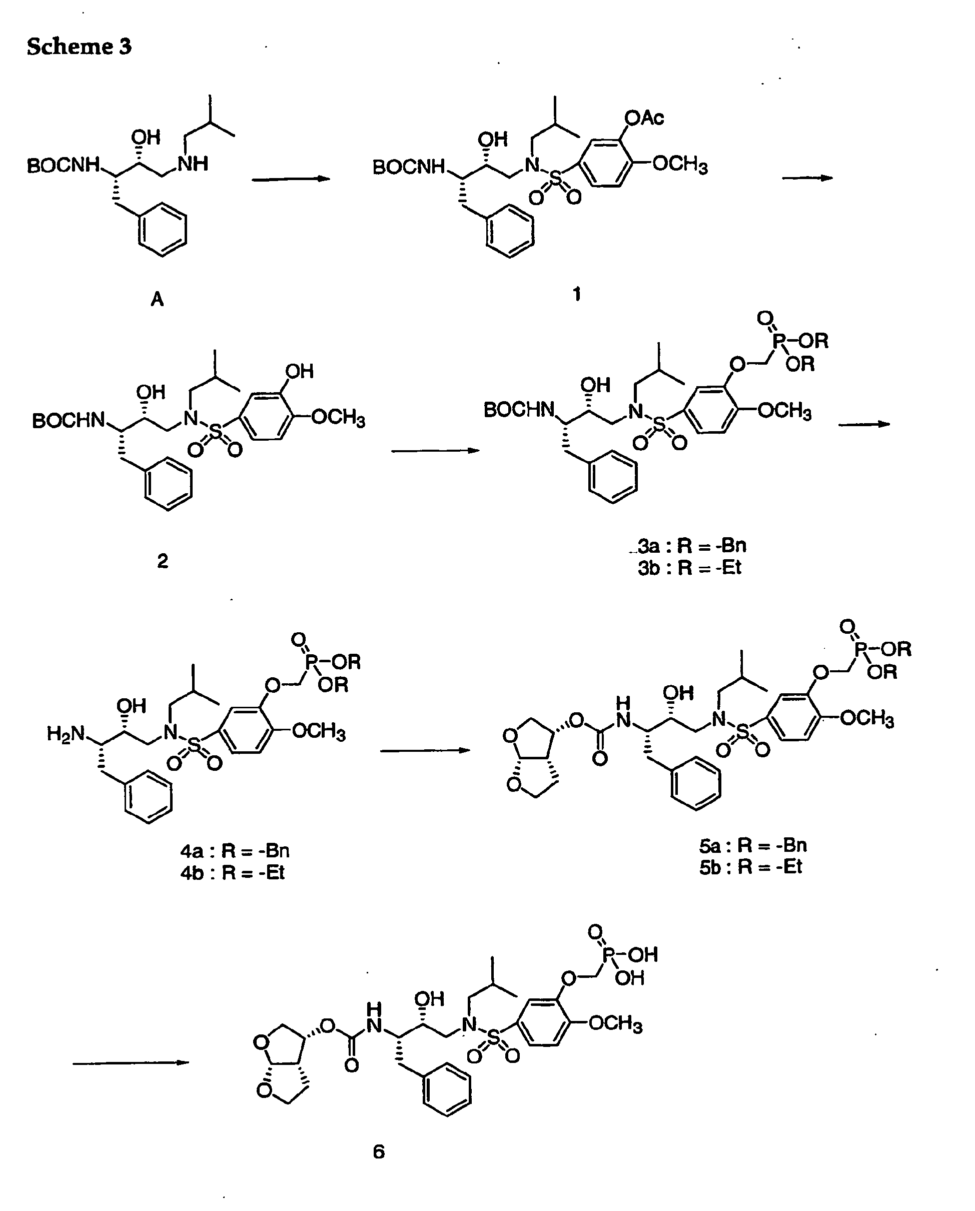

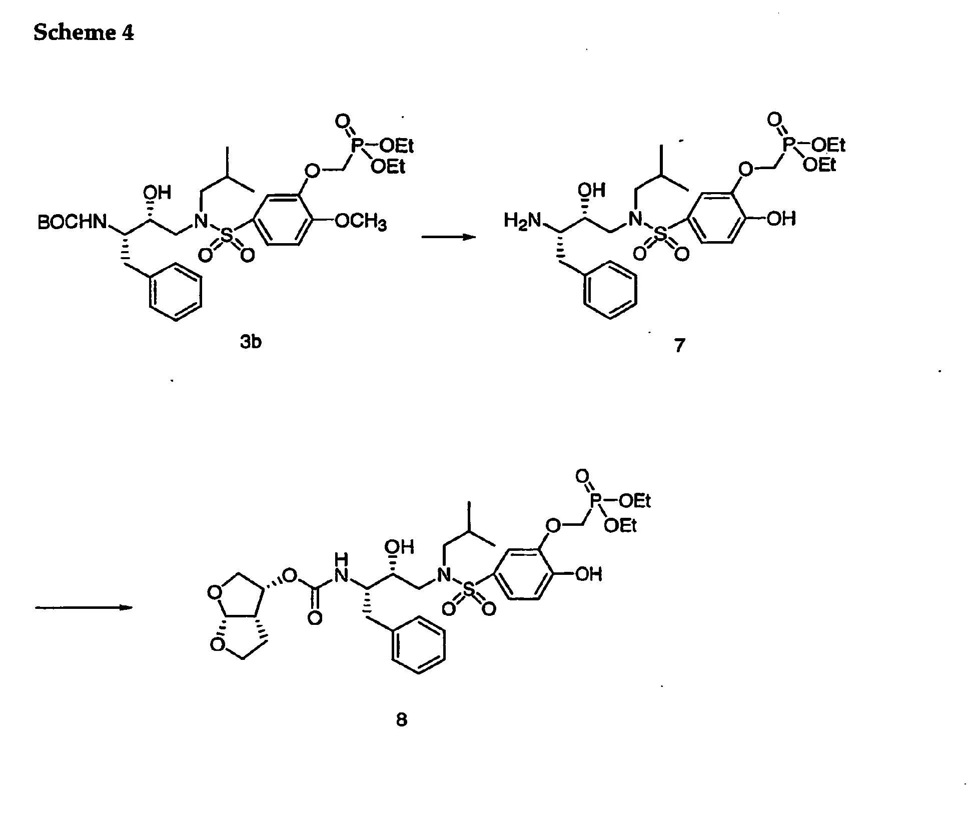

### Scheme Section K

Schemes 1-9 are described in the examples.

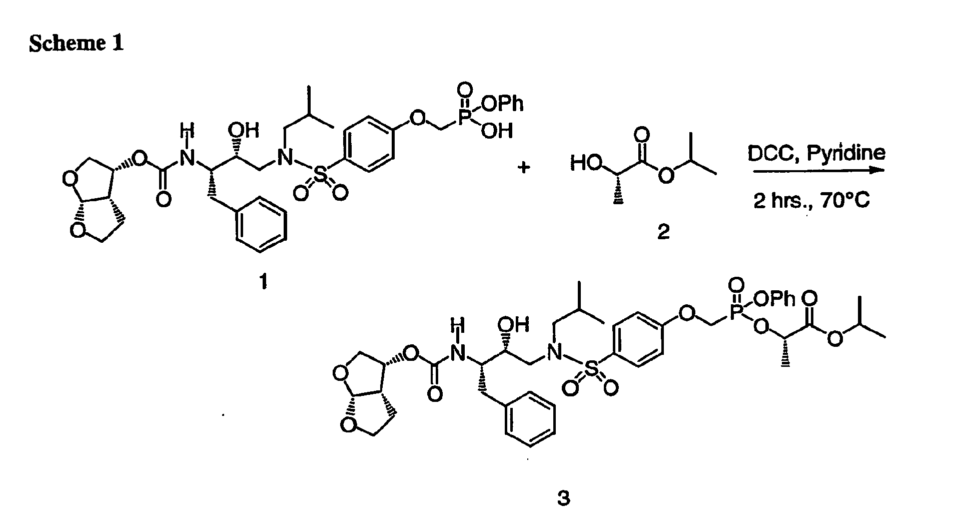

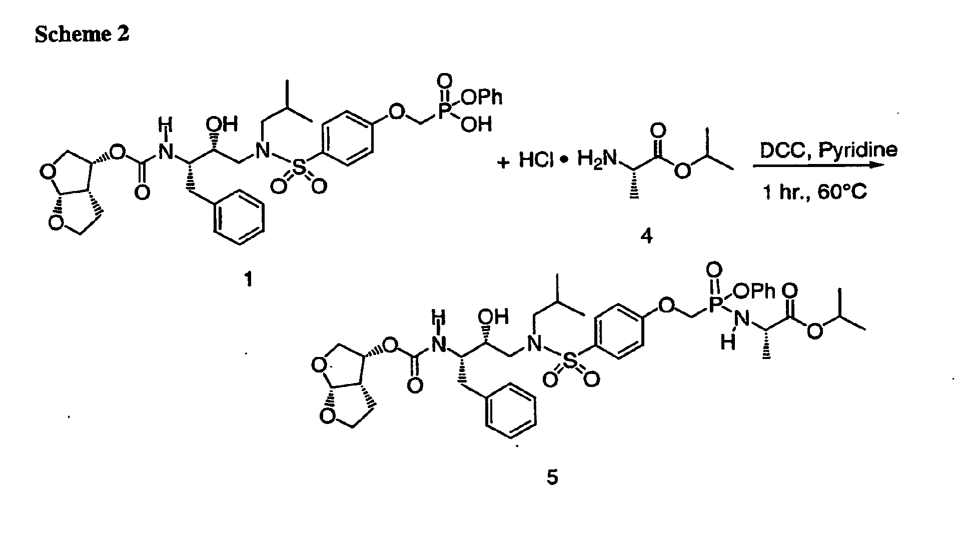

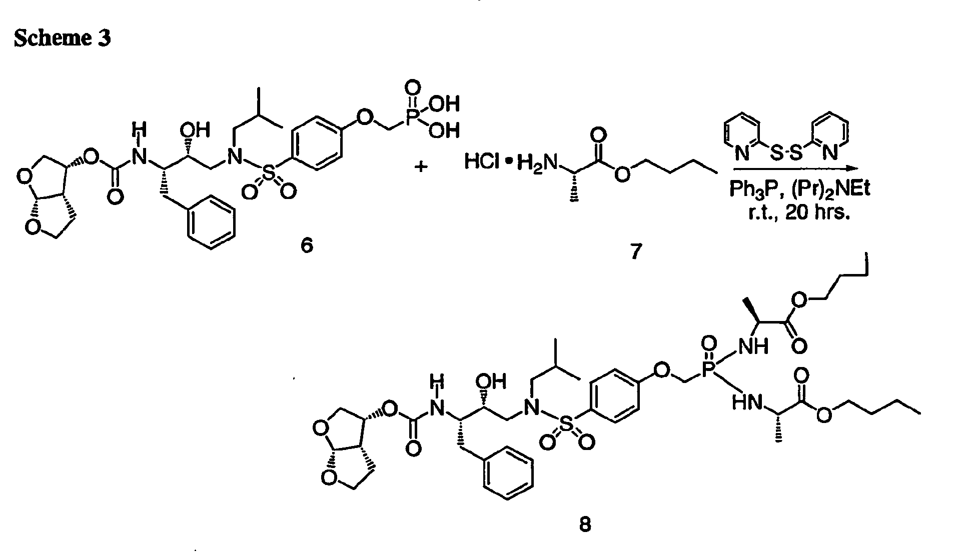

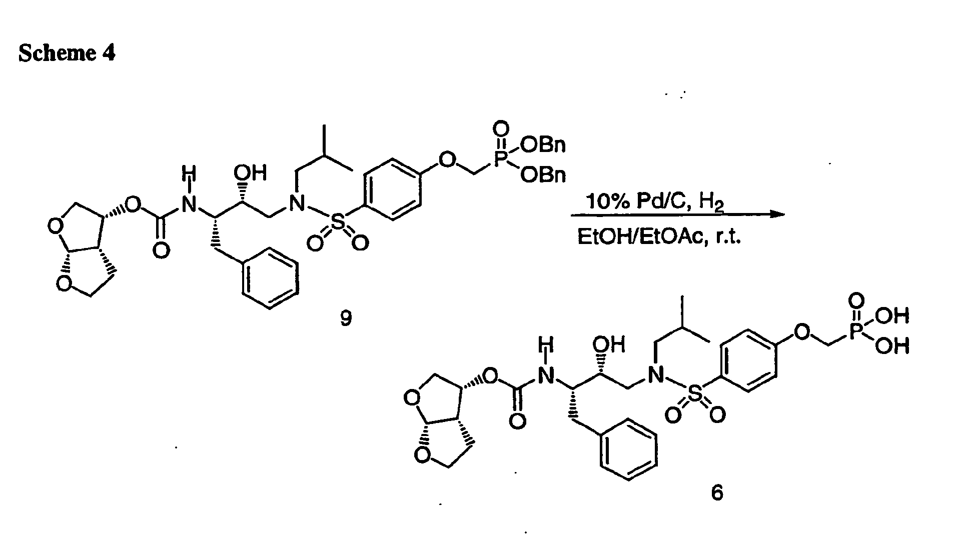

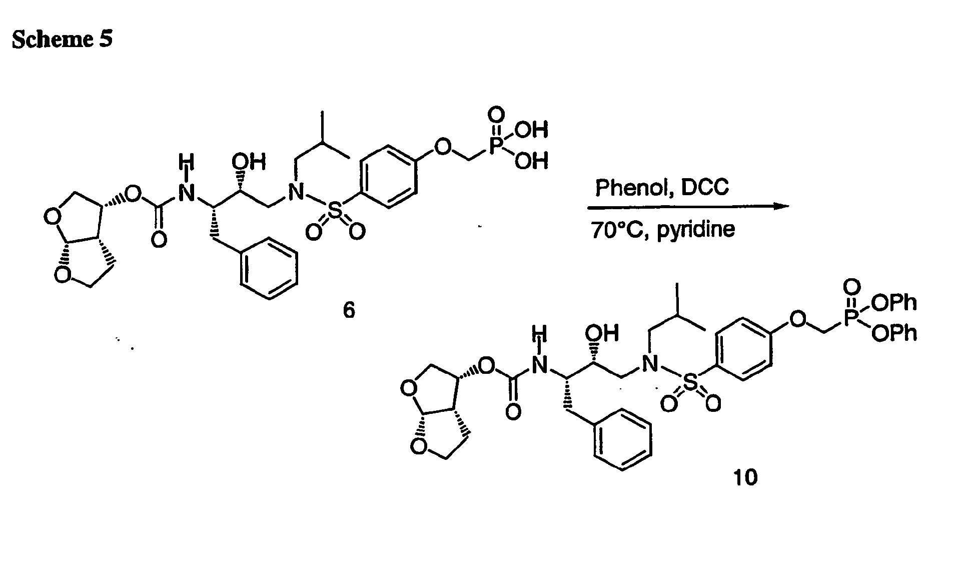

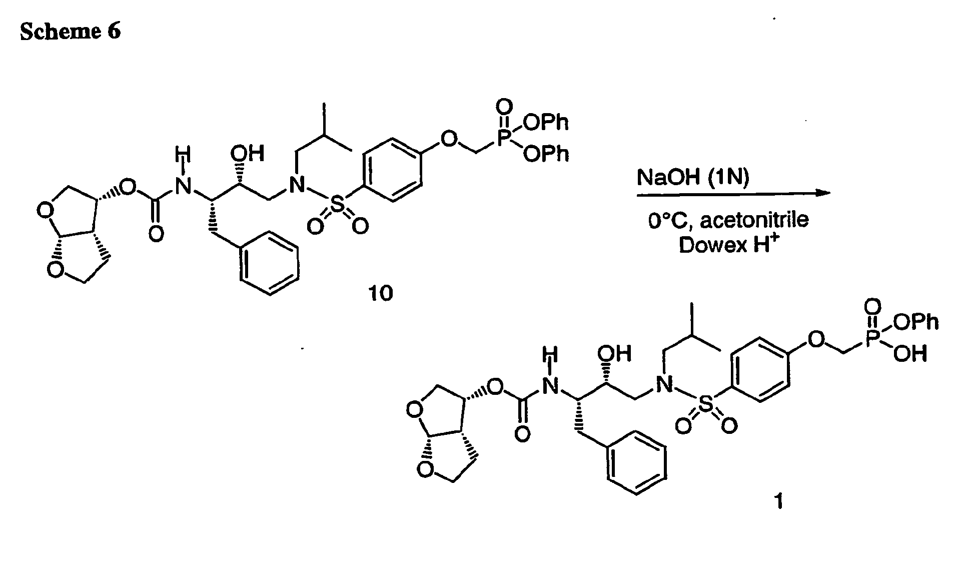

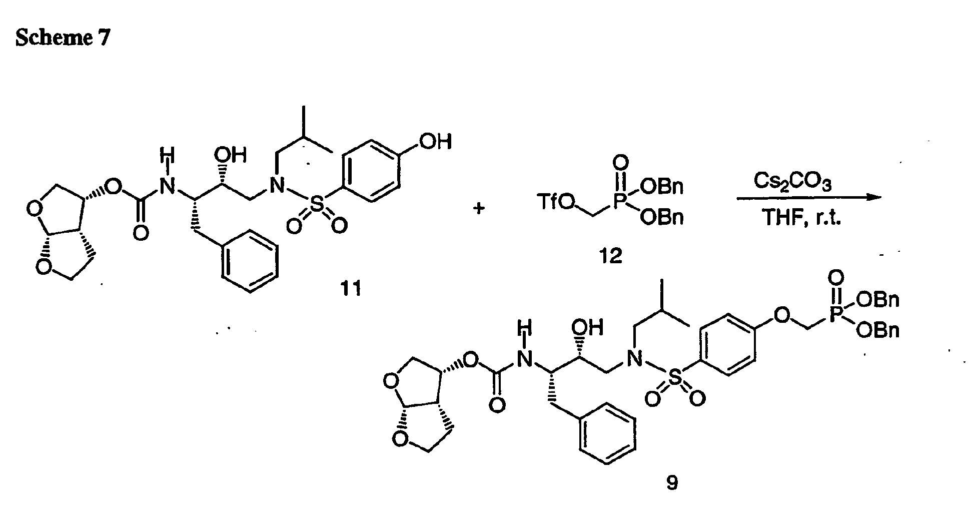

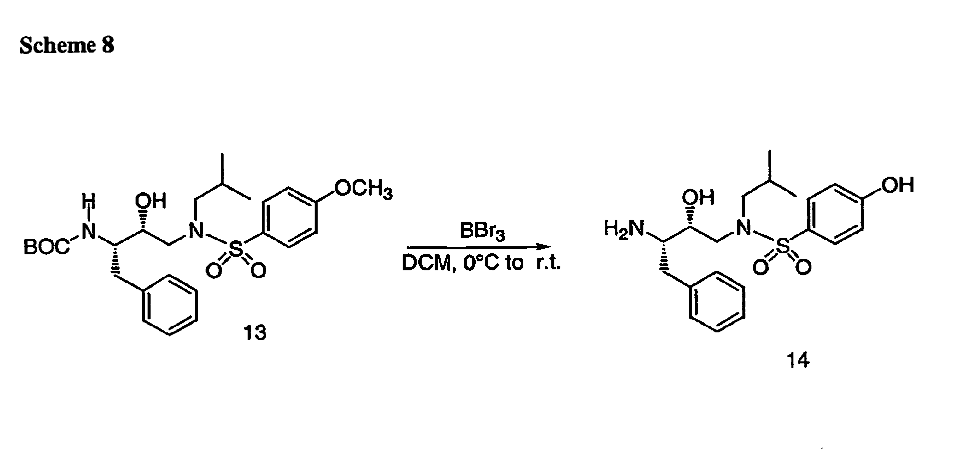

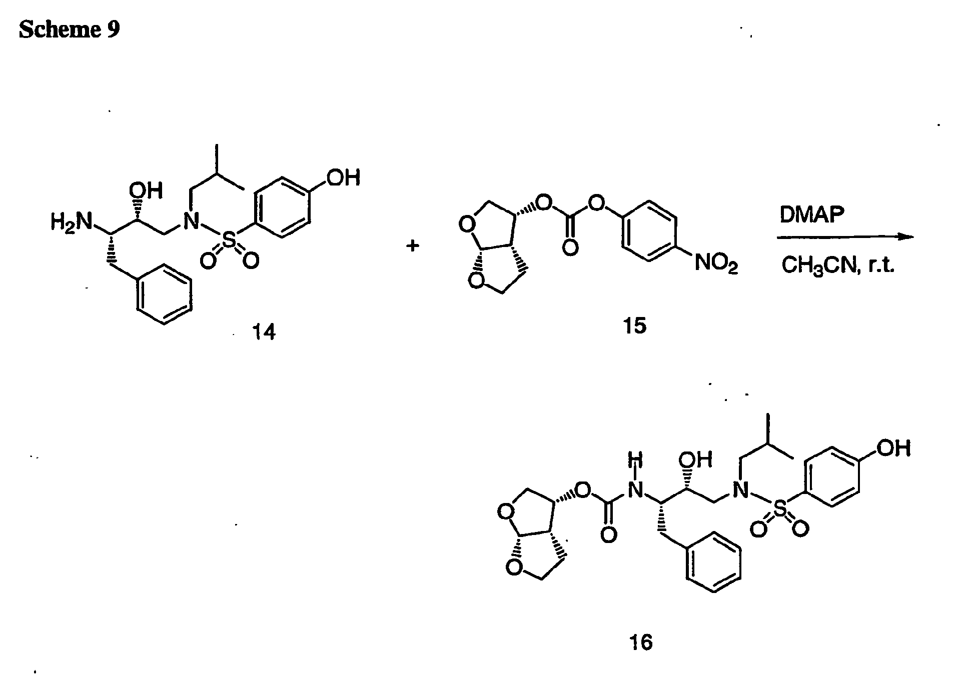

### Scheme Section L

Schemes 1-9 are described in the examples.

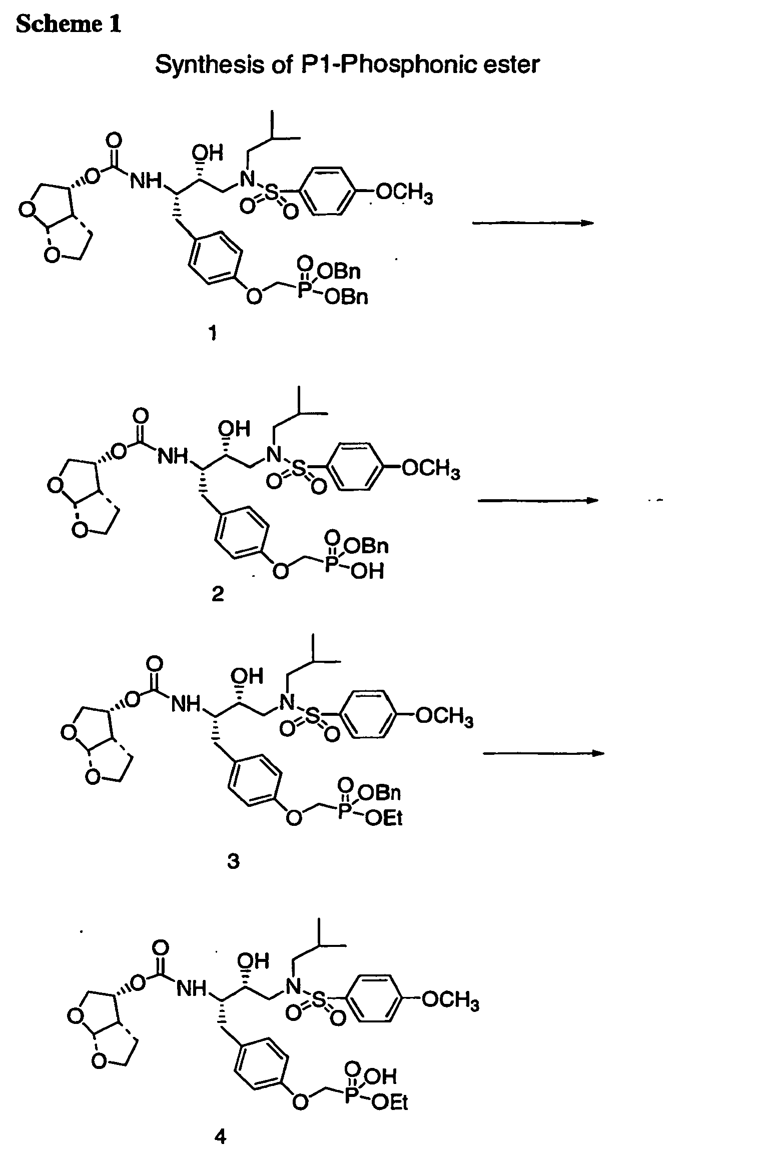

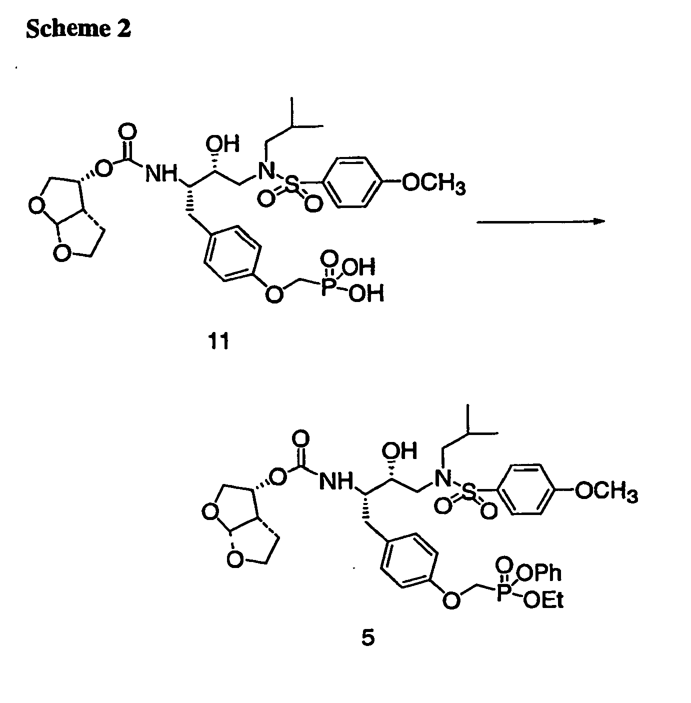

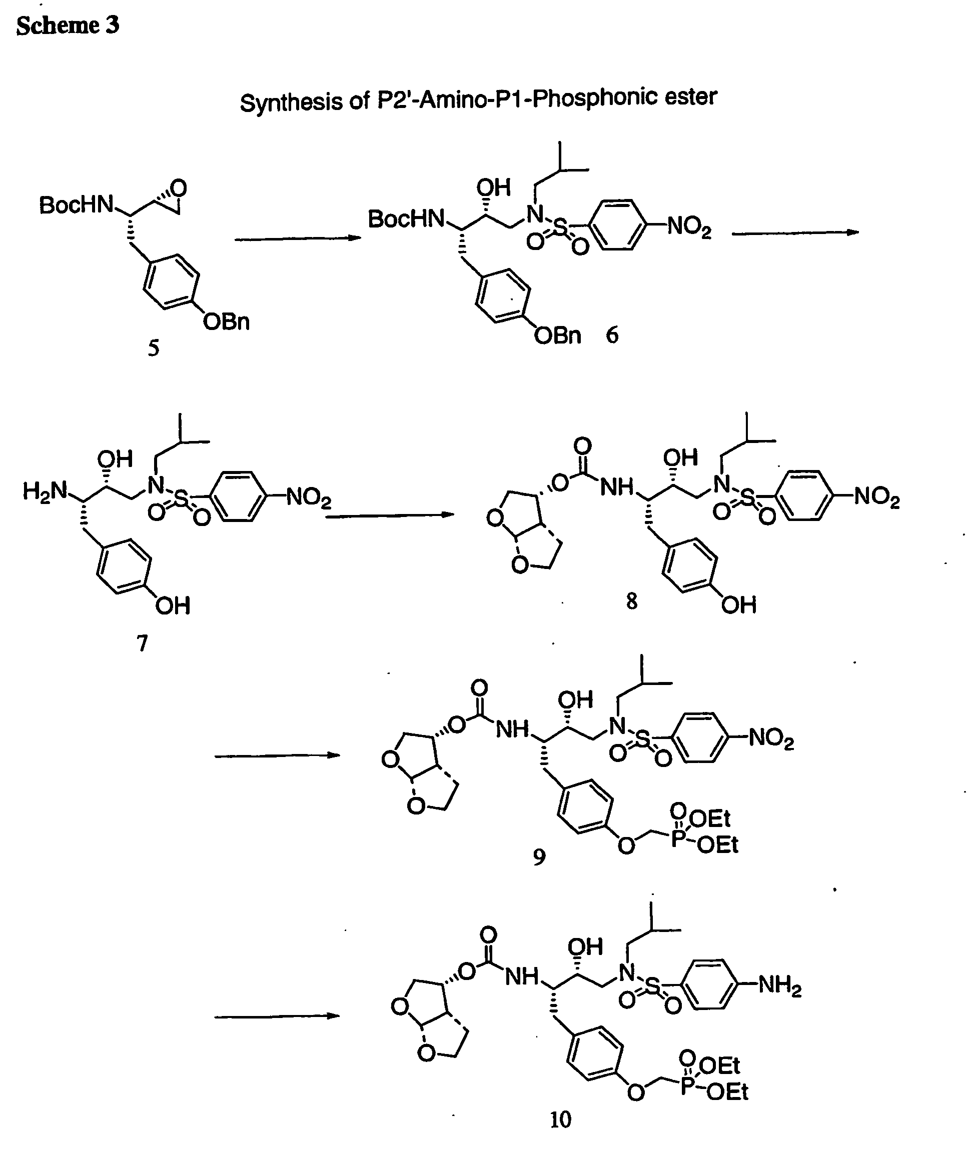

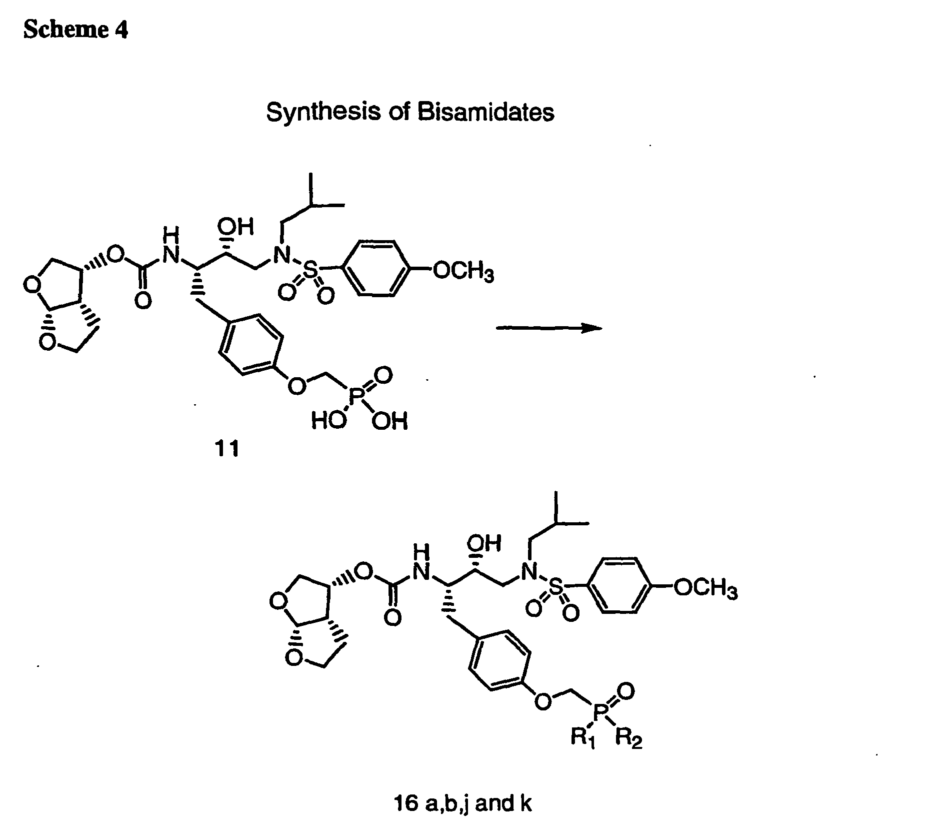

| **Compound** | **R₁** | **R₂** |
|---|---|---|
| 16a | Gly-Et | Gly-Et |
| 16b | Gly-Bu | Gly-Bu |
| 16j | Phe-Bu | Phe-Bu |
| 16k | NHEt | NHEt |

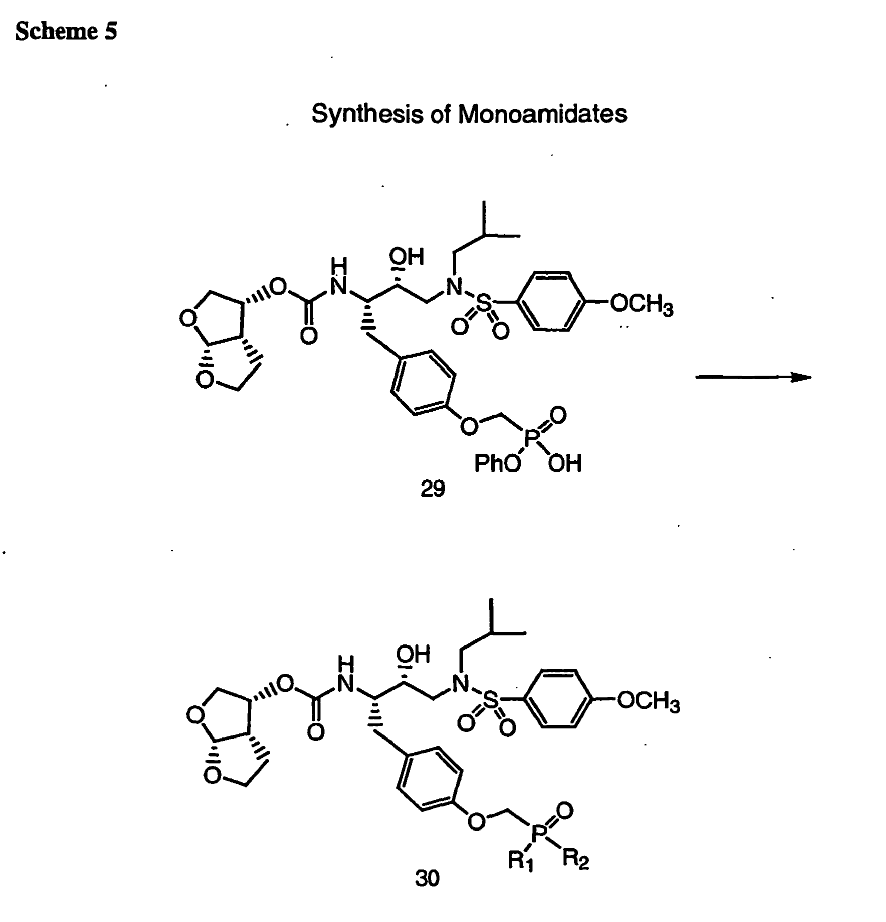

| **Compound** | **R₁** | **R₂** |
|---|---|---|
| 30a | OPh | Ala-Me |
| 30b | OPh | Ala-Et |
| 30c | OPh | (D)-Ala-iPr |
| 30d | OPh | Ala-Bu |
| 30e | OBn | Ala-Et |

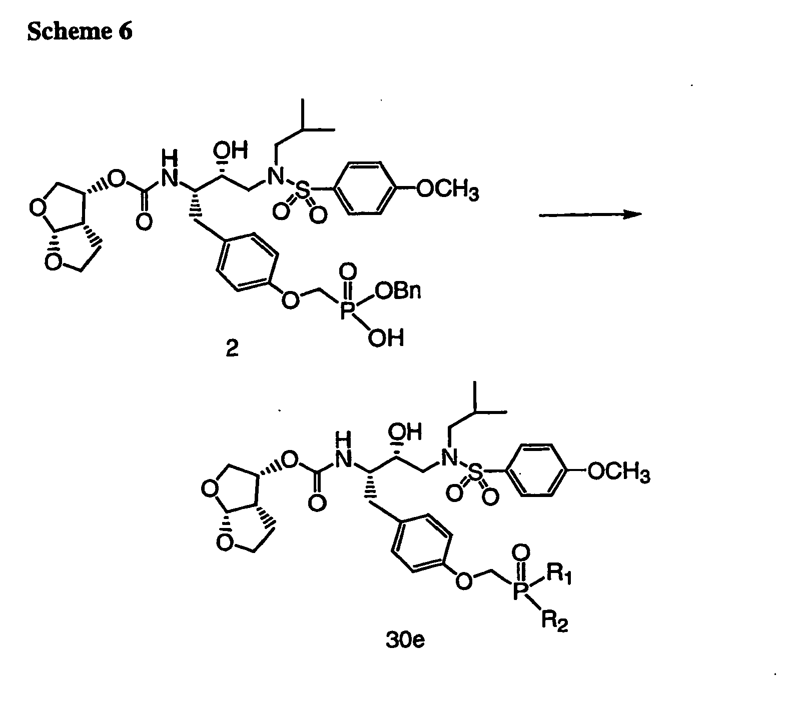

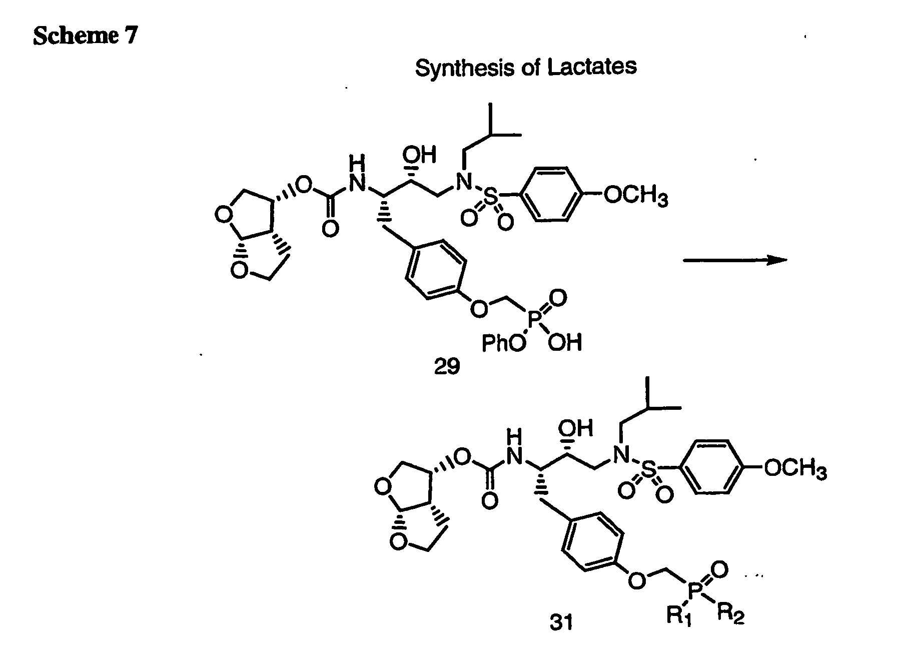

| **Compound** | **R₁** | **R₂** |
|---|---|---|
| 31a | OPh | Lac-iPr |
| 31b | OPh | Lac-Et |
| 31c | OPh | Lac-Bu |
| 31d | OPh | (R)-Lac-Me |
| 31e | OPh | (R)-Lac-Et |

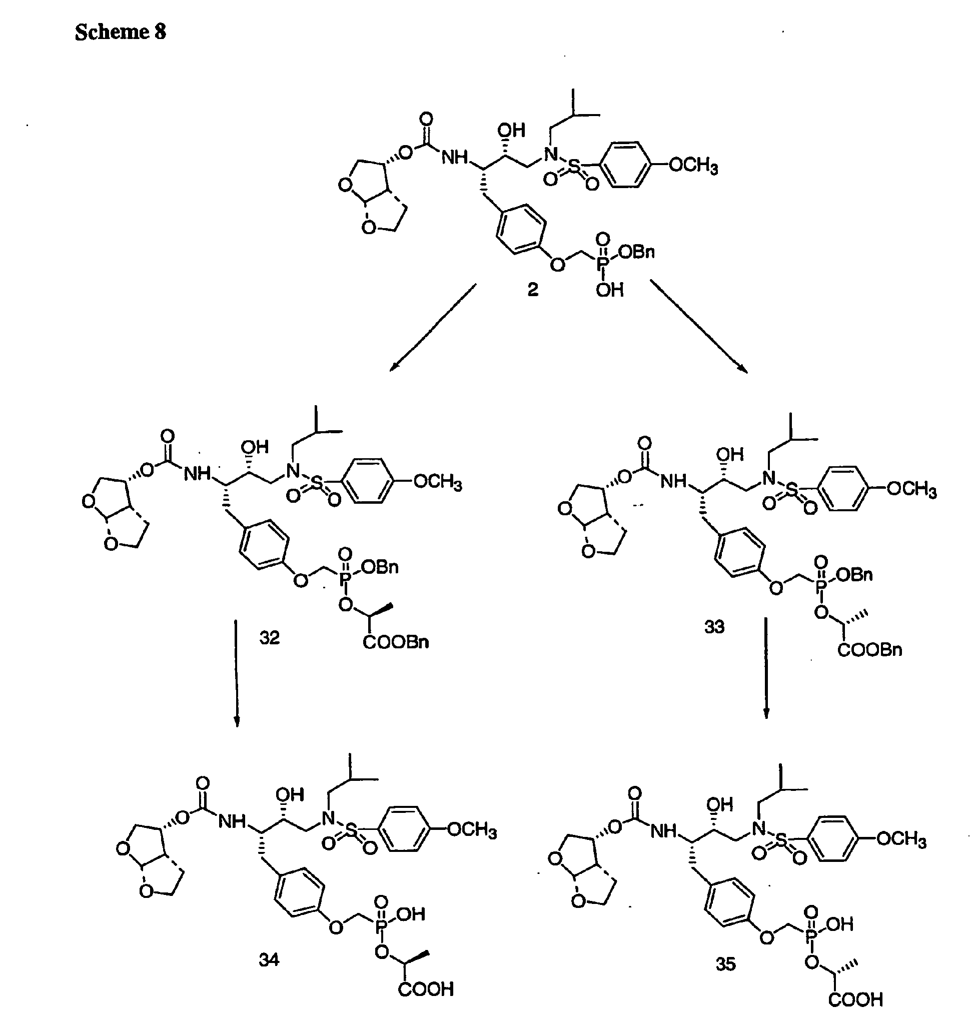

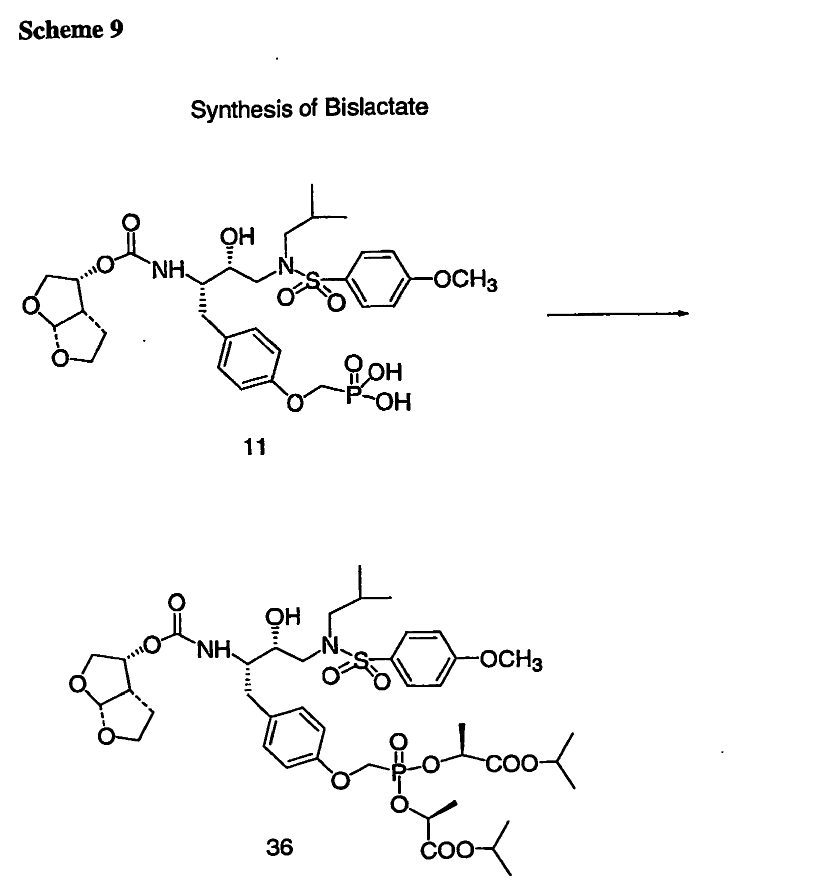

### Examples

The following Examples refer to the Schemes.

Some Examples have been performed multiple times. In repeated Examples, reaction conditions such as time, temperature, concentration and the like, and yields were within normal experimental ranges. In repeated Examples where significant modifications were made, these have been noted where the results varied significantly from those described. In Examples where different starting materials were used, these are noted. When the repeated Examples refer to a "corresponding" analog of a compound, such as a "corresponding ethyl ester", this intends that an otherwise present group, in this case typically a methyl ester, is taken to be the same group modified as indicated.

### Example Section A

### Example 1

Diazo ketone 1: To a solution of N-tert-Butoxycarbonyl-O-benzyl-L-tyrosine (11 g, 30 mmol, Fluka) in dry THF (55 mL) at -25-30°C (external bath temperature) was added isobutylchloroformate (3.9 mL, 30 mmol) followed by the slow addition of N.methylmorpholine (3.3 mL, 30 mmol). The mixture was stirred for 25 min, filtered while cold, and the filter cake was rinsed with cold (0°C) THF (50 mL). The filtrate was cooled to - 25°C and diazomethane (-50 mmol, generated from 15 g Diazald according to Aldrichimica Acta 1983, 16, 3) in ether (-150 mL) was poured into the mixed anhydride solution. The ' reaction was stirred for 15 min and was then placed in an icebath at 0°C, allowing the bath to warm to room temperature while stirring overnight for 15 h. The solvent was evaporated under reduced pressure and the residue was dissolved in EtOAc, washed with water, saturated NaHC0₃, saturated NaCl, dried (MgSO₄), filtered and evaporated to a pale yellow solid. The crude solid was slurried in hexane, filtered, and dried to afford the diazo ketone (10.9 g, 92%) which was used directly in the next step.

### Example 2

Chloroketone 2: To a suspension of diazoketone 1 (10.8 g, 27 mmol) in ether (600 mL) at 0°C was added 4M HCl in dioxane (7.5 mL, 30 mmol). The solution was removed from the cooling bath, and allowed to warm to room temperature at which time the reaction was stirred 1 h. The reaction solvent was evaporated under reduced pressure to give a solid residue that was dissolved in ether and passed through a short column of silica gel. The solvent was evaporated to afford the chloroketone (10.7 g, 97%) as a solid.

### Example 3

Chloroalcohol 3: To a solution of chloroketone 2 (10.6 g, 26 mmol) in THF (90 mL) was added water (10 mL) and the solution was cooled to 3-4°C (internal temperature). A solution of NaBH₄ (1.5 g, 39 mmol) in water (5 mL) was added dropwise over a period of 10 min. The mixture was stirred for 1h at 0°C and saturated KHSO₄ was slowly added until the pH<4 followed by saturated NaCl. The organic phase was washed with saturated NaCl, dried (MgSO₄) filtered and evaporated under reduced pressure. The crude product consisted of a 70:30 mixture of diastereomers by HPLC analysis (mobile phase, 77:25-CH₃CN:H₂O; flow rate: 1 mL/min; detection: 254 nm; sample volume: 20 µL; column: 5µ C18, 4.6X250 mm, Varian; retention times: major diastereomer 3, 5.4 min, minor diastereomer 4, 6.1 min). The residue was recrystallized from EtOAc/hexane twice to afford the chloro alcohol 3 (4.86g, >99% diastereomeric purity by HPLC analysis) as a white solid.

### Example 4

Epoxide 5: A solution of chloroalcohol 3 (4.32 g, 10.6 mmol) in EtOH (250 mL) and THF (100 mL) was treated with K₂CO₃ (4.4g, 325 mesh, 31.9 mmol) and the mixture was stirred for at room temperature for 20h. The reaction mixture was filtered and was evaporated under reduced pressure. The residue was partitioned between EtOAc and water and the organic phase was washed with saturated NaCl, dried (MgSO₄), filtered, and evaporated under reduced pressure. The crude product was chromatographed on silica gel to afford the epoxide (3.68 g, 94%) as a white solid.

### Example 5

Sulfonamide 6: To a suspension of epoxide 5 (2.08 g, 5.6 mmol) in 2-propanol (20 mL) was added isobutylamine (10.7 mL, 108 mmol) and the solution was refluxed for 30 min. The solution was evaporated under reduced pressure and the crude solid was dissolved in CH₂C1₂ (20 mL) and cooled to 0°C. N,N'-diisopropylethylamine (1.96 mL, 11.3 mmol) was added followed by the addition of 4-methoxybenzenesulfonyl chloride (1.45 g, 7 mmol) in CH₂Cl₂ (5 mL) and the solution was stirred for 40 min at 0°C, warmed to room temperature and evaporated under reduced pressure. The residue was partitioned between EtOAc and saturated NaHCO₃. The organic phase was washed with saturated NaCl, dried (MgSO₄), filtered and evaporated under reduced pressure. The crude product was recrystallized from EtOAc/hexane to give the sulfonamide (2.79 g, 81%) as a small white needles: mp 122-124°C (uncorrected).

### Example 6

Carbamate 7: A solution of sulfonamide 6 (500 mg, 0.82 mmol) in CH₂Cl₂ (5 mL) at 0°C was treated with trifluoroacetic acid (5 mL). The solution was stirred at 0°C for 30 min and was removed from the cold bath stirring for an additional 30 min. Volatiles were evaporated under reduced pressure and the residue was partitioned between CH₂Cl₂ and saturated NaHCO₃. The aqueous phase was extracted twice with CH₂Cl₂ and the combined organic extracts were washed with saturated NaCl, dried (MgSO₄), filtered, and evaporated under reduced pressure. The residue was dissolved in CH₃CN (5 mL) and was treated with (3R, 3aR, 6aS)-hexahydrofuro[2, 3-*b*]furan-2-yl 4-nitrophenyl carbonate (263 mg, 0.89 mmol, prepared according to Ghosh et al., J. Med. Chem. 1996, 39, 3278.) and N,N-dimethylaminopyridine (197 mg, 1.62 mmol). After stirring for 1.5h at room temperature, the reaction solvent was evaporated under reduced pressure and the residue was partitioned between EtOAc and 5% citric acid. The organic phase was washed twice with 1% K₂CO₃, and then was washed with saturated NaCl, dried (MgSO₄), filtered, and evaporated under reduced pressure. The crude product was purified by chromatography on silica gel (1/1 - EtOAc/hexane) affording the carbamate (454 mg, 83%) as a solid: mp 128-129°C (MeOH, uncorrected).

### Example 7

Phenol 8: A solution of carbamate 7 (1.15 g, 1.7 mmol) in EtOH (50 mL) and EtOAc (20 mL) was treated with 10% Pd/C (115 mg) and was stirred under H₂ atmosphere (balloon) for 18h. The reaction solution was purged with N₂, filtered through a 0.45 µM filter and was evaporated under reduced pressure to afford the phenol as a solid that contained residual solvent: mp 131-134°C (EtOAc/hexane, uncorrected).

### Example 8

Dibenzylphosphonate 10: To a solution of dibenzylhydroxymethyl phosphonate (527 mg, 1.8 mmol) in CH₂Cl₂ (5 mL) was treated with 2,6-lutidine (300 µL, 2.6 mmol) and the reaction flask was cooled to -50°C (external temperature). Trifluoromethanesulfonic anhydride (360 µL, 2.1 mmol) was added and the reaction mixture was stirred for 15 min and then the cooling bath was allowed to warm to 0°C over 45 min. The reaction mixture was partitioned between ether and ice-cold water. The organic phase was washed with cold 1M H₃PO₄, saturated NaCl, dried (MgSO₄), filtered and evaporated under reduced pressure to afford triflate 9 (697 mg, 91 %) as an oil which was used directly without any further purification. To a solution of phenol 8 (775 mg, 1.3 mmol) in THF (5 mL) was added CS₂CO₃ (423 mg, 1.3 mmol) and triflate 9 (710 mg, 1.7 mmol) in THF (2 mL). After stirring the reaction mixture for 30 min at room temperature additional C_{S2}CO₃ (423 mg, 1.3 mmol) and triflate (178 mg, 0.33 mmol) were added and the mixture was stirred for 3.5h. The reaction mixture was evaporated under reduced pressure and the residue was partitioned between EtOAc and saturated NaCl. The organic phase was dried (MgSO₄), filtered and evaporated under reduced pressure. The crude product was chromatographed on silica gel eluting (5% 2-propanol/CH₂Cl₂) to give the dibenzylphosphonate as an oil that solidified upon standing. The solid was dissolved in EtOAc, ether was added, and the solid was precipitated at room temperature overnight. After cooling to 0°C, the solid was filtered and washed with cold ether to afford the dibenzylphosphonate (836 mg, 76%) as a white solid: ¹H NMR (CDCl₃) δ 7.66 (d, 2H), 7.31 (s, 10H), 7.08 (d, 2H), 6.94 (d, 2H), 6.76 (d, 2H), 5.59 (d, 1H), 5.15-4.89 (m, 6H), 4.15 (d, 2H), 3.94-3.62 (m, 10H), 3.13-2.69 (m, 7H), 1.78 (m, 1H), 1.70-1.44. (m, 2H), 0.89-0.82 (2d, 6H); ³¹P NMR (CDCl₃) δ 18.7; MS (ESI) 853 (M+H).

### Example 9

Phosphonic acid 11: A solution of dibenzylphosphonate 10 (0.81 g) was dissolved in EtOH/ . EtOAc (30mL/10 mL), treated with 10% Pd/C (80 mg) and was stirred under H₂ atmosphere (balloon) for 1.5h. The reaction was purged with N₂, and the catalyst was removed by filtration through celite. The filtrate was evaporated under reduced pressure and the residue was dissolved in MeOH and filtered with a 0.45 µM filter. After evaporation of the filtrate, the residue was triturated with ether and the solid was collected by filtration to afford the phosphonic acid (634 mg, 99%) as a white solid: ¹H NMR (CDCl₃) δ 7.77 (d, 2H), 7.19 (d, 2H), 7.09 (d, 2H), 6.92 (d, 2H), 5.60 (d, 1H), 4.95 (m, 1H), 4.17 (d, 2H), 3.94 (m, 1H), 3.89 (s, 3H), 3.85-3.68 (m, 5H), 3.42 (dd, 1H), 3.16-3.06 (m, 2H), 2.96-2.84 (m, 3H), 2.50 (m, 1H), 2.02 (m, 1H), 1.58 (m, 1H), 1.40 (dd, 1H), 0.94 (d, 3H), 0.89 (d, 3H); ³¹P NMR (CDCl₃) δ 16.2; MS (ESI) 671 (M-H).

### Example 10

Diethylphosphonate 13: Triflate 12 was prepared from diethyl hydroxymethylphosphonate (2g, 11.9 mmol), 2,6-lutidine (2.1 mL, 17.9 mmol), and trifluoromethanesulfonic anhydride (2.5 mL, 14.9 mmol) as described for compound 9. To a solution of phenol 8 (60 mg, 0.10 mmol) in THF (2 mL) was added Cs₂CO₃ (65mg, 0.20 mmol) and triflate 12 (45 mg, 0.15 mmol) in THF (0.25 mL). The mixture was stirred at room temperature for 2h and additional triflate (0.15 mmol) in THF (0.25 mL) was added. After 2h the reaction mixture was partitioned between EtOAc and saturated NaCl. The organic phase was dried (MgSO₄), filtered, and evaporated under reduced pressure. The crude product was chromatographed on silica gel (EtOAc) to give a residue that was purified by chromatography on silica gel (5% 2-propanol /CH₂Cl₂) to afford the diethylphosphonate as a foam: ¹H NMR (CDCl₃) δ 7.66 (d, 2H), 7.10 (d, 2H), 6.94 (d, 2H), 6.82 (d, 2H), 5.60 (d, 1H), 4.97 (d, 2H), 4.23-4.13 (m, 6H), 3.93-3.62 (m, 10H), 3.12-2.68 (m, 7H), 1.84-1.44 (m, 3H), 1.31 (t, 6H), 0.88-0.82 (2d, 6H); ³¹P NMR (CDCl₃) δ 17.7; MS (ESI) 729 (M+H).

### Example 11

Diphenylphosphonate 14: To a solution of 11 (100mg, 0.15 mmol) and phenol (141 mg, 1.5 mmol) in pyridine (1.5 mL) was added N, N-diisopropylcarbodiimide (50 µL, 0.38 mmol). The solution was stirred for 31h at room temperature and for 20h at 50°C. The solvent was evaporated under reduced pressure and the residue was purified by chromatography on silica gel eluting (EtOAc) to provide diphenylphosphonate 14 (16 mg) as a foam: ³¹P NMR (CDCl₃) δ 10.9; MS (ESI) 847 (M+Na).

### Example 12

Bis-Poc-phosphonate 15: To a solution of 11 (50 mg, 0.74 mmol) and isopropylchloromethyl carbonate (29 mg, 0.19 mmol) in DMF (0.5 mL) was added triethylamine (26 µL, 0.19 mmol) and the solution was heated at 70°C (bath temperature) for 4.5h. The reaction was concentrated under reduced pressure and the residue was purified by preparative layer chromatography (2% 2-propanol/ CH₂Cl₂) to afford 15 (7 mg): ¹H NMR (CDCl₃) δ 7.71 (d, 2H), 7.15 (d, 2H); 7.01 (d, 2H), 6.93 (d, 2H), 5.80-5.71 (m, 4H), 5.67 (d, 1H), 5.07-4.87 (m, 4H), 4.35 (d, 2H), 4.04-3.68 (m, 10H), 3.13 (dd, 1H), 3.04-2.90 (m, 5H), 2.79 (dd, 1H), 1.88-1.50 (m, 3H+H₂O peak), 1.30 (m, 12H), 0:93 (d, 3H), 0.88 (d, 3H); ³¹P NMR (CDCl₃) δ 19.6.

### Example 13

Synthesis of Bisamidates 16a-j. Representative Procedure, Bisamidate 16f: A solution of phosphonic acid 11 (100 mg, 0.15 mmol) and (S)-2-aminobutyric acid butyl ester hydrochloride (116 mg, 0.59 mmol) was dissolved in pyridine (5 mL) and the solvent was distilled under reduced pressure at 40-60°C. The residue was treated with a solution of Ph₃P (117 mg, 0.45 mmol) and 2,2'-dipyridyl disulfide (98 mg, 0.45 mmol) in pyridine (1 mL) stirring for 20h at room temperature. The solvent was evaporated under reduced pressure and the residue was chromatographed on silica gel (1% to 5% 2-propanol/CH₂Cl₂). The purified product was suspended in ether and was evaporated under reduced pressure to afford bisamidate 16f (106 mg, 75%) as a white solid: ¹H NMR (CDCl₃) δ 7.72 (d, 2H), 7.15 (d, 2H), 7.01 (d, 2H), 6.87 (d, 2H), 5.67 (d, 1H), 5.05 (m, 1H), 4.96 (d, 1H), 4.19-3.71 (m overlapping s, 18H,), 3.42 (t, 1H), 3.30 (t, 1H), 3.20 (dd, 1H), 3.20-2.97 (m, 4H), 2.80 (dd, 2H), 1.87-1.54 (m, 19H), 1.42-1.35 (4H), 0.97-0.88 (m, 18H); ³¹P NMR (CDCl₃) δ 20.3; MS (ESI) 955 (M+H).

| **Compound** | **R₁** | **R₂** | **Amino Acid** |
|---|---|---|---|
| 16a | H | Et | Gly |
| 16b | H | Bu | Gly |
| 16c | Me | Et | Ala |
| 16d | Me | Bu | Ala |
| 16e | Et | Et | Aba¹ |
| 16f | Et | Bu | Aba¹ |
| 16g | iBu | Et | Leu |
| 16h | iBu | Bu | Leu |
| 16i | Bn | Et | Phe |
| 16j | Bn | Bu | Phe |

| | | | |
|---|---|---|---|
| ¹ Aba, 2-aminobutyric acid | | | |

### Example 14

Diazo ketone 17: To a solution of N-tert-Butoxycarbonyl-p-bromo-L-phenylalanine (9.9 g, 28.8 mmol, Synthetech) in dry THF (55 mL) at -25-30°C (external bath temperature) was added isobutylchloroformate (3.74 mL, 28.8 mmol) followed by the slow addition of N-methylmorpholine (3.16 mL, 28.8 mmol). The mixture was stirred for 25 min, filtered while cold, and the filter cake was rinsed with cold (0°C) THF (50 mL). The filtrate was cooled to - 25°C and diazomethane (~50 mmol, generated from 15 g diazald according to Aldrichimica Acta 1983, 16, 3) in ether (~150 mL) was poured into the mixed anhydride solution. The reaction was stirred for 15 min and was then placed in an icebath at 0°C, allowing the bath to warm to room temperature while stirring overnight for 15 h. The solvent was evaporated under reduced pressure and the residue was suspended in ether, washed with water, saturated NaHCO₃, saturated NaCl, dried (MgSO₄), filtered and evaporated to a pale yellow solid. The crude solid was slurried in hexane, filtered, and dried to afford diazo ketone 17 (9.73 g, 90%) which was used directly in the next step.

### Example 15

Chloroketone 18: To a solution of diazoketone 17 (9.73 g, 26 mmol) in ether (500 mL) at 0°C was added 4M HCl in dioxane (6.6 mL, 26 mmol). The solution was stirred for 1 h at 0°C and 4M HCl in dioxane (1 mL) was added. After 1h, the reaction solvent was evaporated under reduced pressure to afford the chloroketone 18 (9.79 g, 98%) as a white solid.

### Example 16

Chloroalcohol 19: A solution of chloroketone 18 (9.79g, 26 mmol) in THF (180 mL) and water (16 mL) was cooled to 0°C (internal temperature). Solid NaBH₄ (2.5 g, 66 mmol) was added in several portions over a period of 15 min while maintaining the internal temperature below 5°C. The mixture was stirred for 45 min and saturated KHSO₄ was slowly added until the pH<3. The mixture was partitioned between EtOAc and water. The aqueous phase was extracted with EtOAc and the combined organic extracts were washed with brine, dried (MgSO₄) filtered and evaporated under reduced pressure. The residue was dissolved in EtOAc, and was passed through a short column of silica gel, and the solvent was evaporated. The solid residue was recrystallized from EtOAc/hexane to afford the chloroalcohol 19 (3.84g) as a white solid.

### Example 17

Epoxide 21: A partial suspension of chloroalcohol 19 (1.16g, 3.1 mmol) in EtOH (50 mL) was treated with K₂CO₃ (2g, 14.5 mmol) and the mixture was stirred for 4 h at room temperature. The reaction mixture was diluted with EtOAc, filtered, and the solvents were evaporated under reduced pressure. The residue was partitioned between EtOAc and saturated NaCl, and the organic phase was dried (MgSO₄), filtered, and evaporated under reduced pressure to afford epoxide 21 (1.05g, 92%) as a white crystalline solid.

### Example 18

Sulfonamide 22: To a solution of epoxide 21 (1.05g, 3.1 mmol) in 2-propanol (40 mL) was added isobutylamine (6 mL, 61 mmol) and the solution was refluxed for 30 min. The solution was evaporated under reduced pressure and the crude solid was dissolved in CH₂Cl₂ (20 mL) and cooled to 0°C. Triethylamine (642 µL, 4.6 mmol) was added followed by the addition of (634 mg, 3.4 mmol) in CH₂Cl₂ (5 mL) and the solution was stirred for 2h at 0°C at which time the reaction solution was treated with additional triethylamine (1.5 mmol) and 4-methoxybenzenesulfonyl chloride (0.31 mmol). After 1.5 h, the reaction solution was evaporated under reduced pressure. The residue was partitioned between EtOAc and cold 1M H₃PO₄. The organic phase was washed with saturated NaHCO₃, saturated NaCl, dried (MgSO₄), filtered and the solvent was evaporated under reduced pressure. The crude product was purified on silica gel (15/1 - CH₂Cl₂/EtOAc) to afford 1.67g of a solid which was recrystallized from EtOAc/hexane to give sulfonamide 22 (1.54g, 86%) as a white crystalline solid.

### Example 19

Silyl ether 23: To a solution of the sulfonamide 22 (1.53g, 2.6 mmol) in CH₂Cl₂ (12 mL) at 0°C was added N,N-diisopropylethylamine (0.68 mL, 3.9 mmol) followed by tert-butyldimethylsilyl trifluoromethanesulfonate (0.75 mL, 3.3 mmol). The reaction solution was stirred for 1 h at 0°C and was warmed to room temperature, stirring for 17 h. Additional N,N-diisopropylethylamine (3.9 mmol) and tert-butyldimethylsilyl trifluoromethanesulfonate (1.6 mmol) was added, stirred for 2.5h, then heated to reflux for 3h and stirred at room temperature for 12 h. The reaction mixture was partitioned between EtOAc and cold 1M H₃PO₄. The organic phase was washed with saturated NaHCO₃, saturated NaCl, and was dried (MgSO₄) filtered and evaporated under reduced pressure. The crude product was purified on silica gel (2/1 - hexane/ether) to afford silyl ether 23 (780 mg, 43%) as an oil.

### Example 20

Phosphonate 24: A solution of 23 (260 mg, 0.37 mmol), triethylamine (0.52 mL, 3.7 mmol), and diethylphosphite (0.24 mmol, 1.85 mmol) in toluene (2 mL) was purged with argon and to the solution was added (Ph₃P)₄Pd (43 mg, 10 mol%). The reaction mixture was heated at 110°C (bath temperature) for 6 h, and was then allowed to stir at room temperature for 12h. The solvent was evaporated under reduced pressure and the residue was partitioned between ether and water. The aqueous phase was extracted with ether and the combined organic extracts were washed with saturated NaCl, dried (MgSO₄), filtered, and the solvent was evaporated under reduced pressure. The residue was purified by chromatography on silica gel (2/1 - ethyl acetate/hexane) to afford diethylphosphonate 24 (153 mg, 55%).

### Example 21

Phosphonic acid 26: To a solution of 24 (143 mg) in MeOH (5 mL) was added 4N HCl (2 mL). The solution was stirred at room temperature for 9h and was evaporated under reduced pressure. The residue was triturated with ether and the solid was collected by filtration to provide hydrochloride salt 25 (100 mg, 92%) as a white powder. To a solution of X (47 mg, 0.87 mmol) in CH₃CN (1 mL) at 0°C was added TMSBr (130 µL, 0.97 mmol). The reaction was warmed to room temperature and stirred for 6.5h at which time TMSBr (0.87 mmol) was added and stirring was continued for 16h. The solution was cooled to 0°C and was quenched with several drops of ice-cold water. The solvents were evaporated under reduced pressure and the residue was dissolved in several milliters of MeOH and treated with propylene oxide (2 mL). The mixture was heated to gentle boiling and evaporated. The residue was triturated with acetone and the solid was collected by filtration to give phosphonic acid 26 (32 mg, 76%) as a white solid.

### Example 22

Phosphonate 27: To a suspension of 26 (32 mg, 0.66 mmol) in CH₃CN (1 mL) was added bis(trimethylsilyl)acetamide (100 µL, 0.40 mmol) and the solution was stirred for 30 min at room temperature. The solvent was evaporated under reduced pressure and the residue was dissolved in CH₃CN (1 mL). To this solution was added (3R, 3aR, 6aS)-hexahydrofuro[2, 3-*b*]furan-2-yl 4-nitrophenyl carbonate (20 mg, 0.069 mmol, prepared according to Ghosh et al. J. Med. Chem. 1996, 39, 3278.), N,N-diisopropylethylamine (35 µL, 0.20 mmol), and N,N-dimethylaminopyridine (catalytic amount). The solution was stirred for 22h at room temperature, diluted with water (0.5 mL) and was stirred with IR 120 ion exchange resin (325 mg, H⁺ form) until the pH was <2. The resin was removed by filtration, washed with methanol and the filtrate was concentrated under reduced pressure. The residue was dissolved water, treated with solid NaHCO₃ until pH=8 and was evaporated to dryness. The residue was dissolved in water and was purified on C18 reverse phase chromatography eluting with water followed by 5%, 10% and 20% MeOH in water to give the disodium salt 27 (24 mg) as a pale yellow solid: ¹H NMR (D₂O) δ 7.72 (d, 2H), 7.52 (dd, 2H), 7.13 (dd, 2H), 7.05 (d, 2H), 5.58 (d, 1H), 4.87 (m, 1H), 3.86-3.53 (m overlapping s, 10H), 3.22 (dd, 1H), 3.12-2.85 (6H), 2.44 (m, 1H), 1.83 (m, 1H), 1.61 (m, 1H)1.12 (dd, 1H), 0.77 (m, 6H); ³¹P NMR (D₂O) δ 11.23 ; MS (ESI) 641 (M-H).

### Example 23

Diethylphosphonate 28: To a solution of 25 (16 mg, 0.028 mmol) in CH₃CN (0.5 mL) was added (3R, 3aR, 6aS)-hexahydrofuro[2, 3-*b*]furan-2-yl 4-nitrophenyl carbonate (9 mg, 0.031 mmol), N,N-diisopropylethylamine (20 µL, 0.11 mmol), and N,N-dimethylaminopyridine (catalytic amount). The solution was stirred at room temperature for 48 h and was then concentrated under reduced pressure. The residue was partitioned between EtOAc and saturated NaHCO₃. The organic phase was washed with saturated NaHCO₃, saturated NaCl, and was dried (MgSO₄), filtered, and concentrated under reduced pressure. The residue was purified by silica gel chromatography (2.5-5% 2-propanol/CH₂Cl₂). The residue obtained was further purified by preparative layer chromatography (5% MeOH/CH₂Cl₂) followed by column chromatography on silica gel (10% 2-propanol/CH₂Cl₂) to afford diethylphosphonate 28 (7 mg) as a foam: ¹H NMR (CDCl₃) δ 7.72-7.66 (m, 4H), 7.32-7.28 (2H), 6.96 (d, 2H), 5.60 (d, 1H), 4.97 (m, 2H), 4.18-4.01 (m, 4H), 3.94-3.60 (m overlapping s, 10H), 3.15-2.72 (m, 7H), 1.78 (m, 1H), 1.61 (m+H₂O, ~3H), 1.28 (t; 6H), 0.86 (m, 6H); ³¹P NMR (CDCl₃) δ 18.6 ; MS (ESI) 699 (M+H).

### Prospective Example 24

Diphenyl phosphonate 14 is treated with aqueous sodium hydroxide to provide monophenyl phosphonate 29 according to the method found in J. Med. Chem. 1994, 37, 1857. Monophenyl phosphonate 29 is then converted to the monoamidate 30 by reaction with an amino acid ester in the presence of Ph₃ and 2,2'-dipyridyl disulfide as described in the synthesis of bisamidate 16f. Alteratively, monoamidate 30 is prepared by treating 29 with an amino acid ester and DCC. Coupling conditions of this type are found in Bull. Chem. Soc. Jpn. 1988, 61, 4491.

### Example 25

Diazo ketone 1: To a solution of N-tert-Butoxycarbonyl-O-benzyl-L-tyrosine (25 g, 67 mmol, Fluka) in dry THF (150 mL) at -25-30°C (external bath temperature) was added isobutylchloroformate (8.9 mL, 69 mmol) followed by the slow addition of N.methylmorpholine (37.5 mL, 69 mmol). The mixture was stirred for 40 min, and diazomethane (170 mmol, generated from 25 g 1-methyl 3-nitro-1-nitroso-guanidine according to Aldrichimica Acta 1983, 16, 3) in ether (400 mL) was poured into the mixed anhydride solution. The reaction was stirred for 15 min allowing the bath to warm to room temperature while stirring overnight for 4 h. The mixture was bubbled with N₂ for 30 min., washed with water, saturated NaHCO₃, saturated NaCl, dried (MgSO₄) filtered and evaporated to a pale yellow solid. The crude solid was slurried in hexane, filtered, and dried to afford the diazo ketone (26.8 g, 99%) which was used directly in the next step.

### Example 26

Chloroketone 2: To a suspension of diazoketone 1 (26.8 g, 67 mmol) in ether/THF (750 mL, 3/2) at 0°C was added 4M HCI in dioxane (16.9 mL, 67 mmol). The solution was stirred at 0°C for 2 hr. The reaction solvent was evaporated under reduced pressure to give the chloroketone (27.7 g, 97%) as a solid.

### Example 27

Chloroalcohol 3: To a solution of chloroketone 2 (127.1 g, 67 mmol) in THF (350 mL) was added water (40 mL) and the solution was cooled to 3-4°C (internal temperature). NaBH₄ (6.3 g, 168 mmol) was added in portions. The mixture was stirred for 1h at 0°C and the solvents were removed. The mixture was diluted with ethyl acetate and saturated KHSO₄ was slowly added until the pH<4 followed by saturated NaCl. The organic phase was washed with saturated NaCl, dried (MgSO₄) filtered and evaporated under reduced pressure. The crude product consisted of a 70:30 mixture of diastereomers by HPLC analysis (mobile phase, 77:25-CH₃CN:H₂O; flow rate: 1 mL/min; detection: 254 nm; sample volume: 20 µL; column: 5µ C18, 4.6X250 mm, Varian; retention times: major diastereomer 3, 5.4 min, minor diastereomer 4, 6.1 min). The residue was recrystallized from EtOAc/hexane twice to afford the chloro alcohol 3 (12.2g, >96% diastereomeric purity by HPLC analysis) as a white solid.

### Example 28

Epoxide 5: To a solution of chloroalcohol 3 (12.17 g, 130 mmol) in EtOH (300 mL) was added KOR/EtOH solution (0.71N, 51 mL, 36 mmol). The mixture was stirred for at room temperature for 1.5h. The reaction mixture was evaporated under reduced pressure. The residue was partitioned between EtOAc and water and the organic phase was washed with saturated NH₄Cl, dried (MgSO₄), filtered, and evaporated under reduced pressure to afford the epoxide (10.8 g, 97%) as a white solid.

### Example 29

Sulfonamide 6: To a suspension of epoxide 5 (10.8 g, 30 mmol) in 2-propanol (100 mL) was added isobutylamine (129.8 mL, 300 mmol) and the solution was refluxed for 1 hr. The solution was evaporated under reduced pressure to give a crude solid. The solid (42 mmol) was dissolved in CH₂Cl₂ (200 mL) and cooled to 0°C. Triethylamine (11.7 mL, 84 mmol) was added followed by the addition of 4-methoxybenzenesulfonyl chloride (8.68 g, 42 mmol) and the solution was stirred for 40 min at 0°C, warmed to room temperature and evaporated under reduced pressure. The residue was partitioned between EtOAc and saturated NaHCO₃. The organic phase was washed with saturated NaCl, dried (MgSO₄) filtered and evaporated under reduced pressure. The crude product was recrystallized from EtOAc/hexane to give the sulfonamide (23.4 g, 91%) as a small white needles: mp 122-124°C (uncorrected).

### Example 30

Carbamate 7: A solution of sulfonamide 6 (6.29.mg, 10.1 mmol) in CH₂Cl₂ (20 mL) was treated with trifluoroacetic acid (10 mL). The solution was stirred for 3 hr. Volatiles were evaporated under reduced pressure and the residue was partitioned between EtOAc and 0.5 N NaOH. The organic phase were washed with 0.5 N NaOH (2x), water (2x) and saturated NaCl, dried (MgSO₄), filtered, and evaporated under reduced pressure. The residue was dissolved in CH₃CN (60 mL), cooled to 0°C and was treated with (3R, 3aR, 6aS)-hexahydrofuro[2, 3-*b*]furan-2-yl 4-nitrophenyl carbonate (298.5 g, 10 mmol, prepared according to Ghosh et al. J. Med. Chem. 1996, 39, 3278.) and N,N-dimethylaminopyridine (2.4 g, 20 mmol). After stirring for 1h at 0°C, the reaction solvent was evaporated under reduced pressure and the residue was partitioned between EtOAc and 5% citric acid. The organic phase was washed twice with 1% K₂CO₃, and then was washed with saturated NaCl, dried (MgSO₄), filtered, and evaporated under reduced pressure. The crude product was purified by chromatography on silica gel (1/1 - EtOAc/hexane) affording the carbamate (5.4 g, 83%) as a solid: mp 128-129°C (MeOH, uncorrected).

### Example 31

Phenol 8: A solution of carbamate 7 (5.4 g, 8.0 mmol) in EtOH (260 mL) and EtOAc (130 mL) was treated with 10% Pd/C (540 mg) and was stirred under H₂ atmosphere (balloon) for 3h. The reaction solution stirred with celite for 10 min, and passed through a pad of celite. The filtrate was evaporated under reduced pressure to afford the phenol as a solid (4.9 g) that contained residual solvent: mp 131-134°C (EtOAc/hexane, uncorrected).

### Example 32

Dibenzylphosphonate 10: To a solution of dibenzylhydroxymethyl phosphonate (3.1 g, 10.6 mmol) in CH₂Cl₂ (30 mL) was treated with 2,6-lutidine (1.8 mL, 15.6 mmol) and the reaction flask was cooled to -50°C (external temperature). Trifluoromethanesulfonic anhydride (2.11 mL, 12.6 mmol) was added and the reaction mixture was stirred for 15 min and then the cooling bath was allowed to warm to 0°C over 45 min. The reaction mixture was partitioned between ether and ice-cold water. The organic phase was washed with cold 1M H₃PO₄, saturated NaCl, dried (MgSO₄), filtered and evaporated under reduced pressure to afford triflate 9 (3.6 g, 80%) as an oil which was used directly without any further purification. To a solution of phenol 8 (3.61 g, 6.3 mmol) in THF (90 mL) was added Cs₂CO₃ (4.1 g, 12.6 mmol) and triflate 9 (4.1 g, 9.5 mmol) in THF (10 mL). After stirring the reaction mixture for 30 min at room temperature additional Cs₂CO₃ (6.96 g, 3 mmol) and triflate (1.26 g, 3 mmol) were added and the mixture was stirred for 3.5h. The reaction mixture was evaporated under reduced pressure and the residue was partitioned between EtOAc and saturated NaCl. The organic phase was dried (MgSO₄), filtered and evaporated under reduced pressure. The crude product was chromatographed on silica gel eluting (5% 2-propanol/CH₂Cl₂) to give the dibenzylphosphonate as an oil that solidified upon standing. The solid was dissolved in EtOAc, ether was added, and the solid was precipitated at room temperature overnight. After cooling to 0°C the solid was filtered and washed with cold ether to afford the dibenzylphosphonate (3.43 g, 64%) as a white solid: ¹H NMR (CDCl₃) δ 7.66 (d, 2H), 7.31 (s, 10H), 7.08 (d, 2H), 6.94 (d, 2H), 6.76 (d, 2H), 5.59 (d, 1H), 5.15-4.89 (m, 6H), 4.15 (d, 2H), 3.94-3.62 (m, 10H), 3.13-2.69 (m, 7H), 1.78 (m, 1H), 1.70-1.44 (m, 2H), 0.89-0.82 (2d, 6H); ³¹P NMR (CDCl₃) δ 18.7; MS (ESI) 853 (M+H).

### Example 33

Phosphonic acid 11: A solution of dibenzylphosphonate 10 (3.43 g) was dissolved in EtOH/EtOAc (150 mL/50 mL), treated with 10% Pd/C (350 mg) and was stirred under H₂ atmosphere (balloon) for 3 h. The reaction mixture was stirred with celite, and the catalyst was removed by filtration through celite. The filtrate was evaporated under reduced pressure and the residue was dissolved in MeOH and filtered with a 0.45 µM filter. After evaporation of the filtrate, the residue was triturated with ether and the solid was collected by filtration to afford the phosphonic acid (2.6 g, 94%) as a white solid: ¹H NMR (CDCl₃) δ 7.77 (d, 2H), 7.19 (d, 2H), 7.09 (d, 2H), 6.92 (d, 2H), 5.60 (d, 1H), 4.95 (m, 1H), 4.17 (d, 2H), 3.94 (m, 1H), 3.89 (s, 3H), 3.85-3.68 (m, 5H), 3.42 (dd, 1H), 3.16-3.06 (m, 2H), 2.96-2.84 (m, 3H), 2.50 (m, 1H), 2.02 (m, 1H), 1.58 (m, 1H), 1.40 (dd, 1H), 0.94 (d, 3H), 0.89 (d, 3H); ³¹P NMR (CDCl₃) δ 16.2; MS (ESI) 671 (M-H).

### Example Section B

There is no Section B in this application.

### Example Section C

### Example 1

Diphenyl phosphonate **31:** To a solution of phosphonic acid **30** (11 g, 16.4 mmol) and phenol (11 g, 117 mmol) in pyridine (100 mL) was added 1, 3-dicyclohexylcarbodiimide (13.5 g, 65.5 mmol). The solution was stirred at room temperature for 5 min and then at 70°C for 2h. The reaction mixture was cooled to room temperature, diluted with ethyl acetate (100 mL) and filtered. The filtrate was evaporated under reduced pressure to remove pyridine. The residue was dissolved in ethyl acetate (250 mL) and acidified to pH = 4 by addition of HCl (0.5 N) at 0°C. The mixture was stirred at 0°C for 0.5 h, filtered and the organic phase was separated and washed with brine, dried over MgSO₄, filtered and concentrated under reduced pressure. The residue was purified on silica gel to give diphenyl phosphonate **31** (9 g, 67%) as a solid. ³¹P NMR (CDCl₃) d 12.5.

### Example 2

Monophenyl phosphonate **32:** To a solution of diphenylphosphonate **31** (9.0 g, 10.9 mmol) in acetonitrile (400 mL) was added NaOH (1N, 27 mL) at 0°C. The reaction mixture was stirred at 0°C for 1 h, and then treated with Dowex (50WX8-200, 12 g). The mixture was stirred for 0.5 h at 0°C, and then filtered. The filtrate was concentrated under reduced pressure and co-evaporated with toluene. The residue was dissolved in ethyl acetate and hexane was added to precipitate out the monophenyl phosphonate **32** (8.1 g, 100%). ³¹P NMR (CDCl₃) d 18.3.

### Example 3

Monoamidate 33a (R₁ = Me, R₂ = n-Bu): To a flask charged with monophenyl phosphonate **32** (4.0 g, 5.35 mmol), was added L-alanine n-butyl ester hydrochloride (4.0 g, 22 mmol), 1, 3-dicyclohexylcarbodiimide (6.6 g, 32 mmol), and finally pyridine (30 mL) under nitrogen. The resultant mixture was stirred at 60 - 70°C for 1 h, then cooled to room temperature and diluted with ethyl acetate. The mixture was filtered and the filtrate was concentrated under reduced pressure. The residue was partitioned between ethyl acetate and HCl (0.2 N) and the organic layer was separated. The ethyl acetate phase was washed with water, saturated NaHCO₃, dried over MgSO4, filtered and concentrated under reduced pressure. The residue was purified on silica gel (pre-treated with 10% MeOH / CH₃CO₂Et, eluting with 40% CH₂Cl₂ / CH₃CO₂Et and CH₃CO₂Et) to give two isomers of **33a** in a total yield of 51 %.

Isomer A (1.1 g): 1H NMR (CDCl₃) d 0.88 (m, 9H), 1.3 (m, 2H), 1.35 (d, J = 7 Hz, 3H), 1.55 (m, 2H), 1.55-1.7 (m, 2H), 1.8 (m, 1H), 2.7-3.2 (m, 7H), 3.65-4.1 (m, 9H), 3.85 (s, 3H), 4.2 (m, 1H), 4.3 (d, J = 9.6 Hz, 2H), 5.0 (m, 2H), 5.65 (d, J = 5.4 Hz, 1H), 6.85 (d, J = 8.7 Hz, 2H), 7.0 (d, J = 8.7 Hz, 2H), 7.1-7.3 (m, 7H), 7.7 (d, J = 8.7 Hz, 2H); ³¹P NMR (CDCl₃) d 20.5. Isomer B (1.3 g) 1H NMR (CDCl3) d 0.88 (m, 9H), 1.3 (m, 2H), 1.35 (d, J = 7 Hz, 3H), 1.55 (m, 2H), 1.55-1.7 (m, 2H), 1.8 (m, 1H), 2.7-3.2 (m, 7H), 3.65-4.1 (m, 9H), 3.85 (s, 3H), 4.2-4.35 (m, 3H), 5.0 (m, 2H), 5.65 (d, J = 5.4 Hz, 1H), 6.85 (d, J = 8.7 Hz, 2H), 7.0 (d, J = 8.7 Hz, 2H), 7.1-7.3 (m, 7H), 7.7 (d, J = 8.7 Hz, 2H); ³¹P NMR (CDCl₃) d 19.4.

### Example 4

Monoamidate 33b (R₁ = Me, R₂ = i-Pr) was synthesized in the same manner as 33a in 77% yield. Isomer A : 1H NMR (CDCl3) d 0.9 (2d, J = 6.3Hz, 6H), 1.2 (d, J = 7 Hz, 6H), 1.38 (d, J = 7 Hz, 3H), 1.55-1.9 (m, 3H), 2.7-3.2 (m, 7H), 3.65-4.1 (m, 8H), 3.85 (s, 3H), 4.2 (m, 1H), 4.3 (d, J = 9.6 Hz, 2H), 5.0 (m, 2H), 5.65 (d, J = 5.4 Hz, 1H), 6.85 (d, J = 8.7 Hz, 2H), 7.0 (d, J = 8.7 Hz, 2H), 7.1-7.3 (m, 7H), 7.7 (d, J =8.7 Hz, 2H); ³¹P NMR (CDCl₃) d 20.4. Isomer B: 1H NMR (CDCl3) d 0.9 (2d, J = 6.3Hz, 6H), 1.2 (d, J = 7 Hz, 6H), 1.38 (d, J = 7 Hz, 3H), 1.55-1.9 (m, 3H), 2.7-3.2 (m, 7H), 3.65-4.1 (m, 8H), 3.85 (s, 3H), 4.2 (m, 1H), 4.3 (d, J = 9.6 Hz, 2H), 5.0 (m, 2H), 5.65 (d, J = 5.4 Hz, 1H), 6.85 (d, J = 8.7 Hz, 2H), 7.0 (d, J = 8.7 Hz, 2H), 7.1-7.3 (m, 7H), 7.7 (d, J = 8.7 Hz, 2H); ³¹P NMR (CDCl₃) d 19.5.

### Example Section D

### Example 1

Cyclic Anhydride 1 (6.57 g, 51.3 mmol) was treated according to the procedure of Brown et al., J. Amer. Chem Soc. 1955, 77, 1089 -1091 to afford amino alcohol 3 (2.00g, 33%). *for intermediate 2 :* ¹H NMR (CD₃OD) δ 2.40 (S, 2H), 1.20 (s, 6H).

### Example 2

Amino alcohol 3 (2.0 g, 17 mmol) was stirred in 30 mL 1:1 THF: water. Sodium Bicarbonate (7.2 g, 86 mmol) was added, followed by Boc Anhydride (4.1 g, 19 mmol). The reaction was stirred for 1 hour, at which time TLC in 5% methanol/DCM with ninhydrin stain showed completion. The reaction was partitioned between water and ethyl acetate. The organic layer was dried and concentrated, and the resulting mixture was chromatographed on silica in 1:1 hexane: ethyl acetate to afford two fractions, "upper" and "lower" each having the correct mass. By NMR the correct product 4 was "lower" (0.56 g, 14%) ¹H NMR (CDCl₃) δ 3.7 (t, 2H), 3.0 (d,2H), 1.45 (t, 2H) 1.4 (s, 9H), 0.85 (s, 6H),
MS (ESI): 240 (M + 23).

### Example 3

Sodium Hydride (60% emulsion in oil) was added to a solution of the alcohol 4 (1.1g, 5.2 mmol) in dry DMF in a 3-neck flask under dry nitrogen. Shortly afterward triflate 35 (2.4 g, 5.7 mmol) was added with stirring for 1.5 hrs. Mass spectrometry showed the presence of the starting material (240, M+23), thus 100 mg more 60% sodium hydride emulsion as well as ~1 g more triflate were added with an additional hour of stirring. The reaction was quenched by the addition of saturated NaHCO₃ then partitioned between ethyl acetate and water. The organic layer was dried with brine and MgSO₄ and eluted on silica with 1:1 hexane:ethyl acetate to afford 5 (0.445 g, 15%). NMR showed some contamination with alcohol 4 starting material. ¹H NMR (CDCl₃): δ 7.28 (s, 10H), 5.00 (m, 4H), 3.70 (t, 2H), 2.94, (d, 2H), 1.44 (t, 2H), 1.40 (s, 9H), 0.83 (s, 6H) MS (ESI): 514 (M+23).

### Example 4

Phosphonate ester 5 (0.445 g, 0.906 mmol) was stirred with with 20% TFA in DCM. (5 mL) TLC showed completion in 1 hr time. The reaction was azeotroped with toluene then run on a silica gel column with 10% methanol in DCM. Subsequently, the product was dissolved in ethyl acetate and shaken with saturated sodium bicarbonate: water (1:1), dried with brine and magnesium sulfate to afford the free amine 6 (30mg, 8.5%). ¹H NMR (CDCl₃): δ 7.30 (s, 10H), 5.00 (m, 4H), 3.67 (d, 2H), 3.47, (t, 2H), 2.4-2.6 (brs) 1.45 (t, 2H), 0.82 (s, 6H), MS (ESI): 393 (M+1).

### Example 5

Amine 6 (30 mg, 0.08 mmol) and epoxide 7 (21 mg, 0.08 mmol) were dissolved in 2 mL IprOH and heated to reflux for 1 hr then monitored by TLC in 10% MeOH/DCM. Added -20 mg more epoxide 7 and continued reflux for 1 hr. Cool to room temperature, dilute with ethyl acetate, shake with water and brine, dry with magnesium sulfate. Silica gel chromatography using first 5% then 10% MeOH in EtOAc yielded amine 8 (18 mg, 36%). ¹H NMR (CDCl₃): δ 7.30 (s, 10H), 7.20-7-14 (m, 5H), 5.25-4.91 (m, 4H), 3.83, (m, 1H), 3.71 (d, 2H) 3.64 (m, 1H), 3.54 (t, 2H), 3.02-2.61 (m, 5H), 2.65-2.36 (dd, 2H) (t, 2H), 1.30 (s, 9H) 0.93 (s, 9H) 0.83 (t, 2H) MS (ESl) 655 (M+1).

### Example 6

Amine 8 (18 mg, 0.027 mmol) was dissolved in 1 mL DCM then acid chloride 9 (6 mg, 0.2 mmol) followed by triethylamine (0.004 mL, 0.029 mmol). The reaction was monitored by TLC. Upon completion the reaction was diluted with DCM shaken with 5% citric acid, saturated sodium bicarbonate, brine, and dried with MgSO₄. Purification on silica (1:1 Hexane:EtOAc) afforded sulfonamide 10 (10.5 mg, 46%). ¹H NMR (CDCl₃): δ 7.69 (d, 2H), 7.30 (s, 10H), 7.24-7-18 (m, 5H), 5.00 (m, 4H), 4.73, (d, 1H), 4.19 (s, 1H) 3.81 (m, 1H), 3.80 (s, 3H), 3.71 (d,2H), 3.57 (t, 2H), 3.11-2.95 (m, 5H) 2.75 (m,1H)1.25 (s, 1H), 0.90 (s, 6H) MS (ESI) 847 (M+Na⁺).

### Example 7

Sulfonamide 10 (10.5 mg, 0.013 mmol) was stirred at room temperature in 20% TFA/DCM. Once Boc deprotection was complete by TLC (1:1 Hexane:EtOAc) and MS, the reaction was azeotroped with toluene. The TFA salt of the amine was dissolved in acetonitrile (0.5 mg) and to this were added carbonate 11 (4.3 mg, 0.014 mmol) followed by DMAP (4.6 mg, 0.038 mg). Stir at room temp until TLC (1:1 Hexane:EtOAc) shows completion. Solvent was evaporated and the residue was redissolved in EtOAc then shaken with saturated NaHCO₃. The organic layer was washed with water and brine, then dried with MgSO₄ Purification on silica with Hexane: EtOAc afforded compound 12 (7.1 mg, 50%). ¹H NMR (CDCl₃): δ 7.75 (d, 2H) 7.24-7.35 (15H) 6.98 (d, 2H), 5.62 (d, 1H) 5.04 (m, 4H) 4.98 (m, 1H) 4.03 (m, 1H), 3.85 (s, 3H), 3.61-3.91 (9H), 3.23-3.04 (5H) 2.85 (m, 1H), 2.74 (m,1H) 1.61 (d, 2H), 1.55 (m, 1H) 1.36 (m, 1H) 0.96 (d, 6H) MS (ESI): 903 (M+23).

### Example 8

Compound 12 (6.1 mg, 0.007 mmol) was dissolved in 1 mL 3:1 EtOH:EtoAc. Palladium catalyst (10% on C, 1mg) was added and the mixture was purged three times to vacuum with 1 atmosphere hydrogen gas using a balloon. The reaction was stirred for 2 hrs, when MS and TLC showed completion. The reaction was filtered through Celite with EtOH washing and all solvent to was evaporated to afford final compound 13 (5mg, 100%). ¹H NMR (CD₃OD): δ 7.79 (d, 2H) 7.16-7.24 (5H) 7.09 (d, 2H) 5.58 (d, 1H) 4.92 (m, 1H) 3.97 (m, 1H), 3.92 (dd,1H) 3.89 (s, 3H) 3.66-3.78 (8H) 3.40 (d,1H), 3.37 (dd, 1H), 3.15 (m, 1H) 3.12 (dd,1H) 2.96 (d, 1H), 2.87 (m, 1H), 2.74 (m,1H) 2.53 (m, 1H) 1.70 (m, 2H), 1.53 (m, 1H) 1.32 (m, 1H) 1.04 (d, 6H) MS (ESI): 723 (M+23).

### Example 9

Amino Alcohol 14 (2.67g, 25.9 mmol) was dissolved in THF with stirring and Boc Anhydride (6.78g, 31.1 mmol) was added. Heat and gas evolution ensued. TEA (3.97 mL, 28.5 mmol) was added and the reaction was stirred overnight. In the morning, the reaction was quenched by the addition of saturated NaHCO₃. The organic layer was separated out and shaken with water, dried with brine and MgSO₄ to afford 15 which was used without further purification. (100% yield) (some contamination): ¹H NMR (CDCl₃): δ 3.76 (t ,1H) 3.20, (d,2H), 2.97 (d, 2H), 1.44 (s, 9H), 0.85 (s, 6H).

### Example 10

A solution of the alcohol 15 (500 mg, 2.45 mmol) in dry THF was cooled under dry N₂ with stirring. To this was added n-butyl lithium (1.29 mL, 2.71 mmol) as a solution in hexane in a manner similar to that described in Tetrahedron. 1995, 51 #35, 9737-9746. Triflate 35 (1.15 g, 2.71 mmol) was added neat with a tared syringe. The reaction was stirred for four hours, then quenched with saturated NaHCO₃. The mixture was then partitioned between water and EtOAc. The organic layer was dried with brine and MgSO₄, then chromatographed on silica in 1:1 Hexane:EtOAc to afford phosphonate 16 (445mg, 38%) ¹H NMR (CDCl₃): δ 7.37 (m, 10H), 5.09 (m, 4H), 3.73-3.75 (m, 2H), 3.24 (s,2H), 3.02 (d, 2H), 1.43 (s, 9H), 0.86 (s, 6H).

### Example 11

Phosphonate 16 (249 mg, 0.522 mmol) was stirred in 20% TFA/DCM for 1 br. The reaction was then azeotroped with toluene. The residue was re-dissolved in EtOAc, then shaken with water: saturated NaHCO₃ (1:1). The organic layer was dried with brine and MgSO₄ and solvent was removed to afford amine 17 (143 mg, 73%) ¹H NMR (CDCl₃): δ 7.30 (s, 10H), 5.05-4.99 (m, 4H), 3.73 (d, 2H), 3.23 (s, 2H), 2.46 (brs, 2H), 0.80 (s, 6H) ³¹P NMR (CDCl₃): δ 23.77 (s).

### Example 12

Amine 17 (143 mg, 0.379 mmol) and epoxide 7 (95 mg, 0.360 mmol) were dissolved in 3 mL IprOH and heated to 85°C for 1 hr. The reaction was cooled to room temperature overnight then heated to 85°C for 1 hr more in the morning. The reaction was then diluted with EtOAc, shaken with water, dried with brine MgSO₄ and concentrated. The residue was eluted on silica in a gradient from 5% to 10% MeOH in DCM to afford compound 18 (33 mg, 14%).

### Example 13

Mix compound 18 (33 mg, 0.051 mmol) and chlorosulfonyl compound 9 (11 mg, 0.054 mmol) in 2 mL DCM then add TEA (0.0075 mL, 0.054 mmol), stir for 5 hrs. TLC in 1:1 EtOAc: hexane shows reaction not complete. Place in freezer overnight. In the morning, take out of freezer, stir for 2 hrs, TLC shows completion. Workup done with 5% citric acid, saturated NaHCO₃, then dry with brine and MgSO₄. The reaction mixture was concentrated and chromatographed on a Monster Pipette column in 1:1 hexane: EtOAc then 7:3 hexane: EtOAc to avail compound 19 (28 mg , 67%) ¹H NMR (CDCl₃): δ 7.37 (d, 2H), 7.20 (m, 15H), 6.90 (d, 2H), 5.07-4.93 (m, 4H), 4.16 (brs, 1H), 3.80 (s, 3H), 3.75-3.37 (m, 4H), 3.36 (d, 1H), 3.20-2.93 (m, 6H), 2.80- 2.75 (dd, 1H).

### Example 14

Compound 19 (28 mg, 0.35 mmol) was stirred in 4 mL DCM with addition of 1 mL TFA. Stir for 45 minutes, at which time complete deprotection was noted by TLC as well as MS. Azeotrope with toluene. The residue was dissolved in 1 mL CH₃CN, cooled to 0°C. Bis-Furan *para*-Nitro phenol carbonate 11 (12 mg, 0.038 mmol), dimethyl amino pyridine (~1 mg, 0.008 mmol) and diisopropylethylamine (0.018 mL, 0.103 mmol) were added. The mixture was stirred and allowed to come to room temperature and stirred until TLC in 1:1 hexane:EtOAc showed completion. The reaction mixture was concentrated and the residue was partitioned between saturated NaHCO₃ and EtOAc. The organic layer was dried with brine and MgSO₄, then chromatographed on silica with hexane:EtOAc to afford compound 20 (20 mg, 67%). ¹NMR (CDCl₃): δ 7.76 (d, 2H), 7.34-7.16 (m, 15 H), 7.07 (d, 2H), 5.56 (d, 1H), 5.09 (m, 4H), 4.87 (m, 1H), 4.01 (m, 1H), 3.91 (m, 2H), 3.87 (s, 3H), 3.86 (m, 1H), 3.69 (m, 1H), 3.67 (m, 1H) 3.60 (d, 2H) 3.28 (m, 1H) 3.25 (d, 2H), 3.32 (d, 1H), 3.13 (m, 1H), 3.02 (m, 1H) 2.85 (d, 1H), 2.83 (m, 1H) 2.52 (m, 1H) 1.47 (m, 1H), 1.31 (m, 1H) 0.98 (s, 3H), 0.95 (s,3H).

### Example 15

Compound 20 (7 mg, 0.008 mmol) was treated in a manner identical to example 8 to afford compound 21 (5 mg, 90%) ¹H NMR (CDCl₃): δ 7.80 (d, 2H), 7.25-7.16 (m, 5H), 7.09 (d, 2H), 5.58 (d, 1H), 4.92 (m, 1H), 3.99 (m, 1H), 3.92 (m, 1H), 3.88 (s, 3H), 3.86 (m, 1H), 3.77 (m, 1H), 3.75 (m, 1H), 3.73 (m, 1H), 3.71 (m, 1H) 3.71 (m, 1H), 3.68 (m, 1H), 3.57 (d,1H), 3.41 (d, 1H), 3.36 (m, 1H), 3.29 (d, 1H), 3.25 (d, 2H), 3.18 (m, 1H), 3.12 (m, 1H), 3.01 (d, 1H) 2.86 (m, 1H), 2.53 (m, 1H) 1.50 (m, 1H), 1.33 (m, 1H), 1.02 (s, 3H), 0.99 (s; 3H).

### Example 16

Compound 15 (1.86 g, 9.20 mmol) was treated with triflate 22 in a manner identical to example 10 to afford compound 23 (0.71 g, 21.8%) ¹H NMR (CDCl₃): δ 5.21 (brs, 1H) 4.16-4.07 (m, 4H), 3.71-3.69 (d, 2H), 3.24 (s, 2H), 1.43 (s, 9H), 1.34-1.28 (m, 6H) 0.86 (s, 6H).

### Example 17

Compound 23 (151 mg, 0.427 mmol) was dissolved in 10 mL DCM and 1.0 mL TFA was added. The reaction was stirred until completion. The reaction was azeotroped with toluene and the residue was then dissolved in THF and treated with basic Dowex resin beads. Afterwards, the beads were filtered away and solvent was removed to avail compound 24 (100 mg, 92%) ¹H NMR (CDCl₃): δ 4.15-4.05 (m, 4H), 3.72-3.69 (d, 2H), 3.27 (s, 2H), 1.30-1.26 (m, 6H) 0.81 (s, 6H).

### Example 18

Compound 24 (100 mg, 0.395 mmol) was treated in a manner identical to example 12 to avail compound 25 (123 mg, 60%). ¹H NMR (CDCl₃): δ 7.26-7.13 (m, 5H), 4.48-4.83 (d, 1H) 4.17-4.06 (m, 4H), 3.75 (d, 2H) 3.56 (brs, 1H), 3.33 (s, 2H), 2.93-2.69 (m, 4H), 2.44-2.55 (dd, 2H) 1.32 (m, 6H), 0.916 (s, 6H).

### Example 19

Compound 25 (88 mg, 0.171 mmol) was treated in a manner identical to example 13 to afford compound 26 (65 mg, 55%) ¹H NMR (CDCl₃): δ 7.26-7.13 (m, 5H), 4.48-4.83 (d, 1H) 4.17-4.06 (m, 4H), 3.75 (d, 2H) 3.56 (brs, 1H), 3.33 (s, 2H), 2.93-2.69 (m, 4H), 2.44-2.55 (dd, 2H) 1.32 (m, 6H), 0.916 (s, 6H).

### Example 20

Compound 26 (65 mg, 0.171 mmol) was treated in a manner identical to example 14 to afford compound 27 (49 mg, 70%) ¹H NMR:
(CDCl₃):δ 7.75 (d, 2H), 7.25-7.24 (m,4 H), 7.18 (m, 1H) 6.99 (d, 2H), 5.63 (d, 1H), 5.01 (m, 1H), 4.16 (m, 4H), 3.94 (m, 1H), 3.88 (m, 1H), 3.88 (s, 3H), 3.84 (m, 1H), 3.81 (m, 1H), 3.74 (m, 2H),), 3.70 (m, 1H), 3.69 (m, 1H) 3.43 (m, 1H), 3.24 (m, 1H), 3.22 (m, 2H) 3.21 (m, 2H) 3.12 (m, 1H), 3.02 (m, 1H) 2.86 (m, 1H), 2.72 (m, 1H), 1.54 (m, 1H), 1.38 (m, 1H) 1.35 (m, 6H) 1.00 (s, 3H), 0.96 (s,3H).

### Example 21

Boc protected amine 28 (103 mg, 0.153 mmol) was dissolved in DCM (5 mL). The stirred solution was cooled to 0°C. BBr₃ as a 1.0 M solution in DCM (0.92 mL, 0.92 mmol) was added dropwise over 10 min, and the reaction was allowed to continue stirring at 0°C for 20 min. The reaction was warmed to room temperature and stirring was continued for 2 hours. The reaction was then cooled to 0°C and quenched by dropwise addition of MeOH (1 mL). The reaction mixture was evaporated and the residue suspended in methanol which was removed under reduced pressure. The procedure was repeated for EtOAc and finally toluene to afford free amine HBr salt 29 (107 mg, >100%) which was used without further purification.

### Example 22

Amine HBr salt 29 (50 mg, 0.102 mmol) was suspended in 2 mL CH₃CN with stirring then cooled to 0°C. DMAP (25 mg, 0.205 mmol) was added, followed by Carbonate11. The reaction was stirred at 0°C for 1.5 hrs then allowed to warm to room temperature. The reaction was stirred overnight. A few drops Acetic acid were added to the reaction mixture, which was concentrated and re-diluted with ethyl acetate, shaken with 10% citric acid then saturated NaHCO₃. The organic layer was dried with brine and MgSO₄ and eluted on silica to afford di-phenol 30 (16 mg, 28%) ¹H NMR (CD₃OD): δ 7.61, (d, 2H), 7.01 (d, 2H), 6.87 (d, 2H), 6.62 (d, 2H), 5.55 (d, 1H), 4.93 (m, 1H), 3.92 (m, 2H), 3.79 (m, 5H), 3.35 (m, 1H), 3.07 (m, 2H), 2.88 (m, 3H), 2.41 (m, 1H), 2.00 (m, 1H), 1.54 (m, 1H), 1.31 (dd, 1H) 0.89-0.82 (dd, 6H).

### Example 23

A solution of di-phenol 30 (100 mg, 0.177 mmol) was made in CH₃CN that had been dried over K₂CO₃. To this, the triflate (0.084 mL, 0.23 mmol) was added, followed by Cs₂CO₃ (173 mg, 0.531 mmol). The reaction was stirred for 1 hr. TLC (5% IprOH/DCM) showed 2 spots with no starting materials left. Solvent was evaporated and the residue was partitioned between EtOAc and water. The organic layer was washed with saturated NaHCO₃, then dried with brine and MgSO₄. The mixture was separated by column chromatography on silica with 3% IprOH in DCM. The upper spot 31 (90 mg, 46%) was confirmed to be the *bis* alkylation product. The lower spot required further purification on silica gel plates to afford a single *mono* alkylation product 32 (37 mg, 26%). The other possible alkylation product was not observed. NMR : ¹H NMR (CDCl₃): *for 31*: δ 7.57 (d, 2H), 7.37 (m, 10H) 7.03 (d, 2H), 6.99 (d, 2H), 6.73 (d, 2H), 5.69 (d, 1H), 5.15-5.09 (m, 4H), 5.10 (m, 1H), 4.32 (d, 2H), 4.02 (d, 1H), 3.82 (m, 1H) 3.81 (m, 1H), 3.93-3.81 (m, 2H), 3.74 (d, 1H), 3.06 (m, 1H), 3.00 (m, 1H), 2.96 (m, 1H), 2.91 (m, 1H) 2.77 (m, 1H) 2.64 (m, 1H) 2.47 (m, 1H) 1.82 (m, 2H) 1.79 (m, 1H), 0.94-0.86 (dd, 6H) *for* 32: δ 7.68 (d, 2H), 7.33-7.35 (m, 20H), 7.11 (d, 2H), 6.96 (d, 2H), 6.80 (d, 2H), 5.26 (d, 1H), 5.11(m, 8H), 5.00 (m, 1H) 4.23 (d, 2H), 4.19 (d, 2H), 3.93 (m, 1H), 3.82-3.83 (m, 3H), 3.68-3.69 (m, 2H) 3.12-2.75 (m, 7H), 1.82 (m, 1H), 1.62-1.52 (d, 2H), 0.89-0.86 (dd, 6H).

### Example 24

Ref: J. Med. Chem. 1992, 35 10,1681-1701
To a solution of phosphonate 32 (100 mg, 0.119 mmol) in dry dioxane was added Cs₂CO₃ (233 mg, 0.715 mmol), followed by 2-(dimethylamino) ethyl chloride hydrochloride salt (69 mg, 0.48 mmol). The reaction was stirred at room temperature and monitored by TLC. When it was determined that starting material remained, additional Cs₂CO₃ (233 mg, 0.715 mmol) as well as amine salt (69 mg, 0.48 mmol) were added and the reaction was stirred overnight at 60°C. In the morning when TLC showed completion the reaction was cooled to room temperature, filtered, and concentrated. The product amine 33 (40 mg, 37%) was purified on silica. Decomposition was noted as lower spots were seen to emerge with time using 15% MeOH in DCM on silica.

### Example 25:

Amine 33 (19 mg, 0.021 mmol) was dissolved in 1.5 mL DCM. This solution was stirred in an icebath. Methane sulfonic acid (0.0015 mL, 0.023 mmol) was added and the reaction was stirred for 20 minutes. The reaction was warmed to room temperature and stirred for 1 hour. The product, amine mesylate salt 34 (20 mg, 95%) was precipitated out by addition of hexane. ¹H NMR (CD₃OD): δ 7.69 (d, 2H), 7.35 (m, 10M), 7.15 (m, 4H) 6.85 (m, 2H), 5.49 (d, 1H), 5.10 (m, 4H), 4.83 (m, 1H), 4.62 (d, 2H), 4.22 (m, 2H), 3.82 (m, 1H), 3.56 (m, 1H), 3.48 (m, 2H), 3.35 (m, 1H), 2.99 (m, 1H), 2.95 (m, 1H), 2.84 (s, 6H), 2.78 (m, 1H), 2.75 (m, 1H), 2.70 (m, 1H), 2.40 (m, 1H) 1.94 (m, 1H), 1,43 (m, 1H), 1.27 (m, 1H), 0.77 (dd, 6H).

### Example Section E

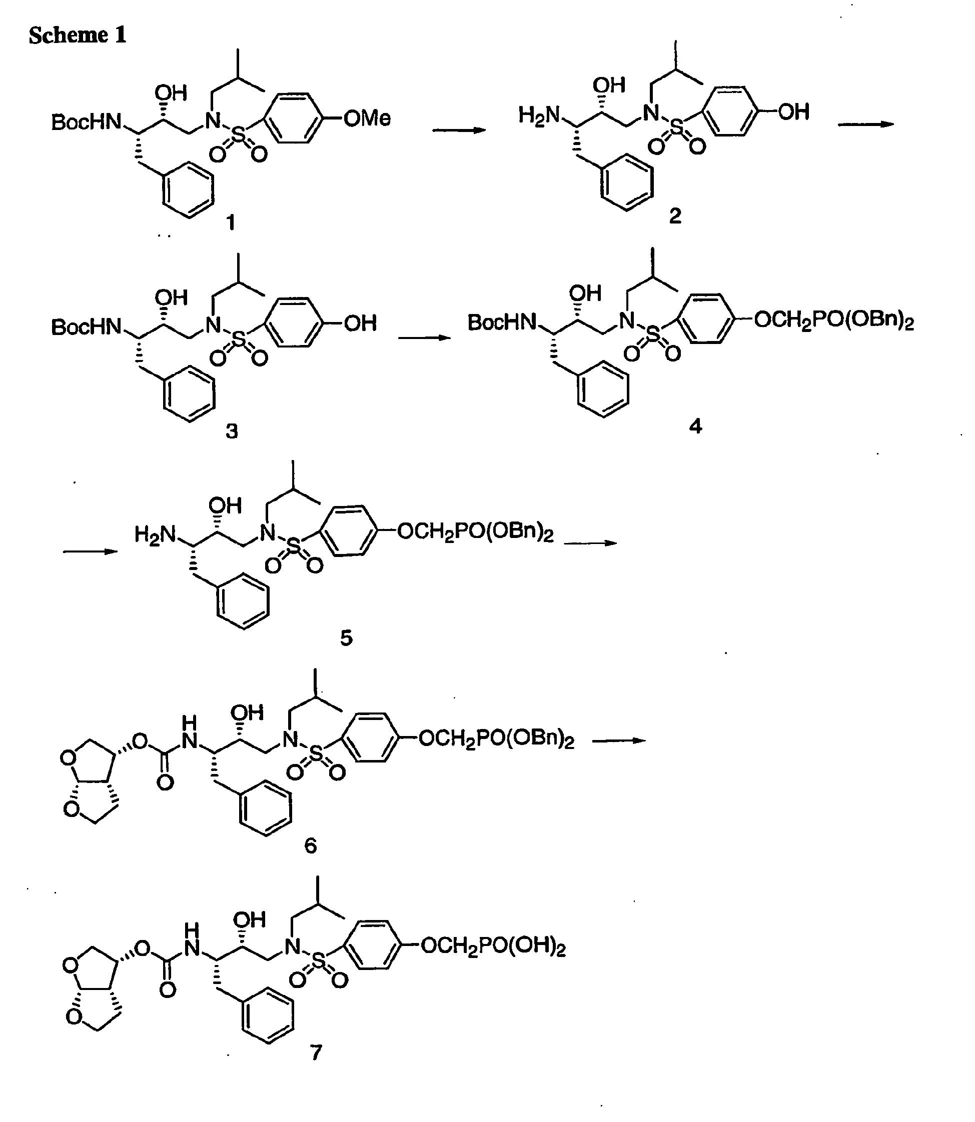

### Example 1

To a solution of phenol 3 (336 mg, 0.68 mmol) in THF (10 mL) was added Cs₂CO₃ (717 mg, 2.2 mmol) and triflate (636 mg, 1.5 mmol) in THF (3 mL). After the reaction mixture was stirred for 30 min at room temperature, the mixture was partitioned between EtOAc and water. The organic phase was dried over Na₂SO₄, filtered, and evaporated under reduced pressure. The crude product was chromatographed on silica gel (eluting 40-50% EtOAc/hexane) to give dibenzylphosphonate 4 (420 mg, 80%) as a colorless oiL

### Example 2

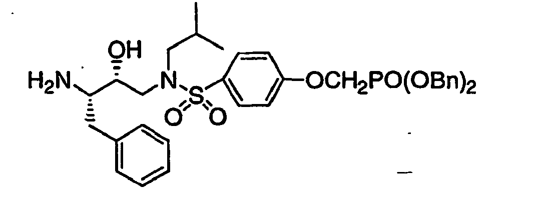

To a solution of dibenzylphosphonate 4 (420 mg, 0.548 mmol) in CH₂Cl₂ (10 mL) was added TFA (0.21 mL, 2.74 mmol). After the reaction mixture was stirred for 2 h at room temperature, additional TFA (0.84 mL, 11 mmol) was added and the mixture was stirred for 3 h. The reaction mixture was evaporated under reduced pressure and the residue was partitioned between EtOAc and 1M NaHCO₃. The organic phase was dried over Na₂SO₄, filtered, and evaporated under reduced pressure to give amine 5 (325 mg, 89%).

### Example 3

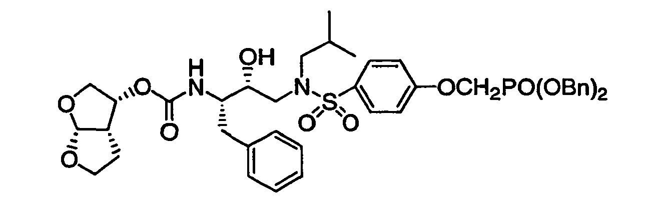

To a solution of carbonate (79 mg, 0.27 mmol), amine 5 (178 mg, 0.27 mmol), and CH₃CN (10 mL) was added DMAP (66 mg, 0.54 mmol) at 0°C. After the reaction mixture was warmed to room temperature and stirred for 16 hours, the mixture was concentrated under reduced pressure. The residue was chromatographed on silica gel (eluting 60-90% EtOAc/hexane) to give a mixture of carbamate 6 and starting carbonate. The mixture was further purified by HPLC on C18 reverse phase chromatography (eluting 60% CH₃CN/water) to give carbamate 6 (49 mg, 22%) as a colorless oil. ¹H NMR (300 MHz, CDCl₃) δ 7.68 (d; 2H), 7.22 (m, 15 H), 6.95 (d, 2H), 5.62 (d, 1H), 5.15 (dt, 4H), 5.00 (m, 2H), 4.21 (d, 2H), 3.88 (m, 4H), 3.67 (m, 3H), 3.15 (m, 2H), 2.98 (m, 3H), 2.80 (m, 2H), 1.82 (m, 1H), 1.61 (m, 1H), 0.93 (d, 3H), 0.88 (d, 3H).

### Example 4

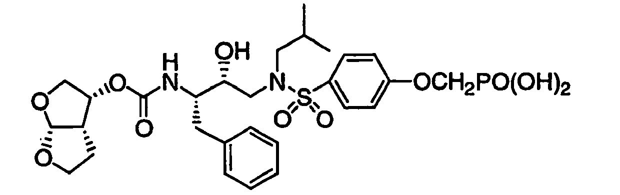

To a solution of carbamate **6** (21 mg, 0.026 mmol) in EtOH / EtOAc (2 mL/1 mL) was added 10% Pd/C (11 mg). After the reaction mixture was stirred under H₂ atmosphere (balloon) for 2 hours, the mixture was filtered through Celite. The filtrate was evaporated under reduced pressure to give phosphonic acid **7** (17 mg, 100%) as a colorless solid. ¹H NMR (300 MHz, CD₃0D) δ 7.73 (d, 2H), 7.19 (m, 5H), 7.13 (d, 2H), 5.53 (d, 1H), 4.26 (d, 2H), 3.86 (m, 1H), 3.64 (m, 5H), 3.38 (d, 1H), 3.13 (d, 1H), 3.03 (dd, 1H), 2.86 (m, 3H), 2.48 (m, 1H), 1.97 (m, 1H), 1.47 (m, 1H), 1.28 (m, 2H), 1.13 (t, 1H), 0.88 (d, 3H), 0.83 (d, 3H).

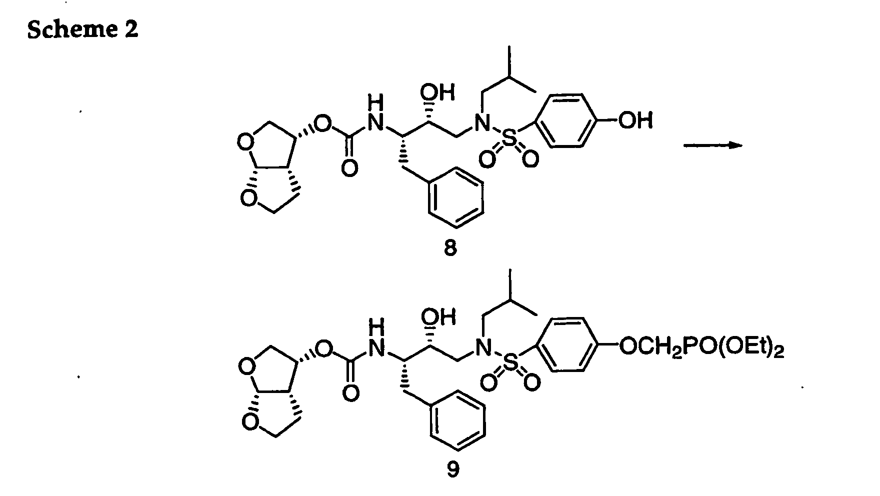

### Example 5

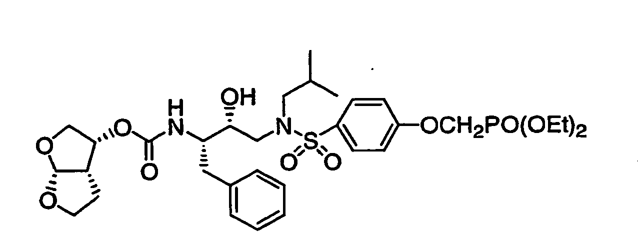

To a solution of phenol **8** (20 mg, 0.036 mmol) and triflate (22 mg, 0.073 mmol) in THF (2 mL) was added Cs₂CO₃ (29 mg, 0.090 mmol). After the reaction mixture was stirred for 30 min at room temperature, the mixture was partitioned between EtOAc and water. The organic phase was dried over Na₂SO₄, filtered, and evaporated under reduced pressure. The crude product was purified by preparative thin layer chromatography (eluting 80% EtOAc/hexane) to give diethylphosphonate **9** (21 mg, 83%) as a colorless oil. ¹H NMR (300 MHz, CDCl₃) δ 7.73 (d, 2H), 7.25 (m, 5H), 7.07 (d, 2H), 5.64 (d, 1H), 5.01 (m, 2H), 4.25 (m, 6H), 3.88 (m, 4H), 3.70 (m, 3H), 2.97 (m, 6H), 1.70 (m, 4H), 1.38 (t, 6H), 0.92 (d, 3H), 0.88 (d, 3H). ³¹P NMR (300 MHz, CDCl₃) δ 18.1.

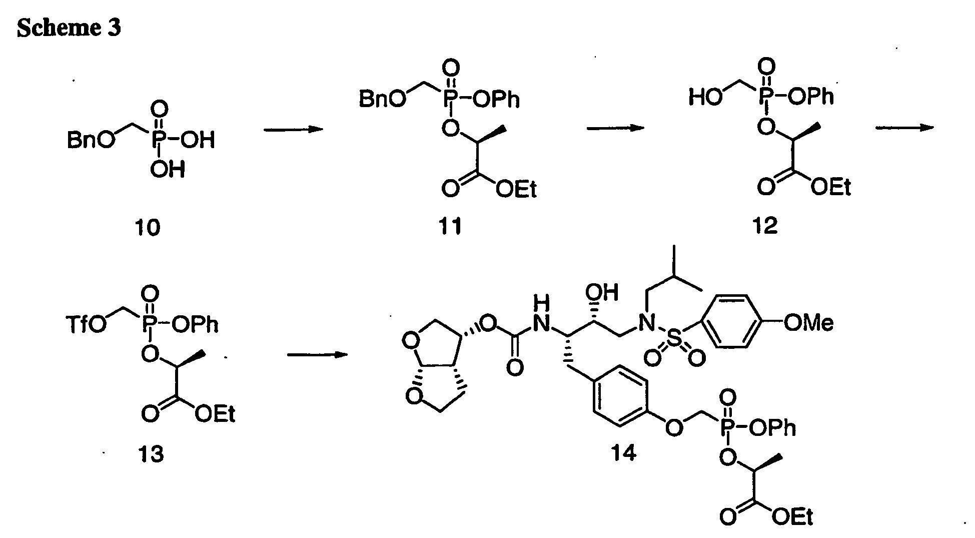

### Example 6

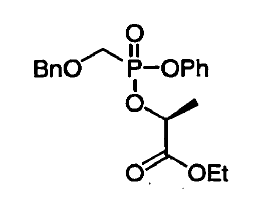

To a solution of phosphonic acid **10** (520 mg, 2.57 mmol) in CH₃CN (5 mL) was added thionyl chloride (0.75 mL, 10.3 mmol) and heated to 70°C in an oil bath. After the reaction mixture was stirred for 2 h at 70°C, the mixture was concentrated and azeotroped with toluene. To a solution of the crude chloridate in toluene (5 mL) was added tetrazole (18 mg, 0.26 mmol) at 0°C. To this mixture was added phenol (121 mg, 1.28 mmol) and triethylamine (0.18 mL, 1.28 mmol) in toluene (3 mL) at 0°C. After the reaction mixture was warmed to room temperature and stirred for 2 h, ethyl lactate (0.29 mL, 2.57 mmol) and triethylamine (0.36 mL, 2.57 mmol) in toluene (2.5 mL) were added. The reaction mixture was stirred for 16 hours at room temperature, at which time the mixture was partitioned between EtOAc and sat. NH₄Cl. The organic phase was washed with sat. NH₄Cl, 1M NaHCO₃, and brine, then dried over Na₂SO₄, filtered, and evaporated under reduced pressure. The crude product was chromatographed on silica gel (eluting 20-40% EtOAc/hexane) to give two diastereomers of phosphonate **11** (66 mg, 109 mg, 18% total) as colorless oils.

### Example 7A

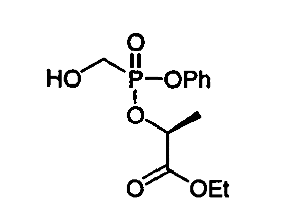

To a solution of phosphonate **11** isomer A (66 mg, 0.174 mmol) in EtOH (2 mL) was added 10% Pd/C (13 mg). After the reaction mixture was stirred under H₂ atmosphere (balloon) for 6 h, the mixture was filtered through Celite. The filtrate was evaporated under reduced pressure to give alcohol **12** isomer A (49 mg, 98%) as a colorless oil.

### Example 7B

To a solution of phosphonate **11** isomer B (110 mg, 0.291 mmol) in EtOH (3 mL) was added 10% Pd/C (22 mg). After the reaction mixture was stirred under H₂ atmosphere (balloon) for 6 h, it was filtered through Celite. The filtrate was evaporated under reduced pressure to give alcohol **12** isomer B (80 mg, 95%) as a colorless oil.

### Example 8A

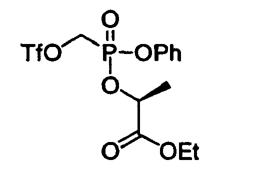

To a solution of alcohol **12** isomer A (48 mg, 0.167 mmol) in CH₂Cl₂ (2 mL) was added 2,6-lutidine (0.03 mL, 0.250 mmol) and trifluoromethanesulfonic anhydride (0.04 mL, 0.217 mmol) at -40°C (dry ice-CH₃CN bath). After the reaction mixture was stirred for 15 min at - 40°C, the mixture was warmed to 0°C and partitioned between Et₂O and 1M H₃PO₄. The organic phase was washed with 1M H₃PO₄ (3 times), dried over Na₂SO₄, filtered, and evaporated under reduced pressure to give triflate **13** isomer A (70 mg, 100%) as a pale yellow oil.

### Example 8B

To a solution of alcohol **12** isomer B (80 mg, 0.278 mmol) in CH₂Cl₂ (3 mL) was added 2,6-lutidine (0.05 mL, 0.417 mmol) and trifluoromethanesulfonic anhydride (0.06 mL, 0.361 mmol) at -40°C (dry ice-CH₃CN bath). After the reaction mixture was stirred for 15 min at - 40°C, the mixture was warmed to 0°C and partitioned between Et₂O and 1M H₃PO₄. The organic phase was washed with 1M H₃PO₄ (3 times), dried over Na₂SO₄, filtered, and evaporated under reduced pressure to give triflate **13** isomer B (115 mg, 98%) as a pale yellow oil.

### Example 9A

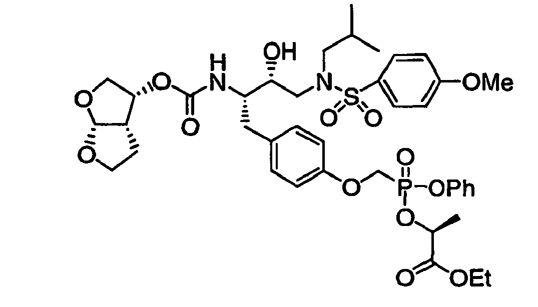

To a solution of phenol (64 mg, 0.111 mmol): 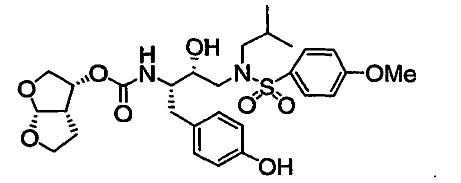 and triflate 13 isomer A (70 mg, 0.167 mmol) in THF (2 mL) was added Cs₂CO₃ (72 mg, 0.222 mmol). After the reaction mixture was stirred for 30 min at room temperature, the mixture was partitioned between EtOAc and water. The organic phase was dried over Na₂SO₄, filtered, and evaporated under reduced pressure. The crude product was chromatographed on silica gel (eluting 60-80% EtOAc/hexane) to give a mixture. The mixture was further purified by HPLC on C18 reverse phase chromatography (eluting 55% CH₃CN/water) to give phosphonate **14** isomer A (30 mg, 32%) as a colorless solid. ¹H NMR (300 MHz, CDCl₃) δ 7.71 (d, 2H), 7.26 (m, 6H), 7.00 (m, 5H), 5.65 (d, 1H), 5.14 (m, 1H), 5.00 (m, 2H), 4.54 (dd, 1H), 4.44 (dd, 1H), 4.17 (m, 2H), 3.96 (dd, 1H), 3.86 (m, 5H), 3.72 (m, 3H), 3.14 (m, 1H), 2.97 (m, 4H), 2.79 (m, 2H), 1.83 (m, 1H), 1.62 (m, .3H), 1.50 (d, 3H), 1.25 (m, 3H), 0.93 (d, 3H), 0.88 (d, 3H). ³¹P NMR (300 MHz, CDCl₃) δ 17.4.

### Example 9B

To a solution of phenol (106 mg, 0.183 mmol): 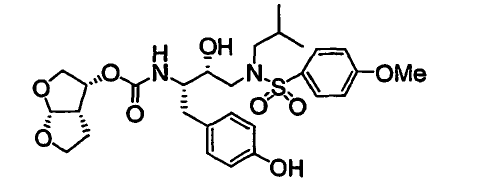 and triflate **13** isomer B (115 mg, 0.274 mmol) in THF (2 mL) was added Cs₂CO₃ (119 mg, 0.366 mmol). After the reaction mixture was stirred for 30 min at room temperature, the mixture was partitioned between EtOAc and water. The organic phase was dried over Na₂SO₄, filtered, and evaporated under reduced pressure. The crude product was chromatographed on silica gel (eluting 60-80% EtOAc/hexane) to give a mixture. The mixture was further purified by HPLC on C18 reverse phase chromatography (eluting 55% CH₃CN/water) to give phosphonate **14** isomer B (28 mg, 18%) as a colorless solid. ¹H NMR (300 MHz, CDCl₃) δ 7.71 (d, 2H), 7.26 (m, 6H), 6.94 (m, 5H), 5.66 (d, 1H), 5.17 (m, 1H), 4.99 (m, 2H), 4.55 (m, 1H), 4.42 (m, 1H), 4.16 (m, 2H), 3.97 (m, 1H), 3.85 (m, 5H), 3.72 (m, 3H), 3.13 (m, 1H), 2.97 (m, 4H), 2.80 (m, 2H), 1.83 (m, 1H), 1.60 (m, 6H), 1.22 (m, 3H), 0.93 (d, 3H), 0.88 (d, 3H). ³¹P NMR (300 MHz, CDCl₃) δ 15.3.

### Resolution of Compound 14 Diastereomers

Analysis was performed on an analytical Alltech Econosil column, conditions described below, with a total of about 0.5 mg 14 injected onto the column. This lot was a mixture of major and minor diastereomers where the lactate ester carbon is a mix of R and S configurations. Up to 2 mg could be resolved on the analytical column. Larger scale injections (up to 50 mg **14**) were performed on an Alltech Econosil semi-preparative column, conditions described below.

The isolated diastereomer fractions were stripped to dryness on a rotary evaporator under house vacuum, followed by a final high vacuum strip on a vacuum pump. The chromatographic solvents were displaced by two portions of dichloromethane before the final high vacuum strip to aid in removal of trace solvents, and to yield a friable foam.

The bulk of the diastereomer resolution was performed with *n*-heptane substituted for hexanes for safety considerations.

Sample Dissolution: While a fairly polar solvent mixture is described below, the sample may be dissolved in mobile phase with a minimal quantity of ethyl alcohol added to dissolve the sample.

### Analytical Column, 0.45 mg Injection, Hexanes - IPA (90:10)

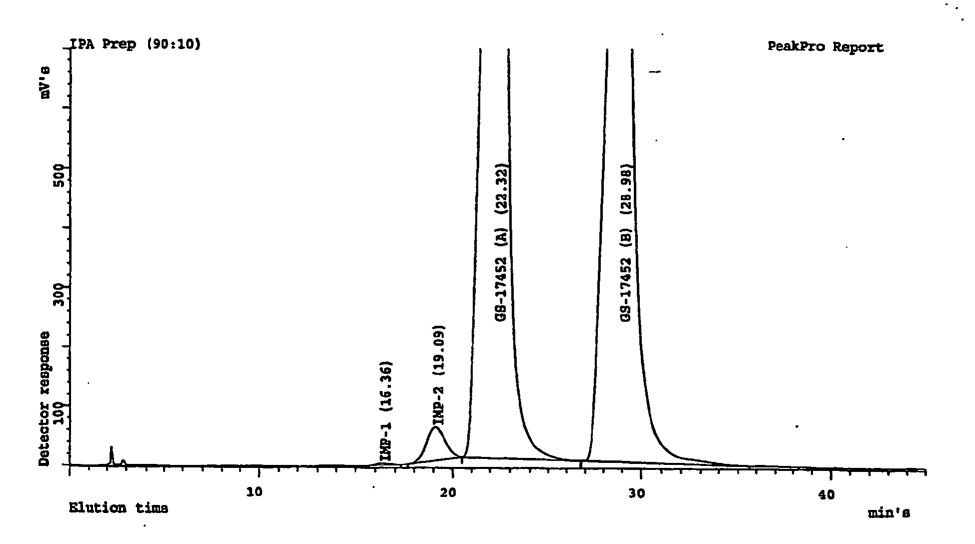

### HPLC CONDITIONS

- Column: : Alltech Econosil, 5 µm, 4.6 x 250 mm
- Mobile Phase: : Hexanes - Isopropyl Alcohol (90:10)
- Flow Rate: : 1.5 mL/min
- Run Time: : 50 min
- Detection: : UV at 242 nm
- Temperature: : Ambient
- Injection Size: : 100 µL
- Sample Prep.: : ~ 5 mg/mL, dissolved in hexanes - ethyl alcohol (75:25)
- Retention Times: : 14~22 min
: **14**~29 min
: Less Polar Impurity ~ 19 min

### Semi-Preparative Column, 50 mg Injection, n-Heptane - IPA (84:16)

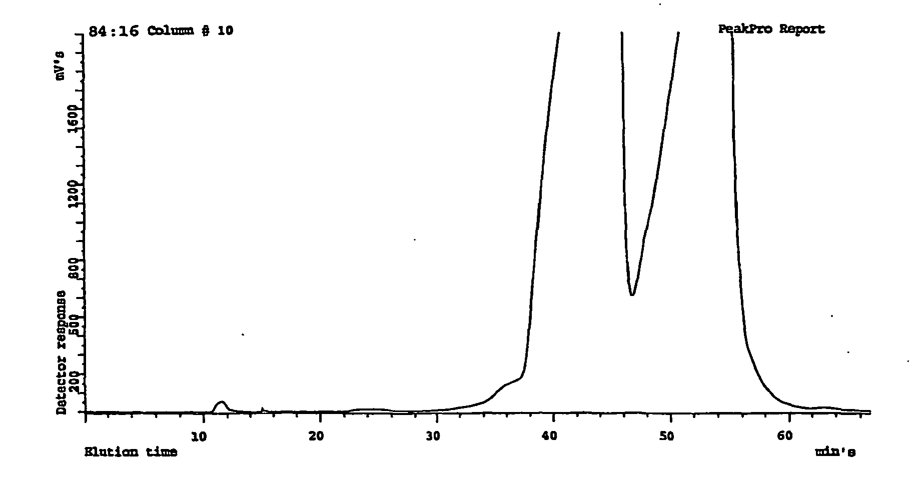

### HPLC CONDITIONS

- Column: : Alltech Econosil, 10 µm, 22 x 250 mm
- Mobile Phase: : *n*-Heptane - Isopropyl Alcohol (84:16)
- Flow Rate: : 10 mL/min
- Run Time: : 65 min
- Detection: : UV at 257 nm
- Temperature: : Ambient
- Injection Size: : ~50 mg
- Dissolution: : 2 mL mobile phase plus ~ 0.75 mL ethyl alcohol
- Retention Times: : **14** ~ 41 min
: **14** ~ 54 min
: Less Polar Impurity ~ Not resolved

### Example Section F

### Example 1

Phosphonic acid 2: To a solution of compound 1 (A. Flohr et al, J. Med. Chem., 42, 12, 1999; 2633-2640) (4.45 g, 17 mmol) in CH₂Cl₂ (50 mL) at room temperature was added bromotrimethylsilane (1.16 mL, 98.6 mmol). The solution was stirred for 19 h. The volatiles were evaporated under reduced pressure to give the oily phosphonic acid 2 (3.44 g, 100%). ¹H NMR (CDCl₃) δ 7.30 (m, 5H), 4.61 (s, 2 H), 3.69 (d, 2H).

### Example 2

Compound 3: To a solution of phosphonic acid 2 (0.67 g, 3.3 mmol) in CH₃CN (5 mL) was added thionyl chloride (1 mL, 13.7 mmol) and the solution was heated at 70°C for 2.5 h. The volatiles were evaporated under reduced pressure and dried in vacuo to afford an oily phophonyl dichloride. The crude chloride intermediate was dissolved in CH₂Cl₂ (20 mL) and cooled in an ice/water bath. Ethyl lactate (1.5 mL, 13.2 mmol) and triethyl amine (1.8 mL, 13.2 mmol) were added dropwise. The mixture was stirred for 4 h at room temperature and dilluted with more CH₂Cl₂ (100 mL). The organic solution was washed with 0.1N HCl, saturated aqueous NaHCO₃, and brine, dried (MgSO₄ filtered and evaporated under reduced pressure. The crude product was chromatographed on silica gel to afford oily compound 3 (0.548 g, 41%). ¹H NMR (CDCl₃) δ 7.30 (m, 5H), 5.00-5.20 (m, 2H), 4.65 (m, 2H), 4.20 (m, 4H), 3.90 (d, 2H), 1.52 (t, 6H), 1.20 (t, 6H).

### Example 3

Alcohol 4: A solution of compound 3 (0.54 g, 1.34 mmol) in EtOH (15 mL) was treated with 10% Pd/C (0.1 g) under H₂ (100 psi) for 4 h. The mixture was filtered and the filtrate was treated with fresh 10% PD/C (0.1 g) under H₂ (1 atmosphere) for 18 h. The mixture was filtered and the filtrate was evaporated to afford alcohol 4 (0.395 g, 94%) as an oil. ¹H NMR (CDCl₃) δ 4.90-5.17 (m, 2H), 4.65 (q, 2H), 4.22 (m, 4H), 4.01 (m, 2H), 1.55 (t, 6H), 1.21 (t, 6H); ³¹P NMR (CDCl₃) δ 22.8.

### Example 4

Triflate 5: To a solution of alcohol 4 (122.8 mg, 0.393 mmol) in CH₂Cl₂ (5 mL) at -40°C were added 2,6-lutidine (0.069 mL, 0.59 mmol) and trifluoromethansulfonic anhydride (0.086 mL, 0.51 mmol). Stirring was continued at 0°C for 2 h. and the mixture partitioned in CH₂Cl₂ and saturated NaHCO₃. The organic layer was washed with 0.1N HCl, saturated NaCl, dried (MgSO₄), filtered and evaporated under reduced pressure. The crude product 5 (150 mg, 87%) was used for the next step without further purification. ¹H NMR (CDCl₃) δ 5.0-5.20 (m, 2H), 4.93 (d, 2H), 4.22 (m, 4H), 1.59 (m, 6H), 1.29 (t, 6H).

### Example 5

Phosphonate 6: A solution of phenol 8 (see Scheme Section A, Scheme 1 and 2) (32 mg, 0.055 mmol) and triflate 5 (50 mg, 0.11 mmol) in THF (1.5 mL) at room temperature was treated with CS₂CO₃ (45.6 mg, 0.14 mmol). The mixture was stirred for 2.5 h and partitioned in EtOAc and saturated NaHCO₃. The organic layer was washed with 0.1N HCl, saturated NaCl, dried (MgSO₄), filtered and evaporated under reduced pressure. The crude product was purified by chromatography on silica gel (30-70% EtOAc/hexane) affording the phosphonate 6 (41 mg, 84%) as a solid. ¹H NMR (CDCl₃) δ 7.71 (d, 2H), 7.13 (d, 2H), 7.00 (d, 2H), 6.90 (d, 2H), 5.65 (d, 1H), 4.90-5.22 (m, 3H), 4.40 (m, 2H), 4.20 (m, 4H), 3.90 (s, 3H), 3.65-4.00 (m, 5H), 2.70-3.20 (m, 6H), 1.52-1.87 (m, 12H), 1.25 (m, 6H), 0.85-0.90 (m, 6H); ³¹P NMR (CDCl₃) δ 20.0.

### Example 6

Compound 7: To a solution of phosphonic acid 2 (0.48 g, 2.37 mmol) in CH₃CN (4 mL) was added thionyl chloride (0.65 mL, 9.48 mmol) and the solution was heated at 70°C for 2.5 h. The volatiles were evaporated under reduced pressure and dried in vacuo to afford an oily phophonyl dichloride. The crude chloride intermediate was dissolved in CH₂Cl₂ (5 mL) and cooled in an ice/water bath. Ethyl glycolate (0.9 mL, 9.5 mmol) and triethyl amine (1.3 mL, 9.5 mmol) were added dropwise. The mixture was stirred for 2 h at room temperature and dilluted with more CH₂Cl₂ (100 mL). The organic solution was washed with 0.1N HCl, saturated aqueous NaHCO₃, and saturated NaCl, dried (MgSO₄) filtered and concentrated under reduced pressure. The crude product was chromatographed on silica gel to afford oily compound 7 (0.223 g, 27%). ¹H NMR (CDCl₃) δ 7.30 (m, 5H), 4.65 (m, 6H), 4.25 (q, 4H), 3.96 (d, 2H), 1.27 (t, 6H); ³¹P NMR (CDCl₃) δ 24.0.

### Example 7

Alcohol 8: A solution of compound 7 (0.22 g, 0.65 mmol) in EtOH (8 mL) was treated with 10% Pd/C (0.04 g) under H₂ (1 atmosphere) for 4 h. The mixture was filtered and the filtrate was evaporated to afford alcohol 8 (0.156 g, 96%) as an oil. ¹H NMR (CDCl₃) δ 4.66 (m, 4H), 4.23 (q, 4H), 4.06 (d, 2H), 1.55 (t, 6H), 1.26 (t, 6H); ³¹P NMR (CDCl₃) δ 26.8.

### Example 8

Triflate 9: To a solution of alcohol 8 (156 mg, 0.62 mmol) in CH₂Cl₂ (5 mL) at -40°C were added 2,6-lutidine (0.11 mL, 0.93 mmol) and trifluoromethansulfonic anhydride (0.136 mL, 0.8 mmol). Stirring was continued at 0°C for 2 h. and the mixture partitioned in CH₂Cl₂ and saturated NaHCO₃. The organic layer was washed with 0.1N HCl, saturated NaCl, dried (MgSO₄), filtered and evaporated under reduced pressure. The crude product 9 (210 mg, 88%) was used for the next step without further purification. ¹H NMR (CDCl₃) δ 4.90 (d, 2H), 4.76 (d, 4H), 4.27 (q, 4H), 1.30 (t, 6H).

### Example 9

Phosphonate 10: A solution of phenol 8 (30 mg, 0.052 mmol) and triflate 9 (30 mg, 0.078 mmol) in THF (1.5 mL) at room temperature was treated with C_{S2}CO₃ (34 mg, 0.1 mmol). The mixture was stirred for 2.5 h and partitioned in EtOAc and saturated NaHCO₃. The organic layer was washed with 0.1N HCl, saturated NaCl, dried (MgSO₄) filtered and evaporated under reduced pressure. The crude product was purified by chromatography on silica gel (30-70% EtOAc/hexane) affording the unreacted phenol (xx) (12 mg, 40%) and the phosphonate 10 (16.6 mg, 38%) as a solid. ¹H NMR (CDCl₃) δ 7.71 (d, 2H), 7.13 (d, 2H), 7.00 (d, 2H), 6.90 (d, 2H), 5.65 (d, 1H), 5.00 (m, 2H), 4.75 (m, 4H), 4.48 (d, 2H), 4.23 (q, 4H), 3.90 (s, 3H), 3.65-4.00 (m, 5H), 2.70-3.20 (m, 6H), 2.23 (b.s., 2H), 1.52-1.87 (m, 4H), 1.25 (t, 6H), 0.85-0.90 (m, 6H); ³¹P NMR (CDCl₃) δ 22.0.

### Example 10

Compound 11: To a solution of phosphonic acid 2 (0.512 g, 2.533 mmol) in CH₃CN (5 mL) was added thionyl chloride (0.74 mL, 10 mmol) and the solution was heated at 70°C for 2.5 h. The volatiles were evaporated under reduced pressure and dried in vacuo to afford an oily phophonyl dichloride. The crude chloride intermediate was dissolved in toluene (8 mL) and cooled in an ice/water bath. A catalytic amount of tetrazol (16 mg, 0.21 mmol) was added followed by the addition of a solution of triethylamine (0.35 mL, 2.53 mmol) and phenol (238 mg, 2.53 mmol) in toluene (5 mL). The mixture was stirred at room temperature for 3 h. A solution of ethyl glycolate (0.36 mL, 3.8 mmol) and triethyl amine (0.53 mL, 3.8 mmol) in toluent (3 mL) was added dropwise. The mixture was stirred for 18 h at room temperature and partitioned in EtOAc and 0.1N HCL The organic solution was washed with saturated aqueous NaHCO₃, and saturated NaCl, dried (MgSO₄) filtered and concentrated under reduced pressure. The crude product was chromatographed on silica gel to afford diphenyl phophonate as a byproduct (130 mg) and compound 11 (0.16 g, 18%). ¹H NMR (CDCl₃) δ 7.15-7.40 (m, 10H), 4.58-4.83 (m, 4H), 4.22 (q, 2H), 4.04 (dd, 2H), 1.24 (t, 3H).

### Example 11

Alcohol 12: A solution of compound 11 (0.16 g, 0.44 mmol) in EtOH (5 mL) was treated with 10% Pd/C (0.036 g) under H₂ (1 atmosphere) for 22 h. The mixture was filtered and the filtrate was evaporated to afford alcohol 12 (0.112 g, 93%) as an oil. ¹H NMR (CDCl₃) δ 7.15-7.36 (m, 5H), 4.81 (dd, 1H), 4.55 (dd, 1H), 4.22 (q, 2H), 4.12 (m, 2H), 3.78 (b.s., 1H), 1.26 (t, 6H) ; ³¹P NMR (CDCl₃) δ 22.9

### Example 12

Triflate 13: To a solution of alcohol 12 (112 mg, 0.41 mmol) in CH₂Cl₂ (5 mL) at -40°C were added 2,6-lutidine (0.072 mL, 0.62 mmol) and triffuoromethansulfonic anhydride (0.09 mL, 0.53 mmol). Stirring was continued at 0°C for 3 h. and the mixture partitioned in CH₂Cl₂ and saturated NaHCO₃. The organic layer was washed with O.1N HCl, saturated NaCl, dried (MgSO₄), filtered and evaporated under reduced pressure. The crude product was purified by chromatography on silica gel (30% EtOAc/hexane) affording triflate 13 (106 mg, 64%). ¹H NMR (CDCl₃) δ 7.36 (m, 2H), 7.25 (m, 3H), 4.80-5.10-(m, 3H), 4.60 (dd, 1H), 4.27 (q, 2H), 1.28 (t, 3H); ³¹P NMR (CDCl₃) δ 11.1

### Example 13

Phosphonate 14: A solution of phenol 8 (32 mg, 0.052 mmol) and triflate 13 (32 mg, 0.079 mmol) in CH₃CN (1.5 mL) at room temperature was treated with Cs₂CO₃ (34 mg, 0.1 mmol). The mixture was stirred for 1 h and partitioned in EtOAc and saturated NaHCO₃. The organic layer was washed with saturated NaCl, dried (MgSO₄), filtered and evaporated under reduced pressure. The crude product was purified by chromatography on silica gel (70% EtOAc/hexane) affording phosphonate 14 (18 mg, 40%). ¹H NMR (CDCl₃) δ 7.71 (d, 2H), 6.75-7.35 (m, 11H, 5.65 (d, 1H), 5.00 (m, 2H); 4.50-4.88 (m, 3H), 4.20 (q, 2H), 3.84 (s, 3H), 3.65-4.00 (m, 5H), 2.70-3.20 (m, 6H), 1.52-1.87 (m, 6H), 1.25 (t, 3H), 0.85-0.90 (m, 6H); ³¹P NMR (CDCl₃) δ 17.9, 17.7.

### Example 14

Piperidine 16: A solution of compound 15 (3.1 g, 3.673 mmol) in MeOH (100 mL) was treated with 10% Pd/C (0.35 g) under H₂ (1 atmosphere) for 18 h. The mixture was filtered and the filtrate was evaporated to afford phenol 16 (2 g, 88%). ¹H NMR (CD₃OD) δ 7.76 (d, 2H), 7.08 (d, 2H), 7.04 (d, 2H), 6.65 (d, 2H), 5.59 (d, 1H), 4.95 (m, 1H), 3.98 (s, 3H), 3.65-4.00 (m, 5H), 3.30-3.50 (m, 3H), 2.80-3.26 (m, 5H), 2.40-2.70 (m, 3H), 1.35-2.00 (m, 7H), 1.16 (m, 2H); MS (ESI) 620 (M+H).

### Example 15

Formamide 17: Piperidine 16 obtained above (193 mg, 0.3118 mmol) in DMF (4 mL) was treated with formic acid (0.035 mL, 0.936 mmol), triethylamine (0.173 mL, 1.25 mmol) and EDCI (179 mg, 0.936 mmol) at room temperature. The mixture was stirred for 18 h and partitioned in EtOAc and saturated NaHCO₃. The organic layer was washed with saturated NaCl, dried (MgSO₄) filtered and evaporated under reduced pressure. The crude product was purified by chromatography on silica gel (EtOAC/hexane) affording formamide 17 (162 mg, 80%). ¹H NMR (CDCl₃) δ 7.96 (s, 1H), 7.68 (d, 2H), 7.04 (d, 2H), 6.97 (d, 2H), 6.76 (d, 2H), 5.63 (d, 1H), 5.37 (bs, 1H), 5.04 (m, 1H), 4.36 (m, 1H), 3.93 (s, 3H), 3.52-3.95 (m, 7H), 2.70-3.20 (m, 8H), 1.48-2.00 (m, 7H), 1.02 (m, 2H).

### Example 16

Dibenzyl phosphonate 18: A solution of phenol 17 (123 mg, 0.19 mmol) and dibenzyl trifluoromethansulfonyloxymethanphosphonate YY (120 mg, 0.28 mmol) in CH₃CN (1.5 mL) at room temperature was treated Cs₂CO₃ (124 mg, 0.38 mmol). The mixture was stirred for 3 h and partitioned in CH₂Cl₂ and saturated NaHCO₃. The organic layer was washed with 0.1N HCl, saturated NaCl, dried (MgSO₄), filtered and evaporated under reduced pressure. The crude product was purified by chromatography on silica gel (10% MeOH/CH₂Cl₂) affording phosphonate 18 (154 mg, 88%). ¹H NMR (CDCl₃) δ 7.96 (s, 1H), 7.68 (d, 2H), 7.35 (m, 10H), 7.10 (d, 2H), 6.97 (d, 2H), 6.80 (d, 2H), 5.63 (d, 1H), 4.96-5.24 (m, 6H), 4.37 (m, 1H), 4.20 (d, 2H), 3.84 (s, 3H), 3.52-3.95 (m, 7H), 2.55-3.20 (m, 8H), 1.48-2.00 (m, 7H), 1.02 (m, 2H). ³¹P NMR (CDCl₃) δ 20.3.

### Example 17

Phosphonic acid 19: A solution of phosphonate 18 (24 mg, 0.026 mmol) in MeOH (3 mL) was treated with 10% Pd/C (5 mg) under H₂ (1 atmosphere) for 4 h. The mixture was filtered and the filtrate was evaporated to afford phosphonic acid 19 as a solid (18 mg, 93%). ¹H NMR (CD₃OD) δ 8.00 (s, 1H), 7.67 (d, 2H), 7.18 (d, 2H), 7.09 (d, 2H), 6.90 (d, 2H), 5.60 (d, 1H), 4.30 (m, 1H), 4.16 (d, 2H), 3.88 (s, 3H), 3.60-4.00 (m, 7H), 3.04-3.58 (m, 5H), 2.44-2.92 (m, 5H), 1.28-2.15 (m, 5H), 1.08 (m, 2H). ³¹P NMR (CDCl₃) δ 16.3.

### Example 18

Diethyl phosphonate 20: A solution of phenol 17 (66 mg, 0.1 mmol) and diethyl triffuoromethansulfonyloxymethanphosphonate XY (46 mg, 0.15mmol) in CH₃CN (1.5 mL) at room temperature was treated Cs₂CO₃ (66 mg, 0.2 mmol). The mixture was stirred for 3 h and partitioned in CH₂Cl₂ and saturated NaHCO₃. The organic layer was washed with O.1N HCl, saturated NaCl, dried (MgSO₄) filtered and evaporated under reduced pressure. The crude product was purified by chromatography on silica gel (10% MeOH%CH₂Cl₂) affording the unreacted 17 (17 mg, 26%) and diethyl phosphonate 20 (24.5 mg, 41%). ¹H NMR (CDCl₃) δ 8.00 (s, 1H), 7.70 (d, 2H), 7.16 (d, 2H), 7.00(d, 2H), 6.88 (d, 2H), 5.66 (d, 1H), 4.98-5.10 (m, 2H), 4.39 (m, 1H), 4.24 (m, 5H), 3.89 (s, 3H), 3.602-3.98 (m, 7H), 2.55-3.16 (m, 8H), 1.50-2.00 (m, 7H), 1.36 (t, 6H), 1.08 (m, 2H). ³¹P NMR (CDCl₃) δ 19.2

### Example 19

N-methyl pepiridine diethyl phosphonate 21: A solution of compound 20 (22.2 mg, 0.0278 mmol) in THF (1.5 mL) at 0°C was treated with a solution of borane in THF (1M, 0.083 mL). The mixture was stirred for 2 h at room temperature and the starting material was consumed completely as monitored by TLC. The reaction mixture was cooled in an ice/water bath and excess methanol (1 mL) was added to quench the reaction. The solution was concentrated in vacuo and the crude product was chromatographed on silica gel with MeOH/EtOAc to afford compound 21 (7 mg, 32%). ¹H NMR (CDCl₃) δ 7.70 (d, 2H), 7.16 (d, 2H), 7.00(d, 2H), 6.88 (d, 2H), 5.66 (d, 1H), 4.98-5.10 (m, 2H), 4.24 (m, 4H), 3.89 (s, 3H), 3.602-3.98 (m, 7H), 2.62-3.15 (m, 9H), 2.26 (s, 3H), 1.52-2.15 (m, 10H), 1.36 (t, 6H). ³¹P NMR (CDCl₃) δ 19.3

### Example Section G

### Example 1

Compound 1: To a solution of 4-nitrobenzyl bromide (21.6 g, 100 mmol) in toluene (100 mL) was added triethyl phosphite (17.15 mL, 100 mL). The mixture was heated at 120°C for 14 hrs. The evaporation under reduced pressure gave a brown oil, which was purified by flash column chromatography (hexane/EtOAc= 2/1 to 100 % EtOAc) to afford compound 1.

### Example 2

Compound 2: To a solution of compound 1 (1.0 g) in ethanol (60 mL) was added 10% Pd-C (300 mg). The mixture was hydrogenated for 14 hrs. Celite was added and the mixture was stirred for 5 mins. The mixture was filtered through a pad of celite, and.washed with ethanol. Concentration gave compound 2.

### Example 3

Compound 3: To a solution of compound 3 (292 mg, 1.2 mmol) and aldehyde (111 mg, 0.2 mmol) in methanol (3 mL) was added acetic acid (48 µL, 0.8 mmol). The mixture was stirred for 5 mins, and sodium cyanoborohydride (25 mg, 0.4 mmol) was added. The mixture was stirred for 14 hrs, and methanol was removed under reduced pressure. Water was added, and was extracted with EtOAc. The organic phase was washed 0.5 N NaOH solution (1x), water (2x), and brine (1x), and was dried over MgSO₄. Purification by flash column chromatography (CH₂Cl₂/MeOH = 100/3) gave compound 3.

### Example 4

Compound 4: To a solution of compound 3 (79 mg , 0.1 mmol) in CH₂C1₂ (5 mL) was added trifluoroacetic acid (1 mL). The mixture was stirred for 2 hrs, and solvents were evaporated under reduced pressure. Coevaporation with EtOAc and CH₂Cl₂ gave an oil. The oil was dissolved in THF (1mL) and tetrabutylamonium fluoride (0.9 mL, 0.9 mmol) was added. The mixture was stirred for 1 hr, and solvent was removed. Purification by flash column chromotogaphy (CH₂Cl₂/MeOH = 100/7) gave compound 4.

### Example 5

Compound 5: To a solution of compound 4 (0.1 mmol) in acetonitrile (1 mL) at 0°C was added DMAP (22 mg, 0.18 mmol), followed by bisfurancarbonate (27 mg, 0.09 mmol). The mixture was stirred for 3 hrs at 0°C, and diluted with EtOAc. The organic phase was washed with 0.5 N NaOH solution (2x), water (2x), and brine (1x), and dried over MgSO₄. Purification by flash column chromotography (CH₂Cl₂/MeOH = 100/3 to 100/5) afford compound 5 (50 mg): ¹H NMR (CDCl₃) δ 7.70 (2 H, d, J = 8.9 Hz), 7.11 (2 H, d, J = 8.5 Hz), 6.98 (2 H, d, J = 8.9 Hz), 6.61 (2 H, d, J = 8.5 Hz), 5.71 (1 H, d, J = 5.2 Hz), 5.45 (1 H, m), 5.13 (1 H, m), 4.0 (6 H, m), 3.98-3.70 (4 H, m), 3.86 (3 H, s), 3.38 (2 H, m), 3.22 (1 H, m), 3.02 (5 H, m), 2.8 (1 H, m), 2.0-1.8 (3 H, m), 1.26 (6 H, t, J = 7.0 Hz), 0.95 (3 H, d, J = 6.7 Hz), 0.89 (3 H, d, J = 6.7 Hz).

### Example 6

Compound 6: To a solution of compound 5 (30 mg, 0.04 mmol) in MeOH (0.8 mL) was added 37% fomaldehyde (30 µL, 0.4 mmol), followed by acetic acid (23 µL, 0.4 mmol). The mixture was stirred for 5 mins, and sodium cyanoborohydride (25 mg, 0.4 mmol) was added. The reaction mixture was stirred for 14 hrs, and diluted with EtOAc. The organic phase was washed 0.5 N NaOH solution (2x), water (2x), and brine, and dried over MgSO₄. Purification by flash column chromatography (CH₂Cl₂/MeOH = 100/3) gave compound 6 (11 mg): ¹H NMR (CDCl₃) δ 7.60 (2 H, d, J = 8.9 Hz), 7.17 (2 H, m), 6.95 (2 H, d, J = 8.9 Hz), 6.77 (2 H, d, J = 8.5 Hz), 5.68 (1 H, d, J = 5.2 Hz), 5.21 (1 H, m), 5.09 (1 H, m), 4.01 (6 H, m), 3.87 (3 H, s), 3.8-3.3 (4 H, m), 3.1-2.6 (7 H, m), 2.90 (3 H, s), 1.8 (3 H, m), 1.25 (6 H, m), 0.91 (6 H, m).

### Example 7

Compound 7: To a solution of compound 1 (24.6 g, 89.8 mmol) in acetonitrile (500 mL) was added TMSBr (36 mL, 269 mmol). The reaction mixture was stirred for 14 hrs, and evaporated under reduced pressure. The mixture was coevaporated with MeOH (2x), toluene (2x), EtOAc (2x), and CH₂Cl₂ to give a yellow solid (20 g). To the suspension of above yellow solid (15.8 g, 72.5 mmol) in toluene (140 mL) was added DMF (1.9 mL), followed by thionyl chloride (53 mL, 725 mmol). The reaction mixture was heated at 60°C for 5 hrs, and evaporated under reduced pressure. The mixture was coevaporated with toluene (2x), EtOAc, and CH₂Cl₂ (2x) to afford a brown solid. To the solution of the brown solid in CH₂Cl₂ at 0°C was added benzyl alcohol (29 mL, 290 mmol), followed by slow addition of pyridine (35 mL, 435 mmol). The reaction mixture was allowed to warm to 25°C and stirred for 14 hrs. Solvents were removed under reduced pressure. The mixture was diluted with EtOAc, and washed with water (3x) and brine (1x), and dried over MgSO₄. Concentration gave a dark oil, which was purified by flash column chromatography (hexanes/EtOAc = 2/1 to 1/1) to afford compound 7.

### Example 8

Compound 8: To a solution of compound 7 (15.3 g) in acetic acid (190 mL) was added Zinc dust (20 g). The mixture was stirred for 14 hrs, and celite was added. The suspension was filtered through a pad of celite, and washed with EtOAc. The solution was concentrated under reduced pressure to dryness. The mixture was diluted with EtOAc, and was washed with 2N NaOH (2x), water (2x), and brine (1x), and dried over MgSO₄. Concentration under reduced pressure gave compound 8 as an oil (15 g).

### Example 9

Compound 9: To a solution of compound 8 (13.5 g, 36.8 mmol) and aldehyde (3.9 g, 7.0 mmol) in methanol (105 mL) was added acetic acid (1.68 mL, 28 mmol). The mixture was stirred for 5 mins, and sodium cyanoborohydride (882 mg, 14 mmol) was added. The mixture was stirred for 14 hrs, and methanol was removed under reduced pressure. Water was added, and was extracted with EtOAc. The organic phase was washed 0.5 N NaOH solution (1x), water (2x), and brine (1x), and was dried over MgSO₄. Purification by flash column chromatography (CH₂Cl₂/MeOH = 100/3) gave compound 9 (6.0 g).

### Example 10

Compound 10: To a solution of compound 9 (6.2 g , 6.8 mmol) in CH₂Cl₂ (100 mL) was added trifluoroacetic acid (20 mL). The mixture was stirred for 2 hrs, and solvents were evaporated under reduced pressure. Coevaporation with EtOAc and CH₂Cl₂ gave an oil. The oil was dissolved in THF (1mL) and tetrabutylamonium fluoride (0.9 mL, 0.9 mmol) was added. The mixture was stirred for 1 hr, and solvent was removed. Purification by flash column chromotogaphy (CH₂Cl₂/MeOH = 100/7) gave compound 10.

### Example 11

Compound 11: To a solution of compound 10 (5.6 mmol) in acetonitrile (60 mL) at 0°C was added DMAP (1.36g, 11.1 mmol), followed by bisfurancarbonate (1.65 g, 5.6 mmol). The mixture was stirred for 3 hrs at 0°C, and diluted with EtOAc. The organic phase was washed with 0.5 N NaOH solution (2x), water (2x), and brine (1x), and dried over MgSO₄.

Purification by flash column chromotography (CH₂Cl_{2/}MeOH = 100/3 to 100/5) afford compound 11 (3.6 g): ¹H NMR (CDCl₃) δ 7.70 (2 H, d, J = 8.9 Hz), 7.30 (10 H, m), 7.07 (2 H, m), 6.97 (2 H, d, J = 8.9 Hz), 6.58 (2 H, d, J = 8.2 Hz), 5.70 (1 H, d, J = 5.2 Hz), 5.42 (1 H, m), 5.12 (1 H, m), 4.91 (4 H, m), 4.0-3.7 (6 H, m), 3.85 (3 H, s), 3.4 (2 H, m), 3.25 (1 H, m), 3.06 (2 H, d, J = 21 Hz), 3.0 (3 H, m), 2.8 (1 H, m), 1.95 (1 H, m), 1.82 (2 H, m), 0.91 (6 H, m).

### Example 12

Compound 12: To a solution of compound 11 (3.6 g) in ethanol (175 mL) was added 10% Pd-C (1.5 g). The reaction mixture was hydrogenated for 14 hrs. The mixture was stirred with celite for 5 mins, and filtered through a pad of celite. Concentration under reduced pressure gave compound 12 as a white solid (2.8 g): ¹H NMR (DMSO-d₆) δ 7.68 (2 H, m), 7.08 (2 H, m), 6.93 (2 H, m), 6.48 (2 H, m), 5.95 (1 H, m), 5.0 (2 H, m), 3.9-3.6 (6 H, m), 3.82 (3 H, s), 3.25 (3 H, m), 3.05 (4 H, m), 2.72 (2 H, d, J = 20.1 Hz), 2.0-1.6 (3 H, m), 0.81 (6 H, m).

### Example 13

Compound 13: Compound 12 (2.6 g, 3.9 mmol) and L-alanine ethyl ester hydrochloride (3.575 g, 23 mmol) were coevaporated with pyridine (2x). The mixture was dissolved in pyridine (20 mL) and diisopropylethylamine (4.1 mL, 23 mmol) was added. To above mixture was added a solution of Aldrithiol (3.46 g, 15.6 mmol) and triphenylphosphine (4.08 g, 15.6 g) in pyridine (20 mL). The reaction mixture was stirred for 20 hrs, and solvents were evaporated under reduced pressure. The mixture was diluted with ethyl acetate, and was washed with 0.5 N NaOH solution (2x), water (2x), and brine, and dried over MgSO₄. Concentration under reduced pressure gave a yellow oil, which was purified by flash column chromatography (CH₂Cl₂/MeOH = 100/5 to 100/10) to afford compound 13 (750 mg): ¹H NMR (CDCl₃) δ 7.71 (2 H, d, J = 8.8 Hz), 7.13 (2 H, m), 6.98 (2 H, d, J = 8.8 Hz), 6.61 (2 H, d, J = 8.0 Hz), 5.71 (1 H, d, J = 5.2 Hz), 5.54 (1 H, m), 5.16 (1 H, m), 4.15 (6 H, m), 4.1-3.6 (6 H, m), 3.86 (3 H, s), 3.4-3.2 (3 H, m), 3.1-2.8 (8 H, m), 2.0 (1 H, m), 1.82 (2 H, m), 1.3 (12 H, m), 0.92 (6 H, m).

### Example 14

Compound 14: To a solution of 4-hydroxypiperidine (19.5 g, 193 mmol) in THF at 0°C was added sodium hydroxide solution (160 mL, 8.10 g, 203 mmol), followed by di-tert-butyl dicarbonate (42.1 g, 193 mmol). The mixture was warmed to 25°C, and stirred for 12 hours. THF was removed under reduced pressure, and the aqueous phase was extracted with EtOAc (2x). The combined organic layer was washed with water (2x) and brine, and dried over MgSO4. Concentration gave a compound 14 as a white solid (35 g).

### Example 15

Compound 15: To a solution of alcohol 14 (5.25 g, 25 mmol) in THF (100 mL) was added sodium hydride (1.2 g, 30 mmol, 60%). The suspension was stirred for 30 mins, and chloromethyl methyl sulfide (2.3 mL, 27.5 mmol) was added. Starting material alcohol 14 still existed after 12 hrs. Dimethy sulfoxide (50 mL) and additional chloromethyl methyl sulfide (2.3 mL, 27.5 mmol) were added. The mixture was stirred for additional 3 hrs, and THF was removed under reduced pressure. The reaction was quenched with water, and extracted with ethyl acetate. The organic phase was washed with water and brine, and was dried over MgSO₄. Purification by flash column chromatography (hexanes/EtOAc = 8/1) gave compound 15 (1.24 g).

### Example 16

Compound 16: To a solution of compound 15 (693 mg, 2.7 mmol) in CH₂Cl₂ (50 mL) at - 78°C was added a solution of sulfuryl chloride (214 µL, 2.7 mmol) in CH₂Cl₂ (5 mL). The reaction mixture was kept at -78°C for 3 hrs, and solvents were removed to give a white solid. The white solid was dissolved in toluene (7 mL), and triethyl phosphite (4.5 mL, 26.6 mmol) was added. The reaction mixture was heated at 120°C for 12 hrs. Solvent and excess reagent was removed under reduced pressure to give compound 16.

### Example 17

Compound 17: To a solution of compound 17 (600 mg) in CH₂Cl₂ (10 mL) was added trifluoroacetic acid (2 mL). The mixture was stirred for 2 hrs, and was concentrated under reduced pressure to give an oil. The oil was diluted with methylene chloride and base resin was added. The suspension was filtered and the organic phase was concentrated to give compound 17.

### Example 18

Compound 18: To a solution of compound 17 (350 mg, 1.4 mmol) and aldehyde (100 mg, 0.2 mmol) in methanol (4 mL) was added acetic acid (156 µL, 2.6 mmol). The mixture was stirred for 5 mins, and sodium cyanoborohydride (164 mg, 2.6 mmol) was added. The mixture was stirred for 14 hrs, and methanol was removed under reduced pressure. Water was added, and was extracted with EtOAc. The organic phase was washed 0.5 N NaOH solution (1x), water (2x), and brine (1x), and was dried over MgSO₄. Purification by flash column chromatography (CH₂Cl₂/MeOH = 100/3) gave compound 18 (62 mg).

### Example 19

Compound 19: To a solution of compound 18 (62 mg, 0.08 mmol) in THF (3 mL) were added acetic acid (9 µL, 0.15 mmol) and tetrabutylamonium fluoride (0.45 mL, 1.0 N, 0.45mmol). The mixture was stirred for 3 hr, and solvent was removed. Purification by flash column chromotogaphy (CH₂Cl₂/MeOH = 100/5) gave an oil. To a solution of above oil in CH₂Cl₂ (2 mL) was added trifluoroacetic acid (2 mL). The mixture was stirred for 1 hrs, and was concentrated under reduced pressure. Coevaporation with EtOAc and CH₂Cl₂ gave compound 19.

### Example 20

Compound 20: To a solution of compound 19 (55 mg 0.08 mmol) in acetonitrile (1 mL) at 0°C was added DMAP (20 mg, 0.16 mmol), followed by bisfurancarbonate (24 mg, 0.08 mmol). The mixture was stirred for 3 hrs at 0°C, and diluted with EtOAc. The organic phase was washed with 0.5 N NaOH solution (2x), water (2x), and brine (1x), and dried over MgSO₄. Purification by flash column chromotography (CH₂Cl₂/MeOH = 100/3 to 100/5) afford compound 20 (46 mg): ¹H NMR (CDCl₃) δ 7.70 (2 H, d, J = 8.9 Hz), 7.01 (2 H, d, J = 8.9 Hz), 5.73 (1 H, d, J = 5.1 Hz), 5.51 (1 H, m), 5.14 (1 H, m), 4.16 (1 H, m), 4.06 (1 H, m), 3.94 (3 H, m), 3.86 (3 H, s), 3.80 (1 H, m), 3.75 (2 H, d, J = 9.1 Hz), 3.58 (1 H, m), 3.47 (1H, m), 3.30 (1 H, m), 3.1-2.6 (8 H, m), 2.3 (2 H, m), 2.1-1.8 (5 H, m), 1.40 (2 H, m), 1.36 (6 H, t, J = 7.0 Hz), 0.93 (3 H, d, J = 6.7 Hz), 0.86 (3 h, d, J = 6.7 Hz).

### Example 21

Compound 21: Compound 21 was made from Boc-4-Nitro-L-Phenylalanine (Fluka) following the procedure for Compound 2 in Scheme Section A, Scheme 1.

### Example 22

Compound 22: To a solution of chloroketone 21 (2.76 g, 8 mmol) in THF (50 mL) and water (6 mL) at 0°C (internal temperature) was added solid NaBH₄ (766 mg, 20 mmol) in several portions over a period of 15 min while maintaining the internal temperature below 5°C. The mixture was stirred for 1.5 hrs at 0°C and solvent was removed under reduced pressure. The mixture was quenched with saturated KHSO₃ and extracted with EtOAc. The organic phase was washed with waster and brine, and dried overMgSO₄. Concentration gave a solid, which was recrystalized from EtOAc/hexane (1/1) to afford the chloroalcohol 22 (1.72 g).

### Example 23

Compound 23: To a suspension of chloroalcohol 22 (1.8 g, 5.2 mmol) in EtOH (50 mL) was added a solution of KOH in ethanol (8.8 mL, 0.71 N, 6.2 mmol). The mixture was stirred for 2 h at room temperature and ethanol was removed under reduced pressure. The reaction mixture was diluted with EtOAc, and washed with water (2x), saturated NH₄Cl (2x), water, and brine, and dried over MgSO₄. Concentration under reduced pressure afforded epoxide 23 (1.57g) as a white crystalline solid.

### Example 24

Compound 24: To a solution of epoxide 23 (20 g, 65 mmol) in 2-propanol (250 mL) was added isobutylamine (65 mL) and the solution was refluxed for 90 min. The reaction mixture was concentrated under reduced pressure and was coevaporated with MeOH, CH₃CN, and CH₂Cl₂ to give a white solid. To a solution of the white solid in CH₂Cl₂ (300 mL) at 0°C was added triethylamine (19 mL, 136 mmol), followed by the addition of 4-methoxybenzenesulfonyl chloride (14.1 g, 65 mmol) in CH₂Cl₂ (50 mL). The reaction mixture was stirred at 0°C for 30 min, and warmed to room temperature and stirred for additional 2 hrs. The reaction solution was concentrated under reduced pressure and was diluted with EtOAc. The organic phase was washed with saturated NaHCO₃, water and brine, and dried over MgSO₄. Concentration under reduced pressure gave compound 24 as a white solid (37.5 g).

### Example 25

Compound 25: To a solution of compound 24 (37.5 g, 68 mmol) in CH₂Cl₂ (100 mL) at 0°C was added a solution of tribromoborane in CH₂Cl₂ (340 mL, 1.0 N, 340 mmol). The reaction mixture was kept at 0°C for 1 hr, and warmed to room temperature and stirred for additional 3 hrs. The mixture was cooled to 0°C, and methanol (200 mL) was added slowly. The mixture was stirred for 1 hr and solvents were removed under reduced pressure to give a brown oil. The brown oil was coevaporated with EtOAc and toluene to afford compound 25 as a brown solid, which was dried under vacuum for 48 hrs.

### Example 26

Compound 26: To a solution of compound 25 in THF (80 mL) was added a saturated sodium bicarbonate solution (25 mL), followed by a solution of Boc20 (982 mg, 4.5 mmol) in THF (20 mL). The reaction mixture was stirred for 5 hrs. THF was removed under reduced pressure, and aqueous phase was extracted with EtOAc. The organic phase was washed with water (2x) and Brine (1x), and dried over MgSO₄. Purification by flash column chromatography (hexanes/EtOAc = 1/1) gave compound 26 (467 mg).

### Example 27

Compound 27: To a solution of compound 26 (300 mg, 0.56 mmol) in THF (6 mL) was added Cs₂CO₃ (546 mg, 1.68 mmol), followed by a solution of triflate (420 mg, 1.39 mmol) in THF (2 mL). The reaction mixture was stirred for 1.5 hrs. The mixture was diluted with EtOAc, and washed with water (3x) and brine (1x), and dried over MgSO₄. Purification by flash column chromatography (hexanes/EtOAc = 1/1 to 1/3) gave compound 27 (300 mg).

### Example 28

Compound 28: To a solution of compound 27 (300 mg, 0.38 mmol) in CH₂Cl₂ (2 mL) was added trifluoroacetic acid (2 mL). The mixture was stirred for 2.5 hrs, and was concentrated under reduced pressure. The mixture was diluted with EtOAc and was washed with 0.5 N NaOH solution (3x), water (2x), and brine (1x), and dried over MgSO₄. Concentration gave a white solid. To the solution of above white solid in acetonitrile (3 mL) at 0°C was added DMAP (93 mg, 0.76 mmol), followed by bisfurancarbonate (112 mg, 0.38 mmol). The mixture was stirred for 3 hrs at 0°C, and diluted with EtOAc. The organic phase was washed with 0.5 N NaOH solution (2x), water (2x), and brine (1x), and dried over MgSO₄. Purification by flash column chromotography (CH₂Cl₂/MeOH = 100/3 to 100/5) afford compound 28 (230 mg): ¹H NMR (CDCl₃) δ 8.16 (2 H, d, J = 8.5 Hz), 7.73 (2 H, d, J = 9.2 Hz), 7.42 (2 H, d, J = 8.5 Hz), 7.10 (2 H, d, J = 9.2 Hz), 5.65 (1 H,d, J = 4.8 Hz), 5.0 (2 H, m), 4.34 (2 H, d, J = 10 Hz), 4.25 (4 H, m), 4.0-3.6 (6 H, m), 3.2-2.8 (7 H, m), 1.82 (1H, m), 1.6(2H,m), 1.39 (6 H, t, J = 7.0 Hz), 0.95 (6 H, m).

### Example 29

Compound 29: To a solution of compound 28 (50 mg) in ethanol (5 mL) was added 10% Pd-C (20 mg). The mixture was hydrogenated for 5 hrs. Celite was added, and the mixture was stirred for 5 mins. The reaction mixture was filtered through a pad of celite. Concentration under reduced pressure gave compound 29 (50 mg): ¹H NMR (CDCl₃) δ 7.72 (2 H, d, J = 8.8 Hz), 7.07 (2 H, 2 H, d, J = 8.8 Hz), 7.00 (2 H, d, J = 8.5 Hz), 6.61 (2 H, d, J = 8.5 Hz), 5.67 (1 H, d, J = 5.2 Hz), 5.05 (1 H, m), 4.90 (1 H, m), 4.34 (2 H, d, J = 10.3 Hz), 4.26 (2 H, m), 4.0-3.7 (6 H, m), 3.17 (1 H, m), 2.95 (4 H, m), 2.75 (2 H, m), 1.82 (1 H, m), 1.65 (2 H, m), 1.39 (6 H, t, J = 7.0 Hz), 0.93 (3 h, d, J = 6.4 Hz), 0.87 (3 h, d, J = 6.4 Hz).

### Example 30

Compound 30: To a solution of compound 29 (50 mg, 0.07 mmol) and formaldehyde (52 µL, 37%, 0.7 mmol) in methanol (1 mL) was added acetic acid (40 µL, 0.7 mmol). The mixture was stirred for 5 mins, and sodium cyanoborohydride (44 mg, 0.7 mmol) was added. The mixture was stirred for 14 hrs, and methanol was removed under reduced pressure. Water was added, and was extracted with EtOAc. The organic phase was washed 0.5 N NaOH solution (1x), water (2x), and brine (1x), and was dried over MgSO₄. Purification by flash column chromatography (CH₂Cl₂/MeOH = 100/3) gave compound 30 (40 mg): ¹H NMR (CDCl₃) δ 7.73 (2 H, d, J = 8.9 Hz), 7.10 (4 H, m), 6.66 (2 H, d, J = 8.2 Hz), 5.66 (1 H, d, J = 5.2 Hz), 5.02 (1 H, m), 4.88 (1 H, m), 4.32 (2 H, d, J =10.1 Hz), 4.26 (4 H, m), 3.98 (1 H, m), 3.85 (3 H, m), 3.75 (2 H, m), 3.19 (1 H, m), 2.98 (4 H, m), 2.93 (6 H, s), 2.80 (2 H, m), 1.82 (1 H, m), 1.62 (2 H, m), 1.39 (6 H, t, J = 7.0 Hz), 0.90 (6 H, m).

### Example 31

Compound 31: To a suspension of compound 25 (2.55 g, 5 mmol) in CH₂Cl₂ (20 mL) at 0°C was added triehtylamine (2.8 mL, 20 mmol), followed by TMSCI (1.26 mL, 10 mmol). The mixture was stirred at 0°C for 30 mins, and warmed to 25°C and stirred for additional 1 hr. Concentration gave a yellow solid. The yellow solid was dissolved in acetonitrile (30 mL) and cooled to 0°C. To this solution was added DMAP (1.22 g, 10 mmol) and Bisfurancarbonate (1.48 g, 5 mmol). The reaction mixture was stirred at 0°C for 2 hrs and for additional 1 hr at 25°C. Acetonitrile was removed under reduced pressure. The mixture was diluted with EtOAc, and washed with 5% citric acid (2x), water (2x), and brine (1x), and dried over MgSO₄. Concentration gave a yellow solid. The yellow solid was dissolved in THF (40 mL), and acetic acid (1.3 mL, 20 mmol) and tetrabutylammonium fluoride (8mL, 1.0 N, 8mmol) were added. The mixture was stirred for 20 mins, and THF was removed under reduced pressure. Purification by flash column chromatography (hexenes/EtOAc = 1/1) gave compound 31 (1.5 g).

### Example 32

Compound 32: To a solution of compound 31 (3.04 g, 5.1 mmol) in THF (75 mL) was added Cs₂CO₃ (3.31 g, 10.2 mmol), followed by a solution of triflate (3.24 g, 7.65 mmol) in THF (2 mL). The reaction mixture was stirred for 1.5 hrs, and THF was removed under reduced pressure. The mixture was diluted with EtOAc, and washed with water (3x) and brine (1x), and dried over MgSO₄. Purification by flash column chromatography (hexanes/EtOAc = 1/1 to 1/3) gave compound 32 (2.4 g): ¹H NMR (CDC1₃) δ 8.17 (2 H, d, J = 8.5 Hz), 7.70 (2 H, J = 9.2 Hz), 7.43-(2 H, d, J = 8.5 Hz), 7.37 (10 H, m), 6.99 (2 H, d, J =9.2 Hz), 5.66 (1 H, d, J = 5.2 Hz), 5.15 (4 H, m), 5.05 (2 H, m), 4.26 (2 H, d, J = 10.2 Hz), 3.9-3.8 (4 H, m), 3.75 (2 H, m), 3.2-2.8 (7 H, m), 1.82 (1 H, m), 1.62 (2 H, m), 0.92 (6 H, m).

### Example 33

Compound 33: To a solution of compound 32 (45 mg) in acetic acid (3 mL) was added zinc (200 mg). The mixture was stirred for 5 hrs. Celite was added, and the mixture was filtered and washed with EtOAc. The solution was concentrated to dryness and diluted with EtOAc. The organic phase was washed with 0.5 N NaOH solution, water, and brine, and dried over MgSO₄. Purification by flash column chromatography (CH₂Cl₂/isoproanol = 100/5) gave compound 33 (25 mg): ¹H NMR (CDCl₃) δ 7.67 (2 H, d, J = 8.8 Hz), 7.36 (10 H, m), 6.98 (4 H, m), 6.60 (2 H, d, J = 8.0 Hz), 5.67 (1 H, d, J = 4.9 Hz), 5.12 (4 H, m), 5.05 (1 H, m), 4.90 (1 H, m), 4.24 (2 H, d, J = 10.4 Hz), 4.0-3.6 (6 H, m), 3.12 (1 H, m), 3.95 (4 H, m), 2.75 (2 H, m), 1.80 (1H, m), 1.2 (2 H, m), 0.9 (6 H, m).

### Example 34

Compound 34: To a solution of compound 32 (2.4 g) in ethanol (140 mL) was added 10% Pd-C (1.0 g). The mixture was hydrogenated for 14 hrs. Celite was added, and the mixture was stirred for 5 mins. The slurry was filtered through a pad of celite, and washed with pyridine. Concentration under reduced pressure gave compound 34: ¹H NMR (DMSO-d₆) δ 7.67 (2 H, d, J = 8.9 Hz), 7.14 (2 H, d, J = 8.9 Hz), 6.83 (2 H, d, J = 8.0 Hz), 6.41 (2 H, d, J = 8.0 Hz), 5.51 (1 H, d, J = 5.2 Hz), 5.0-4.8 (2 H, m), 4.15 (2 H, d, J =10.0 Hz), 3.9-3.2 (8 H, m), 3.0 (2 H, m), 2.8 (4 H, m), 2.25 (1 H, m), 1.4 (2 H, m), 0.8 (6 H, m).

### Example 35

Compound 35: Compound 34 (1.62 g, 2.47 mmol) and L-alanine butyl ester hydrochloride (2.69 g, 14.8 mmol) were coevaporated with pyridine (2x). The mixture was dissolved in pyridine (12 mL) and diisopropylethylamine (2.6 mL, 14.8 mmol) was added. To above mixture was added a solution of Aldrithiol (3.29 g, 14.8 mmol) and triphenylphosphine (3.88 g; 14.8 g) in pyridine (12 mL). The reaction mixture was stirred for 20 hrs, and solvents were evaporated under reduced pressure. The mixture was diluted with ethyl acetate, and was washed with 0.5 N NaOH solution (2x), water (2x), and brine, and dried over MgSO₄. Concentration under reduced pressure gave a yellow oil, which was purified by flash column chromatography (CH₂Cl₂/MeOH = 100/5 to100/15) to afford compound 35 (1.17 g): ¹H NMR (CDCl₃) δ 7.70 (2 H, d, J = 8.6 Hz), 7.05 (2 H, d, J =8.6 Hz), 6.99 (2 H, d, J = 8.0 Hz), 6.61 (2 H, d, J = 8.0 Hz), 5.67 (1 H, d, J = 5.2 Hz), 5.05 (1 H, m), 4.96 (1 H, m), 4.28 (2 H, m), 4.10 (6 H, m), 4.0-3.6 (6 H, m), 3.12 (2 H, m), 2.92 (3 H, m), 2.72 (2 H, m), 1.82 (1 H, m), 1.75-1.65 (2 H, m), 1.60 (4 H, m), 1.43 (6 H, m), 1.35 (4 H, m), 0.91 (12 H, m).

### Example 36

Compound 37: Compound 36 (100 mg, 0.15 mmol) and L-alanine butyl ester hydrochloride (109 mg, 0.60 mmol) were coevaporated with pyridine (2x). The mixture was dissolved in pyridine (1 mL) and diisopropylethylamine (105 µL, 0.6 mmol) was added. To above mixture was added a solution of Aldrithiol (100 mg, 0.45 mmol) and triphenylphosphine (118 mg, 0.45 mmol) in pyridine (1 mL). The reaction mixture was stirred for 20 hrs, and solvents were evaporated under reduced pressure. The mixture was diluted with ethyl acetate, and was washed with water (2x), and brine, and dried over MgSO₄. Concentration under reduced pressure gave an oil, which was purified by flash column chromatography (CH₂Cl₂/MeOH = 100/5 to 100/15) to afford compound 37 (21 mg): ¹H NMR (CDCl₃) δ 7.71 (2 H, d, J = 8.8 Hz), 7.15 (2 H, d, J = 8.2 Hz), 7.01 (2 H, d, J = 8.8 Hz), 6.87 (2 H, d, J = 8.2 Hz), 5.66 (1 H, d, J =5.2 Hz), 5.03 (1 H, m), 4.95 (1 H, m),4.2-4.0 (8 H, m), 3.98 (1 H, m), 3.89 (3 H, s), 3.88-3.65 (5 H, m), 3.15 (1H, m), 2.98 (4 H, m), 2.82 (2 H, m), 1.83 (1 H, m), 1.63 (4 H, m), 1.42(6H,m), 1.35 (4H, m), 0.95 (12H, m).

### Example 37

Compound 38: Compound 36 (100 mg, 0.15 mmol) and L-leucine ethyl ester hydrochloride (117 mg, 0.60 mmol) were coevaporated with pyridine (2x). The mixture was dissolved in pyridine (1 mL) and diisopropylethylamine (105 µL, 0.6 mmol) was added. To above mixture was added a solution of Aldrithiol (100 mg, 0.45 mmol) and triphenylphosphine (118 mg, 0.45 mmol) in pyridine (1 mL). The reaction mixture was stirred for 20 hrs, and solvents were evaporated under reduced pressure. The mixture was diluted with ethyl acetate, and was washed with water (2x), and brine, and dried over MgSO₄. Concentration under reduced pressure gave an oil, which was purified by flash column chromatography (CH₂Cl₂/MeOH = 100/5 to 100/15) to afford compound 38 (12 mg): ¹H NMR (CDCl₃) δ 7.72 (2 H, d, J = 8.5 Hz), 7.14 (2 H, d, J = 8.0 Hz), 7.00 (2 H, d, J = 8.5 Hz), 6.86 (2 H, d, J = 8.0 Hz), 5.66 (1 H, d, J = 5.2 Hz), 5.05 (1 H, m), 4.95 (1 H, m), 4.2-4.0 (8 H, m), 4.0-3.68 (6 H, m), 3.88 (3 H, s), 3.2-2.9 (5 H, m), 2.80 (2 H, m), 1.80 (1 H, m), 1.65 (4 H, m), 1.65-1.50 (4 H, m), 1.24 (6 H, m), 0.94 (18 H, m).

### Example 38

Compound 39: Compound 36 (100 mg, 0.15 mmol) and L-leucine butyl ester hydrochloride (117 mg, 0.60 mmol) were coevaporated with pyridine (2x). The mixture was dissolved in pyridine (1 mL) and diisopropylethylamine (105 µL, 0.6 mmol) was added. To above mixture was added a solution of Aldrithiol (100 mg, 0.45 mmol) and triphenylphosphine (118 mg, 0.45 mmol) in pyridine (1 mL). The reaction mixture was stirred for 20 hrs, and solvents were evaporated under reduced pressure. The mixture was diluted with ethyl acetate, and was washed with water (2x), and brine, and dried over MgSO₄. Concentration under reduced pressure gave an oil, which was purified by flash column chromatography (CH₂Cl₂/MeOH = 100/5 to 100/15) to afford compound 39 (32 mg): ¹H NMR (CDCl₃) δ 7.72 (2 H, d, J = 8.8 Hz), 7.15 (2 H, d, J = 8.0 Hz), 7.0 (2 H, d, J = 8.8 Hz), 6.89 (2 H, d, J = 8.0 Hz), 5.66 (1 H, d, J = 4.3 Hz), 5.07 (1 H, m), 4.94 (1 H, m), 4.2-4.0 (8 H, m), 3.89 (3 H, s), 4.0-3.6 (6 H, m), 3.2-2.9 (5 H, m), 2.8 (2 H, m), 1.81 (1 H, m), 1.78-1.44 (10 H, m), 1.35 (4 H, m), 0.95 (24 H, m).

### Example 39

Compound 41: Compound 40 (82 mg, 0.1 mmol) and L-alanine isopropyl ester hydrochloride (92 mg, 0.53 mmol) were coevaporated with pyridine (2x). The mixture was dissolved in pyridine (1 mL) and diisopropylethylamine (136 µL, 0.78 mmol) was added. To above mixture was added a solution of Aldrithiol (72 mg, 0.33 mmol) and triphenylphosphine (87 mg, 0.33 mmol) in pyridine (1 mL). The reaction mixture was stirred at 75°C for 20 hrs, and solvents were evaporated under reduced pressure. The mixture was diluted with ethyl acetate, and was washed with water (2x), and brine, and dried over MgSO₄. Concentration under reduced pressure gave an oil, which was purified by flash column chromatography (CH₂Cl₂/MeOH = 100/1 to100/3) to afford compound 41 (19 mg): ¹H NMR (CDCl₃) δ 7.71 (2 H, d, J = 8.9 Hz), 7.2-7.35 (5 H, m), 7.15 (2 H, m), 7.01 (2 H, d, J = 8.9 Hz), 6.87 (2 H, m), 5.65 (1 H, d, J = 5.4 Hz), 5.05-4.93 (2 H, m), 4.3 (2 H, m), 4.19 (1 H, m), 3.98 (1 H, m), 3.88 (3 H, s), 3.80 (2 H, m), 3.70 (3 H, m), 3.18 (1 H, m), 2.95 (4 H, m), 2.78 (2 H, m), 1.82 (1 H, m), 1.62 (2 H, m), 1.35 (3 H, m), 1.25-1.17 (6 H, m), 0.93 (3 H, d, J = 6.4 Hz), 0.88 (3 H, d, J = 6.4 Hz).

### Example 40

Compound 42: Compound 40 (100 mg, 0.13 mmol) and L-glycine butyl ester hydrochloride (88 mg, 0.53 mmol) were coevaporated with pyridine (2x). The mixture was dissolved in pyridine (1 mL) and diisopropylethylamine (136 µL, 0.78 mmol) was added. To above mixture was added a solution of Aldrithiol (72 mg, 0.33 mmol) and triphenylphosphine (87 mg, 0.33 mmol) in pyridine (1 mL). The reaction mixture was stirred at 75°C for 20 hrs, and solvents were evaporated under reduced pressure. The mixture was diluted with ethyl acetate, and was washed with water (2x), and brine, and dried over MgSO₄. Concentration under reduced pressure gave an oil, which was purified by flash column chromatography (CH₂Cl₂/MeOH = 100/1 to100/3) to afford compound 42 (18 mg): ¹H NMR (CDCl₃) δ 7.71 (2 H, d, J = 9.2 Hz), 7.35-7.24 (5 H, m), 7.14 (2 H, m), 7.00 (2 H, d, J = 8.8 Hz), 6.87 (2 H, m), 5.65 (1 H, d, J = 5.2 Hz), 5.04 (1 H, m), 4.92 (1 H, m), 4.36 (2 H, m), 4.08 (2 H, m), 3.95 (3 H, m), 3.88 (3 H, s), 3.80 (2 H, m), 3.76 (3 H, m), 3.54 (1H, m), 3.15 (1 H, m), 2.97 (4 H, m), 2.80 (2 H, m), 1.82 (1 H, m), 1.62 (4 H, m), 1.35 (2 H, m), 0.9 (9 H, m).

### Example Section H

### Example 1

Sulfonamide 1: To a suspension of epoxide (20 g, 54.13 mmol) in 2-propanol (250 mL) was added isobutylamine (54 mL, 541 mmol) and the solution was refluxed for 30 min. The solution was evaporated under reduced pressure and the crude solid was dissolved in CH₂Cl₂ (250 mL) and cooled to 0°C. Triethylamine (15.1 mL, 108.26 mmol) was added followed by the addition of 4-nitrobenzenesulfonyl chloride (12 g, 54.13 mmol) and the solution was stirred for 40 min at 0°C, warmed to room temperature for 2 h, and evaporated under reduced pressure. The residue was partitioned between EtOAc and saturated NaHCO₃. The organic phase was washed with saturated NaCl, dried with Na₂SO₄, filtered, and evaporated under reduced pressure. The crude product was recrystallized from EtOAc/hexane to give the sulfonamide (30.59 g, 90%) as an off-white solid.

### Example 2

Phenol 2: A solution of sulfonamide 1 (15.58 g, 24.82 mmol) in EtOH (450 mL) and CH₂Cl₂ (60 mL) was treated with 10% Pd/C (6 g). The suspension was stirred under H₂ atmosphere (balloon) at room temperature for 24 h. The reaction mixture was filtered through a plug of celite and concentrated under reduced pressure. The crude product was purified by column chromatography on silica gel (6% MeOH/CH₂Cl₂) to give the phenol (11.34 g, 90%) as a white solid.

### Example 3

Dibenzylphosphonate 3: To a solution of phenol 2 (18.25 g, 35.95 mmol) in CH₃CN (200 mL) was added Cs₂CO₃ (23.43 g, 71.90 mmol) and triflate (19.83 g, 46.74 mmol). The reaction mixture was stirred at room temperature for 1 h and the solvent was evaporated under reduced pressure. The residue was partitioned between EtOAc and saturated NaCl. The organic phase was dried with Na₂SO₄, filtered, and evaporated under reduced pressure. The crude product was purified by column chromatography on silica gel (2/1-EtOAc/hexane) to give the dibenzylphosphonate (16.87 g, 60%) as a white solid.

### Example 4

Amine 4: A solution of dibenzylphosphonate (16.87 g, 21.56 mmol) in CH₂Cl₂ (60 mL) at 0°C was treated with trifluoroacetic acid (30 mL). The solution was stirred for 30 min at 0°C and then warmed to room temperature for an additional 30 min. Volatiles were evaporated under reduced pressure and the residue was partitioned between EtOAc and 0.5 N NaOH. The organic phase was washed with 0.5 N NaOH (2x), water (2x), saturated NaCl, dried with Na₂SO₄, filtered, and evaporated under reduced pressure to give the amine (12.94 g, 88%) as a white solid.

### Example 5

Carbonate 5: To a solution of (S)-(+)-3-hydroxytetrahydrofuran (5.00 g, 56.75 mmol) in CH₂Cl₂ (80 mL) was added triethylamine (11.86 mL, 85.12 mmol) and bis(4-nitrophenyl)carbonate (25.90 g, 85.12 mmol). The reaction mixture was stirred at room temperature for 24 h and partitioned between CH₂Cl₂ and saturated NaHCO₃. The CH₂Cl₂ layer was dried with Na₂SO₄, filtered, and concentrated. The crude product was purified by column chromatography on silica gel (2/1-EtOAc/hexane) to give the carbonate (8.62 g, 60%) as a pale yellow oil which solidified upon refrigerating.

### Example 6

Carbamate 6: Two methods have been used.
Method 1: To a solution of 4 (6.8 g, 9.97 mmol) and 5 (2.65 g, 10.47 mmol) in CH₃CN (70 mL) at 0 °C was added 4-(dimethylamino)pyridine (2.44 g, 19.95 mmol). The reaction mixture was stirred at 0°C for 3 h and concentrated. The residue was dissolved in EtOAc and washed with 0.5 N NaOH, saturated NaHCO₃, H₂O, dried with Na₂SO₄, filtered, and concentrated. The crude product was purified by column chromatography on silica gel (3% 2-propanol/CH₂Cl₂) to give the carbamate (3.97 g, 50%) as a pale yellow solid.

Method 2: To a solution of 4 (6.0 g, 8.80 mmol) and 5 (2.34 g, 9.24 mmol) in CH₃CN (60 mL) at 0°C was added 4-(dimethylamino)pyridine (0.22 g, 1.76 mmol) and N, N-diisopropylethylamine (3.07 mL, 17.60 mmol). The reaction mixture was stirred at 0°C for 1 h and warmed to room temperature overnight. The solvent was evaporated under reduced pressure. The crude product was dissolved in EtOAc and washed with 0.5 N NaOH, saturated NaHCO₃, H₂O, dried with Na₂SO₄, filtered, and concentrated. The crude product was purified by column chromatography on silica gel (3% 2-propanol/CH₂Cl₂) to give the carbamate (3.85 g, 55%) as a pale yellow solid.

### Example 7

Phosphonic Acid 7: To a solution of 6 (7.52 g, 9.45 mmol) in MeOH (350 mL) was added 10% Pd/C (3 g). The suspension was stirred under H₂ atmosphere (balloon) at room temperature for 48 h. The reaction mixture was filtered through a plug of celite. The filtrate was concentrated and dried under vacuum to give the phosphonic acid (5.24 g, 90%) as a white solid.

### Example 8

Cbz Amide 8: To a solution of 7 (5.23 g, 8.50 mmol) in CH₃CN (50 mL) was added N, O-bis(trimethylsilyl)acetamide (16.54 mL, 68 mmol) and then heated to 70°C for 3 h. The reaction mixture was cooled to room temperature and concentrated. The residue was co-evaporated with toluene and dried under vacuum to afford the silylated intermediate which was used directly without any further purification. To a solution of the silylated intermediate in CH₂Cl₂ (40 mL) at 0°C was added pyridine (1.72 mL, 21.25 mmol) and benzyl chloroformate (1.33 mL, 9.35 mmol). The reaction mixture was stirred at 0°C for 1 h and warmed to room temperature overnight. A solution of MeOH (50 mL) and 1% aqueous HCl (150 mL) was added at 0°C and stirred for 30 min. CH₂Cl₂ was added and two layers were separated. The organic layer was dried with Na₂SO₄, filtered, concentrated, co-evaporated with toluene, and dried under vacuum to give the Cbz amide (4.46 g, 70%) as an off white solid.

### Example 9

Diphenylphosphonate 9: A solution of 8 (4.454 g, 5.94 mmol) and phenol (5.591 g, 59.4 mmol) in pyridine (40 mL) was heated to 70°C and 1,3-dicyclohexylcarbodiimide (4.903 g, 23.76 mmol) was added. The reaction mixture was stirred at 70°C for 4 h and cooled to room temperature. EtOAc was added and the side product 1,3-dicyclohexyl urea was filtered off. The filtrate was concentrated and dissolved in CH₃CN (20 mL) at 0°C. The mixture was treated with DOWEX 50W x 8-400 ion-exchange resin and stirred for 30 min at 0°C. The resin was filtered off and the filtrate was concentrated. The crude product was purified by column chromatography on silica gel (4% 2-propanol/CH₂Cl₂) to give the diphenylphosphonate (2.947 g, 55%) as a white solid.

### Example 10

Monophosphonic Acid 10: To a solution of 9 (2.945 g, 3.27 mmol) in CH₃CN (25 mL) at 0°C was added 1N NaOH (8.2 mL, 8.2 mmol). The reaction mixture was stirred at 0°C for 1 h. DOWEX 50W x 8-400 ion-exchange resin was added and the reaction mixture was stirred for 30 min at 0°C. The resin was filtered off and the filtrate was concentrated and co-evaporated with toluene. The crude product was triturated with EtOAc/hexane (1/2) to give the monophosphonic acid (2.427 g, 90%) as a white solid.

### Example 11

Cbz Protected Monophosphoamidate 11: A solution of 10 (2.421 g, 2.93 mmol) and L-alanine isopropyl ester hydrochloride (1.969 g, 11.73 mmol) in pyridine (20 mL) was heated to 70°C and 1,3-dicyclohexylcarbodiimide (3.629 g, 17.58 mmol) was added. The reaction mixture was stirred at 70°C for 2 h and cooled to room temperature. The solvent was evaporated under reduced pressure and the residue was partitioned between EtOAc and 0.2 N HCl. The EtOAc layer was washed with 0.2 N HCl, H₂O, saturated NaHCO₃, dried with Na₂SO₄, filtered, and concentrated. The crude product was purified by column chromatography on silica gel (4% 2-propanol/CH₂Cl₂) to give the monoamidate (1.569 g, 57%) as a white solid.

### Example 12

Monophosphoamidate12: To a solution of 11 (1.569 g, 1.67 mmol) in EtOAc (80 mL) was added 10% Pd/C (0.47 g). The suspension was stirred under H₂ atmosphere (balloon) at room temperature overnight. The reaction mixture was filtered through a plug of celite. The filtrate was concentrated and the crude product was purified by column chromatography on silica gel (CH₂Cl₂ to 1-8% 2-propanol/CH₂Cl₂) to give the monophosphoamidate 12a (1.12 g, 83%, **GS 108577**, 1:1 diastereomeric mixture A/B) as a white solid: ¹H NMR (CDCl₃) δ 7.45 (dd, 2H), 7.41-7.17 (m, 7H), 6.88 (dd, 2H), 6.67 (d, J = 8.4 Hz, 2H), 5.16 (broad s, 1H), 4.95 (m, 1H), 4.37-4.22 (m, 5H), 3.82-3.67 (m, 7H), 2.99-2.70 (m, 6H), 2.11-1.69 (m, 3H), 1.38 (m, 3H), 1.19 (m, 6H), 0.92 (d, J = 6.3 Hz, 3H), 0.86 (d, J = 6.3 Hz, 3H); ³¹P NMR (CDCl₃) δ 20.5, 19.6. 12b (29 mg, 2%, **GS108578**, diastereomer A) as a white solid: ¹H NMR (CDCl₃) δ 7.43 (d, J = 7.8 Hz, 2H), 7.35-7.17 (m, 7H), 6.89 (d, J = 8.4 Hz, 2H), 6.67 (d, J = 8.4 Hz, 2H), 5.16 (broad s, 1H), 4.96 (m, 1H), 4.38-4.32 (m, 4H), 4.20 (m, 1H), 3.82-3.69 (m, 7H), 2.99-2.61 (m, 6H), 2.10 (m, 1H), 1.98 (m, 1H), 1.80 (m, 1H), 1.38 (d, J = 7.2 Hz, 3H), 1.20 (d, J = 6.3 Hz, 6H), 0.92 (d, J = 6.3 Hz, 3H), 0.86 (d, J = 6.3 Hz, 3H); ³¹P NMR (CDCl₃) δ 20.5. 12c (22 mg, 1.6%, **GS 108579**, diastereomer B) as a white solid : ¹H NMR (CDCl₃) δ 7.45 (d, J = 8.1 Hz, 2H), 7.36-7.20 (m, 7H), 6.87 (d, J = 8.7 Hz, 2H), 6.67 (d, J = 8.4 Hz, 2H), 5.15 (broad s, 1H), 4.95 (m, 1H), 4.34-4.22 (m, 5H), 3.83-3.67 (m, 7H), 2.99-2.64 (m, 6H), 2.11-1.68 (m, 3H), 1.33 (d, J = 6.9 Hz, 3H), 1.20 (d, J = 6.0 Hz, 6H), 0.92 (d, J = 6.3 Hz, 3H), 0.86 (d, J = 6.3 Hz, 3H); ³¹P NMR (CDCl₃) δ 19.6.

### Example 13

Sulfonamide 13: To a suspension of epoxide (1.67 g, 4.52 mmol) in 2-propanol (25 mL) was added isobutylamine (4.5 mL, 45.2 mmol) and the solution was refluxed for 30 min. The solution was evaporated under reduced pressure and the crude solid was dissolved in CH₂Cl₂ (20 mL) and cooled to 0°C. Triethylamine (1.26 mL, 9.04 mmol) was added followed by the treatment of 3-nitrobenzenesulfonyl chloride (1.00 g, 4.52 mmol). The solution was stirred for 40 min at 0°C, warmed to room temperature for 2 h, and evaporated under reduced pressure. The residue was partitioned between EtOAc and saturated NaHCO₃. The organic phase was washed with saturated NaCl, dried with Na₂SO₄, filtered, and evaporated under reduced pressure. The crude product was purified by column chromatography on silica gel (1/1-EtOAc/hexane) to give the sulfonamide (1.99 g, 70%) as a white solid.

### Example 14

Phenol 14: Sulfonamide 13 (1.50 g, 2.39 mmol) was suspended in HOAc (40 mL) and concentrated HCl (20 mL) and heated to reflux for 3 h. The reaction mixture was cooled to room temperature and concentrated under reduced pressure. The crude product was partitioned between 10% MeOH/CH₂Cl₂ and saturated NaHCO₃. The organic layers were washed with NaHCO₃, H₂O, dried with Na₂SO₄, filtered, and concentrated to give a yellow solid. The crude product was dissolved in CHCl₃ (20 mL) and treated with triethylamine (0.9 mL, 6.45 mmol) followed by the addition of Boc₂O (0.61 g, 2.79 mmol). The reaction mixture was stirred at room temperature for 6 h. The product was partitioned between CHCl₃ and H₂O. The CHCl₃ layer was washed with NaHCO₃, H₂O, dried with Na₂SO₄, filtered, and concentrated. The crude product was purified by column chromatography on silica gel (1-5% MeOH/CH₂Cl₂) to give the phenol (0.52 g, 45%) as a pale yellow solid.

### Example 15

Dibenzylphosphonate 15: To a solution of phenol 14 (0.51 g, 0.95 mmol) in CH₃CN (8 mL) was added Cs₂CO₃ (0.77 g, 2.37 mmol) and triflate (0.8 g, 1.90 mmol). The reaction mixture was stirred at room temperature for 1.5 h and the solvent was evaporated under reduced pressure. The residue was partitioned between EtOAc and saturated NaCl. The organic phase was dried Na₂SO₄, filtered, and evaporated under reduced pressure. The crude product was purified by column chromatography on silica gel (3% MeOH/CH₂Cl₂) to give the dibenzylphosphonate (0.62 g, 80%) as a white solid.

### Example 16

Amine 16: A solution of dibenzylphosphonate 15 (0.61 g, 0.75 mmol) in CH₂Cl₂ (8 mL) at 0°C was treated with trifluoroacetic acid (2 mL). The solution was stirred for 30 min at 0°C and then warmed to room temperature for an additional 30 min. Volatiles were evaporated under reduced pressure and the residue was partitioned between EtOAc and 0.5 N NaOH. The organic phase was washed with 0.5 N NaOH (2x), water (2x), saturated NaCl, dried (Na₂SO₄), filtered, and evaporated under reduced pressure to give the amine (0.48 g, 90%) which was used directly without any further purification.

### Example 17

Carbamate 17: To a solution of amine 16 (0.48 g, 0.67 mmol) in CH₃CN (8 mL) at 0°C was treated with (3R, 3aR, 6aS)-hexahydrofuro[2, 3-*b*]furan-2-yl 4-nitrophenyl carbonate (0.2 g, 0.67 mmol, prepared according to Ghosh et al. J. Med. Chem. 1996, 39, 3278.) and 4-(dimethylamino)pyridine (0.17 g, 1.34 mmol). After stirring for 2 h at 0°C, the reaction solvent was evaporated under reduced pressure and the residue was partitioned between EtOAc and 0.5 N NaOH. The organic phase was washed with 0.5N NaOH (2 x), 5% citric acid (2 x), saturated NaHCO₃, dried with Na₂SO₄, filtered, and evaporated under reduced pressure. The crude product was purified by column chromatography on silica gel (3% 2-propanol/CH₂Cl₂) to give the carbamate (0.234 g, 40%) as a white solid.

### Example 18

Analine 18: To a solution of carbamate 17 (78 mg, 0.09 mmol) in 2 mL HOAc was added zinc powder. The reaction mixture was stirred at room temperature for 1.5 h and filtered through a small plug of celite. The filtrate was concentrated and co-evaporated with toluene. The crude product was purified by column chromatography on silica gel (5% 2-propanaol/CH₂Cl₂) to give the analine (50 mg, 66%) as a white solid.

### Example 19

Phosphonic Acid 19: To a solution of analine (28 mg, 0.033mmol) in MeOH (1 mL) and HOAc (0.5 mL) was added 10% Pd/C (14 mg). The suspension was stirred under H₂ atmosphere (balloon) at room temperature for 6 h. The reaction mixture was filtered through a small plug of celite. The filtrate was concentrated, co-evaporated with toluene, and dried under vacuum to give the phosphonic acid (15 mg, 68%, **GS 17424)** as a white solid: ¹H NMR (DMSO-d₆) δ 7.16-6.82 (m, 8H), 5.50 (d, 1H), 4.84 (m, 1H), 3.86-3.37 (m, 9H), 2.95-2.40 (m, 6H), 1.98 (m, 1H), 1.42-1.23 (m, 2H), 0.84 (d, J = 6.3 Hz, 3H), 0.79 (d, J = 6.3 Hz,3H). MS (ESI) 657 (M-H).

### Example 20

Phenol 21: A suspension of aminohydrobromide salt 20 (22.75 g, 44 mmol) in CH₂Cl₂ (200 mL) at 0°C was treated with triethylamine (24.6 mL, 176 mmol) followed by slow addition of chlorotrimethylsilane (11.1 mL, 88 mmol). The reaction mixture was stirred at 0°C for 30 min and warmed to room temperature for 1 h. The solvent was removed under reduced pressure to give a yellow solid. The crude product was dissolved in CH₂Cl₂ (300 mL) and treated with triethylamine (18.4 mL, 132 mmol) and Boc₂O (12 g, 55 mmol). The reaction mixture was stirred at room temperature overnight. The product was partitioned between CH₂Cl₂ and H₂O. The CH₂Cl₂ layer was washed with NaHCO₃, H₂O, dried with Na₂SO₄, filtered, and concentrated. The crude product was dissolved in THF (200 mL) and treated with 1.0 M TBAF (102 mL, 102 mmol) and HOAc (13 mL). The reaction mixture was stirred at room temperature for 1 h and concentrated under reduced pressure. The residue was partitioned between CH₂Cl₂ and H₂O, dried with Na₂SO₄, filtered, and concentrated. The crude product was purified by column chromatography on silica gel (1-3% 2-propanol/CH₂Cl₂) to give the phenol (13.75 g, 58%) as a white solid.

### Example 21

Dibenzylphosphonate 22: To a solution of phenol 21 (13.70 g, 25.48 mmol) in THF (200 mL) was added Cs₂CO₃ (16.61 g, 56.96 mmol) and triflate (16.22 g, 38.22 mmol). The reaction mixture was stirred at room temperature for 1 h and the solvent was evaporated under reduced pressure. The residue was partitioned between EtOAc and saturated NaCL The organic phase was dried with Na₂SO₄, filtered, and evaporated under reduced pressure. The crude product was purified by column chromatography on silica gel (3% MeOH/CH₂Cl₂) to give the dibenzylphosphonate (17.59 g, 85%) as a white solid.

### Example 22

Amine 23: A solution of dibenzylphosphonate 22 (17.58 g, 21.65 mmol) in CH₂Cl₂ (60 mL) at 0°C was treated with trifluoroacetic acid (30 mL). The solution was stirred for 30 min at 0°C and then warmed to room temperature for an additional 1.5 h. Volatiles were evaporated under reduced pressure and the residue was partitioned between EtOAc and 0.5 N NaOH. The organic phase was washed with 0.5 N NaOH (2x), water (2x), saturated NaCl, dried with Na₂SO₄, filtered, and evaporated under reduced pressure to give the amine (14.64 g, 95%) which was used directly without any further purification.

### Example 23

Carbamate 24: To a solution of amine 23 (14.64 g, 20.57 mmol) in CH₃CN (200 mL) at 0°C was treated with (3R, 3aR, 6aS)-hexahydrofuro[2, 3-*b*]furan-2-yl 4-nitrophenyl carbonate (6.07 g, 20.57 mmol, prepared according to Ghosh et al., J. Med. Chem. 1996, 39, 3278.) and 4-(dimethylamino)pyridine (5.03 g, 41.14mmol). After stirring for 2 h at 0°C, the reaction solvent was evaporated under reduced pressure and the residue was partitioned between EtOAc and 0.5 N NaOH. The organic phase was washed with 0.5N NaOH (2 x), 5% citric acid (2 x), saturated NaHCO₃, dried with Na₂SO₄, filtered, and evaporated under reduced pressure. The crude product was purified by column chromatography on silica gel (3% 2-propanol/CH₂Cl₂) to give the carbamate (10 g, 56%) as a white solid.

### Example 24

Phosphonic Acid 25: To a solution of carbamate 24 (8 g, 9.22 mmol) in EtOH (500 mL was added 10% Pd/C (4 g). The suspension was stirred under H₂ atmosphere (balloon) at room temperature for 30 h. The reaction mixture was filtered through a plug of celite. The celite paste was suspended in pyridine and stirred for 30 min and filtered. This process was repeated twice. The combined solution was concentrated under reduced pressure to give the phosphonic acid (5.46 g, 90%) as an off-white solid.

### Example 25

Cbz Amide 26: To a solution of 25 (5.26 g, 7.99 mmol) in CH₃CN (50 mL) was added N, O-bis(trimethylsilyl)acetamide (15.6 mL, 63.92 mmol) and then heated to 70°C for 3 h. The reaction mixture was cooled to room temperature and concentrated. The residue was co-evaporated with toluene and dried under vacuum to afford the silylated intermediate which was used directly without any further purification. To a solution of the silylated intermediate in CH₂Cl₂ (40 mL) at 0°C was added pyridine (1.49 mL, 18.38 mmol) and benzyl chloroformate (1.25mL, 8.79 mmol). The reaction mixture was stirred at 0°C for 1 h and warmed to room temperature overnight. A solution of MeOH (50 mL) and 1% aqueous HCl (150 mL) was added at 0°C and stirred for 30 min. CH₂Cl₂ was added and two layers were separated. The organic layer was dried with Na₂SO₄, filtered, concentrated, co-evaporated with toluene, and dried under vacuum to give the Cbz amide (4.43 g, 70%) as an off-white solid.

### Example 26

Diphenylphosphonate 27: A solution of 26 (4.43 g, 5.59 mmol) and phenol (4.21 g, 44.72 mmol) in pyridine (40 mL) was heated to 70°C and 1,3-dicyclohexylcarbodiimide (4.62 g, 22.36 mmol) was added. The reaction mixture was stirred at 70°C for 36 h and cooled to room temperature. EtOAc was added and the side product 1,3-dicyclohexyl urea was filtered off. The filtrate was concentrated and dissolved in CH₃CN (20 mL) at 0°C. The mixture was treated with DOWEX 50W x 8-400 ion-exchange resin and stirred for 30 min at 0°C. The resin was filtered off and the filtrate was concentrated. The crude product was purified by column chromatography on silica gel (2/1-EtOAc/hexane to EtOAc) to give the diphenylphosphonate (2.11 g, 40%) as a pale yellow solid.

### Example 27

Monophosphonic Acid 28: To a solution of 27 (2.11 g, 2.24 mmol) in CH₃CN (15 mL) at 0°C was added 1N NaOH (5.59 mL, 5.59 mmol). The reaction mixture was stirred at 0°C for 1 h. DOWEX 50W x 8-400 ion-exchange resin was added and the reaction mixture was stirred for 30 min at 0°C. The resin was filtered off and the filtrate was concentrated and co-evaporated with toluene. The crude product was triturated with EtOAc/hexane (1/2) to give the monophosphonic acid (1.75 g, 90%) as a white solid.

### Example 28

Cbz Protected Monophosphoamidate 29: A solution of 28 (1.54 g, 1.77 mmol) and L-alanine isopropyl ester hydrochloride (2.38 g, 14.16 mmol) in pyridine (15 mL) was heated to 70°C and 1,3-dicyclohexylcarbodiimide (2.20 g, 10.62 mmol) was added. The reaction mixture was stirred at 70°C overnight and cooled to room temperature. The solvent was removed under reduced pressure and the residue was partitioned between EtOAc and 0.2 N HCl. The EtOAc layer was washed with 0.2 N HCl, H₂O, saturated NaHCO₃, dried with Na₂SO₄, filtered, and concentrated. The crude product was purified by column chromatography on silica gel (3% MeOH/CH₂Cl₂) to give the monophosphoamidate (0.70g, 40%) as an off-white solid.

### Example 29

Monophosphoamidate 30a-b: To a solution of 29 (0.70 g, 0.71 mmol) in EtOH (10 mL) was added 10% Pd/C (0.3 g). The suspension was stirred under H₂ atmosphere (balloon) at room temperature for 6 h. The reaction mixture was filtered through a small plug of celite. The filtrate was concentrated and the crude products were purified by column chromatography on silica gel (7-10% MeOH/CH₂Cl₂) to give the monoamidates 30a (0.106 g, 18%, GS 77369, 1/1 diastereomeric mixture) as a white solid: ¹H NMR (CDCl₃) δ 7.71 (d, J = 8.7 Hz, 2H), 7.73-7.16 (m, 5H), 7.10-6.98 9m, 4H), 6.61 (d, J = 8.1 Hz, 2H), 5.67 (d, J = 4.8 Hz, 1H), 5.31-4.91 (m, 2H), 4.44 (m, 2H), 4.20 (m, 1H), 4.00-3.61 (m, 6H), 3.18-2.74 (m, 7H), 1.86-1.64 (m, 3H), 1.38 (m, 3H), 1.20 (m, 6H), 0.93 (d, J = 6.6 Hz, 3H), 0.87 (d, J = 6.6 Hz, 3H); ³¹P NMR (CDCl₃) □ 19.1,18; MS(ESI) 869 (M+Na). 30b (0.200 g, 33%, GS 77425, 1/1 diastereomeric mixture) as a white solid: ¹H NMR (CDCl₃) δ 7.73 (dd, J = 8.7 Hz, J = 1.5 Hz, 2H), 7.36-7.16 (m, 5H), 7.09-7.00 (m, 4H), 6.53 (d, J = 8.7 Hz, 2H), 5.66 (d, J = 5.4 Hz, 1H), 5.06-4.91 (m, 2H), 4.40 (m, 2H), 4.20 (m, 1H), 4.00-3.60 (m, 6H), 3.14 (m, 3H), 3.00-2.65 (m, 6H), 1.86-1.60 (m, 3H), 1.35 (m, 3H), 1.20 (m, 9H), 0.92 (d, J = 6.6 Hz, 3H), 0.87 (d, J = 6.6 Hz, 3H); ³¹ P NMR (CDCl₃) □ 19.0, 17.9. MS (ESI) 897 (M+Na).

### Example 30

Synthesis of Bisamidates 32: A solution of phosphonic acid 31 (100 mg, 0.15 mmol) and L-valine ethyl ester hydrochloride (108 mg, 0.60 mmol) was dissolved in pyridine (5 mL) and the solvent was distilled under reduced pressure at 40-60°C. The residue was treated with a solution of Ph₃P (117 mg, 0.45 mmol) and 2,2'-dipyridyl disulfide (98 mg, 0.45 mmol) in pyridine (1 mL) followed by addition of N,N-diisopropylethylamine (0.1 mL, 0.60 mmol). The reaction mixture was stirred at room temperature for two days. The solvent was evaporated under reduced pressure and the residue was purified by column chromatography on silica gel to give the bisamidate (73 mg, 53%, **GS 17389**) as a white solid: ¹H NMR (CDCl₃) δ 7.72 (d, J = 8.7 Hz, 2H), 7.15 (d, J = 8.1 Hz, 2H), 7.00 (d, J = 8.7 Hz, 2H), 6.86 (d, J = 8.1 Hz, 2H), 5.66 (d, J = 4.8 Hz, 1H), 5.05 (m, 1H), 4.95 (d, J = 8.7 Hz, 1H), 4.23-4.00 (m ,4H,), 3.97-3.68 (m, 11H), 3.39-2.77 (m, 9H), 2.16 (m, 2H), 1.82-1.60 (m, 3H), 1.31-1.18 (m, 6H), 1.01-0.87 (m, 18H); ³¹P NMR (CDCl₃) δ 21.3; MS (ESI) 950 (M+Na).

### Example 31

Triflate 34: To a solution of phenol 33 (2.00 g, 3.46 mmol) in THF (15 mL) and CH₂Cl₂ (5 mL) was added N-phenyltrifluoromethanesulfonimide (1.40 g, 3.92 mmol) and cesium carbonate (1.40 g, 3.92 mmol). The reaction mixture was stirred at room temperature overnight and concentrated. The crude product was partitioned between CH₂Cl₂ and saturated NaCl, dried with Na₂SO₄, filtered, and concentrated. The crude product was purified by column chromatography on silica gel (3% MeOH/CH₂Cl₂) to give the triflate (2.09 g, 85%) as a white solid.

### Example 32

Aldehyde 35: To a suspension of triflate 34 (1.45 g, 2.05 mmol), palladium (II) acetate (46 mg, 0.20 mmol) and 1,3-bis(diphenylphosphino)propane (84 mg, 0.2 mmol) in DMF (8 mL) under CO atmosphere (balloon) was slowly added triethylamine (1.65 mL, 11.87 mmol) and triethylsilane (1.90 mL, 11.87 mmol). The reaction mixture was heated to 70°C under CO atmosphere (balloon) and stirred overnight. The solvent was concentrated under reduced pressure and partitioned between CH₂Cl₂ and H₂O. The organic phase was dried with Na₂SO₄, filtered, and concentrated. The crude product was purified by column chromatography on silica gel (4% 2-propanol/CH₂Cl₂) to give the aldehyde (0.80 g, 66%) as a white solid.

### Example 33

Substituted Benzyl Alcohol 36: To a solution of aldehyde 35 (0.80g, 1.35 mmol) in THF (9 mL) and H₂O (1 mL) at -10°C was added NaBH₄ (0.13 g, 3.39 mmol). The reaction mixture was stirred for 1 h at -10°C and the solvent was evaporated under reduced pressure. The residue was dissolved in CH₂Cl₂ and washed with NaHSO₄, H₂O, dried with Na₂SO₄, filtered, and concentrated. The crude product was purified by column chromatography on silica gel (6% 2-propanol/CH₂Cl₂) to give the alcohol (0.56 g, 70%) as a white solid.

### Example 34

Substituted Benzyl Bromide 37: To a solution of alcohol 36 (77 mg, 0.13 mmol) in THF (1 mL) and CH₂Cl₂ (1 mL) at 0°C was added triethylamine (0.027 mL, 0.20 mmol) and methanesulfonyl chloride (0.011 mL, 0.14 mmol). The reaction mixture was stirred at 0°C for 30 min and warmed to room temperature for 3 h. Lithium bromide (60 mg, 0.69 mmol) was added and stirred for 45 min. The reaction mixture was concentrated and the residue was partitioned between CH₂Cl₂ and H₂O, dried with Na₂SO₄, filtered, and concentrated. The crude product was purified by column chromatography on silica gel (2% MeOH/CH₂Cl₂) to give the bromide (60 mg, 70%).

### Example 35

Diethylphosphonate 38: A solution of bromide 37 (49 mg, 0.075 mmol) and triethylphosphite (0.13 mL, 0.75 mmol) in toluene (1.5 mL) was heated to 120°C and stirred overnight. The reaction mixture was cooled to room temperature and concentrated under reduced pressure. The crude product was purified by column chromatography on silica gel (6% MeOH/CH₂Cl₂) to give the diethylphosphonate (35 mg, 66%, **GS 191338**) as a white solid: ¹H NMR (CDCl₃) δ 7.72 (d, J = 8.7 Hz, 2H), 7.27-7.16 (m, 4H), 7.00 (d, J = 8.7 Hz, 2H), 5.66 (d, J = 5.1 Hz, 1H), 5.00 (m, 2H), 4.04-3.73 (m, 13H), 3.13-2.80 (m, 9H), 1.82-1.64 (m, 3H), 1.25 (t, J = 6.9 Hz, 6H), 0.92 (d, J = 6.3 Hz, 3H), 0.88 (d, J = 6.3 Hz, 3H); ³¹P NMR (CDCl₃) □ 26.4; MS (ESI) 735 (M+Na).

### Example 36

N-tert-Butoxycarbonyl-O-benzyl-L-serine 39: To a solution of Boc-L-serine (15 g, 73.09 mmol) in DMF (300 mL) at 0°C was added NaH (6.43 g, 160.80 mmol, 60% in mineral oil) and stirred for 1.5 h at 0°C. After the addition of benzyl bromide (13.75 g, 80.40 mmol), the reaction mixture was warmed to room temperature and stirred overnight. The solvent was evaporated under reduced pressure and the residue was dissolved in H₂O. The crude product was partitioned between H₂O and Et₂O. The aqueous phase was acidified to pH<4 with 3 N HCl and extracted with EtOAc three times. The combined EtOAc solution was washed with H₂O, dried with Na₂SO₄, filtered, and concentrated to give the N-tert-butoxycarbonyl-O-benzyl-L-serine (17.27 g, 80%).

### Example 37

Diazo Ketone 40: To a solution of N-tert-Butoxycarbonyl-O-benzyl-L-serine 39 (10 g, 33.86 mmol) in dry THF (120 mL) at -15°C was added 4-methylmorpholine (3.8 mL, 34.54 mmol) followed by the slow addition of isobutylchloroformate (4.40 mL, 33.86 mmol). The reaction mixture was stirred for 30 min and diazomethane (-50 mmol, generated from 15 g Diazald according to Aldrichimica Acta 1983, 16, 3) in ether (- 150 mL) was poured into the mixed anhydride solution. The reaction was stirred for 15 min and was then placed in an ice bath at 0°C and stirred for 1 h. The reaction was allowed to warm to room temperature and stirred overnight. The solvent was evaporated under reduced pressure and the residue was dissolved in EtOAc, washed with water, saturated NaHCO₃, saturated NaCl, dried with Na₂SO₄, filtered and evaporated. The crude product was purified by column chromatography (EtOAc/hexane) to afford the diazo ketone (7.50 g, 69%) as a yellow oil.

### Example 38

Chloroketone 41: To a suspension of diazoketone 40 (7.50 g, 23.48 mmol) in ether (160 mL) at 0°C was added 4N HCl in dioxane (5.87 mL, 23.48 mmol). The reaction mixture was stirred at 0°C for 1 h. The reaction solvent was evaporated under reduced pressure to give the chloroketone which was used directly without any further purification.

### Example 39

Chloroalcohol 42: To a solution of chloroketone 41 (7.70 g, 23.48 mmol) in THF (90 mL) was added water (10 mL) and the solution was cooled to 0°C. A solution of NaBH₄ (2.67 g, 70.45 mmol) in water (4 mL) was added dropwise over a period of 10 min. The mixture was stirred for 1 h at 0°C and saturated KHSO₄ was slowly added until the pH<4 followed by saturated NaCl. The organic phase was washed with saturated NaCl, dried with Na₂SO₄, filtered, and evaporated under reduced pressure. The crude product was purified by column chromatography on silica gel (1/4 EtOAc/hexane) to give the chloroalcohol (6.20 g, 80%) as a diastereomeric mixture.

### Example 40

Epoxide 43: A solution of chloroalcohol 42 (6.20 g, 18.79 mmol) in EtOH (150 mL) was treated with 0.71 M KOH (1.27 g, 22.55 mmol) and the mixture was stirred at room temperature for 1 h. The reaction mixture was evaporated under reduced pressure and the residue was partitioned between EtOAc and water. The organic phase was washed with saturated NaCl, dried with Na₂SO₄, filtered, and evaporated under reduced pressure. The crude product was purified by column chromatography on silica gel (1/6 EtOAc/hexane) to afford the desired epoxide 43 (2.79 g, 45%) and a mixture of diastereomers 44 (1.43 g, 23%).

### Example 41

Sulfonamide 45: To a suspension of epoxide 43 (2.79 g, 8.46 mmol) in 2-propanol (30 mL) was added isobutylamine (8.40 mL, 84.60 mmol) and the solution was refluxed for 1 h. The solution was evaporated under reduced pressure and the crude solid was dissolved in CH₂Cl₂ (40 mL) and cooled to 0°C. Triethylamine (2.36 mL, 16.92 mmol) was added followed by the addition of 4-methoxybenzenesulfonyl chloride (1.75 g, 8.46 mmol). The solution was stirred for 40 min at 0°C, warmed to room temperature, and evaporated under reduced pressure. The residue was partitioned between EtOAc and saturated NaHCO₃. The organic phase was washed with saturated NaCl, dried with Na₂SO₄, filtered, and evaporated under reduced pressure. The crude product was directly used without any further purification.

### Example 42

Silyl Ether 46: A solution of sulfonamide 45 (5.10 g, 8.46 mmol) in CH₂Cl₂ (50 mL) was treated with triethylamine (4.7 mL, 33.82 mmol) and TMSOTf (3.88 mL, 16.91 mmol). The reaction mixture was stirred at room temperature for 1 h and partitioned between CH₂Cl₂ and saturated NaHCO₃. The aqueous phase was extracted twice with CH₂Cl₂ and the combined organic extracts were washed with saturated NaCl, dried with Na₂SO₄, filtered, and evaporated under reduced pressure. The crude product was purified by column chromatography on silica gel (1/6 EtOAc/hexane) to give the silyl ether (4.50 g, 84%) as a thick oil.

### Example 43

Alcohol 47: To a solution of silyl ether 46 (4.5 g, 7.14 mmol) in MeOH. (50 mL) was added 10% Pd/C (0.5 g). The suspension was stirred under H₂ atmosphere (balloon) at room temperature for 2 h. The reaction mixture was filtered through a plug of celite and concentrated under reduced pressure. The crude product was purified by column chromatography on silica gel (3% MeOH/CH₂Cl₂) to give the alcohol (3.40 g, 85%) as a white solid.

### Example 44

Aldehyde 48: To a solution of alcohol 47 (0.60 g, 1.07 mmol) in CH₂Cl₂ (6 mL) at 0°C was added Dess Martin reagent (0.77 g, 1.82 mmol). The reaction mixture was stirred at 0°C for 3 h and partitioned between CH₂Cl₂ and NaHCO₃. The organic phase was washed with H₂O, dried with Na₂SO₄, filtered, and concentrated. The crude product was purified by column chromatography on silica gel (1/4 EtOAc/hexane) to give the aldehyde (0.45 g, 75%) as a pale yellow solid.

### Example 45

Sulfonamide 50: To a suspension of epoxide (2.00 g, 5.41 mmol) in 2-propanol (20 mL) was added amine 49 (4.03 g, 16.23 mmol) (prepared in 3 steps starting from 4-(aminomethyl)piperidine according to Bioorg. Med. Chem. Lett., 2001, 11, 1261.). The reaction mixture was heated to 80°C and stirred for 1 h. The solution was evaporated under reduced pressure and the crude solid was dissolved in CH₂Cl₂ (20 mL) and cooled to 0°C. Triethylamine (4.53 mL, 32.46 mmol) was added followed by the addition of 4-methoxybenzenesulfonyl chloride (3.36 g, 16.23 mmol). The solution was stirred for 40 min at 0°C, warmed to room temperature for 1.5 h, and evaporated under reduced pressure. The residue was partitioned between EtOAc and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide (2.50 g, 59%).

### Example 46

Amine 51: A solution of sulfonamide 50 (2.50 g, 3.17 mmol) in CH₂Cl₂ (6 mL) at 0°C was treated with trifluoroacetic acid (3 mL). The solution was stirred for 30 min at 0°C and then warmed to room temperature for an additional 1.5 h. Volatiles were evaporated under reduced pressure and the residue was partitioned between EtOAc and 0.5 N NaOH. The organic phase was washed with 0.5 N NaOH (2x), water (2x) and saturated NaCl, dried with Na₂SO₄, filtered, and evaporated under reduced pressure to give the amine (1.96 g, 90%) which was used directly without any further purification.

### Example 47

Carbamate 52: To a solution of amine 51 (1.96 g, 2.85 mmol) in CH₃CN (15mL) at 0°C was treated with (3R, 3aR, 6aS)-hexahydrofuro[2, 3-*b*]furan-2-yl 4-nitrophenyl carbonate (0.84g, 2.85mmol, prepared according to Ghosh et al., J. Med. Chem 1996, 39, 3278.) and 4-(dimethylamino)pyridine (0.70 g, 5.70 mmol). After stirring for 2 h at 0°C, the reaction solvent was evaporated under reduced pressure and the residue was partitioned between EtOAc and 0.5 N NaOH. The organic phase was washed with 0.5N NaOH (2 x), 5% citric acid (2 x), saturated NaHCO₃, dried with Na₂SO₄, filtered, and evaporated under reduced pressure. The crude product was purified by column chromatography on silica gel (3% 2-propanol/CH₂Cl₂) to give the carbamate (1.44 g, 60%) as a white solid.

### Example Section I

### Example 1

Carbonate 2: To a solution of (R)-(+)-3-hydroxytetrahydrofuran (1.23 g, 14 mmol) in CH₂Cl₂ (50 mL) was added triethylamine (2.9 mL, 21 mmol) and bis(4-nitrophenyl)carbonate (4.7 g, 15.4 mmol). The reaction mixture was stirred at room temperature for 24 h and partitioned between CH₂Cl₂ and saturated NaHCO₃. The CH₂Cl₂ layer was dried with Na₂SO₄, filtered, and concentrated. The crude product was purified by column chromatography on silica gel (2/1-EtOAc/hexane) to give the carbonate (2.3 g, 65%) as a pale yellow oil which solidified upon standing.

### Example 2

Carbamate 3: To a solution of 1 (0.385 g, 0.75 mmol) and 2 (0.210 g, 0.83 mmol) in CH₃CN (7 mL) at room temperature was added N, N-diisopropylethylamine (0.16 mL, 0.90 mmol). The reaction mixture was stirred at room temperature for 44 h. The solvent was evaporated under reduced pressure. The crude product was dissolved in EtOAc and washed with saturated NaHCO₃, brine, dried with Na₂SO₄, filtered, and concentrated. The crude product was purified by column chromatography on silica gel (1/1-EtOAc/hexane) to give the carbamate (0.322 g, 69%) as a white solid: mp 98-100°C (uncorrected).

### Example 3

Phenol 4: To a solution of 3 (0.31 g, 0.49 mmol) in EtOH (10 mL) and EtOAc (5 mL) was added 10% Pd/C (30 mg). The suspension was stirred under H₂ atmosphere (balloon) at room temperature for 15 h. The reaction mixture was filtered through a plug of celite. The filtrate was concentrated and dried under vacuum to give the phenol (0.265 g) in quantitative yield.

### Example 4

Diethylphosphonate 5: To a solution of phenol 4 (100 mg, 0.19 mmol) in THF (3 mL) was added C_{S2}CO₃ (124 mg, 0.38 mmol) and triflate (85 mg, 0.29 mmol). The reaction mixture was stirred at room temperature for 4 h and the solvent was evaporated under reduced pressure. The residue was partitioned between EtOAc and saturated NaCl. The organic phase was dried with Na₂SO₄, filtered, and evaporated under reduced pressure. The crude product was purified by column chromatography on silica gel (5% 2-propanol/CH₂Cl₂) to give the diethylphosphonate (63 mg, 49%, **GS 16573**) as a white solid: ¹H NMR (CDCl₃) δ 7.65 (d, J = 8.7Hz, 2H), 7.21 (d, J = 8.7 Hz, 2H), 6.95 (d, J = 9 Hz, 2H), 6.84 (d, J = 8.4 Hz, 2H), 5.06 (broad, s, 1H), 4.80 (d, J = 7.5 Hz, 1H), 4.19 (m, 6H), 3.83 (s, 3H), 3.80-3.70 (m, 6H), 3.09-2.72 (m, 6H), 2.00 (m, 1H), 1.79 (m, 2H), 1.32 (t, J = 7.5 Hz, 6H), 0.86 (d, J = 6.6 Hz, 3H), 0.83 (d, J = 6.6 Hz, 3H); ³¹P NMR δ 17.8.

### Example 5

Dibenzylphosphonate 6: To a solution of phenol 4-(100 mg, 0.19 mmol) in THF (3 mL) was added Cs₂CO₃ (137 mg, 0.42 mmol) and triflate (165 mg, 0.39 mmol). The reaction mixture was stirred at room temperature for 6 h and the solvent was evaporated under reduced pressure. The residue was partitioned between EtOAc and saturated NaCl. The organic phase was dried with Na₂SO₄, filtered, and evaporated under reduced pressure. The crude product was purified by column chromatography on silica gel (5% 2-propanol/CH₂Cl₂) to give the dibenzylphosphonate (130 mg, 84%, **GS 16574**) as a white solid: ¹H NMR (CDCl₃) δ 7.65 (d, J = 9 Hz, 2H), 7.30 (m, 10H), 7.08 (d, J = 8.4Hz, 2H), 6.94 (d, J = 9 Hz, 2H), 6.77 (d, J = 8.7 Hz, 2H), 5.16-5.04 (m, 5H), 4.80 (d, J = 8.1 Hz, 1H), 4.16 (d, J = 10.2 Hz, 2H), 3.82 (s, 3H), 3.75-3.71 (m, 6H), 3.10-2.72 (m, 6H), 2.00 (m, 1H), 1.79 (m, 2H), 0.86 (d, J = 6.6 Hz, 3H), 0.83 (d, J = 6.6 Hz, 3H); ³¹ P NMR (CDCl₃) δ 18.8.

### Example 6

Phosphonic Acid 7: To a solution of 6 (66 mg, 0.08 mmol) in EtOH (3 mL) was added 10% Pd/C (12 mg). The suspension was stirred under H₂ atmosphere (balloon) at room temperature for 15 h. The reaction mixture was filtered through a plug of celite. The filtrate was concentrated under reduced pressure and triturated with EtOAc to give the phosphonic acid (40 mg, 78%, **GS 16575**) as a white solid.

### Example 7

Carbonate 8: To a solution of (S)-(+)-3-hydroxytetrahydrofuran (2 g, 22.7 mmol) in CH₃CN (50 mL) was added triethylamine (6.75 mL, 48.4 mmol) and N,N'-disuccinimidyl carbonate (6.4 g, 25 mmol). The reaction mixture was stirred at room temperature for 5 h and concentrated under reduced pressure. The residue was partitioned between EtOAc and H₂O. The organic phase was dried with Na₂SO₄, filtered, and concentrated under reduced pressure. The crude product was purified by column chromatography on silica gel (EtOAc as eluant) followed by recrystallization (EtOAc/hexane) to give the carbonate (2.3 g, 44%) as a white solid.

### Example 8

Carbamate 9: To a solution of 1 (0.218 g, 0.42 mmol) and 8 (0.12 g, 0.53 mmol) in CH₃CN (3 mL) at room temperature was added N, N-diisopropylethylamine (0.11 mL, 0.63 mmol). The reaction mixture was stirred at room temperature for 2 h. The solvent was evaporated and the residue was partitioned between EtOAc and saturated NaHCO₃. The organic phase was washed with brine, dried with Na₂SO₄, filtered, and concentrated. The crude product was purified by column chromatography on silica gel (1/1-EtOAc/hexane) to give the carbamate (0.176 g, 66%) as a white solid.

### Example 9

Phenol 10: To a solution of 9 (0.176 g, 0.28 mmol) in EtOH (10 mL) was added 10% Pd/C (20 mg). The suspension was stirred under H₂ atmosphere (balloon) at room temperature for 4 h. The reaction mixture was filtered through a plug of celite. The filtrate was concentrated and dried under vacuum to give the phenol (0.151 g, **GS 10**) in quantitative yield.

### Example 10

Diethylphosphonate 11: To a solution of phenol 10 (60 mg, 0.11 mmol) in THF (3 mL) was added Cs₂CO₃ (72 mg, 0.22 mmol) and triflate (66 mg, 0.22 mmol). The reaction mixture was stirred at room temperature for 4 h and the solvent was evaporated under reduced pressure. The residue was partitioned between EtOAc and saturated NaCl The organic phase was dried with Na₂SO₄, filtered, and evaporated under reduced pressure. The crude product was purified by column chromatography on silica gel (5% 2-propanol/CH₂Cl₂) to give the diethylphosphonate (38 mg, 49%, **GS 11**) as a white solid.

### Example Section J

### Example 1

Triflate 1: To a solution of A (4 g, 6.9 mmol) in THF (30 mL) and CH₂Cl₂ (10 mL) was added Cs₂CO₃ (2.7 g, 8 mmol) and N-phenyltrifluoromethanesulfonimide (2.8 g, 8.0 mmol) and stirred at room temperature for 16 h. The reaction mixture was concentrated under reduced pressure. The residue wsa partitioned between CH₂Cl₂ and saturated brine twice. The organic phase was dried over sodium sulfate and used for next reaction without further purification.

### Example 2

Aldehyde 2: A solution of crude above triflate 1 (-6.9 mmol) in DMF (20 mL) was degassed (high vacumn for 5 min, argon purge, repeat 3 times). To this solution were quickly added Pd(OAc)₂ (120 mg, 266 µmol) and bis(diphenylphosphino-propane (dppp ,220 mg, 266 µmol), and heated to 70°C. To this reaction mixture was rapidly introduced carbon monoxide, and stirred at room temperature under an atmopheric pressure of carbon monoxide, followed by slow addition of TEA (5.4 mL, 38 mmol) and triethylsilane (3 mL, 18 mmol). The resultant mixture was stirred at 70°C for 16 h, then cooled to room temperature, concentrated under reduced pressure, partitioned between CH₂Cl₂ and saturated brine. The organic phase was concentrated under reduced pressure and purified on silica gel column to afford aldehyde 2 (2.1 g, 51%) as white solid.

### Example 3

Compounds 3a-3e: Respresentative Procedure, 3c: A solution of aldehyde 2 (0.35 g, 0.59 mmol), L-alanine isopropyl ester hydrochloride (0.2 g, 1.18 mmol), glacial acetic acid (0.21 g, 3.5 mmol) in 1,2-dichloroethane (10 mL) was stirred at room temperature for 16 h, followed by addition of sodium cyanoborohydride (0.22 g, 3.5 mmol) and methanol (0.5 mL). The resulting solution was stirred at room temperature for one h. The reaction mixture was washed with sodium bicarbonate solution, saturated brine, and chromatographed on silica gel to afford 3c (0.17 g, 40%). ¹H NMR (CDCl₃): δ 7.72 (d, 2H), 7.26 (d, 2H), 7.20 (d, 2H), 7.0 (d, 2H), 5.65 (d, 1H), 4.90-5.30 (m, 3H), 3.53-4.0 (m overlapping s, 13H), 3.31 (q, 1H), 2.70-3.20 (m, 7H), 1.50-1.85 (m, 3H), 1.25-1.31 (m, 9H), 0.92 (d, 3H), 0.88 (d, 3H). MS: 706 (M+1).

| **Compound** | **R₁** | **R₂** | **Amino Acid** |
|---|---|---|---|
| 3a | Me | Me | Ala |
| 3b | Me | Et | Ala |
| 3c | Me | iPr | Ala |
| 3d | Me | Bn | Ala |
| 3e | iPr | Et | Val |

### Example 4

Sulfonamide 1: To a solution of crude amine A (1 g, 3 mmol) in CH₂Cl₂ was added TEA (0.6 g, 5.9 mmol) and 3-methoxybenzenesulfonyl chloride (0.6 g, 3 mmol). The resulting solution was stirred at room temperature for 5 h, and evaporated under reduced pressure. The residue was chromatographed on silica gel to afford sulfonamide 1 (1.0 g, 67%).

### Example 5

Amine 2: To a 0°C cold solution of sulfonamide 1 (0.85 g, 1.6 mmol) in CH₂Cl₂ (40 mL) was treated with BBr₃ in CH₂Cl₂ (10 mL of 1 M solution, 10 mmol). The solution was stirred at 0°C 10 min and then warmed to room temperature and stirred for 1.5 h. The reaction mixture was quenched with CH₃OH, concentrated under reduced pressure, azeotroped with CH₃CN three times. The crude amine 2 was used for next reaction without further purification.

### Example 6

Carbamate 3: A solution of crude amine 2 (0.83 mmol) in CH₃CN (20 mL) and was treated with (3R, 3aR, 6aS)-hexahydrofuro[2, 3-*b*]furan-2-yl 4-nitrophenyl carbonate (245 mg, 0.83 mmol, prepared according to Ghosh et al., J. Med. Chem 1996, 39, 3278.) and N,N-dimethylaminopyridine (202 mg, 1.7 mmol). After stirring for 16 h at room temperature, the reaction solvent was evaporated under reduced pressure and the residue was partitioned between CH₂Cl₂ and saturated NaHCO₃ three times. The organic phase was evaporated under reduced pressure. The residue was purified by chromatography on silica gel affording the carbamate 3 (150 mg, 33%) as a solid.

### Example 7

Diethylphosphonate 4: To a solution of carbamate 3(30 mg, 54 µmol) in THF (5 mL) was added Cs₂CO₃ (54 mg, 164 µmol) and triflate # (33 mg, 109 µmol). After stirring the reaction mixture for 30 min at room temperature, additional Cs₂CO₃ (20 mg, 61 µmol) and triflate (15 mg, 50 µmol) were added and the mixture was stirred for 1 more hour. The reaction mixture was evaporated under reduced pressure and the residue was partitioned between CH₂Cl₂ and water. The organic phase was dried (Na₂SO₄), filtered and evaporated under reduced pressure. The crude product was chromatographed on silica gel and repurified by HPLC (50% CH₃CN-50% H₂O on C18 column) to give the diethylphosphonate 4 (15 mg, 39%). ¹H NMR (CDCl₃): δ 7.45 (m, 3H), 7.17-7.30 (m, 6H), 5.64 (d, 1H), 5.10 (d, 1H), 5.02 (q, 1H), 4.36 (d, 2H), 4.18-4.29 (2 q overlap, 4H), 3.60-3.98 (m, 7H), 2.70-3.10 (m, 7H), 1.80-1.90 (m, 1H), 1.44-1.70 (m, 2H + H2O), 1.38 (t, 6H), 0.94 (d, 3H), 0.90 (d, 3H). ³¹P NMR (CDCl₃): 18.7 ppm; MS (ESI) 699 (M + H).

### Example 8

Dibenzylphosphonate 5: To a solution of carbamate 3 (100 mg, 182 µmol) in THF (10 mL) was added Cs₂CO₃ (180 mg, 550 µmol) and dibenzylhydroxymethyl phosphonate triflate, Section A, Scheme 2, Compound 9, (150 mg, 360 µmol). After stirring the reaction mixture for 1 h at room temperature, the reaction mixture was evaporated under reduced pressure and the residue was partitioned between CH₂Cl₂ and water. The organic phase was dried (Na₂SO₄), filtered and evaporated under reduced pressure. The residue was purified by HPLC (50% CH₃CN-50% H₂O on C18 column) to give the dibenzylphosphonate 5 (110 mg, 72%). ¹H NMR (CDCl₃): δ 7.41 (d, 2H), 7.35 (s, 10 H), 7.17-7.30 (m, 6H), 7.09-7.11 (m, 1H), 5.64 (d, 1H), 4.90-5.15 (m, 6H), 4.26 (d, 2H), 3.81-3.95 (m, 4H), 3.64-3.70 (m, 2H), 2.85-3.25 (m, 7H), 1.80-1.95 (m, 1H), 1.35-1.50 (m, 1H), 0.94 (d, 3H), 0.91 (d, 3H). ³¹P NMR (CDCl₃) δ 19.4 ppm; MS (ESI): 845 (M + Na), 1666 (2M + Na).

### Example 9

Phosphonic acid 6: A solution of dibenzylphosphonate 5 (85 mg, 0.1 mmol) was dissolved in MeOH (10 mL) treated with 10% Pd/C (40 mg) and stirred under H₂ atmosphere (balloon) overnight. The reaction was purged with N₂, and the catalyst was removed by filtration through celite. The filtrate was evaporated under reduced pressure to afford phosphonic acid 6 (67 mg, quantitatively). ¹H NMR (CD₃OD): δ 7.40-7.55 (m, 3H), 7.10-7.35 (m, 6H), 5.57 (d, 1H), 4.32 (d, 2H), 3.90-3.95 (m, 1H), 3.64-3.78 (m, 5H), 3.47 (m, 1H), 2.85-3.31 (m, 5H), 2.50-2.60 (m, 1H), 2.00-2.06 (m, 1H), 1.46-1.60 (m, 1H), 1.30-1.34 (m, 1H), 0.9 (d, 3H), 0.90 (d, 3H). ³¹P NMR (CD₃OD): 16.60 ppm; MS (ESI): 641 (M - H).

### Example 10

Sulfonamide 1: To a solution of crude amine A (0.67 g, 2 mmol) in CH₂Cl₂ (50 mL) was added TEA (0.24 g, 24 mmol) and crude 3-acetoxy-4-methoxybenzenesulfonyl chloride (0.58 g, 2.1 mmol, was prepared according to Kratzl et al., Monatsh. Chem.1952, 83, 1042-1043), and the solution was stirred at room temperature for 4 h, and evaporated under reduced pressure. The residue was chromatographed on silica gel to afford sulfonamide 1 (0.64 g, 54%). MS: 587 (M + Na), 1150 (2M + Na)

Phenol 2: Sulfonamide 1 (0.64 g, 1.1 mmol) was treated with saturated NH₃ in MeOH (15 mL) at room temperature for 15 min., then evaporated under reduced pressure. The residue was purified on silica gel column to afford phenol 2 (0.57 g, 96%).

### Example 11

Dibenzylphosphonate 3a: To a solution of phenol 2 (0.3 g, 0.57 mmol) in THF (8 mL) was added Cs₂CO₃ (0.55 g, 1.7 mmol)) and dibenzylhydroxymethyl phosphonate triflate (0.5 g, 1.1 mmol). After stirring the reaction mixture for 1 h at room temperature, the reaction mixture was quenched with water and partitioned between CH₂Cl₂ and saturated ammonium chloride aqueous solution. The organic phase was dried (Na₂SO₄), filtered and evaporated under reduced pressure. The residue was chromatographed on silica gel (40% EtOAc/ 60% hexane) to give the dibenzylphosphonate 3a (0.36 g, 82%). ¹H NMR (CDCl₃): δ 7.20-7.40 (m, 17H), 6.91 (d, 1H), 5.10-5.25 (2 q(ab) overlap, 4H), 4.58-4.70 (m, 1H), 4.34 (d, 2H), 3.66-3.87 (m + s, 5H), 2.85-3.25 (m, 6H), 1.80-1.95 (m, 1H), 1.58 (s, 9H), 0.86-0.92 (2d, 6H).

### Example 12

Diethylphosphonate 3b: To a solution of phenol 2 (0.15 g, 0.28 mmol) in THF (4 mL) was added Cs₂CO₃ (0.3 g, 0.92 mmol)) and diethylhydroxymethyl phosphonate triflate (0.4 g, 1.3 mmol). After stirring the reaction mixture for 1 h at room temperature, the reaction mixture was quenched with water and partitioned between CH₂Cl₂ and saturated NaHCO₃ aqueous solution. The organic phase was dried (Na₂SO₄), filtered and evaporated under reduced pressure. The residue was chromatographed on silica gel (1% CH₃OH-CH₂Cl₂) to give the diethylphosphonate 3b (0.14 g, 73%).

### Example 13

Amine 4a: To a solution of 3a (0.35 g, 0.44 mmol) in CH₂Cl₂ (10 mL) was treated with TFA (0.75 g, 6.6 mmol) at room temperature for 2 h. The reaction was evaporated under reduced pressure, azeotroped with CH₃CN twice, dried to afford crude amine 4a. This crude 4a was used for next reaction without further purification.

### Example 14

Amine 4b: To a solution of 3b (60 mg, 89 µmol) in CH₂Cl₂ (1 mL) was treated with TFA (0.1 mL, 1.2 mmol) at room temperature for 2 h. The reaction was evaporated under reduced pressure, azeotroped with CH₃CN twice, dried to afford crude amine 4b (68 mg). This crude 4b was used for next reaction without further purification.

### Example 15

Carbamate 5a: An ice-cold solution of crude amine 4a (0.44 mmol) in CH₃CN (10 mL) and was treated with (3R, 3aR, 6aS)-hexahydrofuro[2, 3-*b*]furan-2-yl 4-nitrophenyl carbonate (120 mg, 0.4 mmol) and N,N-dimethylaminopyridine (DMAP, 110 mg, 0.88 mmol). After 4 h, more DMAP (0.55 g, 4.4 mmol) was added to the reaction mixture. After stirring for 1.5 h at room temperature, the reaction solvent was evaporated under reduced pressure and the residue was partitioned between CH₂Cl₂ and saturated NaHCO₃. The organic phase was evaporated under reduced pressure. The residue was purified by chromatography on silica gel affording the crude carbamate 5a (220 mg) containing some p-nitrophenol The crude 5a was repurified by HPLC (50% CH₃CN /50% H₂O) to afford pure carbamate 5a (176 mg, 46%, 2 steps). ¹H NMR (CDCl₃): δ 7.20-7.36 (m, 1H), 6.94 (d, 1H), 5.64 (d, 1H), 5.10-5.25 (2 q(ab) overlap, 4H), 4.90-5.10 (m, 1H), 4.90 (d, 1H), 4.34 (d, 2H), 3.82-3.91 (m + s, 6H), 3.63-3.70 (m, 3H), 2.79-3.30 (m, 7H), 1.80-1.90 (m, 1H), 1.40-1.50 (m, 1H), 0.94 (d, 3H), 0.89 (d, 3H). ³¹P NMR (CDCl₃): 17.2 ppm.

### Example 16

Carbamate 5b: An ice-cold solution of crude amine 4b (89 µmol)) in CH₃CN (5 mL) and was treated with (3R, 3aR, 6aS)-hexahydrofuro[2, 3-*b*]furan-2-yl 4-nitrophenyl carbonate (26mg, 89 µmol) and N,N-dimethylaminopyridine (DMAP, 22 mg, 0.17 mmol). After 1 h at 0°C more DMAP (10 mg. 82 µmol) was added to the reaction mixture. After stirring for 2 h at room temperature, the reaction solvent was evaporated under reduced pressure and the residue was partitioned between CH₂Cl₂ and saturated NaHCO₃. The organic phase was evaporated under reduced pressure. The residue was purified by HPLC (C18 column, 45% CH₃CN/55% H₂O) to afford pure carbamate 5b (18.8 mg, 29%, 3 steps). ¹H NMR (CDCl₃): δ 7.38 (d, 2H), 7.20-7.36 (m, 6H), 7.0 (d, 1H), 5.64 (d, 1H), 4.96-5.03 (m, 2H), 4.39 (d, 2H), 4.20-4.31 (2q overlap, 4H) 3.80-4.00 ((s overlap with m, 7H), 3.60-3.73 (m, 2H), 3.64-3.70 (m, 2H), 2.85-3.30 (m, 7H), 1.80-1.95 (m, 1H), 1.55-1.75 (m, 1H), 1.35-1.50 (s overlap with m, 7H), 0.94 (d, 3H), 0.88 (d, 3H). ³¹P NMR (CDCl₃): 18.1ppm.

### Example 17

Phosphonic acid 6: A solution of dibenzylphosphonate 5a (50 mg, 58 µmol) was dissolved in MeOH (5 mL) and EtOAc (3 mL) and treated with 10% Pd/C (25 mg) and was stirred at room temperature under H₂ atmosphere (balloon) for 8 h. The catalyst was filtered off. The filtrate was concentrated and redissolved in MeOH (5 mL), treated with 10% Pd/C (25 mg) and was stirred at room temperature under H₂ atmosphere (balloon) overnight. The catalyst was filtered off. The filtrate was evaporated under reduced pressure to afford phosphonic acid 6 (38 mg, quantitatively). ¹H NMR (CD₃OD) : δ 7.42 (m, 1H), 7.36 (s, 1H), 7.10-7.25 (m, 6H), 5.58 7 (d, 1H), 4.32 (d, 2H), 3.90 (s, 3H), 3.60-3.80 (m, 6H), 3.38 (d, 1H), 2.85-3.25 (m, 5H), 2.50-2.60 (m, 1H), 1.95-2.06 (m, 1H), 1.46-1.60 (m, 1H), 1.30-1.40 (m, 1H), 0.93(d, 3H), 0.89 (d, 3H). ³¹P NMR (CD₃OD): 14.8 ppm; MS (ESI): 671 (M- H).

### Example 18

Amine 7: To a 0°C cold solution of diethylphosphonate 3b (80 mg, 0.118 mmol) in CH₂Cl₂ was treated with BBr₃ in CH₂Cl₂ (0.1 mL of 1 M solution, 1 mmol). The solution was stirred at 0°C 10 min and then warmed to room temperature and stirred for 3 h. The reaction mixture was concentrated under reduced pressure. The residue was redissolved in CH₂Cl₂ (containing some CH₃OH concentrated, azeotroped with CH₃CN three times. The crude amine 7 was used for next reaction without further purification.

### Example 19

Carbamate 8: An ice-cold solution of crude amine 7 (0.118 mmol) in CH₃CN (5 mL) and was treated with (3R, 3aR, 6aS)-hexahydrofuro[2, 3-*b*]furan-2-yl 4-nitrophenyl carbonate (35 mg, 0.118 mmol) and N,N-dimethylaminopyridine (29 mg, 0.24mmol), warmed to room temperature. After stirring for 1 h at room temperature, more DMAP (20 mg, 0.16 mmol) was added to reaction mixture. After 2 h stirred at room temperature, the reaction solvent was evaporated under reduced pressure and the residue was partitioned between CH₂Cl₂ and saturated NaHCO₃. The organic phase was evaporated under reduced pressure. The residue was purified by HPLC on C18 (CH₃CN-55%H₂O) to afford the desired carbamate 8 (11.4 mg, 13.4%) as an off-white solid. ¹H NMR (CDCl₃): δ 7.20-7.40 (m, 7H), 7.00 (d, 1H), 5.64 (d, 1H), 5.00-5.31 (m, 2H), 4.35 (d, 2H), 4.19-4.30 (2q overlap, 4H), 3.80-4.00 (m, 4H), 3.68-3.74 (m, 2H), 3.08-3.20 (m, 3H), 2.75-3.00 (m, 4H), 1.80-1.90 (m, 1H), 1.55-1.75 (m, 1H), 1.38 (t, 6H), 0.91 (2d overlap, 6H). ³¹P NMR (CD₃OD): δ19.5 ppm.

### Example Section K

### Example 1

Monophenyl-monolactate 3: A mixture of monoacid 1 (0.500 g, 0.7 mmol), alcohol 2 (0.276 g, 2.09 mmol) and dicyclohexylcarbodiimide (0.431 g, 2.09 mmol) in dry pyridine (4 mL) was placed into a 70°C oil bath and heated for two hours. The reaction was monitored by TLC assay (SiO₂, 70% ethyl acetate in hexanes as eluent, product R_{f}= 0.68, visualization by UV). The reaction contents were cooled to ambient temperature with the aid of a cool bath and diluted with dichloromethane (25 mL). TLC assay may show presence of starting material. The diluted reaction mixture was filtered to remove solids. The filtrate was then cooled to 0°C and charged with 0.1 N HCl (10 mL). The pH 4 mixture was stirred for 10 minutes and poured into separatory funnel to allow the layers to separate. The lower organic layer was collected and dried over sodium sulfate. The drying agent was filtered off and the filtrate concentrated to an oil via rotary evaporator (< 30°C warm bath). The crude product oil was purified on pretreated silica gel (deactivated using 10% methanol in dichlorormethane followed by rinse with 60% ethyl acetate in dichloromethane). The product was eluted with 60% ethyl acetate in dichloromethane to afford the product monophenyl-monolactate 3 as a white foam (0.497 g, 86% yield). ¹H NMR (CDCl₃) δ 7.75 (d, 2H), 7.40-7.00 (m, 14H), 5.65 (d, 1H), 5.20-4.90 (m, 4H), 4.70 (d, 1H), 4.55-4.50 (m, 1H), 4.00-3.80 (m, 4H), 3.80-3.60 (m, 3H), 3.25-2.75 (m, 7H), 1.50 (d, 3H), 1.30-1.20 (m, 7H), 0.95 (d, 3H), 0.85 (d, 3H). ³¹P NMR (CDCl₃) δ 16.2, 13.9.

### Example 2

Monophenyl-monoamidate 5: A mixture of monoacid 1 (0.500 g, 0.70 mmol), amine hydrochloride 4 (0.467 g, 2.78 mmol) and dicyclohexylcarbodiimide (0.862 g, 4.18 mmol) in dry pyridine (8 mL) was placed into a 60°C oil bath, and heated for one hour (at this temperature, product degrades if heating continues beyond this point). The reaction was monitored by TLC assay (SiO₂, 70% ethyl acetate in hexanes as eluent, product R*_{f}*= 0.39, visualization by UV). The contents were cooled to ambient temperature and diluted with ethyl acetate (15 mL) to precipitate a white solid. The mixture was filtered to remove solids and the filtrate was concentrated via rotary evaporator to an oil. The oil was diluted with dichloromethane (20 mL) and washed with 0.1 N HCl (2 x 20 mL), water (1 x 20 mL) and dilute sodium bicarbonate (1 x 20 mL). The organic layer was dried over sodium sulfate, filtered, and concentrated to an oil via rotary evaporator. The crude product oil was dissolved in dichloromethane (10 mL). Hexane was slowly charged to the stirring solution until cloudiness persisted. The cloudy mixture was stirred for a few mintues until TLC assay showed that the dichloromethane/hexane layer contained no product. The dichloromethane/hexanes layer was decanted and the solid was further purified on silica gel first pretreated with 10% methanol in ethyl acetate and rinsed with 50% ethyl acetate in hexanes. The product 5 was eluted with 50% ethyl acetate in hexanes to afford a white foam (0.255 g, 44% yield) upon removal of solvents. ¹H NMR (CDCl₃) δ 7.75 (d, 2H), 7.40-7.15 (m, 10H), 7.15-7.00 (t, 2H), 5.65 (d, 1H), 5.10-4.90 (m, 3H), 4.50-4.35 (m, 2H), 4.25-4.10 (m, 1H), 4.00-3.60 (m, 8H), 3.20-2.75 (m, 7H), 1.40-1.20 (m, 11H), 0.95 (d, 3H), 0.85 (d, 3H). ³¹P NMR (CDCl₃) δ 19.1, 18.0.

### Example 3

Bisamidate 8: A solution of triphenylphosphine (1.71 g, 6.54 mmol) and aldrithiol (1.44 g, 6.54 mmol) in dry pyridine (5 mL), stirred for at least 20 minutes at room temperature, was charged into a solution of diacid 6 (1.20 g, 1.87 mmol) and amine hydrochloride 7 (1.30 g, 7.47 mmol) in dry pyridine (10 mL). Diisopropylethylamine (0.97 g, 7.48 mmol) was then added to this combined solution and the contents were stirred at room temperature for 20 hours. The reaction was monitored by TLC assay (SiO₂, 5:5:1 ethyl acetate/hexanes/methanol as eluent, product R*_{f}*= 0.29, visualization by UV). The reaction mixture was concentrated via rotary evaporator and dissolved in dichloromethane (50 mL). Brine (25 mL) was charged to wash the organic layer. The aqueous layer was back extracted with dichloromethane (1 x 50 mL). The combined organic layers were dried over sodium sulfate, filtered, and concentrated via rotary evaporator to afford an oil. The crude product oil was purified on silica gel using 4% isopropanol in dichloromethane as eluent. The combined fractions containing the product may have residual amine contamination. If so, the fractions were concentrated via rotary evaporator and further purified by silica gel chromatography using a gradient of 1:1 ethyl acetate/hexanes to 5:5:1 ethyl acetate/hexanes/methanol solution as eluent to afford the product 8 as a foam (0.500 g, 30% yield).

### Example 4

Diacid 6: A solution of dibenzylphosphonate 9 (8.0 g, 9.72 mmol) in ethanol (160 mL) and ethyl acetate (65 mL) under a nitrogen atmosphere and at room temperature was charged 10% Pd/C (1.60 g, 20 wt%). The mixture was stirred and evacuated by vacuum and purged with hydrogen several times. The contents were then placed under atmospheric pressure of hydrogen via a balloon. The reaction was monitored by TLC assay (SiO₂, 7:2.5:0.5 dichloromethane/methanol/ammonium hydroxide as eluent, product R*_{f}* = 0.05, visualization by UV) and was judged complete in 4 to 5 hours. The reaction mixture was filtered through a pad of celite to remove Pd/C and the filter cake rinsed with ethanol/ethyl acetate mixture (50 mL). The filtrate was concentrated via rotary evaporation followed by several co-evaporations using ethyl acetate (3 x 50 mL) to remove ethanoL The semi-solid diacid 6, free of ethanol, was carried forward to the next step without purification.

### Example 5

Diphenylphosphonate 10: To a solution of diacid 6 (5.6 g, 8.71 mmol) in pyridine (58 mL) at room temperature was charged phenol (5.95 g, 63.1 mmol). To this mixture, while stirring, was charged dicyclohexylcarbodiimide (7.45 g, 36.0 mmol). The resulting cloudy, yellow mixture was placed in a 70-80°C oil bath. The reaction was monitored by TLC assay (Si0₂, 7:2.5:0.5 dichloromethane/methanol/ammonium hydroxide as eluent, diacid R*_{f}*= 0.05, visualization by UV for the disappearance of starting materiaL SiO₂, 60% ethyl acetate in hexanes as eluent, diphenyl R*_{f}*= 0.40, visualization by UV) and was judged complete in 2 hours. To the reaction mixture was charged isopropyl acetate (60 mL) to produce a white precipitation. The slurry was filtered through a pad of celite to remove the white precipitate and the filter cake rinsed with isopropyl acetate (25 mL). The filtrate was concentrated via rotary evaporator. To the resulting yellow oil was charged a premixed solution of water (58 mL) and 1N HCl (55 mL) followed by isopropyl acetate (145 mL). The mixture was stirred for one hour in an ice bath. After separating the layers, the aqueous layer was back extracted with ethyl acetate (2 x 50 mL). The combined organic layers were dried over sodium sulfate, filtered, and concentrated via rotary evaporator. The crude product oil was purified by silica gel column chromatography using 50% ethyl acetate in hexanes as eluent to afford the product 10 as a white foam (3.52 g, 51% yield). ¹H NMR (CDCl₃) δ 7.75 (d, 2H), 7.40-7.20 (m, 15H), 7.10 (d, 2H), 5.65 (d, 1H), 5.10-4.90 (m, 2H), 4.65 (d, 2H), 4.00-3.80 (m, 4H), 3.75-3.65 (m, 3H), 3.25-2.75 (m, 7H), 1.90-1.75 (m, 1H), 1.70-1.60 (m, 1H), 1.50-1.40 (m, 1H), 0.90 (d, 3H), 0.85 (d, 3H). ³¹P NMR (CDCl₃) δ 10.9.

### Example 6

Monophenyl 1: To a solution of diphenyl 10 (3.40 g, 4.28 mmol) in acetonitrile (170 mL) at 0°C was charged 1N sodium hydroxide (4.28 mL). The reaction was monitored by TLC assay (SiO₂, 7:2.5:0.5 dichloromethane/methanol/ammonium hydroxide as eluent, diphenyl *R_{f}=* 0.65, visualization by UV for the disappearance of starting material. Product monophenyl R*_{f}=* 0.80, visualization by UV). Additional 1N NaOH was added (if necessary) until the reaction was judged complete. To the reaction contents at 0°C was charged Dowex H⁺ (Dowex 50WX8-200) (4.42 g) and stirred for 30 minutes at which time the pH of the mixture reached pH 1 (monitored by pH paper). The mixture was filtered to remove the Dowex resin and the filtrate was concentrated via rotary evaporation (water bath < 40°C). The resulting solution was co-evaporated with toluene to remove water (3 x 50 mL). The white foam was dissolved in ethyl acetate (8 mL) followed by slow addition of hexanes (16 mL) over 30 minutes to induce precipitation. A premixed solution of 2:1 hexnaes/ethyl acetate solution (39 mL) was charged to the precipitated material and stirred. The product 1 was filtered and rinsed with premixed solution of 2:1 hexanes/ethyl acetate solution (75 mL) and dried under vacuum to afford a white powder (2.84 g, 92% yield). ¹H NMR (CD₃OD) δ 7.80 (d, 2H), 7.40-7.30 (m, 2H), 7.20-7.15 (m, 11H), 5.55 (d, 1H), 4.50 (d, 2H), 3.95-3.85 (m, 1H), 3.80-3.60 (m, 5H), 3.45 (bd, 1H). 3.25-3.15 (m, 2H), 3.00-2.80 (m, 3H), 2.60-2.45 (m, 1 H), 2.10-1.95 (m, 2H), 1.85-1.60 (m, 2H), 1.50-1.40 (m, 1 H), 1.40-1.30 (m, 1H), 0.95 (d, 3H), 0.85 (d, 3H). ³¹P NMR (CDC1₃) δ 13.8. The monophenyl product 1 is sensitive to silica gel. On contact with silica gel 1 converts to an unknown compound possessing ³¹P NMR chemical shift of 8 ppm. However, the desired monophenyl product 1 can be regenerated by treatment of the unknown compound with 2.5 M NaOH in acetonitrile at 0°C for one hour followed by Dowex H⁺ treatment as described above.

### Example 7

Dibenzylphosphonate 9: To a solution of phenol 11 (6.45 g, 11.8 mmol) in tetrahydrofuran (161 mL) at room temperature was charged triflate reagent 12 (6.48 g, 15.3 mmol). Cesium carbonate (11.5 g, 35.3 mmol) was added and the mixture was stirred and monitored by TLC assay (SiO₂, 5% methanol in dichloromethane as eluent, dibenzyl product R*_{f}*= 0.26, visualization by UV or ninhydrin stain and heat). Additional Cs₂CO₃ was added until the reaction was judged complete. To the reaction contents was charged water (160 mL) and the mixture extracted with ethyl acetate (2 x 160 mL). The combined organic layer was dried over sodium sulfate, filtered, and concentrated via rotary evaporator to afford a viscous oil.

The crude oil was purified by silica gel column chromatography using a gradient of 100% dichloromethane to 1% methanol in dichloromethane to afford product 9 as a white foam (8.68 g, 90% yield). ¹H NMR (CDCl₃) δ 7.75 (d, 2H), 7.40-7.20 (m, 16H), 6.95 (d, 2H), 5.65 (d, 1H), 5.20-4.90 (m, 6H), 4.25 (d, 2H), 4.00-3.80 (m, 4H), 3.75-3.65 (m, 3H), 3.20-2.75 (m, 7H), 1.90-1.75 (m, 1H), 1.30-1.20 (m, 1H), 0.90 (d, 3H), 0.85 (d, 3H). ³¹P NMR (CDCl₃) δ 19.1.

### Example 7a

Hydroxyphenylsulfonamide 14: To a solution of methoxyphenylsulfonamide 13 (35.9 g, 70.8 mmol) in dichloromethane (3.5 L) at 0°C was charged boron tribromide (1M in DCM, 40.1 mL, 425 mmol). The reaction content was allowed to warm to room temperature, stirred over two hours, and monitored by TLC assay (SiO₂, 10% methanol in dichloromethane as eluent, dibenzyl product R_{f}= 0.16, visualization by UV). To the contents at 0°C was slowly charged propylene oxide (82 g, 1.42 mmol). Methanol (200 mL) was added and the reaction mixture was concentrated via rotary evaporator to afford a viscous oil. The crude product mixture was purified by silica gel column chromatography using 10% methanol in dichloromethane to afford the product 14 as a foam (22 g, 80% yield). ¹H NMR (DMSO) δ 7.60 (d, 2H), 7.30-7.20 (m, 5H), 6.95 (d, 2H), 3.90-3.75 (m, 1H), 3.45-3.20 (m, 5H), 3.00-2.55 (m, 5H), 2.50-2.40 (m, 1H), 1.95-1.85 (m, 1H), 0.85 (d, 3H), 0.80 (d, 3H).

### Example 8

Cisfuran carbamate 16: To a solution of amine 14 (20.4 g, 52.0 mmol) in acetonitrile (600 mL) at room temperature was charged dimethylaminopyridine (13.4 g, 109 mmol) followed by cisfuran *p*-nitrophenylcarbonate reagent 15 (14.6 g, 49.5 mmol). The resulting solution was stirred at room temperature for at least 48 hours and monitored by TLC assay (SiO₂, 10% methanol in dichloromethane as eluent, cisfuran product R*_{f}* = 0.34, visualization by UV). The reaction mixture was concentrated via rotary evaporator. The crude product mixture was purified by silica gel column chromatography using a gradient of 60% ethyl acetate in hexanes to 70% ethyl acetate in hexanes to afford the product 16 as a solid (18.2 g, 64% yield). ¹H NMR (DMSO) δ 10.4 (bs, 1H), 7.60 (d, 2H), 7.30-7.10 (m, 6H), 6.95 (d, 2H), 5.50 (d, 1H), 4.85 (m, 1H), 3.85 (m, 1H), 3.70 (m, 1H), 3.65-3.50 (m, 4H), 3.30 (d, 1H), 3.05-2.95 (m, 2H), 2.80-2.65 (m, 3H), 2.50-2.40 (m, 1H), 2.00-1.90 (m, 1H), 1.45-1.20 (m, 2H), 0.85 (d, 3H), 0.80 (d, 3H).

### Example Section L

### Example 1

Monobenzyl phosphonate 2 A solution of dibenzylphosphonate 1(150 mg, 0.175mmol) was dissolved in toluene (1 mL), treated with DABCO (20 mg, 0.178 mmol) and was refluxed under N2 atmosphere (balloon) for 3 h. The solvent was removed and the residual was dissolved in aqueous HCl (5%). The aqueous layer was extracted with ethyl acetate and the organic layer was dried over sodium sulfate. After evaporation to yield the monobenzyl phosphonate 2 (107 mg, 80%) as a white powder. ¹H NMR (CD₃OD) δ 7.75 (d, J = 5.4 Hz, 2H), 7.42-7.31 (m, 5H) 7.16 (d, J = 5.4 Hz, 2H), 7.01 (d, J = 5.4 Hz, 2H), 6.86 (d, J = 5.4 Hz, 2H), 5.55 (d, J = 3.3 Hz, 1H), 5.14 (d, J = 5.1 Hz, 2H), 4.91 (m, 1H), 4.24-3.66 (m overlapping s, 11H), 3.45 (m, 2H), 3.14-2.82 (m, 6H), 2.49 (m, 1H), 2.01 (m, 1H), 1.51-1.34 (m, 2H), 0.92 (d, J = 3.9 Hz, 3H), 0.87 (d, J = 3.9 Hz, 3H); ³¹P NMR (CD₃OD) δ 20.5; MS (ESI) 761 (M-H).

### Example 2

Monobenzyl, ethyl phosphonate 3 To a solution of monobenzyl phosphonate 2 (100 mg, 0.13 mmol) in dry THF (5 mL) at room temperature under N₂ was added Ph₃P (136 mg, 0.52 mmol) and ethanol (30 µL, 0.52 mmol). After cooled to 0°C, DEAD (78µL, 0.52 mmol) was added. The mixture was stirred for 20 h at room temperature. The solvent was evaporated under reduced pressure and the residue was purified by using chromatograph on silica gel (10% to 30% ethyl acetate / hexane) to afford the monobenzyl, ethyl phosphonate 3 (66 mg, 64%) as white solid. ¹H NMR (CDCl₃) 7.70 (d, J = 8.7 Hz, 2H), 7.43-734 (m, 5H) 7.14 (d, J = 8.4 Hz, 2H), 7.01 (d, J = 8.7Hz, 2H), 6.84 (d, J = 8.4 Hz, 2H), 5.56 (d, J = 5.4 Hz, 1H), 5.19 (d, J = 8.7 Hz, 2H), 5.00 (m, 2H), 4.22-3.67 (m overlapping s, 13H), 3.18-2.76 (m, 7H), 1.82-1.54 (m, 3H), 1.33 (t, J = 7.0 Hz, 3H), 0.92 (d, J = 6.6 Hz, 3H), 0.88 (d, J = 6.6 Hz, 3H); ³¹P NMR (CDCl₃) δ 19.8; MS (ESI) 813 (M+Na).

### Example 3

Monoethyl phosphonate 4 A solution of monobenzyl, ethyl phosphonate 3 (60 mg) was dissolved in EtOAc (2 mL), treated with 10% Pd/C (6 mg) and was stirred under H₂ atmosphere (balloon) for 2h. The catalyst was removed by filtration through celite. The filtered was evaporated under reduced pressure, the residue was triturated with ether and the solid was collected by filtration to afford the monoethyl phosphonate 4 (50 mg, 94%) as white solid. ¹H NMR (CD₃OD) 7.76 (d, J = 8.7 Hz, 2H), 7.18 (d, J = 8.4 Hz, 2H), 7.01 (d, J = 8.7Hz, 2H), 6.89 (d, J = 8.4 Hz, 2H), 5.58 (d, J = 5.4 Hz, 1H), 5.90 (m, 1H), 4.22-3.67 (m overlapping s, 13H), 3.18-2.50 (m, 7H), 1.98(m, 1H), 1.56 (m, 2H), 1.33 (t, J = 6.9 Hz, 3H), 0.92 (d, J = 6.6Hz, 3H), 0.87 (d, J = 6.6 Hz, 3H); ³¹P NMR (CD₃OD) δ 18.7; MS (ESI) 700 (M-H).

### Example 4

Monophenyl, ethyl phosphonate 5 To a solution of phosphonic acid 11 (800 mg, 1.19 mmol) and phenol (1.12 g, 11.9 mmol) in pyridine (8 mL) was added ethanol (69 µL, 1.19 mmol) and 1, 3-dicyclohexylcarbodiimide (1g, 4.8 mmol). The solution was stirred at 70°C for 2h. The reaction mixture was cooled to room temperature, then diluted with ethyl acetate (10 mL) and filtered. The filtrate was evaporated under reduced pressure to remove pyridine. The residue was dissolved in ethyl acetate and the organic phase was separated and washed with brine, dried over MgSO4, filtered and concentrated. The residue was purified by chromatography on silica gel to give monophenyl, ethyl phosphonate 5 (600 mg, 65%) as white solid. ¹H NMR (CDCl₃) 7.72 (d, J = 9 Hz, 2H), 7.36-7.18 (m, 5H), 7.15 (d, J = 8.7 Hz, 2H), 6.98 (d, J = 9Hz, 2H), 6.87 (d, J = 8.7 Hz, 2H), 5.64 (d, J = 5.4 Hz, 1H), 5.00 (m, 2H), 4.34 (m, 4H), 3.94-3.67 (m overlapping s, 9H), 3.18-2.77 (m, 7H), 1.82-1.54 (m, 3H), 1.36 (t, J = 7.2 Hz, 3H), 0.92 (d, J = 6.6 Hz, 3H), 0.87 (d, J = 6.6 Hz, 3H); ³¹P NMR (CDCl₃) δ 16.1; MS (ESI) 799 (M+Na).

### Example 5

Sulfonamide 6 To a suspension of epoxide 5 (3 g, 8.12 mmol) in 2-propanol (30 mL) was added isobutylamine (8 mL, 81.2 mmol) and the solution was stirred at 80°C for 1 h. The solution was evaporated under reduced pressure and the crude solid was dissolved in CH₂Cl₂ (40 mL) and cooled to 0°C. TEA (2.3 mL, 16.3mmol) was added followed by the addition of 4-nitrobenzenesulfonyl chloride (1.8 g, 8.13 mmol) in CH₂Cl₂ (5 mL) and the solution was stirred for 30 min at 0°C, warmed to room temperature and evaporated under reduced pressure. The residue was partitioned between EtOAc and saturated NaHCO₃. The organic phase was washed with saturated NaCl, dried over Na₂SO₄, filtered and evaporated under reduced pressure. The crude product was recrystallized from EtOAc/hexane to give the sulfonamide 6 (4.6 g, 91%) as an off-white solid. MS (ESI) 650 (M+Na).

### Example 6

Phenol 7 A solution of sulfonamide 6 (4.5 g, 7.1 mmol) in CH₂Cl₂ (50 mL) at 0°C was treated with BBr₃ (1M in CH₂Cl₂, 50mL). The solution was stirred at 0°C to room temperature for 48h. CH₃OH (10 mL) was carefully added. The solvent was evaporated under reduced pressure and the residue was partitioned between EtOAc and saturated NaHCO₃. The organic phase washed with saturated NaCl, dried over Na₂SO₄, filtered, and evaporated under reduced pressure. The crude product was purified by chromatography on silica gel (10% - McOH/CH₂Cl₂) to give the phenol 7 (2.5 g, 80%) as an off white solid. MS (ESI) 528 (M+H).

### Example 7

Carbamate 8 A solution of sulfonamide 7 (2.5 g, 5.7 mmol) in CH₃CN (100 mL) and was treated with proton-sponge (3 g, 14 mmol) and followed by (3R, 3aR, 6aS)-hexahydrofuro[2, 3-b]furan-2-yl 4-nitrophenyl carbonate (1.7 g, 5.7 mmol) at 0°C. After stirring for 48h at room temperature, the reaction solvent was evaporated under reduced pressure and the residue was partitioned between EtOAc and 10% HCl. The organic phase was washed with saturated NaCl, dried over Na₂SO₄, filtered, and evaporated under reduced pressure. The crude product was purified by chromatography on silica gel (10% MeOH/CH₂Cl₂) affording the carbamate 8 (2.1g, 62 %) as a white solid. MS (ESI) 616 (M+Na).

### Example 8

Diethylphosphonate 9 To a solution of carbamate 8 (2.1 g, 3.5 mmol) in CH₃CN (50 mL) was added Cs₂CO₃ (3.2 g, 9.8 mmol) and diethyltriflate (1.6g, 5.3 mmol). The mixture was stirred at room temperature for 1h. After removed the solvent, the residue was partitioned between EtOAc and saturated NaCl The organic phase was dried over Na₂SO₄, filtered, and evaporated under reduced pressure. The crude product was chromatographed on silica gel (1% to 5% MeOH /CH₂Cl₂) to afford the diethylphosphonate 9 as a white solid: ¹H NMR (CDCl₃) δ 8.35 (d, J = 9 Hz, 2H), 7.96 (d, J = 9 Hz, 2H), 7.13 (d, J = 8.4 Hz, 2H), 6.85 (d, J = 8.4 Hz, 2H), 5.63 (d, J = 5.1 Hz, 1H), 5.18-5.01 (m, 2H), 4.27-4.17 (m, 6H), 3.94-3.67 (m, 7H), 3.20-2.73 (m, 7H), 1.92-1.51 (m, 3H), 1.35 (t, J = 7.2 Hz, 6H), 0.88-0.85 (m, 6H); ³¹P NMR (CDCl₃) δ 19.2; MS (ESI) 756 (M+Na).

### Example 9

Amine 10 A solution of diethylphosphonate 9 (1 g) was dissolved in EtOH (100 mL), treated with 10% Pd/C (300 mg) and was stirred under H₂ atmosphere (balloon) for 3h. The reaction was purged with N₂, and the catalyst was removed by filtration through celite. After evaporation of the filtrate, the residue was triturated with ether and the solid was collected by filtration to afford the amine 10 (920 mg, 96%) as a white solid. ¹H NMR (CDCl₃) ¹H NMR (CDCl₃) δ 7.41 (d, J = 8.4 Hz, 2H), 7.17 (d, J = 8.4 Hz, 2H), 6.88 (d, J = 8.4 Hz, 2H), 6.68 (d, J = 8.4 Hz, 2H), 5.67 (d, J = 5.1 Hz, 1H), 5.13-5.05 (m, 2H), 4.42 (s, 2H), 4.29-4.20 (m, 6H), 4.00-3.69 (m, 7H), 3.00-2.66 (m, 7H), 1.80-1.69 (m, 3H), 1.38 (m, 6H), 0.94 (d, J = 6.4 Hz, 3H), 0.86 (d, J = 6.4 Hz, 6H); ³¹P NMR (CDCl₃) δ 19.4; MS (ESI) 736 (M+Na).

| **Compound** | **R₁** | **R₂** |
|---|---|---|
| 16a | Gly-Et | Gly-Et |
| 16b | Gly-Bu | Gly-Bu |
| 16j | Phe-Bu | Phe-Bu |
| 16k | NHEt | NHEt |

### Example 10

Synthesis of Bisamidates 16a. A solution of phosphonic acid 11 (100 mg, 0.15 mmol) L-alanine ethyl ester hydrochloride (84 mg, 0.6 mmol) was dissolved in pyridine (5 mL) and the solvent was distilled under reduced pressure at 40-60°C. The residue was treated with a solution of Ph₃P (118 mg, 0.45 mmol) and 2,2'-dipyridyl disulfide (99 mg, 0.45 mmol) in pyridine (1 mL) stirring for 20h at room temperature. The solvent was evaporated under reduced pressure and the residue was chromatographed on silica gel (1% to 5% 2-propano/CH₂Cl₂). The purified product was suspended in ether and was evaporated under reduced pressure to afford bisamidate 16a (90 mg, 72%) as a white solid: ¹H NMR (CDCl₃) δ 7.72 (d, J = 8.7 Hz, 2H), 7.15 (d, J = 8.7 Hz, 2H), 7.01 (d, J = 8.7 Hz, 2H), 6.87 (d, J = 8.7 Hz, 2H), 5.68 (d, J = 5.1 Hz,, 1H), 5.05 (m, 1H), 4.25 (d, J = 9.9 Hz, 2H), 4.19 (q, 4H), 3.99-3.65 (m overlapping s, 13H,), 3.41 (m, 1H), 3.20-2.81 (m, 7H), 1.85-1.60 (m, 3H), 1.27 (t, J = 7.2 Hz, 6H), 0.93 (d, J = 6.3 Hz, 3H), 0.89 (d, J = 6.3 Hz, 3H); ³¹P NMR (CDCl₃) δ 21.8; MS (ESI) 843 (M+H).

### Example 11

Synthesis of Bisamidates 16b. A solution of phosphonic acid 11 (100 mg, 0.15 mmol) L-alanine n-butyl ester hydrochloride (101 mg, 0.6 mmol) was dissolved in pyridine (5 mL) and the solvent was distilled under reduced pressure at 40-60°C. The residue was treated with a solution of Ph₃P (118 mg, 0.45 mmol) and 2,2'-dipyridyl disulfide (99 mg, 0.45 mmol) in pyridine (1 mL) stirring for 20h at room temperature. The solvent was evaporated under reduced pressure and the residue was chromatographed on silica gel (1% to 5% 2-propanol/CH₂Cl₂). The purified product was suspended in ether and was evaporated under reduced pressure to afford bisamidate 16b (100 mg, 74%) as a white solid: ¹H NMR (CDCl₃) δ 7.72 (d, J = 9 Hz, 2H), 7.15 (d, J = 9 Hz, 2H), 7.01 (d, J = 9 Hz, 2H), 6.87 (d, J = 9 Hz, 2H), 5.67 (d, J = 5.4 Hz, 1H), 5.05 (m, 1H), 4.96 (m, 1H), 4.25 (d, J = 9.9 Hz, 2H), 4.11 (t, J = 6.9 Hz, 4H), 3.99-3.71 (m overlapping s, 13H,), 3.41 (m, 1H), 3.20-2.80 (m, 7H), 1.87-1.60 (m, 7H), 1.42 (m, 4H), 0.96-0.88 (m, 12H); ³¹P NMR (CDCl₃) δ 21.8; MS (ESI) 890 (M+H).

### Example 12

Synthesis of Bisamidates 16j. A solution of phosphonic acid 11 (100 mg, 0.15 mmol) L-phenylalanine n-butyl ester hydrochloride (155 mg, 0.6 mmol) was dissolved in pyridine (5 mL) and the solvent was distilled under reduced pressure at 40-60°C. The residue was treated with a solution of Ph₃P (118 mg, 0.45 mmol) and 2,2'-dipyridyl disulfide (99 mg, 0.45 mmol) in pyridine (1 mL) stirring for 36h at room temperature. The solvent was evaporated under reduced pressure and the residue was chromatographed on silica gel (1% to 5% 2-propanol/CH₂Cl₂). The purified product was suspended in ether and was evaporated under reduced pressure to afford bisamidate 16j (106 mg, 66%) as a white solid. ¹H NMR (CDCl₃) δ 7.72 (d, J = 8.7 Hz, 2H), 7.31-7.10 (m, 12H), 7.01 (d, J = 9 Hz, 2H), 6.72 (d, J = 8.7 Hz, 2H), 5.67 (d, J= 5.1 Hz, 1H), 5.05 (m, 1H), 4.96 (m, 1H), 4.35-3.98 (m., 7H), 3.90-3.61 (m overlapping s, 10H,), 3.19-2.78 (m, 11H), 1.87-1.25 (m, 11H), 0.96-0.88 (m, 12H); ³¹P NMR (CDCl₃) δ 19.3; MS (ESI) 1080 (M+H).

### Example 13

Synthesis of Bisamidates 16k. A solution of phosphonic acid 11 (80 mg, 0.12 mmol), ethylamine (0.3 mL,2M in THF, 0.6 mmol) was dissolved in pyridine (5 mL) and the solvent was distilled under reduced pressure at 40-60°C. The residue was treated with a solution of Ph₃P (109 mg, 0.42 mmol) and 2,2'-dipyridyl disulfide (93 mg, 0.42 mmol) in pyridine (1 mL) stirring for 48h at room temperature. The solvent was evaporated under reduced pressure and the residue was chromatographed on silica gel (1% to 5% 2-propanol/CH₂Cl₂). The purified product was suspended in ether and was evaporated under reduced pressure to afford bisamidate 16k (60 mg, 70%) as a white solid: ¹H NMR (CDCl₃) δ 7.72 (d, J =8.7 Hz, 2H), 7.15 (d, J = 8.7 Hz, 2H), 7.01 (d, J = 8.7 Hz, 2H), 6.87 (d, J = 8.7 Hz, 2H), 5.67 (d, J = 5.1 Hz, 1H), 5.05-4.95 (m, 2H), 4.15 (d, J = 9.6 Hz, 2H), 3.99-3.72 (m overlapping s, 9H,), 3.18-2.81 (m, 11H), 2.55 (br, 1H), 1.85-1.65 (m, 3H), 1.18 (t, J = 7.2 Hz, 6H), 0.93 (d, J = 6.3 Hz, 3H), 0.89 (d, J = 6.3 Hz, 3H); ³¹P NMR (CDCl₃) δ 21.6; MS (ESI) 749 (M+Na).

| **Compound** | **R₁** | **R₂** |
|---|---|---|
| 30a | OPh | Ala-Me |
| 30b | OPh | Ala-Et |
| 30c | OPh | (D)-Ala-iPr |
| 30d | OPh | Ala-Bu |
| 30e | OBn | Ala-Et |

### Example 14

Monoamidate 30a (R1 = OPh, R2 = Ala-Me) To a flask was charged with monophenyl phosphonate 29 (75 mg, 0.1 mmol), L-alanine methyl ester hydrochloride (4.0 g, 22 mmol) and 1, 3-dicyclohexylcarbodiimide (84 mg, 0.6 mmol), then pyridine (1 mL) was added under N2. The resulted mixture was stirred at 60 - 70°C for 2 h, then cooled to room temperature and diluted with ethyl acetate. The mixture was filtered and the filtrate was evaporated. The residue was partitioned between ethyl acetate and HCl (0.2 N), the ethyl acetate phase was washed with water and NaHCO₃, dried over Na₂SO₄ filtered and concentrated. The residue was purified by chromatography on silica gel (ethyl acetate/hexane 1:5) to give 30a (25 mg, 30%) as a white solid. ¹H NMR (CDCl₃) δ 7.72 (d, J = 8.7 Hz, 2H), 7.73-7.24 (m, 5H) 7.19-7.15 (m, 2H), 7.01 (d, J = 8.7 Hz, 2H), 6.90-6.83 (m, 2H), 5.65 (d, J = 5.1 Hz, 1H), 5.01 (m, 2H), 4.30 (m, 2H), 3.97-3.51 (m overlapping s, 12H), 3.20-2.77 (m, 7H), 1.81 (m, 1H), 1.58 (m, 3H), 0.92 (d, J = 6.3 Hz, 3H), 0.88 (d, J = 6.3 Hz, 3H); ³¹P NMR (CDCl₃) δ 20.4 and 19.3; MS (ESI) 856 (M+Na).

### Example 15

Monoamidate 30b (R1 = OPh, R2 = Ala-Et) was synthesized in the same manner in 35% yield. ¹H NMR (CDCl₃) δ 7.72 (d, J = 8.7 Hz, 2H), 7.73-7.24 (m, 5H) 7.19-7.15 (m, 2H), 7.01 (d, J = 8.7 Hz, 2H), 6.90-6.83 (m, 2H), 5.65 (d, J = 5.4 Hz, 1H), 5.01 (m, 3H), 4.30 -3.67 (m overlapping s, 14H), 3.18-2.77 (m, 7H), 1.81-1.35 (m, 6H), 1.22 (m, 3H), 0.92 (d, J = 6.3 Hz, 3H), 0.88 (d, J = 6.3 Hz, 3H); ³¹P NMR (CDCl₃) δ 20.4 and 19.3; MS (ESI) 870 (M+Na).

### Example 16

Monoamidate 30c (R1 = OPh, R2 = (D)-Ala-iPr) was synthesized in the same manner in 52% yield. Isomer A ¹H NMR (CDCl₃) δ 7.72 (d, J = 8.7 Hz, 2H), 7.73-7.24 (m, 5H) 7.19-7.15 (m, 2H), 7.01 (d, J = 8.7 Hz, 2H), 6.90-6.83 (m, 2H), 5.66 (m,, 1H), 5.01 (m, 3H), 4.30 -3.67 (m overlapping s, 14H), 3.18-2.77 (m, 7H), 1.81-1.35 (m, 6H), 1.23 (m, 6H), 0.92 (d, J = 6.3 Hz, 3H), 0.88 (d, J = 6.3 Hz, 3H); ³¹P NMR (CDCl₃) δ 20.4; MS (ESI) 884 (M+Na). Isomer B ¹H NMR (CDCl₃) δ 7.72 (d, J = 8.7 Hz, 2H), 7.73-7.24 (m, 5H) 7.19-7.15 (m, 2H), 7.01 (d, J = 8.7 Hz, 2H), 6.90-6.83 (m, 2H), 5.66 (m,, 1H), 5.01 (m, 3H), 4.30 -3.67 (m overlapping s, 14H), 3.18-2.77 (m, 7H), 1.81-1.35 (m, 6H), 1.23 (m, 6H), 0.92 (d, J = 6.3 Hz, 3H), 0.88 (d, J = 6.3 Hz, 3H); ³¹P NMR (CDCl₃) δ 19.3; MS (ESI) 884 (M+Na).

### Example 17

Monoamidate 30d (R1 = OPh, R2 = Ala-Bu) was synthesized in the same manner in 25% yield. ¹H NMR (CDC1₃) δ 7.72 (d, J = 8.7 Hz, 2H), 7.73-7.24 (m, 5H) 7.19-7.15 (m, 2H), 7.01 (d, J = 8.7 Hz, 2H), 6.90-6.83 (m, 2H), 5.65 (d, J = 5.4 Hz, 1H), 5.01 (m, 3H), 4.30 -3.67 (m overlapping s, 16H), 3.18-2.77 (m, 7H), 1.81-1.35 (m, 8H), 1.22 (m, 3H), 0.92 (d, J = 6.3 Hz, 3H), 0.88 (d, J = 6.3 Hz, 3H); ³¹P NMR (CDCl₃) δ 20.4 and 19.4; MS (ESI) 898 (M+Na).

### Example 18

Monoamidate 30e (R1 = OBn, R2 = Ala-Et) To a flask was charged with monobenzyl phosphonate 2 (76 mg, 0.1 mmol), L-alanine methyl ester hydrochloride (4.0 g, 22 mmol) and 1, 3-dicyclohexylcarbodiimide (84 mg, 0.6 mmol), then pyridine (1 mL) was added under N2. The resulted mixture was stirred at 60 - 70°C for 2 h, then cooled to room temperature and diluted with ethyl acetate. The mixture was filtered and the filtrate was evaporated. The residue was partitioned between ethyl acetate and HCl (0.2 N), the ethyl acetate phase was washed with water and NaHCO₃, dried over Na₂SO₄ filtered and concentrated. The residue was purified by chromatography on silica gel (ethyl acetate / hexane 1:5) to give 30a (25 mg, 30%) as a white solid. ¹H NMR (CDCl₃) δ 7.72 (d, J = 8.7 Hz, 2H), 7.38-7.34 (m, 5H), 7.13 (d, J = 8.7 Hz, 2H), 7.00 (d, J = 8.7 Hz, 2H), 6.86-6.80 (m, 2H), 5.65 (d, J = 5.4 Hz, 1H), 5.15-5.01 (m, 5H), 4.30 -3.67 (m overlapping s, 14H), 3.18-2.77 (m, 7H), 1.81-1.35 (m, 6H), 1.22 (m, 3H), 0.92 (d, J = 6.3 Hz, 3H), 0.88 (d, J = 6.3 Hz, 3H); ³¹P NMR (CDCl₃) δ 23.3 and 22.4; MS (ESI) 884 (M+Na).

| **Compound** | **R₁** | **R₂** |
|---|---|---|
| 31a | OPh | Lac-iPr |
| 31b | OPh | Lac-Et |
| 31c | OPh | Lac-Bu |
| 31d | OPh | (R)-Lac-Me |
| 31e | OPh | (R)-Lac-Et |

### Example 19

Monolactate 31a (R1 = OPh, R2 = Lac-iPr): To a flask was charged with monophenyl phosphonate 29 (1.5 g, 2 mmol), isopropyl-(s)-lactate (0.88 mL, 6.6 mmol) and 1, 3-dicyclohexylcarbodiimide (1.36 g, 6.6 mmol), then pyridine (15 mL) was added under N₂. The resulted mixture was stirred at 60 - 70°C for 2 h, then cooled to room temperature and diluted with ethyl acetate. The mixture was filtered and the filtrate was evaporated. The residue was washed with ethyl acetate and the combined organic phase was washed with NH₄Cl, brine and water, dried over Na₂SO₄, filtered and concentrated. The residue was purified by chromatography on silica gel (ethyl acetate / CH₂Cl₂ 1:5) to give 31a (1.39g, 81 %) as a white solid. Isomer A ¹H NMR (CDCl₃) δ 7.72 (d, J = 8.7 Hz, 2H), 7.73-7.19 (m, 5H), 7.15 (d, J = 8.4 Hz, 2H), 7.00 (d, J = 8.7 Hz, 2H), 6.92 (d, J = 8.4 Hz, 2H), 5.65 (d, J= 5.4 Hz, 1H), 5.15-5.00 (m, 4H), 4.56-4.44 (m, 2H), 3.96 -3.68 (m overlapping s, 9H), 3.13-2.78 (m, 7H), 1.81-1.23 (m, 6H), 1.22 (m, 6H), 0.92 (d, J = 6.6 Hz, 3H), 0.88 (d, J = 6.6 Hz, 3H); ³¹P NMR (CDCl₃) δ 17.4; MS (ESI) 885 (M+Na). Isomer B ¹H NMR (CDCl₃) δ 7.72. (d, J = 8.7 Hz, 2H), 7.73-7.19 (m, 5H), 7.14 (d, J = 8.4 Hz, 2H), 7.00 (d, J = 8.7 Hz, 2H), 6.88 (d, J = 8.4 Hz, 2H), 5.64 (d, J= 5.4 Hz, 1H), 5.15-5.00 (m, 4H), 4.53 -4.41 (m, 2H), 3.96 -3.68 (m overlapping s, 9H), 3.13-2.78 (m, 7H), 1.81-1.23 (m, 6H), 1.22 (m, 6H), 0.92 (d, J = 6.6 Hz, 3H), 0.88 (d, J = 6.6 Hz, 3H); ³¹P NMR (CDCl₃) δ 15.3; MS (ESI) 885 (M+Na).

### Example 20

Monolactate 31b (R1 = OPh, R2 = Lac-Et) was synthesized in the same manner in 75% yield. ¹H NMR (CDCl₃) δ 7.72 (d, J = 8.7 Hz, 2H), 7.73-7.14 (m, 7H), 6.99 (d, J = 8.7 Hz, 2H), 6.88 (d, J = 8.7 Hz, 2H), 5.63 (m, 1H), 5.19-4.95 (m, 3H), 4.44-4.40 (m, 2H), 4.17-4.12 (m, 2H), 3.95 -3.67 (m overlapping s, 9H), 3.15-2.77 (m, 7H), 1.81-1.58 (m, 6H), 1.23 (m, 3H), 0.91 (d, J = 6.6 Hz, 3H), 0.87 (d, J = 6.6 Hz, 3H); ³¹P NMR (CDCl₃) δ 17.5 and 15.4; MS (ESI) 872 (M+Na).

### Example 21

Monolactate 31c (R1 = OPh, R2 = Lac-Bu) was synthesized in the same manner in 58% yield. Isomer A ¹H NMR (CDCl₃) δ 7.72 (d, J = 8.7 Hz, 2H), 7.73-7.19 (m, 5H), 7.14 (d, J = 8.4 Hz, 2H), 7.00 (d, J = 8.7 Hz, 2H), 6.90 (d, J = 8.4 Hz, 2H), 5.63 (d, J= 5.4 Hz, 1H), 5.15-5.00 (m, 3H), 4.56-4.51 (m, 2H), 4.17-4.10 (m, 2H), 3.95 -3.67 (m overlapping s, 9H), 3.10-2.77 (m, 7H), 1.81-1.23 (m, 10H), 1.23 (m, 6H), 0.91 (d, J = 6.6 Hz, 3H), 0.87 (d, J = 6.6 Hz, 3H); ³¹P NMR (CDCl₃) δ 17.3; MS (ESI) 899 (M+Na). Isomer B ¹H NMR (CDCl₃) δ 7.72 (d, J = 8.7 Hz, 2H), 7.73-7.19 (m, 5H), 7.14 (d, J = 8.4 Hz, 2H), 7.00 (d, J = 8.7 Hz, 2H), 6.90 (d, J = 8.4 Hz, 2H), 5.64 (d, J= 5.4 Hz, 1H), 5.15-5.00 (m, 3H), 4.44 -4.39 (m, 2H), 4.17-4.10 (m, 2H), 3.95 -3.67 (m overlapping s, 9H), 3.10-2.77 (m, 7H), 1.81-1.23 (m, 10H), 1.23 (m, 6H), 0.91 (d, J = 6.6 Hz, 3H), 0.87 (d, J = 6.6 Hz, 3H); ³¹P NMR (CDCl₃) δ 15.3; MS (ESI) 899 (M+Na).

### Example 22

Monolactate 31d (R1 = OPh, R2 = (R)-Lac-Me): To a stirred solution of monophenyl phosphonate 29 (100 mg, 0.13 mmol) in 10 mL of THF at room temperature under N₂ was added methyl-(S)-lactate (54 mg, 0.52 mmol) and Ph₃P (136 mg g" 0.52 mmol), followed by DEAD (82µL, 0.52 mmol). After 2 h, the solvent was removed under reduced pressure, and the resulting crude mixture was purified by chromatography on silica gel (ethyl acetate / hexane 1:1) to give 31d (33 mg, 30%) as a white solid. ¹H NMR (CDCl₃) δ 7.72 (d, J = 8.7 Hz, 2H), 7.73-7.14 (m, 7H), 6.99 (d, J = 8.7 Hz, 2H), 6.88 (d, J = 8.7 Hz, 2H), 5.63 (m, 1H), 5.19-4.95 (m, 3H), 4.44-4.40 (m, 2H), 3.95 -3.64 (m overlapping s, 12H), 3.15-2.77 (m, 7H), 1.81-1.55 (m, 4H), 0.91 (d, J = 6.6 Hz, 3H), 0.87 (d, J = 6.6 Hz, 3H); ³¹P NMR (CDCl₃) δ 17.4 and 15.3; MS (ESI) 857 (M+Na).

### Example 23

Monolactate 31e (R1 = OPh, R2 = (R)-Lac-Et): To a stirred solution of monophenyl phosphonate 29 (50 mg, 0.065 mmol) in 2.5 mL of THF at room temperature under N₂ was added ethyl-(s)-lactate (31 mg, 0.52 mmol) and Ph₃P (68 mg g, 0.26 mmol), followed by DEAD (41µL, 0.52 mmol). After 2 h, the solvent was removed under reduced pressure, and the resulting crude mixture was purified by chromatography on silica gel (ethyl acetate / hexane 1:1) to give 31e (28 mg, 50%) as a white solid. ¹H NMR (CDCl₃) δ 7.72 (d, J = 8.7 Hz, 2H), 7.73-7.14 (m, 7H), 6.99 (d, J = 8.7 Hz, 2H), 6.85(m, 2H), 5.63 (m, 1H), 5.19-4.95 (m, 3H), 4.44-4.40 (m, 2H), 4.17-4.12 (m, 2H), 3.95 -3.67 (m overlapping s, 9H), 3.15-2.77 (m, 7H), 1.81-1.58 (m, 6H), 1.23 (m, 3H), 0.91 (d, J = 6.6 Hz, 3H), 0.87 (d, J = 6.6 Hz, 3H); ³¹P NMR (CDCl₃) δ 17.5 and 15.4; MS (ESI) 872 (M+Na).

### Example 24

Monolactate 32 (R1 = OBn, R2 = (S)-Lac-Bn): To a stirred solution of monobenzyl phosphonate 2 (76 mg, 0.1 mmol) in 0.5 mL of DMF at room temperature under N₂ was added benzyl-(s)-lactate (27 mg, 0.15 mmol) and PyBOP (78 mg, 0.15mmol), followed by DIEA (70µL, 0.4 mmol). After 3 h, the solvent was removed under reduced pressure, and the resulting crude mixture was purified by chromatography on silica gel (ethyl acetate / hexane 1:1) to give 32 (46 mg, 50%) as a white solid. ¹H NMR (CDCl₃) δ 7.72 (d, J = 8.7 Hz, 2H), 7.38-7.44 (m, 10H), 7.13 (d, J = 8.4 Hz, 2H), 6.99 (d, J = 8.7 Hz, 2H), 6.81(m, 2H), 5.63 (d, J = 5.1 Hz, 1H), 5.23-4.92 (m, 7H), 4.44-22 (m, 2H), 3.96 -3.67 (m overlapping s, 9H:), 3.15-2.77 (m, 7H), 1.81-1.58 (m, 6H), 0.93 (d, J = 6.3 Hz, 3H), 0.88 (d, J = 6.3 Hz, 3H); ³¹P NMR (CDCl₃) δ 20.8 and 19.6; MS (ESI) 947 (M+Na).

### Example 25

Monolactate 33 (R1 = OBn, R2 = (R)-Lac-Bn): To a stirred solution of monobenzyl phosphonate 2 (76 mg, 0.1 mmol) in 5 mL of THF at room temperature under N₂ was added benzyl-(s)-lactate (72 mg, 0.4 mmol) and Ph₃P (105 mg g, 0.4mmol), followed by DEAD (60µL, 0.4 mmol). After 20 h, the solvent was removed under reduced pressure, and the resulting crude mixture was purified by chromatography on silica gel (ethyl acetate / hexane 1:1) to give 33 (44 mg, 45%) as a white solid. ¹H NMR (CDCl₃) δ 7.72 (d, J = 8.7 Hz, 2H), 7.38-7.44 (m, 10H), 7.13 (m, 2H), 6.99 (d, J = 8.7 Hz, 2H), 6.81(m, 2H), 5.63 (m, 1H), 5.23-4.92 (m, 7H), 4.44-22 (m, 2H), 3.96 -3.67. (m overlapping s, 9H), 3.15-2.77 (m, 7H), 1.81-1.58 (m, 6H), 0.93 (d, J = 6.3 Hz, 3H), 0.88 (d, J = 6.3 Hz, 3H); ³¹P NMR (CDCl₃) δ 20.8 and 19.6; MS (ESI) 947 (M+Na).

### Example 26

Monophosphonic acid 34: A solution of monobenzyllactate 32 (20 mg) was dissolved in EtOH/ EtOAc (3 mL/1 mL), treated with 10% Pd/C (4 mg) and was stirred under H2 atmosphere (balloon) for 1.5 h. The catalyst was removed by filtration through celite. The filtered was evaporated under reduced pressure, the residue was triturated with ether and the solid was collected by filtration to afford the monophosphonic acid 33 (15 mg, 94%) as a white solid. ¹H NMR (CD₃OD) δ 7.76 (d, J = 8.7 Hz, 2H), 7.18 (d, J = 8.7 Hz, 2H), 7.08 (d, J = 8.7 Hz, 2H), 6.90 (d, J = 8.7 Hz, 2H), 5.69 (d, J = 5.7 Hz, 1H), 5.03-4.95 (m, 2H), 4.20 (m, 2H), 3.90 -3.65 (m overlapping s, 9H), 3.41 (m, 2H), 3.18-2.78 (m, 5H), 2.44 (m, 1H), 2.00 (m, 1H), 1.61-1.38 (m, 5H), 0.93 (d, J = 6.3 Hz, 3H), 0.88 (d, J = 6.3 Hz, 3H); ³¹P NMR (CD₃OD) δ 18.0; MS (ESI) 767 (M+Na).

### Example 27

Monophosphonic acid 35: A solution of monobenzyllactate 33(20 mg) was dissolved in EtOH (3 mL), treated with 10% Pd/C (4 mg) and was stirred under H2 atmosphere (balloon) for 1h. The catalyst was removed by filtration through celite. The filtered was evaporated under reduced pressure, the residue was triturated with ether and the solid was collected by filtration to afford the monophosphonic acid 35 (15 mg, 94%) as a white solid. ¹H NMR (CD₃OD) δ 7.76 (d, J = 8.7 Hz, 2H), 7.18 (d, J = 8.7 Hz, 2H), 7.08 (d, J = 8.7 Hz, 2H), 6.90 (d, J = 8.7 Hz, 2H), 5.69 (d, J = 5.7 Hz, 1H), 5.03-4.95 (m, 2H), 4.20 (m, 2H), 3.90 -3.65 (m overlapping s, 9H), 3.41 (m, 2H), 3.18-2.78 (m, 5H), 2.44 (m, 1H), 2.00 (m, 1H), 1.61-1.38 (m, 5H), 0.93 (d, J = 6.3 Hz, 3H), 0.88 (d, J = 6.3 Hz, 3H); ³¹P NMR (CD₃OD) δ 18.0; MS (ESI) 767 (M+Na).

### Example 28

Synthesis of Bislactate 36: A solution of phosphonic acid 11 (100 mg, 0.15 mmol) isopropyl-(S)-lactate (79 mg, 0.66 mmol) was dissolved in pyridine (1 mL) and the solvent was distilled under reduced pressure at 40-60°C. The residue was treated with a solution of Ph₃P (137 mg, 0.53 mmol) and 2,2'-dipyridyl disulfide (116 mg, 0.53 mmol) in pyridine (1 mL) stirring for 20h at room temperature. The solvent was evaporated under reduced pressure and the residue was chromatographed on silica gel (1% to 5% 2-propanol/CH₂Cl₂). The purified product was suspended in ether and was evaporated under reduced pressure to afford bislactate 36 (42 mg, 32%) as a white solid: ¹H NMR (CDCl₃) δ 7.72 (d, J = 8.7 Hz, 2H), 7.14 (d, J = 8.7 Hz, 2H), 7.01 (d, J = 8.7 Hz, 2H), 6.89 (d, J = 8.7 Hz, 2H), 5.66 (d, J = 5.1 Hz, 1H), 5.05 (m, 3H), 4.25 (d, J = 9.9 Hz, 2H), 4.19 (q, 4H), 3.99-3.65 (m overlapping s, 9H,), 3.41 (m, 1H), 3.20-2.81 (m, 7H), 1.85-1.60 (m, 3H),1.58 (m, 6H), 1.26 (m, 12H), 0.93 (d, J = 6.3 Hz, 3H), 0.89 (d, J = 6.3 Hz, 3H); ³¹P NMR (CDCl₃) δ 21.1; MS (ESI) 923 (M+Na).

### Example 29

Triflate derivative **1**: A THF-CH₂Cl₂ solution (30mL-10 mL) of **8** (4 g, 6.9 mmol), cesium carbonate (2.7 g, 8 mmol), and N-phenyltrifluoromethane sulfonimide (2.8 g, 8 mmol) was reacted overnight. The reaction mixture was worked up, and concentrated to dryness to give crude triflate derivative **1.**

Aldehyde **2:** Crude triflate **1** (4.5 g, 6.9 mmole) was dissolved in DMF (20 mL), and the solution was degassed (high vacuum for 2 min, Ar purge, repeat 3 times). Pd(OAc)2 (0.12 g, 0.27 mmol), and bis(diphenylphosphino)propane (dppp, 0.22 g, 0.27 mmol) were added and the solution was heated to 70°C. Carbon monoxide was rapidly bubbled through the solution, then under 1 atmosphere of carbon monoxide. To this solution were slowly added TEA (5.4 mL, 38 mmol), and triethylsilane (3 mL, 18 mmol). The resulting solution was stirred overnight at room temperature. The reaction mixture was worked up, and purified on silica gel column chromatograph to afford aldehyde **2** (2.1 g, 51 %). (Hostetler, et al. J. Org. Chem., 1999. 64,178-185).

Lactate prodrug **4:** Compound 4 is prepared as described above procedure for 3a-e by the reductive amination between 2 and 3 with NaBH₃CN in 1,2-dichloroethane in the presence of HOAc.

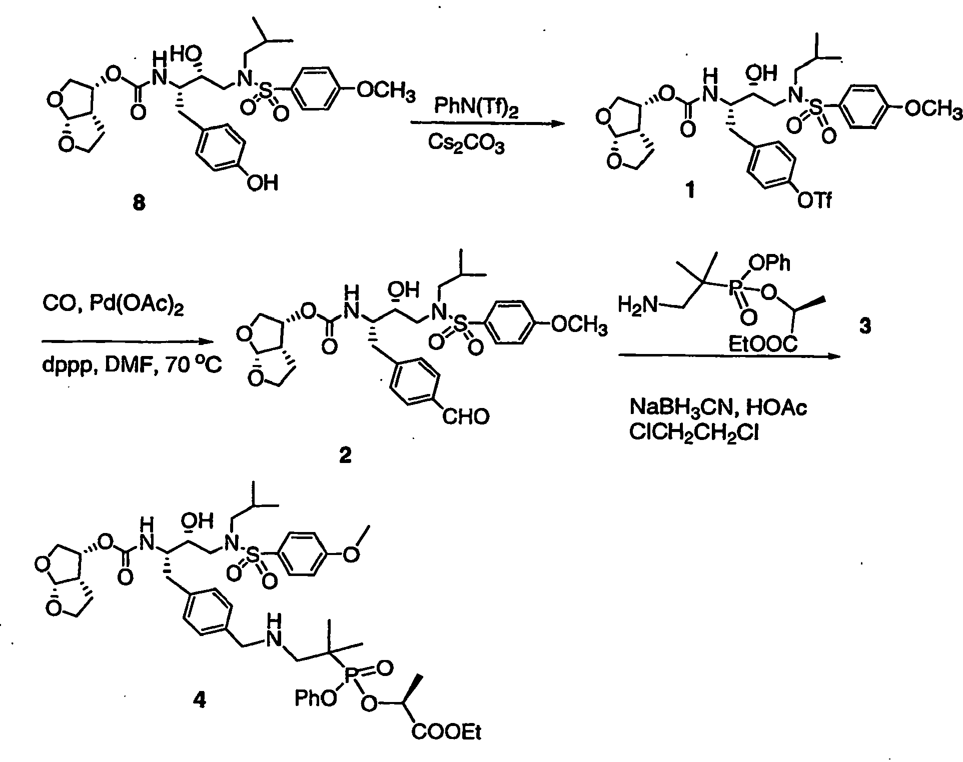

### Example 30

Preparation of compound **3** Diethyl (cyano(dimethyl)methyl) phosphonate 5: A THF solution (30 mL) of NaH (3.4 g of 60% oil dispersion, 85 mmole) was cooled to -10°C, followed by the addition of diethyl (cyanomethyl)phosphonate (5g, 28.2 mmol) and iodomethane (17 g, 112 mmol). The resulting solution was stirred at-10°C for 2 hr, then 0°C for 1 hr, was worked up, and purified to give dimethyl derivative **5** (5 g, 86%). Dietyl (2-amino-1,1-diemthyl-ethyl)phosphonate **6:** Compound **5** was reduced to amine derivative **6** by the described procedure (J. Med. Chem. 1999, 42, 5010-5019). A ethanol (150 mL) and 1N HCI aqueous solution (22 mL) of 5 (2.2 g, 10.7 mmol) was hydrogenated at 1 atmosphere in the presence of PtO₂ (1.25 g) at room temperature overnight. The catalyst was filtered through a celite pad. The filtrate was concentrated to dryness, to give crude **6** (2.5g, as HCl salt).

2-Amino-1,1-dimethyl-ethyl phosphonic acid **7:** A CH₃CN (30 mL) of crude **6** (2.5 g) was cooled to 0°C, and treated with TMSBr (8 g, 52 mmol) for 5 hr. The reaction mixture was stirred with methanol for 1.5 hr at room temperature, concentrated, recharged with methanol, concentrated to dryness to give crude **7** which was used for next reaction without further purification.

Lactate phenyl (2-amino-l,l-diemthyl-ethyl)phosphonate **3:** Compound **3** is synthesized according to the procedures described in a previous scheme for the preparation of a lactate phenyl 2-aminoethyl phosponate. Compound **7** is protected with CBZ, followed by the reaction with thionyl chloride at 70°C. The CBZ protected dichlorodate is reacted phenol in the presence of DIPEA. Removal of one phenol, follow by coupling with ethyl L-lactate leads N-CBZ-2-amino-1,1-dimethyl-ethyl phosphonated derivative. Hydrogenation of N-CBZ derivative at 1 atmosphere in the presence of 10% Pd/C and 1 equivalent of TFA affords compound **3** as TFA salt.

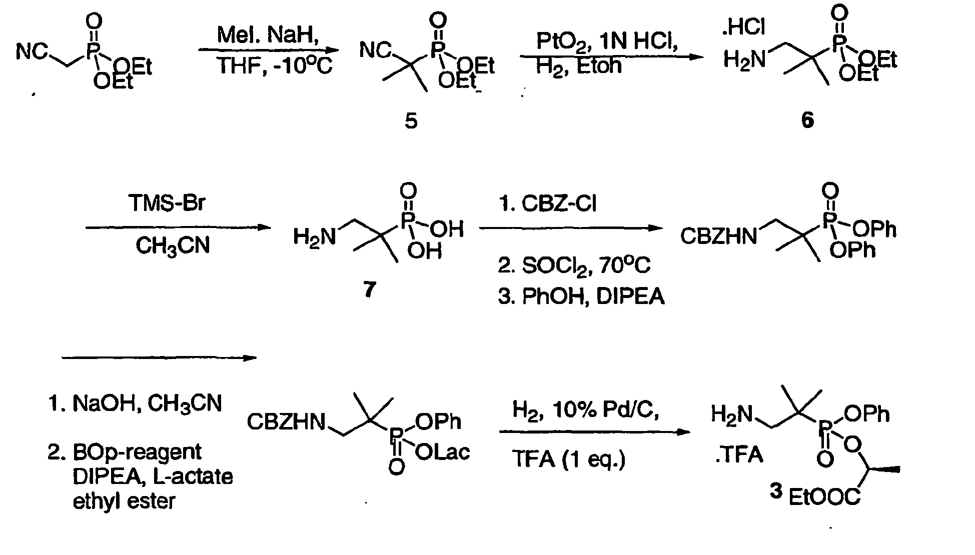

### Example Section M

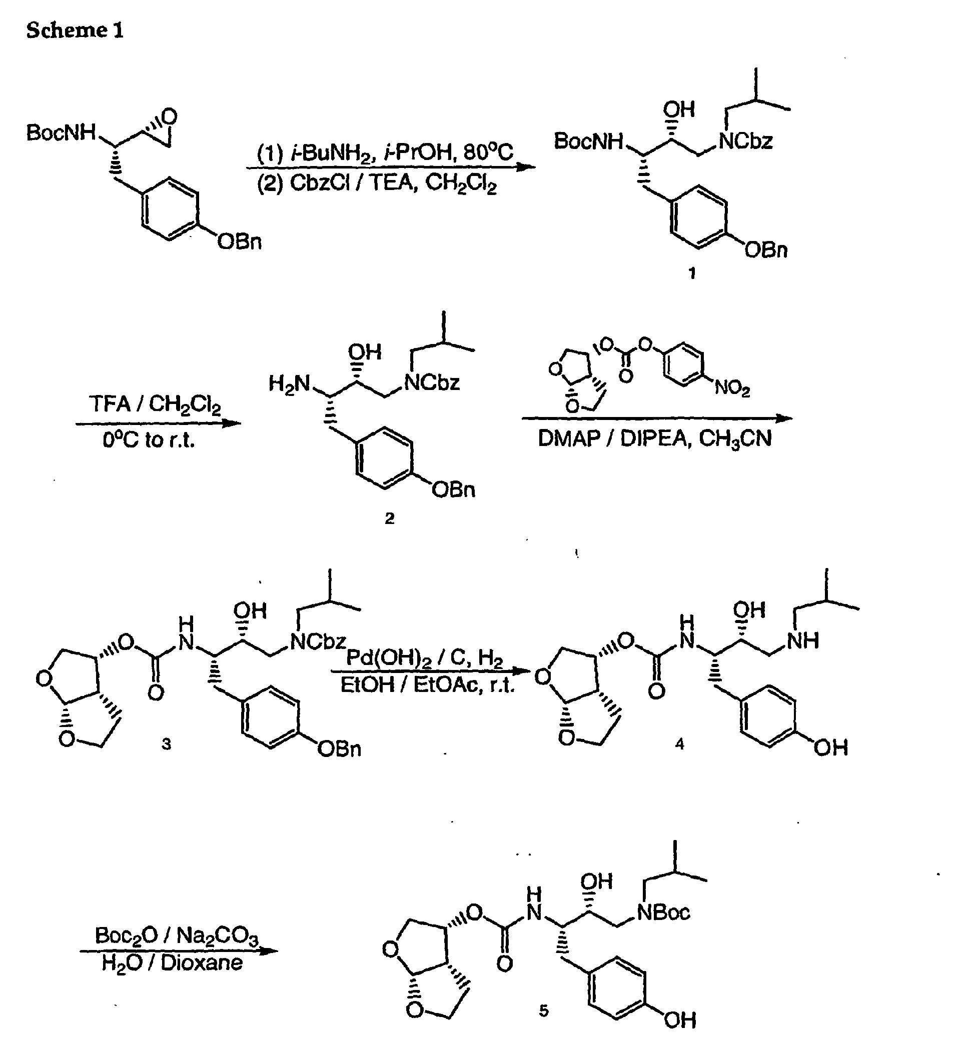

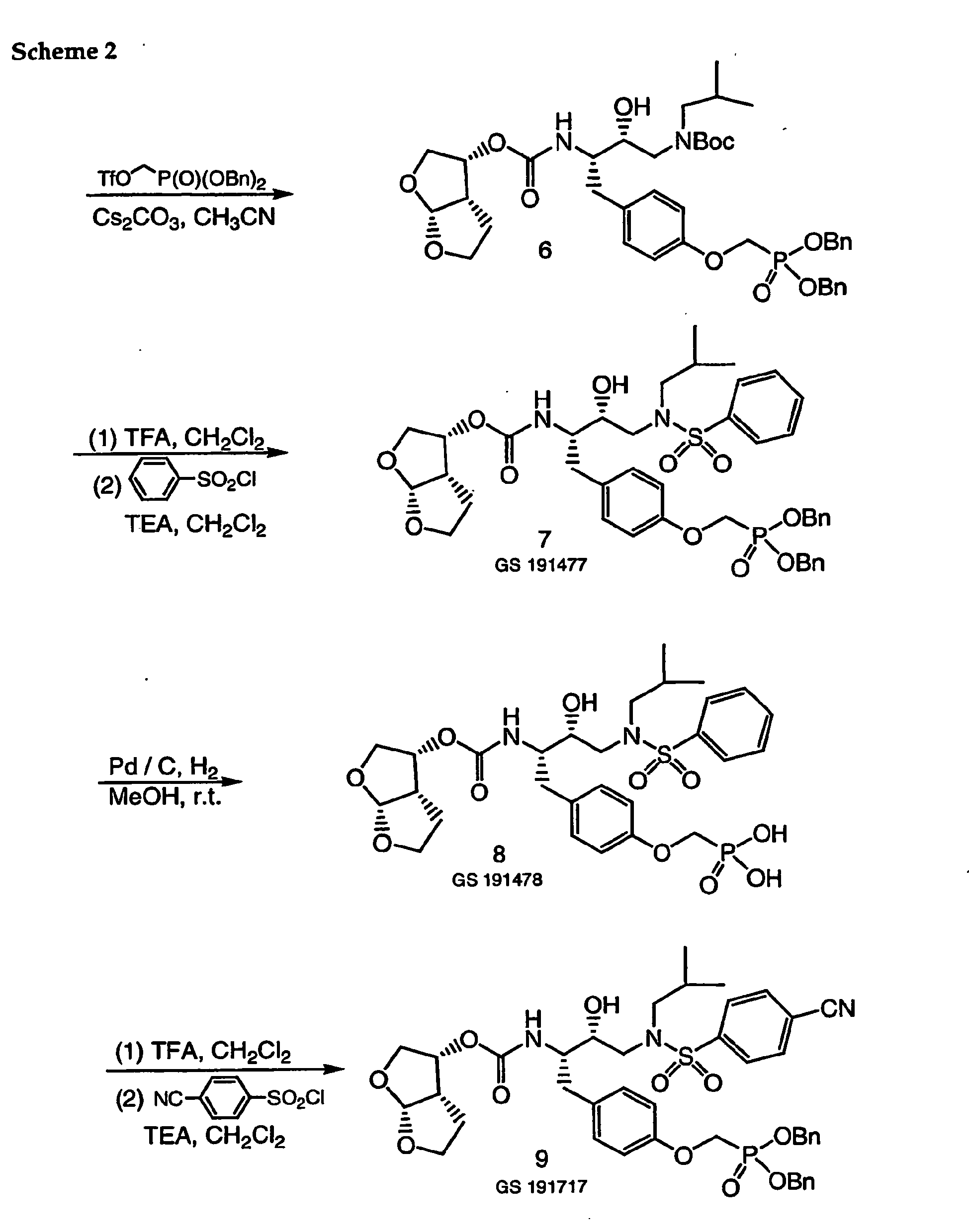

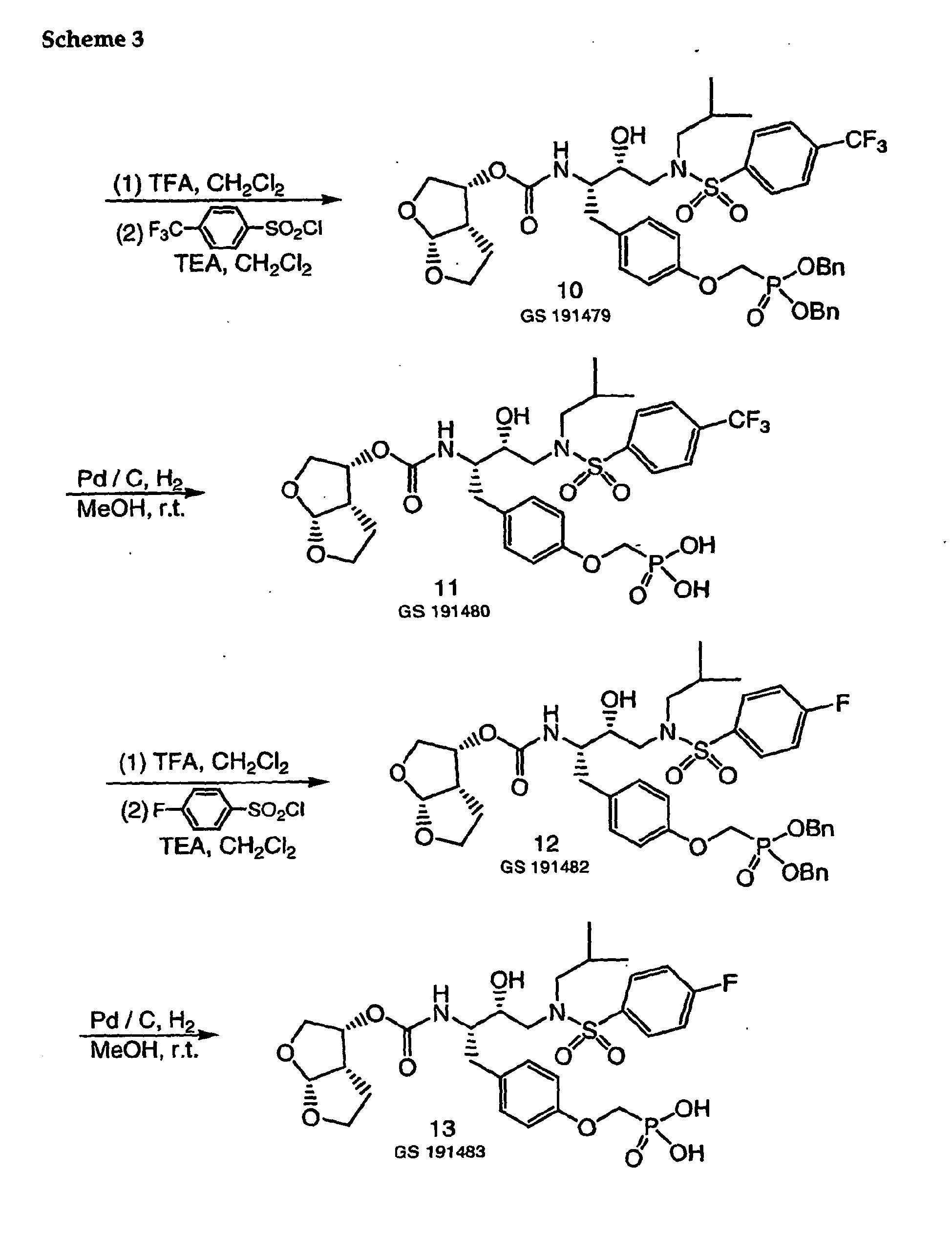

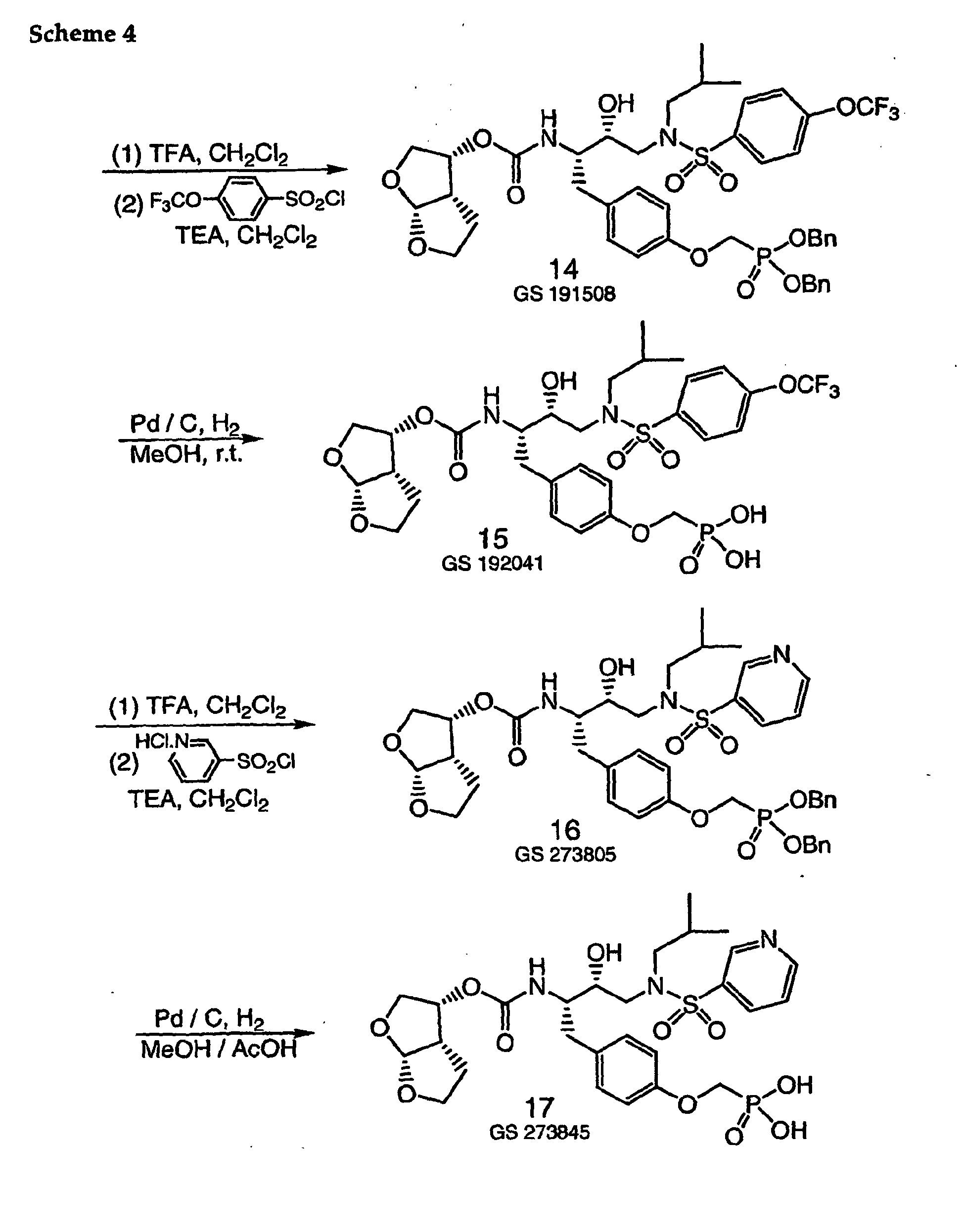

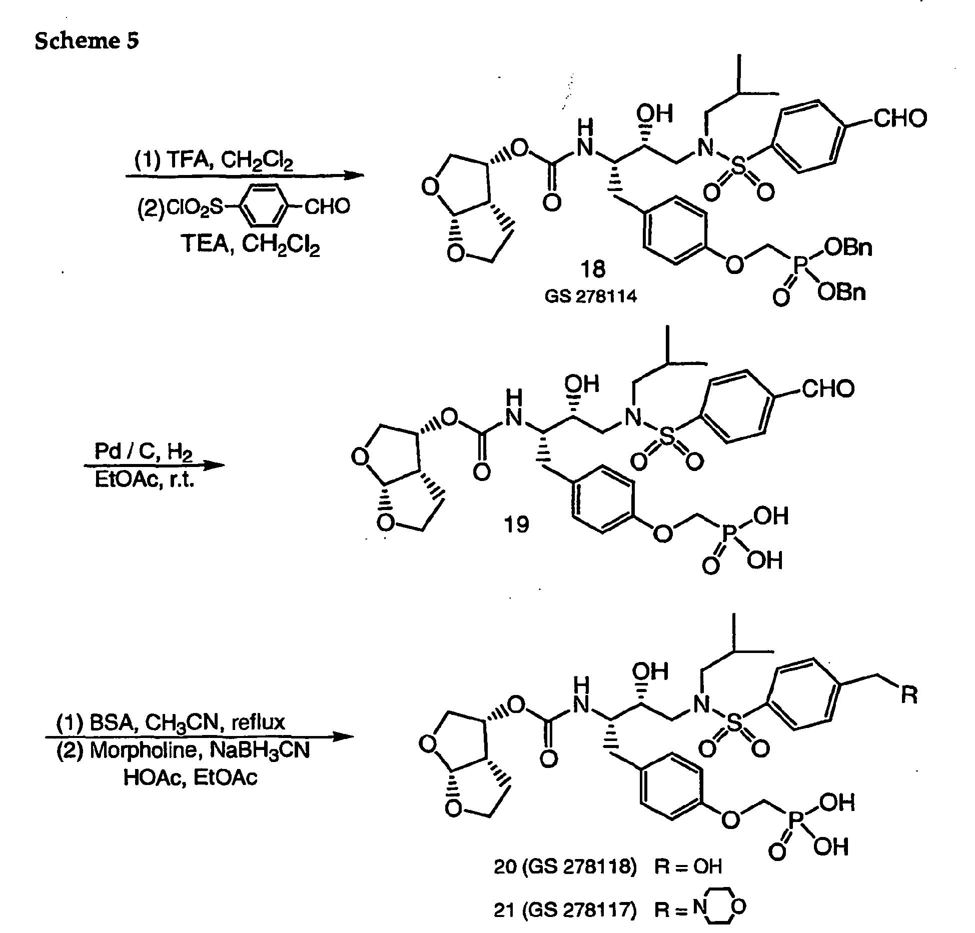

### Example 1

Cbz Amide 1: To a suspension of epoxide (34 g, 92.03 mmol) in 2-propanol (300 mL) was added isobutylamine (91.5 mL, 920 mmol) and the solution was refluxed for 1 h. The solution was evaporated under reduced pressure and the crude solid was dried under vacuum to give the amine (38.7 g, 95%) which was dissolved in CH₂Cl₂ (300 mL) and cooled to 0°C. Triethylamine (18.3 mL, 131 mmol) was added followed by the addition of benzyl chloroformate (13.7 mL, 96.14 mmol) and the solution was stirred for 30 min at 0°C, warmed to room temperature overnight, and evaporated under reduced pressure. The residue was partitioned between EtOAc and 0.5 M H₃PO₄. The organic phase was washed with saturated NaHCO₃, brine, dried with Na₂SO₄, filtered, and evaporated under reduced pressure. The crude product was purified by column chromatography on silica gel (1/2-EtOAc/hexane) to give the Cbz amide (45.37 g, 90%) as a white solid.

### Example 2

Amine 2: A solution of Cbz amide 1 (45.37 g, 78.67 mmol) in CH₂Cl₂ (160 mL) at 0°C was treated with trifluoroacetic acid (80 mL). The solution was stirred for 30 min at 0°C and then warmed to room temperature for an additional 30 min. Volatiles were evaporated under reduced pressure and the residue was partitioned between EtOAc and 0.5 N NaOH. The organic phase was washed with 0.5 N NaOH (2 x), water (2 x), saturated NaCl, dried with Na₂SO₄, filtered, and evaporated under reduced pressure to give the amine (35.62 g, 95%) as a white solid.

### Example 3

Carbamate 3: To a solution of amine 2 (20.99 g, 44.03 mmol) in CH₃CN (250 mL) at 0°C was treated with (3R, 3aR, 6aS)-hexahydrofuro[2, 3-*b*]furan-2-yl 4-nitrophenyl carbonate (13.00 g, 44.03 mmol, prepared according to Ghosh et al. J. Med. Chem. 1996, 39, 3278.), *N,N*-diisopropylethylamine (15.50 mL, 88.06 mmol) and 4-dimethylaminopyridine (1.08 g, 8.81 mmol). The reaction mixture was stirred at 0°C for 30 min and then warmed to room temperature overnight. The reaction solvent was evaporated under reduced pressure and the residue was partitioned between EtOAc and 0.5 N NaOH. The organic phase was washed with 0.5 N NaOH (2 x), 5% citric acid (2 x), saturated NaHCO₃, dried with Na₂SO₄, filtered, and evaporated under reduced pressure. The crude product was purified by column chromatography on silica gel (3% 2-propanol/CH₂Cl₂) to give the carbamate (23.00 g, 83%) as a white solid.

### Example 4

Amine 4: To a solution of 3 (23.00 g, 36.35 mmol) in EtOH (200 mL) and EtOAc (50 mL) was added 20% Pd(OH)₂/C (2.30 g). The suspension was stirred under H₂ atmosphere (balloon) at room temperature for 3 h. The reaction mixture was filtered through a plug of celite. The filtrate was concentrated and dried under vacuum to give the amine (14.00 g, 94%) as a white solid.

### Example 5

Phenol 5: To a solution of amine 4 (14.00 g, 34.27 mmol) in H₂O (80 mL) and 1,4-dioxane (80 mL) at 0°C was added Na₂CO₃ (5.09 g, 47.98 mmol) and di-*tert*-butyl dicarbonate (8.98 g, 41.13 mmol). The reaction mixture was stirred at 0°C for 2 h and then warmed to room temperature for 30 min. The residue was partitioned between EtOAc and H₂O. The organic layer was dried with Na₂SO₄, filtered, and concentrated. The crude product was purified by column chromatography on silica gel (3% MeOH/CH₂Cl₂) to give the phenol (15.69 g, 90%) as a white solid.

### Example 6

Dibenzylphosphonate 6: To a solution of phenol 5 (15.68 g, 30.83 mmol) in CH₃CN (200 mL) was added Cs₂CO₃ (15.07 g, 46.24 mmol) and triflate (17.00 g, 40.08 mmol). The reaction mixture was stirred at room temperature for 1 h, the salt was filtered off, and the solvent was evaporated under reduced pressure. The residue was partitioned between EtOAc and saturated NaCl. The organic phase was dried with Na₂SO₄, filtered, and evaporated under reduced pressure. The crude product was purified by column chromatography on silica gel (3% 2-propanol/CH₂Cl₂) to give the dibenzylphosphonate (15.37 g, 73%) as a white solid.

### Example 7

Sulfonamide 7: A solution of dibenzylphosphonate 6 (0.21 g, 0.26 mmol) in CH₂Cl₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.15 mL, 1.04 mmol) was added followed by the treatment of benzenesulfonyl chloride (47 mg, 0.26 mmol). The solution was stirred for 1 h at 0°C and the product was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide 7 (0.12 g, 55%, GS 191477) as a white solid: ¹HNMR (CDCl₃) δ 7.79 (dd, 2H), 7.61-7.56 (m, 3H), 7.38-7.36 (m, 10H), 7.13 (d, J = 8.4 Hz, 2H), 6.81 (d, J = 8.4 Hz, 2H), 5.65 (d, J = 5.4 Hz, 1H), 5.18 (m, 4H), 5.05 (m, 1H), 4.93 (d, J = 8.7 Hz, 1H), 4.20 (d, J = 10.2 Hz, 2H), 4.0-3.67 (m, 7H), 3.15-2.8 (m, 7H), 1.84 (m, 1H), 1.65-1.59 (m, 2H), 0.93 (d, J = 6.6 Hz, 3H), 0.88 (d, J = 6.3 Hz, 3H); ³¹P NMR (CDC1₃) δ 20.36.

### Example 8

Phosphonic Acid 8: To a solution of 7 (70 mg, 0.09 mmol) in MeOH (4 mL) was added 10% Pd/C (20 mg). The suspension was stirred under H₂ atmosphere (balloon) at room temperature overnight. The reaction mixture was filtered through a plug of celite. The filtrate was concentrated and dried under vacuum to give the phosphonic acid (49 mg, 90% GS 191478) as a white solid: ¹HNMR (CD₃OD) δ 7.83 (dd, 2H), 7.65-7.56 (m, 3H), 7.18 (d, J = 8.4 Hz, 2H), 6.91 (d, J = 7.8 Hz, 2H), 5.59 (d, J = 5.4 Hz, 1H), 4.96 (m, 1H), 4.15 (d, J = 9.9 Hz, 2H), 3.95-3.68 (m, 6H), 3.44 (dd, 2H), 3.16 (m, 2H), 2.99-2.84 (m, 4H), 2.48 (m, 1H), 2.02 (m, 1H), 1.6 (m, 1H), 1.37 (m, 1H), 0.93 (d, J = 6.3 Hz, 3H), 0.87 (d, J = 6.3 Hz, 3H); ³¹P NMR (CD₃OD) δ 17.45.

### Example 9

Sulfonamide 9: A solution of dibenzylphosphonate 6 (0.24 g, 0.31 mmol) in CH₂Cl₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.17 mL, 1.20 mmol) was added followed by the treatment of 4-cyanobenzenesulfonyl chloride (61.4 mg, 0.30 mmol). The solution was stirred for 1 h at 0°C and the product was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide 9 (0.20 g, 77%, GS 191717) as a white solid: ¹H NMR (CDCl₃) δ 7.90 (d, J = 8.4 Hz, 2H), 7.83 (d, J = 7.8 Hz, 2H), 7.36 (m, 10H), 7.11 (d, J = 8.4 Hz, 2H), 6.82 (d, J = 8.7 Hz, 2H), 5.65 (d, J = 5.4 Hz, 1H), 5.2-4.9 (m, 5H), 4.8 (d, 1H), 4.2 (d, J = 9.9 Hz, 2H), 3.99 (m 1H), 3.94 (m, 3H), 3.7 (m, 2H), 3.48 (broad, s, 1H), 3.18-2.78 (m, 7H), 1.87 (m, 1H), 1.66-1.47 (m, 2H), 0.91 (d, J = 6.3 Hz, 3H), 0.87 (d, J = 6.3 Hz, 3H); ³¹P NMR (CDCl₃) δ 20.3..

### Example 10

Sulfonamide 10: A solution of dibenzylphosphonate 6 (0.23 g, 0.29 mmol) in CH₂Cl₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.16 mL, 1.17 mmol) was added followed by the treatment of 4-trifluoromethyl benzenesulfonyl chloride (72 mg, 0.29 mmol). The solution was stirred for 1 h at 0°C and the product was partitioned- between CH₂Cl₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide (0.13 g, 50%, GS 191479) as a white solid: ¹H NMR (CDCl₃) δ 7.92 (d, J = 8.1 Hz, 2H), 7.81 (d, J = 8.1 Hz, 2H), 7.36 (m, 10H), 7.12 (d, J = 8.4 Hz, 2H), 6.81 (d, J = 8.4 Hz, 2H), 5.65 (d, J = 5.1 Hz, 1H), 5.20-4.89 (m, 6H), 4.20 (d, J = 9.9 Hz, 2H), 3.95 (m, 1H), 3.86 (m, 3H), 3.71 (m, 2H), 3.19-2.78 (m, 7H), 1.86 (m, 1H), 1.65 (m, 2H), 0.93 (d, J = 6.3 Hz, 3H), 0.88 (d, J = 6.3 Hz, 3H); ³¹P NMR (CDCl₃) δ 20.3.

### Example 11

Phosphonic Acid 11: To a solution of 10 (70 mg, 0.079 mmol) in MeOH (4 mL) was added 10% Pd/C (20 mg). The suspension was stirred under H₂ atmosphere (balloon) at room temperature overnight. The reaction mixture was filtered through a plug of celite. The filtrate was concentrated and dried under vacuum to give the phosphonic acid (50 mg, 90%, GS 191480) as a white solid: ¹H NMR (CD₃OD) δ 8.03 (dd, 2H), 7.90 (dd, 2H), 7.17 (d, J = 8.1 Hz, 2H), 6.91 (d, J = 7.8 Hz, 2H), 5.59 (d, J = 5.7 Hz, 1H), 4.94 (m, 1H), 4.15 (d, J = 10.2 Hz, 2H), 3.94-3.72 (m, 6H), 3.48 (m, 1H), 3.2-3.1 (m, 3H), 3.0-2.9 (m, 2H), 2.47 (m, 1H), 2.06 (m, 1H), 1.56 (m, 1H), 1.37 (m, 1H), 0.93 (d, J = 6.3 Hz, 3H), 0.88 (d, J = 6.3 Hz, 3H); ³¹P NMR (CD₃OD) δ 17.5.

### Example 12

Sulfonamide 12: A solution of dibenzylphosphonate 6 (0.23 g, 0.29 mmol) in CH₂Cl₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂CI₂ (3 mL) and cooled to 0°C. Triethylamine (0.16 mL, 1.17 mmol) was added followed by the treatment of 4-fluorobenzenesulfonyl chloride (57 mg, 0.29 mmol). The solution was stirred for 1 h at 0°C and the product was partitioned between CH₂CI₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide (0.13 g, 55%, GS 191482) as a white solid: ¹H NMR (CDCl₃) δ 7.81 (m, 2H), 7.38 (m, 10H), 7.24 (m, 2H), 7.12 (d, J = 8.1 Hz, 2H), 6.82 (d, J = 8.4 Hz, 2H), 5.65 (d, J = 5.4 Hz, 1H), 5.17 (m, 4H), 5.0 (m, 1H), 4.90 (d, 1H), 4.20 (d, J = 9.9 Hz, 2H), 3.97 (m, 1H), 3.86 (m, 3H), 3.73 (m, 2H), 3.6 (broad, s, 1H), 3.13 (m, 1H), 3.03-2.79 (m, 6H), 1.86 (m, 1H), 1.66-1.58 (m, 2H), 0.92 (d, J = 6.6 Hz, 3H), 0.88 (d, J = 6.6 Hz, 3H); ³¹P NMR (CDCl₃) δ 20.3.

### Example 13

Phosphonic Acid 13: To a solution of 12 (70 mg, 0.083 mmol) in MeOH (4 mL) was added 10% Pd/C (20 mg). The suspension was stirred under H₂ atmosphere (balloon) at room temperature overnight. The reaction mixture was filtered through a plug of celite. The filtrate was concentrated and dried under vacuum to give the phosphonic acid (49 mg, 90%, GS 191483) as a white solid: ¹H NMR (CD₃OD) δ 7.89 (m, 2H), 7.32 (m, 2H), 7.18 (d, J = 8.4 Hz, 2H), 6.9 (d, J = 8.1 Hz, 2H), 5.59 (d, J = 5.1 Hz, 1H), 4.94 (m, 1H), 4.16 (d, J = 9.9 Hz, 2H), 3.94 (m, 1H), 3.85-3.7 (m, 5H), 3.43 (dd, 1H), 3.15-2.87 (m, 5H), 2.48 (m, 1H), 2.03 (m, 1H), 1.59-1.36 (m, 2H), 0.93 (d, J = 6.3 Hz, 3H), 0.87 (d, J = 6.3 Hz, 3H); ³¹P NMR (CD₃OD) δ 17.5.

### Example 14

Sulfonamide 14: A solution of dibenzylphosphonate 6 (0.21 g, 0.26 mmol) in CH₂Cl₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.15 mL, 1.04 mmol) was added followed by the treatment of 4-trifluoromethoxybenzenesulfonyl chloride (69 mg, 0.26 mmol). The solution was stirred for 1 h at 0°C and the product was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide (0.17 g, 70%, GS 191508) as a white solid: ¹H NMR (CDCl₃) δ 7.84 (d, J = 9 Hz, 2H), 7.36 (m, 12H), 7.12 (d, J = 8.7 Hz, 2H), 6.81 (d, J = 8.7 Hz, 2H), 5.65 (d, J = 5.4 Hz, 1H), 5.16 (m, 4H), 5.03 (m, 1H), 4.89 (d, 1H), 4.2 (d, J = 9.9 Hz, 2H), 3.97 (m, 1H), 3.85 (m, 3H), 3.7 (m, 2H), 3.59 (broad, s, 1H), 3.18 (m, 1H), 3.1-3.0 (m, 3H), 2.96-2.78 (m, 3H), 1.86 (m, 1H), 1.66-1.5 (m, 2H), 0.93 (d, J = 6.6 Hz, 3H), 0.88 (d, J = 6.6 Hz, 3H); ¹P NMR (CDCl₃) δ 20.3.

### Example 15

Phosphonic Acid 15: To a solution of 14 (70 mg, 0.083 mmol) in MeOH (4 mL) was added 10% Pd/C (20 mg). The suspension was stirred under H₂ atmosphere (balloon) at room temperature overnight. The reaction mixture was filtered through a plug of celite. The filtrate was concentrated and dried under vacuum to give the phosphonic acid (50 mg, 90%, GS 192041) as a white solid: ¹H NMR (CD₃OD) δ 7.95 (dd, 2H), 7.49 (dd, 2H), 7.17 (dd, 2H), 6.92 (dd, 2H), 5.58 (d, J = 5.4 Hz, 1H), 4.89 (m, 1H), 4.17 (d, J = 9 Hz, 2H), 3.9 (m, 1H), 3.82-3.7 (m, 5H), 3.44 (m, 1H), 3.19-2.9 (m, 5H), 2.48 (m, 1H), 2.0 (m 1H), 1.6 (m, 1H), 1.35 (m, 1H), 0.93 (d, J = 6.0 Hz, 3H), 0.88 (d, J = 6.0 Hz, 3H); ³¹P NMR (CD₃OD) δ 17.4.

### Example 16

Sulfonamide 16: A solution of dibenzylphosphonate 6 (0.59 g, 0.76 mmol) in CH₂Cl₂ (2.0 mL) at 0°C was treated with trifluoroacetic acid (1.0 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.53 mL, 3.80 mmol) was added followed by the treatment of hydrogen chloride salt of 3-pyridinylsulfonyl chloride (0.17 g, 0.80 mmol, prepared according to Karaman, R. et al. J. Am. Chem. Soc. 1992, 114, 4889). The solution was stirred for 30 min at 0°C and warmed to room temperature for 30 min. The product was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (4% 2-propanol/CH₂Cl₂) to give the sulfonamide (0.50 g, 80%, GS 273805) as a white solid: ¹H NMR (CDCl₃) δ 9.0 (d, J = 1.5 Hz, 1H), 8.8 (dd, 1H), 8.05 (d, J = 8.7 Hz, 1H), 7.48 (m, 1H), 7.36 (m, 10H), 7.12 (d, J = 8.4 Hz, 2H), 6.82 (d, J = 9.0 Hz, 2H), 5.65 (d, J = 5.1 Hz, 1H), 5.18 (m, 4H), 5.06 (m, 1H), 4.93 (d, 1H), 4.21 (d, J = 8.4 Hz, 2H), 3.97 (m, 1H), 3.86 (m, 3H), 3.74 (m, 2H), 3.2 (m, 1H), 3.1-2.83 (m, 5H), 2.76 (m, 1H), 1.88 (m, 1H), 1.62 (m, 2H), 0.92 (d, J = 6.3 Hz, 3H), 0.88 (d, J = 6.3 Hz, 3H); ³¹P NMR (CDCl₃) δ 20.3.

### Example 17

Phosphonic Acid 17: To a solution of 16 (40 mg, 0.049 mmol) in MeOH (3 mL) and AcOH (1 mL) was added 10% Pd/C (10 mg). The suspension was stirred under H₂ atmosphere (balloon) at room temperature overnight. The reaction mixture was filtered through a plug of celite. The filtrate was concentrated and dried under vacuum to give the phosphonic acid (28 mg, 90%, GS 273845) as a white solid: ¹H NMR (CD₃OD) δ 8.98 (s, 1H), 8.77 (broad, s, 1H), 8.25 (dd, 1H), 7.6 (m, 1H), 7.15 (m, 2H), 6.90 (m, 2H), 5.6 (d, J = 5.4 Hz, 1H), 4.98 (m, 1H), 4.15 (d, 2H), 3.97-3.7 (m, 6H), 3.45-2.89 (m, 6H), 2.50 (m, 1H), 2.0 (m, 1H), 1.6-1.35 (m, 2H), 0.9 (m, 6H).

### Example 18

Sulfonamide 18: A solution of dibenzylphosphonate 6 (0.15 g, 0.19 mmol) in CH₂Cl₂ (0.60 mL) at 0°C was treated with trifluoroacetic acid (0.30 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (2 mL) and cooled to 0°C. Triethylamine (0.11 mL, 0.76 mmol) was added followed by the treatment of 4-formylbenzenesulfonyl chloride (43 mg, 0.21 mmol). The solution was stirred for 30 min at 0°C and warmed to room temperature for 30 min. The product was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (3% 2-propanol/CH₂Cl₂) to give the sulfonamide (0.13 g, 80%, GS 278114) as a white solid: ¹H NMR (CDCl₃) δ 10.1 (s, 1H), 8.04 (d, J = 8.1 Hz, 2H), 7.94 (d, J = 8.1 Hz, 2H), 7.35 (m, 10H), 7.13 (m, J = 8.1 Hz, 2H), 6.82 (d, J = 8.1 Hz, 2H), 5.65 (d, J = 5.4 Hz, 1H), 5.17 (m, 4H), 5.06 (m, 1H), 4.93 (m, 1H), 4.2 (d, J = 9.9 Hz, 2H), 3.94 (m, 1H), 3.85 (m, 3H), 3.7 (m, 2H), 3.18-2.87 (m, 5H), 2.78 (m, 1H), 1.86 (m, 1H), 1.67-1.58 (m, 2H), 0.93 (d, J = 6.6 Hz, 3H), 0.88 (d, J = 6.6 Hz, 3H); ³¹P NMR (CDCl₃) δ 20.3.

### Example 19

Phosphonic Acid 19: To a solution of 18 (0.12 g, 0.15 mmol) in EtOAc (4 mL) was added 10% Pd/C (20 mg). The suspension was stirred under H₂ atmosphere (balloon) at room temperature for 6 h. The reaction mixture was filtered through a plug of celite. The filtrate was concentrated and dried under vacuum to give the phosphonic acid (93 mg, 95%) as a white solid.

### Example 20

Phosphonic Acids 20 and 21: Compound 19 (93 mg, 0.14 mmol) was dissolved in CH₃CN (2 mL). *N*,*O*-Bis(trimethylsilyl)acetamide (BSA, 0.28 g, 1.4 mmol) was added. The reaction mixture was heated to reflux for 1 h, cooled to room temperature and concentrated. The residue was co-evaporated with toluene and chloroform and dried under vacuum to give a semi-solid which was dissolved in EtOAc (2 mL). Morpholine (60 µL, 0.9 mmol), AcOH (32 µL, 0.56 mmol), and NaBH₃CN (17 mg, 0.28 mmol) were added and the reaction mixture was stirred at room temperature overnight. The reaction was quenched with H₂O, stirred for 2 h, filtered, and concentrated. The crude product was purified by HPLC to give the phosphonic acid 20 (10 mg, GS 278118) as a white solid: ¹H NMR (CD₃OD) δ 7.80 (d, J = 7.8 Hz, 2H), 7.56 (d, J = 7.5 Hz, 2H), 7.17 (d, J = 7.8 Hz, 2H), 6.91 (d, J = 7.5 Hz, 2H), 5.59 (d, J = 5.1 Hz, 1H), 5.06 (m, 1H), 4.7 (s, 2H), 4.15 (d, J = 10.2 Hz, 2H), 3.92 (m, 1H), 3.82-3.7 (m, 5H), 3.43 (dd; 1H), 3.11-2.89 (m, 6H), 2.50 (m, 1H), 2.0 (m, 1H), 1.6-1.35 (m, 2H), 0.93 (d, J = 6.3 Hz, 3H), 0.88 (d, J = 6.3 Hz, 3H); ³¹P NMR (CD₃OD) δ 17.3. Phosphonic acid 21 (15 mg, GS 278117) as a white solid: ¹H NMR (CD₃OD) δ 7.8-7.7 (m, 4H), 7.20 (d, J = 8.4 Hz, 2H), 6.95 (d, J = 8.4 Hz, 2H), 5.62 (d, J = 5.1 Hz, 1H), 5.00 (m, 1H), 4.42 (s, 2H), 4.20 (dd, 2H), 3.98-3.68 (m, 9H), 3.3-2.92 (m, 11H), 2.6 (m, 1H), 2.0 (m, 1H), 1.6 (m, 2H), 0.92 (d, J = 6.6 Hz, 3H), 0.88 (d, J = 6.6 Hz, 3H); ³¹P NMR (CD₃OD) δ 16.2.

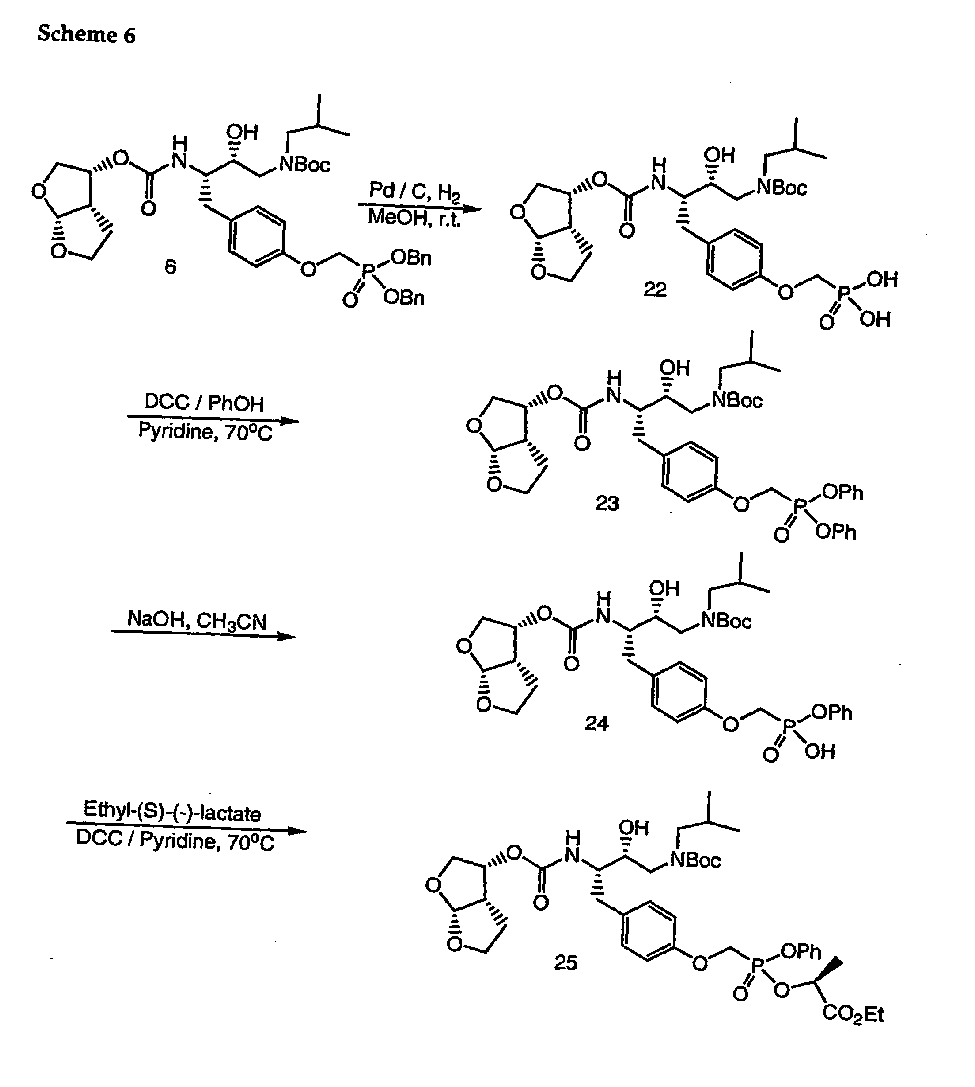

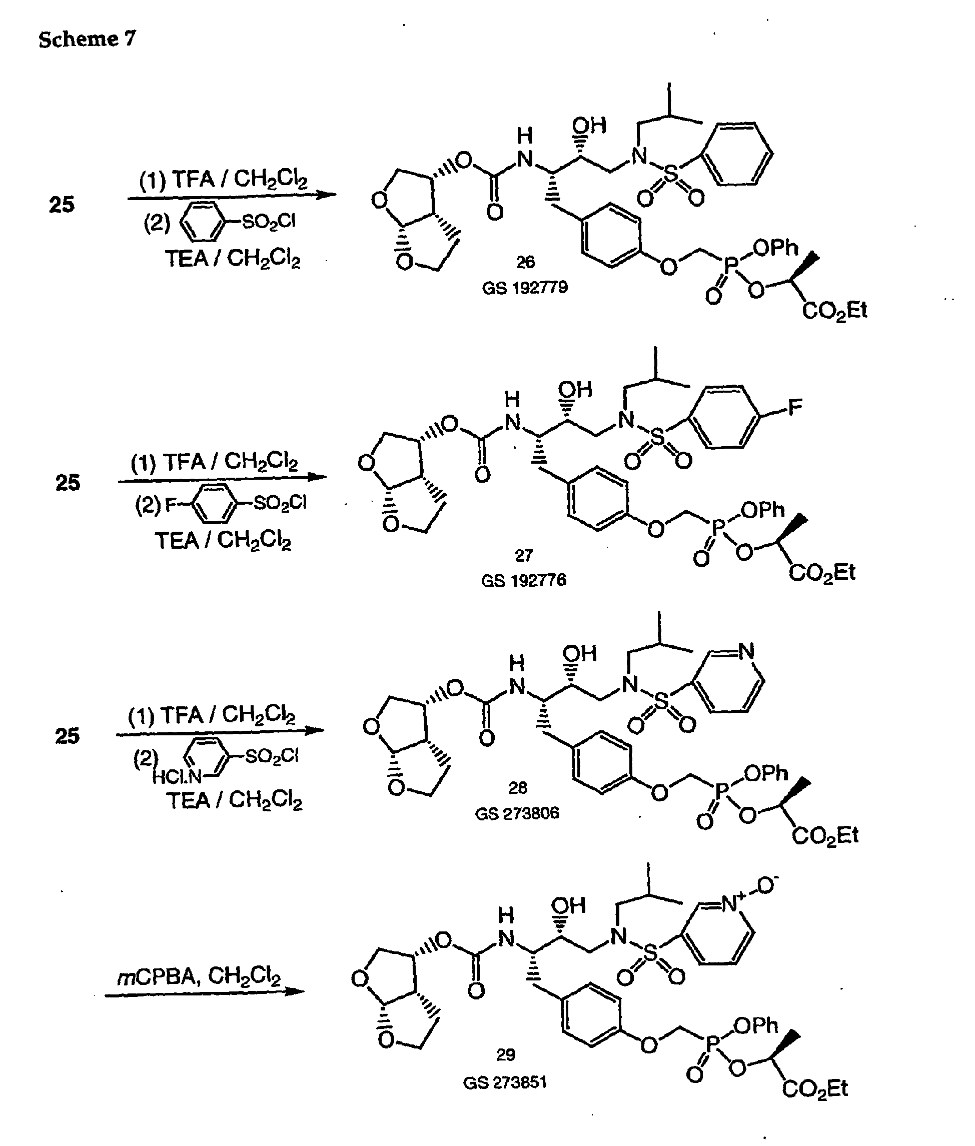

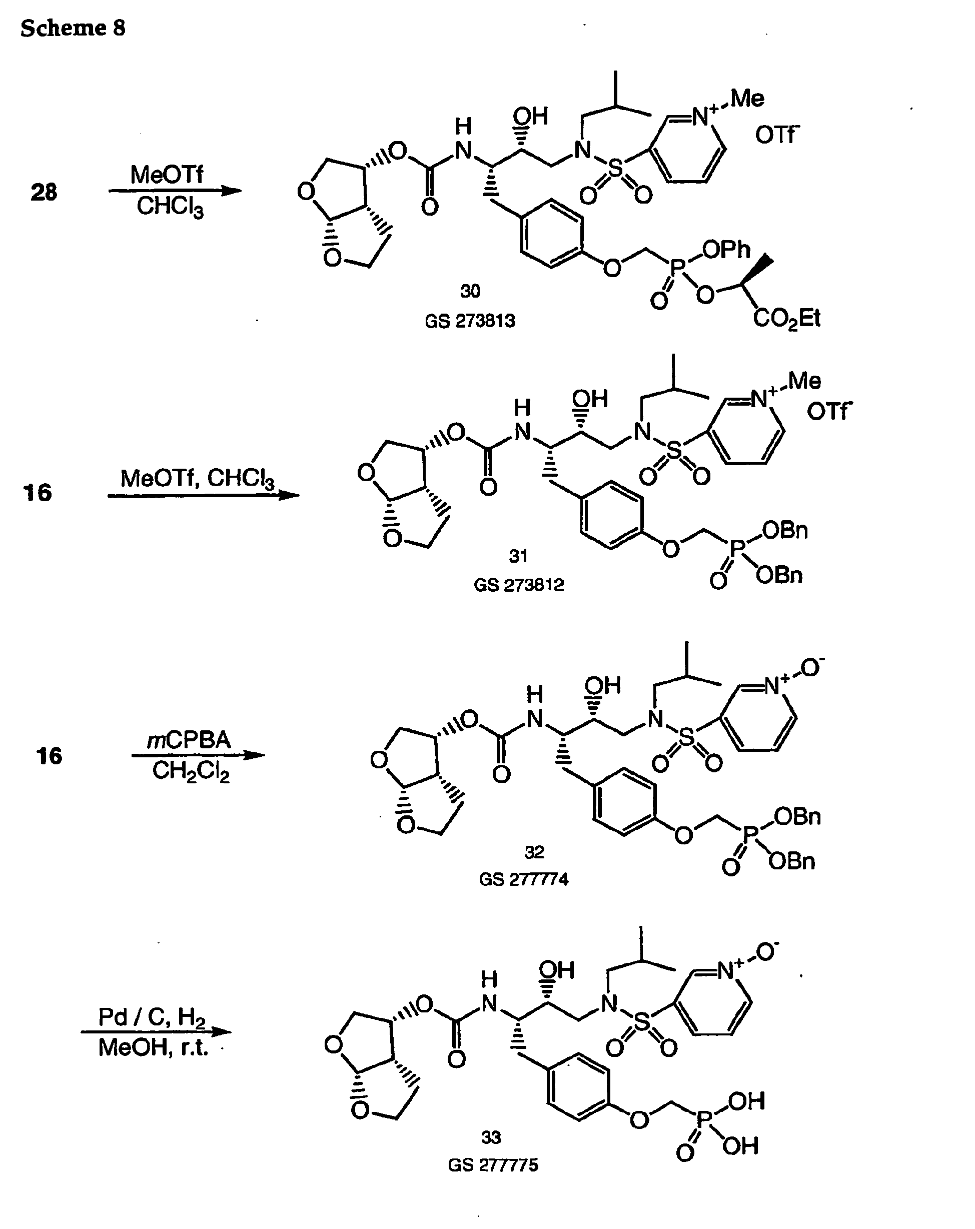

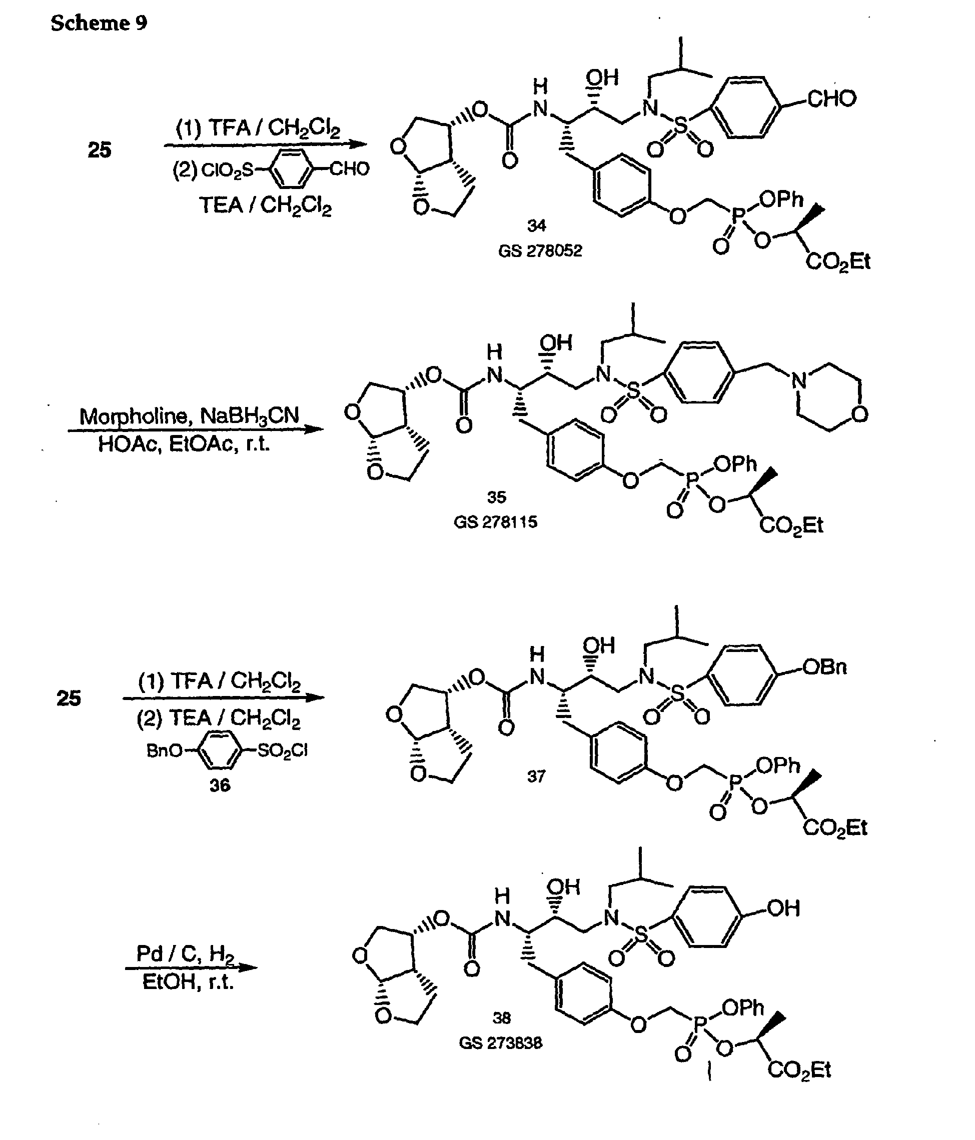

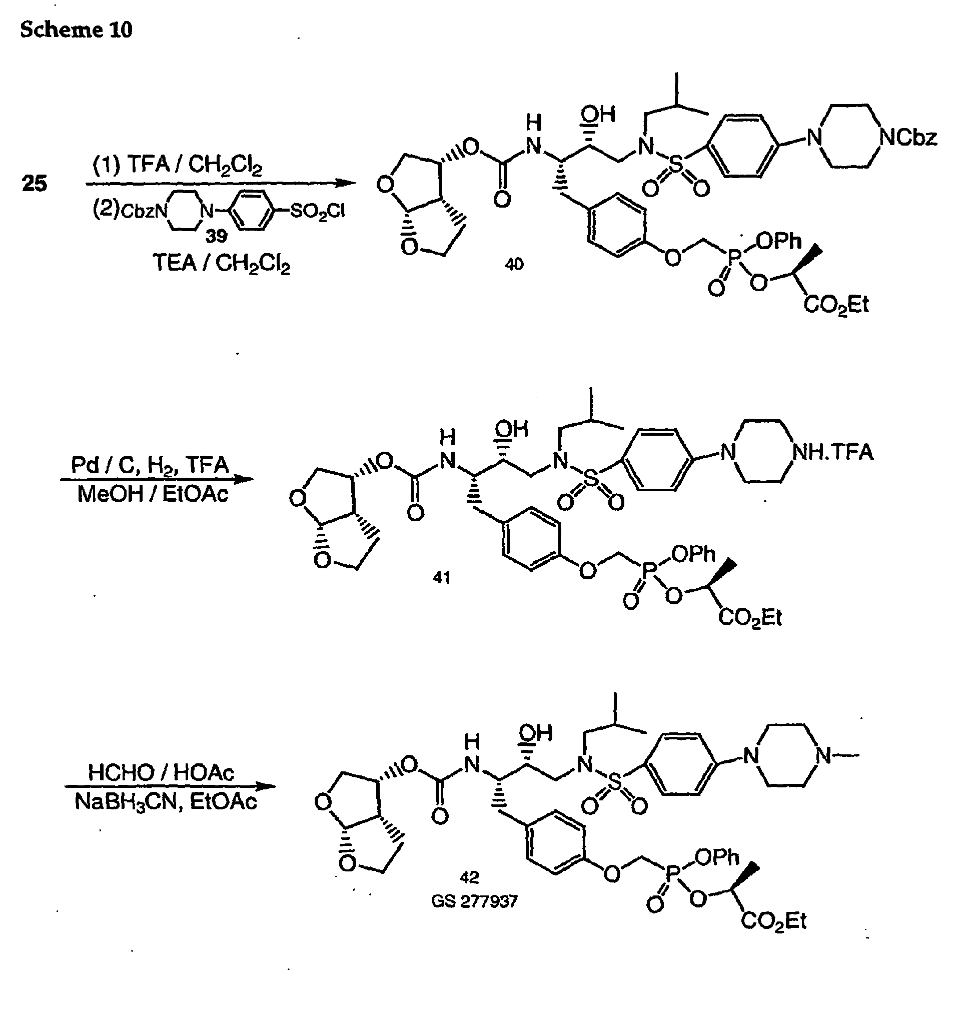

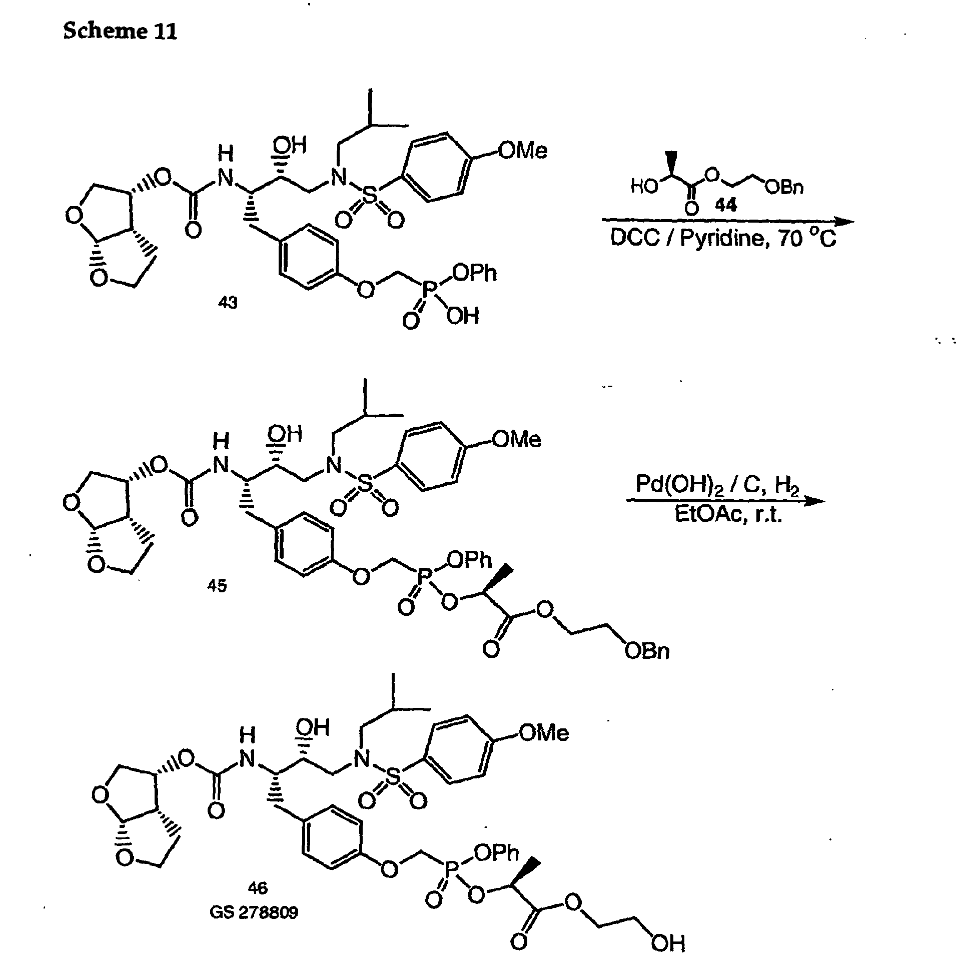

### Example 21

Phosphonic Acid 22: To a solution of dibenzylphosphonate 6 (5.00 g, 6.39 mmol) in EtOH (100 mL) was added 10% Pd/C (1.4 g). The suspension was stirred under H₂ atmosphere (balloon) at room temperature overnight. The reaction mixture was filtered through a plug of celite. The filtrate was concentrated and dried under vacuum to give the phosphonic acid (3.66 g, 95%) as a white solid.

### Example 22

Diphenylphosphonate 23: A solution of 22 (3.65 g, 6.06 mmol) and phenol (5.70 g, 60.6 mmol) in pyridine (30 mL) was heated to 70°C and 1,3-dicyclohexylcarbodiimide (5.00 g, 24.24 mmol) was added. The reaction mixture was stirred at 70°C for 2 h and cooled to room temperature. EtOAc was added and the side product 1,3-dicyclohexyl urea was filtered off. The filtrate was concentrated and dissolved in CH₃CN (20 mL) at 0°C. The mixture was treated with DOWEX 50W x 8-400 ion-exchange resin and stirred for 30 min at 0°C. The resin was filtered off and the filtrate was concentrated. The crude product was purified by column chromatography on silica gel (3% 2-propanol/CH₂Cl₂) to give the diphenylphosphonate (2.74 g, 60%) as a white solid.

### Example 23

Monophosphonic Acid 24: To a solution of 23 (2.74 g, 3.63 mmol) in CH₃CN (40 mL) at 0°C was added 1 N NaOH (9.07 mL, 9.07 mmol). The reaction mixture was stirred at 0°C for 1 h. DOWEX 50W x 8-400 ion-exchange resin was added and the reaction mixture was stirred for 30 min at 0°C. The resin was filtered off and the filtrate was concentrated and co-evaporated with toluene. The crude product was triturated with EtOAc/hexane (1/2) to give the monophosphonic acid (2.34 g, 95%) as a white solid.

### Example 24

Monophospholactate 25: A solution of 24 (2.00 g, 2.95 mmol) and ethyl-(S)-(-)-lactate (1.34 mL, 11.80 mmol) in pyridine (20 mL) was heated to 70°C and 1,3-dicyclohexylcarbodiimide (2.43 g, 11.80 mmol) was added. The reaction mixture was stirred at 70°C for 2 h and cooled to room temperature. The solvent was removed under reduced pressure. The residue was suspended in EtOAc and 1,3-dicyclohexyl urea was filtered off. The product was partitioned between EtOAc and 0.2 N HCl. The EtOAc layer was washed with 0.2 N HCl, H₂O, saturated NaCl, dried with Na₂SO₄, filtered, and concentrated. The crude product was purified by column chromatography on silica gel (3% 2-propanol/CH₂Cl₂) to give the monophospholactate (1.38 g, 60%) as a white solid.

### Example 25

Monophospholactate 26: A solution of 25 (0.37 g, 0.48 mmol) in CH₂Cl₂ (0.80 mL) at 0°C was treated with trifluoroacetic acid (0.40 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.27 mL, 1.92 mmol) was added followed by the treatment of benzenesulfonyl chloride (84 mg, 0.48 mmol). The solution was stirred for 30 min at 0°C and then warmed to room temperature for 30 min. The product was partitioned between CH₂Cl₂ and 0.2 N HCL The organic phase was washed with saturated NaCL dried with Na₂SO₄, filtered, and evaporated under reduced pressure. The crude product was purified by column chromatography on silica gel (3% 2-propanol/CH₂Cl₂) to give the monophospholactate (0.33 g, 85%, GS 192779, 1:1 diastereomeric mixture) as a white solid: ¹H NMR (CDCl₃) δ 7.78 (dd, 2H), 7.59 (m, 3H), 7.38-7.18 (m, 7H), 6.93 (dd, 2H), 5.66 (m, 1H), 5.18-4.93 (m, 3H), 4.56-4.4 (m, 2H), 4.2 (m, 2H), 4.1-3.7 (m, 6H), 3.17 (m, 1H), 3.02-2.8 (m, 6H), 1.84 (m, 1H), 1.82-1.5 (m, 5H), 1.27 (m, 3H), 0.93 (d, J = 6.3 Hz, 3H), 0.88 (d, J = 6.3 Hz, 3H); ³¹P NMR (CDCl₃) δ 17.4, 15.3.

### Example 26

Monophospholactate 27: A solution of 25 (0.50 g, 0.64 mmol) in CH₂Cl₂ (1.0 mL) at 0°C was treated with trifluoroacetic acid (0.5 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (4 mL) and cooled to 0°C. Triethylamine (0.36 mL, 2.56 mmol) was added followed by the treatment of 4-fluorobenzenesulfonyl chloride (0.13 g, 0.64 mmol). The solution was stirred for 30 min at 0°C and then warmed to room temperature for 30 min. The product was partitioned between CH₂Cl₂ and 0.2 N HCL The organic phase was washed with saturated NaCl, dried with Na₂SO₄, filtered, and evaporated under reduced pressure. The crude product was purified by column chromatography on silica gel (3% 2-propanol/CH₂Cl₂) to give the monophospholactate (0.44 g, 81%, GS 192776,3/2 diastereomeric mixture) as a white solid: ¹H NMR (CDCl₃) δ 7.80 (m, 2H), 7.38-7.15 (m, 9H), 6.92 (m, 2H), 5.66 (m, 1H), 5.2-4.9 (m, 3H), 4.57-4.4 (m, 2H), 4.2 (m, 2H), 4.1-3.7 (m, 6H), 3.6 (broad, s, 1H), 3.17 (m, 1H), 3.02-2.75 (m, 6H), 1.85 (m, 1H), 1.7-1.5 (m, 5H), 1.26 (m, 3H), 0.93 (d, J = 6.3 Hz, 3H), 0.88 (d, J = 6.3 Hz, 3H); ³¹P NMR (CDCl₃) δ 17.3, 15.2.

### Example 27

Monophospholactate 28: A solution of 25 (0.50 g, 0.64 mmol) in CH₂Cl₂ (1.0 mL) at 0°C was treated with trifluoroacetic acid (0.5 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.45 mL, 3.20 mmol) was added followed by the treatment of hydrogen chloride salt of 3-pyridinylsulfonyl chloride (0.14 g, 0.65 mmol). The solution was stirred for 30 min at 0°C and then warmed to room temperature for 30 min. The product was partitioned between CH₂Cl₂ and H₂O. The organic phase was washed with saturated NaCl, dried with Na₂SO₄, filtered, and evaporated under reduced pressure. The crude product was purified by column chromatography on silica gel (4% 2-propanol/CH₂Cl₂) to give the monophospholactate (0.41 g, 79%, GS 273806, 1:1 diastereomeric mixture) as a white solid: ¹H NMR (CDCl₃) δ 9.0 (s, 1H), 8.83 (dd, 1H), 8.06 (d, J = 7.8 Hz, 1H), 7.5 (m, 1H), 7.38-7.15 (m, 7H), 6.92 (m, 2H), 5.66 (m, 1H), 5.18-4.95 (m, 3H), 4.6-4.41 (m, 2H), 4.2 (m, 2H), 4.0 (m, 1H), 3.95-3.76 (m, 6H), 3.23-2.8 (m, 7H), 1.88 (m, 1H), 1.7-1.5 (m, 5H), 1.26 (m, 3H), 0.93 (d, J = 6.6 Hz, 3H), 0.83 (d, J = 6.6 Hz, 3H); ³¹P NMR (CDCl₃) δ 17.3, 15.3.

### Example 28

Monophospholactate 29: A solution of compound 28 (0.82 g, 1.00 mmol) in CH₂Cl₂ (8 mL) at 0°C was treated with *m*CPBA (1.25 eq). The solution was stirred for 1 h at 0°C and then warmed to room temperature for an additional 6 h. The reaction mixture was partitioned between CH₂Cl₂ and saturated NaHCO₃. The organic phase was washed with saturated NaCl, dried with Na₂SO₄, filtered, and evaporated under reduced pressure. The crude product was purified by column chromatography on silica gel (10% 2-propanol/CH₂Cl₂) to give the monophospholactate (0.59 g, 70%, GS 273851, 1:1 diastereomeric mixture) as a white solid: ¹H NMR (CDCl₃) δ 8.63 (dd, 1H), 8.3 (dd, 1H), 7.57 (m, 1H), 7.44 (m, 1H), 7.38-7.13 (m, 7H), 6.92 (m, 2H), 5.66 (m, 1H), 5.2-5.05 (m, 2H), 4.57-4.4 (m, 2H), 4.2 (m, 2H), 4.0-3.73 (m, 6H), 3.2 (m, 2H), 3.0 (m, 4H), 2.77 (m, 1H), 1.92 (m, 1H),.1.7-1.49 (m, 5H), 1.26 (m, 3H), 0.91 (m, 6H); ³¹P NMR (CDCl₃) δ 17.3, 15.3.

### Example 29

Monophospholactate 30: A solution of compound 28 (71 mg, 0.087 mmol) in CHCl₃ (1 mL) was treated with MeOTf (18 mg, 0.11 mmol). The solution was stirred at room temperature for 1 h. The reaction mixture was concentrated and co-evaporated with toluene (2 x), CHC1₃ (2 x) and dried under vacuum to give the monophospholactate (81 mg, 95%, GS 273813,1:1 diastereomeric mixture) as a white solid: ¹H NMR (CDCl₃) δ 9.0 (dd, 1H), 8.76 (m, 2H), 8.1 (m, 1H), 7.35-7.1 (m, 7H), 6.89 (m, 2H), 5.64 (m, 1ED, 5.25-5.0 (m, 3H), 4.6-4.41 (m, 5H), 4.2 (m, 2H), 3.92-3.72 (m, 6H), 3.28 (m, 2H), 3.04-2.85 (m, 3H), 2.62 (m, 1H), 1.97 (m, 1H), 1.62-1.5 (m, 5H), 1.25 (m, 3H), 0.97 (m, 6H) ; ³¹P NMR (CDCl₃) δ 17.4, 15.4.

### Example 30

Dibenzylphosphonate 31: A solution of compound 16 (0.15 g, 0.18 mmol) in CHCl₃ (2 mL) was treated with MeOTf (37 mg, 0.23 mmol). The solution was stirred at room temperature for 2 h. The reaction mixture was concentrated and co-evaporated with toluene (2 x), CHCl₃ (2 x) and dried under vacuum to give the dibenzylphosphonate (0.17 g, 95%, GS 273812) as a white solid: ¹H NMR (CDCl₃) δ 9.0 (dd, 1H), 8.73 (m, 2H), 8.09 (m, 1H), 7.35 (m, 10H), 7.09 (d, J = 8.4 Hz, 2H), 6.79 (d, J = 8.1 Hz, 2H), 5.61 (d, J = 4.2 Hz, 1H), 5.2-4.96 (m, 6H), 4.54 (s, 3H), 4.2 (dd, 2H), 3.92-3.69 (m, 6H), 3.3 (m, 2H), 3.04-2.6 (m, 5H), 1.97 (m, 1H), 1.6 (m, 2H), 0.98 (m, 6H); ³¹P NMR (CDCl₃) δ 20.4.

### Example 31

Dibenzylphosphonate 32: A solution of compound 16 (0.15 g, 0.18 mmol) in CH₂Cl₂ (3 mL) at 0°C was treated with *m*CPBA (1.25 eq). The solution was stirred for 1 h at 0°C and then warmed to room temperature overnight. The reaction mixture was partitioned between 10% 2-propanol/CH₂Cl₂ and saturated NaHCO₃. The organic phase was washed with saturated NaCI, dried with Na₂SO₄, filtered, and evaporated under reduced pressure. The crude product was purified by column chromatography on silica gel (10% 2-propanol/CH₂Cl₂) to give the dibenzylphosphonate (0.11 g, 70%, **GS 277774**) as a white solid: ¹H NMR (CDC1₃) δ 8.64 (m, 1H), 8.27 (d, J = 6.9 Hz, 1H), 7.57 (d, J = 8.4 Hz, 1H), 7.36 (m, 11H), 7.10 (d, J = 8.4 Hz, 2H), 6.81 (d, J = 8.7 Hz, 2H), 5.65 (d, J = 5.4 Hz, 1H), 5.22-5.02 (m, 6H), 4.21 (dd, 2H), 3.99-3.65 (m, 6H), 3.2 (m, 2H), 3.03-2.73 (m, 5H), 1.90 (m, 1H), 1.66-1.56 (m, 2H), 0.91 (m, 6H); ³¹P NMR (CDCl₃) δ 20.3.

### Example 32

Phosphonic Acid 33: To a solution of dibenzylphosphonate 32 (0.1 g, 0.12 mmol) in MeOH (4 mL) was added 10% Pd/C (20 mg). The suspension was stirred under H₂ atmosphere (balloon) at room temperature for 1 h. The reaction mixture was filtered through a plug of celite. The filtrate was concentrated and purified by HPLC to give the phosphonic acid (17 mg, GS 277775) as a white solid: ¹H NMR (CD₃OD) δ 8.68 (s, 1H), 8.47 (d, J = 6.0 Hz, 1H), 7.92 (d, J = 7.8 Hz, 1H), 7.68 (m, 1H), 7.14 (m, 2H), 6.90 (d, J = 7.8 Hz, 2H), 5.58 (d, J = 5.4 Hz, 1H), 5.00 (m, 1H), 4.08 (d, J = 9.9 Hz, 2H), 3.93-3.69 (m, 6H), 3.4-2.9 (m. 7H), 2.5 (m, 1H), 2.04 (m, 1H), 1.6-1.35 (m, 2H), 0.92 (m, 6H); ³¹P NMR (CD₃OD) δ 15.8.

### Example 33

Monophospholactate 34: A solution of 25 (2.50 g, 3.21 mmol) in CH₂Cl₂ (5.0 mL) at 0°C was treated with trifluoroacetic acid (2.5 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (30 mL) and cooled to 0°C. Triethylamine (1.79 mL, 12.84 mmol) was added followed by the treatment of 4-formylbenzenesulfonyl chloride (0.72 g, 3.53 mmol) and the solution was stirred at 0°C for 1 h. The product was partitioned between CH₂Cl₂ and 5% HCL The organic phase was washed with H₂O, saturated NaCl, dried with Na₂SO₄, filtered, and evaporated under reduced pressure. The crude product was purified by column chromatography on silica gel (3% 2-propanol/CH₂Cl₂) to give the monophospholactate (2.11 g, 77%, GS 278052, 1:1 diastereomeric mixture) as a white solid: ¹H NMR (CDCl₃) δ 10.12 (s, 1H), 8.05 (d, J = 8.7 Hz, 2H), 7.95 (d, J = 7.5 Hz, 2H), 7.38-7.15 (m, 7H), 6.94 (m, 2H), 5.67 (m, 1H), 5.18-4.91 (m, 3H), 4.57-4.4 (m, 2H), 4.2 (m, 2H), 4.0-3.69 (m, 6H), 3.57 (broad, s, 1H), 3.19-2.8 (m, 7H), 1.87 (m, 1H), 1.69-1.48 (m, 5H), 1.25 (m, 3H), 0.93 (d, J = 6.3 Hz, 3H), 0.88 (d, J = 6.3 Hz, 3H); ³¹P NMR (CDCl₃) δ 17.3, 15.2.

### Example 34

Monophospholactate 35: A solution of 34 (0.60 g, 0.71 mmol) and morpholine (0.31 mL, 3.54 mmol) in EtOAc (8 mL) was treated with HOAc (0.16 mL, 2.83 mmol) and NaBH₃CN (89 mg, 1.42 mmol). The reaction mixture was stirred at room temperature for 4 h. The product was partitioned between EtOAc and H₂O. The organic phase was washed with brine, dried with Na₂SO₄, filtered, and concentrated. The crude product was purified by column chromatography on silica gel (6% 2-propanol/CH₂Cl₂) to give the monophospholactate (0.46 g, 70%, GS 278115, 1:1 diastereomeric mixture) as a white solid: ¹H NMR (CDCl₃) δ 7.74 (d, J = 8.4 Hz, 2H), 7.52 (d, J = 8.4 Hz, 2H), 7.38-7.15 (m, 7H), 6.92 (m, 2H), 5.66 (m, 1H), 5.2-5.0 (m, 2H), 4.57-4.4 (m, 2H), 4.2 (m, 2H), 3.97-3.57 (m, 12H), 3.2-2.78 (m, 7H), 2.46 (broad, s, 4H), 1.87 (m, 1H), 1.64-1.5 (m, 5H), 1.25 (m, 3H), 0.93 (d, J = 6.3 Hz, 3H), 0.88 (d, J = 6.3 Hz, 3H); ³¹P NMR (CDCl₃) δ 17.3, 15.3.

### Example 35

Monophospholactate 37: A solution of 25 (0.50 g, 0.64 mmol) in CH₂Cl₂ (2.0 mL) at 0°C was treated with trifluoroacetic acid (1 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (3 mL) and cooled to 0°C. Triethylamine (0.45 mL, 3.20 mmol) was added followed by the treatment of 4-benzyloxybenzenesulfonyl chloride (0.18 g, 0.64 mmol, prepared according to Toja, E. et al. Eur. J. Med. Chem. 1991, 26, 403). The solution was stirred for 30 min at 0°C and then warmed to room temperature for 30 min. The product was partitioned between CH₂Cl₂ and 0.1 N HCL The organic phase was washed with saturated NaCl, dried with Na₂SO₄, filtered, and concentrated. The crude product was purified by column chromatography on silica gel (4% 2-propanol/CH₂Cl₂) to give the monophospholactate (0.51 g, 85%) as a white solid.

### Example 36

Monophospholactate 38: To a solution of 37 (0.48 g, 0.52 mmol) in EtOH (15 mL) was added 10% Pd/C (0.10 g). The suspension was stirred under H₂ atmosphere (balloon) at room temperature overnight. The reaction mixture was filtered through a plug of celite. The filtrate was concentrated and the crude product was purified by column chromatography on silica gel (5% 2-propanol/CH₂Cl₂) to give the monophospholactate (0.38 g, 88%, GS 273838, 1:1 diastereomeric mixture) as a white solid: ¹H NMR (CDCl₃) δ 8.86 (dd, 1H), 7.42-7.25 (m, 9H), 6.91 (m, 4H), 5.73 (d, J = 5.1 Hz, 1H), 5.42 (m, 1H), 5.18 (m, 2H), 4.76-4.31 (m, 2H), 4.22 (m, 2H), 4.12-3.75 (m, 6H), 3.63 (broad, s, 1H), 3.13 (m, 3H), 2.87 (m, 1H), 2.63 (m, 1H), 2.4 (m, 1H), 2.05 (m, 2H), 1.9 (m, 1H), 1.8(m, 1H), 1.6 (m, 3H), 1.25 (m, 3H), 0.95 (d, J = 6.6 Hz, 3H), 0.85 (d, J = 6.6 Hz, 3H); ³¹P NMR (CDCl₃) δ 17.1, 15.7.

### Example 37

Monophospholactate 40: A solution of 25 (0.75 g, 0.96 mmol) in CH₂Cl₂ (2.0 mL) at 0°C was treated with trifluoroacetic acid (1 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (4 mL) and cooled to 0°C. Triethylamine (0.67 mL, 4.80 mmol) was added followed by the treatment of 4-(4'-benzyloxycarbonyl piperazinyl)benzenesulfonyl chloride (0.48 g, 1.22 mmol, prepared according to Toja, E. et al. Arzneim Forsch. 1994, 44, 501). The solution was stirred at 0°C for 1 h and then warmed to room temperature for 30 min. The product was partitioned between 10% 2-propanol/CH₂Cl₂ and 0.1 N HCl. The organic phase was washed with saturated NaCl, dried with Na₂SO₄, filtered, and concentrated. The crude product was purified by column chromatography on silica gel (3% 2-propanol/CH₂Cl₂) to give the monophospholactate (0.63 g, 60%) as a white solid.

### Example 38

Monophospholactate 41: To a solution of 40 (0.62 g, 0.60 mmol) in MeOH (8 mL) and EtOAc (2 mL) was added 10% Pd/C (0.20 g). The suspension was stirred under H₂ atmosphere (balloon) at room temperature overnight. The reaction mixture was filtered through a plug of celite. The filtrate was treated with 1.2 equivalent of TFA, co-evaporated with CHCl₃ and dried under vacuum to give the monophospholactate (0.55 g, 90%) as a white solid.

### Example 39

Monophospholactate 42: A solution of 41 (0.54 g, 0.53 mmol) and formaldehyde (0.16 mL, 5.30 mmol) in EtOAc (10 mL) was treated with HOAc (0.30 mL, 5.30 mmol) and NaBH₃CN (0.33 g, 5.30 mmol). The reaction mixture was stirred at room temperature overnight. The product was partitioned between EtOAc and H₂O. The organic phase was washed with brine, dried with Na₂SO₄, filtered, and concentrated. The crude product was purified by column chromatography on silica gel (6% 2-propanol/CH₂Cl₂) to give the monophospholactate (97.2 mg, 20%, GS 277937,1:1 diastereomeric mixture) as a white solid: ¹H NMR (CDCl₃) δ 7.64 (d, J = 9.0 Hz, 2H), 7.38-7.17 (m, 7H), 6.95-6.88 (m, 4H), 5.67 (m, 1H), 5.2-4.96 (m, 2H), 4.57-4.4 (m, 2H), 4.2 (m, 2H), 3.97-3.64 (m, 8H), 3.49-3.37 (m, 4H), 3.05-2.78 (m, 12H), 1.88-1.62 (m, 3H), 1.58 (m, 3H), 1.25 (m, 3H), 0.93 (d, J = 6.3 Hz, 3H), 0.88 (d, J = 6.3 Hz, 3H); ³¹P NMR (CDCl₃) δ 17.3, 15.3.

### Example 4.0

Monophospholactate 45: A solution of 43 (0.12 g, 0.16 mmol) and lactate 44 (0.22 g, 1.02 mmol) in pyridine (1 mL) was heated to 70°C and 1,3-dicyclohexylcarbodiimide (0.17 g, 0.83 mmol) was added. The reaction mixture was stirred at 70°C for 4 h and cooled to room temperature. The solvent was removed under reduced pressure. The residue was suspended in EtOAc and 1,3-dicyclohexyl urea was filtered off. The product was partitioned between EtOAc and 0.2 N HCL The EtOAc layer was washed with 0.2 N HCl, H₂O, saturated NaCl, dried with Na₂SO₄, filtered, and concentrated. The crude product was purified by column chromatography on silica gel (3% 2-propanol/CH₂Cl₂) to give the monophospholactate (45 mg, 26%) as a white solid.

### Example 41

Alcohol 46: To a solution of 45 (40 mg, 0.042 mmol) in EtOAc (2 mL) was added 20% Pd(OH)₂/C (10 mg). The suspension was stirred under H₂ atmosphere (balloon) at room temperature for 3 h. The reaction mixture was filtered through a plug of celite. The filtrate was concentrated and the product was dried under vacuum to give the alcohol (33 mg, 90%, GS 278809,3/2 diastereomeric mixture) as a white solid: ¹H NMR (CDCl₃) δ 7.72 (d, J = 8.7 Hz, 2H), 7.39-7.15 (m, 7H), 7.02-6.88 (m, 4H), 5.66 (d, J = 4.5 Hz, 1H), 5.13-5.02 (m, 2H), 4.54-4.10 (m, 4H), 4.00-3.69 (m, 11H), 3.14 (m, 1H), 3.02-2.77 (m, 6H), 1.85-1.6 (m, 6H), 0.94 (d, J = 6.3 Hz, 3H), 0.89 (d, J = 6.3 Hz, 3H); ³¹P NMR (CDCl₃) δ 17.4, 15.9.

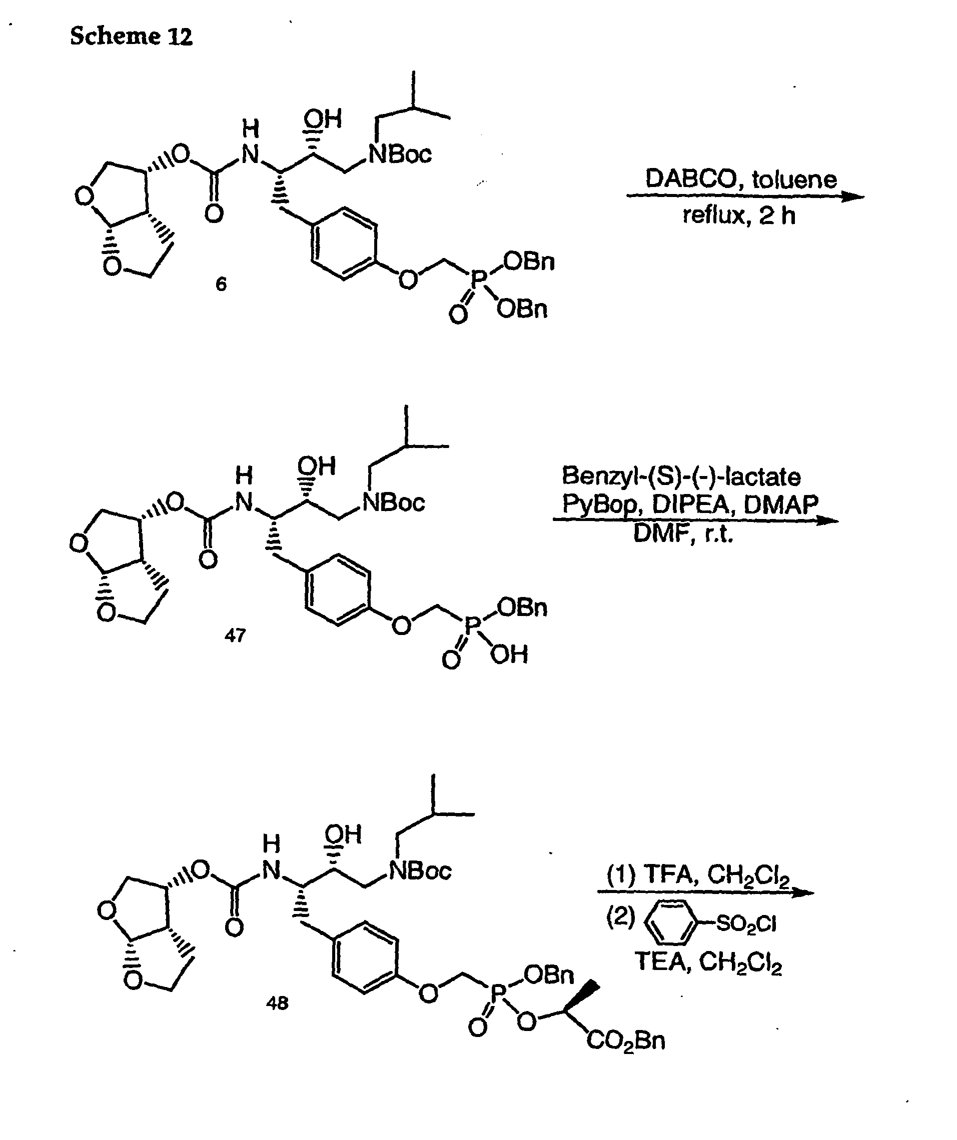

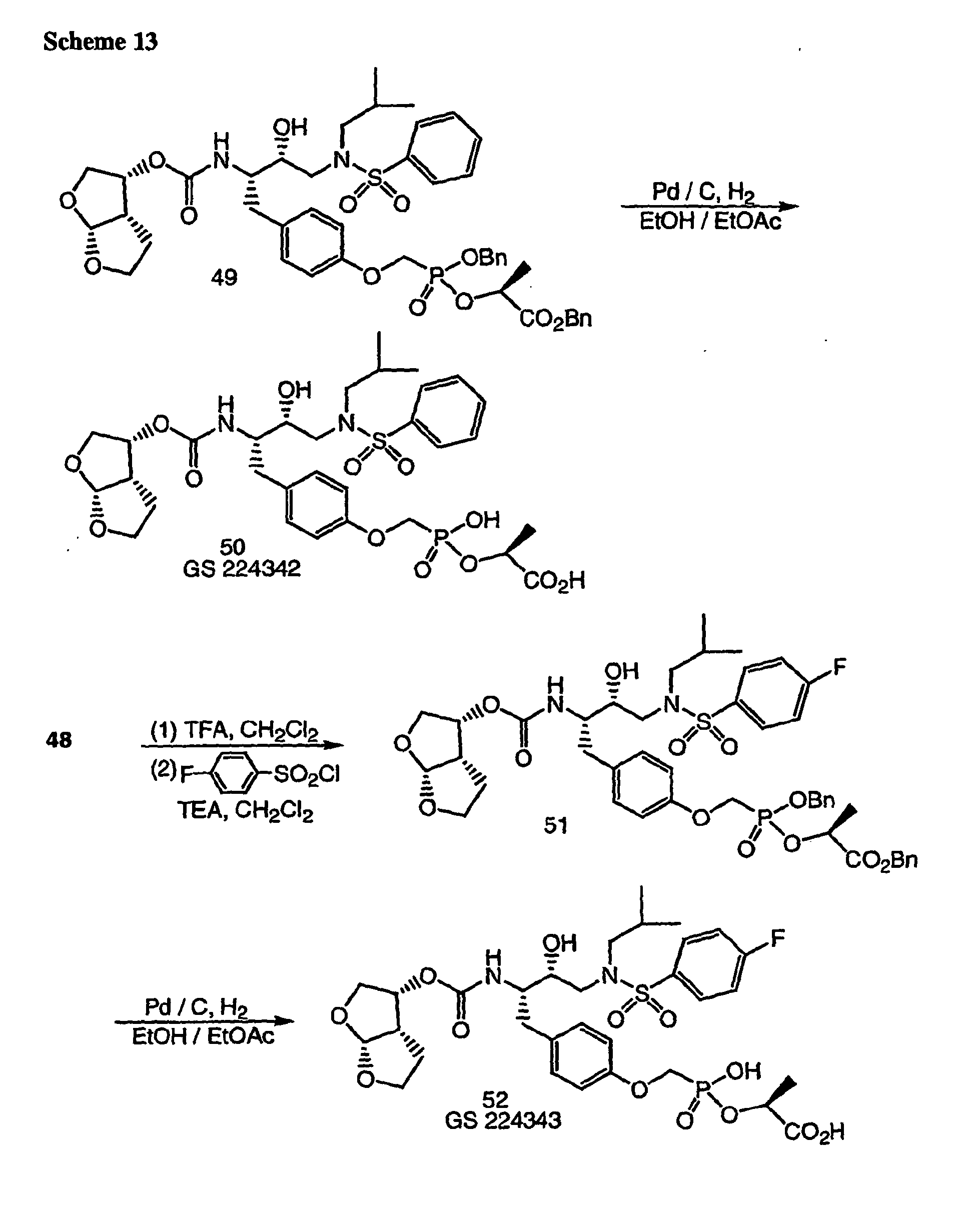

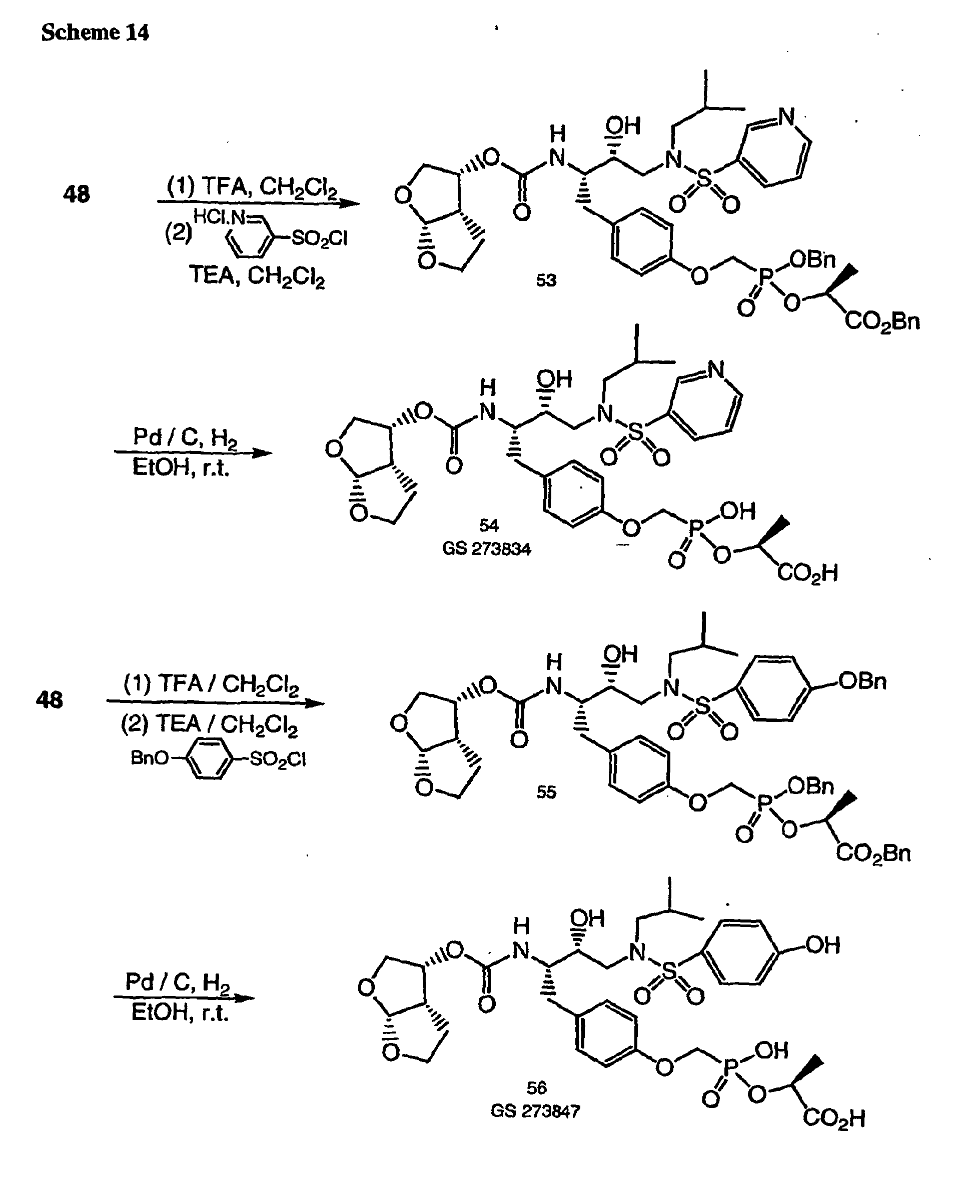

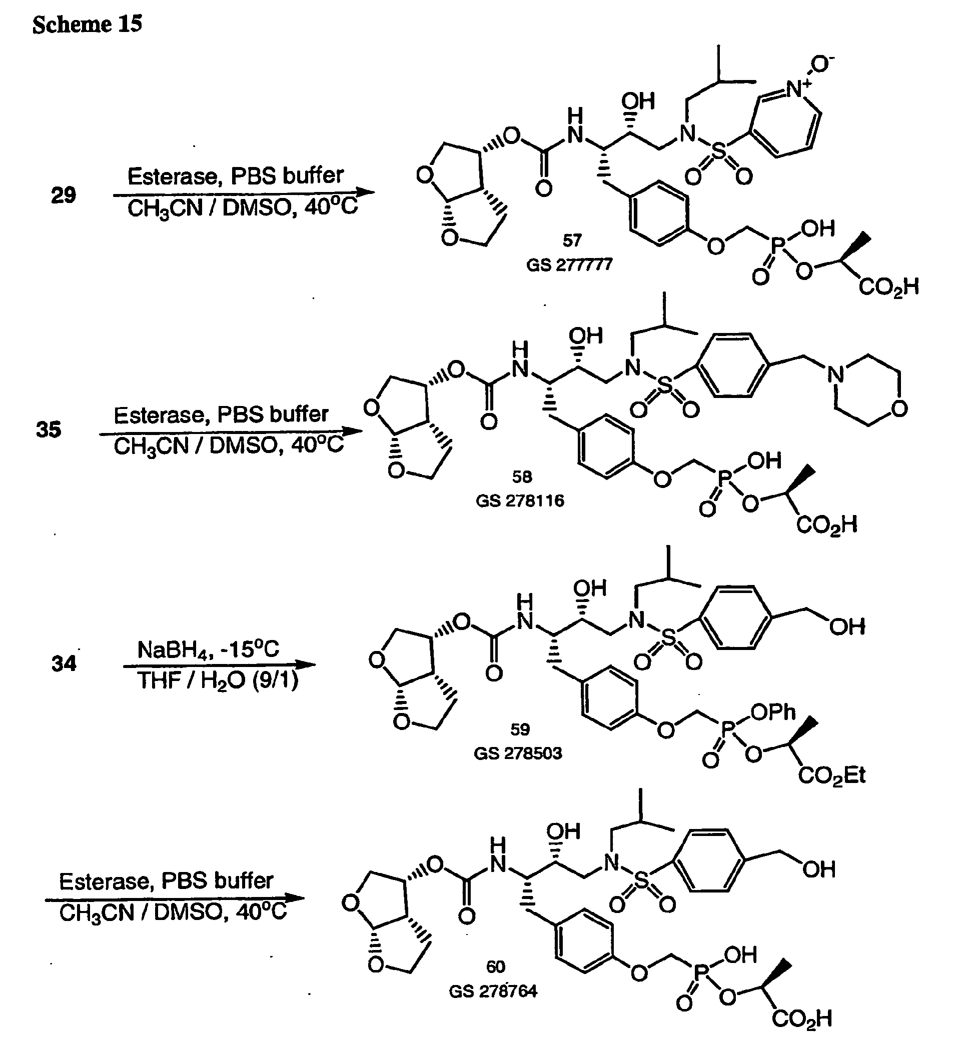

### Example 42

Monobenzylphosphonate 47: A solution of 6 (2.00 g, 2.55 mmol) and DABCO (0.29 g, 2.55 mmol) in toluene (10 mL) was heated to reflux for 2 h. The solvent was evaporated under reduced pressure. The residue was partitioned between EtOAc and 0.2 N HCL The EtOAc layer was washed with H₂O, saturated NaCl, dried with Na₂SO₄, filtered, and concentrated.

The crude product was dried under vacuum to give the monobenzylphosphonate (1.68 g, 95%) as a white solid.

### Example 43

Monophospholactate 48: To a solution of 47 (2.5 g, 3.61 mmol) and benzyl-(S)-(-)-lactate (0.87 mL, 5.42 mmol) in DMF (12 mL) was added PyBop (2.82 g, 5.42 mmol) and *N,N-*diisopropylethylamine (2.51 mL, 14.44 mmol). The reaction mixture was stirred at room temperature for 3 h and concentrated. The residue was partitioned between EtOAc and 0.2 N HCl. The EtOAc layer was washed with H₂O, saturated NaCl, dried with Na₂SO₄, filtered, and concentrated. The crude product was purified by column chromatography on silica gel (3% 2-propanol/CH₂Cl₂) to give the monophospholactate (1.58 g, 51%) as a white solid.

### Example 44

Monophospholactate 49: A solution of 48 (0.30 g, 0.35 mmol) in CH₂Cl₂ (0.6 mL) at 0°C was treated with trifluoroacetic acid (0.3 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (2 mL) and cooled to 0°C. Triethylamine (0.20 mL, 1.40 mmol) was added followed by the treatment of benzenesulfonyl chloride (62 mg, 0.35 mmol). The solution was stirred at 0°C for 30 min and then warmed to room temperature for 30 min. The product was partitioned between CH₂Cl₂ and 0.1 N HCL The organic phase was washed with saturated NaCl, dried with Na₂SO₄, filtered, and concentrated. The crude product was purified by column chromatography on silica gel (3% 2-propanol/CH₂Cl₂) to give the monophospholactate (0.17 g, 53%) as a white solid.

### Example 45

Metabolite X 50: To a solution of 49 (80 mg, 0.09 mmol) in EtOH (6 mL) and EtOAc (2 mL) was added 10% Pd/C (20 mg). The suspension was stirred under H₂ atmosphere (balloon) at room temperature for 8 h. The reaction mixture was filtered through a plug of celite. The filtrate was concentrated, co-evaporated with CHCl₃ and dried under vacuum to give the metabolite X (61 mg, 95%, GS 224342) as a white solid: ¹H NMR (CD₃OD) δ 7.83 (d, J = 6.9 Hz, 2H), 7.65-7.58 (m, 3H), 7.18 (d, J = 7.8 Hz, 2H), 6.90 (d, J = 7.8 Hz, 2H), 5.59 (d, J = 4.8 Hz, 1H), 5.0 (m, 1H), 4.27 (d, J = 10.2 Hz, 2H), 3.95-3.68 (m, 6H), 3.45 (dd, 1H), 3.18-2.84 (m, 6H), 2.50 (m, 1H), 2.02 (m, 1H), 1.6-1.38 (m, 5H), 0.93 (d, J = 6.3 Hz, 3H), 0.88 (d, J = 6.3 Hz, 3H); ³¹P NMR (CD₃OD), δ 18.0.

### Example 46

Monophospholactate 51: A solution of 48 (0.28 g, 0.33 mmol) in CH₂Cl₂ (0.6 mL) at 0°C was treated with trifluoroacetic acid (0.3 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (2 mL) and cooled to 0°C. Triethylamine (0.18 mL, 1.32 mmol) was added followed by the treatment of 4-fluorobenzenesulfonyl chloride (64 mg, 0.33 mmol). The solution was stirred at 0°C for 30 min and then warmed to room temperature for 30 min. The product was partitioned between CH₂Cl₂ and 0.1 N HCL The organic phase was washed with saturated NaCl, dried with Na₂SO₄, filtered, and concentrated. The crude product was purified by column chromatography on silica gel (3% 2-propanol/CH₂Cl₂) to give the monophospholactate (0.16 g, 52%) as a white solid.

### Example 47

Metabolite X 52: To a solution of 51 (80 mg, 0.09 mmol) in EtOH (6 mL) and EtOAc (2 mL) was added 10% Pd/C (20 mg). The suspension was stirred under H₂ atmosphere (balloon) at room temperature for 8 h. The reaction mixture was filtered through a plug of celite. The filtrate was concentrated, co-evaporated with CHCl₃ and dried under vacuum to give the metabolite X (61 mg, 95%, GS 224343) as a white solid: ¹H NMR (CD₃OD) δ 7.9 (dd, 2H), 7.32 (m, 2H), 7.18 (dd, 2H), 6.90 (dd, 2H), 5.59 (d, J = 5.4 Hz, 1H), 5.0 (m, 1H), 4.28 (d, J =10.2 Hz, 2H), 3.95-3.72 (m, 6H), 3.44 (dd, 1H), 3.15-2.85 (m, 6H), 2.5 (m, 1H), 2.02 (m, 1H), 1.55-1.38 (m, 5H), 0.93 (d, J = 6.3 Hz, 3H), 0.88 (d, J = 6.3 Hz, 3H). ³¹P NMR (CD₃OD) δ 18.2.

### Example 48

Monophospholactate 53: A solution of 48 (0.20 g, 0.24 mmol) in CH₂Cl₂ (0.6 mL) at 0°C was treated with trifluoroacetic acid (0.3 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (2 mL) and cooled to 0°C. Triethylamine (0.16 mL, 1.20 mmol) was added followed by the treatment of hydrogen chloride salt of 3-pyridinysulfonyl chloride (50 mg, 0.24 mmol). The solution was stirred at 0°C for 30 min and then warmed to room temperature for 30 min. The product was partitioned between CH₂Cl₂ and H₂O. The organic phase was washed with saturated NaCl, dried with Na₂SO₄, filtered, and concentrated. The crude product was purified by column chromatography on silica gel (4% 2-propanol/CH₂Cl₂) to give the monophospholactate (0.11 g, 53%) as a white solid.

### Example 49

Metabolite X 54: To a solution of 53 (70 mg, 0.09 mmol) in EtOH (5 mL) was added 10% Pd/C (20 mg). The suspension was stirred under H₂ atmosphere (balloon) at room temperature for 5 h. The reaction mixture was filtered through a plug of celite. The filtrate was concentrated, co-evaporated with CHCl₃ and dried under vacuum to give the metabolite X (53 mg, 95%, GS 273834) as a white solid: ¹H NMR (CD₃OD) δ 8.99 (s, 1H), 8.79 (d, J = 4.2 Hz, 1H), 8.29 (d, J = 7.5 Hz, 1H), 7.7 (m, 1H), 7.15 (d, J = 8.4 Hz, 2H), 6.9 (d, J = 7.8 Hz, 2H), 5.59 (d, J = 5.4 Hz, 1H), 5.0 (m, 1H), 4.28 (d, J = 9.9 Hz, 2H), 3.97-3.70 (m, 6H), 3.44 (dd, 1H), 3.17-2.85 (m, 6H), 2.5 (m, 1H), 2.03 (m, 1H), 1.65-1.38 (m, 5H), 0.93 (d, J = 6.3 Hz, 3H), 0.88 (d, J = 6.3 Hz, 3H). ³¹P NMR (CD₃OD) δ 17.8.

### Example 50

Monophospholactate 55: A solution of 48 (0.15 g, 0.18 mmol) in CH₂Cl₂ (1 mL) at 0°C was treated with trifluoroacetic acid (0.5 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (2 mL) and cooled to 0°C. Triethylamine (0.12 mL, 0.88 mmol) was added followed by the treatment of 4-benzyloxybenzenesulfonyl chloride (50 mg, 0.18 mmol). The solution was stirred at 0°C for 30 min and then warmed to room temperature for 30 min. The product was partitioned between CH₂Cl₂ and 0.1 N HCl. The organic phase was washed with saturated Nad, dried with Na₂SO₄, filtered, and concentrated. The crude product was purified by column chromatography on silica gel (3% 2-propanol/CH₂Cl₂) to give the monophospholactate (0.11 g, 63%) as a white solid.

### Example 51

Metabolite X 56: To a solution of 55 (70 mg, 0.07 mmol) in EtOH (4 mL) was added 10% Pd/C (20 mg). The suspension was stirred under H₂ atmosphere (balloon) at room temperature for 4 h. The reaction mixture was filtered through a plug of celite. The filtrate was concentrated, co-evaporated with CHCl₃ and dried under vacuum to give the metabolite X (46 mg, 90%, GS 273847) as a white solid: ¹H NMR (CD₃OD), δ 7.91 (s, 1H), 7.65 (d, J = 8.4 Hz, 2H), 7.17 (d, J = 8.1 Hz, 2H), 6.91 (m, 4H), 5.59 (d, J = 5.1 Hz, 1H), 5.0 (m, 1H), 4.27 (d, J =10.2 Hz, 2H), 3.97-3.74 (m, 6H), 3.4 (dd, 1H), 3.17-2.8 (m, 6H), 2.5 (m, 1H), 2.0 (m, 1H), 1.6-1.38 (m, 5H), 0.93 (d, J = 6.3 Hz, 3H), 0.88 (d, J = 6.3 Hz, 3H); ³¹P NMR (CD₃OD) δ 17.9.

### Example 52

Metabolite X 57: To a suspension of 29 (40 mg, 0.05 mmol) in CH₃CN (1 mL), DMSO (0.5 mL), and 1.0 M PBS buffer (5 mL) was added esterase (200 µL). The suspension was heated to 40°C for 48 h. The reaction mixture was concentrated, suspended in MeOH and filtered. The filtrate was concentrated and purified by HPLC to give the metabolite X (20 mg, 57%, GS 277777) as a white solid: ¹H NMR (CD₃OD) δ 8.68 (s, 1H), 8.47 (d, J = 6.0 Hz, 1H), 7.93 (d, J = 7.8 Hz, 1H), 7.68 (m, 1H), 7.15 (d, J = 8.4 Hz, 2H), 6.9 (d, J = 8.4 Hz, 2H), 5.59 (d, J = 5.4 Hz, 1H), 5.0 (m, 1H), 4.23 (d, J = 10.5 Hz, 2H), 3.97-3.68 (m, 6H), 3.45 (dd, 1H), 3.15-2.87 (m, 6H), 2.46 (m, 1H), 2.0 (m, 1H), 1.6-1.38 (m, 5H), 0.95 (d, J = 6.6 Hz, 3H), 0.92 (d, J = 6.6 Hz, 3H); ³¹P NMR (CD₃OD) δ 17.2.

### Example 53

Metabolite X 58: To a suspension of 35 (60 mg, 0.07 mmol) in CH₃CN (1 mL), DMSO (0.5 mL), and 1.0 M PBS buffer (5 mL) was added esterase (400 µL). The suspension was heated to 40°C for 3 days. The reaction mixture was concentrated, suspended in MeOH and filtered. The filtrate was concentrated and purified by HPLC to give the metabolite X (20 mg, 38%, GS 278116) as a white solid: ¹H NMR (CD₃OD) δ 7.74 (d, J = 6.9 Hz, 2H), 7.63 (d, J = 7.5 Hz, 2H), 7.21 (d, J = 8.4 Hz, 2H), 6.95 (d, J = 8.1 Hz, 2H), 5.64 (d, J = 5.1 Hz, 1H), 5.0 (m, 2H), 4.41 (m, 2H), 4.22 (m, 2H), 3.97-3.65 (m, 12H), 3.15-2.9 (m, 8H), 2.75 (m, 1H), 2.0 (m, 1H), 1.8 (m, 2H), 1.53 (d, J = 6.9 Hz, 3H), 0.88 (m, 6H).

### Example 54

Monophospholactate 59: A solution of 34 (2.10 g, 2.48 mmol) in THF (72 mL) and H₂O (8 mL) at -15°C was treated with NaBH₄ (0.24 g, 6.20 mmol). The reaction mixture was stirred for 10 min at -15°C. The reaction was quenched with 5% aqueous NaHSO₃ and extracted with CH₂Cl₂ (3 x). The combined organic layers were washed with H₂O, dried with Na₂SO₄, filtered, and concentrated. The crude product was purified by column chromatography on silica gel (5% 2-propanol/CH₂Cl₂) to give monophospholactate (1.89 g, 90%, GS 278053, 1:1 diastereomeric mixture) as a white solid: ¹H NMR (CDCl₃) δ 7.64 (m, 2H), 7.51(m, 2H), 7.38-7.19 (m, 7H), 6.92 (m, 2H), 5.69 (d, J = 4.8 Hz, 1H), 5.15 (m, 2H), 4.76 (s, 2H), 4.54 (d, J = 10.5 Hz, 1H), 4.44 (m, 1H), 4.2 (m, 2H), 4.04-3.68 (m, 6H), 3.06-2.62 (m, 7H), 1.8 (m, 3H), 1.62-1.5 (dd, 3H), 1.25 (m, 3H), 0.94 (d, J = 6.3 Hz, 3H), 0.87 (d, J = 6.3 Hz, 3H); ³¹P NMR (CDCl₃) δ 17.4, 15.4.

### Example 55

Metabolite X 60: To a suspension of 59 (70 mg, 0.08 mmol) in CH₃CN (1 mL), DMSO (0.5 mL), and 1.0 M PBS buffer (5 mL) was added esterase (600 µL). The suspension was heated to 40°C for 36 h. The reaction mixture was concentrated, suspended in MeOH and filtered. The filtrate was concentrated and purified by HPLC to give the metabolite X (22 mg, 36%, GS 278764) as a white solid: ¹H NMR (CD₃OD) δ 7.78 (dd, 2H), 7.54 (dd, 2H), 7.15 (m, 2H), 6.9 (m, 2H), 5.57 (d, 1H), 5.0 (m, 2H), 4.65 (m, 4H), 4.2 (m, 2H), 3.9-3.53 (m, 6H), 3.06-2.82 (m, 6H), 2.5 (m, 1H), 2.0 (m, 2H), 1.62-1.35 (m, 3H), 0.94 (m, 6H).

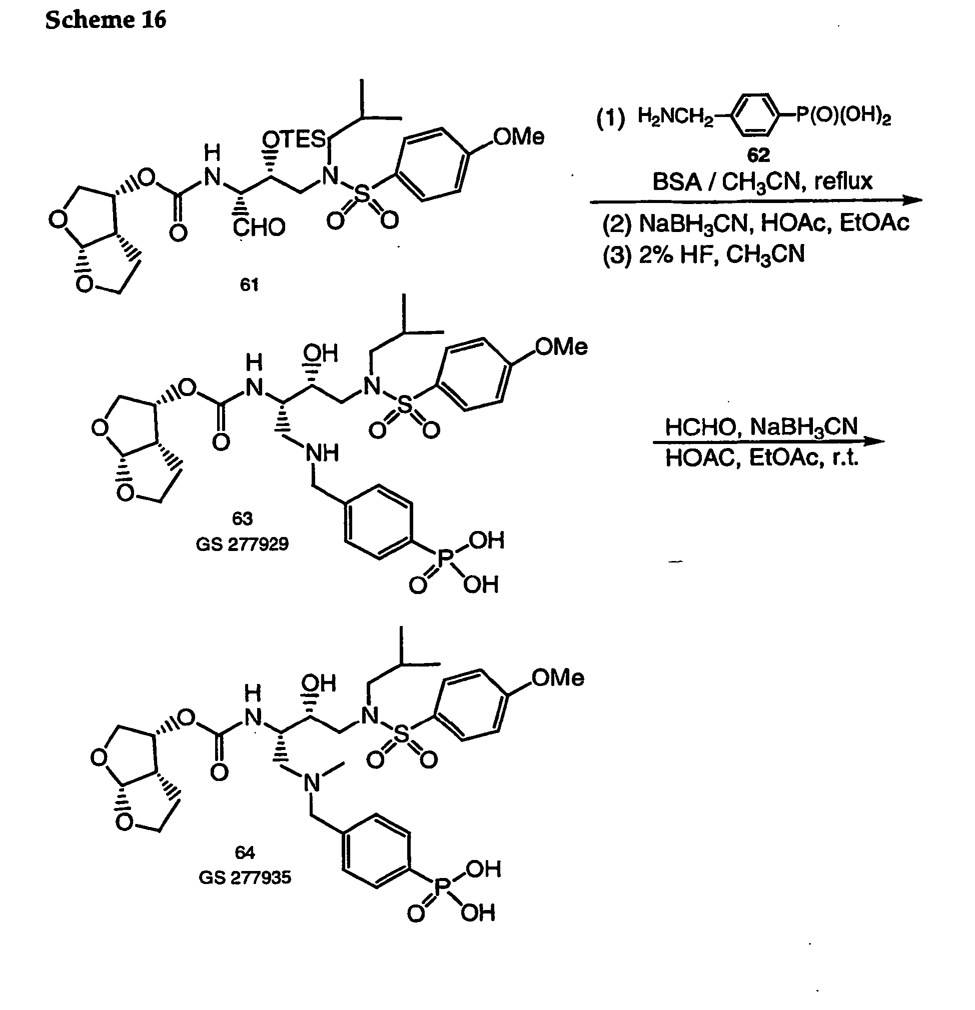

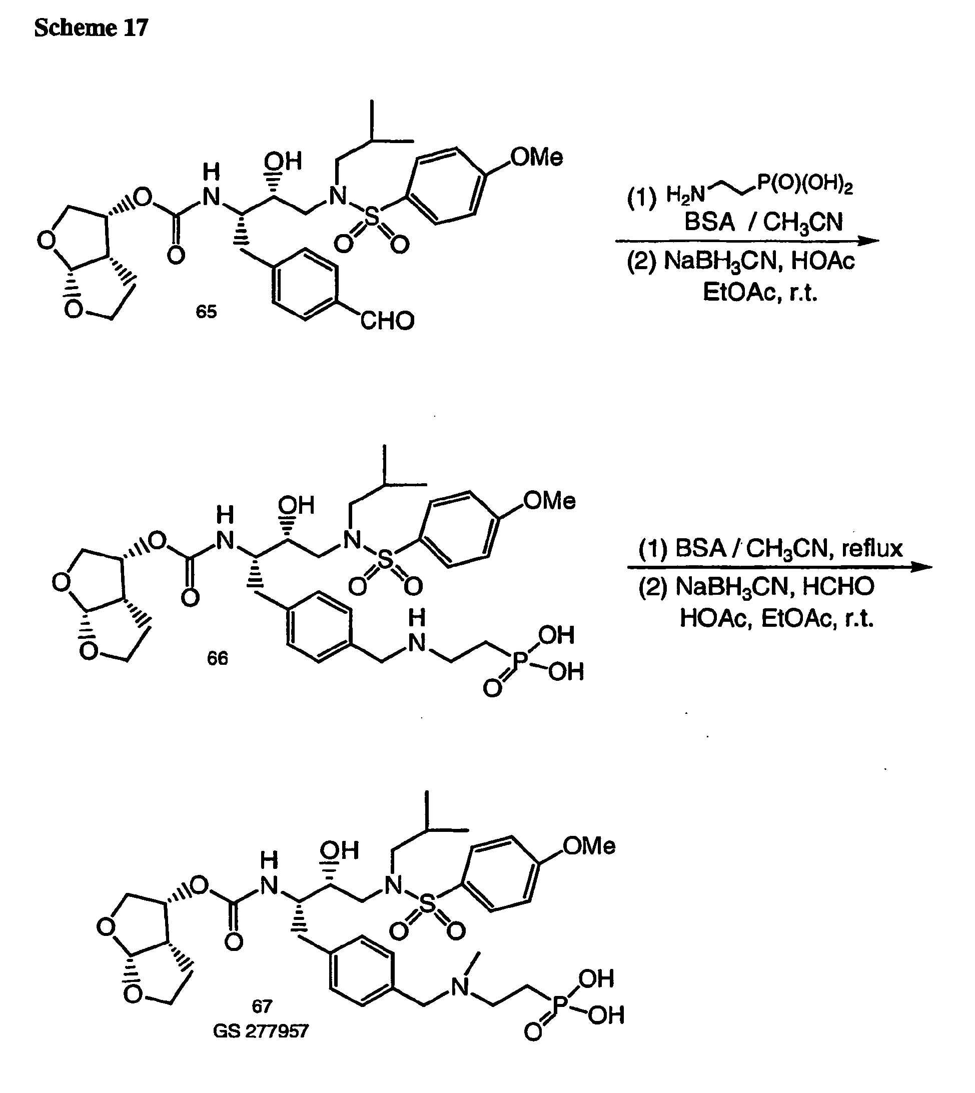

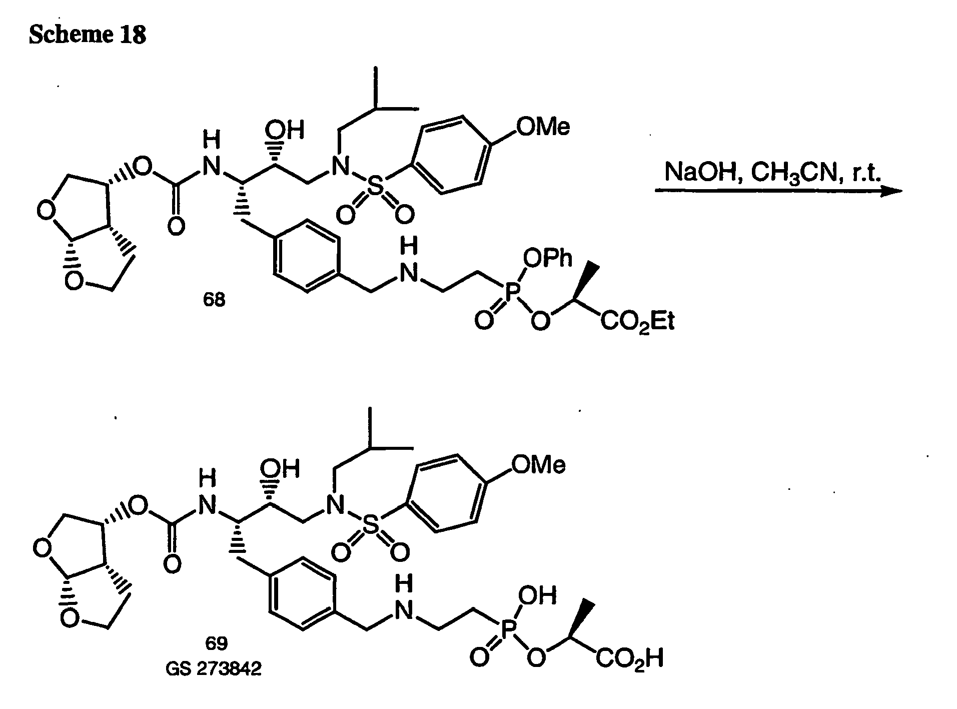

### Example 56

Phosphonic Acid 63: Compound 62 (0.30 g, 1.12 mmol) was dissolved in CH₃CN (5 mL). *N,O*-Bis(trimethylsilyl)acetamide (BSA, 2.2 mL, 8.96 mmol) was added. The reaction mixture was heated to reflux for 2 h, cooled to room temperature, and concentrated. The residue was co-evaporated with toluene and chloroform and dried under vacuum to give a thick oil which was dissolved in EtOAc (4 mL) and cooled to 0°C. Aldehyde 61 (0.20 g, 0.33 mmol), AcOH (0.18 mL, 3.30 mmol), and NaBH₃CN (0.20 g, 3.30 mmol) were added. The reaction mixture was warmed to room temperature and stirred overnight. The reaction was quenched with H₂O, stirred for 30 min, filtered, and concentrated. The crude product was dissolved in CH₃CN (13 mL) and 48% aqueous HF (0.5 mL) was added. The reaction mixture was stirred at room temperature for 2 h and concentrated. The crude product was purified by HPLC to give the phosphonic acid (70 mg, 32%, GS 277929) as a white solid: ¹H NMR (CD₃OD) δ 7.92 (dd, 2H), 7.73 (d, J = 8.7 Hz, 2H), 7.63 (dd, 2H), 7.12 (d, J = 8.7 Hz, 2H), 5.68 (d, J = 5.1 Hz, 1H), 5.13 (m, 1H), 4.4 (m, 2H), 4.05-3.89 (m, 8H), 3.75 (m, 1H), 3.5 (m, 1H), 3.37 (m, 1H), 3.23-3.0 (m, 3H), 2.88-2.7 (m, 2H), 2.2 (m, 1H), 1.8 (m, 2H), 0.92 (d, J = 6.3 Hz, 3H), 0.85 (d, J = 6.3 Hz, 3H); ³¹P NMR (CD₃OD) δ 14.5.

### Example 57

Phosphonic Acid 64: A solution of 63 (50 mg, 0.07 mmol) and formaldehyde (60 mg, 0.70 mmol) in EtOAc (2 mL) was treated with HOAc (43 µL, 0.70 mmol) and NaBH₃CN (47 mg, 0.7 mmol). The reaction mixture was stirred at room temperature for 26 h. The reaction was quenched with H₂O, stirred for 20 min, and concentrated. The crude product was purified by HPLC to give the phosphonic acid (15 mg, 29%, **GS 277935**) as a white solid: ¹H NMR (CD₃OD) δ 7.93 (m, 2H), 7.75 (m, 2H), 7.62 (m, 2H), 7.11 (m, 2H), 5.66 (m, 1H), 5.13 (m, 1H), 4.4 (m, 2H), 4.05-3.89 (m, 8H), 3.75 (m, 2H), 3.09-2.71 (m, 6H), 2.2 (m, 1H), 1.9 (m, 5H), 0.92 (d, J = 6.3 Hz, 3H), 0.85 (d, J = 6.3 Hz, 3H); ³¹P NMR (CD₃OD) δ 14.0.

### Example 58

Phosphonic Acid 66: 2-Aminoethylphosphonic acid (2.60 g, 21.66 mmol) was dissolved in CH₃CN (40 mL). *N,O*-Bis(trimethylsilyl)acetamide (BSA, 40 mL) was added. The reaction mixture was heated to reflux for 2 h and cooled to room temperature and concentrated. The residue was co-evaporated with toluene and chloroform and dried under vacuum to give a thick oil which was dissolved in EtOAc (40 mL). Aldehyde 65 (1.33 g, 2.25 mmol), AcOH (1.30 mL, 22.5 mmol) and NaBH₃CN (1.42 g, 22.5 mmol) were added. The reaction mixture was stirred at room temperature overnight. The reaction was quenched with H₂O, stirred for 1 h, filtered, and concentrated. The residue was dissolved in MeOH and filtered. The crude product was purified by HPLC to give the phosphonic acid (1.00 g, 63%) as a white solid.

### Example 59

Phosphonic Acid 67: Phosphonic acid 66 (0.13 g, 0.19 mmol) was dissolved in CH₃CN (4 mL). *N*,*O*-Bis(trimethylsilyl)acetamide (BSA, 0.45 mL, 1.90 mmol) was added. The reaction mixture was heated to reflux for 2 h, cooled to room temperature, and concentrated. The residue was co-evaporated with toluene and chloroform and dried under vacuum to give a thick oil which was dissolved in EtOAc (3 mL). Formaldehyde (0.15 mL, 1.90 mmol), AcOH (0.11 mL, 1.90 mmol) and NaBH₃CN (63 mg, 1.90 mmol) were added. The reaction mixture was stirred at room temperature overnight. The reaction was quenched with H₂O, stirred for 6 h, filtered, and concentrated. The residue was dissolved in MeOH and filtered. The crude product was purified by HPLC to give the phosphonic acid (40 mg, 30%, GS 277957) as a white solid: ¹H NMR (CD₃OD) δ 7.78 (d, J = 8.4 Hz, 2H), 7.4 (m, 4H), 7.09 (d, J = 8.4 Hz, 2H), 5.6 (d, J = 5.1 Hz, 1H), 4.33 (m, 2H), 3.95-3.65 (m, 9H), 3.5-3.05 (m, 6H), 2.91-2.6 (m, 7H), 2.0 (m, 3H), 1.5 (m, 2H), 0.93 (d, J = 6.3 Hz, 3H), 0.87 (d, J = 6.3 Hz, 3H); ³¹P NMR (CD₃OD) δ 19.7.

### Example 60

Metabolite X 69: Monophospholactate 68 (1.4 g, 1.60 mmol) was dissolved in CH₃CN (20 mL) and H₂O (20 mL). 1.0 N NaOH (3.20 mL, 3.20 mmol) was added. The reaction mixture was stirred at room temperature for 1.5 h and cooled to 0°C. The reaction mixture was acidified to pH = 1-2 with 2 N HCl (1.6 mL, 3.20 mmoL). The solvent was evaporated under reduced pressure. The crude product was purified by HPLC to give the metabolite X (0.60 g, 49%, GS 273842) as a white solid: ¹H NMR (DMSO-d₆) δ 7.72 (d, J = 8.7 Hz, 2H), 7.33 (m, 4H), 7.09 (d, J = 9.0 Hz, 2H), 5.52 (d, J = 5.7 Hz, 1H), 5.1 (broad, s, 1H), 4.85 (m, 1H), 4.63 (m, 1H), 4.13 (m, 2H), 3.8 (m, 5H), 3.6 (m, 4H), 3.36 (m, 1H), 3.03 (m, 4H), 2.79 (m, 3H), 2.5 (m, 1H), 2.0 (m, 3H), 1.5-1.3 (m, 5H), 0.85 (d, J=6.6 Hz, 3H), 0.79 (d, J = 6.6 Hz, 3H); ³¹P NMR (DMSO-d₆) δ 21.9.

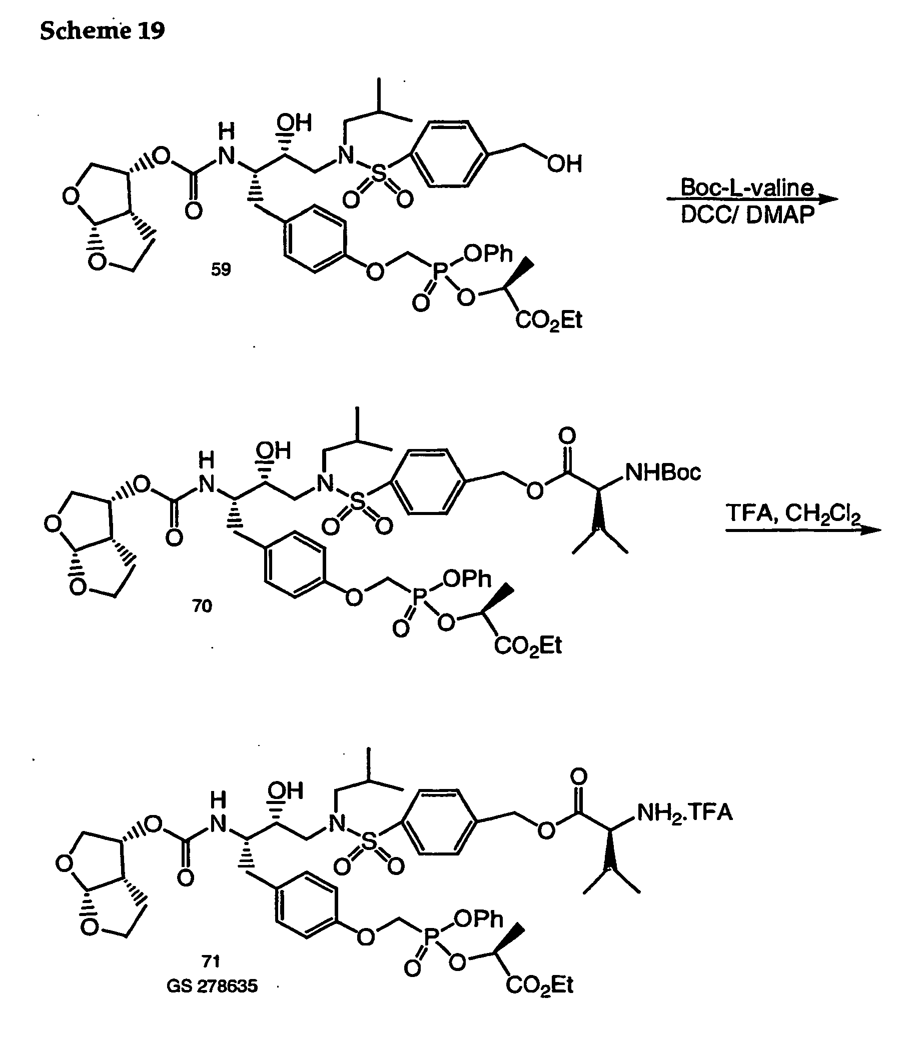

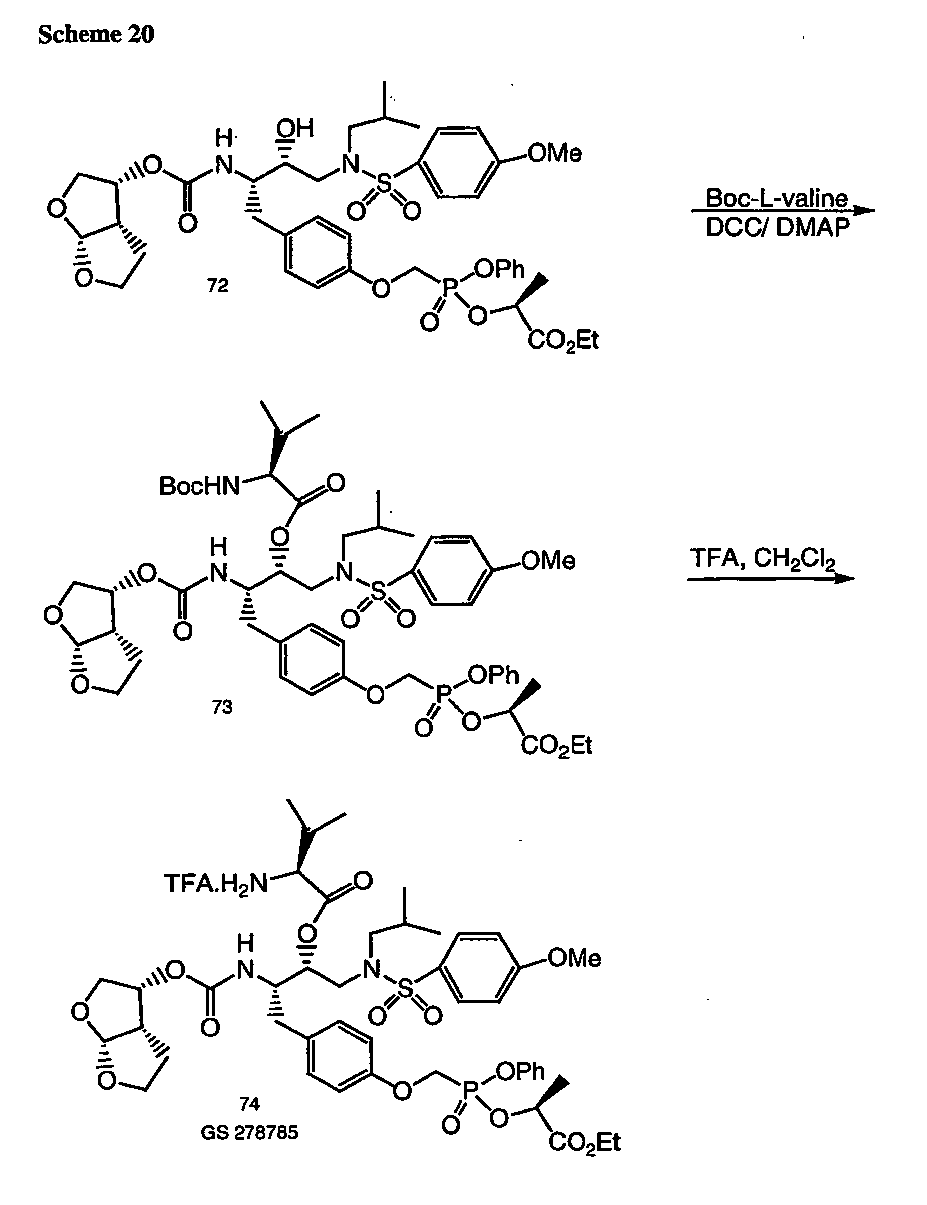

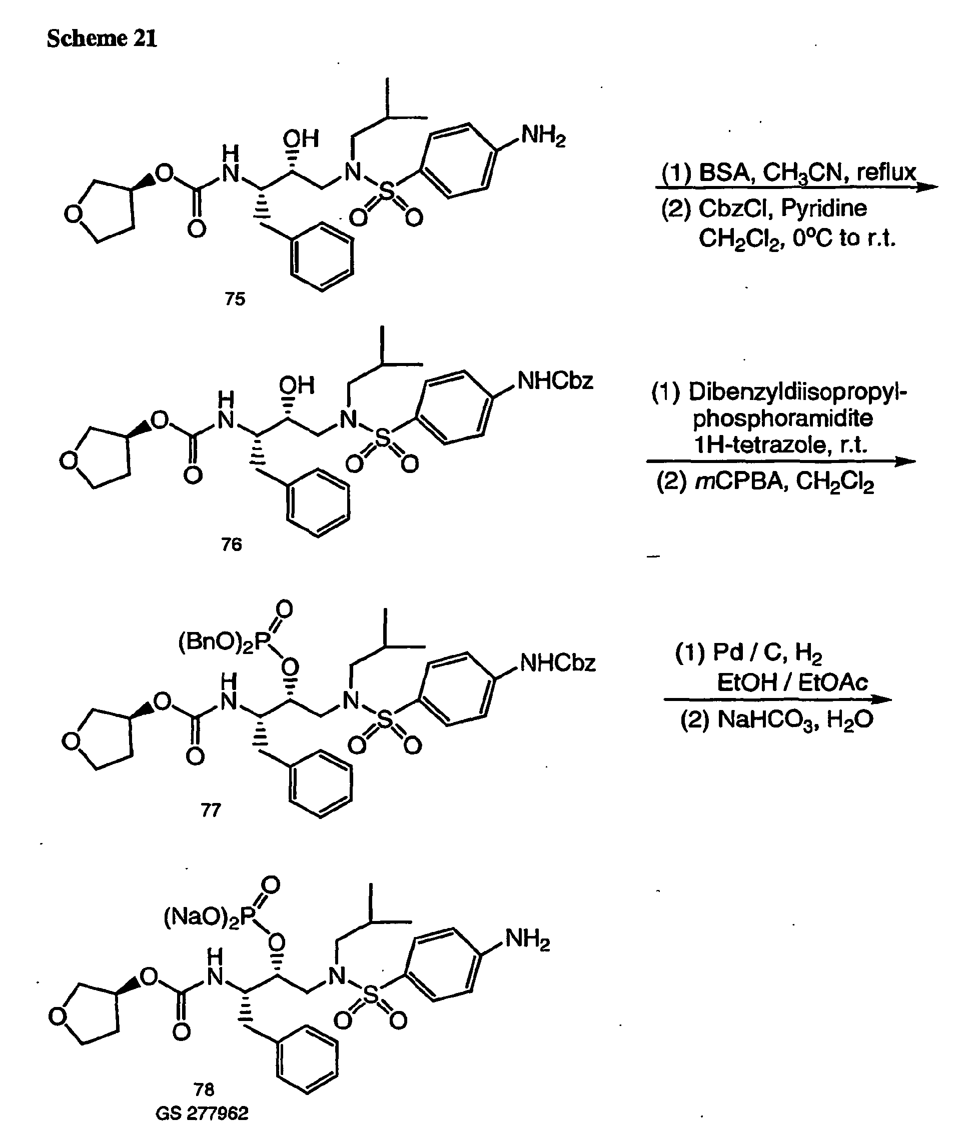

### Example 61

Monophospholactate 70: A solution of 59 (1.48 g, 1.74 mmol) and Boc-L-valine (0.38 g, 1.74 mmol) in CH₂Cl₂ (30 mL) at 0°C was treated with 1,3- dicyclohexylcarbodiimide (0.45 g, 2.18 mmol) and 4-dimethylaminopyridine (26 mg, 0.21 mmol). The reaction mixture was stirred at 0°C for 1 h and then warmed to room temperature for 2 h. The product was partitioned between CH₂Cl₂ and 0.2 N HCl. The organic layer was washed with H₂O, dried with Na₂SO₄, filtered, and concentrated. The crude product was purified by column chromatography on silica gel (4% 2-propanol/CH₂Cl₂) to give the monophospholactate (1.65 g, 90%) as a white solid.

### Example 62

Monophospholactate 71: A solution of 70 (1.65 g, 1.57 mmol) in CH₂Cl₂ (8 mL) at 0°C was treated with trifluoroacetic acid (4 mL). The solution was stirred for 30 min at 0°C and then warmed to room temperature for an additional 30 min. The reaction mixture was diluted with toluene and concentrated under reduced pressure. The crude product was purified by column chromatography on silica gel (10% 2-propanol/CH₂Cl₂) to give the monophospholactate (1.42 g, 85%, GS 278635, 2/3 diastereomeric mixture) as a white solid: ¹H NMR (CDCl₃) δ 7.73 (m, 2H), 7.49 (d, J = 7.2 Hz, 2H), 7.4-7.1 (m, 7H), 6.89 (m, 2H), 5.64 (m, 1H), 5.47 (m, 1H), 5.33-5.06 (m, 4H), 4.57-4.41 (m, 2H), 4.2 (m, 2H), 3.96-3.7 (m, 7H), 3.15-2.73 (m, 7H), 2.38 (m, 1H), 1.9 (m, 1H), 1.7 (m, 1H), 1.63-1.5 (m, 4H), 1.24 (m, 3H), 1.19 (m, 6H), 0.91 (d, 3H), 0.88 (d, 3H); ³¹P NMR (CDCl₃) δ 17.3, 15.4.

### Example 63

Monophospholactate 73: A solution of 72 (0.43 g, 0.50 mmol) and Boc-L-valine (0.11 g, 0.50 mmol) in CH₂Cl₂ (6 mL) was treated with 1,3-dicyclohexylcarbodiimide (0.13 g, 0.63 mmol) and 4-dimethylaminopyridine (62 mg, 0.5 mmol). The reaction mixture was stirred at room temperature overnight. The product was partitioned between CH₂Cl₂ and 0.2 N HCl. The organic layer was washed with H₂O, dried with Na₂SO₄, filtered, and concentrated. The crude product was purified by column chromatography on silica gel (2% 2-propanol/CH₂Cl₂) to give the monophospholactate (0.45 g, 85%) as a white solid.

### Example 64

Monophospholactate 74: A solution of 73 (0.44 g, 0.42 mmol) in CH₂Cl₂ (1 mL) at 0°C was treated with trifluoroacetic acid (0.5 mL). The solution was stirred for 30 min at 0°C and then warmed to room temperature for an additional 30 min. The reaction mixture was diluted with toluene and concentrated under reduced pressure. The crude product was purified by column chromatography on silica gel (10% 2-propanol/CH₂Cl₂) to give the monophospholactate (0.40 g, 90%, GS 278785, 1:1 diastereomeric mixture) as a white solid: ¹H NMR (CDCl₃) δ 7.69 (d, J = 8.4 Hz, 2H), 7.34-7.2 (m, 7H), 6.98 (d, J = 8.4 Hz, 2H), 6.88 (m, 2H), 6.16 (m, 1H), 5.64 (m, 1H), 5.46 (m, 1H), 5.2-5.0 (m, 2H), 4.5 (m, 2H), 4.2 (m, 3H), 4.0-3.4 (m, 9H), 3.3 (m, 1H), 3.0-2.8 (m, 5H), 2.5 (m, 1H), 1.83 (m, 1H), 1.6-1.5 (m, 5H), 125 (m, 3H), 1.15 (m, 6H), 0.82 (d, J = 6.0 Hz, 3H), 0.76 (d, J = 6.0 Hz, 3H); ³¹P NMR (CDCl₃) δ 17.3, 15.5.

### Example 65

Cbz Amide 76: Compound 75 (0.35 g, 0.69 mmol) was dissolved in CH₃CN (6 mL). *N,O-*Bis(trimethylsilyl)acetarnide (BSA, 0.67 mL, 2.76 mmol) was added. The reaction mixture was heated to reflux for 1 h, cooled to room temperature, and concentrated. The residue was co-evaporated with toluene and chloroform and dried under vacuum to give a thick oil which was dissolved in CH₂Cl₂ (3 mL) and cooled to 0°C. Pyridine (0.17 mL, 2.07 mmol) and benzyl chloroformate (0.12 mL, 0.83 mmol) were added. The reaction mixture was stirred at 0°C for 1 h and then warmed to room temperature overnight. The reaction was quenched with MeOH (5 mL) and 10% HCl (20 mL) at 0°C and stirred for 1 h. The product was extracted with CH₂Cl₂, washed with brine, dried with Na₂SO₄, filtered, and concentrated. The crude product was purified by column chromatography on silica gel (3% 2-propanol/CH₂Cl₂) to give the CBz amide (0.40 g, 90%) as a white solid.

### Example 66

Dibenzylphosphonate 77: A solution of 76 (0.39 g, 0.61 mmol) and 1*H*-tetrazole (54 mg, 0.92 mmol) in CH₂Cl₂ (8 mL) was treated with dibenzyldiisopropylphosphoramidite (0.32 g, 0.92 mmol) and stirred at room temperature overnight. The solution was cooled to 0°C, treated with *m*CPBA, stirred for 1 h at 0°C and then warmed to room temperature for 1 h. The reaction mixture was poured into a mixture of aqueous Na₂SO₃ and NaHCO₃ and extracted with CH₂Cl₂. The organic layer was washed with H₂O, dried with Na₂SO₄, filtered, and concentrated. The crude product was purified by column chromatography on silica gel (3% 2-propanol/CH₂Cl₂) to give the dibenzylphosphonate (0.42 g, 76%) as a white solid.

### Example 67

Disodium Salt of Phosphonic Acid 78: To a solution of 77 (0.18 g, 0.20 mmol) in EtOH (20 mL) and EtOAc (4 mL) was added 10% Pd/C (40 mg). The suspension was stirred under H₂ atmosphere (balloon) at room temperature for 4 h. The reaction mixture was filtered through a plug of celite. The filtrate was concentrated and dried under vacuum to give the phosphonic acid (0.11 g, 95%) which was dissolved in H₂O (4 mL) and treated with NaHCO₃ (32 mg, 0.38 mmol). The reaction mixture was stirred at room temperature for 1 h and lyopholyzed overnight to give the disodium salt of phosphonic acid (0.12 g, 99%, GS 277962) as a white solid: ¹H NMR (D₂O) δ 7.55 (dd, 2H), 7.2 (m, 5H), 7.77 (dd, 2H), 4.65 (m, 1H), 4.24 (m, 1H), 4.07 (m, 1H), 3.78-2.6 (m, 12H), 1.88-1.6 (m, 3H), 0.75 (m, 6H).

### Example Section N

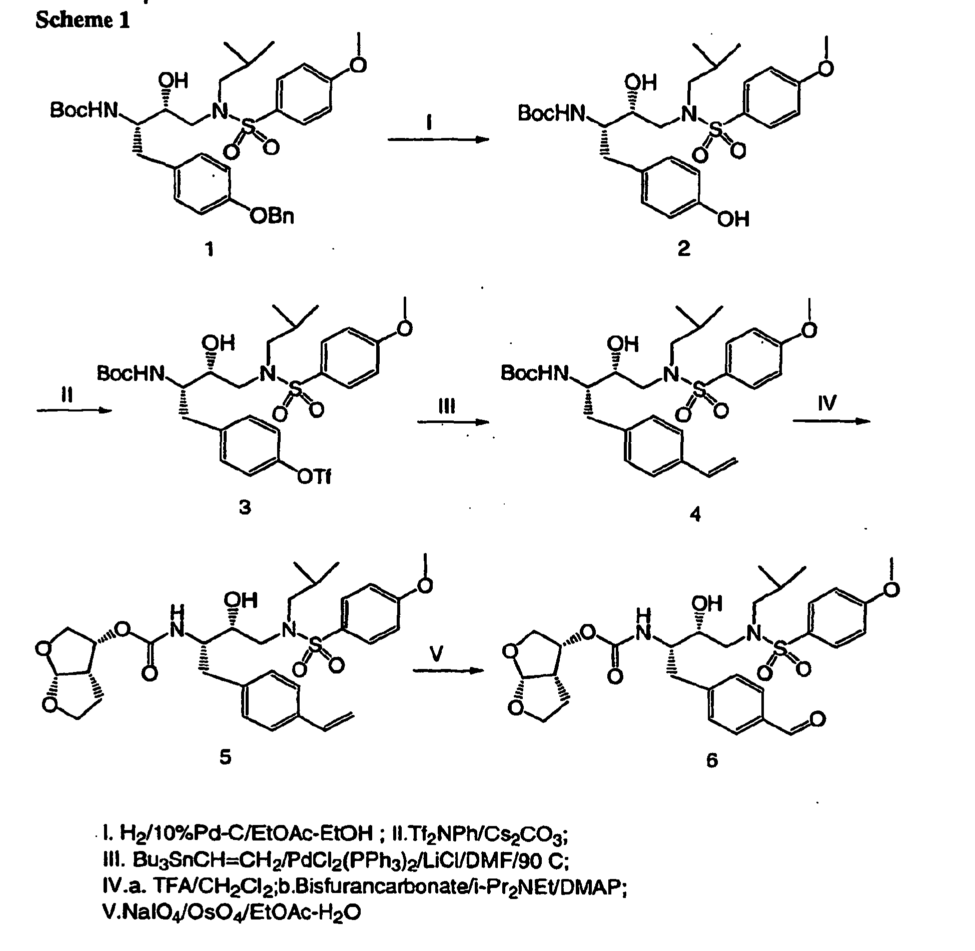

### Example 1

Compound 1 was prepared by methods from Examples herein.

### Example 2

Compound 2: To a solution of compound 1 (47.3 g) in EtOH/EtOAc (1000 mL/500 mL) was added 10% Pd-C (5 g). The mixture was hydrogenated for 19 hours. Celite was added and the mixture was stirred for 10 minutes. The mixture was filtered through a pad of celite and was washed with ethyl acetate. Concentration gave compound 2 (42.1 g).

### Example 3

Compound 3: To a solution of compound 2 (42.3 g, 81 mmol) in CH₂Cl₂ (833 mL) was added N-phenyltrifluoromethanesulfonimide (31.8 g, 89 mmol), followed by cesium carbonate (28.9 g, 89 mmol). The mixture was stirred for 24 hours. The solvent was removed under reduced pressure, and ethyl acetate was added. The reaction mixture was washed with water (3x) and brine (1x), and was dried over MgSO₄. Purification by flash column chromatography (CH₂Cl₂/EtOAc = 13/1) gave compound 3 (49.5 g) as a white powder.

### Example 4

Compound 4: To a solution of compound 3 (25.2, 38.5 mmol) in DMF (240 mL) was added lithium chloride (11.45 g, 270 mmol), followed by dichlorobis(triphenylphosphine) palladium(II) (540 mg, 0.77 mmol). The mixture was stirred for 3 minutes under high vacuum and recharged with nitrogen. To the above solution was added tributylvinyltin (11.25 mL). The reaction mixture was heated at 90°C for 6 hours and cooled to 25°C. Water was added to the reaction, and the mixture was extracted with ethyl acetate (3X). The combined organic layer was washed with water (6x) and brine, and dried over MgSO₄. Concentration gave an oil. The oil was diluted with dichloromethane (40 mL), water (0.693 mL, 38.5 mmol) and DBU (5.76 mL, 38.5 mmol) were added. The mixture was stirred for 5 minutes, and subjected to flash column chromatography (hexanes/EtOAc = 2.5/1). Compound 4 was obtained as white solid (18.4 g).

### Example 5

Compound 5: To a solution of compound 4 (18.4 g, 34.5 mmol) in CH₂C1₂ (70 mL) at 0°C was added trifluoroacetic acid (35 mL). The mixture was stirred at 0°C for 2 hrs, and solvents were evaporated under reduced pressure. The reaction mixture was quenched with saturated sodium carbonate solution, and was extracted with ethyl acetate (3x). The combined organic layer was washed with saturated sodium carbonate solution(lx), water (2x), and brine (1x), and dried over MgSO₄. Concentration gave a solid. To a solution of the above solid in acetonitrile (220 mL) at 0°C was added bisfurancarbonate (10.09 g, 34.2 mmol), followed by di-isopropylethylamine (12.0 mL, 69.1 mmol) and DMAP (843 mg, 6.9 mmol). The mixture was warmed to 25°C and stirred for 12 hours. Solvents were removed under reduced pressure. The mixture was diluted with ethyl acetate, and was washed with water (2X), 5% hydrochloric acid (2x), water (2x), 1N sodium hydroxide (2x), water (2x), and brine (1x), and dried over MgSO₄. Purification by flash column chromatography (hexanes/EtOAc = 1/1)) gave compound 5 (13.5 g).

### Example 6

Compound 6: To a solution of compound 5 (13.5 g, 23 mmol) in ethyl acetate (135 mL) was added water (135 mL), followed by 2.5% osmium tetraoxide/tert-butanol (17 mL). Sodium periodate (11.5 g) was added in portions over 2 minutes period. The mixture was stirred for 90 minutes, and was diluted with ethyl acetate. The organic layer was separated and washed with water (3x) and brine (1x), and dried over MgSO₄. Purification by flash column chromatography (hexanes/EtOAc =½) gave compound 6 as white powder (12 g): ¹H NMR (CDCl₃) δ 9.98 (1 H, s), 7.82 (2 H, m), 7.75 (2 H, m), 7.43 (2 H, m), 6.99 (2 H, m), 5.64 (1 H, m), 5.02 (2 H, m), 4.0-3.8 (9 H, m), 3.2-2.7 (7 H, m), 1.9-1.4 (3 H, m), 0.94 (6 H, m).

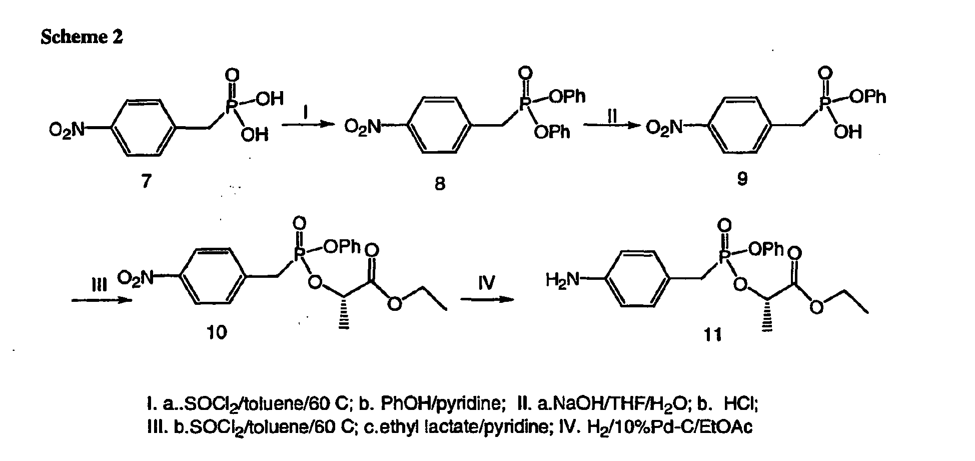

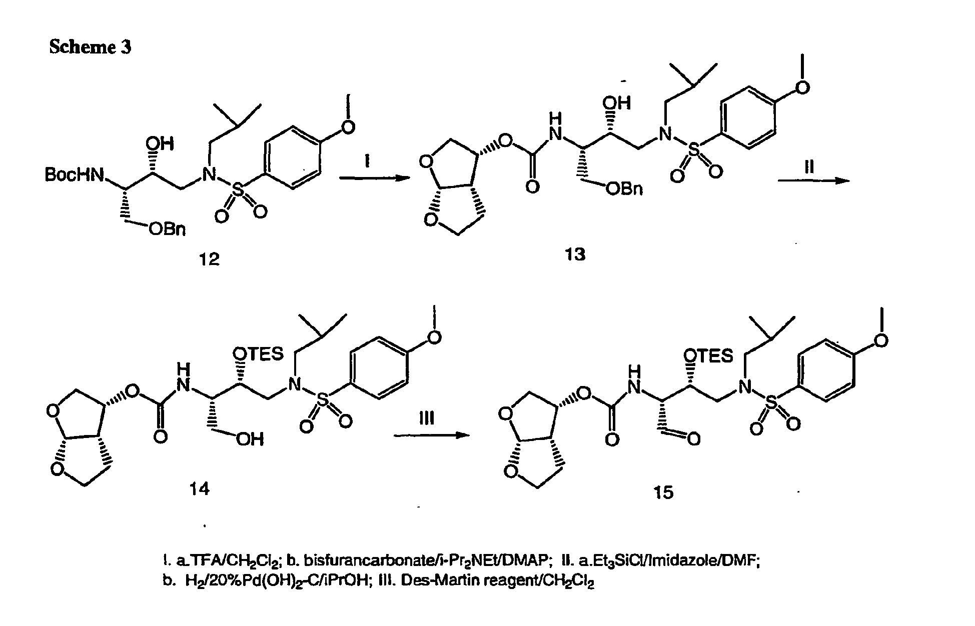

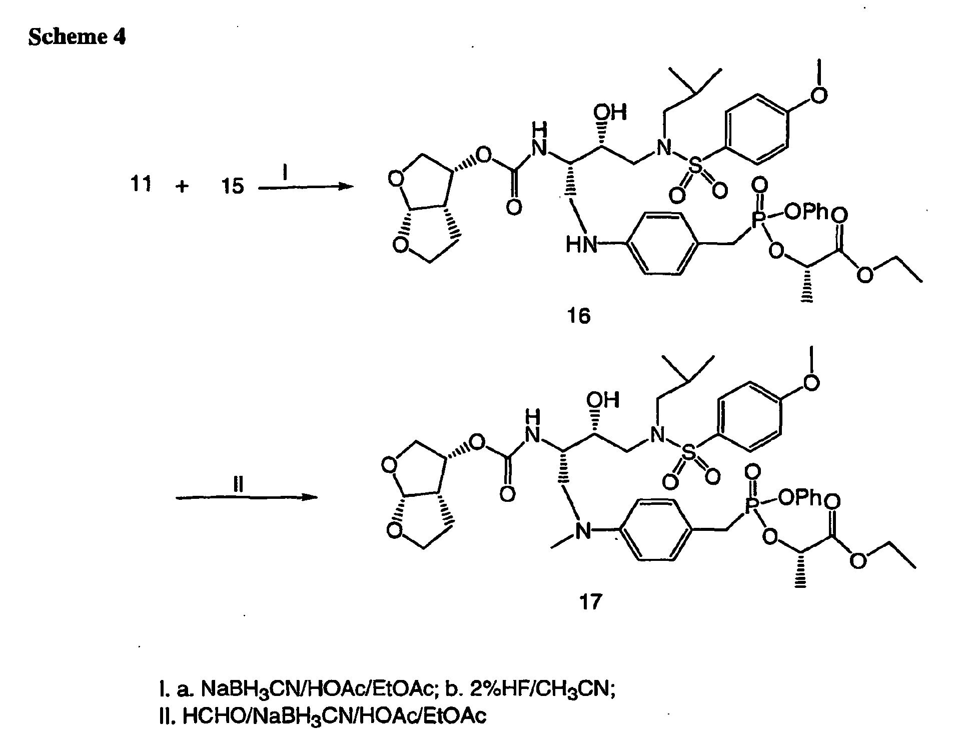

### Example 8

Compound 8: To the suspension of compound 7 (15.8 g, 72.5 mmol) in toluene (140 mL) was added DMF (1.9 mL), followed by thionyl chloride (53 mL, 725 mmol). The reaction mixture was heated at 60°C for 5 hrs, and evaporated under reduced pressure. The mixture was coevaporated with toluene (2x), EtOAc, and CH₂Cl₂ (2x) to afford a brown solid. To the solution of the brown solid in CH₂Cl₂ at 0°C was added phenol (27.2 g, 290 mmol), followed by slow addition of pyridine (35 mL, 435 mmol). The reaction mixture was allowed to warm to 25°C and stirred for 14 hrs. Solvents were removed under reduced pressure. The mixture was diluted with EtOAc, and washed with water (3x) and brine (1x), and dried over MgSO₄. Concentration gave a dark oil, which was purified by flash column chromatography (hexanes/EtOAc = 4/1 to 1/1) to afford compound 8 (12.5 g).

### Example 9

Compound 9: To a solution of compound 8 (2.21 g, 6 mmol) in THF (30 mL) was added 12 mL of 1.0 N NaOH solution. The mixture was stirred at 25°C for 2 hours, and THF was removed under reduced pressure. The mixture was diluted with water, and acetic acid (343 mL, 6 mmol) was added. The aqueous phase was washed with EtOAc (3x), and then acidified with concentrated HCl until pH = 1. The aqueous was extracted with EtOAc (3x). The combined organic layer was washed with water (1x) and brine (1x), and dried over MgSO₄. Concentration under reduced pressure gave compound 9 as a solid (1.1 g).

### Example 10

Compound 10: To a suspension of compound 9 (380 mg, 1.3 mmol) in toluene (2.5 mL) was added thionyl chloride (1 mL, 13 mmol), followed by DMF (1 drop). The mixture was heated at 60°C for 2 hours. The solvent and reagent were removed under reduced pressure. The mixture was coevaporated with toluene (2x) and CH₂Cl₂ to give a white solid. To the solution of the above solid in CH₂Cl₂ (5 ml) at -20°C was added ethyl lactate (294 µL, 2.6 mmol), followed by pyridine (420 µL, 5.2 mmol). The mixture was warmed to 25°C and stirred for 12 hours. The reaction mixture was concentrated under reduced pressure to give a yellow solid, which was purified by flash column chromatography to generate compound 10 (427 mg).

### Example 11

Compound 11: To a solution of compound 10 (480 mg) in EtOAc (20 mL) was added 10% Pd-C (80 mg). The reaction mixture was hydrogenated for 6 hrs. The mixture was stirred with celite for 5 mins, and filtered through a pad of celite. Concentration under reduced pressure gave compound 11 (460 mg).

### Example 12

Compound 12 was prepared by the methods of the Examples herein

### Example 13

Compound 13: To a solution of compound 12 (536 mg, 1.0 mmol) in CH₂Cl₂ (10 mL) was added trifluoroacetic acid (2 mL). The mixture was stirred for 2 hrs, and was concentrated under reduced pressure. The liquid was coevaporated with CH₂Cl₂ (3x) and EtOAc (3x) to give a brown solid. To the solution of above brown solid in acetonitrile (6.5 mL) at 0°C was added bisfurancarbonate (295 mg, 1.0 mmol), followed by diisopropylethylamine (350 µL, 2.0 mmol) and DMAP (24 mg). The mixture was warmed to 25°C, and was stirred for 12 hrs. The mixture was diluted with EtOAc, and was washed sequentially with water (2x), 0.5 N HCl (2x), water (2x), 0.5 N NaOH solution (2x), water (2x), and brine (1x), and dried over MgSO₄. Purification by flash column chromatography (hexanes/EtOAc = 1/1) afford compound 13 (540 mg).

### Example 14

Compound 14: To a solution of compound 13 (400 mg, 0.67 mmol) in DMF (3 mL) was added imidazole (143 mg, 2.10 mmol), followed by triethylchlorosilane (224 µL, 1.34 mmol). The mixture was stirred for 12 hours. The mixture was diluted with EtOAc, and was washed with water (5x) and brine, and dried over MgSO₄. Purification by flash column chromatography (hexanes/EtOAc = 2/1) gave a white solid (427 mg). To the solution of above solid in isopropanol (18 mL) was added 20% palladium(II) hydroxide on carbon (120 mg). The mixture was hydrogenated for 12 hours. The mixture was stirred with celite for 5 mins, and filtered through a pad of celite. Concentration under reduced pressure gave compound 14(360 mg).

### Example 15

Compound 15: To a solution of compound 14 (101 mg, 0.18 mmol) in CH₂Cl₂ (5 mL) was added Dess-Martin periodiane (136 mg, 0.36 mmol). The mixture was stirred for 1 hour. Purification by flash column chromatography (hexanes/EtOAc = 2/1) gave compound 15 (98 mg).

### Example 16

Compound 16: To a solution of compound 15 (50 mg, 0.08 mmol) in EtOAc (0.5 mL) was added compound 11 (150 mg, 0.41 mmol). The mixture was cooled to 0°C, acetic acid (19 µL, 0.32 mmol) was added, followed by sodium cyanoborohydride (10 mg, 0.16 mmol). The mixture was warmed to 25°C, and was stirred for 14 hrs. The mixture was diluted with EtOAc, and was washed with water (3x) and brine, and was dried over MgSO₄. Concentration gave a oil. To the solution of above oil in acetonitrile (2.5 mL) was added 48% HF/CH₃CN (0.1 mL). The mixture was stirred for 30 minutes, and was diluted with EtOAc. The organic phase was washed with water (3x) and brine (1x), and was dried over MgSO₄. Purification by flash column chromatography (CH₂Cl₂/iPrOH = 100/3) gave compound 16 (50 mg): ¹H NMR (CDCl₃) δ 7.72 (2 H, d, J = 8.9 Hz), 7.15-7.05 (7 H, m), 7.30 (2 H, d, J = 8.9 Hz), 6.64 (2 H, m), 5.73 (1 H, m), 5.45 (1 H, m), 5.13 (1 H, m), 4.93 (1 H, m), 4.22-3.75 (11 H, m), 3.4 (4 H, m), 3.35-2.80 (5 H, m), 2.1-1.8 (3 H, m), 1.40-1.25 (6 H, m), 0.94 (6 H, m).

### Example 17

Compound 17: To a solution of compound 16 (30 mg, 0.04 mmol) in EtOAc (0.8 mL) was added 37% formaldehyde (26 µL, 0.4 mmol). The mixture was cooled to 0°C, acetic acid (20 µL, 0.4 mmol) was added, followed by sodium cyanoborohydride (22 mg, 0.4 mmol). The mixture was warmed to 25°C, and was stirred for 14 hrs. The mixture was diluted with EtOAc, and was washed with water (3x) and brine, and was dried over MgSO₄. Purification by flash column chromatography (CH₂Cl₂/iPrOH = 100/3) gave compound 17 (22 mg): ¹H NMR (CDCl₃) δ 7.63 (2 H, m), 7.3-6.9 (9 H, m), 6.79 (2 H, m), 5.68 (1 H, m), 5.2 (1 H, m), 5.10 (1 H, m), 4.95 (1H, m), 4.22 (2 H, m), 4.2-3.7 (21 H, m), 2.0-1.7 (3 H, m), 1.4-1.2 (6 H, m), 0.93 (6 H, m).

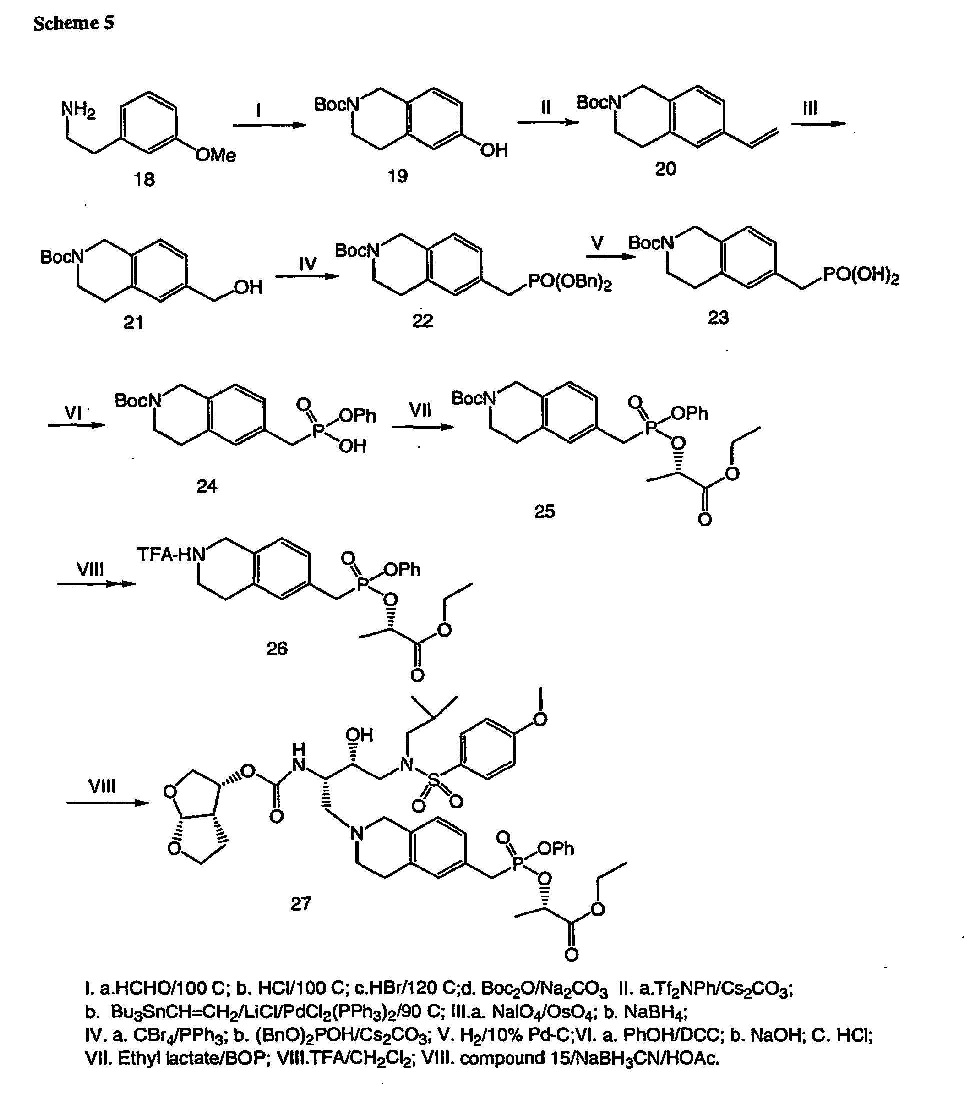

### Example 18

Compound 18: Compound 18 was purchased from Aldrich.

### Example 19

Compound 19: To compound 18 (12.25 g, 81.1 mmol) was added 37% formaldehyde (6.15 mL, 82.7 mmol) slowly. The mixture was heated at 100°C for 1 hour. The mixture was cooled to 25°C, and was diluted with benzene, and was washed with water (2x). Concentration under reduced pressure gave a yellow oil. To above oil was added 20% HCl (16 mL), and the mixture was heated at 100°C for 12 hours. The mixture was basified with 40% KOH solution at 0°C, and was extracted with EtOAc (3x). The combined organic layer was washed with water and brine, and was dried over MgSO₄. Concentration gave a oil. To the oil was added 48% HBr (320 mL), and the mixture was heated at 120°C for 3 hours. Water was removed at 100°C under reduced pressure to give a brown solid. To the solution of above solid in water/dioxane (200 mL/200mL) at 0°C was added sodium carbonate (25.7 g, 243 mmol) slowly, followed by di-tert-butyl dicarbonate (19.4 g, 89 mmol). The mixture was warmed to 25°C and stirred for 12 hours. Dioxane was removed under reduced pressure, and the remaining was extracted with EtOAc (3x). The combined organic phase was washed with water (3x) and brine, and was dried over MgSO₄. Purification by flash column chromatography (hexanes/EtOAc = 4/1 to 3/1) gave compound 19 as white solid (13.6 g).

### Example 20

Compound 20: To a solution of compound 19 (2.49 g, 10 mmol) in CH₂Cl₂ (100 mL) was added N-phenyltrifluoromethanesulfonimide (3.93 g, 11 mmol), followed by cesium carbonate (3.58 g, 11 mmol). The mixture was stirred for 48 hours. The solvent was removed under reduced pressure, and ethyl acetate was added. The reaction mixture was washed with water (3x) and brine (1x), and was dried over MgSO₄. Purification by flash column chromatography (hexanes/EtOAc = 6/1) gave a white solid (3.3 g). To the solution of above solid (2.7 g, 7.1 mmol) in DMF (40 mL) was added lithium chloride (2.11 g, 49.7 mmol), followed by dichlorobis(triphenylphosphine) palladium(II) (100 mg, 0.14 mmol). The mixture was stirred for 3 minutes under high vacuum and recharged with nitrogen. To ' the above solution was added tributylvinyltin (2.07 mL, 7.1 mmol). The reaction mixture was heated at 90°C for 3 hours and cooled to 25°C. Water was added to the reaction, and the mixture was extracted with ethyl acetate (3X). The combined organic layer was washed with water (6x) and brine, and dried over MgSO₄. Concentration gave an oil. The oil was diluted with CH₂Cl₂ (5 mL), water (128 µL, 7.1mmol) and DBU (1 mL, 7.1 mmol) were added. The mixture was stirred for 5 minutes, and was subjected to flash column chromatography (hexanes/EtOAc = 9/1). Compound 20 was obtained as white solid (1.43 g).

### Example 21

Compound 21: To a solution of compound 20 (1.36 g, 5.25 mmol) in ethyl acetate (16 mL) was added water (16 mL), followed by 2.5% osmium tetraoxide/tert-butanol (2.63 mL). Sodium periodate (2.44 g) was added in portions over 2 minutes period. The mixture was stirred for 45 minutes, and was diluted with ethyl acetate. The organic layer was separated and washed with water (3x) and brine (1x), and dried over MgSO₄. Concentration gave a brown solid. To the solution of above solid in methanol (100 mL) at 0°C was added sodium borohydride. The mixture was stirred for 1 hour at 0°C, and was quenched with saturated NH₄Cl (40 mL). Methanol was removed under reduced pressure, and the remaining was extracted with EtOAc (3x). The combined organic layer was washed with water and brine, and was dried over MgSO₄. Purification by flash column chromatography (hexanes/EtOAc = 2/1) gave compound 21 (1.0 g).

### Example 22

Compound 22: To a solution of compound 21 (657 mg, 2.57 mmol) in CH₂Cl₂ (2 mL) was added a solution of tetrabromocarbon (1.276 g, 3.86 mmol) in CH₂Cl₂ (2 mL). To the above mixture was added a solution of triphenylphsophine (673 mg, 2.57 mmol) in CH₂Cl₂ (2 mL) over 30 minutes period. The mixture was stirred for 2 hours, and was concentrated under reduced pressure. Purification by flash column chromatography (hexanes/EtOAc = 9/1) gave the bromide intermediate (549 mg). To the solution of above bromide (548. mg, 1.69 mmol) in acetonitrile (4.8 mL) was added dibenzyl phosphite (0.48 mL, 2.19 mmol), followed by cesium carbonate (828 mg, 2.54 mmol). The mixture was stirred for 48 hours, and was diluted with EtOAc.
The mixture was washed with water (3x) and brine, and was dried over MgSO₄. Purification by flash column chromatography (hexanes/EtOAc = 3/1 to 100% EtOAc) gave compound 22 (863 mg).

### Example 23

Compound 23: To a solution of compound 22 (840 mg) in ethanol (80 mL) was added 10% palladium on carbon (200 mg). The mixture was hydrogenated for 2 hours. The mixture was stirred with celite for 5 mins, and was filtered through a pad of celite. Concentration under reduced pressure gave compound 23 (504 mg).

### Example 24

Compound 24: To a solution of compound 23 (504 mg, 1.54 mmol) in pyridine (10.5 mL) was added phenol (1.45 g, 15.4 mmol), followed by DCC (1.28 g, 6.2 mmol). The mixture was heated at 65°C for 3 hours, and pyridine was removed under reduced pressure. The mixture was diluted with EtOAc (5 ml), and was filtered and washed with EtOAc (2x5 mL). Concentration gave a oil, which was purified by flash column chromatography (CH₂Cl₂/isopropanol = 100/3) to give diphenylphosphonate intermediate (340 mg). To a solution of above compound (341 mg, 0.71 mmol) in THF (1 mL) was added 0.85 mL of 1.0 N NaOH solution. The mixture was stirred at 25°C for 3 hours, and THF was removed under reduced pressure. The mixture was diluted with water, and was washed with EtOAc (3x), and then acidified with concentrated HCl until pH = 1. The aqueous was extracted with EtOAc (3x). The combined organic layer was washed with water (1x) and brine (1x), and dried over MgSO₄. Concentration under reduced pressure gave compound 24 as a solid (270 mg).

### Example 25:

Compound 25: To a solution of compound 24 (230 mg, 0.57 mmol) in DMF (2 mL) was added ethyl (s)-lactate (130 µL, 1.14 mmol), followed by diisopropylethylamine (400 µL, 2.28 mmol) and benzotriazol-1-yloxytris(dimethylamino)phosphonium hexafluorophosphate (504 mg, 1.14 mmol). The mixture was stirred for 14 hours, was diluted with EtOAc. The organic phase was washed with water (5x) and brine (1x), and was dried over MgSO₄. Purification by flash column chromatography (CH₂Cl₂/isopropanol = 100/3) gave compound 25 (220 mg).

### Example 26

Compound 26: To a solution of compound 25 (220 mg) in CH₂Cl₂ (2 mL) was added trifluoroacetic acid (1 mL). The mixture was stirred for 2 hrs, and was concentrated under reduced pressure. The mixture was diluted with EtOAc, and was washed with saturated sodium carbonate solution, water, and brine, and was dried over MgSO₄. Concentration gave compound 26 (170 mg).

### Example 27

Compound 27: To a solution of compound 15 (258 mg, 0.42 mmol) in EtOAc (2.6 mL) was added compound 26 (170 mg, 0.42 mmol), followed by acetic acid (75 µL, 1.26 mmol). The mixture was stirred for 5 minutes, and sodium cyanoborohydride (53 mg, 0.84 mmol) was added. The mixture was stirred for 14 hrs. The mixture was diluted with EtOAc, and was washed with saturated sodium bicarbonate solution, water (3x) and brine, and was dried over MgSO₄. Purification by flash column chromatography (CH₂Cl₂/iPrOH = 100/4 to 100/6) gave the intermediate (440 mg). To the solution of above compound (440 mg) in acetonitrile (10 mL) was added 48% HF/ CH₃CN (0.4 mL). The mixture was stirred for 2 hours, and acetonitrile was removed under reduced pressure. The remaining was diluted with EtOAc, and was washed with water (3x) and brine (1x), and was dried over MgSO₄. Purification by flash column chromatography (CH₂Cl₂/iPrOH = 100/5) gave compound 27 (120 mg): ¹H NMR (CDCl₃) δ 7.70 (2 H, m), 7.27 (2 H, m), 7.15 (5 H, m), 6.95 (3 H, m), 5.73 (1H, m), 5.6-5.4 (1H, m), 5.16 (1H, m), 4.96 (1H, m), 4.22-3.60 (13 H, m), 3.42 (2 H, m), 3.4-2.6 (11 H, m), 2.1-3.8 (3 H, m), 1.39 (3 H, m), 1.24 (3 H, m), 0.84 (6 H, m).

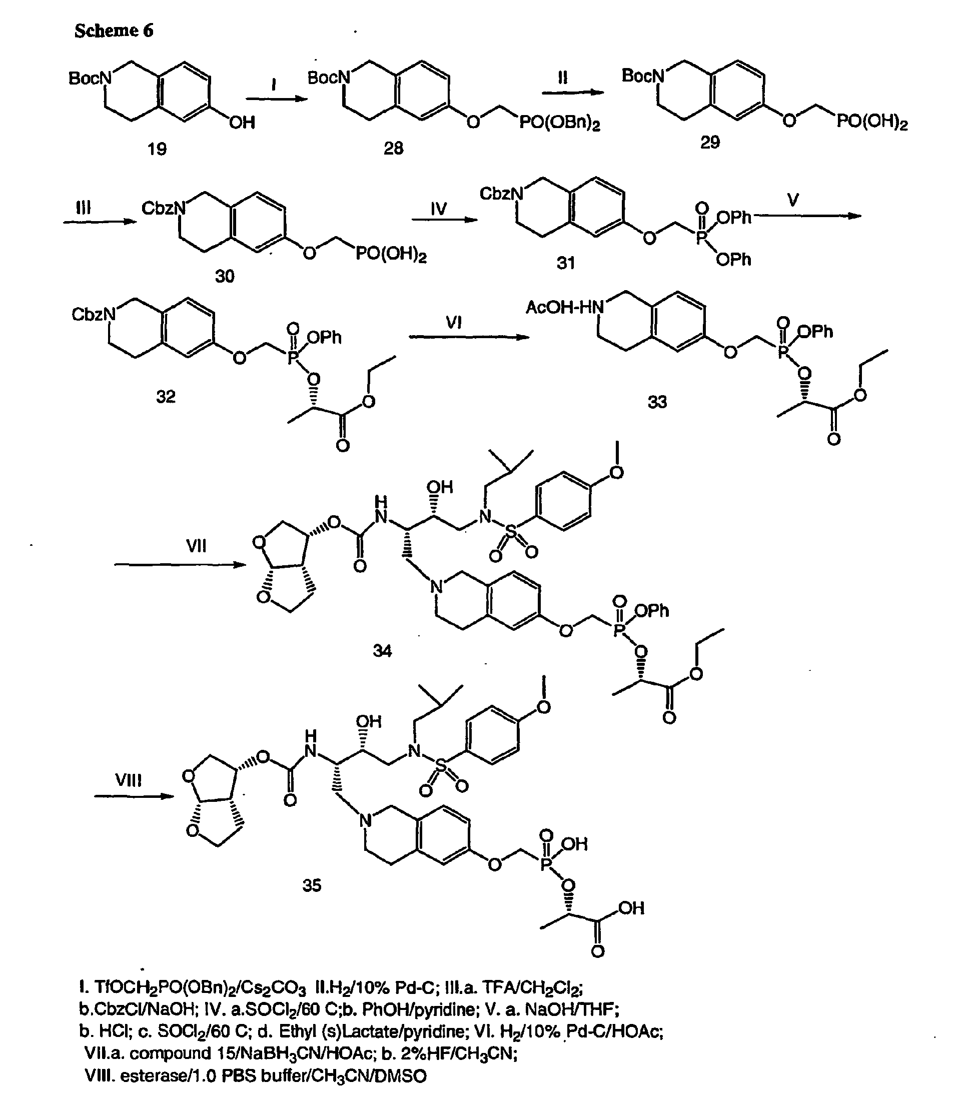

### Example 28

Compound 28: To a solution of compound 19 (7.5 g, 30 mmol) in acetonitrile (420 mL) was added dibenzyl triflate (17.8 g, 42 mmol), followed by cesium carbonate (29.4 g, 90 mmol).

The mixture was stirred for 2.5 hours, and was filtered. Acetonitrile was removed under reduced pressure, and the remaining was diluted with EtOAc. The mixture was washed with water (3x) and brine, and was dried over MgSO₄. Purification by flash column chromatography (hexanes/EtOAc = 2/1 to 1/1) gave compound 28 (14.3 g).

### Example 29

Compound 29: To a solution of compound 28 (14.3 g) in ethanol (500 mL) was added 10% palladium on carbon (1.45 g). The mixture was hydrogenated for 2 hours. The mixture was stirred with celite for 5 mins, and was filtered through a pad of celite. Concentration under reduced pressure gave compound 29 (9.1 g).

### Example 30

Compound 30: To a solution of compound 29 (9.1 g) in CH₂Cl₂ (60 mL) was added trifluoroacetic acid (30 mL). The mixture was stirred for 4 hrs, and was concentrated under reduced pressure. The mixture was coevaporated with CH₂Cl₂ (3x) and toluene, and was dried under high vacuum to give a white solid. The white solid was dissolved in 2.0 N NaOH solution (45 mL, 90 mmol), and was cooled to 0°C. To the above solution was added slowly a solution of benzyl chloroformate (6.4 mL, 45 mmol) in toluene (7 mL). The mixture was warmed to 25°C, and was stirred for 6 hours. 2.0 N sodium hydroxide was added to above solution until pH =11. The aqueous was extracted with ethyl ether (3x), and was cooled to 0°C. To the above aqueous phase at 0°C was added concentrated HCl until pH = 1. The aqueous was extracted with EtOAc (3x). The combine organic layers were washed with brine, and were dried over MgSO₄. Concentration gave compound 30 (11.3 g) as a white solid.

### Example 31

Compound 31: To the suspension of compound 30 (11.3 g, 30 mmol) in toluene (150 mL) was added thionyl chloride (13 mL, 180 mmol), followed by DMF (a few drops). The reaction mixture was heated at 65°C for 4.5 hrs, and evaporated under reduced pressure. The mixture was coevaporated with toluene (2x) to afford a brown solid. To the solution of the brown solid in CH₂Cl₂ (120 ml) at 0°C was added phenol (11.28 g, 120 mmol), followed by slow addition of pyridine (14.6 mL, 180 mmol). The reaction mixture was allowed to warm to 25°C and stirred for 14 hrs. Solvents were removed under reduced pressure. The mixture was diluted with EtOAc, and washed with water (3x) and brine (1x), and dried over MgSO₄. Concentration gave a dark oil, which was purified by flash column chromatography (hexanes/EtOAc = 3/1 to 1/1) to afford compound 31 (9.8 g).

### Example 32

Compound 32: To a solution of compound 31 (9.8 g, 18.5 mmol) in THF (26 mL) was added 20.3 mL of 1.0 N NaOH solution. The mixture was stirred at 25°C for 2.5 hours, and THF was removed under reduced pressure. The mixture was diluted with water, and was washed with EtOAc (3x). The aqueous phase was cooled to 0°C, and was acidified with concentrated HCl until pH = 1. The aqueous was extracted with EtOAc (3x). The combined organic layer was washed with water (1x) and brine (1x), and dried over MgSO₄. Concentration under reduced pressure gave a solid (8.2 g). To a suspension of above solid (4.5 g, 10 mmol) in toluene (50 mL) was added thionyl chloride (4.4 mL, 60 mmol), followed by DMF (0.2 mL). The mixture was heated at 70°C for 3.5 hours. The solvent and reagent were removed under reduced pressure. The mixture was coevaporated with toluene (2x) to give a white solid. To the solution of the above solid in CH₂Cl₂ (40 mL) at 0°C was added ethyl (s)-lactate (2.3 mL, 20 mmol), followed by pyridine (3.2 mL, 40 mmol). The mixture was warmed to 25°C and stirred for 12 hours. The reaction mixture was concentrated under reduced pressure, and was diluted with EtOAc. The organic phase was washed with 1 N HCl, water, and brine, and was dried over MgSO₄. Purification by flash column chromatography (hexanes/EtOAc = 2/1 to 1/1) gave compound 32 (4.1 g).

### Example 33

Compound 33: To a solution of compound 32 (3.8 g, 6.9 mmol) in EtOAc/EtOH (30 mL/30 mL) was added 10% palladium on carbon (380 mg), followed by acetic acid (400 µL, 6.9 mmol). The mixture was hydrogenated for 3 hours. The mixture was stirred with celite for 5 mins, and was filtered through a pad of celite. Concentration under reduced pressure gave compound 33 (3.5 g).

### Example 34

Compound 34: To a solution of compound 15 (1.70 g, 2.76 mmol) in EtOAc (17 mL) was added compound 33 (3.50 g, 6.9 mmol). The mixture was stirred for 5 minutes, and was cooled to 0°C, and sodium cyanoborohydride (347 mg, 5.52 mmol) was added. The mixture was stirred for 6 hrs. The mixture was diluted with EtOAc, and was washed with saturated sodium bicarbonate solution, water (3x) and brine, and was dried over MgSO₄. Purification by flash column chromatography (CH₂Cl₂/iPrOH = 100/6) gave the intermediate (3.4 g). To the solution of above compound (3.4 g) in acetonitrile (100 mL) was added 48% HF/ CH₃CN (4 mL). The mixture was stirred for 2 hours, and acetonitrile was removed under reduced pressure. The remaining was diluted with EtOAc, and was washed with saturated sodium carbonate, water (3x), and brine (1x), and was dried over MgSO₄. Purification by flash column chromatography (CH₂Cl₂/iPrOH = 100/5) gave compound 34 (920 mg): ¹H NMR (CDCl₃) δ 7.71 (2 H, m), 7.38-7.19 (5 H, m), 6.92 (3 H, m), 6.75 (2 H, m), 5.73 (1 H, m), 5.57-5.35 (1 H, m), 5.16 (2 H, m), 4.5 (2 H, m), 4.2-3.6 (13 H, m), 3.25-2.50 (11 H, m), 2.0-1.8(3H,m), 1.5 (3 H, m), 1.23 (3 H, m), 0.89 (6 H, m).

### Example 35

Compound 35: To a solution of compound 34 (40 mg) in CH₃CN /DMSO (1 mL/0.5 mL) was added 1.0 M PBS buffer (5 mL), followed by esterase (200 µL). The mixture was heated at 40°C for 48 hours. The mixture was purified by reverse phase HPLC to give compound 35 (11 mg).

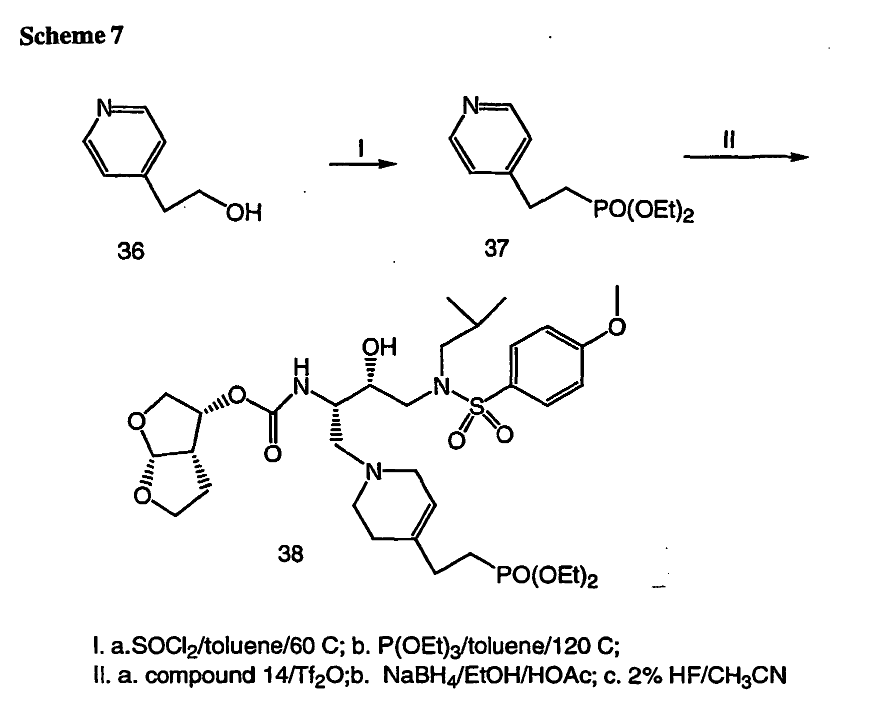

### Example 36

Compound 36: Compound 36 was purchased from Aldrich.

### Example 37

Compound 37: To a solution of compound 36 (5.0 g, 40 mmol) in chloroform (50 mL) was added thionyl chloride (12 mL) slowly. The mixture was heated at 60°C for 2.5 hours. The mixture was concentrated under reduced pressure to give a yellow solid. To the suspension of above solid (5.2 g, 37 mmol) in toluene (250 mL) was added triethyl phosphite (19 mL, 370 mmol). The mixture was heated at 120°C for 4 hours, and was concentrated under reduced pressure to give a brown solid. The solid was dissolved in EtOAc, and was basified with 1.0 N NaOH. The organic phase was separated and was washed with water (2x) and brine, and was dried over MgSO₄. Purification by flash column chromatography (CH₂Cl₂/iPrOH = 9/1) gave compound 37 (4.8 g).

### Example 38

Compound 38: To a solution of compound 14 (100 mg, 0.16 mmol) and compound 37 (232 mg, 0.74 mmol) in CH₂Cl₂ (1 mL) at -40°C was added triflic anhydride (40 µL, 0.24 mmol) slowly. The mixture was warmed to 25°C slowly, and was stirred for 12 hours. The mixture was concentrated, and was diluted with EtOH/EtOAc (2 mL/0.4 mL). To the above solution at 0°C was added sodium borohydride (91 mg) in portions. The mixture was stirred at 0°C for 3 hours, and was diluted with EtOAc. The mixture was washed with saturated sodium bicarbonate, water, and brine, and was dried over MgS0₄. Purification by flash column chromatograph (CH₂Cl₂/iPrOH = 100/5 to 100/10) gave the intermediate (33 mg). To the solution of above intermediate in acetonitrile (2.5 mL) was added 48% HF/ CH₃CN (0.1 mL). The mixture was stirred for 30 minutes, and was diluted with EtOAc. The organic solution was washed with 0.5 N sodium hydroxide, water, and brine, was dried over MgSO₄. Purification by reverse HPLC gave compound 38 (12 mg): ¹H NMR (CDCl₃) δ 7.72 (2 H, d, J = 8.9 Hz), 7.02 (2 H, d, J = 8.9 Hz), 5.70 (1 H, m), 5.45 (1 H, m), 5.05 (1 H, m), 4.2-3.4 (19 H, m), 3.4-2.8 (5 H, m), 2.45-2.20 (4 H, m), 2.15-1.81 (5 H, m), 1.33 (6 H, m), 0.89 (6 H, m).

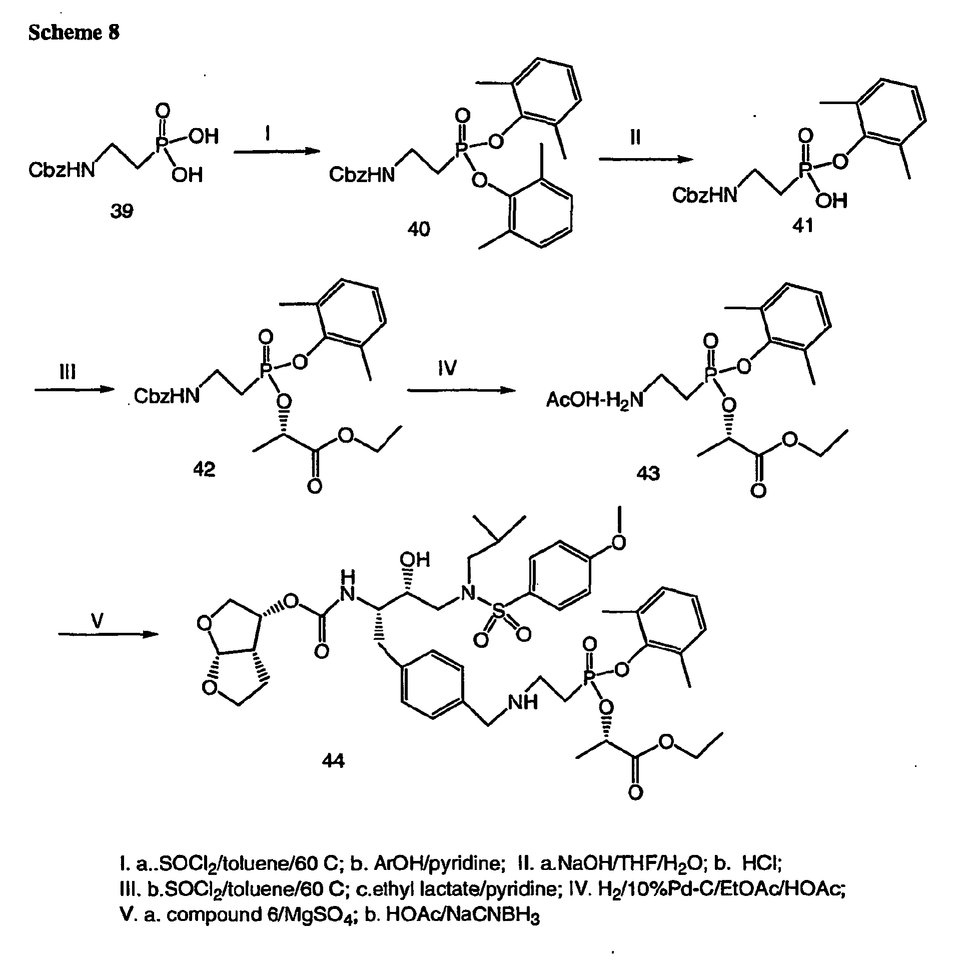

### Example 39

Compound 39 was prepared by the methods of the previous Examples.

### Example 40

Compound 40: To the suspension of compound 39 (4.25 g, 16.4 mmol) in toluene (60 mL) was added thionyl chloride (7.2 mL, 99 mmol), followed by DMF (a few drops). The reaction mixture was heated at 65°C for 5 hrs, and evaporated under reduced pressure. The mixture was coevaporated with toluene (2x) to afford a brown solid. To the solution of the brown solid in CH₂Cl₂ (60 ml) at 0°C was added 2,6-dimethylphenol (8.1 g, 66 mmol), followed by slow addition of pyridine (8mL, 99 mmol). The reaction mixture was allowed to warm to 25°C and stirred for 14 hrs. Solvents were removed under reduced pressure. The mixture was diluted with EtOAc, and washed with water (3x) and brine (1x), and dried over MgSO₄. Purification by flash column chromatography (hexanes/EtOAc = 3/1 to 1/1) afforded compound 40 (1.38 g).

### Example 41

Compound 41: To a solution of compound 40 (1.38 g, 1.96 mmol) in THF (6 mL) was added 3.55 mL of 1.0 N NaOH solution. The mixture was stirred at 25°C for 24 hours, and THF was removed under reduced pressure. The mixture was diluted with water, and was washed with EtOAc (3x). The aqueous phase was cooled to 0°C, and was acidified with concentrated HCl until pH = 1. The aqueous was extracted with EtOAc (3x). The combined organic layer was washed with water (1x) and brine (1x), and dried over MgSO₄. Concentration under reduced pressure gave compound 41 as a white solid (860 mg).

### Example 42

Compound 42: To a suspension of compound 41 (1.00 g, 2.75 mmol) in toluene (15 mL) was added thionyl chloride (1.20 mL, 16.5 mmol), followed by DMF (3 drops). The mixture was heated at 65°C for 5 hours. The solvent and reagent were removed under reduced pressure. The mixture was coevaporated with toluene (2x) to give a brown solid. To the solution of the above solid in CH₂Cl₂ (11 mL) at 0°C was added ethyl (s)-lactate (1.25, 11 mmol), followed by pyridine (1.33 mL, 16.6 mmol). The mixture was warmed to 25°C and stirred for 12 hours. The reaction mixture was concentrated under reduced pressure, and was diluted with EtOAc. The organic phase was washed with 1 N HCl, water, and brine, and was dried over MgSO₄. Purification by flash column chromatography (hexaneslEtOAc = 1.5/1 to 1/1) gave compound 42 (470 mg).

### Example 43

Compound 43: To a solution of compound 42 (470 mg) in EtOH (10 mL) was added 10% palladium on carbon (90 mg), followed by acetic acid (150 µL). The mixture was hydrogenated for 6 hours. The mixture was stirred with celite for 5 mins, and was filtered through a pad of celite. Concentration under reduced pressure gave compound 43 (400 mg).

### Example 44

Compound 44: To a solution of compound 6 (551 mg, 0.93 mmol) in 1,2-dichloroethane (4 mL) was added compound 43 (400 mg, 1.0 mmol), followed by MgSO₄ (1 g). The mixture was stirred for 3 hours, and acetic acid (148 µL) and sodium cyanoborohydride (117 mg, 1.86 mmol) were added sequentially. The mixture was stirred for 1 hour. The mixture was diluted with EtOAc, and was washed with saturated sodium bicarbonate solution, water (3x) and brine, and was dried over MgSO₄. Purification by flash column chromatography (EtOAc to EtOAc/EtOH = 9/1) gave compound 44. Compound 44 was dissolved in CH₂Cl₂ (25 mL), and trifluoroacetic acid (100 µL) was added. The mixture was concentrated to give compound 44 as a TFA salt (560 mg): ¹H NMR (CDCl₃) 5 7.74 (2 H, m), 7.39 (2 H, m), 7.20 (2 H, m), 7.03 (5 H, m), 5.68 (1 H, m), 5.43 (1 H, m), 5.01 (1 H, m), 4.79 (1 H, m), 4.35-4.20 (4 H, m), 4.18-3.4 (11 H, m), 3.2-2.6 (9 H, m), 2.30 (6 H, m), 1.82 (1 H, m), 1.70 (2 H, m), 1.40-1.18 (6 H, m), 0.91 (6 H, m).

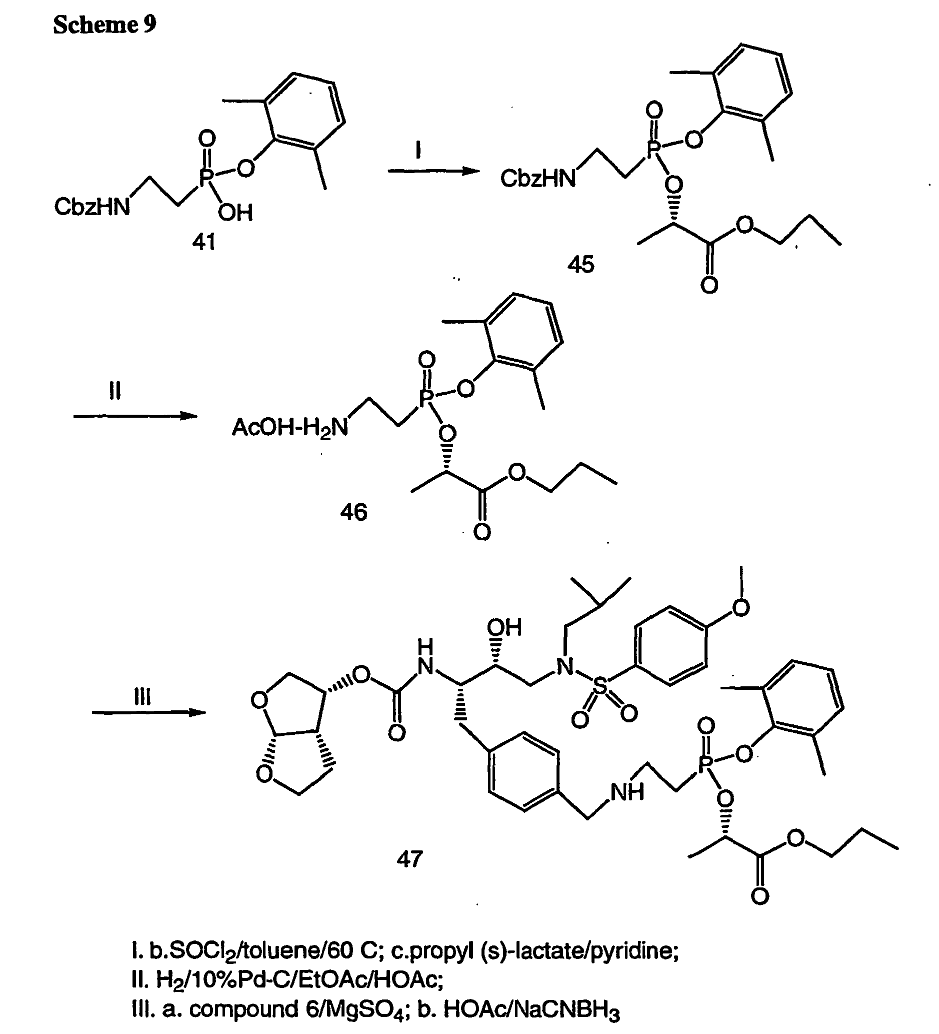

### Example 45

Compound 45: To a suspension of compound 41 (863 mg, 2.4 mmol) in toluene (13 mL) was added thionyl chloride (1.0mL, 14.3 mmol), followed by DMF (3 drops). The mixture was heated at 65°C for 5 hours. The solvent and reagent were removed under reduced pressure. The mixture was coevaporated with toluene (2x) to give a brown solid. To the solution of the above solid in CH₂Cl₂ (10 mL) at 0°C was added propyl (s)-lactate (1.2mL, 9.6 mmol), followed by triethylamine (2.0 mL, 14.4 mmol). The mixture was warmed to 25°C and stirred for 12 hours. The reaction mixture was concentrated under reduced pressure, and was diluted with EtOAc. The organic phase was washed with water and brine, and was dried over MgSO₄. Purification by flash column chromatography (hexanes/EtOAc = 1.5/1 to 1/1) gave compound 45 (800 mg).

### Example 46

Compound 46: To a solution of compound 45 (785 mg) in EtOH (17 mL) was added 10% palladium on carbon (150 mg), followed by acetic acid (250 µL). The mixture was hydrogenated for 16 hours. The mixture was stirred with celite for 5 mins, and was filtered through a pad of celite. Concentration under reduced pressure gave compound 46 (700 mg).

### Example 47

Compound 47: To a solution of compound 6 (550 mg, 0.93 mmol) in 1,2-dichloroethane (4 mL) was added compound 43 (404 mg, 1.0 mmol), followed by MgSO₄ (1 g). The mixture was stirred for 3 hours, and acetic acid (148 µL) and sodium cyanoborohydride (117 mg, 1.86 mmol) were added sequentially. The mixture was stirred for 1 hour. The mixture was diluted with EtOAc, and was washed with saturated sodium bicarbonate solution, water (3x) and brine, and was dried over MgSO₄. Purification by flash column chromatography (EtOAc to EtOAc/EtOH = 9/1) gave compound 47. Compound 47 was dissolved in CH₂Cl₂ (25 mL), and trifluoroacetic acid (100 µL) was added. The mixture was concentrated to give compound 47 as a TFA salt (650 mg): ¹H NMR (CDCl₃) δ 7.74 (2 H, m), 7.41 (2 H, m), 7.25-7.1 (2 H, m), 7.02 (5 H, in), 5.65 (1 H, m), 5.50 (1 H, m), 5.0-4.75 (2 H, m), 4.25-4.05 (4 H, m), 4.0-3.4 (11 H, m), 3.2-2.6 (9 H, m), 2.31 (6 H, m), 1.82-1.51 (3 H, m), 1.45-1.2 (5 H, m), 0.93 (9 H, m).

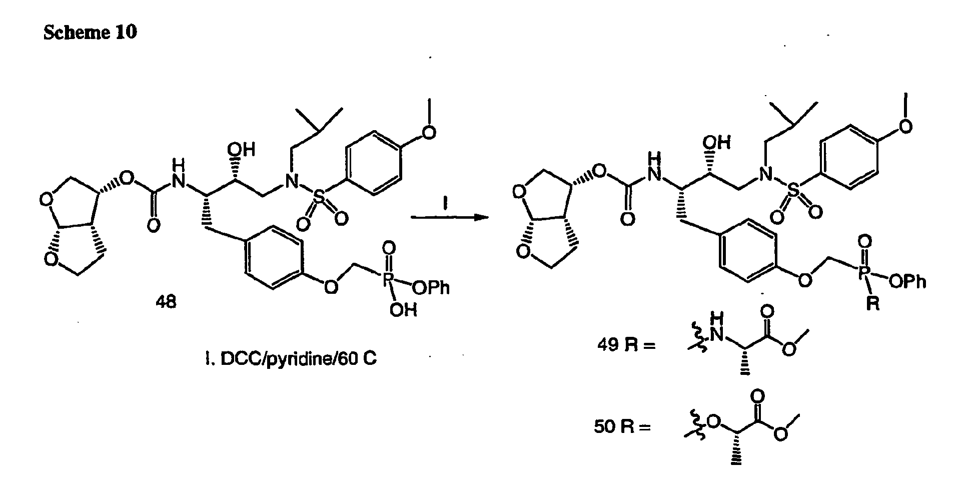

### Example 48

Compound 48 was made by the methods of the previous Examples.

### Example 49

Compound 49: To a solution of compound 48 (100 mg, 0.13 mmol) in pyridine (0.75 mL) was added L-alanine methyl ester hydrochloride (73 mg, 0.52 mmol), followed by DCC (161 mg, 0.78 mmol). The mixture was heated at 60°C for 1 hour. The mixture was diluted with EtOAc, and was washed with 0.2 N HCl, water, 5% sodium bicarbonate, and brine, and was dried over MgSO₄. Purification by flash column chromatography (CH₂Cl₂/iPrOH = 100/5) gave compound 49 (46 mg): ¹H NMR (CDCl₃) δ 7.73 (2H, m), 7.38-7.18 (7 H, m), 7.03 (2 H, m), 6.89 (2H, m), 5.68 (1 H, m), 5.05 (1 H, m), 4.95 (1 H, m), 4.30 (3H, m), 4.0-3.6 (12 H, m), 3.2-2.8 (7 H, m), 1.84-1.60 (3 H, m), 1.38 (3 H, m), 0.93 (6 H, m).

### Example 50

Compound 50: To a solution of compound 48 (100 mg, 0.13 mmol) in pyridine (0.75 mL) was added methyl (s)-lactate (41 mg, 0.39 mmol), followed by DCC (81 mg, 0.39 mmol). The mixture was heated at 60°C for 2 hours, and pyridine was removed under reduced pressure. The mixture was diluted with EtOAc (5 mL), and was filtered. Purification by flash column chromatography (CH₂Cl₂/iPrOH = 100/5) gave compound 50 (83 mg): ¹H NMR (CDCl₃) δ 7.74 (2 H, m), 7.38-7.14 (7 H, m), 7.02 (2 H, m), 6.93 (2 H, m), 5.67 (1 H, m), 5.18 (1 H, m), 5.04 (1 H, m), 4.92 (1 H, m), 4.5 (2 H, m), 4.0-3.68 (12 H, m), 3.2-2.75 (7 H, m), 1.82 (1 H, m), 1.75-1.50 (5 H, m), 0.93 (6 H, m).

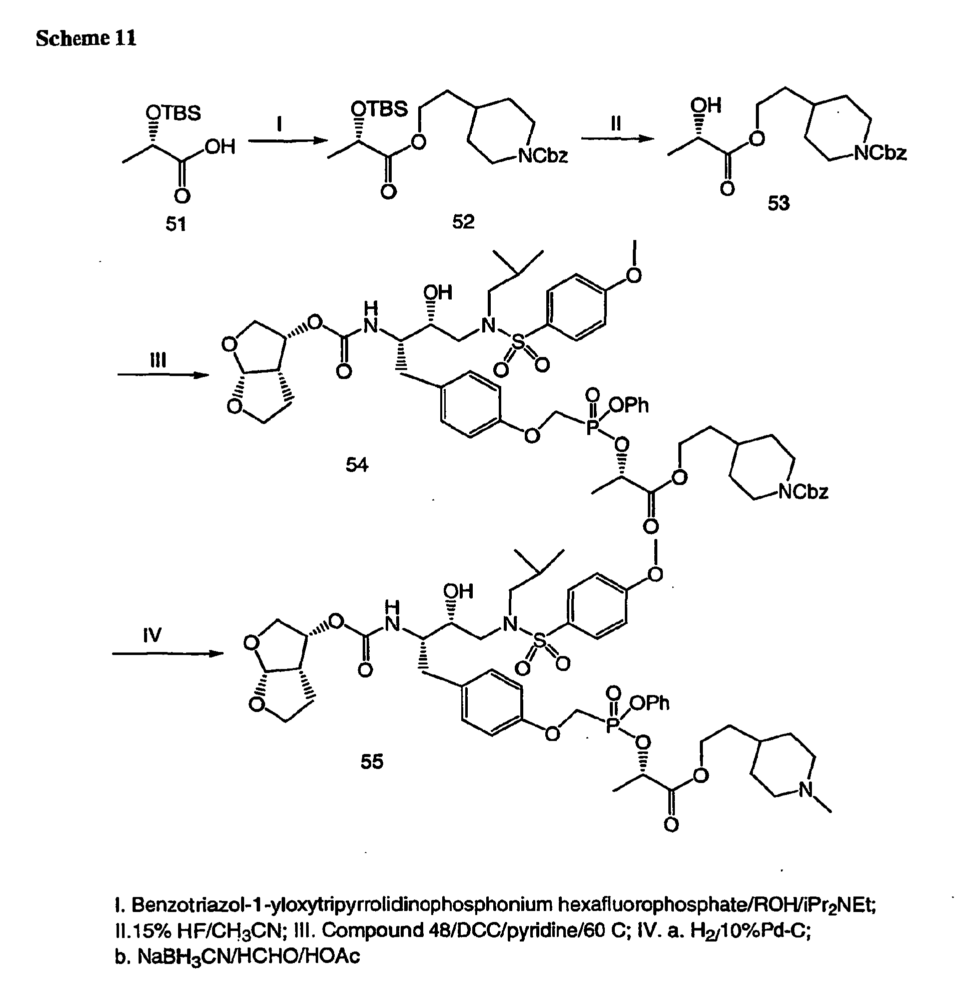

### Example 51

Compound 51: To a solution of benzyl (s)-lactate (4.0 g, 20 mmol) in DMF (40 mL) was added imidazole (2.7 g, 20 mmol), followed by tert-butyldimethylsilyl chloride (3.3 g, 22 mmol). The mixture was stirred for 14 hours, and diluted with EtOAc. The organic phase was washed with 1.0 N HCl solution (2x), water (2x), and brine (1x), and dried over MgSO₄.

Concentration gave the lactate intermediate (6.0 g). To the solution of the above intermediate in EtOAc (200 mL) was added 10% Palladium on carbon (700 mg). The mixture was hydrogenated for 2 hours. The mixture was stirred with celite for 5 minutes, and was filtered through a pad of celite. Concentration gave compound 51 (3.8 g).

### Example 52

Compound 52: To a solution of compound 51 (1.55 g, 7.6 mmol) in CH₂Cl₂ (20 mL) was added 4-benzyloxycarbonylpiperidineethanol (2.00 g, 7.6 mmol), followed by benzotriazol-1-yloxytripyrrolidinophosphonium hexafluorophosphate (4.74 g, 9.1 mmol) and diisopropylethylamine (1.58 mL, 9.1 mmol). The mixture was stirred for 14 hours, and dichloromethane was removed. The mixture was diluted with EtOAc, and was washed with brine, and dried with MgSO₄. Purification by flash column chromatography (hexanes/EtOAc = 10/1) gave compound 52 (1.50 g).

### Example 53

Compound 53: To a solution of compound 52 (1.50 g) in CH₃CN was added 58% HF/CH₃CN (5 mL). The mixture was stirred for 30 minutes, and acetonitrile was removed under reduced pressure. The mixture was diluted with EtOAc, and was washed with water and brine, and was dried over MgSO₄. Purification by flash column chromatography (hexanes/EtOAc = 1/1) gave compound 53 (1.00 g).

### Example 54

Compound 54: To a solution of compound 48 (769 mg, 1.0 mmol) in pyridine (6.0 mL) was added compound 53 (1.0 g, 3.0 mmol), followed by DCC (618 mg, 3.0 mmol). The mixture was heated at 60°C for 2 hours, and pyridine was removed under reduced pressure. The mixture was diluted with EtOAc (5 mL), and was filtered. Purification by flash column chromatography (CH₂Cl₂/iPrOH = 100/4) gave compound 54 (630 mg).

### Example 55

Compound 55: To a solution of compound 54 (630 mg, 0.58 mmol) in EtOAc (30 mL) was added 10% Palladium on carbon (63 mg), followed by acetic acid (80 µL). The mixture was hydrogenated for 2 hours. The mixture was stirred with celite for 5 minutes, and was filtered through a pad of celite. Concentration gave the intermediate. To the solution of the above intermediate in EtOAc (10 mL) was added 37% formaldehyde (88 µL, 1.18 mmol), followed by acetic acid (101 µL, 1.77 mmol). The mixture was cooled to 0°C, and sodium cyanoborohydride (74 mg, 1.18 mmol) was added. The mixture was stirred at 25°C for 80 minutes, and was diluted with EtOAc. The mixture was washed with water and brine, and was dried over MgSO₄. Concentration gave compound 55 as a white solid (530 mg): ¹H NMR (CDCl₃) δ 7.74 (2 H, m), 7.40-7.15 (7 H, m), 7.03 (2 H, m), 6.92 (2 H, m), 5.66 (1H, m), 5.20-5.00 (3 H, m), 4.58 -4.41 (2 H, m), 4.16 (2 H, m), 4.0-3.7 (9 H, m), 3.4-2.6 (14 H, m), 1.90-1.50 (13 H, m), 0.92 (6 H, m).

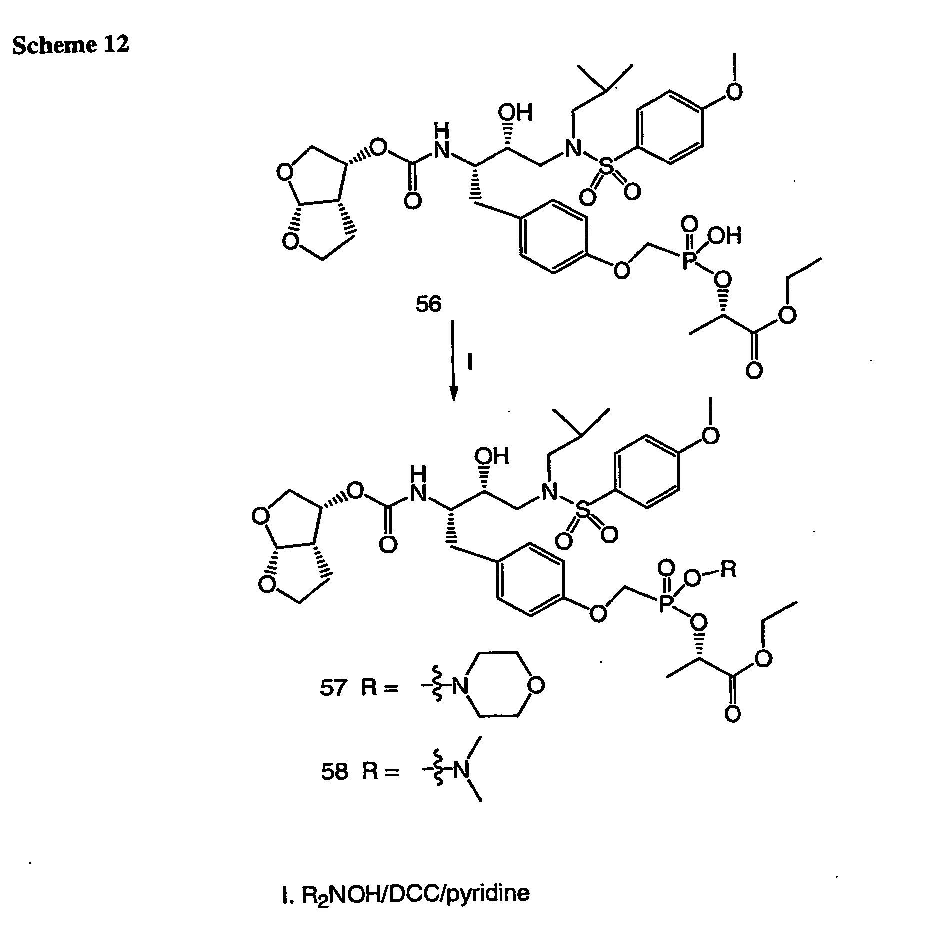

### Example 56

Compound 56 was made by the methods of the previous Examples.

### Example 57

Compound 57: To a solution of compound 56 (100 mg, 0.12 mmol) in pyridine (0.6 mL) was added N-hydroxymorpholine (50 mg, 0.48 mmol), followed by DCC (99 mg, 0.48 mmol). The mixture was stirred for 14 hours, and pyridine was removed under reduced pressure. The mixture was diluted with EtOAc, and was filtered. Purification by flash column chromatography (CH₂Cl₂/iPrOH = 100/5) gave compound 57 (53 mg): ¹H NMR (CDCl₃) δ 7.71 (2 H, d, J = 8.6 Hz), 7.15 (2 H, d, J = 7.6 Hz), 6.99 (2 H, d, J = 8.8 Hz), 6.90 (2 H, m), 5.67 (1 H, m), 5.18 (1 H, m), 5.05 (1 H, m), 4.95 (1 H, m), 4.58-4.38 (2 H, m), 4.21 (2 H, m), 4.02-3.80 (13 H, m), 3.55-3.38 (2 H, m), 3.2-2.78 (9 H, m), 1.9-1.8 (1 H, m), 1.8-0.95 (5 H, m), 1.29 (3 H, m), 0.93 (6 H, m).

### Example 58

Compound 58: To a solution of compound 56 (100 mg, 0.12 mmol) in pyridine (0.6 mL) was added N,N-dimethylhydroxylamine hydrochloride (47 mg, 0.48 mmol), followed by DCC (99 mg, 0.48 mmol). The mixture was stirred for 6 hours, and pyridine was removed under reduced pressure. The mixture was diluted with EtOAc, and was filtered. Purification by flash column chromatography (CH₂Cl₂/iPrOH = 100/5) gave compound 58 (35 mg). ¹H NMR (CDCl₃) δ 7.71 (2 H, d, J = 8.9 Hz), 7.15 (2 H, d, J = 8.2 Hz), 6.99 (2 H, d, J =8.4 Hz), 6.89 (2 H, m), 5.65 (1H, d, J = 5.2 Hz), 5.15 (1H, m), 4.98 (2 H, m), 4.42 (2 H, m), 4.18 (2 H, m), 4.0-3.6 (9 H, m), 3.2-2.7 (13 H, m), 1.92-1.45 (6 H, m), 1.25 (3 H, m), 0.90 (6 H, m).

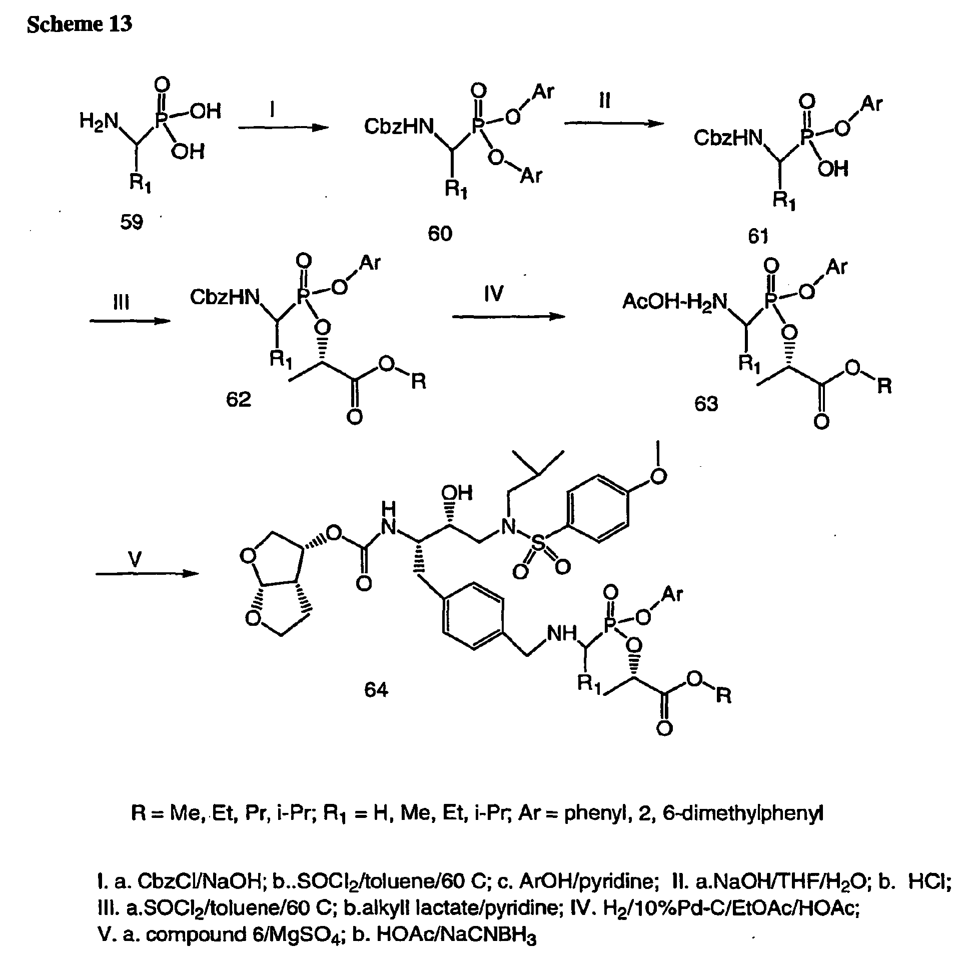

Aminomethylphosphonic acid 59 is protected as benzyl carbamate. The phosphonic acid is treated with thionyl chloride to generate dichloridate, which reacts with phenol or 2,6-dimethylphenol to give compound 60. Compound 60 is hydrolyzed with sodium hydroxide, followed by acidification to afford monoacid 61. Monoacid 61 is treated with thionyl chloride to generate monochloridate, which reacts with different alkyl (s)-lactates to form compound 62. Compound 62 is hydrogenated with 10%Pd-C in the presence of acetic acid to give compound 63. Compound 63 reacts with aldehyde 6 in the presence of MgSO₄ to form imine, which is reduced with sodium cyanoborohydride to generate compound 64.

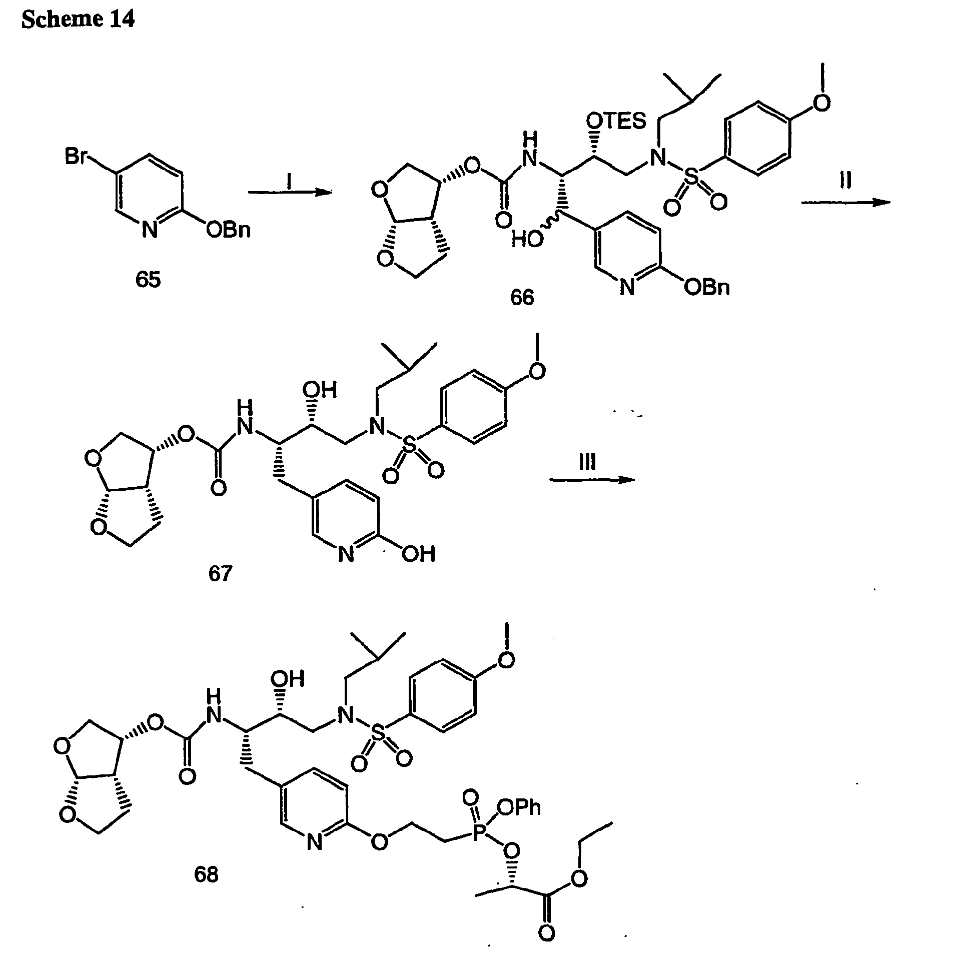

### I.a. n-BuLi; b. compound 15; II. H₂/10%Pd-C/HOAc; IV. PPh₃/DEAD

Compound 65 is prepared from 2-hydroxy-5-bromopyridine by alkylation. J. Med. Chem. 1992, 35, 3525. Compound 65 is treated with n-Butyl lithium to generate aryl lithium, which reacts with aldehyde 15 to form compound 66. J. Med. Chem 1994, 37., 3492. Compound 66 is hydrogenated with 10%Pd-C in the presence of acetic acid to give compound 67. J. Med. Chem. 2000,43,721. Compound 68 is prepared from compound 67 with corresponding alcohol under Mitsunobu reaction conditions. Bioorg. Med. Chem. Lett. 1999, 9,2747.

### Example Section O

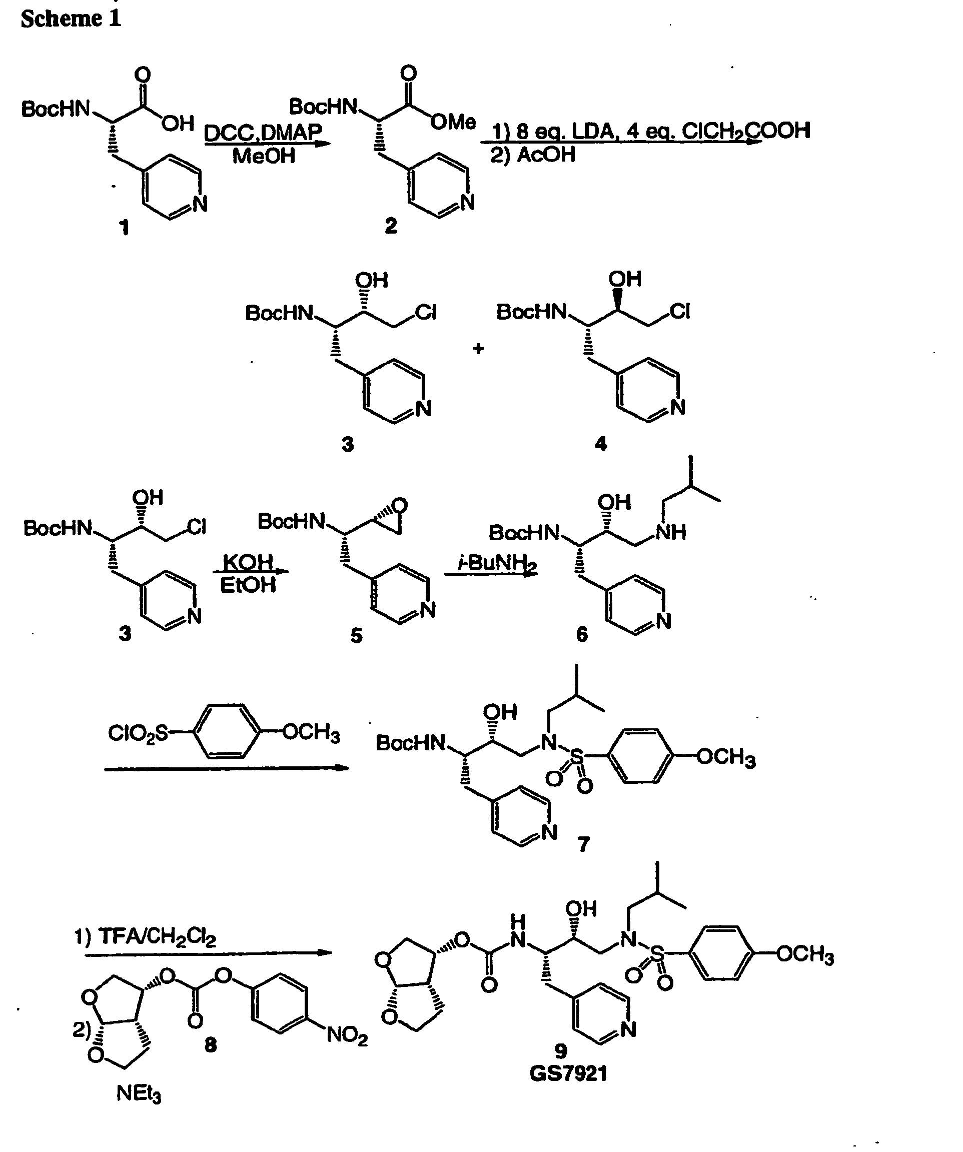

### Example 1

Methyl 2-(*S*)-(dimethylethoxycarbonylamino)-3-(4-pyridyl)propanoate (2): A solution of N-tert-Butoxycarbonyl-4-pyridylalanine (1, 9.854 g, 37 mmol, Peptech), 4-dimethylaminopyridine (4.52 g, 37 mmol, Aldrich), and dicyclohexylcarbodiimide (15.30 g, 74.2 mmol, Aldrich) in methanol (300 mL) was stirred at 0°C for 2 h and at room temperature for 12 h. After the solids were removed by filtration, the filtrate was concentrated under reduced pressure. More dicyclohexylurea was removed by repeated trituration of the concentrated residue in EtOAc followed by filtration. The residue was chromatographed on silica gel to afford the methyl ester 2 (9.088 g, 88%): ¹H NMR (CDCl₃) δ 8.53 (d, 2H, *J* = 5.7 Hz), 7.09 (d, 2H, *J=* 5.7 Hz), 5.04 (br, 1H), 4.64 (br, 1H), 3.74 (s, 3H), 3.16 (dd, 1M, *J =* 13.5 and 5.7 Hz), 3.02 (dd, 1H, *J =* 13.5 and 6.3 Hz), 1.42 (s, 9H); MS (ESI) 281 (M+H).

### Example 2

1-Chloro-3-(*S*)-(dimethylethoxycarbonylamino)-4-(4-pyridyl)-2-(*S*)-butanol (3): A solution of diisopropylamine (37.3 mL, 266 mmol, Aldrich) in THF (135 mL) was stirred at -78°C as a solution of *n*-butyllithium (102 mL of 2.3 M solution and 18 mL of 1.4 M solution 260 mmol, Aldrich) in hexane was added. After 10 min, the cold bath was removed and stirred the solution for 10 min at the ambient temperature. The solution was cooled at -78°C again and stirred as a solution of chloroacetic acid (12.255 g, 130 mmol, Aldrich) in THF (50 mL) was added over 20 min. After the solution was stirred for 15 min, this dianion solution was transferred to a stirred solution of the methyl ester **2** (9.087 g, 32.4 mmol) in THF (100 mL) at 0°C over 15 min. The resulting yellow slurry was stirred at 0°C for 10 min and cooled at - 78°C. A solution of acetic acid (29 mL, 507 mmol, Aldrich) in THF (29 mL) was added quickly to the slurry and the resulting slurry was stirred at -78°C for 30 min, at 0°C for 30 min, and at room temperature for 15 min. The resulting slurry was dissolved in saturated NaHCO₃ solution (750 mL) and EtOAc (500 mL). The separated aqueous layer was extracted with EtOAc (300 mL x 2) and the combined organic fractions were washed with water (750 mL x 2) and saturated NaCl solution (250 mL). The resulting solution was dried (MgSO₄) and evaporated under reduced pressure.
A solution of the residue in THF (170 mL) and water (19 mL) was stirred at 0°C as NaBH₄ (3.375 g, 89.2 mmol, Aldrich) was added. After 30 min, the solution was evaporated under reduced pressure and the residue was dissolved in EtOAc, acidified with aqueous NaHSO₄, and then neutralized by adding saturated aqueous NaHCO₃ solution. The separated aqueous fraction was extracted with EtOAc (100 mL) and the combined organic fractions were washed with water (500 mL) and saturated NaCl solution (100 mL). The solution was dried (MgSO₄) and evaporated under reduced pressure. The residue was chromatographed on silica gel to afford the chlorohydrin **3** and **4** (4.587 g, 47%) as a mixture of two diastereomers (3~4:1). The obtained mixture was recrystallized from EtOAc-hexane twice to obtain pure desired diastereomer **3** (2.444 g, 25%) as yellow crystals: ¹H NMR (CDCl₃) δ 8.53 (d, 2H, *J* = 5.7 Hz), 7.18 (d, 2H, *J=* 5.7 Hz), 4.58 (br, 1H), 3.94 (m, 1H), 3.87 (br, 1H). 3.75-3.54 (m, 2H), 3.05 (dd, 1H, *J* = 13.8 and 3.9 Hz), 2.90 (dd, 1H, *J* = 13.8 and 8.4 Hz), 1.36 (s, 9H); MS (ESI) 301 (M+H).

### Example 3

The epoxide 5: A solution of the chlorohydrin 3 (1.171 g, 3.89 mmol) in ethanol (39 mL) was stirred at room temperature as 0.71 M KOH in ethanol (6.6 mL) was added. After 1.5 h, the mixture was concentrated under reduced pressure and the residue was dissolved in EtOAc (60 mL) and water (60 mL). The separated aqueous fraction was extracted with EtOAc (60 mL) and the combined organic fractions were washed with saturated NaCl solution, dried (MgS0₄), and concentrated under reduced pressure to obtain the epoxide (1.058 g, quantitative): ¹H NMR (CDCl₃) δ 8.52 (d, 2H, *J* = 6.0 Hz), 7.16 (d, 2H, *J* = 6.0 Hz), 4.57 (d, 1H, *J* = 7.8 Hz), 3.76 (br, 1H), 3.02-2.92 (m, 2H), 2.85-2.79 (m, 2H), 2.78-2.73 (m, 1H), 1.37 (s, 9H); MS (ESI) 265 (M+H).

### Example 4

The hydroxy-amine 6: A solution of the epoxide 5 obtained above and *i*-BuNH₂ (3.9 mL, 39.2 mmol, Aldrich) in 58 mL of i-PrOH was stirred at 65°C for 2 h and the solution was concentrated under reduced pressure. The residual *i*-PrOH was removed by dissolving the residue in toluene and concentration of the solution twice: ¹H NMR (CDCl₃) δ 8.51 (d, 2H, *J* = 6.0 Hz), 7.18 (d, 2H, *J* = 6.0 Hz), 4.70 (d, 1H, *J* = 9.6 Hz), 3.86 (br, 1H), 3.46 (q, 1H, *J* = 5.8 Hz), 3.06 (dd, 1H, *J =* 14.1 and 3.9 Hz), 2.79 (dd, 1H, *J=* 14.1 and 9.0 Hz), 2.76-2.63 (m, 3H), 2.43 (m, 2H, *J* = 6.9 Hz), 1.73 (m, 1H, *J =* 6.6 Hz), 1.36 (s, 9H), 0.93 (d, 3H, *J* = 6.6 Hz), 0.92 (d, 3H, *J*= 6.6 Hz); MS (ESI) 338 (M+H).

### Example 5

The sulfoamide 7: A solution of the crude **6** and *p*-methoxybenzene sulfonyl chloride (890 mg, 4.31 mmol, Aldrich) in CH₂Cl₂ (24 mL) was stirred at 0°C for 2 h and at room temperature for 13 h. The solution was washed with saturated NaHCO₃ solution and the aqueous washing was extracted with CH₂Cl₂ (60 mL). After the combined organic fractions were dried (MgSO₄) and concentrated under reduced pressure, the residue was purified by chromatography on silica gel to obtain the sulfoamide **7** (1.484 g, 75%): ¹H NMR (CDCl₃) δ 8.51 (d, 2H, *J* = 5.7 Hz), 7.73 (d, 2H, J = 8.7 Hz), 7.21 (d, 2H, *J* = 5.7 Hz), 7.00 (d, 2H, *J =* 8.7 Hz), 4.68 (d, 1H, *J =* 8.1 Hz), 4.08 (br, 1H), 3.88 (s, 3H), 3.83 (br, 2H), 3.09 (d, 2H, *J =* 5.1 Hz), 3.06-2.80 (m, 4H), 1.85 (m, 1H, *J =* 7.0 Hz), 1.34 (s, 9H), 0.92 (d, 3H, *J =* 6.3 Hz), 0.89 (d, 3H, *J* = 6.6 Hz); MS (ESI) 508 (M+H).

### Example 6

The bisfurancarbamate 9: A solution of the sulfoamide **7** (1.484 g, 2.92 mmol) and trifluoroacetic acid (6.8 mL, 88.3 mmol, Aldrich) in CH₂Cl₂ (18 mL) was stirred at room temperature for 2 h. After the solution was evaporated under reduced pressure, the residue was dissolved in acetonitrile (10 mL) and toluene (10 mL), and evaporated to dryness twice to result crude amine as TFA salt. A solution of the crude amine, dimethylaminopyridine (72 mg, 0.59 mmol, Aldrich), diisopropylethylamine (2.55 mL, 14.6 mmol, Aldrich) in acetonitrile was stirred at 0°C as the bisfurancarbonate **8** (907 mg, 3.07 mmol, obtained from Azar) was added in portion. The solution was stirred at 0°C for 1 h and at room temperature for 19 h, and concentrated under reduced pressure. The residue was dissolved in EtOAc (60 mL) and washed with saturated NaHCO₃ solution (60 mL). After the aqueous washing was extracted with EtOAc (60 mL), the combined organic fractions were washed with saturated NaHCO₃ (60 mL) and saturated NaCl solution (60 mL), dried (MgSO₄), and concentrated under reduced pressure. The residue was purified by chromatography on silica gel to obtain the carbamate 9 (1.452 g, 88%): ¹H NMR (CDCl₃) δ 8.50 (d, 2H, *J*= 5.7 Hz), 7.72 (d, 2H, *J* = 8.7 Hz), 7.19 (d, 2H, *J* = 5.7 Hz), 7.01 (d, 2H, *J* = 8.7 Hz), 5.65 (d, 1H, *J =* 5.1 Hz), 5.12 (d, 1H, *J =* 9.3 Hz), 5.02 (q, 1H, *J =* 6.7 Hz), 4.01-3.77 (m, 4H), 3.88 (s, 3H), 3.76-3.63 (m, 2H), 3.18-2.76 (m, 7H), 1.95-1.77 (m, 1H), 1.77-1.56 (m, 2H), 1.56-1.41 (m, 1H), 0.94 (d, 3H, *J* = 6.6 Hz), 0.90 (d, 3H, *J* = 6.9 Hz); MS (ESI) 564 (M+H).

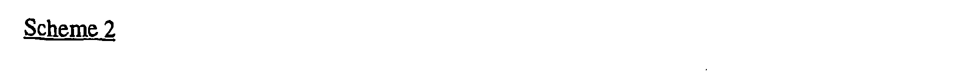

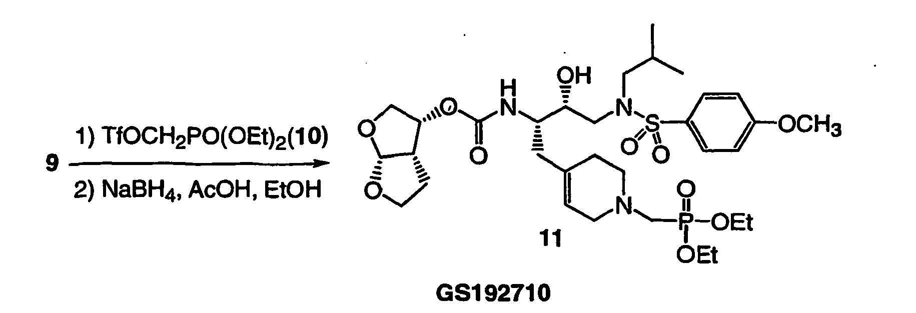

### Example 7

The tetrahydropyridine-diethyl phosphonate 11: A solution of the pyridine **9** (10.4 mg, 0.018 mmol) and the triflate **10** (8.1 mg, 0.027 mmol, in acetone-d₆ (0.75 mL) was stored at room temperature for 9 h and the solution was concentrated under reduced pressure: ³¹P NMR (acetone-d₃) δ 14.7; MS (ESI) 714 (M⁺). The concentrated crude pyridinium salt was dissolved in ethanol (2 mL) and stirred at room temperature as NaBH₄ (~10 mg, Aldrich) was added occasionally over 4 h. To the mixture was added a solution of acetic acid (0.6 mL, Aldrich) in ethanol (3 mL) until the pH of the mixture became 3-4. More NaBH₄ and acetic acid were added until the reaction was completed. The mixture was carefully concentrated under reduced pressure and the residue was dissolved in saturated NaHCO₃ solution (10 mL). The product was extracted using EtOAc (10 mL x 3) and washed with saturated NaCl solution, dried (MgSO₄), and concentrated under reduced pressure. The residue was purified by chromatography on silica gel to obtain the product **11** (8.5 mg, 64%): ¹H NMR (CDCl₃) δ 7.73 (d, 2H, *J* = 8.7 Hz), 7.00 (d, 2H, *J =* 8.7 Hz), 5.71 (d, 1H, *J =* 5.1 Hz), 5.41 (br, 1H), 5.15-5.08 (m, 1H), 5.00 (br, 1H), 4.14 (dq, 4H, J = 7.2 Hz), 4.06-3.94 (m, 2H), 3.88 (s, 3H), 3.92-3.80 (m, 2H), 3.75 (dd, 1H, *J* = 9.6 and 6.6 Hz), 3.79-3.61 (m, 1H), 3.24-2.94 (m, 6H), 2.85 (d, 2H, *J* = 11.7 Hz), 2.88-2.76 (m, 2H), 2.75-2.63 (m, 1H), 2.38-2.29 (m, 1H), 2.24-2.2.12 (m, 2H), 2.12-1.78 (m, 4H), 1.30 (t, 6H, *J* = 7.1 Hz), 0.94 (d, 3H, *J =* 6.6 Hz), 0.91 (d, 3H, *J* = 6.3 Hz); ³¹P NMR (CDCl₃) δ 24.6; MS (ESI) 740 (M+Na).

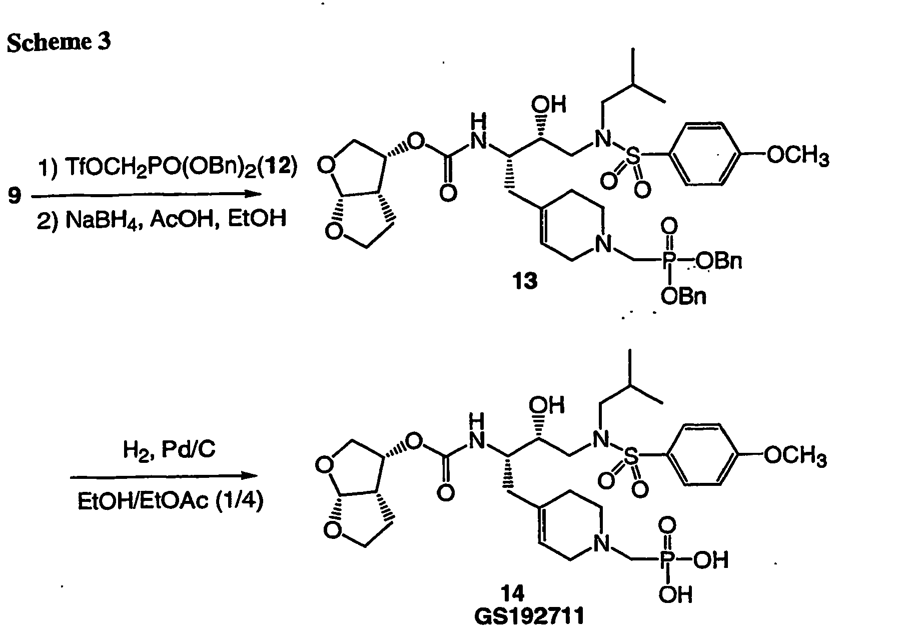

### Example 8

The tetrahydropyridine-dibenzyl phosphonate 13: The compound **13** was obtained by the same procedure as described for compound **11** using the pyridine **9** (10.0 mg, 0.018 mmol) and the triflate **12** (9.4 mg, 0.022 mmol). The product **13** was purified by preparative TLC to afford the dibenzyl phosphonate **13** (8.8 mg, 59%): ¹H NMR (CDCl₃) δ 7.73 (d, 2H, *J* = 8.7 Hz), 7.35 (s, 10H), 7.00 (d, 2H, *J* = 8.7 Hz), 5.65 (d, 1H2H, *J* = 5.1 Hz), 5.39 (br, 1H), 5.15-4.92 (m, 6H), 4.03-3.77 (m, 6H), 3.77-3.62 (m, 2H), 3.56 (br, 1H), 3.24-2.62 (m, 9H), 2.32 (d, 1H, *J* = 13.5 Hz), 2.24-1.75 (m, 6H), 0.94 (d, 3H, *J* = 6.6 Hz), 0.89 (d, 3H, *J* = 6.3 Hz); ³¹P NMR (CDCl₃) δ 25.5; MS (ESI) 842 (M+H).

### Example 9

The phosphonic acid 14: A mixture of the dibenzyl phosphonate **13** (8.8 mg, 0.011 mmol) and 10% Pd/C in EtOAc (2 mL) and EtOH (0.5 mL) was stirred under H₂ atmosphere for 10 h at room temperature. After the mixture was filtered through celite, the filtrate was concentrated to dryness to afford the product **14** (6.7 mg, quantitative): ¹H NMR (CD₃OD) δ 7.76 (d, 2H, *J*= 9.0 Hz), 7.10 (d, 2H, *J=* 9.0 Hz), 5.68 (d, 1H, *J* = 5.1 Hz), 5.49 (br, 1H), 5.11 (m, 1H), 3.90 (s, 3H), 4.04-3.38 (m, 10H), 3.22 (d, 2H, *J* = 12.9 Hz), 3.18-3.00 (m, 2H), 2.89-2.75 (m, 2H), 2.68-2.30 (m, 3H), 2.21-1.80 (m, 4H), 0.92 (d, 3H, *J*= 6.3 Hz), 0.85 (d, 3H, *J* = 6.3 Hz); ³¹P NMR (CD₃OD) δ 6.29; MS (ESI) 662 (M+H).

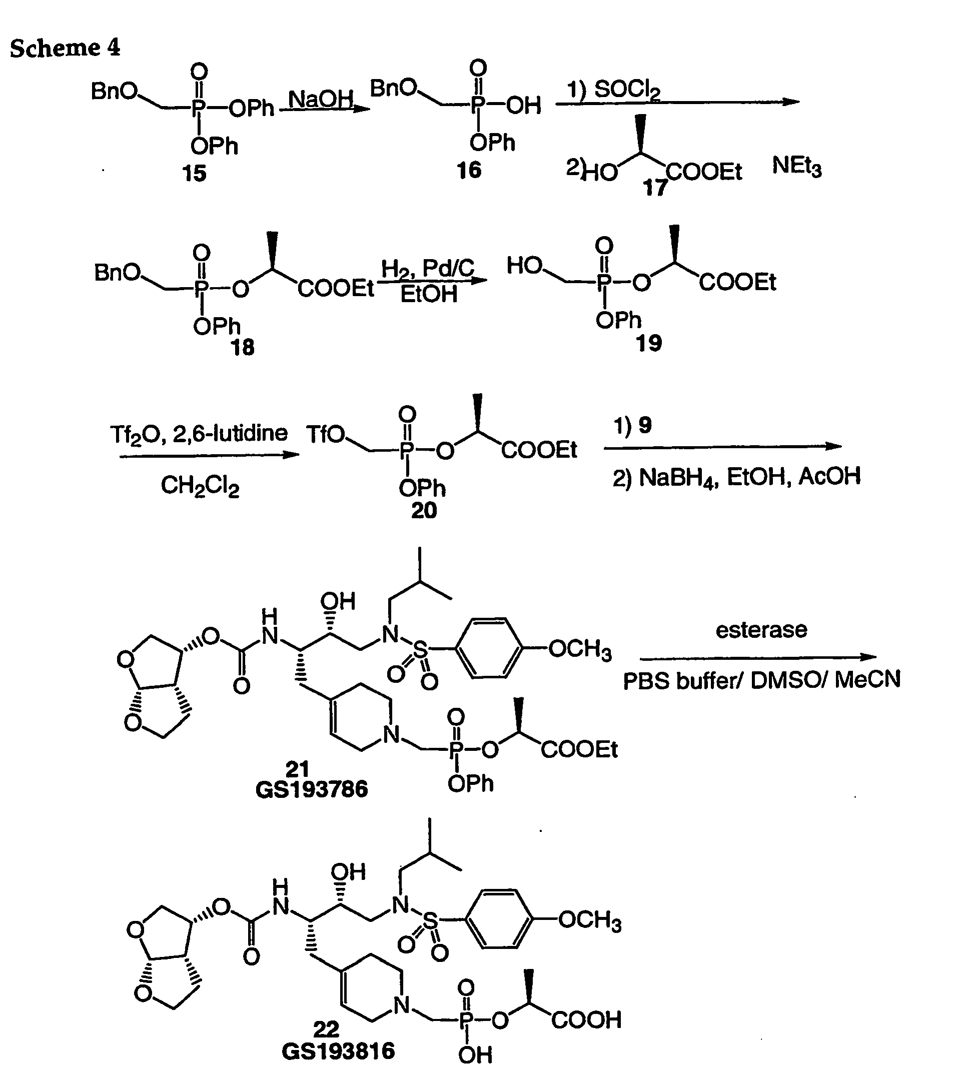

### Example 10

Diphenyl benzyloxymethylphosphonate 15: To a solution of diphenylphosphite (46.8 g, 200 mmol, Aldrich) in acetonitrile (400 mL) (at ambient temperature) was added potassium carbonate (55.2 g, 400 mmol) followed by the slow addition of benzyl chloromethyl ether (42 mL, 300 mmol, about 60%, Fluka). The mixture was stirred overnight, and was concentrated under reduced pressure. The residue was dissolved in EtOAc, washed with water, saturated NaCl, dried (Na₂SO₄), filtered and evaporated. The crude product was chromatographed on silica gel to afford the benzylether (6.8 g, 9.6%) as a colorless liquid.

### Example 11

Monoacid 16: To a solution of diphenyl benzyloxymethylphosphonate 15 (6.8 g, 19.1.mmol) in THF (100 mL) at room temperature was added 1N NaOH in water (21 mL, 21 mmol). The solution was stirred 3 h. The THF was evaporated under reduced pressure and water (100 mL) was added. The aqueous solution was cooled to 0°C, neutralized to pH 7 with 3N HCl and washed with EtOAc. The aqueous solution was again cooled to 0°C, acidified with 3N HCl to pH 1, saturated with sodium chloride, and extracted with EtOAc. The organic layer was washed with brine and dried (Na₂SO₄), filtered and evaporated, then co-evaporated with toluene to yield the monoacid (4.0 g, 75%) as a colorless liquid. ¹H NMR (CDCl₃) δ 7.28-7.09 (m, 10H), 4.61 (s, 2H), 3.81 (d, 2H);. ³¹P NMR (CDCl₃) δ 20.8.

### Example 12

Ethyl lactate phosphonate 18: To a solution of monoacid 16 (2.18 g,7.86 mmol) in anhydrous acetonitrile (50 mL) under a nitrogen atmosphere was slowly added thionyl chloride (5.7 mL, 78mmol). The solution was stirred in a 70°C oil bath for three hours, cooled to room temperature and concentrated. The residue was dissolved in anhydrous dichloromethane (50mL), and this solution cooled to 0°C and stirred under a nitrogen atmosphere. To the stirring solution was added ethyl (S)-(-)-lactate (2.66 mL, 23.5 mmol) and triethylamine (4.28 mL, 31.4 mmol). The solution was warmed to room temperature and allowed to stir for one hour. The solution was diluted with ethyl acetate, washed with water, brine, citric acid and brine again, dried (MgSO₄), filtered through Celite, concentrated under reduced pressure and chromatographed on silica gel using 30% ethylacetate in hexane. The two diastereomers were pooled together. ¹H NMR (CDCl₃) δ 7.40-7.16 (m, 20H), 5.18-5.13 (m, 2H), 4.73 (s, 2H), 4.66 (d, 2H), 4.28-4.11 (m, 5H), 4.05 (d, 2H), 3.95 (d, 2H), 1.62 (d, 3H), 1.46 (d, 3H), 1.30-1.18 (m, 6H); ³¹P NMR (CDCl₃) δ 19.6, 17.7.

### Example 13

Ethyl lactate phosphonate with free alcohol 19: Ethyl lactate phosphonate 18 was dissolved in EtOH (50mL) and under a nitrogen atmosphere 10% Pd-C (approximately 20 wt %) was added. The nitrogen atmosphere was replaced with hydrogen (latm) and the suspension stirred for two hours. 10% Pd-C was again added (20 wt %) and the suspension stirred five hours longer. Celite was added, the reaction mixture was filtered through Celite and the filtrate was concentrated to afford 1.61 g (71% from monoacid 16) of the alcohol as a colorless liquid. ¹H NMR (CDCl₃) δ 7.40-7.16 (m, 10H), 5.16-5.03 (m, 2H), 4.36-4.00 (m, 8H), 1.62 (d, 3H), 1.46 (d, 3H), 1.30-1.22 (m, 6H); ³¹P NMR (CDCl₃) δ 22.3,20.0.

### Example 14

Triflate 20: To a solution of ethyl lactate phosphonate with free alcohol 19 (800 mg, 2.79 mmol) in anhydrous dichloromethane (45 mL) chilled to -40°C under a nitrogen atmosphere was added triflic anhydride (0.516 mL, 3.07 mmol) and 2-6 lutidine (0.390 mL, 3.34 mmol). The solution was stirred for 3 hr, then warmed to -20°C and stirred one hour longer. 0.1 equivalents of triflic anhydride and 2-6 lutidine were then added and stirring was resumed for 90 minutes more. The reaction mixture was diluted with ice-cold dichloromethane, washed with ice-cold water, washed with ice-cold brine and the organic layer was dried (MgSO₄) and filtered. The filtrate was concentrated and chromatographed on silica gel using 30% EtOAc in hexane as eluent to afford 602 mg (51%) of the triflate diastereomers as a slightly pink, transparent liquid. ¹H NMR (CDCl₃) δ 7.45-7.31 (m, 4H), 7.31-7.19 (m, 6H), 5.15-4.75 (m, 6H), 4.32-4.10 (4H), 1.62 (d, 3H), 1.50 (d, 3H), 1.30-1.22 (m, 6H); ³¹P NMR (CDCl₃) 810.3, 8.3.

### Example 15

The tetrahydropyridine-prodrug 21: A solution of the pyridine **9** (11.1 mg, 0.020 mmol) and the triflate **20** (11.4 mg, 0.027 mmol) in acetone-d₆ (0.67 mL, Aldrich) was stored at room temperature for 7 h and the solution was concentrated under reduced pressure: ³¹P NMR (acetone-d₆) δ 11.7, 10.9; MS (ESI) 838 (M+H). The concentrated crude pyridinium salt was dissolved in ethanol (1 mL) and added 2-3 drops of a solution of acetic acid (0.6 mL, Aldrich) in ethanol (3 mL). The solution was stirred at 0°C as NaBH₄ (7-8 mg, Aldrich) was added. More acetic acid solution was added to adjust pH 3-4 of the reaction mixture. Additions of NaBH₄ and the acetic acid solution were repeated until the reaction was completed. The mixture was carefully concentrated under reduced pressure and the residue was purified by chromatography on C18 reverse phase column material followed by preparative TLC using C18 reverse phase plate to obtain the prodrug **21** (13.6 mg, 70%) as a 2:3 mixture of two diastereomers: ¹H NMR (CD₃CN) δ 7.78 (d, 2H, *J*= 9.0 Hz), 7.48-7.42 (m, 2H), 7.35-7.27 (m, 3H), 7.10 (d, 2H, *J* = 9.0 Hz), 5.86 (m, 1H), 5.60 (m, 1H), 5.48 (br, 1H), 5.14-5.03 (m, 2H), 4.29-4.13 (m, 2H), 3.89 (s, 3H), 3.97-3.32 (m, 12H), 3.29 (br, 0.4H), 3.24 (br, 0.6H), 3.02-2.82 (m, 4H), 2.64-2.26 (m, 3H), 2.26-2.08 (m, 1H), 1.94-1.76 (m, 3H), 1.57 (d, 1.8H, *J* = 6.9 Hz), 1.46 (d, 1.2H, *J* = 6.9 Hz), 1.28 (d, 1.2H, *J* = 6.9 Hz), 1.21 (d, 1.8H, *J* = 7.2 Hz), 0.92-0.88 (m, 6H); ³¹P NMR (CD₃CN) δ 14.4 (0.4P), 13.7 (0.6P); MS (ESI) 838 (M+H).

### Example 16

Metabolite 22: To a solution of the prodrug **21** (10.3 mg, 0.011 mmol) in DMSO (0.1 mL) and acetonitrile (0.2 mL) was added 0.1 MPBS buffer (3 mL) mixed thoroughly to result a suspension. To the suspension was added porcine liver esterase suspension (0.05 mL, EC3.1.1.1, Sigma). After the suspension was stored in 37°C for 1.5 h, the mixture was centrifuged and the supernatant was taken. The product was purified by HPLC and the collected fraction was lyophilized to result the product **22** as trifluoroacetic acid salt (7.9 mg, 86%): ¹H NMR (D₂O) δ 7.70 (d, 1H), 7.05 (d, 2H), 5.66 (d, 1H), 5.40 (br, 1H), 5.02 (br, 1H), 4.70 (br, 1H), 3.99-3.89 (m, 2H), 3.81 (s, 3H), 3.83-3.50 (m, 8H), 3.34-2.80 (m, 7H), 2.50-2.18 (m, 3H), 2.03 (m, 1H), 1.92-1.70 (m, 3H), 1.39 (d, 3H), 0.94 (d, 3H), 0.93 (d, 3H); ³¹P NMR (D₂O) δ 9.0, 8.8; MS (ESI) 734 (M+H).

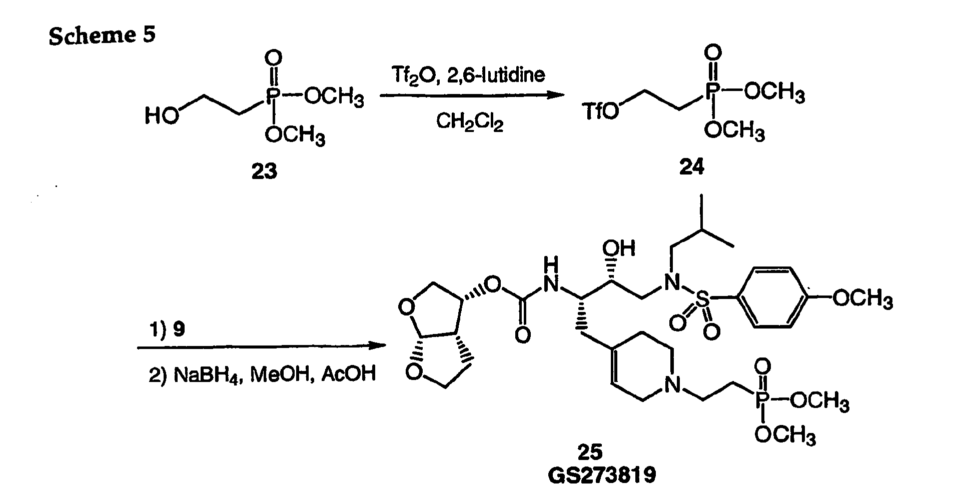

### Example 17

Triflate 24: Triflate **24** was prepared analogously to triflate **20,** except that dimethylhydroxyethylphosphonate **23** (Aldrich) was substituted for ethyl lactate phosphonate with free alcohol **19.**

### Example 18

Tetrahydropyridine 25: Tetrahydropyridine **25** was prepared analogously to tetrahydropyridine **30,** except that triflate **24** was substituted for triflate **29.** ¹H NMR (CDCl₃) δ 7.71 (d, 2H), 7.01 (d, 2H), 5.71 (d, 2H), 5.43 (bs, 1H), 5.07-4.87 (m, 1H), 4.16-3.46 (m, 13H), 3.34-3.18 (m, 3H), 3.16-2.80 (m, 5H), 2.52-1.80 (m, 12H), 1.28-1.04 (m, 3H+H₂O peak), 0.98-0.68 (m, 6H).

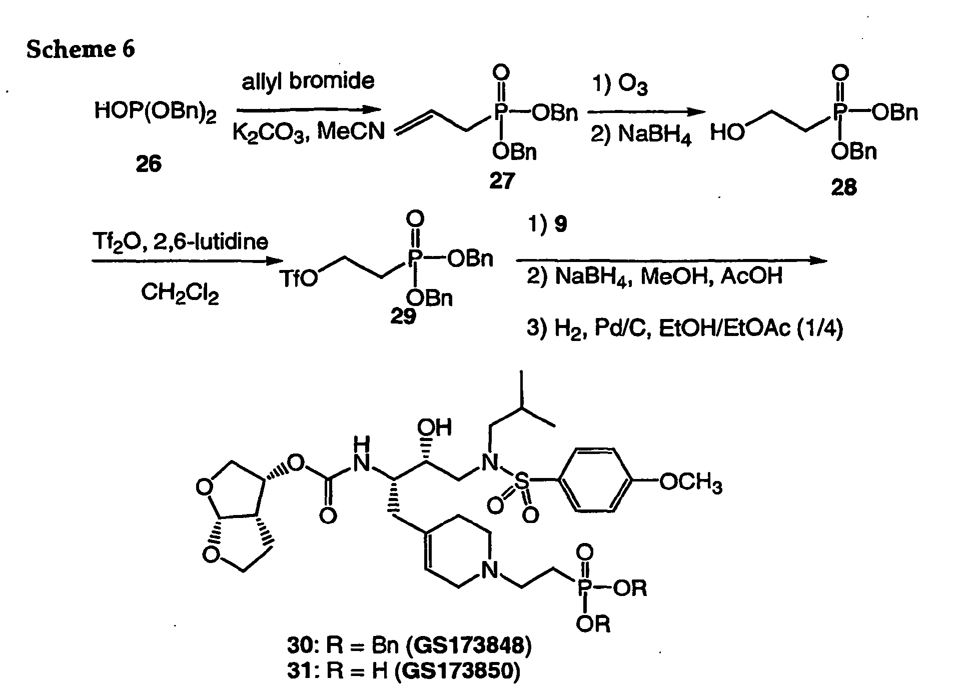

### Example 19

Dibenzyl phosphonate with double bond 27: To a stirring solution of allyl bromide (4.15 g, 34 mmol, Aldrich) and dibenzylphosphite (6 g, 23 mmol, Aldrich) in acetonitrile (25 mL) was added potassium carbonate (6.3 g, 46 mmol, powder 325 mesh Aldrich) to create a suspension, which was heated to 65°C and stirred for 72 hours. The suspension was cooled to room temperature, diluted with ethyl acetate, filtered, and the filtrate was washed with water, then brine, dried (MgSO₄), concentrated and used directly in the next step.

### Example 20

Dibenzylhydroxyethylphosphonate 28: Dibenzyl phosphonate with double bond 27 was dissolved in methanol (50mL), chilled to -78°C, stirred, and subjected to ozone by bubbling ozone into the solution for three hours until the solution turned pale blue. The ozone flow was stopped and oxygen bubbling was done for 15 minutes until the solution became colorless. Sodium borohydride (5 g, excess) was added slowly portionwise. After the evolution of gas subsided the solution was allowed to warm to room temperature, concentrated, diluted with ethyl acetate, made acidic with acetic acid and water and partitioned. The ethyl acetate layer was washed with water, then brine and dried (MgSO₄), filtered, concentrated and chromatographed on silica gel eluting with a gradient of eluent from 50% ethyl acetate in hexane to 100% ethyl acetate, affording 2.76 g of the desired product. ¹H NMR (CDCl₃) δ 7.36 (m, 10H), 5.16-4.95 (m, 4H), 3.94-3.80 (dt, 2m, 2.13-2.01 (dt, 2H); ³¹P NMR (CDCl₃) δ 31.6.

### Example 21

Dibenzyl phosphonate 30: A solution of the alcohol **28** (53.3 mg, 0.174 mmol) and 2,6-lutidine (0.025 mL, 0.215 mmol, Aldrich) in CH₂Cl₂ (1 mL) was stirred at -45°C as trifluoromethanesulfonic anhydride (0.029 mL, 0.172 mmol, Aldrich) was added. The solution was stirred for 1 h at -45°C and evaporated under reduced pressure to obtain the crude triflate **29.**
A solution of the crude triflate **29,** 2,6-lutidine (0.025 mL, 0.215 mmol, Aldrich), and the pyridine **9** in acetone-d₆ (1.5 mL, Aldrich) was stored at room temperature for 2 h. The solution was concentrated under reduced pressure to obtain crude pyridinium product: ³¹P NMR (acetone-d₆) δ 25.8; MS (ESI) 852 (M⁺).
To a solution of the crude pyridinium salt in ethanol (2 mL) was added 7-8 drops of a solution of acetic acid (0.4 mL, Aldrich) in ethanol (2 mL). The solution was stirred at 0°C as NaBH₄ (7-8 mg) was added. The solution was maintained to be pH 3-4 by adding the acetic acid solution. More NaBH₄ and the acetic acid were added until the reduction was completed. After 4 h, the mixture was concentrated and the remaining residue was dissolved in saturated NaHCO₃ (10 mL). The product was extracted with EtOAc (10 mL x 3), dried (MgSO₄), and concentrated under reduced pressure. The residue was purified by repeated chromatography on silica gel followed by HPLC purification. Lyophilization of the collected fraction resulted the product 30 (13.5 mg, 26%) as trifluoroacetic acid salt: ¹H NMR (CDCl₃) δ 7.72 (d, 2H, *J* = 8.7 Hz), 7.36 (br, 10H), 7.00 (d, 2H, *J* = 8.7 Hz), 5.69 (d, 1H, *J* = 5.1 Hz), 5.41 (br, 1H), 5.13-4.93 (m, 6H), 4.05-2.5 (m, 19H), 3.88 (s, 3H), 2.5-1.9 (m, 5H), 1.90-1.74 (m, 2H), 0.88 (d, 6H, *J* = 6.1 Hz); ³¹P NMR (CDCl₃) δ 25.8; MS (ESI) 856 (M+H).

### Example 22

Phosphonic acid 31: A mixture of the dibenzyl phosphonate **30** (9.0 mg, 0.009 mmol) and 10% Pd/C (5.2 mg, Aldrich) in EtOAc (2 mL) and ethanol (0.5 mL) was stirred under H₂ atmosphere for 3 h at room temperature. After the mixture was filtered through celite, a drop of trifluoroacetic acid (Aldrich) was added to the filtrate and the filtrate was concentrated to dryness to afford the product 31 (6.3 mg, 86%): ¹H NMR (CD₃O) δ 7.76 (d, 2H, *J* = 9.0 Hz), 7.11 (d, 2H, *J=* 9.0 Hz), 5.69 (d, 1H, *J=* 5.1 Hz), 5.54 (br, 1H), 5.09 (br, 1H), 4.05-3.84 (m, 4H), 3.89 (s, 3H), 3.84-3.38 (m, 9H), 3.07 (dd, 2H, *J* = 13.5 and 8.4 Hz), 2.9-2.31 (m, 5H), 2.31-1.83 (m, 6H), 0.92 (d, 3H, *J* = 6.3 Hz), 0.85 (d, 3H, *J* = 6.9 Hz); ³¹P NMR (CD₃OD) δ 21.6; MS (ESI) 676 (M+H).

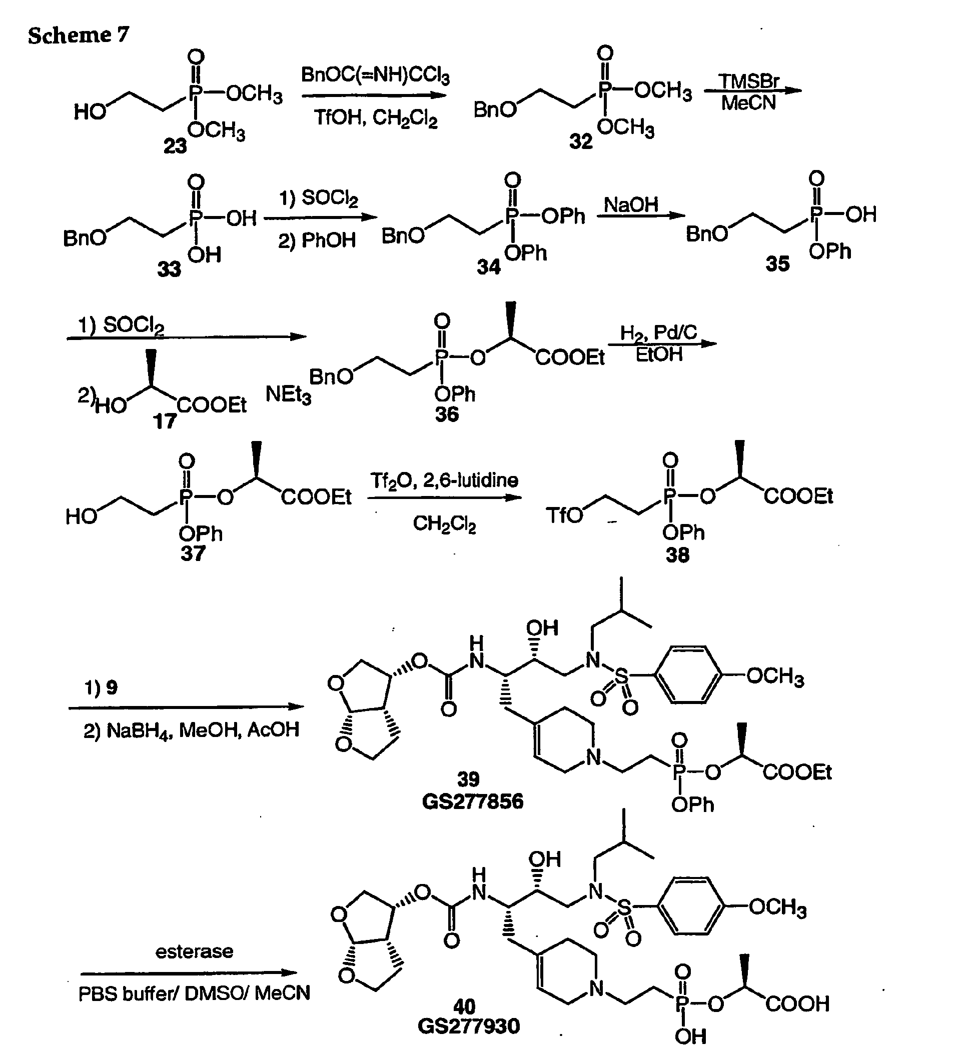

### Example 23

Benzylether 32: A solution of dimethyl hydroxyethylphosphonate (5.0 g, 32.5 mmol, Across) and benzyl 2,2,2-trichloroacetimidate (97.24 mL, 39.0 mmol, Aldrich) in CH₂Cl₂ (100 mL) at 0°C under a nitrogen atmosphere was treated with trifluoromethanesulfonic acid (0.40 mL). Stirring was performed for three hours at 0°C and the reaction was then allowed to warm to room temperature while stirring continued. The reaction continued for 15 hours, and the reaction mixture was then diluted with dichloromethane, washed with saturated sodium bicarbonate, washed with brine, dried (MgSO₄) concentrated under reduced pressure and chromatographed on silica gel eluting with a gradient of eluent from 60% EtOAc in hexane to 100% EtOAc to afford 4.5 g, (57%) of the benzyl ether as a colorless liquid. ³¹P NMR (CDCl₃) δ 31.5.

### Example 24

Diacid 33: A solution of benzylether 32 (4.5 g, 18.4 mmol) was dissolved in anhydrous acetonitrile (100mL), chilled to 0°C under a nitrogen atmosphere and treated with TMS bromide (9.73 mL, 74mmol). The reaction mixture was warmed to room temperature and after 15 hours of stirring was concentrated repeatedly with MeOH/water to afford the diacid, which was used directly in the next step. ³¹P NMR (CDCl₃) δ 31.9.

### Example 25

Diphenylphosphonate 34 : Diacid 33 (6.0 g, 27 mmol) was dissolved in toluene and concentrated under reduced pressure three times, dissolved in anhydrous acetonitrile, stirred under a nitrogen atmosphere, and treated with thionyl chloride (20 mL, 270 mmol) by slow addition. The solution was heated to 70°C for two hours, then cooled to room temperature, concentrated and dissolved in anhydrous dichloromethane, chilled to -78°C and treated with phenol (15 g, 162 mmol) and triethylamine (37 mL, 270 mmol). The reaction mixture was warmed to room temperature and stirred for 15 hours, and was then diluted with ice cold dichloromethane, washed with ice cold 1 N. NaOH, washed with ice cold water, dried (MgSO₄), and concentrated under reduced pressure. The resulting residue was used directly in the next step. ¹H NMR (CDCl₃) δ 7.40-7.16 (d, 15H), 4.55 (s, 2H), 3.98-3.84 (m, 2H), 2.55-2.41 (m, 2H); ³¹P NMR (CDCl₃) δ 22.1.

### Example 26

Mono acid 35: Monoacid 35 was prepared using conditions analogous to those used to prepare monoacid 16, except that diphenylphosphonate 34 was substituted for benzylether 15. ¹H NMR (CDCl₃) δ 7.38-7.16 (d, 10H), 4.55 (s, 2H), 3.82-3.60 (m, 3H), 2.33-2.21 (m, 2H); ³¹P NMR (CDCl₃) δ 29.0.

### Example 27

Ethyl lactate phosphonate 36: Ethyl lactate phosphonate 36 was prepared analogously to ethyl lactate phosphonate 18 except monoacid 35 was substituted for monoacid 16. ³¹P NMR (CDCl₃) δ 27.0, 25.6.

### Example 28

Ethyl lactate phosphonate with free alcohol 37: Ethyl lactate phosphonate with free alcohol 37 was prepared analogously to ethyl lactate phosphonate with free alcohol 19 except that ethyl lactate phosphonate 36 was substituted for ethyl lactate phosphonate 18. ³¹P NMR (CDCl₃) δ 28.9, 26.8.

### Example 29

Triflate 38: A solution of the alcohol 37 (663 mg, 2.19 mmol) and 2,6-lutidine (0.385 mL, 3.31 mmol, Aldrich) in CH₂Cl₂ (5 mL) was stirred at -45°C as trifluoromethanesulfonic anhydride (0.48 mL, 2.85 mmol, Aldrich) was added. The solution was stirred for 1.5 h at -45°C, diluted with ice-cold water (50 mL), and extracted with EtOAc (30 mL x 2). The combined extracts were washed with ice cold water (50 mL), dried (MgSO₄), and concentrated under reduced pressure to obtain a crude mixture of two diastereomers (910 mg, 96%, 1:3 ratio): ¹H NMR (acetone-d₆) δ 7.48-7.37 (m, 2H), 7.37-7.18 (m, 3H), 5.2-4.95 (m, 3H), 4.3-4.02 (m, 2H), 3.38-3.0 (m, 1H), 3.0-2.7 (m, 2H), 2.1-1.9 (m. 1H), 1.52 (d, 1H), 1.4 (d, 2H), 1.4-1.1)m, 3H); ³¹P NMR (acetone-d₆) δ 21.8 (0.75P), 20.5 (0.25P).

### Example 30

The prodrug 39: A solution of the crude triflate 38 (499 mg, 1.15 mmol) and the pyridine 9 (494 mg, 0.877 mmol) in acetone (5 mL) was stirred at room temperature for 16.5 h. The solution was concentrated under reduced pressure to obtain the crude pyridinium salt.
To a solution of the crude pyridinium salt in ethanol (10 mL) was added 5 drops of a solution of acetic acid (1 mL) in ethanol (5 mL). The solution was stirred at 0°C as NaBH₄ (~10 mg, Aldrich) was added. The solution was maintained to be pH 3-4 by adding the acetic acid solution. More NaBH₄ and the acetic acid were added until the reduction was completed. After 5.5 h, the mixture was concentrated under reduced pressure and the remaining residue was dissolved in ice-cold saturated NaHCO₃ (50 mL). The product was extracted with ice-cold EtOAc (30 mL x 2) and the combined extracts were washed with 50% saturated NaHCO₃ (50 mL), dried (MgSO₄), and concentrated under reduced pressure. The residue was purified by a chromatography on silica gel followed by a chromatography on C18 reverse phase column material. Lyophilization of the collected fraction resulted the product **39** mixture (376 mg, 50%, ~2.5:1 ratio) as trifluoroacetic acid salt: ¹H NMR (CD₃CN+TFA) δ 7.78 (d, 2H, *J* = 8.7 Hz), 7.52-7.42 (m, 2H); 7.37-7.22 (m 3H), 7.10 (d, 2H, *J =* 8.7 Hz), 5.78 (d, 1H, *J=* 9.0 Hz), 5.64 (m, 1H), 5.50 (br, 1H), 5.08 (m, 2H), 4.31-4.12 (m, 2H), 4.04-3.42 (m, 11H), 3.90 (s, 3H), 3.29 (m, 2H), 3.23 -3.16 (m, 1H), 3.08-2.78 (m, 6H), 2.76-2.27 (m, 5H), 2.23-2.11 (m, 1H), 2.08-1.77 (m, 3H),1.58 (d, 0.9H, *J*=7.2Hz),1.45 (d, 2.1H, *J* = 6.6 Hz), 1.32-1.20 (m, 3H), 0.95 - 0.84 (m, 6H); ³¹P NMR (CD₃CN+TFA) δ 24.1 and 23.8, 22.2 and 22.1; MS (ESI) 852 (M+H).

### Example 31

Metabolite 40: To a solution of the prodrug 39 (35.4 mg, 0.037 mmol) in DMSO (0.35 mL) and acetonitrile (0.70 mL) was added 0.1 M PBS buffer (10.5 mL) mixed thoroughly to result a suspension. To the suspension was added porcine liver esterase suspension (0.175 mL, EC3.1.1.1, Sigma). After the suspension was stored in 37°C for 6.5 h, the mixture was filtered through 0.45 um membrane filter and the filtrate was purified by HPLC. The collected fraction was lyophilized to result the product **40** as trifluoroacetic acid salt (28.8 mg, 90%): ¹H NMR (D₂O) δ 7.96 (d, 2H, *J=* 8.7 Hz), 7.32 (d, 2H, *J*= 8.7 Hz), 5.89 (d, 1H, *J* = 5.1 Hz), 5.66 (br, 1H), 5.27 (m, 1H), 4.97 (m, 1H), 4.23-4.12 (m, 2H), 4.08 (s, 3H), 4.06-3.10 (m, 14H), 3.03 (dd, 1H, *J* = 14.1 and 6.6 Hz), 2.78-1.97 (m, 9H), 1.66 (d, 3H, *J =* 6.9 Hz),1.03 (d, 3H, *J =* 7.5 Hz),1.01 (d, 3H, *J =* 6.9 Hz); ³¹P NMR (CD₃CN+TFA) δ 20.0, 19.8; MS (ESI) 748 (M+H).

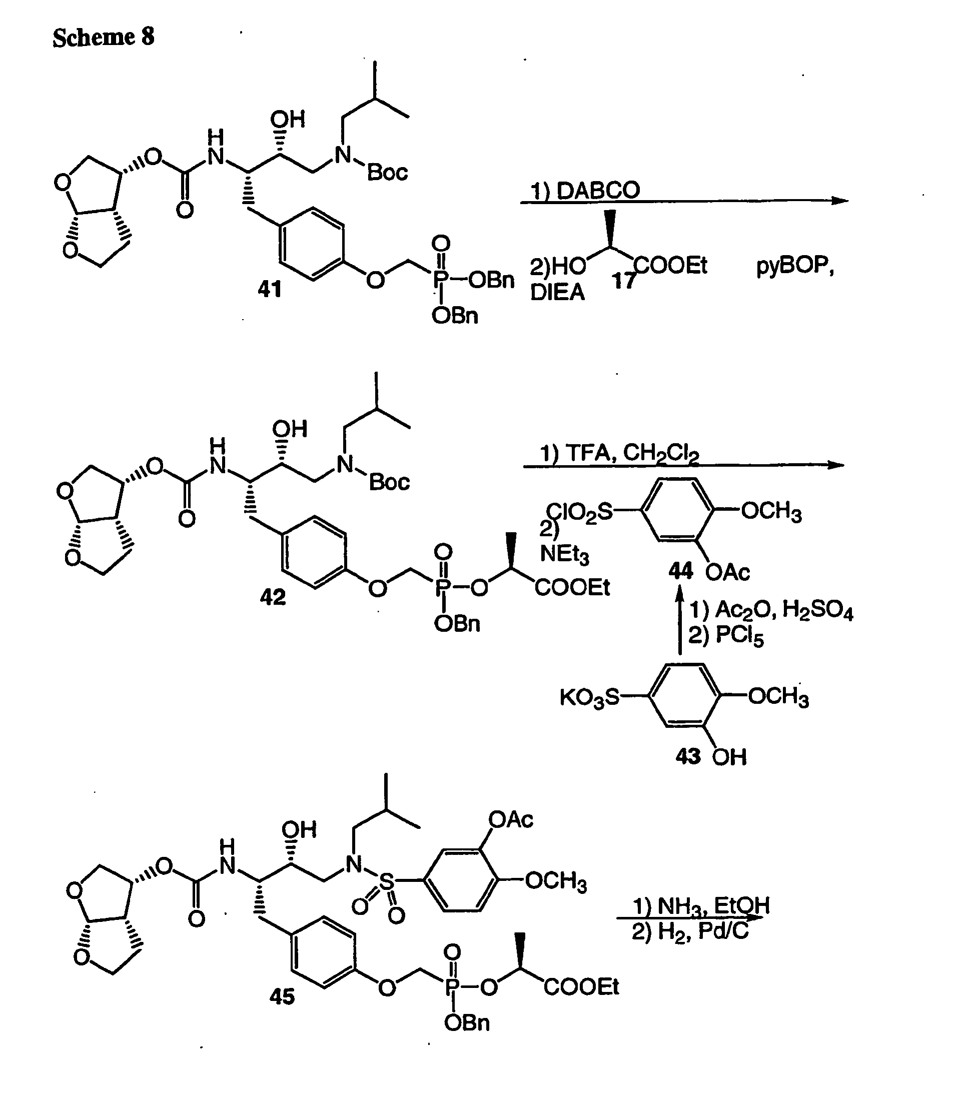

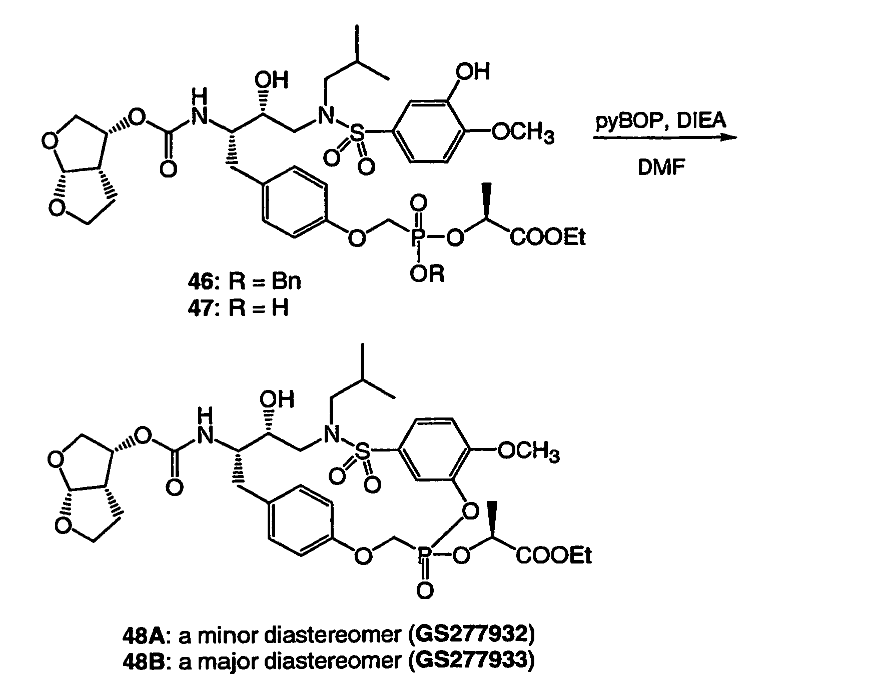

### Example 32

Compound 42: The dibenzyl phosphonate **41** (947 mg, 1.21 mmol) was treated with DABCO (140.9 mg, 1.26mmol, Aldrich) in 4.5 mL toluene to obtain the monoacid (890 mg, 106%). The crude monoacid (890 mg) was dried by evaporation with toluene twice and dissolved in DMF (5.3 mL) with ethyl (*S*)-lactate (0.3 mL, 2.65 mmol, Aldrich) and pyBOP (945 mg, 1.82 mmol, Aldrich) at room temperature. After diisopropylethylamine (0.85 mL, 4.88 mmol, Aldrich) was added, the solution was stirred at room temperature for 4 h and concentrated under reduced pressure to a half volume. The resulting solution was diluted with 5% aqueous HCl (30 mL) and the product was extracted with EtOAc (30 mL x 3). After the combined extracts were dried (MgSO₄) and concentrated, the residue was chromatographed on silica gel to afford the compound **42** (686 mg, 72%) as a mixture of two diastereomers (2:3 ratio): ¹H NMR (CDCl₃) δ 7.46-7.32 (m, 5H), 7.13 (d, 2H, *J* = 8.1 Hz), 6.85 (t, 2H, *J =* 8.1 Hz), 5.65 (m, 1H), 5.35-4.98 (m, 4H), 4.39 (d, 0.8H, *J* = 10.2 H), 4.30-4.14 (m, 3.2H), 3.98 (dd, 1H, *J =* 9.3 and 6.0 Hz), 3.92-3.78 (m, 3H), 3.78-3.55 (m, 3H), 3.16-2.68 (m, 6H), 1.85 (m, 1H), 1.74-1.55 (m, 2H), 1.56 (d, 1.8H, *J*= 7.2 Hz), 1.49 (d, 1.2H), 1.48 (s, 9H), 1.30-1.23 (m, 3H), 0.88 (d, 3H, *J* = 6.3 Hz), 0.87 (d, 3H, *J* = 6.3 Hz); ³¹P NMR (CDCl₃) δ 20.8 (0.4P), 19.5 (0.6P); MS (ESI) 793 (M+H).

### Example 33

Compound 45: A solution of compound **42** (101 mg, 0.127 mmol) and trifluoroacetic acid (0.27 mL, 3.5 mmol, Aldrich) in CH₂Cl₂ (0.6 mL) was stirred at 0°C for 3.5 h and concentrated under reduced pressure. The resulting residue was dried in vacuum to result the crude amine as TFA salt.
A solution of the crude amine salt and triethylamine (0.072 mL, 0.52 mmol, Aldrich) in CH₂Cl₂ (1 mL) was stirred at 0°C as the sulfonyl chloride **42** (37 mg, 0.14 mmol) was added. After the solution was stirred at 0°C for 4 h and 0.5 h at room temperature, the reaction mixture was diluted with saturated NaHCO₃ (20 mL) and extracted with EtOAc (20 mL x 1; 15 mL x 2). The combined organic fractions were washed with saturated NaCl solution, dried (MgSO₄) and concentrated under reduced pressure. Purification by chromatography on silica gel provided the sulfonamide **45** (85 mg, 72%) as a mixture of two diastereomers (~1:2 ratio): ¹H NMR (CDCl₃) δ 7.45-7.31 (m, 7H), 7.19 (d, 1H, *J* = 8.4 Hz), 7.12 (d, 2H, *J* = 7.8 Hz), 6.85 (m, 2H), 5.65 (d, 1H, *J*= 5.4 Hz), 5.34-5.16 (m, 2H), 5.13-4.97 (m, 2H), 4.97-4.86 (m, 1H), 4.38 (d, 0.7H, *J =* 10.8 Hz), 4.29-4.12 (m, 3.3H), 3.96 (dd, 1H, *J =* 9.3 and 6.3 Hz), 3.89 (s, 3H), 3.92-3.76 (m, 3H), 3.76-3.64 (m, 2H), 3.64-3.56 (br, 1H), 3.34-3.13 (m, 1H), 3.11-2.70 (m, 6H), 2.34 (s, 3H), 1.86 (m, 1H, *J* = 7.0 Hz), 1.75-1.58 (m, 2H), 1.56 (d, 2H, *J* = 7.2 Hz), 1.49 (d, 1H, *J* = 7.2 Hz), 1.29-1.22 (m, 3H), 0.94 (d, 3H, *J* = 6.6 Hz), 0.90 (d, 3H, *J* = 6.9 Hz); ³¹P NMR (CDCl₃) δ 20.7 (0.3P), 19.5 (0.7P); MS (ESI) 921 (M+H).

### Example 34

Compound 46: Compound **45** (257 mg, 0.279 mmol) was stirred in a saturated solution of ammonia in ethanol (5 mL) at 0°C for 15 min and the solution was concentrated under reduced pressure. Purification of the residue by chromatography on silica gel provided compound **46** (2.6 mg, 84%): ¹H NMR (CDCl₃) δ 7.48-7.34 (m, 4H), 7.22-7.05 (m, 5H), 7.01 (d, 1H, *J* = 8.1 Hz), 6.87-6.80 (m, 2H), 5.68 (d, 1H, *J =* 4.8 Hz), 5.32 (dd, 1.3H, *J =* 8.7 and 1.8 Hz), 5.22 (d, 0.7H, *J* = 9.0 Hz), 5.11-5.00 (m, 3H), 4.47-4.14 (m, 4H), 4.00 (dd, 1H, *J* = 9.9 and 6.6 Hz), 3.93 (s, 3H), 3.95-3.63 (m, 5H), 3.07-2.90 (m, 4H), 2.85-2.75 (m, 1H), 2.75-2.63 (m, 2H), 1.88-1.67 (m, 3H), 1.65-1.55 (m, 2H), 1.57 (d, 2H, *J*= 6.9.Hz), 1.50 (d, 1H, *J* = 7.2 Hz), 1.31-1.20 (m, 3H), 0.95 (d, 3H, *J* = 6.6 Hz), 0.88 (d, 3H, *J=* 6.3 Hz); ³¹P NMR (CDCl₃) δ 20.7 (0.3P), 19.6 (0.7P); MS (ESI) 879 (M+H).

### Example 35

Compound 47: A mixture of compound **46** (176 mg, 0.200 mmol) and 10% Pd/C (9.8 mg, Aldrich) in EtOAc (4 mL) and ethanol (1 mL) was stirred under H₂ atmosphere for 3 h at room temperature. After the mixture was filtered through celite, the filtrate was concentrated to dryness to afford compound **47** (158 mg, 100%) as white powder: ¹H NMR (CDCl₃) δ 7.30-7.16 (m, 2H), 7.12 (d, 2H, *J =* 7.5 Hz), 7.01 (d, 1H, *J* = 7.8 Hz), 6.84 (d, 2H, *J* = 7.5 Hz), 5.66 (d, 1H, *J*= 4.5 Hz), 5.13-4.97 (m, 2H), 4.38-4.10 (m, 4H), 3.93 (s, 3H), 4.02-3.66 (m, 6H), 3.13-2.69 (m, 7H), 1.96-1.50 (m, 3H), 1.57 (d, 3H, *J =* 6.6 Hz), 1.26 (t, 3H, *J =* 7.2 Hz), 0.93 (d, 3H, *J* = 6.0 Hz), 0.88 (d, 3H, *J =* 6.0 Hz); ³¹P NMR (CDCl₃) δ 20.1; MS (ESI) 789 (M+H).

### Example 36

Compound 48A and 48B: A solution of pyBOP (191 mg, 0.368 mmol, Aldrich) and diisopropylethylamine (0.1 mL, 0.574 mmol, Aldrich) in DMF (35 mL) was stirred at room temperature as a solution of compound **47** (29 mg, 0.036 mmol) in DMF (5.5 mL) was added over 16 h. After addition, the solution was stirred at room temperature for 3 h and concentrated under reduced pressure. The residue was dissolved in ice-cold water and extracted with EtOAc (20 mL x1; 10 mL x 2). The combined extracts were dried (MgSO₄) and concentrated under reduced pressure. The residue was purified by chromatography on silica gel followed by preparative TLC gave two isomers of structure 48 (1.0 mg, 3.6% and 3.6 mg, 13%). Isomer **48A**: ¹H NMR (CDCl₃) δ 7.39 (m, 1H), 7.12 (br, 1H), 7.01 (d, 2H, *J* = 8.1 Hz), 6.98 (br, 1H), 6.60 (d, 2H, *J*= 8.1 Hz), 5.75 (d, 1H, *J* = 5.1 Hz), 5.37-5.28 (m, 2H), 5.18 (q, 1H, *J =* 8.7 Hz), 4.71 (dd, 1H, *J =* 14.1 and 7.5 Hz), 4.29 (m, 3H), 4.15-4.06 (m, 1H), 3.99 (s, 3H), 4.05-3.6 (m, 5H), 3.35 (m, 1H), 3.09 (br, 1H), 2.90-2.78 (m, 3H), 2.2-2.0 (m, 3H), 1.71 (d, 3H, *J* = 6.6 Hz), 1.34 (t, 3H, *J* = 6.9 Hz), 1.01 (d, 3H, *J* = 6.3 Hz), 0.95 (d, 3H, *J* = 6.3 Hz); ³¹P NMR (CDCl₃) δ 17.8; MS (ESI) 793 (M+Na); isomer **48B**: ¹H NMR (CDCl₃) δ 7.46 (d, 1H, *J* = 9.3 Hz), 7.24 (br, 1H), 7.00 (d, 2H, *J* = 8.7 Hz), 6.91 (d, 1H, *J* = 8.7 Hz), 6.53 (d, 2H, *J=* 8.7 Hz), 5.74 (d, 1H, *J=* 5.1 Hz), 5.44 (m, 1H), 5.35 (d, 1H, *J =* 9.0 Hz), 5.18 (q, 1H, *J* = 7.2 Hz), 4.68 (dd, 1H, *J* = 14.4 and 6.3 Hz), 4.23 (m, 3H), 4.10 (m, 1H), 4.04 (s, 3H), 3.77-4.04 (m, 6H), 3.46 (dd, 1H, *J* =12.9 and 11.4 Hz), 3.08 (br, 1H), 2.85 (m, 2H), 2.76 (dd, 1H, *J* = 12.9 and 4.8 Hz), 1.79-2.11 (m, 3H), 1.75 (d, 3H, *J=* 6.6 Hz), 1.70 (m, 2H), 1.27 (t, 3H, *J =* 6.9 Hz), 1.01 (d, 3H, *J* = 6.6 Hz), 0.93 (d, 3H, *J =* 6.6 Hz); ³¹P NMR (CDCl₃) δ 15.4; MS (ESI) 793 (M+Na).

### Example Section P

### Example 1

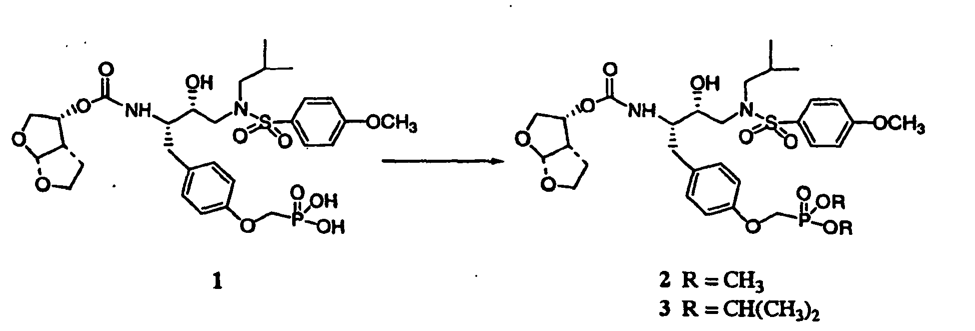

### Example 1A

Dimethylphosphonic ester 2 (R = CH₃): To a flask was charged with phosphonic acid 1 (67 mg, 0.1 mmol), methanol (0.1 mL, 2.5 mmol) and 1, 3-dicyclohexylcarbodiimide (83 mg, 0.4 mmol), then pyridine (1 mL) was added under N₂. The resulted mixture was stirred at 60 -70°C for 2 h, then cooled to room temperature and diluted with ethyl acetate. The mixture was filtered and the filtrate was evaporated. The residue was diluted with ethyl acetate and the combined organic phase was washed with NH₄Cl, brine and water, dried over Na₂SO₄, filtered and concentrated. The residue was purified by chromatography on silica gel (isopropanol/CH₂Cl₂, 1% to 7%) to give 2 (39 mg, 56 %) as a white solid. ¹H NMR (CDCl₃) δ 7.71(d, J = 8.7 Hz, 2H), 7.15 (d, J = 8.7Hz, 2H), 7.00 (d, J = 8.7 Hz, 2H), 6.87 (d, J = 8.7 Hz, 2H), 5.65 (d, J = 5.1 Hz, 1H), 5.10-4.92 (m, 4H), 4.26 (d, J = 9.9 Hz, 2H), 3.96 -3.65 (m overlapping s, 15H), 3.14-2.76 (m, 7H), 1.81-1.55 (m, 3H), 0.91 (d, J = 6.6 Hz, 3H), 0.88 (d, J = 6.6 Hz, 3H); ³¹P NMR (CDCl₃) δ 21.7; MS (ESI) 723 (M+Na).

### Example 1B

Diisopropylphosphonic ester 3 (R = CH (CH₃)₂) was synthesized in the same manner in 60% yield. ¹H NMR (CDCl₃) δ 7.71(d, J = 8.7 Hz, 2H), 7.15 (d, J = 8.7Hz, 2H), 7.15 (d, J = 8.7 Hz, 2H), 6.99 (d, J = 8.7 Hz, 2H), 6.87 (d, J = 8.7 Hz, 2H), 5.66 (d, J = 5.1 Hz, 1H), 5.08-4.92 (m, 3H), 4.16 (d, J = 10.5 Hz, 2H), 3.98 -3.68 (m overlapping s, 9H), 3.16-2.78 (m, 7H), 1.82-1.56 (m, 3H), 1.37 (t, J = 6.3 Hz, 6H), 0.93 (d, J = 6.6 Hz, 3H), 0.88 (d, J = 6.6 Hz, 3H); ³¹P NMR (CDCl₃) δ 17.3; MS (ESI) 779 (M+Na).

### Example 2

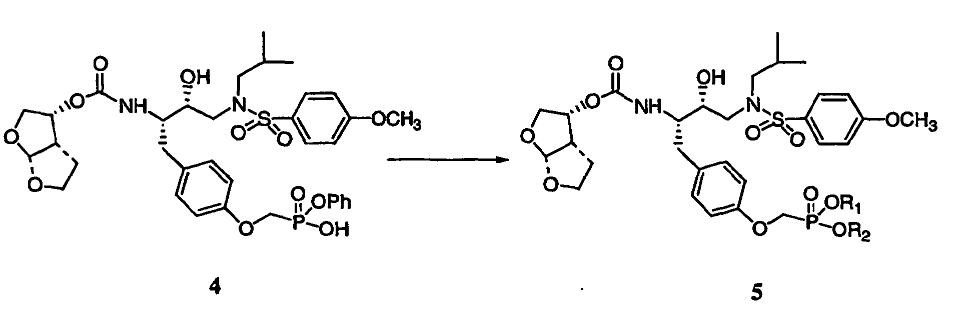

| **Compound** | **R₁** | **R₂** |
|---|---|---|
| 5a | OPh | mix-Hba-Et |
| 5b | OPh | (S)-Hba-Et |
| 5c | OPh | (S)-Hba-tBu |
| 5d | OPh | (S)-Hba-EtMor |
| 5e | OPh | (R)-Hba-Et |

### Example 2A

Monolactate **5a** (R1 = OPh, R2 = Hba-Et): To a flask was charged with monophenyl phosphonate **4** (250 mg, 0.33 mmol), 2-hydroxy-n-butyric acid ethyl ester (145 mg, 1.1 mmol) and 1, 3-dicyclohexylcarbodiimide (226 mg, 1.1 mmol), then pyridine (2.5 mL) was added under N₂. The resulted mixture was stirred at 60-70°C for 2 h, then cooled to room temperature and diluted with ethyl acetate. The mixture was filtered and the filtrate was evaporated. The residue was diluted with ethyl acetate and the combined organic phase was washed with NE₄Cl, brine and water, dried over Na₂SO₄, filtered and concentrated. The residue was purified by chromatography on silica gel (EtOAc/CH₂Cl₂, 1:1) to give 5a (150 mg, 52 %) as a white solid. ¹H NMR (CDCl₃) δ 7.70 (d, J = 8.7 Hz, 2H), 7.37-7.19 (m, 5H), 7.14 (d, J = 8.7 Hz, 2H), 7.00 (d, J = 8.7 Hz, 2H), 6.91 (d, J = 8.7 Hz, 1H), 6.86 (d, J = 8.7 Hz, 1H), 5.65 (m, 1H), 5.10-4.95 (m, 3H), 4.57-4.39 (m, 2H), 4.26 (m, 2H), 3.96 -3.68 (m overlapping s, 9H), 3.15-2.77 (m, 7H), 1.81-1.55 (m, 5H), 1.21 (m, 3H), 1.04-0.86 (m, 6H); ³¹P NMR (CDCl₃) δ 17.5 and 15.1; MS (ESI) 885 (M+Na).

### Example 2B

Monolactate **5b** (R1 = OPh, R2 = (S)-Hba-Et): To a flask was charged with monophenyl phosphonate **4** (600 mg, 0.8 mmol), (*S*)-2-hydroxy-n-butyric acid ethyl ester (317 mg, 2.4 mmol) and 1, 3-dicyclohexylcarbodiimide (495 mg, 2.4 mmol), then pyridine (6 mL) was added under N₂. The resulted mixture was stirred at 60-70°C for 2 h, then cooled to room temperature and diluted with ethyl acetate. The mixture was filtered and the filtrate was evaporated. The residue was diluted with ethyl acetate and the combined organic phase was washed with NH₄Cl, brine and water, dried over Na₂SO₄, filtered and concentrated. The residue was purified by chromatography on silica gel (EtOAc/CH₂Cl₂, 1:1) to give **5b** (360 mg, 52 %) as a white solid. ¹H NMR (CDCl₃) δ 7.71 (d, J = 8.7 Hz, 2H), 7.37-7.19 (m, 5H), 7.15 (d, J = 8.7 Hz, 2H), 7.00 (d, J = 8.7 Hz, 2H), 6.92 (d, J = 8.7 Hz, 1H), 6.86 (d, J = 8.7 Hz, 1H), 5.65 (m, 1H), 5.10-4.95 (m, 3H), 4.57-4.39 (m, 2H), 4.26 (m, 2H), 3.96 -3.68 (m overlapping s, 9H), 3.15-2.77 (m, 7H), 1.81-1.55 (m, 5H), 1.23 (m, 3H), 1.04-0.86 (m, 6H); ³¹P NMR (CDCl₃) δ 17.5 and 15.2; MS (ESI) 885 (M+Na).

### Example 2C

Monolactate **5c**(R1 = OPh, R2 = (*S*)-Hba-tBu): To a flask was charged with monophenyl phosphonate **4** (120 mg, 0.16 mmol), tert-butyl (*S*)-2-hydroxybutyrate (77 mg, 0.48 mmol) and 1, 3-dicyclohexylcarbodiimide (99 mg, 0.48 mmol), then pyridine (1 mL) was added under N₂. The resulted mixture was stirred at 60-70°C for 2 h, then cooled to room temperature and diluted with ethyl acetate. The mixture was filtered and the filtrate was evaporated. The residue was diluted with ethyl acetate and the combined organic phase was washed with NH₄Cl, brine and water, dried over Na₂SO₄, filtered and concentrated. The residue was purified by chromatography on silica gel (EtOAc/CH₂Cl₂, 1:1) to give 5c (68 mg, 48 %) as a white solid. ¹H NMR (CDCl₃) δ 7.71 (d, J = 8.7 Hz, 2H), 7.37-7.19 (m, 5H), 7.14 (d, J = 8.7 Hz, 2H), 7.00 (d, J = 8.7 Hz, 2H), 6.93 (d, J = 8.7 Hz, 1H), 6.86 (d, J = 8.7 Hz, 1H), 5.64 (m, 1H), 5.10-4.95 (m, 3H), 4.57-4.39 (m, 2H), 4.26 (m, 2H), 3.96 -3.68 (m overlapping s, 9H), 3.15-2.77 (m, 7H), 1.81-1.55 (m, 5H), 1.44 (d, J = 11 Hz, 9H), 1.04-0.86 (m, 9H); ³¹P NMR (CDCl₃) δ 17.5 and15.2; MS (ESI) 913 (M+Na).

### Example 2D

Monolactate **5d** (R1 = OPh, R2 = (*S*)-Lac-EtMor): To a flask was charged with monophenyl phosphonate **4** (188 mg, 0.25 mmol), (*S*)-lactate ethylmorpholine ester (152 mg, 0.75 mmol) and 1, 3-dicyclohexylcarbodiimide (155 mg, 0.75 mmol), then pyridine (2mL) was added under N₂. The resulted mixture was stirred at 60-70°C for 2 h, then cooled to room temperature and diluted with ethyl acetate. The mixture was filtered and the filtrate was evaporated. The residue was washed with ethyl acetate and the combined organic phase was washed with NH₄Cl, brine and water, dried over Na₂SO₄, filtered and concentrated. The residue was purified by chromatography on silica gel (isopropanol/CH₂Cl₂, 1:9) to give **5d** (98 mg, 42 %) as a white solid. ¹H NMR (CDCl₃) δ 7.72 (d, J = 8.7 Hz, 2H), 7.34-7.20 (m, 5H), 7.15 (d, J = 8.7 Hz, 2H), 7.00 (d, J = 8.7 Hz, 2H), 6.92 (d, J = 8.7 Hz, 1H), 6.87 (d, J = 8.7 Hz, 1H), 5.65 (m, 1H), 5.21-4.99 (m, 3H), 4.57-4.20 (m, 4H), 3.97 -3.63 (m overlapping s, 13H), 3.01-2.44 (m, 13H), 1.85-1.50 (m, 6H), 0.92 (d, J = 6.5 Hz, 3H), 0.88 (d, J = 6.5, 3H); ³¹P NMR (CDCl₃) δ 17.4 and 15.3; MS (ESI) 934(M).

### Example 2E

Monolactate **5e** (R1 = OPh, R2 = (R)-Hba-Et): To a flask was charged with monophenyl phosphonate **4** (600 mg, 0.8 mmol), (*R*)-2-hydroxy-n-butyric acid ethyl ester (317 mg, 2.4 mmol) and 1, 3-dicyclohexylcarbodiimide (495 mg, 2.4 mmol), then pyridine (6 mL) was added under N₂. The resulted mixture was stirred at 60-70°C for 2 h, then cooled to room temperature and diluted with ethyl acetate. The mixture was filtered and the filtrate was evaporated. The residue was diluted with ethyl acetate and the combined organic phase was washed with NH₄Cl, brine and water, dried over Na₂SO₄, filtered and concentrated. The residue was purified by chromatography on silica gel (EtOAc/CH₂Cl₂, 1:1) to give 5e (345 mg, 50 %) as a white solid. ¹H NMR (CDCl₃) δ 7.70 (d, J=8.7 Hz, 2H), 7.37-7.19 (m, 5H), 7.15 (d, J = 8.7 Hz, 2H), 7.00 (d, J = 8.7 Hz, 2H), 6.92 (d, J=8.7 Hz, 1H), 6.86 (d, J = 8.7 Hz, 1H), 5.65 (m, 1H), 5.10-4.95 (m, 3H), 4.57-4.39 (m, 2H), 4.26 (m, 2H), 3.96 -3.68 (m overlapping s, 9H), 3.15-2.77 (m, 7H), 1.81-1.55 (m, 5H), 1.23 (m, 3H), 1.04-0.86 (m, 6H); ³¹P NMR (CDCl₃) δ 17.5 and 15.1 ; MS (ESI) 885 (M+Na).

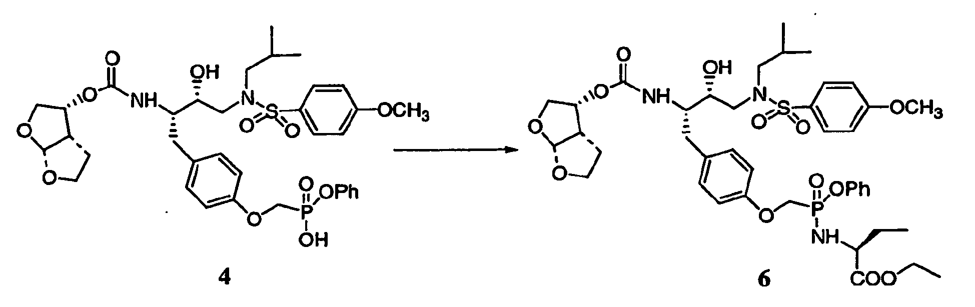

### Example 3

Monoamidate **6**: To a flask was charged with monophenyl phosphonate **4** (120 mg, 0.16 mmol), L-alanine butyric acid ethyl ester hydrochloride (160 mg, 0.94 mmol) and 1, 3-dicyclohexylcarbodiimide (132 mg, 0.64 mmol), then pyridine (1 mL) was added under N₂. The resulted mixture was stirred at 60-70°C for 2 h, then cooled to room temperature and diluted with ethyl acetate. The mixture was filtered and the filtrate was evaporated. The residue was diluted with ethyl acetate and the combined organic phase was washed with NH₄Cl, brine and water, dried over Na₂SO₄, filtered and concentrated. The residue was purified by chromatography on silica gel (isopropanol/CH₂Cl₂, 1:9) to give **6** (55 mg, 40 %) as a white solid. ¹H NMR (CDCl₃) δ 7.72 (d, J = 8.7 Hz, 2H), 7.37-7.23 (m, 5H), 7.16 (d, J = 8.7 Hz, 2H), 7.00 (d, J = 8.7 Hz, 2H), 6.90-6.83 (m, 2H), 5.65 (d, J = 5.1Hz, 1H), 5.10-4.92 (m, 3H), 4.28 (m, 2H), 3.96 -3.68 (m overlapping s, 9H), 3.15-2.77 (m, 7H), 1.81-1.55 (m, 5H), 1.23 (m, 3H), 1.04-0.86 (m, 6H); ³¹P NMR (CDCl₃) δ 20.7 and 19.6; MS (ESI) 884(M+Na).

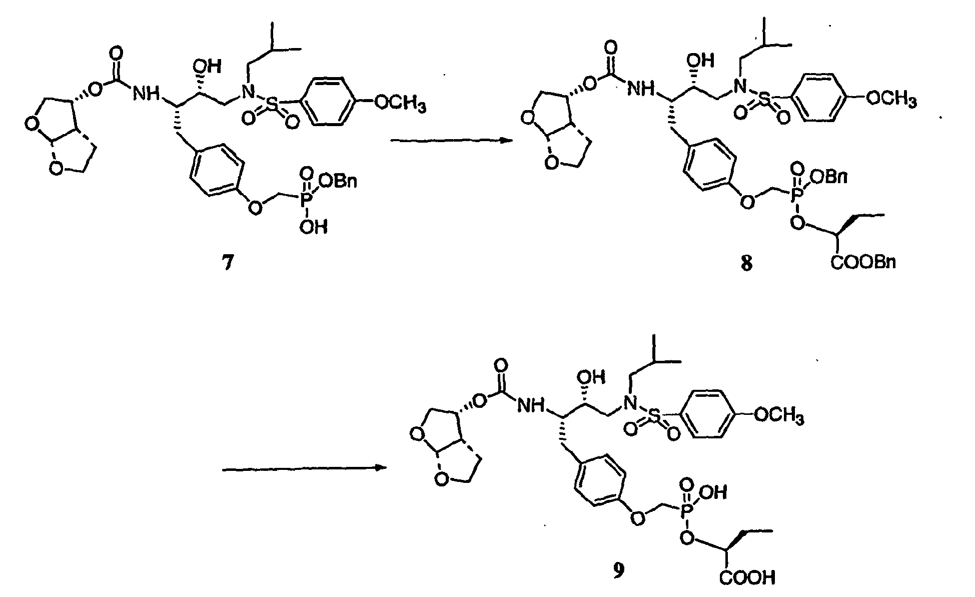

### Example 4A

Compound **8**: To a stirred solution of monobenzyl phosphonate 7 (195 mg, 0.26mmol) in 1 mL of DMF at room temperature under N₂ was added benzyl-(s)-lactate (76 mg, 0.39 mmol) and PyBOP (203 mg, 0.39mmol), followed by DIEA (181µL, 1 mmol). After 3 h, the solvent was removed under reduced pressure, and the resulting crude mixture was purified by chromatography on silica gel (ethyl acetate/hexane 1:1) to give **8** (120 mg, 50%) as a white solid. ¹H NMR (CDCl₃) δ 7.71 (d, J = 8.7 Hz, 2H), 7.38-7.34 (m, 5H), 7.12 (d, J = 8.7 Hz, 2H), 6.99 (d, J = 8.7 Hz, 2H), 6.81(d, J = 8.7 Hz, 2H), 5.64 (d, J = 5.4 Hz, 1H), 5.24-4.92 (m, 7H), 4.28 (m, 2H), 3.96 -3.67 (m overlapping s, 9H), 3.16-2.76 (m, 7H), 1.95-1.62 (m, 5H), 0.99-0.87 (m, 9H); ³¹P NMR (CDCl₃) δ 21.0 and 19.7; MS (ESI) 962 (M+Na).

### Example 4B

Compound **9:** A solution of compound **8** (100 mg) was dissolved in EtOH/ EtOAc (9 mL/3mL), treated with 10 % Pd/C (10 mg) and was stirred under H₂ atmosphere (balloon) for 1.5 h. The catalyst was removed by filtration through celite. The filtered was evaporated under reduced pressure, the residue was triturated with ether and the solid was collected by filtration to afford the compound **9** (76mg, 94%) as a white solid. ¹H NMR (CD₃OD) δ 7.76 (d, J = 8.7 Hz, 2H), 7.18 (d, J = 8.7 Hz, 2H), 7.08 (d, J = 8.7 Hz, 2H), 6.90 (d, J = 8.7 Hz, 2H), 5.59 (d, J = 5.4 Hz, 1H), 5.03-4.95 (m, 2H), 4.28 (m, 2H), 3.90 -3.65 (m overlapping s, 9H), 3.41 (m, 2H), 3.18-2.78 (m, 5H), 2.44 (m, 1H), 1.96 (m, 3H), 1.61 (m, 2H), 1.18 (m, 3H), 0.93 (d, J = 6.3 Hz, 3H), 0.87 (d, J = 6.3 Hz, 3H); ³¹P NMR (CD₃OD) δ 18.3; MS (ESI) 782 (M+Na).

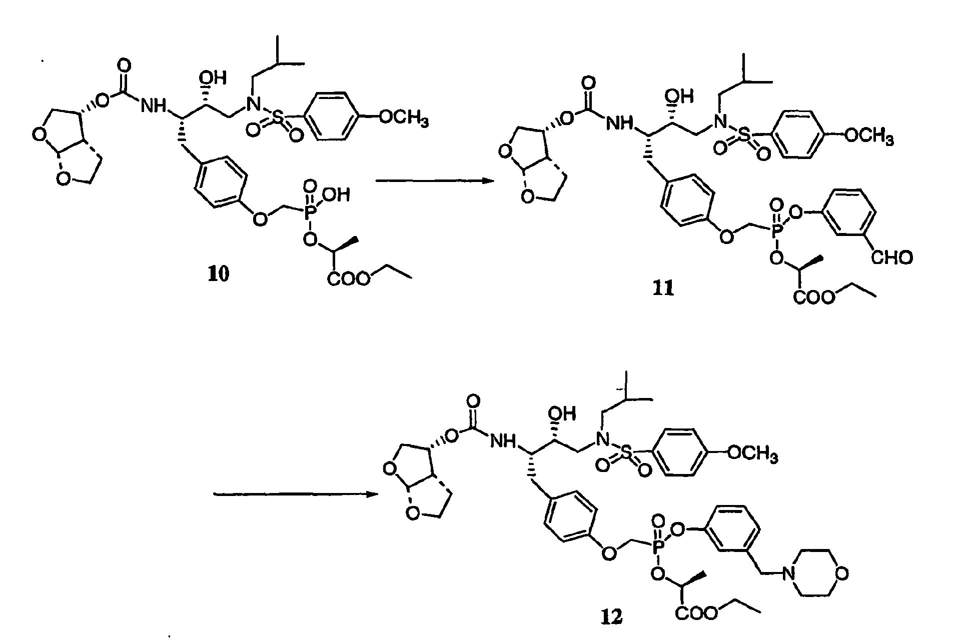

### Example 5A

Compound **11:** To a stirred solution of compound **10** (1 g, 1.3mmol) in 6 mL of DMF at room temperature under N₂ was added 3-hydroxybenzaldehyde (292 mg, 2.6 mmol) and PyBOP (1 g, 1.95mmol), followed by DIEA (0.9 mL, 5.2 mmol). After 5 h, the solvent was removed under reduced pressure, and the resulting crude mixture was purified by chromatography on silica gel (ethyl acetate/hexane 1:1) to give **11** (800 mg, 70%) as a white solid. ¹H NMR (CDCl₃) δ 9.98 (s, 1H), 7.79-6.88 (m, 12H), 5.65 (m, 1H), 5.21-4.99 (m, 3H), 4.62-4.16 (m, 4H), 3.99 -3.61 (m overlapping s, 9H), 3.11-2.79 (m, 5H), 1.85-1.53 (m, 6H), 1.25 (m, 3H), 0.90 (m, 6H); ³¹P NMR (CDCl₃) δ 17.9 and 15.9; MS (ESI) 899 (M+ Na).

### Example 5B

Compound **12:** To a stirred solution of compound **11** (920 mg, 1.05 mmol) in 10 mL of ethyl acetate at room temperature under N₂ was added morpholine (460 mg, 5.25 mmol) and acedic acid (0.25 mL, 4.2 mmol), followed by sodium cyanoborohydride (132 mg, 2.1 mmol). After 20h, the solvent was removed under reduced pressure, and the residue was diluted with ethyl acetate and the combined organic phase was washed with NH₄Cl, brine and water, dried over Na₂SO₄, filtered and concentrated. The residue was purified by chromatography on silica gel (isopropanol / CH₂Cl₂, 6%) to give **12** (600 mg, 60%) as a white solid. ¹H NMR (CDCl₃) δ 7.71 (d, J = 8.7 Hz, 2H), 7.27 (m, 4H), 7.15 (d, J = 8.7 Hz, 2H), 6.95 (d, J = 8.7 Hz, 2H), 6.89 (m, 2H), 5.65 (m, 1H), 5.21-5.02 (m, 3H), 4.58-4.38 (m, 2H), 4.21-4.16 (m, 2H), 3.99 -3.63 (m overlapping s, 15H), 3.47 (s, 2H), 3.18-2.77 (m, 7H), 2.41 (s, 4H), 1.85-1.53 (m, 6H), 1.25 (m, 3H), 0.90 (m, 6H); ³¹P NMR (CDCl₃) δ 17.4 and 15.2; MS (ESI) 971 (M+Na).

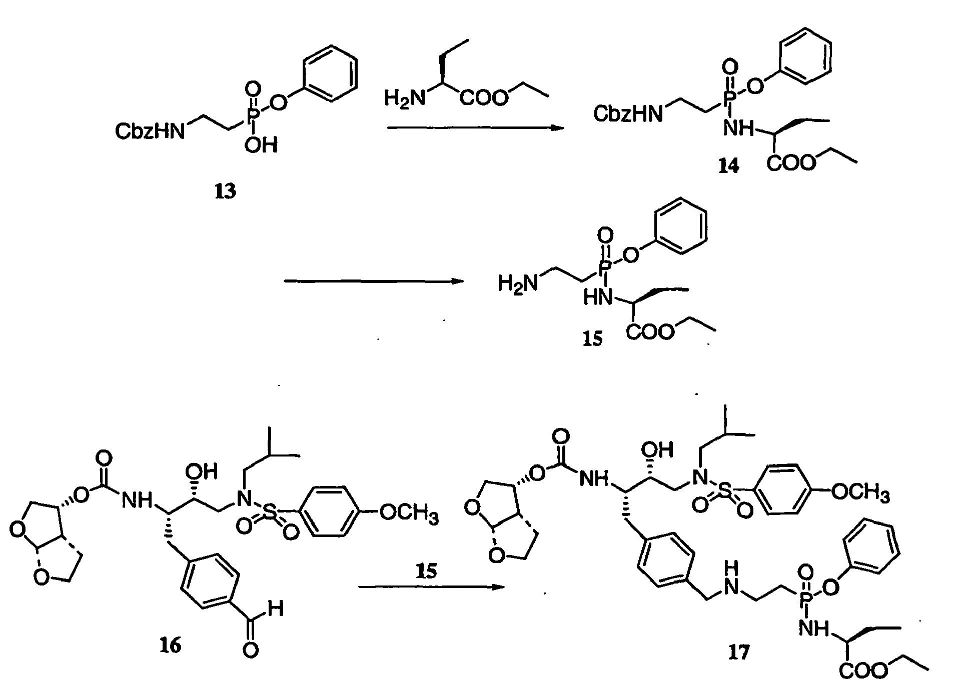

### Example 6A

Compound **14:** To a stirred solution of compound **13** (1 g, 3 mmol) in 30 mL of acetonitrile at room temperature under N₂ was added thionyl chloride (0.67 mL, 9 mmol). The resulted mixture was stirred at 60-70°C for 0.5 h. After cooled to room temperature, the solvent was removed under reduced pressure, and the residue was added 30 mL of DCM, followed by DIEA (1.7 mL, 10 mmol), L-alanine butyric acid ethyl ester hydrochloride (1.7 g, 10 mmol) and TEA (1.7 mL, 12 mmol). After 4h at room temperature, the solvent was removed under reduced pressure, and the residue was diluted with DCM and washed with brine and water, dried over Na₂SO₄, filtered and concentrated. The residue was purified by chromatography on silica gel (Hexane/EtOAc 1:1) to give **14** (670 mg, 50%) as a yellow oil. ¹H NMR (CDCl₃) δ 7.33-7.11 (m, 10H), 5.70 (m, 1H), 5.10 (s, 2H), 4.13-3.53 (m, 5H), 2.20-2.10 (m, 2H), 1.76-1.55 (m, 2H), 1.25-1.19 (m, 3H), 0.85-0.71 (m, 3H); ³¹P NMR (CDCl₃) δ 30.2 and 29.9; MS (ESI) 471 (M+Na).

### Example 6B

Compound 15: A solution of compound **14** (450mg) was dissolved in 9 mL of EtOH, then 0.15 mL of acetic acid and 10 % Pd/C (90 mg) was added. The resulted mixture was stirred under H2 atmosphere (balloon) for 4 h. After filtration through celite, the filtered was evaporated under reduced pressure to afford the compound 15 (300mg, 95%) as a colorless oil. ¹H NMR (CDCl₃) δ 7.29-7.12 (m, 5H), 4.13-3.53 (m, 5H), 2.20-2.10 (m, 2H), 1.70-1.55 (m, 2H), 1.24-1.19 (m, 3H), 0.84-0.73(m, 3H); ³¹P NMR (CDCl₃) δ 29.1 and 28.5; MS (ESI) 315 (M+1).

### Example 6C

Monoamdidate **17:** To a stirred solution of compound **16** (532 mg, 0.9 mmol) in 4 mL of 1,2-dichloroethane was added compound **15** (300 mg, 0.96 mmol) and MgSO₄ (50 mg), the resulted mixture was stirred at room temperature under argon for 3h, then acetic acid (1.3 mL, 23 mmol) and sodium cyanoborohydride (1.13 g, 18 mmol) were added. The reaction mixture was stirred at room temperature for 1 h under argon. Then aqueous NaHCO₃ (50 mL) was added, and the mixture was extracted with ethyl acetate, and the combined organic layers were washed with brine and water, dried over Na₂SO₄, filtered and concentrated. The residue was purified by chromatography on silica gel (EtOH / EtOAc, 1/9) to give **17** (600 mg, 60%) as a white solid. ¹H NMR (CDCl₃) δ 7.73 (d, J = 8.7 Hz, 2H), 7.33-7.13 (m, 9H), 7.00 (d, J = 8.7 Hz, 2H), 5.65 (d, J = 5.4 Hz, 1H), 5.11-4.98 (m, 2H), 4.22 -3.68 (m overlapping s, 15H), 3.20-2.75 (m, 9H), 2.21-2.10 (m, 2H), 1.88-1.55(m, 5H), 1.29-1.19 (m, 3H), 0.94-0.70 (m, 9H); ³¹P NMR (CDCl₃) δ 31.8 and 31.0; MS (ESI) 889 (M).

### Example 7

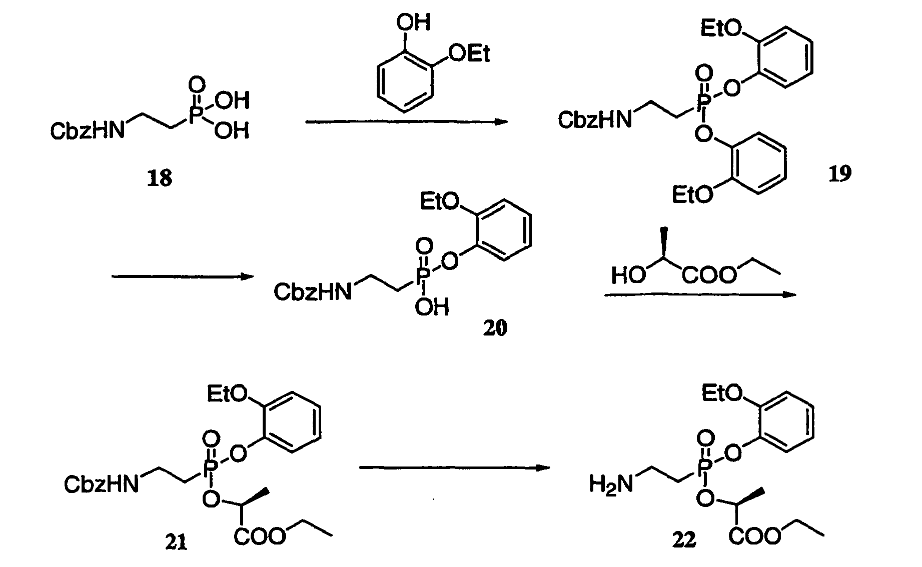

### Example 7A

Compound **19:** To a stirred solution of compound **18** (3.7 g, 14.3 mmol) in 70 mL of acetonitrile at room temperature under N₂ was added thionyl chloride (6.3 mL, 86 mmol). The resulted mixture was stirred at 60-70°C for 2 h. After cooled to room temperature, the solvent was removed under reduced pressure, and the residue was added 150 mL of DCM, followed by TEA (12 mL, 86 mmol) and 2-ethoxyphenol (7.2 mL, 57.2 mmol). After 20h at room temperature, the solvent was removed under reduced pressure, and the residue was diluted with ethyl acetate and washed with brine and water, dried over Na₂SO₄, filtered and concentrated. The residue was purified by chromatography on silica gel (DCM/EtOAc 9:1) to give **19** (4.2 g, 60%) as a yellow oil. ¹H NMR (CDCl₃) δ 7.32-6.83 (m, 13H), 5.22 (m, 1H), 5.12 (s, 2H), 4.12-3.73 (m, 6H), 2.52-2.42 (m, 2H), 1.41-1.37 (m, 6H); ³¹P NMR (CDCl₃) δ 25.4; MS (ESI) 522 (M+Na).

### Example 7B

Compound **20:** A solution of compound **19**(3 g, 6 mmol) was dissolved in 70 mL of acetonitrile at 0°C, then 2N NaOH (12 mL, 24 mmol) was added dropwisely. The reaction mixture was stirred at room temperature for 1.5 h. Then the solvent was removed under reduced pressure, and the residue diluted with water and extracted with ethyl acetate. The aqueous layer was acidified with conc. HCl to PH = 1, then extracted with ethyl acetate, combined the organic layer and dried over Na₂SO₄, filtered and concentrated to give compound **20** (2 g, 88%) as a off-white solid. ¹H NMR (CDCl₃) δ 7.33-6.79 (m, 9H), 5.10 (s, 2H), 4.12-3.51 (m, 6H), 2.15-2.05 (m, 2H), 1.47-1.33 (m, 3H); ³¹P NMR (CDCl₃) δ 30.5; MS (ESI) 380 (M+1).

### Example 7C

Compound **21:** To a stirred solution of compound **20** (1 g, 2.6 mmol) in 20 mL of acetonitrile at room temperature under N₂ was added thionyl chloride (1.1 mL, 15.6 mmol). The resulted mixture was stirred at 60-70°C for 45 min. After cooled to room temperature, the solvent was removed under reduced pressure, and the residue was added 25 mL of DCM, followed by TEA (1.5 mL, 10.4 mmol) and (S) lactate ethyl ester (0.9 mL, 7.8 mmol). After 20h at room temperature, the solvent was removed under reduced pressure, and the residue was diluted with DCM and washed with brine and water, dried over Na₂SO₄, filtered and concentrated. The residue was purified by chromatography on silica gel (DCM / EtOAc 3:1) to give **21** (370 mg, 30%) as a yellow oil. ¹H NMR (CDCl₃) δ 7.33- 6.84 (m, 9H), 6.17-6.01 (m, 1H), 5.70 (m, 1H), 5.18-5.01 (m, 3H), 4.25-4.04 (m, 4H), 3.78-3.57 (m, 2H), 2.38-2.27 (m, 2H), 1.5-1.23 (m, 9H); ³¹P NMR (CDCl₃) δ 29.2 and 27.3; MS (ESI) 502 (M+Na).

### Example 7D

Compound **22:** A solution of compound **21** (370mg) was dissolved in 8 mL of EtOH, then 0.12 mL of acetic acid and 10 % Pd/C (72 mg) was added. The resulted mixture was stirred under H₂ atmosphere (balloon) for 4 h. After filtration through celite, the filtered was evaporated under reduced pressure to afford the compound **22** (320mg, 96%) as a colorless oil. ¹H NMR (CDCl₃) 7.27- 6.86 (m, 4H), 5.98 (s, 2H), 5.18-5.02 (m, 1H), 4.25-4.06 (m, 4H), 3.34-3.24 (m, 2H), 2.44-2.30 (m, 2H), 1.62-1.24 (m, 9H); ³¹P NMR (CDCl₃) δ 28.3 and 26.8; MS (ESI) 346 (M+1).

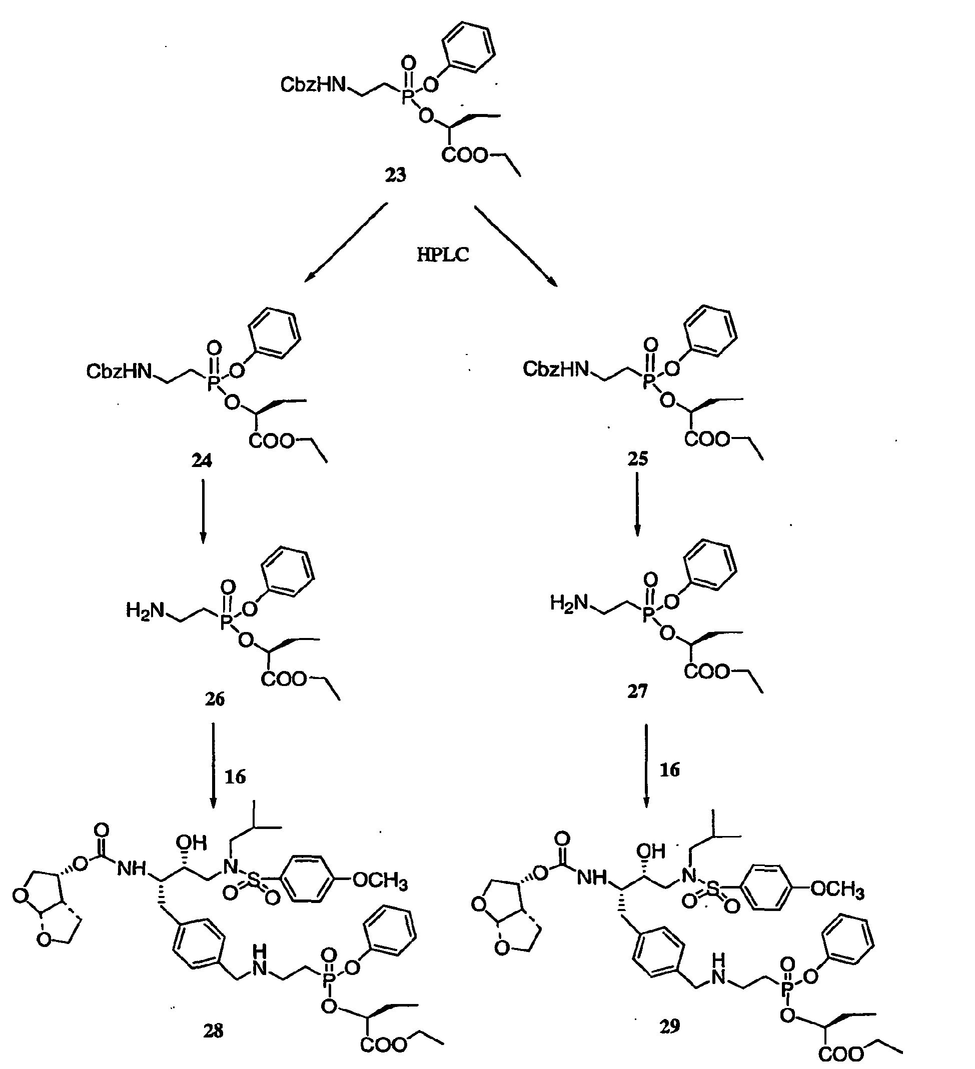

### Example 8A

Compound **24:** Compound **23** was purified using a Dynamax SD-200 HPLC system. The mobile phase consisted of acetonitrile: water (65:35, v/v) at a flow rate of 70 mL/ min. The injection volume was 4 mL. The detection was by fluorescence at 245 nm and peak area ratios were used for quantitations. Retention time was 8.2 min for compound **24** as yellow oil. ¹H NMR (CDCl₃) δ 7.36-7.19 (m, 10H), 5.88 (m, 1H), 5.12 (s, 2H), 4.90-4.86 (m, 1H), 4.26-4.12 (m, 2H), 3.72-3.61(m, 2H), 2.36-2.29 (m, 2H), 1.79-1.74 (m, 2H); 1.27 (t, J = 7.2 Hz, 3H), 0.82 (t, J = 7.2 Hz, 3H); ³¹P NMR (CDCl₃) δ 28.3; MS (ESI) 472 (M+Na).

### Example 8B

Compound 25 was purified in the same manner and retention time was 7.9 min for compound 25 as yellow oil. ¹H NMR (CDCl₃) δ 7.34-7.14 (m, 10H), 5.75 (m, 1H), 5.10 (s, 2H), 4.96-4.91 (m, 1H), 4.18-4.12 (m, 2H), 3.66-3.55(m, 2H), 2.29-2.19 (m, 2H), 1.97-1.89 (m, 2H); 1.21 (t, J = 7.2 Hz, 3H), 0.97 (t, J = 7.2 Hz, 3H); ³¹P NMR (CDCl₃) δ 26.2; MS (ESI) 472 (M+Na).

### Example 8C

Compound **26:** A solution of compound 24 (1 g) was dissolved in 20 mL of EtOH, then 0.3 mL of acetic acid and 10 % Pd/C (200 mg) was added. The resulted mixture was stirred under H2 atmosphere (balloon) for 4 h. After filtration through celite, the filtered was evaporated under reduced pressure to afford the compound 26 (830mg, 99 %) as a colorless oil. ¹H NMR (CDCl₃) δ 7.46-7.19 (m, 5H), 4.92-4.81 (m, 1H), 4.24-4.21 (m, 2H), 3.41-3.28 (m, 2H), 2.54-2.38 (m, 2H), 1.79-1.74 (m, 2H), 1.27 (t, J = 7.2 Hz, 3H), 0.80 (t, J = 7.2 Hz, 3H); ³¹P NMR (CDCl₃) δ 26.9; MS (ESI) 316 (M+1).

### Example 8D

Compound **27:** A solution of compound **25** (700g) was dissolved in 14 mL of EtOH, then 0.21 mL of acetic acid and 10 % Pd/C (140 mg) was added. The resulted mixture was stirred under H2 atmosphere (balloon) for 4 h. After filtration through celite, the filtered was evaporated under reduced pressure to afford the compound 27 (510mg, 98 %) as a colorless oil. ¹H NMR (CDCl₃) δ 7.39-7.18 (m, 5H), 4.98-4.85 (m, 1H), 4.25-4.22 (m, 2H), 3.43-3.28 (m, 2H), 2.59-2.41 (m, 2H), 1.99-1.85 (m, 2H), 1.28 (t, J = 7.2 Hz, 3H), 1.02 (t, J = 7.2 Hz, 3H); ³¹P NMR (CDCl₃) δ 24.2; MS (ESI) 316 (M+1).

### Example 8E

Compound **28:** To a stirred solution of compound **16** (1.18 g, 2 mmol) in 9 mL of 1,2-dichloroethane was added compound **26** (830 mg, 2.2 mmol) and MgSO₄ (80 mg), the resulted mixture was stirred at room temperature under argon for 3h, then acetic acid (0.34 mL, 6 mmol) and sodium cyanoborohydride (251mg, 4 mmol) were added. The reaction mixture was stirred at room temperature for 2 h under argon. Then aqueous NaHCO₃ (50 mL) was added, and the mixture was extracted with ethyl acetate, and the combined organic layers were washed with brine and water, dried over Na₂SO₄, filtered and concentrated. The residue was purified by chromatography on silica gel (EtOH/EtOAc, 1/9) to give **28** (880 mg, 50 %) as a white solid. ¹H NMR (CDCl₃) δ 7.71 (d, J = 8.7 Hz, 2H), 7.35-7.16 (m, 9H), 6.99 (d, J = 8.7 Hz, 2H), 5.64 (d, J = 5.4 Hz, 1H), 5.03-4.85 (m, 3H), 4.24 -3.67 (m overlapping s, 15H), 3.14-2.70 (m, 9H), 2.39-2.28 (m, 2H), 1.85-1.51 (m, 5H), 1.29-1.25 (m, 3H), 0.93-0.78 (m, 9M; ³¹P NMR (CDCl₃) δ 29.2; MS (ESI) 912 (M+Na).

### Example 8F

Compound **29:** To a stirred solution of compound **16** (857 g, 1.45 mmol) in 7 mL of 1,2-dichloroethane was added compound **27** (600 mg, 1.6 mmol) and MgSO₄ (60 mg), the resulted mixture was stirred at room temperature under argon for 3h, then acetic acid (0.23 mL, 3 mmol) and sodium cyanoborohydride (183mg, 2.9 mmol) were added. The reaction mixture was stirred at room temperature for 2 h under argon. Then aqueous NaHCO₃ (50 mL) was added, and the mixture was extracted with ethyl acetate, and the combined organic layers were washed with brine and water, dried over Na₂SO₄, filtered and concentrated. The residue was purified by chromatography on silica gel (EtOH/EtOAc, 1/9) to give 29 (650 mg, 50 %) as a white solid. ¹H NMR (CDCl₃) δ 7.72 (d, J = 8.7 Hz, 2H), 7.35-7.16 (m, 9H), 7.00 (d, J = 8.7 Hz, 2H), 5.64 (d, J = 5.4 Hz, 1H), 5.03-4.90 (m, 3H), 4.17 -3.67 (m overlapping s, 15H), 3.16-2.77 (m, 9H), 2.26-2.19 (m, 2H), 1.94-1.53 (m, 5H), 1.26-1.18 (m, 3H), 1.00-0.87 (m, 9H); ³¹P NMR (CDCl₃) δ 27.4; MS (ESI) 912 (M+Na).

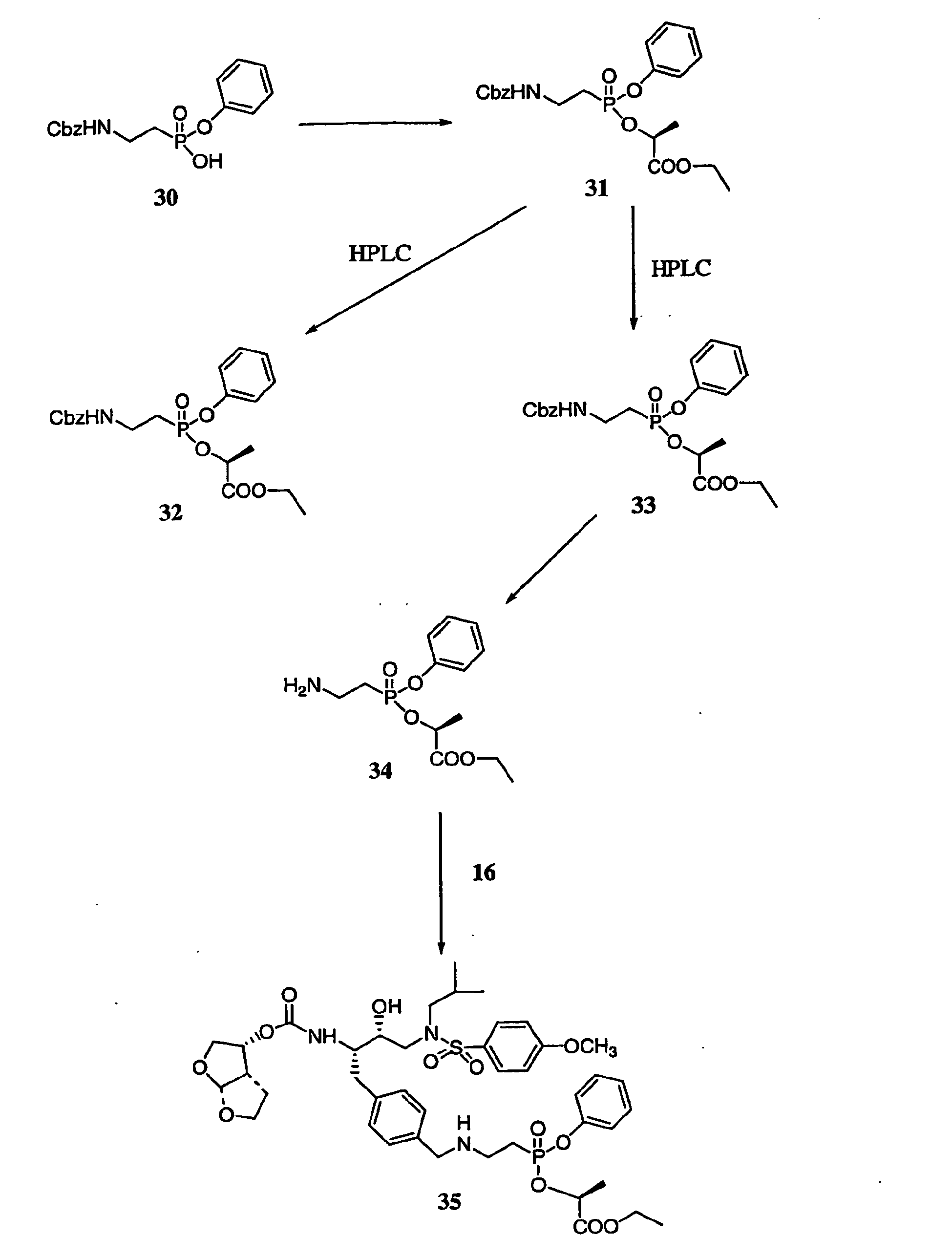

### Example 9A

Compound **31:** To a stirred solution of compound **30** (20 g, 60 mmol) in 320 mL of toluene at room temperature under N₂ was added thionyl chloride (17.5 mL, 240 mmol) and a few drops of DMF. The resulted mixture was stirred at 60-70°C for 3 h. After cooled to room temperature, the solvent was removed under reduced pressure, and the residue was added 280 mL of DCM, followed by TEA (50 mL, 360 mmol) and (S) lactate ethyl ester (17 mL, 150 mmol). After 20h at room temperature, the solvent was removed under reduced pressure, and the residue was diluted with DCM and washed with brine and water, dried over Na₂SO₄, filtered and concentrated. The residue was purified by chromatography on silica gel (DCM / EtOAc , 1:1) to give **31** (24 g, 92 %) as a yellow oil. ¹H NMR (CDCl₃) δ 7.33-7.18 (m, 10H), 5.94-6.63 (m, 1H), 5.70 (m, 1H), 5.12-4.95 (m, 3H), 4.24-4.14 (m, 2H), 3.72-3.59(m, 2H), 2.35-2.20 (m, 2H), 1.58-1.19 (m, 6H); ³¹P NMR (CDCl₃) δ 28.2 and 26.2; MS (ESI) 458 (M+Na).

### Example 9B

Compound **32:** Compound **31** was purified using a Dynamax SD-200 HPLC system. The mobile phase consisted of acetonitrile: water (60:40, v/v) at a flow rate of 70 mL/ min. The injection volume was 3 mL. The detection was by fluorescence at 245 nm and peak area ratios were used for quantitations. Retention time was 8.1 min for compound **32** as yellow oil. ¹H NMR (CDCl₃) δ 7.33-7.18 (m, 10H), 5.94-6.63 (m, 1H), 5.70 (m, 1H), 5.12-4.95 (m, 3H), 4.24-4.14 (m, 2H), 3.72-3.59(m, 2H), 2.35-2.20 (m, 2H), 1.58-1.19 (m, 6H); ³¹P NMR (CDCl₃) δ 28.2; MS (ESI) 458 (M+Na).

### Example 9C

Compound **33** was purified in the same manner and retention time was 7.9 min for compound 33 as yellow oil. ¹H NMR (CDCl₃) δ 7.33-7.18 (m, 10H), 5.94-6.63 (m, 1H), 5.70 (m, 1H), 5.12-4.95 (m, 3H), 4.24-4.14 (m, 2H), 3.72-3.59(m, 2H), 2.35-2.20 (m, 2H), 1.58-1.19 (m, 6H); ³¹P NMR (CDCl₃) δ 26.2; MS (ESI) 458 (M+Na).

### Example 9D

Compound **34:** A solution of compound 33 (3.2 g) was dissolved in 60 mL of EtOH, then 0.9 mL of acetic acid and 10 % Pd/C (640 mg) was added. The resulted mixture was stirred under H₂ atmosphere (balloon) for 4 h. After filtration through celite, the filtered was evaporated under reduced pressure to afford the compound **34** (2.7 g, 99 %) as a colorless oil. ¹H NMR (CDCl₃) δ 7.42-7.18 (m, 5H), 6.10 (s, 1H), 5.15-5.02 (m, 1H), 4.24-4.05 (m, 2H), 3.25-3.16 (m, 2H), 2.36-2.21 (m, 2H), 1.61-1.58 (m, 3H), 1.35-1.18, m, 3H); ³¹P NMR (CDCl₃) δ 26.1; MS (ESI) 302 (M+1).

### Example 9E

Compound **35:** To a stirred solution of compound **16** (8.9 g, 15 mmol) in 70 mL of 1,2-dichloroethane was added compound **34** (8.3 g, 23 mmol) and MgSO₄ (80 mg), the resulted mixture was stirred at room temperature under argon for 2.5h, then acetic acid (3 mL, 52.5 mmol) and sodium cyanoborohydride (1.9g, 30 mmol) were added. The reaction mixture was stirred at room temperature for 1.5 h under argon. Then aqueous NaHCO₃ (100 mL) was added, and the mixture was extracted with ethyl acetate, and the combined organic layers were washed with brine and water, dried over Na₂SO₄, filtered and concentrated. The residue was purified by chromatography on silica gel (EtOH/EtOAc, 1/9) to give **35** (8.4 g, 64 %) as a white solid. ¹H NMR (CDCl₃) δ 7.73 (d, J = 8.7 Hz, 2H), 7.36-7.17(m, 9H), 7.00 (d, J = 8.7 Hz, 2H), 5.64 (d, J = 5.1 Hz, 1H), 5.07-4.97 (m, 3H), 4.19 -3.67 (m overlapping s, 13H), 3.15-2.78 (m, 9H), 2.25-2.19 (m, 2H), 1.91-1.54 (m, 6H), 1.24-1.20 (m, 3H), 0.94-0.87 (m, 6H); ³¹P NMR (CDCl₃) δ 27.4; MS (ESI) 876 (M+1).

### Resolution of Compound 35 Diastereomers

Analysis was performed on an analytical Daicel Chiralcel OD column, conditions described below, with a total of about 3.5 mg compound **35** free base injected onto the column. This lot was about a 3:1 mixture of major to minor diastereomers where the lactate ester carbon is a 3:1 mix of R and S configurations.
Two injections of 3.8 and 3.5 mg each were made using the conditions described below. The isolated major diastereomer fractions were evaporated to dryness on a rotary evaporator under house vacuum. The chromatographic solvents were displaced by two portions of ethyl acetate followed by a single portion of ethyl acetate - trifluoroacetic acid (about 95:5) and a final high vacuum strip to aid in removal of trace solvents. This yielded the major diastereomer trifluoroacetate salt as a gummy solid.

**The resolved minor diastereomer was isolated for biological evaluation by an 11 mg injection, performed on an analytical Daicel Chiralcel OD column, using the conditions described in below. The minor diastereomer of 35 was isolated as the trifluoroacetate salt by the conditions described above.**

Larger scale injections (- 300 mg **35** per injection) were later performed on a Daicel Chiralcel OD column semi-preparative column with a guard column, conditions described below. A minimal quantity of isopropyl alcohol was added to heptane to dissolve the 3:1 diastereomeric mix of **35** and the resolved diastereomers sample, and the isolated fractions were refrigerated until the eluted mobile phase was stripped.

### Analytical Column, ~ 4 mg Injection, Heptane - EtOH (20:80) Initial

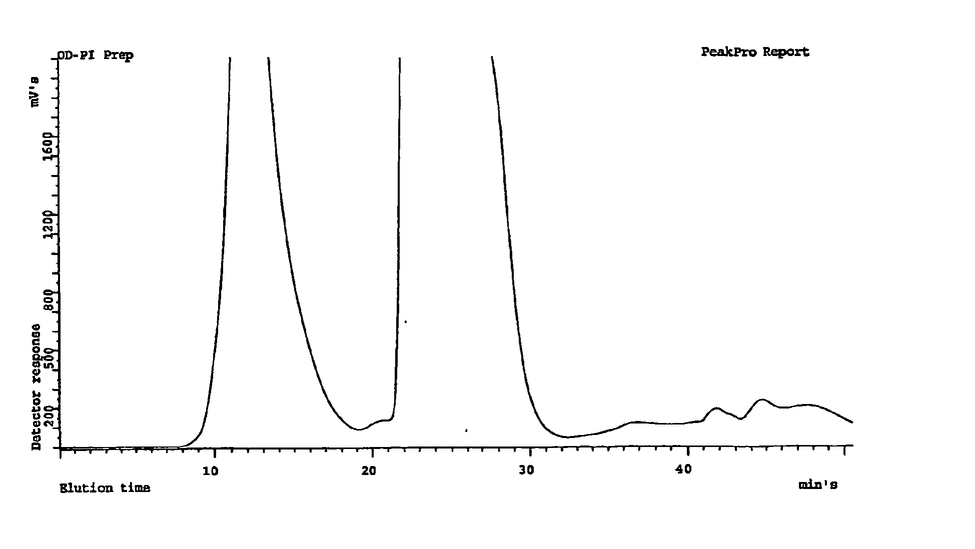

### HPLC CONDITIONS

- Column: : Chiralcel OD, 10 µm, 4.6 x 250 mm
- Mobile Phase: : Heptane - Ethyl Alcohol (20:80 initial)
: 100% Ethyl Alcohol (final)
**Note: Final began after first peak eluted**
- Flow Rate: : 1.0 mL/min
- Run Time: : As needed
- Detection: : UV at 250 nm
- Temperature: : Ambient
- Injection: : ~ 4 mg on Column
- Sample Prep.: : Dissolved in ~ 1 mL heptane -
ethyl alcohol (50:50)
- Retention Times: : 35 Minor ~ 14 min
: 35 Major ~ 25 min

### Analytical Column. ~ 6 mg Injection, Heptane - EtOH (65:35) Initial

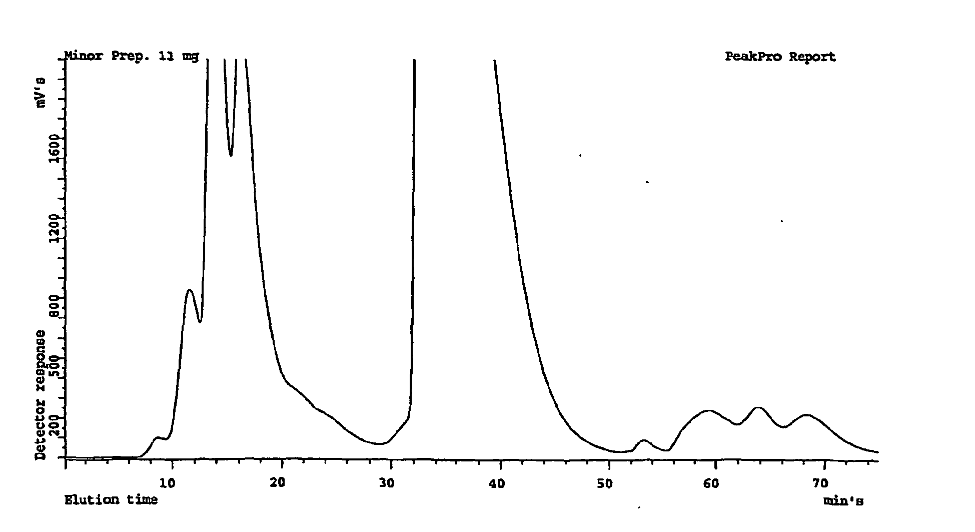

### HPLC CONDITIONS

- Column: : Chiralcel OD, 10 µm, 4.6 x 250 mm
- Mobile Phase: : Heptane - Ethyl Alcohol (65:35 initial)
: Heptane - Ethyl Alcohol (57.5:42.5 intermediate)
Note: Intermediate began after impurity peaks eluted
: Heptane - Ethyl Alcohol (20:80 final)
Note: Final mobile phase began after minor
diastereomer eluted
- Flow Rate: : 1.0 mL/min
- Run Time: : As needed
- Detection: : UV at 250 nm
- Temperature: : Ambient
- Injection: : ~ 4 mg on Column
- Sample Prep.: : Dissolved in ~ 1 mL heptane -
ethyl alcohol (50:50)
- Retention Times: : 35 Minor ~ 14 min
: 35 Major ~ 40 min

### Semi-Preparative Column. ~ 300 mg Injection, Heptane - EtOH (65:35) Initial

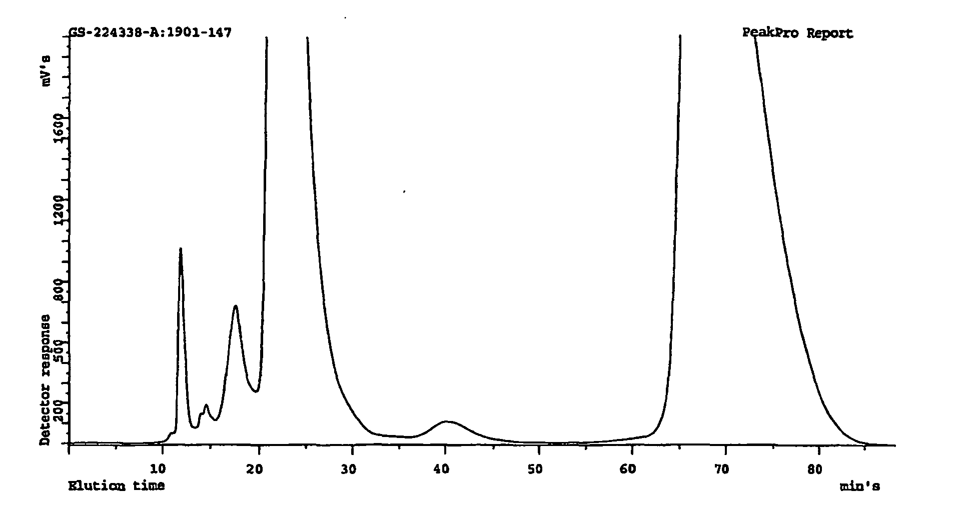

### HPLC CONDITIONS

- Columns: : Chiralcel OD, 20 µm, 21 x 50 mm (guard)
: Chiralcel OD, 20 µm, 21 x 250 mm
- Mobile Phase: : Heptane - Ethyl Alcohol (65:35 initial)
: Heptane - Ethyl Alcohol (50:50 intermediate)
Note: Intermediate began after minor diastereomer peak eluted
: Heptane - Ethyl Alcohol (20:80 final)
Note: Final mobile phase began after major diastereomer began to elute
- Flow Rate: : 10.0 mL/min
- Run Time: : As needed
- Detection: : UV at 260 nm
- Temperature: : Ambient
- Injection: : ~ 300 mg on Column
- Sample Prep.: : Dissolved in ~ 3.5 mL hetpane -
ethyl alcohol (70:30)
- Retention Times: : 35 Minor ~ 14 min
: 35 Major ~ 40 min

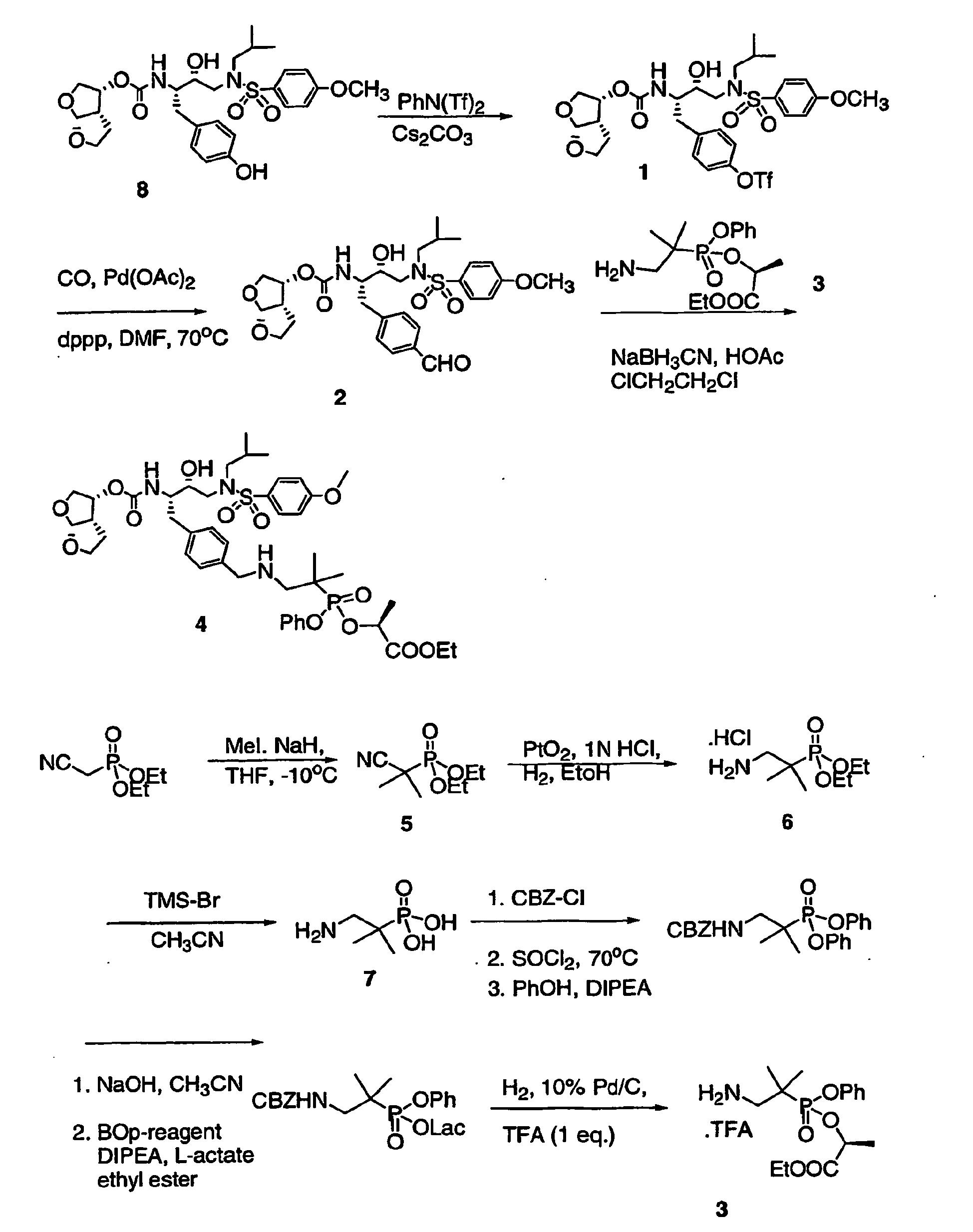

### Example 29

Triflate derivative 1: A THF-CH₂Cl₂ solution (30mL-10 mL) of **8** (4 g, 6.9 mmol), cesium carbonate (2.7 g, 8 mmol), and N-phenyltrifluoromethane sulfonimide (2.8 g, 8 mmol) was reacted overnight. The reaction mixture was worked up, and concentrated to dryness to give crude triflate derivative **1.**

Aldehyde 2: Crude triflate **1** (4.5 g, 6.9 mmol) was dissolved in DMF (20 mL), and the solution was degassed (high vacuum for 2 min, Ar purge, repeat 3 times). Pd(OAc)2 (0.12 g, 0.27 mmol), and bis(diphenylphosphino)propane (dppp, 0.22 g, 0.27 mmol) were added, the solution was heated to 70°C. Carbon monoxide was rapidly bubbled through the solution, then under 1 atmosphere of carbon monoxide. To this solution were slowly added TEA (5.4 mL, 38 mmol), and triethylsilane (3 ml), 18 mmol). The resulting solution was stirred overnight at room temperature. The reaction mixture was worked up, and purified on silica gel column chromatograph to afford aldehyde 2 (2.1 g, 51 %). (Hostetler, et al J. Org. Chem., 1999. 64, 178-185).

Lactate prodrug **4:** Compound 4 is prepared as described above procedure for Example 9E, Compound 35 by the reductive amination between 2 and 3 with NaBH₃CN in 1,2-dichloroethane in the presence of HOAc.

### Example 30 Preparation of Compound 3

Diethyl (cyano(dimethyl)methyl) phosphonate **5:** A THF solution (30 mL) of NaH (3.4 g of 60% oil dispersion, 85 mmol) was cooled to -10°C, followed by the addition of diethyl (cyanomethyl)phosphonate (5g, 28.2 mmol) and iodomethane (17 g, 112 mmol). The resulting solution was stirred at -10°C for 2 hr, then 0°C for 1 hr, was worked up, and purified to give dimethyl derivative 5 (5 g, 86 %).
Dietyl (2-amino-1,1-dimethyl-ethyl)phosphonate **6:** Compound **5** was reduced to amine derivative **6** by the described procedure (J. Med. Chem. 1999, 42, 5010-5019). A solution of ethanol (150 mL) and 1N HCl aqueous solution (22 mL) of 5 (2.2 g, 10.7 mmol) was hydrogenated at 1 atmosphere in the presence of PtO₂ (1.25 g) at room temperature overnight. The catalyst was filtered through a celite pad. The filtrate was concentrated to dryness, to give crude **6** (2.5g, as HCl salt).

2-Amino-1,1-dimethyl-ethyl phosphonic acid **7:** A solution of CH₃CN (30 mL) of crude **6** (2.5 g) was cooled to 0°C, and treated with TMSBr (8 g, 52 mmol) for 5 hr. The reaction mixture was stirred with methanol for 1.5 hr at room temperature, concentrated, recharged with methanol, concentrated to dryness to give crude **7** which was used for next reaction without further purification.

Lactate phenyl (2-amino-1,1-dimethyl-ethyl)phosphonate **3:** Compound 3 is synthesized according to the procedures described in Example 9D, Compound 34 for the preparation of lactate phenyl 2-aminoethyl phosphonate 34. Compound **7** is protected with CBZ, followed by the reaction with thionyl chloride at 70°C. The CBZ protected dichlorodate is reacted phenol in the presence of DIPEA. Removal of one phenol, follow by coupling with ethyl L-lactate leads N-CBZ-2-amino-1,1-dimethyl-ethyl phosphonate derivative. Hydrogenation of N-CBZ derivative at 1 atmosphere in the presence of 10 % Pd/C and 1 eq. of TFA affords compound **3** as TFA salt.

### Example Section Q

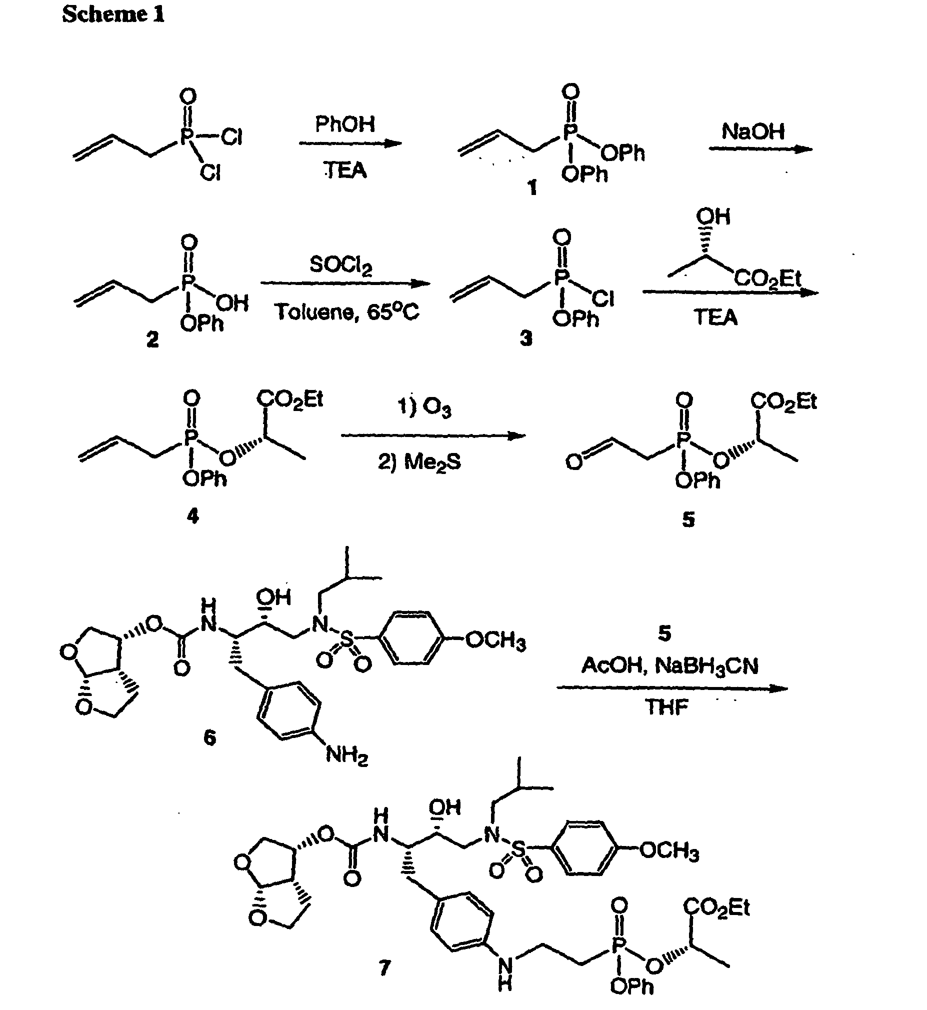

### Example 1

Monophenol Allylphosphonate 2: To a solution of anylphosphonic dichloride (4 g, 25.4 mmol) and phenol (5.2 g, 55.3 mmol) in CH₂Cl₂ (40 mL) at 0°C was added TEA (8.4 mL, 60 mmol). After stirred at room temperature for 1.5 h, the mixture was diluted with hexane-ethyl acetate and washed with HCl (0.3 N) and water. The organic phase was dried over MgSO₄, filtered and concentrated under reduced pressure. The residue was filtered through a pad of silica gel (eluted with 2:1 hexane-ethyl acetate) to afford crude product diphenol allylphosphonate **1** (7.8 g, containing the excessive phenol) as an oil which was used directly without any further purification. The crude material was dissolved in CH₃CN (60 mL), and NaOH (4.4N, 15 mL) was added at 0°C. The resulted mixture was stirred at room temperature for 3 h, then neutralized with acetic acid to pH = 8 and concentrated under reduced pressure to remove most of the acetonitrile. The residue was dissolved in water (50 mL) and washed with CH₂Cl₂ (3X25 mL). The aqueous phase was acidified with concentrated HCl at 0°C and extracted with ethyl acetate. The organic phase was dried over MgSO₄, filtered, evaporated and co-evaporated with toluene under reduced pressure to yield desired monophenol allylphosphonate 2 (4.75 g. 95%) as an oil.

### Example 2

Monolactate Allylphosphonate **4:** To a solution of monophenol allylphosphonate 2 (4.75 g, 24 mmol) in toluene (30 mL) was added SOCl₂ (5 mL, 68 mmol) and DMF (0.05 mL). After stirred at 65°C for 4 h, the reaction was completed as shown by ³¹P NMR. The reaction mixture was evaporated and co-evaporated with toluene under reduced pressure to give mono chloride 3 (5.5 g) as an oil. To a solution of chloride 3 in CH₂Cl₂ (25 mL) at 0°C was added ethyl (s)-lactate (3.3 mL, 28.8 mmol), followed by TEA. The mixture was stirred at 0°C for 5 min then at room temperature for 1 h, and concentrated under reduced pressure. The residue was partitioned between ethyl acetate and HCl (0.2N), the organic phase was washed with water, dried over MgSO₄, filtered and concentrated under reduced pressure. The residue was purified by chromatography on silica gel to afford desired monolactate **4** (5.75 g, 80%) as an oil (2:1 mixture of two isomers): ¹H NMR (CDCl₃) δ 7.1-7.4 (m, 5H), 5.9 (m, 1H), 5.3 (m, 2H), 5.0 (m, 1H), 4.2 (m, 2H), 2.9 (m, 2H), 1.6; 1.4 (d, 3H), 1.25 (m, 3H); ³¹P NMR (CDCl₃) δ 25.4, 23.9.

### Example 3

Aldehyde **5:** A solution of allylphosphonate **4** (2.5 g, 8.38 mmol) in CH₂Cl₂ (30 mL) was bubbled with ozone air at -78°C until the solution became blue, then bubbled with nitrogen until the blue color disappeared. Methyl sulfide (3 mL) was added at -78°C. The mixture was warmed up to room temperature, stirred for 16 h and concentrated under reduced pressure to give desired aldehyde **5** (3.2 g, as a 1:1 mixture of DMSO): ¹H NMR (CDCl₃) δ 9.8 (m, 1H), 7.1-7.4 (m, 5H), 5.0 (m, 1H), 4.2 (m, 2H), 3.4 (m, 2H), 1.6; 1.4 (d, 3H), 1.25 (m, 3H); ³¹P NMR (CDCl₃) δ 17.7, 15.4.

### Example 4

Compound **7:** To a solution of aniline **6** (reported before) (1.62 g, 2.81 mmol) in THF (40 mL) was added acetic acid (0.8 mL, 14 mmol), followed by aldehyde **5** (1.3 g, 80%, 3.46 mmol) and MgSO₄ (3 g). The mixture was stirred at room temperature for 0.5 h, then NaBH₃CN (0.4 g, 6.37 mmol) was added. After stirred for 1 h, the reaction mixture was filtered. The filtrate was diluted with ethyl acetate and washed with NaHC0₃, dried over MgSO₄, filtered and concentrated under reduced pressure. The residue was purified by chromatography on silica gel to give compound 6 (1.1g, 45%) as a 3:2 mixture of two isomers, which were separated by HPLC (mobile phase, 70% CH₃CN/H₂O; flow rate: 70 mL/min; detection: 254 nm; column: 8µ C18, 41X250 mm, Varian). Isomer A (0.39 g): ¹H NMR (CDCl₃) δ 7.75 (d, 2H), 7.1-7.4 (m, 5H), 7.0 (m, 4H), 6.6 (d, 2H), 5.65 (d, 1H), 5.05 (m, 2H), 4.9 (d, 1H), 4.3 (brs, 1H), 4.2 (q, 2H), 3.5-4.0 (m, 6H), 3.9 (s, 3H), 2.6-3.2 (m, 9H), 2.3 (m, 2), 1.6-1.9 (m, 5H), 1.25 (t, 3H), 0.9 (2d, 6H); ³¹P NMR (CDCl₃) δ 26.5; MS (ESI): 862 (M+H). Isomer B (0.59 g): ¹H NMR (CDCl₃) δ 7.75 (d, 2H), 7.1-7.4 (m, 5H); 7.0 (m, 4H), 6.6 (d, 2H), 5.65 (d, 1H), 5.05 (m, 2H), 4.9 (d, 1H), 4.5 (brs, 1H), 4.2 (q, 2H), 3.5-4.0 (m, 6H), 3.9 (s, 3H), 2.7-3.2 (m, 9H), 2.4 (m, 2), 1.6-1.9 (m, 2H), 1.4 (d, 3H), 1.25 (t, 3H), 0.9 (2d, 6H); ³¹P NMR (CDCl₃) δ 28.4; MS (ESI): 862 (M+H).

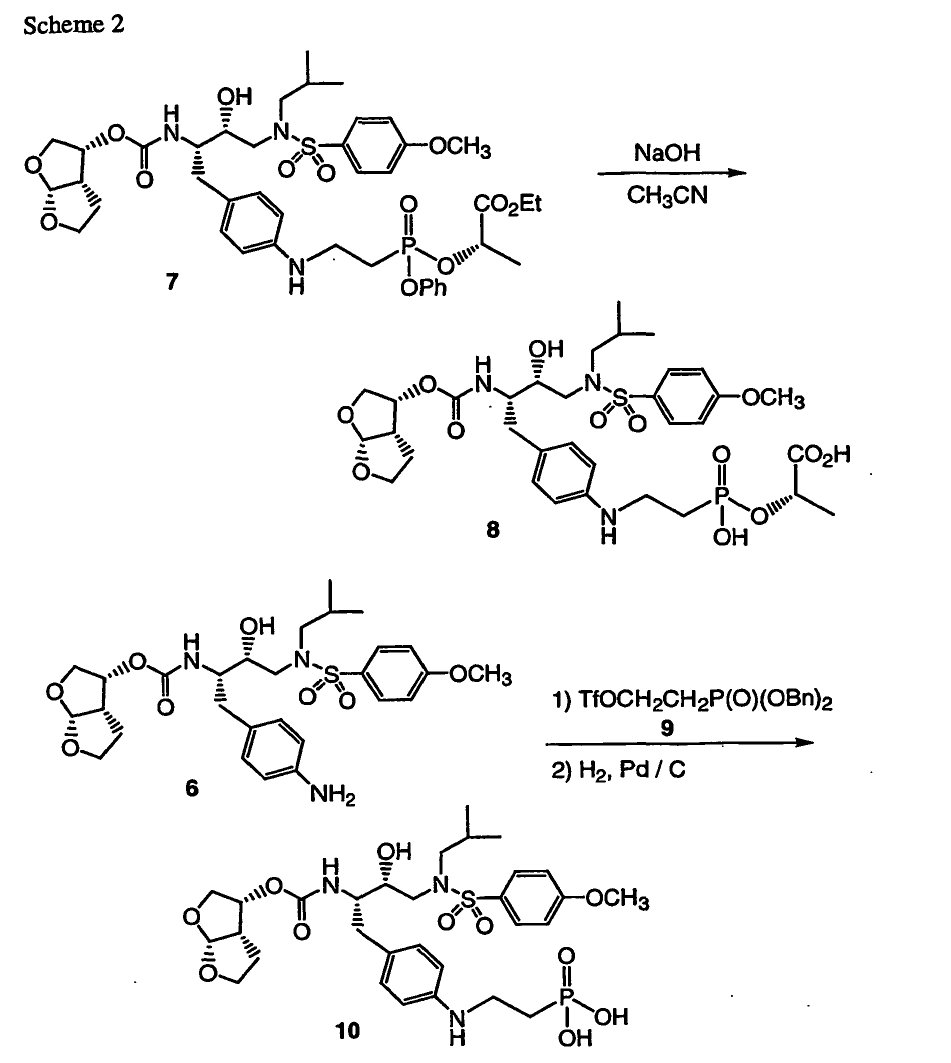

### Example 5

Acid **8:** To a solution of compound **7** (25 mg, 0.029 mmol) in acetonitrile (1 mL) at 0°C was added NaOH (1N, 0.125 mL). The mixture was stirred at 0°C for 0.5 h and at room temperature for 1 h. The reaction was quenched with acetic acid and purified by HPLC to give acid **8** (10 mg, 45%). ¹H NMR (CD₃OD) δ 7.8 (d, 2H), 7.5 (d, 2H), 7.4 (d, 2H), 7.1 (d, 2H), 5.6 (d, 1H), 4.9 (m, 3H), 3.2-4.0 (m, 6H), 3.9 (s, 3H), 2.6-3.2 (m, 9H), 2.05 (m, 2), 1.4-1.7 (m, 2H), 1.5 (d, 3H), 0.9 (2d, 6H); ³¹P NMR (CD₃OD) δ 20.6; MS (ESI): 758 (M+H).

### Example 6

Diacid **10:** To a solution of triflate **9** (94 mg, 0.214 mmol) in CH₂Cl₂ (2 mL) was added a solution of aniline 6 (100 mg, 0.173 mmol) in CH₂Cl₂ (2 mL) at -40°C, followed by 2,6-lutidine (0.026 mL). The mixture was warmed up to room temperature and stirred for 1 h. Cesium carbonate (60 mg) was added and the reaction mixture was stirred for additional 1 h. The mixture was diluted with ethyl acetate, washed with HCl (0.2N), dried over MgSO₄, filtered and concentrated under reduced pressure. The residue was purified by HPLC to afford dibenzyl phosphonate (40 mg). To a solution of this dibenzyl phosphonate in ethanol (3 mL) and ethyl acetate (1 mL) was added 10% Pd/C (40 mg). The mixture was stirred under hydrogen atmosphere (balloon) for 4 h. The reaction mixture was diluted with methanol, filtered and concentrated under reduced pressure. The residue was washed with ethyl acetate and dried to give desired product diacid **10** (20 mg). ¹H NMR (CD₃OD) δ 7.8 (d, 2H), 7.3 (d, 2H), 7.1 (2d, 4H), 5.6 (d, 1H), 4.9 (m, 2H), 3.4-4.0 (m, 6H), 3.9 (s, 3H), 2.5-3.2 (m, 9H), 2.0 (m, 2), 1.4-1.7 (m, 2H), 0.9 (2d, 6H); ³¹P NMR (CD₃OD) δ 22.1; MS (ESI): 686 (M+H).

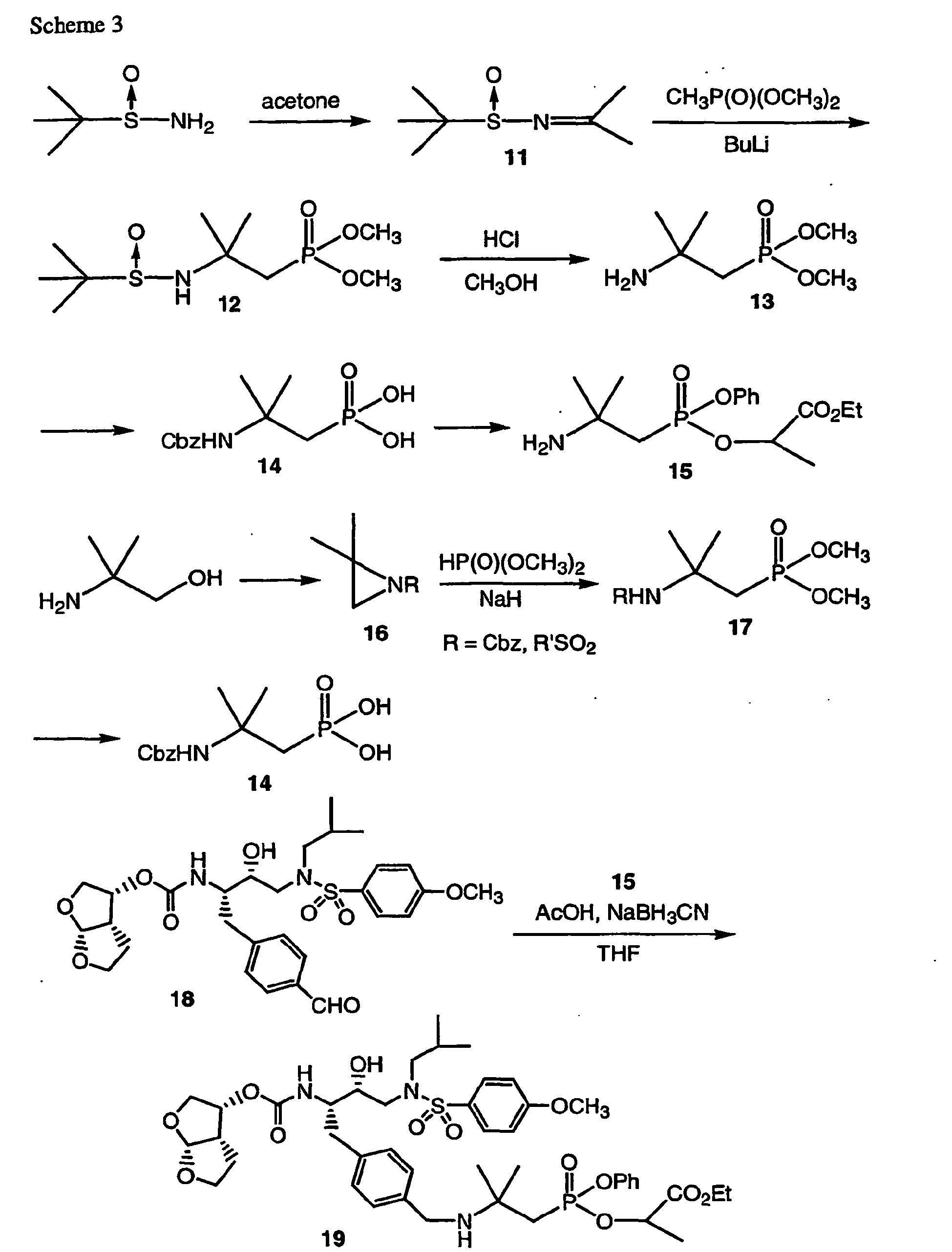

The synthesis of compound **19** is outlined in Scheme 3. Condensation of 2-methyl-2-propanesulfinamide with acetone give sulfinyl imine **11** (J. Org. Chem 1999, 64, 12). Addition of dimethyl methylphosphonate lithium to **11** afford **12.** Acidic methanolysis of **12** provide amine **13.** Protection of amine with Cbz group and removal of methyl groups yield phosphonic acid **14,** which can be converted to desired **15** using methods reported earlier on. An alternative synthesis of compound **14** is also shown in Scheme 3. Commercially available 2-amino-2-methyl-l-propanol is converted to aziridines **16** according to literature methods (J. Org. Chem. 1992, 57, 5813; and Syn. Lett. 1997, 8, 893). Aziridine opening with phosphite give **17** (Tetrahedron Lett. 1980, 21, 1623). Deprotection (and, if necessary, reprotection) of **17** afford **14.** Reductive amination of amine **15** and aldehyde **18** provides compound **19.**

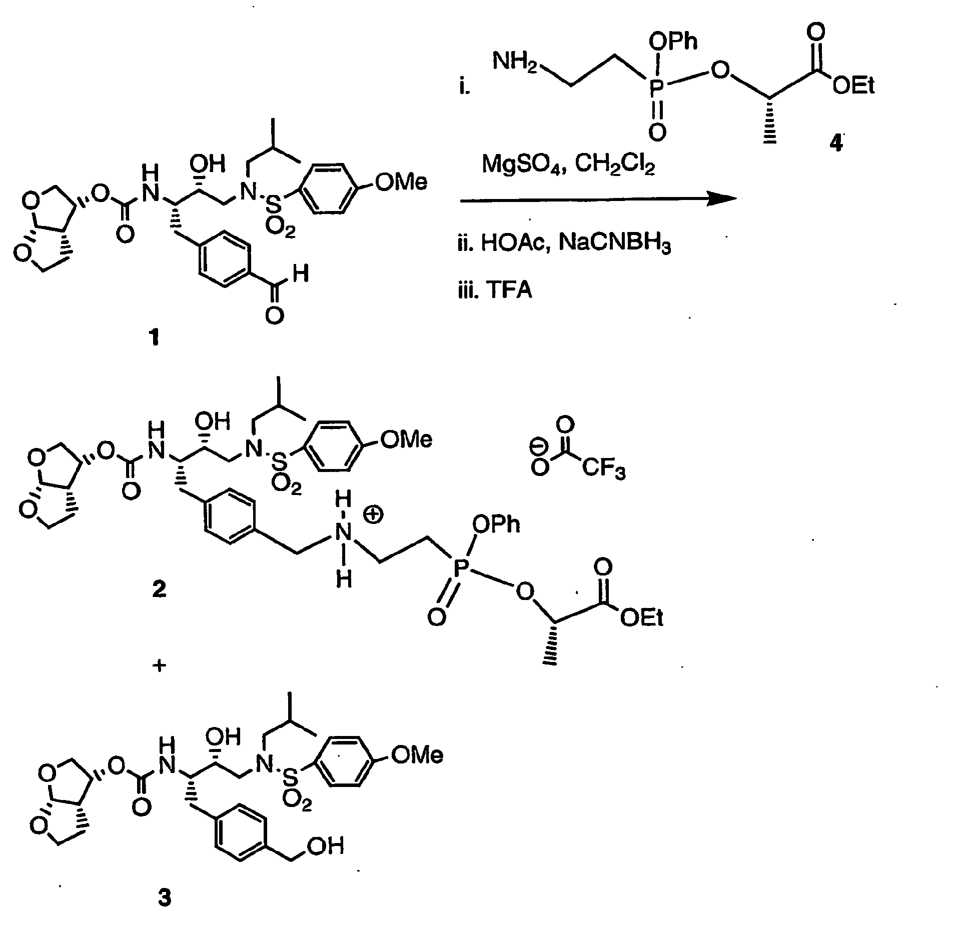

### Example section R

### Example 1

2- {[2-(4- {2-(Hexahydro-furo[2,3-b]furan-3-yloxycarbonylamino)-3-hydroxy-4-[isobutyl-(4-methoxy-benzenesulfonyl)-amino]-butyl}-benzylamino)-ethyl]-phenoxy-phosphinoyloxy}-propionic acid ethyl ester 2 (Compound 35, previous Example 9E).

A solution of **1** (2.07 g, 3.51 mmol) and 4 (1.33 g, 3.68 mmol of a 4:1 mixture of two diastereomers at the phosphorous center) were dissolved in 14 mL of (CH₂Cl₂)₂ to provide a clear solution. Addition of MgSO₄ (100 mg) to the solution resulted in a white cloudy mixture. The solution was stirred at ambient temperature for 3 hours when acetic acid (0.80 mL. 14.0 mmol) and sodium cyanoborohydride (441 mg, 7.01 mmol) were added. Following the reaction progress by TLC showed complete consumption of the aldehyde starting materials in 1 hour. The reaction mixture was worked up by addition of 200 mL of saturated aqueous NaHCO₃ and 400 mL of CH₂Cl₂. The aqueous layer was extracted with CH₂Cl₂ two more times (2 x 300 mL). The combined organic extracts were dried *in vacuo* and purified by column chromatography (EtOAc- 10% MeOH: EtOAc) to provide the desired product as a foam. The early eluting compound from the column was collected and characterized as alcohol 3 (810 mg, 39%). Addition of TFA (3 x 1 mL) generated the TFA salt which was lyopholized from 50 mL of a 1:1 CH₃CN: H₂O to provide 1.63 g (47%) of the product 2 as a white powder. ¹H NMR (CD₃CN) δ 8.23 (br s, 2H), 7.79 (d, *J=* 8.4 Hz, 2H), 7.45- 7.13 (m, 9H), 7.09 (d, *J*= 8.4 Hz, 2H), 5.86 (d, *J*= 9.0 Hz, 1H), 5.55 (d, *J*= 4.8 Hz, 1H), 5.05-4.96 (m, 1H), 4.96- 4.88 (m, 1H), 4.30-4.15 (m, 4H), 3.89 (s, 3H), 3.86- 3.76 (m, 4H), 3.70- 3.59 (m, 4H), 3.56- 3.40 (m, 2H), 3.34 (d, *J*= 15 Hz, 1H), 3.13 (d, *J*= 13.5 Hz, 1H), 3.06- 2.93 (m, 2H), 2.92- 2.80 (m, 2H), 2.69- 2.43 (m 3H), 2.03- 1.86 (m, 1H), 1.64- 1.48 (m, 1H), 1.53 and 1.40 (d, *J*= 6.3 Hz, *J=* 6.6 Hz, 3H), 1.45- 1.35 (m, 1H), 1.27 and 1.23 (t, *J*= 6.9 Hz, *J=* 7.2 Hz, 3H), 0.90 (t, *J*= 6.9 Hz, 6H). ³¹P NMR (CD₃CN) δ 24.47, 22.86. ESI (M+ H)⁺ 876.4.

### Example 2

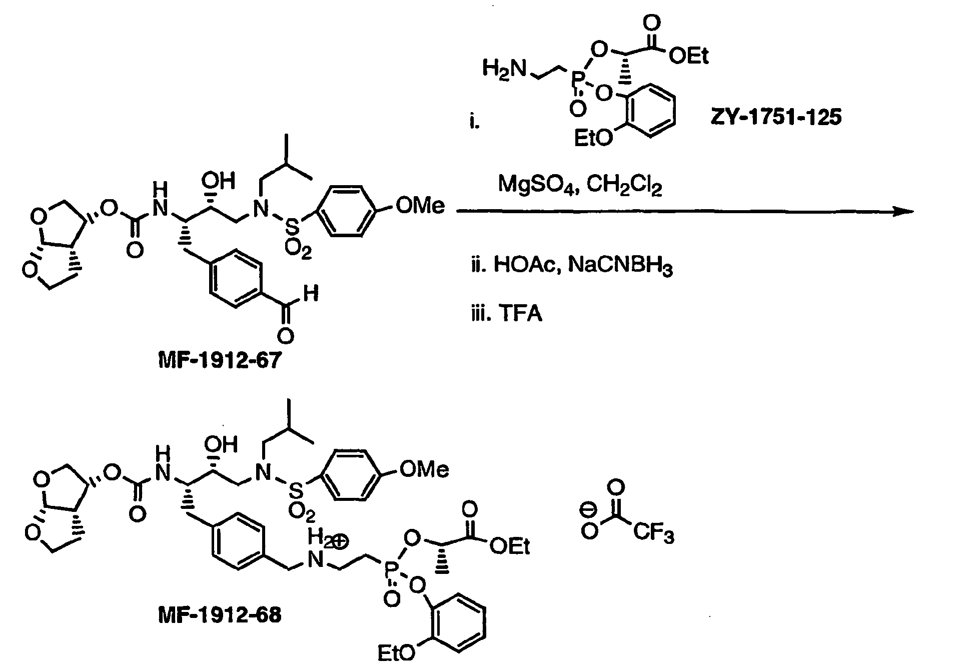

2- {[2-(4- {2-(Hexahydro-furo [2,3-b] furan-3-yloxycarbonylamino)-3-hydroxy-4-[isobutyl-(4-methoxy-benzenesulfonyl)-amino]-butyl}-benzylamino)-ethyl]-phenoxy-phosphinoyloxy}-propionic acid ethyl ester **(MF-1912-68):**

A solution of **MF-1912-67** (0.466 g, 0.789 mmol) and **ZY-1751-125** (0.320 g, 0.789 mmol of a 1:1 mixture of two diastereomers at the phosphorous center) were dissolved in 3.1 mL of (CH₂Cl)₂ to provide a clear solution. Addition of MgSO₄ (20 mg) to the solution resulted in a white cloudy mixture. The solution was stirred at ambient temperature for 3 hours when acetic acid (0.181 mL, 3.16 mmol) and sodium cyanoborohydride (99 mg, 1.58 mmol) were added. Following the reaction progress by TLC showed complete consumption of the aldehyde starting materials in 1.5 hour. The reaction mixture was worked up by addition of 50 mL of saturated aqueous NaHCO₃ and 200 mL of CH₂Cl₂. The aqueous layer was extracted with CH₂Cl₂ two more times (2 x 200 mL). The combined organic extracts were dried *in vacuo* and purified by column chromatography (EtOAc- 10% MeOH: EtOAc) to provide the desired product as a foam The early eluting compound from the column was collected and characterized to be MF-1912-48b alcohol (190 mg, 41%). Addition of TFA (3 x 1 mL) generated the TFA salt which was lyopholized from 50 mL of a 1:1 CH₃CN: H₂O to provide 0.389 g (48%) of the product as a white powder. ¹H NMR (CD3CN) δ 8.39 (br s, 2H), 7.79 (d, *J=* 8.7 Hz, 2H), 7.40 (d, *J=* 7.5 Hz, 2H), 7.34 (d, *J=* 8.1 Hz, 2H), 7.26-7.16 (m, 2H), 7.10 (d, *J=* 9 Hz, 3H), 7.01- 6.92 (m, 1H), 5.78 (d, *J=* 9.0 Hz, 1H), 5.55 (d, *J=* 5.1 Hz, 1H), 5.25-5.03 (m, 1H), 4.95- 4.88 (m, 1H), 4.30- 4.17 (m, 4H), 4.16- 4.07 (m, 2H), 3.90 (s, 3H), 3.88-3.73 (m, 4H), 3.72- 3.60 (m, 2H), 3.57- 3.38 (m, 2H), 3.32 (br d, *J=* 15.3 Hz, 1H), 3.13 (br d, *J=* 14.7 Hz, 1H), 3.05- 2.92 (m, 2H), 2.92- 2.78 (m, 2H), 2.68- 2.48 (m, 3H), 2.03-1.90 (m, 1H), 1.62-1:51 (m, 1H), 1.57 and 1.46 (d, *J=* 6.9 Hz, *J=* 6.9 Hz, 3H), 1.36- 1.50 (m, 1H), 1.43- 1.35 (m, 4H), 1.33- 1.22 (m, 3H), 0.91 (t, *J*= 6.6 Hz, 6H). ³¹P NMR (CD₃CN) δ 25.27, 23.56. ESI (M+ H)⁺ 920.5.

### Example Section S

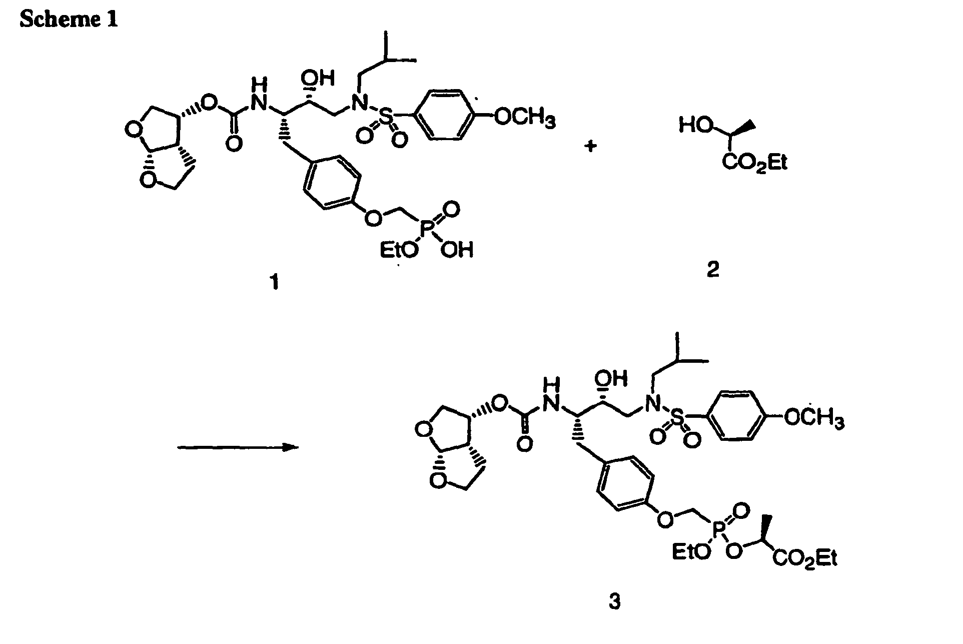

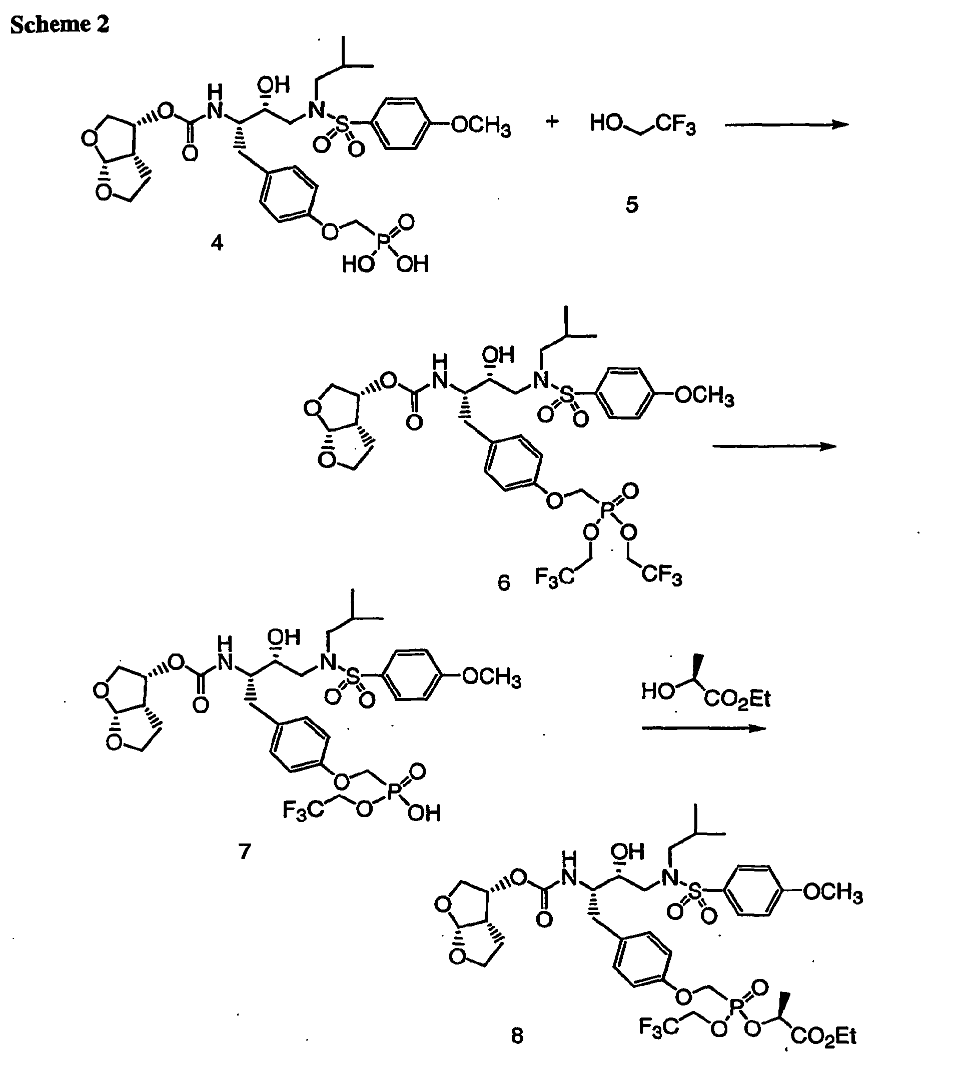

### Example 1

Mono-Ethyl mono-lactate 3: To a solution of 1 (96mg, 0.137 mmol) and ethyl lactate 2 (0.31 1 mL, 2.7 mmol) in pyridine (2 mL) was added N, N-dicyclohexylcarbodiimide (170 mg, 0.822 mmol). The solution was stirred for 18h at 70°C. The mixture was cooled to room temperature and diluted with dichloromethane. The solid was removed by filtration and the filtrate was concentrated. The residue was suspended in diethyl ether/dichloromethane and filtered again. The filtrate was concentrated and mixture was chromatographed on silica gel eluting with EtOAc/hexane to provide compound 3 (43 mg, 40%) as a foam: ¹H NMR (CDCl₃) δ 7.71 (d, 2H), 7.00 (d, 2H); 7.00 (d, 2H), 6.88 (d, 2H), 5.67 (d, 1H), 4.93-5.07 (m, 2H), 4.15-4.39 (m, 6H), 3.70-3.99 (m, 10H), 2.76-3.13 (m, 7H), 1.55-1.85 (m, 9H), 1.23-1.41 (m, 6H), 0.90 (dd, 6H); ³¹P NMR (CDCl₃) δ 19.1, 20.2; MS (ESI) 823 (M+Na).

### Example 2

Bis-2,2,2-trifluoroethyl phosphonate 6: To a solution of 4 (154mg, 0.228 mmol) and 222,-trifluoroethanol 5 (1 mL, 13.7 mmol) in pyridine (3 mL) was added N, N-dicyclohexylcarbodiimide (283 mg, 1.37 mmol). The solution was stirred for 6.5h at 70°C. The mixture was cooled to room temperature and diluted with dichloromethane. The solid was removed by filtration and the filtrate was concentrated. The residue was suspended in dichloromethane and filtered again. The filtrate was concentrated and mixture was chromatographed on silica gel eluting with EtOAc/hexane to provide compound 6 (133 mg, 70%) as a foam: ¹H NMR (CDCl₃) δ 7.71 (d, 2H), 7.21 (d, 2H); 7.00 (d, 2H), 6.88 (dd, 2H), 5.66 (d, 1H), 4.94-5.10 (m, 3H), 4.39-4.56 (m, 6H), 3.71-4.00 (m, 10H), 2.77-3.18 (m, 7H), 1.67-1.83(m, 2H), 0.91 (dd, 4H); ³¹P NMR (CDCl₃) δ 22.2; MS (ESI) 859 (M+Na).

### Example 3

Mono-2,2,2-trifluoroethyl phosphonate 7: To a solution of 6 (930mg, 1.11 mmol) in THF (14 mL) and water (10 mL) was added an aqueous solution of NaOH in water (1N, 2.2 mL). The solution was stirred for 1h at 0°C. An excess amount of Dowex resin (H⁺) was added to until pH=1. The mixture was filtered and the filtrate was concentrated under reduced pressure. The concentrated solution was azeotroped with EtOAc/toluene three times and the white powder was dried *in vacuo* provide compound 7 (830 mg, 100%). ¹H NMR (CDCl₃) δ 7.71 (d, 2H), 7.11 (d, 2H); 6.99 (d, 2H), 6.85 (d, 2H), 5.63 (d, 1H), 5.26 (m, 1H), 5.02 (m, 1H), 4.40 (m, 1H), 4.14 (m, 4H), 3.60-3.95 (m, 12H), 2.62-3.15 (m, 15H), 1.45-1.84 (m, 3H), 1.29 (m, 4H), 0.89 (d, 6H); ³¹P NMR (CDCl₃) δ 19.9; MS (ESI) 723 (M+Na).

### Example 4

Mono-2,2,2-trifluoroethyl mono-lactate 8: To a solution of 7 (754mg, 1 mmol) and N, N-dicyclohexylcarbodiimide (1.237 g, 6 mmol) in pyridine (10 mL) was added ethyl lactate (2.26 mL, 20 mmol). The solution was stirred for 4.5h at 70°C. The mixture was concentrated and the residue was suspended in diethyl ether (5 mL) and dichloromethane (5 mL) and filtered. The solid was washed a few times with diethyl ether. The combined filtrate was concentrated and the crude product was chromatographed on silica gel, eluting with EtOAc and hexane to provide compound 8 (610 mg. 71%) as a foam. ¹H NMR (CDCl₃) δ 7.71 (d, 2H), 7.16 (d, 2H); 6.99 (d, 2H), 6.88 (dd, 2H), 5.66 (d, 1H). 4.95-5.09 (m, 2H). 4.19-4.65 (m, 6H), 3.71-4.00 (m 9H), 2.76-3.13 (m, 6H), 1.57-1.85 (m, 7H), 1.24-1.34 (m. 4H), 0.91 (dd, 6H); ³¹P NMR (CDCl₃) δ 20.29, 21.58 ; MS (ESI) 855 (M+1).

### Example Section T

### Example 1

Boc-protected hydroxylamine **1:** A solution of diethyl hydroxy methyl phosphonate triflate (0.582 g, 1.94 mmol) in dichloromethane (19.4 mL) was treated with triethylamine (0.541 mL, 3.88 mmol). Tert-butyl N-hydroxy-carbamate (0.284 g, 2.13 mmol) was added and the reaction mixture was stirred at room temperature overnight. The mixture was partitioned between dichloromethane and water. The organic phase was washed with saturated NaCl, dried (MgSO₄) and evaporated under reduced pressure. The crude product was purified by chromatography on silica gel (1/1- ethyl acetate/hexane) affording the BOC-protected hydroxylamine 1 (0.41 g, 75%) as an oil: ¹H NMR (CDCl₃) δ 7.83 (s, 1H), 4.21 (d, 2H), 4.18 (q, 4H), 1.47 (s, 9H), 1.36 (t, 6H); ³¹P NMR (CDCl₃) δ 19.3.

### Example 2

Hydroxylamine 2: A solution of BOC-protected hydroxylamine 1 (0.305 g, 1.08 mmol) in dichloromethane (2.40 mL) was treated with trifluoroacetic acid (0.829 mL, 10.8 mmol). The reaction was stirred for 1.5 hours at room temperature and then the volatiles were evaporated under reduced pressure with toluene to afford the hydroxylamine 2 (0.318 g, 100%) as the TFA salt which was used directly without any further purification: ¹H NMR (CDCl₃) δ 10.87 (s, 2H), 4.45 (d, 2H), 4.24 (q, 4H), 1.38 (t, 6H); ³¹P NMR (CDCl₃) δ 16.9; MS (ESI) 184 (M+H).

### Example 3

Oxime **4:** To a solution of aldehyde **3** (96 mg, 0.163 mmol) in 1,2-dichloroethane (0.65 mL) was added hydroxylamine **2** (72.5 mg, 0.244 mmol), triethylamine (22.7 µL, 0.163. mmol) and MgSO₄ (10 mg). The reaction mixture was stirred at room temperature for 2 hours then the mixture was partitioned between dichloromethane and water. The organic phase was washed with saturated NaCl, dried (MgSO₄) and evaporated under reduced pressure. The crude product was purified by chromatography on silica gel (90/10 - ethyl acetate/hexane) affording, GS-277771, oxime 4 (0.104 g, 85%) as a solid: ¹H NMR (CDCl₃) δ 8.13 (s, 1H), 7.72 (d, 2H), 7.51 (d, 2H), 7.27 (d, 2H), 7.00 (d, 2H), 5.67 (d, 1H), 5.02 (m, 2H), 4.54 (d, 2H), 4.21 (m, 4H), 3.92 (m, 1H), 3.89 (s, 3H), 3.88 (m, 1H), 3.97-3.71 (m, 2H), 3.85-3.70 (m, 2H), 3.16-2.99 (m, 2H), 3.16-2.81 (m, 7H), 1.84 (m, 1H), 1.64-1.48 (m, 2H), 1.37 (t, 6H), 0.94-0.90 (dd, 6H); ³¹P NMR (CDCl₃) δ 20.0; MS (ESI) 756 (M+H).

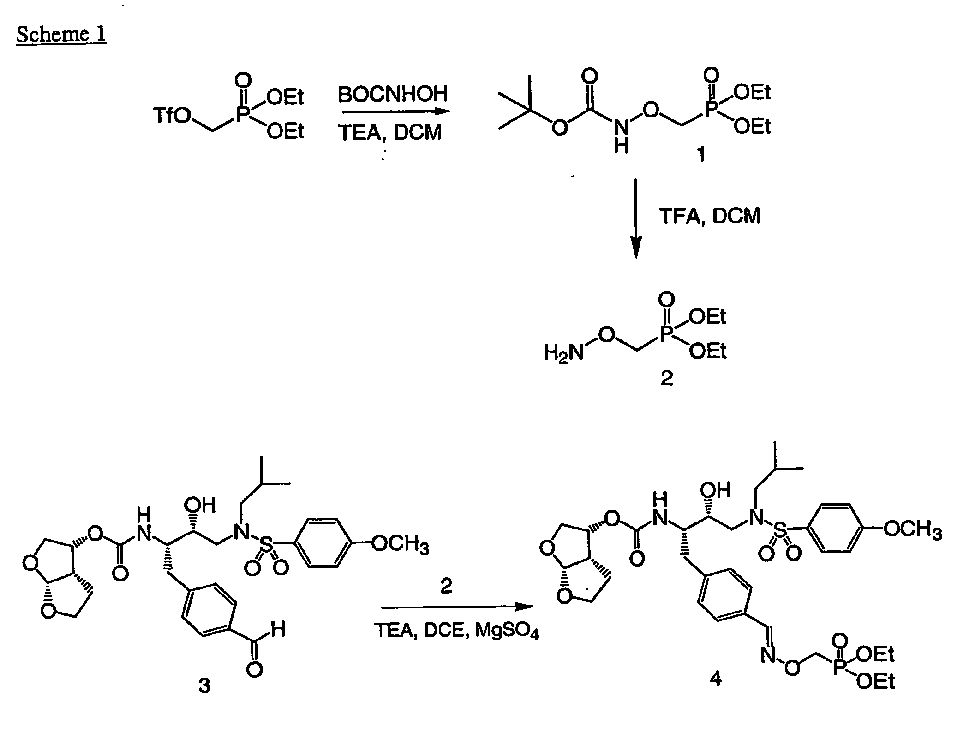

### Example Section U

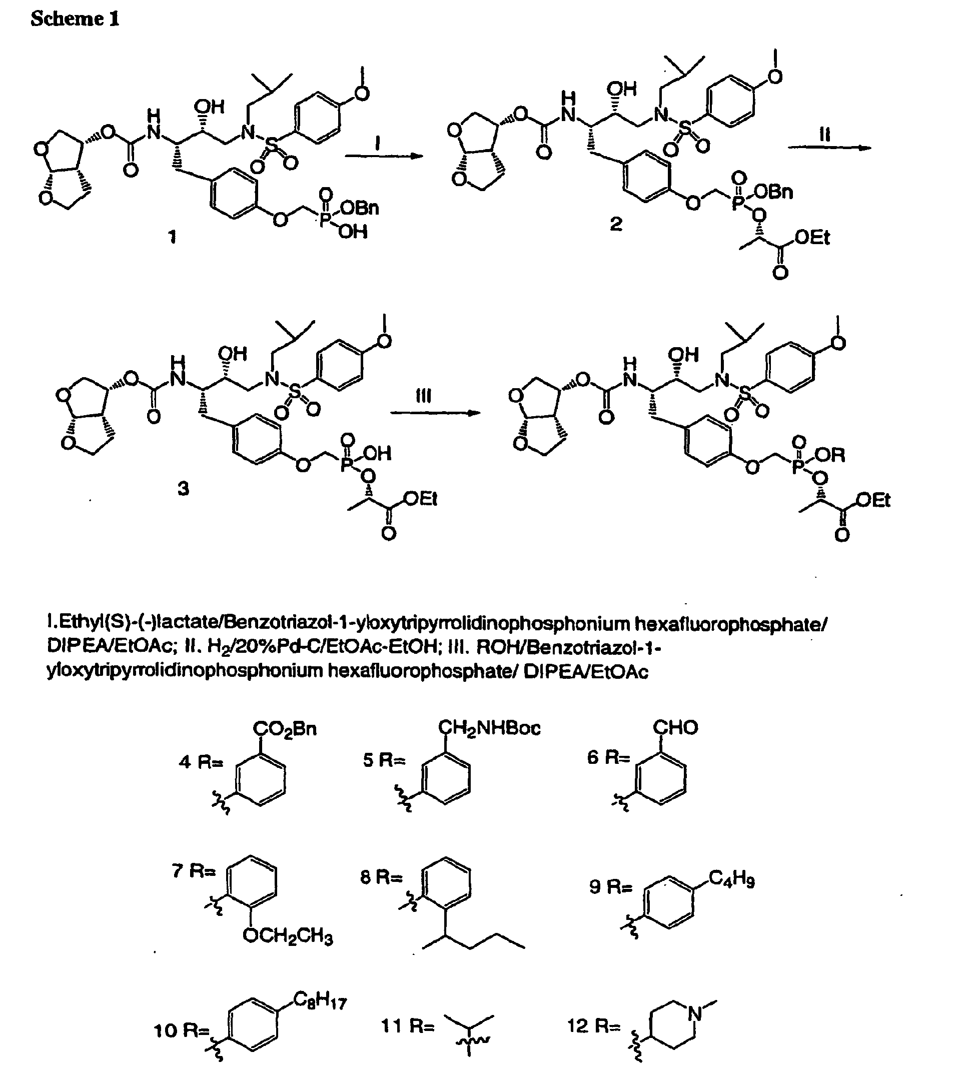

### Example 1

Compound 1 was prepared according to methods from previous Schemes

### Example 2

Compound 2: To a solution of compound 1 (5.50 g, 7.30 mmol), Benzotriazol-1-yloxytripyrrolidinophosphonium hexafluorophosphate (5.70g, 10.95 mmol), and Ethyl(S)-(-)lactate (1.30 g, 10.95 mmol) in DMF (50 mL) was added Diisopropylethylamine(5.08 mL, 29.2 mmol). The mixture was stirred for 7 hours after which was diluted in EtOAc. The organic phase was washed with H₂O (5X), brine, dried over MgSO₄ and *concentrated in vacuo.* The residue was purified by silica gel chromatography (CH₂Cl₂/Isopropanol= 100/4) to give 3.45 g of compound 2.

### Example 3

Compound 3: To the mixture of compound 2 (3.45 g) in EtOH/EtOAc (300 mL/100 mL) was added 20% Pd/C(0.700 g). The mixture was hydrogenated for 1 hour. Celite was added and the mixture was stirred for 10 minutes. The mixture was filtered through a pad of celite and washed with ethanol. Concentration gave 2.61 g of compound 3.

### Example 4

Compound 4: To a solution of compound 3 (1.00 g, 1.29 mmol) in dry dimethylformamide (5 mL) was added 3-Hydroxy-benzoic acid benzyl ester (0.589 g, 2.58 mmol), Benzotriazol-1-yloxytripyrrolidinophosphonium hexafluorophosphate (1.34 g, 2.58 mmol), followed by addition of Diisopropylethylamine (900 µL, 5.16 mmol). The mixture was stirred for 14 hours, the resulting residue was diluted in EtOAc, washed with brine (3x) and dried over sodium sulfate, filtered, and concentrated under reduced pressure. The residue was purified by silica gel chromatography (CH₂Cl₂/Isopropanol= 100/3) to provide 67.3 mg of compound 4: ¹H NMR (CDCl₃) δ 7.91 (2H,d, J=8.9 Hz), 7.75 (2H, m), 7.73-7.3 (13H,m), 7.25 (2H, m), 7.21-6.7(6H, m), 5.87(1H, m), 5.4-4.8(6H, m), 4.78-4.21 (4H, m), 3.98 (3H,s), 2.1-1.75 (8H, m), 1.55 (3H, m), 1.28(3H, m), 0.99(6H, m).

### Example 5

Compound 5: To a solution of compound 3 (1.40 g, 1.81 mmol) in dry dimethylformamide (5 mL) was added (4-Hydroxy-benzyl)-carbamic acid tert-butyl ester (0.80 g, 3.62 mmol), Benzotriazol-1-yloxytripyrrolidinophosphonium hexafluorophosphate (1.74 g, 3.62 mmol), followed by addition of Diisopropylethylamine (1.17 ml, 7.24 mmol). The mixture was stirred for 14 hours, the resulting residue was diluted in EtOAc, washed with brine (3x) and dried over sodium sulfate, filtered, and concentrated under reduced pressure. The residue was purified by silica gel chromatography (CH₂Cl₂/Isopropanol= 100/3.5) to provide 770 mg of compound 5: ¹H NMR (CDCl₃) δ 7.8(2H, d, J=8.9Hz), 7.4 (2H, m), 7.3-6.8 (8H, m), 5.75 (1H, m), 5.3-5.1(2H, m), 4.6-4.23 (4H,m), 3.98 (3H, s), 3.7-2.6 (15H, m), 2.2-1.8 (12H, m), 1.72 (3H, s), 1.58(3H, m), 1.25 (3H, m), 0.95 (6H, m).

### Example 6

Compound 6: To a solution of compound 3 (1.00 g, 1.29 mmol) in dry dimethylformamide (6 mL) was added 3-Hydroxybenzaldehyde (0.320 g, 2.60 mmol), Benzotriazol-1-yloxytripyrrolidinophosphonium hexafluorophosphate (1.35 g, 2.60 mmol), followed by addition of Diisopropylethylamine (901 µL, 5.16 mmol). The mixture was stirred for 14 hours, the resulting residue was diluted in EtOAc, washed with brine (3x) and dried over sodium sulfate, filtered, and concentrated under reduced pressure. The residue was purified by silica gel chromatography (CH₂Cl₂/Isopropanol= 100/5) to provide 880 mg of compound 6.

### Example 7

Compound 7: To a solution of compound 3 (150 mg, 0.190 mmol) in dry dimethylformamide (1 mL) was added 2-Ethoxy-phenol (48.0 µL, 0.380 mmol), Benzotriazol-1-yloxytripyrrolidinophosphonium hexafluorophosphate (198 mg, 0.380 mmol), followed by addition of Diisopropylethylamine (132 µL, 0.760 mmol). The mixture was stirred for 14 hours, the resulting residue was diluted in EtOAc, washed with brine (3x) and dried over sodium sulfate, filtered, and concentrated under reduced pressure. The residue was purified by silica gel chromatography (CH₂Cl₂/Isopropanol= 100/4) to provide 84.7 mg of compound 7: ¹H NMR (CDCl₃) δ 7.73 (2H, d, J=8.9 Hz), 7.15 (2H, m), 7.01-6.9 (8H, m), 5.66 (1H, m), 5.22-5.04 (2H, m), 4.56- 4.2 (6H, m), 4.08 (2H, m), 3.89 (3H, m), 3.85-3.69 (6H, m), 3.17-2.98 (7H, m), 2.80(3H, m) 1.86 (1H, m),1.65(2H, m), , 1.62-1.22 (6H, m), 0.92(6H, m).

### Example 8

Compound 8: To a solution of compound 3 (50.0 mg, 0.0650 mmol) in dry dimethylformamide (1 mL) was added 2-(1-methylbutyl) phenol (21.2 mg, 0.130 mmol), Benzotriazol-1-yloxytripyrrolidinophosphonium hexafluorophosphate (67.1 mg, 0.130 mmol), followed by addition of Diisopropylethylamine (45.0 µL, 0.260 mmol). The mixture was stirred for 14 hours, the resulting residue was diluted in EtOAc, washed with brine (3x) and dried over sodium sulfate, filtered, and concentrated under reduced pressure. The residue was purified by reversed phase HPLC to provide 8.20 mg of compound 8: ¹H NMR (CDCl₃) δ 7.73 (2H, d, J=8.9 Hz), 7.25 (2H, m), 7.21-6.89 (8H, m), 5.7(1H, m), 5.29-4.9 (2H, m), 4.56- 4.2 (6H, m), 3.89 (3H, m), 3.85-3.69 (6H, m), 3.17-2.89 (8H, m), 2.85(3H, m), 2.3-1.65(4H, m), 1.55-1.35 (6H, m), 0.92(6H, m).

### Example 9

Compound 9: To a solution of compound 3 (50.0 mg, 0.0650 mmol) in dry dimethylformamide (1 mL) was added) 4-N-Butylphenol (19.4 mg, 0.130 mmol), Benzotriazol-1-yloxytripyrrolidinophosphonium hexafluorophosphate (67.1 mg, 0.130 mmol), followed by addition (45.0 µL, 0.260 mmol) of Diisopropylethylamine. The mixture was stirred for 14 hours, the resulting residue was diluted in EtOAc, washed with brine (3x) and dried over sodium sulfate, filtered, and concentrated under reduced pressure. The residue was purified by reversed phase HPLC to provide 9.61 mg of compound 9: ¹H NMR (CDCl₃) δ 7.8(2H, d, J=8.9 Hz), 7.4 (2H, m), 7.3-6.8 (8H, m), 5.75 (1H, m), 5.3-4.5 (4H, m), 4.3-3.4.1 (4H, m), 3.9 (3H, m), 3.3-2.59 (11H, m), 2.25 (2H, m), 1.85-1.5 (5H, m), 1.4-1.1(10H, m), 0.95(9H, m).

### Example 10

Compound 10: To a solution of compound 3 (50.0 mg, 0.0650 mmol) in dry dimethylformamide (1 mL) was added 4-Octylphenol (26.6 mg, 0.130 mmol), Benzotriazol-1-yloxytripyrrolidinophosphonium hexafluorophosphate (67.1 mg, 0.130 mmol), followed by addition of Diisopropylethylamine (45.0 µL, 0.260 mmol). The mixture was stirred for 14 hours, the resulting residue was diluted in EtOAc, washed with brine (3x) and dried over sodium sulfate, filtered, and concentrated under reduced pressure. The residue was purified by reversed phase HPLC to provide 7.70 mg of compound 10: ¹H NMR (CDCl₃) δ 7.75 (2H, d, J=8.9 Hz), 7.3 (2H, m), 7.2-6.8 (8H, m), 5.70 (1H, m), 5.3-4.9 (4H, m), 4.6- 3.9 (4H, m), 3.89 (3H, m), 3.85-2.59 (12H, m), 2.18-1.75 (10H, m), 1.69-1.50 (8H, m), 1.4-1.27(6H,m), 0.95(9H, m).

### Example 11

Compound 11: To a solution of compound 3 (100 mg, 0.120 mmol) in dry dimethylformamide (1 mL) was added Isopropanol (20.0 µL, 0.240 mmol), Benzotriazol-1-yloxytripyrrolidinophosphonium hexafluorophosphate (135 mg, 0.240 mmol), followed by addition of Diisopropylethylamine (83.0 µL, 0.480 mmol). The mixture was stirred for 14 hours, the resulting residue was diluted in EtOAc, washed with brine (3x) and dried over sodium sulfate, filtered, and concentrated under reduced pressure. The residue was purified by silica gel chromatography (CH₂Cl₂/Isopropanol= 100/4) to provide 12.2 mg of compound 11: ¹H NMR (CDCl₃) δ 7.71 (2H, d, J=8.9 Hz), 7.15 (2H, m), 7.0 (2H, m), 6.89 (2H, m), 5.65 (1H, m), 5.03-4.86(4H, m), 4.34-4.19 (3H, m), 3.89 (3H, s), 3.88 (1H, m), 3.82 (2H, m), 3.65 (4H, m), 3.2-2.9 (11H, m), 2.80(3H, m) 1.65(2H, m), 1.86 (1H, m), 1.6(3H, m), 1.30(3H,m), 0.92(6H, m).

### Example 12

Compound 12: To a solution of compound 3 (100 mg, 0.120 mmol) in dry dimethylformamide (1mL) was added 4-Hyrdroxy-1-methylpiperidine (30.0 mg, 0.240 mmol), Benzotriazol-1-yloxytripyrrolidinophosphonium hexafluorophosphate (135 mg, 0.240 mmol), followed by addition of Diisopropylethylamine (83.0 µL, 0.480 mmol). The mixture was stirred for 14 hours, the resulting residue was diluted in EtOAc, washed with brine (3x) and dried over sodium sulfate, filtered, and concentrated under reduced pressure. The residue was purified by reversed phase HPLC to provide 50.1 mg of compound 12: ¹H NMR (CDCl₃) δ 7.73 (2H, d, J=8.9 Hz), 7.18 (2H, m), 7.0 (2H, m), 6.9 (2H, m), 5.67 (1H, m), 5.2-4.9 (4H, m), 4.30-4.11 (4H, m), 3.98 (1H, m), 3.89 (3H, s), 3.87 (1H, m), 3.75 (2H, m), 3.5-3.3 (4H, m), 3.2-2.9 (14H, m), 2.80(3H, m) 1.65(2H, m), 1.86 (1H, m), 1.6(3H, m), 1.30(3H,m), 0.92(6H, m).

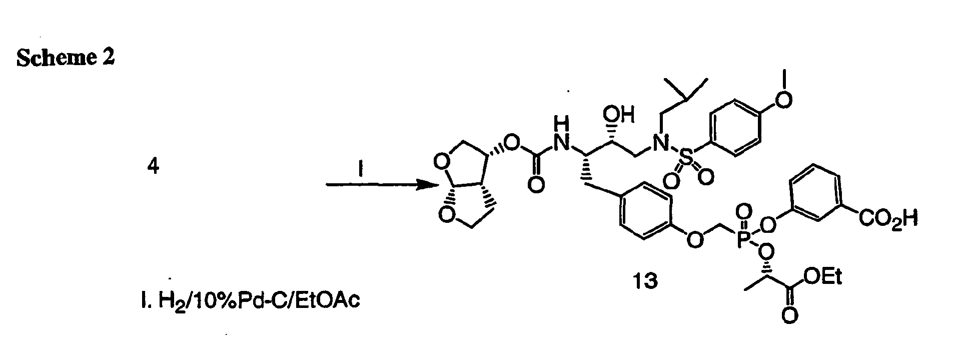

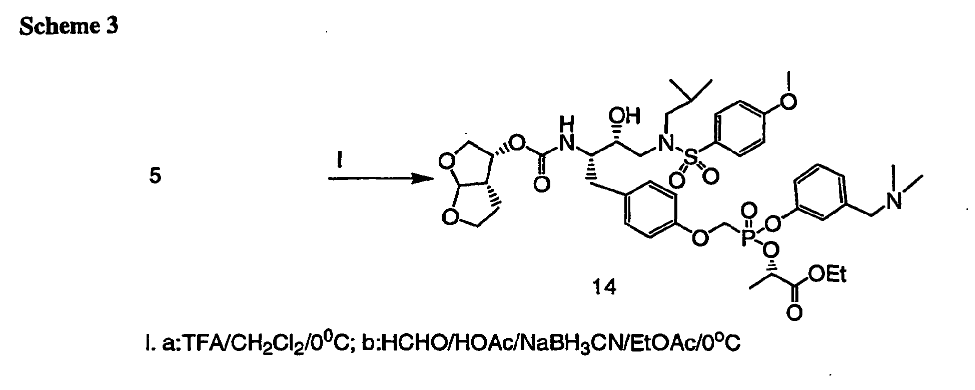

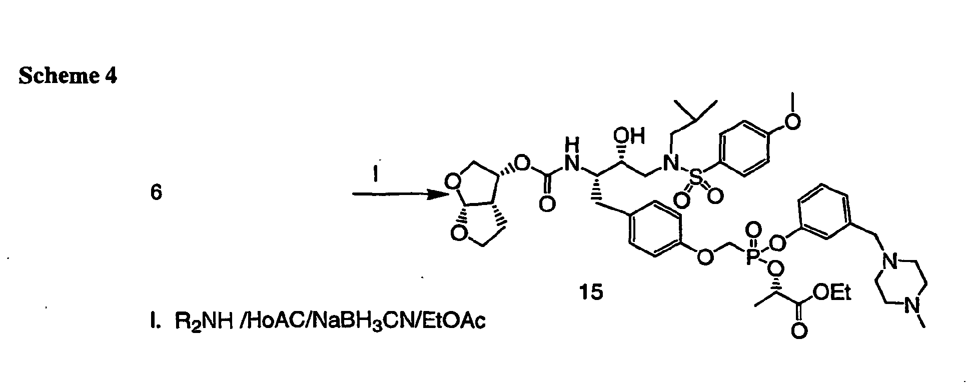

### Example 13

Compound 13: To a solution of compound 4 (4.9 g)) in EtOAc (150ml) was added 20% Pd/C (0.90 g),the reaction mixture was hydrogenated for 1 hour. Celite was added and the mixture was stirred for 10 minutes. The mixture was filtered through a pad of celite and washed with ethanol. Concentration gave 4.1 g of compound 13: ¹H NMR (CDCl₃) δ 7.91 (2H,d, J=8.9 Hz), 7.75 (2H, m), 7.73-7.3 (8H, m), 7.25 (2H, m), 7.21-6.7(6H, m), 5.4-4.8(6H, m), 4.78-4.21 (4H, m), 3.98 (3H,s), 2.1-1.75 (8H, m), 1.55 (3H, m), 1.28(3H, m), 0.99(6H, m).

### Example 14

Compound 14: To a solution of compound 5 (0.770 g, 0.790 mmol) in dichloromethane (10 mL), under ice-cooling, was added triflouroacetic acid (5 mL), the resulting mixture was stirred at 25°C for two hours. The reaction mixture was concentrated under reduced pressure and the residue was co-evaporated with EtOAc to provide an yellow oil. To a solution of the above oil in (10 mL) of EtOAc, under ice-cooling and stirring was added formaldehyde (210 µL, 2.86 mmol), acetic acid (252 µL, 4.30 mmol), followed by sodium cyanoborohydride (178 mg, 2.86 mmol). The mixture was further stirred at 25°C for 2 hours. The above mixture was concentrated and diluted with EtOAc and washed with H₂O (3X), brine, dried over sodium sulfate, filtered, and concentrated under reduced pressure. The residue was purified using reversed-phase HPLC to provide 420 mg of compound 14: ¹H NMR (CDCl₃) δ 7.8(2H, d, J=8.9Hz), 7.4 (2H, m), 7.3-6.8 (8H, m), 5.75 (1H, m), 5.3-5.1(2H, m), 4.6-4.23 (4H,m), 3.98 (3H, s), 3.7-2.6 (15H, m), 2.2-1.8 (8H, m), 1.72 (3H, s), 1.58(3H, m), 1.25 (3H, m), 0.95 (6H, m).

### Example 15

Compound 15: To a solution of compound 6 (100mg, 0.114 mmol) in EtOAc (1 mL) was added 1-Methyl-piperazine (63.2 mg, 0.570 mmol), acetic acid (34.0 µl, 0.570 mmol) followed by Sodium Cyanoborohydride (14.3 mg, 0.228mmol). The mixture was stirred at 25°C for 14 hours. The reaction mixture was concentrated and diluted with EtOAc and washed with H₂O (5X), brine (2x), dried over sodium sulfate, filtered, and concentrated under reduced pressure: The residue was purified using silica gel chromatography (CH₂Cl₂/Isopropanol= 100/6.5) to give 5.22 mg of compound 15: ¹H NMR (CDCl₃) δ 7.73 (2H, d, J=8.9 Hz), 7.4-7.18(8H, m), 7.1-6.89 (2H, m), 5.67 (1H, m), 5.2-4.9 (4H, m), 4.30-4.11 (4H, m), 3.98 (1H, m), 3.89 (3H, s), 3.87 (1H, m), 3.75 (2H, m), 3.5-3.3 (4H, m), 3.2-2.9 (10H, m), 2.80-2.25 (8H,m) 1.65(2H, m), 1.86 (1H, m), 1.6(3H, m), 1.30(3H,m), 0.92(6H, m).

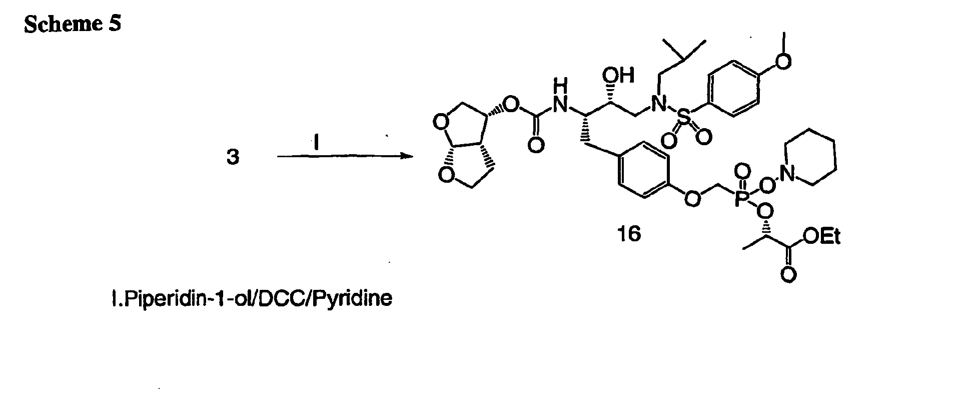

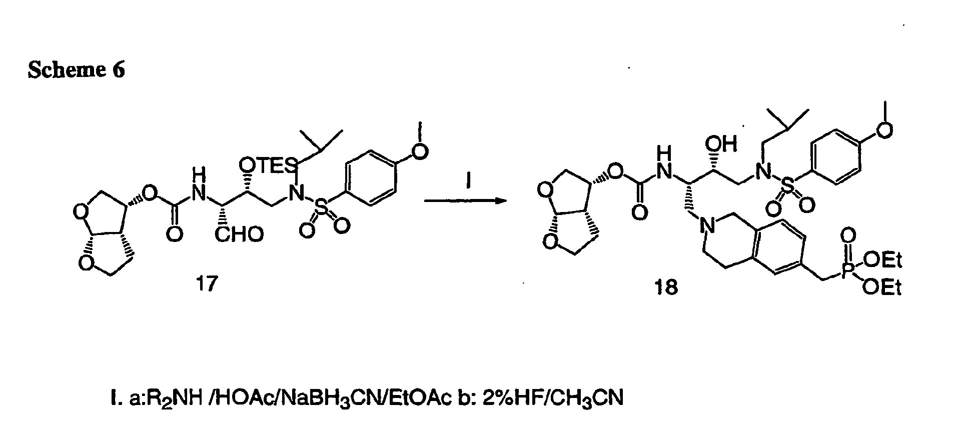

### Example 16

Compound 16: To a solution of compound 3 (100mg, 0.120 mmol) in Pyridine (600 µL) was added Piperidin-1-ol (48.5 mg, 0.480 mmol), followed by N,N-Dicyclohexylcarbodiimide (99.0 mg, 0.480 mmol). The mixture was stirred for 6 hours, the solvent was concentrated under reduced pressure. The resulting residue was purified by silica gel chromatography (CH₂Cl₂/Methanol= 100/5) to provide 17 mg of compound 16: ¹H NMR (CDCl₃) δ 7.73 (2H, d, J=8.9 Hz), 7.16 (2H, m), 7.0 (2H, m), 6.9 (2H, m), 5.68 (1H, m), 5.17 (1H, m), 5.04 (1H, m), 4.5-4.2 (4H, m), 3.90 (3H, s), 3.75 (2H, m), 3.5-3.3 (4H, m), 3.2-2.9 (10H, m), 2.80(3H, m) 1.65(2H, m), 1.86 (1H, m), 1.6(3H, m), 1.5-1.27 (9H,m), 0.92(6H, m).

### Example 17

Compound 18: To a solution of compound 17 (148 mg, 0.240 mmol) in 4 mL of Methanol was added (1,2,3,4-Tetrahydro-isoquinolin-6-ylmethyl)-phosphonic acid diethyl ester (70.0 mg, 0.240 mmol), acetic acid (43.0 µL, 0.720 mmol). The reaction mixture was stirred for 3 minutes, followed by addition of Sodium Cyanoborohydride (75.3 mg, 1.20 mmol). The reaction mixture was stirred at 25°C for 14 hours. The reaction mixture was diluted with EtOAc and washed with H₂O (3X), brine, dried over sodium sulfate, filtered, and concentrated under reduced pressure. The residue was purified using silica gel chromatography (CH₂Cl₂/Isopropanol= 100/5) to give 59 mg of TES protected intermediate. 83 µL of 48% HF solution was added to acetonitrile (4 mL) to prepare the 2% HF solution. The above 2% HF solution was added to TES protected intermediate (47 mg, 0.053 mmol) and the reaction mixture was stirred for 2 hours. The solvent was concentrated and the residue was diluted with EtOAc, dried over sodium sulfate, filtered, and concentrated under reduced pressure. The residue was purified using silica gel chromatography (CH₂Cl₂/Methanol= 100/10) to give 35.2 mg of compound 18: ¹H NMR (CDCl₃) δ 7.73 (2H, d, J=8.9 Hz), 7.05 (2H, m), 6.89 (2H, m), 6.76 (1H, m), 5.75 (1H, m), 5.67 (1H, m), 5.3 (2H, m), 4.2-3.6 (12 H, m), 3.4-2.4 (11 H, m), 2.1-1.8 (6H, m), 1.4-1.28 (8 H, m), 0.92(6H, m).

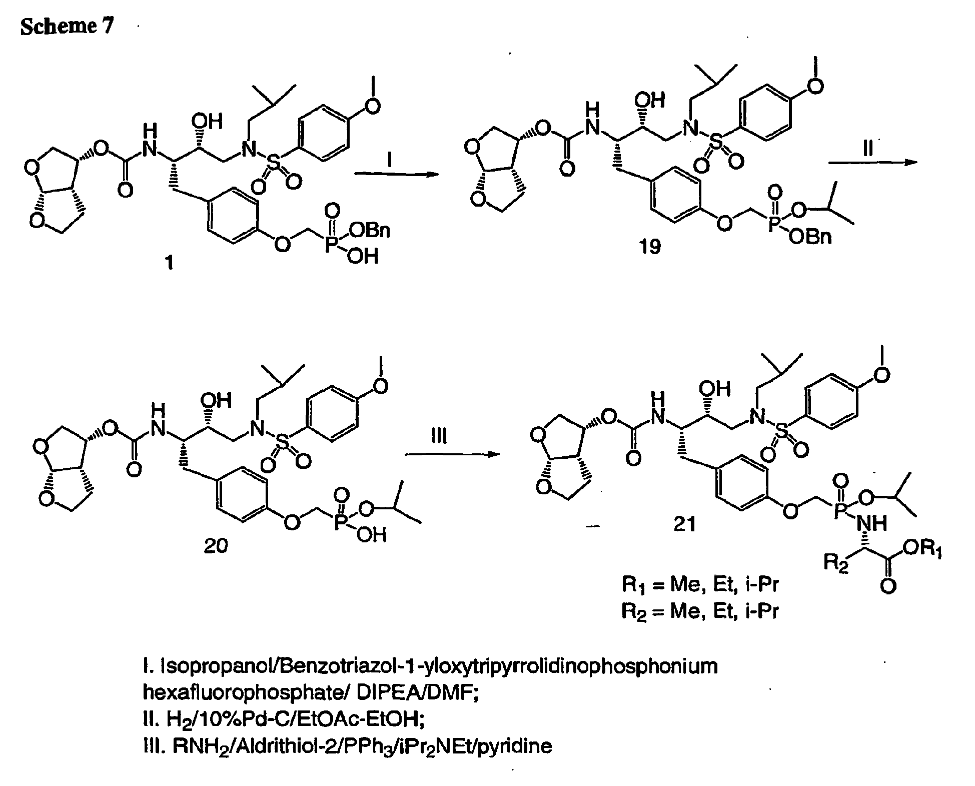

Compound 19 is prepared following the procedure for compound 2 by using monoacid 1. Compound 20 is made following a hydrogenation of compound 19. Mono acid 20 reacts with corresponding amino esters in the presence of Aldrithiol-2 and triphenylphosphine to form compound 21.

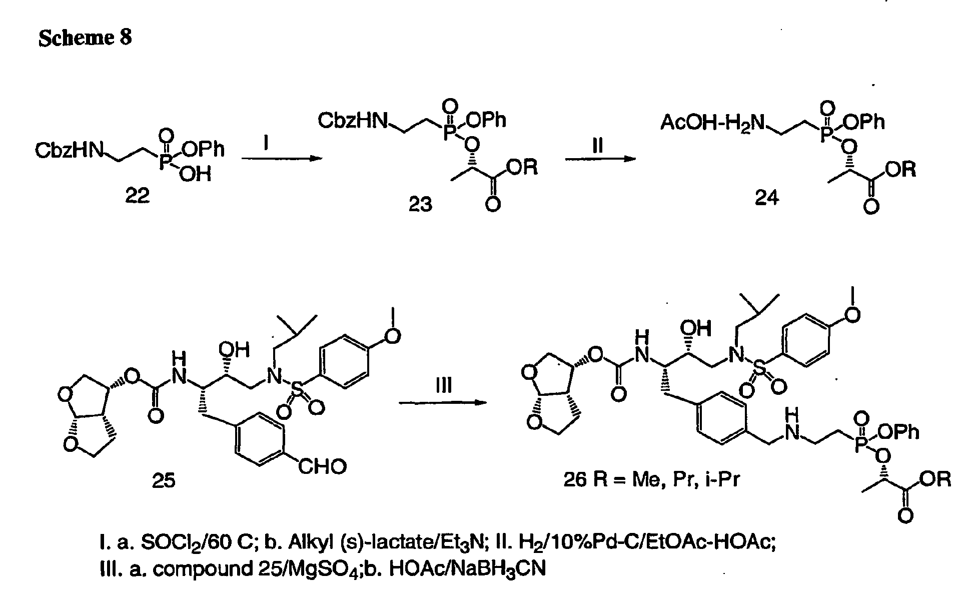

Monoacid 22 is treated with thionyl chloride at 60°C to form monochloridate, which reacts with corresponding alkyl (s)lactate to generate monolactate 23. Monolactate 23 is hydrogenated with 10%Pd-C in the presence of acetic acid to form amine 24. Aldehyde 25 reacts with amine 24 in the presence of MgSO₄ to form the intermediate imine, which is reduced with sodium cyanoborohydride to afford compound 26.

### Example Section V

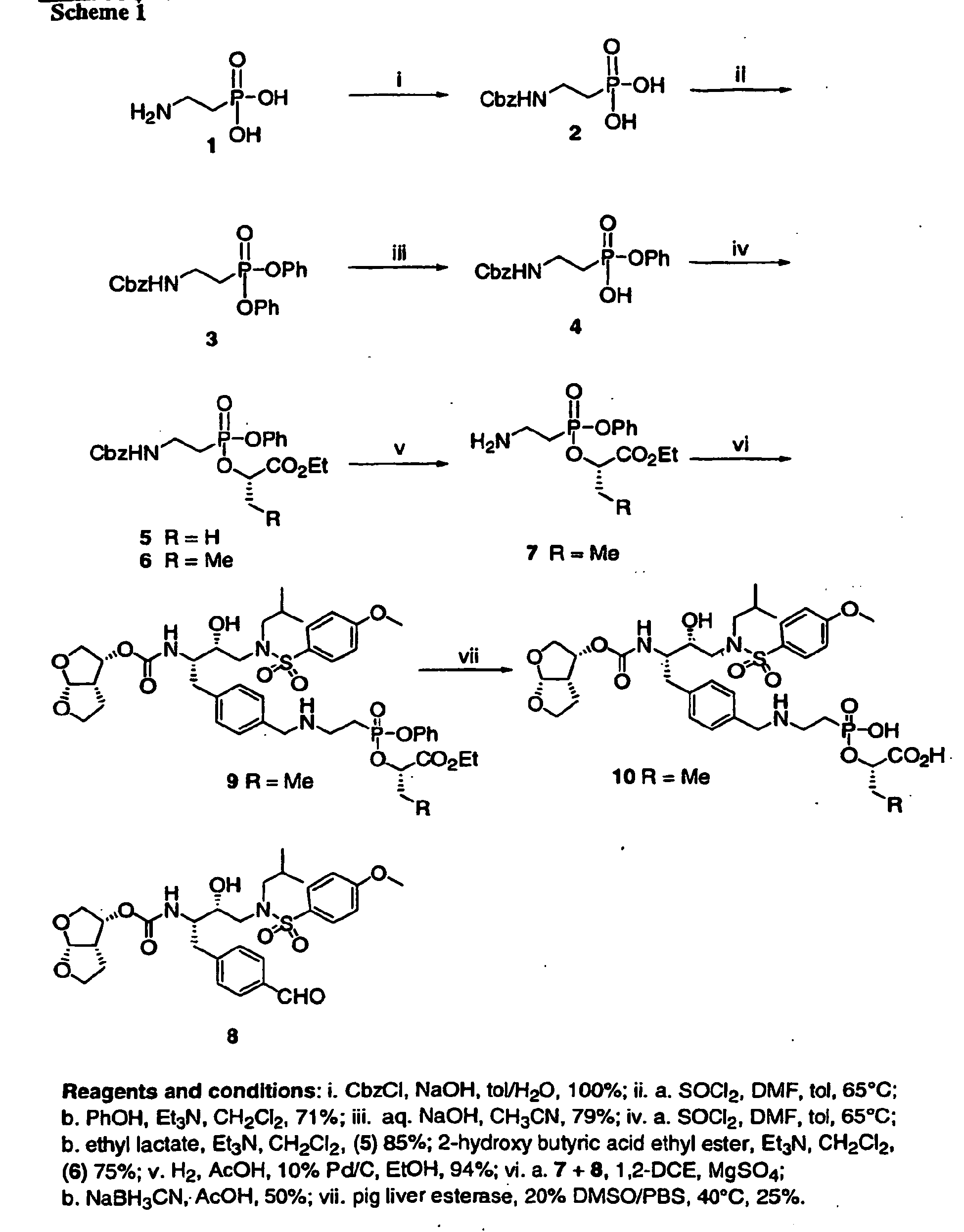

### Example 1

Compound 2: A 3L, 3-neck flask was equipped with a mechanical stirrer and addition funnel and charged with 2-aminoethyl phosphonic acid (60.0g, 480 mmol). 2N Sodium hydroxide (480 mL, 960 mmol) was added and flask cooled to 0°C. Benzyl chloroformate (102.4 g, 600 mmol) in toluene (160mL) was added dropwise with vigorous stirring. The reaction mixture was stirred at 0°C for 30 minutes, then at room temperature for 4 h. 2N sodium hydroxide (240 mL, 480 mmol) was added, followed by benzyl chloroformate (20.5 g, 120 mmol) and the reaction mixture was vigorously stirred for 12 h. The reaction mixture was washed with diethyl ether (3x). The aqueous layer was acidified to pH 2 with concentrated HCl to give a white precipitate. Ethyl acetate was added to the mixture and concentrated HCl (80 mL, 960 mmol) was added. The aqueous layer was extracted with ethyl acetate and combined organic layer was dried (MaSO₄) and concentrated to give a waxy, white solid (124 g, 479 mmol, 100%). ¹H NMR (300 MHz, CD₃OD) δ 7.45-7.30 (m, 5 H, Ar), 5.06 (d, *J* = 14.7 Hz, 2 H, C*H*₂Ph), 3.44-3.31 (m, 2 H, N*CH*₂CH₂), 2.03-1.91 (m, 2 H, CH₂*CH*₂P); ³¹P NMR (121 MHz, CD₃OD) : δ 26.3.

### Example 2

Compound 3: To a mixture of compound 2 (50.0 g, 193 mmol) in toluene (1.0 L) was added DMF (1.0 mL) followed by thionyl chloride (56 mL, 768 mmol). The reaction mixture was heated at 65°C for 3-4 h under a stream of argon. The reaction mixture was cooled to room temperature and concentrated. Residual solvent was removed under high vacuum for 1 h. The residue was dissolved in CH₂Cl₂ (1.0 L) and cooled to 0°C. Triethylamine (161 mL, 1158 mmol) was added, followed by phenol (54.5 g, 579 mmol). The reaction mixture was warmed to room temperature overnight, then washed with 1.0N HCl, saturated NaHCO₃ solution, brine and dried (MgSO₄). Concentrated and purified (silica gel, 1:1 EtOAc/Hex) to give a pale yellow solid (56 g, 136 mmol, 71%). ¹H NMR (300 MHz, CDCl₃): δ 7.40-7.10 (m, 15 H, Ar), 5.53 (br s, 1 H, N*H),* 5.11 (br s, 2 H, C*H*₂Ph), 3.72-3.60 (m, 2 H, NC*H*₂CH₂), 2.49-2.30 (m, 2 H, CH₂CH₂P); ³¹P NMR (121 MHz, CDCl₃): δ 22.9.

### Example 3

Compound 4: To a solution of compound **3** (64 g, 155.6 mmol) in acetonitrile (500 mL) at 0°C was added 2.0M sodium hydroxide. The reaction mixture was stirred at 0°C for 30 min, then at room temperature for 2.5 h. The reaction mixture was concentrated to 100 mL and diluted with H₂O (500 mL). The aqueous solution was washed with EtOAc (3 x 300 mL). The aqueous layer was acidified to pH 1 with concentrated HCl, producing a white precipitated. The mixture was extracted with EtOAc (4 x 300 mL) and combined organic layer was washed with brine and dried (MgSO₄). Concentration gave a solid, which was recrystallized from hot EtOAc (450 mL) to give a white solid (41.04 g, 122 mmol, 79%). ¹H NMR (300 MHz, CD₃OD): δ 7.45-7.10 (m, 10 H, Ar), 5.09 (s, 2 H, C*H*₂Ph), 3.53-3.30 (m, 2 H, NC*H*₂CH₂), 2.25-2.10 (m, 2 H, CH₂C*H*₂P); ³¹P NMR (121 MHz, CD₃OD): δ 24.5.

### Example 4

Compound 5: To a mixture of compound **4** (28 g, 83 mmol) in toluene (500 mL) was added DMF (1.0 mL), followed by thionyl chloride (36.4 mL, 499 mmol). The mixture was heated at 65°C for 2 h providing a pale yellow solution. The reaction mixture was concentrated and dried for 45 min under high vacuum The residue was dissolved in anhydrous CH₂Cl₂ (350 mL) and cooled to 0°C. Triethylamine (45.3 mL, 332 mmol) was added slowly, followed by the dropwise addition of ethyl lactate (18.8 mL, 166 mmol). The reaction mixture was stirred at 0°C for 30 min, then warmed to room temperature overnight. The reaction mixture was diluted with CH₂Cl₂ and washed with 1 N HCl, saturated NaHCO₃ solution, brine and dried (MgSO₄). Concentration and purification (silica gel, 1:5 to 1:0 EtOAc/Hex) gave a pale yellow oil (30.7 g, 71 mmol, 85%) as a mixture of diastereomers which were separated by HPLC (Dynamax reverse phase C-18 column, 60% acetonitrile/H₂O). More polar diastereomer: ¹H NMR (300 MHz, CDCl₃): δ 7.40-7.10 (m, 10 H, Ar), 5.65 (s, 1 H, N*H*), 5.12 (s, 2 H, C*H*₂Ph), 5.10-5.00 (m, 1 H, OC*H*C) 4.17 (q, *J* = 6.9 Hz, 2 H, OC*H*₂CH₃), 3.62 (dt, *J*₁ = 20.4 Hz, *J*₂ = 6.0 Hz, 2 H, NC*H*₂CH₂), 2.25 (dt, *J*₁ = 18.0 Hz, *J*₂ = 6.0 Hz, 2 H, CH₂C*H*₂P), 1.60 (dd, *J*₁ = *J*₂= 6.9 Hz, 3 H, CHC*H*₃), 1.23 (t, *J*= 6.9 Hz, 3 H, OCH₂CH₃); ³¹P NMR (121 MHz, CDCl₃): δ 26.2. Less polar diastereomer: ¹H NMR (300 MHz, CDCl₃): δ 7.40-7.10 (m, 10 H, Ar), 5.87 (s, 1 H, N*H*), 5.13 (s, 2 H, C*H*₂Ph), 5.10-5.00 (dq, *J*₁ = *J*₂ = 6.9 Hz, 1 H, OC*H*C) 4.22 (q, *J =* 7.2 Hz, 2 H, OC*H*₂CH₃), 3.68 (dt, *J*₁ = 21.6 Hz, *J₂* = 6.9 Hz, 2 H, NC*H*₂CH₂), 2.40-2.20 (m, 2 H, CH₂C*H*₂P), 1.49 (dd, *J*₁ = 70.2 Hz, J₂ = 6.9 Hz, 3 H, CHC*H*₃), 1.28 (t, *J*= 6.9 Hz, 3 H, OCH₂C*H*₃); ³¹P NMR (121 MHz, CDCl₃): δ 28.3.

### Example 5

Compound 6: 2-Hydroxy-butyric acid ethyl ester was prepared as follows: To a solution of L-2-aminobutyric acid (100g, 970 mmol) in 1.0 N H₂SO₄ (2 L) at 0°C was added NaNO₂ (111 g, 1610 mmol) in H₂O (400 mL) over 2 h. The reaction mixture was stirred at room temperature for 18h. Reaction mixture was extracted with EtOAc (4x) and combined organic layer was dried (MgSO₄) and concentrated to give a yellow solid (41.5 g). This solid was dissolved in absolute ethanol (500 mL) and concentrated HCl (3.27 mL, 39.9 mmol) was added. Reaction mixture was heated to 80°C. After 24 h, concentrated HCl (3 mL) was added and reaction continued for 24 h. Reaction mixture was concentrated and product was distilled to give a colorless oil (31 g, 235 mmol, 59%).
To a mixture of compound **4** (0.22 g, 0.63 mmol) in anhydrous acetonitrile (3.0 mL) was added thionyl chloride (0.184 mL, 2.52 mmol). The mixture was heated at 65°C for 1.5 h providing a pale yellow solution. The reaction mixture was concentrated and dried for 45 min under high vacuum. The residue was dissolved in anhydrous CH₂Cl₂ (3.3 mL) and cooled to 0°C. Triethylamine (0.26 mL, 1.89 mmol) was added slowly, followed by the dropwise addition of 2-hydroxy-butyric acid ethyl ester (0.167 mL, 1.26 mmol). The reaction mixture was stirred at 0°C for 5 min, then warmed to room temperature overnight. The reaction mixture was concentrated, dissolved in EtOAc and washed with 1.0 N HCl, saturated NaHCO₃ solution, brine and dried (MgSO₄). Concentration and purification (silica gel, 3:2 EtOAc/Hex) gave a pale yellow oil (0.21 g, 0.47 mmol, 75%). For major diastereomer, ¹H NMR (300 MHz, CDCl₃) : δ 7.35-7.10 (m, 10 H, Ar), 5.91 (s, 1 H, N*H*)), 5.12 (s, 2 H, C*H*₂Ph), 4.94-4.83 (m, 1 H, OC*H*C), 4.27-4.12 (m, 2 H, OC*H*₂CH₃), 3.80-3.50 (m, 2 H, NC*H*₂CH₂), 2.39-2.19 (m, 2 H, CH₂C*H*₂P), 1.82-1.71 (m, 2 H, CHC*H*₂CH₃), 1.30-1.195 (m, 3 H, OCH₂C*H*₃), 0.81 (t, *J=* 7.5 Hz, 3 H, CHCH₂CH₃); ³¹P NMR (120 MHz, CDCl₃): δ 28.3. For minor diastereomer,¹H NMR (300 MHz, CDCl₃): δ 7.35-7.10 (m, 10 H, Ar), 5.74 (s, 1 H, N*H*)), 5.11 (s, 2 H, C*H*₂Ph), 4.98-4.94 (m, 1 H, OC*H*C), 4.27-4.12 (m, 2 H, OC*H*₂CH₃), 3.80-3.50 (m, 2 H, NCH₂CH₂), 2.39-2.19 (m, 2 H, CH₂C*H*₂P), 1.98-1.82 (m, 2 H, CHC*H*₂CH₃), 1.30-1.195 (m, 3 H, OCH₂C*H*₃), 1.00 (t, *J*= 7.5 Hz, 3 H, CHCH₂C*H*₃); ³¹P NMR (121 MHz, CDCl₃): δ 26.2.

### Example 6

Compound 7: A mixture of compound **6,** (0.53 g, 1.18 mmol) acetic acid (0.135 mL, 2.36 mmol) and 10% palladium on activated carbon (0.08 g) in absolute ethanol (12 mL) was stirred under a hydrogen atmosphere (1 atm) for 3 h. Reaction mixture was filtered through Celite, concentrated, and resubjected to identical reaction conditions. After 2 h, Celite was added to the reaction mixture and mixture was stirred for 2 min, then filtered through a pad of Celite and concentrated. Dried under high vacuum to give the diasteromeric acetate salt as a oil (0.42 g, 1.11 mmol, 94%). ¹H NMR (300 MHz, CDCl₃): δ 7.40-7.10 (m, 5 H, Ar), 5.00-4.80 (m, 1 H, OC*H*C), 4.28-4.10 (m, 2 H, OC*H*₂CH₂), 3.32-3.14 (m, 2 H, NC*H*₂CH₂), 2.45-2.22 (m, 2 H, CH₂C*H*₂P), 1.97 (s, 3 H, Ac), 1.97-1.70 (m, 2 H, CHC*H*₂CH₃), 1.30-1.18 (m, 3 H, OCH₂C*H*₃), 1.00 (t, *J* = 7.5 Hz, 1 H, CHCH₂C*H*₃), 0.80 (t, *J* = 7.5 Hz, 2 H, CHCH₂C*H*₃); ³¹P NMR (121 MHz, CDCl₃): δ 27.6 (major, 1.85), 26.0 (minor, 1.01).

### Example 7

Compound 9: A solution of aldehyde **8** (0.596 g, 1.01 mmol) and compound **7** (0.42 g, 1.11 mmol) were stirred together in 1,2-dichloroethane (4.0 mL) in the presence of MgSO₄ for 3 h. Acetic acid (0.231 mL, 4.04 mmol) and sodium cyanoborohydride (0.127 g, 2.02 mmol) were added and reaction mixture was stirred for 50 min at room temperature. Reaction mixture was quenched with saturated NaHCO₃ solution, diluted with EtOAc, and vigorously stirred for 5 min. Brine was added and extracted with EtOAc (2x). Combined organic layer was dried (MaSO₄) concentrated and purified (silica gel, EtOAc, then 10% EtOH/EtOAc) to give a colorless foam. Acetonitrile (4 mL) and trifluoroacetic acid (0.06 mL) were added and concentrated to a volume of 1 mL. H₂O (10 mL) was added and lyophilized to give the TFA salt as a white powder (0.51 g, 0.508 mmol, 50%). ¹H NMR (300 MHz, CD₃CN): δ 7.79 (d, *J*= 8.4 Hz, 2 H, (SO₂C(C*H*)₂), 7.43-7.20 (m, 9 H, Ar), 7.10 (d, *J* = 8.4 Hz, 2 H, (C*H*)₂COCH₃), 5.85 (d, *J*= 8.4 Hz, 1 H, N*H*), 5.55 (d, *J* = 4.5 Hz, 1 H, OC*H*O), 5.00-4.75 (m, 2 H, CH₂C*H*OC(O), POC*H*C), 4.39-4.05 (m, 2 H, PhC*H*₂N, OC*H*₂CH₃), 3.89 (s, 3 H, OC*H*₃), 3.88-3.30 (m, 9H), 3.15-2.84 (m, 5 H), 2.65-2.42 (m, 3 H), 2.10-1.68 (m, 5 H), 1.65-1.15 (m, 5 H), 1.05-0.79 (m, 9 H); ³¹P NMR (121 MHz, CD₃CN): δ 24.8 (major, 1.85), 23.1 (minor, 1.01).

### Example 8

Compound 10: Compound **9** (0.041 g, 0.041 mmol) was dissolved in DMSO (1.9 mL) and to this solution was added phosphate buffered saline, pH 7.4 (10 mL) and pig liver esterase (Sigma, 0.2 mL). Reaction mixture was stirred for 24 h at 40°C. After 24 h, additional esterase (0.2 mL) was added and reaction was continued for 24 h. Reaction mixture was concentrated, resuspended in methanol and filtered. Filtrate was concentrated and purified by reverse phase chromatography to give a white powder after lyophilization (8 mg, 0.010 mmol, 25%). ¹H NMR (500 MHz, CD₃OD): δ 7.78 (d, *J* = 8.9 Hz, 2 H, (SO₂C(C*H*)₂), 7.43-7.35 (m, 4 H, Ar), 7.11 (d, *J* = 8.9 Hz, 2 H, (CH)₂COCH₃), 5.62 (d, J = 5.2 Hz, 1 H, OCHO), 4.96-4.77 (m, 2 H, CH₂C*H*OC(O), POCHC), 4.21 (br s, 2 H, PhC*H*₂N), 3.97-3.70 (m, 6 H), 3.90 (s, 3 H, OCH₃), 3.50-3.30 (m, 3 H), 3.26-3.02 (m, 2 H), 2.94-2.58 (m, 4 H), 2.09-1.78 (m, 5 H), 1.63-1.52 (m, 2 H), 1.05-0.97 (m, 3 H); 0.94 (d, *J* = 6.7 Hz, 3 H), 0.88 (d, *J* = 6.7 Hz, 3 H); ³¹P NMR (121 MHz, CD₃OD): δ 20.8.

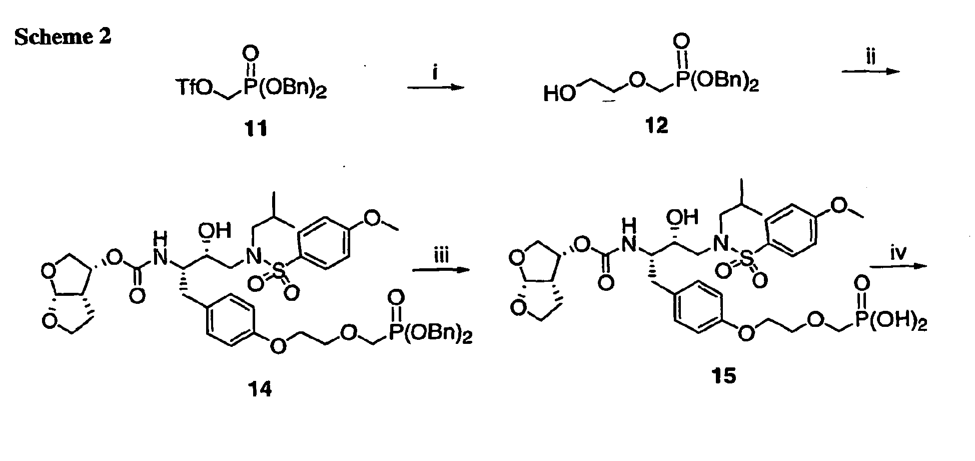

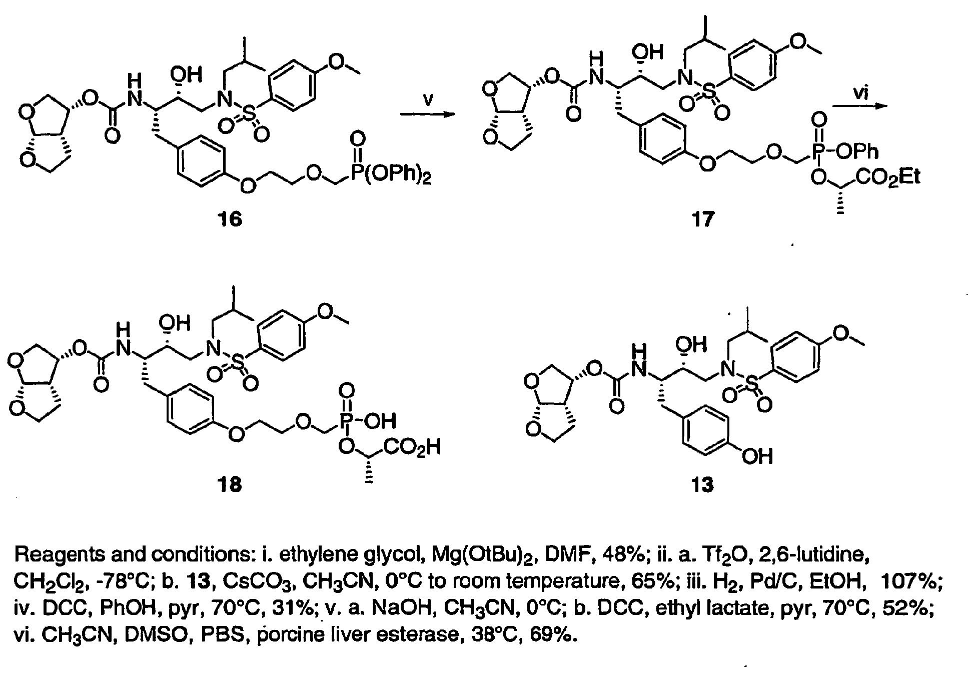

### Example 9

Compound 12: To a solution of compound **11** (4.10 g, 9.66 mmol) and anhydrous ethylene glycol (5.39 mL, 96.6 mmol) in anhydrous DMF (30 mL) at 0°C was added powdered magnesium *tert*-butoxide (2.05 g, 12.02 mmol). The reaction mixture was stirred at 0°C for 1.5 h, then concentrated. The residue was partitioned between EtOAc and H₂O and washed with 1 N HCl, saturated NaHCO₃ solution, and brine. Organic layer dried (MgSO₄), concentrated and purified (silica gel, 4% MeOH/CH₂Cl₂) to give a colorless oil (1.55 g, 48%). ¹H NMR (300 MHz, CDCl₃): δ 7.37 (s, 10 H, Ar), 5.40-5.05 (m, 4 H, C*H*₂Ph), 3.84 (d, *J* = 8.1 Hz, 2 H, PC*H*₂O), 3.70.3.60 (m, 4 H, OC*H*₂CH₂O, OCH₂C*H*₂O); ³¹P NMR (121 MHz, CDCl₃): δ 22.7.

### Example 10

Compound 14: To a solution of compound 12 (0.75 g, 2.23 mmol) and 2,6-lutidine (0.78 mL, 6.69 mmol) in CH₂Cl₂ (20 mL) at -78°C was added trifluoromethanesulfonic anhydride (0.45 mL, 2.68 mmol). The reaction mixture was stirred at -78°C for 40 min, then diluted with CH₂Cl₂ and washed with 1 N HCl, saturated NaHCO₃ and dried (MgSO₄). Concentration gave a yellow oil that was dissolved in anhydrous acetonitrile (20 mL). Phenol **13** (1.00 g, 1.73 mmol) was added to the solution, which was cooled to 0°C. Cesium carbonate (0.619 g, 1.90 mmol) was added and reaction mixture was stirred at 0°C for 2 h, then at room temperature for 1.5 h. Additional cesium carbonate (0.200 g, 0.61 mmol) was added and reaction was continued for 1.5 h, then filtered. Concentration of the filtrate and purification (silica gel, 3% MeOH/CH₂Cl₂) gave a yellow gum (1.005 g, 65%). ¹H NMR (300 MHz, CDCl₃): δ 7.71 (d, *J*= 8.7 Hz, 2 H, SO₂C(*CH*)₂), 7.34 (s, 10 H, PhC*H*₂O), 7.11 (d, *J*= 8.1Hz, 2 H, CH₂C(C*H*)₂(CH)₂), 6.98 (d, *J* = 8.7 Hz, 2 H, (C*H*)₂COCH₃), 6.78 (d, *J* = 8.7 Hz, 2 H, (C*H*)₂COCH₂), 5.62 (d, *J* = 5.4 Hz, 1 H, OC*H*O), 5.16-4.97 (m, 6 H), 4.05-3.65 (m, 12 H), 3.86 (s, 3 H, OC*H*₃), 3.19-2.66 (m, 7 H), 1.95-1.46 (m, 3 H), 0.92 (d, *J* = 6.6 Hz, 3 H, CH(C*H*₃)₂), 0.88 (d, *J* = 6.6 Hz, 3 H, CH(C*H*₃)₂); ³¹P NMR (121 MHz, CDCl₃): δ 21.9.

### Example 11

Compound 15: A mixture of compound **14** (0.410 g, 0.457 mmol) and 10% palladium on carbon (0.066 g) in ethanol (5.0 mL) was stirred under a hydrogen atmosphere (1 atm) for 16 h. Celite was added and the mixture was stirred for 5 min, then filtered through Celite and concentrated to give a foam (0.350 g, 107%). ¹H NMR (300 MHz, CD₃OD): δ 7.76 (d, *J* = 8.7 Hz, 2 H, SO₂C(*CH*)₂), 7.15 (d, *J* = 8.4Hz, 2 H, CH₂C(C*H*)₂(CH)2), 7.08 (d, *J* = 8.4 Hz, 2 H, (C*H*)₂COCH₃), 6.82 (d, *J* = 8.4 Hz, 2 H, (C*H*)₂COCH₂), 5.59 (d, *J* = 5.4 Hz, 1 H, OC*H*O), 5.16-4.97 (masked by CD₃OH, 1 H), 4.09-4.02 (m, 2 H), 3.99-3.82 (m, 10 H), 3.88 (s, 3 H, OC*H*₃), 3.52-3.32 (m, 1 H), 3.21-2.75 (m, 5 H), 2.55-2.40 (m, 1 H), 2.10-1.95 (m, 1 H), 1.75-1.25 (m, 2 H), 0.93 (d, *J* = 6.3 Hz, 3 H, CH(C*H*₃)₂), 0.88 (d, *J* = 6.6 Hz, 3 H, CH(C*H*₃)₂); ³¹P NMR (121 MHz, CD₃OD): δ 19.5.

### Example 12

Compound 16: Compound **15** (0.350 g, 0.488 mmol) was coevaporated with anhydrous pyridine (3 x 10 mL), each time filling with N₂. Residue was dissolved in anhydrous pyridine (2.5 mL) and phenol (0.459 g, 4.88 mmol) was added. This solution was heated to 70°C, then 1,3-dicyclohexylcarbodiimide (0.403 g, 1.93 mmol) was added and reaction mixture was heated at 70°C for 7 h. Reaction mixture was concentrated, coevaporated with toluene and residue obtained was diluted with EtOAc, precipitating 1,3-dicyclohexylurea. The mixture was filtered and filtrate concentrated and residue obtained was purified (silica gel, 2% MeOH/CH₂C1₂, then another column 75% EtOAc/Hex) to give a clear oil (0.1324 g, 31%). ¹H NMR (300 MHz, CDCl₃): δ 7.71 (d, *J* = 8.7 Hz, 2 H, SO₂C(C*H*)₂), 7.41-7.18 (m, 10 H, Ar), 7.14 (d, *J* = 8.4Hz, 2 H, CH₂C(C*H*)₂(CH)₂), 6.99 (d, *J* = 9.0 Hz, 2 H, (C*H*)₂COCH₃), 6.83 (d, *J* = 8.4 Hz, 2 H, (C*H*)₂COCH₂), 5.64 (d, *J* = 5.1 Hz, 1 H, OC*H*O), 5.16-4.92 (m, 2 H), 4.32-3.62 (m, 12 H), 3.87 (s, 3 H, OC*H*₃), 3.22-2.73 (m, 7 H), 1.95-1.75 (m, 3 H), 0.93 (d, *J* = 6.6 Hz, 3 H, CH(C*H*₃)₂), 0.88 (d, *J* = 6.6 Hz, 3 H, CH(C*H*₃)₂); ³¹P NMR (121 MHz, CDCl₃): δ 14.3.

### Example 13

Compound 17: To a solution of compound **16** (0.132 g, 0.152 mmol) in acetonitrile (1.5 mL) at 0°C was added 1.0 M NaOH (0.38 mL, 0.381 mmol). Reaction mixture was stirred for 2 h at 0°C, then Dowex 50 (H+) resin was added until pH = 1. The resin was removed by filtration and the filtrate was concentrated and washed with EtOAc/Hex (1:2, 25 mL), then dried under high vacuum to give a clear film (0.103 g, 85%). This film was coevaporated with anhydrous pyridine (3 x 5 mL), filling with N₂. The residue was dissolved in anhydrous pyridine (1 mL) and ethyl lactate (0.15 mL, 1.30 mmol) was added and reaction mixture was heated at 70°C. After 5 min, 1,3-dicyclohexylcarbodiimide (0.107 g, 0.520 mmol) was added and reaction mixture was stirred at 70°C for 2.5 h. Additional 1,3-dicyclohexylcarbodiimide (0.055 g, 0.270 mmol) was added and reaction continued for another 1.5 h. Reaction mixture was concentrated and coevaporated with toluene and diluted with EtOAc, precipitating 1,3-dicyclohexylurea. The mixture was filtered and filtrate concentrated and residue obtained was purified (silica gel, 80 to 100% EtOAc/Hex) to give a white foam (0.0607 g, 52%). ¹H NMR (300 MHz, CDCl₃): δ 7.71 (d, *J=* 8.7 Hz, 2 H, SO₂C(C*H*)₂), 7.39-7.16 (m, 5 H, Ar), 7.13 (d, *J* = 8.1Hz; 2 H, CH₂C(C*H*)₂(CH)₂), 6.99 (d, *J =* 9.0 Hz, 2 H, (C*H*)₂COCH₃), 6.82 (d, *J* = 8.4 Hz, 2 H, (C*H*)₂COCH₂), 5.64 (d, *J* = 5.1 Hz, 1 H, OC*H*O), 5.16-4.92 (m, 3 H), 4.35-3.65 (m, 14 H), 3.87 (s, 3 H, OC*H*₃), 3.22-2.73 (m, 7 H), 1.95-1.80 (m, 3 H), 1.59 (d, *J* = 6.9Hz, 1.5 H, CCHC*H*₃), 1.47 (d, *J* = 7.2 Hz, 1.5 H, CCHC*H*₃), 1.37-1.18 (m, 3 H), 0.92 (d, *J* = 6.6 Hz, 3H, CH(C*H*₃)₂), 0.88 (d, *J* = 6.6 Hz, 3 H, CH(C*H*₃)₂); ³¹P NMR (121 MHz, CDCl₃): δ 19.2, 17.2.

### Example 14

Compound 18: Compound **17** (11.5 mg, 0.013 mmol) was dissolved in DMSO (0.14 mL) and acetonitrile (0.29 mL). PBS (pH 7.4, 1.43 mL) was added slowly with stirring. Porcine liver esterase (Sigma, 0.1 mL) was added and reaction mixture was gently stirred at 38°C. After 24 h, additional porcine liver esterase (0.1 mL) and DMSO (0.14 mL) were added and reaction mixture stirred for 48 h at 38°C. Reaction mixture concentrated and methanol was added to precipitate the enzyme. The mixture was filtered, concentrated and purified by reverse phase chromatography to give a white powder after lyophilization (7.1 mg, 69%). ¹H NMR (300 MHz, CD₃OD): δ 7.76 (d, *J* = 8.7 Hz, 2 H, SO₂C(C*H*)₂), 7.15 (d, *J* = 8.4 Hz, 2 H, CH₂C(C*H*)₂(CH)₂), 7.08 (d, *J* = 9.0 Hz, 2 H, (C*H*)₂COCH₃), 6.83 (d, *J* = 8.7 Hz, 2 H, (C*H*)₂COCH₂), 5.59 (d, *J =* 5.1 Hz, 1 H, OC*H*O), 5.16-4.90 (masked by CD₃OH, 2 H), 4.19-3.65 (m, 12 H), 3.88 (s, 3 H, OC*H*₃), 3.50-3.27 (m, 1 H), 3.20-2.78 (m, 5 H), 2.55-2.40 (m, 1 H), 2.05-1.90 (m, 1 H), 1.75-1.30 (m, 2 H), 1.53 (d, *J* = 6.6 Hz, 3 H, CCHC*H*₃), 0.93 (d, *J =* 6.6 Hz, 3 H, CH(C*H*₃)₂), 0.88 (d, *J =* 6.6 Hz, 3 H, CH(C*H*₃)₂); ³¹P NMR (121 MHz, CD₃OD): δ 16.7.

Alternatively, compound 17 was prepared as described below (Scheme 3).

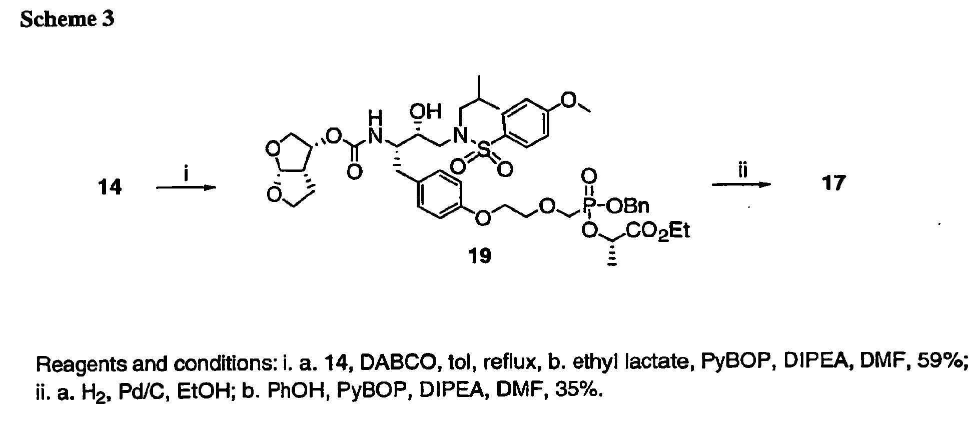

### Example 15

Compound 19: To a solution of compound **14** (0.945 g, 1.05 mmol) in anhydrous toluene (10.0 mL) was added 1,4-diazobicyclo[2.2.2] octane (0.130 g, 1.16 mmol) and reaction mixture was refluxed for 2 h. After cooling to room temperature, reaction mixture was diluted with EtOAc and washed with 1.0 N HCl and dried (MgSO₄). Concentration gave a white foam (0.785 g, 93%). Residue was dissolved in anhydrous DMF (10.0 mL) and to this solution was added ethyl (S)-lactate (0.23 mL, 2.00 mmol) and diisopropylethylamine (0.70 mL, 4.00 mmol), followed by benzotriazol-1-yloxytripyrroldinophosphonium hexafluorophosphate (1.041 g, 2.00 mmol). Reaction mixture was stirred for 20 h, then concentrated and residue was dissolved in EtOAc and washed with 1.0 N HCl, saturated NaHCO₃, brine and dried (MgSO₄). Concentration and purification (silica gel, 2 % MeOH/CH₂Cl₂) gave an off-white foam (0.520 g, 59%). ¹H NMR (300 MHz, CDCl₃): δ 7.72 (d, *J* = 7.5 Hz, 2 H, SO₂C(C*H*)₂), 7.50-7.27 (m, 4 H, Ar), 7.12 (d, *J* = 8.1Hz, 2 H, CH₂C(C*H*)₂(CH)₂), 7.00 (d, *J =* 6.6 Hz, 2 H, (C*H*)₂COCH₃), 6.81 (d, *J* = 8.4 Hz, 2 H, (C*H*)₂COCH₂), 5.64 (d, *J =* 5.1 Hz, 1 H, OC*H*O), 5.37-4.90 (m, 5 H), 4.35-3.65 (m, 14 H), 3.88 (s, 3 H, OC*H*₃), 3.24-2.70 (m, 7 H), 1.90-1.70 (m, 3 H), 1.54 (d, *J* = 6.9Hz, 1.5 H, CCHC*H*₃), 1.47 (d, *J* = 6.9 Hz, 1.5 H, CCHC*H*₃), 1.37-1.22 (m, 3 H), 0.93 (d, *J* = 6.3 Hz, 3 H, CH(C*H*₃)₂), 0.89 (d, *J*= 6.0 Hz, 3 H, CH(C*H*₃)₂); ³¹P NMR (121 MHz, CDCl₃): δ 22.3, 21.2.

### Example 16

Compound 17: A mixture of compound **19** (0.520 g, 0.573 mmol) and 10% palladium on carbon (0.055 g) in ethanol (10 mL) was stirred under a hydrogen atmosphere (1 atm) for 2 h. Celite was added to the reaction mixture and stirred for 5 min, then mixture was filtered through Celite and concentrated to give a white foam (0.4649 g, 99%). Residue was dissolved in anhydrous DMF (5.0 mL) and to this solution was added phenol (0.097 g, 1.03 mmol), diisopropylethylamine (0.36 mL, 2.06 mmol) followed by benzotriazol-1-yloxytripyrroldinophosphonium hexafluorophosphate (0.536 g, 1.03 mmol). Reaction mixture was stirred for 20 h, then concentrated and residue was dissolved in EtOAc and washed with 1 N HCl, H₂O, sat. NaHCO₃, brine and dried (MgSO₄). Concentration and purification (silica gel, 2 % MeOH/CH₂Cl₂) gave a white foam (0.180 g, 35%).

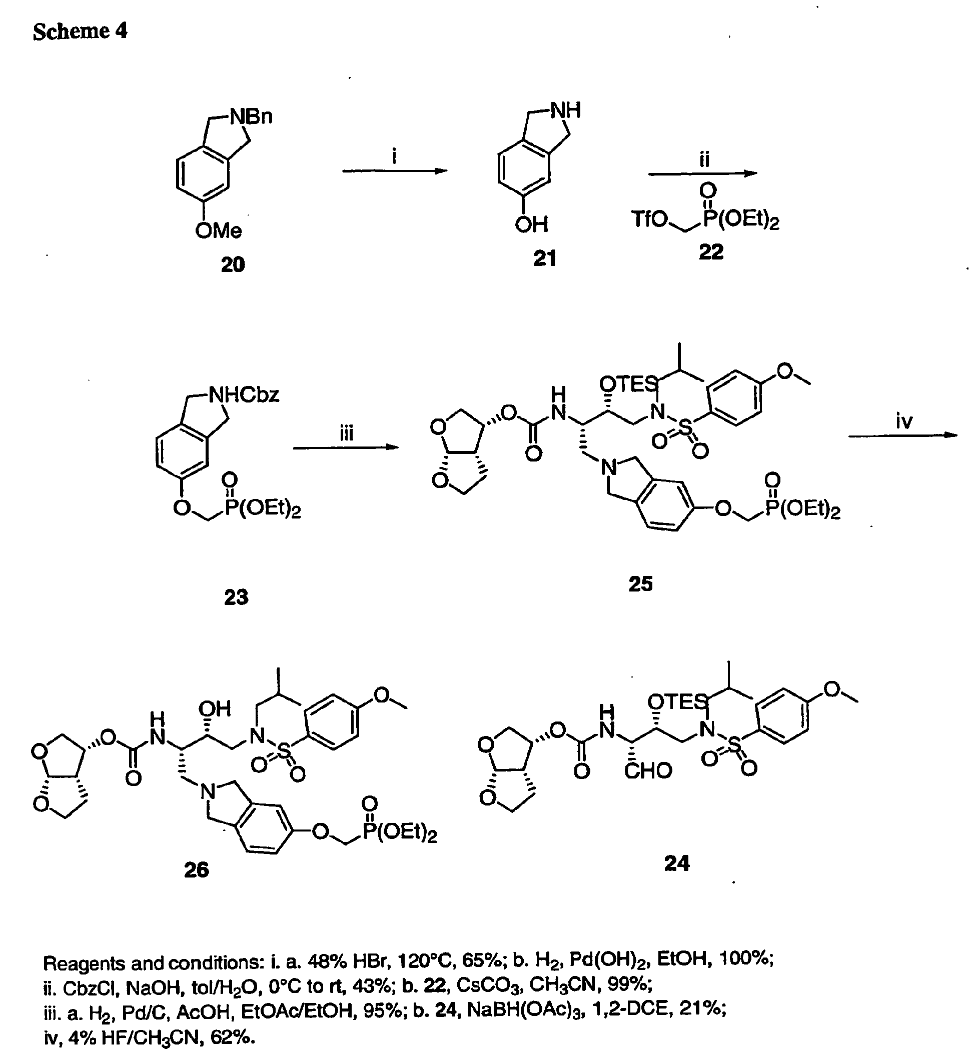

### Example 17

Compound 21: Compound **20** (11.5 g, 48.1 mmol) in 48% HBr (150 mL) was heated at 120°C for 4 h, then cooled to room temperature and diluted with EtOAc. Mixture was neutralized with saturated NaHCO₃ solution and solid NaHCO₃ and extracted with EtOAc containing MeOH. Organic layer dried (MgSO₄), concentrated, and purified (silica gel, 1:2 EtOAc/Hex with 1% MeOH) to give a brown solid (7.0 g, 65%). The resulting compound (7.0 g, 31.1 mmol) and 10% palladium hydroxide (2.1 g) in EtOH (310 mL) was stirred under a hydrogen atmosphere for 1 d, then filtered through Celite and concentrated to give an off-white solid (4.42 g, 100%). ¹H NMR (300 MHz, CDCl₃): δ 7.01 (d, *J* = 7.8 Hz, 1 H, Ar), 6.64 (s, 1 H, Ar), 6.61 (d, *J* = 8.1 Hz, 2 H, Ar), 4.07 (s, 2 H, ArC*H*₂N), 4.05 (s, 2 H, ArC*H*₂N).

### Example 18

Compound 22: To a solution of compound **21** (4.42 g, 32.7 mmol) in 1.0 M NaOH (98 mL, 98.25 mmol) at 0°C was added dropwise benzyl chloroformate (7.00 mL, 49.13 mmol) in toluene (7 mL). After addition was complete, reaction mixture was stirred overnight at room temperature. Reaction mixture was diluted with EtOAc and extracted with EtOAc (3x). Combined organic layer was dried (MgSO₄), concentrated and purified (silica gel, 2% MeOH/CH₂Cl₂) to give a white solid (3.786 g, 43%). The resulting compound (0.6546 g, 2.43 mmol) was dissolved in anhydrous acetonitrile (10 mL), and compound 23 (0.782 g, 2.92 mmol) was added, followed by cesium carbonate (1.583 g, 4.86 mmol). Reaction mixture was stirred for 2h at room temperature, then filtered, concentrated, and purified (3% MeOH/CH₂Cl₂) to give a brownish oil (1.01 g, 99%).

### Example 19

Compound 25: To a solution of compound **22** (0.100 g, 0.238 mmol) in EtOAc/EtOH (2 mL, 1:1) was added acetic acid (14 µL, 0.238 mmol) and 10% palladium on carbon (0.020 g) and the mixture was stirred under a hydrogen atmosphere for 2 h. Celite was added to the reaction mixture and stirred for 5 min, then filtered through Celite. Concentration and drying under high vacuum gave a reddish film (0.0777 g, 95%). The resulting amine (0.0777g, 0.225 mmol) and aldehyde **24** (0.126 g, 0.205 mmol) in 1,2-dichloroethane (1.2 mL) were stirred for 5 min at 0°C, then sodium triacetoxyborohydride (0.0608 g, 0.287 mmol) was added. Reaction mixture was stirred for 1 h at 0°C, then quenched with saturated NaHCO₃ solution and brine. Extracted with EtOAc, the organic layer was dried (MgSO₄), concentrated and purified (silica gel, 2% MeOH/CH₂Cl₂) to give a brown foam (38.7 mg, 21%). ¹H NMR (300 MHz, CDCl₃): δ 7.74 (d, *J* = 8.7 Hz, 2 H, Ar), 7.09 (d, *J* = 8.7 Hz, 1 H, Ar), 7.05-6.72 (m, 4 H, Ar), 5.71 (d, *J =* 5.1 Hz, 1 H), 5.22-5.07 (m, 2 H), 4.22-4:17 (m, 7 H), 4.16-3.69 (m, 9 H), 3.82 (s, 3 H), 3.25-2.51 (m, 7 H), 2.22-1.70 (m, 3 H), 1.37 (t, *J*= 6.9 Hz, 6 H), 1.10-0.58 (m, 21 H); ³¹P NMR (121 MHz, CDCl₃): δ 19.5.

### Example 20

Compound 26: To a solution of compound **25** (38.7 mg, 0.0438 mmol) in acetonitrile (0.5 mL) at 0°C was added 48% HF (0.02 mL). The reaction mixture was stirred at room temperature for 2 h, then quenched with saturated NaHCO₃ solution and extracted with EtOAc. Organic layer was separated, dried (MgSO₄), concentrated and purified (silica gel, 3 to 5% MeOH/CH₂Cl₂) to give a red film (21.2 mg, 62%). ¹H NMR (300 MHz, CDCl₃): δ 7.73 (d, *J* = 8.7 Hz, 2 H, Ar), 7.10 (d, *J* = 8.7 Hz, 1 H, Ar), 6.97 (d, *J* = 8.70 Hz, 2 H), 6.90-6.76 (m, 2 H), 5.72 (d, *J* = 5.1 Hz, 1 H), 5.41 (d, *J* = 9.0 Hz, 1 H), 5.15 (q, *J* = 6.6 Hz, 1 H), 4.38-4.17 (m, 7 H), 4.16-3.65 (m, 9 H), 3.87 (s, 3 H), 3.20-2.82 (m, 7 H), 2.75-1.79 (m, 3 H), 1.37 (t, *J* = 6.9 Hz, 6 H), 0.90 (d, *J* = 6.6 Hz, 3 H), 0.88 (d, *J* = 6.6 Hz, 3 H); ³¹P NMR (121 MHz, CDCl₃): δ 19.3.

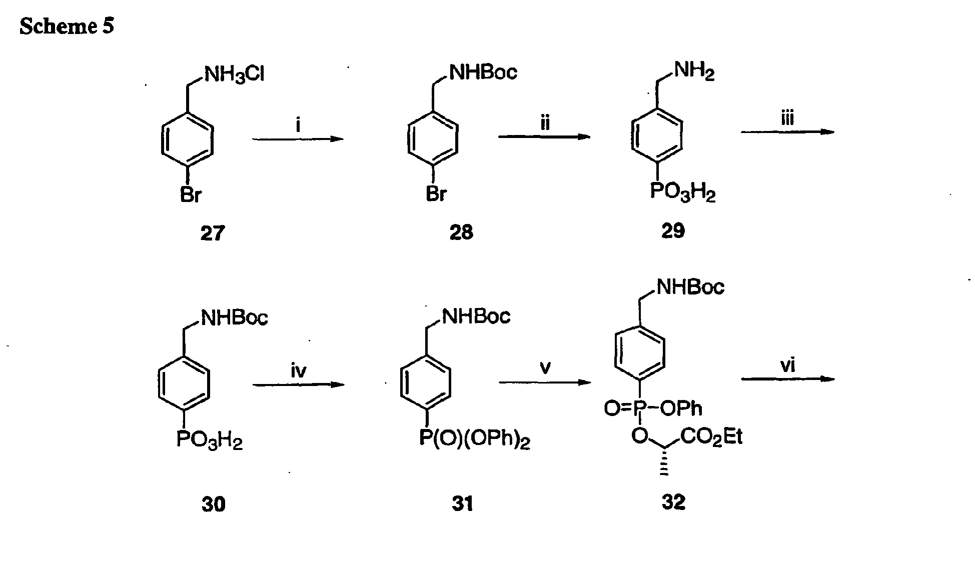

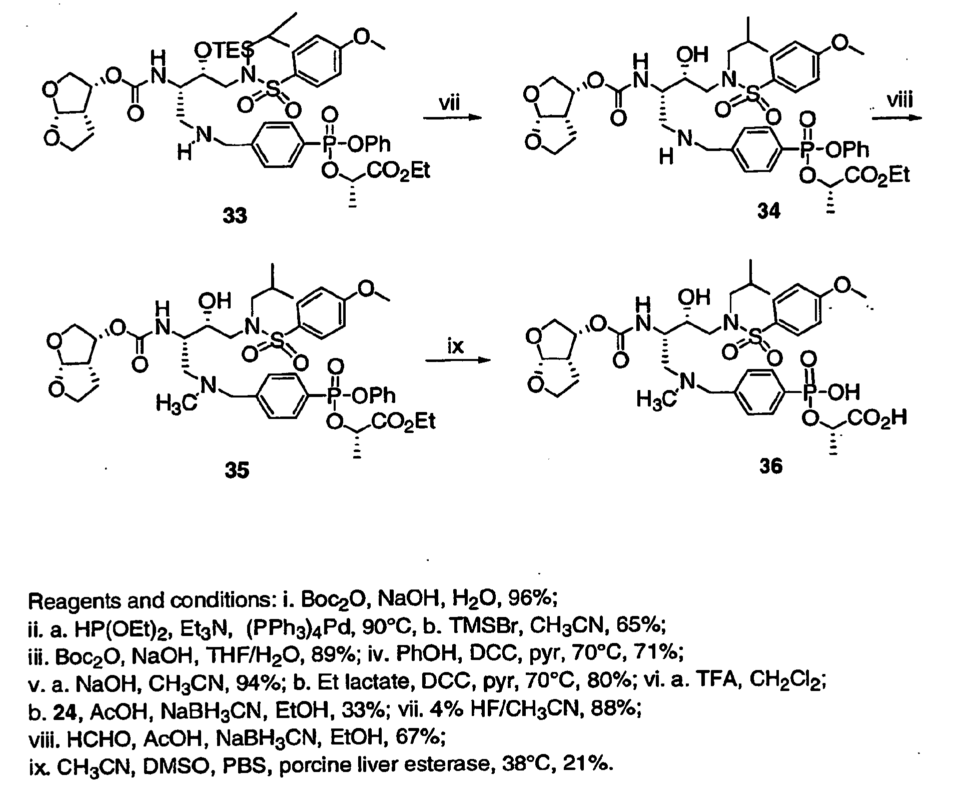

### Example 21

Compound 28: To a mixture of 4-bromobenzylamine hydrochloride (15.23 g, 68.4 mmol) in H₂O (300 mL) was added sodium hydroxide (8.21 g, 205.2 mmol), followed by di-*tert*-butyl dicarbonate (16.45g, 75.3 mmol). Reaction mixture was vigorously stirred for 18 h, then diluted with EtOAc (500 mL). Organic layer separated and aqueous layer extracted with EtOAc (200 mL). Combined organic layer was dried (MgSO₄) concentrated and dried under high vacuum to give a white solid (18.7 g, 96%). ¹H NMR (300 MHz, CDCl₃): δ 7.41 (d, *J* = 8.4 Hz, 2 H), 7.12 (d, *J* = 8.3 Hz, 2 H), 4.82 (s, 1 H, N*H*), 4.22 (d, *J* = 6.1 Hz, 2 H), 1.41 (s, 9 H).

### Example 22

Compound 29: Compound **28** (5.00 g, 17.47 mmol) was coevaporated with toluene. Diethyl phosphite (11.3 mL, 87.36 mmol) was added and mixture was coevaporated with toluene (2x). Triethylamine (24.0 mL, 174.7 mmol) was added and mixture was purged with argon for 10 min, then tetrakis(triphenylphosphine) palladium(0) (4.00 g, 3.49 mmol) was added. Reaction mixture was refluxed for 18 h, cooled, concentrated and diluted with EtOAc. Washed with 0.5 N HCl, 0.5 M NaOH, H₂O, brine and dried (MgSO₄). Concentrated and purification (silica gel, 70% EtOAc/Hex) gave an impure reaction product as a yellow oil (6.0 g). This material (6.0 g) was dissolved in anhydrous acetonitrile (30 mL) and cooled to 0°C. Bromotrimethylsilane (11.5 mL, 87.4 mmol) was added and reaction mixture was warmed to room temperature over 15 h. Reaction mixture was concentrated, dissolved in MeOH (50 mL) and stirred for 1.5 h. H₂O (1 mL) was added and mixture stirred for 2 h. Concentrated to dryness and dried under high vacuum, then triturated with Et₂O containing 2% MeOH to give a white solid (3.06 g, 65 %). ¹H NMR (300 MHz, D₂O): δ 7.67 (dd, *J* = 12.9, 7.6 Hz, 2 H), 7.45-7.35 (m, 2 H), 4.10 (s, 2 H); ³¹P NMR (121 MHz, D₂O): δ 12.1.

### Example 23

Compound 30: Compound **29** (4.78 g, 17.84 mmol) was dissolved in H₂O (95 mL) containing sodium hydroxide (3.57 g, 89.20 mmol). Di-*tert*-butyl dicarbonate (7.63 g, 34.94 mmol) was added, followed by THF (25 mL). The clear reaction mixture was stirred overnight at room temperature then concentrated to -100 mL. Washed with EtOAc and acidified to pH 1 with 1 N HCl and extracted with EtOAc (7x). Combined organic layer was dried (MgSO₄), concentrated and dried under high vacuum. Trituration with Et₂O gave a white powder (4.56 g, 89%). ¹H NMR (300 MHz, CD₃OD) δ 7.85-7.71 (m, 2 H), 7.39-7.30 (m, 2 H), 4.26 (s, 2 H), 1.46 (s, 9 H); ³¹P NMR (121 MHz, CD₃OD): δ 16.3.

### Example 24

Compound 31: Compound **30** (2.96 g, 10.32 mmol) was coevaporated with anhydrous pyridine (3 x 10 mL). To this residue was added phenol (9.71 g, 103.2 mmol) and mixture was coevaporated with anhydrous pyridine (2 x 10 mL). Pyridine (50 mL) was added and solution heated to 70°C. After 5 min, 1,3-dicyclohexylcarbodiimide (8.51 g, 41.26 mmol) was added and resulting mixture was stirred for 8 h at 70°C. Reaction mixture was cooled and concentrated and coevaporated with toluene. Residue obtained was diluted with EtOAc and the resulting precipitate was removed by filtration. The filtrate was concentrated and purified (silica gel, 20 to 40% EtOAc/Hex, another column 30 to 40% EtOAc/Hex) to give a white solid (3.20 g, 71 %). ¹H NMR (300 MHz, CDCl₃):δ 7.90 (dd, *J* =13.8, 8.2 Hz, 2 H), 7.41-7.10 (m, 14 H), 5.17 (br s, 1 H, N*H*), 4.35 (d, *J* = 5.2 Hz, 2 H), 1.46 (s, 9 H); ³¹P NMR (121 MHz, CDCl₃): δ 11.8.

### Example 25

Compound 32: To a solution of compound **31** (3.73 g, 8.49 mmol) in acetonitrile (85 mL) at 0°C was added 1 M NaOH (21.2 mL, 21.21 mmol). Reaction mixture was stirred at 0°C for 30 min, then warmed to room temperature over 4 h. Reaction mixture cooled to 0°C and Dowex (H+) residue was added to pH 2. Mixture was filtered, concentrated and residue obtained was triturated with EtOAc/Hex (1:2) to give a white powder (2.889 g, 94%). This compound (2.00 g, 5.50 mmol) was coevaporated with anhydrous pyridine (3 x 10 mL). The residue was dissolved in anhydrous pyridine (30 mL) and ethyl (S)-lactate (6.24 mL, 55 mmol) and reaction mixture was heated to 70°C. After 5 min, 1,3-dicyclocarbodiiimide (4.54 g, 22.0 mmol) was added. Reaction mixture was stirred at 70°C for 5 h, then cooled and concentrated. Residue was dissolved in EtOAc and precipitate was removed by filtration. The filtrate was concentrated and purified (25 to 35% EtOAc/Hex, another column 40% EtOAc/Hex) to give a colorless oil (2.02 g, 80%). ¹H NMR (300 MHz, CDCl₃): δ 7.96-7.85 (m, 2 H), 7.42-7.35 (m, 2 H), 7.35-7.08 (m, 4 H), 5.16-5.00 (m, 1 H), 4.93 (s, 1 H, N*H*), 4.37 (d, *J =* 5.5 Hz, 1 H), 4.21 (q, *J* = 7.3 Hz, 1 H), 4.11 (dq, *J* = 5.7, 2.2 Hz, 1 H), 1.62-1.47 (m, 3 H), 1.47 (s, 9 H), 1.27 (t, *J* = 7.3 Hz, 1.5 H), 1.17 (t, *J* = 7.3 Hz, 1.5 H); ³¹P NMR (121 MHz, CDCl₃): δ 16.1, 15.0.

### Example 26

Compound 33: Compound **32** (2.02 g, 4.36 mmol) was dissolved in CH₂Cl₂ (41 mL) and cooled to 0°C. To this solution was added trifluoroacetic acid (3.5 mL) and reaction mixture was stirred at 0°C for 1 h, then at room temperature for 3 h. Reaction mixture was concentrated, coevaporated with EtOAc and diluted with H₂O (400 mL). Mixture was neutralized with Amberlite IRA-67 weakly basic resin, then filtered and concentrated. Coevaporation with MeOH and dried under high vacuum to give the TFA amine salt as a semi-solid (1.48 g, 94%). To a solution of the amine (1.48 g, 4.07 mmol) in absolute ethanol (20 mL) at 0°C was added aldehyde 24 (1.39 g, 2.26 mmol), followed by acetic acid (0.14 mL, 2.49 mmol). After stirring for 5 min, sodium cyanoborohydride (0.284 g, 4.52 mmol) was added and reaction mixture stirred for 30 min at 0°C. Reaction was quenched with saturated NaHCO₃ solution and diluted with EtOAc and H₂O. Aqueous layer was extracted with EtOAc (3x) and combined organic layer was dried (MgSO₄), concentrated and purified (silica gel, 2 to 4% MeOH/CH₂Cl₂) to give white foam (0.727 g, 33%). ¹H NMR (300 MHz, CDCl₃): δ 7.98-7.86 (m, 2 H), 7.71 (d, *J* = 8.6 Hz, 2 H), 7.49 (br s, 2H), 7.38-7.05 (m, 5 H), 6.98 (d, *J* = 8.8 Hz, 2H), 5.72 (d, *J* = 5.1 Hz, 1 H), 5.28-5.00 (m, 2 H), 4.30-3.72 (m, 12 H), 3.42-3.58 (m, 1 H), 3.20-2.68 (m, 7 H), 2.25-1.42 (m, 6 H), 1.26 (t, *J =* 7.2 Hz, 1.5 H), 1.17 (t, *J* = 7.2 Hz, 1.5 H), 1.08-0.50 (m, 21 H); ³¹P NMR (121 MHz, CDCl₃): δ 16.1, 15.1.

### Example 27

Compound 34: To a solution of compound **33** (0.727 g, 0.756 mmol) in acetonitrile (7.6 mL) at 0°C was added 48% hydrofluoric acid (0.152 mL) and reaction mixture was stirred for 40 min at 0°C, then diluted with EtOAc and H₂O. Saturated NaHCO₃ was added and aqueous layer was extracted with EtOAc (2x). Combined organic layer was dried (MgSO₄), concentrated and purified (silica gel, 4 to 5% MeOH/CH₂Cl₂) to give a colorless foam (0.5655 g, 88%). ¹H NMR (300 MHz, CDCl₃): δ 7.95-7.82 (m, 2 H), 7.67 (d, *J* = 8.1 Hz, 2 H), 7.41 (br s, 2 H), 7.38-7.05 (m, 5 H), 6.95 (d, *J =* 7.2 Hz, 2 H), 5.76 (d, *J* = 7.9 Hz, 1 H), 5.67 (d, *J =* 5.0 Hz, 1 H), 5.32-4.98 (m, 2 H), 4.25-3.75 (m, 13 H), 3.25-2.70 (m, 7 H), 2.15-1.76 (m, 3 H), 1.53-1.41 (m, 3 H), 1.25-1.08 (m, 3 H), 0.87 (d, *J* = 4.2 Hz, 6 H); ³¹P NMR (121 MHz, CDCl₃): δ 16.1, 15.0.

### Example 28

Compound 35: To a solution of compound **33** (0.560 g, 0.660 mmol) in absolute ethanol (13 mL) at 0°C was added 37% formaldehyde (0.54 mL, 6.60 mmol), followed by acetic acid (0.378 mL, 6.60 mmol). The reaction mixture was stirred at 0°C for 5 min, then sodium cyanoborohydride (0.415 g, 6.60 mmol) was added. Reaction mixture was warmed to room temperature over 2 h, then quenched with saturated NaHCO₃ solution. EtOAc was added and mixture was washed with brine. Aqueous layer was extracted with EtOAc (2x) and combined organic layer was dried (MgSO₄), concentrated and purified (silica gel, 3% MeOH/CH₂Cl₂) to give a white foam (0.384 g, 67%). ¹H NMR (300 MHz, CDCl₃): δ 7.95-7.82 (m, 2 H), 7.71 (d, *J* = 8.4 Hz, 2 H), 7.38 (br s, 2 H), 7.34-7.10 (m, 5 H), 6.98 (d, *J* = 8.8 Hz, 2 H), 5.72 (d, *J* = 5.0 Hz, 1 H), 5.50 (br s, 1H), 5.19-5.01 (m, 2 H), 4.29-3.75 (m, 10 H), 3.85 (s, 3 H), 3.35-2.70 (m, 7 H), 2.23 (s, 3 H), 2.17-1.79 (m, 3 H), 1.54 (d, *J* = 6.9 Hz, 1.5 H), 1.48 (d, *J* = 6.8 Hz, 1.5 H), 1.25 (t, J = 7.2 Hz, 1.5 H), 1.16 (t, *J =* 7.2 Hz, 1.5 H), 0.92 (d, *J* = 6.6 Hz, 3 H), 0.87 (d, *J =* 6.6 Hz, 3 H). ³¹P NMR (121 MHz, CDCl₃): δ 16.0, 14.8.

### Example 29

Compound 36: To a solution of compound **35** (44 mg, 0.045 mmol) in acetonitrile (1.0 mL) and DMSO (0.5 mL) was added phosphate buffered saline (pH 7.4, 5.0 mL) to give a cloudy white suspension. Porcine liver esterase (200 µL) was added and reaction mixture was stirred for 48 h at 38°C. Additional esterase (600 µL) was added and reaction was continued for 4 d. Reaction mixture was concentrated, diluted with MeOH and the resulting precipitate removed by filtration. Filtrate was concentrated and purified by reverse phase HPLC to give a white powder after lyophilization (7.2 mg, 21%). ¹H NMR (300 MHz, CD₃OD): δ 7.95 (br s, 2 H), 7.76 (d, *J* = 8.4 Hz, 2 H), 7.64 (br s, 2 H), 7.13 (d, *J* = 8.7 Hz, 2 H), 5.68 (d, *J* = 5.1 Hz, 1 H), 5.14 (br s, 1 H), 4.77 (br s, 1 H), 4.35-3.59 (m, 8 H), 3.89 (s, 3 H), 3.45-2.62 (m, 10 H), 2.36-1.86 (m, 3 H), 1.44 (d, *J* = 6.3 Hz, 3 H), 0.92 (d, *J* = 6.6 Hz, 3 H), 0.84 (d, *J* = 6.6 Hz, 3 H); ³¹P NMR (121 MHz, CD₃OD): δ 13.8.

### Example Section W

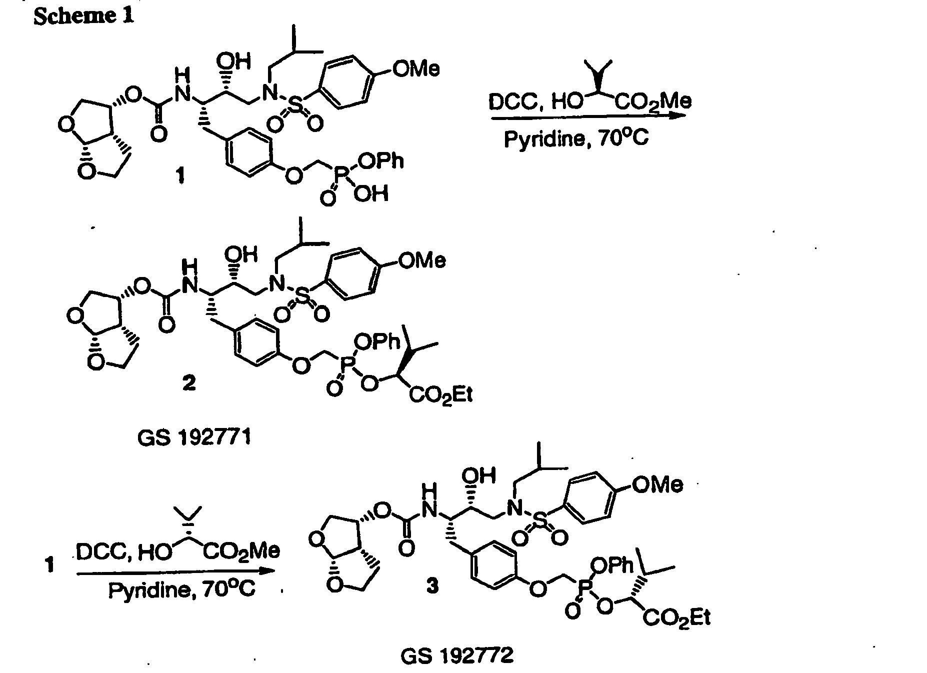

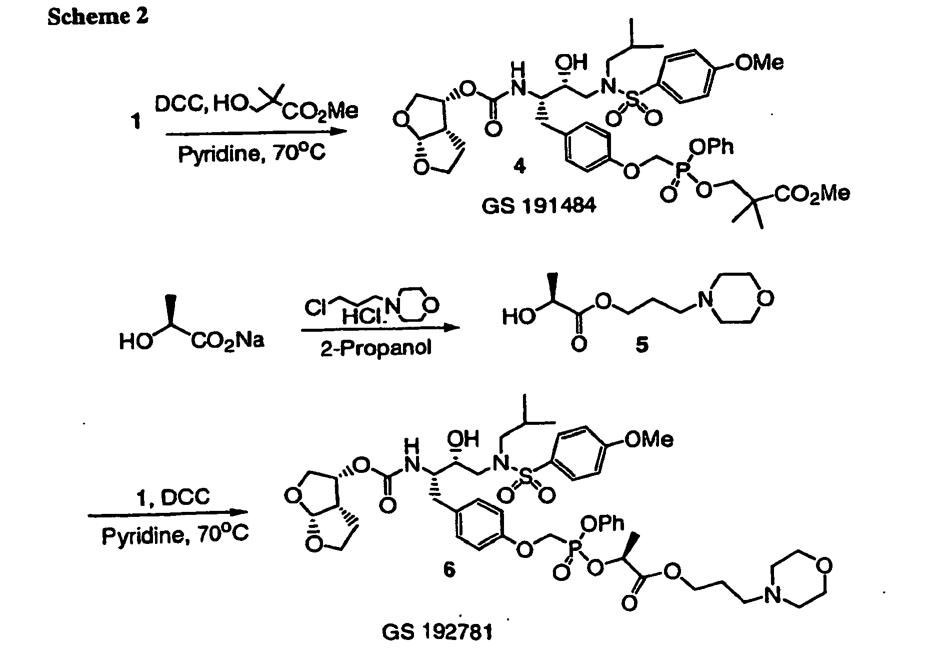

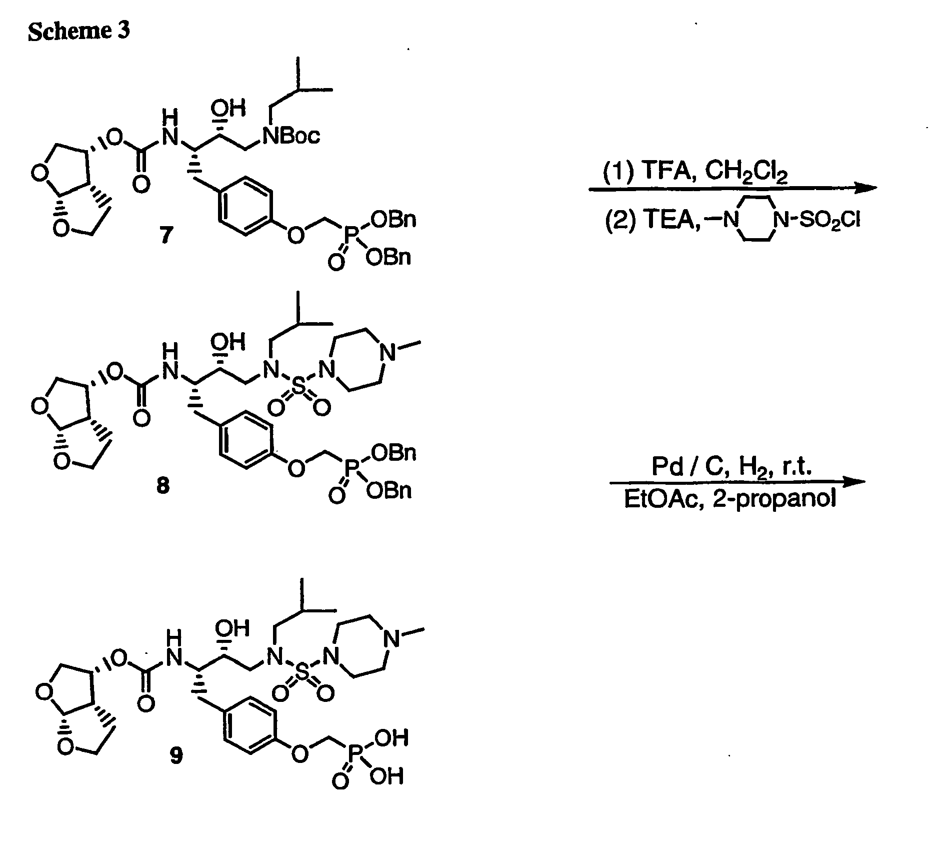

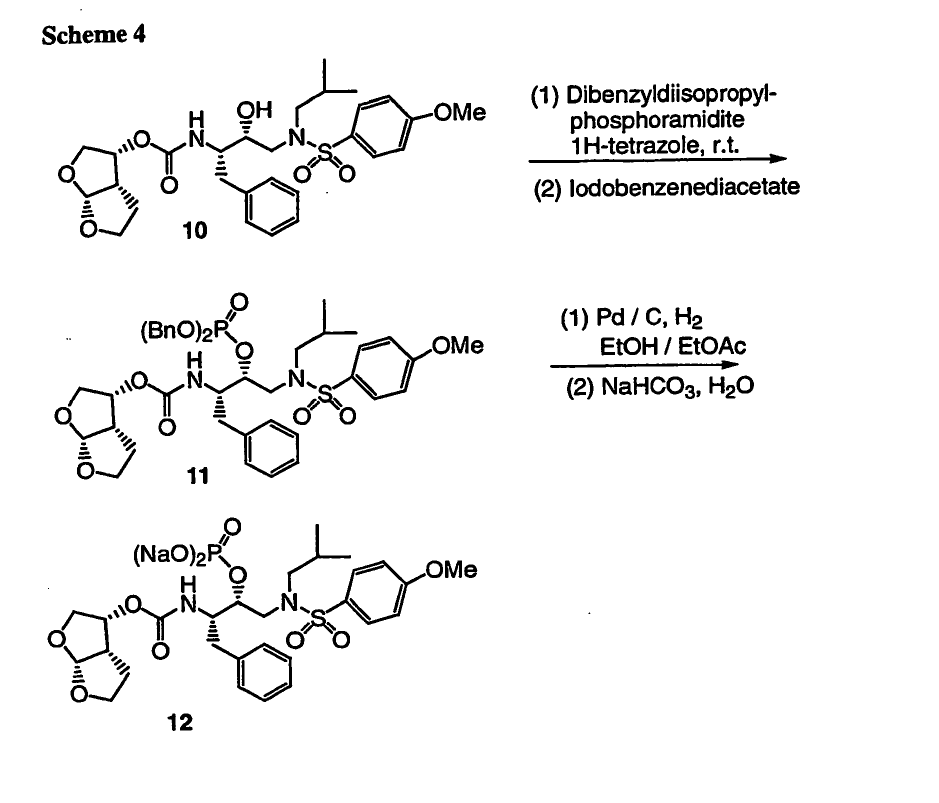

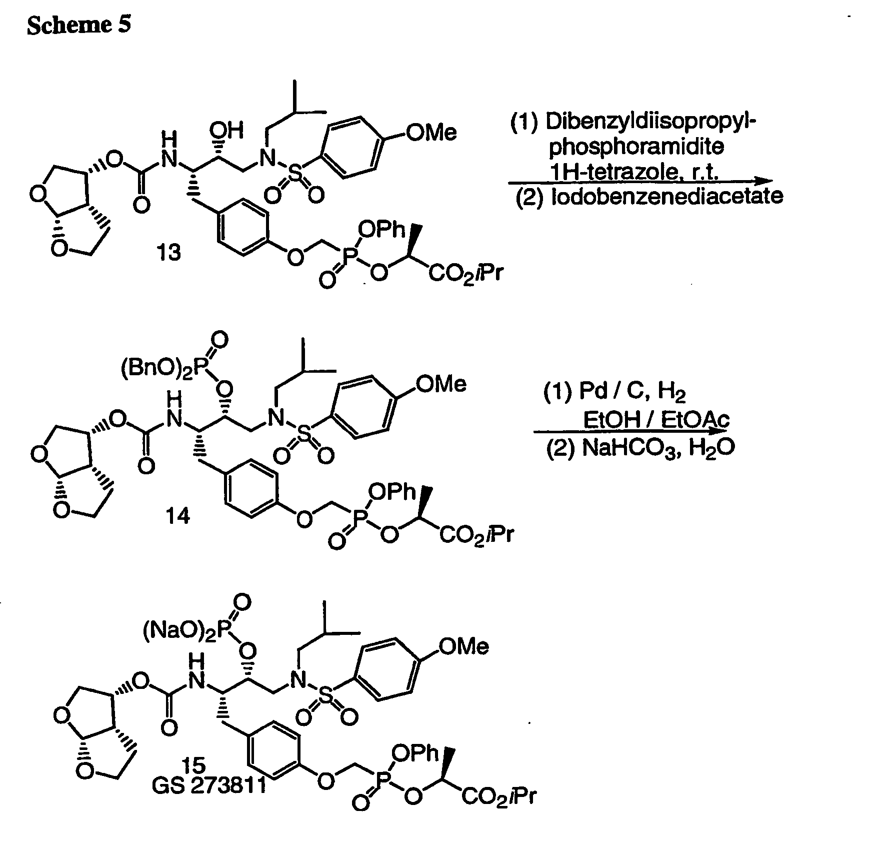

### Example 1

Monophospholactate 2: A solution of 1 (0.11 g, 0.15 mmol) and α-hydroxyisovaleric acid ethyl-(S)-ester (71 mg, 0.49 mmol) in pyridine (2 mL) was heated to 70°C and 1,3-dicyclohexylcarbodiimide (0.10 g, 0.49 mmol) was added. The reaction mixture was stirred at 70°C for 2 h and cooled to room temperature. The solvent was removed under reduced pressure. The residue was suspended in EtOAc and 1,3-dicyclohexyl urea was filtered off. The product was partitioned between EtOAc and 0.2 N HCl. The EtOAc layer was washed with 0.2 N HCl, H₂O, saturated NaCl, dried with Na₂SO₄, filtered, and concentrated. The crude product was purified by column chromatography on silica gel (3% 2-propanoI/CH₂Cl₂) to give the monophospholactate (35 mg, 28%, GS 192771, 1/1 diastereomeric mixture) as a white solid: ¹H NMR (CDCl₃) δ 7.71 (d, J = 8.7 Hz, 2H), 7.36-7.14 (m, 7H), 6.99 (d, J = 8.7 Hz, 2H), 6.94-6.84 (dd, 2H), 5.65 (d, J = 5.4 Hz, 1H), 5.00-4.85 (m, 3H), 4.55 (dd, 1H), 4.41 (dd, 1H), 4.22-4.07 (m, 2H), 3.96-3.68 (m, 9H), 3.12-2.74 (m, 7H), 2.29 (m, 1H), 1.85-1.57 (m, 3H), 1.24 (m, 3H), 1.05 (d, J = 6.6 Hz, 3H), 0.98 (d, J = 6.6 Hz, 3H), 0.9 (m, 6H); ³¹P NMR (CDCl₃) δ 17.7, 15.1.

### Example 2

Monophospholactate 3: A solution of 1 (0.11 g, 0.15 mmol) and α-hydroxyisovaleric acid ethyl-(R)-ester (71 mg, 0.49 mmol) in pyridine (2 mL) was heated to 70°C and 1,3-dicyclohexylcarbodiimide (0.10 g, 0.49 mmol) was added. The reaction mixture was stirred at 70°C for 2 h and cooled to room temperature. The solvent was removed under reduced pressure. The residue was suspended in EtOAc and 1,3-dicyclohexyl urea was filtered off. The product was partitioned between EtOAc and 0.2 N HCl. The EtOAc layer was washed with 0.2 N HCl, H₂O, saturated NaCl, dried with Na₂SO₄, filtered, and concentrated. The crude product was purified by column chromatography on silica gel (3% 2-propanol/CH₂Cl₂) to give the monophospholactate (35 mg, 28%, GS 192772,1/1 diastereomeric mixture) as a white solid: ¹H NMR (CDCl₃) δ 7.71 (d, J = 8.7 Hz, 2H), 7.35-7.13 (m, 7H), 6.98 (d, J = 8.7 Hz, 2H), 6.93-6.83 (dd, 2H), 5.64 (d, J = 5.4 Hz, 1H), 5.04-4.85 (m, 3H), 4.54 (dd, 1H), 4.39 (dd, 1H), 4.21-4.06 (m, 2H), 3.97-3.67 (m, 9H), 3.12-2.75 (m, 7H), 2.27 (m, 1H), 1.83-1.57 (m, 3H), 1.26 (m, 3H), 1.05 (d, J = 6.6 Hz, 3H), 0.98 (d, J = 6.6 Hz, 3H), 0.9 (m, 6H); ³¹P NMR (CDCl₃) δ 17.7, 15.1.

### Example 3

Monophospholactate 4: A solution of 1 (0.10 g, 0.13 mmol) and methyl-2,2-dimethyl-3-hydroxypropionate (56 µL, 0.44 mmol) in pyridine (1 mL) was heated to 70°C and 1,3-dicyclohexylcarbodiimide (91 mg, 0.44 mmol) was added. The reaction mixture was stirred at 70°C for 2 h and cooled to room temperature. The solvent was removed under reduced pressure. The residue was suspended in EtOAc and 1,3-dicyclohexyl urea was filtered off. The product was partitioned between EtOAc and 0.2 N HCL The EtOAc layer was washed with 0.2 N HCl, H₂O, saturated NaCl, dried with Na₂SO₄, filtered, and concentrated. The crude product was purified by column chromatography on silica gel (3% 2-propanol/CH₂Cl₂) to give the monophospholactate (72 mg, 62%, GS 191484) as a white solid: ¹H NMR (CDCl₃) δ 7.71 (d, J = 8.7 Hz, 2H), 7.34 (m, 2H), 7.25-7.14 (m, 5H), 7.00 (d, J = 9.0 Hz, 2H), 6.87 (d, J = 8.7 Hz, 2H), 5.65 (d, J = 5.4 Hz, 1H), 5.05 (m, 2H), 4.38 (d, J = 9.6 Hz, 2H), 4.32-4.20 (m, 2H), 4.00 (m, 2H), 3.87-3.63 (m, 12H), 3.12-2.78 (m, 7H), 1.85-1.67 (m, 3H), 1.20 (m, 6H), 0.91 (d, J = 6.6 Hz, 3H), 0.88 (d, J = 6.6 Hz, 3H); ³¹P NMR (CDCl₃) δ 16.0.

### Example 4

Lactate 5: To a suspension of lactic acid sodium salt (5 g, 44.6 mmol) in 2-propanol (60 mL) was added 4-(3-chloropropyl)morpholine hydrochloride (8.30 g, 44.6 mmol). The reaction mixture was heated to reflux for 18 h and cooled to room temperature. The solid was filtered and the filtrate was recrystallized from EtOAc / hexane to give the lactate (1.2 g, 12%).

### Example 5

Monophospholactate 6: A solution of 1 (0.10 g, 0.13 mmol) and lactate 5 (0.10 g, 0.48 mmol) in pyridine (2 mL) was heated to 70°C and 1,3-dicyclohexylcarbodiimide (0.10 g, 0.49 mmol) was added. The reaction mixture was stirred at 70°C for 2 h and cooled to room temperature. The solvent was removed under reduced pressure. The residue was suspended in EtOAc and 1,3-dicyclohexyl urea was filtered off. The product was partitioned between EtOAc and H₂O. The EtOAc layer was washed with saturated NaCl, dried with Na₂SO₄, filtered, and concentrated. The crude product was purified by column chromatography on silica gel (4% 2-propanol/CH₂Cl₂) to give the monophospholactate (30 mg, 24%, GS 192781, 1/1 diastereomeric mixture) as a white solid: ¹H NMR (CDCl₃) δ 7.71 (d, J = 8.7 Hz, 2H), 7.38-7.15 (m, 7H), 7.00 (d, J = 8.7 Hz, 2H), 6.91 (m, 2H), 5.65 (d, J = 3.3 Hz, 1H), 5.18-4.98 (m, 3H), 4.54 (dd, 1H), 4.42 (dd, 1H), 4.2 (m, 2H), 4.00-3.67 (m, 16H), 3.13-2.77 (m, 7H), 2.4 (m, 5H), 1.85-1.5 (m, 5H), 1.25 (m, 2H), 0.93 (d, J = 6.6 Hz, 3H), 0.88 (d, J = 6.6 Hz, 3H); ³¹P NMR (CDCl₃) δ 17.4, 15.4.

### Example 6

Sulfonamide 8: A solution of dibenzylphosphonate 7 (0.1 g, 0.13 mmol) in CH₂CI₂ (0.5 mL) at 0°C was treated with trifluoroacetic acid (0.25 mL). The solution was stirred for 30 min at 0°C and then warmed to room temperature for an additional 30 min. The reaction mixture was diluted with toluene and concentrated under reduced pressure. The residue was co-evaporated with toluene (2 x), chloroform (2 x), and dried under vacuum to give the ammonium triflate salt which was dissolved in CH₂Cl₂ (1 mL) and cooled to 0°C. Triethylamine (72 µL, 0.52 mmol) was added followed by the treatment of 4-methylpiperazinylsulfonyl chloride (25 mg, 0.13 mmol). The solution was stirred for 1 h at 0°C and the product was partitioned between CH₂Cl₂ and H₂O. The organic phase was washed with saturated NaCl, dried with Na₂SO₄, filtered, and evaporated under reduced pressure. The crude product was purified by column chromatography on silica gel (5% 2-propanol/CH₂Cl₂) to give the sulfonamide 8 (32 mg, 30%, GS 273835) as a white solid: ¹HNMR (CDCl₃) δ 7.35 (m, 10H), 7.11 (d, J = 8.7 Hz, 2H), 6.81 (d, J = 8.7 Hz, 2H), 5.65 (d, J = 5.4 Hz, 1H), 5.2-4.91 (m, 4H), 4.2 (d, J = 10.2 Hz, 2H), 4.0-3.69 (m, 6H), 3.4-3.19 (m, 5H), 3.07-2.75 (m, 5H), 2.45 (m, 4H), 2.3 (s, 3H), 1.89-1.44 (m, 7H), 0.93 (m, 6H); ³¹P NMR (CDCl₃) δ 20.3.

### Example 7

Phosphonic Acid 9: To a solution of 8 (20 mg, 0.02 mmol) in EtOAc (2 mL) and 2-propanol (0.2 mL) was added 10% Pd/C (5 mg). The suspension was stirred under H₂ atmosphere (balloon) at room temperature overnight. The reaction mixture was filtered through a plug of celite. The filtrate was concentrated and dried under vacuum to give the phosphonic acid (10 mg, 64%) as a white solid.

### Example 8

Dibenzylphosphonate 11: A solution of 10 (85 mg, 0.15 mmol) and 1*H*-tetrazole (14 mg, 0.20 mmol) in CH₂Cl₂ (2 mL) was treated with Dibenzyldiisopropylphosphoramidite (60 µL, 0.20 mmol) and stirred at room temperature overnight. The product was partitioned between CH₂Cl₂ and H₂O, dried with Na₂SO₄, filtered and concentrated. The crude product was purified by column chromatography to give the intermediate dibenzylphosphite (85 mg, 0.11 mmol) which was dissolved in CH₃CN (2 mL) and treated with iodobenzenediacetate (51 mg, 0.16 mmol). The reaction mixture was stirred at room temperature for 3 h and concentrated. The residue was partitioned between EtOAc and NaHC0₃. The organic layer was washed with H₂O, dried with Na₂SO₄, filtered, and concentrated. The crude product was purified by column chromatography on silica gel (3% 2-propanol/CH₂Cl₂) to give the dibenzylphosphonate (45 mg, 52%) as a white solid.

### Example 9

Disodium Salt of Phosphonic Acid 12: To a solution of 11 (25 mg, 0.03 mmol) in EtOAc (2 mL) was added 10% Pd/C (10 mg). The suspension was stirred under H₂ atmosphere (balloon) at room temperature for 4 h. The reaction mixture was filtered through a plug of

### Biological assays used for the characterization of PI prodrugs

### HIV-1 Protease Enzyme Assay, (Ki)

The assay is based on the fluorimetric detection of synthetic hexapeptide substrate cleavage by HIV-1 protease in a defined reaction buffer as initially described by M.V.Toth and G.R.Marshall, Int. J. Peptide Protein Res. 36, 544 (1990)

*Substrate:* (2-aminobenzoyl)Thr-Ile-Nle-(p-nitro)Phe-Gln-Arg
Substrate supplied by Bachem California, Inc. (Torrance, CA; Cat. no. H-2992)

*Enzyme:* recombinant HIV-1 protease expressed in E.Coli
Enzyme supplied by Bachem California, Inc. (Torrance, CA; Cat. no. H-9040)
- *Reaction buffer:*: 100 mM ammonium acetate, pH 5.3
1 M sodium chloride
1 mM ethylendiaminetetraacetic acid
1 mM dithiothreitol
10% dimethylsulfoxide

### Assay protocol for the determination of inhibition constant Ki:

1. Prepare series of solutions containing identical amount of the enzyme (1 to 2.5 nM) and a tested inhibitor at different concentrations in the reaction buffer
2. Transfer the solutions (190 uL each) into a white 96-well plate
3. Preincubate for 15 min at 37°C
4. Solubilize the substrate in 100% dimethylsulfoxide at a concentration of 800 µM. Start the reaction by adding 10 µL of 800 µM substrate into each well (final substrate concentration of 40 µM
5. Measure the real-time reaction kinetics at 37°C by using Gemini 96-well plate fluorimeter (Molecular Devices, Sunnyvale, CA) at λ(Ex) = 330 nm and λ(Em) = 420 nm
6. Determine initial velocities of the reactions with different inhibitor concentrations and calculate Ki (in picomolar concentration units) value by using EnzFitter program (Biosoft, Cambridge, U.K.) according to an algorithm for tight-binding competitive inhibition described by Ermolieff J., Lin X., and Tang J., Biochemistry 36, 12364 (1997)

### Anti-HIV-1 Cell Culture Assay (EC₅₀)

The assay is based on quantification of the HIV 1-associated cytopathic effect by a colorimetric detection of the viability of virus-infected cells in the presence or absence of tested inhibitors. The HIV-1-induced cell death is determined using a metabolic substrate 2,3-bis(2-methoxy-4-nitro-5-sulfophenyl)-2H-tetrazolium-5-carboxanilide (XTT) which is converted only by intact cells into a product with specific absorption characteristics as described by Weislow OS, Kiser R, Fine DL, Bader J, Shoemaker RH and Boyd MR, J. NatL Cancer Inst. 81, 577 (1989).

### Assay protocol for detennination of EC₅₀:

1. Maintain MT2 cells in RPMI-1640 medium supplemented with 5% fetal bovine serum and antibiotics.
2. Infect the cells with the wild-type HIV-1 strain IIIB (Advanced Biotechnologies, Columbia, MD) for 3 hours at 37°C using the virus inoculum corresponding to a multiplicity of infection equal to 0.01.
3. Prepare a set of solutions containing various concentrations of the tested inhibitor by making 5-fold serial dilutions in 96-well plate (100 µL/well). Distribute the infected cells into the 96-well plate (20,000 cells in 100 µL/well). Include samples with untreated infected and untreated mock-infected control cells.
4. Incubate the cells for 5 days at 37°C.
5. Prepare XTT solution (6 mL per assay plate) at a concentration of 2mg/mL in a phosphate-buffered saline pH 7.4. Heat the solution in water-bath for 5 min at 55°C. Add 50 µL of N-methylphenazonium methasulfate (5 µg/mL) per 6 mL of XTT solution.
6. Remove 100 µL media from each well on the assay plate.
7. Add 100 µL of the XTT substrate solution per well and incubate at 37°C for 45 to 60 min in a CO₂ incubator.
8. Add 20 µL of 2% Triton X-100 per well to inactivate the virus.
9. Read the absorbance at 450 nm with subtracting off the background absorbance at 650 nm.
10. Plot the percentage absorbance relative to untreated control and estimate the EC₅₀ value as drug concentration resulting in a 50% protection of the infected cells.

### Cytotoxicity Cell Culture Assay (CC₅₀):

The assay is based on the evaluation of cytotoxic effect of tested compounds using a metabolic substrate 2,3-bis(2-methoxy-4-nitro-5-sulfophenyl)-2H-tetrazolium 5-carboxanilide (XTT) as described by Weislow OS, Kiser R, Fine DL, Bader J, Shoemaker RH and Boyd MR, J. Natl. Cancer Inst. 81, 577 (1989).

### Assay protocol for determination of CC₅₀:

1. Maintain MT-2 cells in RPMI-1640 medium supplemented with 5% fetal bovine serum and antibiotics.
2. Prepare a set of solutions containing various concentrations of the tested inhibitor by making 5-fold serial dilutions in 96-well plate (100 µL, /well). Distribute cells into the 96-well plate (20,000 cells in 100 µL/well). Include samples with untreated cells as a control.
3. Incubate the cells for 5 days at 37°C.
4. Prepare XTT solution (6 mL per assay plate) in dark at a concentration of 2mg/mL in a phosphate-buffered saline pH 7.4. Heat the solution in a water-bath at 55°C for 5 min. Add 50 µL of N-methylphenazonium methasulfate (5 µg/mL) per 6 mL of XTT solution.
5. Remove 100µL, media from each well on the assay plate and add 100 µL of the XTT substrate solution per well. Incubate at 37°C for 45 to 60 min in a CO₂ incubator.
6. Add 20 µL of 2% Triton X-100 per well to stop the metabolic conversion of XTT.
7. Read the absorbance at 450 nm with subtracting off the background at 650 nm.
8. Plot the percentage absorbance relative to untreated control and estimate the CC50 value as drug concentration resulting in a 50% inhibition of the cell growth. Consider the absorbance being directly proportional to the cell growth.

### Resistance Evaluation (150V and 184VlL90M fold change)

The assay is based on the determination of a difference in the susceptibility to a particular HIV protease inhibitor between the wild-type HIV-1 strain and a mutant HIV-1 strain containing specific drug resistance-associated mutation(s) in the viral protease gene. The absolute susceptibility of each virus (EC₅₀) to a particular tested compound is measured by using the XTT-based cytopathic assay as described above. The degree of resistance to a tested compound is calculated as fold difference in EC₅₀ between the wild type and a specific mutant virus. This represents a standard approach for HIV drug resistance evaluation as documented in various publications (e.g. Maguire et al., Antimicrob. Agents Chemother. 46: 731, 2002; Gong et al., Antimicrob. Agents Chemother. 44: 2319,2000; Vandamme and De Clercq, in Antiviral Therapy (Ed. E. De Clercq), pp. 243, ASM Press, Washington, DC, 2001).

### HIV-1 strains used for the resistance evaluation:

Two strains of mutant viruses containing 150V mutation in the protease gene have been used in the resistance assays: one with M46I/I47V/I50V mutations (designated 150V #1) and the other with L10I/M46I/I50V (designated I50V #2) mutations in the viral protease gene. A third virus with 184V/L90M mutations was also employed in the resistance assays. Mutants I50V #1 and I84V/L90M were constructed by a homologous recombination between three overlapping DNA fragments: 1. linearized plasmid containing wild-type HIV-1 proviral DNA (strain HXB2D) with the protease and reverse transcriptase genes deleted, 2. DNA fragment generated by PCR amplification containing reverse transcriptase gene from HXB2D strain (wild-type), 3. DNA fragment of mutated viral protease gene that has been generated by PCR amplification. An approach similar to that described by Shi and Mellors in Antimicrob. Agents Chemother. 41: 2781-85, 1997 was used for the construction of mutant viruses from the generated DNA fragments. Mixture of DNA fragments was delivered into Sup-Tl cells by using a standard electroporation technique. The cells were cultured in RPMI-1640 medium supplemented with 10% fetal bovine serum and antibiotics until the recombinant virus emerged (usually 10 to 15 days following the electroporation). Cell culture supernatant containing the recombinant virus was harvested and stored in aliquots. After verification of protease gene sequence and determination of the infectious virus titer, the viral stock was used for drug resistance studies. Mutant I50V #2 is an amprenavir-resistant HIV-1 strain selected *in vitro* from the wild-type IIIB strain in the presence of increasing concentration of amprenavir over a period of > 9 months using an approach similar to that described by Partaledis et al., J. Virol. 69: 5228-5235, 1995. Virus capable of growing in the presence of 5 µM amprenavir was harvested from the supernatant of infected cells and used for resistance assays following the titration and protease gene sequencing.

### Example Section X

### Activity of the Tested Compound

The enzyme inhibitory potency (Ki), antiviral activity (EC50), and cytotoxicity (CC50) of the tested compounds are summarized in Table 1.

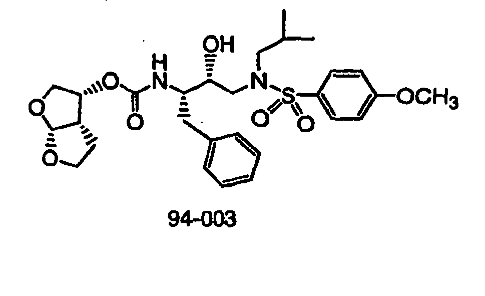

**Table 1: Enzyme inhibition activity (Ki), antiviral cell culture activity (EC50), and cytotoxicity (CC50) of the tested compounds.**

| Substitution of (P1)phenyl | Compound | Phosphonate substitution | HIV-1 protease inhibition Ki [pM] | Anti-HIV-1 Cell Culture Activity EC50 [nM] | Cytotoxicity CC50 [µM] |
|---|---|---|---|---|---|
| none | Amprenavir | none | 45.6 ± 18.2 | 16 ± 2.2 | |
| none | 94-003 | none | 1.46 ± 0.58 | 1.4 ± 0.3 | |
| phosphonyl | 27 | diacid | 11.8 ± 6.0 | > 100,000 | > 100 |
| | 28 | diethyl | 1.2 ± 0.8 | 5.0 ± 2.8 | 70 |
| phosphonyl methoxy | 11 | diacid | 2.1 ± 0.2 | 4,800 ± 1,800 | > 100 |
| | 13 | diethyl | 2.6 ±1.5 | 3.0 ± 0 | 50 |
| | 14 | dibenzyl | 12.7 ± 1.9 | 2.3 ± 0.4 | 35 |
| | 16c | bis(Ala-ethylester) | 15.4 ± 0.85 | 105 ± 43 | 60 |
| | 16d | bis(Ala-butylester) | 18.75 ± 3.04 | 6.0 ± 1.4 | |
| | 16e | bis(ABA-ethylester) | 8.8 ± 1.7 | 12.5 ± 3.5 | |
| | 16f | bis(ABA-butylester) | 3.5 ± 1.4 | 4.8 ± 1.8 | |
| | 16a | bis(Gly-ethylester) | 29 ± 8.2 | 330 ± 230 | |
| | 16b | bis(Gly butylester) | 4.9 ± 1.8 | 17.5 ± 10.5 | |
| | 16g | bis(Leu-ethylester) | 29 ± 9 | 6.8 ± 0.4 | |
| | 16h | bis(Leu-butylester) | 31.7 ± 19.3 | 120 ± 42 | |
| | 16i | bis(Phe-ethylester) | | 17 ± 12 | |
| | 16j | bis(Phe-butylester) | | 35 ± 7 | |
| | 15 | bis(POC) | 36 | 825 ± 106 | |
| | 11 | Monoethyl, monoacid | 0.45 ± 0.15 | 700 ± 0 | |

### Cross-Resistance Profile Assay

The assay is based on the determination of a difference in the susceptibility to a particular HIV protease inhibitor between the wild-type HIV-1 strain and a recombinant HIV-1 strain expressing specific drug resistance-associated mutation(s) in the viral protease gene. The absolute susceptibility of each virus to a particular tested compound is measured by using the XTT-based cytopathic assay as described in Example B. The degree of resistance to a tested compound is calculated as fold difference in EC50 between the wild type and a specific mutant virus.

### Recombinant HIV-1 strains with resistance mutations in the protease gene:

One mutant virus (82T/84V) was obtained from NIH AIDS Research and Reference Reagent Program (Rockville, MD). Majority of the mutant HIV-1 strains were constructed by a homologous recombination between three overlapping DNA fragments: 1. linearized plasmid containing wild-type HIV-1 proviral DNA (strain HXB2D) with the protease and reverse transcriptase genes deleted, 2. DNA fragment generated by PCR amplification containing reverse transcriptase gene from HXB2D strain (wild-type), 3. DNA fragment generated by RT-PCR amplification from patients plasma samples containing viral protease gene with specific mutations selected during antiretroviral therapy with various protease inhibitors. Additional mutant HIV-1 strains were constructed by a modified procedure relying on a homologous recombination of only two overlapping DNA fragments: 1. linearized plasmid containing wild-type HIV-1 proviral DNA (strain HXB2D) with only the protease gene deleted, and 2. DNA fragment generated by RT-PCR amplification from patients plasma samples containing viral protease gene with specific mutations. In both cases, mixture of DNA fragments was delivered into Sup-T1 cells by using a standard electroporation technique. The cells were cultured in RPMI-1640 medium supplemented with 10% fetal bovine serum and antibiotics until the recombinant virus emerged (usually 10 to 15 days following the electroporation). Cell culture supernatant containing the recombinant virus was harvested and stored in aliquots. After determination of the virus titer the virus stock was used for drug resistance studies.

### Cross-Resistance Profile of the Tested Compounds

Cross-resistance profile of currently used HIV-1 protease inhibitors was compared with that of the newly invented compounds (Table 2).

**Table 2. Cross-resistance profile of HIV-1 protease inhibitors**

| Compound | EC 50 [nM] WT HIV -1 | Fold Change in EC₅₀ Relative to WT HIV-1 | | | | | | | | | | | |
|---|---|---|---|---|---|---|---|---|---|---|---|---|---|
| | | 8K*^{a}* 46I **90M** | 46I **84A** | 10I **48V** 54V **82A** | 46I 47V **50V** | 10R 46I **82T** **84V** | **30N** 50S **82I** 88D | 54V 71V **82S** | 10F 46I 71V **82T** **90M** | 10I **48V** 71V **82A** **90M** | **48V** 54V 71V **82S** | 10I **84V** 71V 73S **90M** | Total No. of Resistant Viruses*^{b}* |
| Amprenavir | 20 | 1.25 | **14** | 2 | **38** | 4 | 0.8 | 4 | **13** | 2.5 | 2 | 10 | 4 |
| Nelfinavir | 14 | 13 | **11** | **11.5** | 2 | 3 | **43** | **12** | **33** | **27** | **12** | **65** | 9 |
| Indinavir | 15 | 4 | **10** | **15** | nd | **7** | 1 | **10** | 13 | **28** | **23** | **43** | 8 |
| Ritonavir | 15 | **34** | **18** | **20** | **13** | **47** | 2 | **20** | **32** | **22** | **>50** | **42** | 10 |
| Saquinavir | 4 | 1 | 2.5 | **11** | 1 | 2.5 | 1 | 3 | 2.5 | **12** | **45** | **40** | 4 |
| Lopinavir | 8 | nd | 9 | nd | **19** | 11 | nd | nd | **7.5** | 4.5 | **60** | **11** | 6 |
| Tipranavir | 80 | nd | 1 | 0.4 | 0.5 | 5 | 0.5 | 3.5 | 3 | 0.3 | 2 | **nd** | 1 |
| 94-003 | 0.5 | nd | 8 | 0.5 | **29** | nd | 0.4 | 3.5 | nd | nd | nd | 8 | 3 |
| | | | | | | | | | | | | | |
| **GS 16503** | **16** | 1.2 | 1 | 0.4 | 3.3 | 1 | 0.6 | 0.9 | 1 | 0.4 | 0.5 | 2 | 0 |
| GS 16571 | 22 | 1.8 | 1 | 0.3 | 0.8 | 0.6 | 0.7 | 0.6 | 0.8 | 0.2 | 0.2 | 0.9 | 0 |
| GS 16587 | 15 | 1.5 | 1 | 0.5 | 2 | 1 | 1 | 0.9 | 1 | 0.4 | 0.4 | 1 | 0 |

| | | | | | | | | | | | | | |
|---|---|---|---|---|---|---|---|---|---|---|---|---|---|
| *^{a}* Resistance-associated mutations present in the viral protease. The highlighted changes represent primary resistance mutations. *^{b}* Resistance is considered as a 5-fold and higher change in the EC50 value of the mutant virus relative to the wild-type virus. | | | | | | | | | | | | | |

### Example Section Y

### Plasma and PBMC Exposure Following Intravenous and Oral Administration of Prodrug to Beagle Dogs

The pharmacokinetics of a phosphonate prodrug GS77366 (P1-monoLac-iPr), its active metabolite (metabolite X, or GS77568), and GS8373 were studied in dogs following intravenous and oral administration of the prodrug.

Dose Administration and Sample Collection. The in life phase of this study was conducted in accordance with the USDA Animal Welfare Act and the Public Health Service Policy on Humane Care and Use of Laboratory Animals, and followed the standards for animal husbandry and care found in the Guide for the Care and Use of Laboratory Animals, 7th Edition, Revised 1996. All animal housing and study procedures involving live animals were carried out at a facility which had been accredited by the Association for Assessment and Accreditation of Laboratory Animal Care - International (AAALAC).

Each animal in a group of 4 female beagle dogs was given a bolus dose of GS77366 (P1-monoLac-iPr) intravenously at 1 mg/kg in a formulation containing 40% PEG 300, 20% propylene glycol and 40% of 5% dextrose. Another group of 4 female beagle dogs was dosed with GS77366 via oral gavage at 20 mg/kg in a formulation containing 60% Vitamin-E TPGS, 30% PEG 400 and 10% propylene glycoL

Blood samples were collected pre-dose, and at 5 min, 15 min, 30 min, 1 hr, 2 hr, 4 hr, 8 hr, 12 hr and 24 hr post-dose. Plasma (0.5 to 1 mL) was prepared from each sample and kept at - 70°C until analysis. Blood samples (8 mL) were also collected from each dog at 2, 8 and 24 hr post dose in Becton-Dickinson CPT vacutainer tubes. PBMCs were isolated from the blood by centrifugation for 15 minutes at 1500 to 1800 G. After centrifugation, the fraction containing PBMCs was transferred to a 15 mL conical centrifuge tube and the PBMCs were washed twice with phosphate buffered saline (PBS) without Ca²⁺ and Mg²⁺. The final wash of the cell pellet was kept at -70°C until analysis.

Measurement of the prodrug, metabolite X and GS8373 in plasma and PBMCs. For plasma sample analysis, the samples were processed by a solid phase extraction (SPE) procedure outlined below. Speedisk C18 solid phase extraction cartridges (1 mL, 20 mg, 10 µM, from J.T. Baker) were conditioned with 200 µL of methanol followed by 200 µL of water. An aliquot of 200 µL of plasma sample was applied to each cartridge, followed by two washing steps each with 200 µL of deionized water. The compounds were eluted from the cartridges with a two-step process each with 125 µL of methanol. Each well was added 50 µL of water and mixed. An aliquot of 25 µL of the mixture was injected onto a ThermoFinnigan TSQ Quantum LC/MS/MS system.

The column used in liquid chromatography was HyPURTTY® C18 (50 x 2.1 mm, 3.5 um) from Thermo-Hypersil. Mobile phase A contained 10% acetonitrile in 10 mM ammonium formate, pH 3.0. Mobile phase B contained 90% acetonitrile in 10 mM ammonium formate, pH 4.6. The chromatography was carried out at a flow rate of 250 µL/min under an isocratic condition of 40% mobile phase A and 60% mobile phase B. Selected reaction monitoring (SRM) were used to measure GS77366, GS8373 and Metabolite X with the positive ionization mode on the electrospray probe. The limit of quantitation (LOQ) was 1 nM for GS77366, GS8373 and GS77568 (Metabolite X) in plasma.
For PBMC sample analysis, phosphate buffered saline (PBS) was added to each PBMC pellet to bring the total sample volume to 500 µL in each sample. An aliquot of 150 µL from each PBMC sample was mixed with an equal volume of methanol, followed by the addition of 700 µL of 1% formic acid in water. The resulting mixture was applied to a Speedisk C18 solid phase extraction cartridge (1 mL, 20 mg, 10 um, from J.T. Baker) which had been conditioned as described above. The compounds were eluted with methanol after washing the cartridge 3 times with 10% methanol The solvent was evaporated under a stream of N₂, and the sample was reconstituted in 150 µL of 30% methanol An aliquot of 75 µL of the solution was injected for LC/MS/MS analysis. The limit of quantitation was 0.1 ng/mL in the PBMC suspension.

Pharmacokinetic Calculations. The pharmacokinetic parameters were calculated using WinNonlin. Noncompartmental analysis was used for all pharmacokinetic calculation. The intracellular concentrations in PBMCs were calculated from the measured concentrations in PBMC suspension on the basis of a reported volume of 0.2 picoliter/cell (B.L: Robins, R.V. Srinivas, C.Kim, N.Bischofberger, and A.Fridland, (1998) Antimicrob. Agents Chemother. 42, 612).

### Plasma and PBMC Concentration-time Profiles.

The concentration-time profiles of GS77366, GS77568 and GS8373 in plasma and PBMCs following intravenous dosing of GS77366 were compared at 1 mg/kg in dogs. The data demonstrate that the prodrug can effectively deliver the active components (metabolite X and GS8373) into cells that are primarily responsible for HIV replication, and that the active components in these cells had much longer half life than in plasma.
The pharmacokinetic properties of GS77568 in PBMCs following oral administration of GS77366 in dogs are compared with that of nelfinavir and amprenavir, two marketed HIV protease inhibitors (Table 3). These data show that the active component (GS77568) from the phosphonate prodrug had sustained levels in PBMCs compared to nelfinavir and amprenavir.

**Table 3. Comparison of GS77568 with nelfinavir and amprenavir in PBMCs following oral administration in beagle dogs.**

| Compound | Dose | t_{½}(hr) | AUC_{(2-24 hr)} |
|---|---|---|---|
| Nelfinavir | 17.5 mg/kg | 3.0 hr | 33,000 nM·hr |
| Amprenavir | 20 mg/kg | 1.7 hr | 102,000 nM·hr |
| GS77568 | 20 mg/kg of GS77366 | > 20 hr | 42,200 nM·hr |

### Example Section Z

### Intracellular Metabolism/In Vitro Stability

### 1. Uptake and Persistence in MT2 cells, quiescent and stimulated PBMC

The protease inhibitor (PI) phosphonate prodrugs undergo rapid cell uptake and metabolism to produce acid metabolites including the parent phosphonic acid. Due to the presence of charges, the acid metabolites are significantly more persistent in the cells than non-charged PI's. In order to estimate the relative intracellular levels of the different PI prodrugs, three compounds representative of three classes of phosphonate PI prodrugs - bisamidate phosphonate, monoamidate phenoxy phosphonate and monolactate phenoxy phosphonate (Figure 1) were incubated at 10 µM for 1 hr with MT-2 cells, stimulated and quiescent peripheral blood mononuclear cells (PBMC) (pulse phase). After incubation, the cells were washed, resuspended in the cell culture media and incubated for 24 hr (chase phase). At specific time points, the cells were washed, lysed and the lysates were analyzed by HPLC with UV detection. Typically, the cell lysates were centrifuged and 100 uL of the supernatant were mixed with 200 µL of 7.5 uM amprenavir (Internal Standard) in 80% acetonitrile/20% water and injected into an HPLC system (70 µL).

### HPLC Conditions:

Analytical Column: Prodigy ODS-3, 75 x 4.6, 3u + C18 guard at 40°C.
Gradient:
Mobile Phase A: 20 mM ammonium acetate in 10% ACN/90% H₂O
Mobile Phase B: 20 mM ammonium acetate in 70% ACN/30% H₂O
30-100%B in 4 min, 100%B for 2 min, 30%B for 2 min at 2.5 mL/min.
Run Time: 8 min
UV Detection at 245 nm

Concentrations of Intracellular metabolites were calculated based on cell volume 0.2 µL/mLn cells for PBMC and 0.338 µL / mLn (0.676 uL / mL) for MT-2 cells.

Chemical Structures of Selected Protease Inhibitor Phosphonate Prodrugs and Intracellular Metabolites:

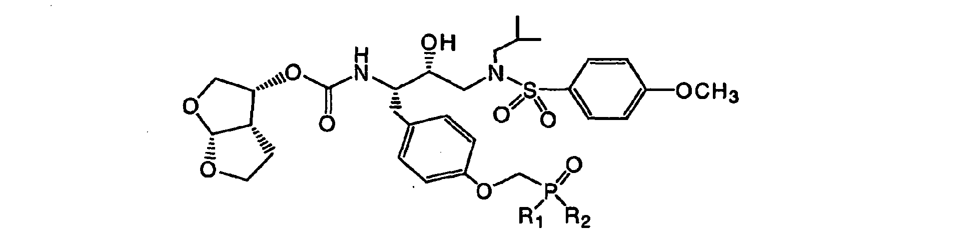

**Table 4:**

| **GS No.** | **R1** | **R2** | **EC₅₀ (nM)** |
|---|---|---|---|
| 8373 | OH | OH | 4,800±1,800 |
| 16503 | HNCH(CH₃)COOBu | HNCH(CH₃)COOBu | 6.0±1.4 |
| 16571 | OPh | HNCH(CH₃)COOEt | 15±5 |
| 17394 | OPh | OCH(CH₃)COOEt | 20±7 |
| 16576 | OPh | HNCH(CH₂CH₃)COOEt | 12.6±4.8 |
| Met X | OH | HNCH(CH₃)COOH | >10,000 |
| Met LX | OH | OCH(CH₃)COOEt | 1750±354 |

A significant uptake and conversion of all 3 compounds in all cell types was observed (Table 4). The uptake in the quiescent PBMC was 2-3-fold greater than in the stimulated cells. GS-16503 and GS-16571 were metabolized to Metabolite X and GS-8373. GS-17394 metabolized to the Metabolite LX. Apparent intracellular half-lives were similar for all metabolites in all cell types (7-12 hr). A persistence of Total Acid Metabolites of Protease Inhibitor Prodrugs in Stimulated (A), Quiescent PBMC (B) and MT-2 Cells (C) (1 hr, 10 uM Pulse, 24 hr Chase) was observed.

### 2. Uptake and Persistence in Stimulated and Quiescent T-cells

Since HIV mainly targets T-lymphocytes, it is important to establish the uptake, metabolism and persistence of the metabolites in the human T-cells. In order to estimate the relative intracellular levels of the different PI prodrugs, GS-16503, 16571 and 17394 were incubated at 10 µM for 1 hr with quiescent and stimulated T-cells (pulse phase). The prodrugs were compared with a non-prodrug PI, nelfinavir. After incubation, the cells were washed, resuspended in the cell culture media and incubated for 4 hr (chase phase). At specific time points, the cells were washed, lysed and the lysates were analyzed by HPLC with UV detection. The sample preparation and analysis were similar to the ones described for MT-2 cells, quiescent and stimulated PBMC.

Table 5 demonstrate the levels of total acid metabolites and corresponding prodrugs in T-cells following pulse/chase and continuous incubation. There was significant cell uptake/metabolism in T-lymphocytes. There was no apparent difference in uptake between stimulated and quiescent T-lymphocytes. There was significantly higher uptake of phosphonate PTs than nelfinavir. GS 17394 demonstrates higher intracellular levels than GS16571 and GS16503. The degree of conversion to acid metabolites varied between different prodrugs. GS-17394 demonstrated the highest degree of conversion, followed by GS-16503 and GS-16571. The metabolites, generally, were an equal mixture of the mono-phosphonic acid metabolite and GS-8373 except for GS-17394, where Metabolite LX was stable, with no GS-8373 formed.

**Table 5. Intracellular Levels of Metabolites and Intact Prodrug Following Continuous and 1 hr Pulse/4 hr Chase Incubation (10 µM/0.7 mLn cells/1 mL) of 10 µM PI Prodrugs and Nelfinavir with Quiescent and Stimulated T-cell**

| Compound | Time (h) | Continuous Incubation | | | | 1 hr Pulse /4 hr Chase | | | |
|---|---|---|---|---|---|---|---|---|---|
| | | Quiescent T-cells | | Stimulated T-cells | | Quiescent T-cells | | Stimulated T-cells | |
| | | Acid Met (µM) | Prodrug (µM) | Acid Met (µM) | Prodrug (µM) | Acid Met (µM) | Prodrug (µM) | Acid Met (µM) | Prodrug (µM) |
| | 0 | 1180 | 42 | 2278 | 0 | 2989 | 40 | 1323 | 139 |
| 16503 | 2 | 3170 | 88 | 1083 | 116 | 1867 | 4 | 1137 | 31 |
| | 4 | 5262 | 0 | 3198 | 31 | 1054 | 119 | 1008 | 0 |
| | | | | | | | | | |
| | 0 | 388 | 1392 | 187 | 1417 | 1042 | 181 | 858 | 218 |
| 16571 | 2 | 947 | 841 | 1895 | 807 | 1170 | 82 | 1006 | 35 |
| | 4 | 3518 | 464 | 6147 | 474 | 1176 | 37 | 616 | 25 |
| | | | | | | | | | |
| | 0 | 948 | 1155 | 186 | 1194 | 4480 | 14 | 2818 | 10 |
| 17394 | 2 | 7231 | 413 | 3748- | 471 | 2898 | 33 | 1083 | 51 |
| | 4 | 10153 | 167 | 3867 | 228 | 1548 | 39 | 943 | 104 |
| | | | | | | | | | |
| | 0 | | 101 | | 86 | | 886 | | 1239 |
| Nelfinavir | 2 | | 856 | | 846 | | 725 | | 770 |
| | 4 | | 992 | | 1526 | | 171 | | 544 |

### 3. PBMC Uptake and Metabolism of Selected PI Prodrugs Following 1-hr Incubation in MT-2 Cells at 10,5 and 1 µM.

To were similar to the determine if the cell uptake/metabolism is concentration dependent, selected PI's were incubated with the 1 mL of MT-2 cell suspension (2.74 mLn cells/mL) for 1 hr at 37°C at 3 different concentrations: 10, 5 and 1 µM. Following incubation, cells were washed twice with the cell culture medium, lysed and assayed using HPLC with UV detection. The sample preparation and analysis ones described for MT-2 cells, quiescent and stimulated PBMC. Intracellular concentrations were calculated based on cell count, a published single cell volume of 0.338 pl for MT-2 cells, and concentrations of analytes in cell lysates. Data are shown in Table 6.
Uptake of all three selected PI's in MT-2 cells appears to be concentration-independent in the 1-10 µM range. Metabolism (conversion to acid metabolites) appeared to be concentration-dependent for GS-16503 and GS-16577 (3-fold increase at 1 µM vs. 10 µM) but independent for GS-17394 (monolactate). Conversion from a respective metabolite X to GS-8373 was concentration-independent for both GS-16503 and GS-16577 (no conversion was observed for metabolite LX of GS-17394).

**Table 6. Uptake and Metabolism of Selected PI Prodrugs Following 1-hr Incubation in MT-2 Cells at 10, 5 and 1 µM.**

| Compound | Extracellular Concentration, µM | Cell-Assosiated Prodrug and Metabolites Concentration, µM | | | | % Conversion to acid metabolites |
|---|---|---|---|---|---|---|
| | | Metabolite X | GS8373 | Prodrug | Total | |
| | 10 | 1358 | 0 | 635 | 1993 | 68 |
| GS-17394 | 5 | 916 | 0 | 449 | 1365 | 67 |
| | 1 | 196 | 0 | 63 | 260 | 76 |
| | 10 | 478 | 238 | 2519 | 3235 | 22 |
| GS-16576 | 5 | 250 | 148 | 621 | 1043 | 40 |
| | 1 | 65 | 36 | 61 | 168 | 64 |
| | 10 | 120 | 86 | 1506 | 1712 | 12 |
| GS-16503 | 5 | 58 | 60 | 579 | 697 | 17 |
| | 1 | 12 | 18 | 74 | 104 | 29 |

| | | | | | | |
|---|---|---|---|---|---|---|
| * For GS 16576, Metabolite X is mono-aminobutyric acid | | | | | | |

### 4. PBMC Uptake and Metabolism of Selected PI Prodrugs Following 1-hr Incubation in Human Whole Blood at 10 µM.

In order to estimate the relative intracellular levels of the different PI prodrugs under conditions simulating the in vivo environment, compounds representative of three classes of phosphonate PI prodrugs - bisamidate phosphonate (GS-16503), monoamidate phenoxy phosphonate (GS-16571) and monolactate phenoxy phosphonate(GS-17394) were incubated at 10 µM for 1 hr with intact human whole blood at 37°C. After incubation, PBMC were isolated, then lysed and the lysates were analyzed by HPLC with UV detection. The results of analysis are shown in Table 7. There was significant cell uptake/metabolism following incubation in whole blood. There was no apparent difference in uptake between GS-16503 and GS-16571. GS-17394 demonstrated significantly higher intracellular levels than GS-16571 and GS-16503.

The degree of conversion to acid metabolites varies between different prodrugs after 1 hr incubation. GS-17394 demonstrated the highest degree of conversion, followed by GS-16503 and GS-16571 (Table 7). The metabolites, generally, were an equimolar mixture of the mono-phosphonic acid metabolite and GS-8373 (parent acid) except for GS-17394, where Metabolite LX was stable with no GS-8373 formed.

**Table 7. PBMC Uptake and Metabolism of Selected PI Prodrugs Following 1-hr Incubation in Human Whole Blood at 10 µM (Mean ± SD, N=3).**

| GS# | Intracellular Prodrug and Metabolites Concentration, µM | | | Major Intracellular Metabolites |
|---|---|---|---|---|
| | Acid Metabolite | Prodrug, µM | Total, µM | |
| 16503 | 279 ± 47 | 61 ± 40 | 340 ± 35 | X , GS-8373 |
| 16571 | 319 ± 112 | 137 ± 62 | 432 ± 208 | X, GS-8373 |
| 17394 | 629 ± 303 | 69 ± 85 | 698 ± 301 | LX |

| | | | | |
|---|---|---|---|---|
| * PBMC Intracellular Volume = 0.2 µL/mln | | | | |

### 5. Distribution of PI Prodrugs in PBMC

In order to compare distribution and persistence of PI phosphonate prodrugs with those of non-prodrug PI's, GS-16503, GS-17394 and nelfinavir, were incubated at 10 µM for 1 hr with PBMC (pulse phase). After incubation, the cells were washed, resuspended in the cell culture media and incubated for 20 more hr (chase phase). At specific time points, the cells were washed and lysed. The cell cytosol was separated from membranes by centrifugation at 9000 xg. Both cytosol and membranes were extracted with acetonitrile and analyzed by HPLC with UV detection.

Table 8 shows the levels of total acid metabolites and corresponding prodrugs in the cytosol and membranes before and after the 22 hr chase. Both prodrugs exhibited complete conversion to the acid metabolites (GS-8373 and X for GS-16503 and LX for GS-17394, respectively). The levels of the acid metabolites of the PI phosphonate prodrugs in the cytosol fraction were 2-3-fold greater than those in the membrane fraction after the 1 hr pulse and 10-fold greater after the 22 hr chase. Nelfinavir was present only in the membrane fractions. The uptake of GS-17394 was about 3-fold greater than that of GS-16503 and 30-fold greater than nelfinavir. The metabolites were an equimolar mixture of metabolite X and GS-8373 (parent acid) for GS-16503 and only metabolite LX for GS-17394.

**Table 8. Uptake and Cell Distribution of Metabolites and Intact Prodrugs Following Continuous and 1 hr Pulse/22 hr Chase Incubation of 10 µM PI Prodrugs and Nelfinavir with Quiescent PBMC.**

| GS# | Cell Type | Fraction | Cell-Associated PI, pmol/mln cells | | | |
|---|---|---|---|---|---|---|
| | | | 1 hr Pulse/ 0 hr Chase | | 1 hr Pulse/ 22 hr Chase | |
| | | | Acid Metabolites | Prodrug | Acid Metabolites | Prodrug |
| GS-16503 | PBMC | Membrane | 228 | 0 | 9 | 0 |
| GS-16503 | PBMC | Cytosol | 390 | 0 | 130 | 0 |
| GS-17394 | PBMC | Membrane | 335 | 0 | 26 | 0 |
| GS-17394 | PBMC | Cytosol | 894 | 0 | 249 | 0 |
| Nelfinavir | PBMC | Membrane | | 42 | | 25 |
| Nelfinavir | PBMC | Cytosol | | 0 | | 0 |

Uptake and cell distribution of metabolites and intact prodrugs following 1 hr pulse/22 hr chase incubation of 10 µM PI prodrugs and Nelfinavir with quiescent PBMC were measured.

### 6. PBMC Extract/Dog Plasma/Human Serum Stability of Selected PI Prodrugs

The *in vitro* metabolism and stability of the PI phosphonate prodrugs were determined in PBMC extract, dog plasma and human serum (Table 9). Biological samples listed below (120 µL) were transferred into an 8-tube strip placed in the aluminum 37°C heating block/holder and incubated at 37°C for 5 min. Aliquots (2.5 µL) of solution containing 1 mM of test compounds in DMSO, were transferred to a clean 8-tube strip, placed in the aluminum 37°C heating block/holder. 60 µL aliquots of 80% acetonitrile/20% water containing 7.5 µM of amprenavir as an internal standard for HPLC analysis were placed into five 8-tube strips and kept on ice/refrigerated prior to use. An enzymatic reaction was started by adding 120 µL aliquots of a biological sample to the strip with the test compounds using a multichannel pipet. The strip was immediately vortex-mixed and the reaction mixture (20 µL) was sampled and transferred to the Internal Standard/ACN strip. The sample was considered the time-zero sample (actual time was 1-2 min). Then, at specific time points, the reaction mixture (20 µL) was sampled and transferred to the corresponding IS/ACN strip. Typical sampling times were 6, 20, 60 and 120 min. When all time points were sampled, an 80 µL aliquot of water was added to each tube and strips were centrifuged for 30 min at 3000xG. The supernatants were analyzed with HPLC under the following conditions:
Column: Inertsil ODS-3, 75 x 4.6 mm, 3 µm at 40°C.
Mobile Phase A: 20 mM ammonium acetate in 10%ACN/90%water
Mobile Phase B 20 mM ammonium acetate in 70%ACN/30%water
Gradient: 20% B to 100% B in 4 min, 2 min 100% B, 2 min 20% B
Flow Rate: 2 mL/min
Detection: UV at 243 nm
Run Time: 8 min

The biological samples evaluated were as follows:
PBMC cell extract was prepared from fresh cells using a modified published procedure (A. Pompon, I. Lefebvre, J-L. Imbach, S. Kahn, and D. Farquhar, Antiviral Chemistry & Chemotherapy, 5, 91 - 98 (1994)). Briefly, the extract was prepared as following: The cells were separated from their culture medium by centrifugation (1000 g, 15 min, ambient temperature). The residue (about 100 µL, 3.5 x 10⁸ cells) was resuspended in 4 mL of a buffer (0.010 M HEPES, pH 7.4, 50 mM potassium chloride, 5 mM magnesium chloride and 5 mM dl-dithiothreitol) and sonicated. The lysate was centrifuged (9000 g, 10 min, 4°C) to remove membranes. The upper layer (0.5 mg protein/mL) was stored at -70°C. The reaction mixture contained the cell extract at about 0.5 mg protein/mL.
**Human serum** (pooled normal human serum from George King Biomedical Systems, Inc.). Protein concentration in the reaction mixture was about 60 mg protein/mL.
**Dog Plasma** (pooled normal dog plasma (EDTA) from Pel Freez, Inc.). Protein concentration in the reaction mixture was about 60 mg protein/mL.

**Table 9: PBMC Extract/Dog Plasma/Human Serum Stability of Selected PI Prodrugs**

| GS# | PBMC Extract ¹ T_{1/2}, min | Dog Plasma T_{1/2}, min | Human Serum T_{1/2}, min | HIV EC₅₀ (nM) |
|---|---|---|---|---|
| 16503 | 2 | 368 | >>400 | 6.0 ± 1.4 |
| 16571 | 49 | 126 | 110 | 15 ± 5 |
| 17394 | 15 | 144 | 49 | 20 ± 7 |

### Example Section AA

**Table 10: Enzymatic and Cellular data**

| | |
|---|---|
| Formula II ALPPI activity | |
| 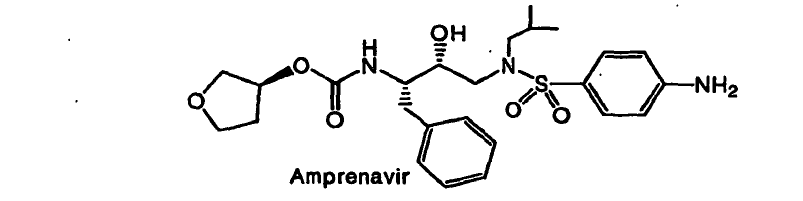 | |
| 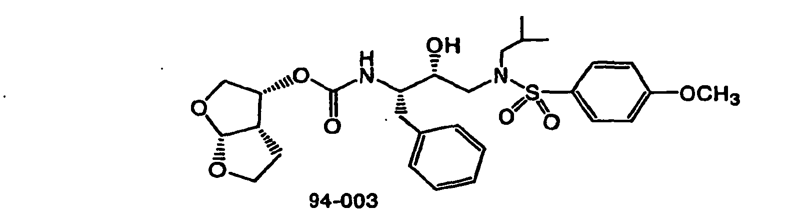 | |

| **Ki[pM]** | |
|---|---|
| ≤10 | +++ |
| >10 to ≤ 100 | ++ |
| > 100 to ≤ 1,000 | + |
| > 1,000 | - |

| EC₅₀ [nM] | |
|---|---|
| ≤ 50 | +++ |
| > 50 to ≤ 500 | ++ |
| > 500 to S 5,000 | + |
| > 5,000 | - |

| I50V and I84V/L90M fold change | |
|---|---|
| > 30 | +++ |
| > 10 to ≤ 30 | ++ |
| > 3 to ≤ 10 | + |
| ≤ 3 | - |

| CC₅₀ [µM] | |
|---|---|
| ≤ 5 | ++ |
| > 5 to ≤ 50 | + |
| >50 | - |

| **Compound** | Ki (pM) | EC₅₀ (nM) | I50V (#1) fold change | I50V (#2) fold change | I84V/L90 M fold change | CC₅₀ (µM) |
|---|---|---|---|---|---|---|
| Saquinavir | ++ | +++ | - | - | +++ | |
| Nelfinavir | + | +++ | - | + | +++ | |
| Indinavir | + | +++ | - | + | +++ | |
| Ritonavir | ++ | +++ | ++ | ++ | +++ | |
| Lopinavir | ++ | +++ | ++ | +++ | ++ | |
| Amprenavir | + | +++ | +++ | +++ | ++ | - |
| Atazanavir | ++ | +++ | - | - | +++ | |
| Tipranavir | ++ | ++ | - | - | + | |
| 94-003 | +++ | +++ | +++ | +++ | ++ | + |
| TMC 114 | +++ | +++ | ++ | ++ | - | |

P1-Phosphonic acid and esters

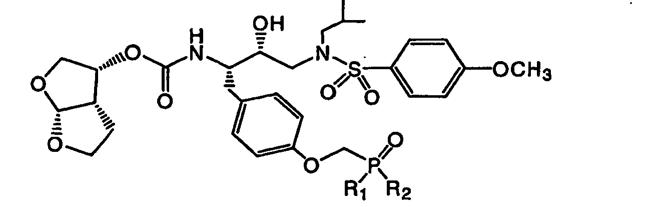

| R1 | R2 | Ki (pM) | EC₅₀ (nM) | I50V (#1) fold change | I84V/L90M fold change | CC₅₀(µM) |
|---|---|---|---|---|---|---|
| OH | OH | +++ | + | - | - | - |
| OMe | OMe | ++ | +++ | | | |
| OEt | OEt | +++ | +++ | - | - | + |
| OCH₂CF₃ | OCH₂CF₃ | ++ | - | | | |
| OiPr | OiPr | ++ | +++ | - | - | |
| OPh | OPh | | +++ | | | |
| OMe | OPh | ++ | +++ | | | |
| OEt | OPh | +++ | +++ | | | |
| OBn | OBn | ++ | +++ | - | - | + |
| OEt | OBn | ++ | +++ | | | ++ |
| OPoc | OPoc | | + | | | |
| OH | OEt | | ++ | | | |
| OH | OPh | +++ | - | | | |
| OH | OBn | | + | - | - | |

P1-Phosphonic acid and esters

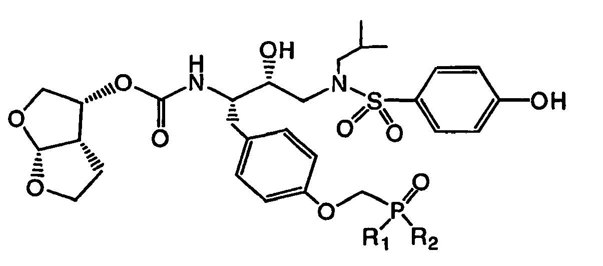

| R1 | R2 | Ki (pM) | EC₅₀ (nM) | 150V (#1) fold change | I84V/L90M fold change | CC₅₀µM |
|---|---|---|---|---|---|---|
| OH | OH | +++ | + | | | |
| Et | Et | +++ | +++ | | | |

P1-Direct phosphonic acid and esters

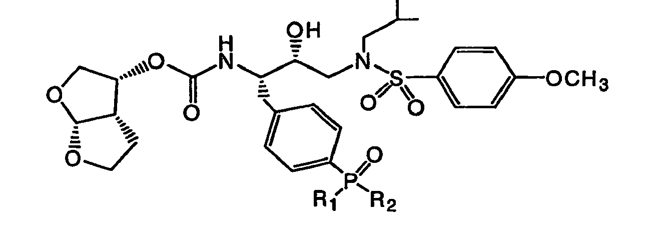

| R1 | R2 | Ki (pM) | EC₅₀ (nM) | I50V (#1) fold change | I84V/L90M fold change | CC₅₀ µM |
|---|---|---|---|---|---|---|
| OH | OH | ++ | - | | | |
| OEt | OEt | +++ | +++ | + | - | |

P1-CH₂-phosphonic acid and esters

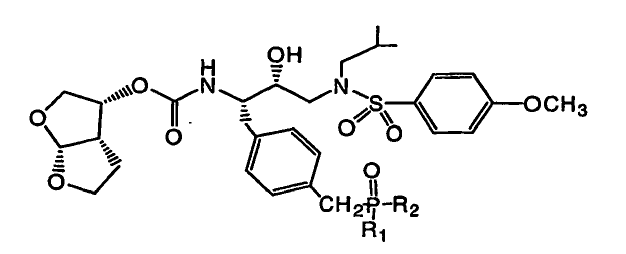

| R1 | R2 | Ki (pM) | EC₅₀ (nM) | 150V (#1) fold change | I84V/L90M fold change | CC₅₀µM |
|---|---|---|---|---|---|---|
| OE | OE | +++ | +++ | + | + | |

P1-P-Bisamidates

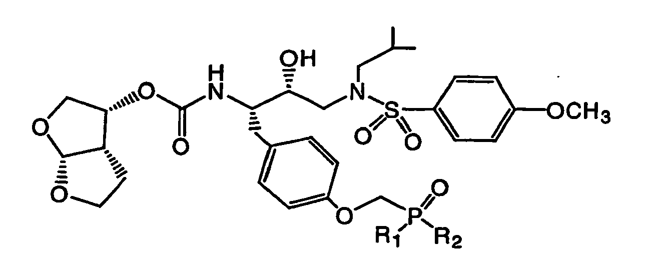

| R1 | R2 | Ki (pM) | EC₅₀ (nM) | I50V (#1) fold change | I84V/L90M fold change | CC₅₀ µM |
|---|---|---|---|---|---|---|
| NHEt | NHEt | +++ | ++ | - | - | |
| Gly-Et | Gly-Et | ++ | ++ | | | |
| Gly-Bu | Gly-Bu | +++ | +++ | | | |
| Ala-Et | Ala-Et | ++ | ++ | | - | - |
| Ala-Bu | Ala-Bu | ++ | +++ | + | - | |
| Aba-Et | Aba-Et | +++ | +++ | | | |
| Aba-Bu | Aba-Bu | +++ | +++ | ++ | + | |
| Val-Et | Val-Et | + | +++ | - | - | |
| Leu-Et | Leu-Et | ++ | +++ | | | |
| Leu-Bu | Leu-Bu | ++ | ++ | + | + | |
| Phe-Et | Phe-Et | | +++ | | | |
| Phe-Bu | Phe-Bu | | +++ | | | |

P1-P-Bislactates

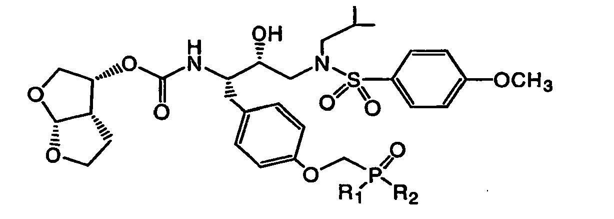

| R1 | R2 | Ki (pM) | EC₅₀ (nM) | I50V (#1) fold change | I84V/L90M fold change | CC₅₀ µM |
|---|---|---|---|---|---|---|
| Glc-Et | Glc-Et | +++ | + | - | - | |
| Lac-Et | Lac-Et | ++ | ++ | - | - | |
| Lac-iPr | Lac-iPr | ++ | +++ | | - | |

P1-P-Monoamidates

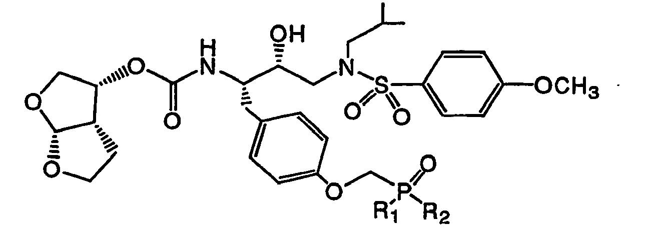

| R1 | R2 | Ki (pM) | EC₅₀ (nM) | 150V (#1) fold change | I84V/L90M fold change | CC₅₀µM |
|---|---|---|---|---|---|---|
| OPh | Gly-Bu | ++ | ++ | - | - | |
| OPh | Ala-Me | ++ | +++ | | - | |
| OPh | Ala-Et | +++ | +++ | - | - | |
| OPh | Ala-iPr | ++ | +++ | - | - | |
| OPh | Ala-iPr | +++ | +++ | | | |
| OPh | Ala-iPr | ++ | +++ | | | |
| OPh | (D)Ala-iPr | ++ | +++ | | - | |
| OPh | (D)Ala-iPr | +++ | +++ | | | |
| OPh | (D)Ala-iPr | +++ | +++ | | | |
| OPh | Ala-Bu | ++ | +++ | - | - | |
| OPh | Ala-Bu | ++ | +++ | - | | |
| OPh | Ala-Bu | ++ | +++ | - | | |
| OPh | Aba-Et | | +++ | | | |
| OPh | Aba-Et | | +++ | - | - | |
| OPh | Aba-Et | | ++ | | | |
| OPh | Aba-Bu | | +++ | + | - | |
| OPh | Aba-Bu | | ++ | - | - | |
| OBn | Ala-Et | +++ | +++ | - | - | |
| OH | Ala-OH | +++ | - | | | |
| OH | Ala-Bu | | - | | | |

P1-P-Monolactates (1)

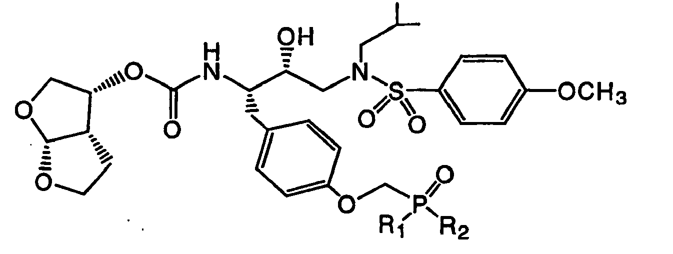

| R1 | R2 | Ki (pM) | EC₅₀ (nM) | 150V (#1) fold change | 150V (#2) fold change | I84V/L90M fold change | CC₅₀µM |
|---|---|---|---|---|---|---|---|
| OPh | Glc-Et | +++ | +++ + | - | | - | |
| OPh | Lac-Me | | ++ | - | | | |
| OPh | Lac-Et | | +++ | - | + | - | + |
| OPh | Lac-Et | +++ | +++ | - | | - | |
| OPh | Lac-Et | ++ | +++ | - | | - | |
| OPh | Lac-iPr | ++ | +++ | - | | - | |
| OPh | Lac-iPr | +++ | +++ | | | | |
| OPh | Lac-iPr | ++ | +++ | | | | |
| OPh | Lac-Bu | ++ | ++ | | | - | |
| OPh | Lac-Bu | ++ | ++ | | | | |
| OPh | Lac-Bu | ++ | ++ | | | | |
| OPh | Lac-EtMor | | - | | | | |
| OPh | Lac-PrMor | | - | | | | |
| OPh | (R)Lac-Me | +++ | +++ | | | | |
| OPh | (R)Lac-Et | +++ | +++ | - | | - | |
| OEt | Lac-Et | | ++ | | | | |
| OCH₂CF₃ | Lac-Et | | ++ | | | | |
| OBn | Lac-Bn | ++ | ++ | | | | |
| OBn | (R)Lac-Bn | | | | | | |
| OH | Lac-OH | +++ | + | | | - | |
| OH | (R)Lac-OH | ++ | + | | | - | |

P1-P-Monolactates (2)

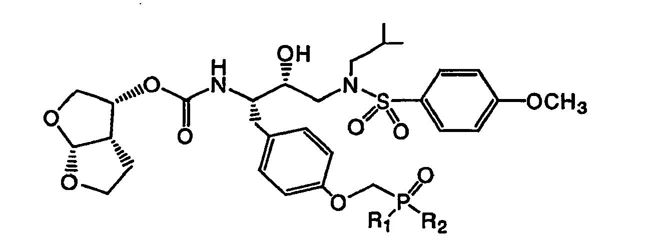

| R1 | R2 | Ki (pM) | EC₅₀ (nM) | I50V (#1) fold change | I84V/L90M fold change | CC₅₀ µM |
|---|---|---|---|---|---|---|
| OPh | mix-Hba-Et | ++ | +++ | + | - | |
| OPh | (S)Hba-Et | + | +++ | | | |
| OPh | (S)Hba-tBu | | +++ | | | |
| OH | (S)Hba-OH | ++ | | | | |
| OPh | (R)Hba-Et | | +++ | | | |
| OPh | (S)MeBut-Et | | +++ | | | |
| OPh | (R)MeBut-Et | | +++ | | | |
| OPh | DiMePro-Me | ++ | | | | |
| OPh | (S)Lac-EtMor | | - | | | |
| OPh | (S)Lac-PrMor | | - | | | |
| OPh | (S)Lac-EtPip | | ++ | - | - | |

P1-P-Monolactates (3)

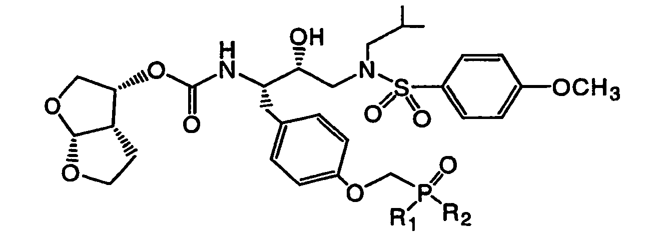

| R1 | R2 | Ki (pM) | EC₅₀ (nM) | 150V (#1) fold change | I84V/L90M fold change | CC₅₀µM |
|---|---|---|---|---|---|---|
| OPh-*o*-i-But | (S)Lac-Et | | +++ | | | |
| OPh-*p*-n-Oct | (S)Lac-Et | | ++ | | | |
| OPh-*p*-n-But | (S)Lac-Et | | +++ | | | |
| OPh-*m*-COOBn | (S)Lac-Et | | ++ | | | |
| OPh-*m*-COOH | (S)Lac-Et | | ++ | | | |
| OPh-*m*-CH₂OH | (S)Lac-Et | | ++ | - | - | |
| OPh-*m*-CH₂NH₂ | (S)Lac-Et | ++ | ++ | | | |
| OPh-*m*-CH₂NM_{C2} | (S)Lac-Et | | + | | | |
| OPh-*m*-M₂Mor | (S)Lac-Et | | ++ | - | - | |
| OPb-*m*-CH₂Pip | (S)Lac-Et | | ++ | | | |
| OPh-*m*-CH₂NMeC2OM | (S)Lac-Et | | ++ | | | |
| OPh-*o*-OEt | (S)Lac-Et | | +++ | | | |
| ONMe₂ | (S)Lac-Et | | ++ | | | |
| OPip | (S)Lac-Et | | + | | | |
| OMor | (S)Lac-Et | | _ | | | |

P1-C₂H₄-P-Monolactates

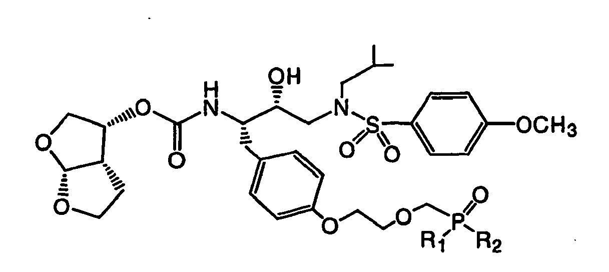

| R1 | R2 | Ki (pM) | EC₅₀ (nM) | I50V (#1) fold change | I84V/L90M fold change | CC₅₀ µM |
|---|---|---|---|---|---|---|
| -OC₂H₄OBn | | | +++ | | | |
| OEt | OEt | | +++ | - | - | |
| OPh | Lac-Et | | ++ | - | - | |
| OH | OH | ++ | | | | |
| OH | Lac | ++ | | | | |

P1-CH₂N-P-diester and monolactate (1)

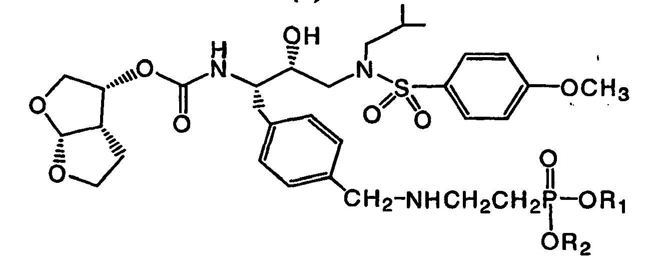

| R₁ | R₂ | Ki (pM) | EC₅₀ (nM) | 150V (#1) fold change | 150V (#2) fold change | I84V/L9M fold change | CC₅₀ µM |
|---|---|---|---|---|---|---|---|
| Et | Et | ++ | +++ | | - | | |
| H | H | ++ | - | | + | | |
| Ph | Lac-Et | | ++ | - | ++ | - | |
| Ph | Lac-Et | | + | | + | - | - |
| Ph | Lac-Et | | + | | ++ | - | |
| Ph | Aba-Et | | + | | + | - | |
| Ph-oEt | Lac-Et | ++ | ++ | - | ++ | - | |
| Ph-dM | Lac-Et | | +++ | | + | + | |
| Ph-dM | Lac-Pr | | +++ | | | | |
| H | Lac | ++ | | | | | |
| Ph | Hba-Et | | ++ | | ++ | - | |
| Ph | Hba-Et | | ++ | | ++ | - | + |
| Ph | Hba-Et | | ++ | | ++ | - | |
| H | Hba | + | | | | | |

P1-CH₂N-P-diester and monolactate (2)

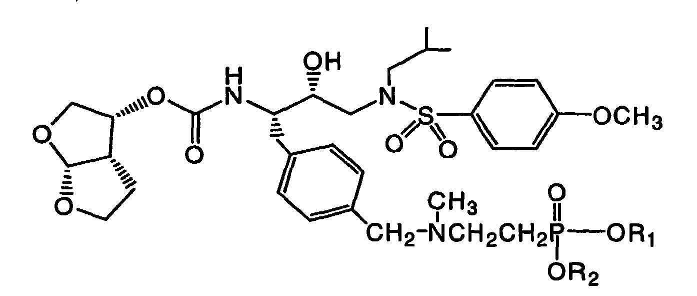

| R₁ | R₂ | Ki (pM) | EC₅₀ (nM) | 150V (#1) fold change | I84V/L90M fold change | CC_{5 0}µM |
|---|---|---|---|---|---|---|
| Ph | Lac-Et | + | ++ | + | + | |
| H | H | ++ | | | | |

P1-CH₂N-P-diester and monolactate (3)

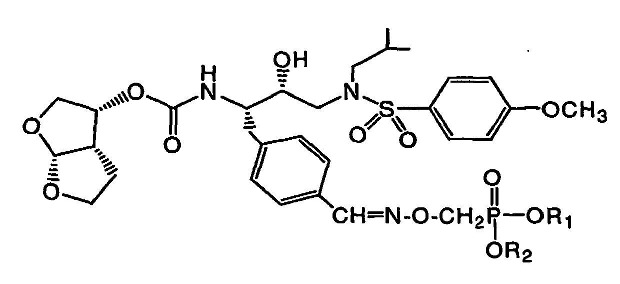

| R₁ | R₂ | Ki (pM) | EC₅₀ (nM) | I50V (#1) fold change | I84V/L90M fold change | CC₅₀µM |
|---|---|---|---|---|---|---|
| Et | Et | ++ | +++ | | - | |

P1-N-P1-Phosphonic acid and esters (1)

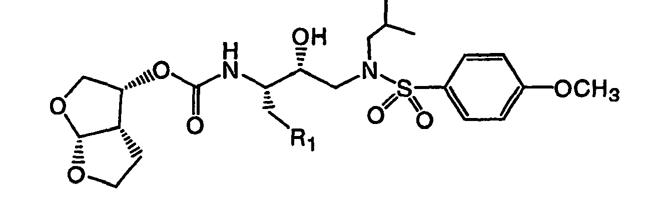

| R1 | Ki (pM) | EC₅₀ (nM) | 150V (#1) fold change | I84V/L90M fold change | CC₅₀ µM |
|---|---|---|---|---|---|
| 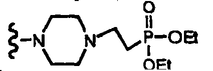 | - | ++ | | | |
| 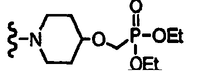 | - | ++ | | | |
| 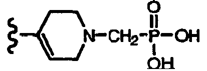 | - | | | | |
| 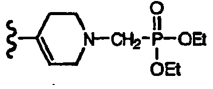 | ++ | +++ | | + | |
| 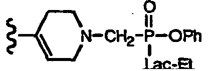 | | - | | | |
| 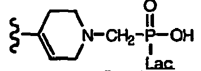 | - | | | | |
| 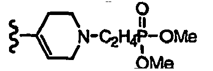 | + | ++ | | | |
| 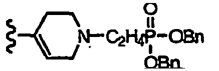 | ++ | +++ | | + | |
| 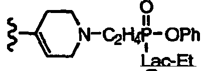 | | - | | | |
| 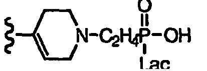 | | - | | | |
| 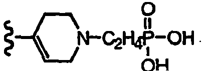 | - | | | | |
| 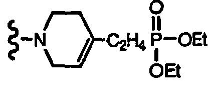 | + | +++ | | + | |

P1-N-P1-Phosphonic acid and esters (2)

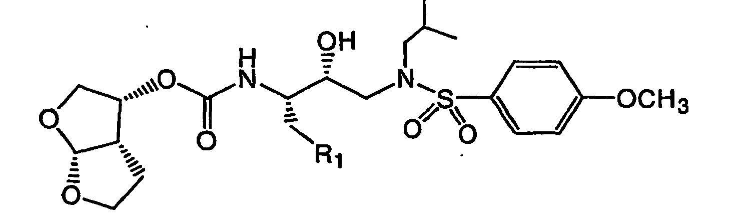

| R1 | Ki (pM) | EC₅₀ (nM) | 150V (#1) fold change | I84V/L90M fold change | CC₅₀ µM |
|---|---|---|---|---|---|
| 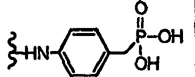 | + | + | | + | |
| 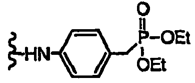 | ++ | +++ | | + | |
| 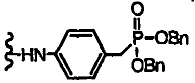 | ++ | +++ | | | |
| 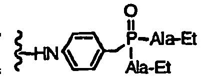 | ++ | ++ | | - | |
| 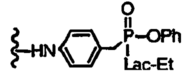 | | +++ | | | |
| 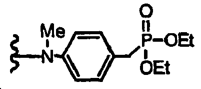 | ++ | +++ | | + | |
| 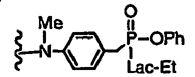 | | +++ | | - | |
| 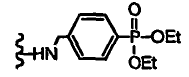 | - | +++ | | ++ | |
| 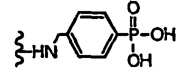 | - | | | | |
| 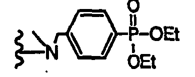 | + | +++ | +++ | - | |
| 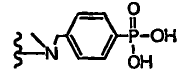 | - | | | | |
| 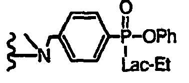 | | +++ | ++ | + | |
| 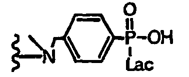 | - | | | | |

P1-N-P1-Phosphonic acid and esters (3)

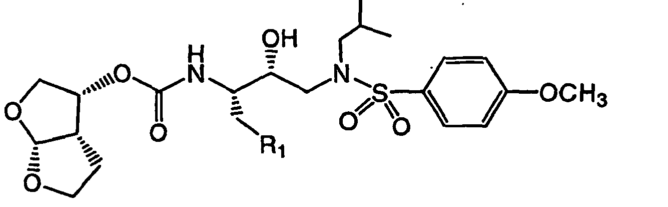

| R1 | Ki (pM) | EC₅₀ (nM) | 150V (#1) fold change | I84V/L90M fold change | CC₅₀µM |
|---|---|---|---|---|---|
| 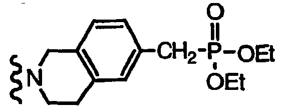 | ++ | +++ | + | + | |
| 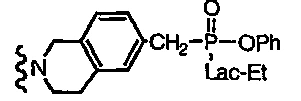 | + | ++ | + | + | |
| 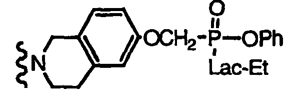 | + | ++ | + | + | |
| 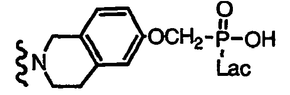 | + | | | | |
| 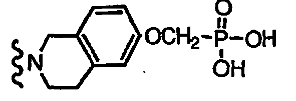 | | | | | |
| 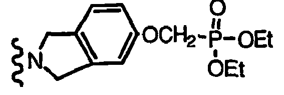 | - | - | | | |

P1-N-P1-Phosphonic acid and esters (4)

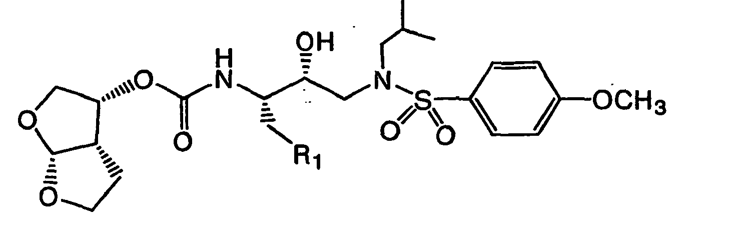

| R₁ | Ki (pM) | EC₅₀ (nM) | 150V (#1) fold change | I84V/L90M fold change | CC₅₀µM |
|---|---|---|---|---|---|
| 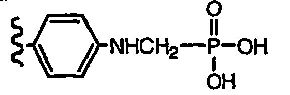 | +++ | | | | |
| 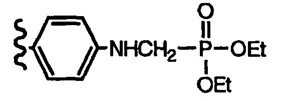 | +++ | +++ | - | - | |
| 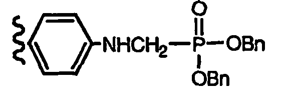 | ++ | +++ | + | - | |
| 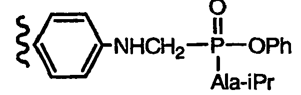 | ++ | +++ | | | |
| 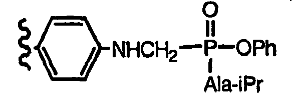 | ++ | ++ | | | |
| 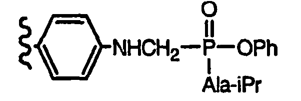 | +++ | +++ | | | |
| 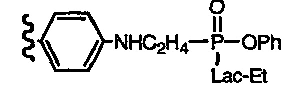 | | +++ | ++ | - | |
| 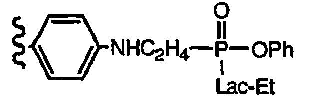 | | +++ | ++ | - | |
| 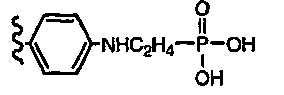 | ++ | | | | |
| 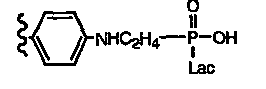 | ++ | | | | |

P1- P-cyclic monolactate

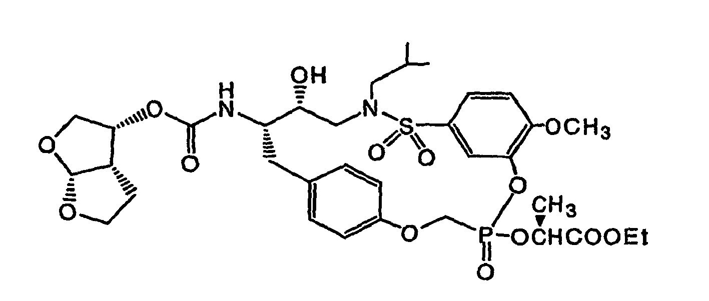

| R₁ | R₂ | Ki (pM) | EC₅₀ (nM) | I50V (#1) fold change | 184V/L90M fold change | CC₅₀µM |
|---|---|---|---|---|---|---|
| | | nd | nd | | | |
| | | nd | nd | | | |

P1'-N-P1-Phosphonic acid and esters

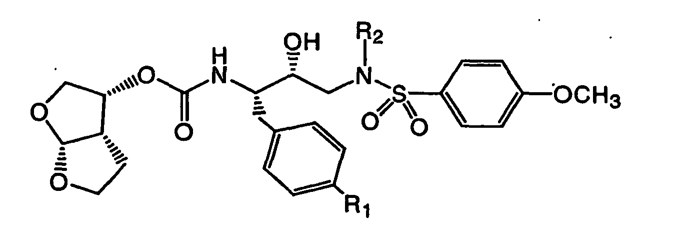

| R1 | R2 | Ki (pM) | EC₅₀ (nM) | 150V (#1) fold change | I84V/L90M fold change | CC₅₀µM |
|---|---|---|---|---|---|---|
| CH₃ | 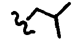 | ++ | +++ | ++ | + | |
| OH | 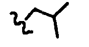 | | +++ | - | - | |
| CH₂OH | 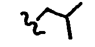 | +++ | +++ | - | - | |
| OBn | 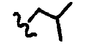 | +++ | +++ | - | - | |
| OH | 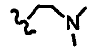 | - | ++ | - | - | |
| OBn | 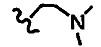 | - | +++ | | - | |
| 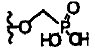 | 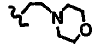 | - | - | + | + | |
| 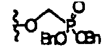 | 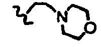 | + | ++ | + | + | |
| OH | 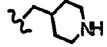 | - | - | | | |
| 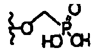 | 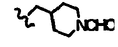 | ++ | - | | | |
| 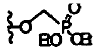 | 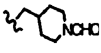 | ++ | - | | | |
| 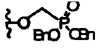 | 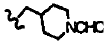 | ++ | ++ | | | |
| 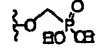 | 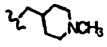 | + | - | | | |

P1'-Phosphonic acid and esters

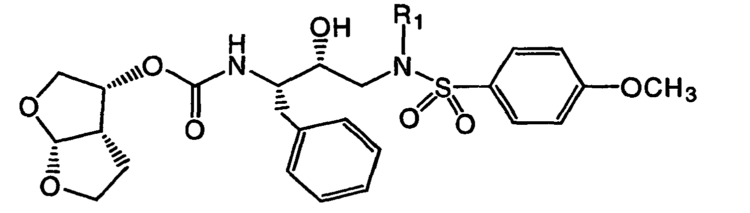

| R1 | Ki (pM) | EC₅₀ (nM) | I50V (#1) fold change | I84VAL90M fold change | CC₅₀ µM |
|---|---|---|---|---|---|
| 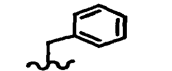 | ++ | +++ | +++ | +++ | |
| 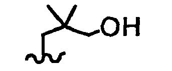 | +++ | +++ | +++ | +++ | |
| 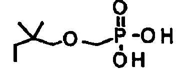 | ++ | + | | +++ | |
| 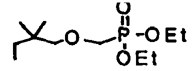 | ++ + | +++ | | +++ | |
| 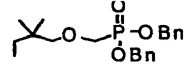 | +++ | +++ | | ++ | |
| 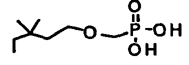 | ++ | ++ | ++ | ++ | |
| 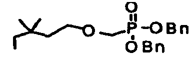 | ++ | +++ | +++ | +++ | |

P2-Monofuran-P1-phosphonic acid and esters

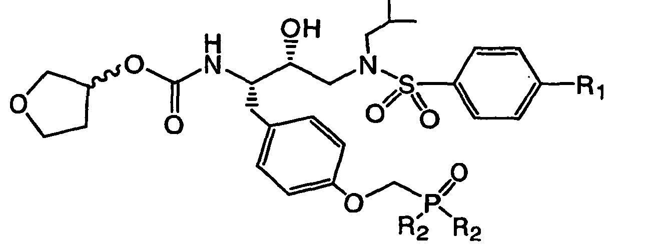

| R1 | R2 | Ki (pM) | EC₅₀ (nM) | 150V (#1) fold change | I84V/L90M fold change | CC₅₀µM |
|---|---|---|---|---|---|---|
| OMe | OH | | - | +++ | +++ | |
| OMe | OEt | +++ | +++ | +++ | ++ | |
| OMe | OBn | | +++ | ++ | ++ | |
| OMe | phenol | +++ | +++ | +++ | + | |
| OMe | OEt | ++ | +++ | +++ | ++ | |
| NH₂ | phenol | + | ++ | + | - | |
| NH₂ | OH | | - | | + | |
| NH₂ | OBn | ++ | ++ | | + | |

P2-Monofuran-P1-P-monoamidates

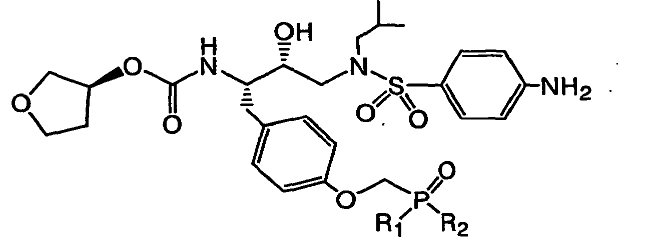

| R1 | R2 | Ki (pM) | EC₅₀ (nM) | 150V (#1) fold change | I84V/L90M fold change | CC₅₀µM |
|---|---|---|---|---|---|---|
| OPh | Ala-iPr | ++ | ++ | | + | |
| OPh | Ala-iPr | ++ | ++ | | | |
| OPh | Ala-iPr | + | ++ | | | |

P2-Other modifications-P1-phosphonic acid and esters

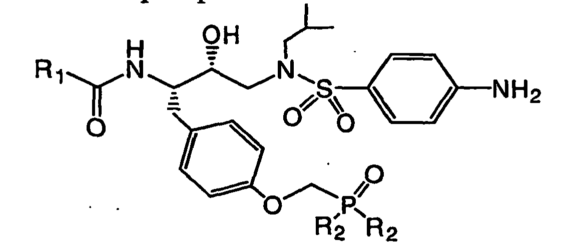

| R1 | R2 | Ki (pM) | EC₅₀ (nM) | I50V (#1) fold change | I84V/L90M fold change | CC₅₀ µM |
|---|---|---|---|---|---|---|
| 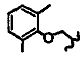 | phenyl | + | +++ | +++ | ++ | |
| 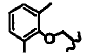 | phenol | + | ++ | ++ | + | |
| 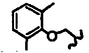 | OH | - | - | ++ | - | |
| 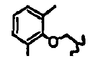 | OBn | + | ++ | + | - | |
| 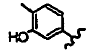 | phenyl | + | ++ | +++ | + | |
| 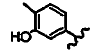 | OH | + | - | ++ | + | |
| 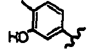 | OBn | + | ++ | +++ | + | |
| 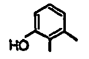 | phenyl | - | ++ | | ++ | |
| 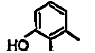 | phenol | + | + | | - | |
| 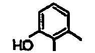 | OH | + | - | - | - | |
| 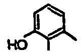 | OBn | ++ | ++ | + | - | |

P2'-Amino-P1-phosphonic acid and esters

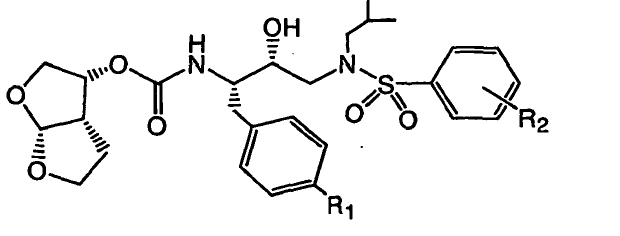

| R1 | R2 | Ki (pM) | EC₅₀ (nM) | 150V (#1) fold change | I84V/L90M fold change | CC₅₀µM |
|---|---|---|---|---|---|---|
| OH | *p*-NH₂ | ++ | ++ | - | - | |
| 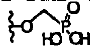 | *p*-NH₂ | ++ | - | + | - | |
| 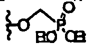 | *p*-NH₂ | ++ | +++ | | - | |
| 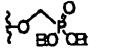 | *p*-NO₂ | ++ | +++ | | - | |
| 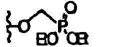 | *p*-NHEt | ++ | +++ | | - | |
| 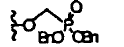 | *p*-NH₂ | ++ | +++ | - | - | |
| OH | *m*-NH₂ | ++ | ++ | | - | |
| 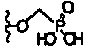 | *m*-NH₂ | ++ | + | | - | |
| 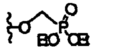 | *m*-NH₂ | ++ | ++ | | - | |
| 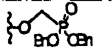 | *m*-NH₂ | ++ | +++ | - | - | |
| 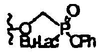 | *m*-NH₂ | + | ++ | - | - | |
| 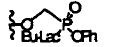 | *m*-NH₂ | ++ | ++ | | | |
| 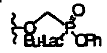 | *m*-NH₂ | + | ++ | | | |

P2'-Substituted-P1-phosphonic acid and esters (1)

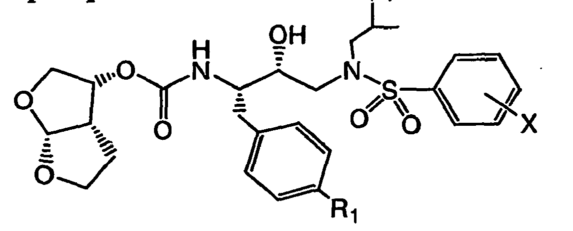

| R1 | X | Ki (pM) | EC₅₀ (nM) | I50V (#1) fold change | I84V/L90M fold change | CC₅₀ µM |
|---|---|---|---|---|---|---|
| 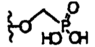 | *p*-OH | +++ | + | | | |
| 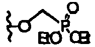 | *p*-OH | +++ | +++ | | | |
| 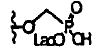 | *p*-OH | ++ | | | | |
| 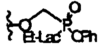 | *p*-OH | | +++ | | - | |
| 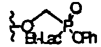 | *p*-OBn | | ++ | | | |
| 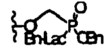 | *p*-OBn | | *-* | | | |
| 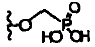 | *p*-H | ++ | - | | | |
| 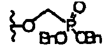 | *p*-H | ++ | +++ | | + | |
| 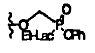 | p-H | | +++ | + | + | |
| 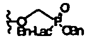 | *p*-H | | ++ | | | |
| 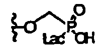 | *p*-H | ++ | | | | |
| 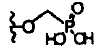 | *p*-F | ++ | + | | | |
| 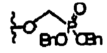 | *p*-F | ++ | +++ | | + | |
| 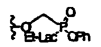 | *p*-F | | +++ | + | + | |
| 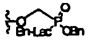 | *p*-F | | ++ | + | + | |
| 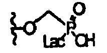 | *p*-F | ++ | | | | |
| 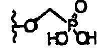 | *p*-CF₃ | +++ | + | | | |
| 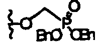 | *p*-CF₃ | ++ | +++ | | - | |
| 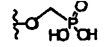 | *p*-OCF₃ | ++ | + | | | |
| 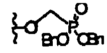 | *p*-OCF₃ | ++ | +++ | | + | |
| 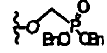 | *p*-CN | ++ | +++ | | - | |
| 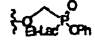 | *p*-Pip | - | - | | | |
| 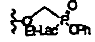 | *p*-Pip-Me | - | - | | | |

P2'-Substituted-P1-phosphonic acid and esters (2)

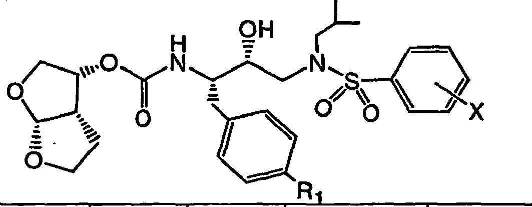

| R1 | X | Ki (pM) | EC₅₀ (nM) | 150V (#1) fold change | I84V/L90M fold change | CC₅₀ µM |
|---|---|---|---|---|---|---|
| 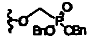 | *m*-Py | ++ | +++ | | | |
| 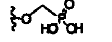 | *m*-Py | ++ | | | | |
| 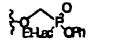 | *m*-Py | ++ | ++ | + | - | |
| 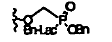 | *m*-Py | ++ | ++ | | | |
| 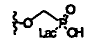 | *m*-Py | ++ | | | | |
| 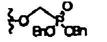 | *m*-Py-Me⁺ | | + | | | |
| 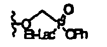 | *m*-Py-Me⁺ | | ++ | | | |
| 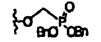 | *m*-Py-oxide | | ++ | | | |
| 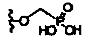 | *m*-Py-oxide | ++ | | | | |
| 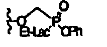 | *m*-Py-oxide | ++ | ++ | | - | |
| 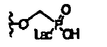 | *m*-Py-oxide | + | | | | |
| 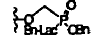 | *m*-Py-oxide | | - | | | |
| *p*-Py-oxide | *p*-OMe | ++ | - | | | |
| 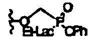 | *p*-CHO | | +++ | | | |
| 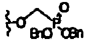 | *p*-CHO | | +++ | | | |
| 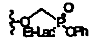 | *p*-CH2 OH | | +++ | - | - | |
| 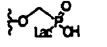 | *p*-CH2 OH | ++ | | | | |
| 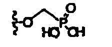 | *p*-CH2 OH | ++ | | | | |
| 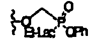 | *p*-CH2 Mor | | ++ | - | - | |
| 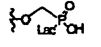 | *p*-CH2 Mor | - | | | | |
| 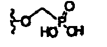 | *p*-CH2 Mor | - | | | | |

P2'-Alkylsulfonyl-P1-phosphonic acid and esters

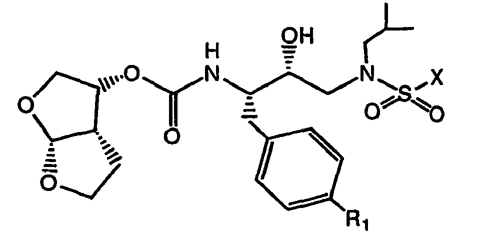

| R1 | X | Ki (pM) | EC₅₀ (nM) | I50V (#1) fold change | I84V/L90M fold change | CC₅₀ µM |
|---|---|---|---|---|---|---|
| 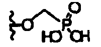 | 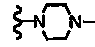 | - | - | | | |
| 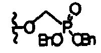 | 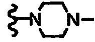 | + | ++ | | | |

P2'-Carbonyl-substituted-P1-phosphonic acid and esters

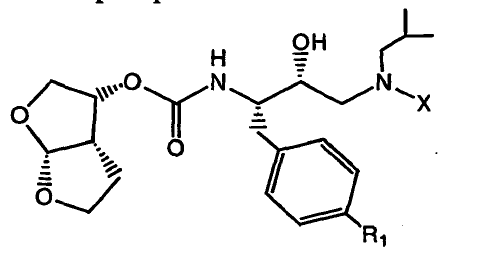

| R1 | X | Ki (pM) | EC₅₀ (nM) | 150V (#1) fold change | 184V/L90M fold change | CC₅₀ µM |
|---|---|---|---|---|---|---|
| 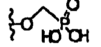 | 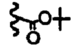 | - | | | | |
| 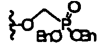 | 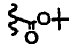 | - | ++ | | | |
| 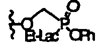 | 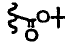 | | + | | | |

P2'-Phosphonic acid and esters

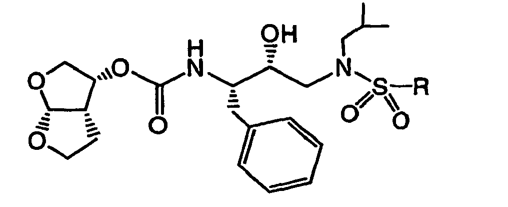

| R | Ki (pM) | EC₅₀ (nM) | 150V (#1) fold change | I84V/L90M fold change | CC₅₀ µM |
|---|---|---|---|---|---|
| 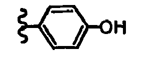 | +++ | +++ | - | - | |
| 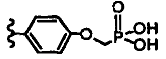 | +++ | + | - | - | |
| 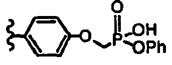 | ++ | - | | | |
| 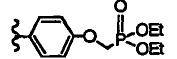 | ++ | +++ | ++ | ++ | |
| 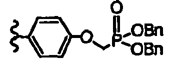 | + | ++ | +++ | +++ | |
| 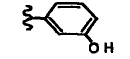 | +++ | +++ | + | + | |
| 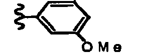 | +++ | +++ | +++ | ++ | |
| 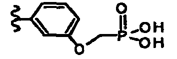 | ++ | ++ | ++ | + | |
| 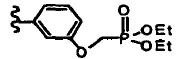 | +++ | +++ | +++ | ++ | |
| 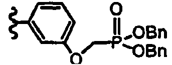 | ++ | +++ | ++ | ++ | |
| 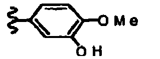 | +++ | +++ | - | - | |
| 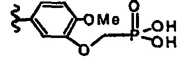 | +++ | ++ | + | - | |
| 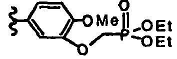 | + | ++ | + | + | |
| 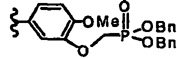 | - | + | +++ | ++ | |
| 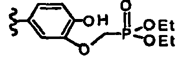 | + | ++ | + | - | |

P2'-P-Bisamidate, monoamidate, and monolactate

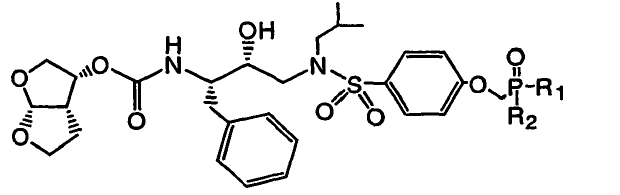

| R₁ | R₂ | Ki (pM) | EC₅₀ (nM) | 150V (#1) fold change | I84V/L90M fold change | CC₅₀µM |
|---|---|---|---|---|---|---|
| Ala-Bu | Ala-Bu | + | ++ | + | + | |
| OPh | Ala-iPr | ++ | ++ | | | |
| OPh | Lac-iPr | + | + | | | |
| OH | Ala-OH | ++ | | | | |

P1-N-P2'-Phosphonic acid and esters

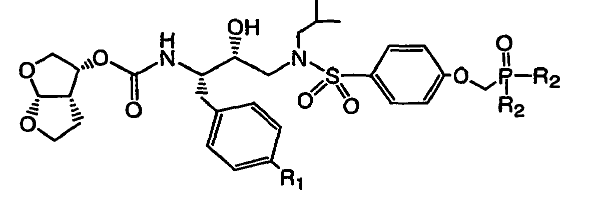

| R₁ | R₂ | Ki (pM) | EC₅₀ (nM) | 150V (#1) fold change | I84V/L90M fold change | CC₅₀ µM |
|---|---|---|---|---|---|---|
| NO₂ | phenol | | +++ | - | | |
| NH₂ | OH | ++ | - | | | |
| NH₂ | OEt | + | ++ | | ++ | |
| NH₂ | OBn | + | + | | + | |
| NMe₂ | OEt | ++ | +++ | | ++ | |
| OH | OH | ++ | - | | | |
| OH | OBn | ++ | ++ | | | |
| OC₂H₄NMe₂ | OH | +++ | + | | | |
| OC₂H₄-NMe₂ | OBn | ++ | ++ | | | |

P1-N-P2'-P-Bisamidate and monoamidate

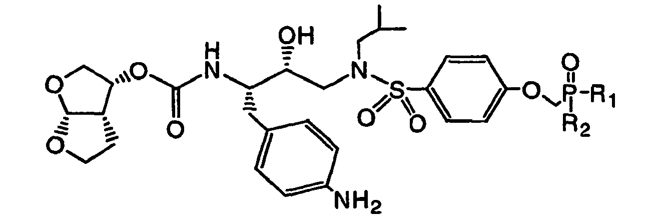

| R₁ | R₂ | Ki (pM) | EC₅₀ (nM) | 150V (#1) fold change | I84V/L90M fold change | CC₅₀ µM |
|---|---|---|---|---|---|---|
| Ala-Bu | Ala-Bu | + | + | | | |
| OPh | Ala-iPr | + | - | | | |
| OPh | Ala-iPr | ++ | - | | | |

P1-NEt-P2'-P-Bisamidate and monoamidate

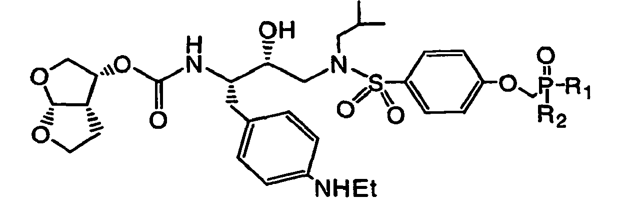

| R₁ | R₂ | Ki (pM) | EC₅₀ (nM) | 150V (#1) fold change | I84V/L90M fold change | CC₅₀ µM |
|---|---|---|---|---|---|---|
| OPh | Ala-iPr | + | + | | | |
| OPh | Ala-iPr | + | + | - | - | |

Phosphate prodrug of ampenavir

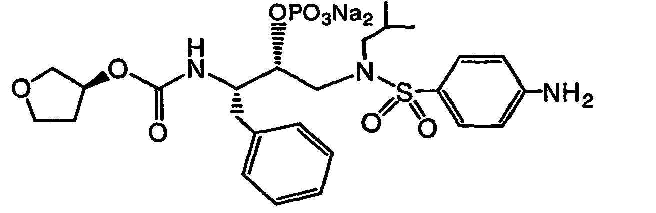

| R₁ | R₂ | Ki (pM) | EC₅₀ (nM) | 150V (#1) fold change | I84V/L90M fold change | CC₅₀ µM |
|---|---|---|---|---|---|---|
| | | ++ | | | | |

Phosphate prodrug of 94-003

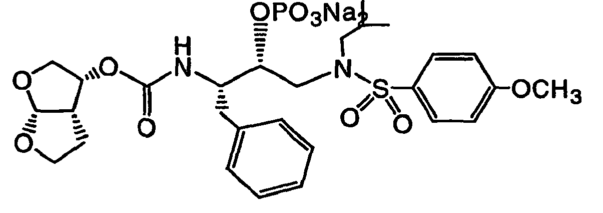

| R₁ | R₂ | Ki (pM) | EC₅₀ (nM) | 150V (#1) fold change | 184VAL90M fold change | CC₅₀ µM |
|---|---|---|---|---|---|---|
| | | | +++ | | | |

Phosphate prodrug of GS77366 (P1-mono(S)Lac-iPr)

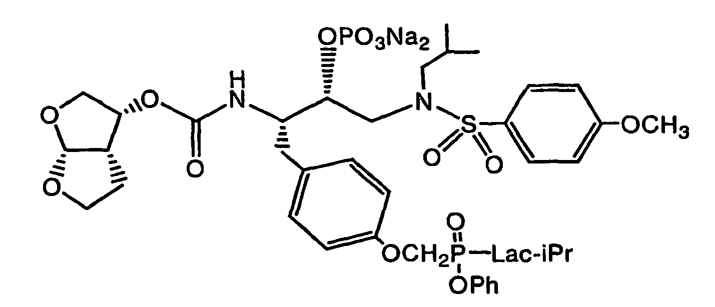

| R₁ | R₂ | Ki (pM) | EC₅₀ (nM) | 150V (#1) fold change | 184V/L90M fold change | CC₅₀ µM |
|---|---|---|---|---|---|---|
| | | | +++ | | | |

Valine prodrug of (P1-mono(S)Lac-Et)

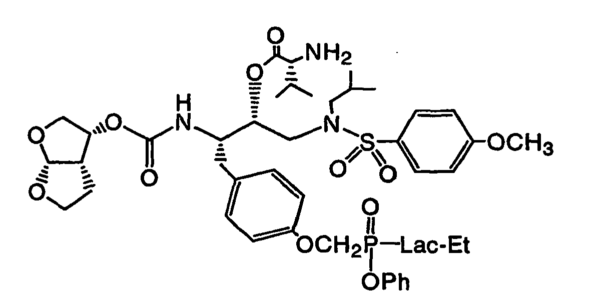

| R₁ | R₂ | Ki (pM) | EC₅₀ (nM) | 150V (#1) fold change | 184V/L90M fold change | CC₅₀µM |
|---|---|---|---|---|---|---|
| | | | ++ | | | |

Valine prodrug of GS278053 (P1-mono(S)Lac-Et,P2'-CH₂OH)

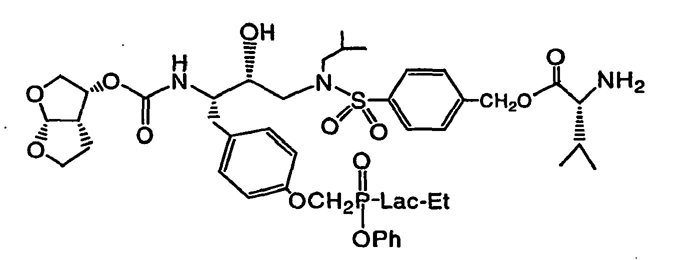

| R₁ | R₂ | Ki (pM) | EC₅₀ (nM) | 150V (#1) fold change | I84V/L90M fold change | CC₅₀ µM |
|---|---|---|---|---|---|---|
| | | | ++ | | | |

Although certain embodiments have been described in detail above, those having ordinary skill in the art will clearly understand that many modifications are possible in the embodiments without departing from the teachings thereof. All such modifications are intended to be encompassed within the claims of the invention.

## Claims

1. A compound selected from the formulae 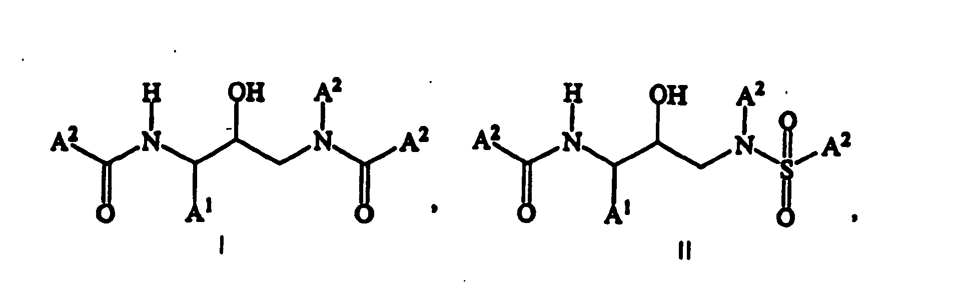 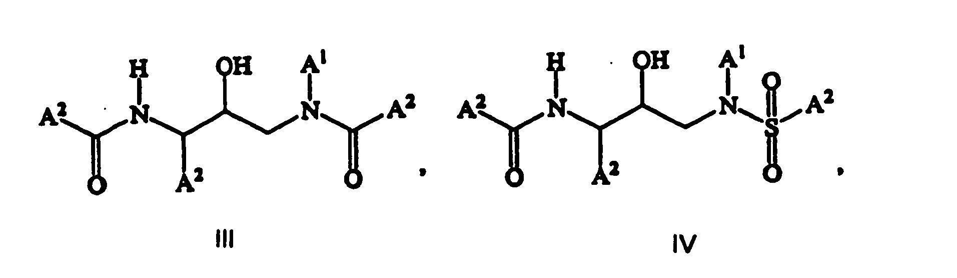 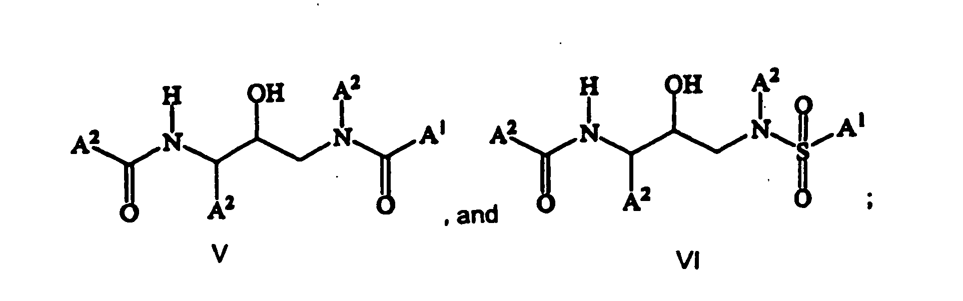 wherein:
A¹ is: 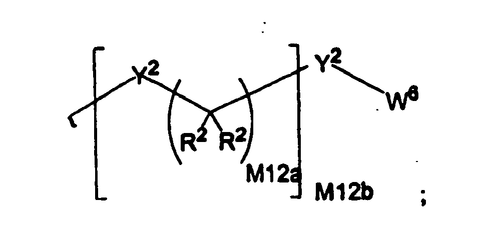
A² is: 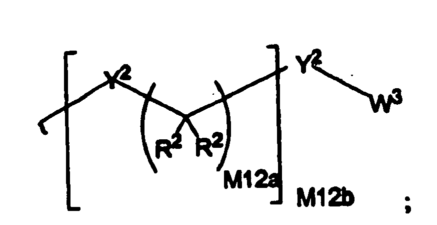
A³ is: 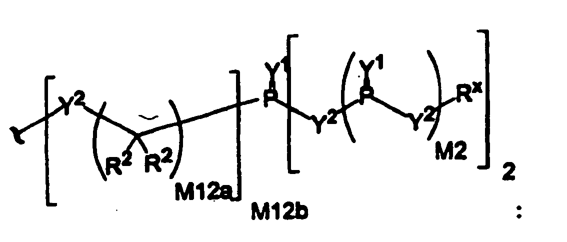
Y¹ is independently O, S, N(R^{x}), N(O)(R^{x}), N(OR^{x}), N(O)(OR^{x}), or N(N(R^{x})(R^{x})):
Y² is independently a bond. O, N(R^{x}), N(O)(R^{x}), N(OR^{x}), N(O)(OR^{x}). N(N(R^{x})(R^{x})), -S(O)_{M2}-, or -S(O)_{M2}-S(O)_{M2}-:
R^{x} is independently H, R¹, W³. a protecting group, or the formula: 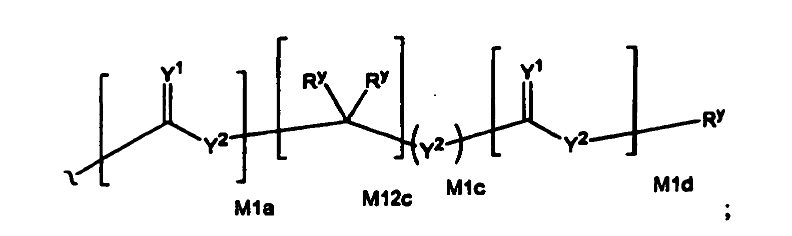
R^{y} is Independently H, W³, R² or a protecting group;
R¹ is Independently H or an alkyl of 1 to 18 carbon atoms;
R² is Independently H. R¹, R³ or R⁴ wherein each R⁴ Is independently substituted with 0 to 3 R³ groups, or taken together at a carbon atom, two R² groups form a ring of 3 to 8 carbons and the ring may be substituted with 0 to 3 R³ groups;
R³ is R^{3a}, R^{3b}, R^{3c} or R^{3d}. provided that when R³ is bound to a heteroatom, then R³ is R^{3c} or R^{3d};
R^{3a} is F, Cl, Br. 1. -CN, N₃ or -NO₂;
R^{3b} is Y¹;
R^{3c} Is -R^{x}, -N(R^{x})(R^{x}), -SR^{x}, -S(O)R^{x}, -S(O)₂R^{x}, -S(O)(OR^{x}), -S(O)₂(OR^{x}), -OC(Y¹)R^{x}, -OC(Y¹)OR^{x}, -OC(Y¹)(N(R^{x})(R^{x})), -SC(Y¹)R^{x}, -SC(Y¹)OR^{x}, -SC(Y¹)(N(R^{x})(R^{x})), -N(R^{x})C(Y¹)R^{x}, -N(R^{x})C(Y¹)OR^{x}, or -N(R^{x})C(Y¹)(N(R^{x})(R^{x}));
R^{3d} is -C(Y¹)R^{x}, -C(Y¹)OR^{x} or -C(Y¹)(N(R^{x})(R^{x}));
R⁴ is an alkyl of 1 to 18 carbon atoms, alkenyl of 2 to 18 carbon atoms, or alkynyl of 2 to 18 carbon atoms;
R⁵ is R⁴ wherein each R⁴ is substituted with 0 to 3 R³ groups;
W³ is W⁴ or W⁵;
W⁴ is R⁵, -C(Y¹)R⁵, -C(Y¹)W⁵, -SO₂R⁵, or SO₂W⁵;
W⁵ is carbocycle or heterocycle wherein W⁵ is Independently substituted with 0 to 3 R² groups;
W⁶ is W³ independently substituted with 1, 2, or 3 A³ groups:
M2 is 0,1 or 2;
M12a is 1, 2, 3, 4, 5, 6, 7, 8, 9, 10, 11 or 12;
M12b is 0, 1, 2, 3, 4, 5, 6, 7, 8, 9, 10, 11 or 12;
M1a, M1c, and M1d are Independently 0 or 1; and
M12c is 0, 1, 2, 3, 4, 5, 6, 7, 8, 9, 10, 11 or 12, and
the enantiomers and diastereomers, as well as the physiologically acceptable salts thereof.

2. A compound of claim 1 having the formula:

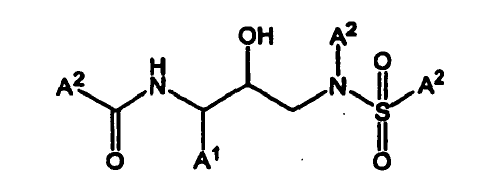

3. A compound of claim 1 having the formula:

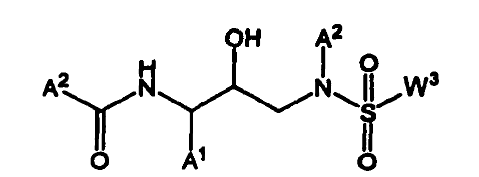

4. The compound of claim 3 having the formula:

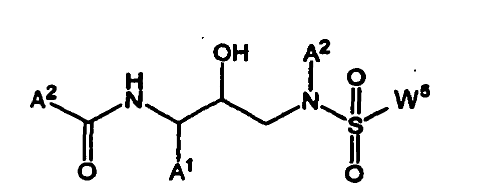

5. A compound of claim 4 having the formula:

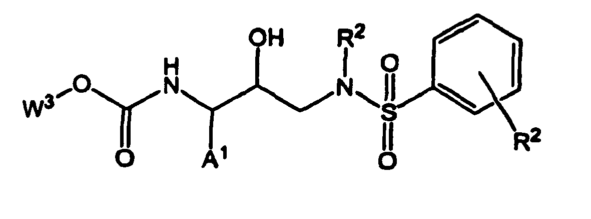

6. A compound of claim 5 having the formula:

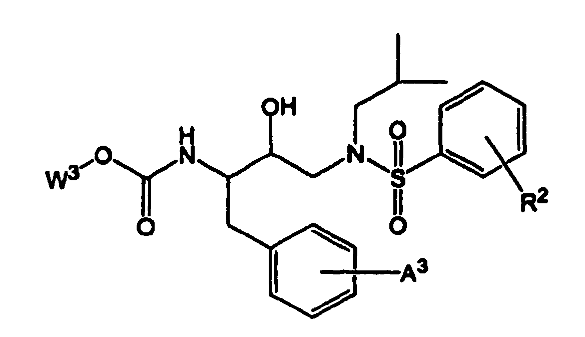

7. A compound of claim 6 having the formula: 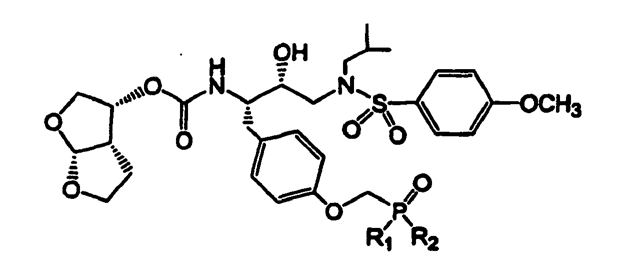 wherein R₁ and R₂ are independently selected from hydroxy, methoxy, ethoxy, trifluoroethoxy, isopropoxy, phenoxy, benzyloxy, and O-pivaloyloxymethyl.

8. A compound of claim 6 having the formula: 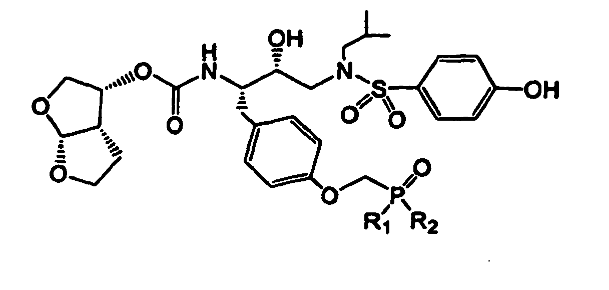 wherein R₁ and R₂ are independently selected from hydroxy, methoxy, ethoxy, trifluoroethoxy, isopropoxy, phenoxy, benzyloxy, and O-pivaloyloxymethyl.

9. A compound of claim 6 having the formula: 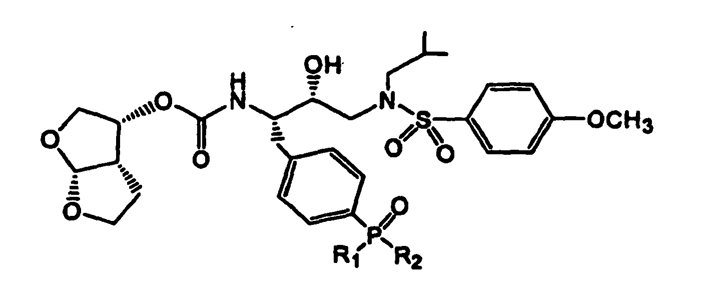 wherein R₁ and R₂ are independently selected from hydroxy, methoxy, ethoxy, trifluoroethoxy, isopropoxy, phenoxy, benzyloxy, and O-pivaloyloxy-methyl.

10. A compound of claim 6 having the formula: 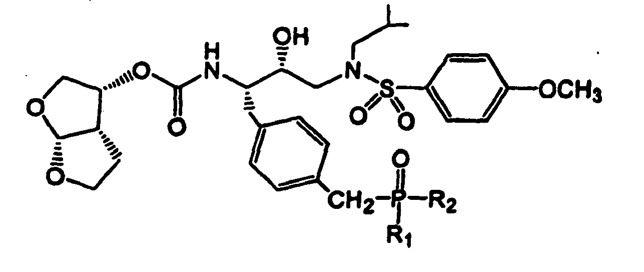 wherein R₁ and R₂ are independently selected from hydroxy, methoxy, ethoxy, trifluoroethoxy, isopropoxy, phenoxy, benzyloxy, and O-pivaloyloxymethyl.

11. A compound of Claim 6 having the formula: 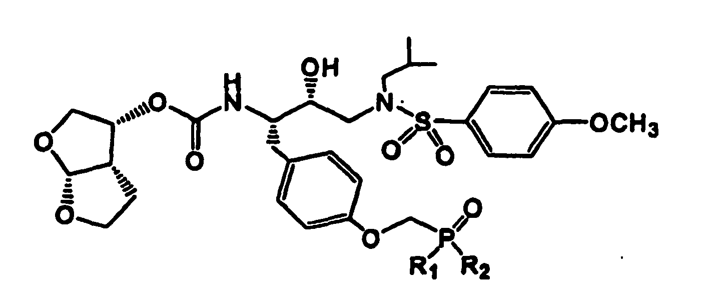 wherein R₁ and R₂ are Independently selected from -NR where R is C₁-C₆ alkyl or an amino acid ester.

12. The compound of claim 11 wherein R₁ and R₂ are independently selected from -NMe, -NEt, Gly-Et, Ala-Et, Aba-Et, Val-Et, Leu-Et, Phe-Bu, and Phe-Et.

13. A compound of claim 6 having the formula: 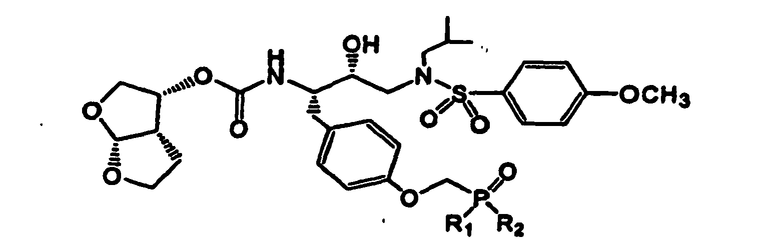 or 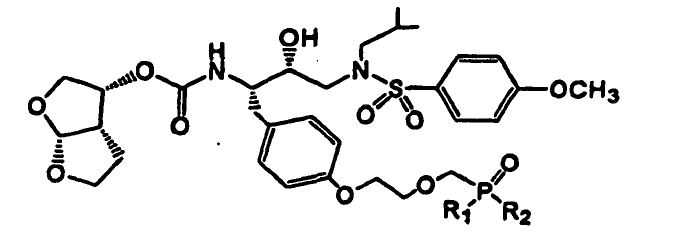 wherein R₁ and R₂ are Independently selected from hydroxy, methoxy, ethoxy, trifluoroethoxy, isopropoxy, phenoxy, benzyloxy, O-pivaloyloxymethyl, and a lactate ester.

14. The compound of claim 13 wherein R₁ is hydroxy, methoxy, ethoxy, trifluoroethoxy, isopropoxy, phenoxy, substituted phenoxy or benzyloxy; and R₂ is Glc-Et. Lac-Me, Lac-Et, Lac-IPr, Lac-Bu, Lac-EtMor, Lac-Me. Lac-Et, Lac-Bn, Lac-OH, Hba-Et, Hba-tBu, Hba-OH, MeBut Et, or DiMePro-Me.

15. A compound of claim 14 where the lactate ester is the (R) configuration.

16. A compound of claim 14 where the lactate ester is the (S) configuration.

17. A compound of claim 6 having the formula : 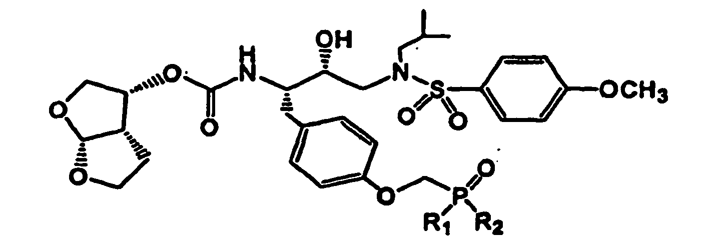 wherein R₁ is phenoxy, benzyloxy, ethoxy, trifluoroethoxy, or hydroxyl; and R₂ is an amino acid ester.

18. The compound of claim 17 wherein the amino acid ester is selected from Gly-Bu, Ala-Me, Ala-Et, Ala-lPr. (D)Ala-iPr, Ala-Bu, Aba-Et, Aba-Bu, and Ala-OH.

19. A compound of claim 6 having the formula: 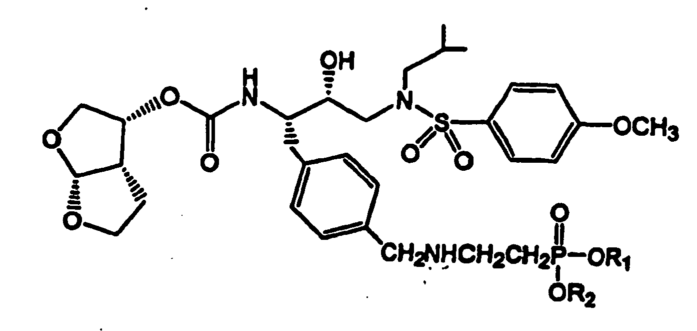 wherein R₁ and R₂ are independently selected from hydroxy, methoxy, ethoxy, trifluoroethoxy, isopropoxy, phenoxy, benzyloxy, O-pivaloyloxymethyl, an amino acid ester and a lactate ester.

20. The compound of claim 19 wherein R₁ is hydroxy, methoxy, ethoxy, trifluoroethoxy, isopropoxy, phenoxy, substituted phenoxy or benzyloxy; and R₂ is a lactate ester selected from Glc-Et, Lac-Me, Lac-Et, Lac-IPr, Lac-Bu, Lac-EtMor, Lac-Me, Lac-Et, Lac-Bn, Lac-OH, Hba-Et, Hba-tBu, Hba-OH, MeBut-Et, and DiMePro-Me.

21. The compound of claim 19 wherein R₁ is hydroxy, methoxy, ethoxy, trifluoroethoxy, isopropoxy, phenoxy, substituted phenoxy or benzyloxy; and R₂ is an amino acid ester is selected from Gly-Bu, Ala-Me, Ala-Et, Ala-iPr, (D)Ala-iPr, Ala-Bu, Aba-Et, Aba-Bu, and Ala-OH.

22. A compound of claim 1 having the formula: 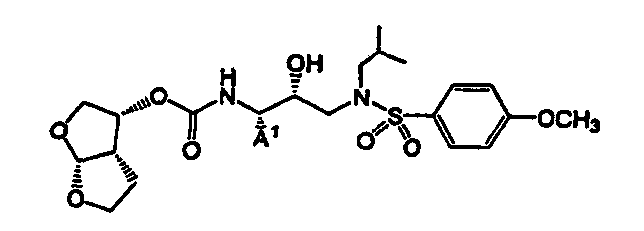 wherein A¹ is selected from the formulas: 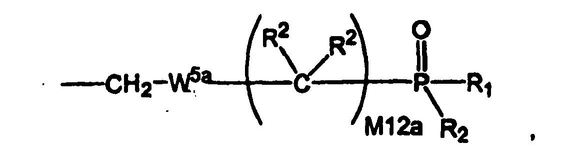 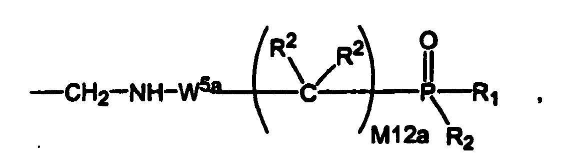 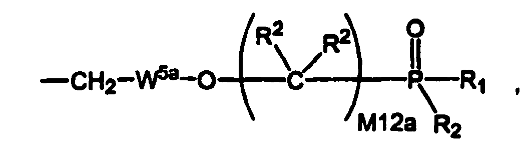 and 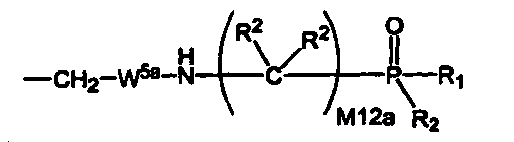 R₁ and R₂ are independently selected from hydroxy, methoxy, ethoxy, trifluoroethoxy, isopropoxy, phenoxy, benzyloxy, O-pivaloyloxymethyl, an amino acid ester and a lactate ester, and
W^{5a} is selected from the formulas:

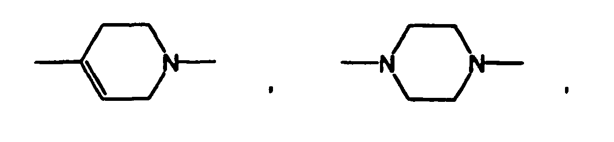

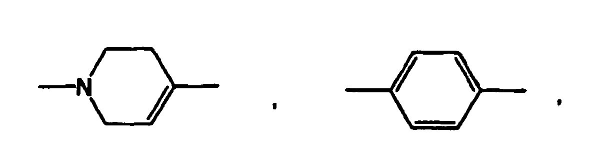

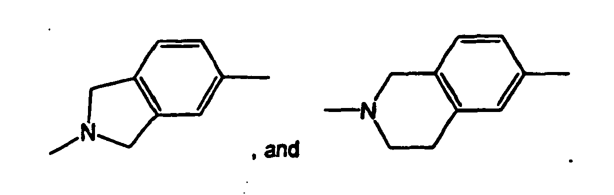

23. A compound of claim 22, wherein A¹ is selected from

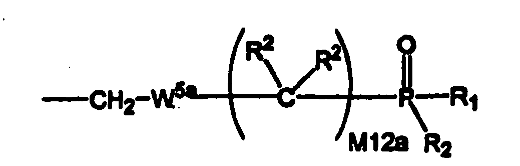

24. A compound of claim 22 or 23, wherein W^{5a} is

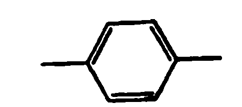

25. A compound of any one of claims 22 to 24, wherein M12a is 1, 2, 3, or 4.

26. A compound of claim 22 wherein A¹ is selected from the formulas:

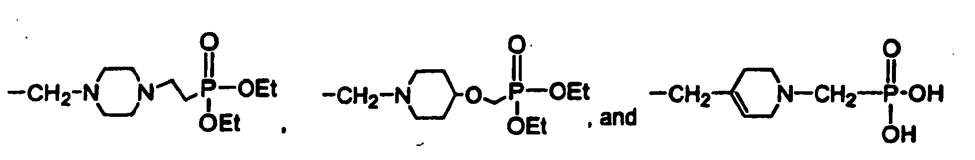

27. The use of a compound according to any one of claims 1 to 26 in the manufacture of a medicament for the treatment or prevention of the symptoms or effects of HIV infection.

28. The use of a compound according to any one of claims 1 to 26 in the manufacture of a medicament for the treatment of disorders affecting white blood cells.

29. A pharmaceutical composition comprising a compound of formulae I to IV according to anyone of claims 1 to 26 and conventional carriers and excipients and optionally other therapeutic agents, wherein in formulae I to VI R^{x} and R^{y} cannot be a protecting group.

30. The pharmaceutical composition of claim 29 comprising a second compound having anti-HIV properties.

31. The pharmaceutical composition of claim 30 wherein the second compound is selected from a nucleotide reverse transcriptase inhibitor, a non-nucleoside reverse transcriptase inhibitor, a HIV protease inhibitor, and a HIV integrase inhibitor.

## Patentansprüche

1. Verbindung, ausgewählt unter den Formeln 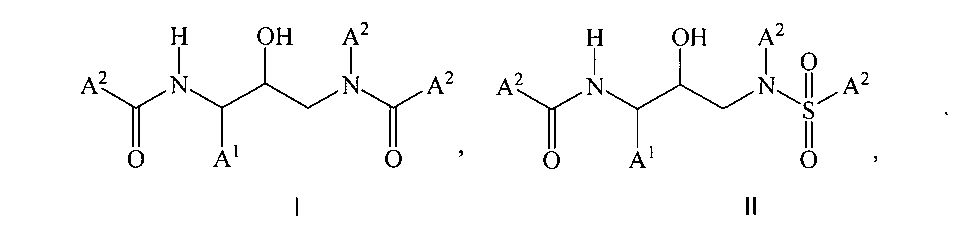 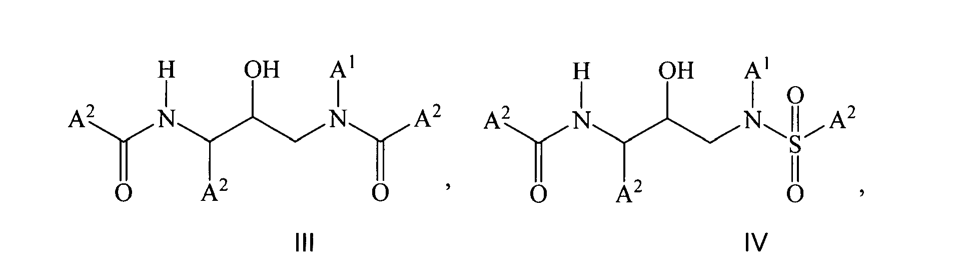 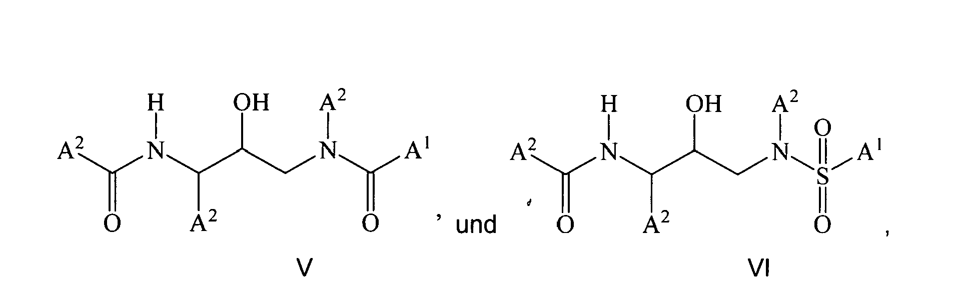 wobei
A¹ für 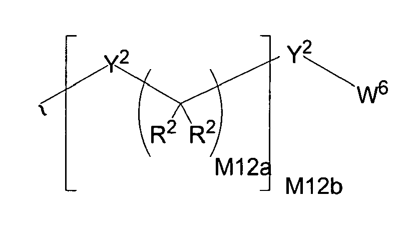 steht;
A² für 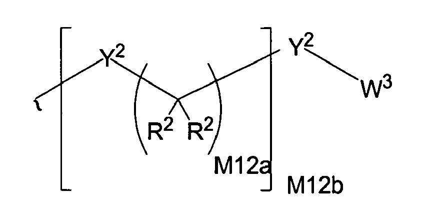 steht;
A³ für 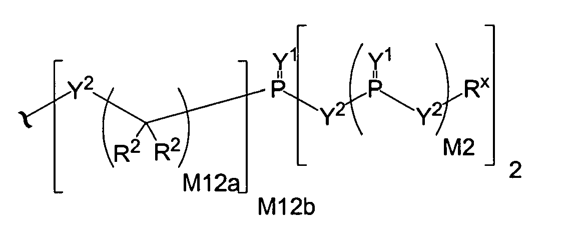 steht;
Y¹ unabhängig für O, S, N(R^{x}), N(O)(R^{x}), N(OR^{x}), N(O)(OR^{x}), oder N(N(R^{x})(R^{x})) steht;
Y² unabhängig für eine Bindung, O, N(R^{x}), N(O)(R^{x}), N(OR^{x}), N(O)(OR^{x}), N(N(R^{x})(R^{x})), -S(O)_{M2}- oder -S(O)_{M2}-S(O)_{M2}- steht;
R^{x} unabhängig für H, R¹, W³, eine Schutzgruppe oder die Formel: 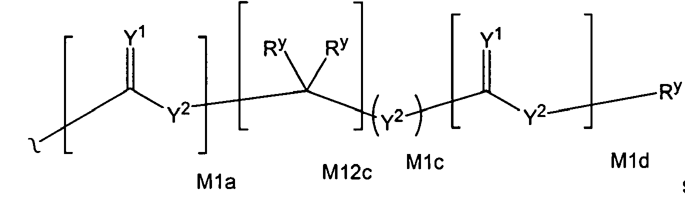 steht;
R^{y} unabhängig für H, W³, R² oder eine Schutzgruppe steht;
R¹ unabhängig für H oder einen Alkylrest mit 1 to 18 Kohlenstoffatomen steht;
R² unabhängig für H, R¹, R³ oder R⁴ steht, wobei jedes R⁴ unabhängig mit 0 bis 3 R³-Gruppen substituiert ist, oder zwei R²-Gruppen zusammen an einem Kohlenstoffatom einen Ring mit 3 bis 8 Kohlenstoffatomen bilden und der Ring mit 0 bis 3 R³-Gruppen substituiert sein kann;
R³ für R^{3a}, R^{3b}, R^{3c} oder R^{3d} steht, mit der Maßgabe, dass, wenn R³ an ein Heteroatom gebunden ist, R³ dann für R^{3c} oder R^{3d} steht;
R^{3a} für F, Cl, Br, I, -CN, N₃ oder -NO₂ steht;
R^{3b} für Y¹ steht;
R^{3c} für -R^{x}, -N(R^{x})(R^{x}), -SR^{x}, -S(O)R^{x}, -S(O)₂R^{x}, -S(O)(OR^{x}), -S(O)₂(OR^{x}), -OC(Y¹)R^{x}, -OC(Y¹)OR^{x}, -OC(Y¹)(N(R^{x})(R^{x})), -SC(Y¹)R^{x}, -SC(Y¹)OR^{x}, -SC(Y¹)(N(R^{x})(R^{x})), -N(R^{x})C(Y¹)R^{x}, -N(R^{x})C(Y¹ )OR^{x} oder -N(R^{x})C(Y¹)(N(R^{x})(R^{x}));
R^{3d} für -C(Y¹)R^{x}, -C(Y¹)OR^{x} oder -C(Y¹)(N(R^{x})(R^{x})) steht;
R⁴ für einen Alkylrest mit 1 to 18 Kohlenstoffatomen, einen Alkenylrest mit 2 to 18 Kohlenstoffatomen oder einen Alkinylrest mit 2 to 18 Kohlenstoffatomen steht;
R⁵ für R⁴ steht, wobei jedes R⁴ mit 0 bis 3 R³-Gruppen substituiert ist;
W³ für W⁴ oder W⁵ steht;
W⁴ für R⁵, -C(Y¹)R⁵, -C(Y¹)W⁵, -SO₂R⁵ oder -SO₂W⁵ steht;
W⁵ für einen Carbozyklus oder Heterozyklus steht, wobei W⁵ unabhängig mit 0 to 3 R²-Gruppen substituiert ist;
W⁶ für unabhängig mit 1, 2 oder 3 A³-Gruppen substituiertes W³ steht;
M2 für 0, 1 oder 2 steht;
M12a für 1, 2, 3, 4, 5, 6, 7, 8, 9, 10, 11 oder 12 steht;
M 12b für 0, 1, 2, 3, 4, 5, 6, 7, 8, 9, 10, 11 oder 12 steht;
M1a, M1c und M1d unabhängig für 0 oder 1 stehen; und
M12c für 0, 1, 2, 3, 4, 5, 6, 7, 8, 9, 10, 11 oder 12 steht, und
die Enantiomeren und Diastereomeren sowie die physiologisch verträglichen Salze davon.

2. Verbindung nach Anspruch 1 der Formel:

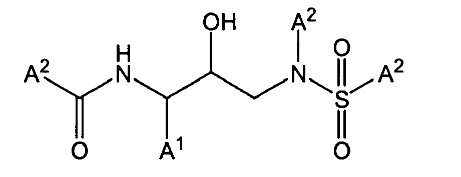

3. Verbindung nach Anspruch 1 der Formel:

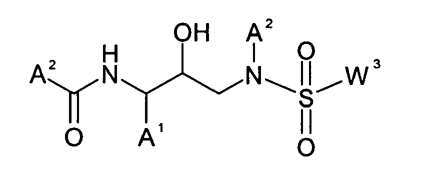

4. Verbindung nach Anspruch 3 der Formel:

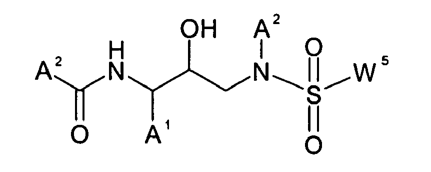

5. Verbindung nach Anspruch 4 der Formel:

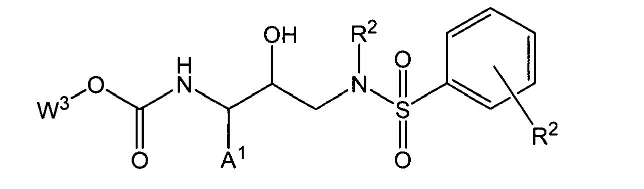

6. Verbindung nach Anspruch 5 der Formel:

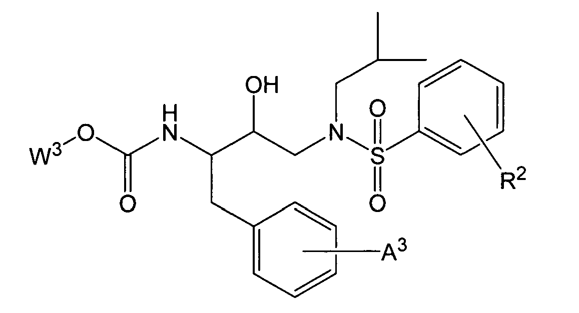

7. Verbindung nach Anspruch 6 der Formel: 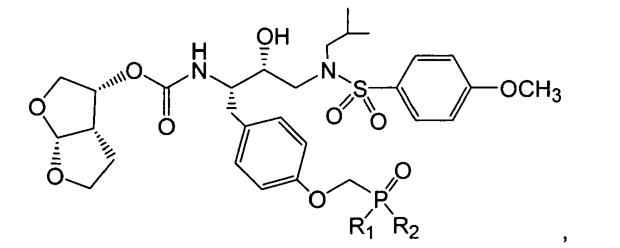 wobei R₁ und R₂ unabhängig unter Hydroxy, Methoxy, Ethoxy, Trifluorethoxy, Isopropoxy, Phenoxy, Benzyloxy und o-Pivaloyloxymethyl ausgewählt sind.

8. Verbindung nach Anspruch 6 der Formel: 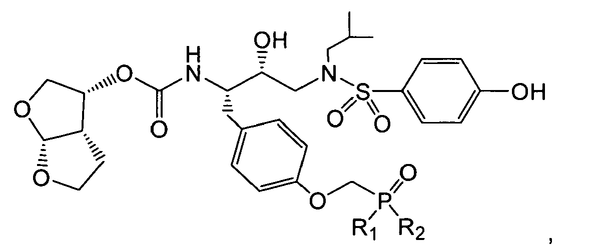 wobei R₁ und R₂ unabhängig unter Hydroxy, Methoxy, Ethoxy, Trifluorethoxy, Isopropoxy, Phenoxy, Benzyloxy und o-Pivaloyloxymethyl ausgewählt sind.

9. Verbindung nach Anspruch 6 der Formel: 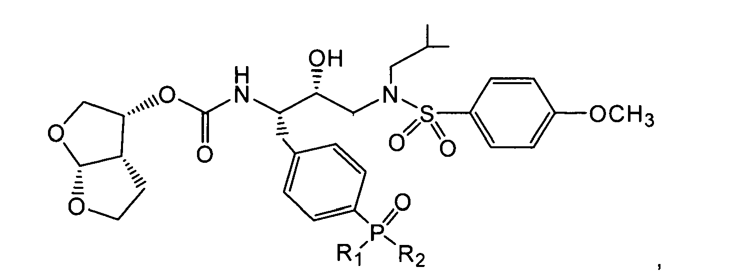 wobei R₁ und R₂ unabhängig unter Hydroxy, Methoxy, Ethoxy, Trifluorethoxy, Isopropoxy, Phenoxy, Benzyloxy und o-Pivaloyloxymethyl ausgewählt sind.

10. Verbindung nach Anspruch 6 der Formel: 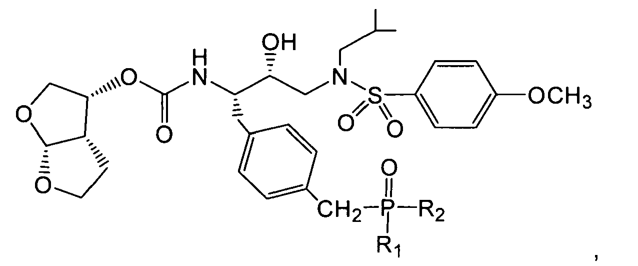 wobei R₁ und R₂ unabhängig unter Hydroxy, Methoxy, Ethoxy, Trifluorethoxy, Isopropoxy, Phenoxy, Benzyloxy und o-Pivaloyloxymethyl ausgewählt sind.

11. Verbindung nach Anspruch 6 der Formel: 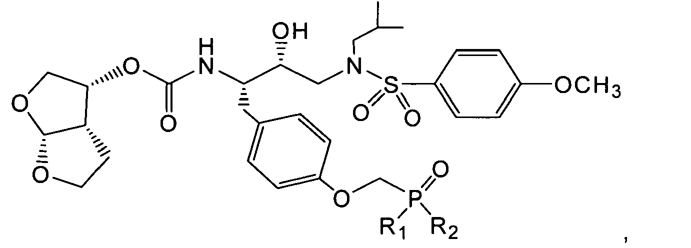 wobei R₁ und R₂ unabhängig unter -NR ausgewählt sind, wobei R für einen C₁-C₆-Alkylrest oder einen Aminosäureester steht.

12. Verbindung nach Anspruch 11, wobei R₁ und R₂ unabhängig unter-NMe, -NEt, Gly-Et, Ala-Et, Aba-Et, Val-Et, Leu-Et, Phe-Bu und Phe-Et ausgewählt sind.

13. Verbindung nach Anspruch 6 der Formel: 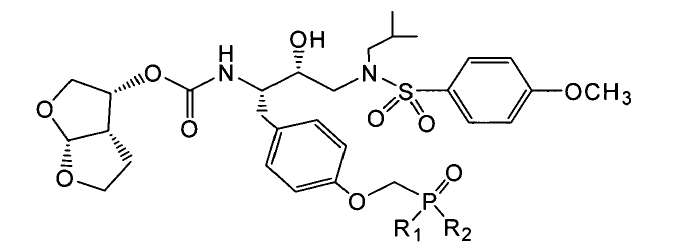 oder 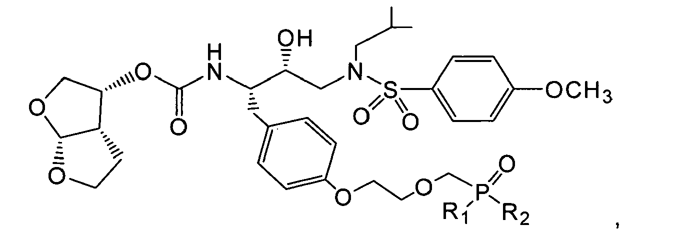 wobei R₁ und R₂ unabhängig unter Hydroxy, Methoxy, Ethoxy, Trifluorethoxy, Isopropoxy, Phenoxy, Benzyloxy, o-Pivaloyloxymethyl und einem Lactatester ausgewählt sind.

14. Verbindung nach Anspruch 13, wobei R₁ für Hydroxy, Methoxy, Ethoxy, Trifluorethoxy, Isopropoxy, Phenoxy, substitutiertes Phenoxy oder Benzyloxy; und R₂ für Glc-Et, Lac-Me, Lac-Et, Lac-iPr, Lac-Bu, Lac-EtMor, Lac-Bn, Lac-OH, Hba-Et, Hba-tBu, Hba-OH, MeBut-Et oder DiMePro-Me steht.

15. Verbindung nach Anspruch 14, wobei der Lactatester in der (R)-Konfiguration vorliegt.

16. Verbindung nach Anspruch 14, wobei der Lactatester in der (S)-Konfiguration vorliegt.

17. Verbindung nach Anspruch 6 der Formel: 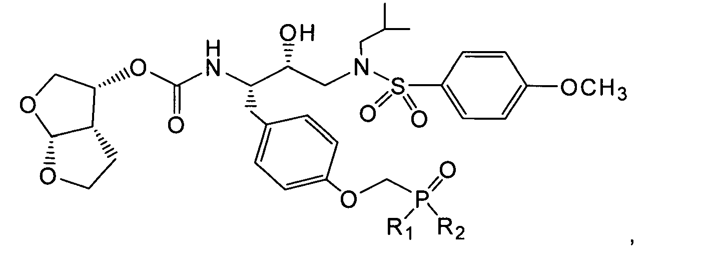 wobei R₁ für Phenoxy, Benzyloxy, Ethoxy, Trifluorethoxy oder Hydroxy; und R₂ für einen Aminosäureester steht.

18. Verbindung nach Anspruch 17, wobei der Aminosäureester unter Gly-Bu, Ala-Me, Ala-Et, Ala-iPr, (D)Ala-iPr, Ala-Bu, Aba-Et, Aba-Bu und Ala-OH ausgewählt ist.

19. Verbindung nach Anspruch 6 der Formel: 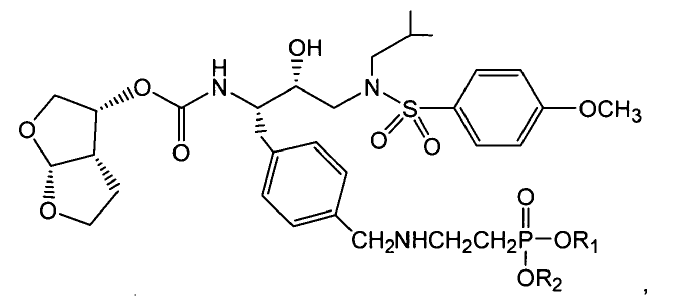 wobei R₁ und R₂ unabhängig unter Hydroxy, Methoxy, Ethoxy, Trifluorethoxy, Isopropoxy, Phenoxy, Benzyloxy, o-Pivaloyloxymethyl, einem Aminosäureester und einem Lactatester ausgewählt sind.

20. Verbindung nach Anspruch 19, wobei R₁ für Hydroxy, Methoxy, Ethoxy, Trifluorethoxy, Isopropoxy, Phenoxy, substitutiertes Phenoxy oder Benzyloxy; und R₂ für einen unter Glc-Et, Lac-Me, Lac-Et, Lac-iPr, Lac-Bu, Lac-EtMor, Lac-Bn, Lac-OH, Hba-Et, Hba-tBu, Hba-OH, MeBut-Et und DiMePro-Me ausgewählten Lactatester steht.

21. Verbindung nach Anspruch 19, wobei R₁ für Hydroxy, Methoxy, Ethoxy, Trifluorethoxy, Isopropoxy, Phenoxy, substitutiertes Phenoxy oder Benzyloxy; und R₂ für einen unter Gly-Bu, Ala-Me, Ala-Et, Ala-iPr, (D)Ala-iPr, Ala-Bu, Aba-Et, Aba-Bu und Ala-OH ausgewählten Aminosäureester steht.

22. Verbindung nach Anspruch 1 der Formel: 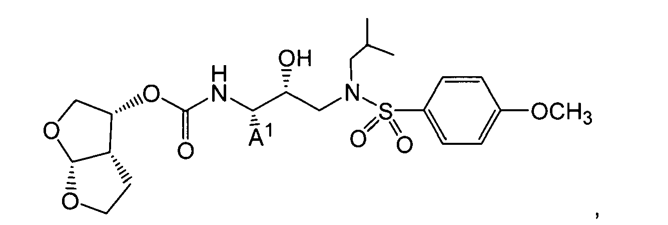 wobei A¹ unter den Formeln: 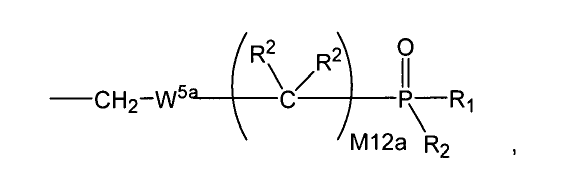 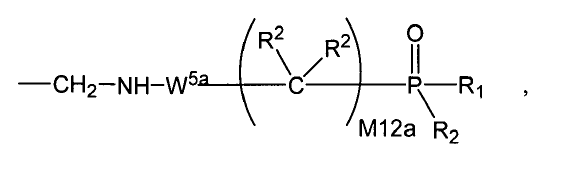 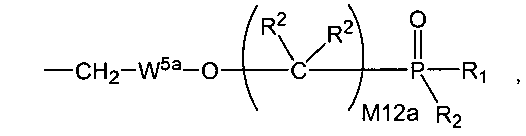 und 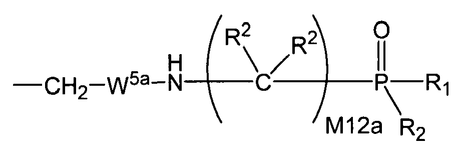 ausgewählt ist,
wobei R₁ und R₂ unabhängig unter Hydroxy, Methoxy, Ethoxy, Trifluorethoxy, Isopropoxy, Phenoxy, Benzyloxy, o-Pivaloyloxymethyl, einem Aminosäureester und einem Lactatester ausgewählt sind, und
W^{5a} unter den Formeln: 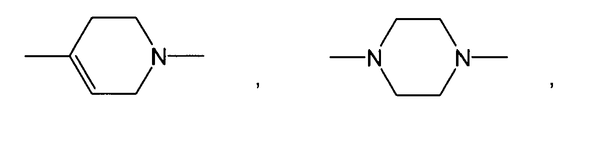 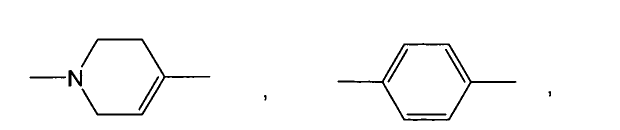 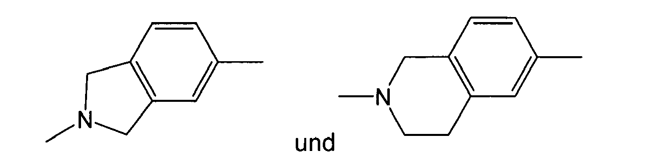 ausgewählt ist.

23. Verbindung nach Anspruch 22, wobei A¹ unter 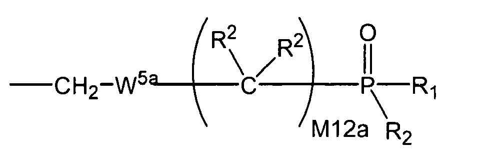 ausgewählt ist.

24. Verbindung nach Anspruch 22 oder 23, wobei W^{5a} für 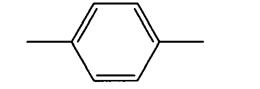 steht.

25. Verbindung nach einem der Ansprüche 22 bis 24, wobei M12a für 1, 2, 3 oder 4 steht.

26. Verbindung nach Anspruch 22, wobei A¹ unter den Formeln: 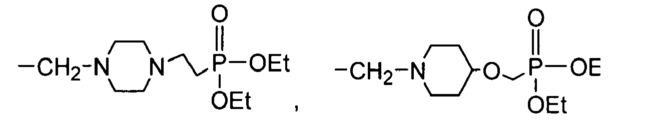 und 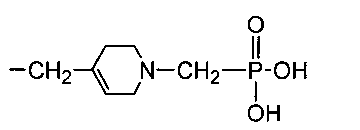 ausgewählt ist.

27. Verwendung einer Verbindung nach einem der Ansprüche 1 bis 26 zur Herstellung eines Medikamentes zur Behandlung oder Prävention der Symptome oder Wirkungen einer HIV-Infektion.

28. Verwendung einer Verbindung nach einem der Ansprüche 1 bis 26 zur Herstellung eines Medikamentes zur Behandlung von Erkrankungen, die Leukozyten betreffen.

29. Pharmazeutische Zusammensetzung, umfassend eine Verbindung der Formeln I bis IV nach einem der Ansprüche 1 bis 26 und herkömmliche Träger und Hilfsstoffe und gewünschtenfalls andere therapeutische Mittel, wobei in den Formeln I to VI R^{x} und R^{y} nicht für Schutzgruppen stehen können.

30. Pharmazeutische Zusammensetzung nach Anspruch 29, enthaltend eine zweite Verbindung mit Anti-HIV-Eigenschaften.

31. Pharmazeutische Zusammensetzung nach Anspruch 30, wobei die zweite Verbindung unter einem Nukleotid-Reverse Transkriptase-Inhibitor, einem Nicht-Nukleotid-Reverse Transkriptase-Inhibitor, einem HIV-Protease-Inhibitor und einem HIV-Integrase-Inhibitor ausgewählt ist.

## Revendications

1. Composé choisi parmi les formules 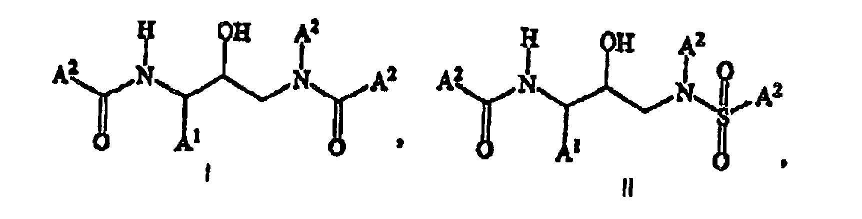 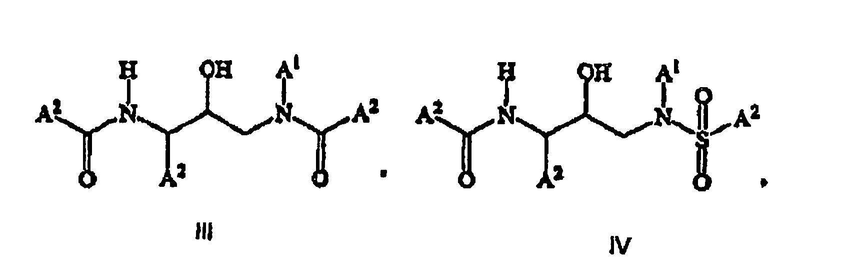 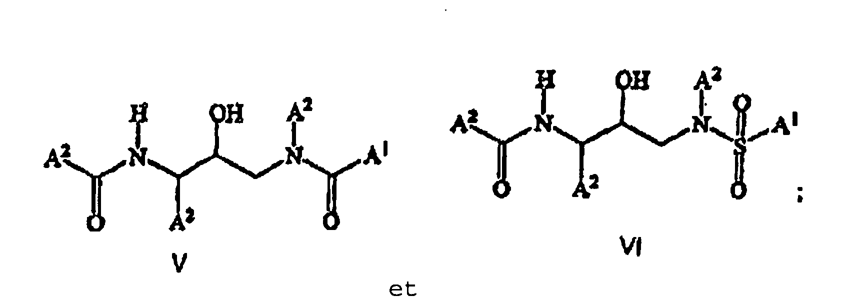 dans lequel:
A¹ est: 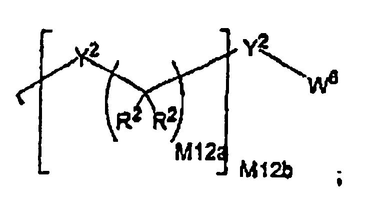
A² est: 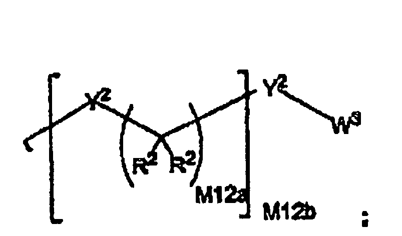
A³ est: 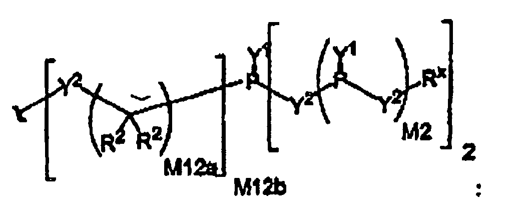
Y¹ est indépendamment O, S, N (R^{x}), N(O) (R^{x}), N (OR^{x}), N (O) (OR^{x}), ou N (N (R^{x}) (R^{x}));
Y² est indépendamment une liaison, O, N(R^{x}), N(O) (R^{x}), N (OR^{x}), N (O) (OR^{x}) , N (N (R^{x}) (R^{x})), -S (O)_{M2}-, ou -S (O) _{M2}-S (O) _{M2} - ;
R^{x} est indépendamment H, R¹, W³, un groupe protecteur, ou la formule: 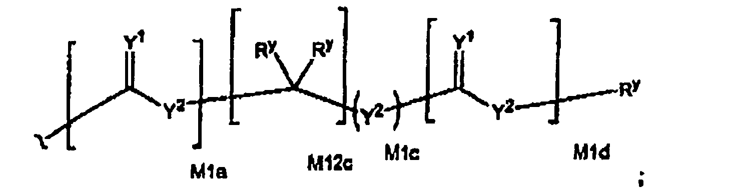
R^{y} est indépendamment H, W³, R² ou un groupe protecteur;
R¹ est indépendamment H ou un alkyle de 1 à 18 atomes de carbone;
R² est indépendamment H, R¹, R³ ou R⁴ où chaque R⁴ est indépendamment substitué avec 0 à 3 groupes R³, ou conjointement avec un atome de carbone, deux groupes R² forment un cycle de 3 à 8 carbones et le cycle peut être substitué avec 0 à 3 groupes R³;
R³ est R^{3a}, R^{3b}, R^{3c} ou R^{3d}, à condition que lorsque R³ est lié à un hétéroatome, alors R³ est R^{3c} ou R^{3d} ;
R^{3a} est F, Cl, Br, I, -CN, N₃ ou -NO₂ ;
R^{3b} est Y¹ ;
R^{3c} est -R^{x}, N(R^{x}) (R^{x}), -SR^{x}, -S (O) R^{x}, -S(O)₂R^{x}, -S (O) (OR^{x}), -S(O)₂(OR^{x}), -OC (Y¹) R^{x}, -OC(Y¹)OR^{x}, -OC (Y¹) (N (R^{x}) (R^{x})), -SC (Y¹) R^{x}, -SC (Y¹) OR^{x}, -SC (Y¹) (N (R^{x}) (R^{x})), -N (R^{x}) C (Y¹) R^{x}, -N (R^{x}) C (Y¹) OR^{x}, ou -N (R^{x}) C (Y¹) (N (R^{x}) (R^{x})) ;
R^{3d} est -C (Y¹) R^{x}, -C(Y¹)OR^{x} ou -C(Y¹) (N(R^{x}) (R^{x}));
R⁴ est un alkyle de 1 à 18 atomes de carbone, un alcényle de 2 à 18 atomes de carbone, ou un alcynyle de 2 à 18 atomes de carbone;
R⁵ est R⁴ où chaque R⁴ est substitué avec 0 à 3 groupes R³;
W³ est W⁴ ou W⁵ ;
W⁴ est R⁵, -C(Y¹)R⁵, -C(Y¹)W⁵, -SO₂R⁵, ou -SO₂W⁵;
W⁵ est un carbocycle ou un hétérocycle dans lequel W⁵ est indépendamment substitué avec 0 à 3 groupes R²;
W⁶ est W³ indépendamment substitué avec 1, 2 ou 3 groupes A³ ;
M2 est 0, 1 ou 2;
M12a est 1, 2, 3, 4, 5, 6, 7, 8, 9, 10, 11 ou 12;
M12b est 0, 1, 2, 3, 4, 5, 6, 7, 8, 9, 10, 11 ou 12;
M1a, M1c et M1d sont indépendamment 0 ou 1; et
M12c est 0, 1, 2, 3, 4, 5, 6, 7, 8, 9, 10, 11 ou 12; et
les énantiomères et diastéréo-isomères, ainsi que les sels physiologiquement acceptables de celui-ci.

2. Composé selon la revendication 1 ayant la formule:

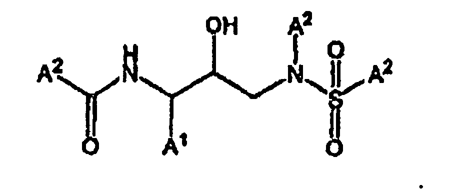

3. Composé selon la revendication 1 ayant la formule:

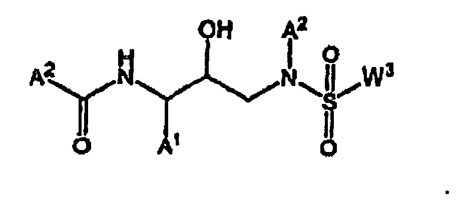

4. Composé selon la revendication 3 ayant la formule:

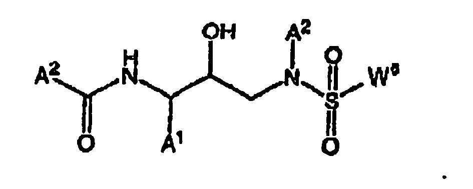

5. Composé selon la revendication 4 ayant la formule:

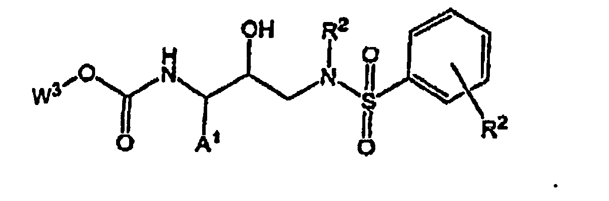

6. Composé selon la revendication 5 ayant la formule:

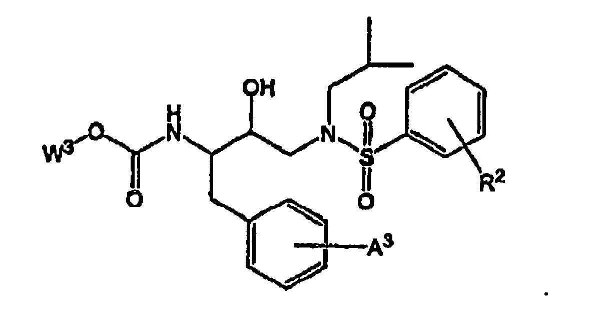

7. Composé selon la revendication 6 ayant la formule: 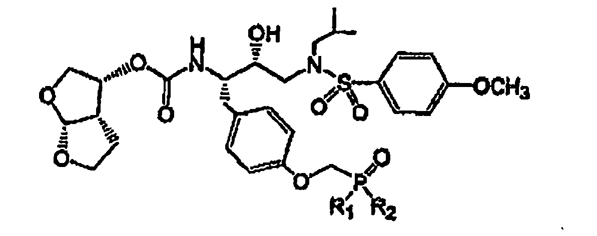 dans lequel R¹ et R² sont indépendamment choisis parmi un hydroxy, un méthoxy, un éthoxy, un trifluoroéthoxy, un isopropoxy, un phénoxy, un benzyloxy, et un O-pivaloyloxyméthyle.

8. Composé selon la revendication 6 ayant la formule: 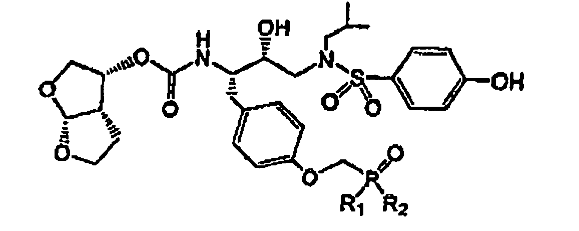 dans lequel R₁ et R₂ sont indépendamment choisis parmi un hydroxy, un méthoxy, un éthoxy, un trifluoroéthoxy, un isopropoxy, un phénoxy, un benzyloxy, et un O-pivaloyloxyméthyle.

9. Composé selon la revendication 6 ayant la formule: 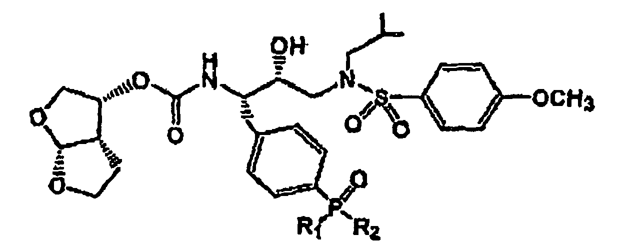 dans lequel R₁ et R₂ sont indépendamment choisis parmi un hydroxy, un méthoxy, un éthoxy, un trifluoroéthoxy, un isopropoxy, un phénoxy, un benzyloxy, et un O-pivaloyloxyméthyle.

10. Composé selon la revendication 6 ayant la formule: 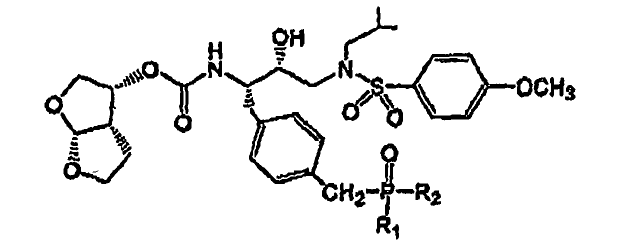 dans lequel R₁ et R₂ sont indépendamment choisis parmi un hydroxy, un méthoxy, un éthoxy, un trifluoroéthoxy, un isopropoxy, un phénoxy, un benzyloxy, et un O-pivaloyloxyméthyle.

11. Composé selon la revendication 6 ayant la formule: 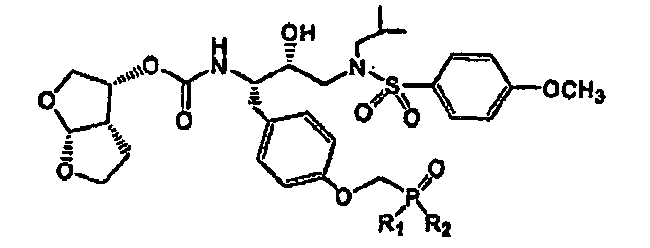 dans lequel R₁ et R₂ sont indépendamment choisis parmi -NR où R est un alkyle en C₁-C₆ ou un ester d'acide aminé.

12. Composé selon la revendication 11 dans lequel R₁ et R₂ sont indépendamment choisis parmi -NMe, -NEt, Gly-Et, Ala-Et, Aba-Et, Val-Et, Leu-Et, Phe-Bu, et Phe-Et.

13. Composé selon la revendication 6 ayant la formule: 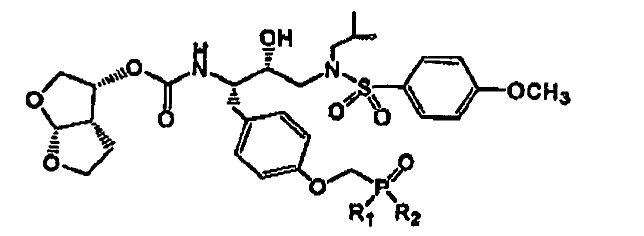 ou 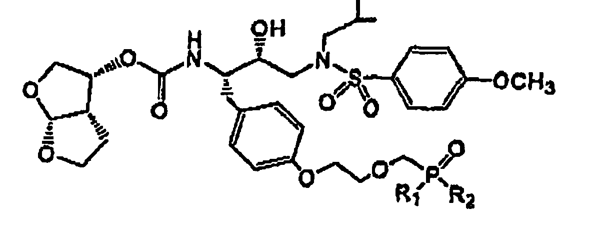 dans lequel R₁ et R₂ sont indépendamment choisis parmi un hydroxy, un méthoxy, un éthoxy, un trifluoroéthoxy, un isopropoxy, un phénoxy, un benzyloxy, un O-pivaloyloxyméthyle et un ester de lactate.

14. Composé selon la revendication 13 dans lequel R¹ est un hydroxy, un méthoxy, un éthoxy, un trifluoroéthoxy, un isopropoxy, un phénoxy, un phénoxy substitué ou un benzyloxy; et
R₂ est Glc-Et, Lac-Me, Lac-Et, Lac-iPr, Lac-Bu, Lac-EtMor, Lac-Me, Lac-Et, Lac-Bn, Lac-OH, Hba-Et, HBa-tBu, Hba-OH, MeBut-Et, ou DiMePro-Me.

15. Composé selon la revendication 14 où l'ester de lactate est la configuration (R).

16. Composé selon la revendication 14 où l'ester de lactate est la configuration (S).

17. Composé selon la revendication 6 ayant la formule: 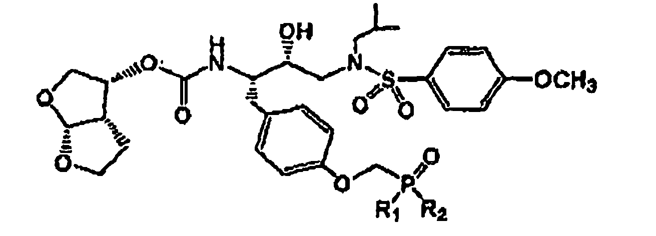 dans lequel R₁ est un phénoxy, un benzyloxy, un éthoxy, un trifluoroéthoxy, ou un hydroxyle; et
R₂ est un ester d'acide aminé.

18. Composé selon la revendication 17 dans lequel l'ester d'acide aminé est choisi parmi Gly-Bu, Ala-Me, Ala--Et, Ala-iPr, (D)Ala-iPr, Ala-Bu, Aba-Et, Aba-Bu, et Ala-OH.

19. Composé selon la revendication 6 ayant la formule: 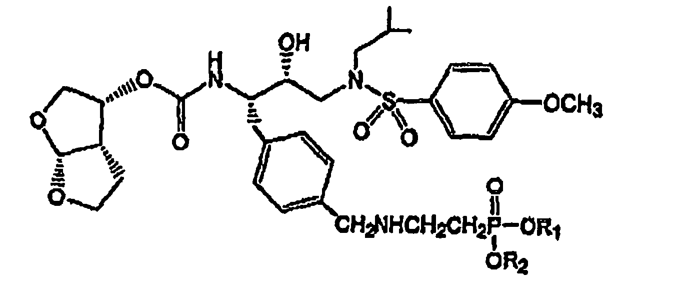 dans lequel R₁ et R₂ sont indépendamment choisis parmi un hydroxy, un méthoxy, un éthoxy, un trifluoroéthoxy, un isopropoxy, un phénoxy, un benzyloxy, un O-pivaloyloxyméthyle, un ester d'acide aminé et un ester de lactate.

20. Composé selon la revendication 19 dans lequel R₁ est un hydroxy, un méthoxy, un éthoxy, un trifluoroéthoxy, un isopropoxy, un phénoxy, un phénoxy substitué ou un benzyloxy; et
R₂ est un ester de lactate choisi parmi Glc-Et, Lac-Me, Lac-Et, Lac-iPr, Lac-Bu, Lac-EtMor, Lac-Me, Lac-Et, Lac-Bn, Lac-OH, Hba-Et, HBa-tBu, Hba-OH, MeBut-Et et DiMePro-Me.

21. Composé selon la revendication 19 dans lequel R₁ est un hydroxy, un méthoxy, un éthoxy, un trifluoroéthoxy, un isopropoxy, un phénoxy, un phénoxy substitué ou un benzyloxy; et
R₂ est un ester d'acide aminé choisi parmi Gly-Bu, Ala-Me, Ala-Et, Ala-iPr, (D)Ala-iPr, Ala-Bu, Aba-Et, Aba-Bu, et Ala-OH.

22. Composé selon la revendication 1 ayant la formule: 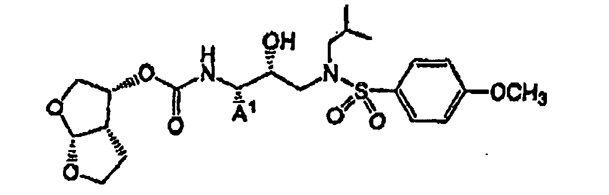 dans lequel A¹ est choisi parmi les formules: 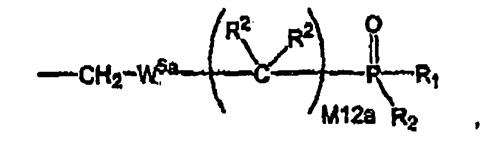 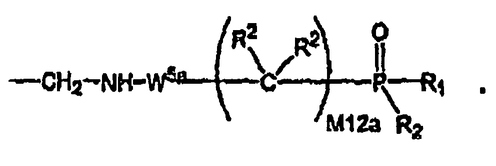 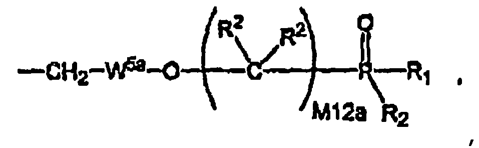 et 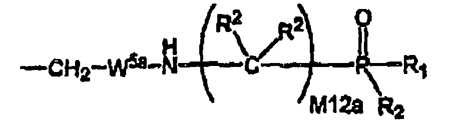 R₁ et R₂ sont indépendamment choisis parmi un hydroxy, un méthoxy, un éthoxy, un trifluoroéthoxy, un isopropoxy, un phénoxy, un benzyloxy, un O-pivaloyloxyméthyle, un ester d'acide aminé et un ester de lactate; et
W^{5a} est choisi parmi les formules:

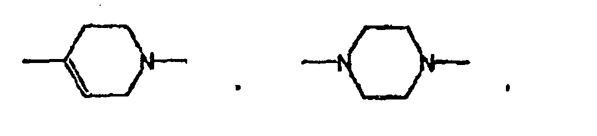

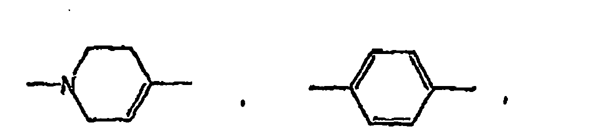

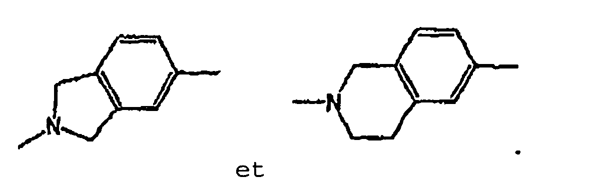

23. Composé selon la revendication 22, dans lequel A¹ est choisi parmi

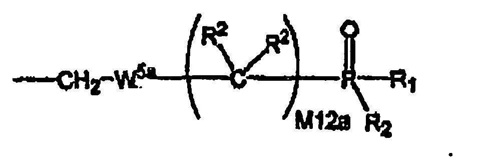

24. Composé selon la revendication 22 ou 23, dans lequel W^{5a} est

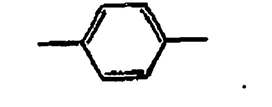

25. Composé selon l'une quelconque des revendications 22 à 24, dans lequel M12a est 1, 2, 3, ou 4.

26. Composé selon la revendication 22 dans lequel A¹ est choisi parmi les formules:

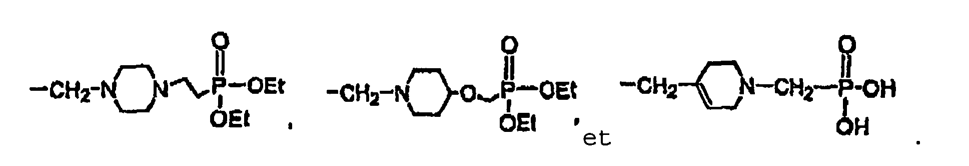

27. Utilisation d'un composé selon l'une quelconque des revendications 1 à 26 dans la fabrication d'un médicament pour le traitement ou la prévention des symptômes ou effets de l'infection par le VIH.

28. Utilisation d'un composé selon l'une quelconque des revendications 1 à 26 dans la fabrication d'un médicament pour le traitement de troubles affectant les globules blancs.

29. Composition pharmaceutique comprenant un composé de formules I à IV selon l'une quelconque des revendications 1 à 26 et des véhicules et excipients conventionnels et facultativement d'autres agents thérapeutiques, où, dans les formules I à VI, R^{x} et R^{y} ne peuvent pas être un groupe protecteur.

30. Composition pharmaceutique selon la revendication 29 comprenant un second composé ayant des propriétés anti-VIH.

31. Composition pharmaceutique selon la revendication 30 dans laquelle le second composé est choisi parmi un inhibiteur de transcriptase inverse de nucléotide, un inhibiteur de transcriptase inverse non-nucléoside, un inhibiteur de protéase de VIH, et un inhibiteur d'intégrase de VIH.
